(19) Europäisches Patentamt
European Patent Office
Office européen des brevets

(11) EP 4 332 100 A1

(12) **EUROPEAN PATENT APPLICATION**
published in accordance with Art. 153(4) EPC

(43) Date of publication:
**06.03.2024 Bulletin 2024/10**

(21) Application number: **22795017.7**

(22) Date of filing: **29.04.2022**

(51) International Patent Classification (IPC):
***C07D 401/14*** *(2006.01)* ***C07F 9/53*** *(2006.01)*
***A61K 31/506*** *(2006.01)* ***A61K 31/675*** *(2006.01)*
***A61P 35/00*** *(2006.01)*

(52) Cooperative Patent Classification (CPC):
**A61K 31/506; A61K 31/675; A61P 35/00; C07D 401/14; C07F 9/53**

(86) International application number:
**PCT/CN2022/090243**

(87) International publication number:
**WO 2022/228547 (03.11.2022 Gazette 2022/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:
**30.04.2021 CN 202110470748**
**25.05.2021 CN 202110570092**
**11.06.2021 CN 202110651028**
**22.07.2021 CN 202110824204**
**03.09.2021 CN 202111025788**
**22.10.2021 CN 202111214457**
**06.01.2022 CN 202210000254**

(71) Applicant: **Xizang Haisco Pharmaceutical Co., Ltd.**
**Lhoka, Tibet 856099 (CN)**

(72) Inventors:
- **ZHANG, Chen**
  **Chengdu, Sichuan 611130 (CN)**
- **WANG, Jianmin**
  **Chengdu, Sichuan 611130 (CN)**
- **ZHAO, Chenfei**
  **Chengdu, Sichuan 611130 (CN)**
- **QIAN, Guofei**
  **Chengdu, Sichuan 611130 (CN)**
- **MA, Junjie**
  **Chengdu, Sichuan 611130 (CN)**
- **HUANG, Zhenggang**
  **Chengdu, Sichuan 611130 (CN)**
- **YUAN, Shuai**
  **Chengdu, Sichuan 611130 (CN)**
- **HUANG, Anbang**
  **Chengdu, Sichuan 611130 (CN)**
- **ZHENG, Shaolong**
  **Chengdu, Sichuan 611130 (CN)**
- **LI, Kai**
  **Chengdu, Sichuan 611130 (CN)**
- **YU, Yan**
  **Chengdu, Sichuan 611130 (CN)**
- **YE, Fei**
  **Chengdu, Sichuan 611130 (CN)**
- **TANG, Pingming**
  **Chengdu, Sichuan 611130 (CN)**
- **LI, Yao**
  **Chengdu, Sichuan 611130 (CN)**
- **NI, Jia**
  **Chengdu, Sichuan 611130 (CN)**
- **YAN, Pangke**
  **Chengdu, Sichuan 611130 (CN)**

(74) Representative: **Hoffmann Eitle**
**Patent- und Rechtsanwälte PartmbB**
**Arabellastraße 30**
**81925 München (DE)**

(54) **PHOSPHONYL DERIVATIVE, AND COMPOSITION AND PHARMACEUTICAL APPLICATION THEREOF**

(57) Provided are the compound shown in general formula (I) or a stereoisomer, a deuterated compound, a solvate, a prodrug, a metabolite, a pharmaceutically acceptable salt, or co-crystal thereof, an intermediate thereof, and a use thereof in EGFR-related diseases such as cancer. B-L-K (I)



Processed by Luminess, 75001 PARIS (FR)

## Description

### Technical Field

The present invention relates to a compound shown in general formula (I) or a stereoisomer, a deuterated compound, a solvate, a prodrug, a metabolite, a pharmaceutically acceptable salt or a co-crystal thereof, and an intermediate thereof and a preparation method therefor, as well as the use thereof in EGFR-related diseases such as cancer.

### Background Art

Epidermal growth factor receptor (EGFR) is a transmembrane protein tyrosine kinase that can act as a receptor for EGF family members to trigger the EGFR signalling pathway in human epithelial cells, thereby regulating cell proliferation, invasion, metastasis, apoptosis and angiogenesis *(*Nat. Rev. Cancer, 2007, 7, 169-181; Expert Opin. Ther. Targets, 2012, 16, 15-31). The overexpression, mutation or amplification of EGFR genes in the human body leads to an abnormal increase in EGFR activity, which may cause many malignant tumours such as oesophageal cancer, glioblastoma, anal cancer, head and neck epithelial cancer, breast cancer, and lung cancer, especially non-small cell lung cancer (NSCLC) *(*Cells, 2019, 8, 350-361).

PROTAC (proteolysis targeting chimera) molecules are a class of bifunctional compounds that can simultaneously bind targeting proteins and E3 ubiquitin ligases. Such compounds can be recognized by proteasomes of cells, causing the degradation of targeting proteins, and can effectively reduce the content of targeting proteins in the cells. By introducing a ligand capable of binding to various targeting proteins into PROTAC molecules, it is possible to apply the PROTAC technology to the treatment of various diseases, and this technology has attracted extensive attention in recent years (ACS Chem. Biol. 2017,12, 892-898; Drug Discovery Today Technol. 2019, 31, 15-27).

The development of novel PROTAC drugs that bind EGFR proteins and E3 ubiquitin ligases for the treatment of EGFR protein-related diseases will have great application prospects.

### Summary of the Invention

An objective of the present invention is to provide a compound with a novel structure, good efficacy, high bioavailability and higher safety that can inhibit and degrade EGFR, for use in the treatment of EGFR-related diseases such as cancer.

The present invention provides a compound or a stereoisomer, a deuterated compound, a solvate, a prodrug, a metabolite, a pharmaceutically acceptable salt or a co-crystal thereof, wherein, the compound is selected from a compound shown in general formula (I),

B-L-K (I);

in some embodiments, L is selected from a bond or $-C_{1-50}$ hydrocarbyl-, wherein the hydrocarbyl has 0 to 20 methylene units optionally further replaced by -Ak- or -Cy-;
in some embodiments, L is selected from a bond or $-C_{1-20}$ hydrocarbyl-, wherein the hydrocarbyl has 0 to 20 methylene units optionally further replaced by -Ak- or -Cy-;
in some embodiments, L is selected from a bond or $-C_{1-10}$ hydrocarbyl-, wherein the hydrocarbyl has 0 to 10 (such as 0, 1, 2, 3, 4, 5, 6, 7, 8, 9 or 10) methylene units optionally further replaced by -Ak- or -Cy-;
in some embodiments, each -Ak- is independently selected from Ak1, Ak2, Ak3, Ak4 or Ak5;
in some embodiments, each -Ak- is independently selected from $-(CH_2)_q-$, - $(CH_2)_q$-O-, -O-$(CH_2)_q$-, $-(CH_2)_q-NR^L-$, $-NR^L-(CH_2)_q-$, $-(CH_2)_q-NR^LC(=O)-$, - $NR^L(CH_2)_qC(=O)-$, $-(CH_2)_q-C(=O)NR^L-$, -C(=O)-, $-C(=O)-(CH_2)_q-NR^L-$, $-(C\equiv C)_q-$, -CH=CH-, $-Si(R^L)_2-$, $-Si(OH)(R^L)-$, $-Si(OH)_2-$, $-P(=O)(OR^L)-$, $-P(=O)(R^L)-$, -S-, - S(=O)-, $-S(=O)_2-$ or a bond, wherein the $-CH_2-$ is optionally further substituted with 0 to 2 (such as 0, 1 or 2) substituents selected from H, halogen, OH, CN, $NH_2$, $C_{1-6}$ alkyl, $C_{1-6}$ alkoxy, halogen-substituted $C_{1-6}$ alkyl, hydroxyl-substituted $C_{1-6}$ alkyl or cyano-substituted $C_{1-6}$ alkyl;
in some embodiments, each -Cy- is independently selected from Cy1, Cy2, Cy3, Cy4 or Cy5;
in some embodiments, each -Cy- is independently selected from a bond, a 4-to 8-membered mono-heterocyclic ring, a 4- to 10-membered fused-heterocyclic ring, a 5- to 12-membered spiro-heterocyclic ring, a 7- to 10-membered bridged-heterocyclic ring, 3- to 7-membered monocycloalkyl, 4- to 10-membered fused cycloalkyl, 5- to 12-membered spiro cycloalkyl, 7- to 10-membered bridged cycloalkyl, 5- to 10-membered heteroaryl or 6- to 10-membered aryl, wherein the aryl, heteroaryl, cycloalkyl, mono-heterocyclic ring, fused-heterocyclic ring, spiro-heterocyclic ring or bridged-heterocyclic ring is optionally further substituted with 0 to 4 (such as 0, 1, 2, 3 or 4) substituents selected

from H, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the heteroaryl, mono-heterocyclic ring, fused-heterocyclic ring, spiro-heterocyclic ring or bridged-heterocyclic ring contains 1 to 4 (such as 1, 2, 3 or 4) heteroatoms selected from O, S or N, and is optionally further substituted with 0, 1 or 2 =O when the heteroatom is selected from S;

in some embodiments, each Cy1, Cy2, Cy3, Cy4 or Cy5 is independently selected from a bond, a 4- to 7-membered mono-heterocyclic ring, a 4- to 10-membered fused-heterocyclic ring, a 5- to 12-membered spiro-heterocyclic ring, a 7- to 10-membered bridged-heterocyclic ring, 3- to 7-membered monocycloalkyl, 4- to 10-membered fused cycloalkyl, 5- to 12-membered spiro cycloalkyl, 7- to 10-membered bridged cycloalkyl, 5- to 10-membered heteroaryl or 6- to 10-membered aryl, wherein the aryl, heteroaryl, cycloalkyl, mono-heterocyclic ring, fused-heterocyclic ring, spiro-heterocyclic ring or bridged-heterocyclic ring is optionally further substituted with 0 to 4 (such as 0, 1, 2, 3 or 4) substituents selected from H, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the heteroaryl, mono-heterocyclic ring, fused-heterocyclic ring, spiro-heterocyclic ring or bridged-heterocyclic ring contains 1 to 4 (such as 1, 2, 3 or 4) heteroatoms selected from O, S or N, and is optionally further substituted with 0, 1 or 2 =O when the heteroatom is selected from S;

in some embodiments, each Cy1, Cy2, Cy3, Cy4 or Cy5 is independently selected from a bond, a 4- to 7-membered nitrogen-containing mono-heterocyclic ring, a 4- to 10-membered nitrogen-containing fused-heterocyclic ring, a 5- to 12-membered nitrogen-containing spiro-heterocyclic ring, a 7- to 10-membered nitrogen-containing bridged-heterocyclic ring, 3- to 7-membered monocycloalkyl, 4- to 10-membered fused cycloalkyl, 5- to 12-membered spiro cycloalkyl, 7- to 10-membered bridged cycloalkyl, 5- to 10-membered heteroaryl or 6- to 10-membered aryl, wherein the mono-heterocyclic ring, fused-heterocyclic ring, bridged-heterocyclic ring, spiro-heterocyclic ring, cycloalkyl, aryl or heteroaryl is optionally further substituted with 0 to 4 (such as 0, 1, 2, 3 or 4) substituents selected from H, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the mono-heterocyclic ring, fused-heterocyclic ring, bridged-heterocyclic ring, spiro-heterocyclic ring or heteroaryl contains 1 to 4 (such as 1, 2, 3 or 4) heteroatoms selected from O, S or N, and is optionally further substituted with 0, 1 or 2 =O when the heteroatom is selected from S;

in some embodiments, L is selected from -Cy1-Ak1-Cy2-Ak2-Cy3-Ak3-Cy4-Ak4-Cy5-Ak5-, -Cy1-Cy2-Cy3-Cy4-Ak1-Ak2-Ak3-Ak4-Ak5-, -Cy1-Ak1-Cy2-Ak2-Cy3-Ak3-Cy4-Ak4-Ak5-, -Ak1-Cy1-Ak2-Cy2-Ak3-Cy3-Ak4-Cy4-Ak5-, -Cy1-Ak1-Cy2-Ak2-Cy3-Cy4-Ak3-Ak4-Ak5-, -Cy1-Ak1-Cy2-Ak2-Ak3-Cy3-Cy4-Ak4-Ak5-, -Cy1-Ak1-Ak2-Ak3-Ak4-Ak5-Cy2-Cy3-Cy4-, -Cy1-Cy2-Ak1-Ak2-Ak3-Ak4-Ak5-Cy3-Cy4-, -Cy1-Cy2-Cy3-Ak1-Ak2-Ak3-Ak4-Ak5-Cy4-, -Cy1-Cy2-Cy3-Cy4-Ak1-Ak2-Ak3-Ak4-Ak5-, -Cy1-Ak1-Cy2-Cy3-Cy4-Ak2-Ak3-Ak4-Ak5-, -Cy1-Cy2-Ak1-Cy3-Cy4-Ak2-Ak3-Ak4-Ak5-, -Cy1-Cy2-Cy3-Ak1-Cy4-Ak2-Ak3-Ak4-Ak5-, -Cy1-Ak1-Ak2-Cy2-Cy3-Cy4-Ak3-Ak4-Ak5-, -Cy1-Cy2-Ak1-Ak2-Cy3-Cy4-Ak3-Ak4-Ak5-, -Cy1-Cy2-Cy3-Ak1-Ak2-Cy4-Ak3-Ak4-Ak5-, -Cy1-Ak1-Ak2-Ak3-Cy2-Cy3-Cy4-Ak4-Ak5-, -Cy1-Cy2-Ak1-Ak2-Ak3-Cy3-Cy4-Ak4-Ak5-, -Cy1-Cy2-Cy3-Ak1-Ak2-Ak3-Cy4-Ak4-Ak5-, -Cy1-Ak1-Ak2-Ak3-Ak4-Cy2-Cy3-Cy4-Ak5-, -Cy1-Cy2-Ak1-Ak2-Ak3-Ak4-Cy3-Cy4-Ak5-, - Cy1-Cy2-Cy3-Ak1-Ak2-Ak3-Ak4-Cy4-Ak5-, -Ak1-Ak2-Ak3-Ak4-Ak5-Cy1-Cy2-Cy3-Cy4-, -Ak1-Cy1-Cy2-Cy3-Cy4-Ak2-Ak3-Ak4-Ak5-, -Ak1-Ak2-Cy1-Cy2-Cy3-Cy4-Ak3-Ak4-Ak5-, -Ak1-Ak2-Ak3-Cy1-Cy2-Cy3-Cy4-Ak4-Ak5-, -Ak1-Ak2-Ak3-Ak4-Cy1-Cy2-Cy3-Cy4-Ak5-, -Ak1-Cy1-Ak2-Ak3-Ak4-Ak5-Cy2-Cy3-Cy4-, -Ak1-Cy1-Cy2-Ak2-Ak3-Ak4-Ak5-Cy3-Cy4-, -Ak1-Cy1-Cy2-Cy3-Ak2-Ak3-Ak4-Ak5-Cy4-, -Ak1-Ak2-Cy1-Ak3-Ak4-Ak5-Cy2-Cy3-Cy4-, -Ak1-Ak2-Cy1-Cy2-Ak3-Ak4-Ak5-Cy3-Cy4-, -Ak1-Ak2-Cy1-Cy2-Cy3-Ak3-Ak4-Ak5-Cy4-, -Ak1-Ak2-Ak3-Cy1-Ak4-Ak5-Cy2-Cy3-Cy4-, -Ak1-Ak2-Ak3-Cy1-Cy2-Ak4-Ak5-Cy3-Cy4-, -Ak1-Ak2-Ak3-Cy1-Cy2-Cy3-Ak4-Ak5-Cy4-, -Ak1-Ak2-Ak3-Ak4-Cy1-Ak5-Cy2-Cy3-Cy4-, -Ak1-Ak2-Ak3-Ak4-Cy1-Cy2-Ak5-Cy3-Cy4-, or - Ak1-Ak2-Ak3-Ak4-Cy1-Cy2-Cy3-Ak5-Cy4-;

in some embodiments, L is selected from -Cy1-, -Cy1-Ak1-, -Cy1-Ak1-Ak2-, -Cy1-Ak1-Ak2-Ak3-, -Cy1-Ak1-Ak2-Ak3-Ak4-, -Cy1-Cy2-, -Cy1-Ak1-Cy2-, - Cy1-Cy2-Ak2-, -Cy1-Ak1-Cy2-Ak2-, -Cy1-Ak1-Cy2-Ak2-Ak3-, -Cy1-Ak1-Cy2-Ak2-Ak3-Ak4-, -Cy1-Cy2-Ak2-Ak3-, -Cy1-Cy2-Ak2-Ak3-Ak4-, -Cy1-Ak1-Cy2-Ak2-Ak3-Ak4-, -Cy1-Ak1-Ak2-Cy3-, -Cy1-Ak1-Ak2-Cy3-Ak3-, -Cy1-Cy2-Cy3-, -Cy1-Ak1-Cy2-Cy3-, -Cy1-Cy2-Ak2-Cy3-, -Cy1-Cy2-Cy3-Ak3-, -Cy1-Ak1-Cy2-Cy3-Ak3-, -Cy1-Cy2-Ak2-Cy3-Ak3-, -Cy1-Ak1-Cy2-Ak2-Cy3-, -Cy1-Ak1-Cy2-Ak2-Cy3-Ak3-, -Cy1-Cy2-Cy3-Ak3-Ak4-, -Cy1-Cy2-Cy3-Ak3-Cy4-, -Cy1-Cy2-Cy3-Cy4-, -Cy1-Ak1-Cy2-Cy3-Cy4-, -Cy1-Cy2-Ak2-Cy3-Cy4-, -Cy1-Cy2-Cy3-Ak3-Cy4-, -Cy1-Cy2-Cy3-Cy4-Ak4-, -Cy1-Ak1-Cy2-Ak2-Cy3-Ak3-Cy4-, -Cyl-Ak1-Cy2-Ak2-Cy3-Cy4-, -Ak1-Ak2-Cy3-Cy4-, -Ak1-Cy2-Ak2-Cy3-, -Ak1-Cy2-Cy3-Ak3-Cy4-, -Ak1-Cy2-Cy3-Cy4-Ak4-Cy5-, -Ak1-Cy2-Ak2-, -Ak1-Ak2-Ak3-Ak4-, -Ak1-Ak2-Ak3-, -Ak1-Ak2-, -Ak1-Ak2-Ak3-Ak4-Ak5-, -Cy1-Cy2-Cy3-Ak3-Ak4-Ak5-, -Cy1-Cy2-Ak2-Cy3-Ak3-Ak4-Ak5-, -Cy1-Ak1-Cy2-Ak2-Ak3-Ak4-Ak5-, -Cy1-Cy2-Cy3-Cy4-Ak4-Ak5-, -Cy1-Ak1-Ak2-Ak3-Ak4-Ak5-, -Ak1-Cy2-Ak2-Ak3-Ak4-Ak5-, -Ak1-Cy2-, -Ak1-Cy2-Ak2-Ak3-Ak4- or -Ak1-Cy2-Ak2-Ak3-;

in some embodiments, L is selected from -Cy1-Cy2- or -Cy1-$CH_2$-Cy2-, wherein Cy1 and Cy2 are each independently selected from piperazine, piperidine, azetidinyl, azacyclopentyl, cyclobutyl-spiro-azacyclohexyl, azetidinyl-spiro-azacyclohexyl, cyclobutyl-spiro-azetidinyl, azetidinyl-spiro-azetidinyl, azacyclohexyl-spiro-azacyclohexyl, cyclohexyl-spiro-azacyclohexyl, cyclopentyl-fused-azacyclopentyl or azacyclopentyl-fused-azacyclopentyl, and the Cy1 and Cy2 are optionally further substituted with 0 to 4 (such as 0, 1, 2, 3 or 4) substituents selected from H, F, Cl, Br, I, OH, $NH_2$, COOH, CN, =O, $C_{1-4}$ alkyl, halogen-substituted $C_{1-4}$ alkyl, hydroxyl-substituted $C_{1-4}$ alkyl or $C_{1-4}$

alkoxy;

in some embodiments, L is selected from a bond or a group in Table A, wherein the left side of the group is linked to B;

Table A L group

| | | | |
|---|---|---|---|
| 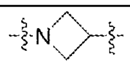 | 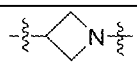 | 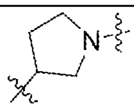 | 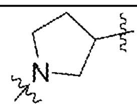 |
| 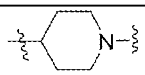 | 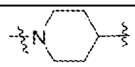 | 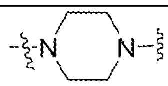 | 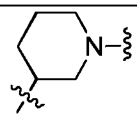 |
| 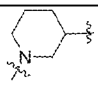 | 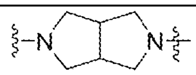 | 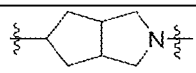 | 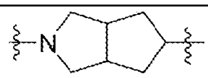 |

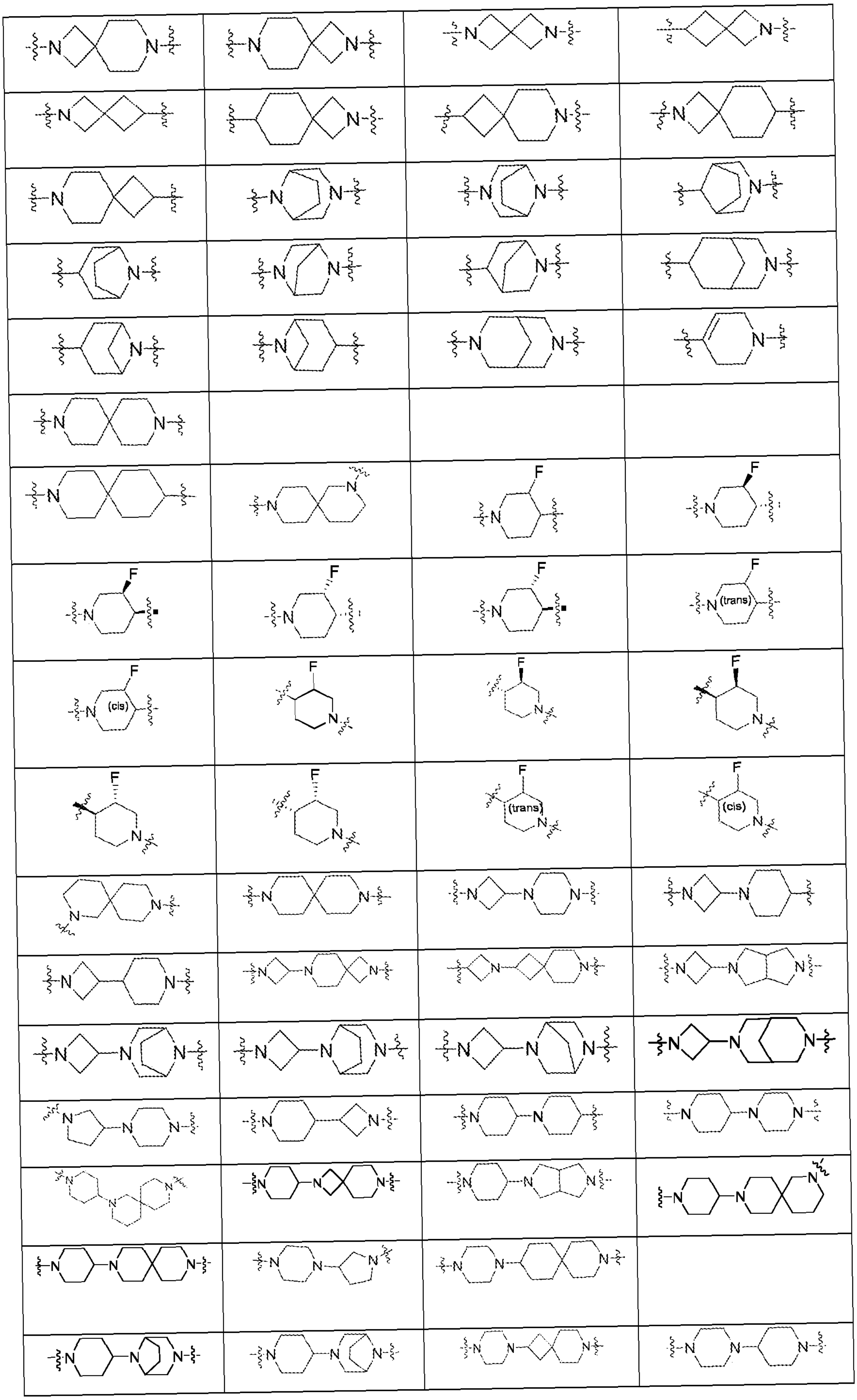

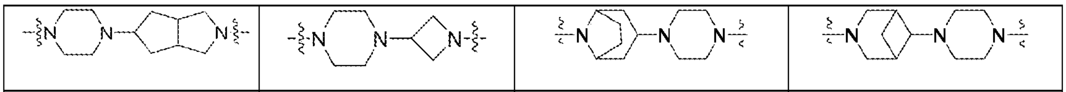

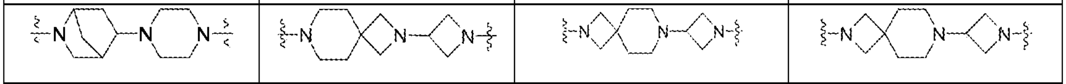

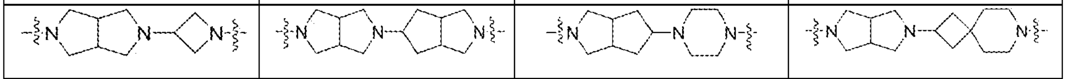

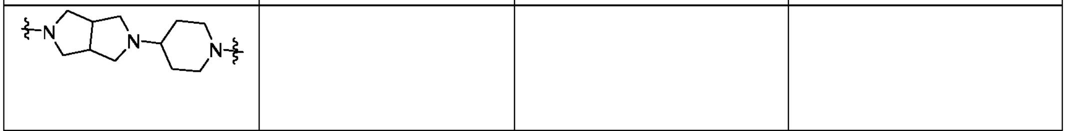

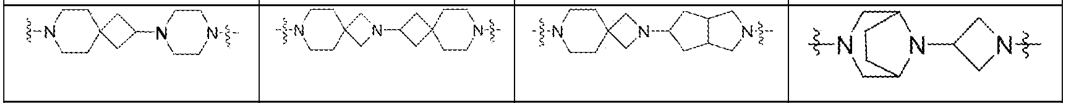

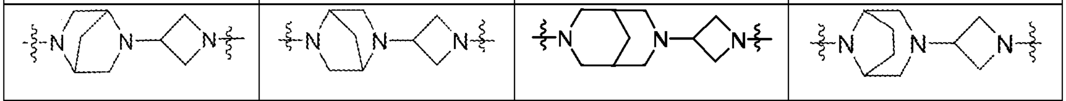

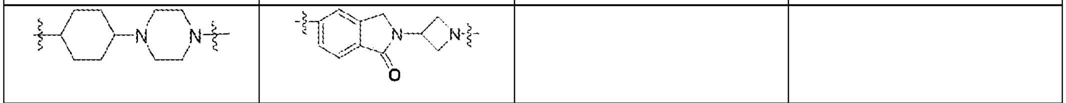

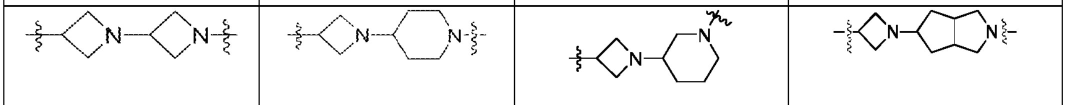

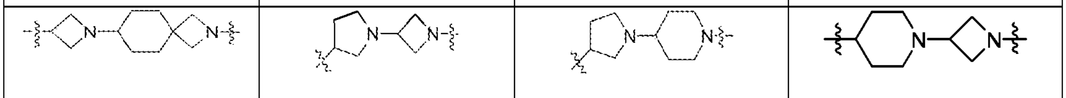

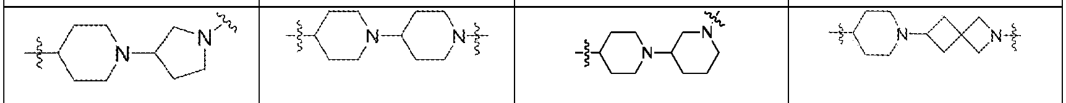

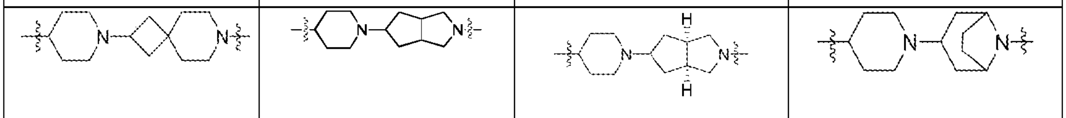

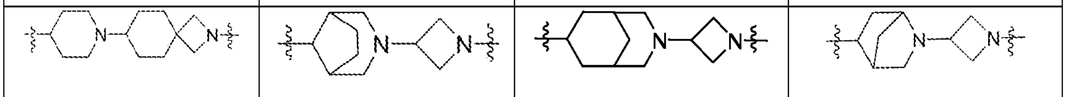

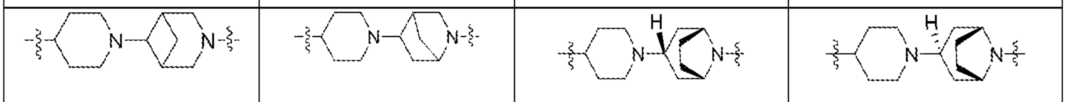

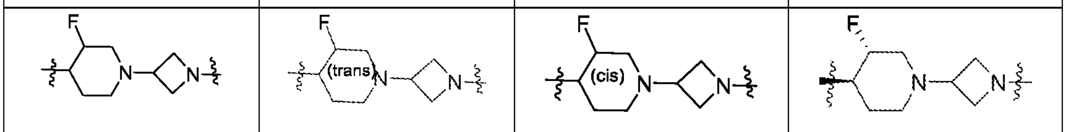

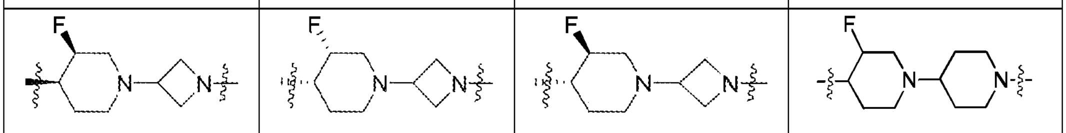

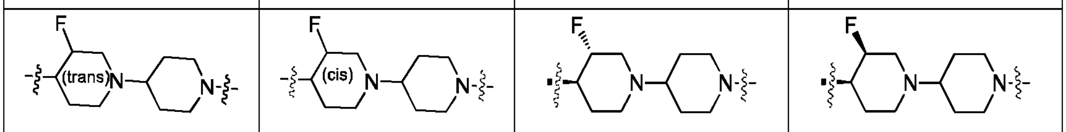

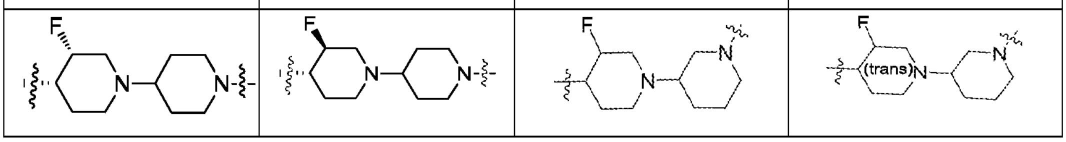

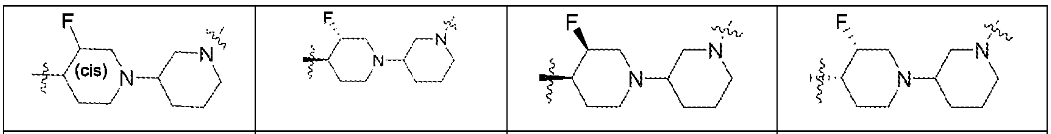
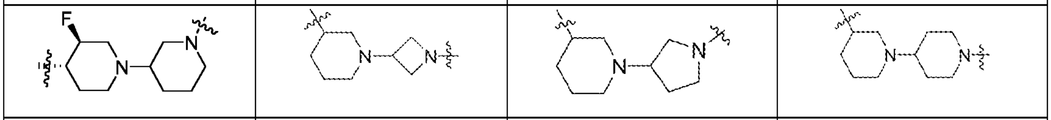
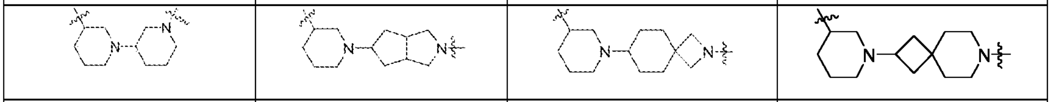
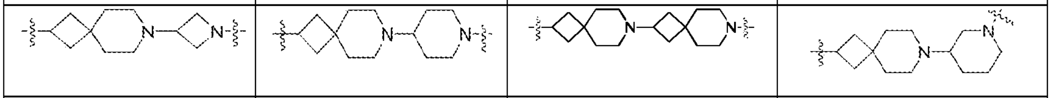
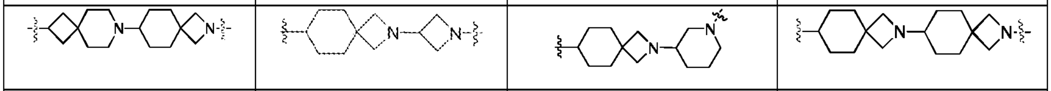
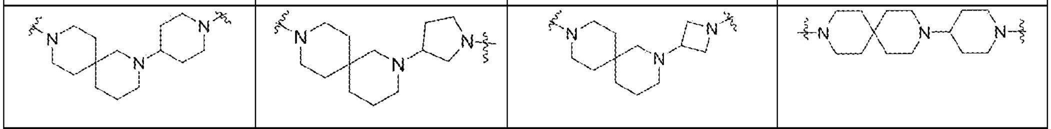
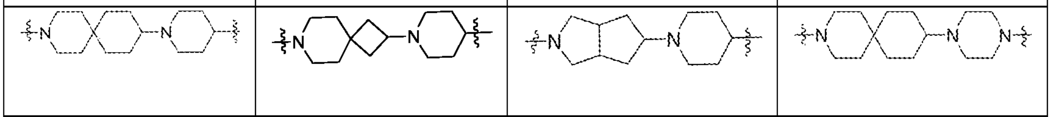
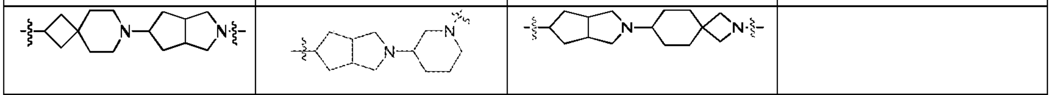
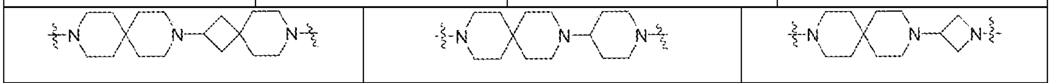
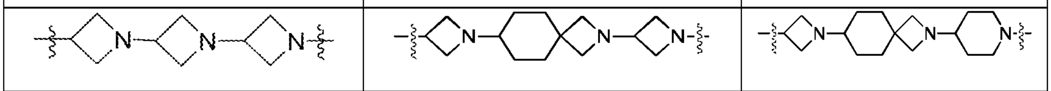
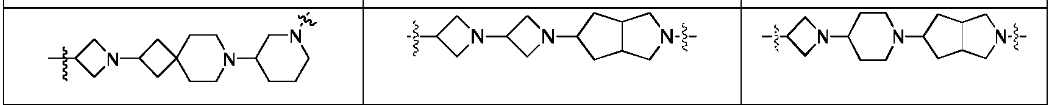
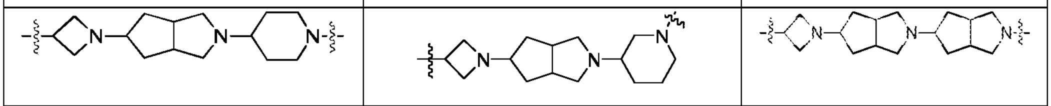
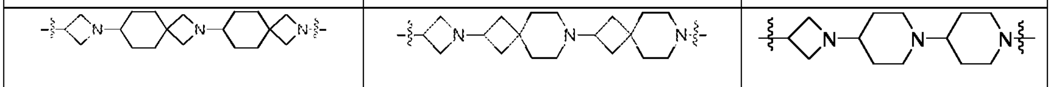
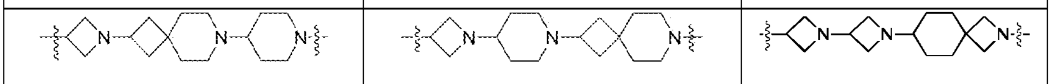
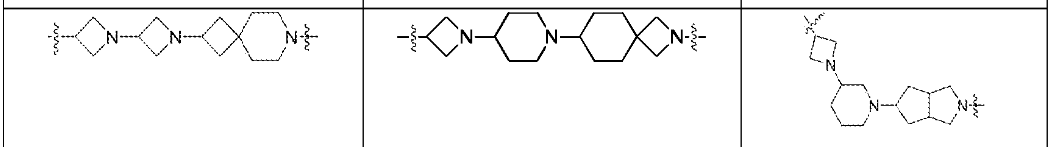
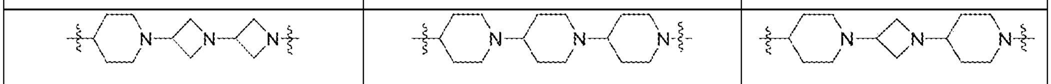
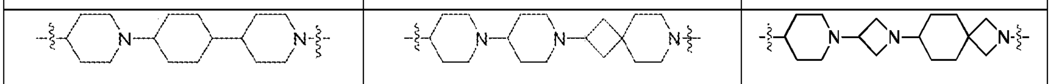
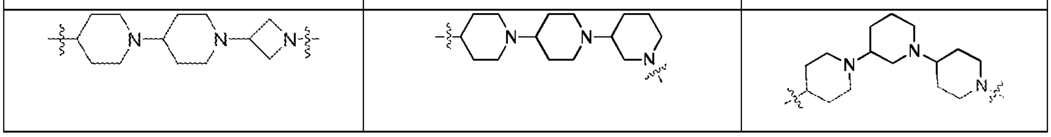

| | | |
|---|---|---|
| 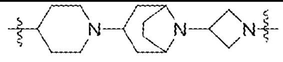 | 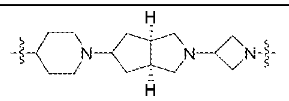 | 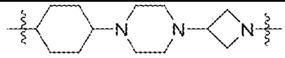 |
| 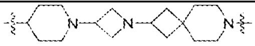 | 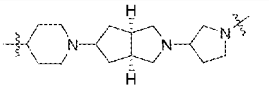 | 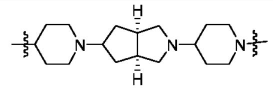 |
| 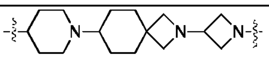 | 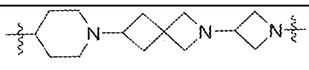 | 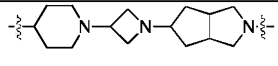 |
| 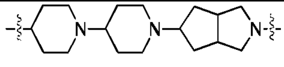 | 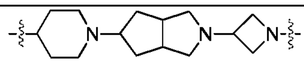 | 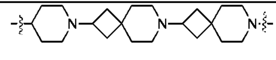 |
| 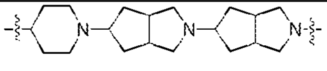 | 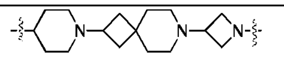 | 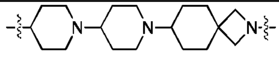 |
| 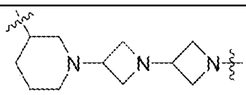 | 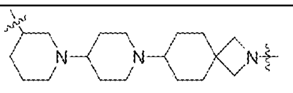 | 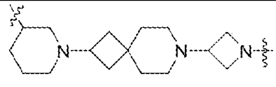 |
| 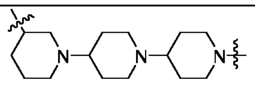 | 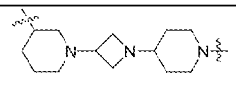 | 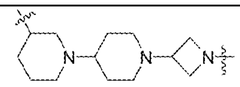 |
| 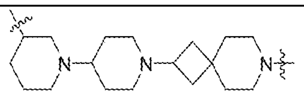 | 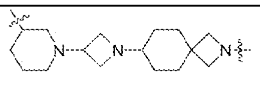 | 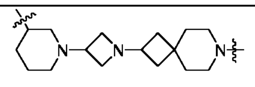 |
| 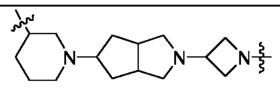 | 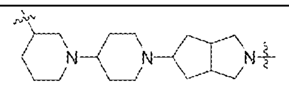 | 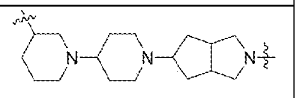 |
| 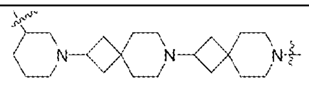 | 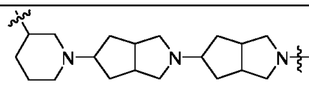 | 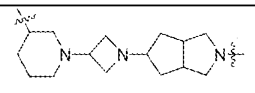 |
| 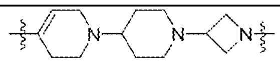 | 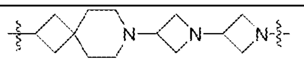 | 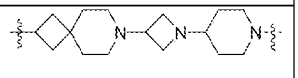 |
| 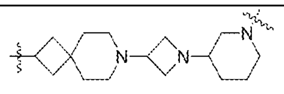 | 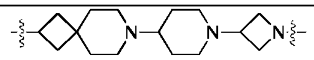 | |
| 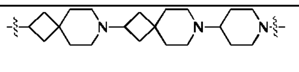 | 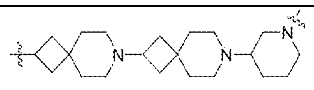 | 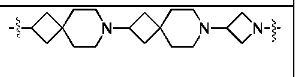 |
| 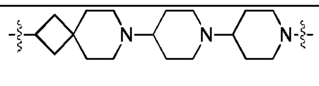 | 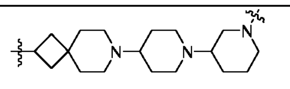 | |
| | | |
| | | |
| | | |
| | | |

| | | |
|---|---|---|
| 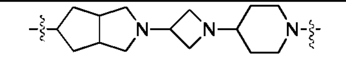 | 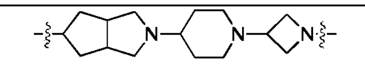 | 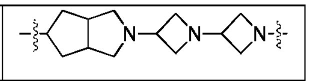 |
| 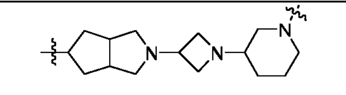 | 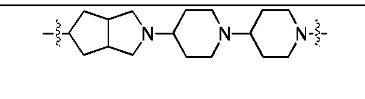 | 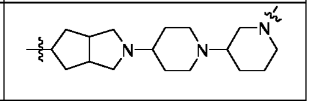 |
| 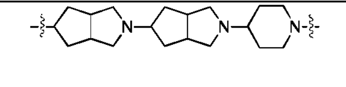 | 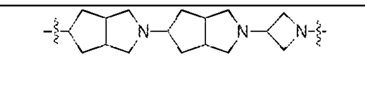 | 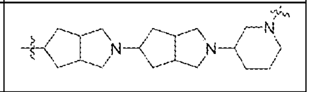 |
| 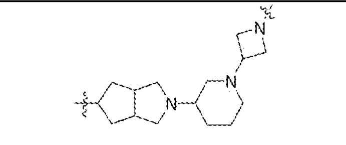 | 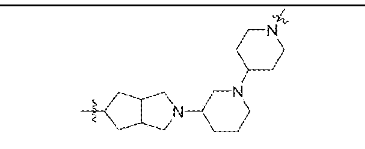 | 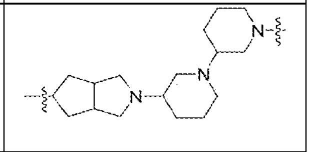 |
| 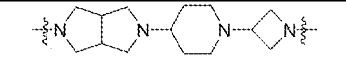 | 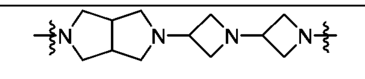 | 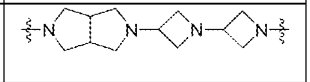 |
| 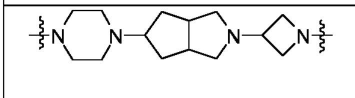 | 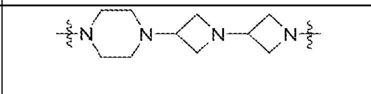 | 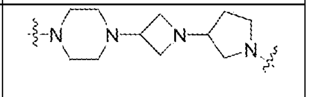 |
| 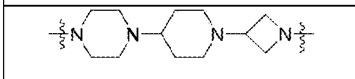 | 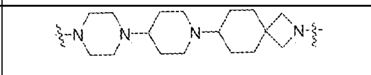 | 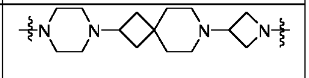 |
| 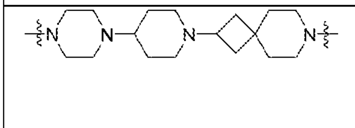 | 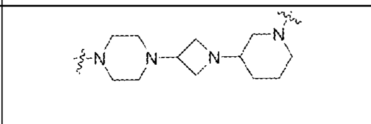 | 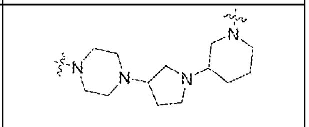 |
| 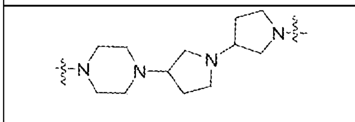 | 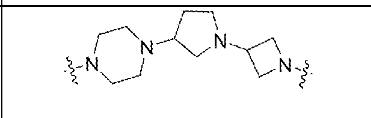 | 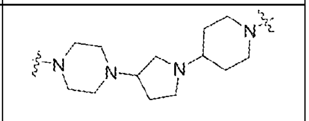 |
| 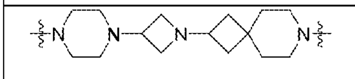 | 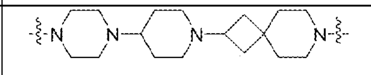 | 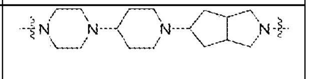 |
| 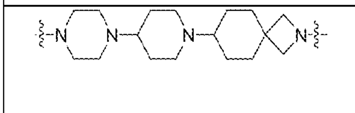 | 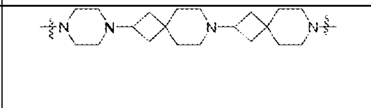 | 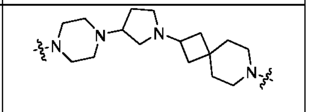 |
| 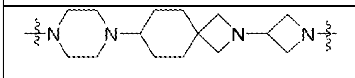 | 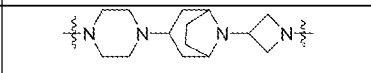 | 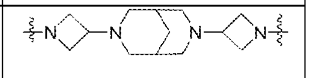 |
| 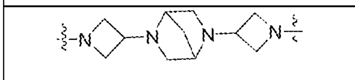 | 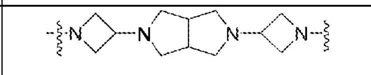 | 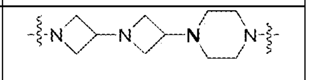 |
| 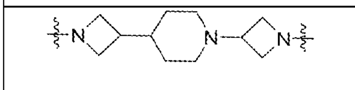 | | |
| | | |
| | | |
| | | |
| | | |
| | | |

| | | |
|---|---|---|
| 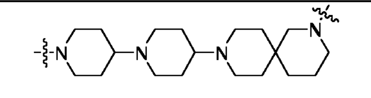 | 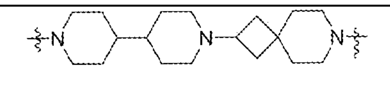 | 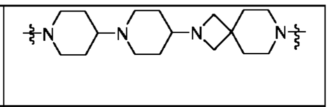 |
| 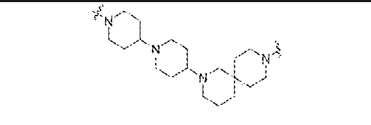 | 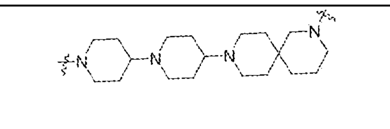 | 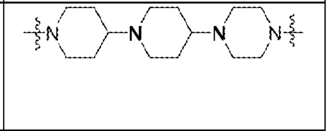 |
| 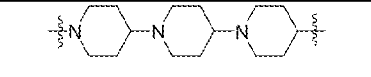 | 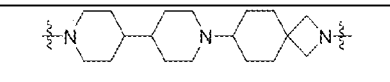 | 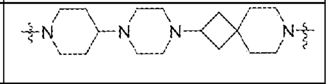 |
| 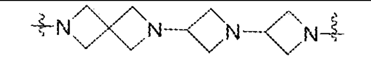 | 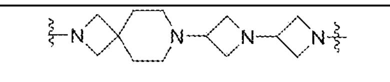 | 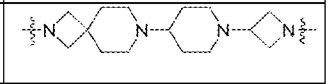 |
| 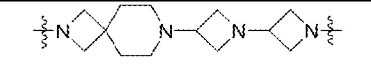 | 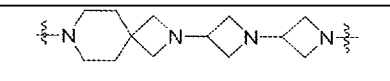 | 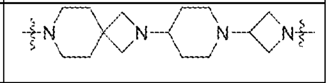 |
| 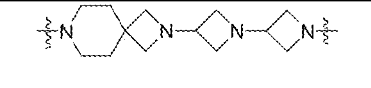 | 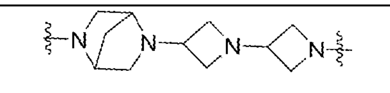 | 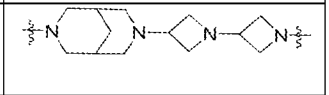 |
| 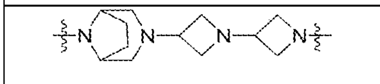 | 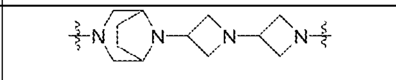 | 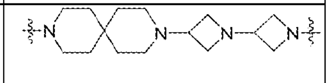 |
| 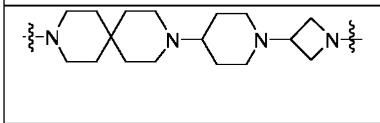 | 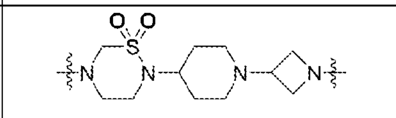 | 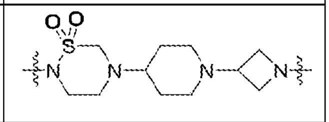 |
| 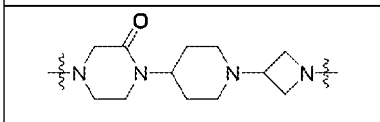 | 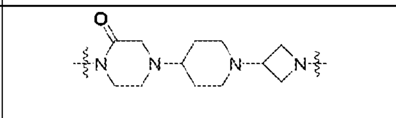 | |

| | |
|---|---|
| 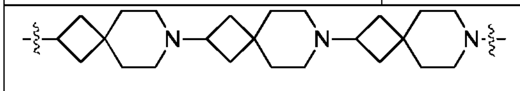 | 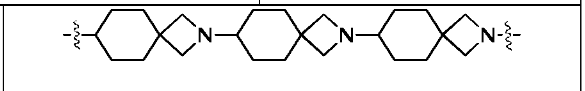 |
| 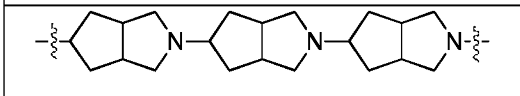 | 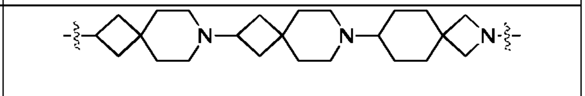 |
| 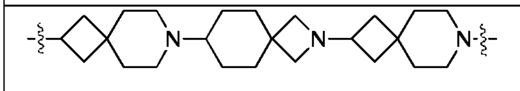 | 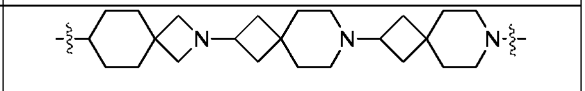 |
| 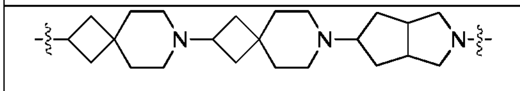 | 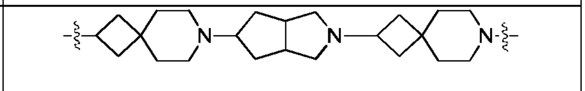 |

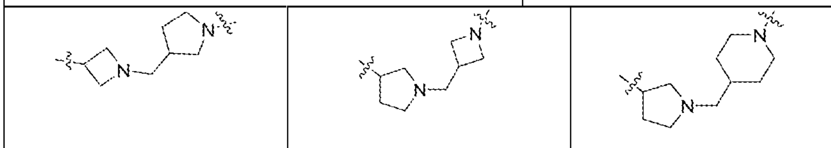

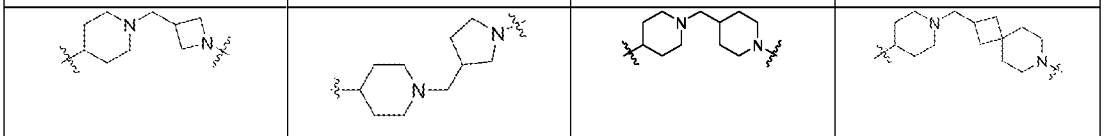

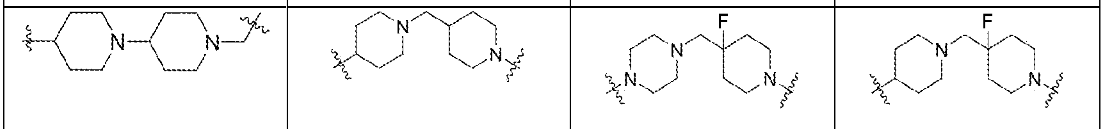

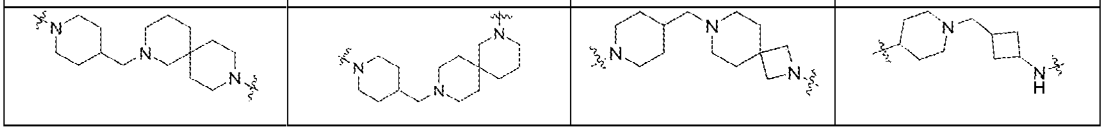

| | | | |
|---|---|---|---|
| 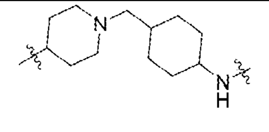 | 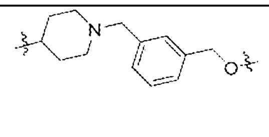 | 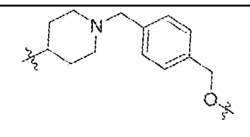 | 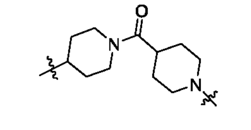 |
| 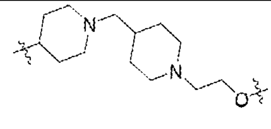 | 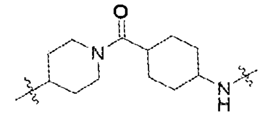 | 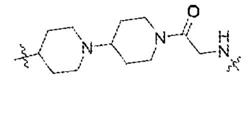 | 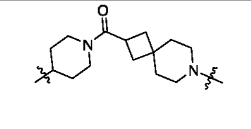 |
| 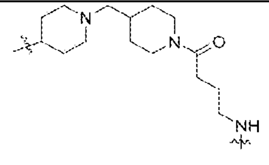 | 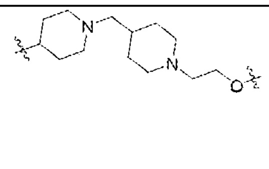 | 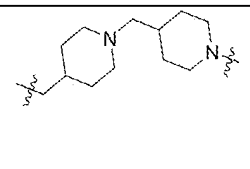 | 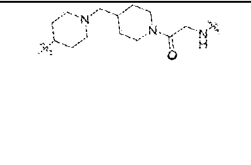 |
| 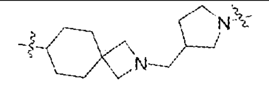 | 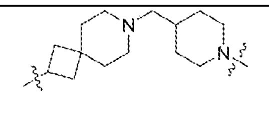 | 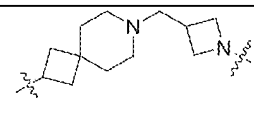 | |
| 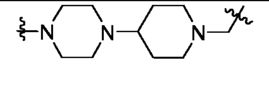 | 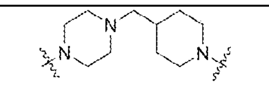 | 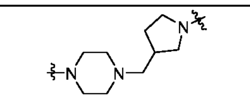 | 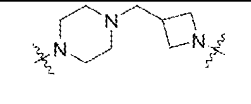 |
| 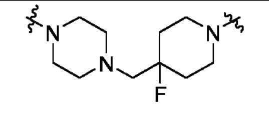 | 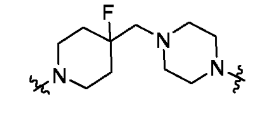 | 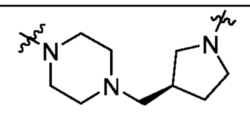 | 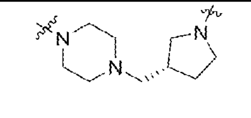 |
| 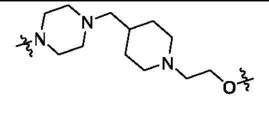 | 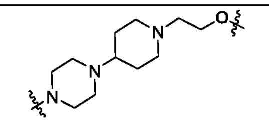 | 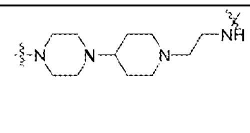 | 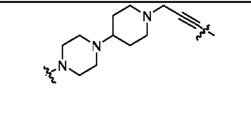 |
| 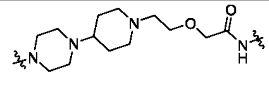 | 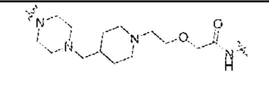 | 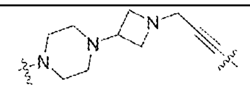 | |
| 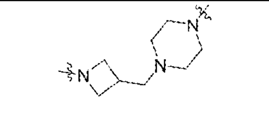 | 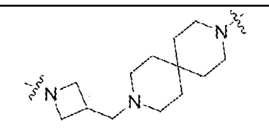 | 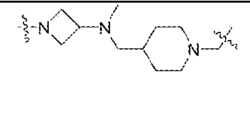 | 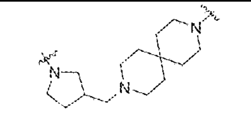 |
| 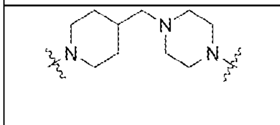 | 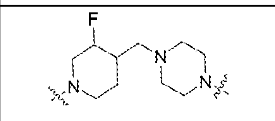 | 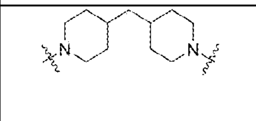 | 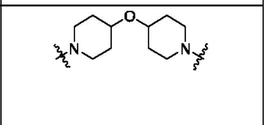 |
| 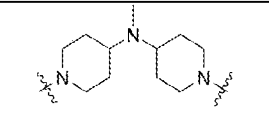 | 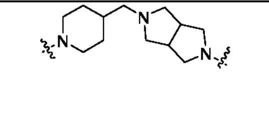 | | |
| | | | |
| | | | |
| | | | |

| | | | |
|---|---|---|---|
| 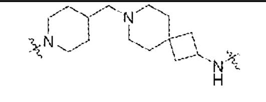 | 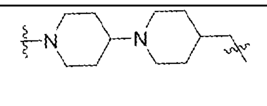 | 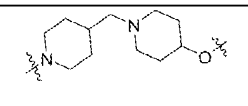 | 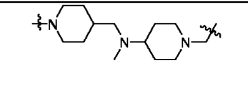 |
| 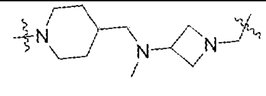 | 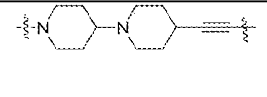 | 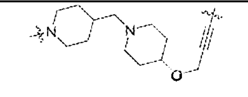 | 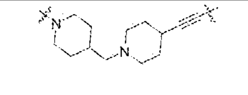 |
| 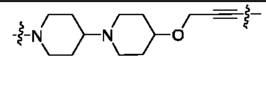 | 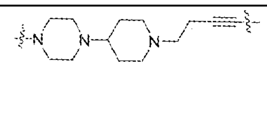 | 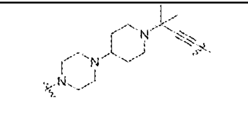 | 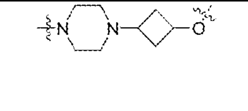 |
| 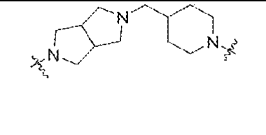 | 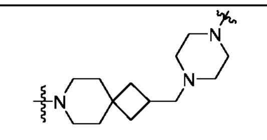 | 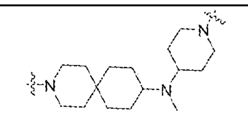 | 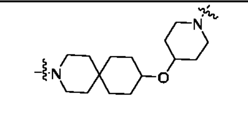 |
| 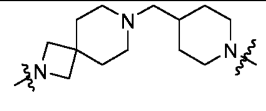 | 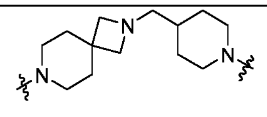 | 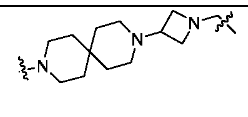 | 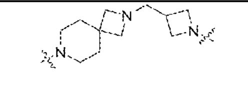 |
| 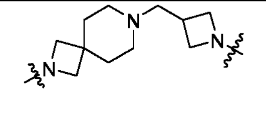 | 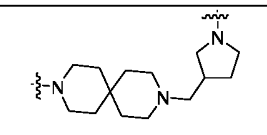 | 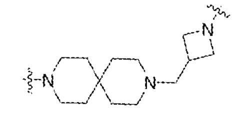 | 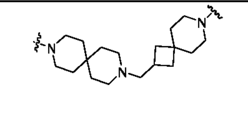 |
| 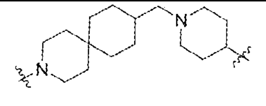 | 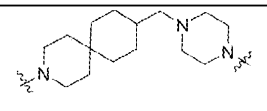 | 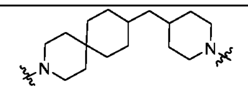 | |
| 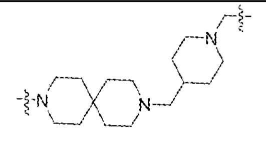 | 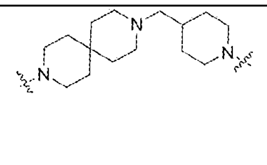 | 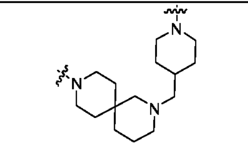 | 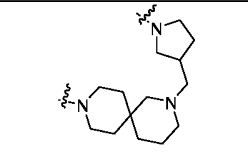 |
| 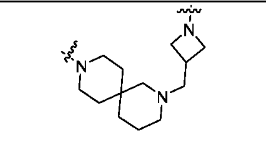 | 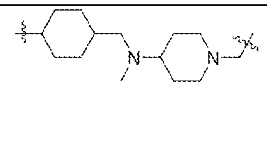 | 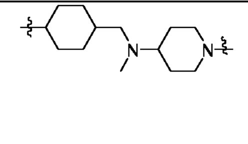 | 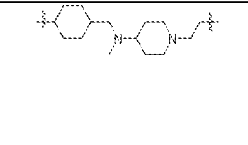 |
| 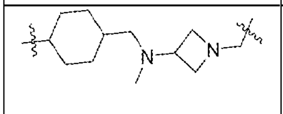 | 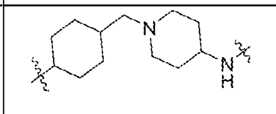 | 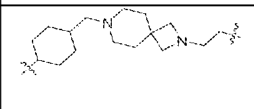 | 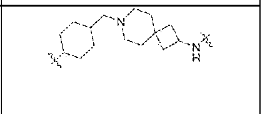 |
| 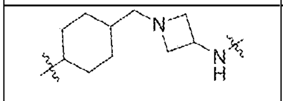 | | | |
| | | | |
| | | | |
| | | | |

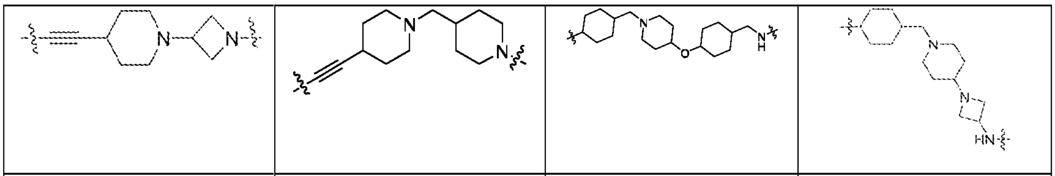

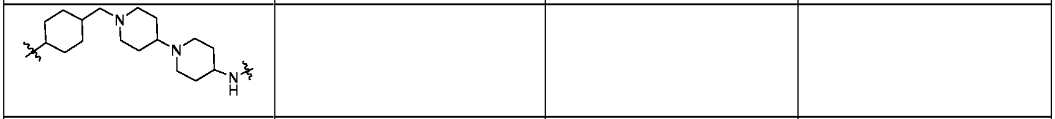

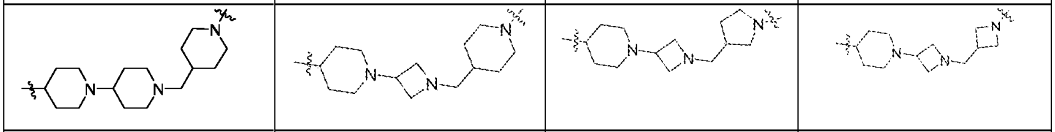

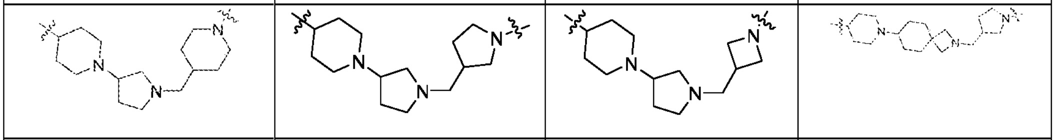

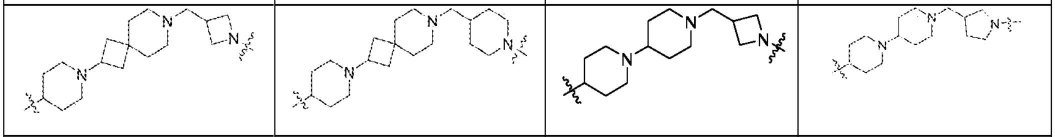

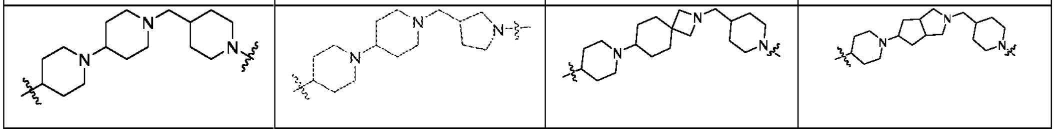

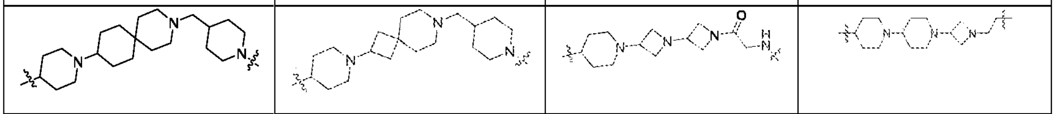

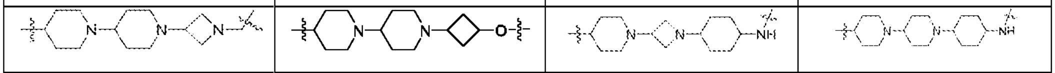

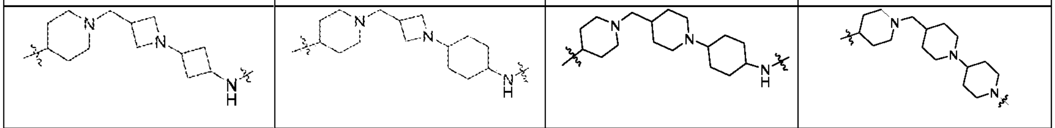

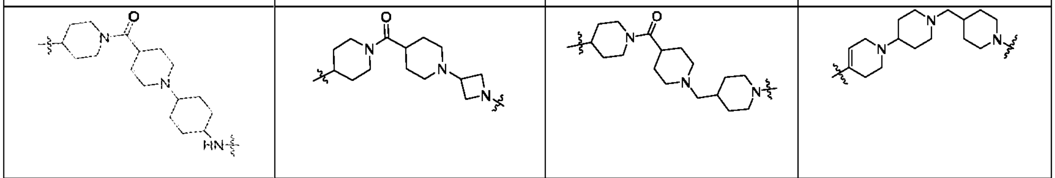

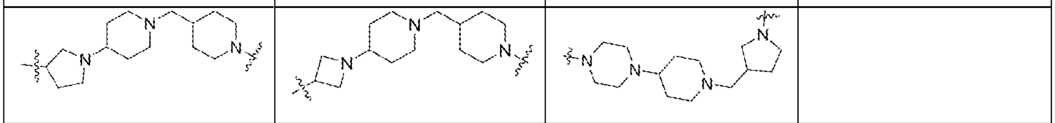

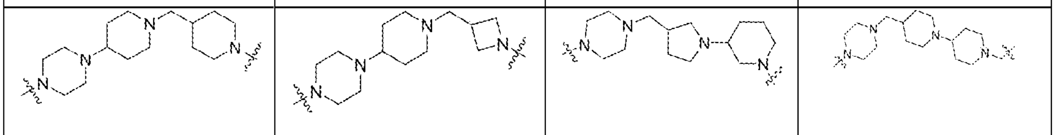

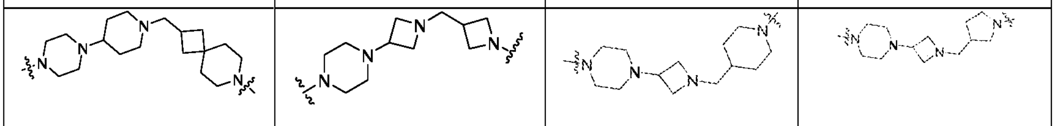

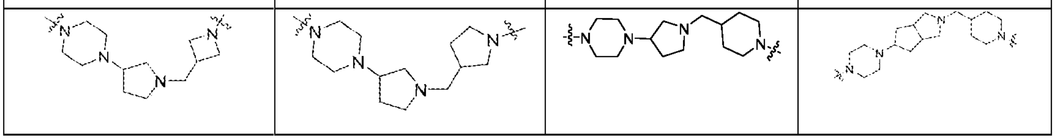

| | | | |
|---|---|---|---|
| 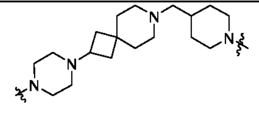 | 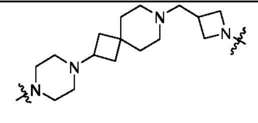 | 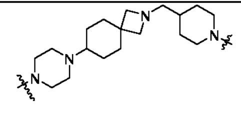 | 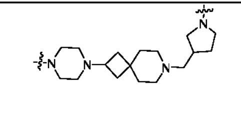 |
| 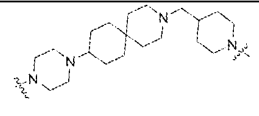 | 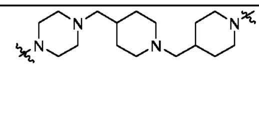 | 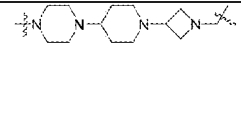 | 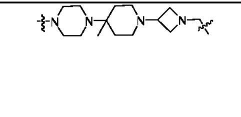 |
| 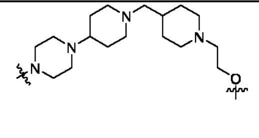 | 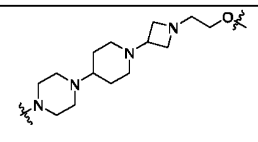 | 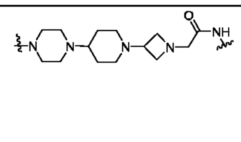 | 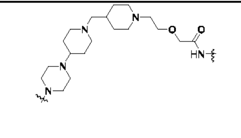 |
| 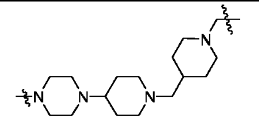 | 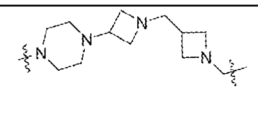 | 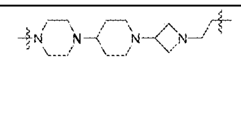 | 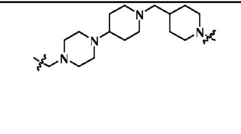 |
| 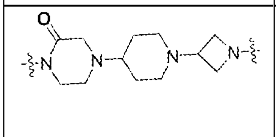 | 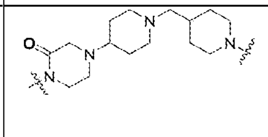 | 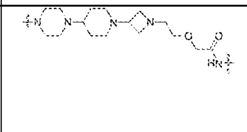 | 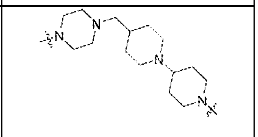 |
| 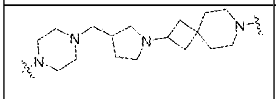 | 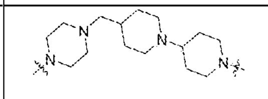 | 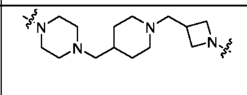 | |
| 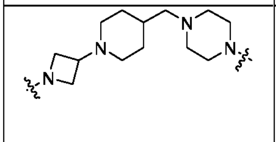 | 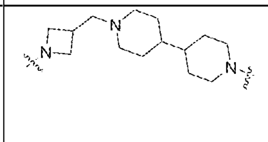 | 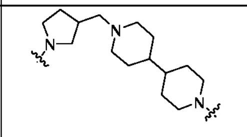 | 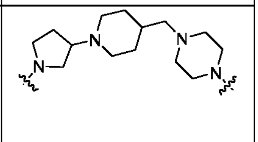 |
| 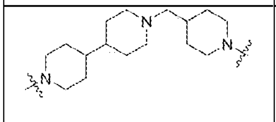 | 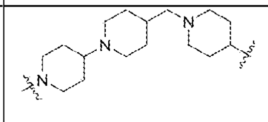 | 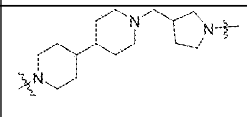 | 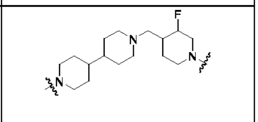 |
| 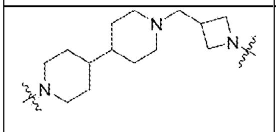 | 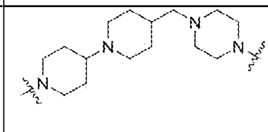 | 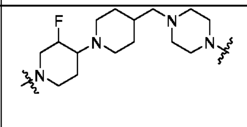 | 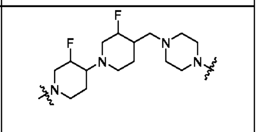 |
| 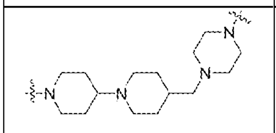 | 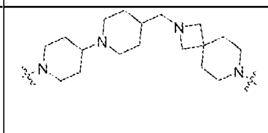 | 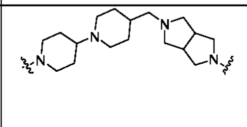 | 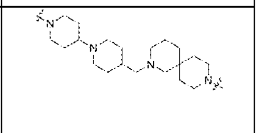 |
| 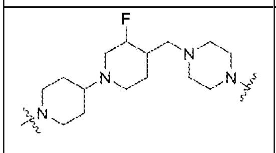 | | | |
| | | | |
| | | | |
| | | | |

| | | | |
|---|---|---|---|
| 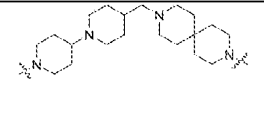 | 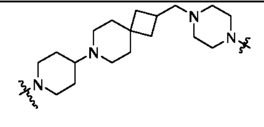 | 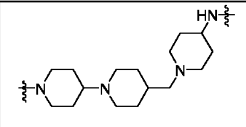 | 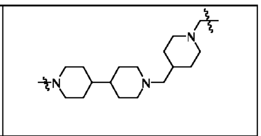 |
| 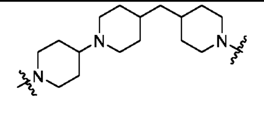 | 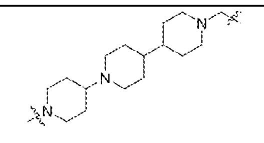 | 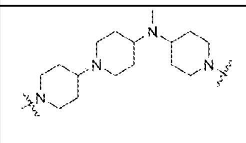 | 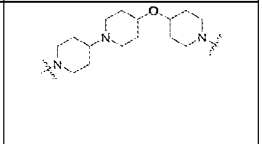 |
| 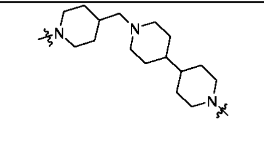 | 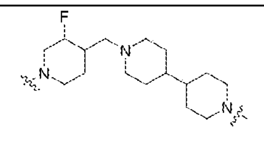 | 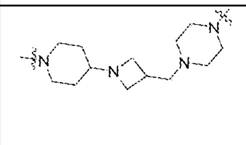 | 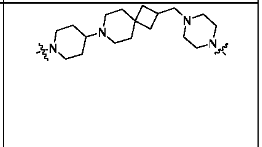 |
| 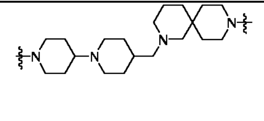 | 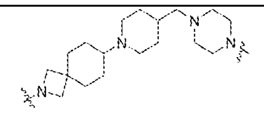 | 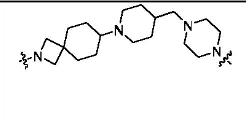 | 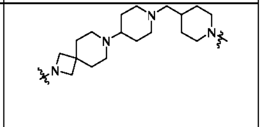 |
| 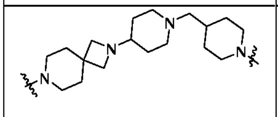 | 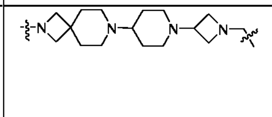 | 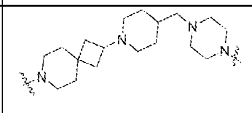 | 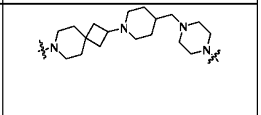 |
| 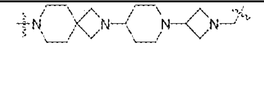 | 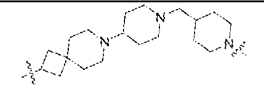 | 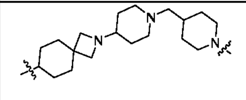 | 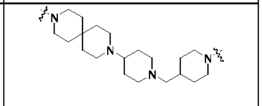 |
| 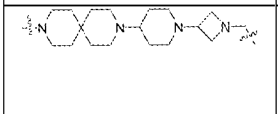 | 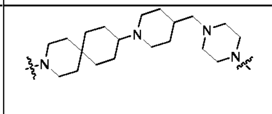 | 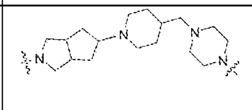 | 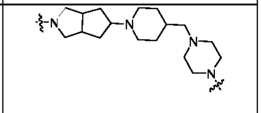 |
| 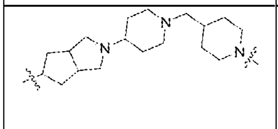 | 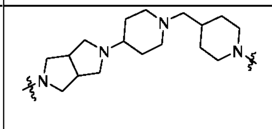 | 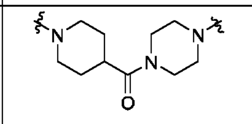 | |
| 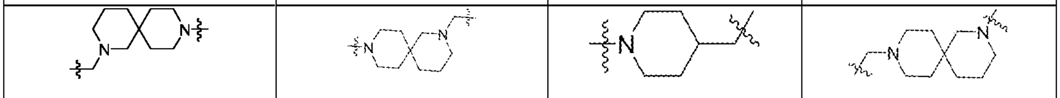 | | | |
| 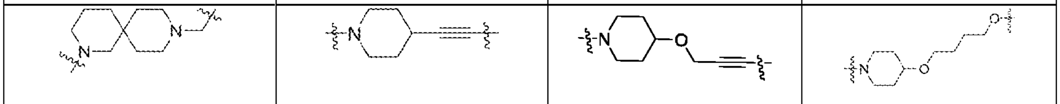 | | | |
| 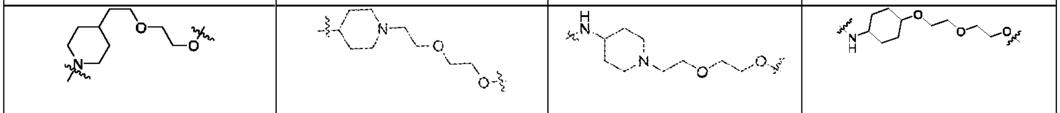 | | | |
| 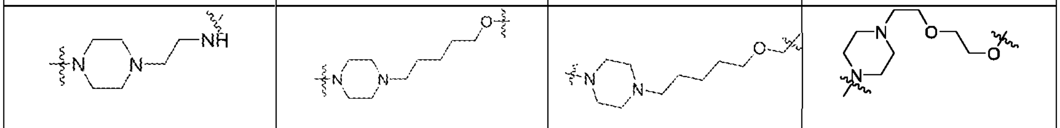 | | | |
| 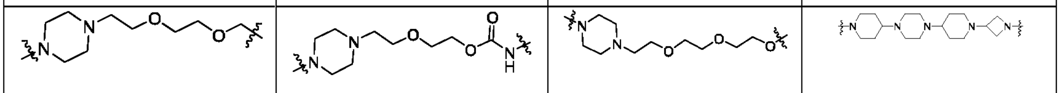 | | | |
| 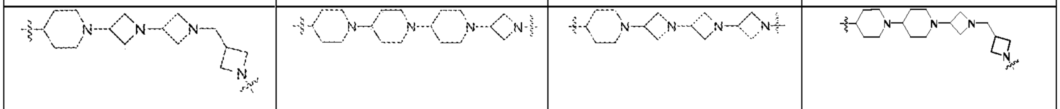 | | | |
| 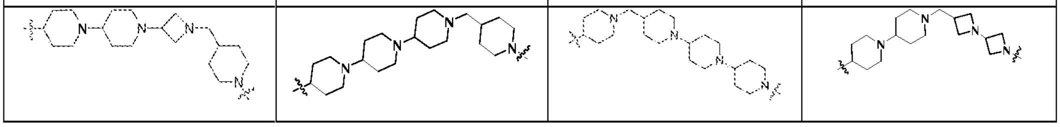 | | | |

| | | | |
|---|---|---|---|
| 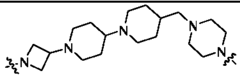 | 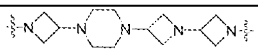 | 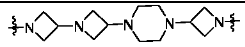 | 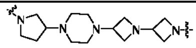 |
| 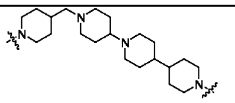 | 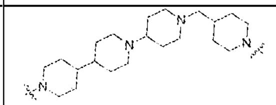 | 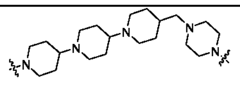 | 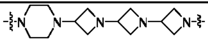 |
| 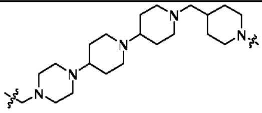 | 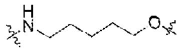 | 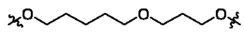 | 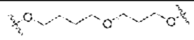 |
| 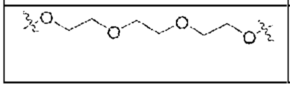 | 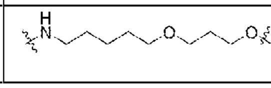 | 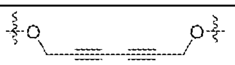 | 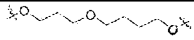 |

in some embodiments, Ak1, Ak2, Ak3, Ak4 and Ak5 are each independently selected from $-(CH_2)_q-$, $-(CH_2)_q-O-$, $-O-(CH_2)_q-$, $-(CH_2)_q-NR^L-$, $-NR^L-(CH_2)_q-$, - $(CH_2)_q-NR^LC(=O)-$, $-(CH_2)_q-C(=O)NR^L-$, -C(=O)-, $-C(=O)-(CH_2)_q-NR^L-$, $-(C{\equiv}C)_q-$or a bond, wherein the $-CH_2-$ is optionally further substituted with 0 to 2 (such as 0, 1 or 2) substituents selected from H, halogen, OH, CN, $NH_2$, $C_{1-4}$ alkyl, $C_{1-4}$ alkoxy, halogen-substituted $C_{1-4}$ alkyl, hydroxyl-substituted $C_{1-4}$ alkyl or cyano-substituted $C_{1-4}$ alkyl;

in some embodiments, Ak1, Ak2, Ak3, Ak4 and Ak5 are each independently selected from -O-, $-OCH_2-$, $-CH_2O-$, $-OCH_2CH_2-$, $-CH_2CH_2O-$, $-C{\equiv}C-$, $-C(CH_3)_2-$, - $CH_2-$, $-CH_2CH_2-$, $-CH_2CH_2CH_2-$, $-N(CH_3)-$, -NH-, $-CH_2N(CH_3)-$, $-CH_2NH-$, - $NHCH_2-$, $-CH_2CH_2N(CH_3)-$, $-CH_2CH_2NH-$, $-NHCH_2CH_2-$, -C(=O)-, - $C(=O)CH_2NH-$, $-CH_2C(=O)NH-$, $-C(=O)N(CH_3)-$, $-N(CH_3)C(=O)-$, -C(=O)NH- or -NHC(=O)-;

in some embodiments, each $R^L$ is independently selected from H, $C_{1-6}$ alkyl, 3- to 7-membered heterocyclyl, 3- to 7-membered cycloalkyl, phenyl or 5- to 6-membered heteroaryl;

in some embodiments, each $R^L$ is independently selected from H or $C_{1-6}$ alkyl;

in some embodiments, each $R^L$ is independently selected from H or $C_{1-4}$ alkyl;

in some embodiments, each $R^L$ is independently selected from H, methyl or ethyl;

in some embodiments, each Cy1, Cy2, Cy3, Cy4 or Cy5 is independently selected from a bond or one of the following substituted or unsubstituted groups: cyclopropyl, cyclobutyl, cyclopentyl, cyclohexyl, azetidinyl, azacyclopentyl, azacyclohexenyl, piperidine, morpholine, piperazine, phenyl, cyclopropyl-fused-cyclopropyl, cyclopropyl-fused-cyclobutyl, cyclopropyl-fused-cyclopentyl, cyclopropyl-fused-cyclohexyl, cyclobutyl-fused-cyclobutyl, cyclobutyl-fused-cyclopentyl, cyclobutyl-fused-cyclohexyl, cyclopentyl-fused-cyclopentyl, cyclopentyl-fused-cyclohexyl, cyclohexyl-fused-cyclohexyl, cyclopropyl-spiro-cyclopropyl, cyclopropyl-spiro-cyclobutyl, cyclopropyl-spiro-cyclopentyl, cyclopropyl-spiro-cyclohexyl, cyclobutyl-spiro-cyclobutyl, cyclobutyl-spiro-cyclopentyl, cyclobutyl-spiro-cyclohexyl, cyclopentyl-spiro-cyclopentyl, cyclopentyl-spiro-cyclohexyl, cyclohexyl-spiro-cyclohexyl, cyclopropyl-fused-azetidinyl, cyclopropyl-fused-azacyclopentyl, cyclopropyl-fused-azacyclohexyl, cyclobutyl-fused-azetidinyl, cyclobutyl-fused-azacyclopentyl, cyclobutyl-fused-azacyclohexyl, cyclopentyl-fused-azetidinyl, cyclopentyl-fused-azacyclopentyl, cyclopentyl-fused-azacyclohexyl, cyclohexyl-fused-azetidinyl, cyclohexyl-fused-azacyclopentyl, cyclohexyl-fused-azacyclohexyl, cyclohexyl-fused-piperidine, azetidinyl-fused-azetidinyl, azetidinyl-fused-azacyclopentyl, azetidinyl-fused-azacyclohexyl, azacyclopentyl-fused-azetidinyl, azacyclopentyl-fused-azacyclopentyl, azacyclopentyl-fused-azacyclohexyl, azacyclohexyl-fused-azetidinyl, azacyclohexyl-fused-azacyclopentyl, azacyclohexyl-fused-azacyclohexyl, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, cyclohexyl-spiro-azacyclohexyl, azetidinyl-spiro-azetidinyl, azetidinyl-spiro-azacyclopentyl, azetidinyl-spiro-azacyclohexyl, azacyclopentyl-spiro-azetidinyl, azacyclopentyl-spiro-azacyclopentyl, azacyclopentyl-spiro-azacyclohexyl, azacyclohexyl-spiro-azetidinyl, azacyclohexyl-spiro-azacyclopentyl, azacyclohexyl-spiro-azacyclohexyl,

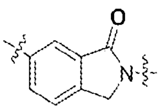 , 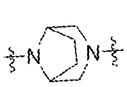 , 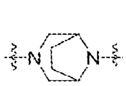 , 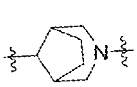 , 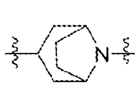 , 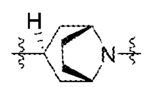 ,

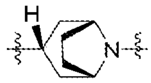, 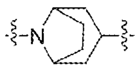, 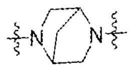, 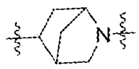, 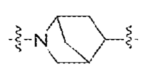, 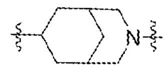,

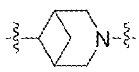, 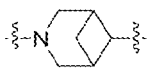, 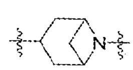, 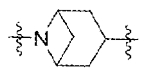, 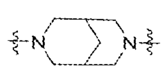, 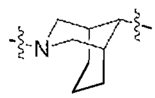,

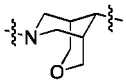, 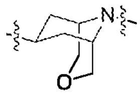, 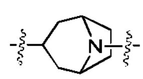, 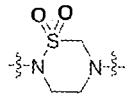, 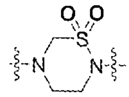,

which, when substituted, is optionally further substituted with 0 to 4 (such as 0, 1, 2, 3 or 4) substituents selected from H, F, Cl, Br, I, OH, $NH_2$, COOH, CN, =O, $C_{1-4}$ alkyl, halogen-substituted $C_{1-4}$ alkyl, hydroxyl-substituted $C_{1-4}$ alkyl or $C_{1-4}$ alkoxy;

in some embodiments, each Cy1, Cy2, Cy3, Cy4 or Cy5 is independently selected from a bond or one of the following substituted or unsubstituted groups:

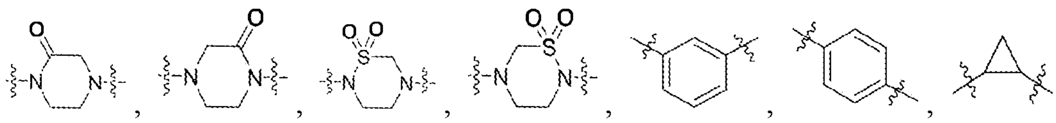,

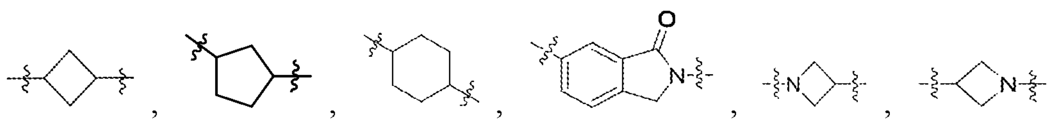,

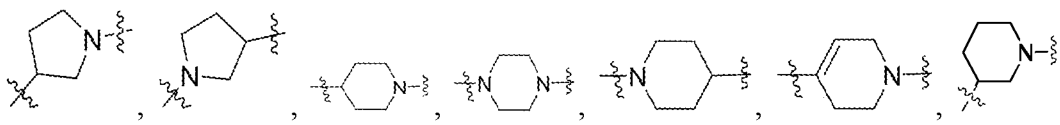,

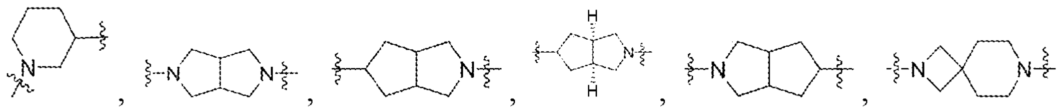,

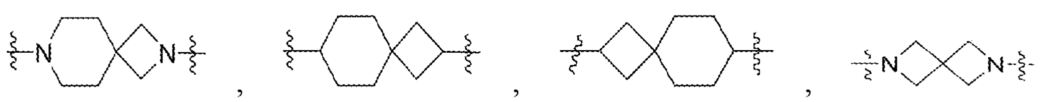,

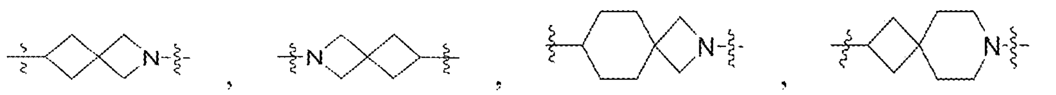,

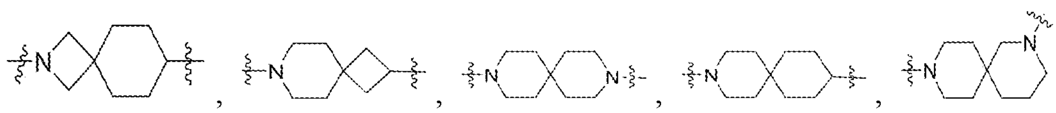,

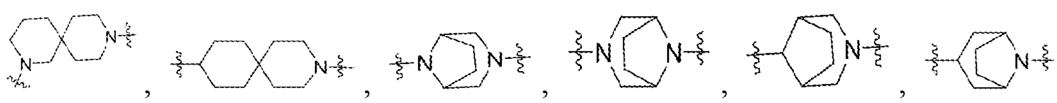,

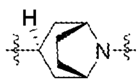, 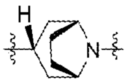, 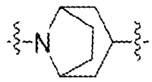, 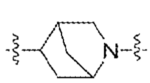, 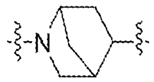, 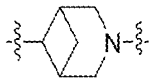,

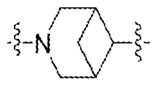, 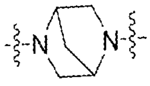, 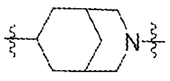, 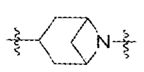, 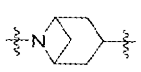, 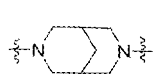,

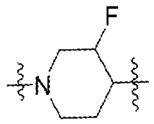, 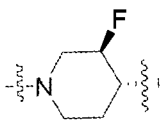, 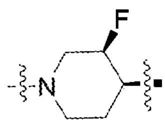, 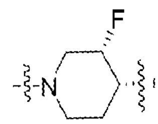, 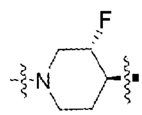, 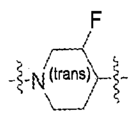,

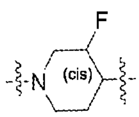, 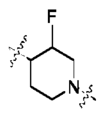, 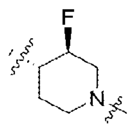, 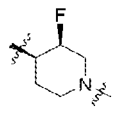, 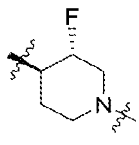, 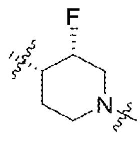, 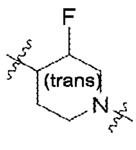 or

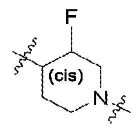,

which, when substituted, is optionally further substituted with 0 to 4 (such as 0, 1, 2, 3 or 4) substituents selected from H, F, $CF_3$, methyl, =O, hydroxymethyl, COOH, CN or $NH_2$;
in some embodiments, K is selected from

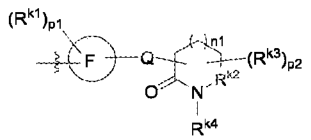,

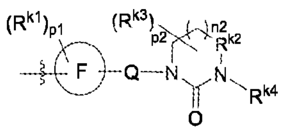, 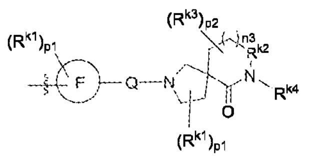, 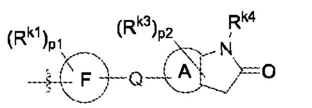,

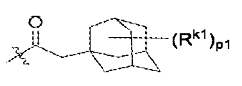, 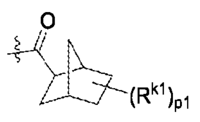, 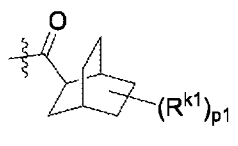, 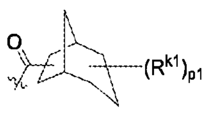,

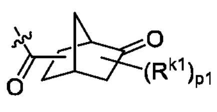, 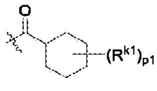, 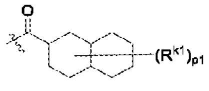, 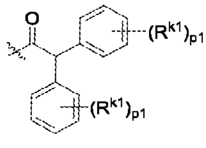, 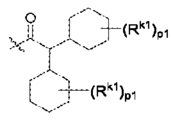,

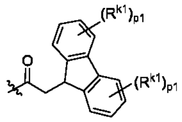, 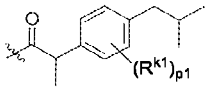, 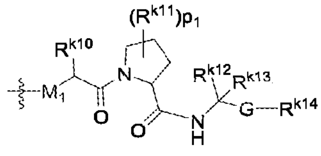, 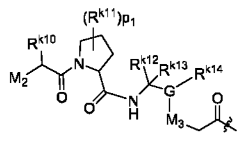

or

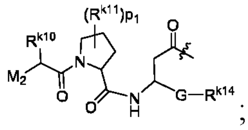;

in some embodiments, K is selected from

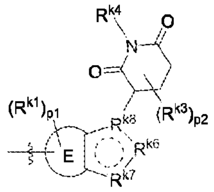, 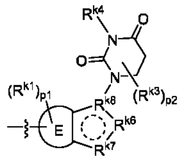,

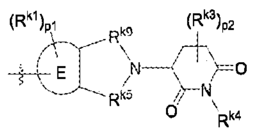, 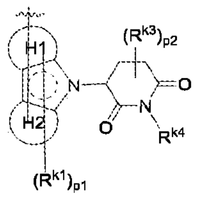, 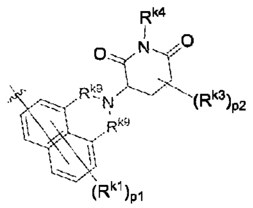, 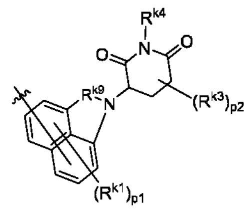,

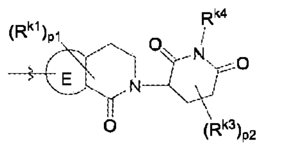, 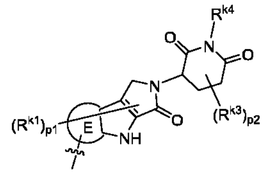, 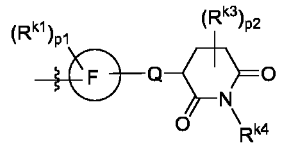,

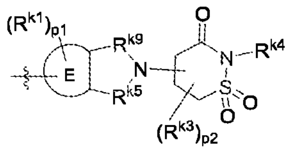, 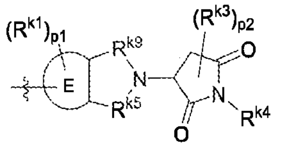, 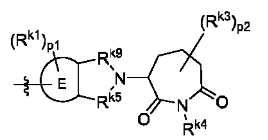,

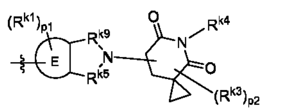, 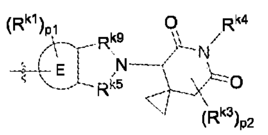, 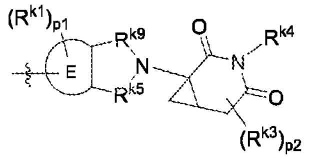,

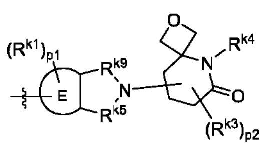, 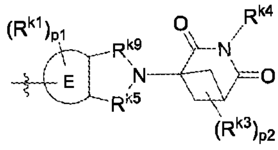, 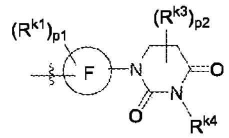, 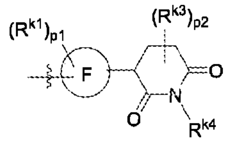,

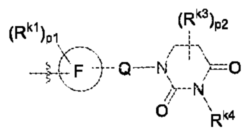, 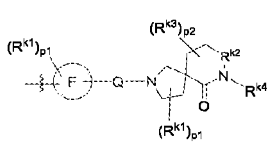, 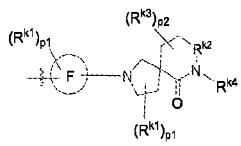, 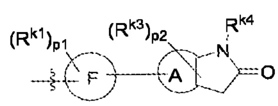,

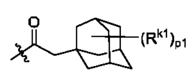, 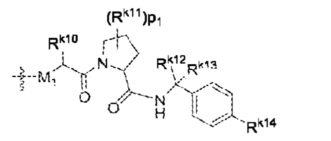, 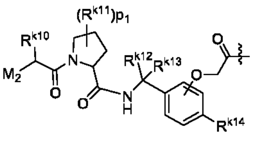

or

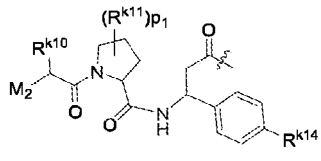,

wherein

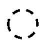

represents a ring selected from an aromatic ring or a non-aromatic ring;
in some embodiments, K is selected from

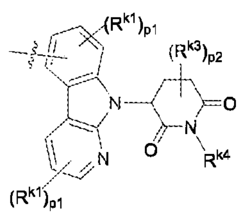,

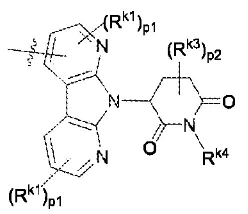, 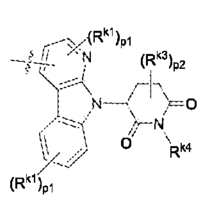, 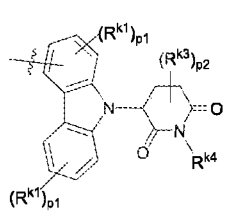, 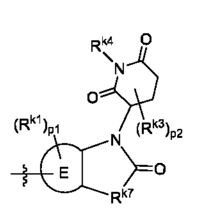,

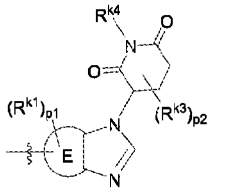, 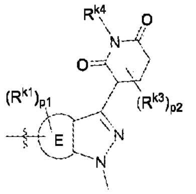, 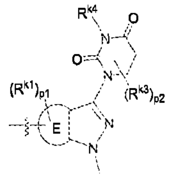, 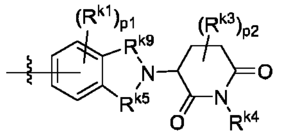,

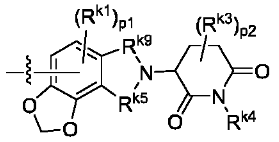, 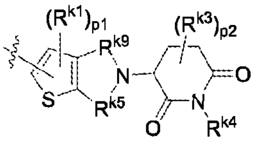, 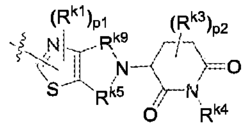,

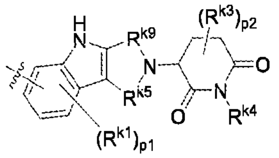, 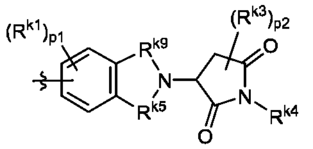, 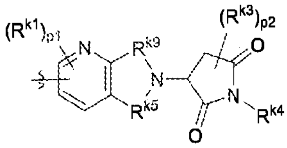,

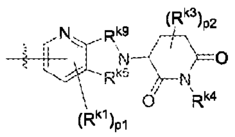, 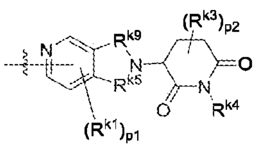, 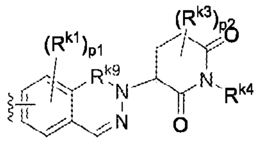, 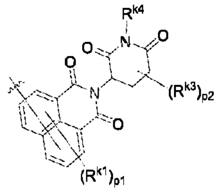,

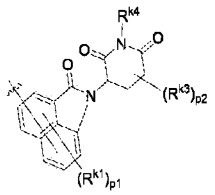, 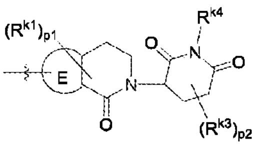, 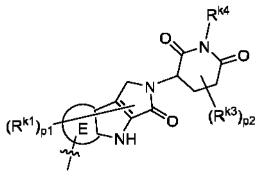, 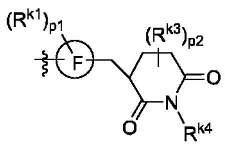,

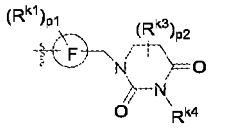, 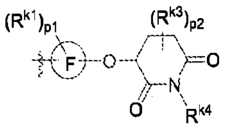, 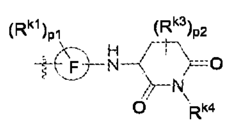, 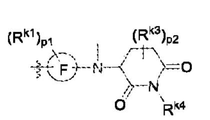,

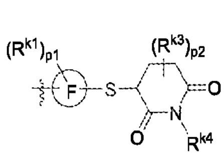, 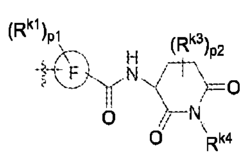, 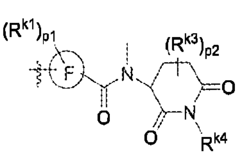, 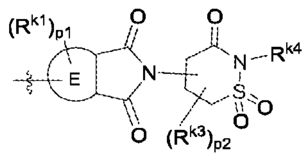,

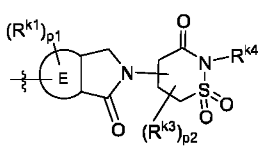, 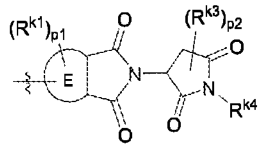, 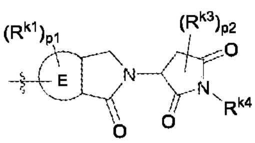,

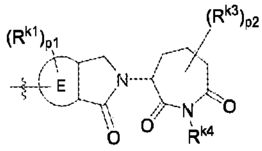 , 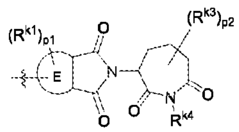 , 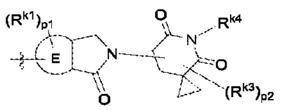 ,

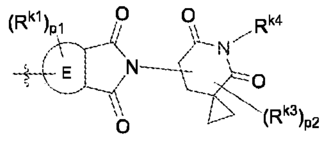 , 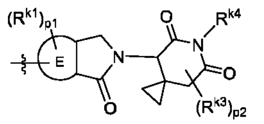 , 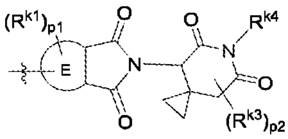 ,

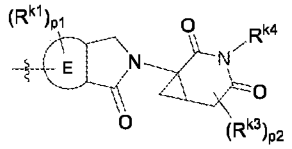 , 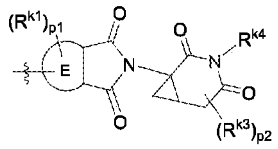 , 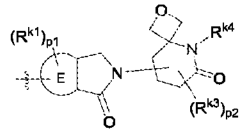 ,

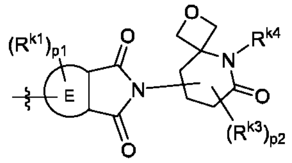 , 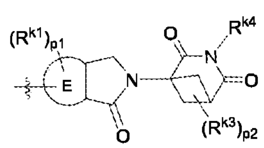 , 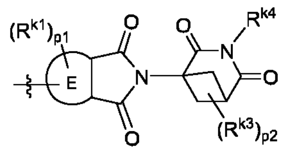 ,

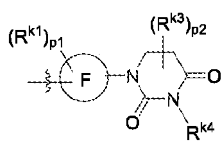 , 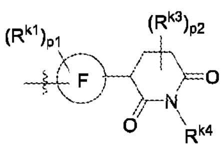 , 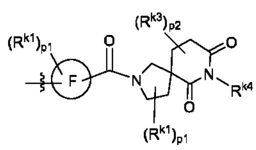 , 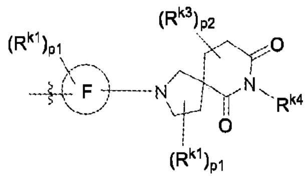 ,

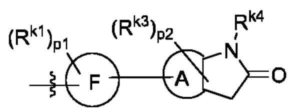 , 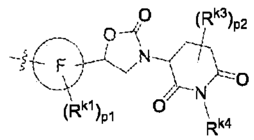 , 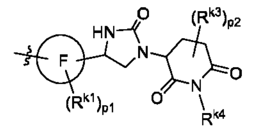 ,

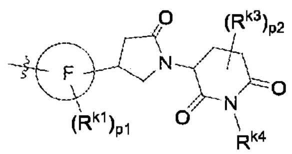 , 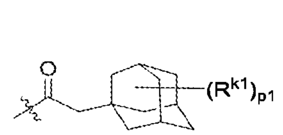 , 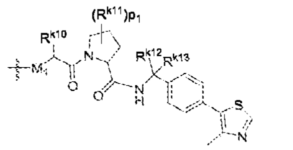 ,

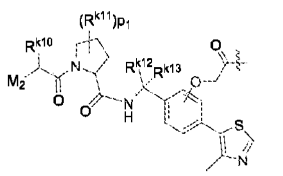 , 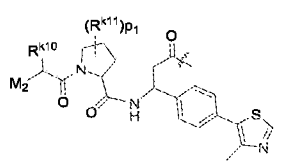 , 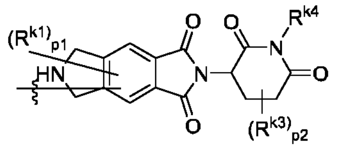 ,

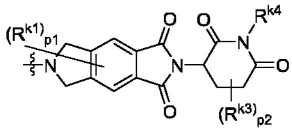, 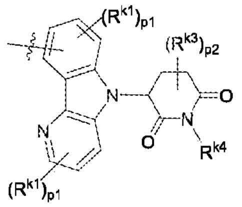 or 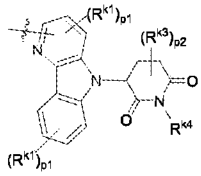;

in some embodiments, each Q is independently selected from a bond, -O-, -S-, $-CH_2-$, $-NR^q-$, -CO-, $-NR^qCO-$, $-CONR^q-$ or 3- to 12-membered heterocycle, wherein the heterocycle is optionally further substituted with 0 to 4 (such as 0, 1, 2, 3 or 4) substituents selected from H, F, Cl, Br, I, OH, =O, $NH_2$, CN, COOH, $CONH_2$, $C_{1-4}$ alkyl or $C_{1-4}$ alkoxy, and the heterocycle contains 1 to 4 (such as 1, 2, 3 or 4) heteroatoms selected from O, S or N;

in some embodiments, each Q is independently selected from -O-, -S-, $-CH_2-$, $-NR^q-$, -CO-, $-NR^qCO-$, $-CONR^4-$ or 4- to 7-membered heterocycle, wherein the heterocycle is optionally further substituted with 0 to 4 (such as 0, 1, 2, 3 or 4) substituents selected from H, F, Cl, Br, I, OH, =O, $NH_2$, CN, COOH, $CONH_2$, $C_{1-4}$ alkyl or $C_{1-4}$ alkoxy, and the heterocycle contains 1 to 4 (such as 1, 2, 3 or 4) heteroatoms selected from O, S or N;

in some embodiments, $R^q$ is selected from H or $C_{1-6}$ alkyl;

in some embodiments, $R^q$ is selected from H or $C_{1-4}$ alkyl;

in some embodiments, $R^q$ is selected from H, methyl or ethyl;

in some embodiments, each E is independently selected from $C_{3-10}$ carbocycle, a $C_{6-10}$ aromatic ring, 3- to 12-membered heterocycle or a 5- to 12-membered heteroaromatic ring, wherein the heterocycle or heteroaromatic ring contains 1 to 4 (such as 1, 2, 3 or 4) heteroatoms selected from O, S or N;

in some embodiments, each E is independently selected from $C_{3-8}$ carbocycle, a benzene ring, 4- to 7-membered heterocycle, 8- to 12-membered heterocycle, a 7-to 12-membered heteroaromatic ring or a 5- to 6-membered heteroaromatic ring, wherein the heterocycle or heteroaromatic ring contains 1 to 4 (such as 1, 2, 3 or 4) heteroatoms selected from O, S or N;

in some embodiments, each E is independently selected from phenyl, pyridyl, pyridazinyl, pyrazinyl, pyrimidinyl, pyrrolyl, pyrazolyl, imidazolyl, thiazolyl, furyl, thienyl, oxazolyl, indolinyl, isoindolinyl, 1,2,3,4-tetrahydroquinolyl or 1,2,3,4-tetrahydroisoquinolinyl;

in some embodiments, each E is independently selected from phenyl, pyridyl, pyridazinyl, pyrazinyl, pyrimidinyl, pyrrolyl, pyrazolyl, imidazolyl, thiazolyl, furyl, thienyl or oxazolyl;

in some embodiments, each E is independently selected from phenyl, pyridyl, pyridazinyl, pyrazinyl or pyrimidinyl;

in some embodiments, each E is independently selected from a benzene ring or a pyridine ring;

in some embodiments, A is selected from $C_{3-10}$ carbocycle, a $C_{6-10}$ aromatic ring, 3- to 10-membered heterocycle or a 5- to 10-membered heteroaromatic ring, wherein the heterocycle or heteroaromatic ring contains 1 to 4 (such as 1, 2, 3 or 4) heteroatoms selected from O, S or N;

in some embodiments, each A, H1 or H2 is independently selected from $C_{3-8}$ carbocycle, a benzene ring, 4- to 7-membered heterocycle or a 5- to 6-membered heteroaromatic ring, wherein the heterocycle or heteroaromatic ring contains 1 to 4 (such as 1, 2, 3 or 4) heteroatoms selected from O, S or N;

in some embodiments, each A, H1 or H2 is independently selected from phenyl, pyridyl, pyridazinyl, pyrazinyl, pyrimidinyl, pyrrolyl, pyrazolyl, imidazolyl, thiazolyl, furyl, thienyl or oxazolyl;

in some embodiments, each A, H1 or H2 is independently selected from phenyl or pyridyl;

in some embodiments, each F is independently selected from $C_{3-20}$ carbocyclyl, $C_{6-20}$ aryl, 3- to 20-membered heterocyclyl or a 5- to 20-membered heteroaromatic ring, wherein the heterocyclyl or heteroaromatic ring contains 1 to 4 (such as 1, 2, 3 or 4) heteroatoms selected from O, S or N;

in some embodiments, each F is independently selected from 3- to 7-membered monocycloalkyl, 4- to 10-membered fused cycloalkyl, 5- to 12-membered spiro cycloalkyl, 5- to 10-membered bridged cycloalkyl, a 4- to 7-membered mono-heterocyclic ring, a 4- to 10-membered fused-heterocyclic ring, a 5- to 12-membered spiro-heterocyclic ring, a 5- to 10-membered bridged-heterocyclic ring, a $C_{6-14}$ aromatic ring or a 5- to 10-membered heteroaromatic ring, wherein the mono-heterocyclic ring, fused-heterocyclic ring, spiro-heterocyclic ring, bridged-heterocyclic ring or heteroaromatic ring contains 1 to 4 (such as 1, 2, 3 or 4) heteroatoms selected from O, S or N;

in some embodiments, each F is independently selected from cyclopropyl, cyclobutyl, cyclopentyl, cyclohexyl, bicyclo[1.1.1]pentyl, 6,7-dihydro-5H-cyclopenta[c]pyridyl, 2,3-dihydro-1H-indenyl, phenyl, naphthyl, anthryl, phenanthryl, azetidinyl, azacyclopentyl, piperidyl, morpholinyl, pyridyl, pyrimidinyl, pyridazinyl, pyrazinyl, triazinyl, pyrrolyl, pyrazolyl, imidazolyl, triazolyl, oxazolyl, furyl, thienyl, thiazolyl, 2-pyridone, benzoxazolyl, pyridoimidazolyl, benzoimidazolyl, benzopyrazolyl, benzothiazolyl, benzothienyl, benzofuryl, benzopyrrolyl, benzopyridyl, benzopyrazinyl, benzopyrimidinyl, benzopyridazinyl, benzotriazinyl, pyrrolopyrrolyl, pyrrolopyridyl, pyrrolopyrimidinyl, pyrrolopyridazinyl, pyrrolopyrazinyl, imidazopyrimidinyl, imidazopyridyl, imidazopyrazinyl, imidazopyridazinyl, pyrazolopyridyl, pyrazolopyrimidinyl, pyrazolopyridazinyl, pyrazolopyrazinyl, pyrimidopyridyl, pyrimidopyrazinyl, pyrimidopyridazinyl, pyrimidopyrimidinyl, pyridopyridyl, pyridopyrazinyl, pyridopyridazinyl, pyridazinopyridazinyl, pyridazinopyrazinyl or pyrazinopyrazinyl;

in some embodiments, each $R^{k2}$ is independently selected from a bond, -CO-, $-SO_2-$, -SO- or $-C(R^{k3})_2-$;

in some embodiments, each $R^{k2}$ is independently selected from -CO-, $-SO_2-$ or $-C(R^{k3})_2-$;

in some embodiments, each $R^{k1}$ is independently selected from H, F, Cl, Br, I, OH, =O, $NH_2$, CN, COOH, $CONH_2$, $C_{1-6}$ alkyl, $C_{1-6}$ alkoxy or $C_{3-6}$ cycloalkyl, wherein the alkyl, alkoxy or cycloalkyl is optionally further substituted with 0 to 4 (such as 0, 1, 2, 3 or 4) substituents selected from H, F, Cl, Br, I, OH, =O, $NH_2$, CN, COOH, $CONH_2$, $C_{1-4}$ alkyl, $C_{1-4}$ alkoxy or $C_{3-6}$ cycloalkyl;

in some embodiments, each $R^{k3}$ is independently selected from H, F, Cl, Br, I, OH, =O, $NH_2$, CN, COOH, $CONH_2$, $C_{1-6}$ alkyl, $C_{1-6}$ alkoxy, $C_{3-8}$ cycloalkyl or 3- to 8-membered heterocyclyl, wherein the alkyl, alkoxy, cycloalkyl or heterocyclyl is optionally further substituted with 0 to 4 (such as 0, 1, 2, 3 or 4) substituents selected from H, F, Cl, Br, I, OH, =O, $NH_2$, CN, COOH, $CONH_2$, $C_{1-4}$ alkyl or $C_{1-4}$ alkoxy, and the heterocyclyl contains 1 to 4 (such as 1, 2, 3 or 4) heteroatoms selected from O, S or N;

in some embodiments, $R^{k1}$ and $R^{k3}$ are each independently selected from H, F, Cl, Br, I, OH, =O, $NH_2$, $CF_3$, CN, COOH, $CONH_2$, $C_{1-4}$ alkyl or $C_{1-4}$ alkoxy, wherein the alkyl or alkoxy is optionally further substituted with 0 to 4 (such as 0, 1, 2, 3 or 4) substituents selected from H, F, Cl, Br, I, OH or $NH_2$;

in some embodiments, $R^{k1}$ and $R^{k3}$ are each independently selected from H, F, Cl, Br, I, OH, =O, $NH_2$, $CF_3$, CN, COOH, $CONH_2$, methyl, ethyl, isopropyl, methoxy, ethoxy or isopropoxy, wherein the methyl, ethyl, isopropyl, methoxy, ethoxy or isopropoxy is optionally further substituted with 0 to 4 (such as 0, 1, 2, 3 or 4) substituents selected from H, F, Cl, Br, I, OH or $NH_2$;

in some embodiments, two $R^{k3}$ together with the carbon atoms or ring backbones to which they are directly attached form 3- to 8-membered carbocycle or 3- to 8-membered heterocycle, and two $R^{k1}$ together with the carbon atoms or ring backbones to which they are directly attached form 3- to 8-membered carbocycle or 3- to 8-membered heterocycle, wherein the carbocycle or heterocycle is optionally further substituted with 0 to 4 (such as 0, 1, 2, 3 or 4) substituents selected from H, F, Cl, Br, I, OH, =O, $NH_2$, CN, COOH, $CONH_2$, $C_{1-4}$ alkyl or $C_{1-4}$ alkoxy, and the heterocycle contains 1 to 4 (such as 1, 2, 3 or 4) heteroatoms selected from O, S or N;

in some embodiments, two $R^{k3}$ together with the carbon atoms or ring backbones to which they are directly attached form 3- to 6-membered carbocycle or 3- to 7-membered heterocycle, and two $R^{k1}$ together with the carbon atoms or ring backbones to which they are directly attached form 3- to 6-membered carbocycle or 3- to 7-membered heterocycle, wherein the carbocycle or heterocycle is optionally further substituted with 0 to 4 (such as 0, 1, 2, 3 or 4) substituents selected from H, F, Cl, Br, I, OH, =O, $NH_2$, CN, COOH, $CONH_2$, $C_{1-4}$ alkyl or $C_{1-4}$ alkoxy, and the heterocycle contains 1 to 4 (such as 1, 2, 3 or 4) heteroatoms selected from O, S or N;

in some embodiments, each $R^{k4}$ is independently selected from H, OH, $NH_2$, CN, $CONH_2$, $C_{1-6}$ alkyl, $C_{3-8}$ cycloalkyl or 3- to 8-membered heterocyclyl, wherein the alkyl, cycloalkyl or heterocyclyl is optionally further substituted with 0 to 4 (such as 0, 1, 2, 3 or 4) substituents selected from H, F, Cl, Br, I, OH, =O, $NH_2$, CN, COOH, $CONH_2$, $C_{1-4}$ alkyl or $C_{1-4}$ alkoxy, and the heterocyclyl contains 1 to 4 (such as 1, 2, 3 or 4) heteroatoms selected from O, S or N;

in some embodiments, each $R^{k4}$ is independently selected from H, OH, $NH_2$, $CF_3$, CN or $C_{1-4}$ alkyl;

in some embodiments, each $R^{k5}$ is independently selected from

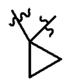,

$C(CH_3)_2$, CO, $CH_2$, $SO_2$,

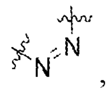, 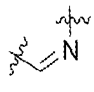 or 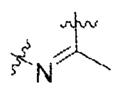;

in some embodiments, each $R^{k5}$ is independently selected from CO, $CH_2$, $SO_2$ or

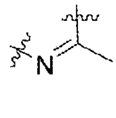;

in some embodiments, each $R^{k6}$ is independently selected from CO, CH, SO, $SO_2$, $CH_2$ or N;

in some embodiments, each $R^{k7}$ is independently selected from

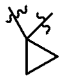,

$C(CH_3)_2$, CO, CH, N, $CH_2$, O, S, $N(CH_3)$, $N(CH_2CH_3)$, N(cyclopropyl) or NH;

in some embodiments, each $R^{k7}$ is independently selected from

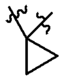,

$C(CH_3)_2$, $CH_2$, O, $N(CH_3)$, $N(CH_2CH_3)$, N(cyclopropyl) or NH;

in some embodiments, each $R^{k7}$ is independently selected from CO, CH, N, $CH_2$, O, S, $N(CH_3)$ or NH;

in some embodiments, each $R^{k7}$ is independently selected from $CH_2$, O, $N(CH_3)$ or NH;

in some embodiments, each $R^{k8}$ is independently selected from C, N or CH;

in some embodiments, each $R^{k9}$ is independently selected from a bond,

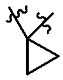,

$C(CH_3)_2$, CO, $CH_2$, $CH_2CH_2$ or $SO_2$;

in some embodiments, each $R^{k9}$ is independently selected from CO, $SO_2$ or $CH_2$;

in some embodiments, $M_1$ is selected from a bond, $-CH_2-C(=O)NH-$ or $-C(=O)CH_2NH-$;

in some embodiments, $M_2$ is selected from $-NHC(=O)-C_{1-6}$ alkyl, $-NHC(=O)-C_{3-6}$ cycloalkyl or 4- to 10-membered heterocyclyl, wherein the alkyl, cycloalkyl or heterocyclyl is optionally further substituted with 0 to 4 (such as 0, 1, 2, 3 or 4) substituents selected from H, F, Cl, Br, I, =O, OH, $NH_2$, $C_{1-4}$ alkyl or $C_{1-4}$ alkoxy, and the heterocyclyl contains 1 to 4 (such as 1, 2, 3 or 4) heteroatoms selected from O, S or N;

in some embodiments, $M_3$ is selected from -NH- or -O-;

in some embodiments, $R^{k10}$ is selected from $C_{1-6}$ alkyl, wherein the alkyl is optionally further substituted with 0 to 4 (such as 0, 1, 2, 3 or 4) substituents selected from H, F, Cl, Br, I, =O, OH, $C_{1-6}$ alkyl or $C_{3-6}$ cycloalkyl;

in some embodiments, G is selected from a 6- to 10-membered aromatic ring or a 5- to 10-membered heteroaromatic ring, wherein the aromatic ring or heteroaromatic ring is optionally further substituted with 0 to 4 (such as 0, 1, 2, 3 or 4) substituents selected from H, F, Cl, Br, I, OH, =O, $CF_3$, CN, $C_{1-4}$ alkyl, halogen-substituted $C_{1-4}$ alkyl, hydroxyl-substituted $C_{1-4}$ alkyl, $C_{1-4}$ alkoxy or $C_{3-6}$ cycloalkyl, wherein the heteroaromatic ring contains 1 to 4 (such as 1, 2, 3 or 4) heteroatoms selected from N, O or S;

in some embodiments, each $R^{k11}$ is independently selected from H, F, Cl, Br, I, =O, OH, SH, $C_{1-6}$ alkyl, $C_{1-6}$ alkoxy, $C_{1-6}$ alkylthio or $-O-C(=O)-C_{1-6}$ alkyl, wherein the alkyl, alkoxy or alkylthio is optionally further substituted with 0 to 4 (such as 0, 1, 2, 3 or 4) substituents selected from H, F, Cl, Br, I, OH, $C_{1-4}$ alkyl or $C_{1-4}$ alkoxy;

in some embodiments, $R^{k12}$ and $R^{k13}$ are each independently selected from H, $C_{1-6}$ alkyl or $C_{3-6}$ cycloalkyl, wherein the alkyl or cycloalkyl is optionally further substituted with 0 to 4 (such as 0, 1, 2, 3 or 4) substituents selected from H, F, Cl, Br, I, =O, OH, $NH_2$, $C_{1-4}$ alkyl or $C_{1-4}$ alkoxy;

in some embodiments, $R^{k14}$ is selected from 5- to 6-membered heteroaryl, wherein the heteroaryl is optionally further substituted with 0 to 4 (such as 0, 1, 2, 3 or 4) substituents selected from H, F, Cl, Br, I, OH, =O, $CF_3$, CN, $C_{1-4}$ alkyl, halogen-substituted $C_{1-4}$ alkyl, hydroxyl-substituted $C_{1-4}$ alkyl, $C_{1-4}$ alkoxy or $C_{3-6}$ cycloalkyl, and the heteroaryl contains 1 to 4 (such as 1, 2, 3 or 4) heteroatoms selected from N, O or S;

in some embodiments, K is selected from one of the structural fragments shown in Table K-1;

in some embodiments, K is selected from one of the structural fragments shown in Table K-2;

in some embodiments, K is selected from one of the structural fragments shown in Table K-3;

Table K-1

| | | | |
|---|---|---|---|
| 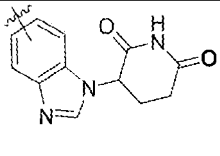 | 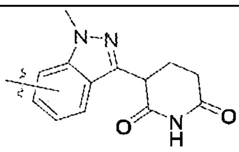 | 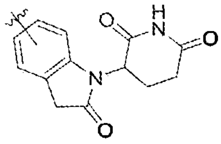 | 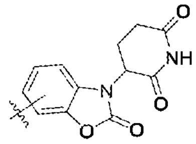 |
| 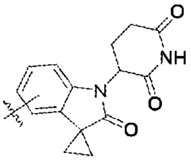 | 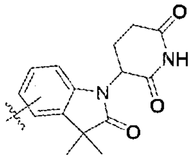 | 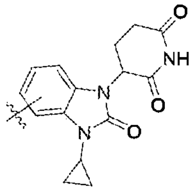 | 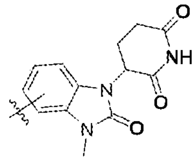 |
| 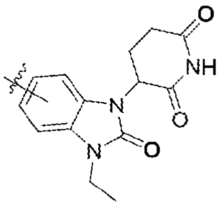 | 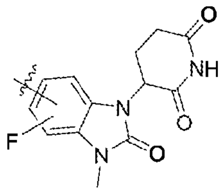 | 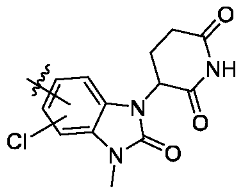 | 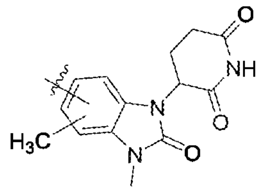 |
| 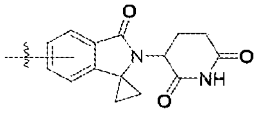 | 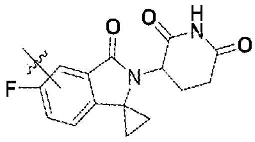 | 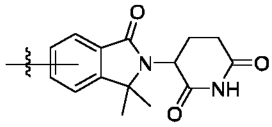 | 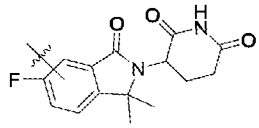 |
| 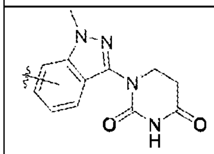 | 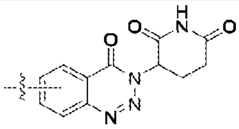 | 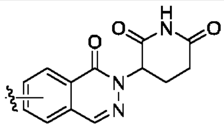 | 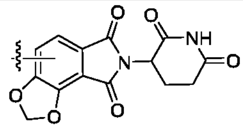 |
| 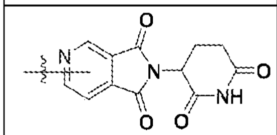 | 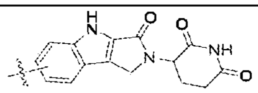 | | |

| | | | |
|---|---|---|---|
| 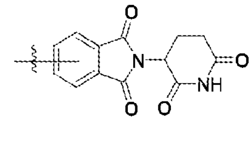 | 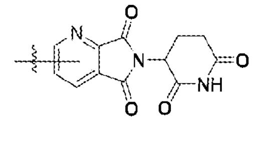 | 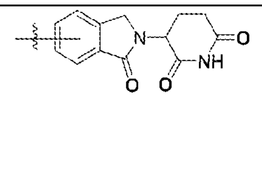 | 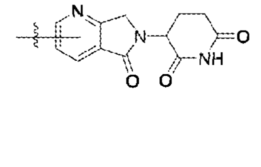 |
| 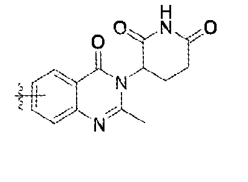 | 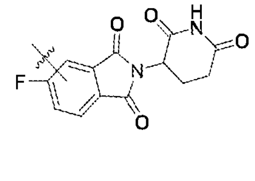 | 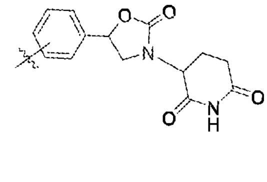 | 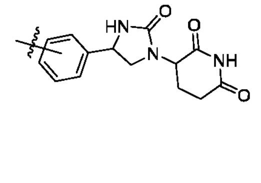 |
| 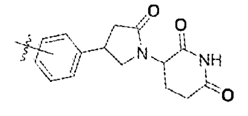 | 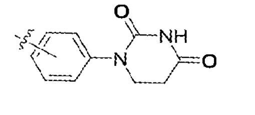 | 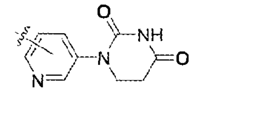 | 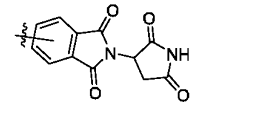 |
| 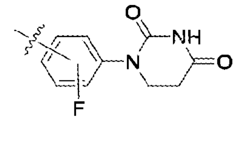 | 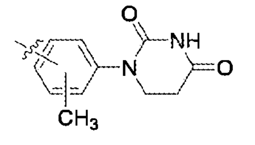 | 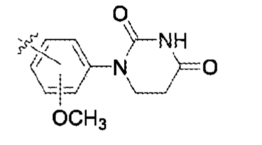 | |
| 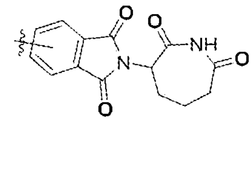 | 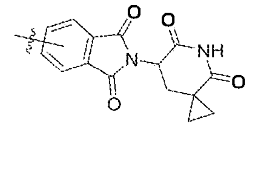 | 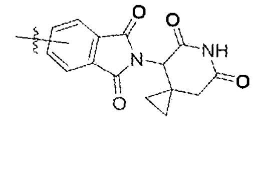 | 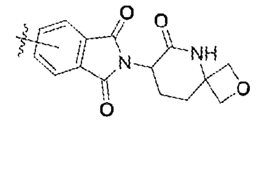 |
| 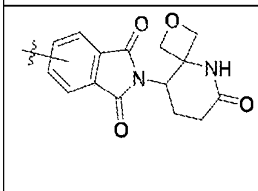 | 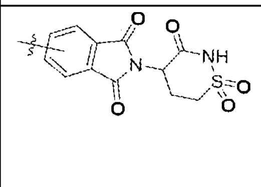 | 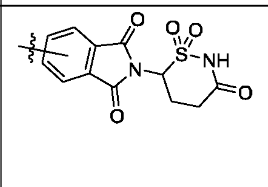 | 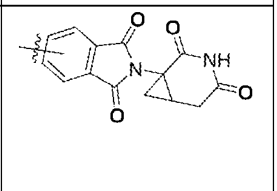 |
| 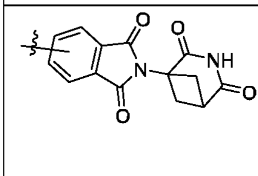 | 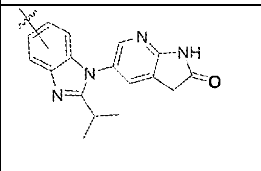 | 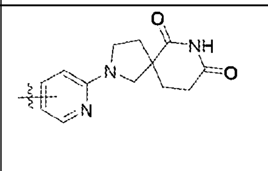 | 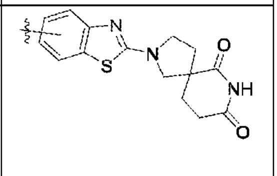 |
| 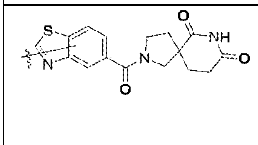 | 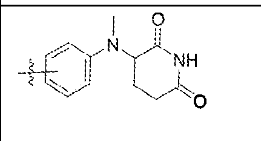 | 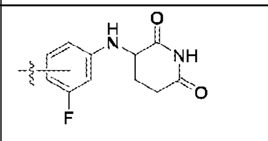 | 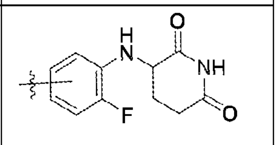 |
| 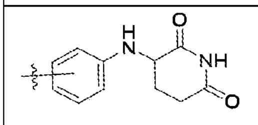 | 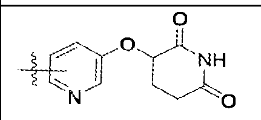 | 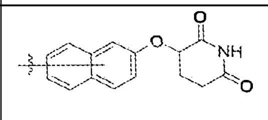 | 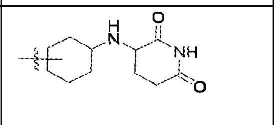 |
| 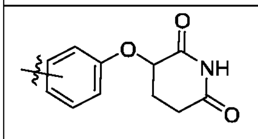 | 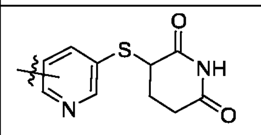 | 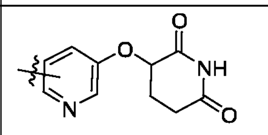 | 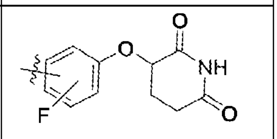 |
| 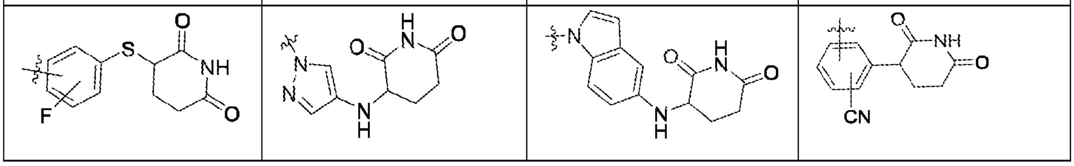 | | | |

| | | | |
|---|---|---|---|
| 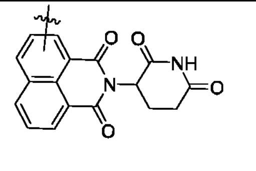 | 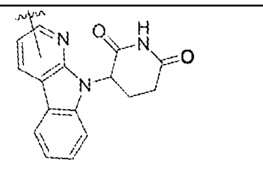 | 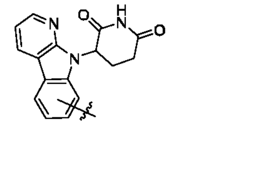 | 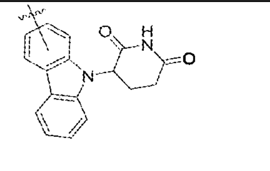 |
| 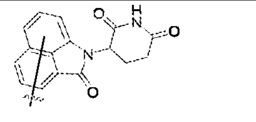 | 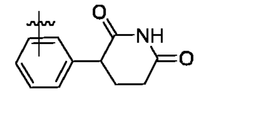 | 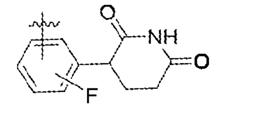 | 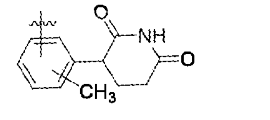 |
| 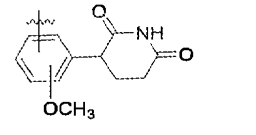 | 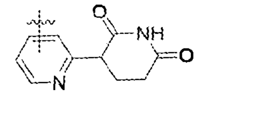 | 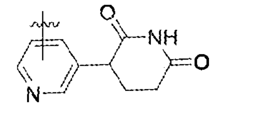 | 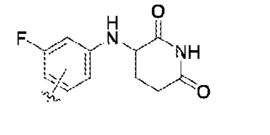 |
| 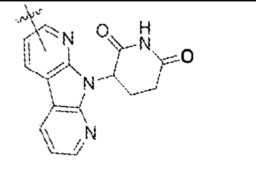 | 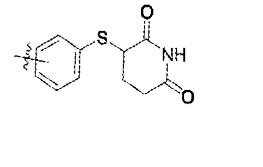 | 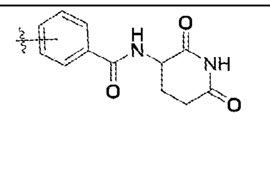 | 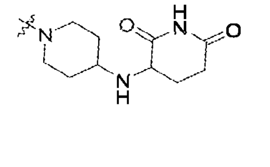 |
| 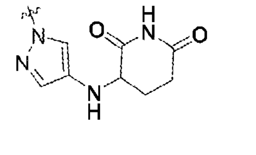 | 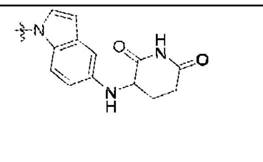 | 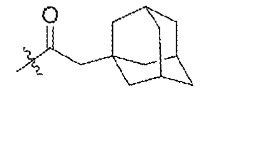 | 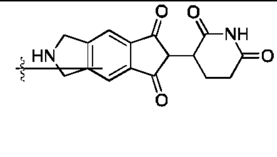 |
| 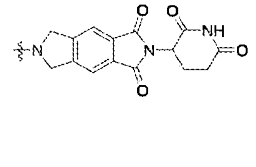 | 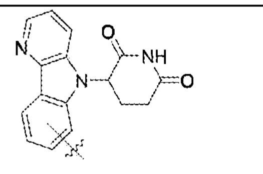 | 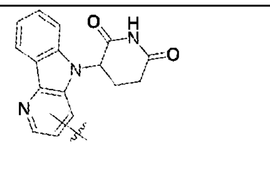 | 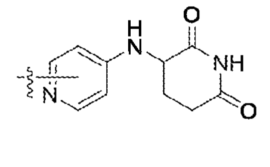 |
| 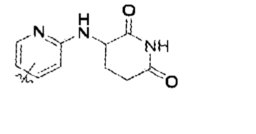 | 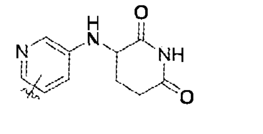 | 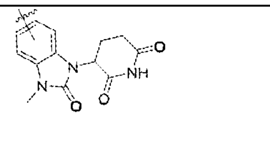 | 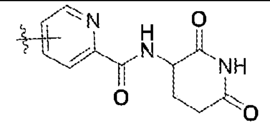 |
| 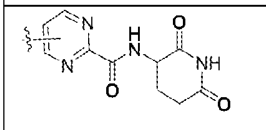 | 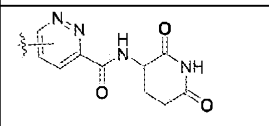 | 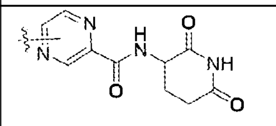 | |
| 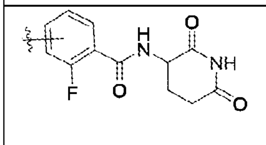 | 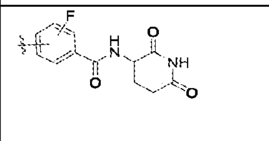 | 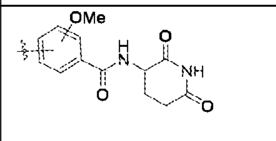 | 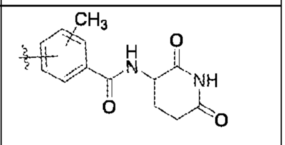 |
| 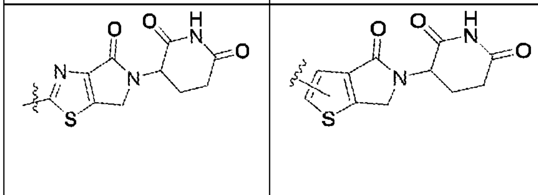 | | 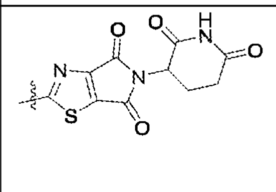 | 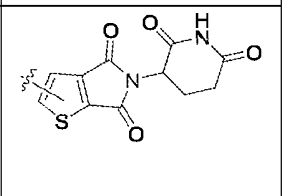 |
| 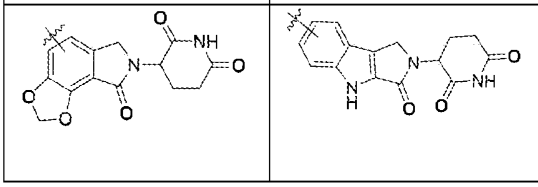 | | 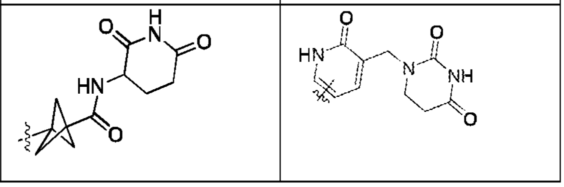 | |

| | | | |
|---|---|---|---|
| 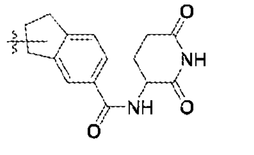 | 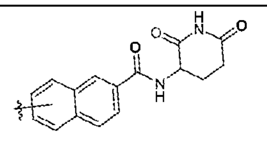 | 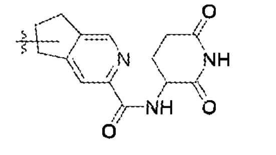 | 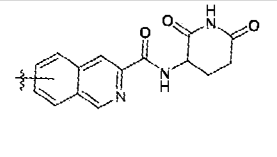 |
| 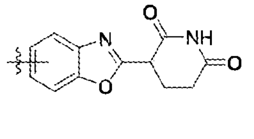 | 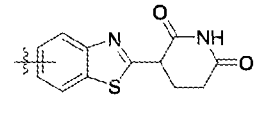 | 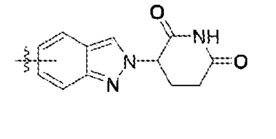 | 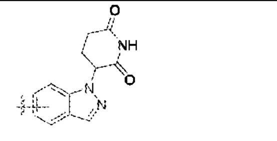 |
| 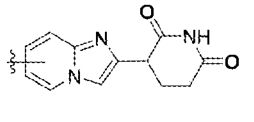 | 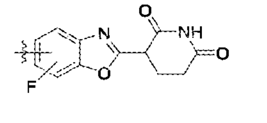 | 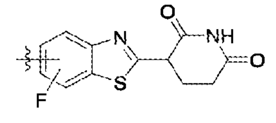 | 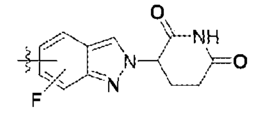 |
| 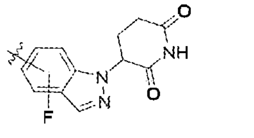 | 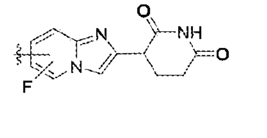 | | |
| 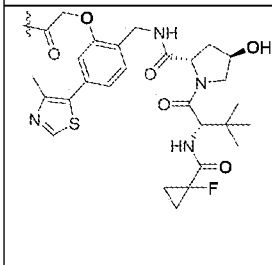 | 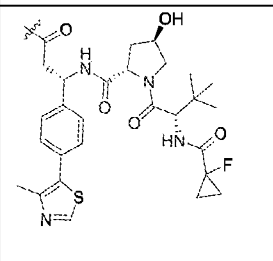 | 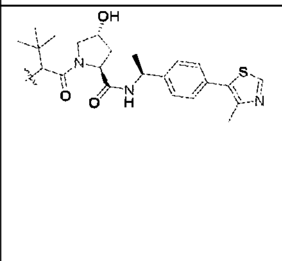 | 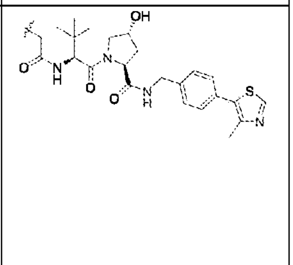 |
| 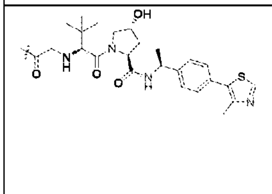 | 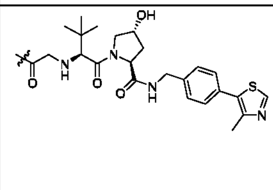 | 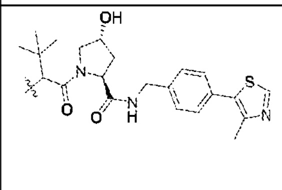 | 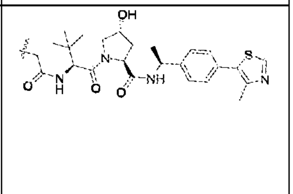 |
| 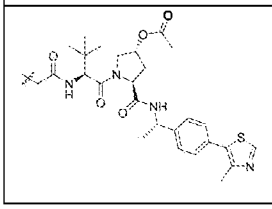 | 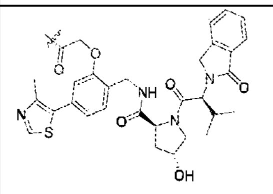 | | |

Table K-2

| | | | |
|---|---|---|---|
| 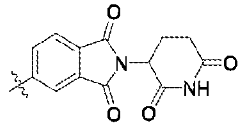 | 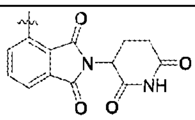 | 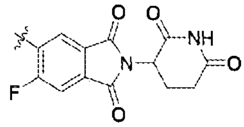 | 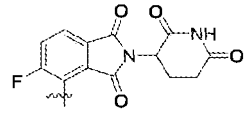 |
| 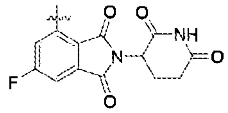 | 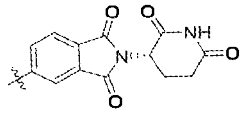 | 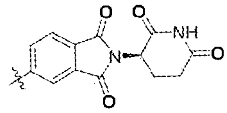 | 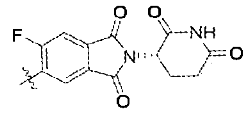 |
| 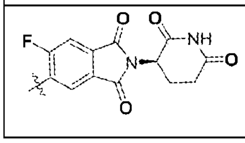 | 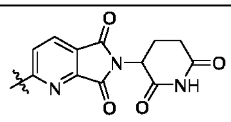 | 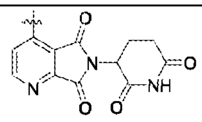 | 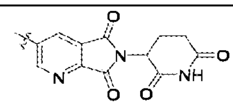 |

| | | | |
|---|---|---|---|
| 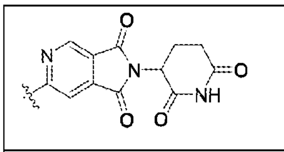 | 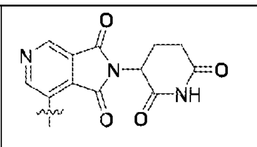 | 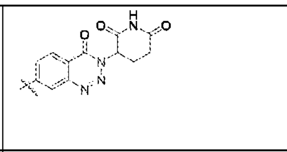 | 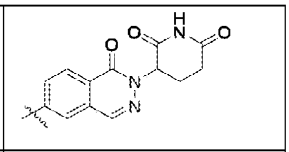 |
| 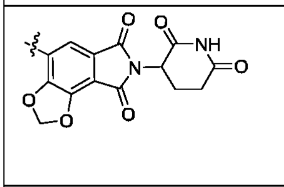 | 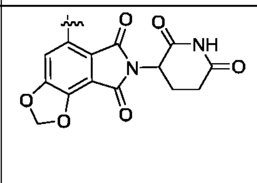 | 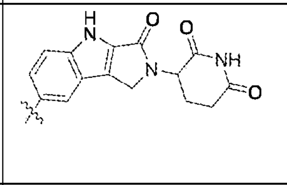 | |
| 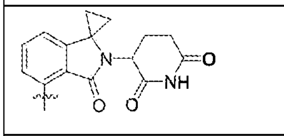 | 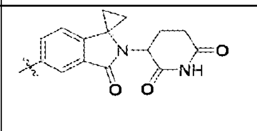 | 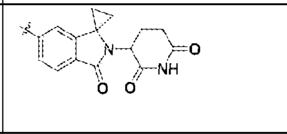 | 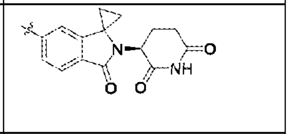 |
| 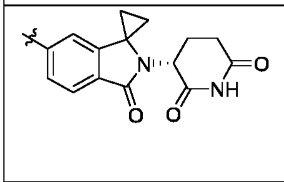 | 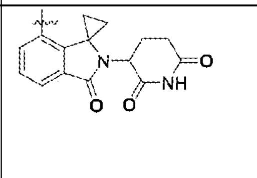 | 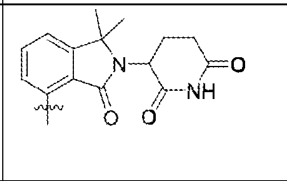 | 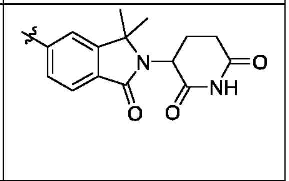 |
| 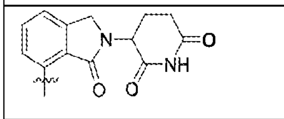 | 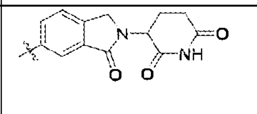 | 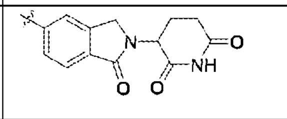 | 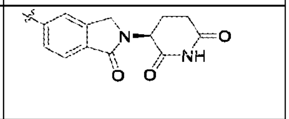 |
| 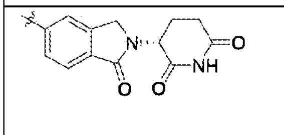 | 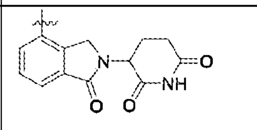 | 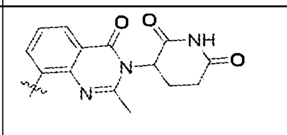 | 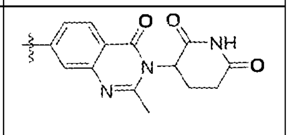 |
| 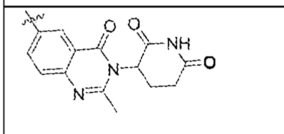 | 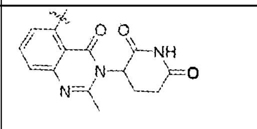 | 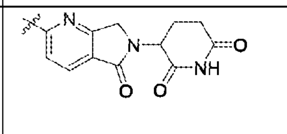 | 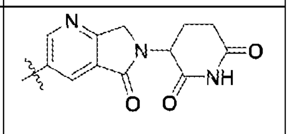 |
| 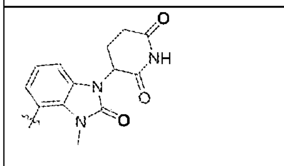 | 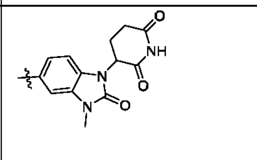 | 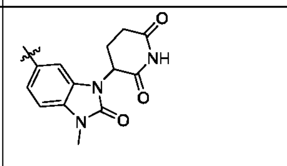 | 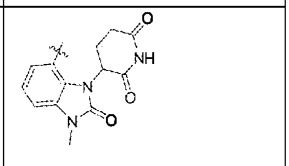 |
| 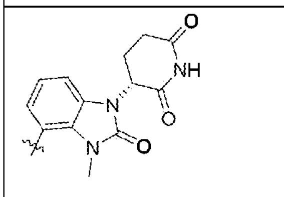 | 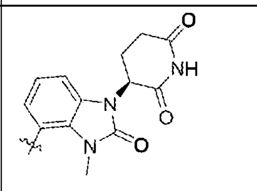 | 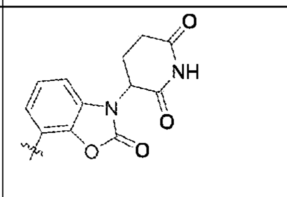 | 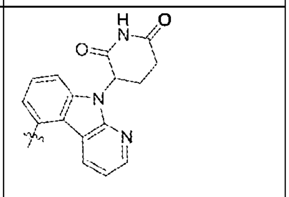 |
| 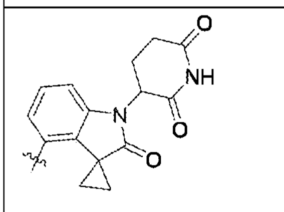 | 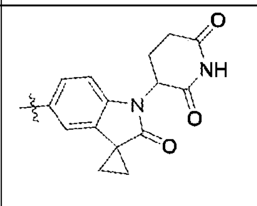 | 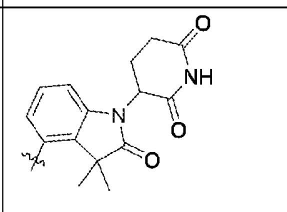 | 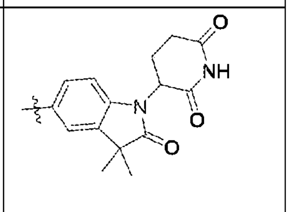 |
| 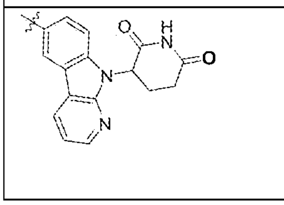 | | | |

| | | | |
|---|---|---|---|
| 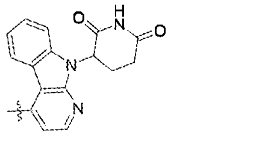 | 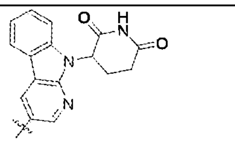 | 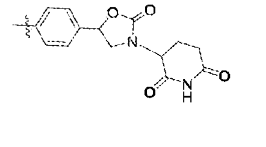 | 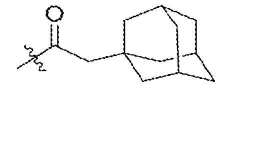 |
| 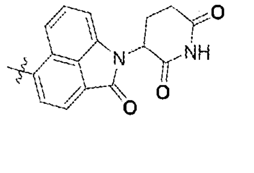 | 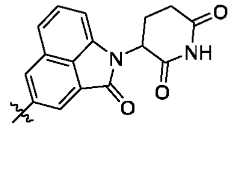 | 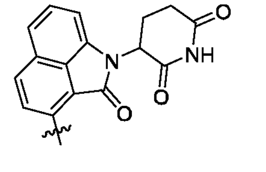 | 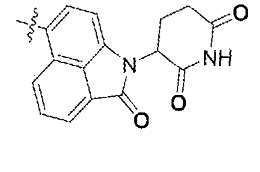 |
| 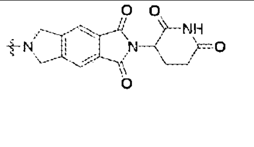 | 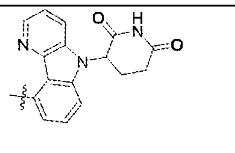 | 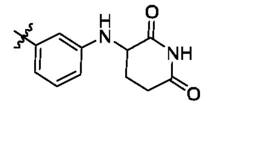 | 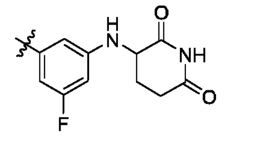 |
| 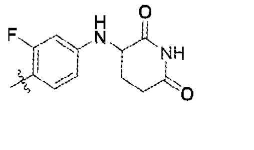 | 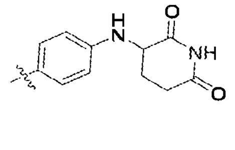 | 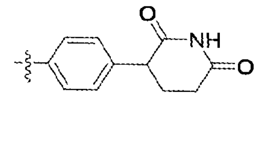 | 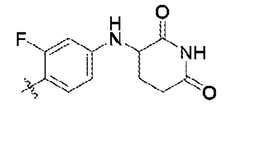 |
| 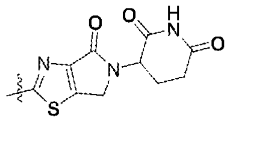 | 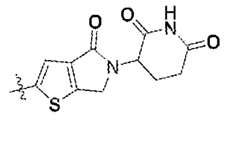 | 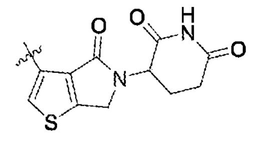 | 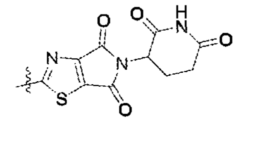 |
| 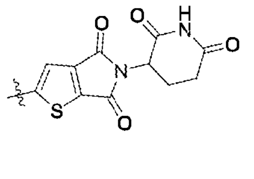 | 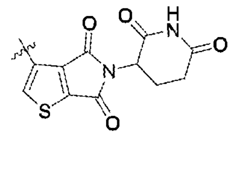 | 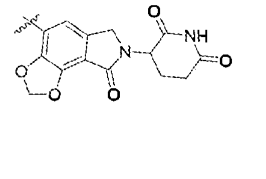 | 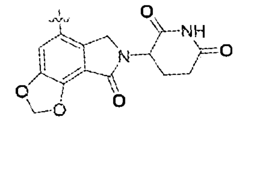 |
| 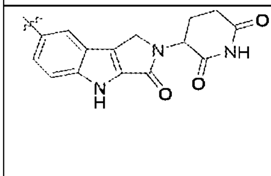 | 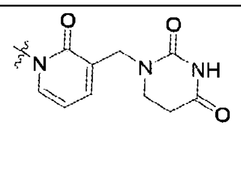 | 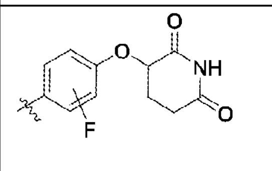 | 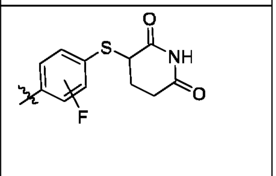 |
| 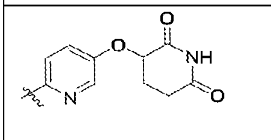 | 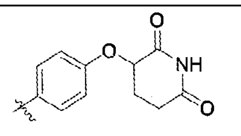 | 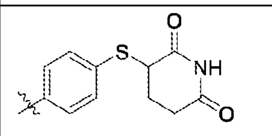 | 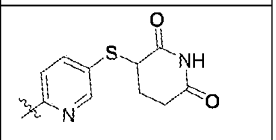 |
| 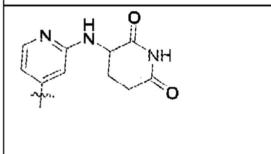 | 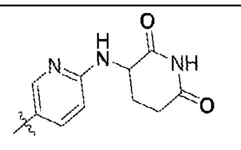 | 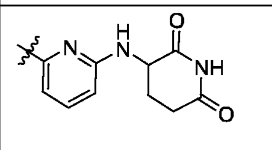 | 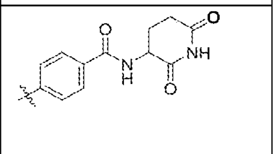 |
| 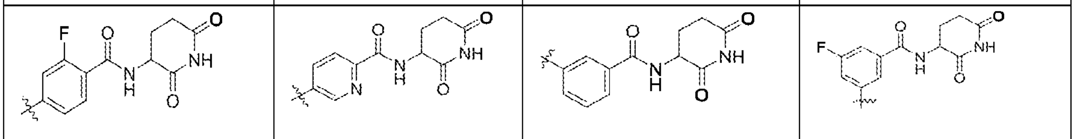 | | | |
| 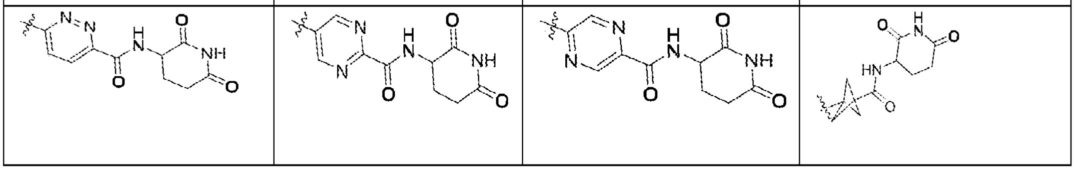 | | | |

| | | | |
|---|---|---|---|
| 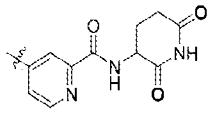 | 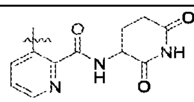 | 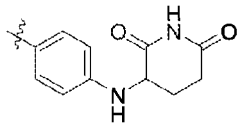 | 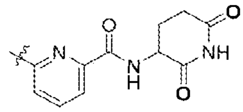 |
| 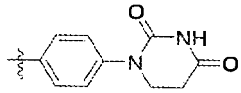 | 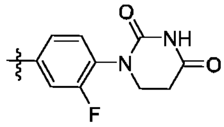 | 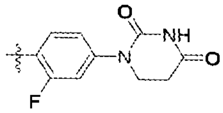 | 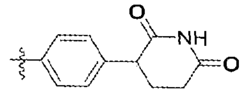 |
| 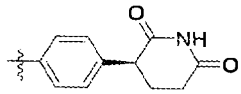 | 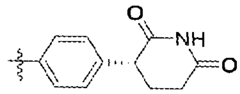 | 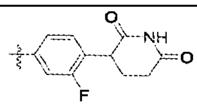 | 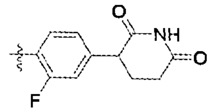 |

Table K-3

| | | | |
|---|---|---|---|
| 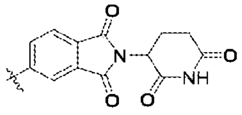 | 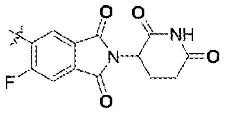 | 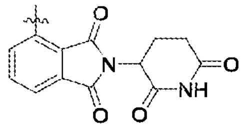 | 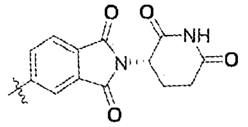 |
| 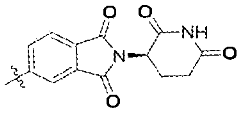 | 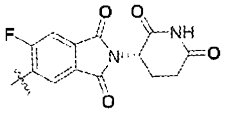 | 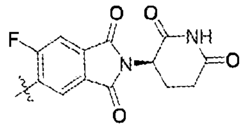 | 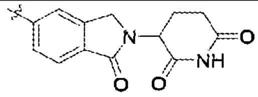 |
| 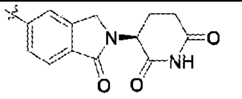 | 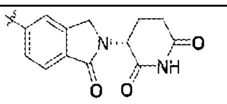 | 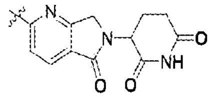 | 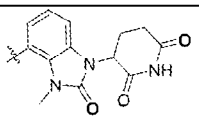 |
| 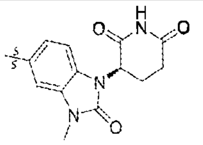 | 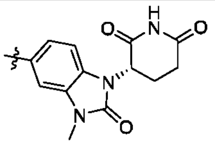 | | |
| 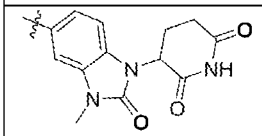 | 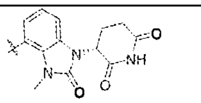 | 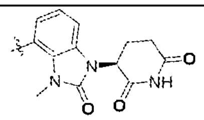 | 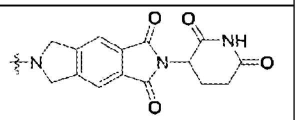 |
| 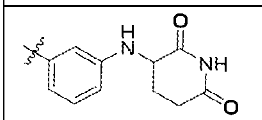 | 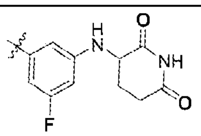 | 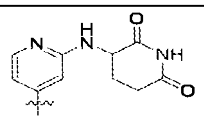 | 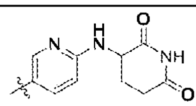 |
| 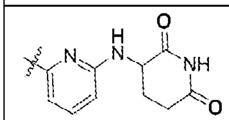 | 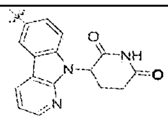 | 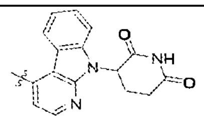 | 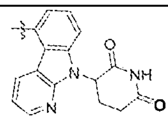 |
| 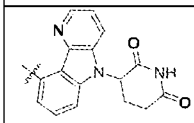 | 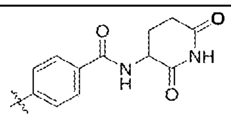 | 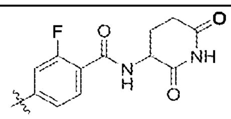 | 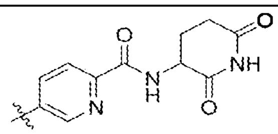 |
| 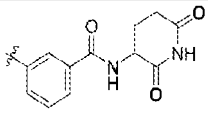 | 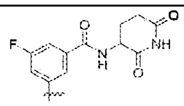 | 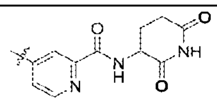 | 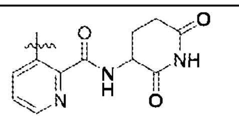 |

| | | | |
|---|---|---|---|
| 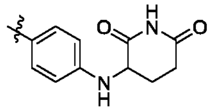 | 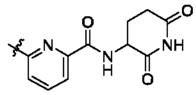 | 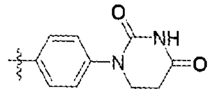 | 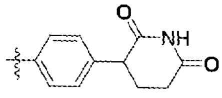 |
| 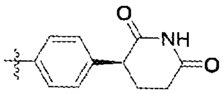 | 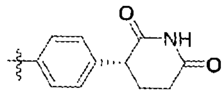 | 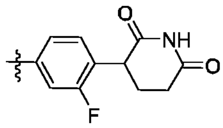 | 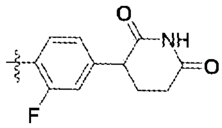 |
| 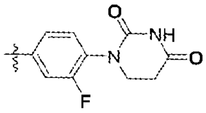 | 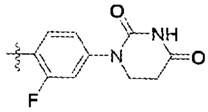 | 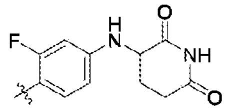 | |
| 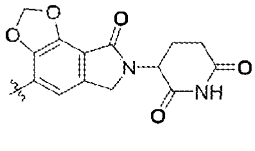 | 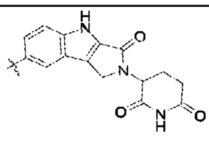 | 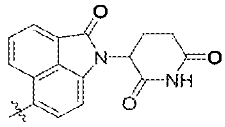 | |

in some embodiments, K is selected from

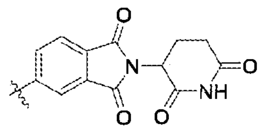 ,

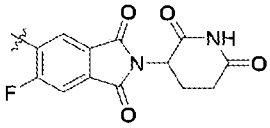 , 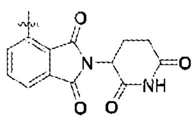 , 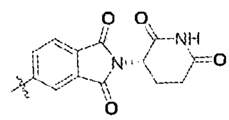 , 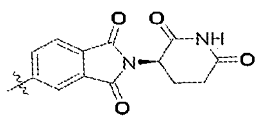 ,

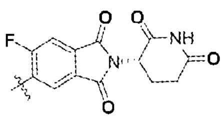 , 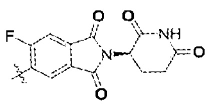 , 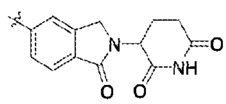 , 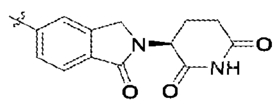 ,

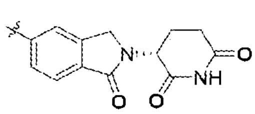 , 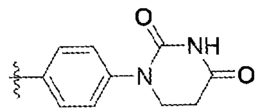 , 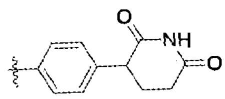 , 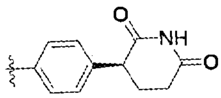 ,

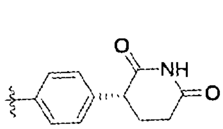 , 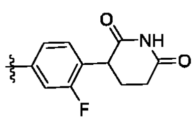 , 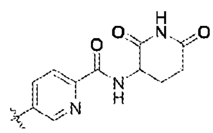 , 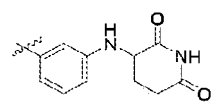 ,

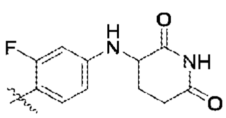 , 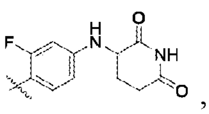 , 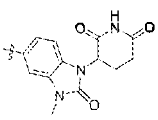 , 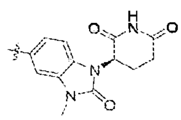 , 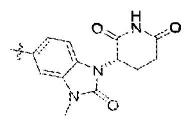 ,

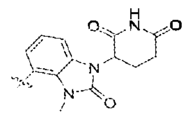, 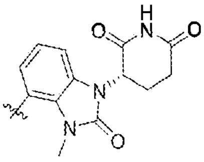 or 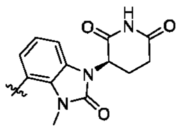;

in some embodiments, B is selected from

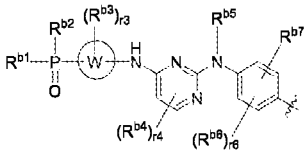

or

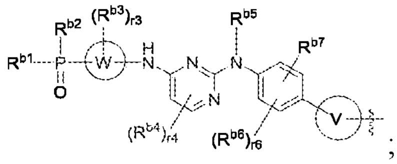;

in some embodiments, B is selected from

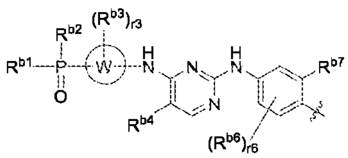,

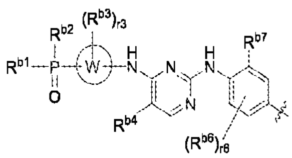, 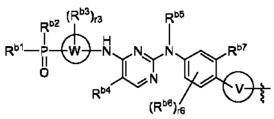 or 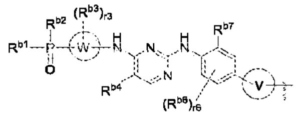;

in some embodiments, B is selected from one of the following structural fragments:

| | | |
|---|---|---|
| 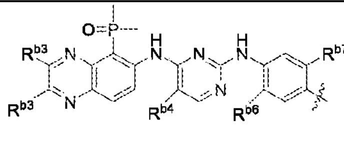 | 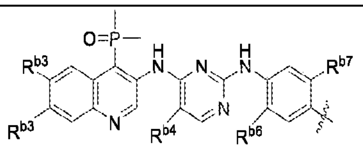 | 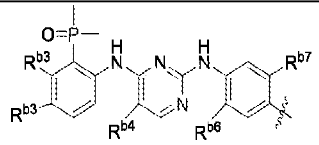 |
| 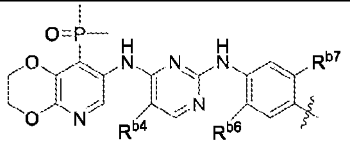 | 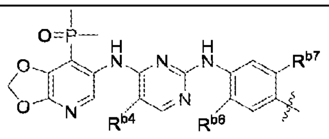 | 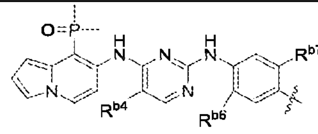 |
| 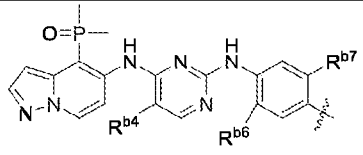 | 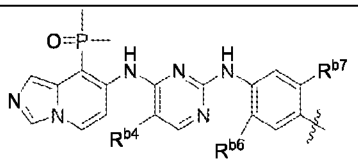 | 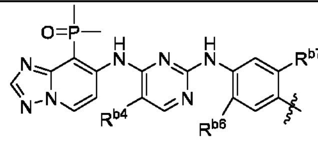 |
| 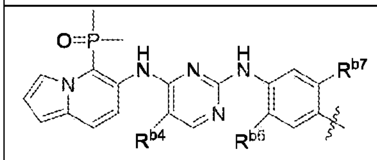 | 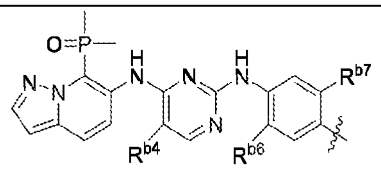 | 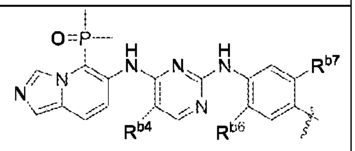 |
| 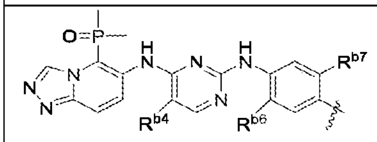 | 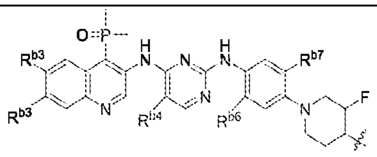 | 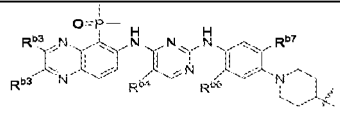 |

| | | |
|---|---|---|
| 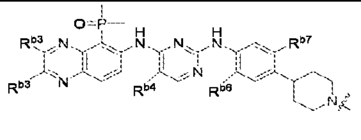 | 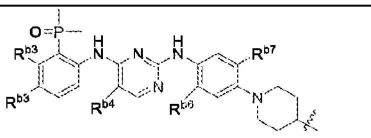 | 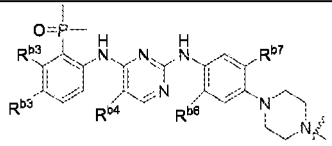 |
| 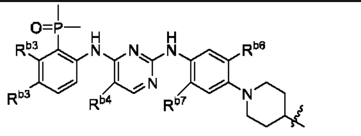 | 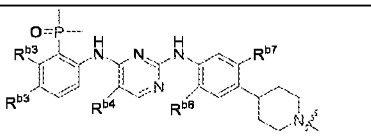 | 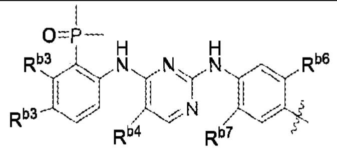 |
| 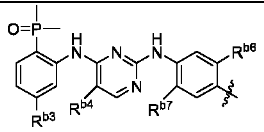 | | |
| 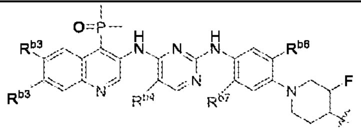 | 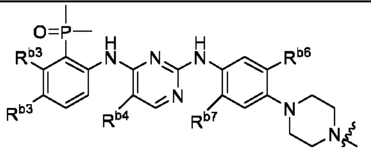 | 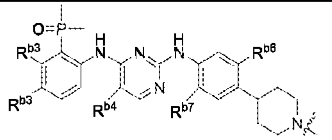 |
| 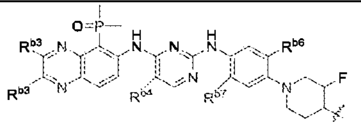 | 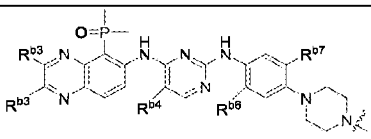 | 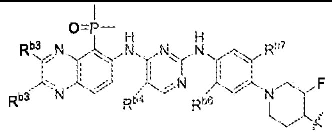 |
| 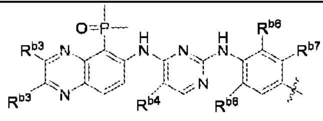 | 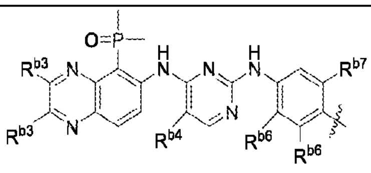 | 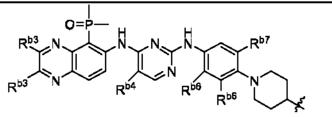 |
| 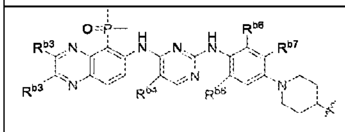 | 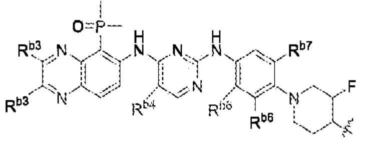 | 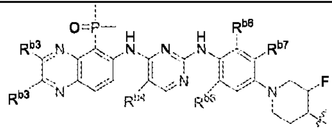 |
| 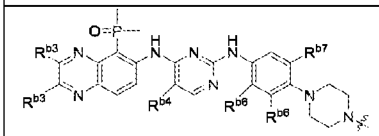 | 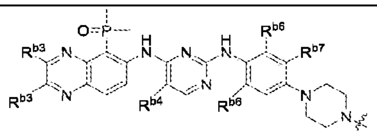 | 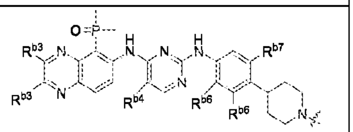 |
| 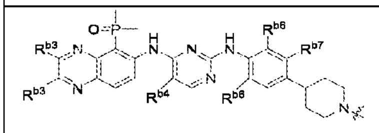 | 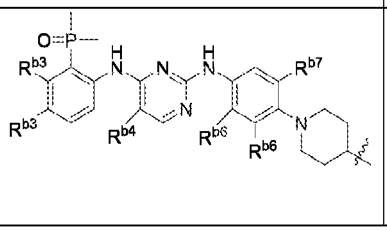 | 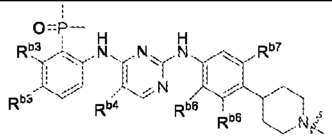 |

| | | |
|---|---|---|
| 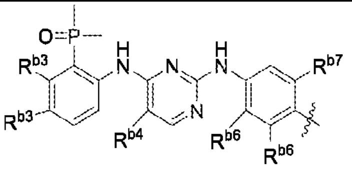 | 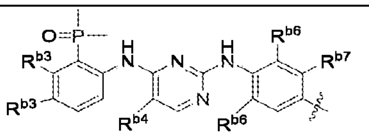 | 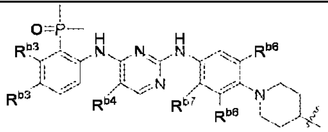 |
| 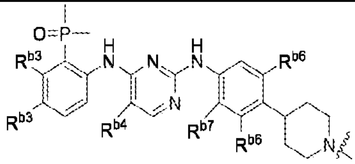 | 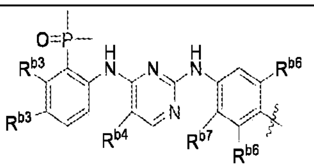 | 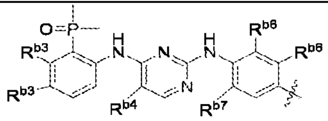 |
| 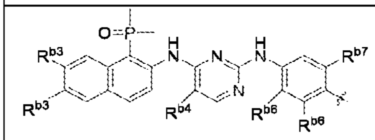 | 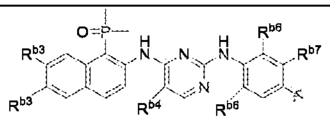 | 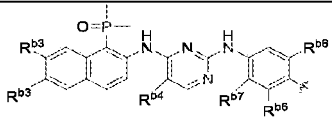 |
| 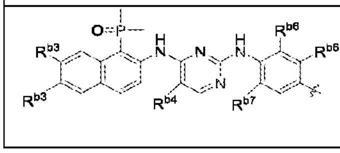 ; | 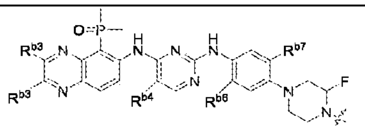 | |

in some embodiments, B is selected from one of the following structural fragments:

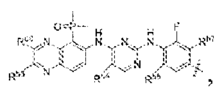 , 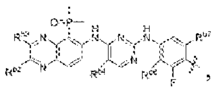 , 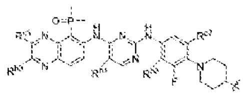 , 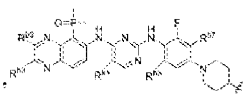 ,

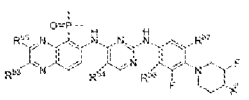 , 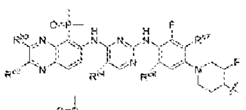 , 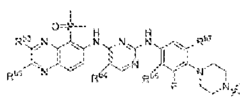 ,

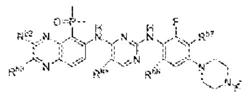 , 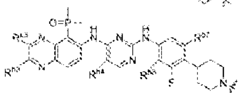 , 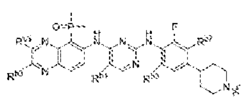 ;

in some embodiments, B is selected from one of the structural fragments shown in Table B-1;

Table B-1

| | | | |
|---|---|---|---|
| 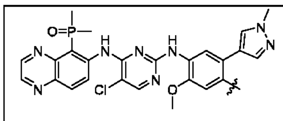 | 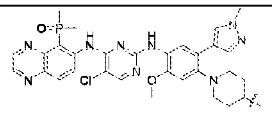 | 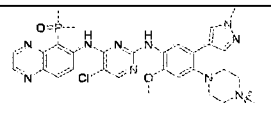 | 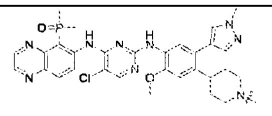 |
| 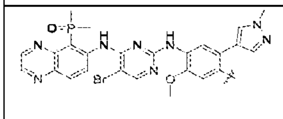 | 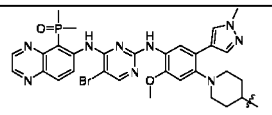 | 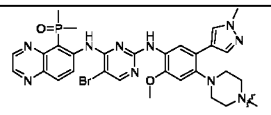 | 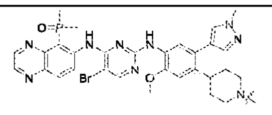 |
| 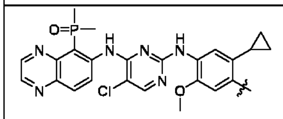 | 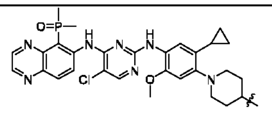 | 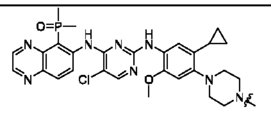 | 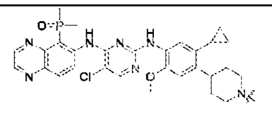 |
| 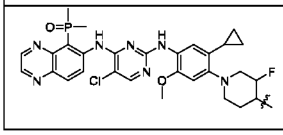 | 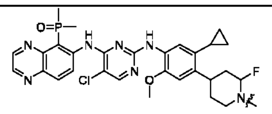 | 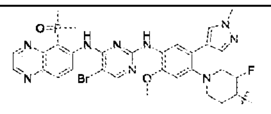 | 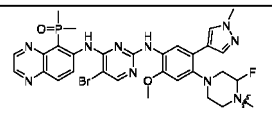 |

| | | | |
|---|---|---|---|
| 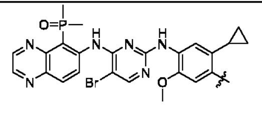 | 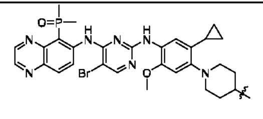 | 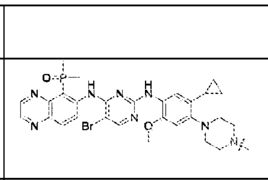 | 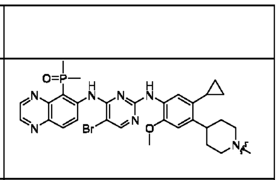 |
| 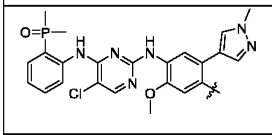 | 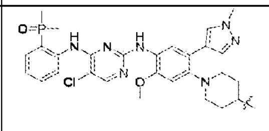 | 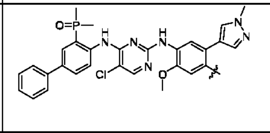 | 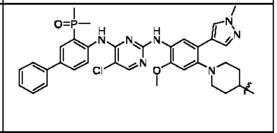 |
| 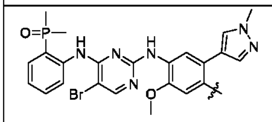 | 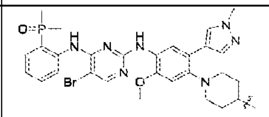 | 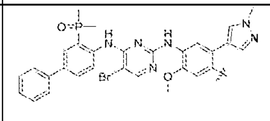 | 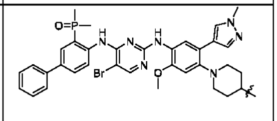 |
| 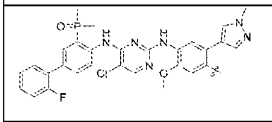 | 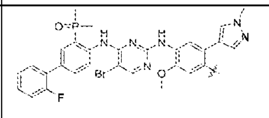 | 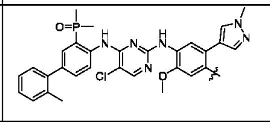 | 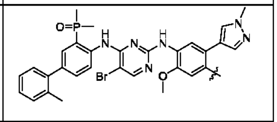 |
| 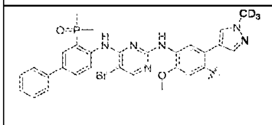 | 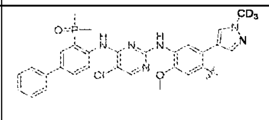 | 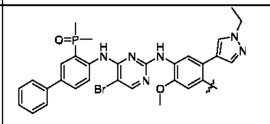 | 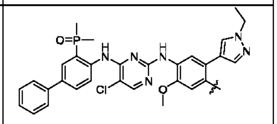 |
| 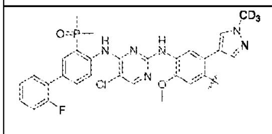 | 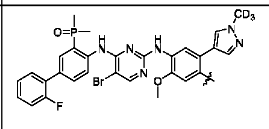 | 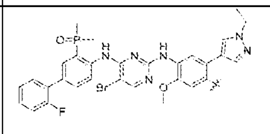 | 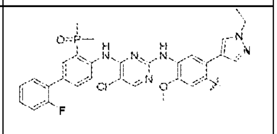 |
| 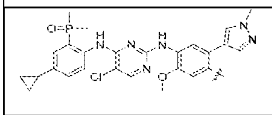 | 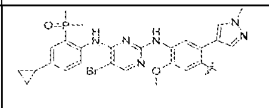 | 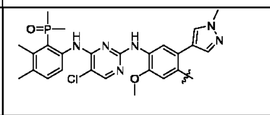 | 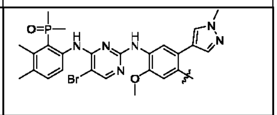 |
| 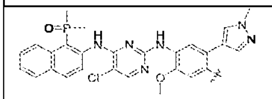 | 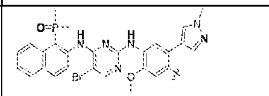 | 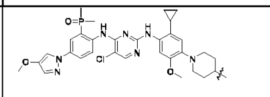 | 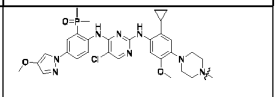 |
| 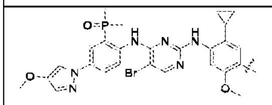 | 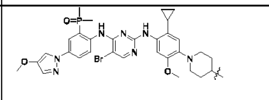 | 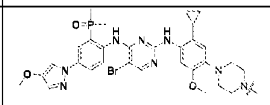 | 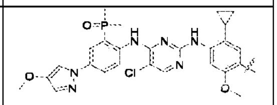 |
| 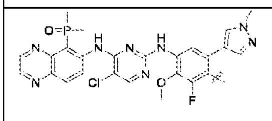 | 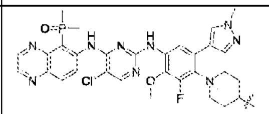 | 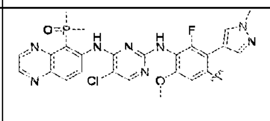 | 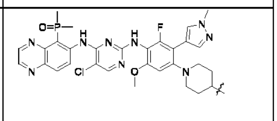 |
| | | | |
| | | | |
| | | | |
| | | | |

| | | | |
|---|---|---|---|
| 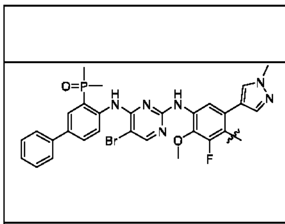 | 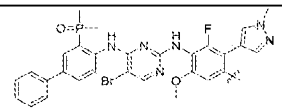 | 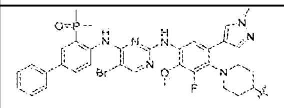 | 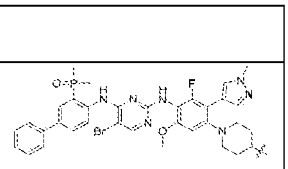 |
| 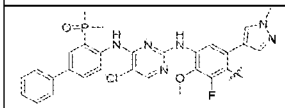 | 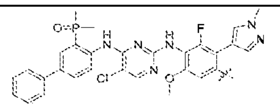 | 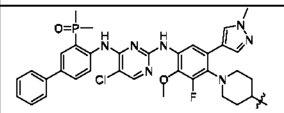 | 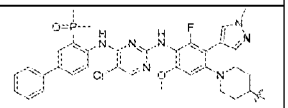 |
| 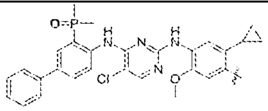 | 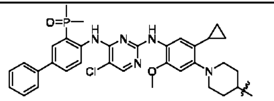 | 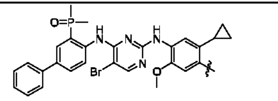 | 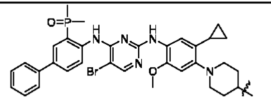 |
| 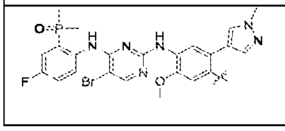 | 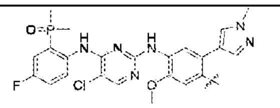 | | |

in some embodiments, B is selected from

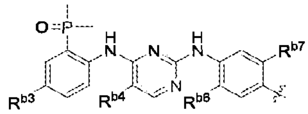

;

in some embodiments, B is selected from

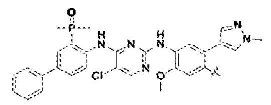

,

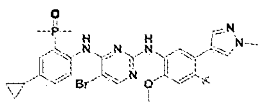 or 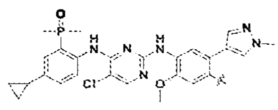 ;

in some embodiments, L is selected from -Cy1-$CH_2$-Cy2-, Cy1 is selected from 4- to 6-membered nitrogen-containing heterocyclyl (preferably azetidinyl, azacyclopentyl, azacyclohexyl and piperazine), and Cy2 is selected from 4- to 6-membered nitrogen-containing heterocyclyl (preferably azetidinyl, azacyclopentyl, azacyclohexyl and piperazine);
in some embodiments, r3 is selected from 0, 1, 2, 3 or 4;
in some embodiments, r4 is selected from 0, 1 or 2;
in some embodiments, r6 is selected from 0, 1, 2 or 3;
in some embodiments, ring W is selected from $C_{6-10}$ carbocycle, 5- to 10-membered heterocycle, a $C_{6-10}$ aromatic ring or a 5- to 10-membered heteroaromatic ring, wherein the heterocycle or heteroaromatic ring contains 1 to 5 (such as 1, 2, 3, 4 or 5) heteroatoms selected from O, S or N;
in some embodiments, ring W is selected from a benzene ring, a naphthalene ring, a 5- to 6-membered monocyclic heteroaromatic ring, a 9- to 10-membered fused heteroaromatic ring or 9- to 10-membered heterocycle, wherein the heterocycle or heteroaromatic ring contains 1 to 5 (such as 1, 2, 3, 4 or 5) heteroatoms selected from O, S or N;
in some embodiments,

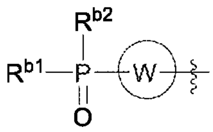

is selected from

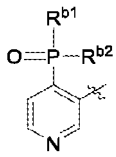, 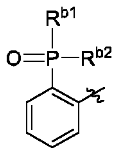,

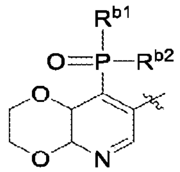, 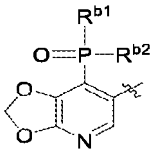, 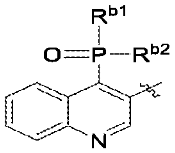, 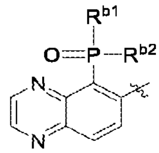, 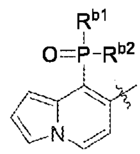, 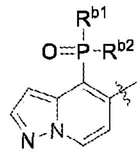,

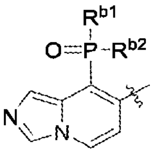, 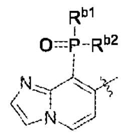, 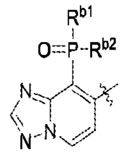, 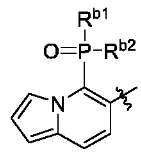, 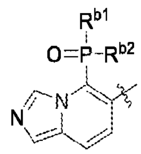, 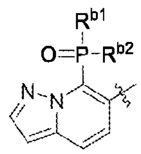, 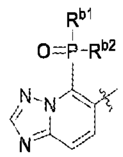,

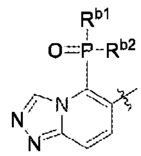 or 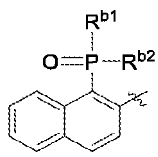;

in some embodiments, ring V is selected from 4- to 10-membered heterocycle or $C_{3-10}$ carbocycle, wherein the heterocycle or carbocycle is optionally further substituted with 0 to 4 (such as 0, 1, 2, 3 or 4) substituents selected from H, F, Cl, Br, I, OH, =O, CN, $C_{1-4}$ alkyl, halogen-substituted $C_{1-4}$ alkyl, cyano-substituted $C_{1-4}$ alkyl or $C_{3-6}$ cycloalkyl;
in some embodiments, ring V is selected from 4- to 6-membered heterocycle or $C_{3-6}$ carbocycle, wherein the heterocycle or carbocycle is optionally further substituted with 0 to 4 (such as 0, 1, 2, 3 or 4) substituents selected from H, F, Cl, Br, I, OH, =O, CN, $C_{1-4}$ alkyl, halogen-substituted $C_{1-4}$ alkyl, cyano-substituted $C_{1-4}$ alkyl or $C_{3-6}$ cycloalkyl;
in some embodiments, ring V is selected from azetidinyl, azacyclopentyl, piperidyl or piperazinyl, wherein the azetidinyl, azacyclopentyl, piperidyl or piperazinyl is optionally further substituted with 0 to 4 (such as 0, 1, 2, 3 or 4) substituents selected from H, F, Cl, Br, I, OH, =O, CN or $C_{1-4}$ alkyl;
in some embodiments, ring V is selected from piperidine or piperazine, wherein the piperidine or piperazine is optionally further substituted with 0 to 2 F;
in some embodiments, $R^{b1}$ and $R^{b2}$ are each independently selected from H, OH or $C_{1-4}$ alkyl;
in some embodiments, $R^{b1}$ and $R^{b2}$ are each independently selected from methyl or ethyl;
in some embodiments, $R^{b1}$ and $R^{b2}$ are selected from methyl;
in some embodiments, each $R^{b3}$ is independently selected from H, F, Cl, Br, I, OH, CN, $C_{1-4}$ alkyl, $C_{2-4}$ alkynyl $C_{1-4}$ alkoxy, $C_{3-6}$ cycloalkyl, 3- to 6-membered heterocyclyl, phenyl or 5- to 6-membered heteroaryl, wherein the alkyl, alkynyl, alkoxy, cycloalkyl, phenyl, heterocyclyl or heteroaryl is optionally further substituted with 0 to 4 (such as 0, 1, 2, 3 or 4) substituents selected from H, F, Cl, Br, I, OH, =O, $NH_2$, CN, COOH, $C_{1-4}$ alkyl, $C_{1-4}$ alkoxy, halogen-substituted $C_{1-4}$ alkyl, cyano-substituted $C_{1-4}$ alkyl or $C_{3-6}$ cycloalkyl, and the heterocyclyl or heteroaryl contains 1 to 3 (such as 1, 2 or 3) heteroatoms selected from O, S or N;
in some embodiments, each $R^{b3}$ is independently selected from H, F, Cl, Br, I, OH, CN, methyl, ethyl, isopropyl,

propyl, ethynyl, methoxy, ethoxy, propoxy, isopropyloxy, cyclopropyl, cyclobutyl, cyclopentyl, cyclohexyl, phenyl or 5- to 6-membered heteroaryl, wherein the methyl, ethyl, isopropyl, propyl, ethynyl, methoxy, ethoxy, propoxy, isopropyloxy, cyclopropyl, cyclobutyl, cyclopentyl, cyclohexyl, phenyl or 5- to 6-membered heteroaryl is optionally further substituted with substituted with 0 to 4 (such as 0, 1, 2, 3 or 4) substituents selected from H, F, Cl, Br, I, OH, =O, $NH_2$, CN, $C_{1-4}$ alkyl, $C_{1-4}$ alkoxy, halogen-substituted $C_{1-4}$ alkyl, cyano-substituted $C_{1-4}$ alkyl or $C_{3-6}$ cycloalkyl, and the heteroaryl contains 1 to 3 (such as 1, 2 or 3) heteroatoms selected from O, S or N;
in some embodiments, each $R^{b3}$ is independently selected from H, F, Cl, Br, I, OH, CN, methyl, ethyl, isopropyl, propyl, ethynyl, methoxy, cyclopropyl, cyclobutyl, cyclopentyl, cyclohexyl, phenyl or 5- to 6-membered heteroaryl, wherein the phenyl or 5- to 6-membered heteroaryl (such as pyrazole or pyrrole) is optionally further substituted with 0 to 4 (such as 0, 1, 2, 3 or 4) substituents selected from H, F, Cl, Br, methyl, ethyl, methoxy or ethoxy, and the heteroaryl contains 1 to 3 (such as 1, 2 or 3) heteroatoms selected from O, S or N;
in some embodiments, each $R^{b4}$ is independently selected from H, F, Cl, Br, I, OH, CN, $C_{1-4}$ alkyl or $C_{1-4}$ alkoxy, wherein the alkyl or alkoxy is optionally further substituted with 0 to 4 (such as 0, 1, 2, 3 or 4) substituents selected from H, F, Cl, Br, I, OH, CN, $C_{1-4}$ alkyl, $C_{1-4}$ alkoxy or $C_{3-6}$ cycloalkyl;
in some embodiments, each $R^{b4}$ is independently selected from H, F, Cl, Br, I, OH, CN, methyl, ethyl, isopropyl, propyl, methoxy, ethoxy, propoxy or isopropoxy, wherein the methyl, ethyl, isopropyl, propyl, methoxy, ethoxy, propoxy or isopropoxy is optionally further substituted with 0 to 4 (such as 0, 1, 2, 3 or 4) substituents selected from H, F, Cl, Br, I, OH, CN, $C_{1-4}$ alkyl, $C_{1-4}$ alkoxy or $C_{3-6}$ cycloalkyl;
in some embodiments, $R^{b4}$ is selected from H, F, Cl or Br;
in some embodiments, $R^{b5}$ is selected from H or $C_{1-4}$ alkyl, wherein the alkyl is optionally further substituted with 0 to 4 (such as 0, 1, 2, 3 or 4) substituents selected from H, F, Cl, Br, I, $C_{1-4}$ alkyl, $C_{1-4}$ alkoxy or $C_{3-6}$ cycloalkyl;
in some embodiments, $R^{b5}$ is selected from H;
in some embodiments, each $R^{b6}$ is independently selected from H, F, Cl, Br, I, $C_{1-4}$ alkyl, $C_{2-4}$ alkynyl or $C_{1-4}$ alkoxy, wherein the alkyl, alkynyl or alkoxy is optionally further substituted with 0 to 4 (such as 0, 1, 2, 3 or 4) substituents selected from H, F, Cl, Br, I, OH, CN, $C_{1-4}$ alkyl, $C_{1-4}$ alkoxy or $C_{3-6}$ cycloalkyl;
in some embodiments, each $R^{b6}$ is independently selected from H, F, Cl, Br, I, methyl, ethyl, isopropyl, propyl, ethynyl, methoxy, ethoxy, propoxy or isopropoxy, wherein the methyl, ethyl, isopropyl, propyl, ethynyl, methoxy, ethoxy, propoxy or isopropoxy is optionally further substituted with 0 to 4 (such as 0, 1, 2, 3 or 4) substituents selected from H, F, Cl, Br, I, OH, CN, $C_{1-4}$ alkyl, $C_{1-4}$ alkoxy or $C_{3-6}$ cycloalkyl;
in some embodiments, each $R^{b6}$ is independently selected from H, F, Cl, Br, methyl, ethyl, methoxy or ethoxy;
in some embodiments, $R^{b7}$ is selected from 4- to 7-membered heterocycle or $C_{3-7}$ carbocycle, wherein the carbocycle or heterocycle is optionally further substituted with 0 to 4 (such as 0, 1, 2, 3 or 4) substituents selected from H, F, Cl, Br, I, OH, =O, CN, deuterated $C_{1-4}$ alkyl, $C_{1-4}$ alkyl, $C_{1-4}$ alkoxy, halogen-substituted $C_{1-4}$ alkyl, cyano-substituted $C_{1-4}$ alkyl or $C_{3-6}$ cycloalkyl, and the heterocyclyl contains 1 to 3 (such as 1, 2 or 3) heteroatoms selected from O, S or N;
in some embodiments, $R^{b7}$ is selected from cyclopropyl, cyclobutyl, cyclopentyl, cyclohexyl, pyrrole, pyrazole, pyridine or phenyl, wherein the $R^{b7}$ is optionally further substituted with 0 to 4 (such as 0, 1, 2, 3 or 4) substituents selected from H, F, Cl, Br, I, OH, =O, CN, deuterated $C_{1-4}$ alkyl, $C_{1-4}$ alkyl, $C_{1-4}$ alkoxy, halogen-substituted $C_{1-4}$ alkyl, cyano-substituted $C_{1-4}$ alkyl or $C_{3-6}$ cycloalkyl;
in some embodiments, $R^{b7}$ is selected from cyclopropyl, cyclobutyl, cyclopentyl, cyclohexyl, pyrrole, pyrazole, pyridine or phenyl, wherein the $R^{b7}$ is optionally further substituted with 0 to 4 (such as 0, 1, 2, 3 or 4) substituents selected from H, F, Cl, Br, I, OH, =O, CN, $CH_2D$, $CHD_2$, $CD_3$, methyl or ethyl;
in some embodiments, each q is independently selected from 0, 1, 2, 3, 4, 5 or 6;
in some embodiments, each q is independently selected from 0, 1, 2, 3 or 4;
in some embodiments, each q is independently selected from 0, 1 or 2;
in some embodiments, n1, n2 and n3 are each independently selected from 0, 1, 2 or 3;
in some embodiments, each p1 or p2 is independently selected from 0, 1, 2, 3, 4 or 5;
in some embodiments, each p1 or p2 is independently selected from 0, 1 or 2;
optionally, the compound shown in general formula (I) is not

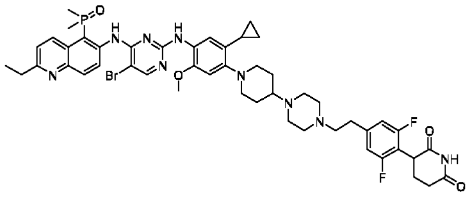 or 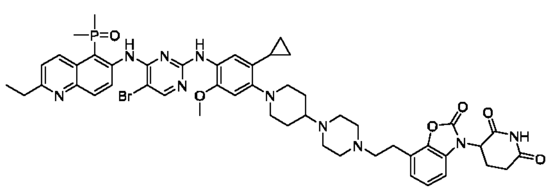.

As a first embodiment of the present invention, provided is the above-mentioned compound shown in general

formula (I) or the stereoisomer, deuterated compound, solvate, prodrug, metabolite, pharmaceutically acceptable salt or co-crystal thereof,

B-L-K (I);

L is selected from a bond or $-C_{1-50}$ hydrocarbyl-, wherein the hydrocarbyl has 0 to 20 methylene units optionally further replaced by -Ak- or -Cy-;

each -Ak- is independently selected from $-(CH_2)_q-$, $-(CH_2)_q-O-$, $-O-(CH_2)_q-$, - $(CH_2)_q-NRL-$, $-NR^L-(CH_2)_q-$, $-(CH_2)_q-NR^LC(=O)-$, $-NR^L(CH_2)_qC(=O)-$, $-(CH_2)_q-C(=O)NR^L-$, -C(=O)-, $-C(=O)-(CH_2)_q-NR^L-$, $-(C\equiv C)_q-$, -CH=CH-, $-Si(R^L)_2-$, - $Si(OH)(R^L)-$, $-Si(OH)_2-$, $-P(=O)(OR^L)-$, $-P(=O)(R^L)-$, -S-, -S(=O)-, $-S(=O)_2-$ or a bond, wherein the $-CH_2-$ is optionally further substituted with 0 to 2 (such as 0, 1 or 2) substituents selected from H, halogen, OH, CN, $NH_2$, $C_{1-6}$ alkyl, $C_{1-6}$ alkoxy, halogen-substituted $C_{1-6}$ alkyl, hydroxyl-substituted $C_{1-6}$ alkyl or cyano-substituted $C_{1-6}$ alkyl;

each q is independently selected from 0, 1, 2, 3, 4, 5 or 6;

each $R^L$ is independently selected from H, $C_{1-6}$ alkyl, 3- to 7-membered heterocyclyl, 3- to 7-membered cycloalkyl, phenyl or 5- to 6-membered heteroaryl;

each -Cy- is independently selected from a bond, a 4- to 8-membered mono-heterocyclic ring, a 4- to 10-membered fused-heterocyclic ring, a 5- to 12-membered spiro-heterocyclic ring, a 7- to 10-membered bridged-heterocyclic ring, 3- to 7-membered monocycloalkyl, 4- to 10-membered fused cycloalkyl, 5- to 12-membered spiro cycloalkyl, 7- to 10-membered bridged cycloalkyl, 5- to 10-membered heteroaryl or 6- to 10-membered aryl, wherein the aryl, heteroaryl, cycloalkyl, mono-heterocyclic ring, fused-heterocyclic ring, spiro-heterocyclic ring or bridged-heterocyclic ring is optionally further substituted with 0 to 4 (such as 0, 1, 2, 3 or 4) substituents selected from H, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the heteroaryl, mono-heterocyclic ring, fused-heterocyclic ring, spiro-heterocyclic ring or bridged-heterocyclic ring contains 1 to 4 (such as 1, 2, 3 or 4) heteroatoms selected from O, S or N, and is optionally further substituted with 0, 1 or 2 =O when the heteroatom is selected from S;

B is selected from

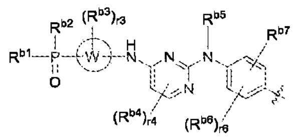 or 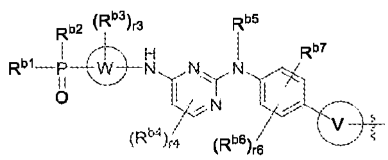 ;

r3 is selected from 0, 1, 2, 3 or 4;

r4 is selected from 0, 1 or 2;

r6 is selected from 0, 1, 2 or 3;

ring W is selected from $C_{6-10}$ carbocycle, 5- to 10-membered heterocycle, a $C_{6-10}$ aromatic ring or a 5- to 10-membered heteroaromatic ring, wherein the heterocycle or heteroaromatic ring contains 1 to 5 (such as 1, 2, 3, 4 or 5) heteroatoms selected from O, S or N;

ring V is selected from 4- to 10-membered heterocycle or $C_{3-10}$ carbocycle, wherein the heterocycle or carbocycle is optionally further substituted with 0 to 4 (such as 0, 1, 2, 3 or 4) substituents selected from H, F, Cl, Br, I, OH, =O, CN, $C_{1-4}$ alkyl, halogen-substituted $C_{1-4}$ alkyl, cyano-substituted $C_{1-4}$ alkyl or $C_{3-6}$ cycloalkyl;

$R^{b1}$ and $R^{b2}$ are each independently selected from H, OH or $C_{1-4}$ alkyl;

each $R^{b3}$ is independently selected from H, F, Cl, Br, I, OH, CN, $C_{1-4}$ alkyl, $C_{2-4}$ alkynyl $C_{1-4}$ alkoxy, $C_{3-6}$ cycloalkyl, 3- to 6-membered heterocyclyl, phenyl or 5- to 6-membered heteroaryl, wherein the alkyl, alkynyl, alkoxy, cycloalkyl, phenyl, heterocyclyl or heteroaryl is optionally further substituted with 0 to 4 (such as 0, 1, 2, 3 or 4) substituents selected from H, F, Cl, Br, I, OH, =O, $NH_2$, CN, COOH, $C_{1-4}$ alkyl, $C_{1-4}$ alkoxy, halogen-substituted $C_{1-4}$ alkyl, cyano-substituted $C_{1-4}$ alkyl or $C_{3-6}$ cycloalkyl, and the heterocyclyl or heteroaryl contains 1 to 3 (such as 1, 2 or 3) heteroatoms selected from O, S or N;

each $R^{b4}$ is independently selected from H, F, Cl, Br, I, OH, CN, $C_{1-4}$ alkyl or $C_{1-4}$ alkoxy, wherein the alkyl or alkoxy is optionally further substituted with 0 to 4 (such as 0, 1, 2, 3 or 4) substituents selected from H, F, Cl, Br, I, OH, CN, $C_{1-4}$ alkyl, $C_{1-4}$ alkoxy or $C_{3-6}$ cycloalkyl;

$R^{b5}$ is selected from H or $C_{1-4}$ alkyl, wherein the alkyl is optionally further substituted with 0 to 4 (such as 0, 1, 2, 3 or 4) substituents selected from H, F, Cl, Br, I, $C_{1-4}$ alkyl, $C_{1-4}$ alkoxy or $C_{3-6}$ cycloalkyl;

each $R^{b6}$ is independently selected from H, F, Cl, Br, I, $C_{1-4}$ alkyl, $C_{2-4}$ alkynyl or $C_{1-4}$ alkoxy, wherein the alkyl, alkynyl or alkoxy is optionally further substituted with 0 to 4 (such as 0, 1, 2, 3 or 4) substituents selected from H,

F, Cl, Br, I, OH, CN, $C_{1-4}$ alkyl, $C_{1-4}$ alkoxy or $C_{3-6}$ cycloalkyl;
$R^{b7}$ is selected from 4- to 7-membered heterocycle or $C_{3-7}$ carbocycle, wherein the carbocycle or heterocycle is optionally further substituted with 0 to 4 (such as 0, 1, 2, 3 or 4) substituents selected from H, F, Cl, Br, I, OH, =O, CN, deuterated $C_{1-4}$ alkyl, $C_{1-4}$ alkyl, $C_{1-4}$ alkoxy, halogen-substituted $C_{1-4}$ alkyl, cyano-substituted $C_{1-4}$ alkyl or $C_{3-6}$ cycloalkyl, and the heterocyclyl contains 1 to 3 (such as 1, 2 or 3) heteroatoms selected from O, S or N;
K is selected from

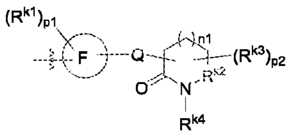, 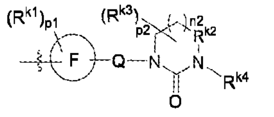,

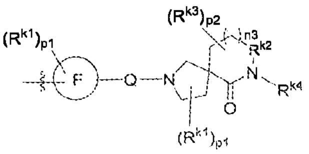, 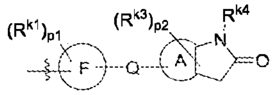, 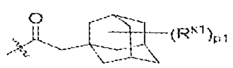, 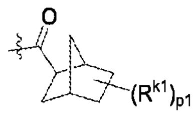,

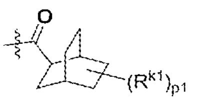, 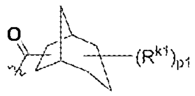, 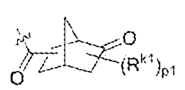, 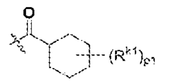, 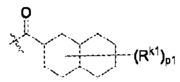,

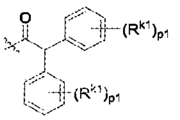, 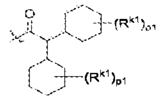, 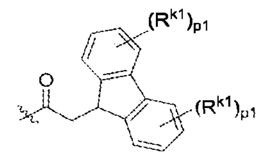, 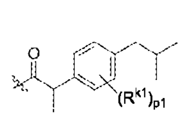,

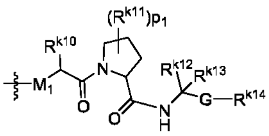, 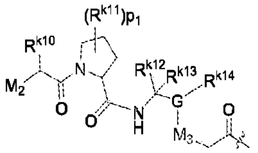 or 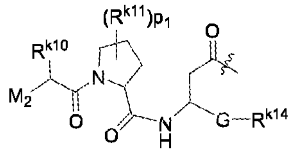;

each Q is independently selected from a bond, -O-, -S-, $-CH_2-$, $-NR^q-$, -CO-, - $NR^qCO-$, $-CONR^q-$ or 3- to 12-membered heterocyclyl, wherein the heterocyclyl is optionally further substituted with 0 to 4 (such as 0, 1, 2, 3 or 4) substituents selected from H, F, Cl, Br, I, OH, =O, $NH_2$, CN, COOH, $CONH_2$, $C_{1-4}$ alkyl or $C_{1-4}$ alkoxy, and the heterocyclyl contains 1 to 4 (such as 1, 2, 3 or 4) heteroatoms selected from O, S or N;
$R^q$ is selected from H or $C_{1-6}$ alkyl;
A is selected from $C_{3-10}$ carbocycle, a $C_{6-10}$ aromatic ring, 3- to 10-membered heterocycle or a 5- to 10-membered heteroaromatic ring, wherein the heterocycle or heteroaromatic ring contains 1 to 4 (such as 1, 2, 3 or 4) heteroatoms selected from O, S or N;
each F is independently selected from $C_{3-20}$ carbocycle, $C_{6-20}$ aromatic ring, 3-to 20-membered heterocycle or a 5- to 20-membered heteroaromatic ring, wherein the heterocycle or heteroaromatic ring contains 1 to 4 (such as 1, 2, 3 or 4) heteroatoms selected from O, S or N;
each $R^{k2}$ is independently selected from a bond, -CO-, $-SO_2-$, -SO- or - $C(R^{k3})_2-$;
each $R^{k1}$ is independently selected from H, F, Cl, Br, I, OH, =O, $NH_2$, CN, COOH, $CONH_2$, $C_{1-6}$ alkyl, $C_{1-6}$ alkoxy or $C_{3-6}$ cycloalkyl, wherein the alkyl, alkoxy or cycloalkyl is optionally further substituted with 0 to 4 (such as 0, 1, 2, 3 or 4) substituents selected from H, F, Cl, Br, I, OH, =O, $NH_2$, CN, COOH, $CONH_2$, $C_{1-4}$ alkyl, $C_{1-4}$ alkoxy or $C_{3-6}$ cycloalkyl;
each $R^{k3}$ is independently selected from H, F, Cl, Br, I, OH, =O, $NH_2$, CN, COOH, $CONH_2$, $C_{1-6}$ alkyl, $C_{1-6}$ alkoxy, $C_{3-8}$ cycloalkyl or 3- to 8-membered heterocyclyl, wherein the alkyl, alkoxy, cycloalkyl or heterocyclyl is optionally further substituted with 0 to 4 (such as 0, 1, 2, 3 or 4) substituents selected from H, F, Cl, Br, I, OH, =O, $NH_2$, CN,

COOH, $CONH_2$, $C_{1-4}$ alkyl or $C_{1-4}$ alkoxy, and the heterocyclyl contains 1 to 4 (such as 1, 2, 3 or 4) heteroatoms selected from O, S or N;

or two $R^{k3}$ together with the carbon atoms or ring backbones to which they are directly attached form 3- to 8-membered carbocycle or 3- to 8-membered heterocycle, and two $R^{k1}$ together with the carbon atoms or ring backbones to which they are directly attached form 3- to 8-membered carbocycle or 3- to 8-membered heterocycle, wherein the carbocycle or heterocycle is optionally further substituted with 0 to 4 (such as 0, 1, 2, 3 or 4) substituents selected from H, F, Cl, Br, I, OH, =O, $NH_2$, CN, COOH, $CONH_2$, $C_{1-4}$ alkyl or $C_{1-4}$ alkoxy, and the heterocycle contains 1 to 4 (such as 1, 2, 3 or 4) heteroatoms selected from O, S or N;

each $R^{k4}$ is independently selected from H, OH, $NH_2$, CN, $CONH_2$, $C_{1-6}$ alkyl, $C_{3-8}$ cycloalkyl or 3- to 8-membered heterocyclyl, wherein the alkyl, cycloalkyl or heterocyclyl is optionally further substituted with 0 to 4 (such as 0, 1, 2, 3 or 4) substituents selected from H, F, Cl, Br, I, OH, =O, $NH_2$, CN, COOH, $CONH_2$, $C_{1-4}$ alkyl or $C_{1-4}$ alkoxy, and the heterocyclyl contains 1 to 4 (such as 1, 2, 3 or 4) heteroatoms selected from O, S or N;

$M_1$ is selected from a bond, $-CH_2-C(=O)NH-$ or $-C(=O)CH_2NH-$;

$M_2$ is selected from -NHC(=O)-$C_{1-6}$ alkyl, -NHC(=O)-$C_{3-6}$ cycloalkyl or 4- to 10-membered heterocyclyl, wherein the alkyl, cycloalkyl or heterocyclyl is optionally further substituted with 0 to 4 (such as 0, 1, 2, 3 or 4) substituents selected from H, F, Cl, Br, I, =O, OH, $NH_2$, $C_{1-4}$ alkyl or $C_{1-4}$ alkoxy, and the heterocyclyl contains 1 to 4 (such as 1, 2, 3 or 4) heteroatoms selected from O, S or N;

$M_3$ is selected from -NH- or -O-;

$R^{k10}$ is selected from $C_{1-6}$ alkyl, wherein the alkyl is optionally further substituted with 0 to 4 (such as 0, 1, 2, 3 or 4) substituents selected from H, F, Cl, Br, I, =O, OH, $C_{1-6}$ alkyl or $C_{3-6}$ cycloalkyl;

each $R^{k11}$ is independently selected from H, F, Cl, Br, I, =O, OH, SH, $C_{1-6}$ alkyl, $C_{1-6}$ alkoxy, $C_{1-6}$ alkylthio or -O-C(=O)-$C_{1-6}$ alkyl, wherein the alkyl, alkoxy or alkylthio is optionally further substituted with 0 to 4 (such as 0, 1, 2, 3 or 4) substituents selected from H, F, Cl, Br, I, OH, $C_{1-4}$ alkyl or $C_{1-4}$ alkoxy;

$R^{k12}$ and $R^{k13}$ are each independently selected from H, $C_{1-6}$ alkyl or $C_{3-6}$ cycloalkyl, wherein the alkyl or cycloalkyl is optionally further substituted with 0 to 4 (such as 0, 1, 2, 3 or 4) substituents selected from H, F, Cl, Br, I, =O, OH, $NH_2$, $C_{1-4}$ alkyl or $C_{1-4}$ alkoxy;

$R^{k14}$ is selected from 5- to 6-membered heteroaryl, wherein the heteroaryl is optionally further substituted with 0 to 4 (such as 0, 1, 2, 3 or 4) substituents selected from H, F, Cl, Br, I, OH, =O, $CF_3$, CN, $C_{1-4}$ alkyl, halogen-substituted $C_{1-4}$ alkyl, hydroxyl-substituted $C_{1-4}$ alkyl, $C_{1-4}$ alkoxy or $C_{3-6}$ cycloalkyl, and the heteroaryl contains 1 to 4 (such as 1, 2, 3 or 4) heteroatoms selected from N, O or S;

G is selected from a 6- to 10-membered aromatic ring or a 5- to 10-membered heteroaromatic ring, wherein the aromatic ring or heteroaromatic ring is optionally further substituted with 0 to 4 (such as 0, 1, 2, 3 or 4) substituents selected from H, F, Cl, Br, I, OH, =O, $CF_3$, CN, $C_{1-4}$ alkyl, halogen-substituted $C_{1-4}$ alkyl, hydroxyl-substituted $C_{1-4}$ alkyl, $C_{1-4}$ alkoxy or $C_{3-6}$ cycloalkyl, wherein the heteroaromatic ring contains 1 to 4 (such as 1, 2, 3 or 4) heteroatoms selected from N, O or S;

n1, n2 and n3 are each independently selected from 0, 1, 2 or 3;

each p1 or p2 is independently selected from 0, 1, 2, 3, 4 or 5.

As a second embodiment of the present invention, provided is the above-mentioned compound shown in general formula (I) or the stereoisomer, deuterated compound, solvate, prodrug, metabolite, pharmaceutically acceptable salt or co-crystal thereof,

L is selected from -Cy1-Ak1-Cy2-Ak2-Cy3-Ak3-Cy4-Ak4-Cy5-Ak5-, -Cyl-Cy2-Cy3-Cy4-Ak1-Ak2-Ak3-Ak4-Ak5-, -Cy1-Ak1-Cy2-Ak2-Cy3 -Ak3 -Cy4-Ak4-Ak5-, -Ak1-Cy1-Ak2-Cy2-Ak3-Cy3-Ak4-Cy4-Ak5-, -Cy1-Ak1-Cy2-Ak2-Cy3-Cy4-Ak3-Ak4-Ak5-, -Cy1-Ak1-Cy2-Ak2-Ak3-Cy3-Cy4-Ak4-Ak5-, -Cy1-Ak1-Ak2-Ak3-Ak4-Ak5-Cy2-Cy3-Cy4-, -Cy1-Cy2-Ak1-Ak2-Ak3-Ak4-Ak5-Cy3-Cy4-, -Cy 1 -Cy2-Cy3 - Ak 1 - Ak2-Ak3 - Ak4-Ak5 -Cy4-, -Cy1-Cy2-Cy3-Cy4-Ak1-Ak2-Ak3-Ak4-Ak5-, -Cy1-Ak1-Cy2-Cy3-Cy4-Ak2-Ak3-Ak4-Ak5-, -Cy1-Cy2-Ak1-Cy3-Cy4-Ak2-Ak3-Ak4-Ak5-, -Cy1-Cy2-Cy3-Ak1-Cy4-Ak2-Ak3-Ak4-Ak5-, - Cy1-Ak1-Ak2-Cy2-Cy3 -Cy4-Ak3 -Ak4-Ak5-, -Cy 1 -Cy2-Ak1-Ak2-Cy3-Cy4-Ak3 - Ak4-Ak5-, -Cy1-Cy2-Cy3-Ak1-Ak2-Cy4-Ak3-Ak4-Ak5-, -Cy1-Ak1-Ak2-Ak3-Cy2-Cy3-Cy4-Ak4-Ak5-, -Cy1-Cy2-Ak1-Ak2-Ak3-Cy3-Cy4-Ak4-Ak5-, -Cy1-Cy2-Cy3-Ak1-Ak2-Ak3-Cy4-Ak4-Ak5-, -Cy1-Ak1-Ak2-Ak3-Ak4-Cy2-Cy3-Cy4-Ak5-, -Cy1-Cy2-Ak1-Ak2-Ak3-Ak4-Cy3-Cy4-Ak5-, -Cy1-Cy2-Cy3-Ak1-Ak2-Ak3-Ak4-Cy4-Ak5-, - Ak 1 -Ak2-Ak3 - Ak4-Ak5 -Cy 1 -Cy2-Cy3 -Cy4-, - Ak 1 -Cy 1 - Cy2-Cy3-Cy4-Ak2-Ak3-Ak4-Ak5-, -Ak 1 -Ak2-Cy 1 -Cy2-Cy3 -Cy4-Ak3 -Ak4-Ak5 -, -Ak1-Ak2-Ak3-Cy1-Cy2-Cy3-Cy4-Ak4-Ak5 - Ak 1 - Ak2-Ak3 - Ak4-Cy 1 -Cy2-Cy3-Cy4-Ak5-, -Ak1-Cy1-Ak2-Ak3-Ak4-Ak5-Cy2-Cy3-Cy4-, -Ak1-Cy1-Cy2-Ak2-Ak3-Ak4-Ak5-Cy3-Cy4-, -Ak1-Cy1-Cy2-Cy3-Ak2-Ak3-Ak4-Ak5-Cy4-, - Ak1-Ak2-Cy1-Ak3-Ak4-Ak5-Cy2-Cy3-Cy4-, -Ak1-Ak2-Cy1-Cy2-Ak3-Ak4-Ak5-Cy3-Cy4-, - Ak 1 - Ak2-Cy 1 -Cy2-Cy3 - Ak3 - Ak4-Ak5 -Cy4-, - Ak 1 - Ak2-Ak3 -Cy 1 - Ak4-Ak5-Cy2-Cy3-Cy4-, -Ak1-Ak2-Ak3-Cy1-Cy2-Ak4-Ak5-Cy3-Cy4-, -Ak1-Ak2-Ak3-Cy1-Cy2-Cy3-Ak4-Ak5-Cy4-, -Ak1-Ak2-Ak3-Ak4-Cy1-Ak5-Cy2-Cy3-Cy4-, -Ak1-Ak2-Ak3-Ak4-Cy1-Cy2-Ak5-Cy3-Cy4-, or -Ak1-Ak2-Ak3-Ak4-Cy1-Cy2-Cy3-Ak5-Cy4-;

Ak1, Ak2, Ak3, Ak4 and Ak5 are each independently selected from $-(CH_2)_q-$, $-(CH_2)_q-O-$, $-O-(CH_2)_q-$, $-(CH_2)_q-NR^L-$, $-NR^L-(CH_2)_q-$, $-(CH_2)_q-NR^LC(=O)-$, - $(CH_2)_q-C(=O)NR^L-$, $-C(=O)-$, $-C(=O)-(CH_2)_q-NR^L-$, $-(C\equiv C)_q-$ or a bond, wherein the $-CH_2-$ is optionally further substituted with 0 to 2 (such as 0, 1 or 2) substituents selected from H, halogen, OH, CN, $NH_2$, $C_{1-4}$ alkyl, $C_{1-4}$ alkoxy, halogen-substituted $C_{1-4}$ alkyl, hydroxyl-substituted $C_{1-4}$ alkyl or cyano-substituted $C_{1-4}$ alkyl;

each Cy1, Cy2, Cy3, Cy4 or Cy5 is independently selected from a bond, a 4-to 7-membered mono-heterocyclic ring, a 4- to 10-membered fused-heterocyclic ring, a 5- to 12-membered spiro-heterocyclic ring, a 7- to 10-membered bridged-heterocyclic ring, 3- to 7-membered monocycloalkyl, 4- to 10-membered fused cycloalkyl, 5- to 12-membered spiro cycloalkyl, 7- to 10-membered bridged cycloalkyl, 5- to 10-membered heteroaryl or 6- to 10-membered aryl, wherein the aryl, heteroaryl, cycloalkyl, mono-heterocyclic ring, fused-heterocyclic ring, spiro-heterocyclic ring or bridged-heterocyclic ring is optionally further substituted with 0 to 4 (such as 0, 1, 2, 3 or 4) substituents selected from H, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the heteroaryl, mono-heterocyclic ring, fused-heterocyclic ring, spiro-heterocyclic ring or bridged-heterocyclic ring contains 1 to 4 (such as 1, 2, 3 or 4) heteroatoms selected from O, S or N, and is optionally further substituted with 0, 1 or 2 =O when the heteroatom is selected from S;

each q is independently selected from 0, 1, 2, 3 or 4;

each $R^L$ is independently selected from H or $C_{1-6}$ alkyl;

the definitions of other groups are the same as those in the first embodiment of the present invention.

As a third embodiment of the present invention, provided is the above-mentioned compound shown in general formula (I) or the stereoisomer, deuterated compound, solvate, prodrug, metabolite, pharmaceutically acceptable salt or co-crystal thereof,

each Cy1, Cy2, Cy3, Cy4 or Cy5 is independently selected from a bond, a 4-to 7-membered nitrogen-containing mono-heterocyclic ring, a 4- to 10-membered nitrogen-containing fused-heterocyclic ring, a 5- to 12-membered nitrogen-containing spiro-heterocyclic ring, a 7- to 10-membered nitrogen-containing bridged-heterocyclic ring, 3- to 7-membered monocycloalkyl, 4- to 10-membered fused cycloalkyl, 5- to 12-membered spiro cycloalkyl, 7- to 10-membered bridged cycloalkyl, 5- to 10-membered heteroaryl or 6- to 10-membered aryl, wherein the mono-heterocyclic ring, fused-heterocyclic ring, bridged-heterocyclic ring, spiro-heterocyclic ring, cycloalkyl, aryl or heteroaryl is optionally further substituted with 0 to 4 (such as 0, 1, 2, 3 or 4) substituents selected from H, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the mono-heterocyclic ring, fused-heterocyclic ring, bridged-heterocyclic ring, spiro-heterocyclic ring or heteroaryl contains 1 to 4 (such as 1, 2, 3 or 4) heteroatoms selected from O, S or N, and is optionally further substituted with 0, 1 or 2 =O when the heteroatom is selected from S;

each $R^L$ is independently selected from H or $C_{1-4}$ alkyl;

ring W is selected from a benzene ring, a naphthalene ring, a 5- to 6-membered monocyclic heteroaromatic ring, a 9- to 10-membered fused heteroaromatic ring or 9- to 10-membered heterocycle, wherein the heterocycle or heteroaromatic ring contains 1 to 5 (such as 1, 2, 3, 4 or 5) heteroatoms selected from O, S or N;

ring V is selected from 4- to 6-membered heterocycle or $C_{3-6}$ carbocycle, wherein the heterocycle or carbocycle is optionally further substituted with 0 to 4 (such as 0, 1, 2, 3 or 4) substituents selected from H, F, Cl, Br, I, OH, =O, CN, $C_{1-4}$ alkyl, halogen-substituted $C_{1-4}$ alkyl, cyano-substituted $C_{1-4}$ alkyl or $C_{3-6}$ cycloalkyl;

K is selected from

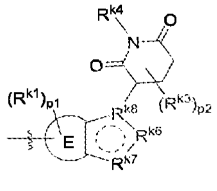 , 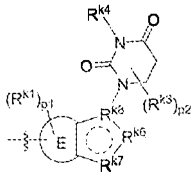 , 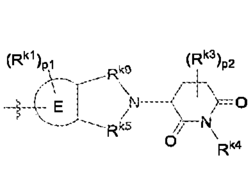 ,

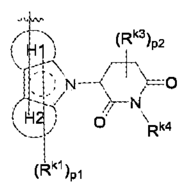 , 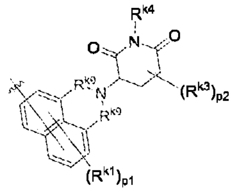 , 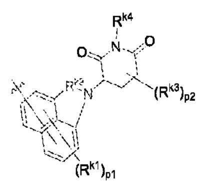 , 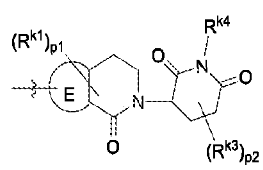 ,

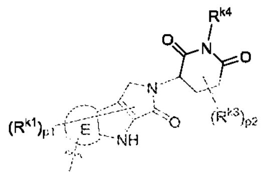, 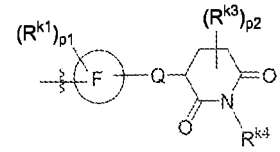, 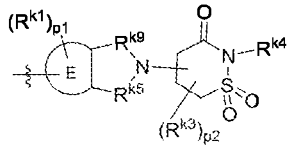,

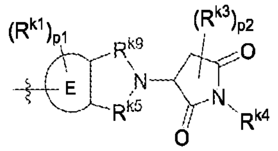, 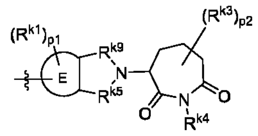, 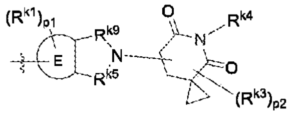,

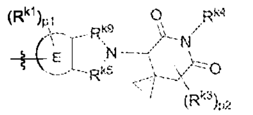, 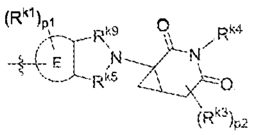, 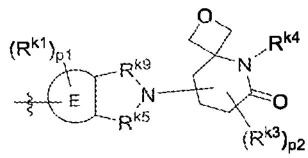,

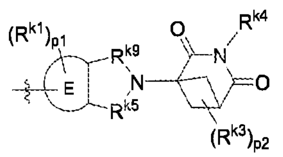, 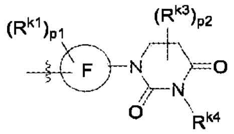, 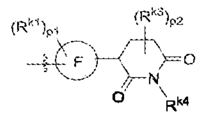, 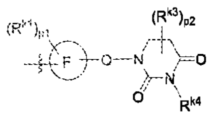,

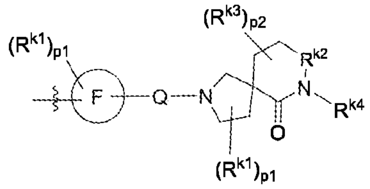, 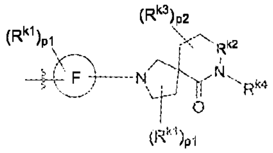, 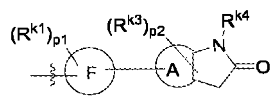,

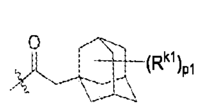, 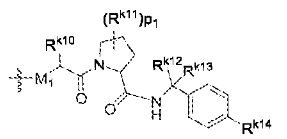, 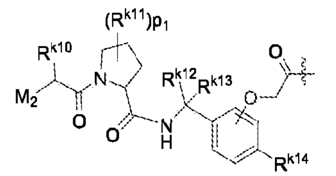

or

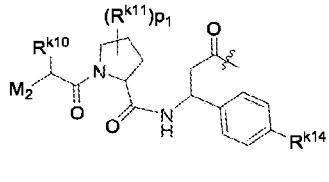;

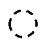

represents a ring selected from an aromatic ring or a non-aromatic ring;

each Q is independently selected from -O-, -S-, $-CH_2-$, $-NR^q-$, -CO-, $-NR^qCO-$, $-CONR^q-$ or 4- to 7-membered heterocyclyl, wherein the heterocyclyl is optionally further substituted with 0 to 4 (such as 0, 1, 2, 3 or 4) substituents

selected from H, F, Cl, Br, I, OH, =O, $NH_2$, CN, COOH, $CONH_2$, $C_{1-4}$ alkyl or $C_{1-4}$ alkoxy, and the heterocyclyl contains 1 to 4 (such as 1, 2, 3 or 4) heteroatoms selected from O, S or N;

$R^q$ is selected from H or $C_{1-4}$ alkyl;

$R^{k1}$ and $R^{k3}$ are each independently selected from H, F, Cl, Br, I, OH, =O, $NH_2$, $CF_3$, CN, COOH, $CONH_2$, $C_{1-4}$ alkyl or $C_{1-4}$ alkoxy, wherein the alkyl or alkoxy is optionally further substituted with 0 to 4 (such as 0, 1, 2, 3 or 4) substituents selected from H, F, Cl, Br, I, OH or $NH_2$;

or two $R^{k3}$ together with the carbon atoms or ring backbones to which they are directly attached form 3- to 6-membered carbocycle or 3- to 7-membered heterocycle, and two $R^{k1}$ together with the carbon atoms or ring backbones to which they are directly attached form 3- to 6-membered carbocycle or 3- to 7-membered heterocycle, wherein the carbocycle or heterocycle is optionally further substituted with 0 to 4 (such as 0, 1, 2, 3 or 4) substituents selected from H, F, Cl, Br, I, OH, =O, $NH_2$, CN, COOH, $CONH_2$, $C_{1-4}$ alkyl or $C_{1-4}$ alkoxy, and the heterocycle contains 1 to 4 (such as 1, 2, 3 or 4) heteroatoms selected from O, S or N;

each $R^{k4}$ is independently selected from H, OH, $NH_2$, $CF_3$, CN or $C_{1-4}$ alkyl;

each $R^{k5}$ is independently selected from

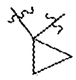,

$C(CH_3)_2$, CO, $CH_2$, $SO_2$,

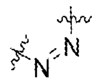, 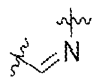 or 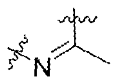;

each $R^{k6}$ is independently selected from CO, CH, SO, $SO_2$, $CH_2$ or N;

each $R^{k7}$ is independently selected from

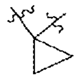,

$C(CH_3)_2$, CO, CH, N, $CH_2$, O, S, $N(CH_3)$, $N(CH_2CH_3)$, N(cyclopropyl) or NH;

each $R^{k8}$ is independently selected from C, N or CH;

each $R^{k9}$ is independently selected from a bond,

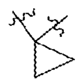,

$C(CH_3)_2$, CO, $CH_2$, $CH_2CH_2$ or $SO_2$;

each A, H1 or H2 is independently selected from $C_{3-8}$ carbocycle, a benzene ring, 4- to 7-membered heterocycle or a 5- to 6-membered heteroaromatic ring, wherein the heterocycle or heteroaromatic ring contains 1 to 4 (such as 1, 2, 3 or 4) heteroatoms selected from O, S or N;

each E is independently selected from $C_{3-8}$ carbocycle, a benzene ring, 4- to 7-membered heterocycle, 8- to 12-membered heterocycle, a 7- to 12-membered heteroaromatic ring or a 5- to 6-membered heteroaromatic ring, wherein the heterocycle or heteroaromatic ring contains 1 to 4 (such as 1, 2, 3 or 4) heteroatoms selected from O, S or N;

each F is independently selected from 3- to 7-membered monocycloalkyl, 4-to 10-membered fused cycloalkyl, 5- to 12-membered spiro cycloalkyl, 5- to 10-membered bridged cycloalkyl, a 4- to 7-membered mono-heterocyclic ring, a 4- to 10-membered fused-heterocyclic ring, a 5- to 12-membered spiro-heterocyclic ring, a 5- to 10-membered bridged-heterocyclic ring, $C_{6-14}$ aryl or 5- to 10-membered heteroaryl, wherein the mono-heterocyclic ring, fused-heterocyclic ring, spiro-heterocyclic ring, bridged-heterocyclic ring or heteroaryl contains 1 to 4 (such as 1, 2, 3 or 4) heteroatoms selected from O, S or N;

the definitions of other groups are the same as those in either the first or second embodiment of the present invention.

As a fourth embodiment of the present invention, provided is the above-mentioned compound shown in general formula (I) or the stereoisomer, deuterated compound, solvate, prodrug, metabolite, pharmaceutically acceptable salt

or co-crystal thereof,

$R^L$ is selected from H, methyl or ethyl;

each q is independently selected from 0, 1 or 2;

each Cy1, Cy2, Cy3, Cy4 or Cy5 is independently selected from a bond or one of the following substituted or unsubstituted groups: cyclopropyl, cyclobutyl, cyclopentyl, cyclohexyl, azetidinyl, azacyclopentyl, azacyclohexenyl, piperidine, morpholine, piperazine, phenyl, cyclopropyl-fused-cyclopropyl, cyclopropyl-fused-cyclobutyl, cyclopropyl-fused-cyclopentyl, cyclopropyl-fused-cyclohexyl, cyclobutyl-fused-cyclobutyl, cyclobutyl-fused-cyclopentyl, cyclobutyl-fused-cyclohexyl, cyclopentyl-fused-cyclopentyl, cyclopentyl-fused-cyclohexyl, cyclohexyl-fused-cyclohexyl, cyclopropyl-spiro-cyclopropyl, cyclopropyl-spiro-cyclobutyl, cyclopropyl-spiro-cyclopentyl, cyclopropyl-spiro-cyclohexyl, cyclobutyl-spiro-cyclobutyl, cyclobutyl-spiro-cyclopentyl, cyclobutyl-spiro-cyclohexyl, cyclopentyl-spiro-cyclopentyl, cyclopentyl-spiro-cyclohexyl, cyclohexyl-spiro-cyclohexyl, cyclopropyl-fused-azetidinyl, cyclopropyl-fused-azacyclopentyl, cyclopropyl-fused-azacyclohexyl, cyclobutyl-fused-azetidinyl, cyclobutyl-fused-azacyclopentyl, cyclobutyl-fused-azacyclohexyl, cyclopentyl-fused-azetidinyl, cyclopentyl-fused-azacyclopentyl, cyclopentyl-fused-azacyclohexyl, cyclohexyl-fused-azetidinyl, cyclohexyl-fused-azacyclopentyl, cyclohexyl-fused-azacyclohexyl, azetidinyl-fused-azetidinyl, azetidinyl-fused-azacyclopentyl, azetidinyl-fused-azacyclohexyl, azacyclopentyl-fused-azetidinyl, azacyclopentyl-fused-azacyclopentyl, azacyclopentyl-fused-azacyclohexyl, azacyclohexyl-fused-azetidinyl, azacyclohexyl-fused-azacyclopentyl, azacyclohexyl-fused-azacyclohexyl, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, cyclohexyl-spiro-azacyclohexyl, azetidinyl-spiro-azetidinyl, azetidinyl-spiro-azacyclopentyl, azetidinyl-spiro-azacyclohexyl, azacyclopentyl-spiro-azetidinyl, azacyclopentyl-spiro-azacyclopentyl, azacyclopentyl-spiro-azacyclohexyl, azacyclohexyl-spiro-azetidinyl, azacyclohexyl-spiro-azacyclopentyl, azacyclohexyl-spiro-azacyclohexyl,

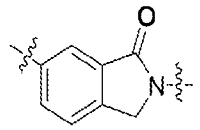, 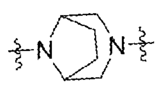, 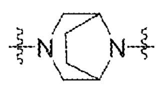, 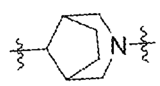,

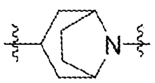, 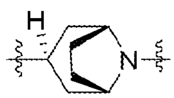, 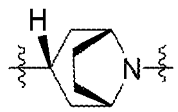, 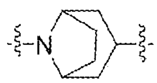, 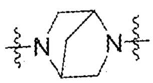, 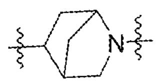,

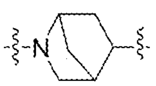, 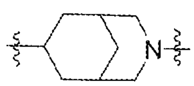, 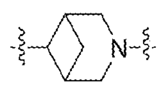, 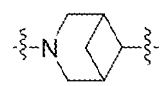, 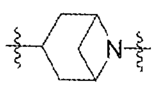, 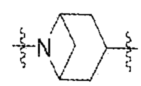,

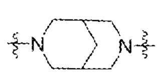, 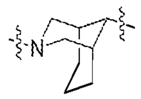, 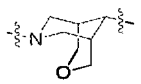, 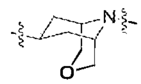, 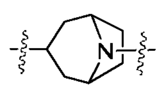, 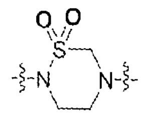

or

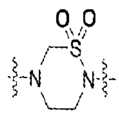,

which, when substituted, is optionally further substituted with 0 to 4 (such as 0, 1, 2, 3 or 4) substituents selected from H, F, Cl, Br, I, OH, $NH_2$, COOH, CN, =O, $C_{1-4}$ alkyl, halogen-substituted $C_{1-4}$ alkyl, hydroxyl-substituted $C_{1-4}$ alkyl or $C_{1-4}$ alkoxy;

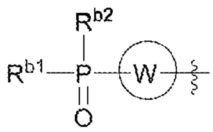

is selected from

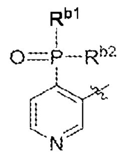 , 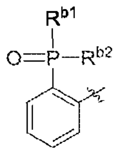 , 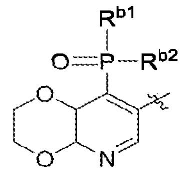 ,

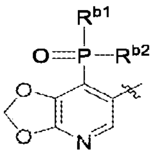 , 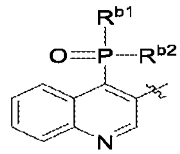 , 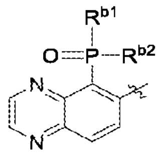 , 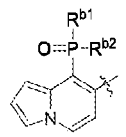 , 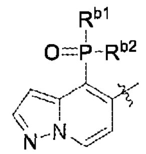 , 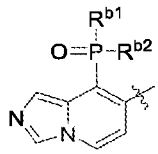 ,

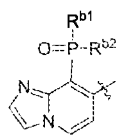 , 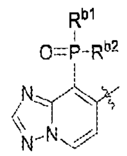 , 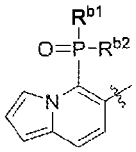 , 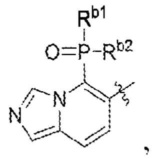 , 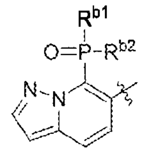 , 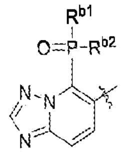 , 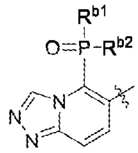

or

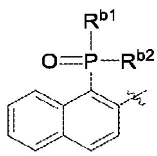 ;

ring V is selected from azetidinyl, azacyclopentyl, piperidyl or piperazinyl, wherein the azetidinyl, azacyclopentyl, piperidyl or piperazinyl is optionally further substituted with 0 to 4 (such as 0, 1, 2, 3 or 4) substituents selected from H, F, Cl, Br, I, OH, =O, CN or $C_{1-4}$ alkyl;

$R^{b1}$ and $R^{b2}$ are each independently selected from methyl or ethyl;

each $R^{b3}$ is independently selected from H, F, Cl, Br, I, OH, CN, methyl, ethyl, isopropyl, propyl, ethynyl, methoxy, ethoxy, propoxy, isopropyloxy, cyclopropyl, cyclobutyl, cyclopentyl, cyclohexyl, phenyl or 5- to 6-membered heteroaryl, wherein the methyl, ethyl, isopropyl, propyl, ethynyl, methoxy, ethoxy, propoxy, isopropyloxy, cyclopropyl, cyclobutyl, cyclopentyl, cyclohexyl, phenyl or 5- to 6-membered heteroaryl is optionally further substituted with substituted with 0 to 4 (such as 0, 1, 2, 3 or 4) substituents selected from H, F, Cl, Br, I, OH, =O, $NH_2$, CN, $C_{1-4}$ alkyl, $C_{1-4}$ alkoxy, halogen-substituted $C_{1-4}$ alkyl, cyano-substituted $C_{1-4}$ alkyl or $C_{3-6}$ cycloalkyl, and the heteroaryl contains 1 to 3 (such as 1, 2 or 3) heteroatoms selected from O, S or N;

each $R^{b4}$ is independently selected from H, F, Cl, Br, I, OH, CN, methyl, ethyl, isopropyl, propyl, methoxy, ethoxy, propoxy or isopropoxy, wherein the methyl, ethyl, isopropyl, propyl, methoxy, ethoxy, propoxy or isopropoxy is optionally further substituted with 0 to 4 (such as 0, 1, 2, 3 or 4) substituents selected from H, F, Cl, Br, I, OH, CN, $C_{1-4}$ alkyl, $C_{1-4}$ alkoxy or $C_{3-6}$ cycloalkyl;

$R^{b5}$ is selected from H;

each $R^{b6}$ is independently selected from H, F, Cl, Br, I, methyl, ethyl, isopropyl, propyl, ethynyl, methoxy, ethoxy, propoxy or isopropoxy, wherein the methyl, ethyl, isopropyl, propyl, ethynyl, methoxy, ethoxy, propoxy or isopropoxy is optionally further substituted with 0 to 4 (such as 0, 1, 2, 3 or 4) substituents selected from H, F, Cl, Br, I, OH, CN, $C_{1-4}$ alkyl, $C_{1-4}$ alkoxy or $C_{3-6}$ cycloalkyl;

$R^{b7}$ is selected from cyclopropyl, cyclobutyl, cyclopentyl, cyclohexyl, pyrrole, pyrazole, pyridine or phenyl, wherein the $R^{b7}$ is optionally further substituted with 0 to 4 (such as 0, 1, 2, 3 or 4) substituents selected from H, F, Cl, Br, I, OH, =O, CN, deuterated $C_{1-4}$ alkyl, $C_{1-4}$ alkyl, $C_{1-4}$ alkoxy, halogen-substituted $C_{1-4}$ alkyl, cyano-substituted $C_{1-4}$ alkyl or $C_{3-6}$ cycloalkyl;

K is selected from

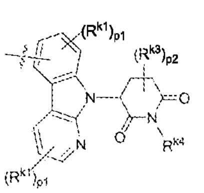 , 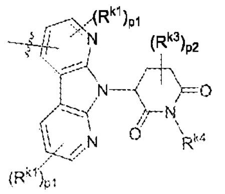 , 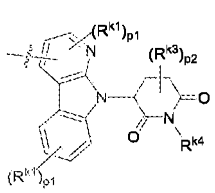 ,

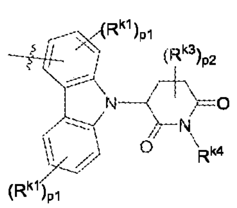 , 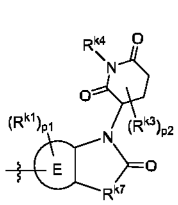 , 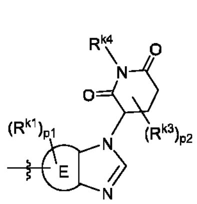 , 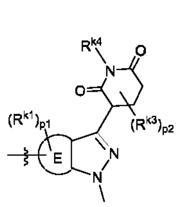 , 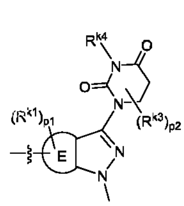 ,

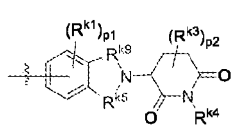 , 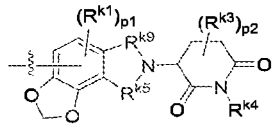 , 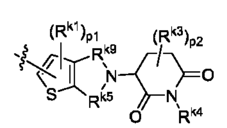 , 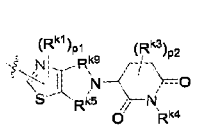 ,

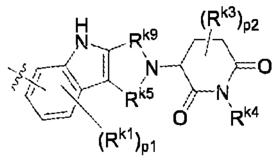 , 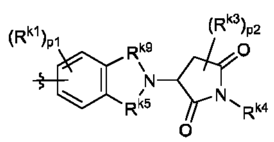 , 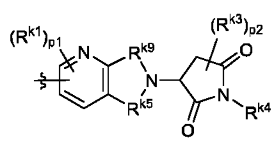 ,

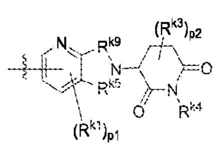 , 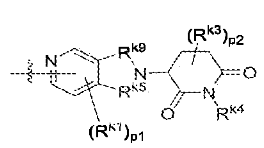 , 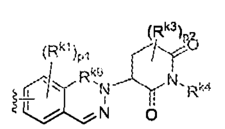 , 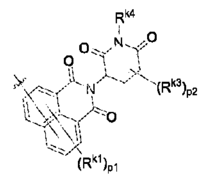 ,

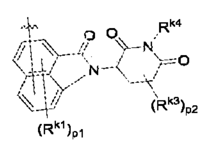 , 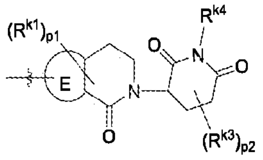 , 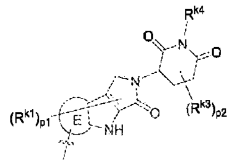 , 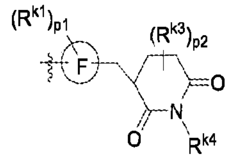 ,

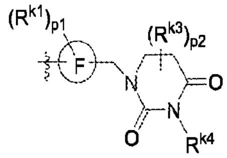 , 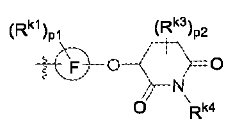 , 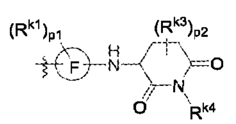 , 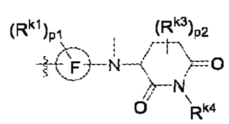 ,

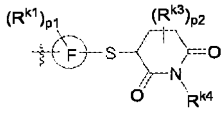, 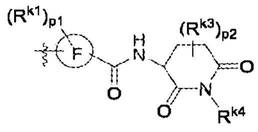, 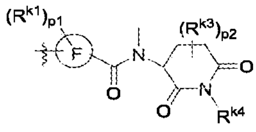, 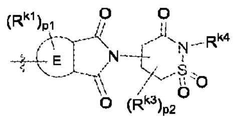,

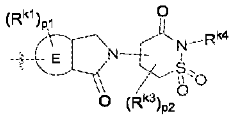, 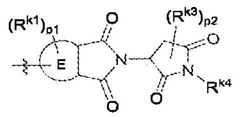, 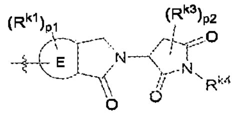, 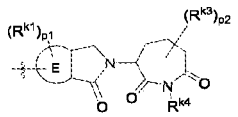,

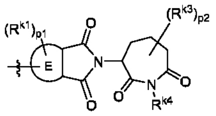, 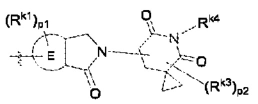, 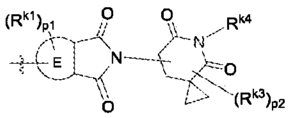, 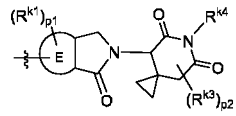,

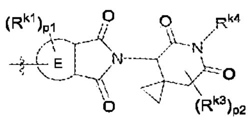, 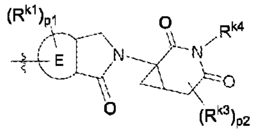, 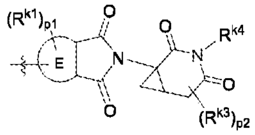, 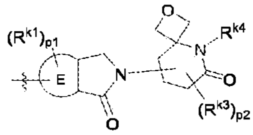,

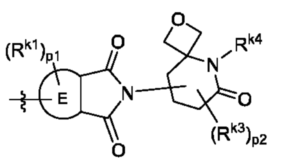, 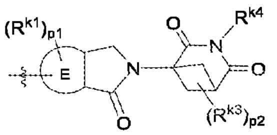, 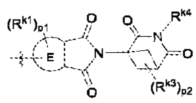, 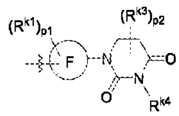,

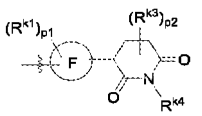, 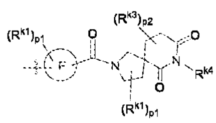, 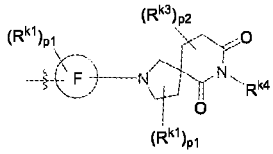, 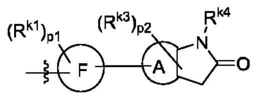,

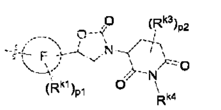, 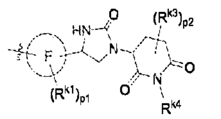, 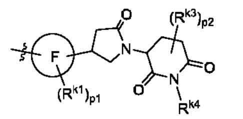, 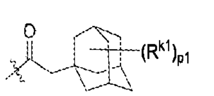,

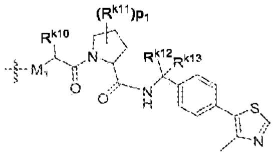, 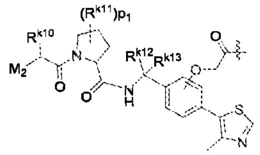, 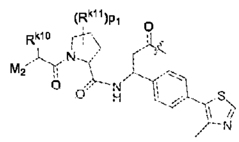, 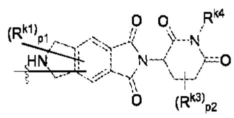,

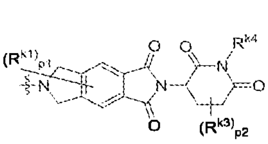, 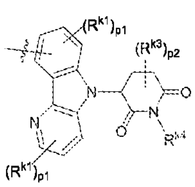 or 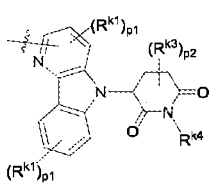;

each E is independently selected from phenyl, pyridyl, pyridazinyl, pyrazinyl, pyrimidinyl, pyrrolyl, pyrazolyl, imidazolyl, thiazolyl, furyl, thienyl or oxazolyl;

each A is independently selected from phenyl, pyridyl, pyridazinyl, pyrazinyl, pyrimidinyl, pyrrolyl, pyrazolyl, imidazolyl, thiazolyl, furyl, thienyl or oxazolyl;

each F is independently selected from cyclopropyl, cyclobutyl, cyclopentyl, cyclohexyl, bicyclo[1.1.1]pentyl, 6,7-dihydro-SH-cyclopenta[c]pyridyl, 2,3-dihydro-1H-indenyl, phenyl, naphthyl, anthryl, phenanthryl, azetidinyl, azacyclopentyl, piperidyl, morpholinyl, pyridyl, pyrimidinyl, pyridazinyl, pyrazinyl, triazinyl, pyrrolyl, pyrazolyl, imidazolyl, triazolyl, oxazolyl, furyl, thienyl, thiazolyl, 2-pyridone, benzoxazolyl, pyridoimidazolyl, benzoimidazolyl, benzopyrazolyl, benzothiazolyl, benzothienyl, benzofuryl, benzopyrrolyl, benzopyridyl, benzopyrazinyl, benzopyrimidinyl, benzopyridazinyl, benzotriazinyl, pyrrolopyrrolyl, pyrrolopyridyl, pyrrolopyrimidinyl, pyrrolopyridazinyl, pyrrolopyrazinyl, imidazopyrimidinyl, imidazopyridyl, imidazopyrazinyl, imidazopyridazinyl, pyrazolopyridyl, pyrazolopyrimidinyl, pyrazolopyridazinyl, pyrazolopyrazinyl, pyrimidopyridyl, pyrimidopyrazinyl, pyrimidopyridazinyl, pyrimidopyrimidinyl, pyridopyridyl, pyridopyrazinyl, pyridopyridazinyl, pyridazinopyridazinyl, pyridazinopyrazinyl or pyrazinopyrazinyl;

each $R^{k7}$ is independently selected from

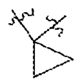,

$C(CH_3)_2$, $CH_2$, O, $N(CH_3)$, $N(CH_2CH_3)$, N(cyclopropyl) or NH;

each p1 or p2 is independently selected from 0, 1 or 2;

the definitions of other groups are the same as those in any one of the first, second and third embodiments of the present invention.

As a fifth embodiment of the present invention, provided is the above-mentioned compound shown in general formula (I) or the stereoisomer, deuterated compound, solvate, prodrug, metabolite, pharmaceutically acceptable salt or co-crystal thereof,

each Cy1, Cy2, Cy3, Cy4 or Cy5 is independently selected from a bond or one of the following substituted or unsubstituted groups:

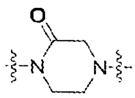, 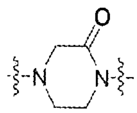,

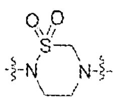, 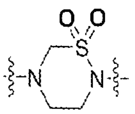, 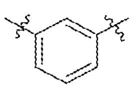, 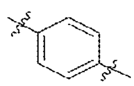, 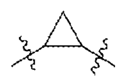, 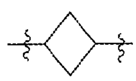, 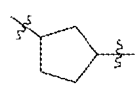,

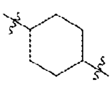, 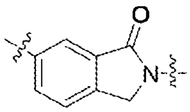, 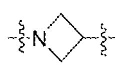, 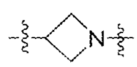, 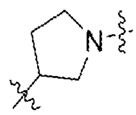, 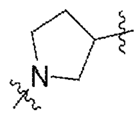, 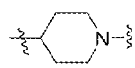,

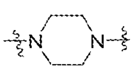, 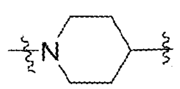, 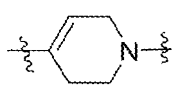, 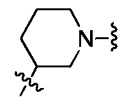, 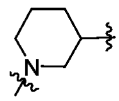, 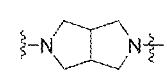,

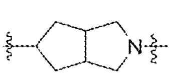, 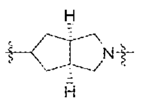, 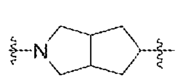, 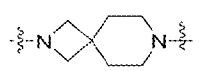, 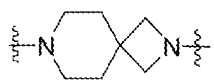,

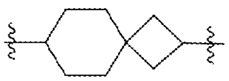, 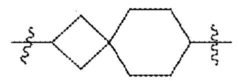, 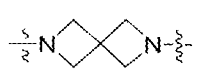, 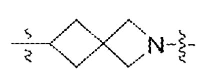,

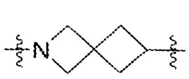 , 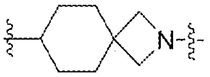 , 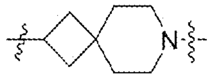 , 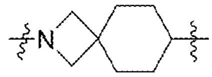 ,

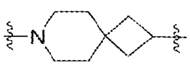 , 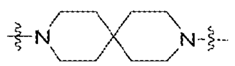 , 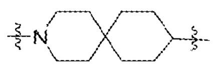 , 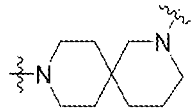 , 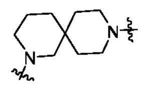 ,

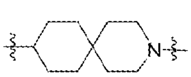 , 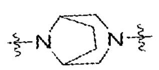 , 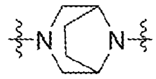 , 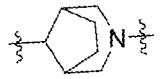 , 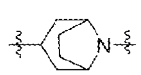 , 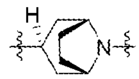 ,

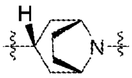 , 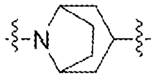 , 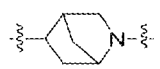 , 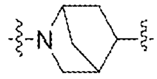 , 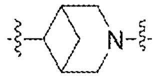 , 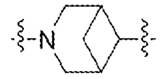 ,

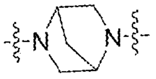 , 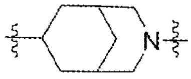 , 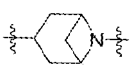 , 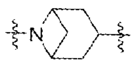 , 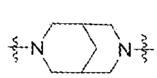 , 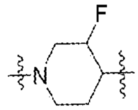 ,

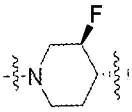 , 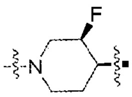 , 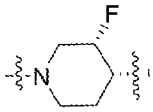 , 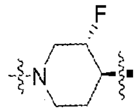 , 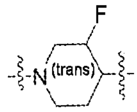 , 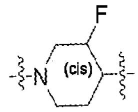 , 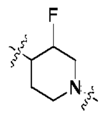 ,

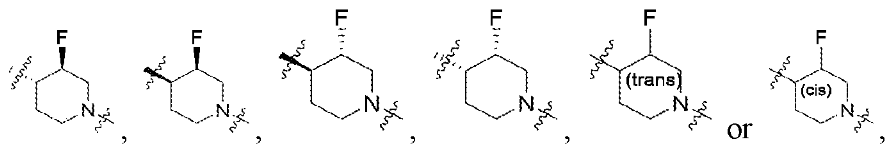 ,

which, when substituted, is optionally further substituted with 0 to 4 (such as 0, 1, 2, 3 or 4) substituents selected from H, F, $CF_3$, methyl, =O, hydroxymethyl, COOH, CN or $NH_2$;
B is selected from

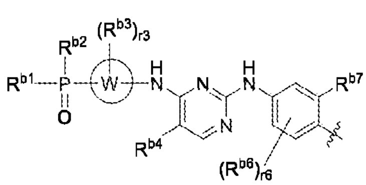 , 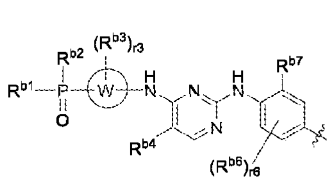 ,

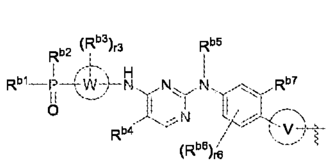 or 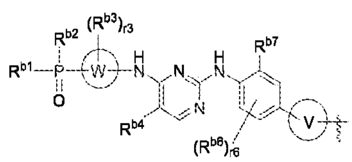 ;

ring V is selected from piperidine or piperazine, wherein the piperidine or piperazine is optionally further substituted with 0 to 2 (such as 0, 1 or 2) F;
$R^{b1}$ and $R^{b2}$ are selected from methyl;
K is selected from one of the structural fragments shown in Table K-1;
the definitions of other groups are the same as those in any one of the first, second, third and fourth embodiment of the present invention.

As a sixth embodiment of the present invention, provided is the above-mentioned compound shown in general formula (I) or the stereoisomer, deuterated compound, solvate, prodrug, metabolite, pharmaceutically acceptable salt or co-crystal thereof,

L is selected from -Cy1-, -Cy1-Ak1-, -Cy1-Ak1-Ak2-, -Cy1-Ak1-Ak2-Ak3-, -Cy1-Ak1-Ak2-Ak3-Ak4-, -Cy1-Cy2-, -Cy1-Ak1-Cy2-, -Cy1-Cy2-Ak2-, -Cyl-Ak1-Cy2-Ak2-, -Cy1-Ak1-Cy2-Ak2-Ak3-, -Cy1-Ak1-Cy2-Ak2-Ak3-Ak4-, -Cyl-Cy2-Ak2-Ak3-, -Cy1-Cy2-Ak2-Ak3-Ak4-, -Cy1-Ak1-Cy2-Ak2-Ak3-Ak4-, -Cyl-Ak1-Ak2-Cy3-, -Cy1-Ak1-Ak2-Cy3-Ak3-, -Cy1-Cy2-Cy3-, -Cy1-Ak1-Cy2-Cy3-, -Cy1-Cy2-Ak2-Cy3-, -Cy1-Cy2-Cy3-Ak3-, -Cy1-Ak1-Cy2-Cy3-Ak3-, -Cy1-Cy2-Ak2-Cy3-Ak3-, -Cy1-Ak1-Cy2-Ak2-Cy3-, -Cy1-Ak1-Cy2-Ak2-Cy3-Ak3-, -Cyl-Cy2-Cy3-Ak3-Ak4-, -Cy1-Cy2-Cy3-Ak3-Cy4-, -Cy1-Cy2-Cy3-Cy4-, -Cy1-Ak1-Cy2-Cy3-Cy4-, -Cy1-Cy2-Ak2-Cy3-Cy4-, -Cy1-Cy2-Cy3-Ak3-Cy4-, -Cy1-Cy2-Cy3-Cy4-Ak4-, -Cy1-Ak1-Cy2-Ak2-Cy3-Ak3 -Cy4-, -Cy1-Ak1-Cy2-Ak2-Cy3-Cy4-, -Ak1-Ak2-Cy3-Cy4-, -Ak1-Cy2-Ak2-Cy3-, -Ak1-Cy2-Cy3-Ak3-Cy4-, - Ak1-Cy2-Cy3-Cy4-Ak4-Cy5-, -Ak1-Cy2-Ak2-, -Ak1-Ak2-Ak3-Ak4-, -Ak1-Ak2-Ak3-, -Ak1-Ak2-, -Ak1-Ak2-Ak3-Ak4-Ak5-, -Cy1-Cy2-Cy3-Ak3-Ak4-Ak5-, - Cy 1 -Cy2-Ak2-Cy3 - Ak3 - Ak4-Ak5 -, -Cy1-Ak1-Cy2-Ak2-Ak3-Ak4-Ak5-, -Cy1-Cy2-Cy3-Cy4-Ak4-Ak5-, -Cy1-Ak1-Ak2-Ak3-Ak4-Ak5-, -Ak1-Cy2-Ak2-Ak3-Ak4-Ak5-, -Ak1-Cy2-, -Ak1-Cy2-Ak2-Ak3-Ak4- or -Ak1-Cy2-Ak2-Ak3-; Ak1, Ak2, Ak3, Ak4 and Ak5 are each independently selected from -O-, - $OCH_2$-, -$CH_2O$-, -$OCH_2CH_2$-, -$CH_2CH_2O$-, -C≡C-, -$C(CH_3)_2$-, -$CH_2$-, -$CH_2CH_2$-, - $CH_2CH_2CH_2$-, -$N(CH_3)$-, -NH-, -$CH_2N(CH_3)$-, -$CH_2NH$-, -$NHCH_2$-, - $CH_2CH_2N(CH_3)$-, -$CH_2CH_2NH$-, -$NHCH_2CH_2$-, -C(=O)-, -C(=O)$CH_2$NH-, - $CH_2$C(=O)NH-, -C(=O)N($CH_3$)-, -N($CH_3$)C(=O)-, -C(=O)NH- or -NHC(=O)-; the definitions of other groups are the same as those in any one of the first, second, third, fourth and fifth embodiments of the present invention.

As a seventh embodiment of the present invention, provided is the above-mentioned compound shown in general formula (I) or the stereoisomer, deuterated compound, solvate, prodrug, metabolite, pharmaceutically acceptable salt or co-crystal thereof,

L is selected from a bond or a group in Table A, wherein the left side of the group is linked to B;
the definitions of other groups are the same as those in any one of the first, second, third, fourth, fifth and sixth embodiments of the present invention.

As an eighth embodiment of the present invention, provided is the above-mentioned compound shown in general formula (I) or the stereoisomer, deuterated compound, solvate, prodrug, metabolite, pharmaceutically acceptable salt or co-crystal thereof,

B is selected from one of the following structural fragments:

| | | |
|---|---|---|
| 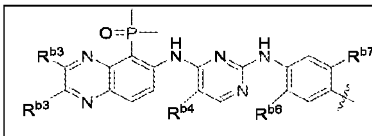 | 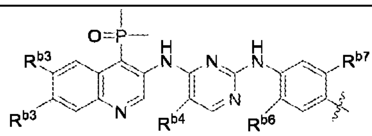 | 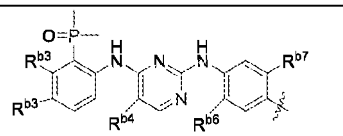 |
| 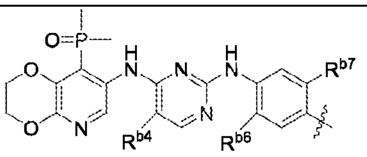 | 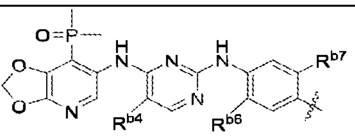 | 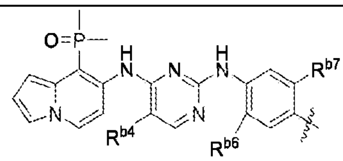 |
| 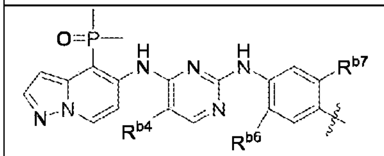 | 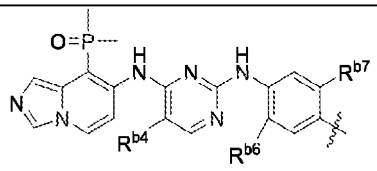 | 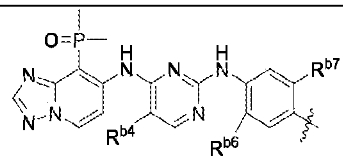 |
| 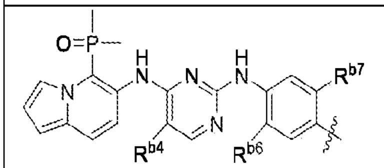 | 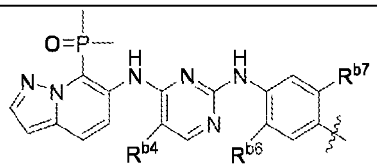 | 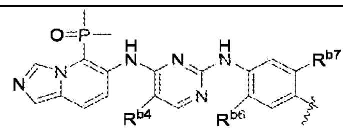 |
| 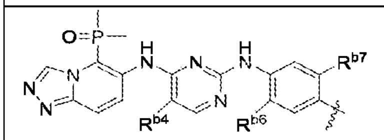 | 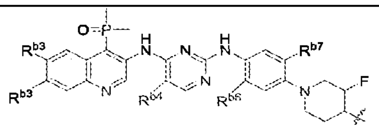 | 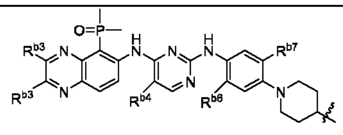 |
| 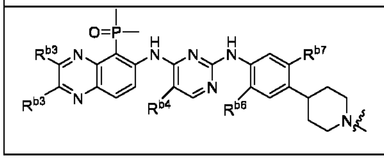 | 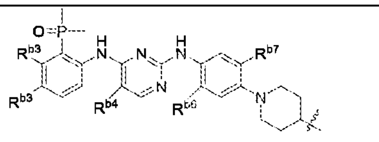 | 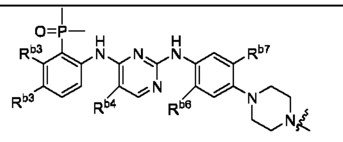 |

| | | |
|---|---|---|
| 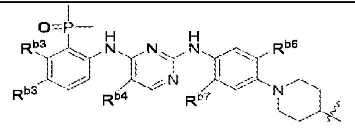 | 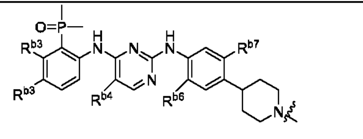 | 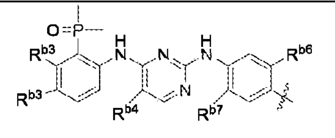 |
| 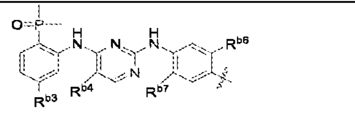 | | |
| 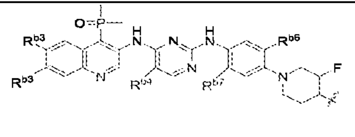 | 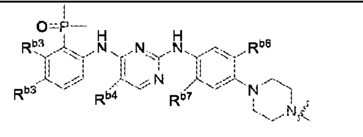 | 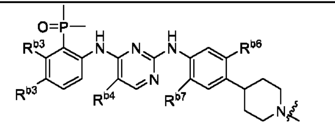 |
| 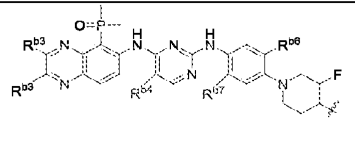 | 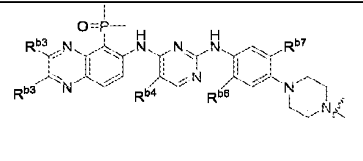 | 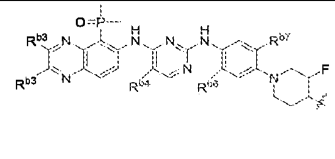 |
| 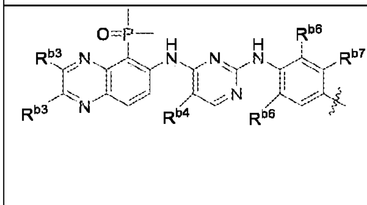 | 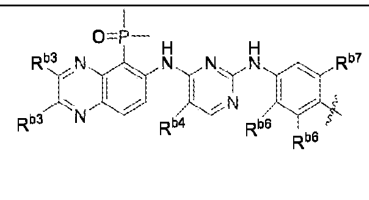 | 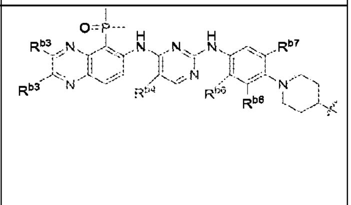 |
| 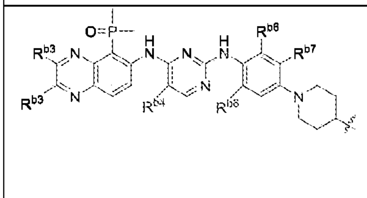 | 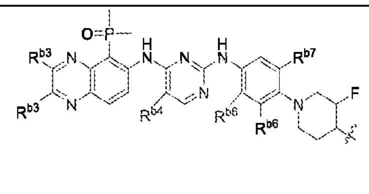 | 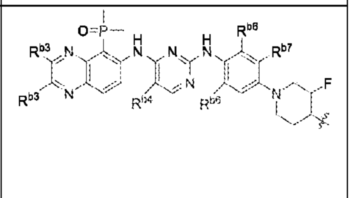 |
| 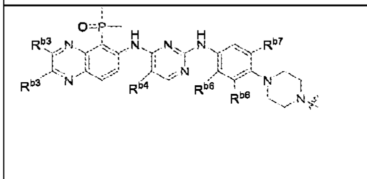 | 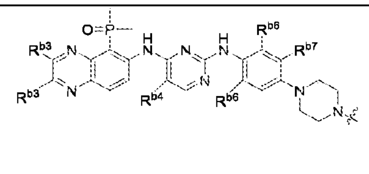 | 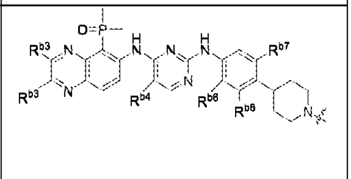 |
| 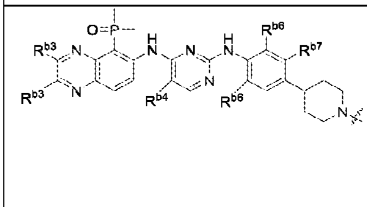 | 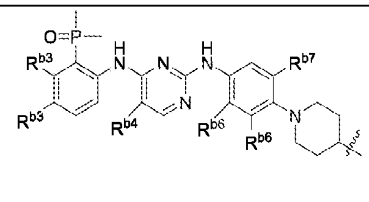 | 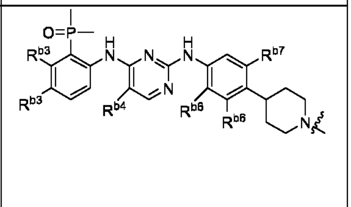 |
| 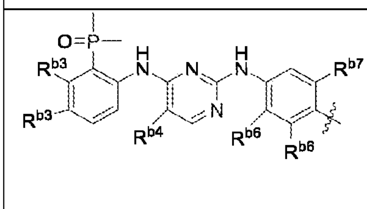 | 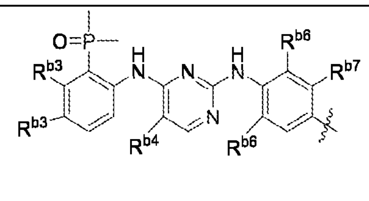 | 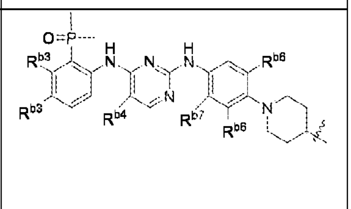 |
| 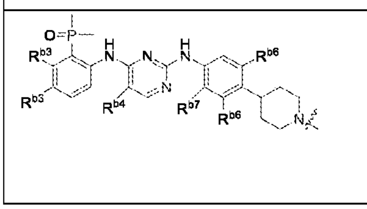 | 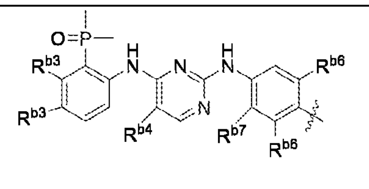 | 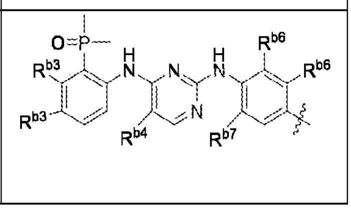 |

| | | |
|---|---|---|
| 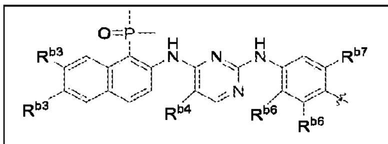 | 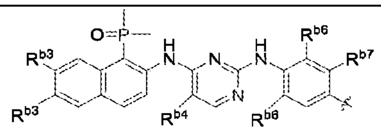 | 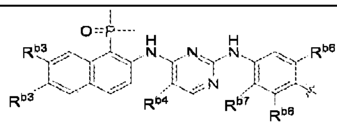 |
| 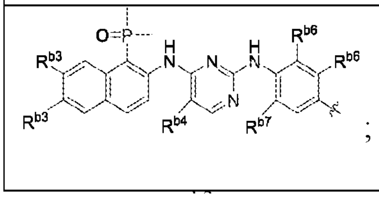 ; | 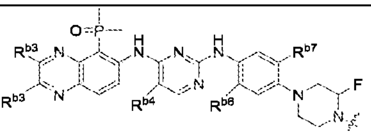 | |

each $R^{b3}$ is independently selected from H, F, Cl, Br, I, OH, CN, methyl, ethyl, isopropyl, propyl, ethynyl, methoxy, cyclopropyl, cyclobutyl, cyclopentyl, cyclohexyl, phenyl or 5- to 6-membered heteroaryl, wherein the phenyl or 5- to 6-membered heteroaryl is optionally further substituted with 0 to 4 (such as 0, 1, 2, 3 or 4) substituents selected from H, F, Cl, Br, methyl, ethyl, methoxy or ethoxy, and the heteroaryl contains 1 to 3 (such as 1, 2, or 3) heteroatoms selected from O, S or N;

$R^{b4}$ is selected from H, F, Cl or Br;

each $R^{b6}$ is independently selected from H, F, Cl, Br, methyl, ethyl, methoxy or ethoxy;

$R^{b7}$ is selected from cyclopropyl, cyclobutyl, cyclopentyl, cyclohexyl, pyrrole, pyrazole, pyridine or phenyl, wherein the $R^{b7}$ is optionally further substituted with 0 to 4 (such as 0, 1, 2, 3 or 4) substituents selected from H, F, Cl, Br, I, OH, =O, CN, $CH_2D$, $CHD_2$, $CD_3$, methyl or ethyl;

K is selected from one of the structural fragments shown in Table K-2;

the definitions of other groups are the same as those in any one of the first, second, third, fourth, fifth, sixth and seventh embodiments of the present invention.

As a ninth embodiment of the present invention, provided is the above-mentioned compound shown in general formula (I) or the stereoisomer, deuterated compound, solvate, prodrug, metabolite, pharmaceutically acceptable salt or co-crystal thereof,

B is selected from one of the structural fragments shown in Table B-1;

K is selected from one of the structural fragments shown in Table K-3;

the definitions of other groups are the same as those in any one of the first, second, third, fourth, fifth, sixth, seventh and eighth embodiments of the present invention.

The present invention relates to a compound as described below or a stereoisomer, deuterated compound, solvate, prodrug, metabolite, pharmaceutically acceptable salt or co-crystal thereof, wherein the compound is selected from one of the structures shown in Table E-1.

The present invention relates to a compound as described below or a stereoisomer, deuterated compound, solvate, prodrug, metabolite, pharmaceutically acceptable salt or co-crystal thereof, wherein the compound is selected from a compound of example 1 to example 173.

The present invention relates to a compound as described below or a stereoisomer, deuterated compound, solvate, prodrug, metabolite, pharmaceutically acceptable salt or co-crystal thereof, wherein the compound is selected from

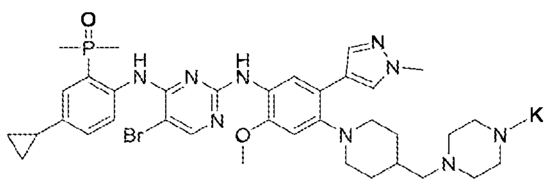,

wherein K is the same as that in Table K-2.

Table E-1

| | |
|---|---|
| 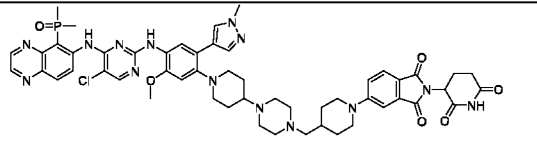 | 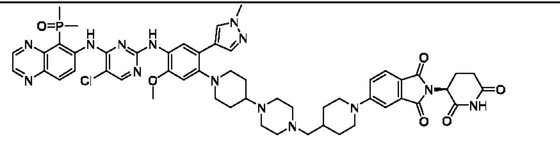 |
| 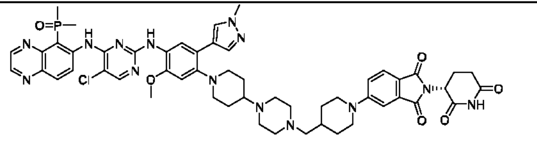 | 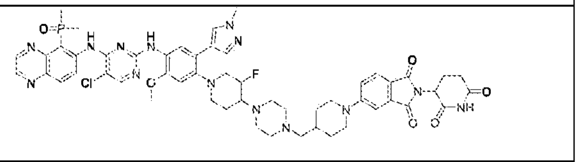 |
| 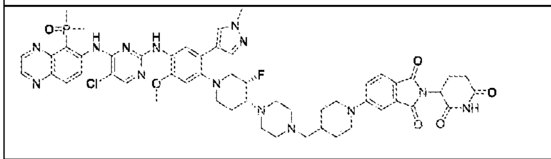 | 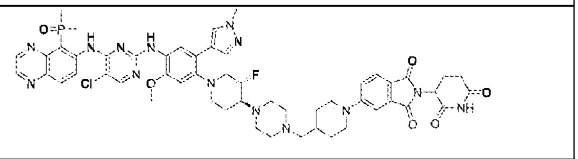 |
| 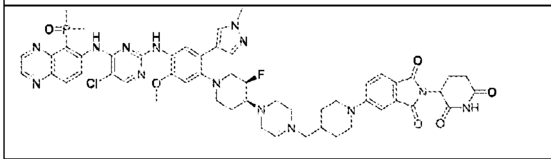 | 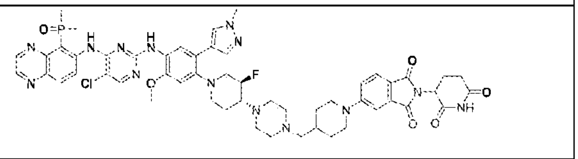 |
| 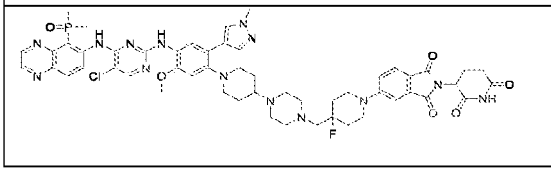 | 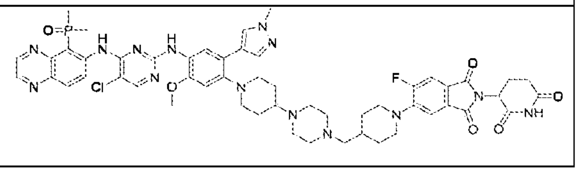 |

| | |
|---|---|
| 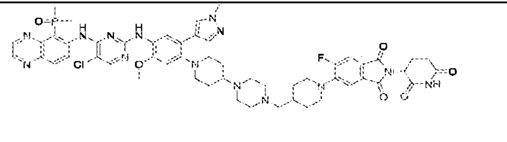 | 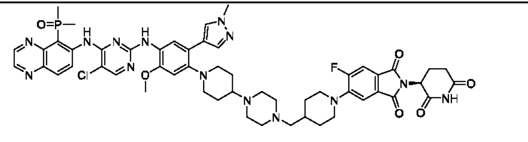 |
| 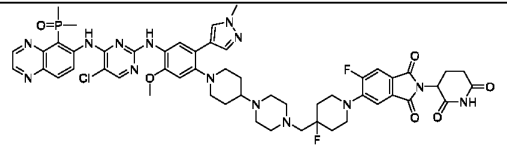 | 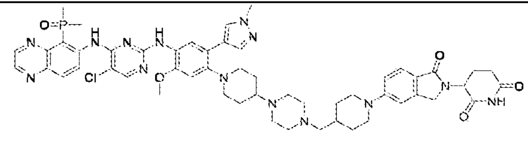 |
| 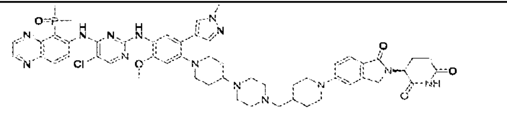 | 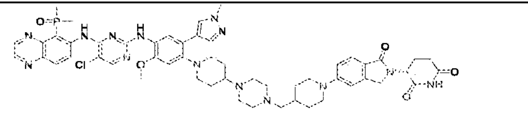 |
| 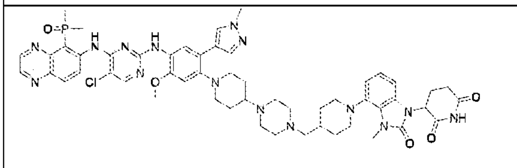 | 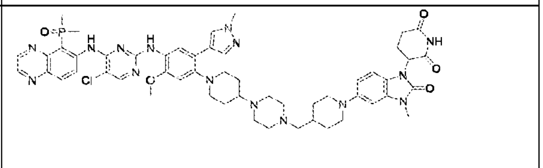 |
| 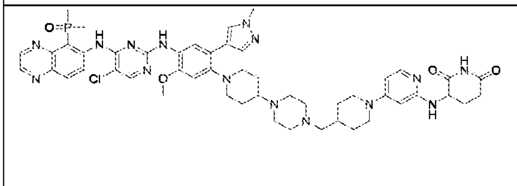 | 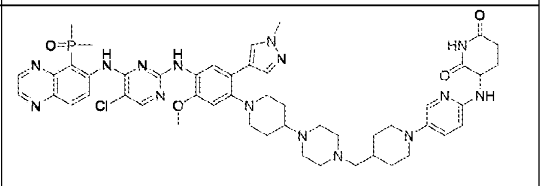 |
| 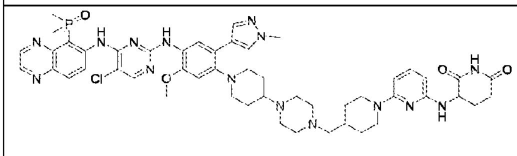 | 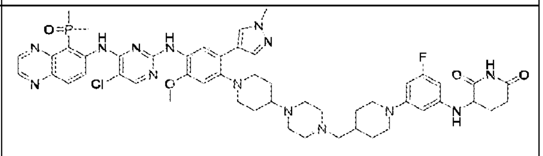 |
| 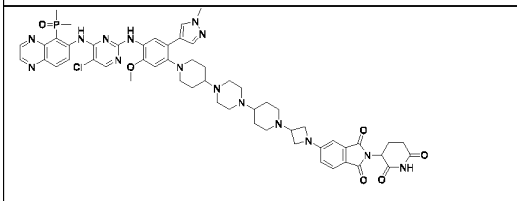 | 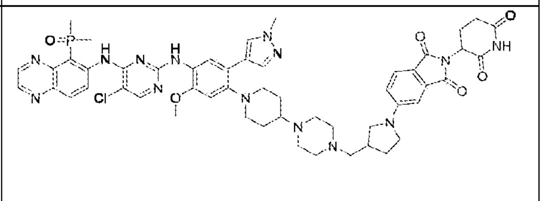 |
| 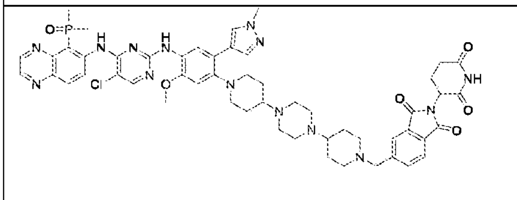 | 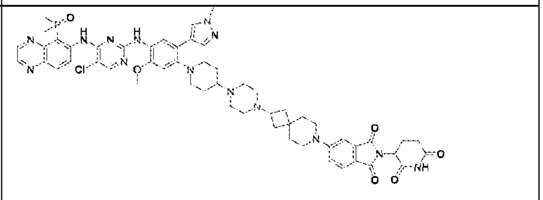 |
| 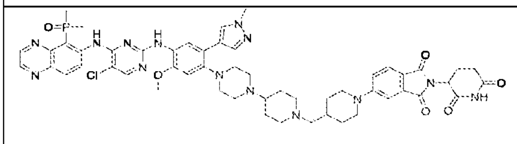 | 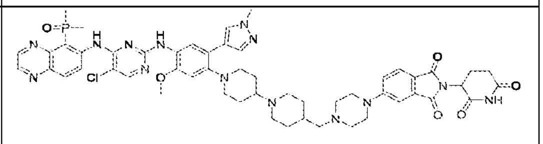 |
| 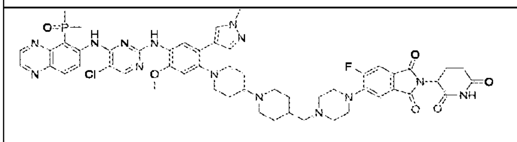 | 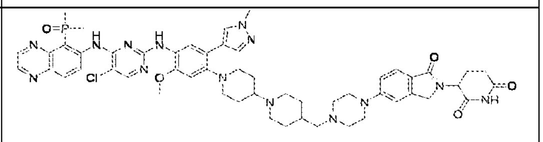 |
| 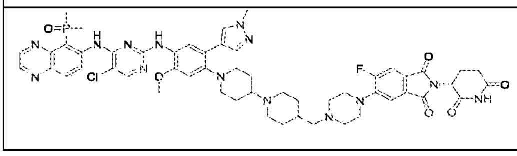 | 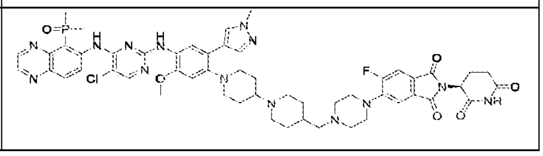 |

| | |
|---|---|
| 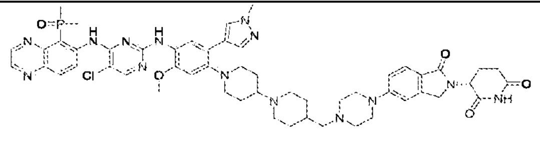 | 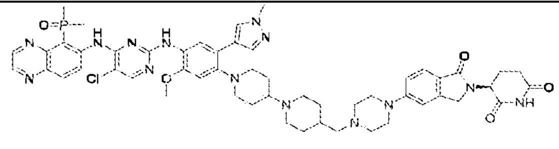 |
| 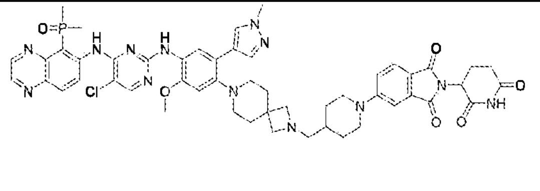 | 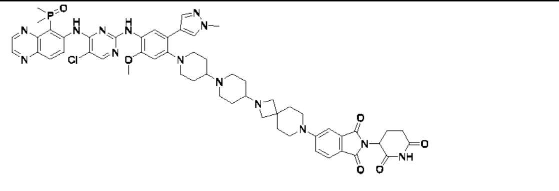 |
| 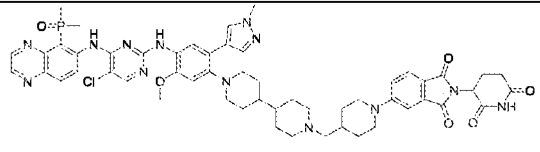 | 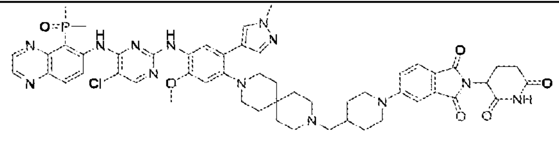 |
| 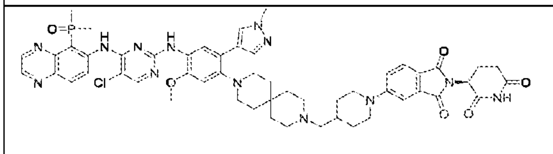 | 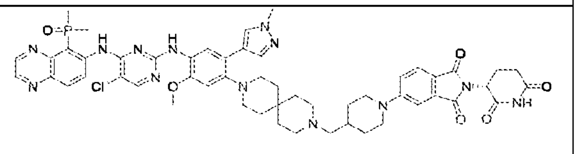 |
| 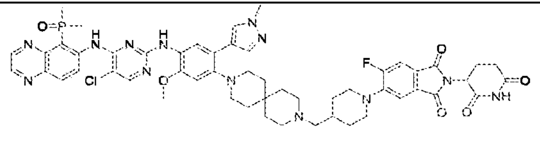 | 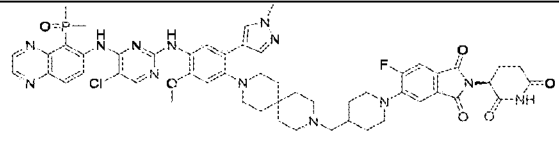 |
| 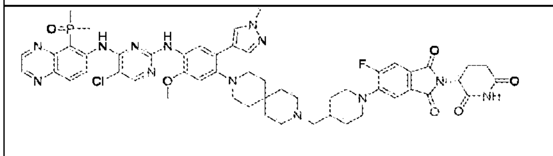 | 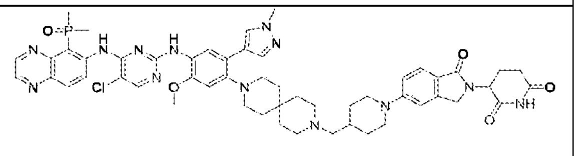 |
| 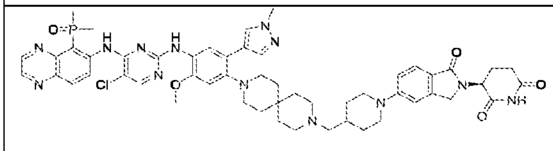 | 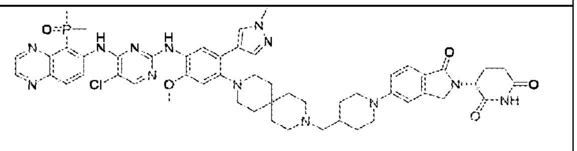 |
| 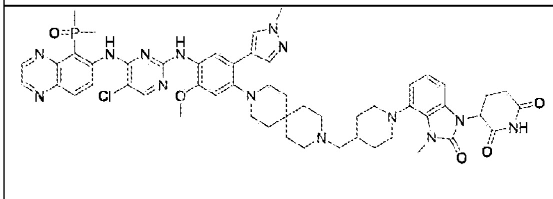 | 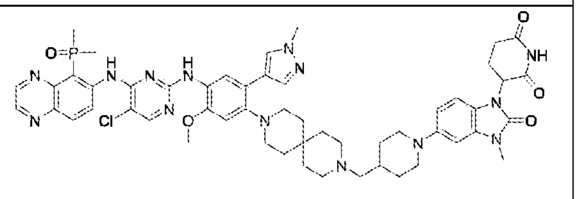 |
| 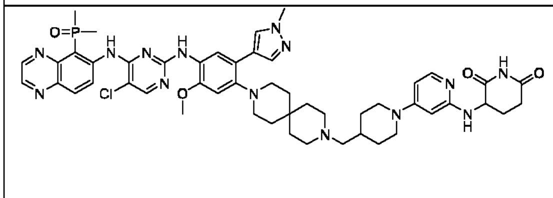 | 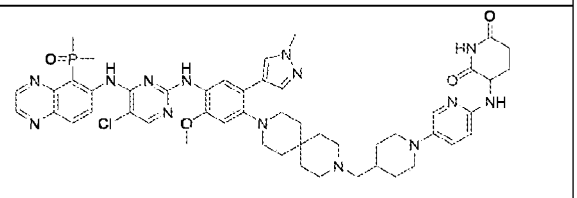 |
| 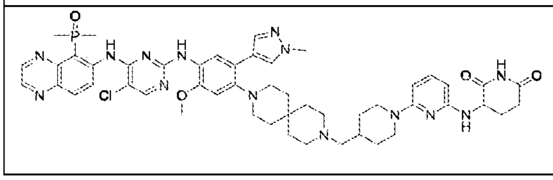 | 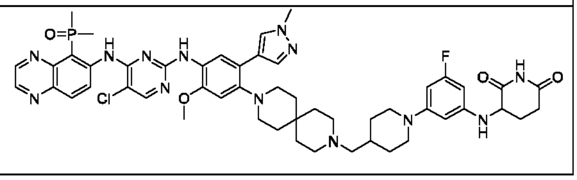 |

| | |
|---|---|
| 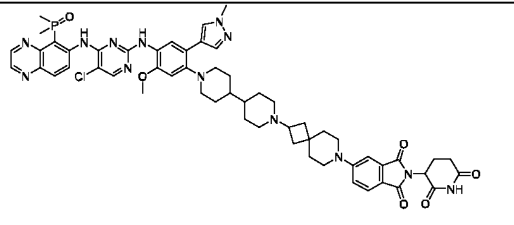 | 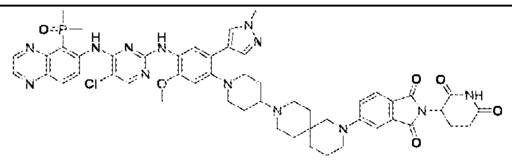 |
| 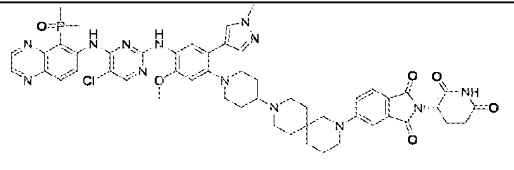 | 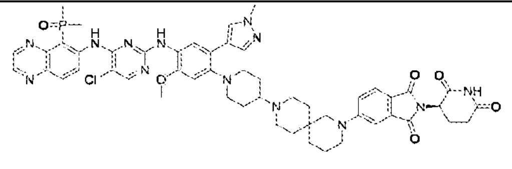 |
| 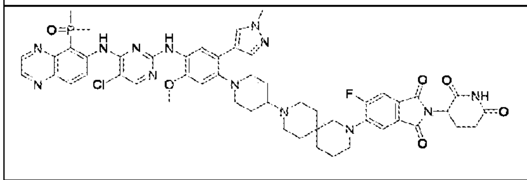 | 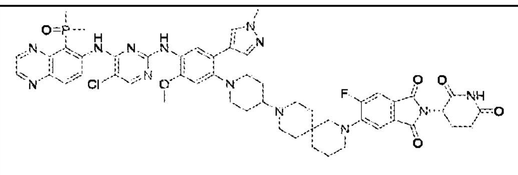 |
| 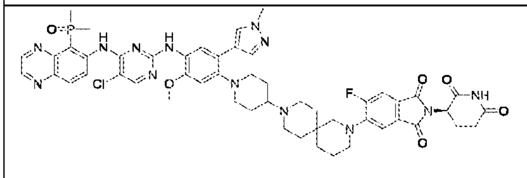 | 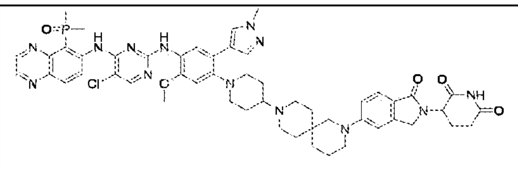 |
| 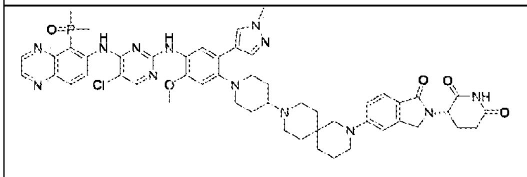 | 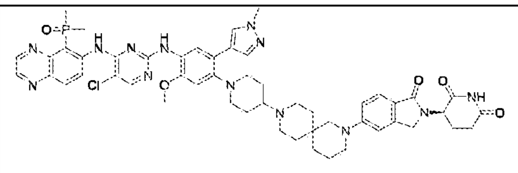 |
| 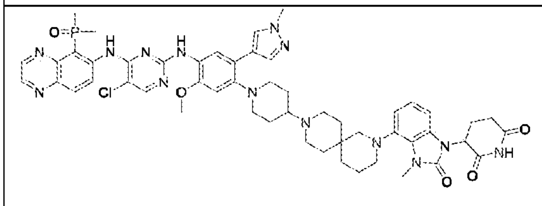 | 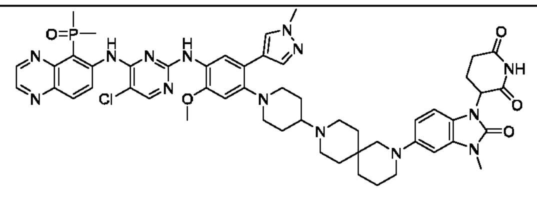 |
| 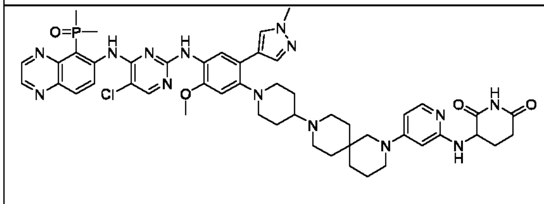 | 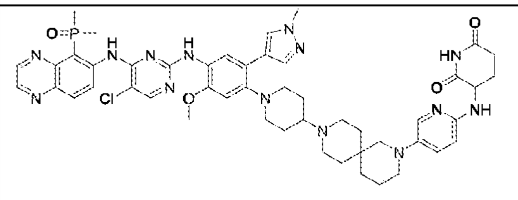 |
| 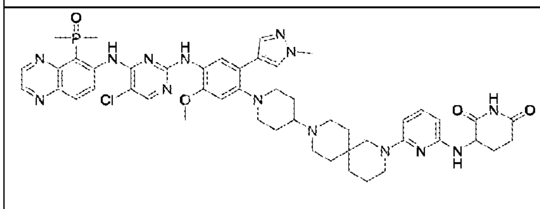 | 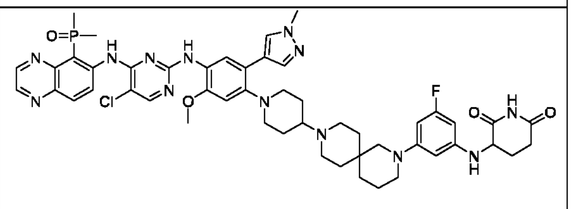 |
| 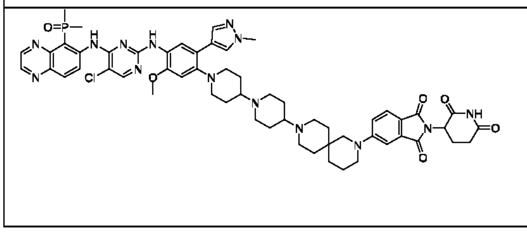 | 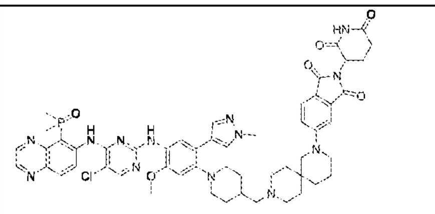 |

| | |
|---|---|
| 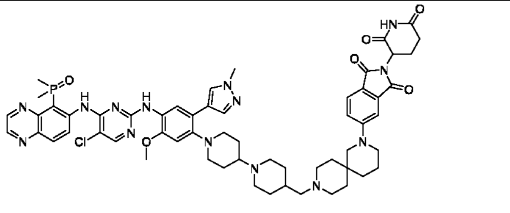 | 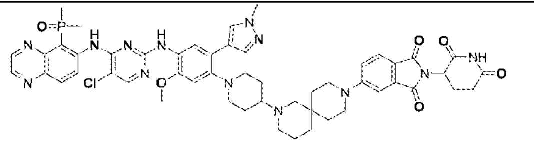 |
| 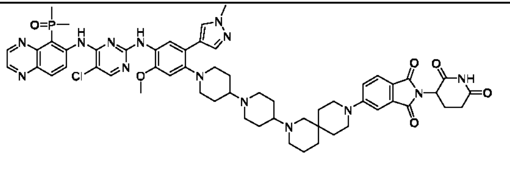 | 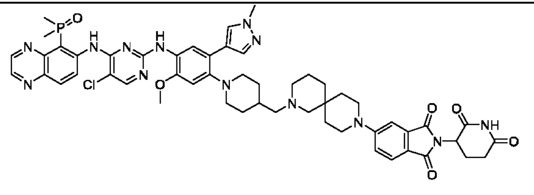 |
| 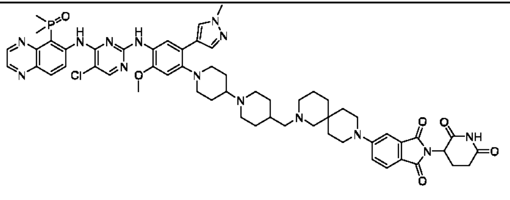 | 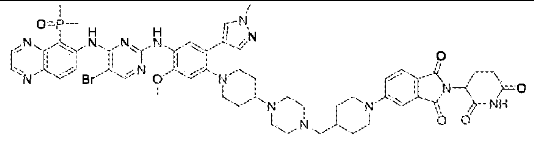 |
| 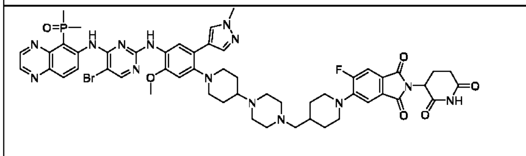 | 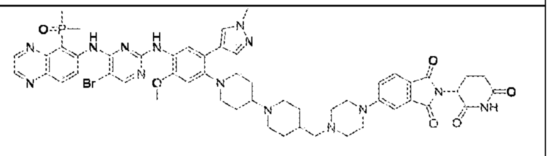 |
| 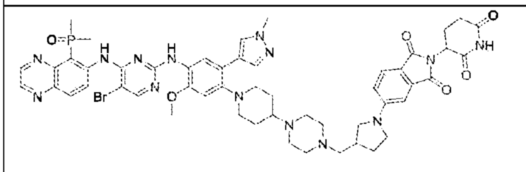 | 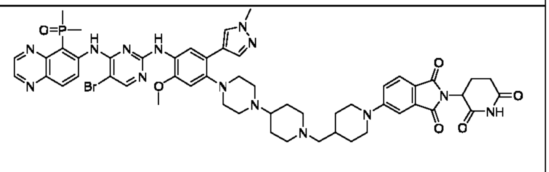 |
| 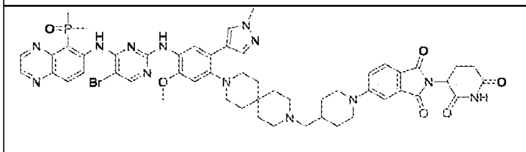 | 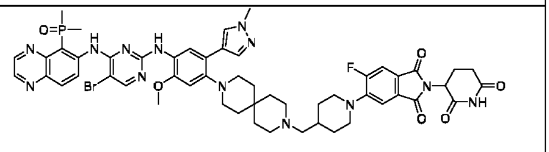 |
| 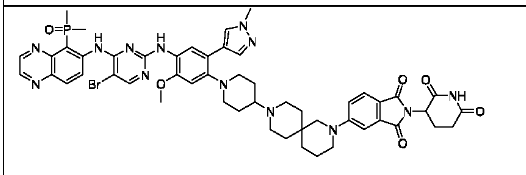 | 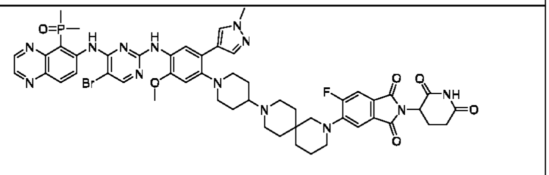 |
| 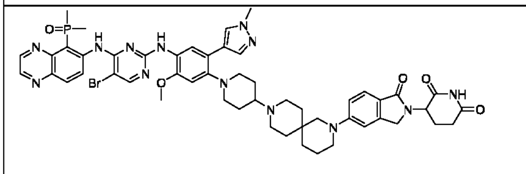 | 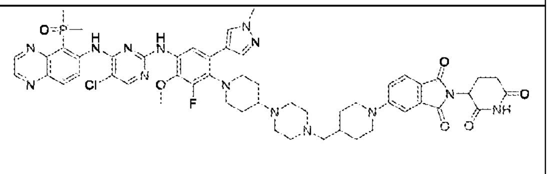 |
| 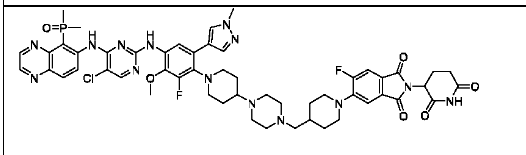 | 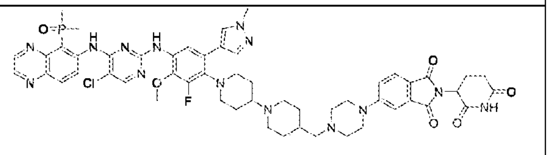 |
| 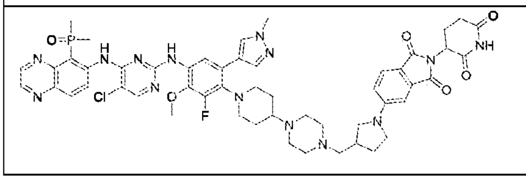 | 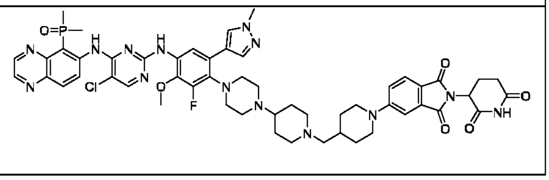 |

| | |
|---|---|
| 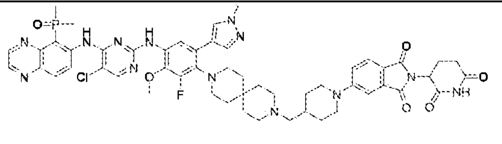 | 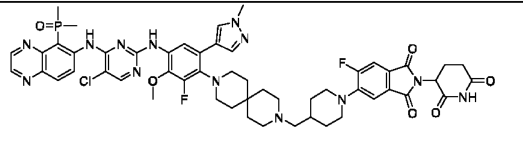 |
| 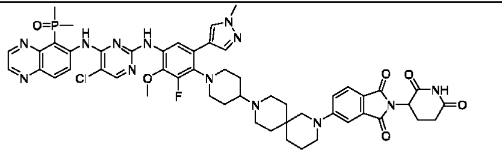 | 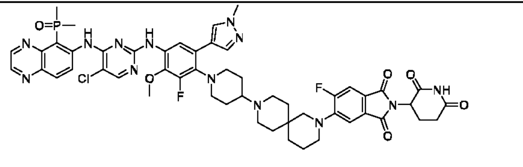 |
| 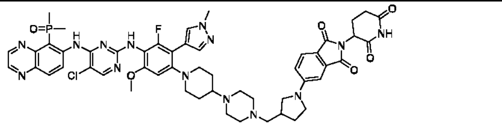 | 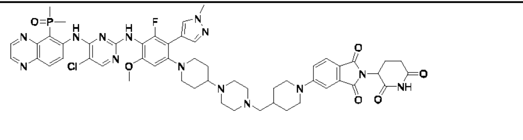 |
| 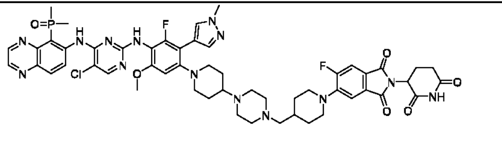 | 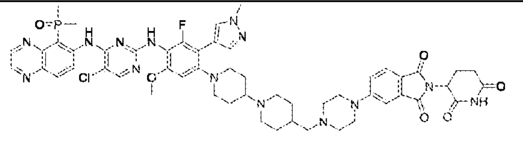 |
| 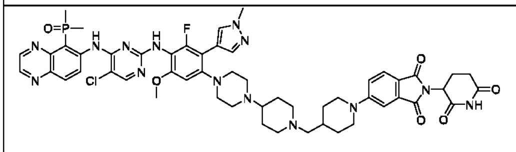 | 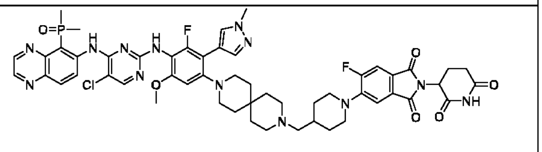 |
| 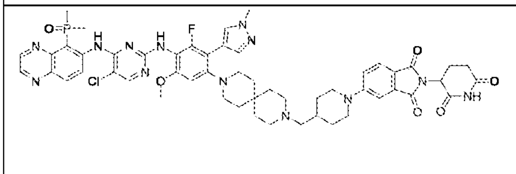 | 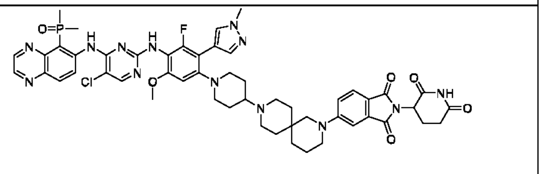 |
| 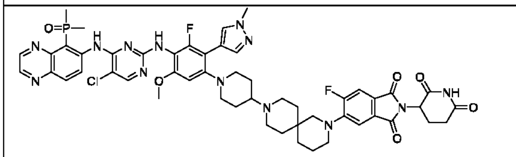 | 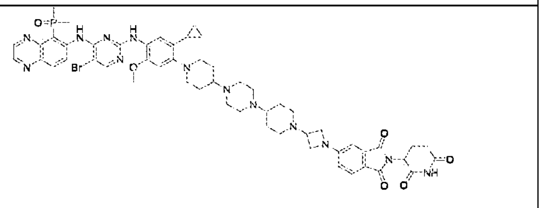 |
| 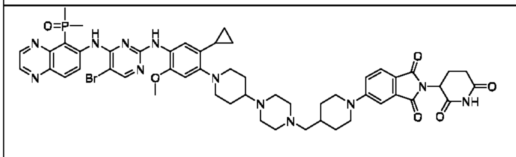 | 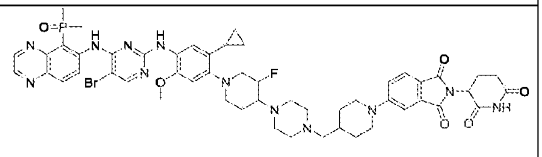 |
| 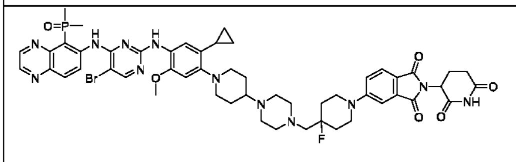 | 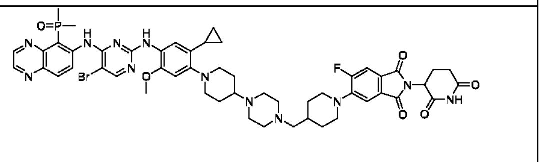 |
| 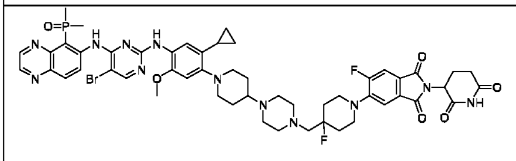 | 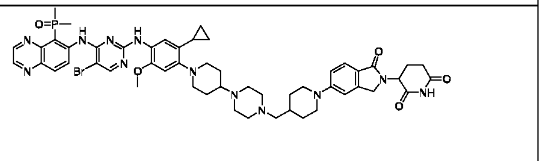 |
| 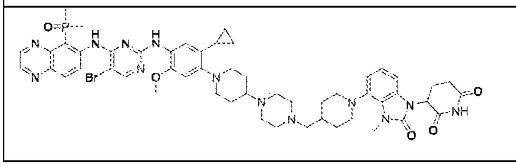 | 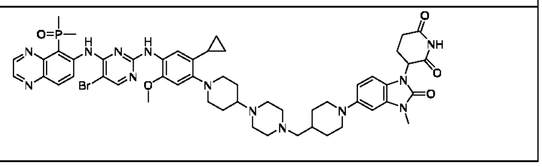 |

| | |
|---|---|
| 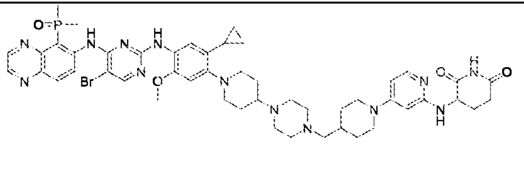 | 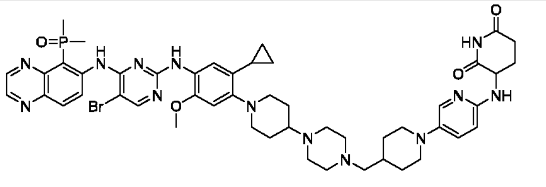 |
| 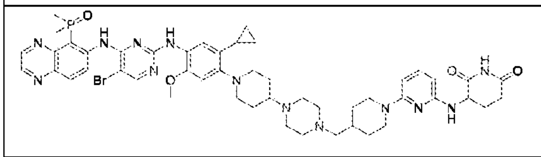 | 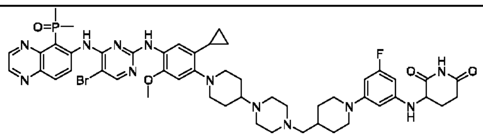 |
| 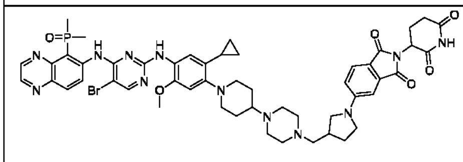 | 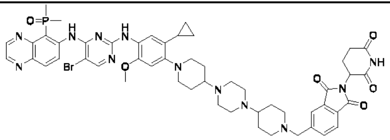 |
| 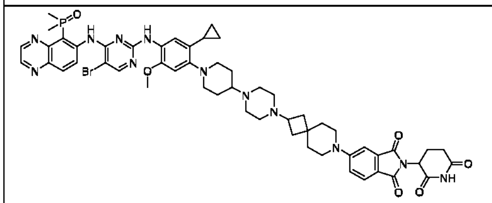 | 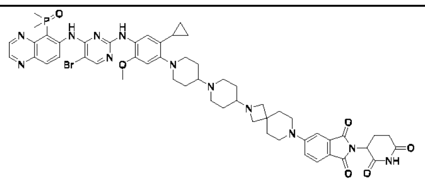 |
| 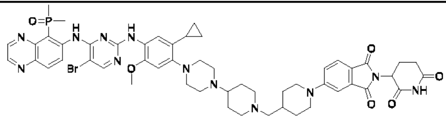 | 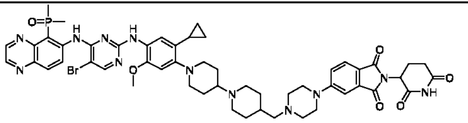 |
| 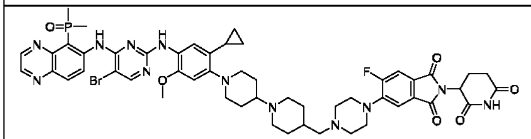 | 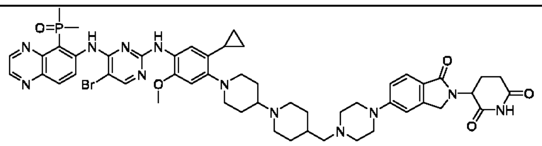 |
| 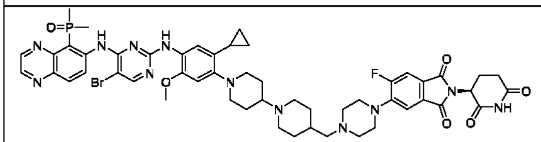 | 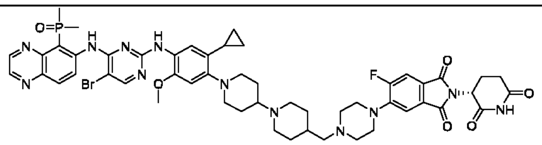 |
| 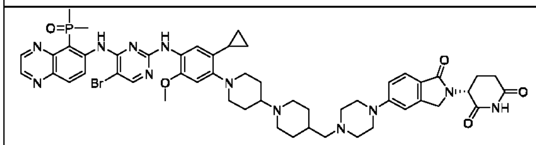 | 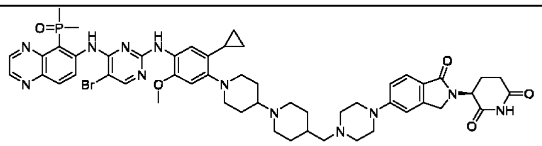 |
| 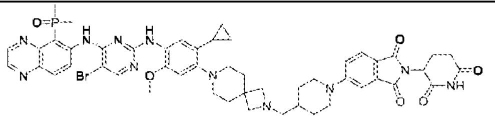 | |
| 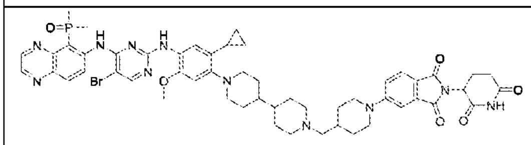 | 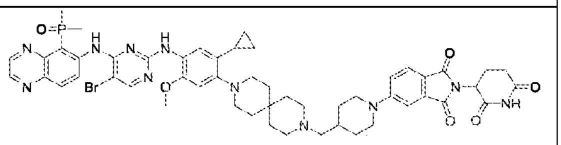 |
| 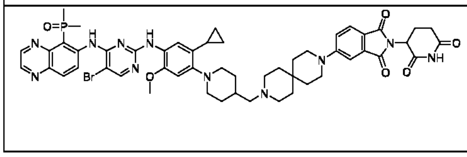 | 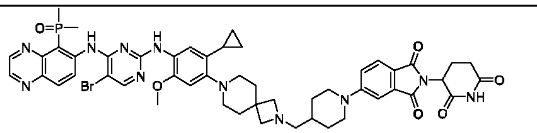 |

| | |
|---|---|
| 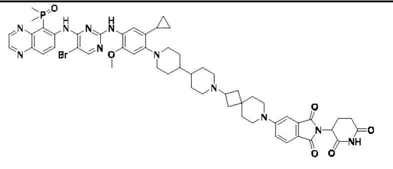 | 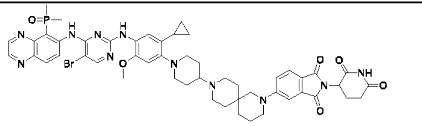 |
| 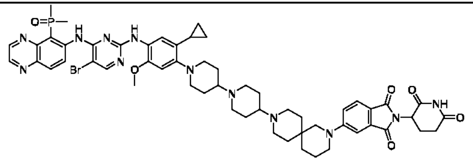 | 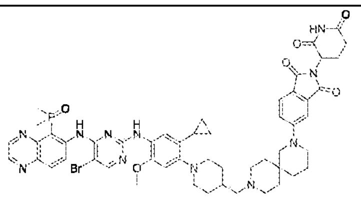 |
| 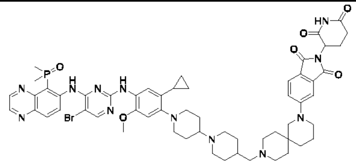 | 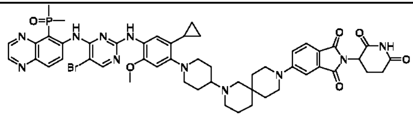 |
| 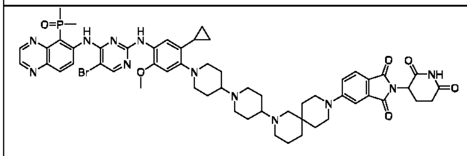 | 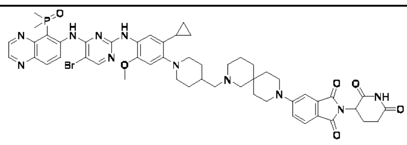 |
| 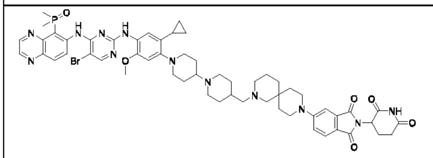 | 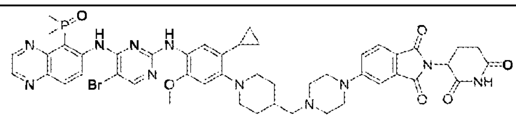 |
| 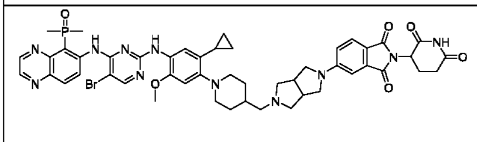 | 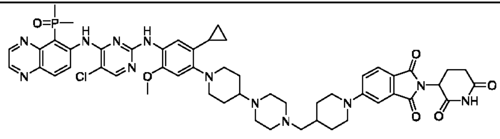 |
| 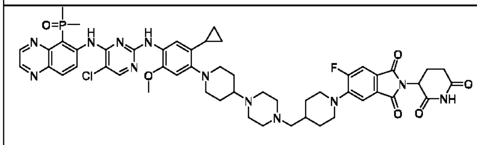 | 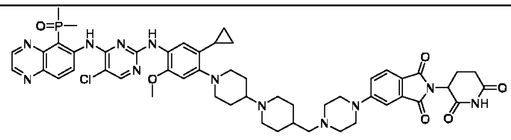 |
| 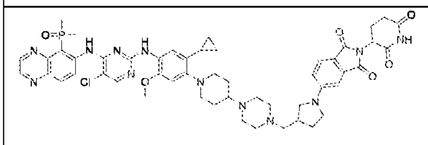 | 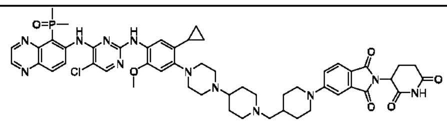 |
| 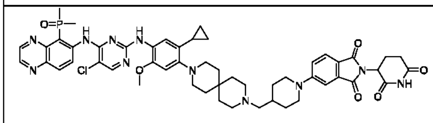 | 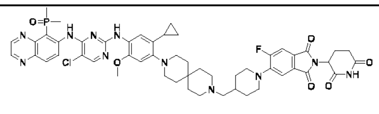 |
| 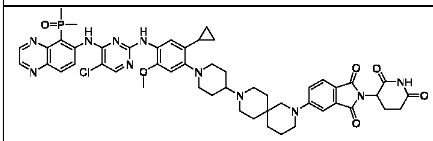 | 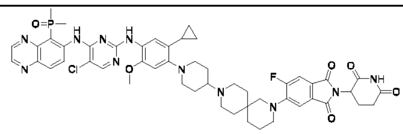 |
| 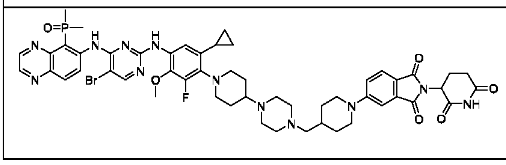 | 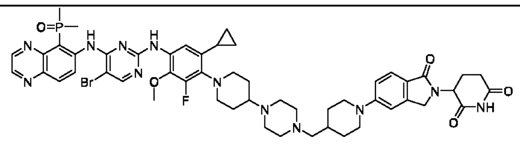 |

| | |
|---|---|
| 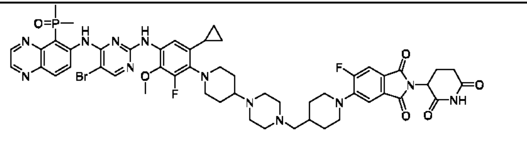 | 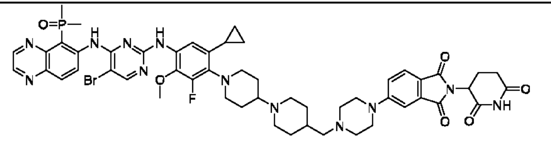 |
| 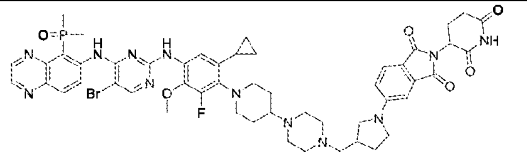 | 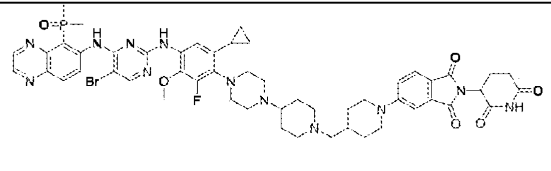 |
| 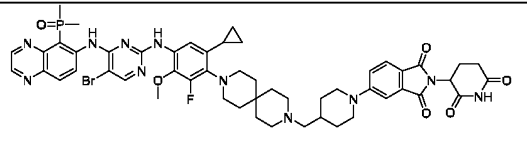 | 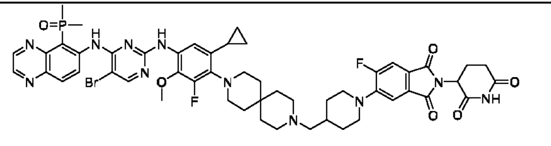 |
| 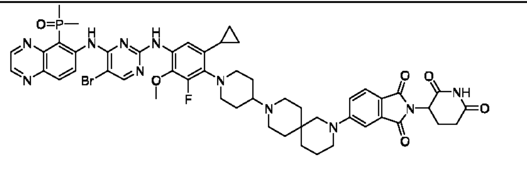 | 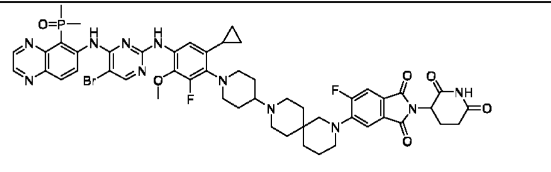 |
| 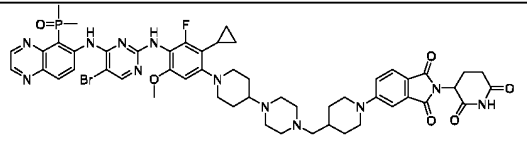 | 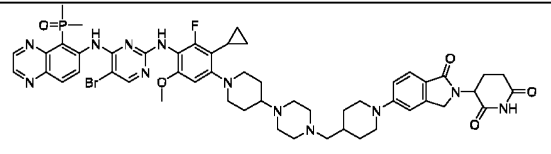 |
| 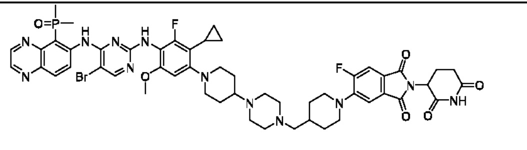 | 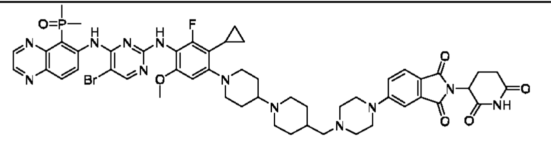 |
| 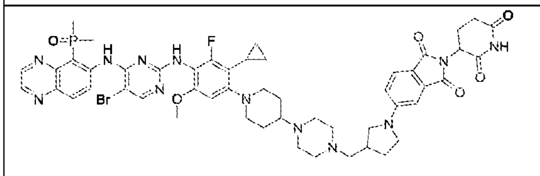 | 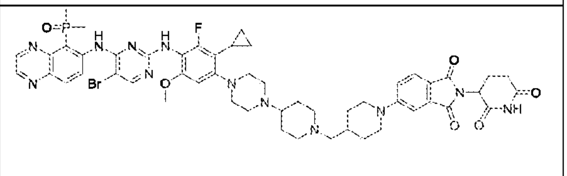 |
| 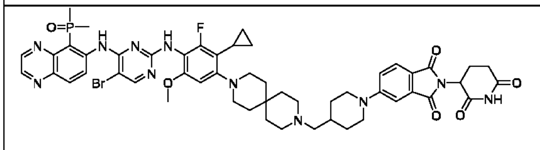 | 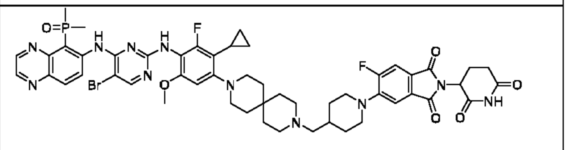 |
| 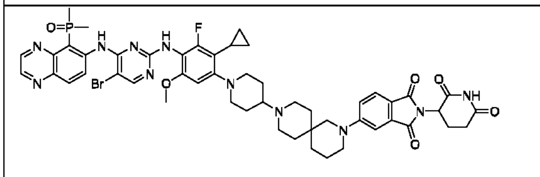 | 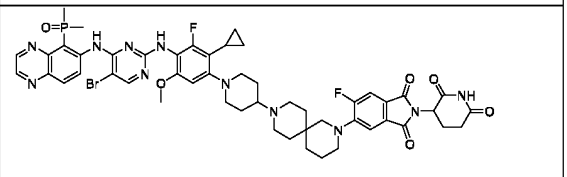 |
| 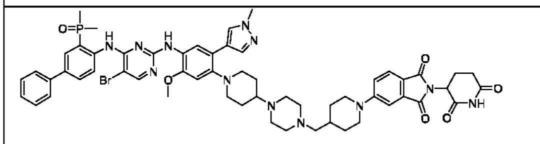 | 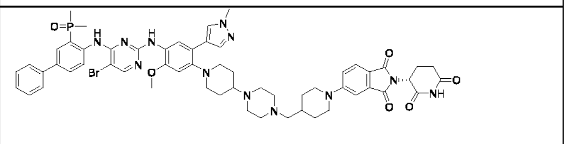 |
| 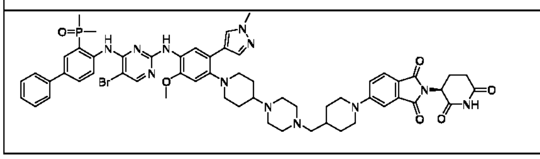 | 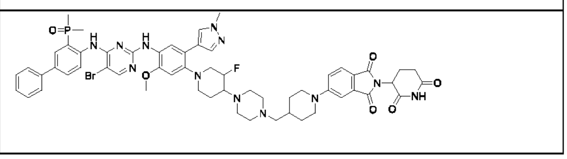 |

| | |
|---|---|
| 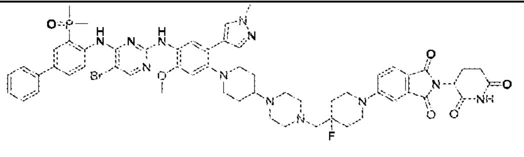 | 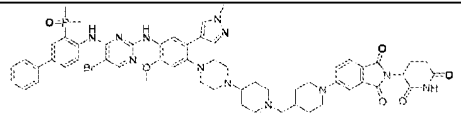 |
| 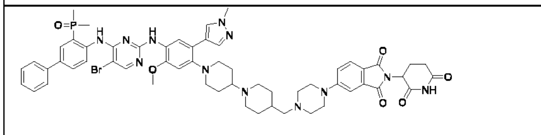 | 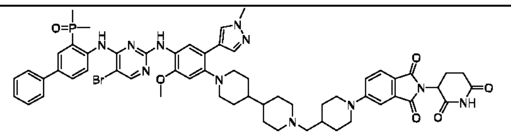 |
| 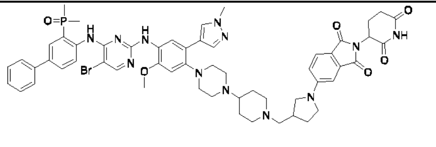 | 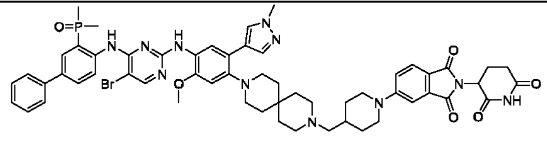 |
| 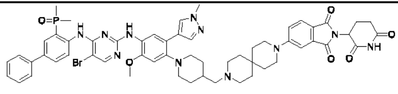 | 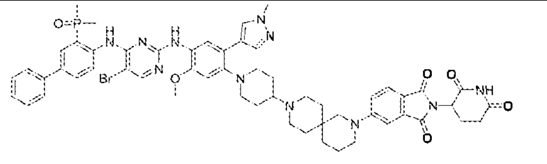 |
| 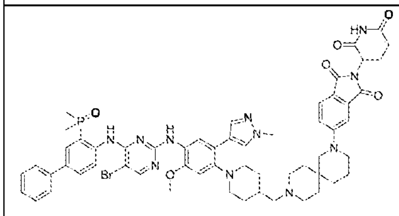 | 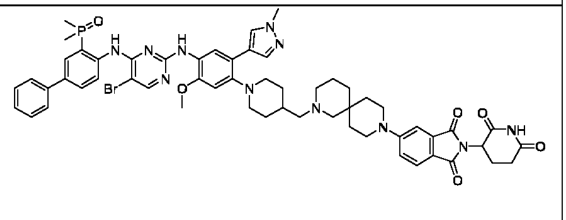 |
| 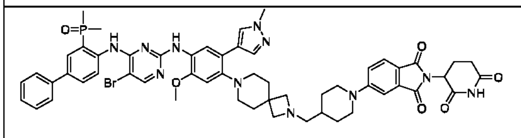 | 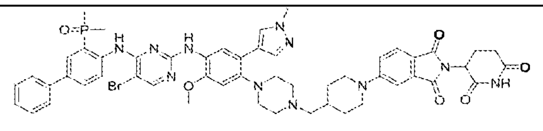 |
| 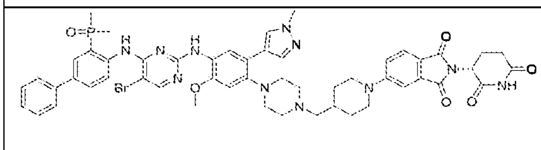 | 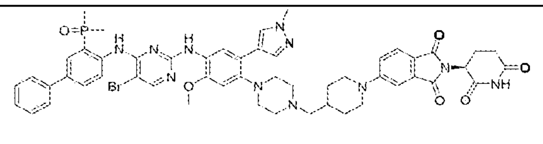 |
| 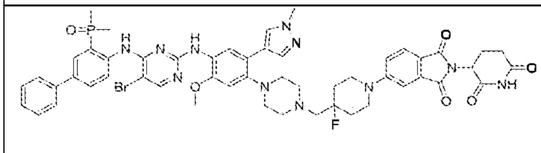 | 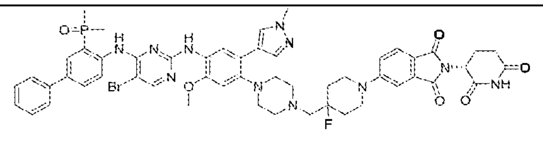 |
| 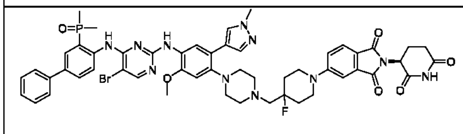 | 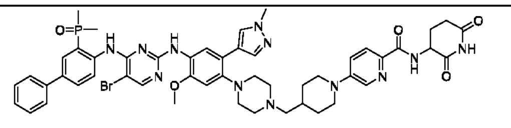 |
| 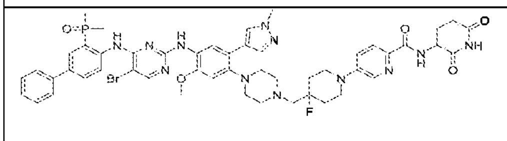 | 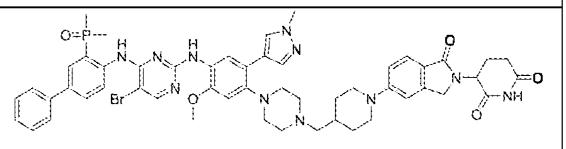 |
| 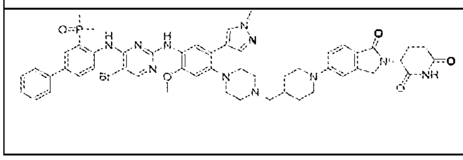 | 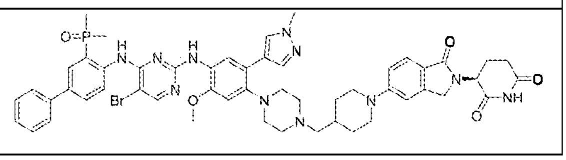 |

| | |
|---|---|
| 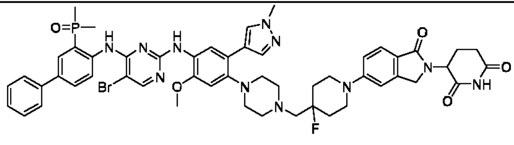 | 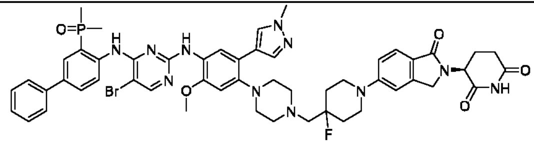 |
| 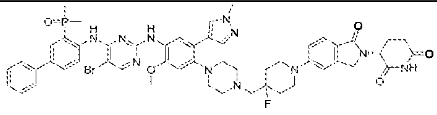 | |
| 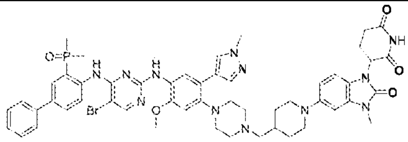 | 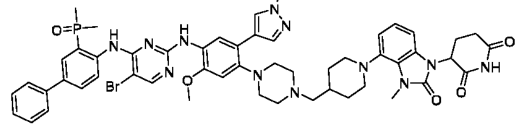 |
| 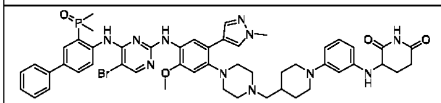 | 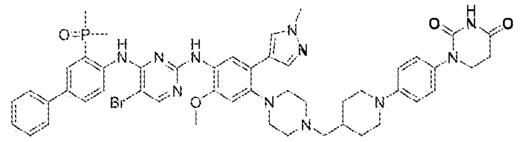 |
| 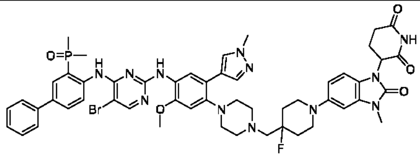 | 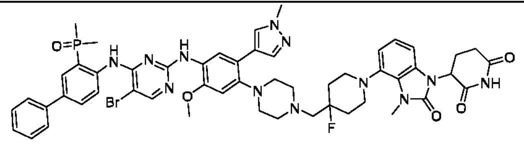 |
| 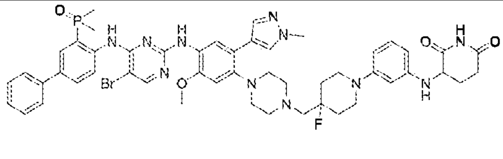 | 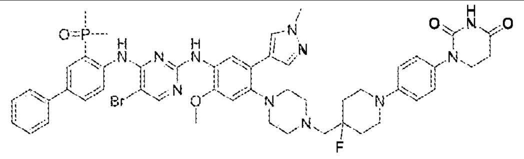 |
| 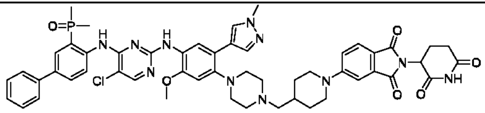 | 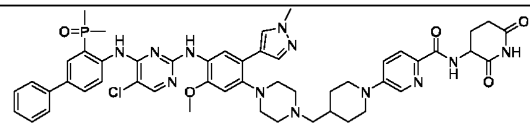 |
| 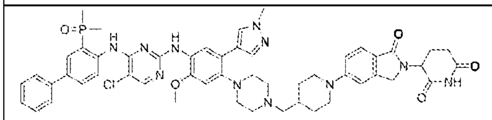 | 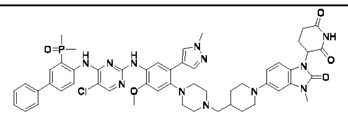 |
| 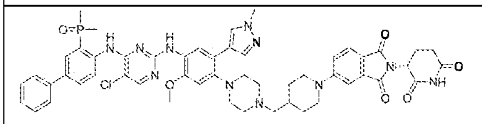 | 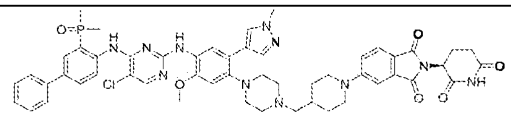 |
| 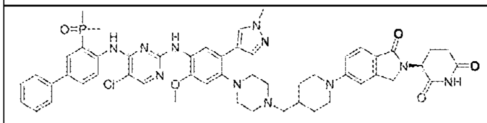 | 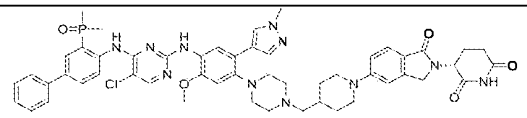 |
| 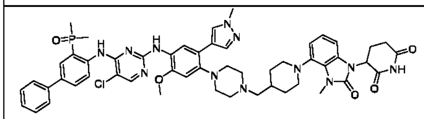 | 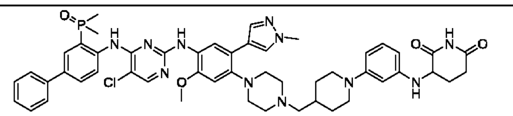 |
| 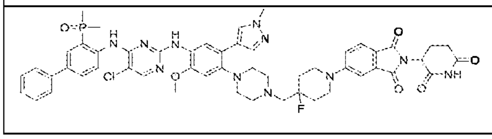 | 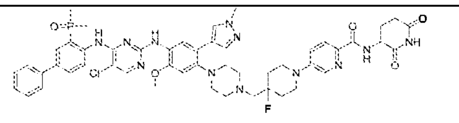 |

| | |
|---|---|
| 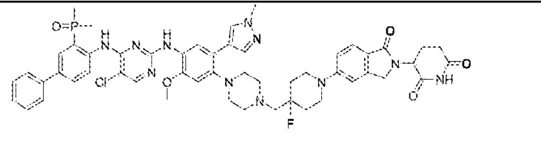 | 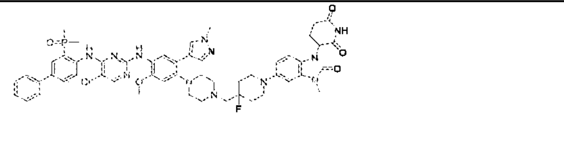 |
| 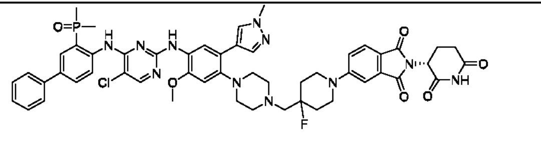 | 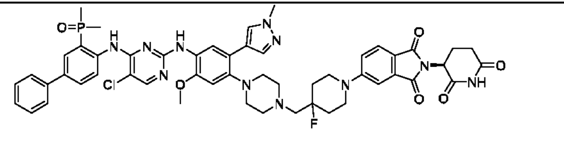 |
| 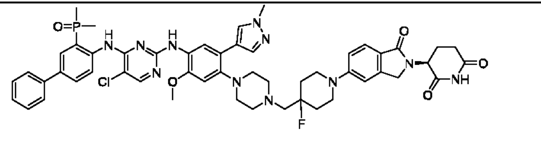 | 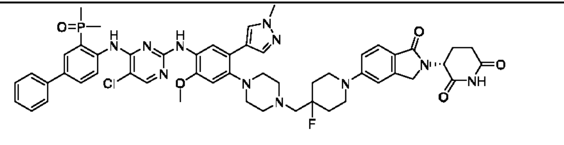 |
| 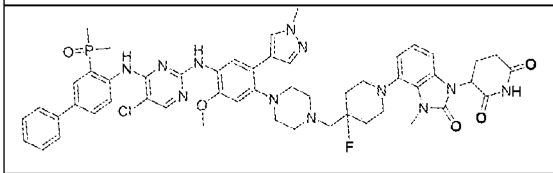 | 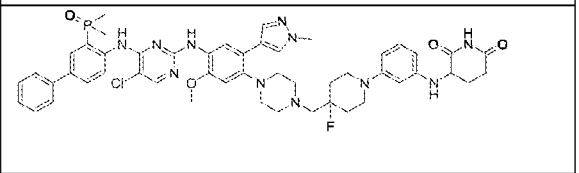 |
| 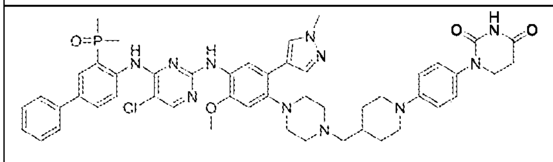 | 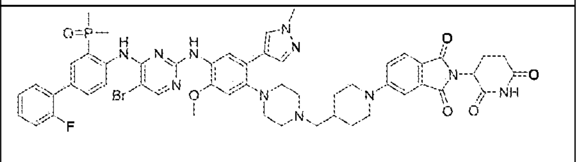 |
| 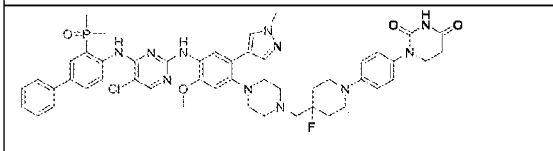 | 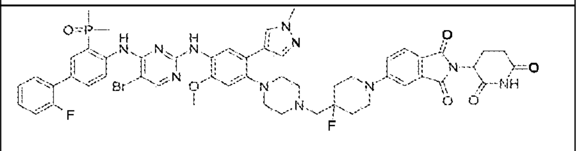 |
| 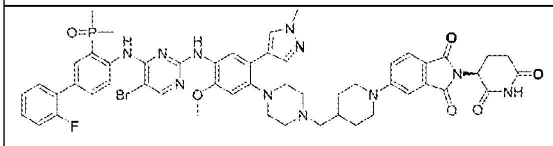 | 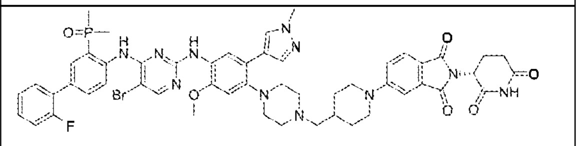 |
| 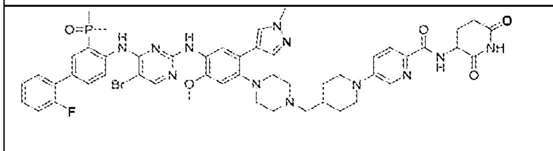 | 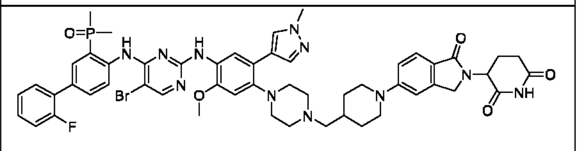 |
| 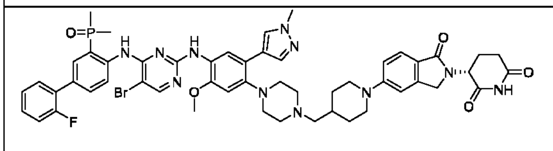 | 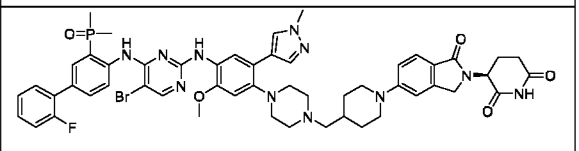 |
| 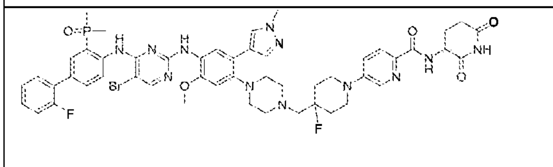 | 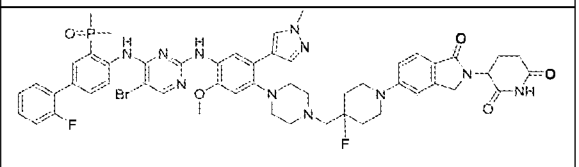 |
| 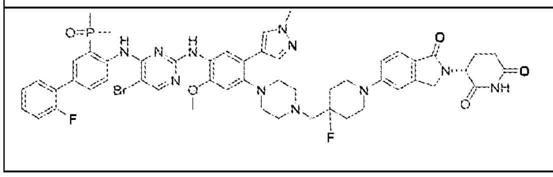 | 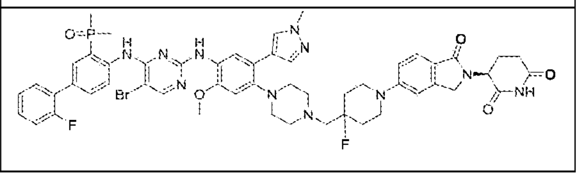 |

| | |
|---|---|
| 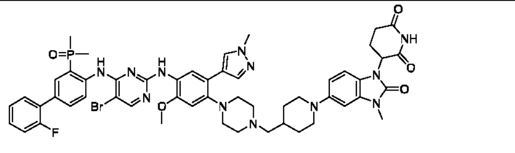 | 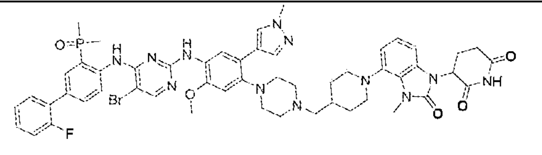 |
| 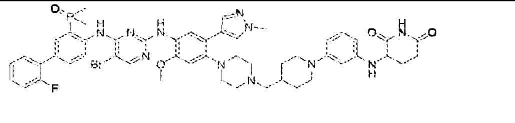 | 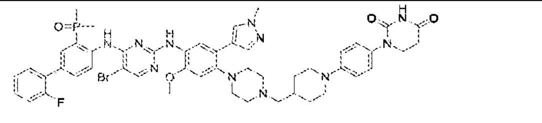 |
| 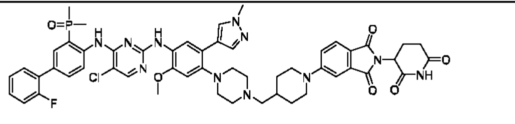 | 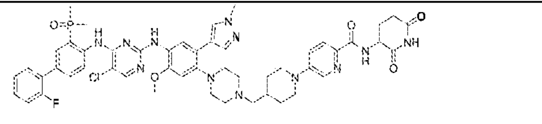 |
| 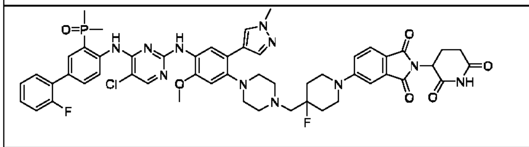 | 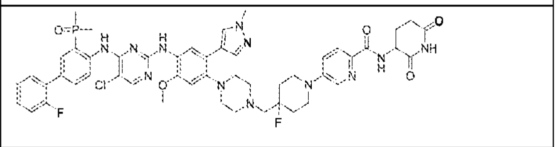 |
| 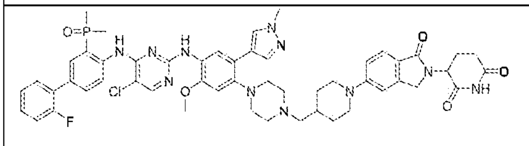 | 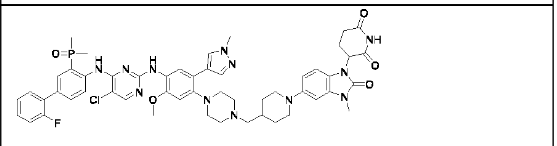 |
| 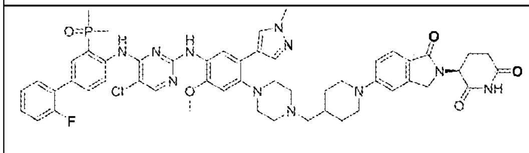 | 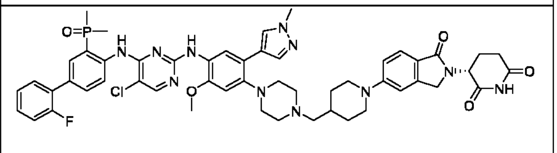 |
| 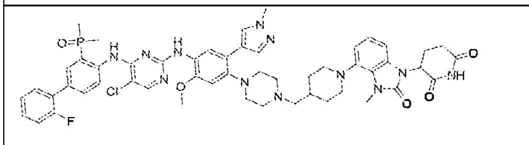 | 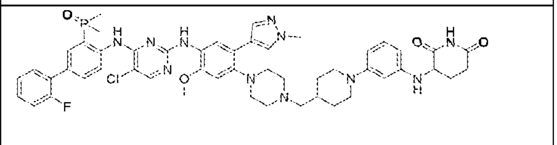 |
| 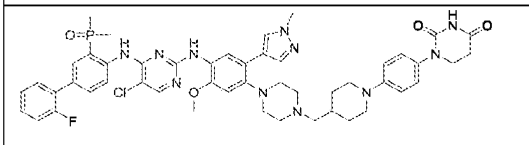 | 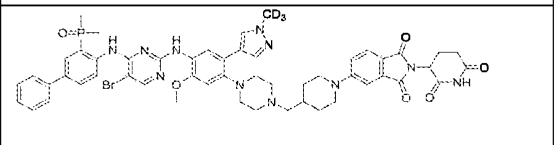 |
| 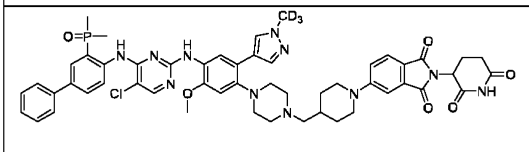 | 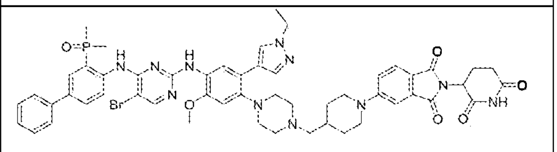 |
| 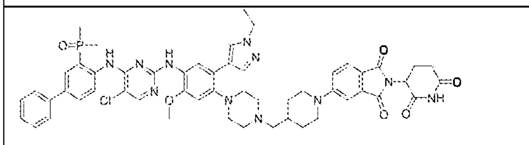 | 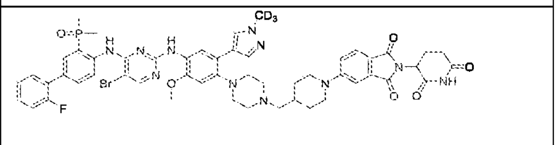 |
| 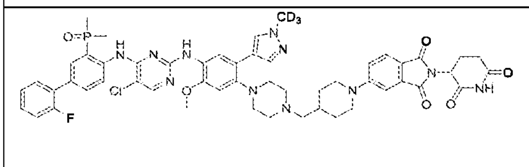 | 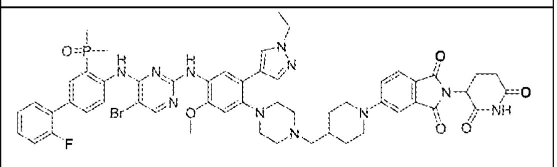 |
| 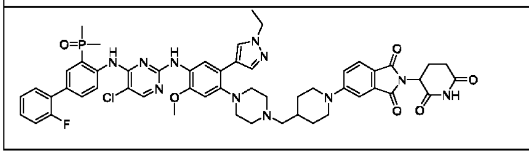 | 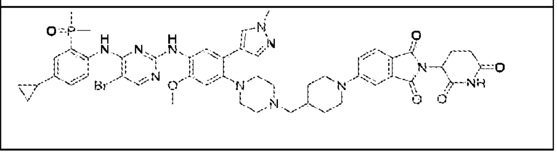 |

| | |
|---|---|
| 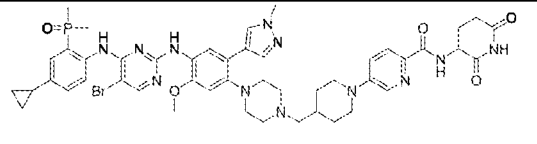 | 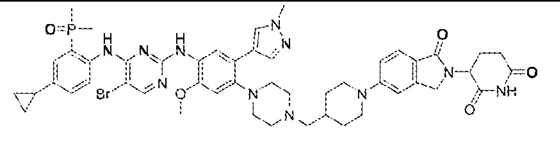 |
| 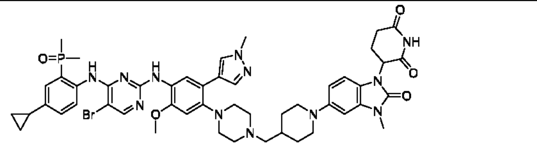 | 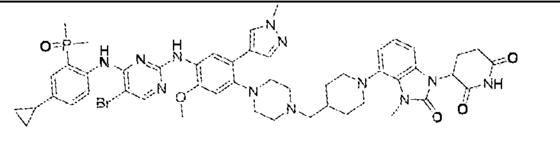 |
| 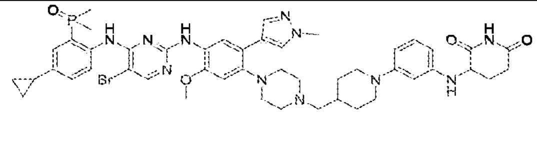 | 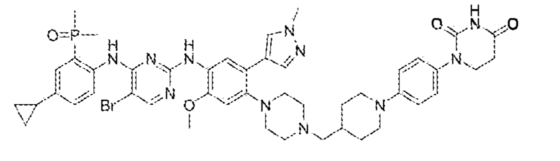 |
| 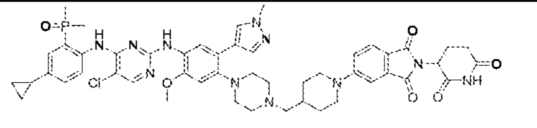 | 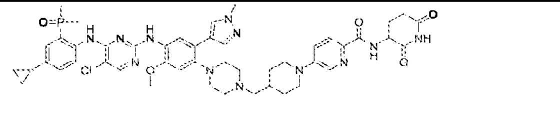 |
| 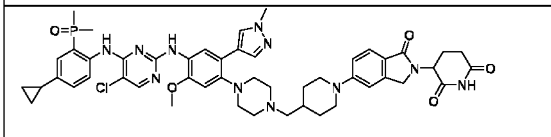 | 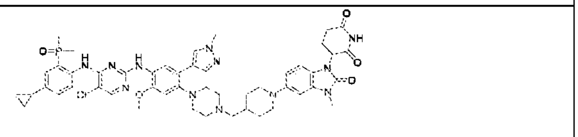 |
| 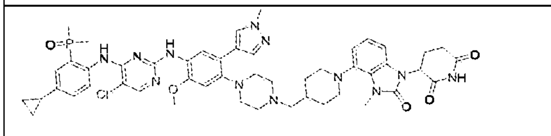 | 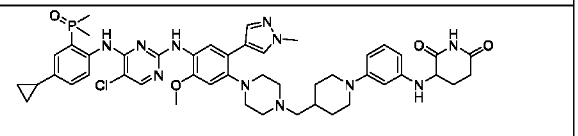 |
| 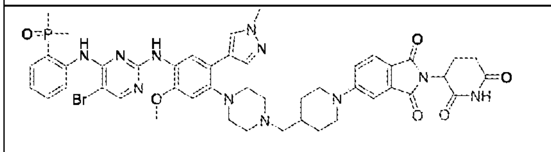 | 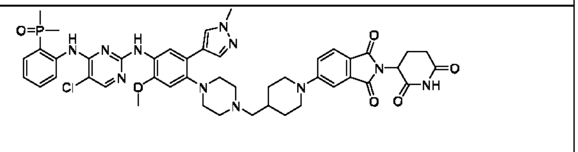 |
| 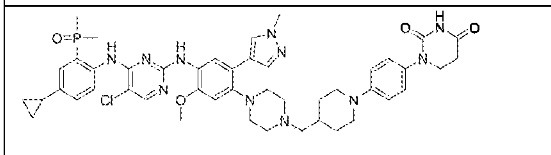 | 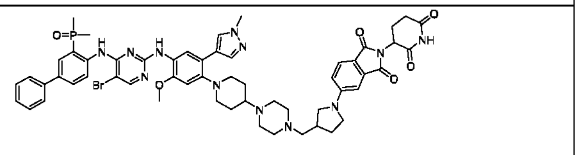 |
| 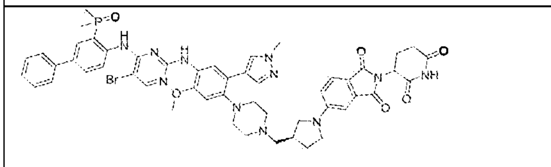 | 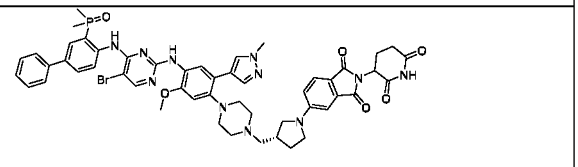 |
| 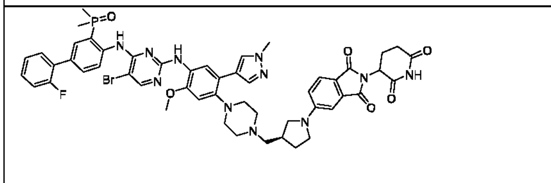 | 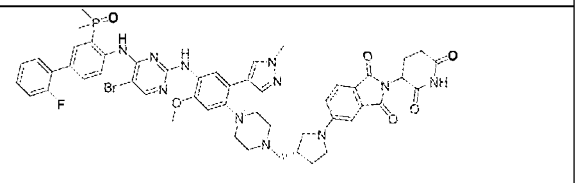 |
| 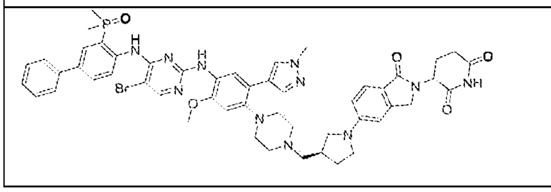 | 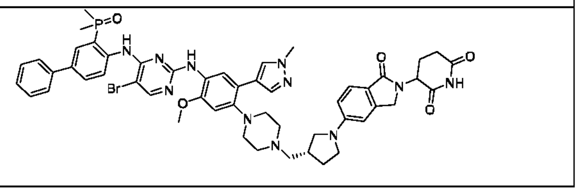 |

| | |
|---|---|
| 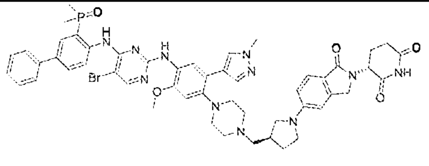 | 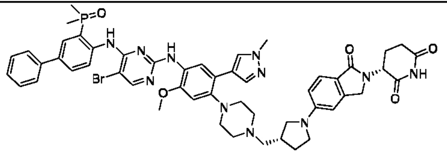 |
| 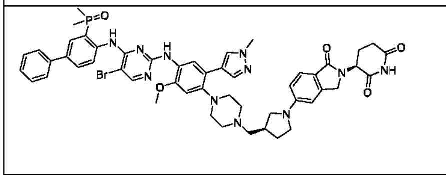 | 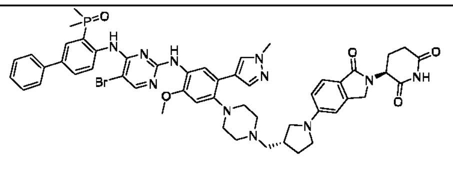 |
| 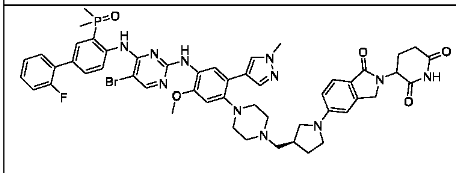 | 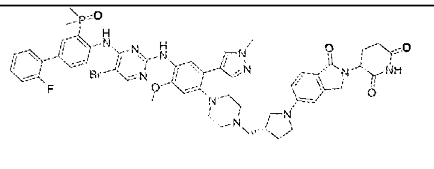 |
| 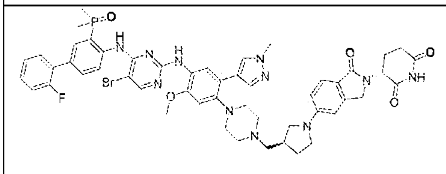 | 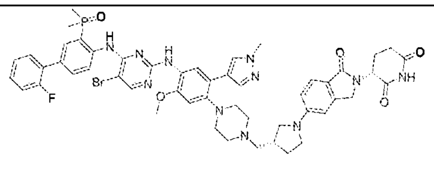 |
| 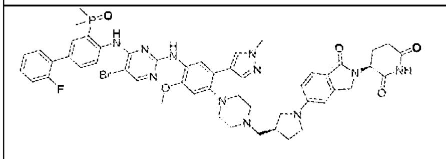 | 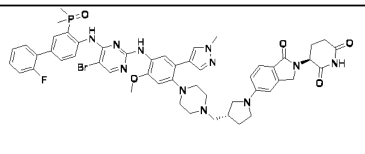 |
| 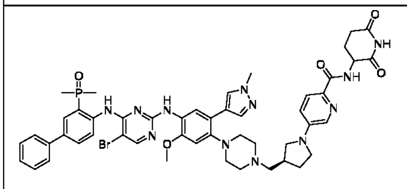 | 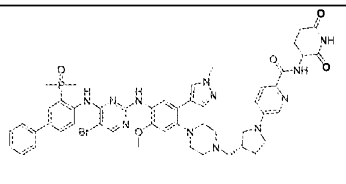 |
| 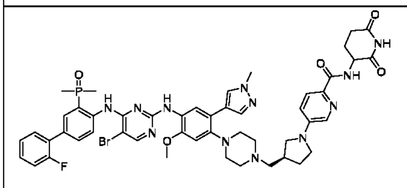 | 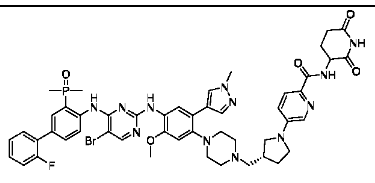 |
| 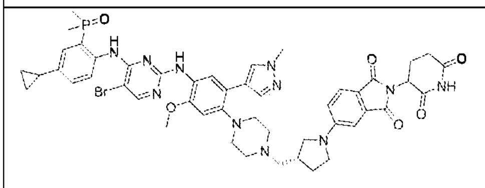 | 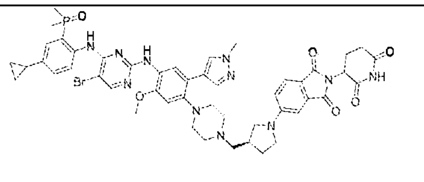 |
| 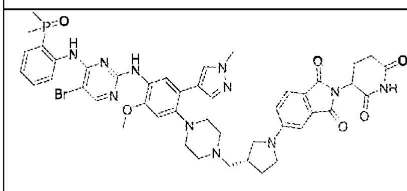 | 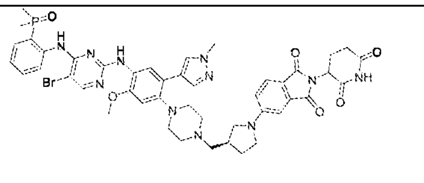 |
| 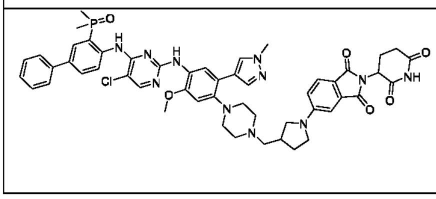 | 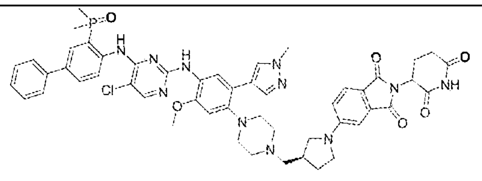 |

| | |
|---|---|
| 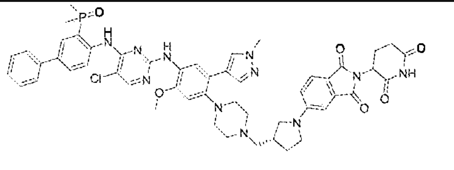 | 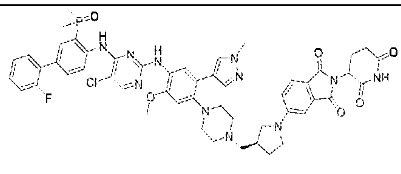 |
| 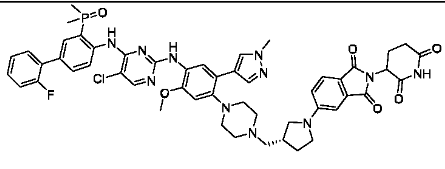 | |
| 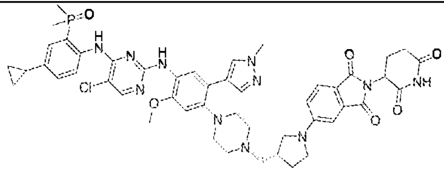 | 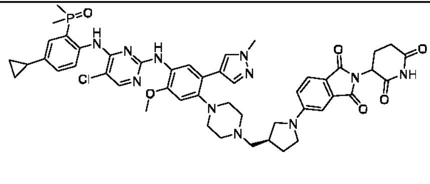 |
| 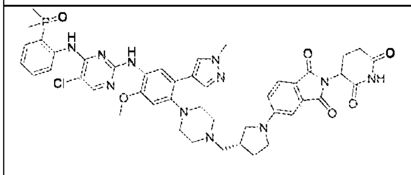 | 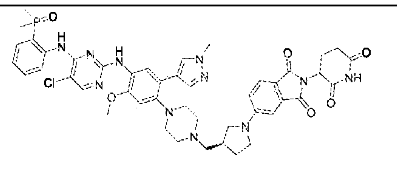 |
| | 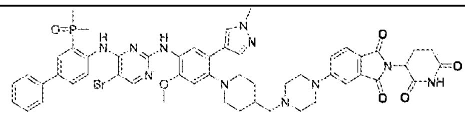 |
| 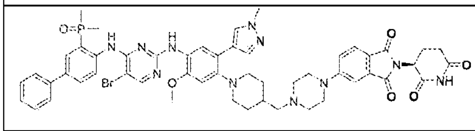 | 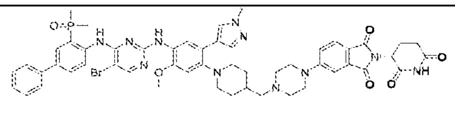 |
| 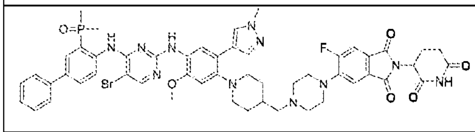 | 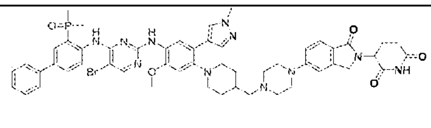 |
| 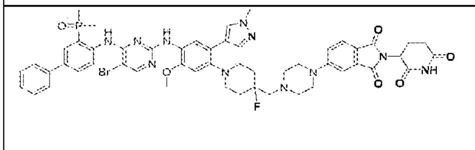 | 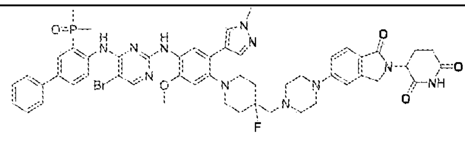 |
| 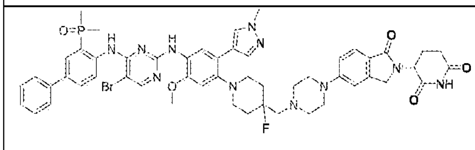 | 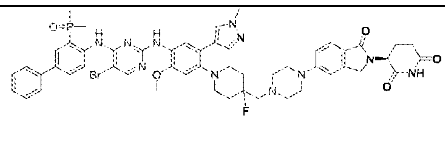 |
| 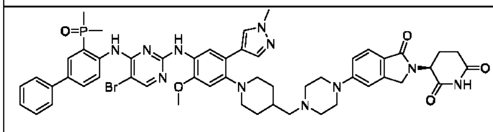 | 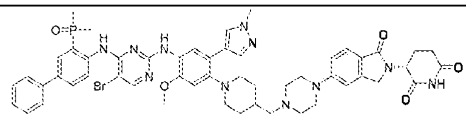 |
| 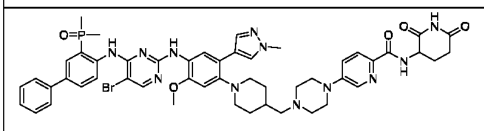 | 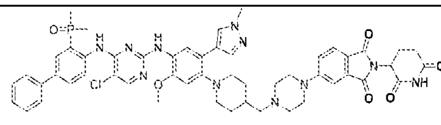 |
| 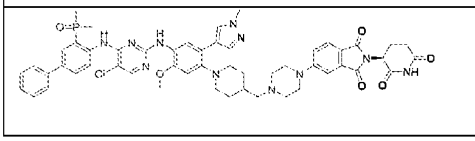 | 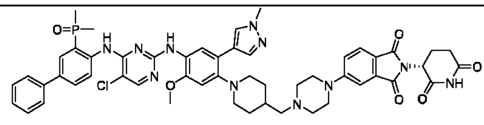 |

| | |
|---|---|
| 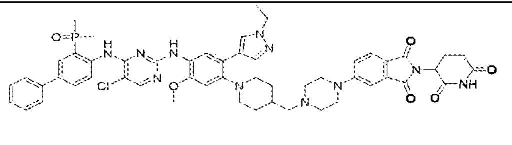 | 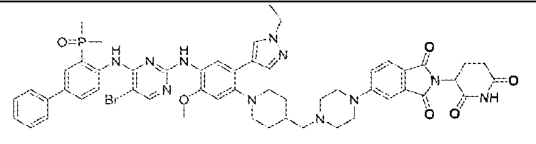 |
| 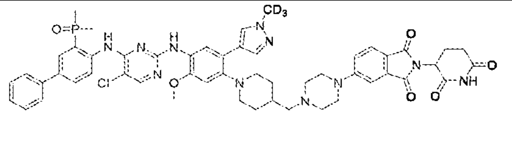 | 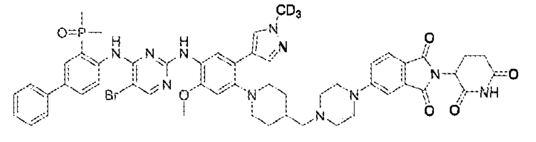 |
| 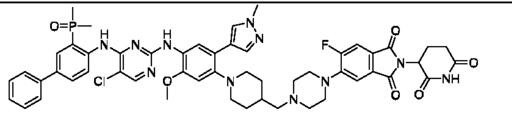 | 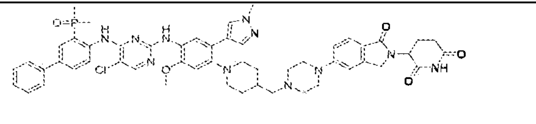 |
| 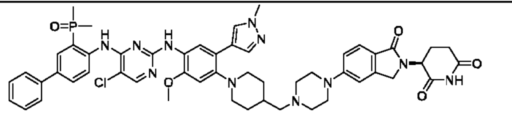 | 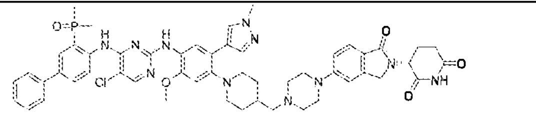 |
| 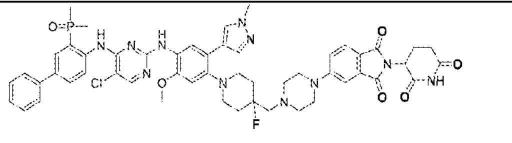 | 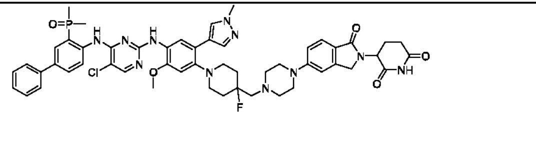 |
| 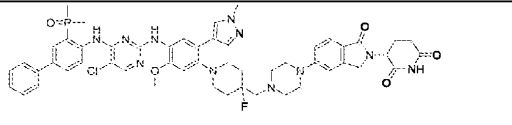 | 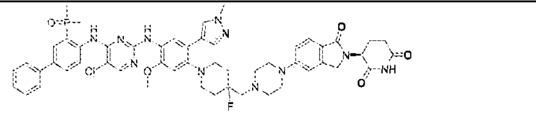 |
| 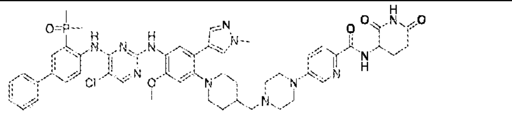 | 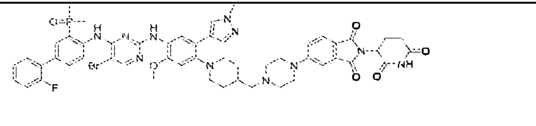 |
| 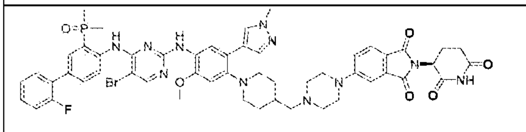 | 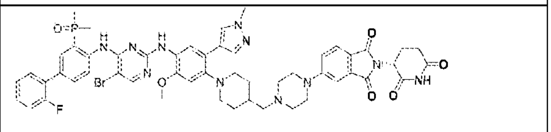 |
| 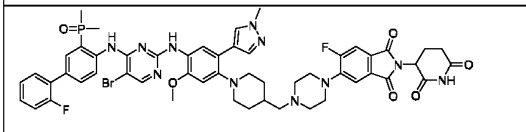 | 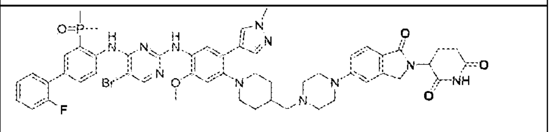 |
| 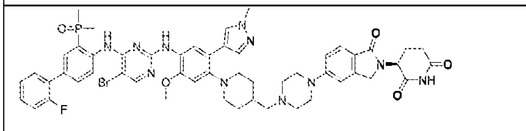 | 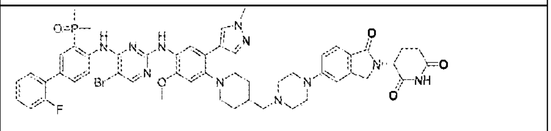 |
| 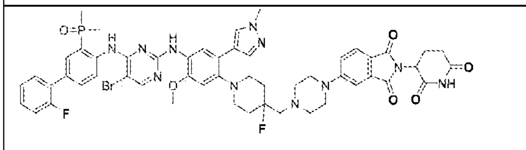 | 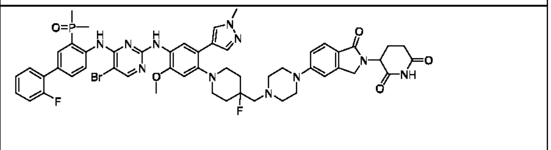 |
| 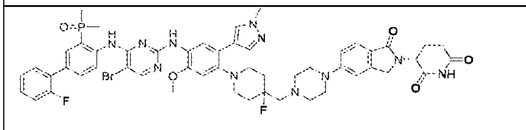 | 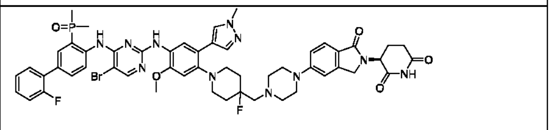 |
| 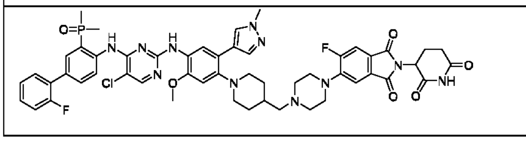 | 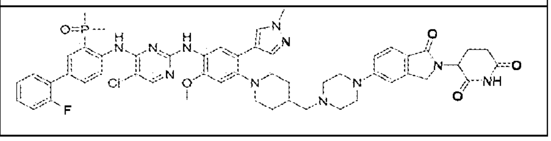 |

| | |
|---|---|
| 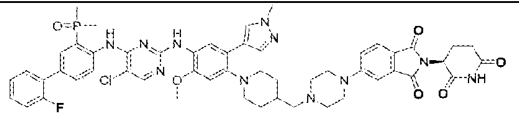 | 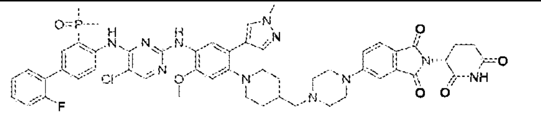 |
| 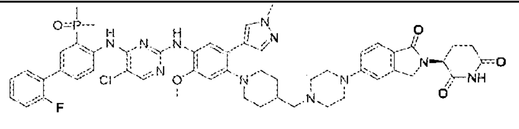 | 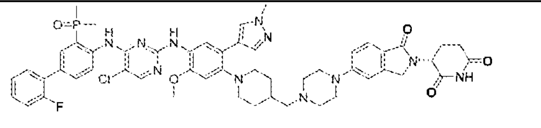 |
| 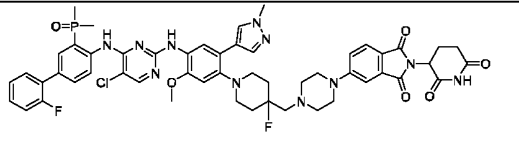 | 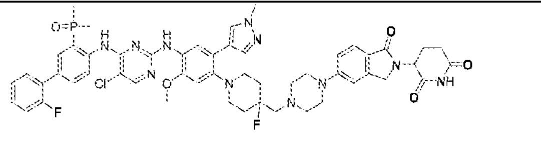 |
| 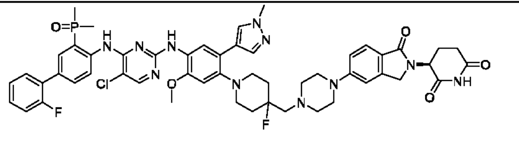 | 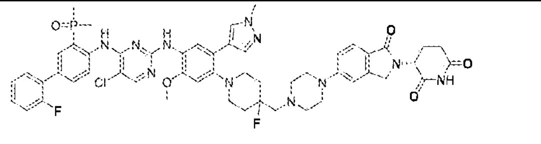 |
| 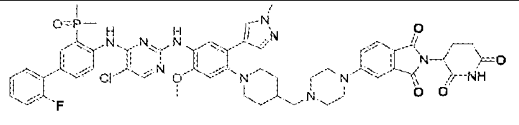 | 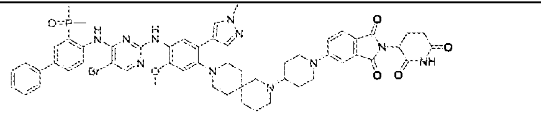 |
| 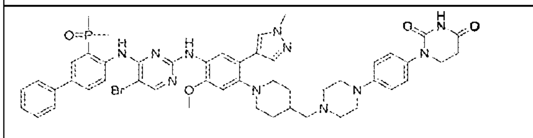 | 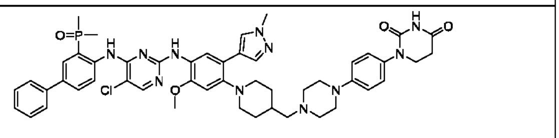 |
| 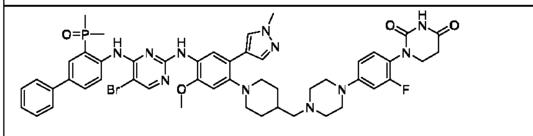 | 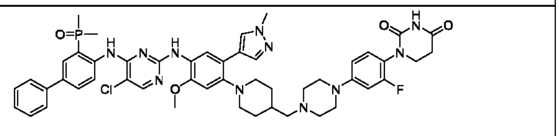 |
| 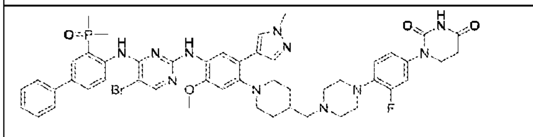 | 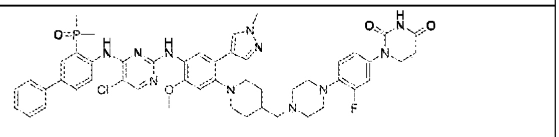 |
| 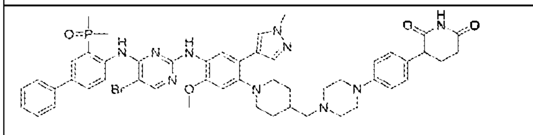 | 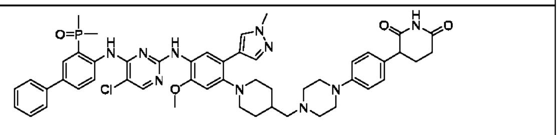 |
| 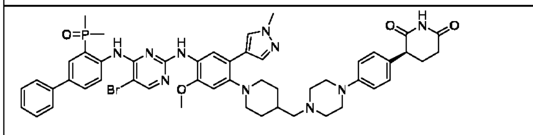 | 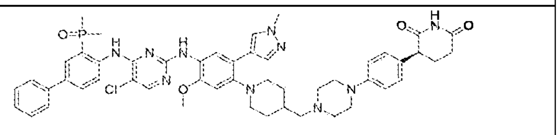 |
| 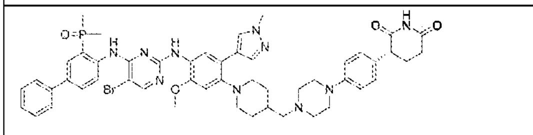 | 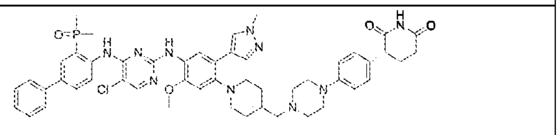 |
| 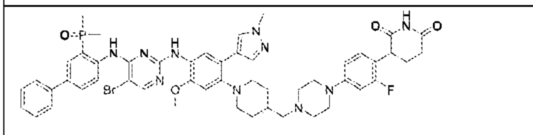 | 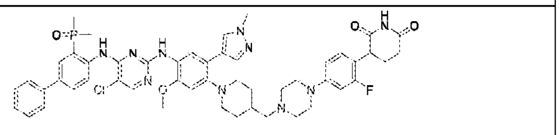 |
| 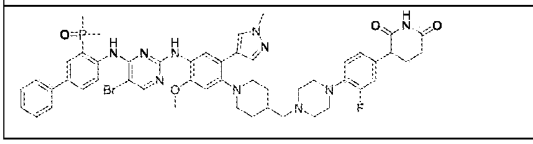 | 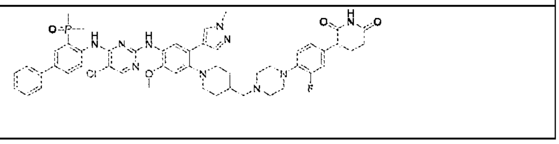 |

| | |
|---|---|
| 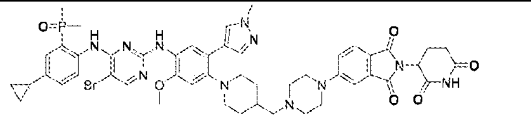 | 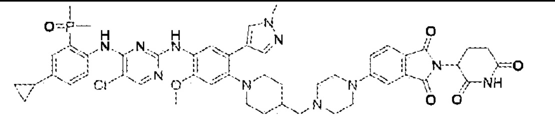 |
| 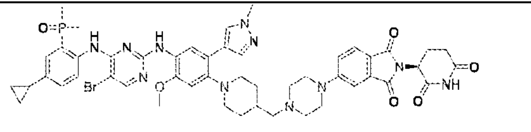 | 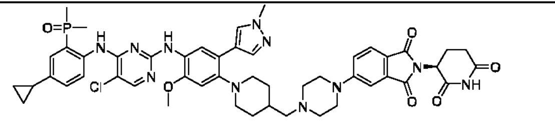 |
| 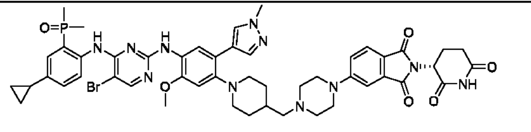 | 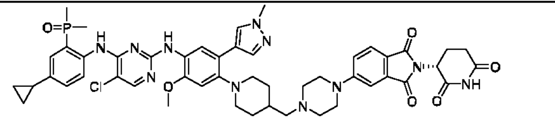 |
| 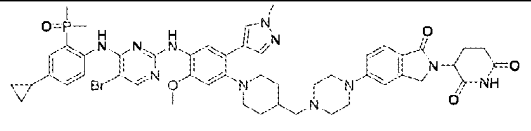 | 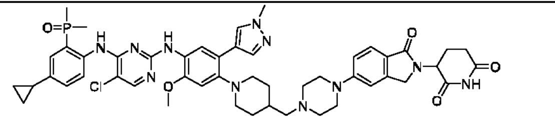 |
| 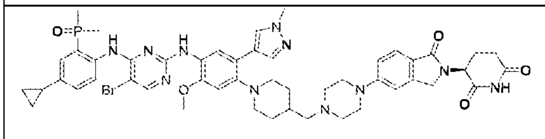 | 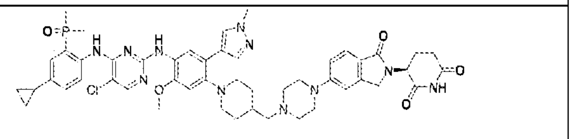 |
| 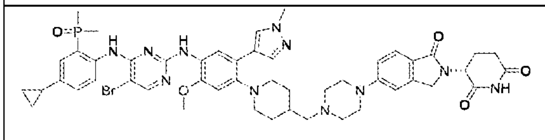 | 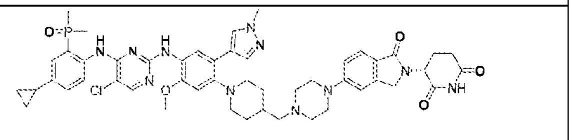 |
| 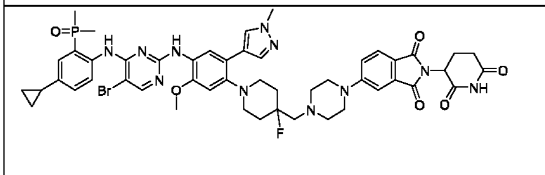 | 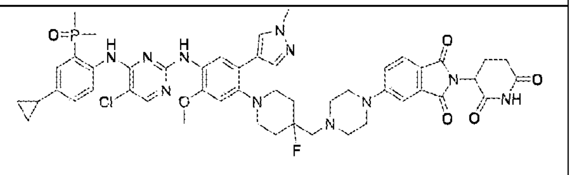 |
| 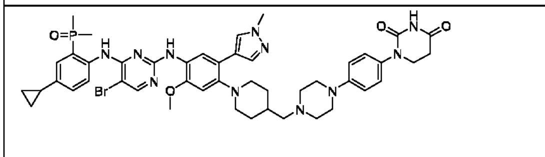 | 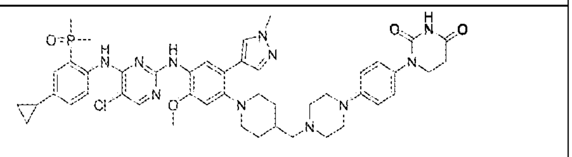 |
| 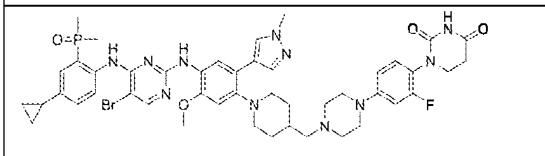 | 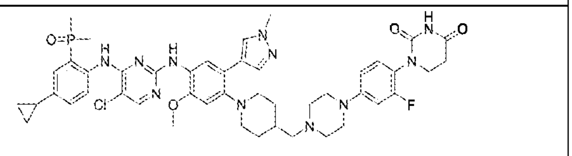 |
| 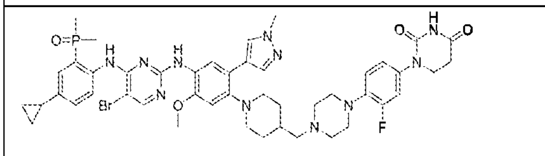 | 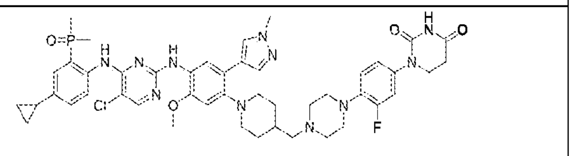 |
| 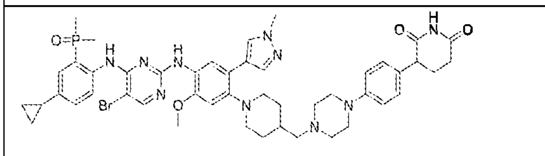 | 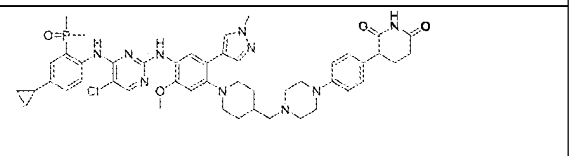 |

| | |
|---|---|
| 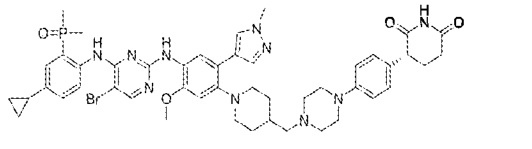 | 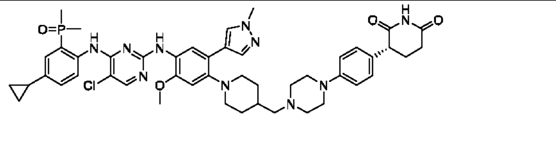 |
| 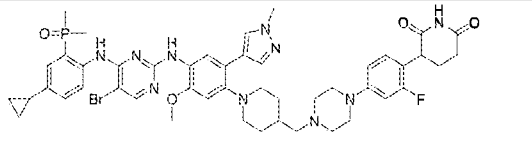 | 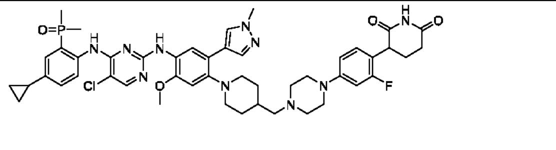 |
| 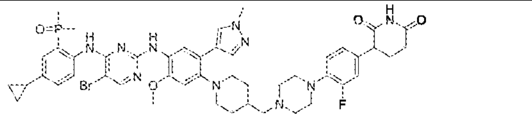 | 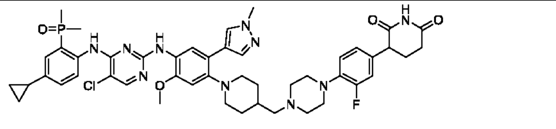 |
| 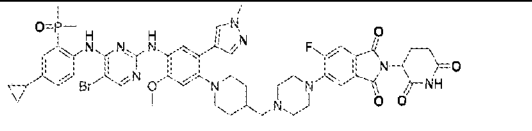 | 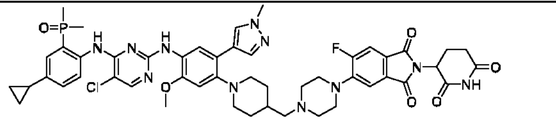 |
| 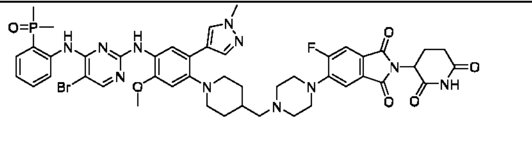 | 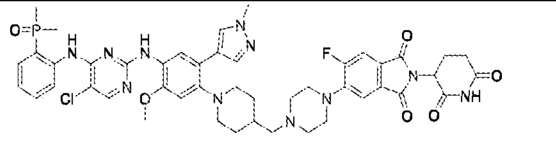 |
| 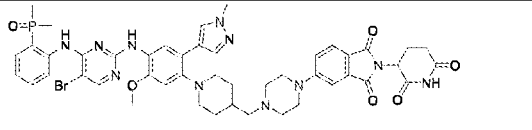 | 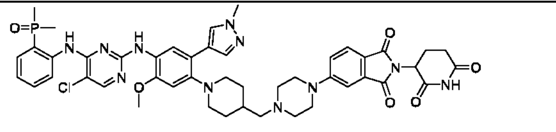 |
| 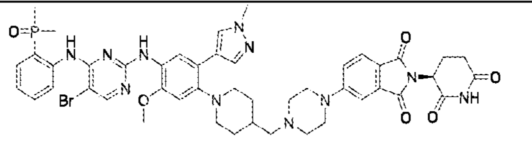 | 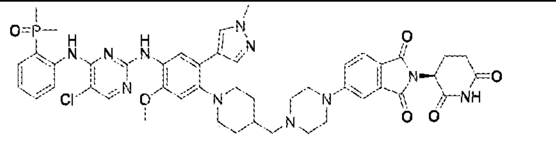 |
| 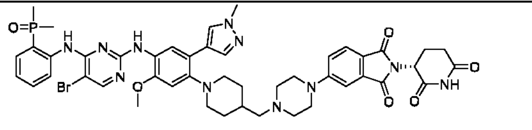 | 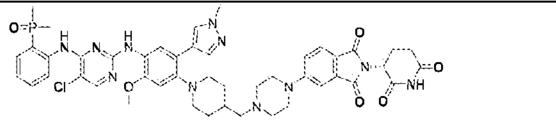 |
| 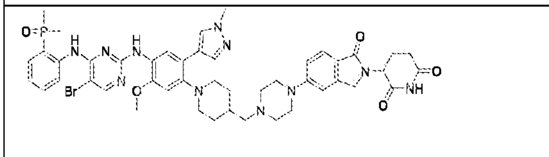 | 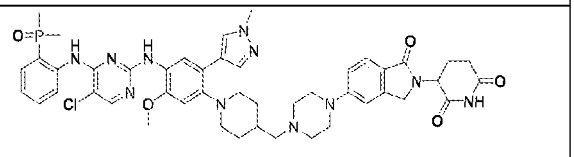 |
| 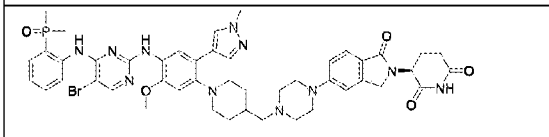 | 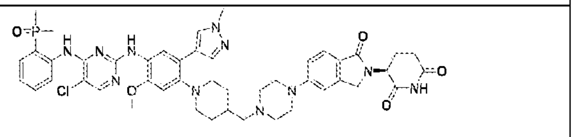 |
| 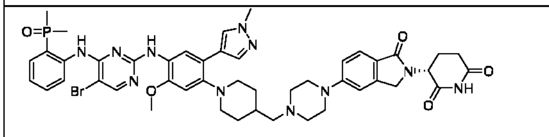 | 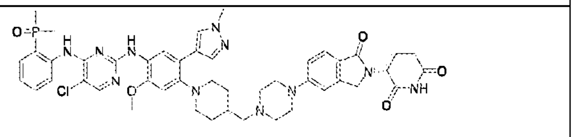 |
| 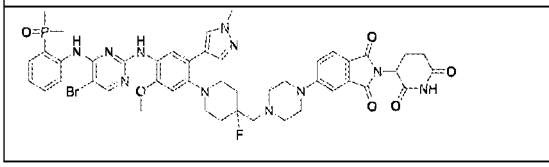 | 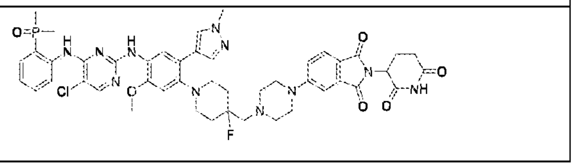 |

| | |
|---|---|
| 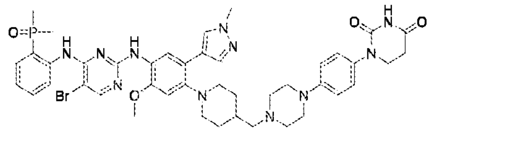 | 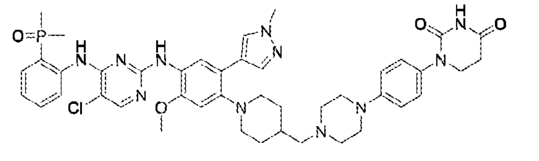 |
| 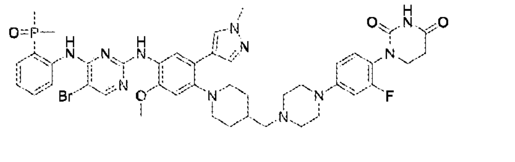 | 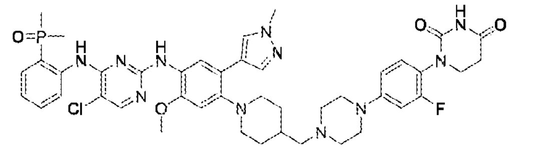 |
| 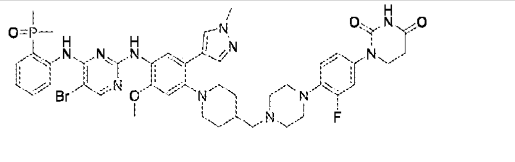 | 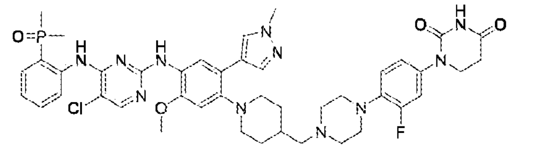 |
| 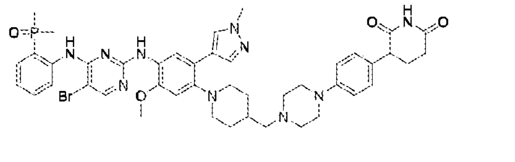 | 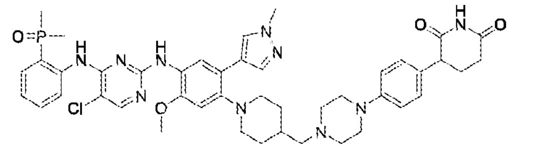 |
| 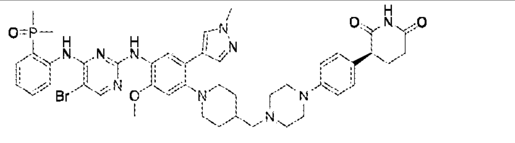 | 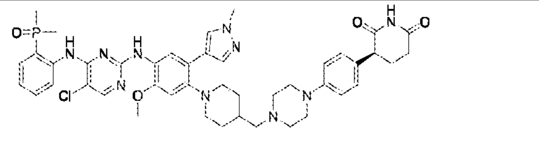 |
| 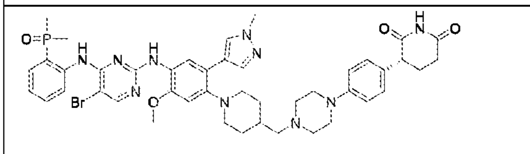 | 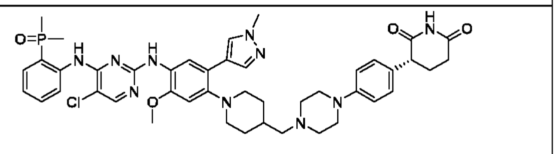 |
| 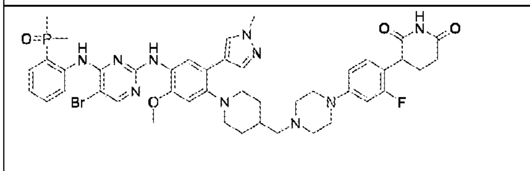 | 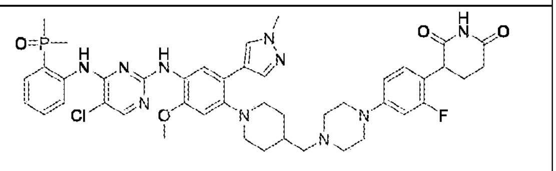 |
| 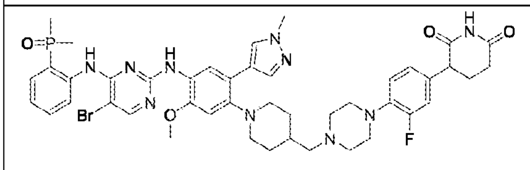 | 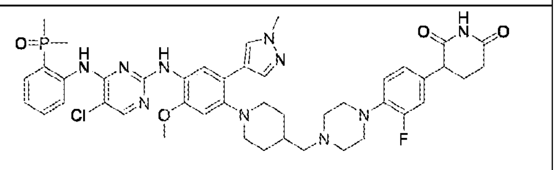 |
| 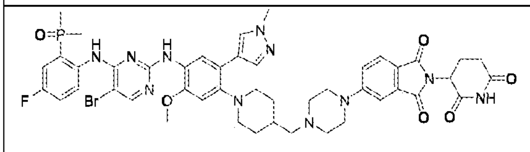 | 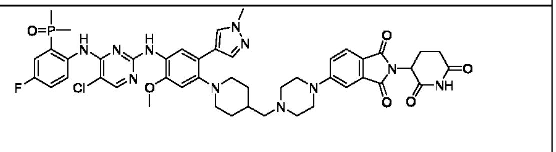 |
| 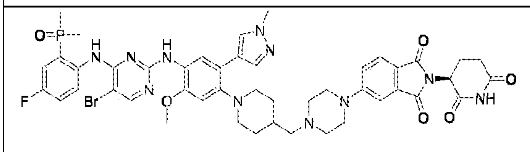 | 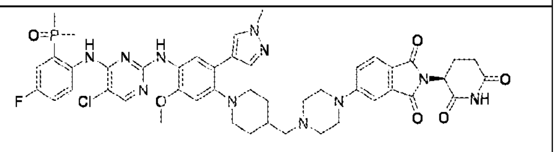 |
| 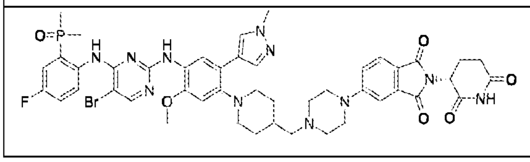 | 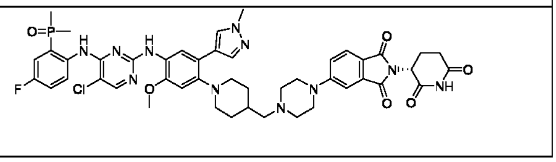 |

| | |
|---|---|
| 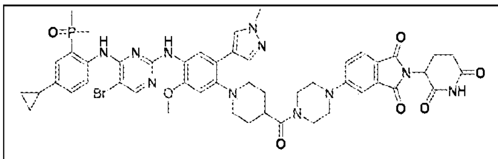 | 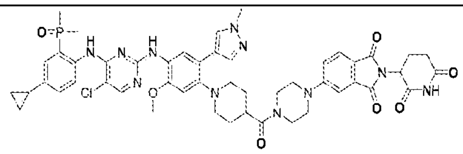 |
| 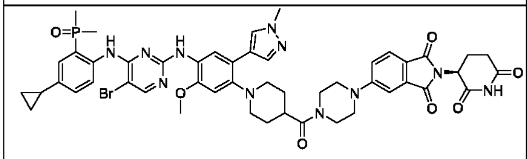 | 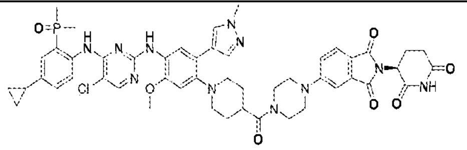 |
| 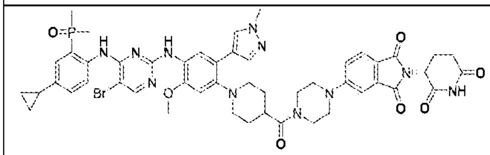 | 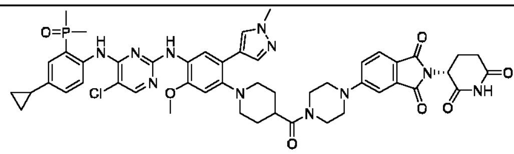 |
| 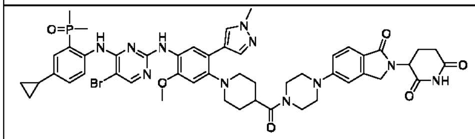 | 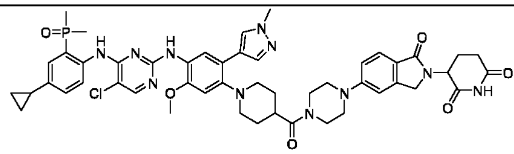 |
| 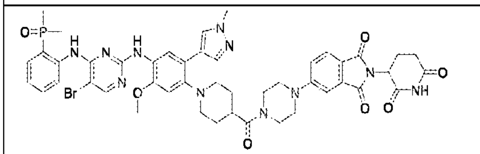 | 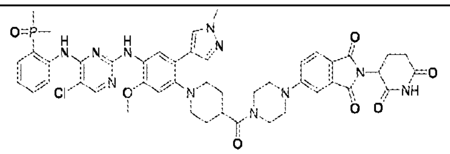 |
| 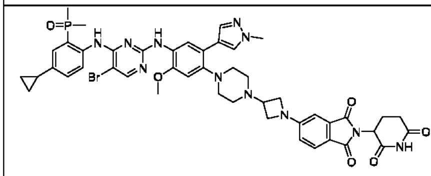 | 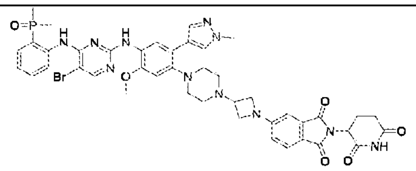 |
| 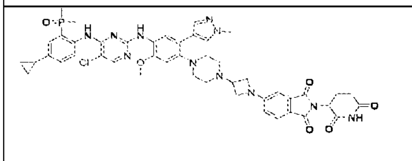 | 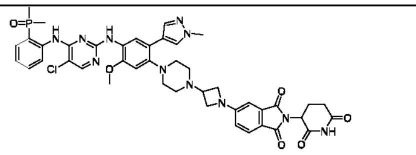 |
| 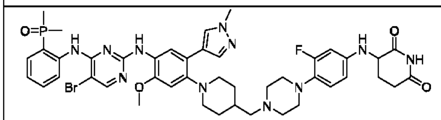 | 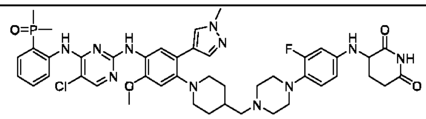 |
| 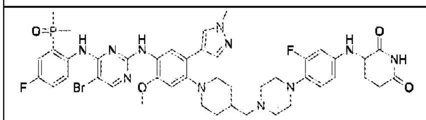 | 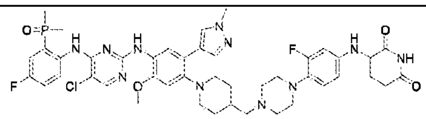 |
| 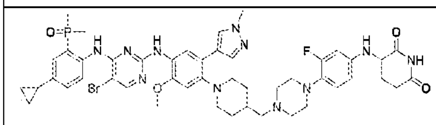 | 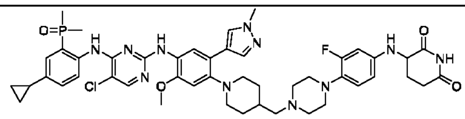 |
| 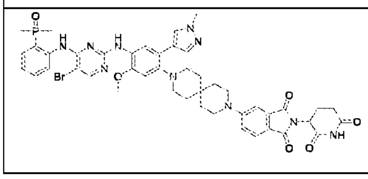 | 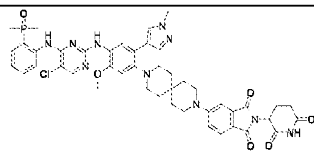 |

| | |
|---|---|
| 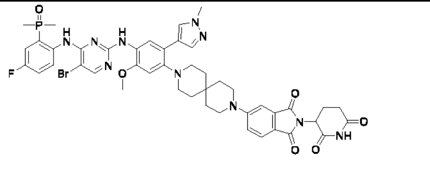 | 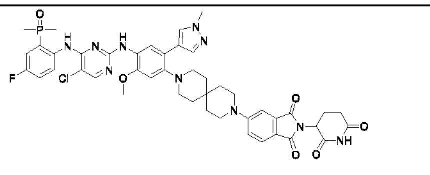 |
| 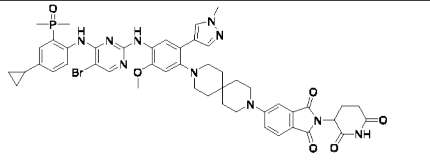 | 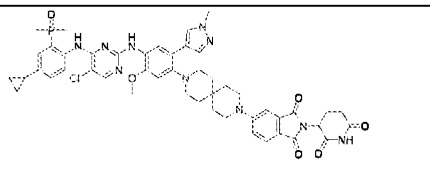 |
| 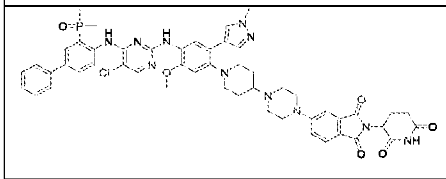 | 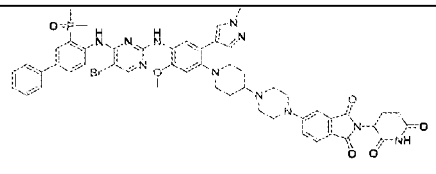 |
| 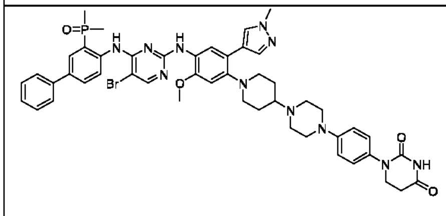 | 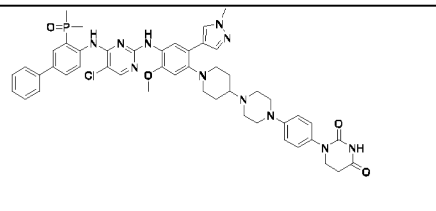 |
| 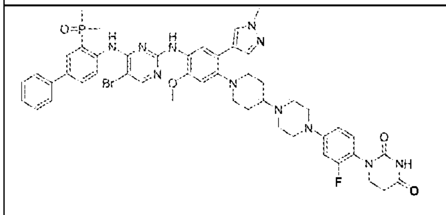 | 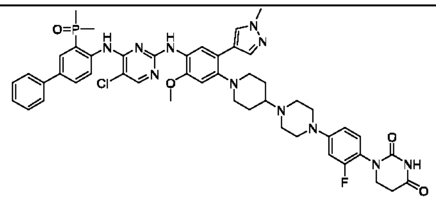 |
| 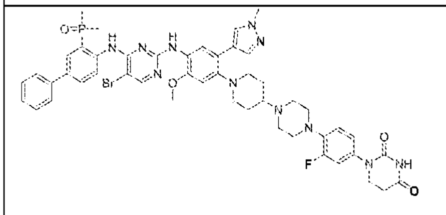 | 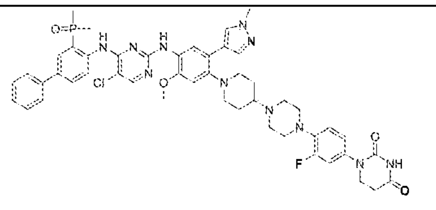 |
| 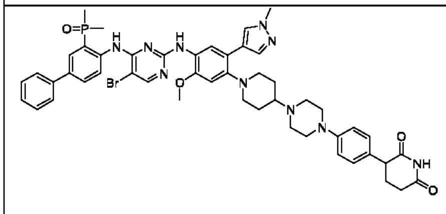 | 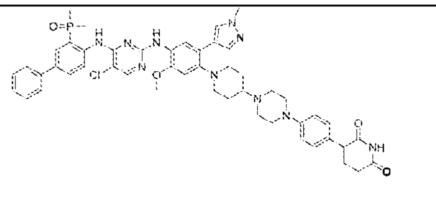 |
| 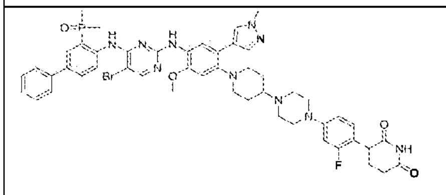 | 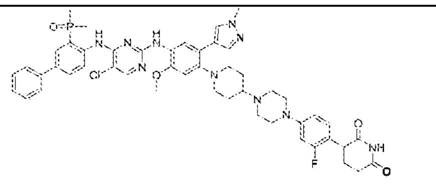 |
| 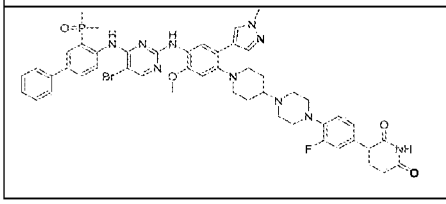 | 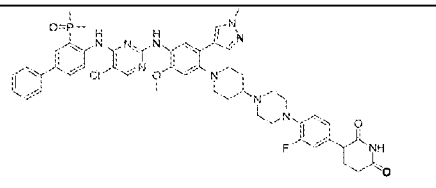 |

| | |
|---|---|
| 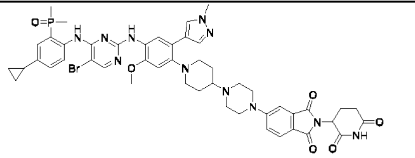 | 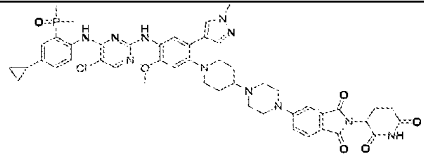 |
| 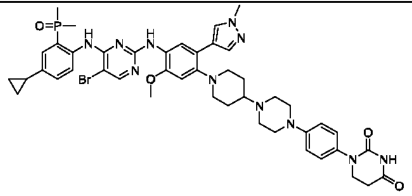 | 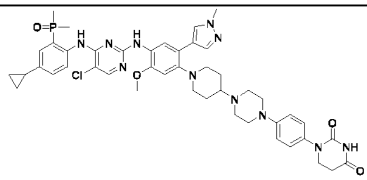 |
| 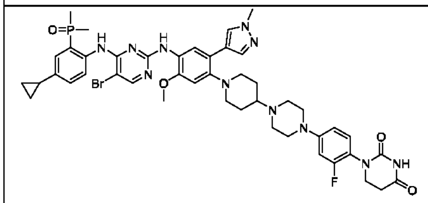 | 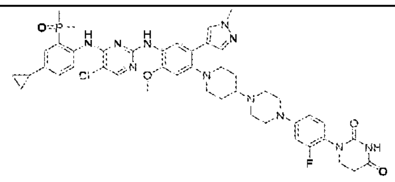 |
| 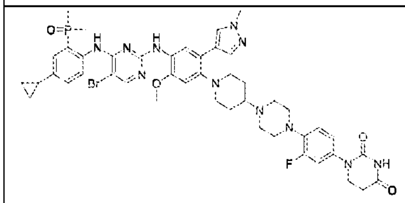 | 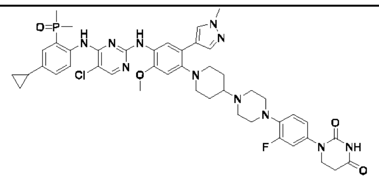 |
| 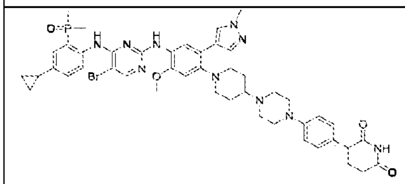 | 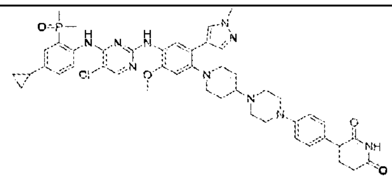 |
| 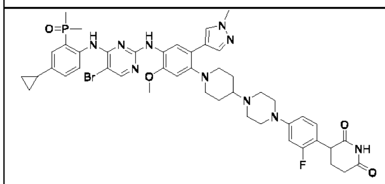 | 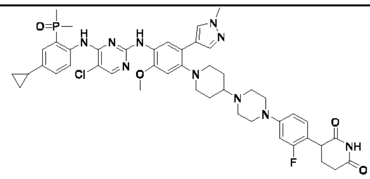 |
| 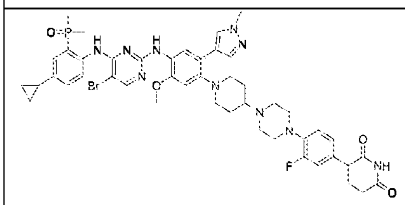 | 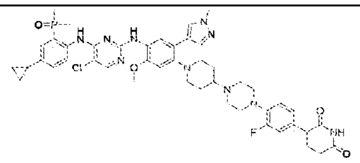 |
| 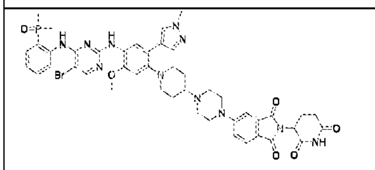 | 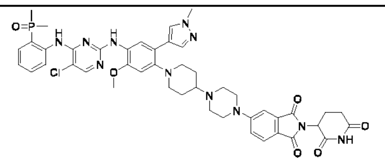 |
| 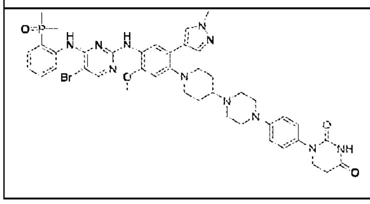 | 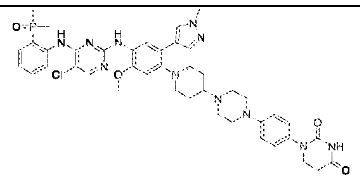 |

| | |
|---|---|
| 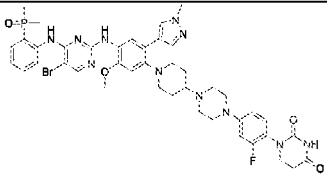 | 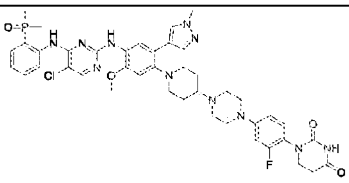 |
| 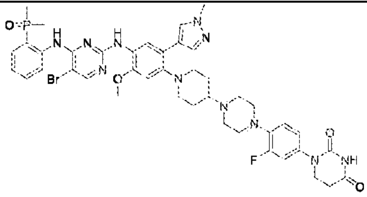 | 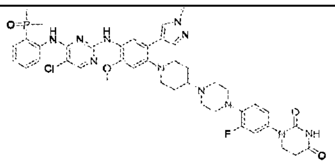 |
| 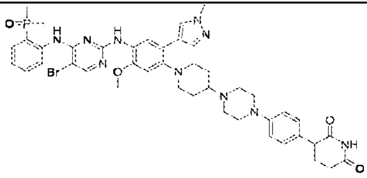 | 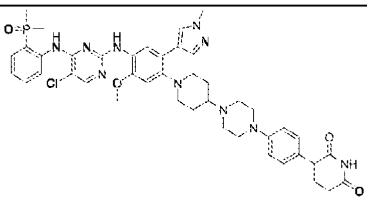 |
| 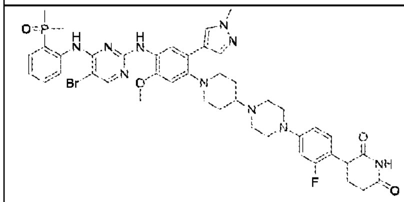 | 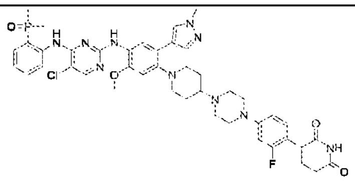 |
| 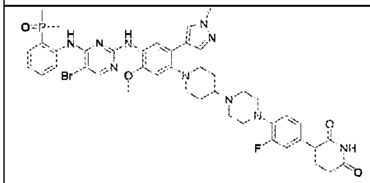 | 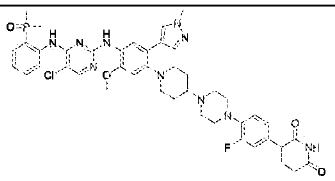 |
| 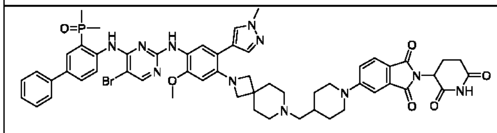 | 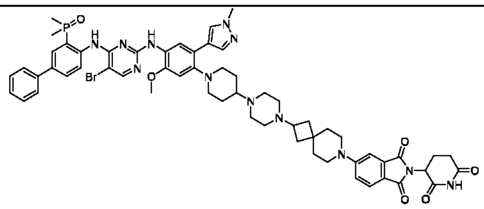 |
| 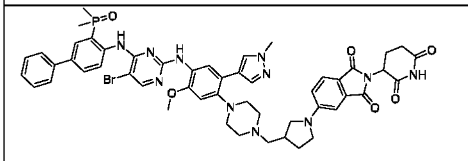 | 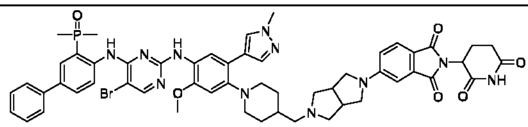 |
| 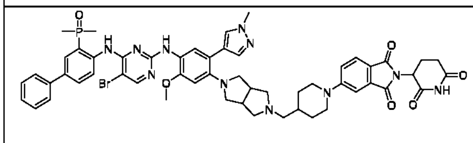 | 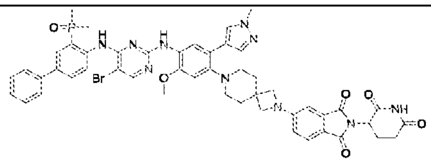 |
| 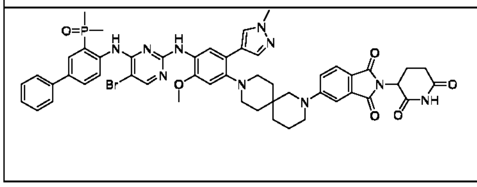 | 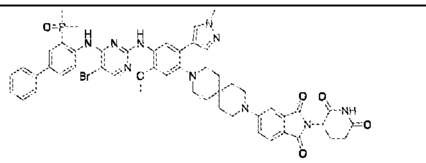 |

| | |
|---|---|
| 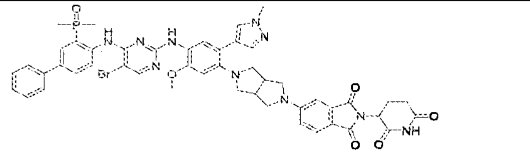 | 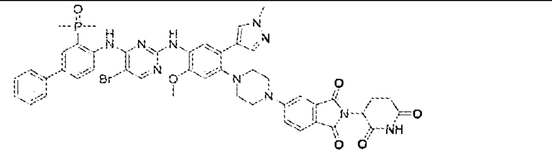 |
| 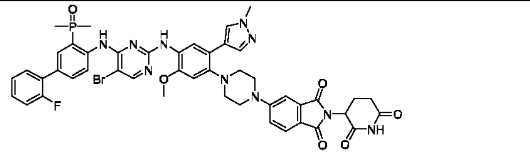 | 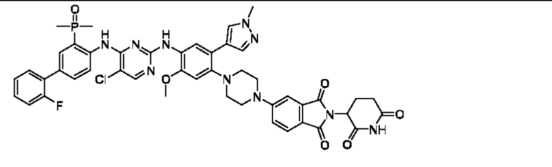 |
| 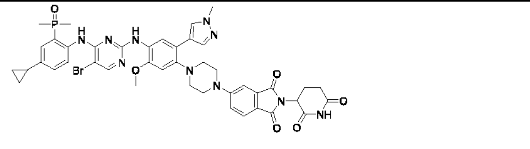 | 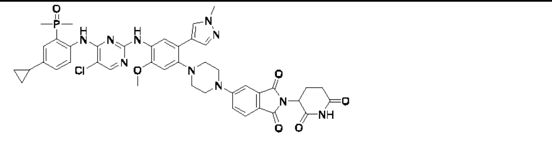 |
| 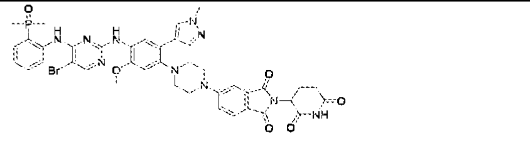 | 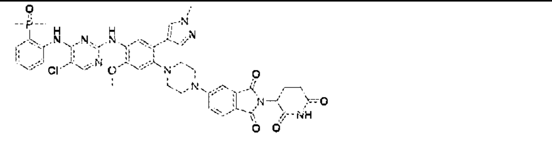 |
| 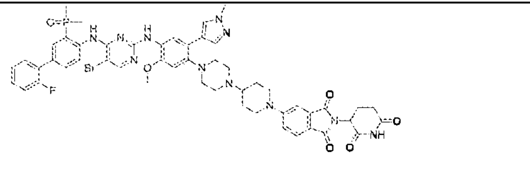 | 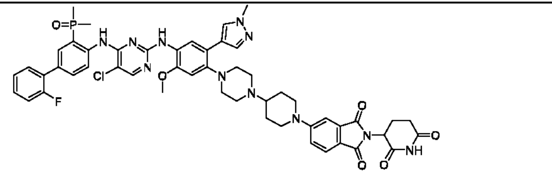 |
| 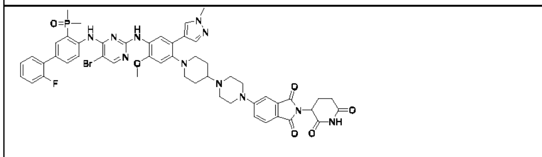 | 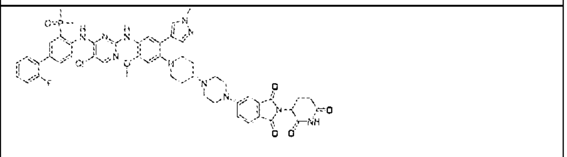 |
| 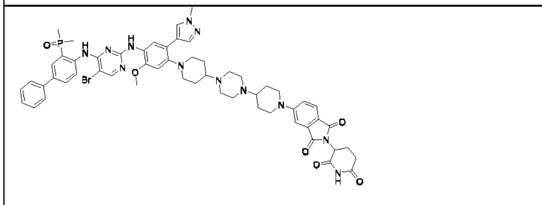 | 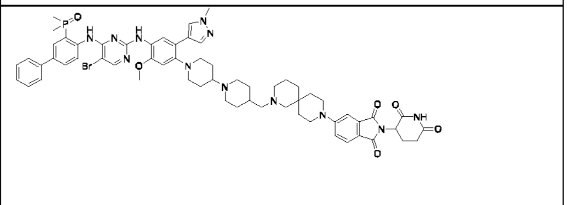 |
| 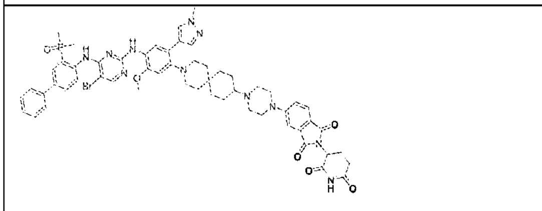 | 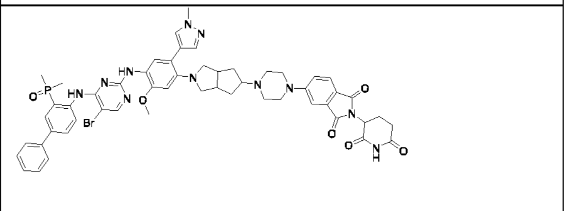 |
| 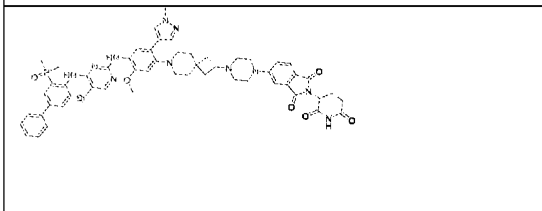 | 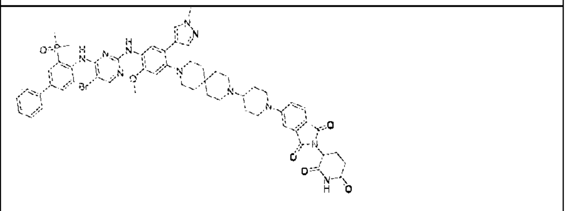 |
| 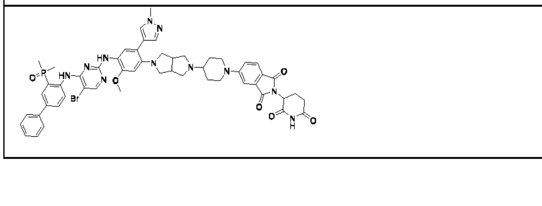 | 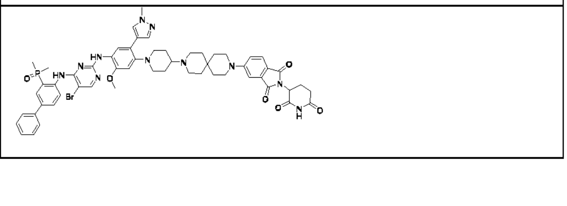 |

| | |
|---|---|
| 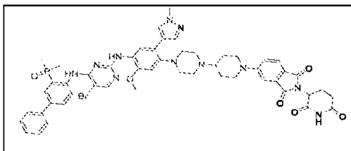 | 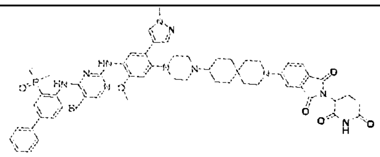 |
| 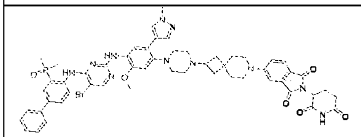 | 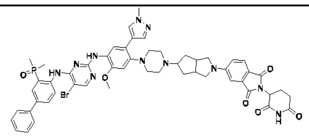 |
| 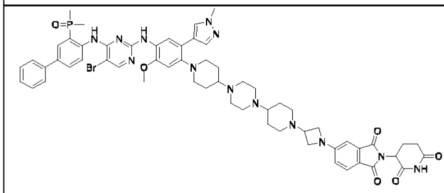 | 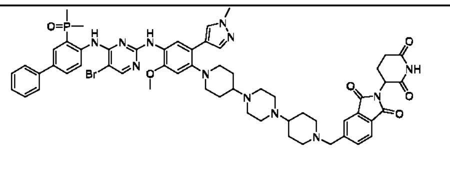 |
| 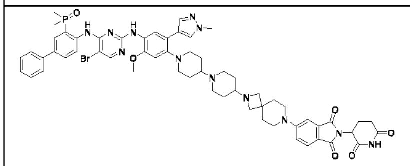 | 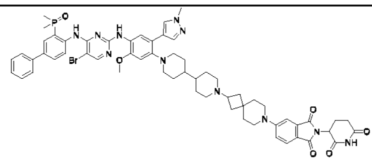 |
| 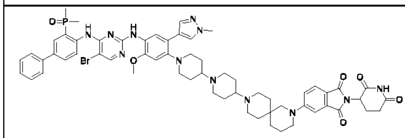 | 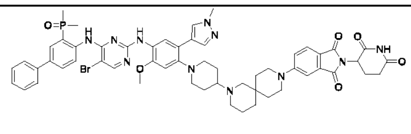 |
| 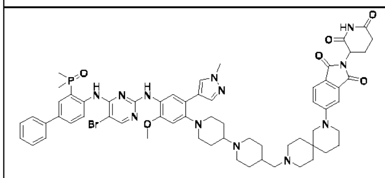 | 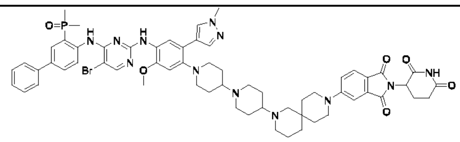 |
| 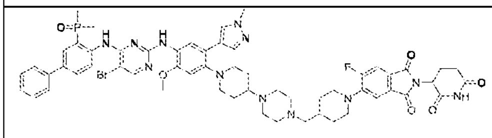 | 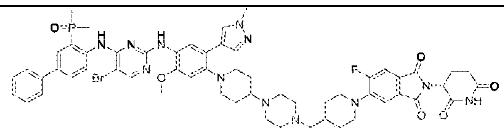 |
| 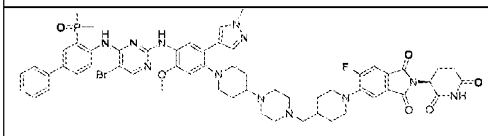 | 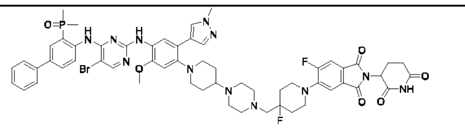 |
| 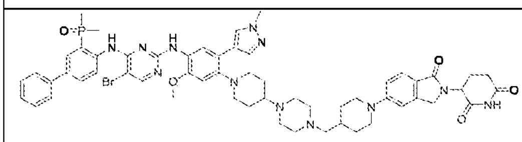 | 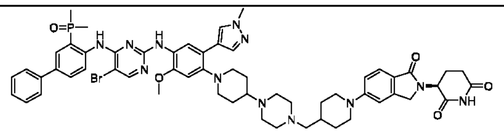 |
| 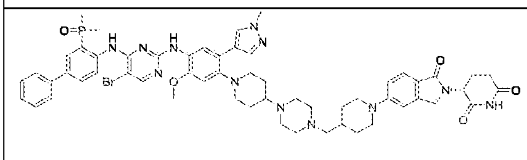 | 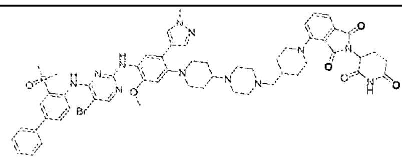 |
| 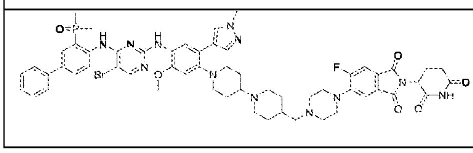 | 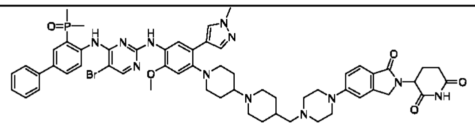 |

| | |
|---|---|
| 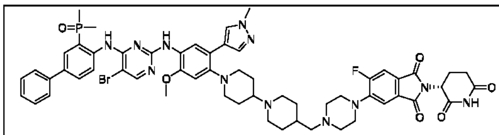 | 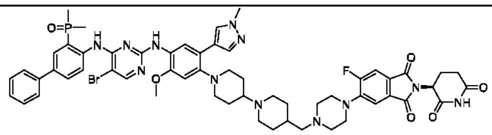 |
| 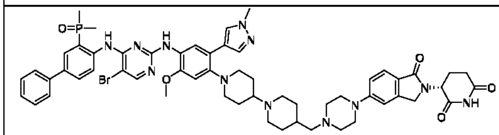 | 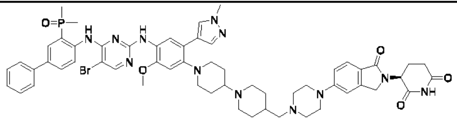 |
| 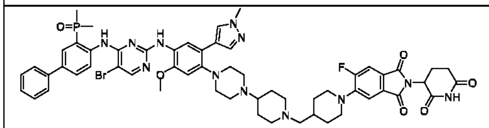 | 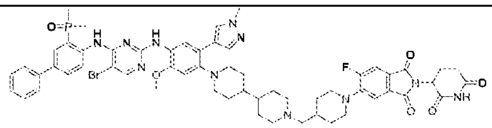 |
| 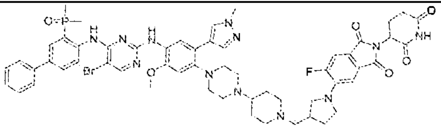 | 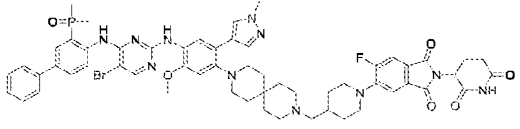 |
| 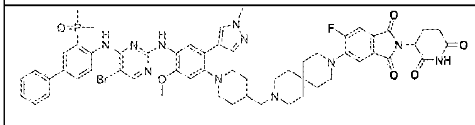 | 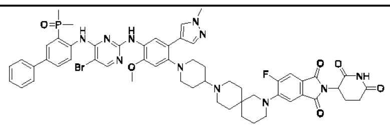 |
| 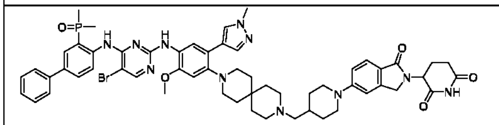 | 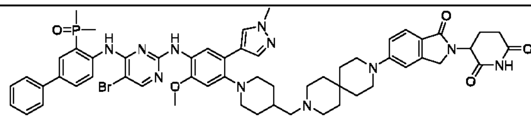 |
| 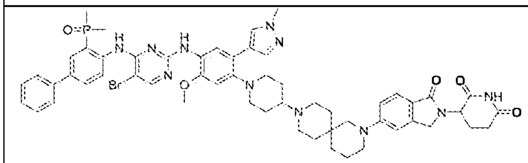 | 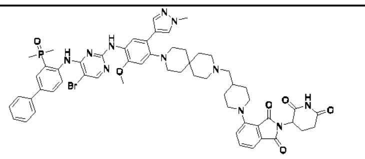 |
| 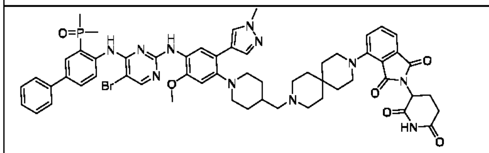 | 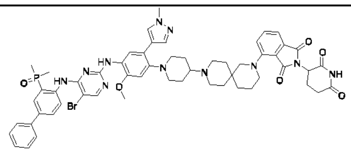 |
| 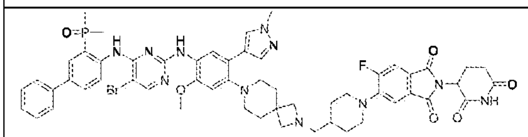 | 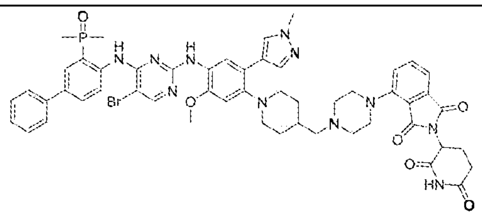 |
| 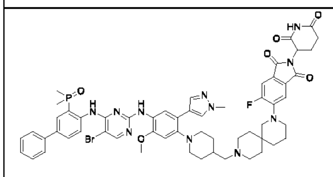 | 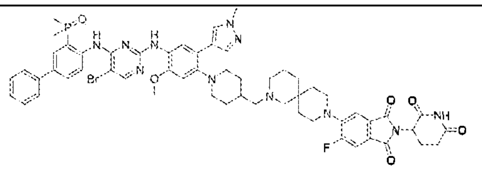 |
| 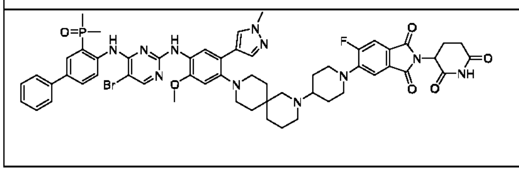 | 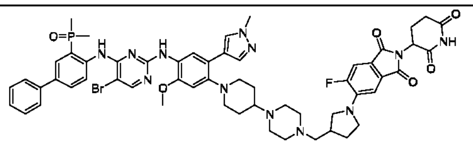 |

| | |
|---|---|
| 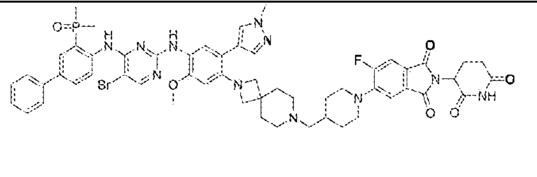 | 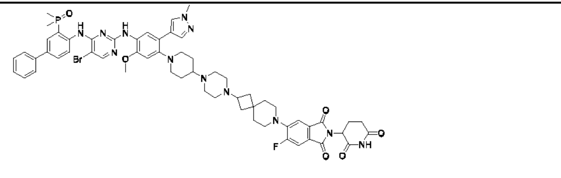 |
| 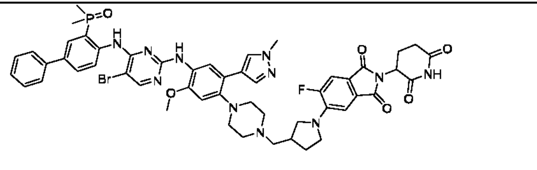 | 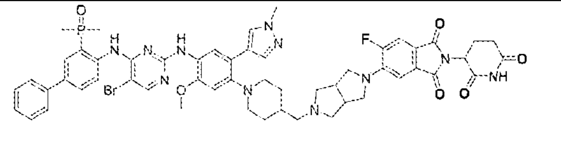 |
| 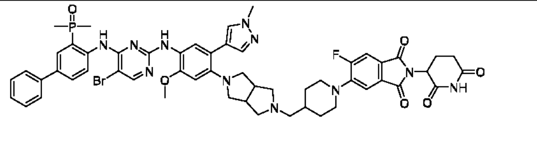 | 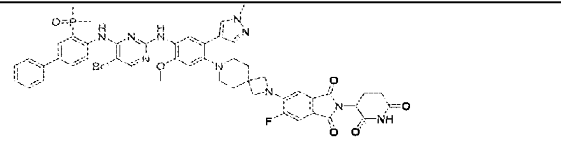 |
| 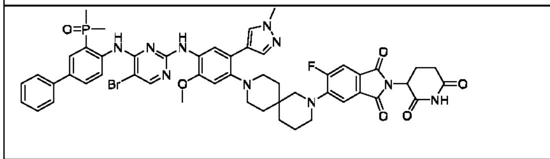 | 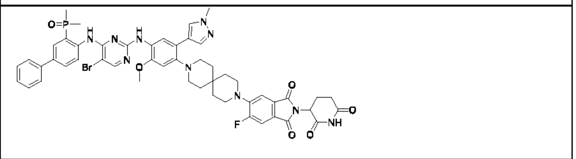 |
| 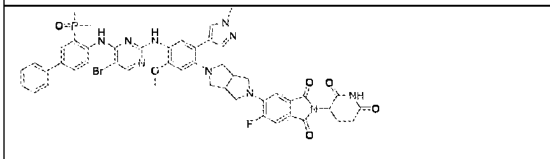 | 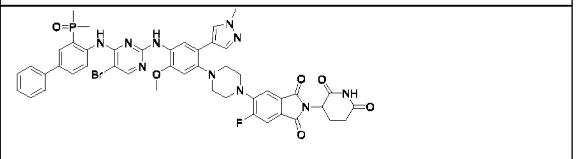 |
| 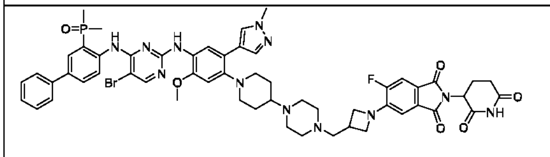 | 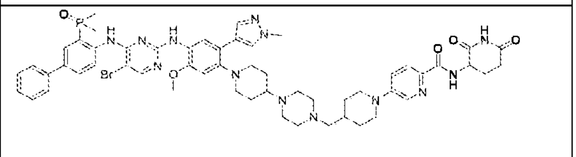 |
| 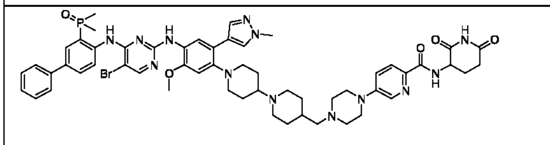 | 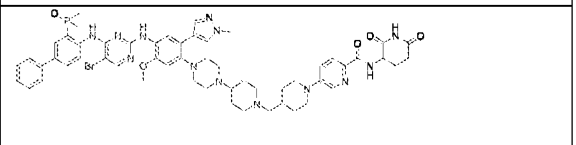 |
| 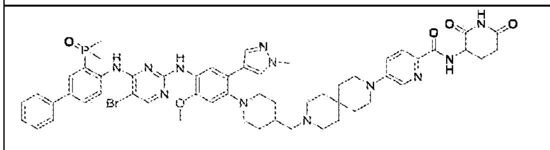 | 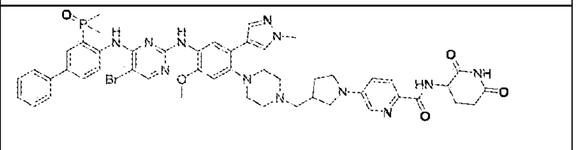 |
| 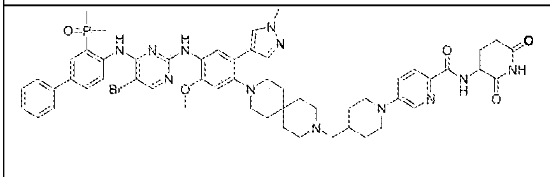 | 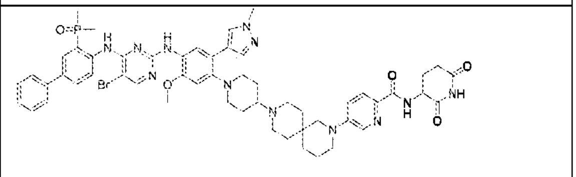 |
| 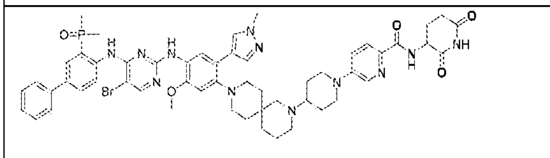 | 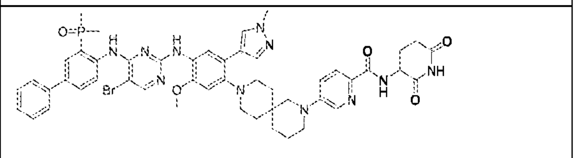 |
| 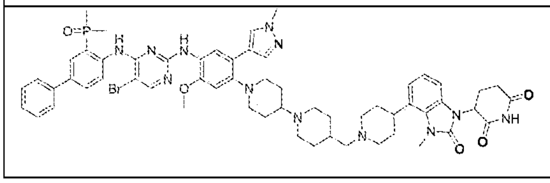 | 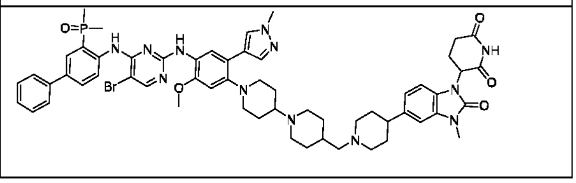 |

| | |
|---|---|
| 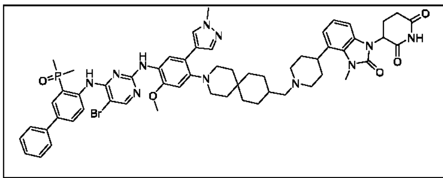 | 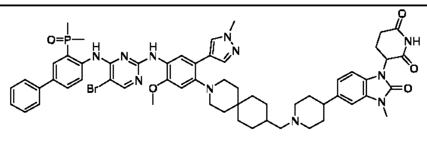 |
| 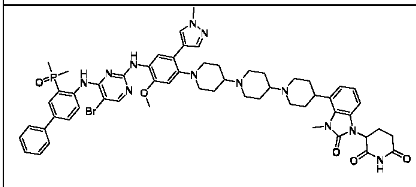 | 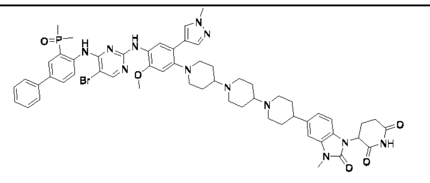 |
| 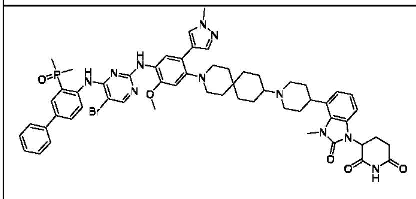 | 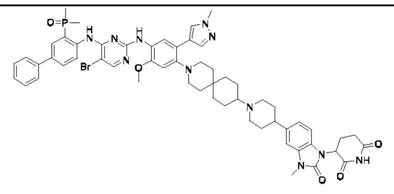 |
| 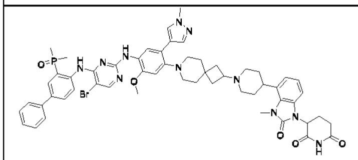 | 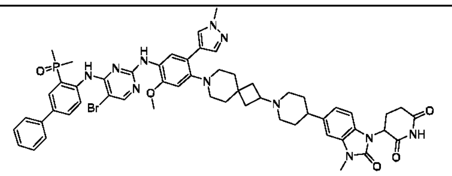 |
| 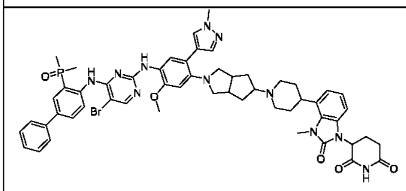 | 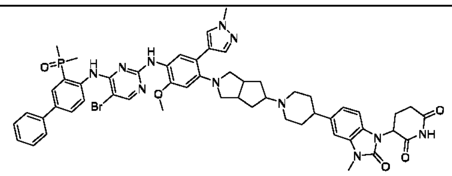 |
| 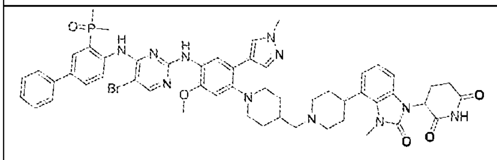 | 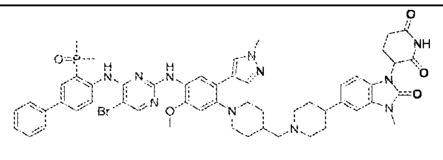 |
| 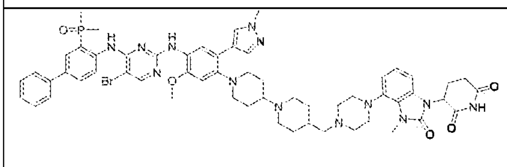 | 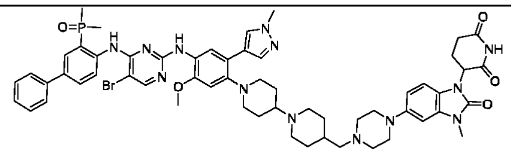 |
| 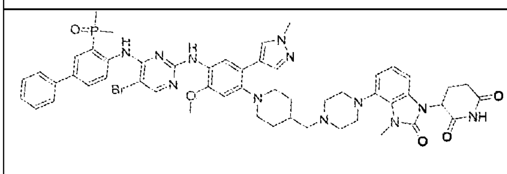 | 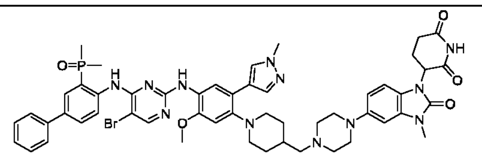 |
| 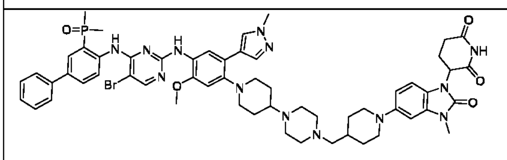 | 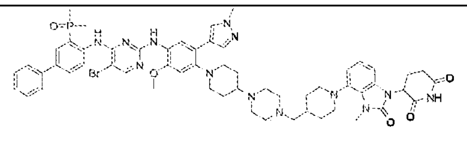 |
| 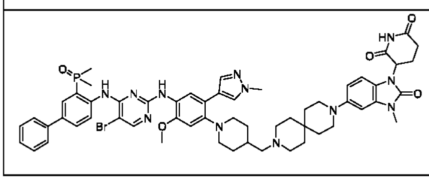 | 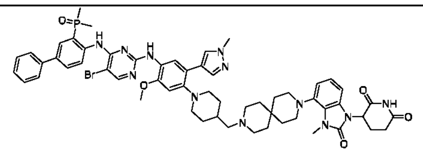 |

| | |
|---|---|
| 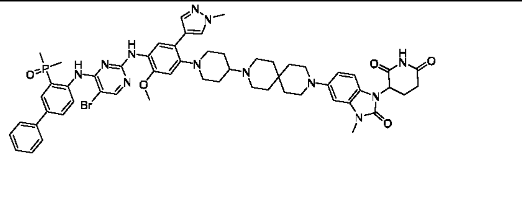 | 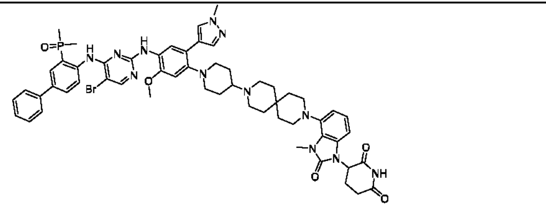 |
| 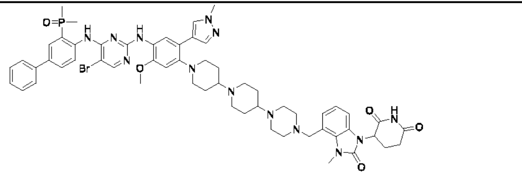 | 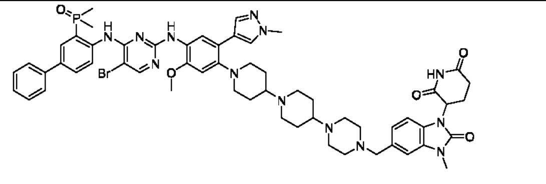 |
| 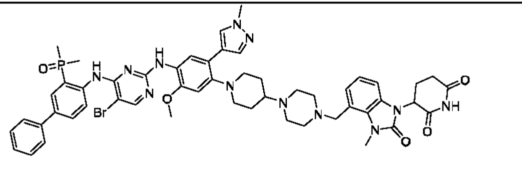 | 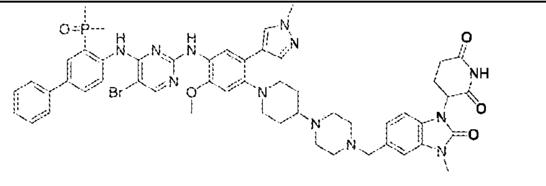 |
| 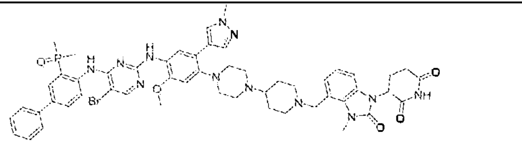 | 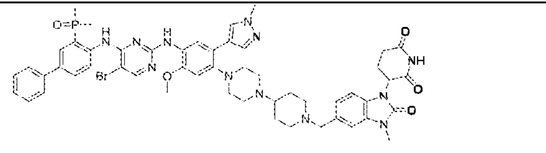 |
| 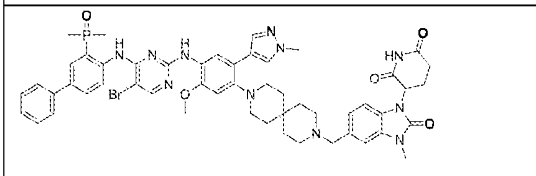 | 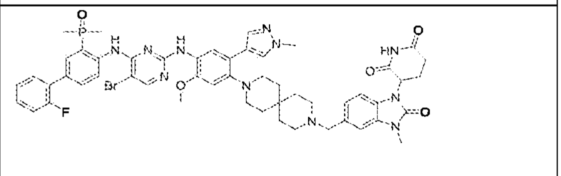 |
| 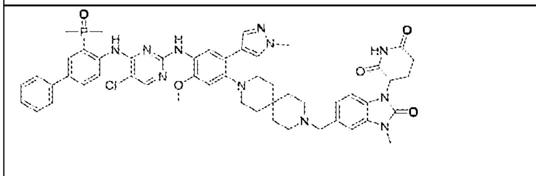 | 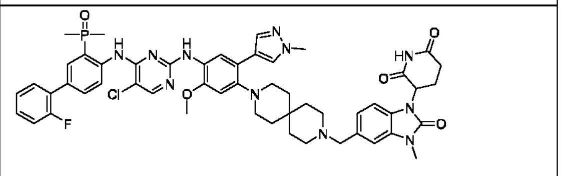 |
| 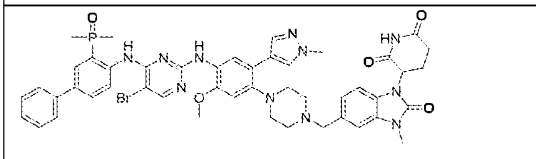 | 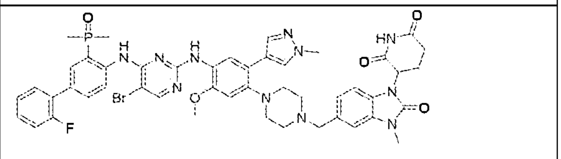 |
| 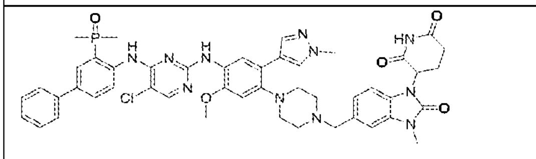 | 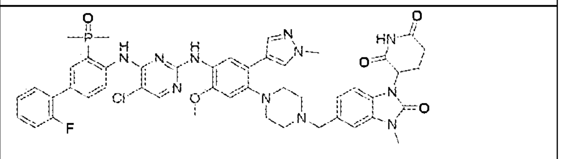 |
| 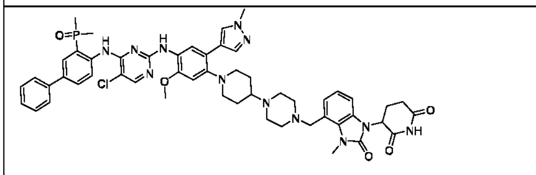 | 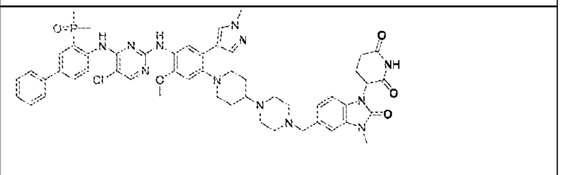 |
| 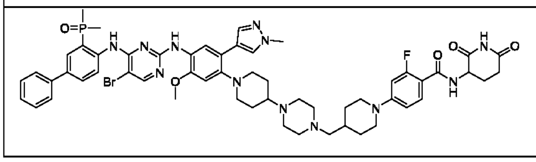 | 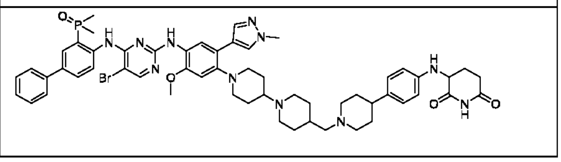 |

| | |
|---|---|
| 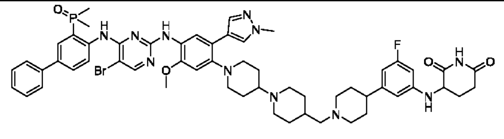 | 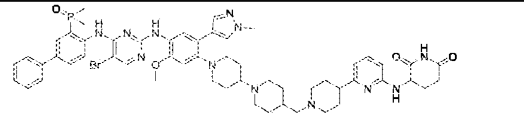 |
| 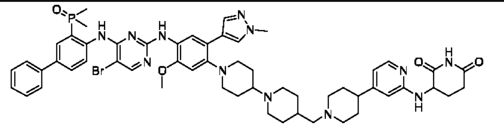 | 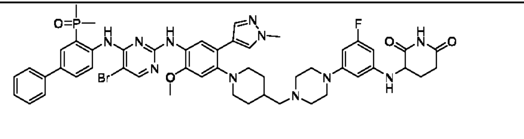 |
| 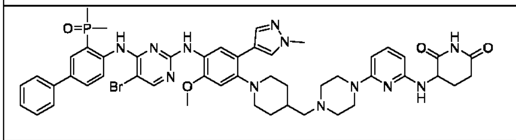 | 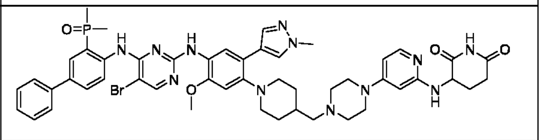 |
| 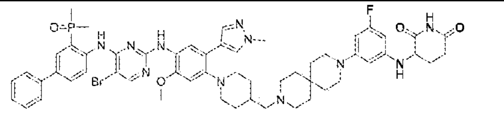 | 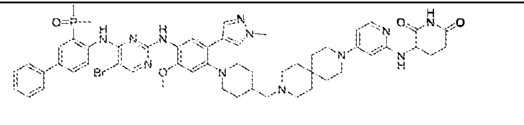 |
| 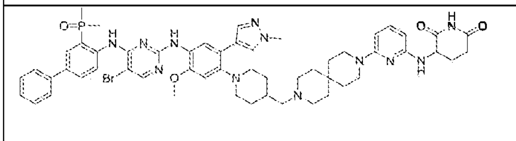 | 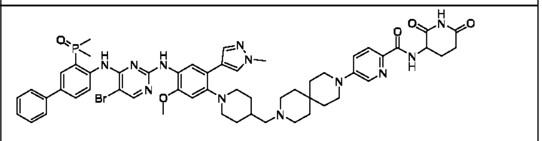 |
| 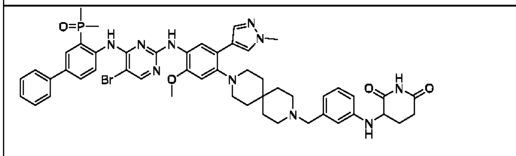 | 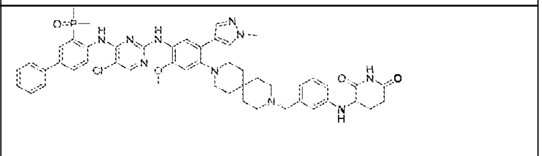 |
| 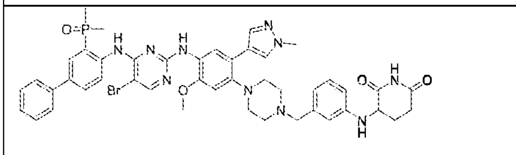 | 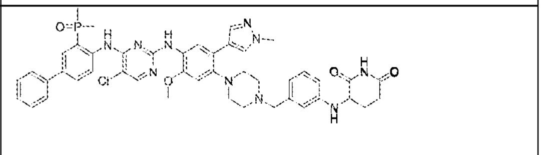 |
| 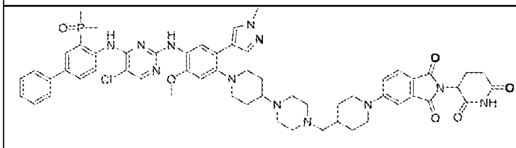 | 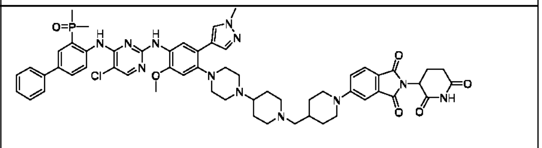 |
| 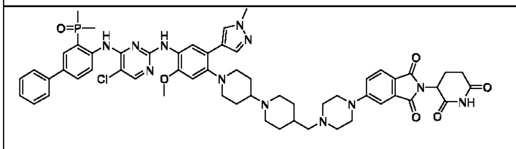 | 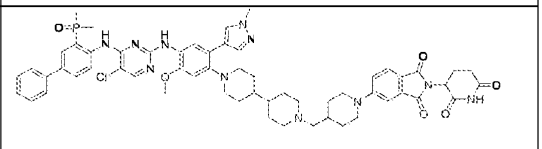 |
| 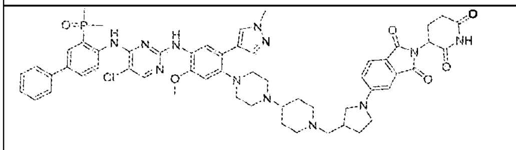 | 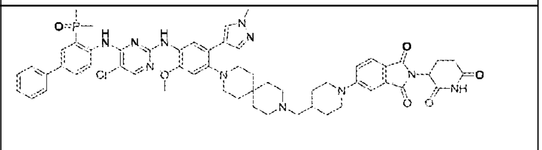 |
| 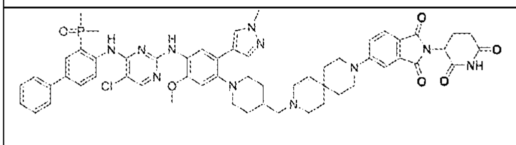 | 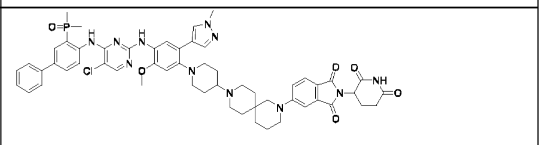 |
| 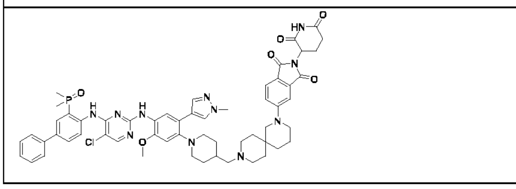 | 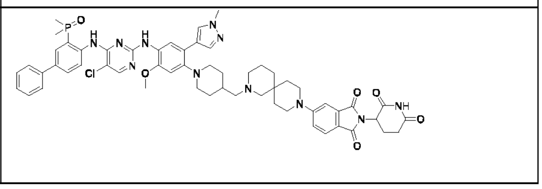 |

| | |
|---|---|
| 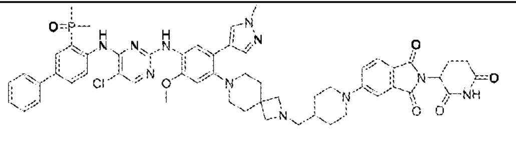 | |
| 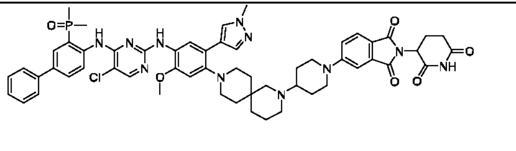 | 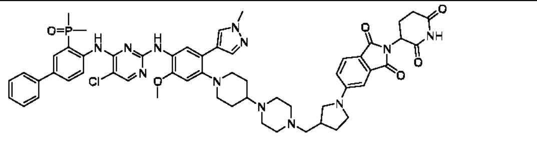 |
| 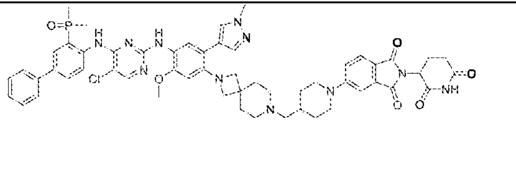 | 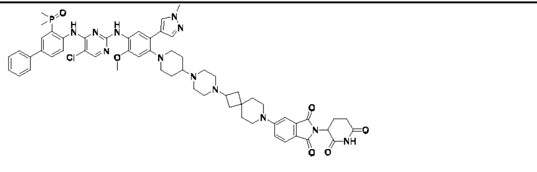 |
| 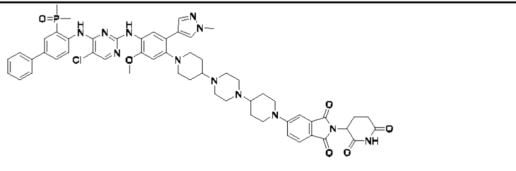 | 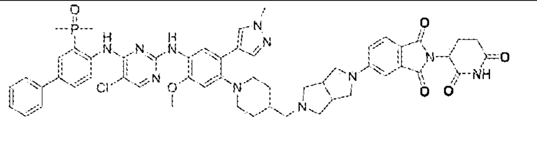 |
| 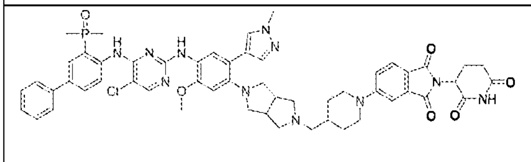 | 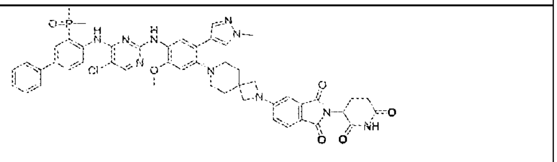 |
| 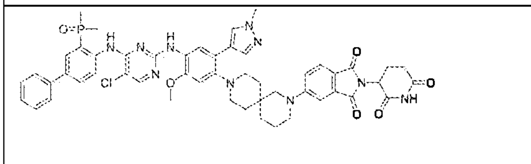 | 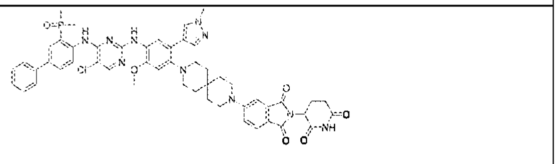 |
| 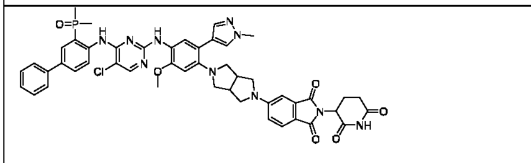 | 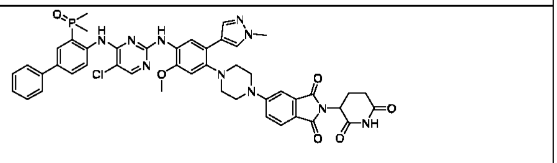 |
| 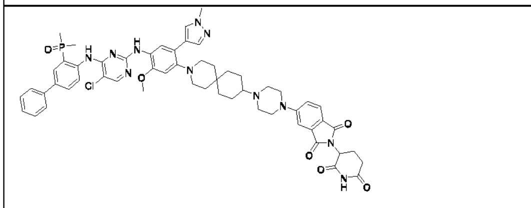 | 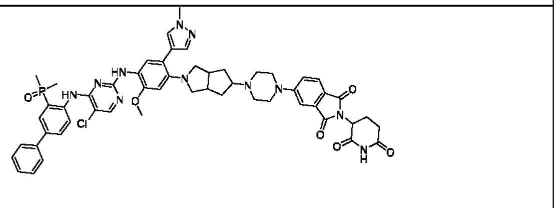 |
| 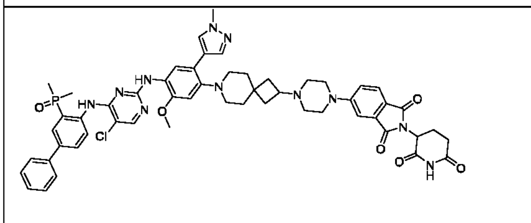 | 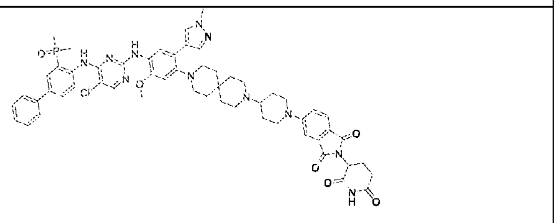 |
| 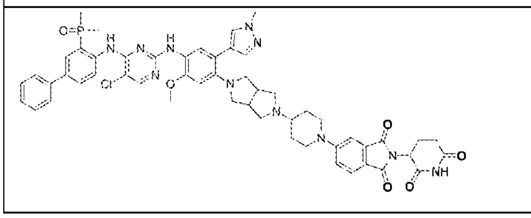 | 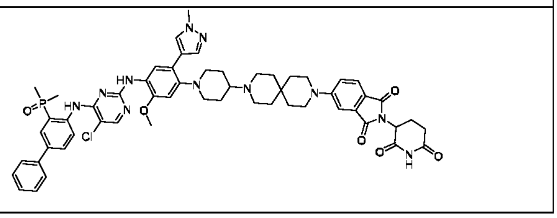 |

| | |
|---|---|
| 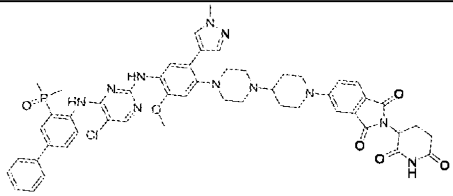 | 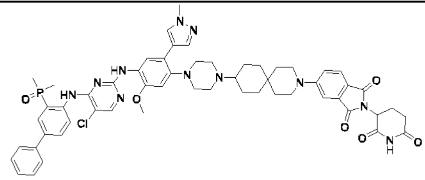 |
| 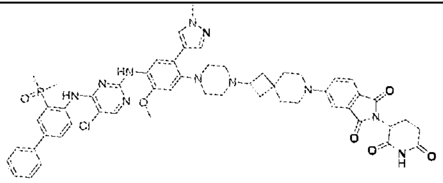 | 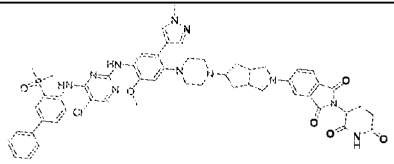 |
| 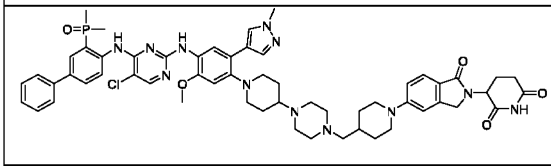 | 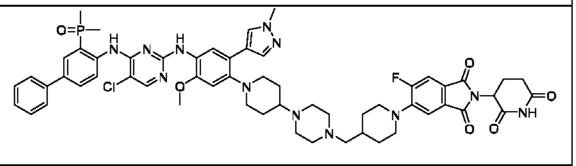 |
| 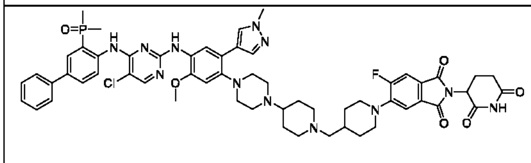 | 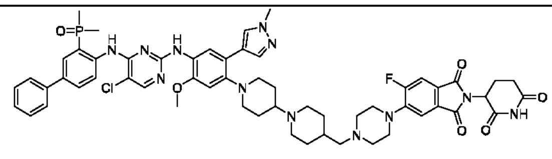 |
| 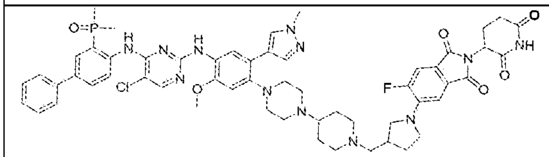 | 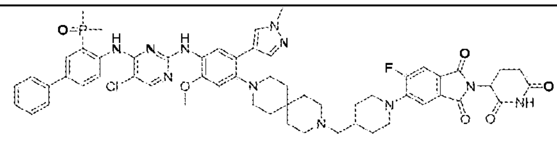 |
| 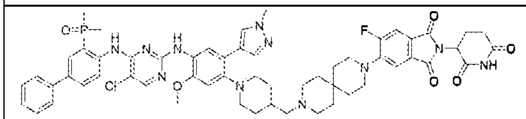 | 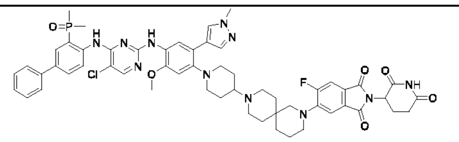 |
| 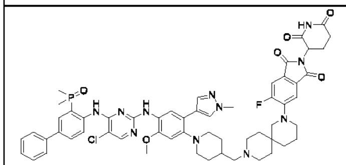 | 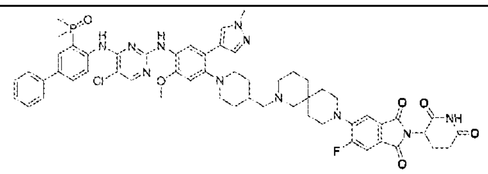 |
| 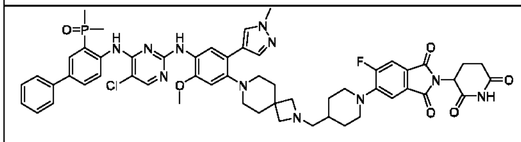 | |
| 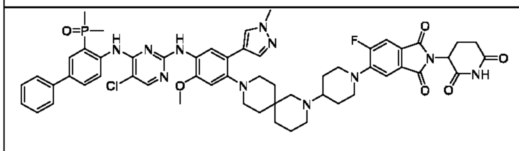 | 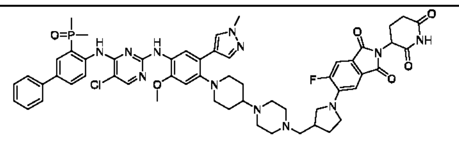 |
| 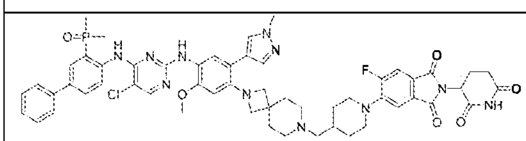 | 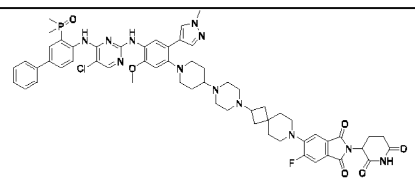 |

| | |
|---|---|
| 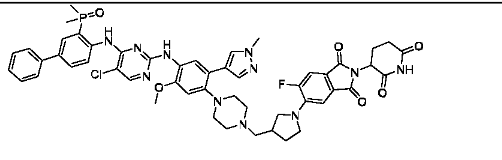 | 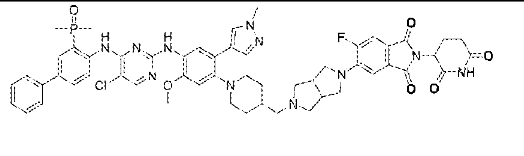 |
| 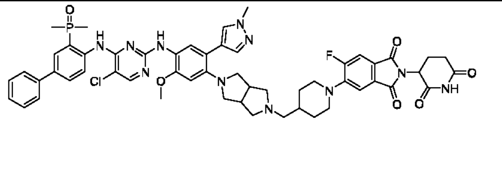 | 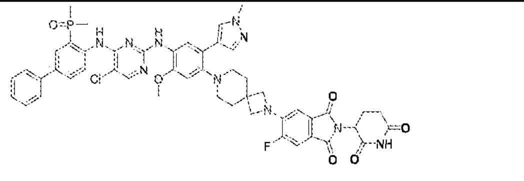 |
| 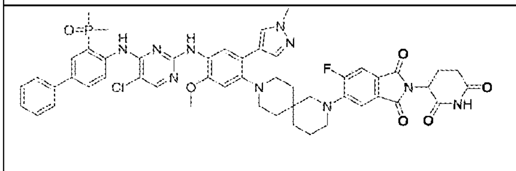 | 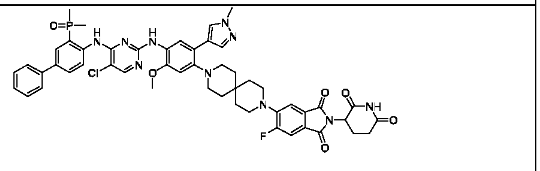 |
| 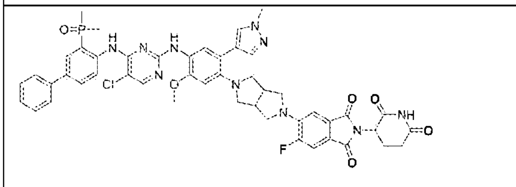 | 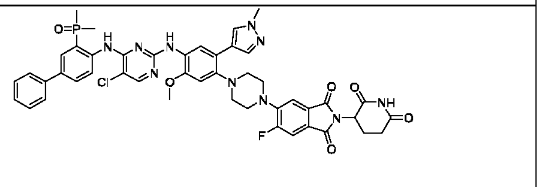 |
| 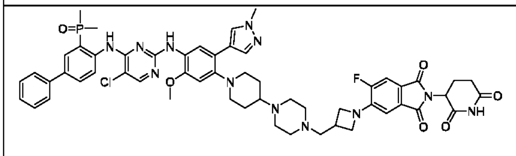 | 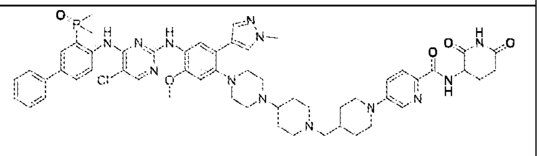 |
| 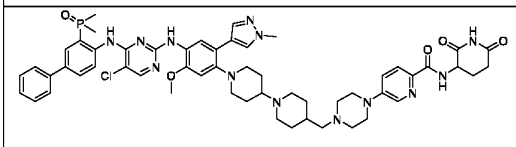 | 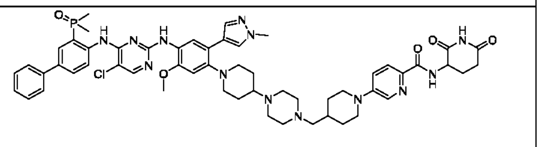 |
| 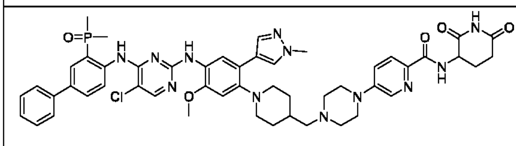 | 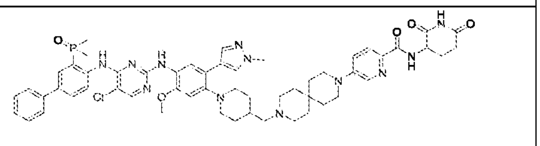 |
| 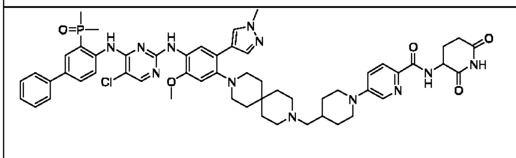 | 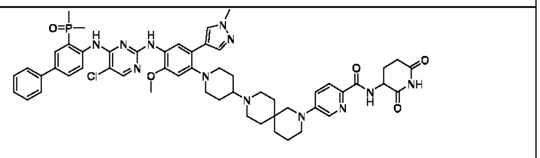 |
| 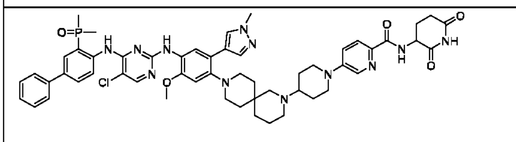 | 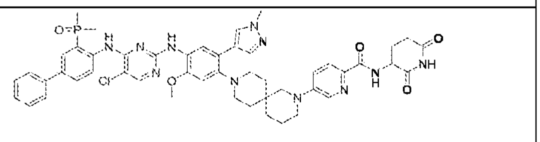 |
| 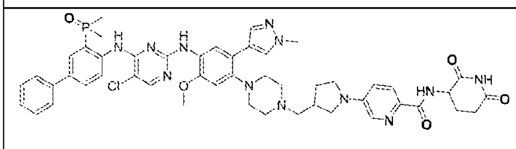 | |
| 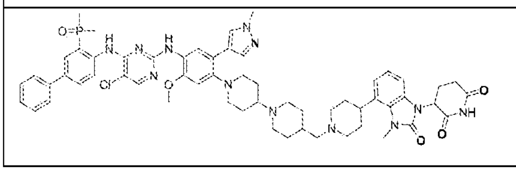 | 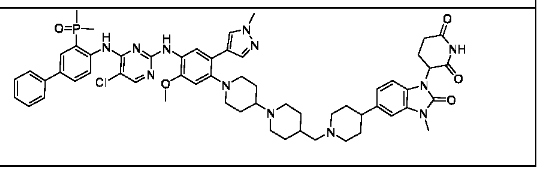 |

| | |
|---|---|
| 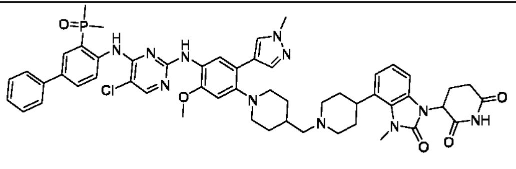 | 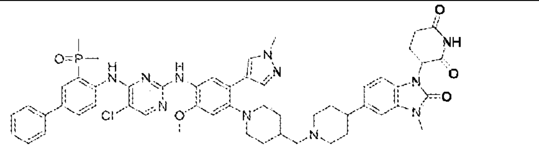 |
| 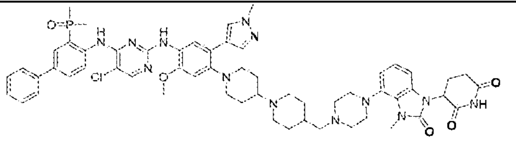 | 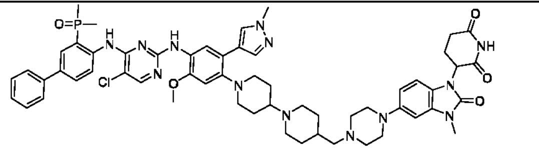 |
| 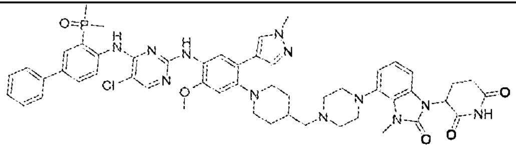 | 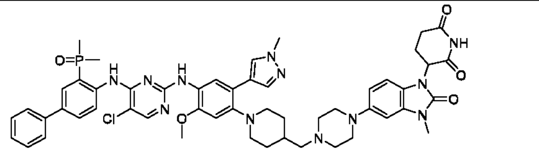 |
| 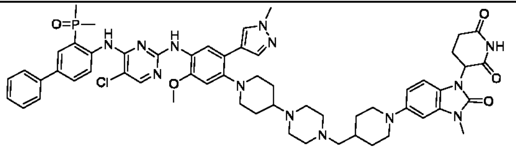 | 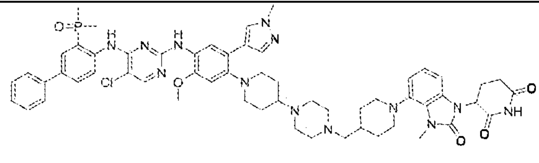 |
| 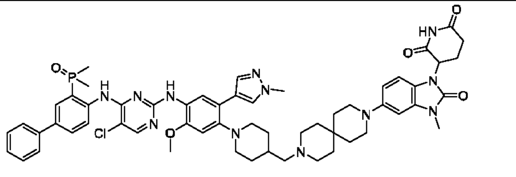 | 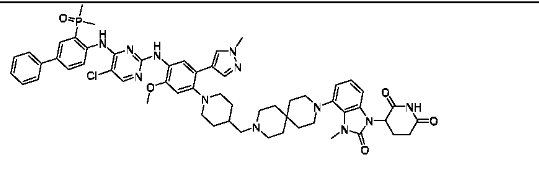 |
| 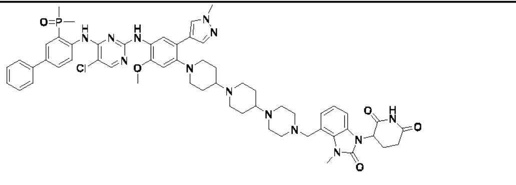 | 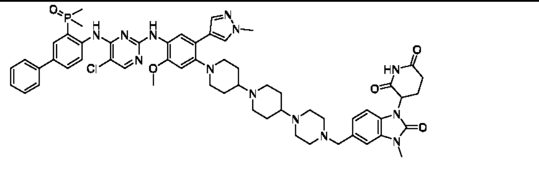 |
| 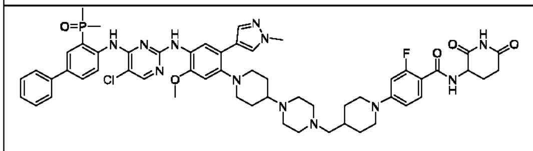 | 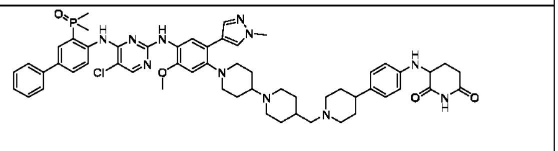 |
| 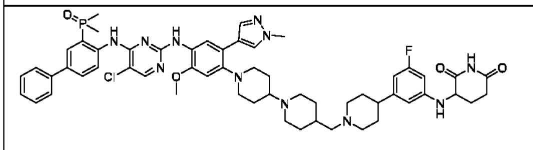 | 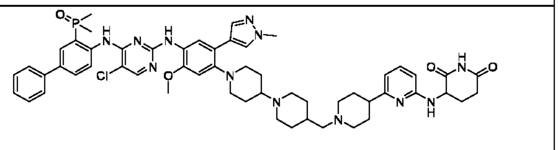 |
| 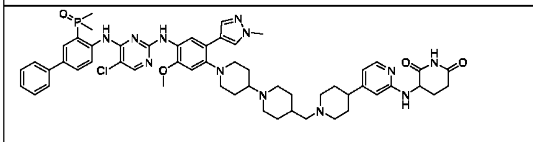 | 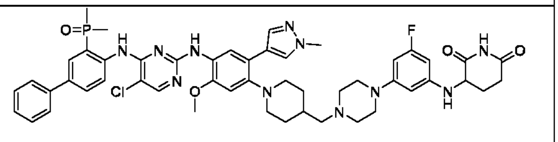 |
| 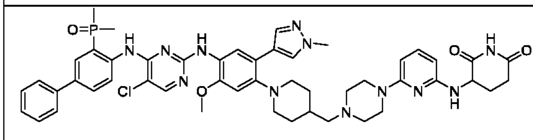 | 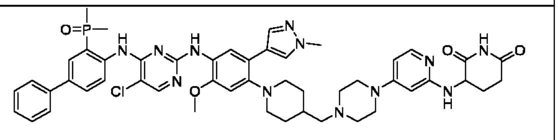 |
| 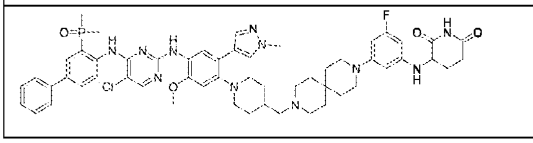 | 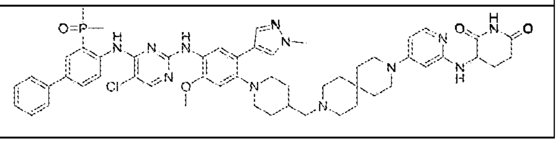 |

| | |
|---|---|
| 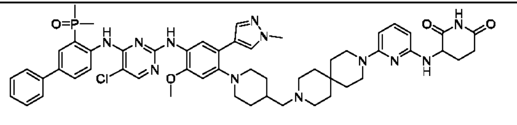 | 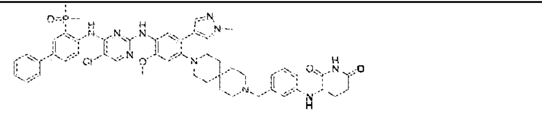 |
| 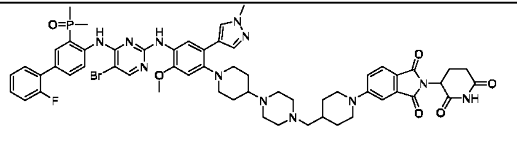 | 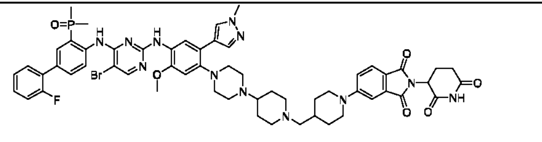 |
| 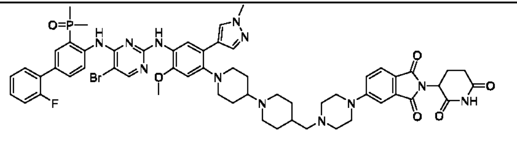 | 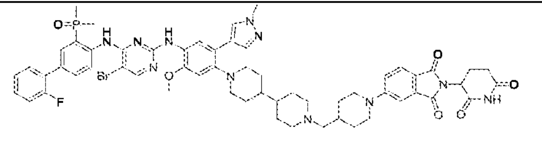 |
| 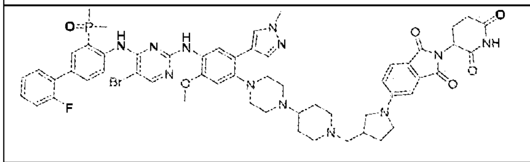 | 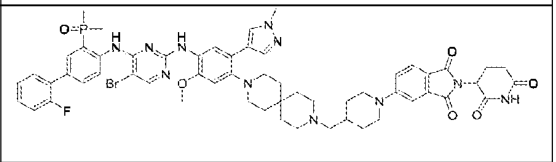 |
| 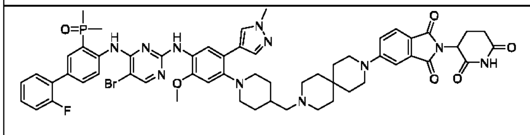 | 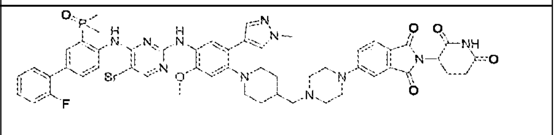 |
| 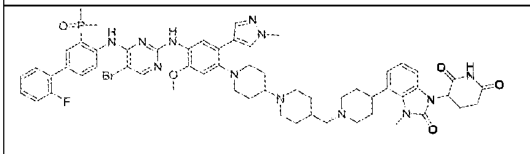 | 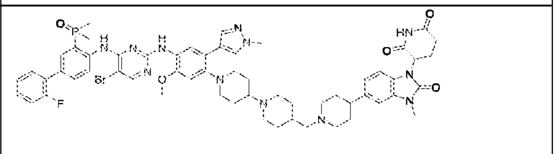 |
| 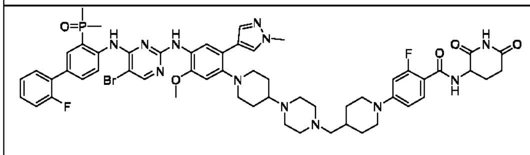 | 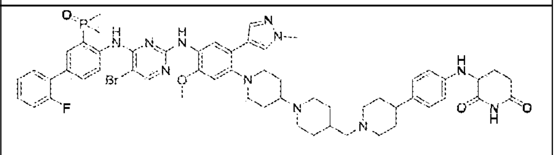 |
| 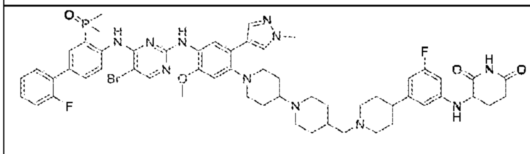 | 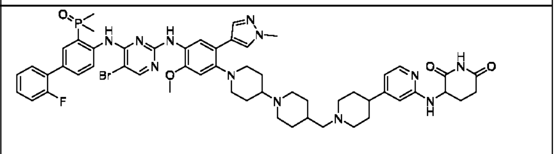 |
| 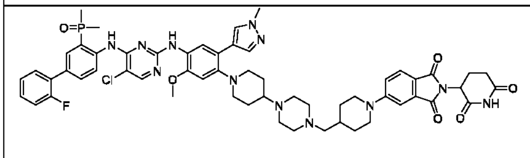 | 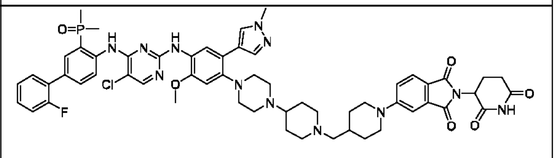 |
| 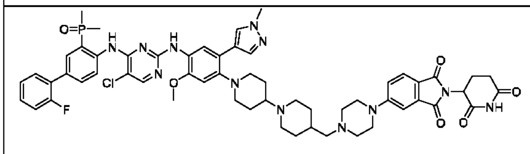 | 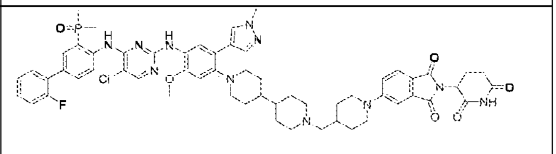 |
| 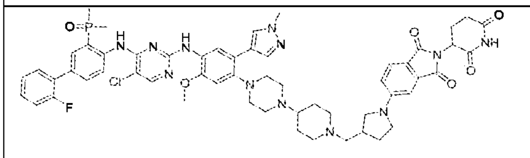 | 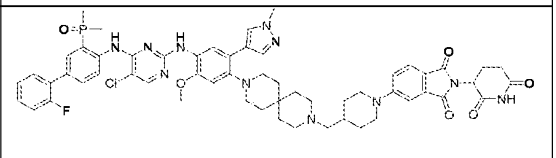 |
| 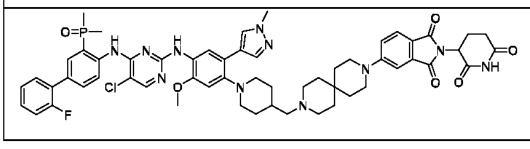 | 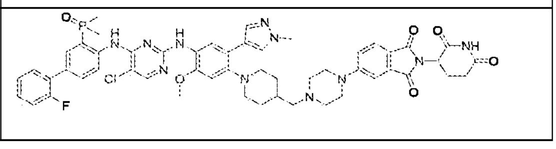 |

| | |
|---|---|
| 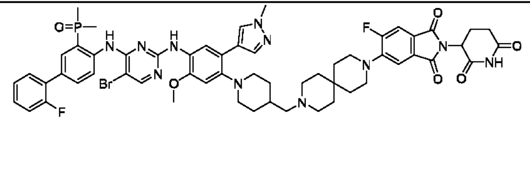 | |
| 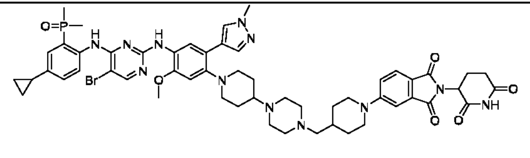 | 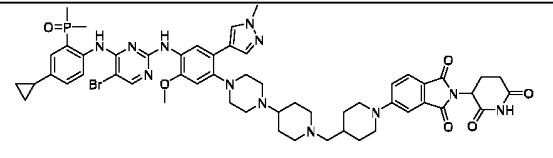 |
| 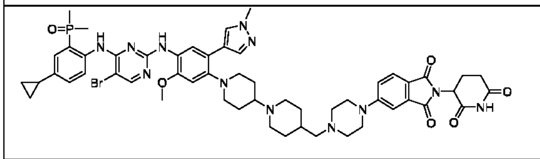 | 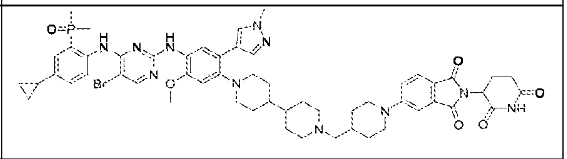 |
| 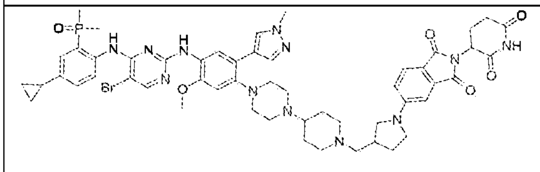 | 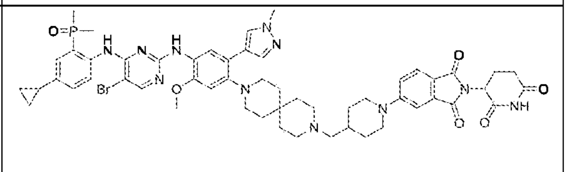 |
| 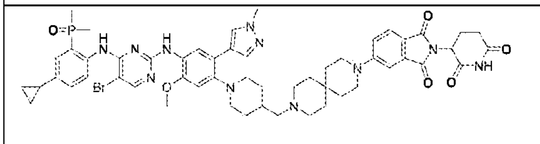 | 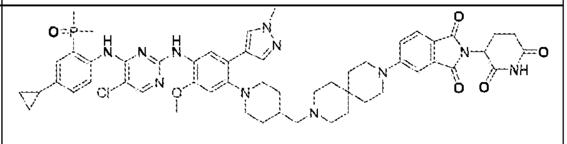 |
| 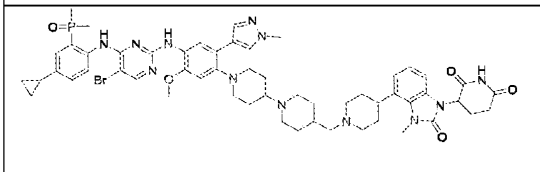 | 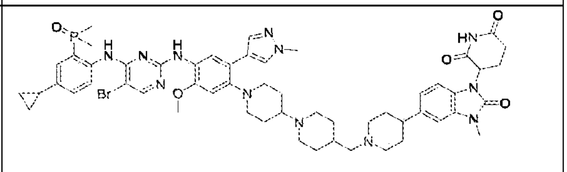 |
| 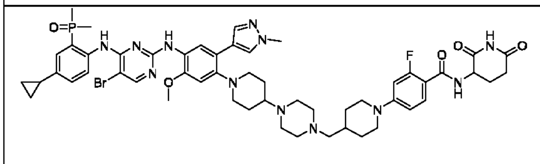 | 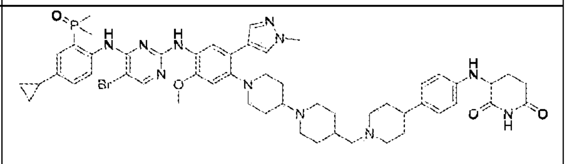 |
| 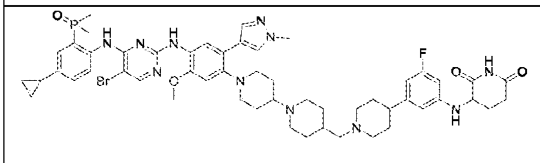 | 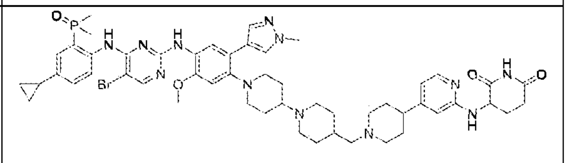 |
| 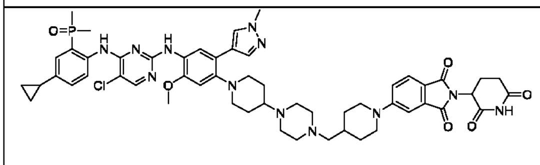 | 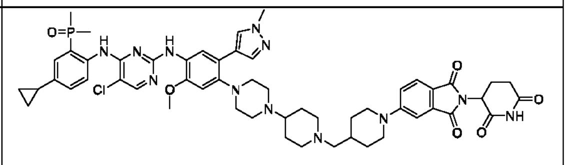 |
| 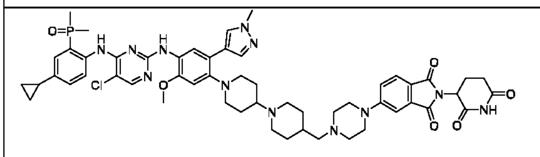 | 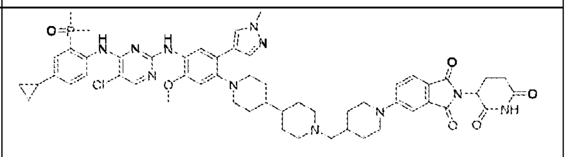 |
| 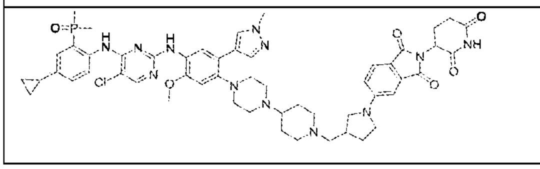 | 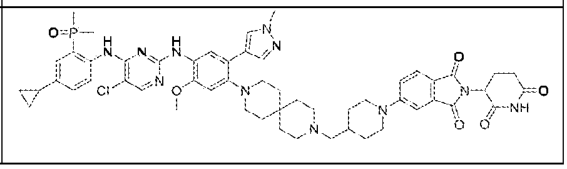 |

| | |
|---|---|
| 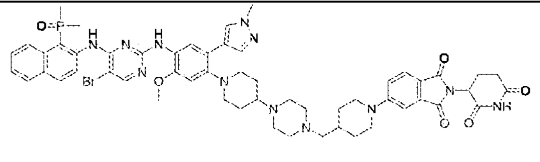 | 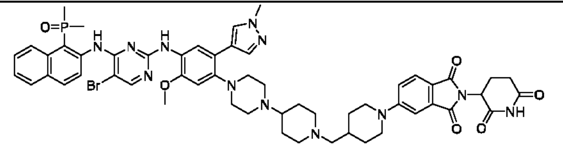 |
| 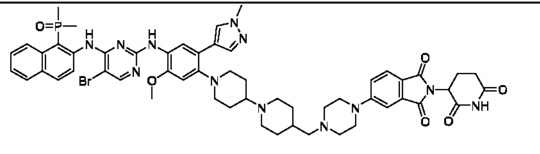 | 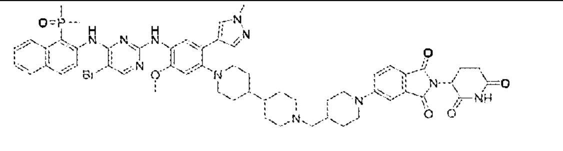 |
| 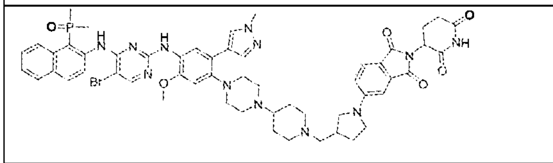 | 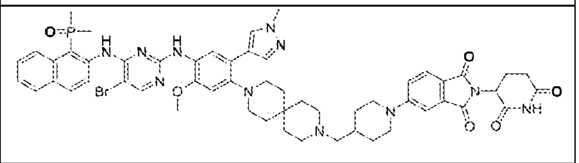 |
| 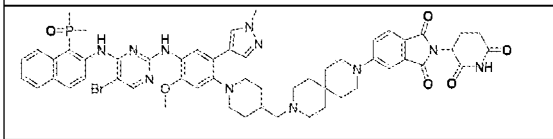 | 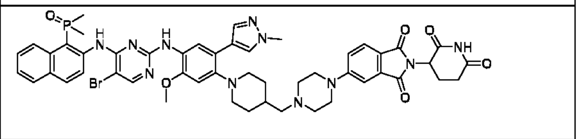 |
| 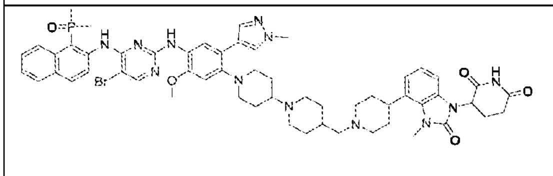 | 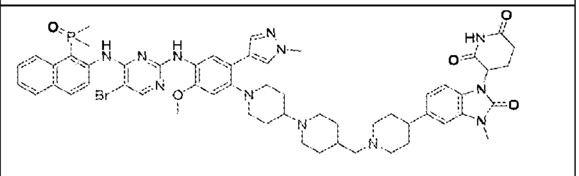 |
| 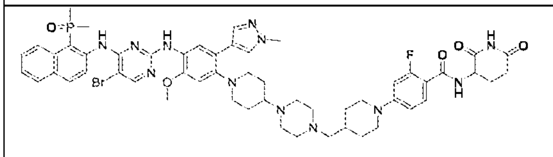 | 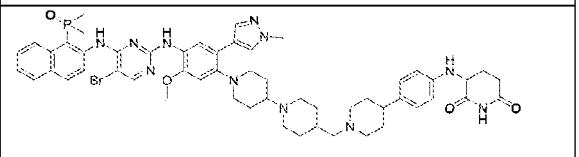 |
| 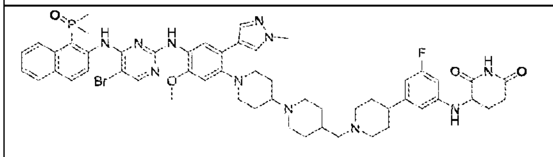 | 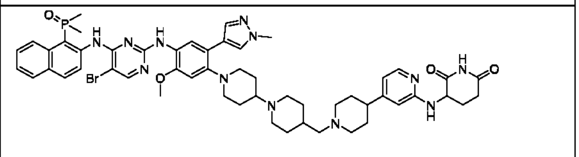 |
| 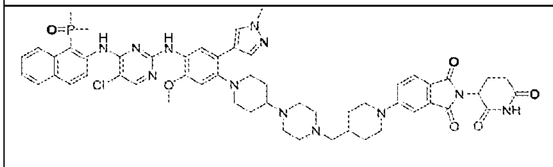 | 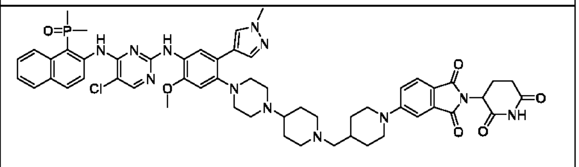 |
| 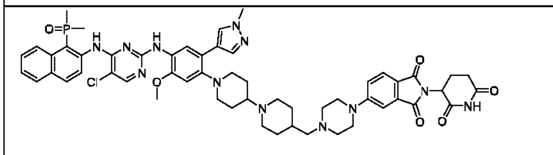 | 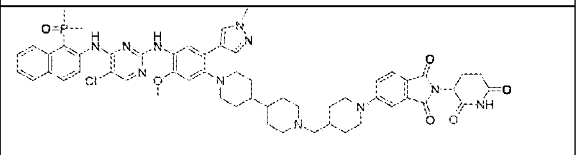 |
| 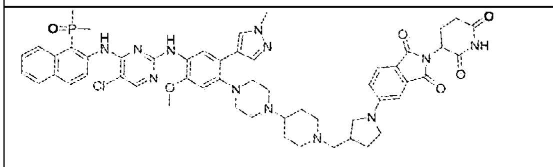 | 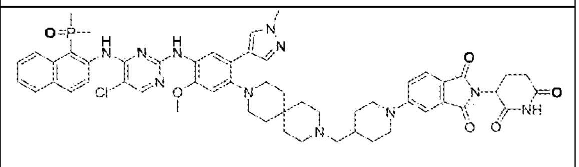 |
| 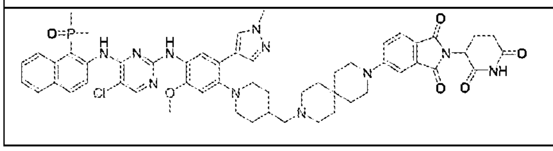 | 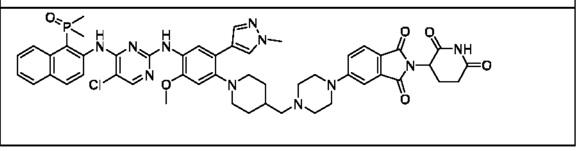 |

| | |
|---|---|
| 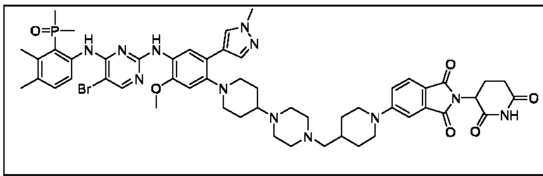 | 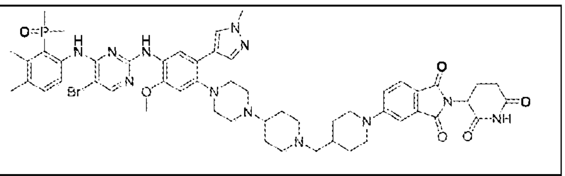 |
| 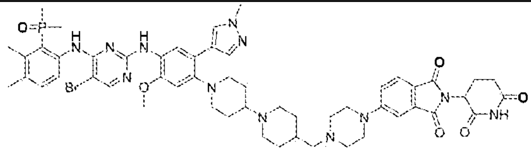 | 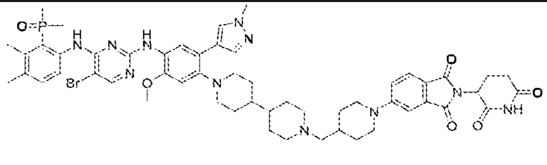 |
| 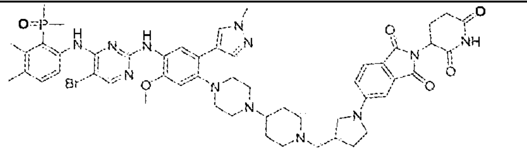 | 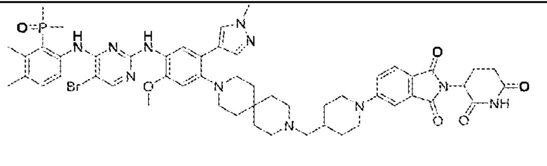 |
| 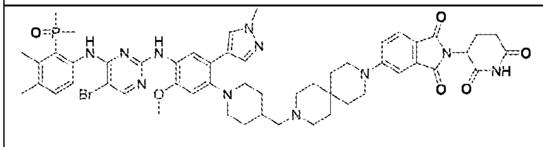 | 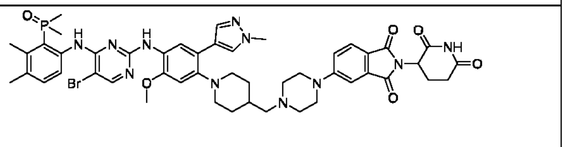 |
| 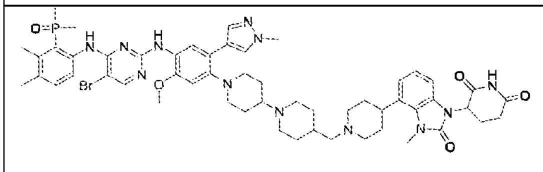 | 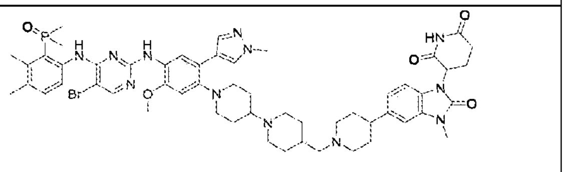 |
| 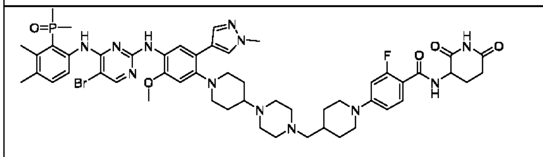 | 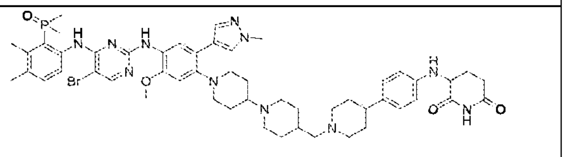 |
| 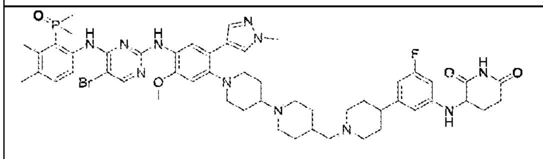 | 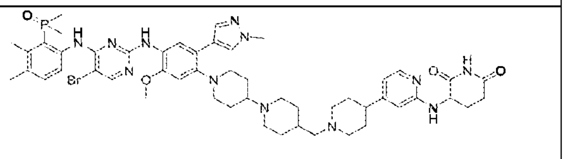 |
| 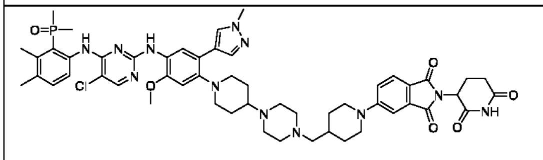 | 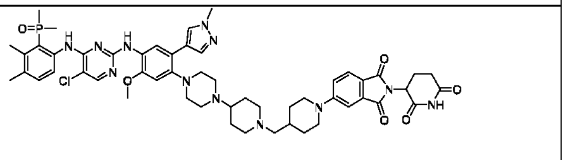 |
| 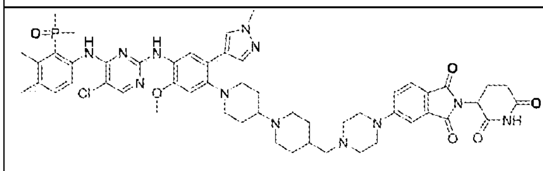 | 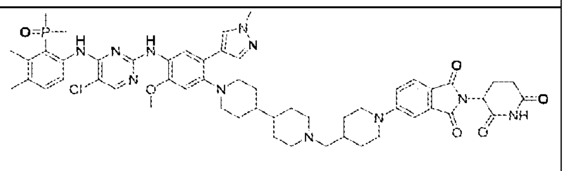 |
| 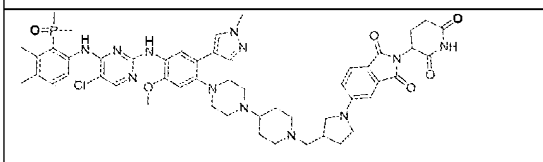 | 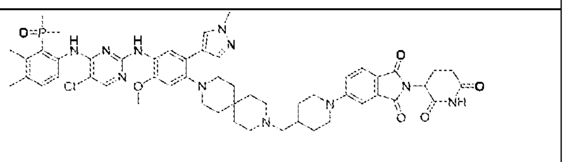 |
| 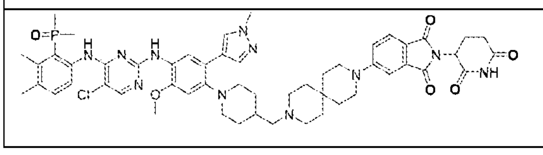 | 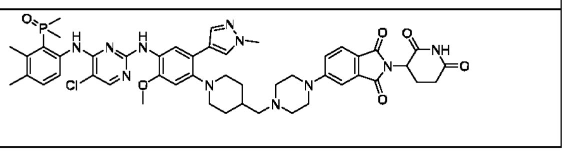 |

| | |
|---|---|
| 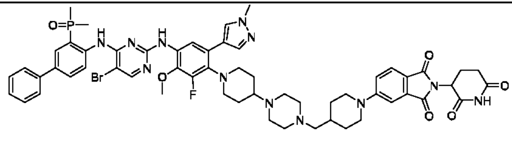 | 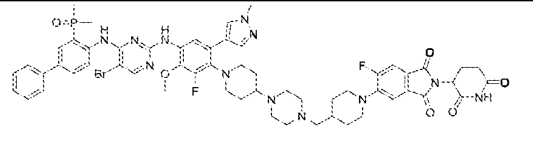 |
| 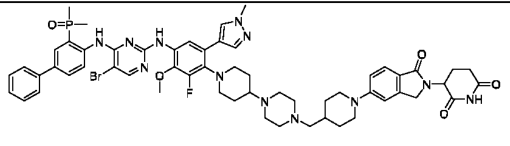 | 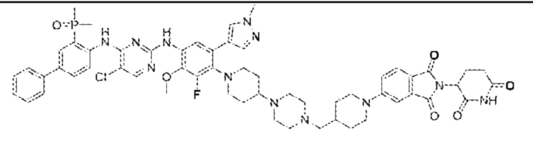 |
| 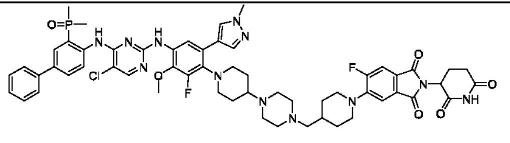 | 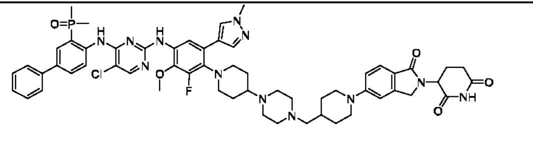 |
| 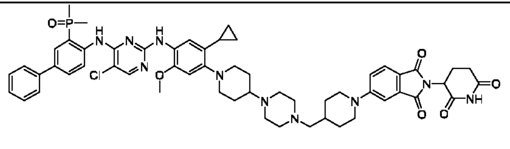 | 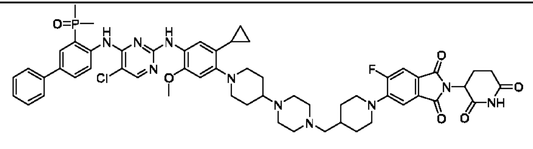 |
| 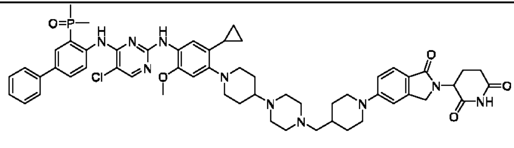 | 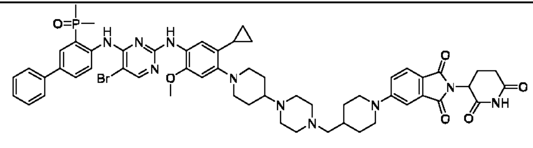 |
| 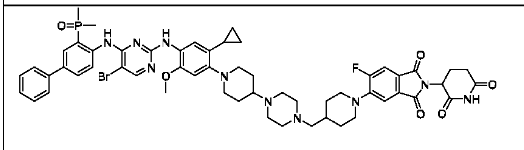 | 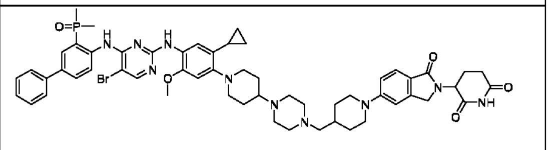 |
| 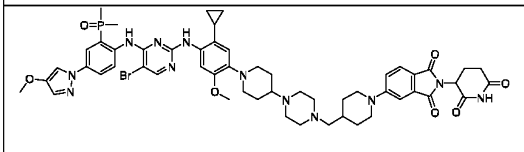 | 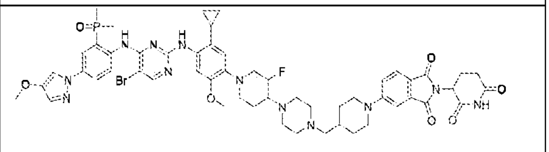 |
| 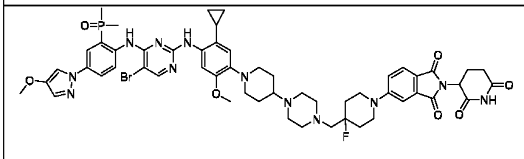 | 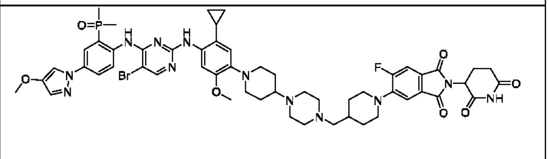 |
| 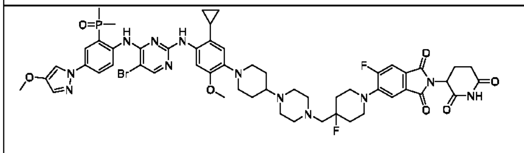 | 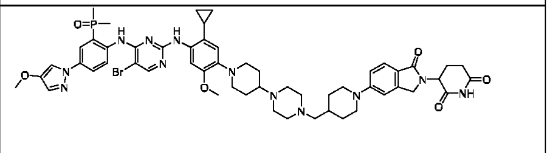 |
| 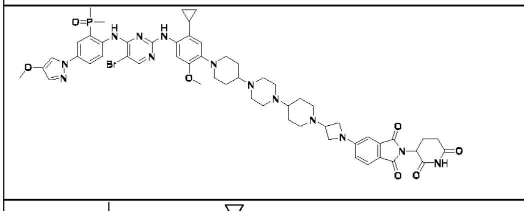 | 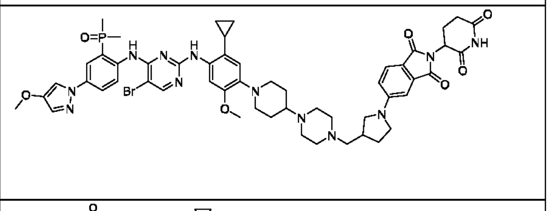 |
| 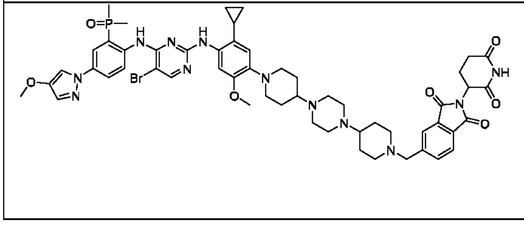 | 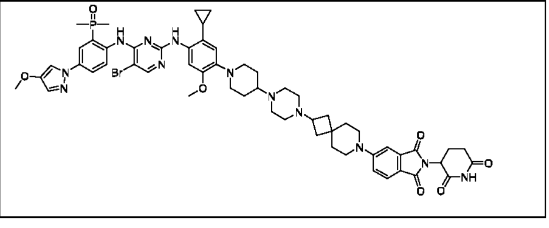 |

| | |
|---|---|
| 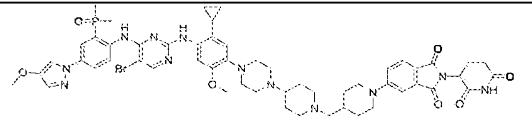 | 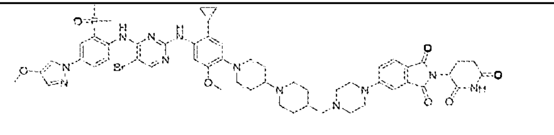 |
| 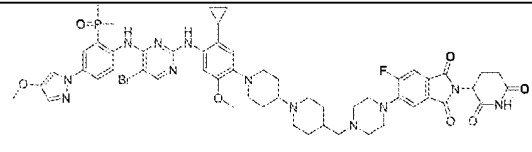 | 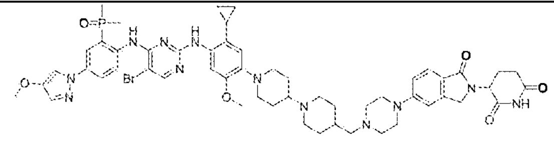 |
| 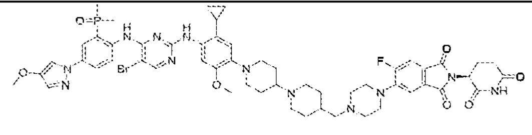 | 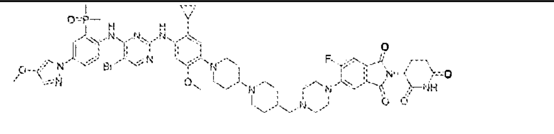 |
| 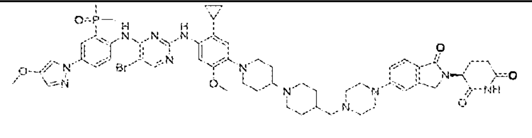 | 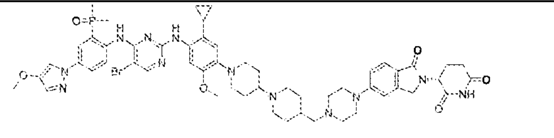 |
| 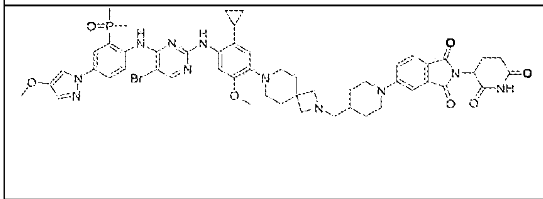 | 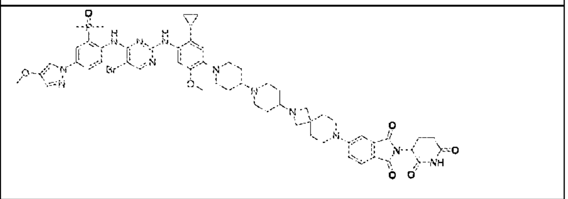 |
| 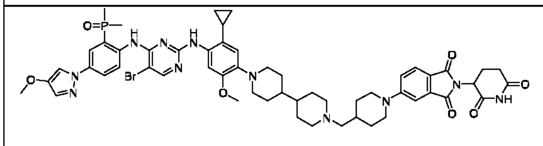 | 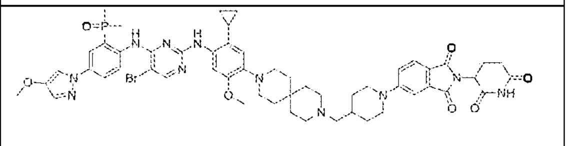 |
| 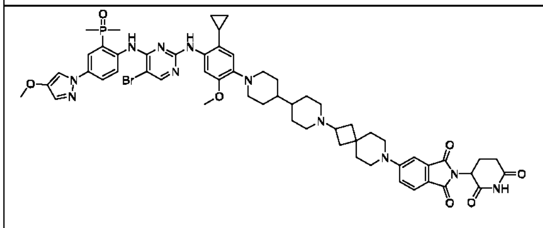 | 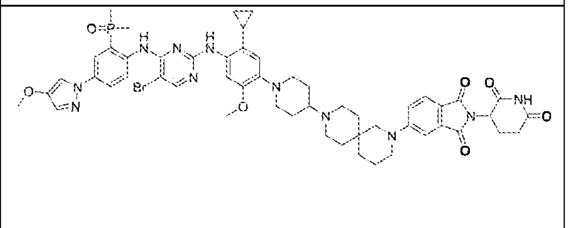 |
| 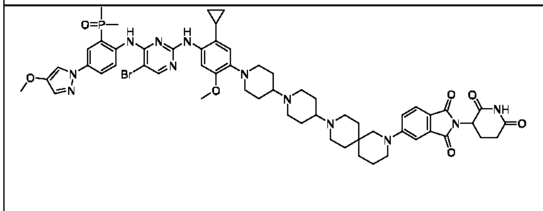 | 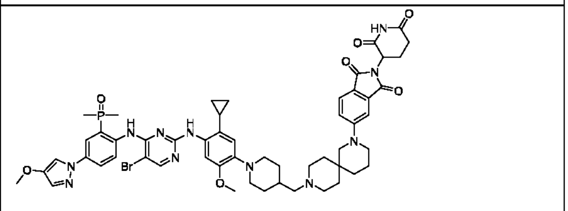 |
| 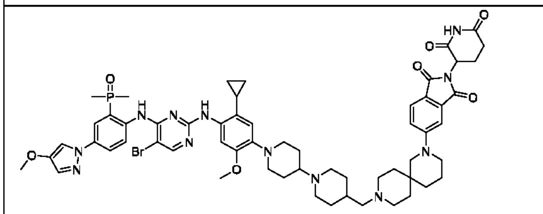 | 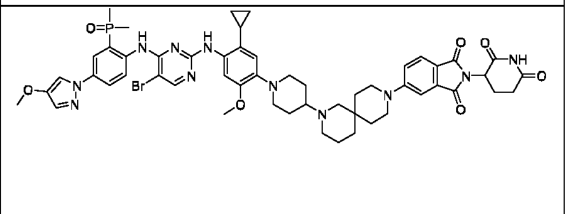 |
| 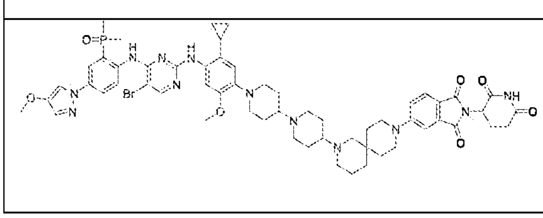 | 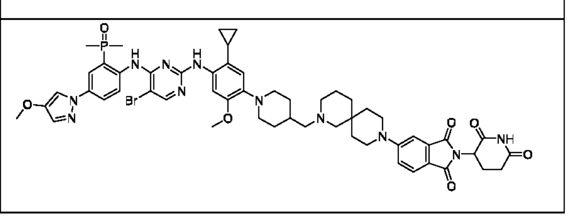 |

| | |
|---|---|
| 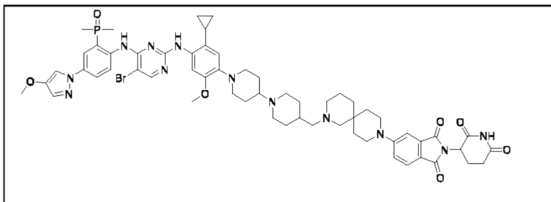 | 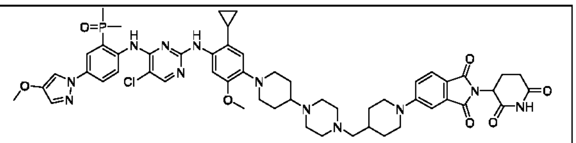 |
| 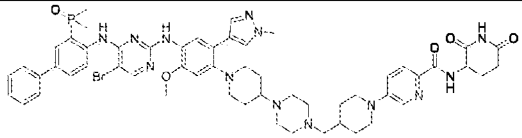 | 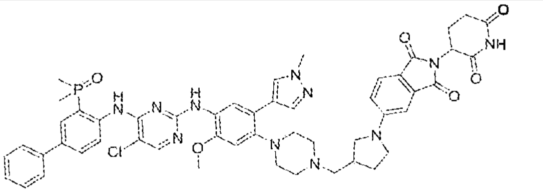 |
| 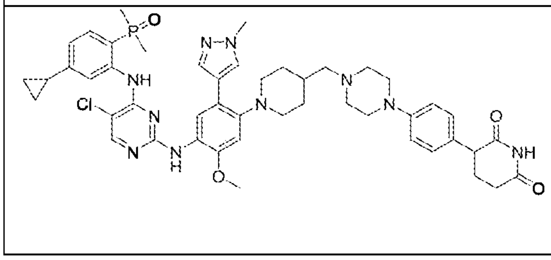 | 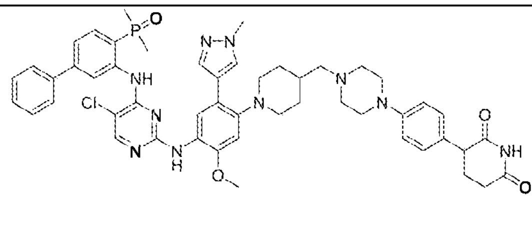. |

The present invention relates to a pharmaceutical composition, comprising the above-mentioned compound in the present invention or the stereoisomer, deuterated compound, solvate, prodrug, metabolite, pharmaceutically acceptable salt or co-crystal thereof, and a pharmaceutically acceptable carrier, optionally comprising one or more other chemotherapeutic agents.

The present invention relates to the use of the above-mentioned compound in the present invention or the stereoisomer, deuterated compound, solvate, prodrug, metabolite, pharmaceutically acceptable salt or co-crystal thereof, or the pharmaceutical composition in the preparation of a medicament for treating a disease related to EGFR activity or expression level.

The present invention relates to the use of the above-mentioned compound in the present invention or the stereoisomer, deuterated compound, solvate, prodrug, metabolite, pharmaceutically acceptable salt or co-crystal thereof, or the pharmaceutical composition in the preparation of a medicament for treating a disease related to the inhibition or degradation of EGFR.

The present invention relates to the use of the above-mentioned compound in the present invention or the stereoisomer, deuterated compound, solvate, prodrug, metabolite, pharmaceutically acceptable salt or co-crystal thereof, wherein the disease is selected from cancer, preferably non-small cell lung cancer.

Unless stated to the contrary, the terms used in the description and claims have the following meanings.

The carbon, hydrogen, oxygen, sulphur, nitrogen or F, Cl, Br, I involved in the groups and compounds of the present invention all comprise their isotopes, and the carbon, hydrogen, oxygen, sulphur or nitrogen involved in the groups and compounds of the present invention is optionally further substituted with one or more of their corresponding isotopes, wherein the isotopes of carbon comprise $^{12}C$, $^{13}C$ and $^{14}C$, the isotopes of hydrogen comprise protium (H), deuterium (D, also known as heavy hydrogen), tritium (T, also known as superheavy hydrogen), the isotopes of oxygen comprise $^{16}O$, $^{17}O$ and $^{18}O$, the isotopes of sulphur comprise $^{32}S$, $^{33}S$, $^{34}S$ and $^{36}S$, the isotopes of nitrogen comprise $^{14}N$ and $^{15}N$, the isotopes of fluorine comprise $^{17}F$ and $^{19}F$, the isotopes of chlorine comprise $^{35}Cl$ and $^{37}Cl$, and the isotopes of bromine comprise $^{79}Br$ and $^{81}Br$.

**"Halogen" refers to F, Cl, Br or I.**

"**Halogen**-substituted" **refers to F, Cl, Br or I substitution, including but not** limited to a substitution with 1 to 10 substituents selected from F, Cl, Br or I, a substitution with 1 to 6 substituents selected from F, Cl, Br or I, or a substitution with 1 to 4 substituents **selected from F, Cl, Br or I. "Halogen-substituted" is referred to simply as "halo".**

**"Alkyl" refers to a substituted or unsubstituted linear or branched saturated** aliphatic hydrocarbyl group, including but not limited to an alkyl group of 1 to 20 carbon atoms, an alkyl group of 1 to 8 carbon atoms, an alkyl group of 1 to 6 carbon atoms, or an alkyl group of 1 to 4 carbon atoms. Non-limiting examples include methyl, ethyl, n-propyl, isopropyl, n-butyl, sec-butyl, neobutyl, tert-butyl, n-pentyl, isoamyl, neopentyl, n-hexyl and various branched isomers thereof. The **definition of the "alkyl" herein is consistent with this definition. Alkyl can be** monovalent, divalent, trivalent or tetravalent.

**"Hydrocarbyl" refers to a substituted or unsubstituted linear or branched** saturated or unsaturated group

consisting of carbon and hydrogen atoms. Hydrocarbyl can be monovalent, divalent, trivalent or tetravalent.

**"Heteroalkyl" refers to a substituted or unsubstituted alkyl group in which one** or more (including but not limited to 2, 3, 4, 5 or 6) carbon atoms are replaced by heteroatoms (including but not limited to N, O or S). Non-limiting examples include $-X(CH_2)v-X(CH_2)v-X(CH_2)v-H$ (v is an integer from 1 to 5; each X is independently selected from a bond or a heteroatom, which includes but is not limited to N, O or S; at least one X is selected from a heteroatom; and N or S in the heteroatom can be oxidized to various oxidation states). Heteroalkyl can be monovalent, divalent, trivalent or tetravalent.

**"Alkylene" refers to** a substituted or unsubstituted linear and branched divalent saturated hydrocarbyl group, including $-(CH_2)_v-$ (v is an integer from 1 to 10), and examples of alkylene include, but are not limited to, methylene, ethylene, propylene, butylene, etc.

**"Heteroalkylene" refers to a substituted or unsubstituted alkylene group in** which one or more (including but not limited to 2, 3, 4, 5 or 6) carbon atoms are replaced by heteroatoms (including but not limited to N, O or S). Non-limiting examples include $-X(CH_2)v-X(CH_2)v-X(CH_2)v-$, wherein v is an integer from 1 to 5, each X is independently selected from a bond, N, O or S, and at least one X is selected from N, O or S.

**"Cycloalkyl" refers to a substituted or unsubstituted saturated carbocyclic** hydrocarbyl group, usually having from 3 to 10 carbon atoms, and non-limiting examples include cyclopropyl, cyclobutyl, cyclopentyl, cyclohexyl, cycloheptyl, **etc. The "cycloalkyl" herein is as defined above. Cycloalkyl can be monovalent,** divalent, trivalent or tetravalent.

**"Heterocycloalkyl" refers to a substituted or unsubstituted saturated** heteroatom-containing cyclic hydrocarbyl group, including but not limited to 3 to 10 atoms, 3 to 8 atoms, or 1 to 3 heteroatoms selected from N, O or S. N and S selectively substituted in the heterocycloalkyl ring can be oxidized to various oxidation states. Heterocycloalkyl can be connected to a heteroatom or a carbon atom; heterocycloalkyl can be connected to an aromatic ring or a non-aromatic ring; and heterocycloalkyl can be connected to a bridged ring or a spiro ring. Non-limiting examples include oxiranyl, azacyclopropyl, oxetanyl, azetidinyl, tetrahydrofuryl, tetrahydro-2*H*-pyranyl, dioxolanyl, dioxanyl, pyrrolidinyl, piperidinyl, imidazolidinyl, oxazolidinyl, oxazinanyl, morpholinyl, hexahydropyrimidinyl or piperazinyl. Heterocycloalkyl can be monovalent, divalent, trivalent or tetravalent.

**"Alkenyl" refers to a substituted or unsubstituted linear and branched** unsaturated hydrocarbyl group having at least 1, usually 1, 2 or 3 carbon-carbon double bonds, with a main chain including but not limited to 2 to 10, 2 to 6, or 2 to 4 carbon atoms. Examples of alkenyl include, but are not limited to vinyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, 1,4-hexadiene, etc. **The definition of the "alkenyl" herein is** consistent with this definition. Alkenyl can be monovalent, divalent, trivalent or tetravalent.

**"Alkynyl" refers to a substituted or unsubstituted linear and branched** monovalent unsaturated hydrocarbyl group having at least 1, usually 1, 2 or 3 carbon-carbon triple bonds, with a main chain including but not limited to 2 to 10 carbon atoms, 2 to 6 carbon atoms or 2 to 4 carbon atoms. Examples of alkynyl include but are not limited to ethynyl, propargyl, 1-propynyl, 2-propynyl, 1-butynyl, 2-butynyl, 3-butynyl, 1-pentynyl, 2-pentynyl, 3-pentynyl, 4-pentynyl, 1-methyl-1-butynyl, 2-methyl-1-butynyl, 2-methyl-3-butynyl, 1-hexynyl, 2-hexynyl, 3-hexynyl, 4-hexynyl, 5- hexynyl, 1-methyl-1-pentynyl, 2-methyl-1-pentynyl, 1-heptynyl, 2-heptynyl, 3-heptynyl, 4-heptynyl, 1-octynyl, 3-octynyl, 1-nonynyl, 3-nonynyl, 1-decynyl, 4-decynyl, etc. Alkynyl can be monovalent, divalent, trivalent or tetravalent.

**"Alkoxy" refers to a substituted or unsubstituted** -O-alkyl group. Non-limiting examples include methoxy, ethoxy, n-propoxy, isopropoxy, n-butoxy, sec-butoxy, tert-butoxy, n-pentoxy, n-hexyloxy, cyclopropoxy and cyclobutoxy.

**"Carbocyclyl" or "carbocycle" refers to a substituted or unsubstituted** saturated or unsaturated aromatic ring or non-aromatic ring, wherein the aromatic ring or non-aromatic ring can be a 3- to 8-membered monocyclic ring, a 4- to 12-membered bicyclic ring or a 10- to 15-membered tricyclic ring system. Carbocyclyl can be connected to an aromatic ring or a non-aromatic ring, wherein the aromatic ring or non-aromatic ring is optionally a monocyclic ring, a bridged ring or a spiro ring. Non-limiting examples include cyclopropane, cyclobutane, cyclopentane, cyclohexane, cycloheptane, 1-cyclopentyl-1-enyl, 1-cyclopentyl-2-enyl, 1-cyclopentyl-3-enyl, cyclohexyl, 1-cyclohexyl-2-enyl, 1-cyclohexyl-3-enyl, cyclohexenyl, benzene ring, naphthalene ring,

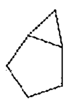 , 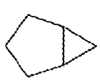 , 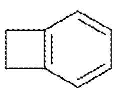 ,

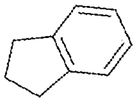 or 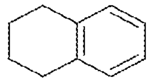.

"Carbocyclyl" or "carbocycle" can be monovalent, divalent, trivalent or tetravalent.

"Heterocyclyl" or "heterocycle" refers to a substituted or unsubstituted saturated or unsaturated aromatic ring or non-aromatic ring, wherein the aromatic ring or non-aromatic ring can be a 3- to 8-membered monocyclic ring, a 4- to 12-membered bicyclic ring or a 10- to 15-membered tricyclic ring system, and contains one or more (including but not limited to 2, 3, 4 or 5) heteroatoms selected from N, O or S, and the selectively substituted N and S in the heterocyclyl ring can be oxidized to various oxidation states. Heterocyclyl can be connected to a heteroatom or a carbon atom; heterocyclyl can be connected to an aromatic ring or a non-aromatic ring; and heterocyclyl can be connected to a bridged ring or a spiro ring. Non-limiting examples include oxiranyl, azacyclopropyl, oxetanyl, azetidinyl, 1,3-dioxolanyl, 1,4-dioxolanyl, 1,3-dioxanyl, azacycloheptyl, pyridyl, furyl, thienyl, pyranyl, N-alkylpyrrolyl, pyrimidinyl, pyrazinyl, pyridazinyl, imidazolyl, piperidyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, tetrahydrothiazolyl, tetrahydropyranyl, benzoimidazolyl, benzopyridyl, pyrrolopyridyl, benzodihydrofuryl, pyrrolyl, pyrazolyl, thiazolyl, oxazolyl, pyrazinyl, indazolyl, benzothienyl, benzofuryl, benzopyrrolyl, benzoimidazolyl, benzothiazolyl, benzoxazolyl, benzopyridyl, benzopyrimidinyl, benzopyrazinyl, piperazinyl, azabicyclo[3.2.1]octanyl, azabicyclo[5.2.0]nonanyl, oxatricyclo[5.3.1.1]dodecyl, azaadamantyl, oxaspiro[3.3]heptanyl,

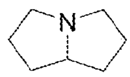,

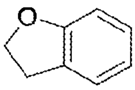, 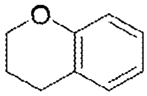, 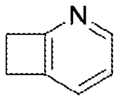, 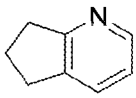, 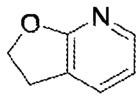, 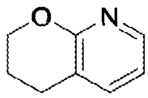, 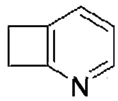,

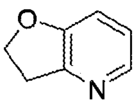, 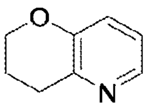, 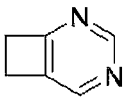, 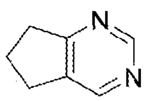, 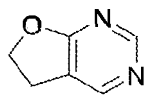 or 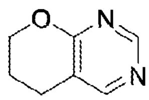.

"Heterocyclyl" or "heterocycle" can be monovalent, divalent, trivalent or tetravalent.

"Spiro ring" or "spiro ring group" refers to a polycyclic group that shares one atom (called a spiro atom) between substituted or unsubstituted monocyclic rings. The number of ring atoms in the spiro ring system includes but is not limited to 5 to 20, 6 to 14, 6 to 12, or 6 to 10, wherein one or more rings may contain 0 or more (including but not limited to 1, 2, 3 or 4) double bonds, and can optionally contain 0 to 5 heteroatoms selected from N, O or $S(=O)_n$.

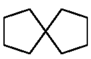 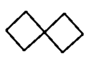 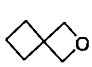 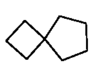 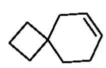 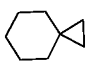 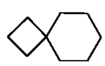

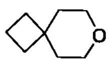 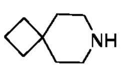 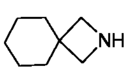 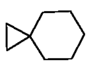 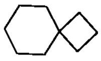 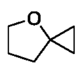 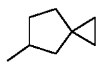

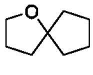 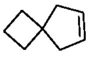 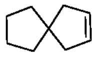 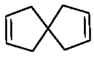 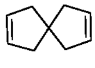 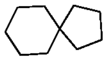 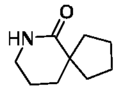

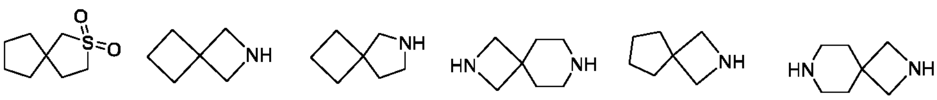

**"Spiro** ring" or "spiro ring group" can be monovalent, divalent, trivalent or tetravalent.

"Fused ring" or "fused ring group" refers to a polycyclic group in which each ring in the system shares an adjacent pair of atoms with other rings in the system, wherein one or more rings may contain 0 or more (including but not limited to 1, 2, 3 or 4) double bonds, and may be substituted or unsubstituted, and each ring in the fused ring system may contain 0 to 5 heteroatoms or groups containing heteroatoms (including but not limited to N, $S(=O)_n$ or O, wherein n is 0, 1 or 2). The number of ring atoms in the fused ring system includes but is not limited to 5 to 20, 5 to 14, 5 to 12, or 5 to 10. Non-limiting examples include:

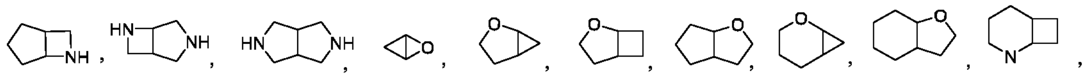

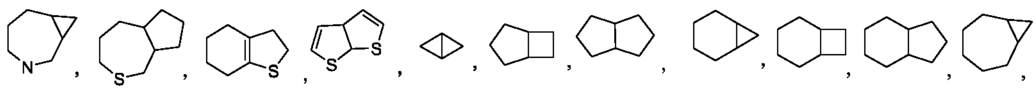

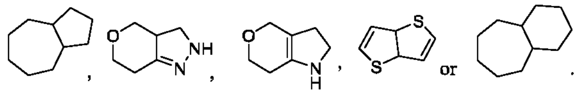

"Fused ring" or "fused ring group" can be monovalent, divalent, trivalent or tetravalent.

**"Bridged ring" or "bridged ring group" refers to a substituted or unsubstituted** polycyclic group containing any two atoms that are not directly connected, and may contain 0 or more double bonds. Any ring in the fused ring system may contain 0 to 5 groups selected from heteroatoms or groups containing heteroatoms (including but not limited to N, S(=O)n or O, wherein n is 0, 1 or 2). The number of ring atoms includes but is not limited to 5 to 20, 5 to 14, 5 to 12 or 5 to 10. Non-limiting examples include

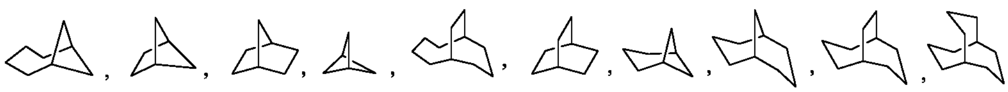

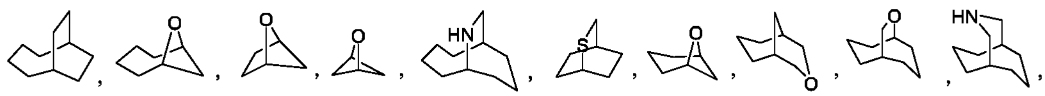

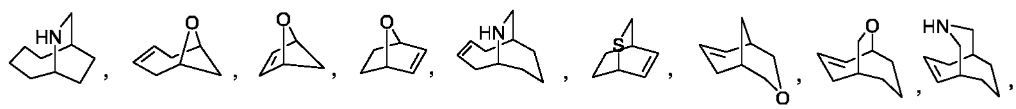

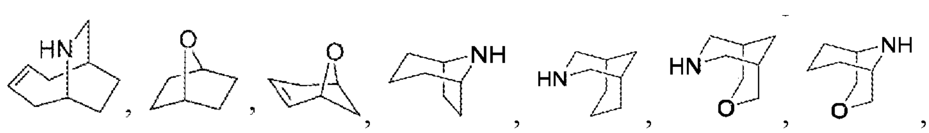

cubane or adamantane. "Bridged ring" or "bridged ring group" can be monovalent, divalent, trivalent or tetravalent.

"Carbospiro ring", "spiro ring carbocyclyl", "spirocarbocyclyl" or "carbospiro ring group" refers to a "spiro ring" with a ring system consisting only of carbon atoms. The definition of the "carbospiro ring", "spiro ring carbocyclyl", "spirocarbocyclyl" or "carbospiro ring group" herein is consistent with that of a spiro ring.

"Carbo-fused ring", "fused ring carbocyclyl", "fused carbocyclyl" or "carbo-fused ring group" refers to a "fused ring" with a ring system consisting only of carbon atoms. The definition of the "carbo-fused ring", "fused ring carbocyclyl", "fused carbocyclyl" or "carbo-fused ring group" herein is consistent with that of a fused ring.

"Carbo-bridged ring", "bridged ring carbocyclyl", "bridged carbocyclyl" or "carbo-bridged ring group" refers to a "bridged ring" with a ring system consisting only of carbon atoms. The definition of the "carbo-bridged ring", "bridged ring carbocyclyl", "bridged carbocyclyl" or "carbo-bridged ring group" herein is consistent with that of a bridged ring.

"Mono-heterocyclic ring", "monocyclic heterocyclyl" or "mono-heterocyclic ring group" refers to "heterocyclyl " or "heterocycle" with a monocyclic system. The definition of the "heterocyclyl", "monocyclic heterocyclyl" or "mono-heterocyclic ring group" herein is consistent with that of heterocycle.

"Hetero-fused ring", "hetero-fused ring group", "fused ring heterocyclyl" or "hetero-fused ring group" means a "fused ring" containing a heteroatom. The definition of hetero-fused ring, "hetero-fused ring group", "fused ring heterocyclyl" or "hetero-fused ring group" herein is consistent with that of a fused ring.

"Spiro-heterocyclic ring", "spiro-heterocyclic ring group", "spiro ring heterocyclyl" or "spiro-heterocyclic ring group" refers to a "spiro ring" containing a heteroatom. The definition of the "spiro-heterocyclic ring", "spiro-heterocyclic ring group", "spiro ring heterocyclyl" or "spiro-heterocyclic ring group" herein is consistent with that of a spiro ring.

"Bridged-heterocyclic ring", "bridged-heterocyclic ring group", "bridged ring heterocyclyl" or "bridged-heterocyclic ring group" refers to a "bridged ring" containing a heteroatom. The definition of the "bridged-heterocyclic ring", "bridged-heterocyclic ring group", "bridged ring heterocyclyl" or "bridged-heterocyclic ring group" herein is consistent with that of a bridged ring.

"Aryl" or "aromatic ring" refers to a substituted or unsubstituted aromatic hydrocarbyl group with a monocyclic ring or a fused ring, wherein the number of ring atoms in the aromatic ring includes but is not limited to 6 to 18, 6 to 12 or 6 to 10 carbon atoms. The aryl ring can be fused to a saturated or unsaturated carbocycle or heterocycle, wherein the ring connected to the parent structure is an aryl ring. Non-limiting examples include a benzene ring, a naphthalene ring, or

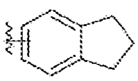, 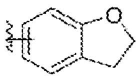, 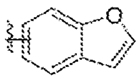, 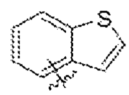, 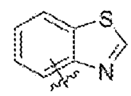, .

"Aryl" or "aromatic ring" can be monovalent, divalent, trivalent or tetravalent. When divalent, trivalent or tetravalent, the point of connection is on the aryl ring.

"Heteroaryl" or "heteroaromatic ring" refers to a substituted or unsubstituted aromatic hydrocarbyl group containing 1 to 5 heteroatoms or groups containing heteroatoms (including but not limited to N, O or S(=O)n, wherein n is 0, 1 or 2), wherein the number of ring atoms in the heteroaromatic ring includes but is not limited to 5-15, 5-10 or 5-6. Non-limiting examples of heteroaryl include, but are not limited to pyridyl, furyl, thienyl, pyridinyl, pyranyl, N-alkylpyrrolyl, pyrimidinyl, pyrazinyl, pyridazinyl, imidazolyl, benzopyrazole, benzimidazolyl, benzopyridine, pyrrolopyridine, etc. The heteroaryl ring may be fused to a saturated or unsaturated carbocycle or heterocycle, wherein the ring connected to the parent structure is an heteroaryl ring. Non-limiting examples include

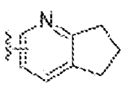, 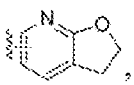, 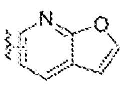, 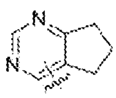, 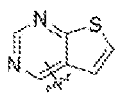, 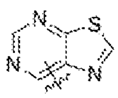, 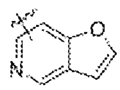

and

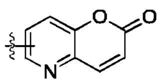.

The definition of the "heteroaryl" herein is consistent with this definition. Heteroaryl can be monovalent, divalent, trivalent or tetravalent. When divalent, trivalent or tetravalent, the point of connection is on the heteroaryl ring.

"5-membered ring fused 5-membered heteroaromatic ring" refers to a 5 fused 5-membered fused heteroaromatic ring, wherein at least one of the two fused rings contains at least one heteroatom (including but not limited to O, S or N), and the entire group is aromatic. Non-limiting examples include a pyrrolopyrrole ring, a pyrazolopyrrole ring, a pyrazolopyrazole ring, a pyrrolofuran ring, a pyrazolofuran ring, a pyrrolothiophene ring and a pyrazolothiophene ring.

"5 fused 6-membered heteroaromatic ring" refers to a 5 fused 6-membered fused heteroaromatic ring, wherein at least one of the two fused rings contains at least one heteroatom (including but not limited to O, S or N), and the entire group is aromatic. Non-limiting examples include a benzo 5-membered heteroaryl and 6-membered heteroaromatic ring fused 5-membered heteroaromatic ring.

"Substitution" or "substituted" refers to a substitution with 1 or more (including but not limited to 2, 3, 4 or 5) substituents including but not limited to H, F, Cl, Br, I, alkyl, cycloalkyl, alkoxy, haloalkyl, mercaptan, hydroxyl, nitro, mercapto, amino, cyano, isocyano, aryl, heteroaryl, heterocyclyl, bridged ring group, spiro ring group, fused ring group, hydroxyalkyl, =O, carbonyl, aldehyde, carboxylic acid, carboxylate, $-(CH_2)_m-C(=O)-R^a$, $-O-(CH_2)_m-C(=O)-R^a$, $-(CH_2)_m-C(=O)-NR^bR^c$, $-(CH_2)_mS(=O)_nR^a$, $-(CH_2)_m$-alkenyl $-R^a$, $OR^d$ or $-(CH_2)_m$-alkynyl - $R^a$ (wherein m and n are 0, 1

or 2), arylthio, thiocarbonyl, silyl, -$NR^bR^c$, etc., wherein $R^b$ and $R^c$ are independently selected from H, hydroxyl, amino, carbonyl, alkyl, alkoxy, cycloalkyl, heterocyclyl, aryl, heteroaryl, sulfonyl, or trifluoromethylsulfonyl. Alternatively, $R^b$ and $R^c$ may form a five- or six-membered cycloalkyl or heterocyclyl; and $R^a$ or $R^d$ is independently selected from aryl, heteroaryl, alkyl, alkoxy, cycloalkyl, heterocyclyl, carbonyl, an ester group, a bridged ring group, a spiro ring group or a fused ring group.

"Containing 1 to 5 heteroatoms selected from O, S or N" means containing 1, 2, 3, 4 or 5 heteroatoms selected from O, S or N.

"Substituted with 0 to X substituents selected from ..." means substituted with 0, 1, 2, 3 ... X substituents selected from ..., wherein X is selected from any integer between 1 and 10. For example, "substituted with 0 to 4 substituents selected from ..." means substituted with 0, 1, 2, 3 or 4 substituents selected from ... For example, "substituted with 0 to 5 substituents selected from ..." means substituted with 0, 1, 2, 3, 4 or 5 substituents selected from ... For example, "bridged-heterocyclic ring is optionally further substituted with 0 to 4 substituents selected from H or F" means that the bridged-heterocyclic ring is optionally further substituted with 0, 1, 2, 3 or 4 substituents selected from H or F.

An X- to Y-membered ring (X is selected from an integer less than Y and greater than or equal to 3, and Y is selected from any integer between 4 and 12) includes X+1-, X+2-, X+3-, X+4-, ..., Y-membered rings. Rings include heterocycle, carbocycle, an aromatic ring, aryl, heteroaryl, cycloalkyl, a mono-heterocyclic ring, a fused heterocyclic ring, a spiro-heterocyclic ring or a bridged-heterocyclic ring. For example, a "4- to 7-membered mono-heterocyclic ring" refers to a 4-, 5-, 6- or 7-membered mono-heterocyclic ring, and a "5- to 10-membered fused heterocyclic ring" refers to a 5-, 6-, 7-, 8-, 9- or 10-membered fused heterocyclic ring.

The term "optional" or "optionally" refers to that the events or circumstances subsequently described may but not necessarily occur, and the description includes the occasions where the events or circumstances occur or do not occur. For example, "alkyl optionally substituted with F" means that the alkyl may but not necessarily be substituted by F, and the description includes the case where the alkyl is substituted with F and the case where the alkyl is not substituted with F.

"Pharmaceutically acceptable salt" or "pharmaceutically acceptable salt thereof' refers to a salt of the compound of the present invention, which salt maintains the biological effectiveness and characteristics of a free acid or a free base, and is obtained by reacting the free acid with a non-toxic inorganic base or organic base, or reacting the free base with a non-toxic inorganic acid or organic acid.

"Pharmaceutical composition" refers to a mixture of one or more compounds of the present invention, or stereoisomers, tautomers, deuterated compounds, solvates, prodrugs, metabolites, pharmaceutically acceptable salts or co-crystals thereof and other chemical components, wherein "other chemical components" refer to pharmaceutically acceptable carriers, excipients and/or one or more other therapeutic agents.

Pharmaceutical compositions are administered in a manner suitable for a disease to be treated (or prevented). Appropriate dosage and suitable duration and frequency of administration will be determined by factors such as patient conditions, the type and severity of the patient's disease, particular forms of active ingredients and administration methods. Optimal dosages can be determined by using experimental models and/or clinical trials.

In some embodiments, the present method involves administering about 0.1 μg to about 500 mg of at least one compound of the present invention/kg body weight of a subject, and more generally using a dosage of about 10 μg to about 200 mg of the compound disclosed in the present application, depending on the physiological response of the subject. For example, the dosage of the compound described in the present application for the treatment and/or prevention of diseases as described in the present application is about 0.001 to about 1 mg/kg body weight of a subject/day, such as about 0.001 mg, about 0.002 mg, about 0.005 mg, about 0.010 mg, 0.015 mg, about 0.020 mg, about 0.025 mg, about 0.050 mg, about 0.075 mg, about 0.1 mg, about 0.15 mg, about 0.2 mg, about 0.25 mg, about 0.5 mg, about 0.75 mg or about 1 mg/kg body weight/day. In some embodiments, the dosage of the compound described in the present application for use in the described methods is about 1 to about 1000 mg/kg body weight of a subject being treated/day, such as about 1 mg, about 2 mg, about 5 mg, about 10 mg, about 15 mg, about 20 mg, about 25 mg, about 50 mg, about 75 mg, about 100 mg, about 150 mg, about 200 mg, about 250 mg, about 500 mg, about 750 mg or about 1000 mg/day.

"Carrier" refers to a material that does not cause significant irritation to an organism and does not eliminate the biological activity and characteristics of a compound administered.

"Excipient" refers to an inert substance added to a pharmaceutical composition to facilitate the administration of a compound. Non-limiting examples include calcium carbonate, calcium phosphate, sugar, starch, cellulose derivatives (including microcrystalline cellulose), gelatine, vegetable oils, polyethylene glycols, diluents, granulating agents, lubricants, adhesives and disintegrants.

"Prodrug" refers to a compound that can be converted into the compound of the present invention with the biological activity by metabolism *in vivo.* The prodrug of the present invention is prepared by modifying an amino or carboxyl group in the compound of the present invention, and the modification can be removed by conventional operations or *in vivo* to obtain a parent compound. When the prodrug of the present invention is administered to a mammalian

individual, the prodrug is split to form a free amino or carboxyl group.

The term "co-crystal" refers to a crystal formed by the combination of active pharmaceutical ingredient (API) and co-crystal former (CCF) under the action of hydrogen bonds or other non-covalent bonds. The pure state of API and CCF are both solid at room temperature, and there is a fixed stoichiometric ratio between various components. The co-crystal is a multi-component crystal, which includes both a binary co-crystal formed between two neutral solids and a multi-element co-crystal formed between a neutral solid and a salt or solvate.

"Animal" is meant to include mammals, such as humans, companion animals, zoo animals, and domestic animals, preferably humans, horses, or dogs.

The term "stereoisomer" refers to an isomer produced as a result of different spatial arrangement of atoms in molecules, including cis-trans isomers, enantiomers and conformational isomers.

"Tautomer" refers to a functional group isomer produced by the rapid movement of an atom between two positions in a molecule, such as keto-enol isomerization and amide-imino alcohol isomerization.

"$IC_{50}$" refers to the concentration of a medicament or inhibitor required to inhibit half of a given biological process (or a component of the process such as an enzyme, a receptor and a cell).

Detailed Description of Embodiments

The technical solutions of the present invention will be described in detail below in conjunction with examples, but the protection scope of the present invention includes but is not limited thereto.

To achieve the objectives of the present invention, according to organic synthesis techniques known to those skilled in the art, and starting from commercially available chemicals and/or compounds described in chemical documents, the prepared compounds, "commercially available chemicals", for use in the reactions described herein are obtained from standard commercial sources, including Shanghai Aladdin Bio-Chem Technology Co., Ltd., Shanghai Macklin Biochemical Co., Ltd., Sigma-Aldrich, Alfa Aesar (China) Chemical Co., Ltd., Tokyo Chemical Industry (Shanghai) Co., Ltd., Energy Chemical Co., Ltd., Shanghai Titan Scientific Co., Ltd., Kelong Chemical Co., Ltd., J&K Scientific and the like.

Synthetic method I:

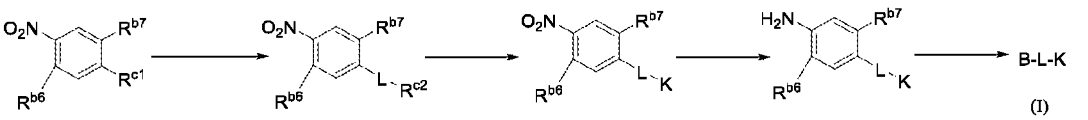

C-1 C-2 C-3 C-4

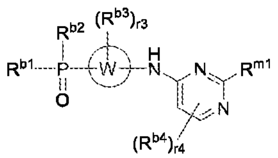

C-7

$R^{c1}$ is selected from halogen;
$R^{c2}$ is selected from H, an amino protecting group or a hydroxyl protecting group;
$R^{m1}$ is selected from a leaving group, preferably halogen, OMs, OTs or OTf,
the definitions of other groups are consistent with those in the description;

A compound of general formula (C-1) is subjected to a nucleophilic substitution reaction or a coupling reaction to obtain a compound of general formula (C-2);

the compound of general formula (C-2) is subjected to a nucleophilic substitution reaction, a coupling reaction, reductive amination or a condensation reaction to obtain a compound of general formula (C-3); or the compound of general formula (C-2) is subjected to protecting group removal, and then subjected to a nucleophilic substitution reaction, a coupling reaction, reductive amination or a condensation reaction to obtain a compound of general formula (C-3);
the compound of general formula (C-3) is subjected to a reduction reaction to obtain a compound of general formula (C-4);

and the compound of general formula (C-4) and compound (C-7) are subjected to a nucleophilic substitution reaction or a coupling reaction to obtain the compound of general formula (I).

Synthetic method II:

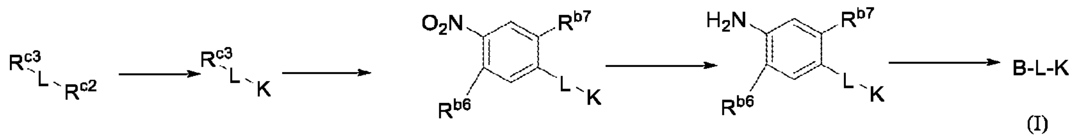

C-5 C-6 C-3 C-4 (I)

$R^{c2}$ is selected from H, an amino protecting group or a hydroxyl protecting group;
$R^{c3}$ is selected from H, an amino protecting group or a hydroxyl protecting group;
the definitions of other groups are consistent with those in the description;

A compound of general formula (C-5) is subjected to a nucleophilic substitution reaction, a coupling reaction, reductive amination or a condensation reaction to obtain a compound of general formula (C-6); or a compound of general formula (C-5) is subjected to protecting group removal, and then subjected to a nucleophilic substitution reaction, a coupling reaction, reductive amination or a condensation reaction to obtain a compound of general formula (C-6);

the compound of general formula (C-6) is subjected to a nucleophilic substitution reaction or a coupling reaction to obtain a compound of general formula (C-3), or the compound of general formula (C-6) is subjected to protecting group removal, and then subjected to a nucleophilic substitution reaction or a coupling reaction to obtain a compound of general formula (C-3);
the compound of general formula (C-3) is subjected to a reduction reaction to obtain a compound of general formula (C-4);
and the compound of general formula (C-4) and compound (C-7) are subjected to a nucleophilic substitution reaction or a coupling reaction to obtain the compound of general formula (I).

The structures of the compounds are determined by nuclear magnetic resonance (NMR) or (and) mass spectrometry (MS). The NMR shift (δ) is given in the unit of $10^{-6}$ (ppm). NMR is determined with (Bruker Avance III 400 and Bruker Avance 300) nuclear magnetic resonance instrument; the solvents for determination are deuterated dimethyl sulfoxide (DMSO-$d_6$), deuterated chloroform ($CDCl_3$) and deuterated methanol ($CD_3OD$); and the internal standard is tetramethylsilane (TMS).

MS is measured with (Agilent 6120B(ESI) and Agilent 6120B(APCI));
HPLC is measured with Agilent 1260DAD high pressure liquid chromatography (Zorbax SB-C18 100 × 4.6 mm, 3.5 μM);
Yantai Huanghai HSGF254 or Qingdao GF254 silica gel plate is used as a thin layer chromatography silica plate, and the silica gel plate for the thin layer chromatography (TLC) is of the specification of 0.15 mm-0.20 mm, and the specification when separating and purifying a product by thin layer chromatography is 0.4 mm - 0.5 mm.

For the column chromatography, Yantai Huanghai silica gel of 200-300 mesh silica gel is generally used as a carrier. SEM:

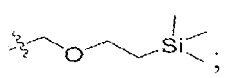

THP:

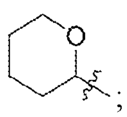

Boc: tert-butoxycarbonyl; Ms:

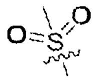;

TBS:

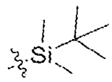;

MTBE: methyl tert-butyl ether; Bn:

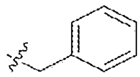;

DIPEA: N,N-diisopropylethylamine; DMAc: N,N-dimethylacetamide; DMSO: dimethyl sulfoxide; DCM: dichloromethane; Cbz:

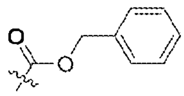;

NMP: N-methylpyrrolidone; DMF: N,N-dimethylformamide; TFA: trifluoroacetic acid F 2-(2,6-dioxopiperidin-3-yl)-5-fluoroisoindoline-1,3-dione:

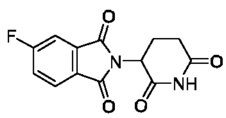

EDCECAS 25952-53-8 HOBT:CAS 2592-95-2 RuPhosPdG3:CAS 1445085-77-7

Pd(dppf)$Cl_2$·DCM:CAS 95464-05-4

Unless otherwise specified, the preparative HPLC of the present invention involves waters 2767 (preparative liquid phase chromatographic instrument) as an instrument, and XBridge@ Prep C18 (30 mm × 150 mm) as a chromatographic column.

Unless otherwise specified, in the preparative purification of the present invention, the solvent IPA refers to isopropanol, CAN refers to acetonitrile, and DEA refers to diethylamine.

**Example 1: Preparation of compound 1**

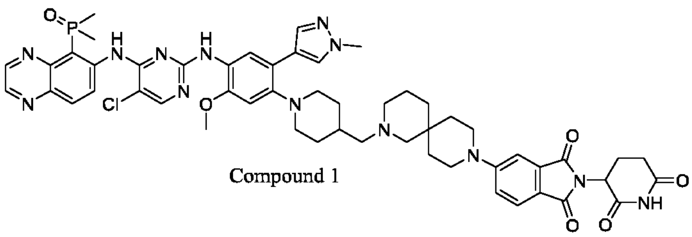

Compound 1

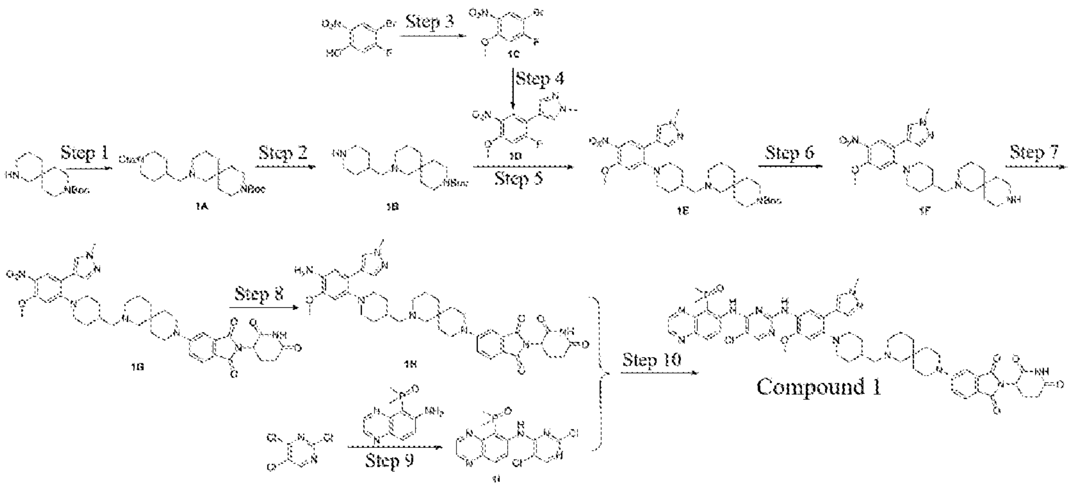


Step 1: tert-butyl 2-((1-((benzyloxy)carbonyl)piperidin-4-yl)methyl)-2,9-diazaspiro[5.5]undecane-9-carboxylate (**1A**)

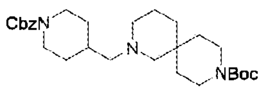

**1A**

Benzyl 4-formylpiperidine-1-carboxylate (2.47 g, 10 mmol) and tert-butyl 2,9-diazaspiro[5.5]undecane-9-carboxylate (2.54 g, 10 mmol) were mixed in dichloromethane (100 mL); acetic acid (1.2 g, 20 mmol) and sodium triacetoxyborohydride (4.24 g, 20 mmol) were successively added; and the mixture was stirred overnight at room temperature. 100 mL of dichloromethane and 50 mL of 1 N aqueous sodium hydroxide solution were added, and then the layers were separated. The organic layer was dried under reduced pressure, and the residue was purified by silica gel column chromatography (mobile phase: ethyl acetate/petroleum ether (V/V) = 10/1-1/1) to obtain **1A** (4.1 g, yield: 84.5%).

LCMS m/z = 486.2 $[M+H]^+$.

Step 2: tert-butyl 2-(piperidin-4-ylmethyl)-2,9-diazaspiro[5.5]undecane-9-carboxylate **(1B)**

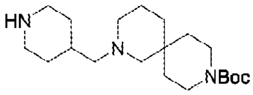

**1B**

**1A** (4.1 g, 0.85 mmol) was dissolved in methanol (30 mL); palladium on carbon (wt% = 10%, 410 mg) was added; the mixture was subjected to hydrogen replacement 3 times, stirred overnight under hydrogen atmosphere (balloon pressure) at room temperature and filtered; and the filtrate was concentrated under reduced pressure to obtain **1B** (2.96 g).

Step 3: 1-bromo-2-fluoro-4-methoxy-5-nitrobenzene **(1C)**

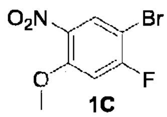

**1C**

4-bromo-5-fluoro-2-nitrophenol (1 g, 4.24 mmol) was dissolved in 10 mL of DMF; potassium carbonate (1.76 g, 12.72 mmol) and iodomethane (1.2 g, 8.48 mmol) were added; and the mixture was reacted at 45°C for 2 h, and cooled to room temperature. The reaction solution was diluted by adding 30 mL of water and subjected to suction filtration under reduced pressure; and the filter cake was **1C** (0.95 g, yield: 90%).

LCMS m/z = 250.0 $[M+H]^+$.

Step 4: 4-(2-fluoro-4-methoxy-5-nitrophenyl)-1-methyl-1H-pyrazole **(1D)**

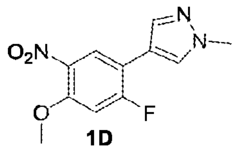

**1D**

Under nitrogen protection, **1C** (500 mg, 2 mmol) and N-methylpyrazole-4-boronic acid (CAS: 847818-55-7, 450 mg, 3.57 mmol) were added to a 50 mL single-necked flask and dissolved in 10 mL of dioxane and 2 mL of water; $Pd(dppf)Cl_2 \cdot DCM$ (160 mg, 0.2 mmol) and potassium carbonate (560 mg, 4 mmol) were added; and the mixture was subjected to nitrogen replacement three times, reacted at 100°C for 2 h and cooled to room temperature. The reaction solution was poured into water and extracted three times with ethyl acetate. The organic phases were combined, dried over anhydrous sodium sulphate and concentrated under reduced pressure, and the residue was purified by silica gel column chromatography to obtain **1D** (400 mg, yield: 80%).

LCMS m/z = 252.1 $[M+H]^+$.

$^1H$ NMR (400 MHz, DMSO-$d_6$) δ 8.27 (d, 1H), 8.18 (s, 1H), 7.92 (s, 1H), 7.40 (d, 1H), 3.95 (s, 3H), 3.89 (s, 3H).

Step 5: tert-butyl 2-((1-(5-methoxy-2-(1-methyl-1H-pyrazol-4-yl)-4-nitrophenyl)piperidin-4-yl)methyl)-2,9-diazaspiro[5.5]undecane-9-carboxylate **(1E)**

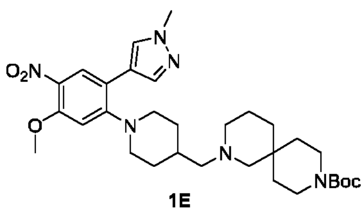

**1E**

**1B** (351 mg, 1 mmol), **1D** (301 mg, 1.2 mmol) and potassium carbonate (414 mg, 3 mmol) were mixed and dissolved in DMSO (10 mL), and the mixture was stirred at 120°C for 16 h, cooled to room temperature and extracted by adding 50 mL of water and 50 mL of ethyl acetate. The organic layer was concentrated under reduced pressure, and the residue was purified by column chromatography (mobile phase: dichloromethane/methanol (V/V) = 100/1-20/1) to obtain **1E** (407 mg, yield: 70%).

LCMS m/z = 583.5 $[M+H]^+$.

Step 6: 2-((1-(5-methoxy-2-(1-methyl-1H-pyrazol-4-yl)-4-nitrophenyl) piperidin-4-yl)methyl)-2,9-diazaspiro[5.5]unde-cane **(1F)**

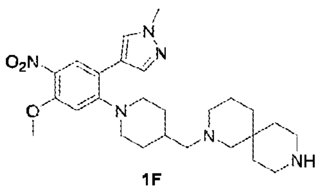

**1F**

**1E** (407 mg, 0.7 mmol) was dissolved in DCM (10 mL); TFA (3 mL) was added at room temperature; and the mixture was stirred for 3 h and concentrated under reduced pressure. 20 mL of DCM was added, and the mixture was adjusted to pH = 9-10 with 1 N aqueous NaOH solution. Liquid separation was performed. The organic layer was concentrated under reduced pressure to obtain **1F** (337 mg). Step 7: 2-(2,6-dioxopiperidin-3-yl)-5-(2-((1-(5-methoxy-2-(1-methyl-1H-pyrazol-4-yl)-4-nitrophenyl)piperidin-4-yl)methyl)-2,9-diazaspiro[5.5]undecan-9-yl)isoindoline-1,3-dione **(1G)**

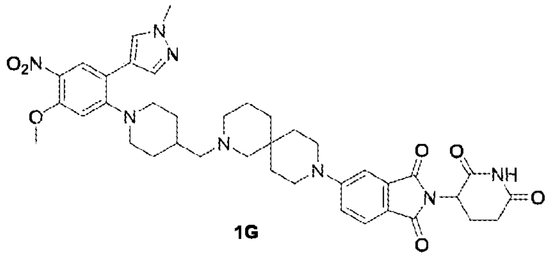

1G

**1F** (337 mg, 0.7 mmol) was dissolved in DMSO (5 mL); 2-(2,6-dioxopiperidin-3-yl)-5-fluoroisoindole-1,3-dione (276 mg, 1 mmol) and DIPEA (0.31 g, 2.4 mmol) were successively added; and the mixture was stirred at 90°C for 3 h, and cooled to room temperature. 10 mL of water was added, and the mixture was filtered. The filter cake was dried under reduced pressure and then purified by silica gel column chromatography (mobile phase: dichloromethane/methanol (V/V) = 100/1-20/1) to obtain **1G** (306 mg, yield: 59.2%).

LCMS m/z = 739.3 $[M+H]^+$.

Step 8: 5-(2-((1-(4-amino-5-methoxy-2-(1-methyl-1H-pyrazol-4-yl)phenyl)piperidin-4-yl)methyl)-2,9-diazaspiro[5.5]undecan-9-yl)-2-(2,6-dioxopiperidin-3-yl)isoindoline-1,3-dione **(1H)**

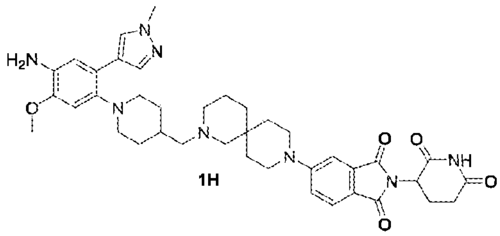

1H

**1G** (306 mg, 4.15 mmol), iron powder (200 mg, 3.57 mmol) and ammonium chloride (200 mg, 3.77 mmol) were dissolved in ethanol (30 mL) and water (10 mL), and the mixture was stirred at 80°C for 2 h, cooled to room temperature and subjected to suction filtration. The filtrate was concentrated to obtain **1H** (250 mg, 86.9%).

LCMS m/z = 355.3 $[(M+2H)/2]^+$.

Step 9: (6-((2,5-dichloropyrimidin-4-yl)amino)quinoxalin-5-yl)dimethylphosphine oxide **(1I)**

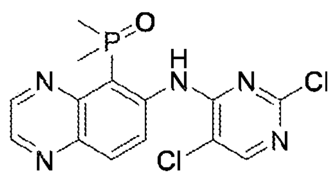

**1I**

(6-aminoquinoxalin-5-yl)dimethylphosphine oxide (2.80 g, 12.66 mmol, see patent WO 2020147838) and 2,4,5-trichloropyrimidine (4.64 g, 25.32 mmol) were dissolved in NMP (15 mL); DIPEA (1.96 g, 5.19 mmol) was added; and under nitrogen protection, the mixture was reacted at 130°C for 6 h, cooled to room temperature and extracted by adding 60 mL of ethyl acetate and 60 mL of water. The organic layer was washed once with 30 mL of saturated brine, dried

over anhydrous sodium sulphate and then concentrated under reduced pressure, and the residue was purified by silica gel column chromatography (ethyl acetate/petroleum ether (V/V) = 1/2-1/0) to obtain **1I** (3 g, yield: 64%).

$^1$H NMR (400 MHz, $CDCl_3$) δ 13.28 (s, 1H), 9.22-9.16 (m, 1H), 8.82 (d,1H), 8.76 (d,1H), 8.37 - 8.22 (m, 2H), 2.15 (s, 3H), 2.12 (s, 3H).

Step 10: 5-(2-((1-(4-((5-chloro-4-((5-(dimethylphosphoryl)quinoxalin-6-yl)amino)pyrimidin-2-yl)amino)-5-methoxy-2-(1-methyl-1H-pyrazol-4-yl)phenyl)piperidin-4-yl)methyl)-2,9-diazaspiro[5.5]undecan-9-yl)-2-(2,6-dioxopiperidin-3-yl)isoindoline-1,3-dione **(compound 1)**

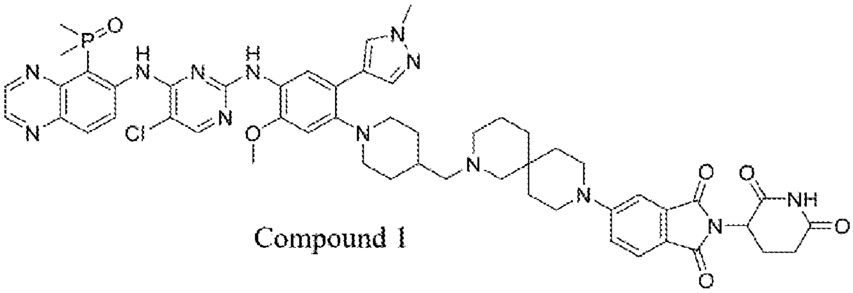

Compound 1

**1H** (180 mg, 0.25 mmol) and **1I** (0.12 g, 0.33 mmol) were dissolved in DMF (5 mL); p-toluenesulfonic acid monohydrate (142 mg, 0.75 mmol) was added; and under nitrogen protection, the mixture was stirred at 100°C for 16 h, and cooled to room temperature. 20 mL of saturated aqueous sodium bicarbonate solution and 50 mL of dichloromethane were added, and the layers were separated. The organic layer was concentrated under reduced pressure, and the residue was purified by silica gel column chromatography (mobile phase: DCM/MeOH (V/V) = 100/1-20/1). The crude was further subjected to preparative HPLC (instrument: waters 2767 (preparative liquid phase chromatographic instrument); chromatographic column: XBridge@ Prep C18 (30 mm × 150 mm); composition of mobile phases: mobile phase A: acetonitrile, and mobile phase B: water (containing 0.1% TFA)) purification to obtain a preparative product, which was alkalized with saturated aqueous sodium bicarbonate solution to obtain **compound 1** (53 mg, yield: 20.1%).

LCMS m/z = 520.8 $[(M+2H)/2]^+$.

$^1$H NMR (400 MHz, DMSO-$d_6$) δ 12.88 (s, 1H), 11.04 (s, 1H), 9.03 - 8.91 (m, 1H), 8.86 - 8.73 (m, 2H), 8.37 (s, 1H), 8.19 (s, 1H), 7.99 (s, 1H), 7.81 (s, 1H), 7.71 - 7.44 (m, 3H), 7.33 - 7.25 (m, 1H), 7.20 (dd, 1H), 6.83 (s, 1H), 5.05 (dd, 1H), 3.79 (s, 3H), 3.76 (s, 3H), 3.55 - 3.39 (m, 4H), 3.14 - 3.04 (m, 2H), 2.94 - 2.82 (m, 1H), 2.72 - 2.51 (m, 6H), 2.37 - 2.31 (m, 1H), 2.25 - 2.13 (m, 3H), 2.08 - 1.94 (m, 7H), 1.81 - 1.72 (m, 2H), 1.70 - 1.47 (m, 7H), 1.39 - 1.28 (m, 4H).

**Example 2: Preparation of compound 2**

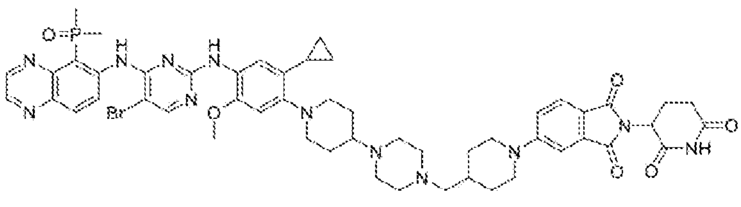

Compound 2

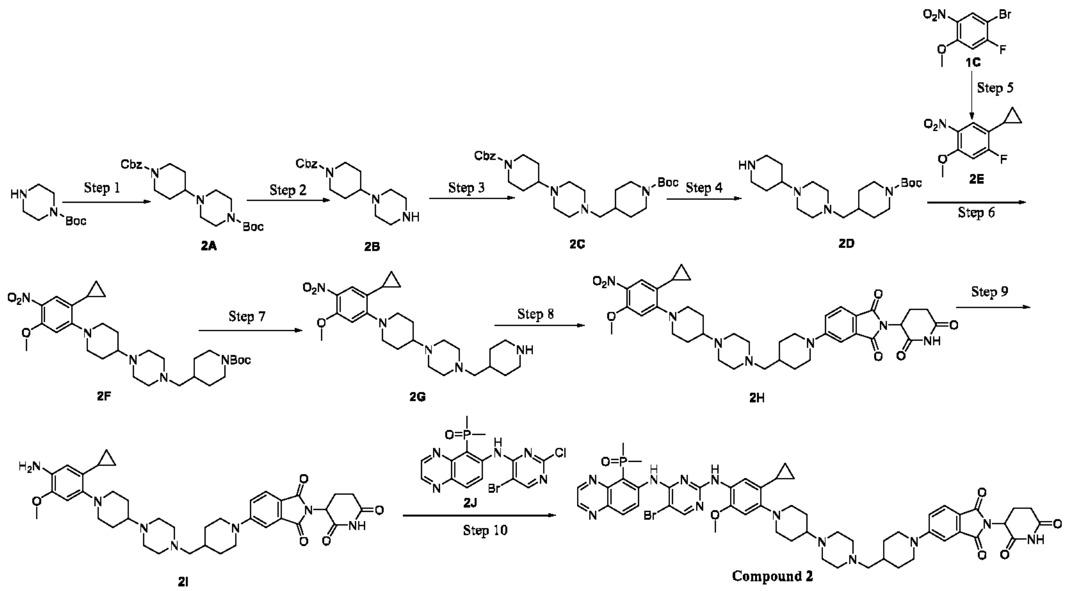


Step 1: tert-butyl 4-(1-[(benzyloxy)carbonyl]piperidin-4-yl)piperazine-1-carboxylate **(2A)**

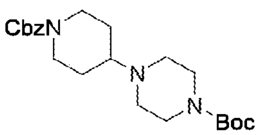

2A

Tert-butyl piperazine-1-carboxylate (20.00 g, 107.38 mmol), 1-Cbz-4-piperidone (20.05 g, 107.38 mmol) and anhydrous sodium sulphate (15.25 g, 107.38 mmol) were mixed in dichloromethane (500 mL); acetic acid (12.90 g, 214.76 mmol) and sodium triacetoxyborohydride (45.52 g, 214.76 mmol) were successively added; and the mixture was reacted at room temperature for 2 h, adjusted to a basic pH by adding 1 N aqueous sodium hydroxide solution and extracted 3 times with dichloromethane. The organic phases were combined, dried over anhydrous sodium sulphate and concentrated under reduced pressure, and the residue was purified by silica gel column chromatography (mobile phase: ethyl acetate/petroleum ether (V/V) = 10/1-1/1) to obtain **2A** (39.6 g, yield: 82%).

LCMS m/z = 404.2 $[M+H]^+$.

Step 2: benzyl 4-(piperazin-1-yl)piperidine-1-carboxylate **(2B)**

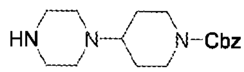

2B

**2A** (20.00 g, 44.61 mmol) was dissolved in dichloromethane (120 mL); trifluoroacetic acid (40 mL) was slowly added; and the mixture was reacted at room temperature for 1 h, concentrated under reduced pressure to remove dichloromethane and trifluoroacetic acid, and slurried by adding methyl tert-butyl ether to obtain a white solid, which was then dissolved by adding 200 mL of dichloromethane. The resulting mixture was adjusted to a basic pH by adding 1 N aqueous sodium hydroxide solution. Liquid separation was performed. The organic phases were collected, and the aqueous phase was extracted 3 times with dichloromethane. All organic phases were combined, dried over anhydrous sodium sulphate and concentrated under reduced pressure to obtain **2B** (10.0 g, yield: 74%), which was directly used in the next step.

LCMS m/z = 304.2 $[M+H]^+$.

Step 3: benzyl 4-(4-[(1-[(tert-butoxy)carbonyl]piperidin-4-yl)methyl]piperazin-1-yl) piperidine-1-carboxylate **(2C)**

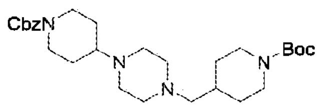

2C

**2B** (10.00 g, 32.96 mmol) and tert-butyl 4-formylpiperidine-1-carboxylate (7.03 g, 32.96 mmol) were dissolved in dichloromethane (120 mL); acetic acid (3.96 g, 65.92 mmol) and sodium triacetoxyborohydride (13.97 g, 65.92 mmol) were successively added; and the mixture was reacted at room temperature for 1 h, adjusted to a basic pH by adding 1 N aqueous sodium hydroxide solution and extracted 3 times with dichloromethane. The organic phases were combined, dried over anhydrous sodium sulphate and concentrated under reduced pressure, and the residue was purified by silica gel column chromatography (mobile phase: ethyl acetate/petroleum ether (V/V) = 1/1 to dichloromethane/methanol (V/V) = 20/1) to obtain **2C** (16.14 g, yield: 98%).

LCMS m/z = 501.1 $[M+H]^+$.

Step 4: tert-butyl 4-[(4-(piperidin-4-yl)piperazin-1-yl)methyl]piperidine-1-carboxylate **(2D)**

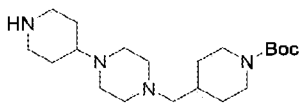

2D

**2C** (6.00 g, 11.98 mmol) was dissolved in a mixed solvent of isopropanol (100 mL) and ammonia methanol solution (25 mL); palladium on carbon (wt% = 10%, 2.93 g) was added; and the mixture was subjected to 1 atm hydrogen replacement 3 times, reacted at room temperature for 1.5 h and filtered over celite. The filter cake was washed with dichloromethane/methanol (V/V = 10/1), and the filtrate was concentrated under reduced pressure to obtain the crude of **2D** (4.3 g), which was directly used in the next step.

LCMS m/z = 367.3 $[M+H]^+$.

Step 5: 1-cyclopropyl-2-fluoro-4-methoxy-5-nitrobenzene **(2E)**

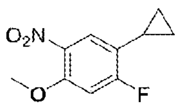

2E

Under nitrogen protection, **1C** (3.0 g, 12.0 mmol), cyclopropyl boronic acid (2.06 g, 24.0 mmol), potassium carbonate (4.98 g, 36.0 mmol) and $Pd(dppf)Cl_2 \cdot DCM$ (880 mg, 1.2 mmol) were added to a round bottom flask; 1,4-dioxane (40 mL) and water (5 mL) were added; and the mixture was reacted at 100°C for 3 h. The reaction solution was cooled to room temperature and subjected to suction filtration over celite. The filtrate was extracted with ethyl acetate (30 mL × 3), dried over anhydrous sodium sulphate and concentrated, and the residue was purified by silica gel column chromatography (PE : EA = 20 : 1) to obtain **2E** (1.8 g, yield: 70%).

$^1H$ NMR (400 MHz, $CDCl_3$) δ 7.55 (d, 1H), 6.75 (d, 1H), 3.93 (s, 3H), 2.07 - 1.92 (m, 1H), 1.07 - 0.92 (m, 2H), 0.76 - 0.64 (m, 2H).

Step 6: tert-butyl 4-[(4-(1-(2-cyclopropyl-5-methoxy-4-nitrophenyl)piperidin-4-yl)piperazin-1-yl) methyl]piperidine-1-carboxylate **(2F)**

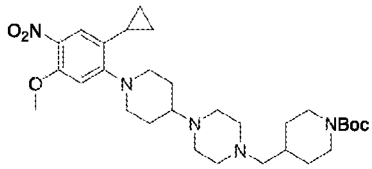

2F

**2E** (0.3 g, 1.42 mmol) and **2D** (0.78 g, 2.13 mmol) were dissolved in DMSO (10 mL); potassium carbonate (0.59 g, 4.26 mmol) was added; and the mixture was reacted at 120°C for 6 h, cooled to room temperature and extracted by adding 30 mL of ethyl acetate and 30 mL of water. The organic layer was washed three times with saturated brine (20 mL × 3), dried over anhydrous sodium sulphate and concentrated under reduced pressure, and the residue was purified by column chromatography (dichloromethane: methanol (V/V) = 100/1-20/1) to obtain **2F** (0.5 g, yield: 63%).
LCMS m/z = 558.5 $[M+H]^+$.

Step 7: 1-(1-(2-cyclopropyl-5-methoxy-4-nitrophenyl)piperidin-4-yl)-4-[(piperidin-4-yl)methyl]piperazine **(2G)**

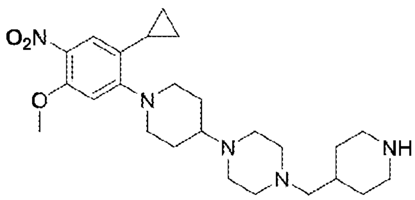

2G

**2F** (0.5 g, 0.9 mmol) was dissolved in dichloromethane (6 mL); trifluoroacetic acid (3.06 g, 26.84 mmol) was added at room temperature; and the mixture was stirred at room temperature for 2 h, and concentrated under reduced pressure. 20 mL of dichloromethane was added to the residue, and the mixture was adjusted to pH>10 with 1 N aqueous sodium hydroxide solution. Liquid separation was performed. The organic layer was then dried over anhydrous sodium sulphate and concentrated under reduced pressure to obtain the crude of **2G** (0.38 g).

Step 8: 5-(4-[(4-(1-(2-cyclopropyl-5-methoxy-4-nitrophenyl)piperidin-4-yl)piperazin-1-yl)methyl]piperidin-1-yl)-2-(2,6-dioxopiperidin-3-yl)-2,3-dihydro-1H-isoindole-1,3-dione **(2H)**

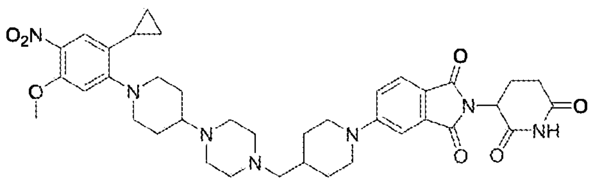

2H

**2G** (0.38 g, 0.83 mmol) and 2-(2,6-dioxopiperidin-3-yl)-5-fluoroisoindole-1,3-dione (0.25 g, 0.91 mmol) were dissolved in DMSO (10 mL); DIPEA (0.21 g, 1.66 mmol) was added; and the mixture was stirred at 90°C for 5 h and cooled to room temperature. 20 mL of water was added, and the mixture was filtered. The filter cake was dried under reduced pressure and then purified by silica gel column chromatography (dichloromethane : methanol (V/V) = 100/1-30/1) to obtain **2H** (0.46 g, yield: 77%)

Step 9: 5-(4-[(4-(1-(4-amino-2-cyclopropyl-5-methoxyphenyl)piperidin-4-yl)piperazin-1-yl)methyl]piperidin-1-yl)-2-(2,6-dioxopiperidin-3-yl)-2,3-dihydro-1H-isoindole-1,3-dione **(2I)**

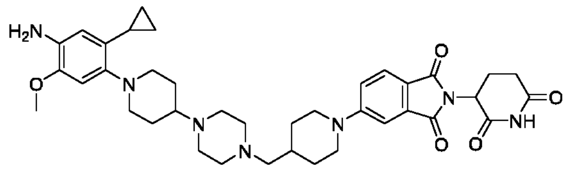

**2I**

**2H** (0.2 g, 0.28 mmol) was dissolved in ethanol (12 mL) and water (3 mL), and the mixture was warmed to 80°C. A mixture of ammonium chloride (0.22 g, 4.05 mmol) and iron powder (0.23 g, 4.05 mmol) was added, and the resulting mixture was stirred at 80°C for 0.5 h, cooled to room temperature and filtered. The filter cake was washed with 50 mL of dichloromethane. 10 mL of saturated brine was added to the filtrate. Liquid separation was performed. The organic layer was dried over anhydrous sodium sulphate and concentrated under reduced pressure to obtain the crude of **2I** (190 mg), which was directly used in the next step.

LCMS m/z = 684.5 $[M+H]^+$.

Step 10: 5-(4-((4-(1-(4-((5-bromo-4-((5-(dimethylphosphoryl)quinoxalin-6-yl)amino)pyrimidin-2-yl)amino)-2-cyclopropyl-5-methoxyphenyl)piperidin-4-yl)piperazin-1-yl)methyl)piperidin-1-yl)-2-(2,6-dioxopiperidin-3-yl)isoindoline-1,3-dione **(compound 2)**

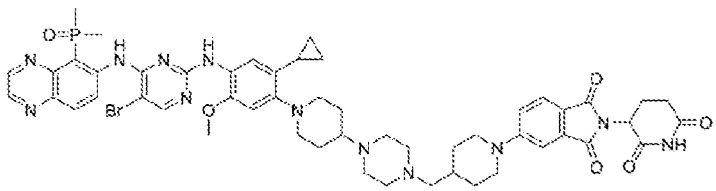

Compound 2

**21** (0.19 g, 0.28 mmol) and (6-((5-bromo-2-chloropyrimidin-4-yl)amino)quinoxalin-5-yl)dimethylphosphine oxide **(2J)** (0.12 g, 0.28 mmol, see patent WO 2020147838) were dissolved in DMF (5 mL); p-toluenesulfonic acid monohydrate (0.16 g, 0.84 mmol) was added; and under nitrogen protection, the mixture was stirred at 100°C for 16 h, and cooled to room temperature. 5 mL of water and 5 mL of saturated aqueous sodium bicarbonate solution were added, and the mixture was filtered. The filter cake was dried under reduced pressure and then purified by silica gel column chromatography (dichloromethane/methanol (V/V) = 100/1-12/1) to obtain a product, which was further purified by preparative HPLC (composition of mobile phases: mobile phase A: acetonitrile, and mobile phase B: water (containing 0.05% ammonia water)) to obtain **compound 2** (100 mg, yield: 33%).

LCMS m/z = 530.3 $[(M+2H)/2]^+$.

$^1H$ NMR (400 MHz, DMSO-$d_6$) δ 12.64 (s, 1H), 11.05 (s, 1H), 8.92 - 8.72 (m, 3H), 8.34 - 8.19 (m, 2H), 7.87 (d, 1H), 7.68 - 7.58 (m, 1H), 7.36 - 7.15 (m, 2H), 6.87 (s, 1H), 6.74 (s, 1H), 5.06 (dd, 1H), 4.03 (d, 2H), 3.76 (s, 3H), 3.38 - 3.18 (m, 2H), 3.02 - 2.82 (m, 3H), 2.70 (t, 2H), 2.64 - 2.51 (m, 6H), 2.44 - 2.25 (m, 5H), 2.18 - 2.06 (m, 3H), 2.06 - 1.95 (m, 7H), 1.93 - 1.74 (m, 5H), 1.69 - 1.55 (m, 2H), 1.21 - 1.06 (m, 2H), 0.78 - 0.66 (m, 2H), 0.47 - 0.31 (m, 2H).

**Example 3: Preparation of compound 3**

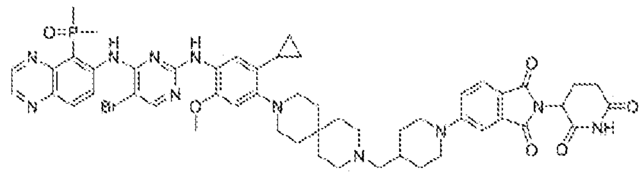

Compound 3

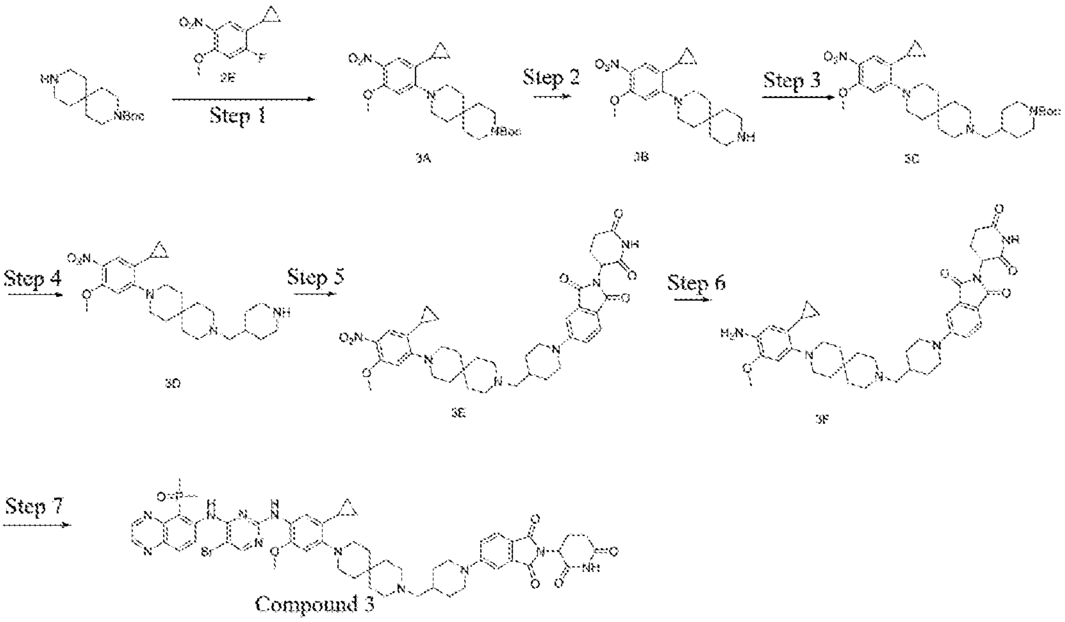


Step 1: tert-butyl 9-(2-cyclopropyl-5-methoxy-4-nitrophenyl)-3,9-diazaspiro[5.5] undecane-3-carboxylate (3A)

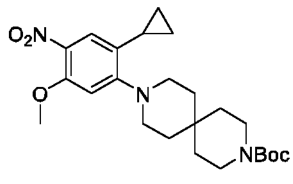

**3A**

**2E** (0.3 g, 1.42 mmol) and tert-butyl 3,9-diazaspiro[5.5]undecane-3-carboxylate (0.43 g, 1.69 mmol) were dissolved in DMSO (10 mL); potassium carbonate (0.59 g, 4.26 mmol) was added; and the mixture was reacted at 120°C for 6 h, cooled to room temperature and extracted by adding 30 mL of ethyl acetate and 30 mL of water. The organic layer was washed with saturated brine (20 mL × 3), dried over anhydrous sodium sulphate and concentrated under reduced pressure, and the residue was purified by column chromatography (ethyl acetate : petroleum ether (V/V) = 10/1-2/1) to obtain **3A** (0.6 g, yield: 95%).

LCMS m/z = 446.3 $[M+H]^+$.

Step 2: 3-(2-cyclopropyl-5-methoxy-4-nitrophenyl)-3,9-diazaspiro[5.5]undecane **(3B)**

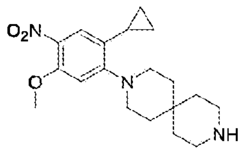

3B

3A (0.6 g, 1.35 mmol) was dissolved in dichloromethane (6 mL); trifluoroacetic acid (3.06 g, 26.84 mmol) was added at room temperature; and the mixture was stirred at room temperature for 2 h, and concentrated under reduced pressure. 20 mL of dichloromethane was added to the residue, and the mixture was adjusted to pH>10 with 1 N aqueous sodium hydroxide solution. Liquid separation was performed. The organic layer was then dried over anhydrous sodium sulphate and concentrated under reduced pressure to obtain the crude of 3B (0.4 g).

Step 3: tert-butyl 4-((9-(2-cyclopropyl-5-methoxy-4-nitrophenyl)-3,9-diazaspiro[5.5] undecan-3-yl)methyl)piperidine-1-carboxylate **(3C)**

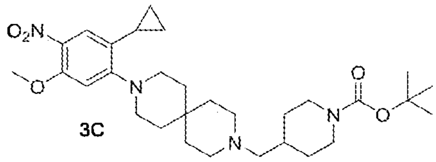

3C

**3B** (0.4 g, 1.16 mmol) and tert-butyl 4-formylpiperidine-1-carboxylate (0.3 g, 1.39 mmol) were dissolved in dichloromethane (10 mL); acetic acid (0.14 g, 2.32 mmol) and sodium triacetoxyborohydride (0.49 g, 2.32 mmol) were successively added; and the mixture was reacted at room temperature for 1 h, adjusted to a basic pH by adding 1 N aqueous sodium hydroxide solution and extracted 3 times with dichloromethane. The organic phases were combined, dried over anhydrous sodium sulphate and concentrated under reduced pressure, and the residue was purified by silica gel column chromatography (mobile phase : ethyl acetate/petroleum ether (V/V) = 1/1 to dichloromethane/methanol (V/V) = 20/1) to obtain **3C** (0.52 g, yield: 82%).

LCMS m/z = 543.3$[M+H]^+$.

Step 4: 3-(2-cyclopropyl-5-methoxy-4-nitrophenyl)-9-(piperidin-4-ylmethyl)-3,9-diazaspiro[5.5]undecane **(3D)**

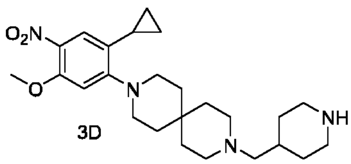

3D

**3C** (0.52 g, 0.96 mmol) was dissolved in dichloromethane (6 mL); trifluoroacetic acid (3.06 g, 26.84 mmol) was added at room temperature; and the mixture was stirred at room temperature for 2 h, and concentrated under reduced pressure. 20 mL of dichloromethane was added to the residue, and the mixture was adjusted to pH>10 with 1 N aqueous sodium hydroxide solution. Liquid separation was performed. The organic layer was then dried over anhydrous sodium sulphate and concentrated under reduced pressure to obtain the crude of **3D** (0.4 g).

Step 5: 5-(4-((9-(2-cyclopropyl-5-methoxy-4-nitrophenyl)-3,9-diazaspiro [5.5]undecan-3-yl)methyl) piperidin-1-yl)-2-(2,6-dioxopiperidin-3-yl)isoindoline-1,3-dione **(3E)**

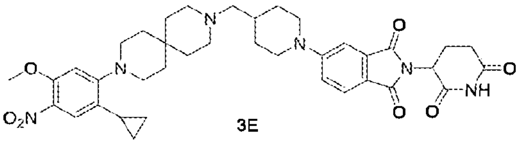

3E

**3D** (0.4 g, 0.90 mmol) and 2-(2,6-dioxopiperidin-3-yl)-5-fluoroisoindole-1,3-dione (0.25 g, 0.91 mmol) were dissolved in DMSO (10 mL); DIPEA (0.21 g, 1.66 mmol) was added; and the mixture was stirred at 90°C for 5 h and cooled to room temperature. 20 mL of water was added, with a solid precipitated, and the mixture was filtered. The filter cake was dried under reduced pressure and then purified by silica gel column chromatography (dichloromethane : methanol (V/V) = 100/1-30/1) to obtain **3E** (0.45 g, yield: 71%).
LCMS m/z = 699.3 $[M+H]^+$.

Step 6: 5-(4-((9-(4-amino-2-cyclopropyl-5-methoxyphenyl)-3,9-diazaspiro [5.5]undecan-3-yl)methyl)piperidin-1-yl)-2-(2,6-dioxopiperidin-3-yl)isoindoline-1,3-dione **(3F)**

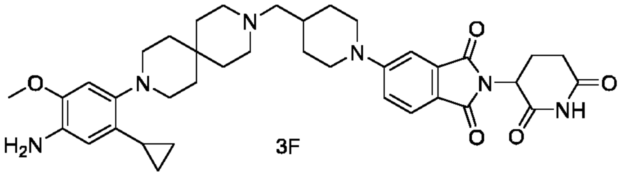

3F

**3E** (0.13 g, 0.19 mmol) was dissolved in ethanol (10 mL) and water (2 mL), and the mixture was warmed to 80°C. A mixture of ammonium chloride (100 mg, 1.90 mmol) and iron powder (106 mg, 1.90 mmol) was added. The resulting mixture was stirred at 80°C for 1 h, cooled to room temperature and filtered. The filter cake was washed with 50 mL of dichloromethane. 10 mL of saturated brine was added to the filtrate. Liquid separation was performed. The organic layer was dried over anhydrous sodium sulphate and concentrated under reduced pressure to obtain the crude of **3F** (120 mg), which was directly used in the next step.
LCMS m/z = 669.3 $[M+H]^+$.

Step 7: 5-(4-((9-(4-((5-bromo-4-((5-(dimethylphosphoryl)quinoxalin-6-yl)amino) pyrimidin-2-yl)amino)-2-cyclopropyl-5-methoxyphenyl)-3,9-diazaspiro[5.5]undecan-3-yl)methyl)piperidin-1-yl)-2-(2,6-dioxopiperidin-3-yl)isoindoline-1,3-dione **(compound 3)**

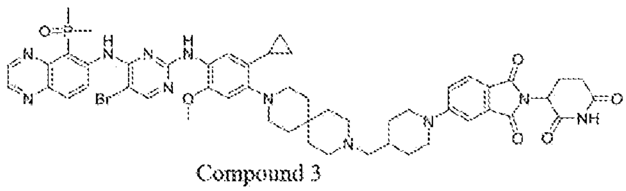

Compound 3

**3F** (0.12 g, 0.18 mmol) and **2J** (74 mg, 0.18 mmol) were dissolved in DMF (5 mL); p-toluenesulfonic acid monohydrate (102 mg, 0.54 mmol) was added; and under nitrogen protection, the mixture was stirred at 100°C for 16 h, and cooled to room temperature. 5 mL of water and 5 mL of saturated aqueous sodium bicarbonate solution were added, and the mixture was filtered. The filter cake was dried under reduced pressure and then purified by silica gel column chromatography (dichloromethane/methanol (V/V) = 100/1-12/1) to obtain the crude (0.10 g), which was further purified by preparative HPLC (composition of mobile phases: mobile phase A: acetonitrile, and mobile phase B: water (containing 0.05% ammonia water)) to obtain **compound 3** (58 mg, yield: 31%).
LCMS m/z = 522.9 $[(M+2H)/2]^+$.
$^1$H NMR (400 MHz, DMSO-$d_6$) δ 12.63 (s, 1H), 11.05 (s, 1H), 8.89 - 8.71 (m, 3H), 8.32 - 8.17 (m, 2H), 7.88 (d, 1H), 7.65 (d, 1H), 7.37 - 7.16 (m, 2H), 6.90 - 6.74 (m, 2H), 5.06 (dd, 1H), 4.12 - 3.97 (m, 2H), 3.77 (s, 3H), 3.04 - 2.81 (m, 7H), 2.68 - 2.52 (m, 2H), 2.44 - 2.30 (m, 4H), 2.21 - 2.06 (m, 3H), 2.06 - 1.97 (m, 7H), 1.89 - 1.73 (m, 3H), 1.69 -

1.46 (m, 8H), 1.21 - 1.07 (m, 2H), 0.80 - 0.60 (m, 2H), 0.51 - 0.27 (m, 2H).

**Example 4: Preparation of compound 4**

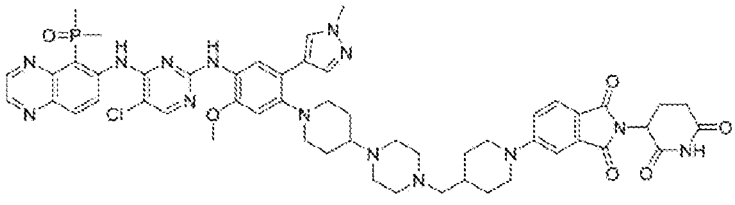

Compound 4

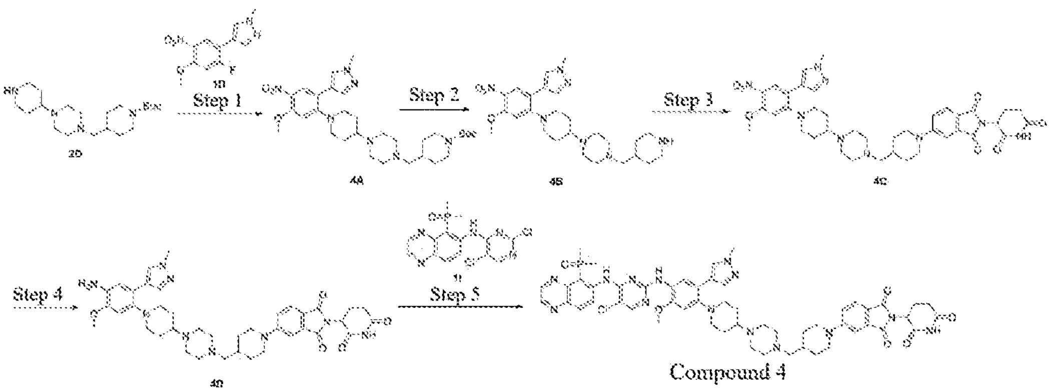


Step 1: tert-butyl 4-((4-(1-(5-methoxy-2-(1-methyl-1H-pyrazol-4-yl)-4-nitrophenyl)piperidin-4-yl)piperazin-1-yl)methyl)piperidine-1-carboxylate **(4A)**

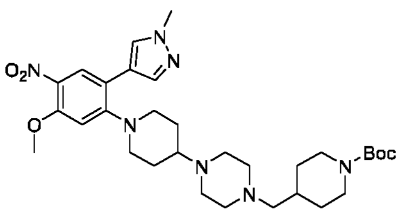

**4A**

**1D** (0.30 g, 1.19 mmol) and **2D** (0.44 g, 1.19 mmol) were dissolved in DMSO (15 mL); potassium carbonate (0.33 g, 2.38 mmol) was added; and the mixture was reacted at 120°C for 4 h. The reaction solution was cooled to room temperature. 20 mL of water was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase : dichloromethane/methanol (V/V) = 50/1-15/1) to obtain **4A** (0.28 g, yield: 40%).

LCMS m/z = 598.5 $[M+H]^+$.

Step 2: 1-(1-(5-methoxy-2-(1-methyl-1H-pyrazol-4-yl)-4-nitrophenyl) piperidin-4-yl)-4-(piperidin-4-ylmethyl)piperazine **(4B)**

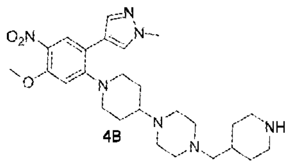

4B

**4A** (0.30 g, 0.50 mmol) was dissolved in dichloromethane (12 mL); trifluoroacetic acid (4 mL) was slowly added; and the mixture was reacted at room temperature for 1.5 h, concentrated under reduced pressure to remove dichloromethane and trifluoroacetic acid, redissolved by adding 50 mL of dichloromethane, and adjusted to a basic pH with 1 N aqueous sodium hydroxide solution. Liquid separation was performed. The organic phases were collected, and the aqueous phase was extracted 3 times with dichloromethane. All organic phases were combined, dried over anhydrous sodium sulphate and concentrated under reduced pressure to obtain **4B** (0.24 g), which was directly used in the next step.
LCMS m/z = 498.4 $[M+H]^+$.

Step 3: 2-(2,6-dioxopiperidin-3-yl)-5-(4-((4-(1-(5-methoxy-2-(1-methyl-1H-pyrazol-4-yl)-4-nitrophenyl)piperidin-4-yl)piperazin-1-yl)methyl)piperidin-1-yl)isoindoline-1,3-dione **(4C)**

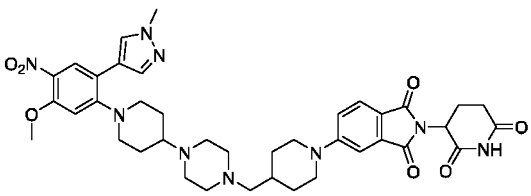

4C

**4B** (0.22 g, 0.45 mmol) and 2-(2,6-dioxopiperidin-3-yl)-5-fluoroisoindole-1,3-dione (0.12 g, 0.45 mmol) were dissolved in DMSO (10 mL); DIPEA (0.17 g, 1.35 mmol) was added dropwise; and the mixture was reacted at 90°C for 3 h. The reaction solution was cooled to room temperature. 20 mL of water was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase: dichloromethane/methanol (V/V) = 50/1-12/1) to obtain **4C** (0.13 g, yield: 34%).
LCMS m/z = 754.4 $[M+H]^+$.

Step 4: 5-(4-((4-(1-(4-amino-5-methoxy-2-(1-methyl-1H-pyrazol-4-yl)phenyl) piperidin-4-yl)piperazin-1-yl)methyl)piperidin-1-yl)-2-(2,6-dioxopiperidin-3-yl)isoindoline-1,3-dione **(4D)**

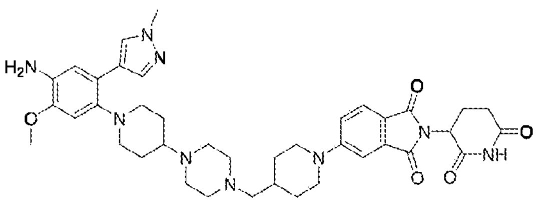

4D

**4C** (0.12 g, 0.15 mmol) was dissolved in ethanol (9 mL); reduced iron powder (0.04 g, 0.75 mmol) was added, and then an aqueous solution (3 mL) of ammonium chloride (0.04 g, 0.75 mmol) was added dropwise; and under nitrogen protection, the mixture was reacted at 80°C for 3 h. The reaction solution was cooled to room temperature. 5 mL of water was added, and then the mixture was extracted 3 times with dichloromethane. The organic phases were combined, dried over anhydrous sodium sulphate and then concentrated under reduced pressure to obtain **4D** (0.11 g).
LCMS m/z = 724.5 $[M+H]^+$.

Step 5: 5-(4-((4-(1-(4-((5-chloro-4-((5-(dimethylphosphoryl)quinoxalin-6-yl)amino)pyrimidin-2-yl)amino)-5-methoxy-2-(1-methyl-1H-pyrazol-4-yl)phenyl)piperidin-4-yl)piperazin-1-yl)methyl)piperidin-1-yl)-2-(2,6-dioxopiperidin-3-yl)isoindoline-1,3-dione **(compound 4)**

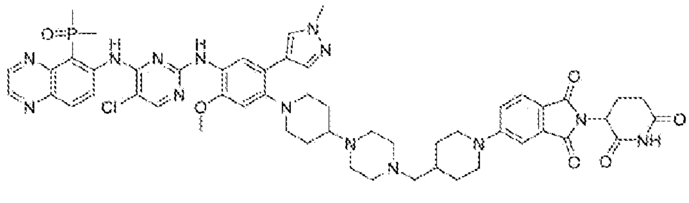

Compound 4

**4D** (0.11 g, 0.16 mmol) and **1I** (0.06 g, 0.16 mmol) were dissolved in DMF (10 mL); p-toluenesulfonic acid hydrate (0.09 g, 0.48 mmol) was added; and under nitrogen protection, the mixture was reacted at 100°C for 16 h. The reaction solution was cooled to room temperature. 10 mL of saturated aqueous sodium bicarbonate solution was added, with a yellow solid precipitated, and the mixture was subjected to suction filtration. The filter cake was washed 3 times with water, redissolved in DCM, dried over anhydrous sodium sulphate and concentrated under reduced pressure, and the residue was purified by column chromatography (mobile phase : dichloromethane/methanol (V/V) = 50/1-12/1) to obtain the crude, which was further purified by preparative HPLC (composition of mobile phases: mobile phase A: acetonitrile, and mobile phase B: water (containing 0.05% ammonia water)) to obtain **compound 4** (36 mg, yield: 21%).

LCMS m/z = 528.3 $[(M+2H)/2]^+$.

$^1H$ NMR (400 MHz, DMSO-$d_6$) δ 12.89 (s, 1H), 11.05 (s, 1H), 9.02 - 8.93 (m, 1H), 8.88 - 8.78 (m, 2H), 8.37 (s, 1H), 8.19 (s, 1H), 8.00 (s, 1H), 7.79 (s, 1H), 7.65 (d, 1H), 7.61 - 7.50 (m, 2H), 7.33 - 7.28 (m, 1H), 7.23 (dd, 1H), 6.84 (s, 1H), 5.06 (dd, 1H), 4.03 (d, 2H), 3.80 (s, 3H), 3.78 (s, 3H), 3.17 - 3.10 (m, 2H), 3.01 - 2.93 (m, 2H), 2.90 - 2.83 (m, 1H), 2.68 - 2.51 (m, 8H), 2.44 - 2.34 (m, 4H), 2.27 - 2.21 (m, 1H), 2.17 - 2.10 (m, 2H), 2.08 - 1.95 (m, 7H), 1.88 - 1.77 (m, 5H), 1.62 - 1.53 (m, 2H), 1.18 - 1.08 (m, 2H).

**Example 5: Preparation of compound 5**

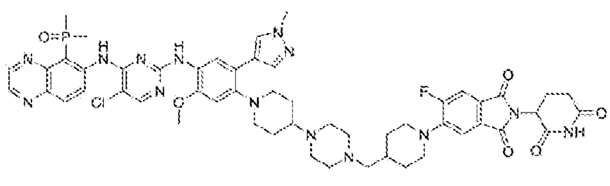

Compound 5

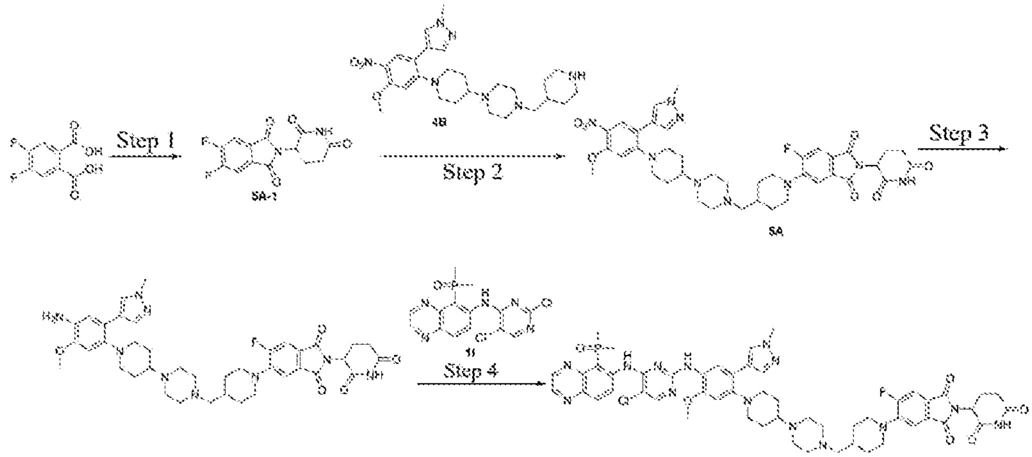


5B

Compound 5

Step 1: 2-(2,6-dioxopiperidin-3-yl)-5,6-difluoroisoindoline-1,3-dione **(5A-1)**

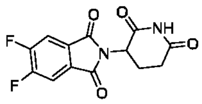

5A-1

4,5-difluorophthalic acid (500 mg, 2.47 mmol) was dissolved in 25 mL of acetonitrile; 3-aminopiperidine-2,6-dione hydrochloride (0.41 g, 2.49 mmol) and N,N'-carbonyldiimidazole (0.8 g, 4.94 mmol) were added; and the reaction was refluxed for 4 h. The reaction system was concentrated under reduced pressure, and then the residue was separated and purified by silica gel column chromatography (petroleum ether/ethyl acetate (v/v) = 2 : 1) to obtain **5A-1** (0.4 g, yield :55.0%).

LCMS m/z = 295.2 $[M+H]^+$.

Step 2: 2-(2,6-dioxopiperidin-3-yl)-5-fluoro-6-(4-((4-(1-(5-methoxy-2-(1-methyl -1H-pyrazol-4-yl)-4-nitrophenyl)piperidin-4-yl)piperazin-1-yl)methyl)piperidin-1-yl)isoindoline-1,3-dione **(5A)**

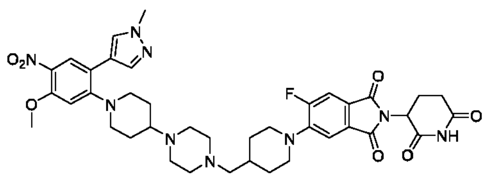

5A

**4B** (0.16 g, 0.54 mmol) and **5A-1** (0.27 g, 0.54 mmol) were dissolved in DMSO (10 mL); DIPEA (0.21 g, 1.62 mmol) was added dropwise; and the mixture was reacted at 90°C for 3 h. The reaction solution was cooled to room temperature. 20 mL of water was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase : dichloromethane/methanol (V/V) = 50/1-12/1) to obtain **5A** as a yellow solid (0.22 g, yield: 53%).

LCMS m/z = 772.4 $[M+H]^+$.

Step 3: 5-(4-((4-(1-(4-amino-5-methoxy-2-(1-methyl-1H-pyrazol-4-yl)phenyl) piperidin-4-yl)piperazin-1-yl)methyl)piperidin-1-yl)-2-(2,6-dioxopiperidin-3-yl)-6-fluoroisoindoline-1,3-dione **(5B)**

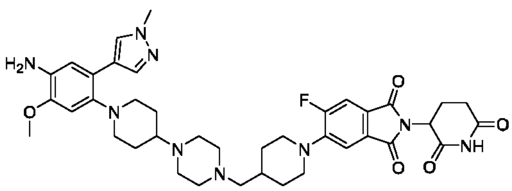

5B

**5A** (0.21 g, 0.27 mmol) was dissolved in ethanol (15 mL); reduced iron powder (0.07 g, 1.35 mmol) was added, and then an aqueous solution (5 mL) of ammonium chloride (0.07 g, 0.75 mmol) was added dropwise; and under nitrogen protection, the mixture was reacted at 80°C for 3 h, and cooled to room temperature. 5 mL of water was added, and then the mixture was extracted 3 times with DCM. The organic phases were combined, dried over anhydrous sodium sulphate and then concentrated under reduced pressure to obtain **5B** (0.19 g).

LCMS m/z = 742.3 $[M+H]^+$.

Step 4: 5-(4-((4-(1-(4-((5-chloro-4-((5-(dimethylphosphoryl)quinoxalin-6-yl) amino)pyrimidin-2-yl)amino)-5-methoxy-2-(1-methyl-1H-pyrazol-4-yl)phenyl)piperidin-4-yl)piperazin-1-yl)methyl)piperidin-1-yl)-2-(2,6-dioxopiperidin-3-yl)-6-fluoroisoindoline-1,3-dione **(compound 5)**

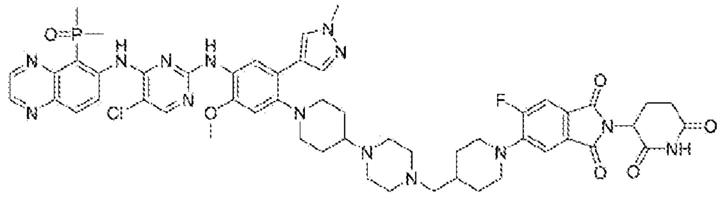

Compound 5

**5B** (0.21 g, 0.28 mmol) and **1I** (0.10 g, 0.28 mmol) were dissolved in DMF (15 mL); p-toluenesulfonic acid hydrate (0.16 g, 0.84 mmol) was added; and under nitrogen protection, the mixture was reacted at 100°C for 16 h. The reaction solution was cooled to room temperature. 10 mL of saturated aqueous sodium bicarbonate solution was added, with a yellow solid precipitated, and the mixture was subjected to suction filtration. The filter cake was washed 3 times with water, redissolved in DCM, dried over anhydrous sodium sulphate and concentrated under reduced pressure, and the residue was purified by column chromatography (mobile phase : dichloromethane/methanol (V/V) = 50/1-12/1) to obtain the crude, which was further purified by preparative HPLC (composition of mobile phases: mobile phase A: acetonitrile, and mobile phase B: water (containing 0.05% ammonia water)) to obtain **compound 5** (50 mg, yield: 17%).

LCMS m/z = 537.3 $[(M+2H)/2]^+$.

$^1H$ NMR (400 MHz, DMSO-$d_6$) δ 12.89 (s, 1H), 11.08 (s, 1H), 9.02 - 8.93 (m, 1H), 8.86 - 8.80 (m, 2H), 8.37 (s, 1H), 8.19 (s, 1H), 8.00 (s, 1H), 7.79 (s, 1H), 7.69 (d, 1H), 7.62 - 7.51 (m, 2H), 7.44 (d, 1H), 6.84 (s, 1H), 5.10 (dd, 1H), 3.80 (s, 3H), 3.78 (s, 3H), 3.61 (d, 2H), 3.14 (d, 2H), 2.94 - 2.83 (m, 3H), 2.70 - 2.52 (m, 8H), 2.47 - 2.34 (m, 4H), 2.30 - 2.24 (m, 1H), 2.22 - 2.13 (m, 2H), 2.07 - 1.98 (m, 7H), 1.89 - 1.79 (m, 4H), 1.77 - 1.69 (m, 1H), 1.63 - 1.53 (m, 2H), 1.33 - 1.19 (m, 2H).

**Example 6: Preparation of compound 6**

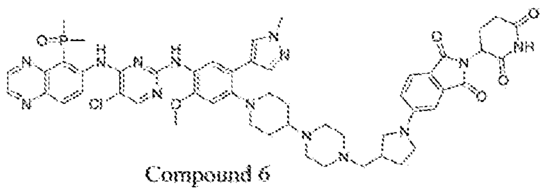

Compound 6

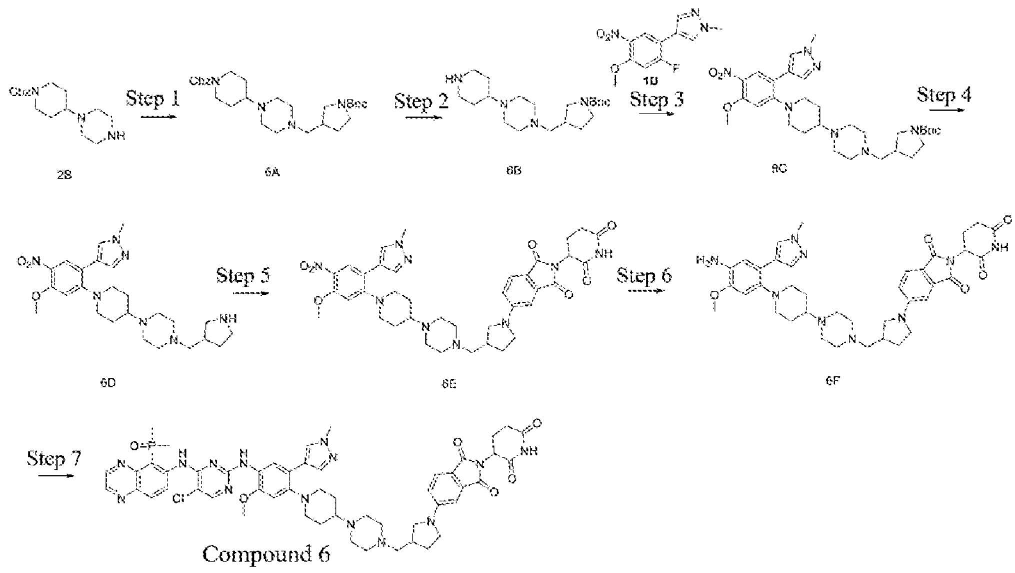


2B 6A 6B 6C 6D 6E 6F Compound 6

Step 1: benzyl 4-(4-((1-(tert-butoxycarbonyl)pyrrolidin-3-yl)methyl)piperazin-1-yl)piperidine-1-carboxylate (6A)

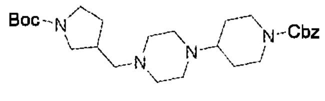

**6A**

**2B** (0.65 g, 2.14 mmol) and 1-Boc-3-pyrrolidinecarbaldehyde (0.51 g, 2.57 mmol) were dissolved in dichloromethane (10 mL); acetic acid (0.26 g, 4.28 mmol) and sodium triacetoxyborohydride (0.91 g, 4.28 mmol) were successively added; and the mixture was reacted at room temperature for 1 h, adjusted to a basic pH by adding 1 N aqueous sodium hydroxide solution, extracted by adding 20 mL of dichloromethane, dried over anhydrous sodium sulphate and concentrated under reduced pressure, and the residue was purified by silica gel column chromatography (mobile phase : ethyl acetate/petroleum ether (V/V) = 1/1 to dichloromethane/methanol (V/V) = 20/1) to obtain **6A** (1 g, yield: 96%).

Step 2: tert-butyl 3-((4-(piperidin-4-yl)piperazin-1-yl)methyl)pyrrolidine-1-carboxylate **(6B)**

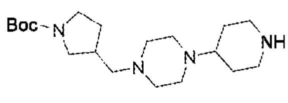

**6B**

**6A** (1.00 g, 2.05 mmol) was dissolved in a mixed solvent of isopropanol (10 mL) and 7 N ammonia methanol solution (2.5 mL); palladium on carbon (wt% = 10%, 0.4 g) was added; and the mixture was subjected to 1 atm hydrogen replacement 3 times, reacted at room temperature for 2 h and filtered over celite. The filter cake was washed with dichloromethane/methanol (V/V = 10/1), and the filtrate was concentrated under reduced pressure to obtain **6B** (0.73 g).
LCMS m/z = 353.3 $[M+H]^+$.

Step 3: tert-butyl 3-((4-(1-(5-methoxy-2-(1-methyl-1H-pyrazol-4-yl)-4-nitrophenyl)piperidin-4-yl)piperazin-1-yl)methyl)pyrrolidine-1-carboxylate **(6C)**

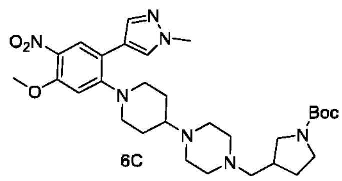

6C

**6B** (0.73 g, 2.08 mmol), **1D** (0.35 g, 1.39 mmol) and potassium carbonate (0.58 g, 4.17 mmol) were mixed and dissolved in DMSO (10 mL), and the mixture was stirred at 120°C for 6 h, cooled to room temperature and extracted by adding 50 mL of water and 50 mL of ethyl acetate. The organic layer was concentrated under reduced pressure, and the residue was purified by column chromatography (mobile phase : dichloromethane/methanol (V/V) = 100/1-20/1) to obtain **6C** (530 mg, yield: 65%).

LCMS m/z = 584.3 $[M+H]^+$.

Step 4: 1-(1-(5-methoxy-2-(1-methyl-1H-pyrazol-4-yl)-4-nitrophenyl) piperidin-4-yl)-4-(pyrrolidin-3-ylmethyl)piperazine **(6D)**

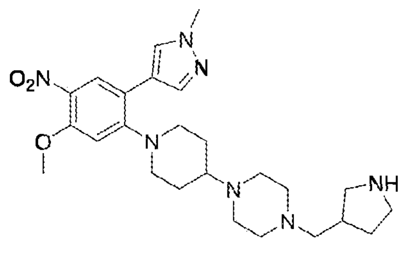

6D

**6C** (0.53 g, 0.91 mmol) was dissolved in dichloromethane (6 mL); trifluoroacetic acid (3.06 g, 26.84 mmol) was added at room temperature; and the mixture was stirred at room temperature for 2 h, and concentrated under reduced pressure. 20 mL of dichloromethane was added to the residue, and the mixture was adjusted to pH>10 with 1 N aqueous sodium hydroxide solution. Liquid separation was performed. The organic layer was then dried over anhydrous sodium sulphate and concentrated under reduced pressure to obtain the crude of **6D** (0.4 g), which was directly used in the next step.

Step 5: 2-(2,6-dioxopiperidin-3-yl)-5-(3-((4-(1-(5-methoxy-2-(1-methyl-1H-pyrazol-4-yl)-4-nitrophenyl)piperidin-4-yl)piperazin-1-yl)methyl)pyrrolidin-1-yl)isoindoline-1,3-dione **(6E)**

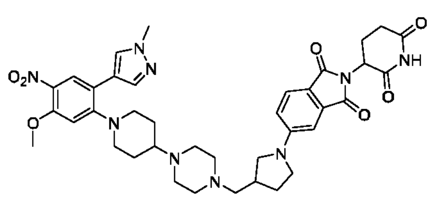

6E

**6D** (0.4 g, 0.83 mmol) and 2-(2,6-dioxopiperidin-3-yl)-5-fluoroisoindole-1,3-dione (0.25 g, 0.91 mmol) were dissolved in DMSO (10 mL); DIPEA (0.21 g, 1.66 mmol) was added; and the mixture was stirred at 90°C for 5 h and cooled to room temperature. 20 mL of water was added, with a solid precipitated, and the mixture was filtered. The filter cake was dried under reduced pressure and then purified by silica gel column chromatography (dichloromethane : methanol (V/V) = 100/1-20/1) to obtain **6E** (0.47 g, yield: 76%) .

Step 6: 5-(3-((4-(1-(4-amino-5-methoxy-2-(1-methyl-1H-pyrazol-4-yl)phenyl) piperidin-4-yl)piperazin-1-yl)methyl)pyrrolidin-1-yl)-2-(2,6-dioxopiperidin-3-yl)isoindoline-1,3-dione **(6F)**

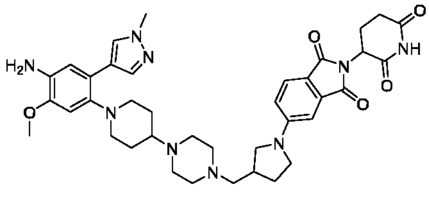

6F

**6E** (0.2 g, 0.27 mmol) was dissolved in ethanol (12 mL) and water (3 mL), and the mixture was warmed to 80°C. A mixture of ammonium chloride (0.22 g, 4.05 mmol) and iron powder (0.23 g, 4.05 mmol) was added. At this temperature, the resulting mixture was stirred for 1 h, cooled to room temperature and filtered. The filter cake was washed with 50 mL of dichloromethane. 10 mL of saturated brine was added to the filtrate. Liquid separation was performed. The organic layer was dried over anhydrous sodium sulphate and concentrated under reduced pressure to obtain the crude of **6F** (190 mg), which was directly used in the next step.
LCMS m/z = 710.3 $[M+H]^+$.

Step 7: 5-(3-((4-(1-(4-((5-chloro-4-((5-(dimethylphosphoryl)quinoxalin-6-yl) amino) pyrimidin-2-yl)amino)-5-methoxy-2-(1-methyl-1H-pyrazol-4-yl)phenyl) piperidin-4-yl)piperazin-1-yl)methyl)pyrrolidin-1-yl)-2-(2,6-dioxopiperidin-3-yl)isoindoline-1,3-dione **(compound 6)**

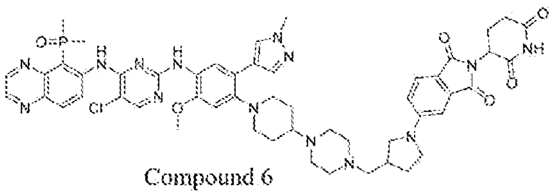

Compound 6

**6F** (0.19 g, 0.27 mmol) and **1I** (99 mg, 0.27 mmol) were dissolved in DMF (5 mL); p-toluenesulfonic acid monohydrate (0.15 g, 0.81 mmol) was added; and under nitrogen protection, the mixture was stirred at 100°C for 16 h, and cooled to room temperature. 5 mL of water and 5 mL of saturated aqueous sodium bicarbonate solution were added, and the mixture was filtered. The filter cake was dried under reduced pressure and then purified by silica gel column chromatography (dichloromethane/methanol (V/V) = 100/1-12/1) to obtain a product, which was further purified by preparative HPLC (composition of mobile phases: mobile phase A: acetonitrile, and mobile phase B: water (containing 0.05% ammonia water)) to obtain **compound 6** (60 mg, yield: 21%).
LCMS m/z = 521.3 $[(M+2H)/2]^+$.
$^1H$ NMR (400 MHz, DMSO-$d_6$) δ 12.89 (s, 1H), 11.05 (s, 1H), 9.06 - 8.77 (m, 3H), 8.37 (s, 1H), 8.19 (s, 1H), 8.00 (s, 1H), 7.79 (s, 1H), 7.70 - 7.47 (m, 3H), 6.94 - 6.74 (m, 3H), 5.05 (dd, 1H), 3.81 (s, 3H), 3.78 (s, 3H), 3.59 - 3.36 (m, 3H), 3.18 - 3.06 (m, 3H), 2.95 - 2.81 (m, 1H), 2.72 - 2.52 (m, 9H), 2.49 - 2.29 (m, 6H), 2.28 - 2.19 (m, 1H), 2.17 - 2.07 (m, 1H), 2.07 - 1.93 (m, 7H), 1.91 - 1.68 (m, 3H), 1.66 - 1.49 (m, 2H).

**Example 7: Preparation of trifluoroacetate of compound 7**

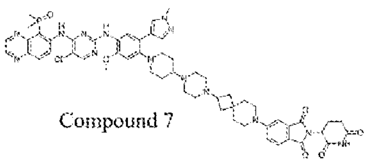

Compound 7

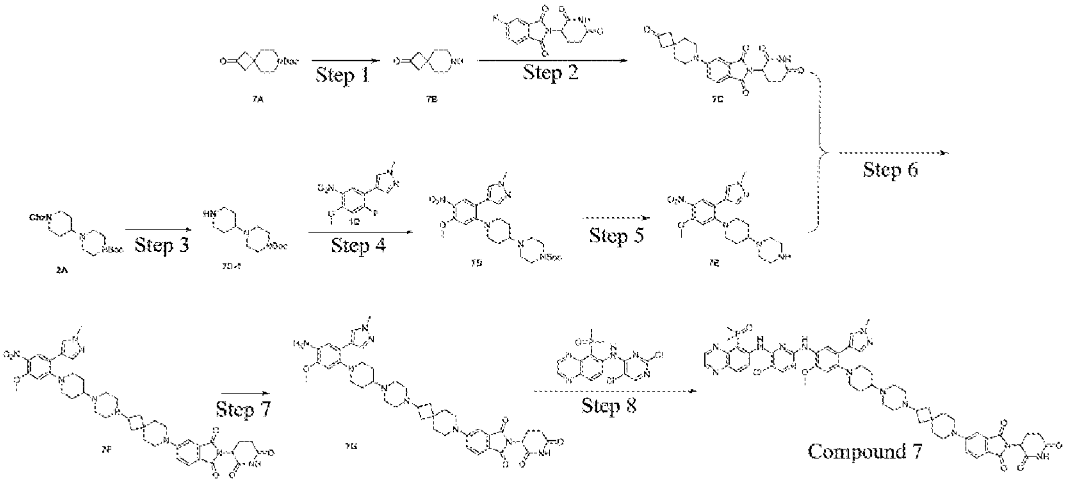


Step 1: 7-azaspiro[3.5]nonan-2-one **(7B)**

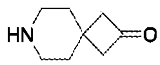

7B

**7A** (13.0 g, 54.32 mmol) was dissolved in dichloromethane (100 mL); trifluoroacetic acid (61.94 g, 543.2 mmol) was added at room temperature; and the mixture was stirred for 1 h and concentrated under reduced pressure. To the residue were added 50 mL of dichloromethane and a sodium bicarbonate solid (70 g). The mixture was subjected to suction filtration, and the filtrate was dried over anhydrous sodium sulphate and concentrated under reduced pressure to obtain **7B** (7.50 g, yield: 99%).

LCMS m/z = 140.1 $[M+H]^+$.

Step 2: 2-(2,6-dioxopiperidin-3-yl)-5-(2-oxo-7-azaspiro[3.5]nonan-7-yl) isoindoline-1,3-dione **(7C)**

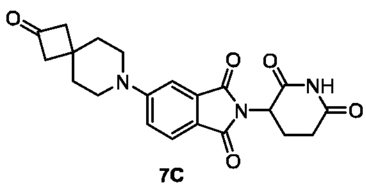

7C

**7B** (7.50 g, 53.88 mmol) was dissolved in dimethyl sulfoxide (100 mL); 2-(2,6-dioxopiperidin-3-yl)-5-fluoroisoindoline-1,3-dione (22.32 g, 80.81 mmol) and DIPEA (13.93 g, 107.78 mmol) were successively added; and the mixture was stirred at 100°C for 3 h, and cooled to room temperature. 100 mL of water was added, and the mixture was stirred for 5-10 min and subjected to suction filtration. The filter cake was dissolved in dichloromethane and extracted. The organic phase was dried over anhydrous sodium sulphate and concentrated under reduced pressure, and the residue was purified by silica gel column chromatography (mobile phase : dichloromethane/methanol (V/V) = 100/1-10/1) to obtain **7C** as a yellow solid (11.40 g, yield: 54%).

LCMS m/z = 396.1 $[M+H]^+$.

Step 3: tert-butyl 4-(piperidin-4-yl)piperazine-1-carboxylate **(7D-1)**

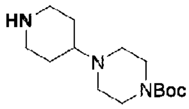

**7D-1**

To a 100 mL reaction flask were successively added **2A** (4.91 g, 12.16 mmol), isopropanol/ammonia methanol solution (v/v = 4/1, 60 mL) and palladium on carbon (2.5 g); and the mixture was subjected to hydrogen replacement three times, reacted at room temperature for 2 h and subjected to suction filtration over celite. The filtrate was concentrated under reduced pressure, and the residue was purified by silica gel column chromatography (mobile phase : dichloromethane/methanol (V/V) = 25/1-10/1) to obtain **7D-1** (1.26 g, yield: 38%).
LCMS m/z = 270.1 $[M+H]^+$.

Step 4: tert-butyl 4-(1-(5-methoxy-2-(1-methyl-1H-pyrazol-4-yl)-4-nitrophenyl) piperidin-4-yl)piperazine-1-carboxylate **(7D)**

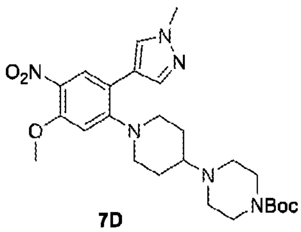

**7D**

**7D-1** (1.23 g, 4.57 mmol), 4-(2-fluoro-4-methoxy-5-nitrophenyl)-1-methyl-1H-pyrazole **(1D)** (0.96 g, 3.80 mmol) and potassium carbonate (1.58 g, 11.41 mmol) were mixed and dissolved in DMSO (20 mL), and the mixture was stirred overnight at 120°C, cooled to room temperature and extracted by adding 50 mL of water and 50 mL of ethyl acetate. The organic layer was concentrated under reduced pressure, and the residue was purified by column chromatography (mobile phase : dichloromethane/methanol (V/V) = 100/1-20/1) to obtain **7D** (970 mg, yield: 62%).
LCMS m/z = 501.5 $[M+H]^+$.

Step 5: 1-(1-(5-methoxy-2-(1-methyl-1H-pyrazol-4-yl)-4-nitrophenyl)piperidin-4-yl)piperazine **(7E)**

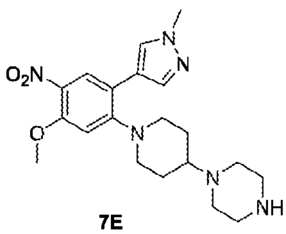

**7E**

**7D** (0.31 g, 0.62 mmol) was dissolved in dichloromethane (10 mL); trifluoroacetic acid (0.71 g, 6.2 mmol) was added at room temperature; and the mixture was stirred for 1 h and concentrated under reduced pressure. To the residue were added 20 mL of dichloromethane and saturated aqueous sodium bicarbonate solution. The mixture was adjusted to pH = 8-9 and extracted with dichloromethane. The organic phase was dried over anhydrous sodium sulphate and concentrated under reduced pressure to obtain **7E** (0.24 g, yield: 97%).
LCMS m/z = 401.2 $[M+H]^+$.

Step 6: 2-(2,6-dioxopiperidin-3-yl)-5-(2-(4-(1-(5-methoxy-2-(1-methyl-1H-pyrazol-4-yl)-4-nitrophenyl)piperidin-4-yl)piperazin-1-yl)-7-azaspiro[3.5]nonan-7-yl)isoindoline-1,3-dione **(7F)**

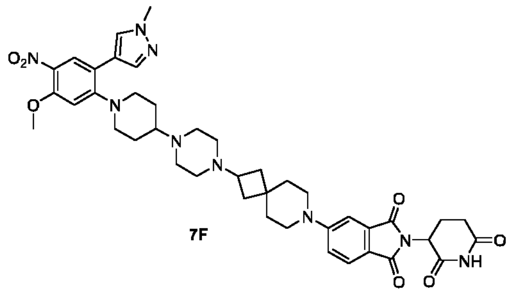

7F

**7E** (0.24 g, 0.60 mmol) and **7C** (0.71 g, 1.80 mmol) were dissolved in 1,2-dichloroethane (10 mL), and a 4Å molecular sieve (200 mg) and acetic acid (0.05 g, 0.90 mmol) were successively added. The mixture was stirred at room temperature for 2 h; sodium triacetoxyborohydride (0.38 g, 1.79 mol) was added; and the resulting mixture was reacted overnight at room temperature. After the reaction was completed, the reaction system was directly concentrated under reduced pressure, and the residue was purified by silica gel column chromatography (mobile phase : dichloromethane/methanol (V/V) = 100/1-10/1) to obtain **7F** (0.23 g, yield: 49%).

LCMS m/z = 780.4 $[M+H]^+$.

Step 7: 5-(2-(4-(1-(4-amino-5-methoxy-2-(1-methyl-1H-pyrazol-4-yl)phenyl) piperidin-4-yl)piperazin-1-yl)-7-azaspiro[3.5]nonan-7-yl)-2-(2,6-dioxopiperidin-3-yl)isoindoline-1,3-dione **(7G)**

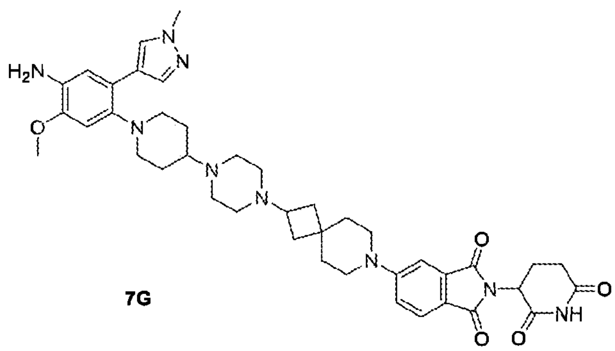

7G

**7F** (0.23 g, 0.29 mmol) was dissolved in ethanol (5 mL) and water (1 mL); an ammonium chloride solid (0.16 g, 2.99 mmol) and iron powder (0.16 g, 2.86 mmol) were added; and the mixture was subjected to $N_2$ replacement 3 times and stirred at 80°C for 1 h. After the reaction was completed, the mixture was cooled to room temperature and filtered. The filter cake was washed with 50 mL of dichloromethane and concentrated under reduced pressure. The resultant obtained from the concentration under reduced pressure was dissolved in dichloromethane and extracted. The organic layer was dried over anhydrous sodium sulphate and concentrated under reduced pressure, and the residue was purified by silica gel column chromatography (mobile phase : dichloromethane/methanol (V/V) = 100/1-10/1) to obtain **7G** (0.18 g, yield: 83%).

LCMS m/z = 750.4 $[M+H]^+$.

Step 8: 5-(2-(4-(1-(4-((5-chloro-4-((5-(dimethylphosphoryl)quinoxalin-6-yl) amino)pyrimidin-2-yl)amino)-5-methoxy-2-(1-methyl-1H-pyrazol-4-yl)phenyl)piperidin-4-yl)piperazin-1-yl)-7-azaspiro[3.5]nonan-7-yl)-2-(2,6-dioxopiperidin-3-yl)isoindoline-1,3-dione **(compound 7)**; 2,2,2-trifluoroacetic acid

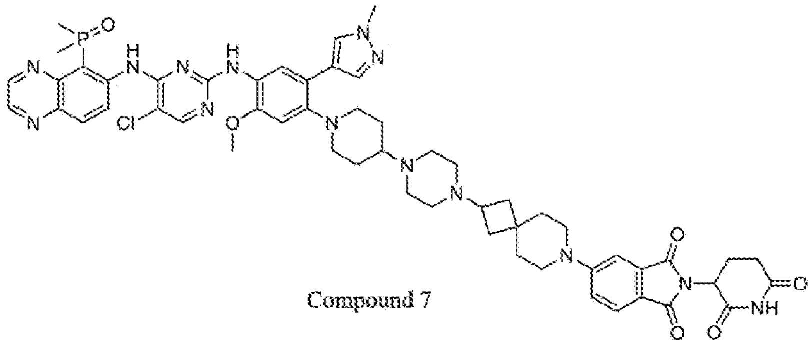

Compound 7

**7G** (0.18 g, 0.24 mmol) and **1I** (0.09 g, 0.24 mmol) were dissolved in DMF (5 mL); p-toluenesulfonic acid monohydrate (0.14 g, 0.74 mol) was added; and under nitrogen protection, the mixture was reacted overnight at 100°C, and cooled to room temperature. 20 mL of saturated aqueous sodium bicarbonate solution was added, and the mixture was subjected to suction filtration. The filter cake was dissolved in dichloromethane and extracted. The organic phase was dried over anhydrous sodium sulphate and then concentrated under reduced pressure, and the residue was purified by silica gel column chromatography (dichloromethane/methanol (V/V) = 100/1-10/1) to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)) to obtain the trifluoroacetate of **compound** 7 (40 mg).

LCMS m/z = 1081.4 $[M+H]^+$.

$^1H$ NMR (400 MHz, DMSO-$d_6$) δ 12.96 (s, 1H), 11.05 (s, 1H), 9.02 - 8.78 (m, 3H), 8.52 (s, 1H), 8.22 (s, 1H), 8.00 (s, 1H), 7.82 (s, 1H), 7.69 - 7.48 (m, 3H), 7.34 (d, 1H), 7.26 (dd, 1H), 6.82 (s, 1H), 5.06 (dd, 1H), 3.82 (s, 3H), 3.78 (s, 3H), 3.72 - 3.37 (m, 9H), 3.28 - 3.11 (m, 5H), 2.95 - 2.81 (m, 2H), 2.74 - 2.52 (m, 5H), 2.26 - 2.16 (m, 2H), 2.14 - 1.90 (m, 11H), 1.87 - 1.57 (m, 6H).

**Example 8: Preparation of compound 8**

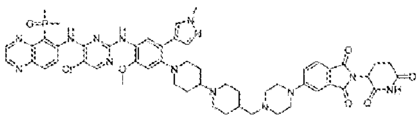

Compound 8

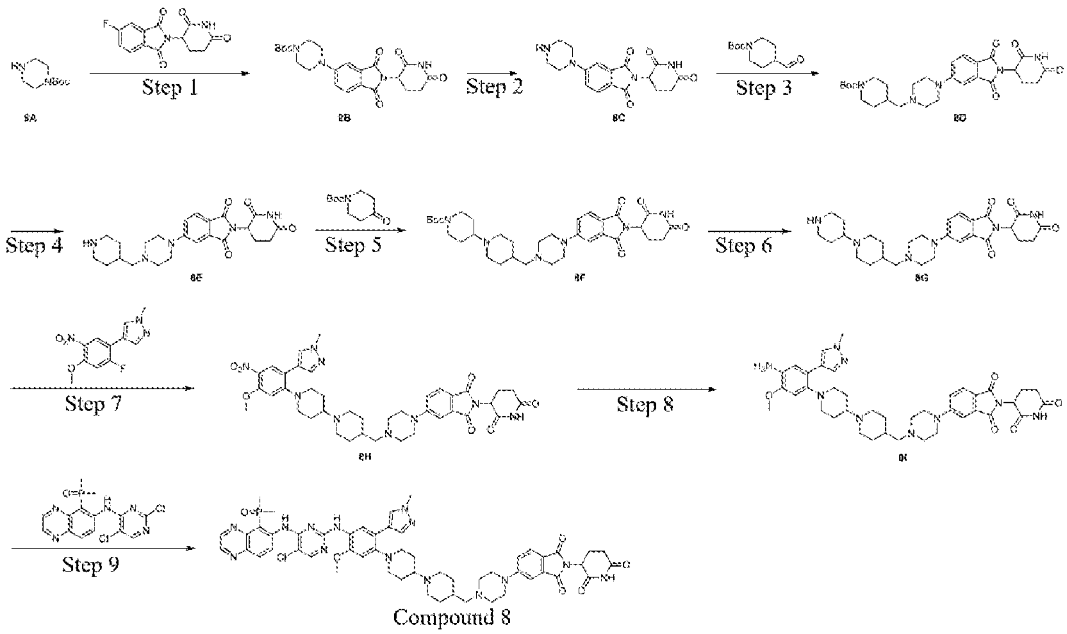


Compound 8

Step 1: tert-butyl 4-(2-(2,6-dioxopiperidin-3-yl)-1,3-dioxoisoindolin-5-yl) piperazine-1-carboxylate **(8B)**

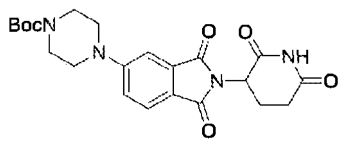

**8B**

**8A** (5.0 g, 26.84 mmol) was dissolved in dimethyl sulfoxide (50 mL); 2-(2,6-dioxopiperidin-3-yl)-5-fluoroisoindoline-1,3-dione (11.12 g, 40.26 mmol) and DIPEA (6.94 g, 53.69 mmol) were successively added; and the mixture was stirred at 100°C for 3 h, and cooled to room temperature. 50 mL of water was added, and the mixture was stirred for 5-10 min and subjected to suction filtration. The filter cake was dissolved in dichloromethane and extracted. The organic phase was dried over anhydrous sodium sulphate and concentrated under reduced pressure, and the residue was purified by silica gel column chromatography (mobile phase : dichloromethane/methanol (V/V) = 100/1-10/1) to obtain **8B** (8.08 g, yield: 68%).

LCMS m/z = 443.2 $[M+H]^+$.

Step 2: 2-(2,6-dioxopiperidin-3-yl)-5-(piperazin-1-yl)isoindoline-1,3-dione **(8C)**

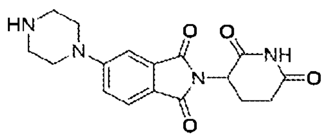

**8C**

**8B** (3 g, 6.78 mmol) was dissolved in dichloromethane (30 mL); trifluoroacetic acid (7.73 g, 67.8 mmol) was added at room temperature; and the mixture was stirred for 1 h and concentrated under reduced pressure. To the residue were added 20 mL of dichloromethane and a sodium bicarbonate solid (20 g); and the mixture was subjected to suction filtration. The filtrate was dried over anhydrous sodium sulphate and concentrated under reduced pressure to obtain the crude of **8C** (2.09 g), which was directly used in the next step.

LCMS m/z = 343.1 $[M+H]^+$.

Step 3: tert-butyl 4-((4-(2-(2,6-dioxopiperidin-3-yl)-1,3-dioxoisoindolin-5-yl) piperazin-1-yl)methyl)piperidine-1-carboxylate **(8D)**

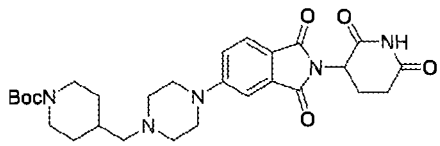

**8D**

**8C** (2.09 g, 6.10 mmol) and tert-butyl 4-formylpiperidine-1-carboxylate (3.91 g, 18.33 mmol) were dissolved in DMAc (40 mL); and a 4Å molecular sieve (300 mg) and acetic acid (0.55 g, 9.16 mmol) were successively added. The mixture was stirred at room temperature for 2 h; sodium triacetoxyborohydride (3.88 g, 18.31 mmol) was added; and the resulting mixture was reacted overnight at room temperature. After the reaction was completed, the mixture was extracted with ethyl acetate (50 mL). The organic phase was dried over anhydrous sodium sulphate and concentrated under reduced pressure, and the residue was purified by silica gel column chromatography (mobile phase : dichloromethane/methanol (V/V) = 100/1-10/1) to obtain **8D** (1.53 g, yield: 46%).

LCMS m/z = 540.3 $[M+H]^+$.

Step 4: 2-(2,6-dioxopiperidin-3-yl)-5-(4-(piperidin-4-ylmethyl)piperazin-1-yl) isoindoline-1,3-dione **(8E)**

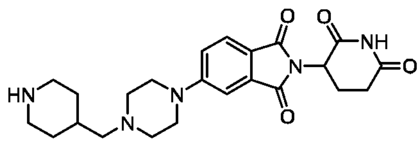

**8E**

**8D** (1.53 g, 2.84 mmol) was dissolved in dichloromethane (15 mL); trifluoroacetic acid (3.23 g, 28.33 mmol) was added at room temperature; and the mixture was stirred for 1 h and concentrated under reduced pressure. To the residue were added 20 mL of dichloromethane and a sodium bicarbonate solid (15 g); and the mixture was subjected to suction filtration. The filtrate was dried over anhydrous sodium sulphate and concentrated under reduced pressure to obtain the crude of **8E** (1.13 g), which was directly used in the next step.

LCMS m/z = 440.2 $[M+H]^+$.

Step 5: tert-butyl 4-((4-(2-(2,6-dioxopiperidin-3-yl)-1,3-dioxoisoindolin-5-yl) piperazin-1-yl)methyl)-[1,4'-bipiperidine]-1'-carboxylate **(8F)**

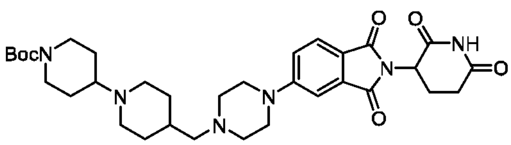

**8F**

**8E** (1.13 g, 2.57 mmol) and tert-butyl 4-oxopiperidine-1-carboxylate (1.54 g, 7.73 mmol) were dissolved in DMAc (20 mL); and a 4Å molecular sieve (200 mg) and acetic acid (0.23 g, 3.83 mmol) were successively added. The mixture was stirred at room temperature for 2 h; sodium triacetoxyborohydride (1.63 g, 7.69 mmol) was added; and the resulting mixture was reacted overnight at room temperature. After the reaction was completed, the mixture was extracted with ethyl acetate (50 mL). The organic phase was dried over anhydrous sodium sulphate and concentrated under

reduced pressure, and the residue was purified by silica gel column chromatography (mobile phase : dichloromethane/methanol (V/V) = 100/1-10/1) to obtain **8F** (1.27 g, yield: 79%).

LCMS m/z = 623.2 $[M+H]^+$.

Step 6: 5-(4-([1,4'-bipiperidin]-4-ylmethyl)piperazin-1-yl)-2-(2,6-dioxopiperidin-3-yl)isoindoline-1,3-dione **(8G)**

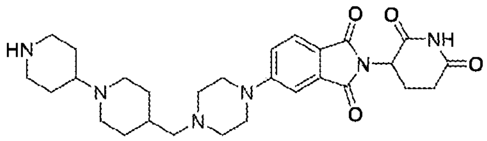

8G

**8F** (1.27 g, 2.04 mmol) was dissolved in dichloromethane (15 mL); trifluoroacetic acid (2.33 g, 20.43 mmol) was added at room temperature; and the mixture was stirred for 1 h and concentrated under reduced pressure. To the residue were added 20 mL of dichloromethane and a sodium bicarbonate solid (20 g); and the mixture was subjected to suction filtration. The filtrate was dried over anhydrous sodium sulphate and concentrated under reduced pressure to obtain the crude of **8G** (0.98 g), which was directly used in the next step.

LCMS m/z = 523.3 $[M+H]^+$.

Step 7: 2-(2,6-dioxopiperidin-3-yl)-5-(4-((1'-(5-methoxy-2-(1-methyl-1H-pyrazol-4-yl)-4-nitrophenyl)-[1,4'-bipiperidin]-4-yl)methyl)piperazin-1-yl)isoindoline-1,3-dione **(8H)**

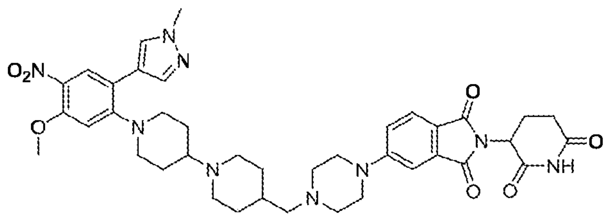

8H

**8G** (980 mg, 1.87 mmol) and 4-(2-fluoro-4-methoxy-5-nitrophenyl)-1-methyl-1H-pyrazole **(1D)** (0.31 g, 1.23 mmol) were dissolved in DMSO (10 mL); a sodium bicarbonate solid (0.32 g, 3.81 mmol) was added; and the mixture was reacted overnight at 100°C. After the reaction was completed, the mixture was cooled to room temperature and extracted by adding 30 mL of ethyl acetate and 30 mL of water. The organic layer was dried over anhydrous sodium sulphate and concentrated under reduced pressure, and the residue was purified by column chromatography (dichloromethane : methanol (V/V) = 100/1-10/1) to obtain **8H** (0.24 g, yield: 17%).

LCMS m/z = 754.4 $[M+H]^+$.

Step 8: 5-(4-((1'-(4-amino-5-methoxy-2-(1-methyl-1H-pyrazol-4-yl)phenyl)-[1,4'-bipiperidin]-4-yl)methyl)piperazin-1-yl)-2-(2,6-dioxopiperidin-3-yl)isoindoline-1,3-dione **(8I)**

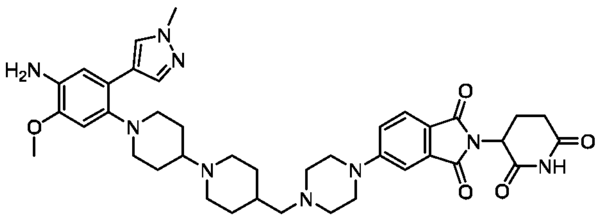

**8I**

**8H** (0.24 g, 0.32 mmol) was dissolved in ethanol (5 mL) and water (1 mL); an ammonium chloride solid (0.17 g, 3.18 mmol) and iron powder (0.18 g, 3.22 mmol) were added; and the mixture was subjected to $N_2$ replacement 3 times and stirred at 80°C for 1 h. After the reaction was completed, the mixture was cooled to room temperature and filtered. The filter cake was washed with 50 mL of dichloromethane and concentrated under reduced pressure. The resultant obtained from the concentration under reduced pressure was dissolved in dichloromethane and extracted. The organic layer was dried over anhydrous sodium sulphate and concentrated under reduced pressure, and the residue was purified by silica gel column chromatography (mobile phase : dichloromethane/methanol (V/V) = 100/1-10/1) to obtain **8I** (0.12 g, yield: 52%).

LCMS m/z = 724.4 $[M+H]^+$.

Step 9: 5-(4-((1'-(4-((5-chloro-4-((5-(dimethylphosphoryl)quinoxalin-6-yl)amino) pyrimidin-2-yl)amino)-5-methoxy-2-(1-methyl-1H-pyrazol-4-yl)phenyl)-[1,4'-bipiperidin]-4-yl)methyl)piperazin-1-yl)-2-(2,6-dioxopiperidin-3-yl)isoindoline-1,3-dione **(compound 8)**

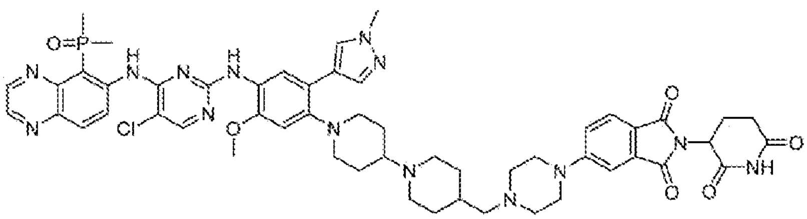

Compound 8

**8I** (0.12 g, 0.17 mmol) and **1I** (0.06 g, 0.16 mmol) were dissolved in DMF (5 mL); p-toluenesulfonic acid monohydrate (0.095 g, 0.50 mmol) was added; and under nitrogen protection, the mixture was reacted overnight at 100°C, and cooled to room temperature. 20 mL of saturated aqueous sodium bicarbonate solution was added, and the mixture was subjected to suction filtration. The filter cake was dissolved in dichloromethane and extracted. The organic phase was dried over anhydrous sodium sulphate and then concentrated under reduced pressure, and the residue was purified by silica gel column chromatography (dichloromethane/methanol (V/V) = 100/1-10/1) to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.05% ammonia water)) to obtain **compound 8** (60 mg, yield: 35%).

LCMS m/z = 1055.4 $[M+H]^+$.

$^1H$ NMR (400 MHz, DMSO-$d_6$) δ 12.89 (s, 1H), 11.06 (s, 1H), 9.06 - 8.77 (m, 3H), 8.37 (s, 1H), 8.19 (s, 1H), 7.99 (s, 1H), 7.80 (s, 1H), 7.67 (d, 1H), 7.62 - 7.49 (m, 2H), 7.33 (d, 1H), 7.24 (dd, 1H), 6.83 (s, 1H), 5.07 (dd, 1H), 3.80 (s, 3H), 3.77 (s, 3H), 3.48 - 3.38 (m, 4H), 3.18 - 3.09 (m, 2H), 2.98 - 2.82 (m, 3H), 2.69 - 2.52 (m, 4H), 2.48 - 2.43 (m, 3H), 2.39 - 2.27 (m, 1H), 2.22 - 2.11 (m, 4H), 2.07 - 1.95 (m, 7H), 1.85 - 1.41 (m, 8H), 1.19 - 1.05 (m, 2H).

**Example 9: Preparation of compound 9**

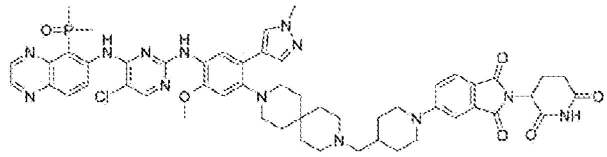

Compound 9

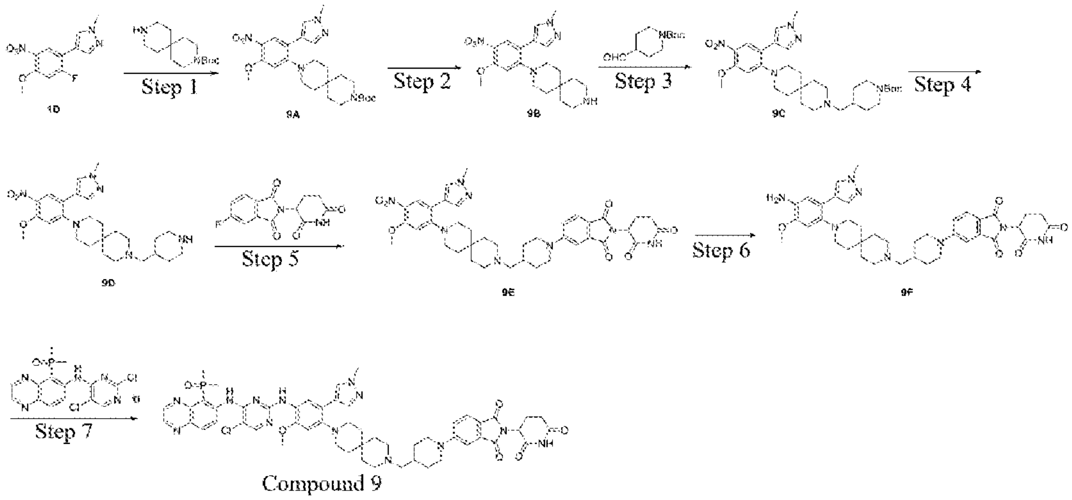


Compound 9

Step 1: tert-butyl 9-(5-methoxy-2-(1-methyl-1H-pyrazol-4-yl)-4-nitrophenyl)-3,9-diazaspiro[5.5]undecane-3-carboxylate **(9A)**

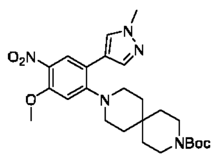

**9A**

**1D** (0.4 g, 1.59 mmol) and tert-butyl 3,9-diazaspiro[5.5]undecane-3-carboxylate (0.61 g, 2.40 mmol) were dissolved in DMSO (10 mL); potassium carbonate (0.88 g, 6.37 mmol) was added; and the mixture was reacted at 130°C for 6 h, cooled to room temperature and extracted by adding 30 mL of ethyl acetate and 30 mL of water. The organic layer was washed three times with saturated brine (20 mL × 3), dried over anhydrous sodium sulphate and concentrated under reduced pressure, and the residue was purified by column chromatography (dichloromethane : methanol (V/V) = 100/1-20/1) to obtain **9A** (0.35 g, yield: 45%).

LCMS m/z = 486.2$[M+H]^+$.

Step 2: 3-(5-methoxy-2-(1-methyl-1H-pyrazol-4-yl)-4-nitrophenyl)-3,9-diazaspiro[5.5]undecane **(9B)**

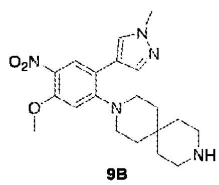

**9B**

**9A** (350 mg, 0.72 mmol) was dissolved in methanol (2 mL); a solution of hydrochloric acid in dioxane (2 N, 10 mL) was added; and the mixture was reacted at room temperature for 1 h and concentrated under reduced pressure.

20 mL of dichloromethane was added to the residue, and the mixture was adjusted to pH = 9-10 with 1 M aqueous NaOH solution. Liquid separation was performed. The organic layer was concentrated under reduced pressure to obtain 9B (264 mg), which was directly used in the next step.

Step 3: tert-butyl 4-((9-(5-methoxy-2-(1-methyl-1H-pyrazol-4-yl)-4-nitrophenyl)-3,9-diazaspiro [5.5]undecan-3-yl)methyl)piperidine-1-carboxylate (9C)

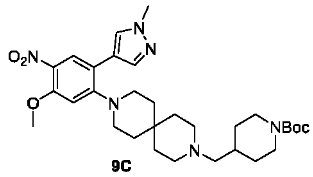

9C

**9B** (264 mg, 0.68 mmol) and tert-butyl 4-formylpiperidine-1-carboxylate (217.5 mg, 1.02 mmol) were dissolved in DMAc (10 mL), and acetic acid (40.8 mg, 0.68 mmol) was added. The mixture was stirred at room temperature for 30 min, and then sodium triacetoxyborohydride (215.3 mg, 1.02 mmol) was added; and the resulting mixture was reacted overnight at room temperature, and extracted by adding 20 mL of saturated aqueous sodium bicarbonate solution and 20 mL of dichloromethane. The organic layer was concentrated under reduced pressure, and the residue was purified by silica gel column chromatography (mobile phase DCM/MeOH (V/V) = 100/1-20/1) to obtain **9C** (260 mg, yield: 66%).
LCMS m/z = 583.3 $[M+H]^+$.

Step 4: 3-(5-methoxy-2-(1-methyl-1H-pyrazol-4-yl)-4-nitrophenyl)-9-(piperidin-4-ylmethyl)-3,9-diazaspiro[5.5]undecane **(9D);** HCl

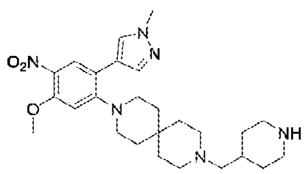

9D

**9C** (260 mg, 0.45 mmol) was dissolved in methanol (2 mL); a solution of hydrochloric acid in dioxane (2 *N,* 6 mL) was added; and the mixture was reacted at room temperature for 1 h and concentrated under reduced pressure, and the residue was directly used in the next step.

Step 5: 2-(2,6-dioxopiperidin-3-yl)-5-(4-((9-(5-methoxy-2-(1-methyl-1H-pyrazol-4-yl)-4-nitrophenyl)-3,9-diazaspiro[5.5]undecan-3-yl)methyl)piperidin-1-yl)isoindoline-1,3-dione **(9E)**

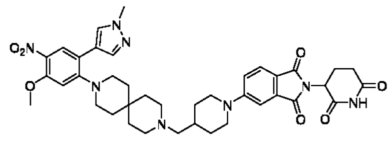

9E

The crude hydrochloride of **9D** from the previous step was dissolved in DMSO (8 mL); 2-(2,6-dioxopiperidin-3-yl)-5-fluoroisoindole-1,3-dione (187.8 mg, 0.68 mmol) and DIPEA (232.7 mg, 1.8 mmol) were successively added; and the mixture was stirred at 100°C for 5 h, and cooled to room temperature. 10 mL of water was added, and the mixture was filtered. The filter cake was dried under reduced pressure and then purified by silica gel column chromatography (mobile phase : dichloromethane/methanol (V/V) = 100/1-20/1) to obtain **9E** (174 mg, yield: 52%).

LCMS m/z = 739.3 $[M+H]^+$.

Step 6: 5-(4-((9-(4-amino-5-methoxy-2-(1-methyl-1H-pyrazol-4-yl)phenyl)-3,9-diazaspiro[5.5]undecan-3-yl)methyl)piperidin-1-yl)-2-(2,6-dioxopiperidin-3-yl)isoindoline-1,3-dione **(9F)**

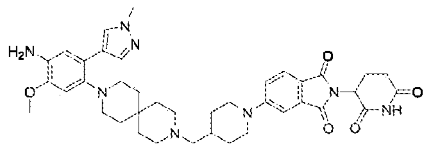

9F

**9E** (174 mg, 0.24 mmol) was dissolved in ethanol/water (8 mL, 3 : 1); iron powder (70 mg, 1.25 mmol) and ammonium chloride (66.9 mg, 1.25 mmol) were successively added; and under nitrogen protection, the mixture was stirred at 80°C for 1 h, cooled to room temperature and subjected to suction filtration under reduced pressure. The filtrate was concentrated under reduced pressure, and then the crude was purified by silica gel column chromatography (mobile phase: dichloromethane/methanol (V/V) = 100/1-20/1) to obtain **9F** (150 mg, yield: 88%).
LCMS m/z = 709.4 $[M+H]^+$.

Step 7: 5-(4-((9-(4-((5-chloro-4-((5-(dimethylphosphoryl)quinoxalin-6-yl) amino)pyrimidin-2-yl)amino)-5-methoxy-2-(1-methyl-1H-pyrazol-4-yl)phenyl)-3,9-diazaspiro[5.5]undecan-3-yl)methyl)piperidin-1-yl)-2-(2,6-dioxopiperidin-3-yl)isoindoline-1,3-dione **(compound 9)**

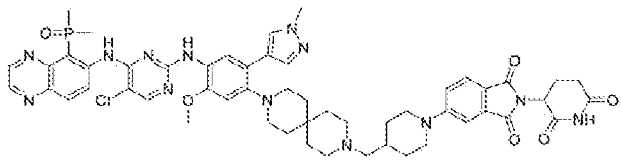

Compound 9

**9F** (150 mg, 0.21 mmol) and **1I** (117.8 mg, 0.32 mmol) were dissolved in DMF (5 mL); p-toluenesulfonic acid monohydrate (119.8 mg, 0.63 mmol) was added; and under nitrogen protection, the mixture was stirred at 100°C for 16 h, and cooled to room temperature. 20 mL of saturated aqueous sodium bicarbonate solution and 50 mL of dichloromethane were added, and the layers were separated. The organic layer was concentrated under reduced pressure, and the residue was purified by silica gel column chromatography (mobile phase: dichloromethane/methanol (V/V) = 100/1-20/1) to obtain **compound 9** (60 mg, yield: 27%).
LCMS m/z = 1040.4 $[M+H]^+$.
$^1H$ NMR (400 MHz, DMSO-$d_6$) δ 12.87 (s, 1H), 11.05 (s, 1H), 9.01 - 8.89 (m, 1H), 8.87 - 8.76 (m, 2H), 8.38 (s, 1H), 8.19 (s, 1H), 8.01 (s, 1H), 7.81 (s, 1H), 7.65 (d, 1H), 7.61 - 7.48 (m, 2H), 7.30 (s, 1H), 7.27 - 7.18 (m, 1H), 6.89 (s, 1H), 5.06 (dd, 1H), 4.10 - 3.97 (m, 2H), 3.81 (s, 3H), 3.77 (s, 3H), 3.04 - 2.77 (m, 7H), 2.70 - 2.53 (m, 2H), 2.42 - 2.28 (m, 4H), 2.24 - 2.09 (m, 2H), 2.06 - 1.95 (m, 7H), 1.90 - 1.72 (m, 3H), 1.66 - 1.46 (m, 8H), 1.21 - 1.09 (m, 2H).

**Example 10: Preparation of compound 10**

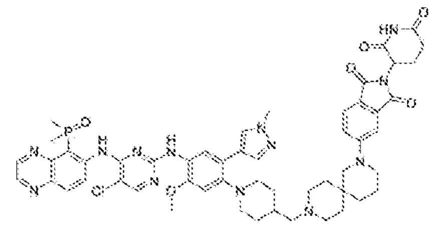

Compound 10

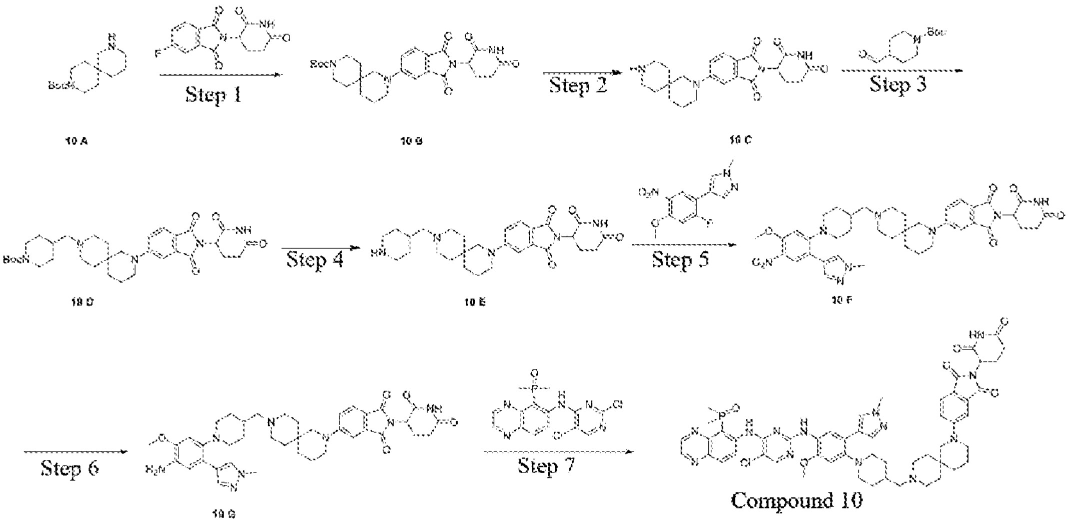


Step 1: tert-butyl 2-(2-(2,6-dioxopiperidin-3-yl)-1,3-dioxoisoindolin-5-yl)-2,9-diazaspiro[5.5]undecane-9-carboxylate **(10B)**

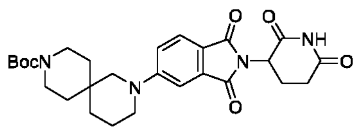

**10 B**

**10A** (2.0 g, 7.86 mmol) was dissolved in DMSO (15 mL); 2-(2,6-dioxopiperidin-3-yl)-5-fluoroisoindoline-1,3-dione (2.5 g, 9.05 mmol) and DIPEA (2.77 g, 21.43 mmol) were successively added; and the mixture was stirred at 90°C for 3 h, and cooled to room temperature. 10 mL of water was added, and the mixture was filtered. The filter cake was dried under reduced pressure and then purified by silica gel column chromatography (mobile phase: dichloromethane/methanol (V/V) = 100/1-20/1) to obtain **10B** as a yellow solid (3.0 g, yield: 75%).
LCMS m/z = 511.3 $[M+H]^+$.

Step 2: 2-(2,6-dioxopiperidin-3-yl)-5-(2,9-diazaspiro[5.5]undecan-2-yl) isoindoline-1,3-dione **(10C);** 2,2,2-trifluoroacetic acid

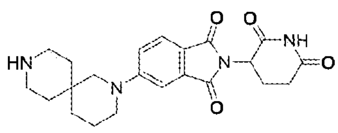

**10 C**

**10B** (3.0 g, 5.88 mmol) was dissolved in dichloromethane (60 mL); trifluoroacetic acid (4.6 g, 40 mmol) was added; and the mixture was stirred at room temperature for 3 h and concentrated under reduced pressure, and the residue was directly used in the next step.

Step 3: tert-butyl 4-((2-(2-(2,6-dioxopiperidin-3-yl)-1,3-dioxoisoindolin-5-yl)-2,9-diazaspiro[5.5]undecan-9-yl)methyl)piperidine-1-carboxylate **(10D)**

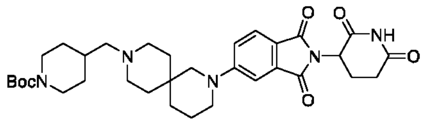

10 D

The trifluoroacetate of **10C** (0.6 g) and tert-butyl 4-formylpiperidine-1-carboxylate (0.4 g, 1.87 mol) were mixed in DMAc (20 mL); acetic acid (0.12 g, 2 mmol) and sodium triacetoxyborohydride (0.42 g, 2 mmol) were successively added; and the mixture was stirred overnight at room temperature. 100 mL of dichloromethane and 50 mL of 1 N aqueous sodium hydroxide solution were added, and then the layers were separated. The organic layer was dried under reduced pressure, and the residue was purified by silica gel column chromatography (mobile phase : dichloromethane/methanol (V/V) = 10/1-1/1) to obtain **10D** (0.44 g, yield: 50%).
LCMS m/z = 608.3 $[M+H]^+$.

Step 4: 2-(2,6-dioxopiperidin-3-yl)-5-(9-(piperidin-4-ylmethyl)-2,9-diazaspiro [5.5]undecan-2-yl)isoindoline-1,3-dione **(10E);** 2,2,2-trifluoroacetic acid

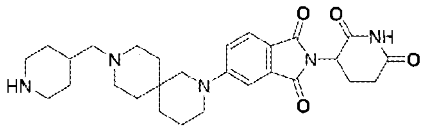

10 E

Compound **10D** (0.44 g, 0.72 mmol) was dissolved in dichloromethane (10 mL); trifluoroacetic acid (2.3 g, 20 mmol) was added; and the mixture was stirred at room temperature for 3 h and concentrated under reduced pressure, and the residue was directly used in the next step.

Step 5: 2-(2,6-dioxopiperidin-3-yl)-5-(9-((1-(5-methoxy-2-(1-methyl-1H-pyrazol-4-yl)-4-nitrophenyl)piperidin-4-yl)methyl)-2,9-diazaspiro[5.5]undecan-2-yl)isoindoline-1,3-dione **(10F)**

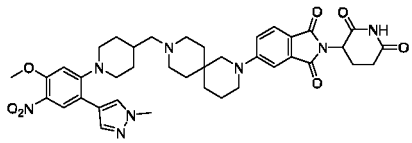

10 F

The crude trifluoroacetate of **10E** from the previous step was dissolved in DMSO (15 mL); 4-(2-fluoro-4-methoxy-5-nitrophenyl)-1-methyl-1H-pyrazole **(1D)** (260 mg, 1.03 mmol) and sodium bicarbonate (250 mg, 3.0 mmol) were successively added; and the mixture was stirred at 100°C for 5 h, and cooled to room temperature. 10 mL of water was added, and the mixture was filtered. The filter cake was dried under reduced pressure and then purified by silica gel column chromatography (mobile phase : dichloromethane/methanol (V/V) = 100/1-20/1) to obtain **10F** (230 mg, yield: 43%).

LCMS m/z = 739.3 $[M+H]^+$.

Step 6: 5-(9-((1-(4-amino-5-methoxy-2-(1-methyl-1H-pyrazol-4-yl)phenyl) piperidin-4-yl)methyl)-2,9-diazaspiro[5.5]undecan-2-yl)-2-(2,6-dioxopiperidin-3-yl)isoindoline-1,3-dione **(10G)**

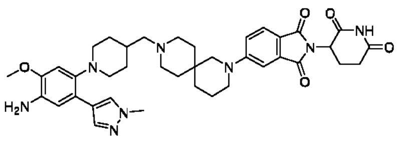

10 G

**10F** (230 mg, 0.31 mmol) was dissolved in ethanol/water (8 mL, 3 : 1); iron powder (53 mg, 0.95 mmol) and ammonium chloride (50 mg, 0.93 mmol) were successively added; and under nitrogen protection, the mixture was stirred at 80°C for 1 h, cooled to room temperature and subjected to suction filtration under reduced pressure. The filtrate was concentrated under reduced pressure, and then the crude was purified by silica gel column chromatography (mobile phase: dichloromethane/methanol (V/V) = 100/1-20/1) to obtain **10G** as a yellow solid (150 mg, yield: 68%).

LCMS m/z = 709.3 $[M+H]^+$.

Step 7: 5-(9-((1-(4-((5-chloro-4-((5-(dimethylphosphoryl)quinoxalin-6-yl)amino) pyrimidin-2-yl)amino)-5-methoxy-2-(1-methyl-1H-pyrazol-4-yl)phenyl)piperidin-4-yl)methyl)-2,9-diazaspiro[5.5]undecan-2-yl)-2-(2,6-dioxopiperidin-3-yl)isoindoline-1,3-dione (compound **10**)

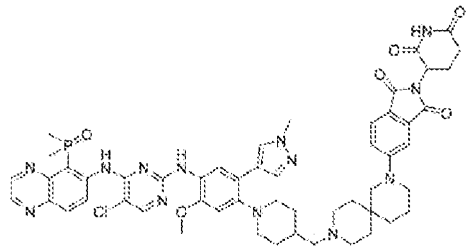

Compound 10

**10G** (150 mg, 0.21 mmol) and **1I** (95.7 mg, 0.26 mmol) were dissolved in DMF (5 mL); p-toluenesulfonic acid monohydrate (97 mg, 0.51 mmol) was added; and under nitrogen protection, the mixture was stirred at 100°C for 16 h, and cooled to room temperature. 20 mL of saturated aqueous sodium bicarbonate solution and 50 mL of dichloromethane were added, and the layers were separated. The organic layer was concentrated under reduced pressure, and the residue was purified by silica gel column chromatography (mobile phase: dichloromethane/methanol (V/V) = 100/1-10/1) to obtain compound **10** (30 mg, yield: 14%).

LCMS m/z = 1040.6 $[M+H]^+$.

$^1H$ NMR (400 MHz, DMSO-$d_6$) $\delta$ 12.89 (s, 1H), 11.05 (s, 1H), 9.06 - 8.89 (m, 1H), 8.87 - 8.76 (m, 2H), 8.37 (s, 1H), 8.19 (s, 1H), 8.00 (s, 1H), 7.79 (s, 1H), 7.67 - 7.46 (m, 3H), 7.29 (d, 1H), 7.23 (dd, 1H), 6.83 (s, 1H), 5.05 (dd, 1H), 3.79 (s, 3H), 3.77 (s, 3H), 3.51 - 3.41 (m, 2H), 3.36 (s, 2H), 3.14 - 3.01 (m, 2H), 2.95 - 2.80 (m, 1H), 2.69 - 2.53 (m, 4H), 2.45 - 2.20 (m, 6H), 2.08 - 1.93 (m, 7H), 1.81 - 1.70 (m, 2H), 1.69 - 1.45 (m, 7H), 1.45 - 1.20 (m, 4H).

**Example 11: Preparation of compound 11**

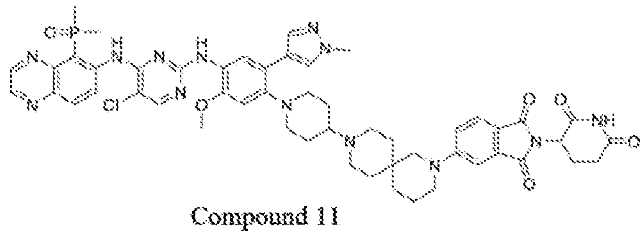

Compound 11

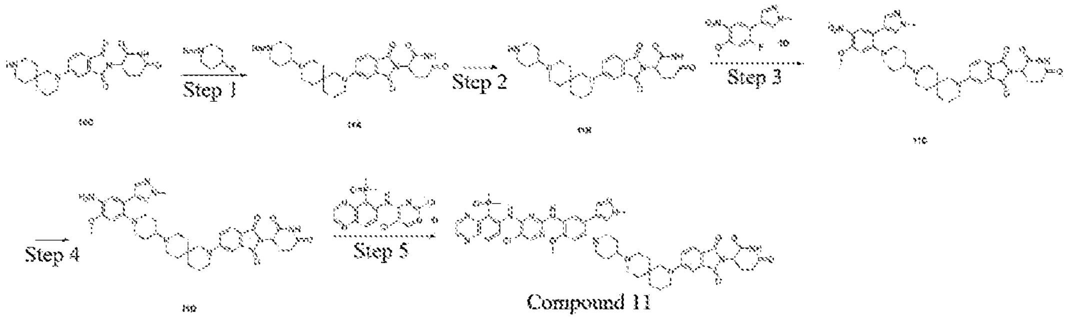


Step 1: tert-butyl 4-(2-(2-(2,6-dioxopiperidin-3-yl)-1,3-dioxoisoindolin-5-yl)-2,9-diazaspiro[5.5]undecan-9-yl)piperidine-1-carboxylate **(11A)**

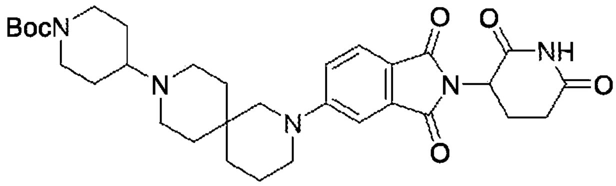

**11A**

The trifluoroacetate of **10C** (0.25 g) and N-tert-butoxycarbonyl-4-piperidone (183 mg, 0.92 mmol) were dissolved in DMAc (10 mL), and acetic acid (39 mg, 0.65 mmol) was added. The mixture was stirred at room temperature for 30 min, and then sodium triacetoxyborohydride (192 mg, 0.91 mmol) was added; and the resulting mixture was reacted overnight at room temperature, and extracted by adding 20 mL of saturated aqueous sodium bicarbonate solution and 20 mL of dichloromethane. The organic layer was concentrated under reduced pressure, and the residue was purified by silica gel column chromatography (mobile phase : dichloromethane/methanol (V/V) = 100/1-20/1) to obtain **11A** (250 mg, yield: 69%).

LCMS m/z = 594.3 $[M+H]^+$.

Step 2: 2-(2,6-dioxopiperidin-3-yl)-5-(9-(piperidin-4-yl)-2,9-diazaspiro[5.5] undecan-2-yl)isoindoline-1,3-dione **(11B);** HCl

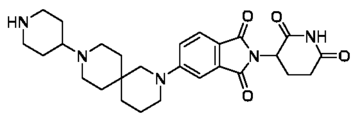

**11B**

**11A** (250 mg, 0.42 mmol) was dissolved in methanol (2 mL); a solution of hydrochloric acid in dioxane (2 *N,* 6

mL) was added; and the mixture was reacted at room temperature for 1 h and concentrated under reduced pressure, and the residue was directly used in the next step.

Step 3: 2-(2,6-dioxopiperidin-3-yl)-5-(9-(1-(5-methoxy-2-(1-methyl-1H-pyrazol-4-yl)-4-nitrophenyl)piperidin-4-yl)-2,9-diazaspiro[5.5]undecan-2-yl)isoindoline-1,3-dione **(11C)**

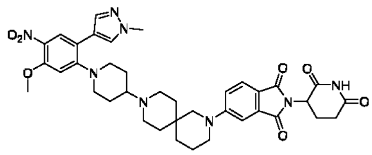

11C

The crude hydrochloride of **11B** from the previous step was dissolved in DMSO (5 mL); 4-(2-fluoro-4-methoxy-5-nitrophenyl)-1-methyl-1H-pyrazole **(1D)** (158 mg, 0.63 mmol) and sodium bicarbonate (353 mg, 4.2 mmol) were successively added; and the mixture was reacted at 100°C for 5 h, and cooled to room temperature. 10 mL of water was added, and the mixture was filtered. The filter cake was dried under reduced pressure and then purified by silica gel column chromatography (mobile phase : dichloromethane/methanol (V/V) = 100/1-20/1) to obtain **11C** (140 mg, yield: 46%).

LCMS m/z = 725.3 $[M+H]^+$.

Step 4: 5-(9-(1-(4-amino-5-methoxy-2-(1-methyl-1H-pyrazol-4-yl)phenyl) piperidin-4-yl)-2,9-diazaspiro[5.5]undecan-2-yl)-2-(2,6-dioxopiperidin-3-yl)isoindoline-1,3-dione **(11D)**

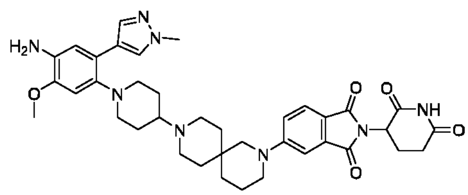

11D

**11C** (140 mg, 0.19 mmol) was dissolved in ethanol/water (8 mL, 3:1); iron powder (53 mg, 0.95 mmol) and ammonium chloride (51 mg, 0.95 mmol) were successively added; and under nitrogen protection, the mixture was reacted at 80°C for 1 h, cooled to room temperature and subjected to suction filtration under reduced pressure. The filtrate was concentrated under reduced pressure, and then the crude was purified by silica gel column chromatography (mobile phase: dichloromethane/methanol (V/V) = 100/1-20/1) to obtain **11D** (120 mg, yield: 91%).

LCMS m/z = 695.3 $[M+H]^+$.

Step 5: 5-(9-(1-(4-((5-chloro-4-((5-(dimethylphosphoryl)quinoxalin-6-yl)amino) pyrimidin-2-yl)amino)-5-methoxy-2-(1-methyl-1H-pyrazol-4-yl)phenyl)piperidin-4-yl)-2,9-diazaspiro[5.5]undecan-2-yl)-2-(2,6-dioxopiperidin-3-yl)isoindoline-1,3-dione **(compound 11)**

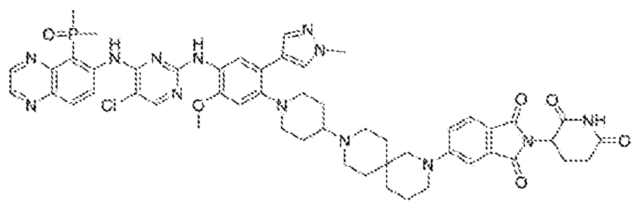

Compound 11

**11D** (120 mg, 0.17 mmol) and (6-((2,5-dichloropyrimidin-4-yl)amino)quinoxalin-5-yl)dimethylphosphine oxide **(1I)** (96 mg, 0.26 mmol) were dissolved in DMF (5 mL); p-toluenesulfonic acid monohydrate (97 mg, 0.51 mmol) was added; and under nitrogen protection, the mixture was reacted at 100°C for 16 h, and cooled to room temperature. 20 mL of saturated aqueous sodium bicarbonate solution and 50 mL of dichloromethane were added, and the layers were separated. The organic layer was concentrated under reduced pressure, and the residue was purified by silica gel column chromatography (mobile phase: dichloromethane/methanol (V/V) = 100/1-20/1) to obtain **compound 11** (40 mg, yield: 23%).

LCMS m/z = 1026.5 $[M+H]^+$.

$^1H$ NMR (400 MHz, DMSO-$d_6$) $\delta$ 12.88 (s, 1H), 11.05 (s, 1H), 9.04 - 8.89 (m, 1H), 8.88 - 8.76 (m, 2H), 8.37 (s, 1H), 8.19 (s, 1H), 7.99 (s, 1H), 7.79 (s, 1H), 7.65 - 7.49 (m, 3H), 7.34 - 7.27 (m, 1H), 7.24 (dd, 1H), 6.83 (s, 1H), 5.05 (dd, 1H), 3.80 (s, 3H), 3.76 (s, 3H), 3.51 - 3.42 (m, 2H), 3.39 - 3.34 (m, 2H), 3.19 - 3.09 (m, 2H), 2.95 - 2.82 (m, 1H), 2.69 - 2.53 (m, 6H), 2.38 - 2.26 (m, 1H), 2.08 - 1.94 (m, 8H), 1.89 - 1.78 (m, 2H), 1.70 - 1.34 (m, 11H).

**Example 12: Preparation of compound 12**

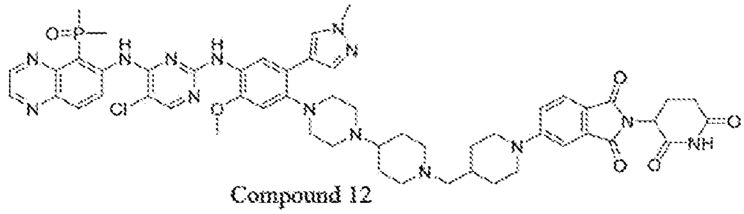

Compound 12

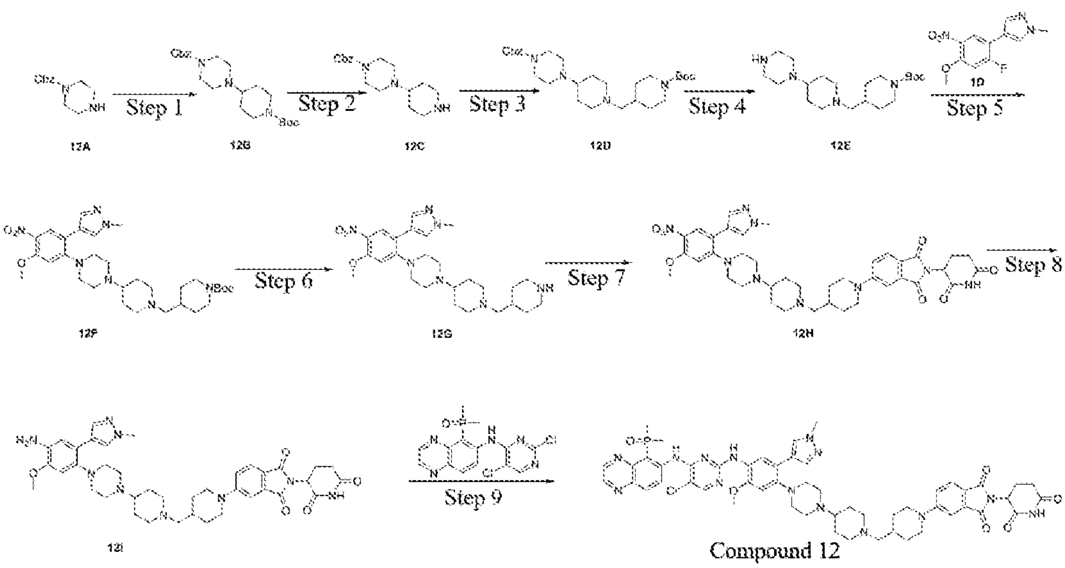


Step 1: benzyl 4-(1-(tert-butoxycarbonyl)piperidin-4-yl)piperazine-1-carboxylate **(12B)**

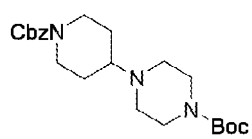

**12B**

**12A** (6.6 g, 30 mmol) and tert-butyl 4-oxopiperidine-1-carboxylate (6.0 g, 30 mmol) were mixed in DMAc (220 mL); acetic acid (1.2 g, 20 mmol) and sodium triacetoxyborohydride (8.4 g, 40 mmol) were successively added; and the mixture was stirred overnight at room temperature. 500 mL of dichloromethane and 250 mL of 1 N aqueous sodium

hydroxide solution were added, and then the layers were separated. The organic layer was dried under reduced pressure, and the residue was purified by silica gel column chromatography (mobile phase : petroleum ether/ethyl acetate (V/V) = 10/1-1/1) to obtain 1**2B** (8 g, yield: 66%).

LCMS m/z = 404.3 [M+H]+.

Step 2: benzyl 4-(piperidin-4-yl)piperazine-1-carboxylate **(12C)**

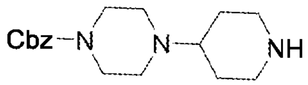

12C

**12B** (8.0 g, 19.8 mmol) was dissolved in dichloromethane (120 mL); trifluoroacetic acid (40 mL) was slowly added; the mixture was reacted at room temperature for 1 h, concentrated under reduced pressure to remove dichloromethane and trifluoroacetic acid, and slurried by adding methyl tert-butyl ether to obtain a white solid, which was then dissolved by adding 200 mL of dichloromethane; and the resulting mixture was adjusted to a basic pH by adding 1 N aqueous sodium hydroxide solution. Liquid separation was performed. The organic phases were collected, and the aqueous phase was extracted 3 times with dichloromethane. All organic phases were combined, dried over anhydrous sodium sulphate and concentrated under reduced pressure to obtain **12C** (5.0 g, yield: 83%), which was directly used in the next step.

LCMS m/z = 304.3 [M+H]+.

Step 3: benzyl 4-(1-((1-(tert-butoxycarbonyl)piperidin-4-yl)methyl)piperidin-4-yl)piperazine-1-carboxylate **(12D)**

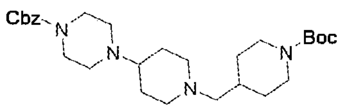

12D

**12C** (5.0 g, 16.5 mmol) and tert-butyl 4-formylpiperidine-1-carboxylate (3.5 g, 16.4 mmol) were mixed in DMAc (100 mL); acetic acid (0.3 g, 5 mmol) and sodium triacetoxyborohydride (4.2 g, 20 mmol) were successively added; and the mixture was stirred overnight at room temperature. 500 mL of dichloromethane and 250 mL of 1 N aqueous sodium hydroxide solution were added, and then the layers were separated. The organic layer was dried under reduced pressure, and the residue was purified by silica gel column chromatography (mobile phase : petroleum ether/ethyl acetate (V/V) = 10/1-1/1) to obtain **12D** (4.8 g, yield: 58%).
LCMS m/z = 501.4[M+H]+

Step 4: tert-butyl 4-((4-(piperazin-1-yl)piperidin-1-yl)methyl)piperidine-1-carboxylate **(12E)**

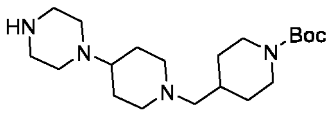

12E

**12D** (4.8 g, 9.6 mmol) was dissolved in methanol solution (100 mL); palladium on carbon (wt% = 10%, 2.93 g) was added; and the mixture was subjected to 1 atm hydrogen replacement 3 times and reacted at room temperature for 1.5 h. The solution was filtered over celite. The filter cake was washed with dichloromethane/methanol (V/V = 10/1), and the filtrate was concentrated under reduced pressure to obtain the crude of **12E** (3.2 g), which was directly used in the next step.

LCMS m/z = 367.3 [M+H]+.

Step 5: tert-butyl 4-((4-(4-(5-methoxy-2-(1-methyl-1H-pyrazol-4-yl)-4-nitrophenyl)piperazin-1-yl)piperidin-1-yl)methyl)piperidine-1-carboxylate **(12F)**

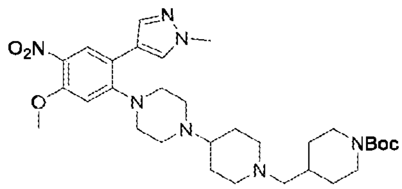

**12F**

**12E** (0.8 g, 2.18 mmol) and **1D** (0.63 g, 2.5 mmol) were dissolved in DMSO (20 mL); potassium carbonate (0.59 mg, 4.26 mmol) was added; and the mixture was reacted at 120°C for 6 h, cooled to room temperature and extracted by adding 30 mL of ethyl acetate and 30 mL of water. The organic layer was washed with saturated brine (20 mL x 3), dried over anhydrous sodium sulphate and concentrated under reduced pressure, and the residue was purified by column chromatography (dichloromethane : methanol (V/V) = 100/1-20/1) to obtain **12F** (0.4 g, yield: 31%).

LCMS m/z = 598.4$[M+H]^+$.

Step 6: 1-(5-methoxy-2-(1-methyl-1H-pyrazol-4-yl)-4-nitrophenyl)-4-(1 - (piperidin-4-ylmethyl) piperidin-4-yl)piperazine **(12G)**

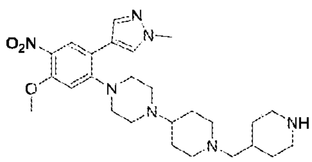

**12G**

**12F** (0.4 g, 0.67 mmol) was dissolved in dichloromethane (6 mL); trifluoroacetic acid (3.06 g, 26.84 mmol) was added at room temperature; and the mixture was stirred at room temperature for 2 h, and concentrated under reduced pressure. 20 mL of dichloromethane was added to the residue, and the mixture was adjusted to pH > 10 with 1 N aqueous sodium hydroxide solution. Liquid separation was performed. The organic layer was then dried over anhydrous sodium sulphate and concentrated under reduced pressure to obtain the crude of **12G** (0.30 g), which was directly used in the next step.

Step 7: 2-(2,6-dioxopiperidin-3-yl)-5-(4-((4-(4-(5-methoxy-2-(1-methyl-1H-pyrazol-4-yl)-4-nitrophenyl)piperazin-1 -yl)piperidin-1 -yl)methyl)piperidin-1 - yl)isoindoline-1,3-dione **(12H)**

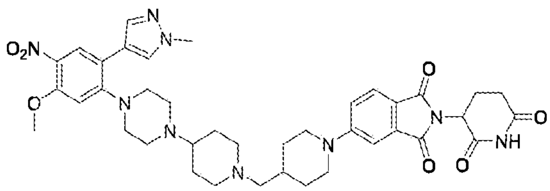

**12H**

**12G** (0.3 g, 0.60 mmol) and 2-(2,6-dioxopiperidin-3-yl)-5-fluoroisoindoline-1,3-dione (0.25 g, 0.91 mmol) were dissolved in DMSO (10 mL); DIPEA (0.21 g, 1.62 mmol) was added; and the mixture was stirred at 90°C for 5 h and cooled to room temperature. 20 mL of water was added, and the mixture was filtered. The filter cake was dried under

reduced pressure and then purified by silica gel column chromatography (dichloromethane : methanol (V/V) = 100/1-15/1) to obtain 1**2H** (0.28 g, yield: 62%).

LCMS m/z = 754.3 $[M+H]^+$.

Step 7: 5-(4-((4-(4-(4-amino-5-methoxy-2-(1-methyl-1H-pyrazol-4-yl)phenyl)piperazin-1-yl)piperidin-1-yl)methyl)piperidin-1-yl)-2-(2,6-dioxopiperidin-3-yl)isoindoline-1,3-dione **(12I)**

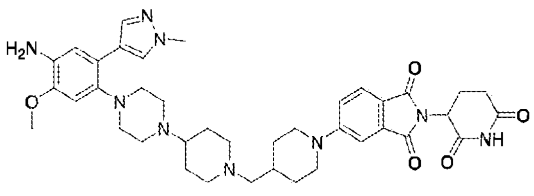

**12I**

**12H** (0.28 g, 0.37 mmol) was dissolved in ethanol (12 mL) and water (3 mL), and the mixture was warmed to 80°C. A mixture of ammonium chloride (0.22 g, 4.11 mmol) and iron powder (0.23 g, 4.12 mmol) was added. At this temperature, the resulting mixture was stirred for 0.5 h, cooled to room temperature and filtered. The filter cake was washed with 50 mL of dichloromethane. 10 mL of saturated brine was added to the filtrate. Liquid separation was performed. The organic layer was dried over anhydrous sodium sulphate and concentrated under reduced pressure to obtain the crude of **12I** (0.19 g), which was directly used in the next step.

LCMS m/z = 724.3 $[M+H]^+$.

Step 9: 5-(4-((4-(4-(4-((5-chloro-4-((5-(dimethylphosphoryl)quinoxalin-6-yl)amino)pyrimidin-2-yl)amino)-5-methoxy-2-(1-methyl-1H-pyrazol-4-yl)phenyl)piperazin-1-yl)piperidin-1-yl)methyl)piperidin-1-yl)-2-(2,6-dioxopiperidin-3-yl)isoindoline-1,3-dione **(compound 12)**

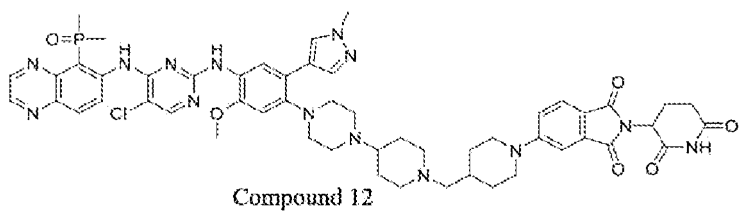

Compound 12

**12I** (0.19 g, 0.26 mmol) and **1I** (0.12 g, 0.33 mmol) were dissolved in DMF (5 mL); p-toluenesulfonic acid monohydrate (0.16 g, 0.84 mmol) was added; and under nitrogen protection, the mixture was stirred at 100°C for 16 h, and cooled to room temperature. 5 mL of water and 5 mL of saturated aqueous sodium bicarbonate solution were added, and the mixture was filtered. The filter cake was dried under reduced pressure and then purified by silica gel column chromatography (dichloromethane/methanol (V/V) = 100/1-12/1) to obtain a product, which was further purified by preparative HPLC ((instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized to obtain the trifluoroacetate of **compound 12** (32 mg).

LCMS m/z = 528.3$[(M+2H)/2]^+$.

$^1H$ NMR (400 MHz, $CDCl_3$) δ 13.49 (s, 1H), 10.60 (s, 1H), 8.95 - 8.68 (m, 4H), 7.95 (s, 1H), 7.91 (s, 1H), 7.67 (d, 1H), 7.63 - 7.50 (m, 3H), 7.25 - 7.22 (m, 1H), 7.08 - 7.01 (m, 1H), 6.70 (s, 1H), 4.96 (dd, 1H), 3.97 - 3.90 (m, 2H), 3.87 - 3.70 (m, 9H), 3.37 - 3.20 (m, 6H), 3.07 - 2.57 (m, 13H), 2.47 - 2.34 (m, 2H), 2.21 - 1.94 (m, 10H), 1.47 - 1.32 (m, 2H).

**Example 13: Preparation of compound 13**

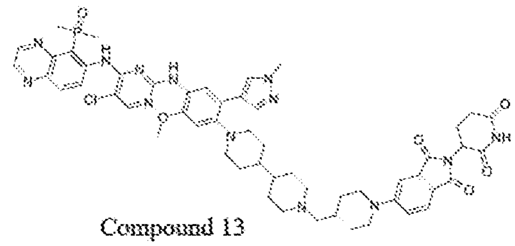

Compound 13

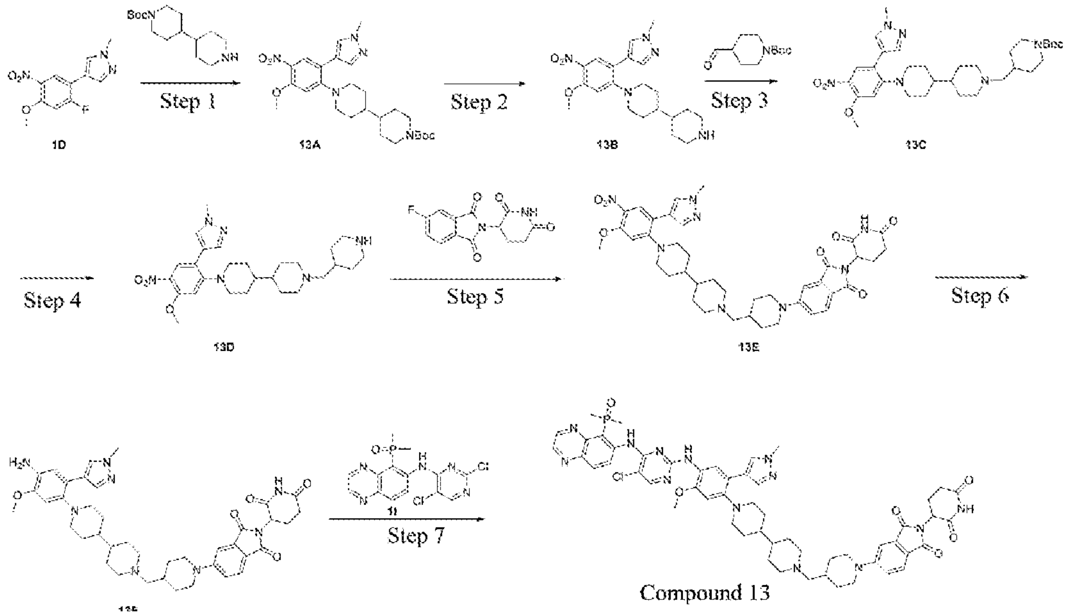


Step 1: tert-butyl 1'-(5-methoxy-2-(1-methyl-1H-pyrazol-4-yl)-4-nitrophenyl)-[4,4'-bipiperidine]-1-carboxylate (13A)

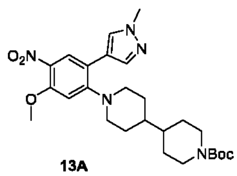

13A

**1D** (1.0 g, 3.98 mmol) and 1-(tert-butoxycarbonyl)-4, 4'-bipiperidine (1.28 g, 4.77 mmol) were dissolved in DMSO (50 mL); potassium carbonate (1.10 mg, 7.96 mmol) was added; and the mixture was reacted at 120°C for 12 h, cooled to room temperature and extracted by adding 100 mL of ethyl acetate and 100 mL of water. The organic layer was washed with saturated brine (30 mL × 3), dried over anhydrous sodium sulphate and concentrated under reduced pressure, and the residue was separated and purified by silica gel column chromatography (ethyl acetate : petroleum ether (V/V) = 10/1-2/1) to obtain **13A** (1.3 g, yield: 65%).
LCMS m/z = 500.3 $[M+H]^+$.

Step 2: 1-(5-methoxy-2-(1-methyl-1H-pyrazol-4-yl)-4-nitrophenyl)-4,4'-bipiperidine **(13B)**

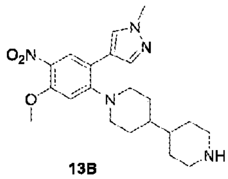

13B

To a 50 mL round bottom flask were successively added **13A** (1.3 g, 2.60 mmol) and a solution of hydrogen chloride in 1,4-dioxane (30 mL, 4 mol/L); and the mixture was reacted at room temperature for 2 h and concentrated under reduced pressure. 1,4-dioxane (20 mL) and ammonia water (5 mL) were added; and the mixture was stirred at room temperature for 20 min and concentrated to dryness under reduced pressure at 50°C to obtain **13B**, which was directly used in the next reaction.

LCMS m/z = 400.3 $[M+H]^+$.

Step 3: tert-butyl 4-((1'-(5-methoxy-2-(1-methyl-1H-pyrazol-4-yl)-4-nitrophenyl)-[4,4'-bipiperidin]-1-yl)methyl)piperidine-1-carboxylate **(13C)**

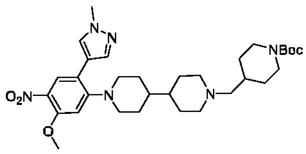

13C

**13B** (0.5 g, 1.25 mmol) and tert-butyl 4-formylpiperidine-1-carboxylate (0.54 g, 2.53 mmol) were dissolved in DMAc (10 mL); a drop of acetic acid was added; and then the mixture was stirred at room temperature for 1 h. Sodium triacetoxyborohydride (0.79 g, 3.73 mmol) was added, and the mixture was reacted overnight at room temperature, adjusted to a basic pH by adding saturated aqueous sodium bicarbonate solution and extracted with ethyl acetate (50 mL × 3). The organic phases were combined, washed sequentially with water (30 mL × 2) and saturated brine (30 mL), dried over anhydrous sodium sulphate and concentrated under reduced pressure, and the residue was purified by silica gel column chromatography (mobile phase : ethyl acetate/petroleum ether (V/V) = 1/1 to dichloromethane/methanol (V/V) = 20/1) to obtain **13C** (0.62 g, yield: 83%).

LCMS m/z = 597.4$[M+H]^+$.

Step 4: 1-(5-methoxy-2-(1-methyl-1H-pyrazol-4-yl)-4-nitrophenyl)-1'-(piperidin-4-ylmethyl)-4,4'-bipiperidine **(13D)**

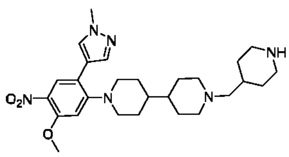

13D

To a 50 mL round bottom flask were successively added **13C** (0.47 g, 0.79 mmol) and a solution of hydrogen chloride in 1,4-dioxane (20 mL, 4 mol/L); and the mixture was reacted at room temperature for 2 h and concentrated under reduced pressure. 1,4-dioxane (20 mL) and ammonia water (5 mL) were added; and the mixture was stirred at room temperature for 20 min and concentrated to dryness under reduced pressure at 50°C to obtain **13D**, which was directly used in the next reaction.

LCMS m/z = 497.3 $[M+H]^+$.

Step 5: 2-(2,6-dioxopiperidin-3-yl)-5-(4-((1'-(5-methoxy-2-(1-methyl-1H-pyrazol-4-yl)-4-nitrophenyl)-[4,4'-bipiperidin]-1-yl)methyl)piperidin-1-yl)isoindoline-1,3-dione **(13E)**

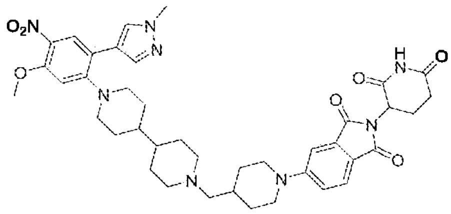

13E

**13D** (387 mg, 0.78 mmol) was dissolved in DMSO (10 mL); 2-(2,6-dioxopiperidin-3-yl)-5-fluoroisoindoline-1,3-dione (258 mg, 0.93 mmol) and DIPEA (504 mg, 3.90 mmol) were added at room temperature; and the mixture was reacted at 100°C for 5 h. 30 mL of water was added to the reaction solution. The aqueous phase was extracted with ethyl acetate (50 mL × 3). The organic phases were combined, washed with water (30 mL × 2), dried over anhydrous sodium sulphate and concentrated. The residue was separated and purified by silica gel column chromatography (DCM : MeOH = 15 : 1) to obtain **13E** (377 mg, yield 64%).

LCMS m/z = 753.4[M+H]$^+$.

Step 6: 5-(4-((1'-(4-amino-5-methoxy-2-(1-methyl-1H-pyrazol-4-yl)phenyl)-[4,4'-bipiperidin]-1 -yl)m ethyl)piperidin-1 -yl)-2-(2,6-dioxopiperi din-3 - yl)isoindoline-1,3-dione **(13F)**

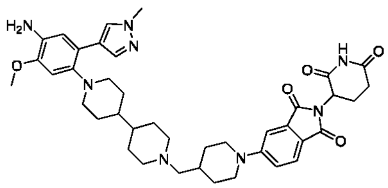

13F

**13E** (377 mg, 0.5 mmol), iron powder (140 mg, 2.5 mmol) and ammonium chloride (134 mg, 2.5 mmol) were dissolved in ethanol (10 mL) and water (5 mL); and the mixture was stirred at 85°C for 2 h, cooled to room temperature and subjected to suction filtration. The filtrate was concentrated to obtain **13F** (236 mg, 65%), which was directly used in the next step.

LCMS m/z = 362.3 [(M+2H)/2]$^+$.

Step 7: 5-(4-((1'-(4-((5-chloro-4-((5-(dimethylphosphoryl)quinoxalin-6-yl)amino) pyrimidin-2-yl)amino)-5-methoxy-2-(1-methyl-1H-pyrazol-4-yl)phenyl)-[4,4'-bipiperidin]-1-yl)methyl)piperidin-1-yl)-2-(2,6-dioxopiperidin-3-yl)isoindoline-1,3-dione **(compound 13)**;2,2,2-trifluoroacetic acid

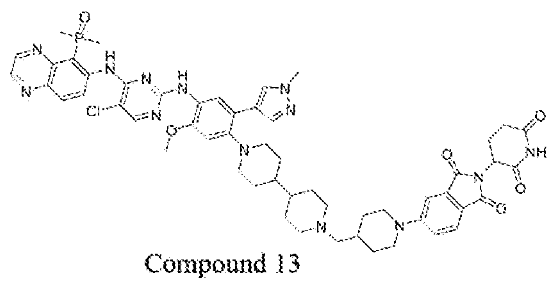

Compound 13

**13F** (236 mg, 0.33 mmol) and **1I** (0.18 g, 0.49 mmol) were dissolved in DMF (5 mL); p-toluenesulfonic acid monohydrate (188 mg, 0.99 mmol) was added; and under nitrogen protection, the mixture was stirred at 100°C for 16 h, and cooled to room temperature. 20 mL of saturated aqueous sodium bicarbonate solution and 50 mL of dichloromethane were added, and the layers were separated. The organic layer was concentrated under reduced pressure, and the residue was purified by silica gel column chromatography (mobile phase : DCM/MeOH (V/V) = 100/1-20/1). The crude was further subjected to preparative HPLC (instrument: waters 2767 (preparative liquid phase chromatographic instrument);

chromatographic column: XBridge@ Prep C18 (30 mm × 150 mm); composition of mobile phases: mobile phase A: acetonitrile, and mobile phase B: water (containing 0.1% TFA)) purification to obtain the trifluoroacetate of **compound 13** (100 mg).

LCMS m/z = 1054.4 $[M+H]^+$.

$^1H$ NMR (400 MHz, DMSO-$d_6$) δ 12.97 (s, 1H), 11.06 (s, 1H), 9.05 - 8.81 (m, 1H), 8.89 - 8.74 (m, 3H), 8.51 (s, 1H), 8.21 (s, 1H), 7.98 (s, 1H), 7.83 (s, 1H), 7.68 (d, 1H), 7.64 - 7.49 (m, 2H), 7.36 (d, 1H), 7.28 (dd, 1H), 6.83 (s, 1H), 5.07 (dd, 1H), 4.17 - 4.04 (m, 2H), 3.81 (s, 3H), 3.76 (s, 3H), 3.65 - 3.51 (m, 2H), 3.20 - 3.09 (m, 2H), 3.09 - 2.80 (m, 7H), 2.68 - 2.52 (m, 4H), 2.24 - 2.10 (m, 1H), 2.10 - 1.90 (m, 9H), 1.90 - 1.80 (m, 2H), 1.80 - 1.68 (m, 2H), 1.61 - 1.15 (m, 8H).

**Example 14: Preparation of compound 14**

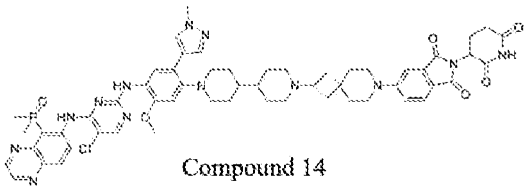

Compound 14

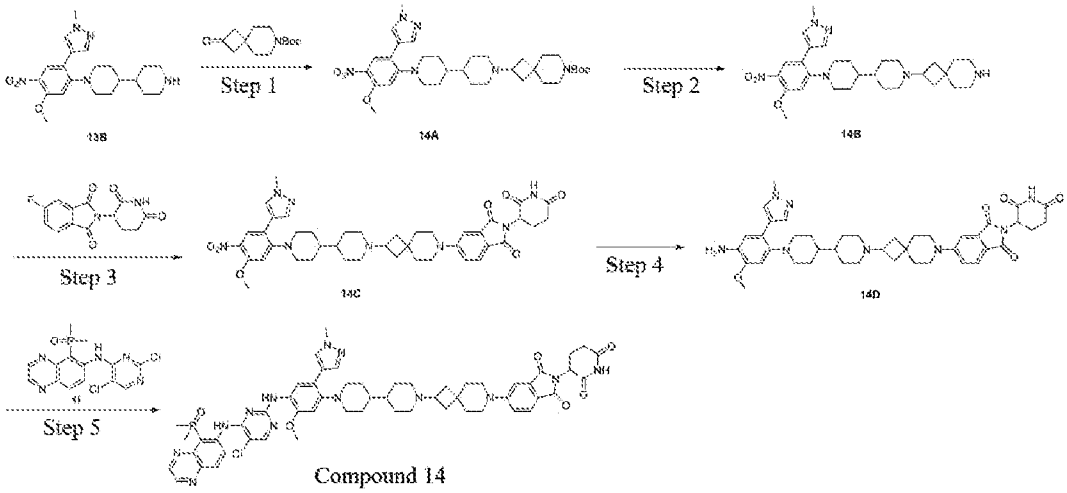


Step 1: tert-butyl 2-(1'-(5-methoxy-2-(1-methyl-1H-pyrazol-4-yl)-4-nitrophenyl)-[4,4'-bipiperidin]-1-yl)-7-azaspiro[3.5]nonane-7-carboxylate **(14A)**

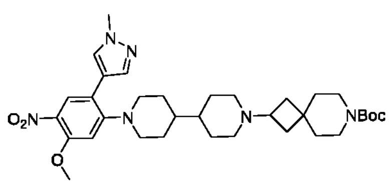

**14A**

**13B** (0.5 g, 1.25 mmol) and tert-butyl 2-oxo-7-azaspiro[3.5]nonane-7-carboxylate (0.54 g, 2.27 mmol) were dissolved in DMAc (10 mL); a drop of acetic acid was added; and then the mixture was stirred at room temperature for 1 h. Sodium triacetoxyborohydride (0.79 g, 3.73 mmol) was added; and the mixture was reacted overnight at room temperature, adjusted to a basic pH by adding saturated aqueous sodium bicarbonate solution and extracted with ethyl acetate (50 mL × 3). The organic phases were combined, washed sequentially with water (30 mL × 2) and saturated brine (30 mL), dried over anhydrous sodium sulphate and concentrated under reduced pressure, and the residue was purified by silica gel column chromatography (mobile phase : ethyl acetate/petroleum ether (V/V) = 1/1 to dichloromethane/methanol (V/V) = 20/1) to obtain **14A** (0.49 g, yield: 63%).
LCMS m/z = 623.4[M+H]+.

Step 2: 2-(1'-(5-methoxy-2-(1-methyl-1H-pyrazol-4-yl)-4-nitrophenyl)-[4,4'-bipiperidin]-1-yl)-7-azaspiro[3.5]nonane **(14B)**

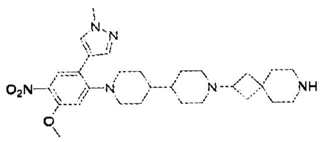

14B

To a 50 mL round bottom flask were successively added **14A** (0.49 g, 0.79 mmol) and a solution of hydrogen chloride in 1,4-dioxane (20 mL, 4 mol/L); and the mixture was reacted at room temperature for 2 h and concentrated under reduced pressure. 1,4-dioxane (20 mL) and ammonia water (5 mL) were added; and the mixture was stirred at room temperature for 20 min and concentrated to dryness under reduced pressure at 50°C to obtain **14B**, which was directly used in the next reaction.
LCMS m/z = 523.4[M+H]+.

Step 3: 2-(2,6-dioxopiperidin-3-yl)-5-(2-(1'-(5-methoxy-2-(1-methyl-1H-pyrazol-4-yl)-4-nitrophenyl)-[4,4'-bipiperidin]-1-yl)-7-azaspiro[3.5]nonan-7-yl)isoindoline-1,3-dione **(14C)**

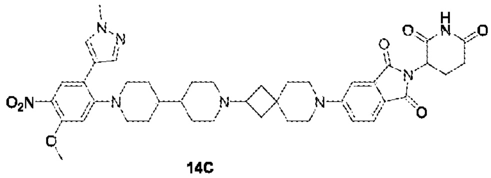

14C

**14B** (402 mg, 0.77 mmol) from the previous step was dissolved in DMSO (10 mL); 2-(2,6-dioxopiperidin-3-yl)-5-fluoroisoindoline-1,3-dione (255 mg, 0.92 mmol) and DIPEA (498 mg, 3.85 mmol) were added at room temperature; and the mixture was stirred at 100°C for 5 h. 30 mL of water was added to the reaction solution. The aqueous phase was extracted with ethyl acetate (50 mL × 3). The organic phases were combined, washed with water (30 mL × 2), dried over anhydrous sodium sulphate and concentrated. The residue was separated and purified by silica gel column chromatography (DCM : MeOH = 15 : 1) to obtain **14C** (350 mg, yield 58%).
LCMS m/z = 779.4[M+H]+.

Step 4: 5-(2-(1'-(4-amino-5-methoxy-2-(1-methyl-1H-pyrazol-4-yl)phenyl)-[4,4'-bipiperidin]-1-yl)-7-azaspiro[3.5]nonan-7-yl)-2-(2,6-dioxopiperidin-3-yl)isoindoline-1,3-dione **(14D)**

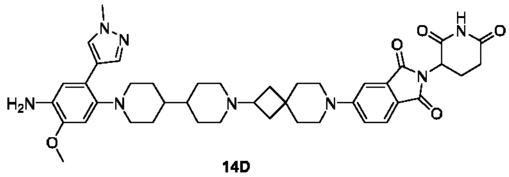

14D

**14C** (350 mg, 0.45 mmol), iron powder (126 mg, 2.26 mmol) and ammonium chloride (134 mg, 2.50 mmol) were dissolved in ethanol (10 mL) and water (5 mL); and the mixture was stirred at 85°C for 2 h, cooled to room temperature and subjected to suction filtration. The filtrate was concentrated to obtain **14D** (144 mg, 43%), which was directly used in the next step.

LCMS m/z = 749.6 $[M+H]^+$.

Step 5: 5-(2-(1'-(4-((5-chloro-4-((5-(dimethylphosphoryl)quinoxalin-6-yl) amino)pyrimidin-2-yl)amino)-5-methoxy-2-(1-methyl-1H-pyrazol-4-yl)phenyl)-[4,4'-bipiperidin]-1-yl)-7-azaspiro[3.5]nonan-7-yl)-2-(2,6-dioxopiperidin-3-yl)isoindoline-1,3-dione **(compound 14)**;2,2,2-trifluoroacetic acid

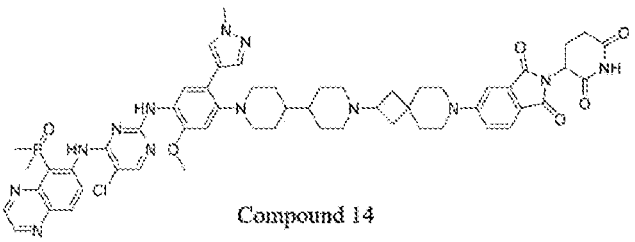

Compound 14

**14D** (144 mg, 0.19 mmol) and **1l** (105 mg, 0.29 mmol) were dissolved in DMF (5 mL); p-toluenesulfonic acid monohydrate (165 mg, 0.87 mmol) was added; and under nitrogen protection, the mixture was stirred at 100°C for 16 h, and cooled to room temperature. 20 mL of saturated aqueous sodium bicarbonate solution and 50 mL of dichloromethane were added, and the layers were separated. The organic layer was concentrated under reduced pressure, and the residue was purified by silica gel column chromatography (mobile phase : DCM/MeOH (V/V) = 100/1-20/1). The crude was further subjected to preparative HPLC (instrument: waters 2767 (preparative liquid phase chromatographic instrument); chromatographic column: XBridge@ Prep C18 (30 mm × 150 mm); composition of mobile phases: mobile phase A: acetonitrile, and mobile phase B: water (containing 0.1% TFA)) purification to obtain the trifluoroacetate of **compound 14** (80 mg).

LCMS m/z = 540.8 $[(M+2H)/2]^+$.

$^1H$ NMR (400 MHz, DMSO-$d_6$) δ 12.96 (s, 1H), 11.06 (s, 1H), 9.38 - 9.17 (m, 1H), 9.05 - 8.91 (m, 1H), 8.89 - 8.77 (m, 2H), 8.49 (s, 1H), 8.21 (s, 1H), 7.99 (s,1H), 7.83 (s, 1H), 7.67 (d, 1H), 7.63 - 7.48 (m, 2H), 7.34 (d, 1H), 7.30 - 7.21 (m, 1H), 6.83 (s, 1H), 5.06 (dd, 1H), 3.86 - 3.59 (m, 7H), 3.57 - 3.30 (m, 6H), 3.22 - 3.11 (m, 2H), 2.96 - 2.82 (m, 1H), 2.81 - 2.65 (m, 2H), 2.65 - 2.53 (m, 3H), 2.30 - 2.15 (m, 2H), 2.09 - 1.90 (m, 10H), 1.83 - 1.56 (m, 7H), 1.53 - 1.14 (m, 7H).

**Example 15: Preparation** of **compound 15**

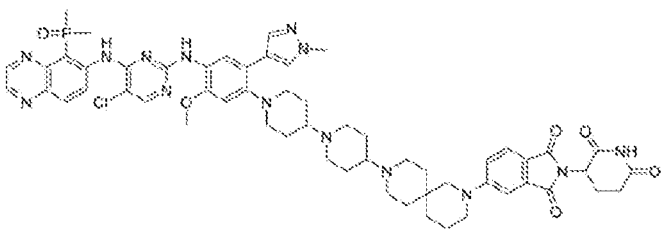

Compound 15

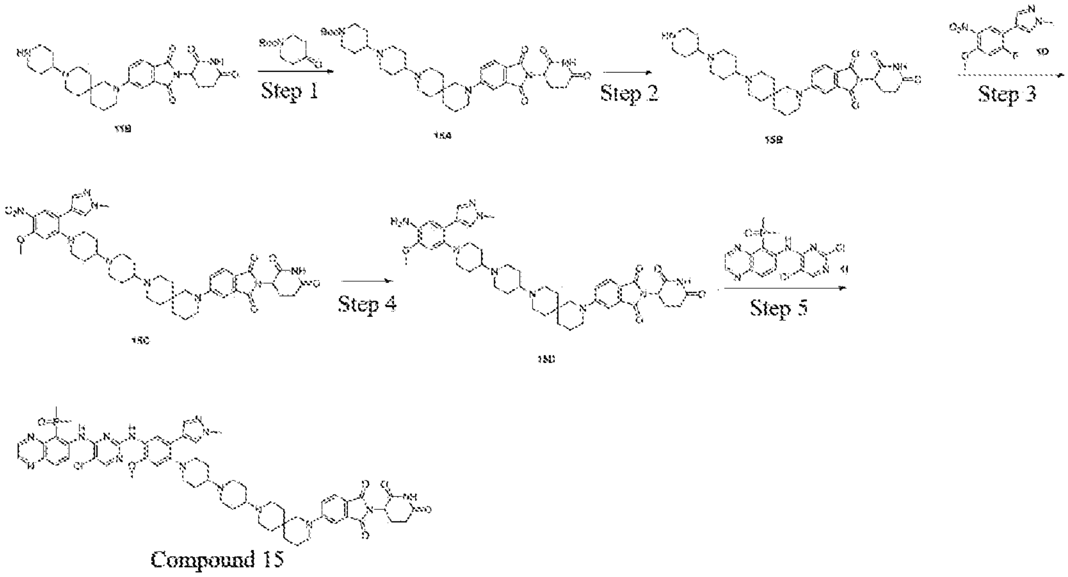


Compound 15

Step 1: tert-butyl 4-(2-(2-(2,6-dioxopiperidin-3-yl)-1,3-dioxoisoindolin-5-yl)-2,9-diazaspiro [5.5]undecan-9-yl)-[1,4'-bipiperidine]-1'-carboxylate **(15A)**

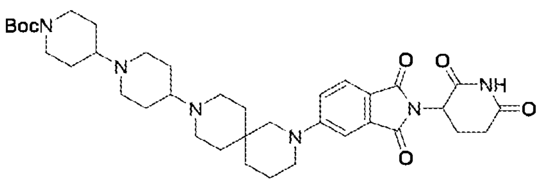

**15A**

**11B** (475 mg, 0.96 mmol) and N-tert-butoxycarbonyl-4-piperidone (287 mg, 1.44 mmol) were dissolved in DMAc (10 mL), and acetic acid (58 mg, 0.96 mmol) was added. The mixture was stirred at room temperature for 60 min, and then sodium triacetoxyborohydride (304 mg, 1.43 mmol) was added; and the resulting mixture was reacted overnight at room temperature, and extracted by adding 20 mL of saturated aqueous sodium bicarbonate solution and 20 mL of dichloromethane. The organic layer was concentrated under reduced pressure, and the residue was purified by silica gel column chromatography (mobile phase : dichloromethane/methanol (V/V) = 100/1-20/1) to obtain **15A** (320 mg, yield: 49%).

LCMS m/z = 677.3 $[M+H]^+$.

Step 2: 5-(9-([1,4'-bipiperidin]-4-yl)-2,9-diazaspiro[5.5]undecan-2-yl)-2-(2,6-dioxopiperidin-3-yl)isoindoline-1,3-dione **(15B)**; HCl

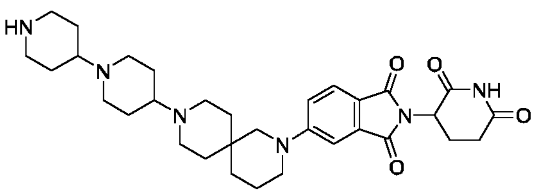

**15B**

Compound **15A** (320 mg, 0.47 mmol) was dissolved in methanol (2 mL); a solution of hydrochloric acid in

dioxane (2 N, 6 mL) was added; and the mixture was reacted at room temperature for 1 h and concentrated under reduced pressure, and the residue was directly used in the next step.

Step 3: 2-(2,6-dioxopiperidin-3-yl)-5-(9-(1'-(5-methoxy-2-(1-methyl-1H-pyrazol-4-yl)-4-nitrophenyl)-[1,4'-bipiperidin]-4-yl)-2,9-diazaspiro[5.5]undecan-2-yl)isoindoline-1,3-dione **(15C)**

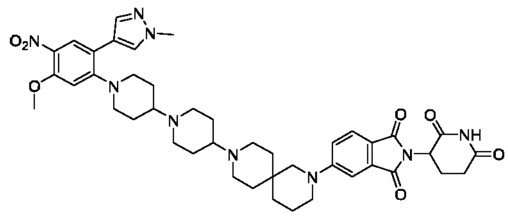

**15C**

The crude hydrochloride of compound **15B** from the previous step was dissolved in DMSO (8 mL); **1D** (163 mg, 0.65 mmol) and sodium bicarbonate (144 mg, 1.71 mmol) were successively added; and the mixture was reacted at 100°C for 6 h, cooled to room temperature and extracted by adding 30 mL of ethyl acetate and 30 mL of water. The organic layer was washed with saturated brine (20 mL × 3), dried over anhydrous sodium sulphate and concentrated under reduced pressure, and the residue was purified by column chromatography (dichloromethane : methanol (V/V) = 100/1-20/1) to obtain **15C** (160 mg, yield: 42%).

LCMS m/z = 808.4 $[M+H]^+$.

Step 4: 5-(9-(1'-(4-amino-5-methoxy-2-(1-methyl-1H-pyrazol-4-yl)phenyl)-[1,4'-bipiperidin]-4-yl)-2,9-diazaspiro[5.5]undecan-2-yl)-2-(2,6-dioxopiperidin-3-yl)isoindoline-1,3-dione **(15D)**

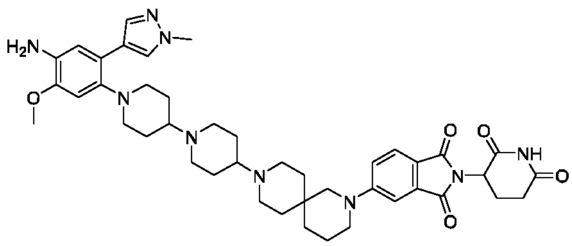

**15D**

**15C** (160 mg, 0.20 mmol) was dissolved in ethanol/water (8 mL, 3 : 1); iron powder (56 mg, 1 mmol) and ammonium chloride (53 mg, 1 mmol) were successively added; and under nitrogen protection, the mixture was stirred at 80°C for 1 h, cooled to room temperature and subjected to suction filtration under reduced pressure. The filtrate was concentrated under reduced pressure, and then the crude was purified by silica gel column chromatography (mobile phase : dichloromethane/methanol (V/V) = 100/1-20/1) to obtain **15D** as a yellow solid (120 mg, yield: 77%).

LCMS m/z = 778.8 $[M+H]^+$.

Step 5: 5-(9-(1'-(4-((5-chloro-4-((5-(dimethylphosphoryl)quinoxalin-6-yl)amino) pyrimidin-2-yl)amino)-5-methoxy-2-(1-methyl-1H-pyrazol-4-yl)phenyl)-[1,4'-bipiperidin]-4-yl)-2,9-diazaspiro[5.5]undecan-2-yl)-2-(2,6-dioxopiperidin-3-yl)isoindoline-1,3-dione **(compound 15)**

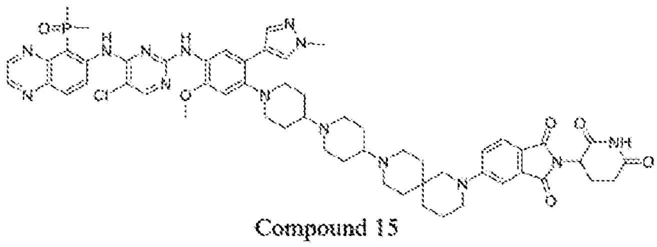

Compound 15

**15D** (120 mg, 0.15 mmol) and **1I** (85 mg, 0.23 mmol) were dissolved in DMF (5 mL); p-toluenesulfonic acid monohydrate (86 mg, 0.45 mmol) was added; and under nitrogen protection, the mixture was stirred at 100°C for 16 h, and cooled to room temperature. 20 mL of saturated aqueous sodium bicarbonate solution and 50 mL of dichloromethane were added, and the layers were separated. The organic layer was concentrated under reduced pressure, and the residue was purified by silica gel column chromatography (mobile phase : dichloromethane/methanol (V/V) = 100/1-20/1) to obtain **compound 15** (30 mg, yield: 18%).

LCMS m/z = 555.3 $[(M+2H)/2]^+$.

$^1H$ NMR (400 MHz, DMSO-$d_6$) δ 12.89 (s, 1H), 11.05 (s, 1H), 9.03 - 8.91 (m, 1H), 8.89 - 8.77 (m, 2H), 8.37 (s, 1H), 8.19 (s, 1H), 7.99 (s, 1H), 7.80 (s, 1H), 7.67 - 7.46 (m, 3H), 7.33 - 7.26 (m, 1H), 7.22 (dd, 1H), 6.82 (s, 1H), 5.05 (dd, 1H), 3.80 (s, 3H), 3.77 (s, 3H), 3.50 - 3.41 (m, 2H), 3.34 (s, 2H), 3.18 - 3.07 (m, 2H), 3.05 - 2.82 (m, 3H), 2.69 - 2.53 (m, 4H), 2.46 - 2.39 (m, 2H), 2.37 - 2.07 (m, 5H), 2.07 - 1.94 (m, 7H), 1.86 - 1.70 (m, 4H), 1.69 - 1.32 (m, 13H).

**Example 16: Preparation of compound 16**

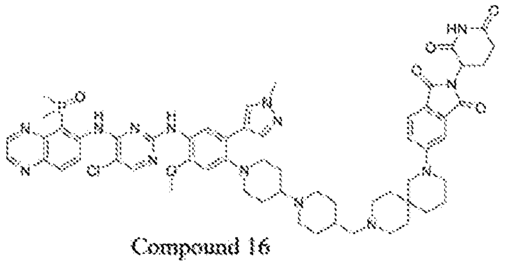

Compound 16

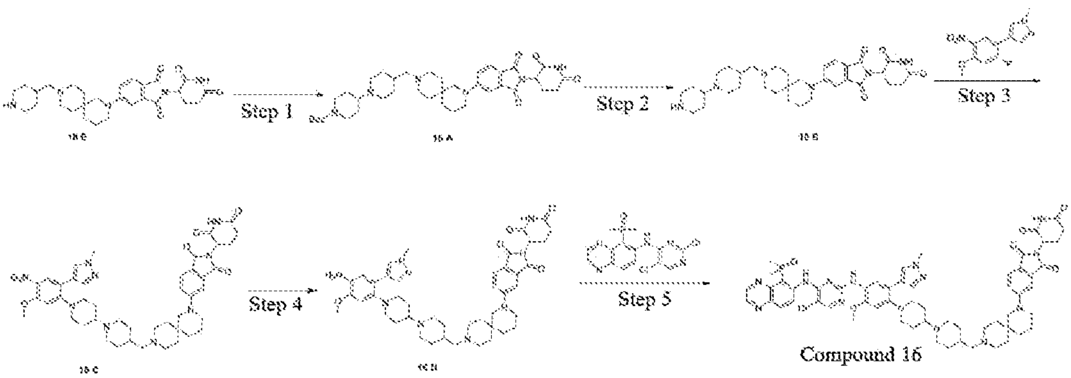


Step 1: tert-butyl 4-((2-(2-(2,6-dioxopiperidin-3-yl)-1,3-dioxoisoindolin-5-yl)-2,9-diazaspiro[5.5]undecan-9-yl)methyl)-[1,4'-bipiperidine]-1'-carboxylate **(16A)**

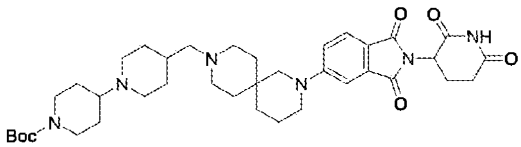

16 A

The trifluoroacetate of **10E** (0.6 g) and tert-butyl 4-oxopiperidine-1-carboxylate (0.4 g, 2.0 mmol) were mixed in DMAc (20 mL); acetic acid (0.12 g, 2 mmol) and sodium triacetoxyborohydride (0.42 g, 2 mmol) were successively added; and the mixture was stirred overnight at room temperature. 100 mL of dichloromethane and 50 mL of 1 N aqueous sodium hydroxide solution were added, and then the layers were separated. The organic layer was dried under reduced pressure, and the residue was purified by silica gel column chromatography (mobile phase : dichloromethane/methanol (V/V) = 10/1-1/1) to obtain **16A** (0.40 g).

LCMS m/z = 691.3 $[M+H]^+$.

Step 2: 5-(9-([1,4'-bipiperidin]-4-ylmethyl)-2,9-diazaspiro[5.5]undecan-2-yl)-2-(2,6-dioxopiperidin-3-yl)isoindoline-1,3-dione **(16B);** 2,2,2-trifluoroacetic acid

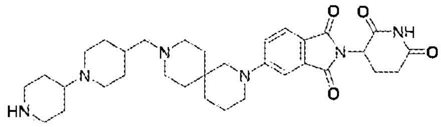

16 B

Compound **16A** (0.40 g, 0.58 mmol) was dissolved in dichloromethane (10 mL); trifluoroacetic acid (2.3 g, 20 mmol) was added; and the mixture was stirred at room temperature for 3 h and concentrated under reduced pressure, and the residue was directly used in the next step.

Step 3: 2-(2,6-dioxopiperidin-3-yl)-5-(9-((1'-(5-methoxy-2-(1-methyl-1H-pyrazol-4-yl)-4-nitrophenyl)-[1,4'-bipiperidin]-4-yl)methyl)-2,9-diazaspiro[5.5]undecan-2-yl)isoindoline-1,3-dione **(16C)**

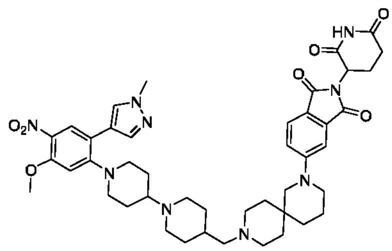

16 C

The crude trifluoroacetate of **16B** from the previous step was dissolved in DMSO (15 mL); 4-(2-fluoro-4-methoxy-5-nitrophenyl)-1-methyl-1H-pyrazole **(1D)** (260 mg, 1.03 mmol) and sodium bicarbonate (250 mg, 3.0 mmol) were successively added; and the mixture was stirred at 100°C for 5 h, and cooled to room temperature. 10 mL of water was added, and the mixture was filtered. The filter cake was dried under reduced pressure and then purified by silica gel column chromatography (mobile phase : dichloromethane/methanol (V/V) = 100/1-10/1) to obtain **16C** (230 mg, yield: 48%).

LCMS m/z = 822.3 $[M+H]^+$.

Step 4: 5-(9-((1'-(4-amino-5-methoxy-2-(1-methyl-1H-pyrazol-4-yl)phenyl)-[1,4'-bipiperidin]-4-yl)methyl)-2,9-diazaspiro[5.5]undecan-2-yl)-2-(2,6-dioxopiperidin-3-yl)isoindoline-1,3-dione **(16D)**

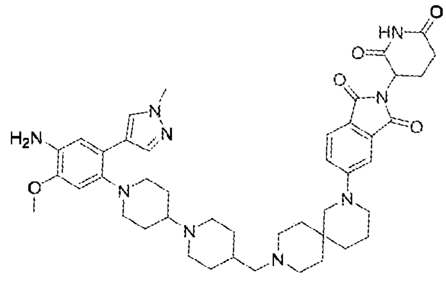

**16 D**

**16C** (230 mg, 0.28 mmol) was dissolved in ethanol/water (8 mL, 3 : 1); iron powder (53 mg, 0.95 mmol) and ammonium chloride (50 mg, 0.93 mmol) were successively added; and under nitrogen protection, the mixture was stirred at 80°C for 1 h, cooled to room temperature and subjected to suction filtration under reduced pressure. The filtrate was concentrated under reduced pressure, and then the crude was purified by silica gel column chromatography (mobile phase: dichloromethane/methanol (V/V) = 100/1-20/1) to obtain **16D** (160 mg, yield: 72%).

LCMS m/z = 792.4 $[M+H]^+$.

Step 5: 5-(9-((1'-(4-((5-chloro-4-((5-(dimethylphosphoryl)quinoxalin-6-yl)amino)pyrimidin-2-yl)amino)-5-methoxy-2-(1-methyl-1H-pyrazol-4-yl)phenyl)-[1,4'-bipiperidin]-4-yl)methyl)-2,9-diazaspiro[5.5]undecan-2-yl)-2-(2,6-dioxopiperidin-3-yl)isoindoline-1,3-dione (compound **16**)

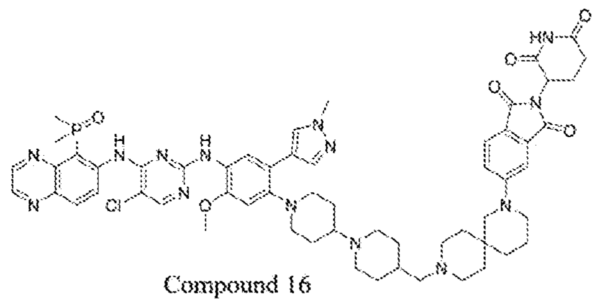

Compound 16

**16D** (160 mg, 0.20 mmol) and **1I** (95.7 mg, 0.26 mmol) were dissolved in DMF (5 mL); p-toluenesulfonic acid monohydrate (97 mg, 0.51 mmol) was added; and under nitrogen protection, the mixture was stirred at 100°C for 16 h, and cooled to room temperature. 20 mL of saturated aqueous sodium bicarbonate solution and 50 mL of dichloromethane were added, and the layers were separated. The organic layer was concentrated under reduced pressure, and the residue was purified by silica gel column chromatography (mobile phase: dichloromethane/methanol (V/V) = 100/1-10/1). The product obtained from the column chromatography was further purified by preparative HPLC ((instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized, and then saturated sodium bicarbonate solution (50 ml) was added. The mixture was extracted with dichloromethane (50 ml). The organic phase was concentrated under reduced pressure to obtain compound **16** (20 mg, yield: 9%).

LCMS m/z = 562.4 $[(M+2H)/2]^+$.

$^1H$ NMR (400 MHz, DMSO-$d_6$) δ 12.94 (s, 1H), 11.06 (s, 1H), 9.01 - 8.91 (m, 1H), 8.88 - 8.77 (m, 2H), 8.53 - 8.42 (m, 1H), 8.22 (s, 1H), 8.01 - 7.82 (m, 2H), 7.70 - 7.48 (m, 3H), 7.44 - 7.20 (m, 2H), 6.82 (s, 1H), 5.11 - 5.02 (m, 1H), 3.82 (s, 3H), 3.77 (s, 3H), 3.69 - 3.58 (m, 2H), 3.56 - 2.82 (m, 18H), 2.75 - 2.53 (m, 4H), 2.16 - 1.96 (m, 10H), 1.93 - 1.57 (m, 10H), 1.54 - 1.45 (m, 2H).

**Example 17: Preparation of compound 17**

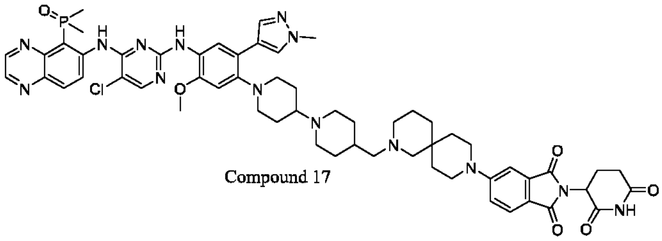

Compound 17

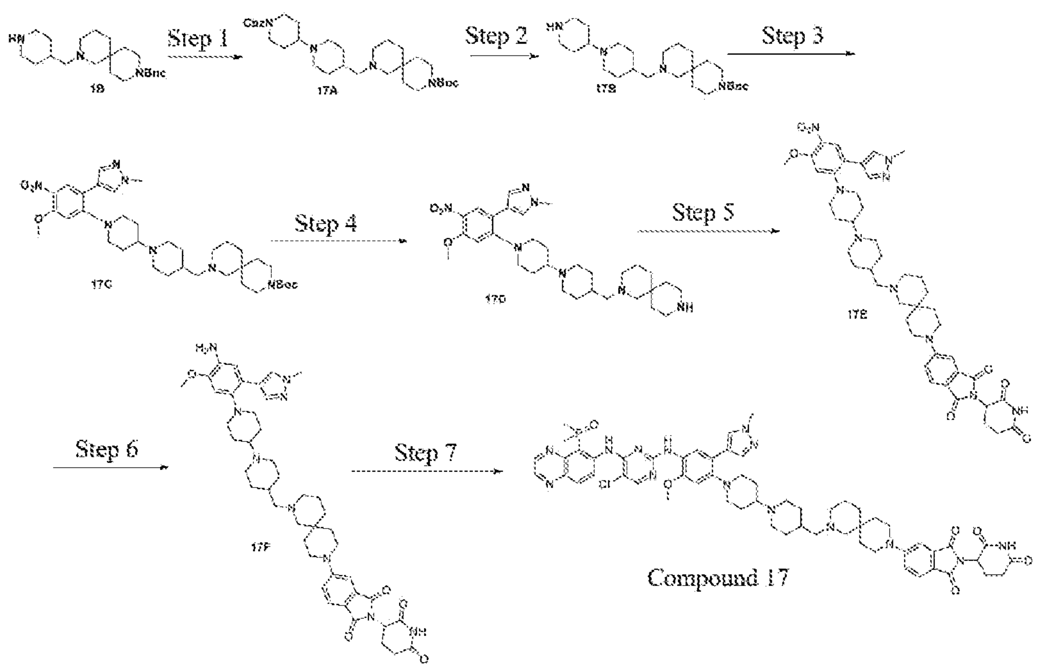


Step 1: tert-butyl 2-((1'-((benzyloxy)carbonyl)-[1,4'-bipiperidin]-4-yl)methyl)-2,9-diazaspiro[5.5]undecane-9-carboxylate **(17A)**

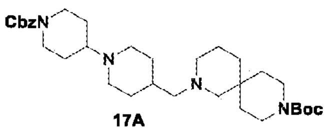

**17A**

**1B** (702 mg, 2 mmol) and benzyl 4-oxopiperidine-1-carboxylate (699 mg, 3 mmol) were mixed in dichloromethane (100 mL); acetic acid (180 mg, 3 mmol) and sodium triacetoxyborohydride (636 mg, 3 mmol) were successively added; and the mixture was stirred overnight at room temperature. 30 mL of dichloromethane and 10 mL of 1 N aqueous sodium hydroxide solution were added, and then the layers were separated. The organic layer was dried under reduced pressure, and the residue was purified by silica gel column chromatography (mobile phase: ethyl acetate/petroleum ether (V/V) = 10/1-1/1) to obtain **17A** (512 mg, yield: 45%).

LCMS m/z = 569.4[M+H]$^+$.

Step 2: tert-butyl 2-([1,4'-bipiperidin]-4-ylmethyl)-2,9-diazaspiro[5.5] undecane-9-carboxylate **(17B)**

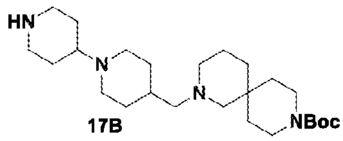

17B

**17A** (512 mg, 0.9 mmol) was dissolved in methanol (10 mL); palladium on carbon (wt% = 10%, 51 mg) was added; the mixture was subjected to hydrogen replacement 3 times, stirred overnight under hydrogen atmosphere (balloon pressure) at room temperature and filtered; and the filtrate was concentrated under reduced pressure to obtain **17B** (390 mg), which was directly used in the next step.

Step 3: tert-butyl2-((1'-(5-methoxy-2-(1-methyl-1H-pyrazol-4-yl)-4-nitrophenyl)-[1,4'-bipiperidin]-4-yl)methyl)-2,9-diazaspiro[5.5]undecane-9-carboxylate **(17C)**

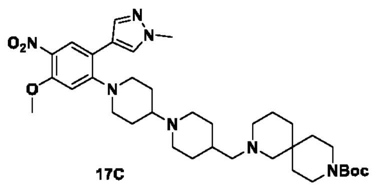

17C

**17B** (390 mg, 0.9 mmol), 4-(2-fluoro-4-methoxy-5-nitrophenyl)-1-methyl-1H-pyrazole **(1D)** (301 mg, 1.2 mmol) and potassium carbonate (414 mg, 3 mmol) were mixed and dissolved in DMSO (10 mL), and the mixture was stirred at 120°C for 16 h, cooled to room temperature and extracted by adding 50 mL of water and 50 mL of ethyl acetate. The organic layer was concentrated under reduced pressure, and the residue was purified by column chromatography (mobile phase: dichloromethane/methanol (V/V) = 100/1-20/1) to obtain **17C** (310 mg, yield: 52%).

LCMS m/z = 666.5$[M+H]^+$.

Step 4: 2-((1'-(5-methoxy-2-(1-methyl-1H-pyrazol-4-yl)-4-nitrophenyl)-[1,4'-bipiperidin]-4-yl)methyl)-2,9-diazaspiro[5.5]undecane **(17D)**

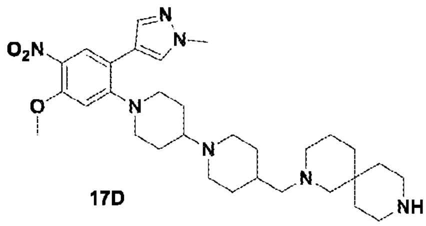

17D

**17C** (310 mg, 0.47 mmol) was dissolved in DCM (10 mL); trifluoroacetic acid (3 mL) was added at room temperature; and the mixture was stirred for 3 h and concentrated under reduced pressure. 20 mL of dichloromethane was added to the residue, and the mixture was adjusted to pH = 9-10 with 1 N aqueous NaOH solution. Liquid separation was performed. The organic layer was concentrated under reduced pressure to obtain **17D** (262 mg), which was directly used in the next step.

Step 5: 2-(2,6-dioxopiperidin-3-yl)-5-(2-((1'-(5-methoxy-2-(1-methyl-1H-pyrazol -4-yl)-4-nitrophenyl)-[1,4'-bipiperidin]-4-yl)methyl)-2,9-diazaspiro[5.5]undecan-9-yl)isoindoline-1,3-dione **(17E)**

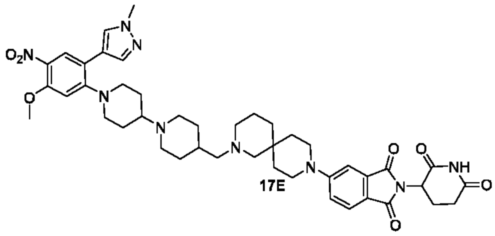

17E

**17D** (262 mg, 0.46 mmol) was dissolved in DMSO (5 mL); 2-(2,6-dioxopiperidin-3-yl)-5-fluoroisoindole-1,3-dione (276 mg, 1 mmol) and DIPEA (0.31 g, 2.4 mmol) were successively added; and the mixture was stirred at 90°C for 3 h, and cooled to room temperature. 10 mL of water was added, and the mixture was filtered. The filter cake was dried under reduced pressure and then purified by silica gel column chromatography (mobile phase: dichloromethane/methanol (V/V) = 100/1-20/1) to obtain **17E** (210 mg, yield: 55%).

Step 6: 5-(2-((1'-(4-amino-5-methoxy-2-(1-methyl-1H-pyrazol-4-yl)phenyl)-[1,4'-bipiperidin]-4-yl)methyl)-2,9-diazaspiro[5.5]undecan-9-yl)-2-(2,6-dioxopiperidin-3-yl)isoindoline-1,3-dione **(17F)**

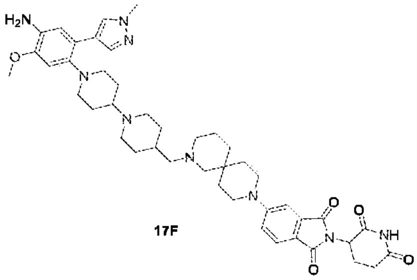

17F

Compound **17E** (210 mg, 0.26 mmol), iron powder (200 mg, 3.57 mmol) and ammonium chloride (200 mg, 3.77 mmol) were dissolved in ethanol (30 mL) and water (10 mL); and the mixture was stirred at 80°C for 2 h, cooled to room temperature and subjected to suction filtration. The filtrate was concentrated to obtain **17F** (150 mg, 74.3%), which was directly used in the next step.

Step 7: 5-(2-((1'-(4-((5-chloro-4-((5-(dimethylphosphoryl)quinoxalin-6-yl)amino) pyrimidin-2-yl)amino)-5-methoxy-2-(1-methyl-1H-pyrazol-4-yl)phenyl)-[1,4'-bipiperidin]-4-yl)methyl)-2,9-diazaspiro[5.5]undecan-9-yl)-2-(2,6-dioxopiperidin-3-yl)isoindoline-1,3-dione **(compound 17)**

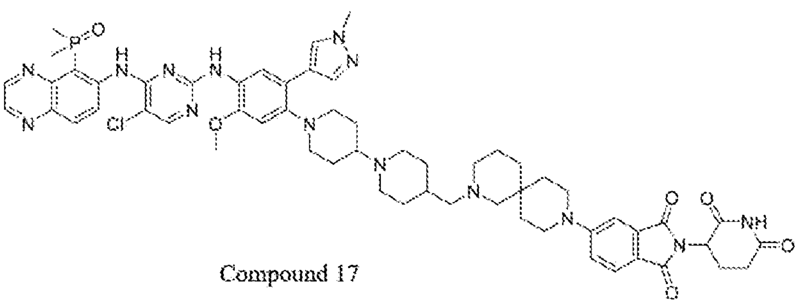

Compound 17

**17F** (70 mg, 0.09 mmol) and (6-((2,5-dichloropyrimidin-4-yl)amino)quinoxalin-5-yl)dimethylphosphine oxide **(1I)** (0.44 g, 0.12 mmol) were dissolved in DMF (5 mL); p-toluenesulfonic acid monohydrate (57 mg, 0.3 mmol) was added; and under nitrogen protection, the mixture was stirred at 100°C for 16 h, and cooled to room temperature. 20 mL of saturated aqueous sodium bicarbonate solution and 50 mL of dichloromethane were added, and the layers were separated. The organic layer was concentrated under reduced pressure, and the residue was purified by silica gel column chromatography (mobile phase: DCM/MeOH (V/V) = 100/1-20/1). The crude was further subjected to preparative HPLC

(instrument: waters 2767 (preparative liquid phase chromatographic instrument); chromatographic column: XBridge@ Prep C18 (30 mm × 150 mm); composition of mobile phases: mobile phase A: acetonitrile, and mobile phase B: water (containing 0.1% TFA)) purification to obtain a preparative product, which was alkalized with saturated aqueous sodium bicarbonate solution to obtain **compound 17** (23 mg, yield: 23.3%).

LCMS m/z = 562.4 $[(M+2H)/2]^+$.

$^1H$ NMR (400 MHz, DMSO-$d_6$) δ 12.88 (s, 1H), 11.15 - 10.89 (m, 1H), 9.10 - 8.89 (m, 1H), 8.88 - 8.77 (m, 2H), 8.37 (s, 1H), 8.19 (s, 1H), 7.98 (s, 1H), 7.80 (s, 1H), 7.67 - 7.46 (m, 3H), 7.31 - 7.19 (m, 2H), 6.82 (s, 1H), 5.05 (dd, 1H), 3.80 (s, 3H), 3.76 (s, 3H), 3.57 - 3.35 (m, 6H), 3.17 - 3.06 (m, 2H), 2.99 - 2.80 (m, 3H), 2.71 - 2.52 (m, 6H), 2.37 - 1.93 (m, 14H), 1.87 - 1.42 (m, 11H), 1.31 - 1.02 (m, 4H).

**Example 18: Preparation of compound 18**

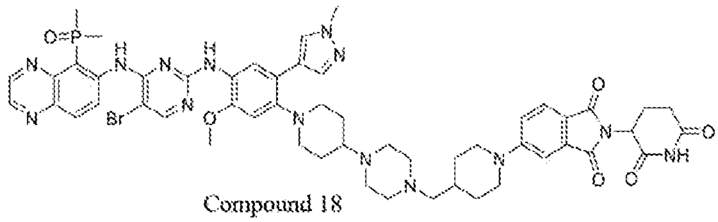

Compound 18

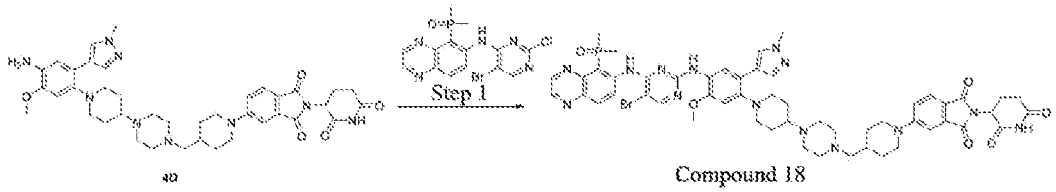


4D

Compound 18

5-(4-((4-(1-(4-((5-bromo-4-((5-(dimethylphosphoryl)quinoxalin-6-yl)amino)pyrimidin-2-yl)amino)-5-methoxy-2-(1-methyl-1H-pyrazol-4-yl)phenyl)piperidin-4-yl)piperazin-1-yl)methyl)piperidin-1-yl)-2-(2,6-dioxopiperidin-3-yl)isoindoline-1,3-dione **(compound 18)**

**4D** (0.22 g, 0.31 mmol) and **2J** (0.13 g, 0.31 mmol) were dissolved in DMF (15 mL); p-toluenesulfonic acid hydrate (0.18 g, 0.93 mmol) was added; and under nitrogen protection, the mixture was reacted at 100°C for 16 h. The reaction solution was cooled to room temperature. 10 mL of saturated aqueous sodium bicarbonate solution was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase: dichloromethane/methanol (V/V) = 50/1-12/1) to obtain a product, which was further purified by preparative HPLC (composition of mobile phases: mobile phase A: acetonitrile, and mobile phase B: water (containing 0.05% ammonia water)) to obtain **compound 18** as a yellow solid (150 mg, yield: 44%).

LCMS m/z = 550.3 $[(M+2H)/2]^+$.

$^1H$ NMR (400 MHz, DMSO-$d_6$) δ 12.68 (s, 1H), 11.05 (s, 1H), 8.89 - 8.76 (m, 3H), 8.35 (s, 1H), 8.27 (s, 1H), 7.96 (s, 1H), 7.77 (s, 1H), 7.65 (d, 1H), 7.62 - 7.50 (m, 2H), 7.31 (s, 1H), 7.23 (d, 1H), 6.82 (s, 1H), 5.06 (dd, 1H), 4.04 (d, 2H), 3.80 (s, 3H), 3.76 (s, 3H), 3.17 - 3.09 (m, 2H), 3.01 - 2.94 (m, 2H), 2.91 - 2.84 (m, 1H), 2.72 - 2.51 (m, 8H), 2.46 - 2.30 (m, 4H), 2.29 - 2.22 (m, 1H), 2.20 - 2.10 (m, 2H), 2.06 - 1.96 (m, 7H), 1.87 - 1.74 (m, 5H), 1.65-1.49 (m, 2H), 1.22 - 1.05 (m, 2H).

**Example 19: Preparation of compound 19**

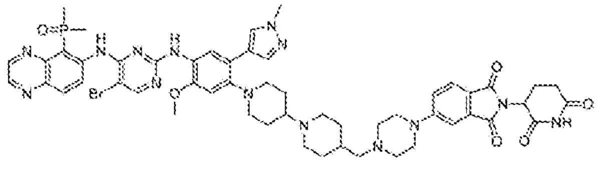

Compound 19

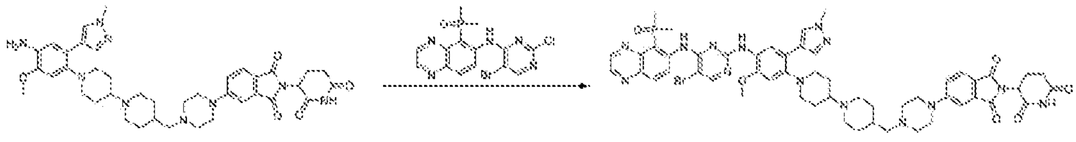

8I

Compound 19

5-(4-((1'-(4-((5-bromo-4-((5-(dimethylphosphoryl)quinoxalin-6-yl)amino)pyrimidin-2-yl)amino)-5-methoxy-2-(1-methyl-1H-pyrazol-4-yl)phenyl)-[1,4'-bipiperidin]-4-yl)methyl)piperazin-1-yl)-2-(2,6-dioxopiperidin-3-yl)isoindoline-1,3-dione **(compound 19)**; 2,2,2-trifluoroacetic acid

**8I** (0.27 g, 0.37 mmol) and **2J** (0.153 g, 0.37 mmol) were dissolved in DMF (5 mL); p-toluenesulfonic acid monohydrate (0.213 g, 1.12 mmol) was added; and under nitrogen protection, the mixture was reacted overnight at 100°C, and cooled to room temperature. 20 mL of saturated aqueous sodium bicarbonate solution was added, and the mixture was subjected to suction filtration. The filter cake was dissolved in 50 mL of dichloromethane and extracted. The organic phase was dried over anhydrous sodium sulphate and then concentrated under reduced pressure, and the residue was purified by silica gel column chromatography (dichloromethane/methanol (V/V) = 100/1-10/1) to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)) to obtain the trifluoroacetate of **compound 19** (50 mg, yellow solid).

LCMS m/z = 1099.4 $[M+H]^+$.

$^1H$ NMR (400 MHz, DMSO-$d_6$) δ 12.72 (s, 1H), 11.08 (s, 1H), 10.13 - 9.45 (m, 2H), 8.90 - 8.73 (m, 3H), 8.45 (s, 1H), 8.29 (s, 1H), 7.96 (s, 1H), 7.84 - 7.75 (m, 2H), 7.62 (s, 1H), 7.57 - 7.49 (m, 2H), 7.38 (dd, 1H), 6.81 (s, 1H), 5.10 (dd, 1H), 3.82 (s, 3H), 3.77 (s, 3H), 3.70 - 3.59 (m, 4H), 3.48 - 2.81 (m, 14H), 2.74 - 2.52 (m, 4H), 2.22 - 1.96 (m, 11H), 1.93 - 1.74 (m, 3H), 1.59 - 1.43 (m, 2H).

**Example 20: Preparation of compound 20**

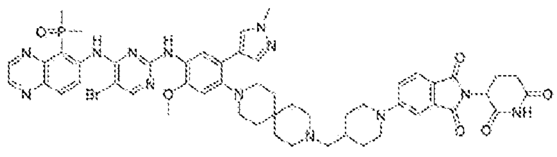

Compound 20

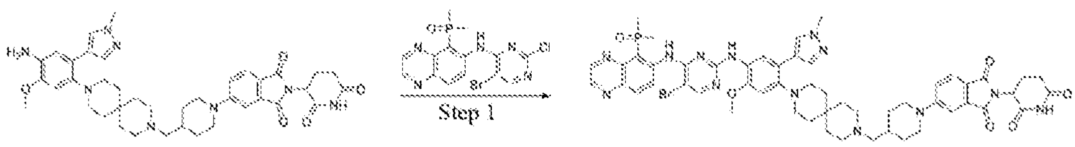


9F

Compound 20

5-(4-((9-(4-((5-bromo-4-((5-(dimethylphosphoryl)quinoxalin-6-yl)amino)pyrimidin-2-yl)amino)-5-methoxy-2-(1-methyl-1H-pyrazol-4-yl)phenyl)-3,9-diazaspiro[5.5]undecan-3-yl)methyl)piperidin-1-yl)-2-(2,6-dioxopiperidin-3-yl)isoindoline-1,3-dione (**compound** 20); 2,2,2-trifluoroacetic acid

**9F** (150 mg, 0.21 mmol) and **2J** (132 mg, 0.32 mmol) were dissolved in DMF (5 mL); p-toluenesulfonic acid monohydrate (120 mg, 0.63 mmol) was added; and under nitrogen protection, the mixture was stirred at 100°C for 16 h, and cooled to room temperature. 20 mL of saturated aqueous sodium bicarbonate solution and 50 mL of dichloromethane were added, and the layers were separated. The organic layer was concentrated under reduced pressure, and the residue was purified by silica gel column chromatography to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)) to obtain the trifluoroacetate of **compound 20** (44 mg).

LCMS m/z = 542.8 $[(M+2H)/2]^+$.

$^1$H NMR (400 MHz, DMSO-$d_6$) δ 12.75 (s, 1H), 11.06 (s, 1H), 8.91 - 8.72 (m, 3H), 8.56 - 8.47 (m, 1H), 8.29 (s, 1H), 7.99 (s, 1H), 7.80 (s, 1H), 7.68 (d, 1H), 7.62 - 7.49 (m, 2H), 7.37 (d, 1H), 7.28 (dd, 1H), 6.89 (s, 1H), 5.07 (dd, 1H), 4.17 - 4.04 (m, 2H), 3.82 (s, 3H), 3.76 (s, 3H), 3.48 - 3.35 (m, 2H), 3.16 - 2.76 (m, 10H), 2.68 - 2.51 (m, 4H), 2.21 - 2.09 (m, 1H), 2.07 - 1.91 (m, 7H), 1.90 - 1.50 (m, 8H), 1.37 - 1.21 (m, 3H).

**Example 21: Preparation of compound 21**

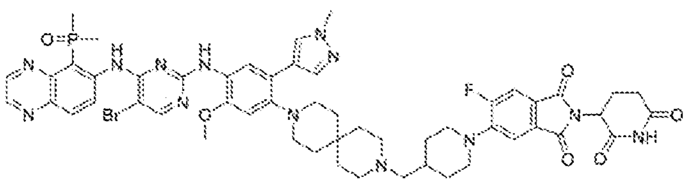

Compound 21

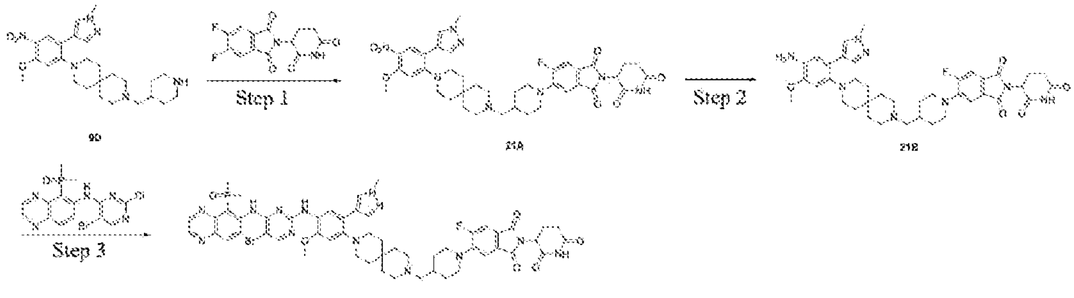


9D

21A

21B

Compound 21

Step 1: 2-(2,6-dioxopiperidin-3-yl)-5-fluoro-6-(4-((9-(5-methoxy-2-(1-methyl-1H-pyrazol-4-yl)-4-nitrophenyl)-3,9-diazaspiro[5.5]undecan-3-yl)methyl)piperidin-1-yl)isoindoline-1,3-dione **(21A)**

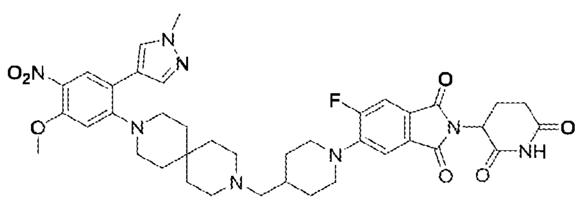

**21A**

The crude of **9D** (470 mg, 0.97 mmol) was dissolved in DMSO (10 mL); 2-(2,6-dioxopiperidin-3-yl)-5,6-difluoroisoindoline-1,3-dione **(5A-1)** (430 mg, 1.46 mmol) and sodium bicarbonate (326 mg, 3.88 mmol) were successively added; and the mixture was stirred at 100°C for 5 h, and cooled to room temperature. 10 mL of water was added, and the mixture was filtered. The filter cake was dried under reduced pressure and then purified by silica gel column chromatography (mobile phase: dichloromethane/methanol (V/V) = 100/1-20/1) to obtain **21A** as a yellow solid (600 mg, yield: 82%).

LCMS m/z = 757.3 $[M+H]^+$.

Step 2: 5-(4-((9-(4-amino-5-methoxy-2-(1-methyl-1H-pyrazol-4-yl)phenyl)-3,9-diazaspiro[5.5]undecan-3-yl)methyl)piperidin-1-yl)-2-(2,6-dioxopiperidin-3-yl)-6-fluoroisoindoline-1,3-dione **(21B)**

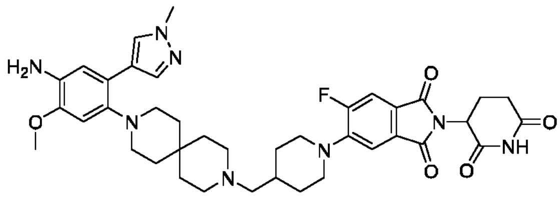

**21B**

**21A** (200 mg, 0.26 mmol) was dissolved in ethanol/water (8 mL, 3:1); iron powder (73 mg, 1.3 mmol) and ammonium chloride (70 mg, 1.3 mmol) were successively added; and under nitrogen protection, the mixture was stirred at 80°C for 1 h, cooled to room temperature and subjected to suction filtration under reduced pressure. The filtrate was concentrated under reduced pressure, and then the crude was purified by silica gel column chromatography (mobile phase: dichloromethane/methanol (V/V) = 100/1-20/1) to obtain **21B** as a yellow solid (180 mg, yield: 95%).

Step 3: 5-(4-((9-(4-((5-bromo-4-((5-(dimethylphosphoryl)quinoxalin-6-yl)amino) pyrimidin-2-yl)amino)-5-methoxy-2-(1-methyl-1H-pyrazol-4-yl)phenyl)-3,9-diazaspiro[5.5]undecan-3-yl)methyl)piperidin-1-yl)-2-(2,6-dioxopiperidin-3-yl)-6-fluoroisoindoline-1,3-dione **(compound 21)**

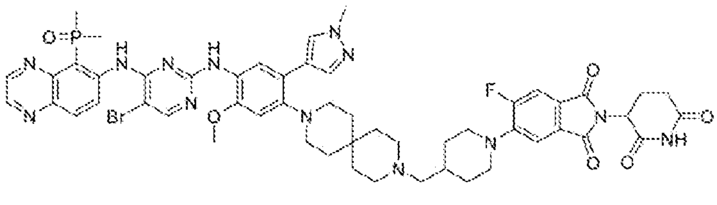

Compound 21

**21B** (180 mg, 0.25 mmol) and **2J** (157 mg, 0.38 mmol) were dissolved in DMF (5 mL); p-toluenesulfonic acid monohydrate (143 mg, 0.75 mmol) was added; and under nitrogen protection, the mixture was stirred at 100°C for 16 h, and cooled to room temperature. 20 mL of saturated aqueous sodium bicarbonate solution and 50 mL of dichloromethane were added, and the layers were separated. The organic layer was concentrated under reduced pressure, and the residue was purified by silica gel column chromatography (mobile phase: dichloromethane/methanol (V/V) = 100/1-20/1) to obtain **compound 21** (85 mg, yield: 31%).

LCMS m/z = 551.8 $[(M+2H)/2]^+$.

$^1H$ NMR (400 MHz, DMSO-$d_6$) δ 12.66 (s, 1H), 11.08 (s, 1H), 8.87 - 8.72 (m, 3H), 8.36 (s, 1H), 8.26 (s, 1H), 7.98 (s, 1H), 7.79 (s, 1H), 7.69 (d, 1H), 7.63 - 7.48 (m, 2H), 7.43 (d, 1H), 6.88 (s, 1H), 5.10 (dd, 1H), 3.81 (s, 3H), 3.76 (s, 3H), 3.67 - 3.55 (m, 2H), 2.96 - 2.76 (m, 7H), 2.68 - 2.52 (m, 2H), 2.43 - 2.29 (m, 4H), 2.24 - 2.14 (m, 2H), 2.06 - 1.94 (m, 8H), 1.88 - 1.69 (m, 3H), 1.62 - 1.50 (m, 7H), 1.32 - 1.25 (m, 2H).

**Example 22: Preparation of compound 22**

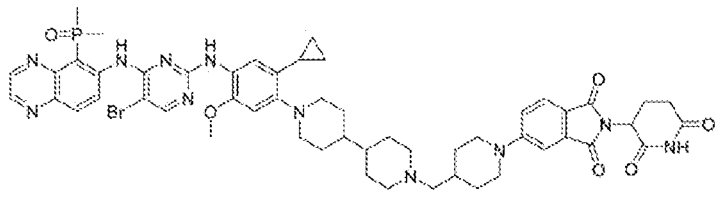

Compound 22

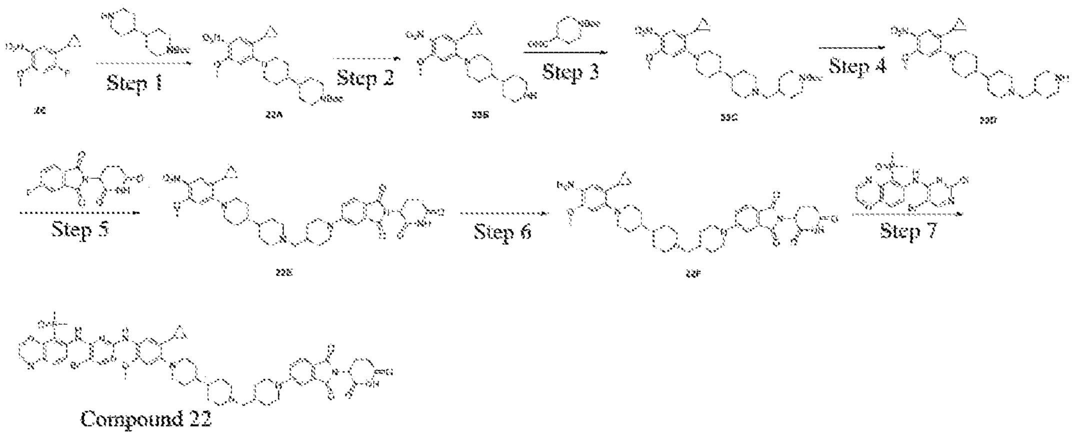


Compound 22

Step 1: tert-butyl 1'-(2-cyclopropyl-5-methoxy-4-nitrophenyl)-[4,4'-bipiperidine] -1-carboxylate **(21A)**

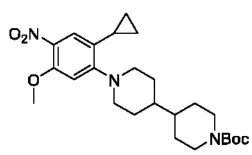

22A

The crude of **2E** (450 mg, 2.13 mmol) was dissolved in DMSO (10 mL); tert-butyl [4,4'-bipiperidine]-1-carboxylate (859 mg, 3.2 mmol) and sodium bicarbonate (716 mg, 8.52 mmol) were successively added; and the mixture was reacted at 100°C for 6 h, cooled to room temperature and extracted by adding 30 mL of ethyl acetate and 30 mL of water. The organic layer was washed with saturated brine (20 mL × 3), dried over anhydrous sodium sulphate and concentrated under reduced pressure, and the residue was purified by column chromatography (dichloromethane: methanol (V/V) = 100/1-20/1) to obtain **22A** (800 mg, yield: 82%).

LCMS m/z = 460.3 $[M+H]^+$.

Step 2: 1-(2-cyclopropyl-5-methoxy-4-nitrophenyl)-4,4'-bipiperidine **(22B)**

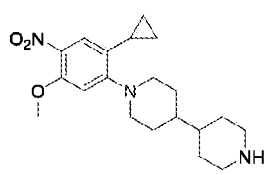

22B

**22A** (800 mg, 1.74 mmol) was dissolved in methanol (4 mL); a solution of hydrochloric acid in dioxane (2 M, 15 mL) was added; and the mixture was reacted at room temperature for 1 h and concentrated under reduced pressure. 20 mL of dichloromethane was added to the residue, and the mixture was adjusted to pH = 9-10 with 1 M aqueous NaOH

solution. Liquid separation was performed. The organic layer was concentrated under reduced pressure to obtain **22B**, which was directly used in the next step.

Step 3: tert-butyl 4-((1'-(2-cyclopropyl-5-methoxy-4-nitrophenyl)-[4,4'-bipiperidin]-1-yl)methyl)piperidine-1-carboxylate **(22C)**

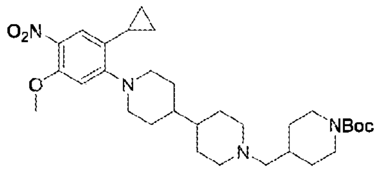

**22C**

**22B** (550 mg, 1.53 mmol) and tert-butyl 4-formylpiperidine-1-carboxylate (491 mg, 2.30 mmol) were dissolved in DMAc (15 mL), and acetic acid (92 mg, 1.53 mmol) was added. The mixture was stirred at room temperature for 30 min, and then sodium triacetoxyborohydride (485 mg, 2.29 mmol) was added; and the resulting mixture was reacted overnight at room temperature, and extracted by adding 20 mL of saturated aqueous sodium bicarbonate solution and 20 mL of dichloromethane. The organic layer was concentrated under reduced pressure, and the residue was purified by silica gel column chromatography (mobile phase: DCM/MeOH (V/V) = 100/1-20/1) to obtain **22C** as a yellow solid (650 mg, yield: 76%).

LCMS m/z = 557.5 $[M+H]^+$.

Step 4: 1-(2-cyclopropyl-5-methoxy-4-nitrophenyl)-1'-(piperidin-4-ylmethyl)-4,4'-bipiperidine **(22D)**; HCl

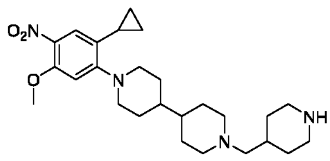

**22D**

**22C** (650 mg, 1.17 mmol) was dissolved in methanol (3 mL); a solution of hydrochloric acid in dioxane (2 M, 10 mL) was added; and the mixture was reacted at room temperature for 1 h and concentrated under reduced pressure, and the residue was directly used in the next step.

Step 5: 5-(4-((1'-(2-cyclopropyl-5-methoxy-4-nitrophenyl)-[4,4'-bipiperidin]-1-yl)methyl)piperidin-1-yl)-2-(2,6-dioxopiperidin-3-yl)isoindoline-1,3-dione **(22E)**

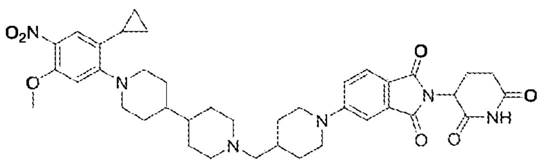

**22E**

The crude hydrochloride of compound **22D** from the previous step was dissolved in DMSO (10 mL); 2-(2,6-dioxopiperidin-3-yl)-5-fluoroisoindoline-1,3-dione (453 mg, 1.64 mmol) and sodium bicarbonate (366 mg, 4.36 mmol) were successively added; and the mixture was stirred at 100°C for 5 h, and cooled to room temperature. 10 mL of water was added, and the mixture was filtered. The filter cake was dried under reduced pressure and then purified by silica

gel column chromatography (mobile phase: dichloromethane/methanol (V/V) = 100/1-20/1) to obtain **22E** as a yellow solid (600 mg, yield: 72%).

LCMS m/z = 713.4 $[M+H]^+$.

Step 6: 5-(4-((1'-(4-amino-2-cyclopropyl-5-methoxyphenyl)-[4,4'-bipiperidin] -1-yl)methyl)piperidin-1-yl)-2-(2,6-dioxopiperidin-3-yl)isoindoline-1,3-dione **(22F)**

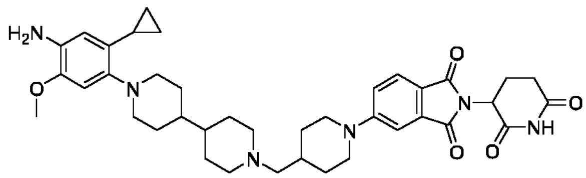

**22F**

**22E** (200 mg, 0.28 mmol) was dissolved in ethanol/water (8 mL, 3:1); iron powder (78 mg, 1.40 mmol) and ammonium chloride (75 mg, 1.40 mmol) were successively added; and under nitrogen protection, the mixture was stirred at 80°C for 1 h, cooled to room temperature and subjected to suction filtration under reduced pressure. The filtrate was concentrated under reduced pressure, and then residue was purified by silica gel column chromatography (mobile phase: dichloromethane/methanol (V/V) = 100/1-20/1) to obtain **22F** (150 mg, yield: 78%).

LCMS m/z = 342.3 $[(M+2H)/2]^+$.

Step 7: 5-(4-((1'-(4-((5-bromo-4-((5-(dimethylphosphoryl)quinoxalin-6-yl)amino) pyrimidin-2-yl)amino)-2-cyclopropyl-5-methoxyphenyl)-[4,4'-bipiperidin]-1-yl)methyl)piperidin-1-yl)-2-(2,6-dioxopiperidin-3-yl)isoindoline-1,3-dione **(compound 22)**; 2,2,2-trifluoroacetic acid

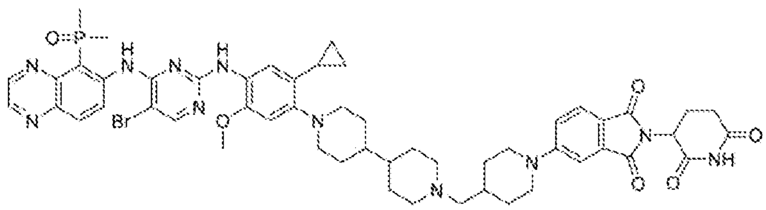

Compound 22

**22F** (150 mg, 0.22 mmol) and **2J** (136 mg, 0.33 mmol) were dissolved in DMF (8 mL); p-toluenesulfonic acid monohydrate (125 mg, 0.66 mmol) was added; and under nitrogen protection, the mixture was stirred at 100°C for 16 h, and cooled to room temperature. 20 mL of saturated aqueous sodium bicarbonate solution and 50 mL of dichloromethane were added, and the layers were separated. The organic layer was concentrated under reduced pressure, and the residue was purified by silica gel column chromatography to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)) to obtain the trifluoroacetate of **compound 22** (83 mg).

LCMS m/z = 529.8 $[(M+2H)/2]^+$.

$^1H$ NMR (400 MHz, DMSO-$d_6$) δ 12.72 (s, 1H), 11.06 (s, 1H), 8.94 - 8.69 (m, 4H), 8.50 - 8.23 (m, 2H), 7.94 - 7.81 (m, 1H), 7.68 (d, 1H), 7.36 (s, 1H), 7.31 - 7.24 (m, 1H), 6.94 - 6.73 (m, 2H), 5.07 (dd, 1H), 4.16 - 4.05 (m, 2H), 3.77 (s, 3H), 3.63 - 3.51 (m, 2H), 3.39 - 3.28 (m, 2H), 3.08 - 2.53 (m, 11H), 2.19 - 1.75 (m, 15H), 1.60 - 1.25 (m, 8H), 0.78 - 0.65 (m, 2H), 0.44 - 0.34 (m, 2H).

**Example 23: Preparation of trifluoroacetate of compound 23**

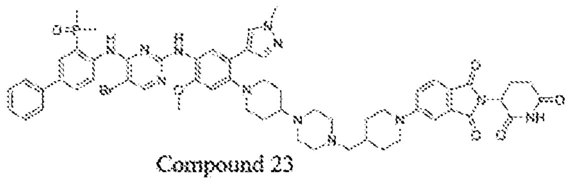

Compound 23

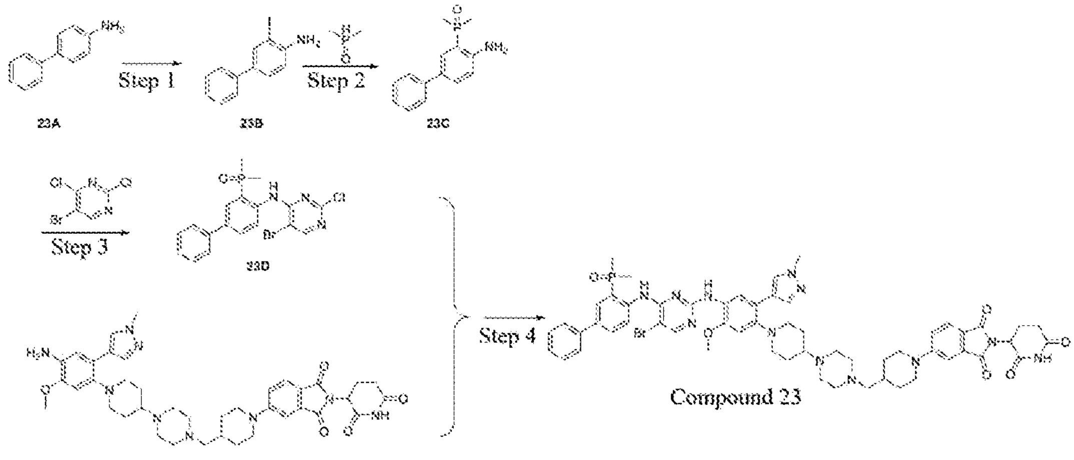


23A 23B 23C 23D 4D Compound 23

Step 1: 3-iodo-[1,1'-biphenyl]-4-amine **(23B)**

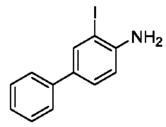

23B

[1,1'-biphenyl]-4-amine **(23A)** (846 mg, 5.0 mmol), iodine (1.27 g, 5.0 mmol) and sodium bicarbonate (1.26 g, 15.0 mmol) were added to a mixed solution of dichloromethane (20 mL) and water (10 mL), and the mixture was stirred overnight at room temperature. After the reaction was completed, the organic layers were separated, and the aqueous layer was extracted with dichloromethane (3 × 20 mL). The organic layers were combined, washed sequentially with saturated $Na_2S_2O_3$ solution (2 × 20 mL) and saturated brine (2 × 20 mL), dried over anhydrous sodium sulphate, filtered and concentrated, and the residue was purified by silica gel column chromatography (ethyl acetate/petroleum ether (V/V) = 1/20-1/5) to obtain **23B** (530 mg, yield: 36%).

LCMS m/z = 296.0 $[M+H]^+$.

Step 2: (4-amino-[1,1'-biphenyl]-3-yl)dimethylphosphine oxide **(23C)**

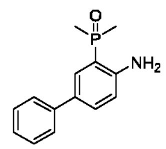

23C

Under nitrogen protection, **23B** (401 mg, 1.36 mmol), dimethylphosphine oxide (117 mg, 1.50 mmol), tripotassium phosphate (318 mg, 1.50 mmol), palladium acetate (30 mg, 0.13 mmol) and 4,5-bisdiphenylphosphine-9,9-dimethylxanthene (79 mg, 0.14 mmol) were added to anhydrous DMF (20 mL), and the mixture was reacted at 150°C for 3 h. After the reaction was completed, the mixture was cooled to room temperature. Water (20 mL) was added, and the

mixture was extracted with ethyl acetate (30 mL × 3). The organic phase was separated, washed sequentially with water (20 mL × 2) and saturated brine (20 mL), dried over anhydrous sodium sulphate and concentrated, and the residue was purified by silica gel column chromatography (ethyl acetate/petroleum ether (V/V) = 1/10-1/3) to obtain **23C** (170 mg, yield: 51%).

LCMS m/z = 246.2 $[M+H]^+$.

$^1H$ NMR (400 MHz, $CDCl_3$) δ 7.52 - 7.44 (m, 3H), 7.45 - 7.37 (m, 2H), 7.34-7.22 (m, 2H), 6.80 (dd, 1H), 1.82 (d, 6H).

Step 3: (4-((5-bromo-2-chloropyrimidin-4-yl)amino)-[1,1'-biphenyl]-3-yl) dimethylphosphine oxide **(23D)**

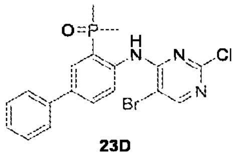

**23D**

**23C** (170 mg, 0.69 mmol) and 5-bromo-2,4-dichloropyrimidine (315 mg, 1.38 mmol) were dissolved in NMP (10 mL); DIPEA (107 mg, 0.83 mmol) was added; and under nitrogen protection, the mixture was stirred at 130°C for 6 h, cooled to room temperature and extracted by adding 60 mL of ethyl acetate and 60 mL of water. The organic layer was washed with 30 mL of saturated brine, dried over anhydrous sodium sulphate and then concentrated under reduced pressure, and the residue was purified by silica gel column chromatography (ethyl acetate/petroleum ether (V/V) = 1/2-1/0) to obtain **23D** (240 mg, yield: 80%).

LCMS m/z = 436.0 $[M+H]^+$.

$^1H$ NMR (400 MHz, $CDCl_3$) δ 11.37 (s, 1H), 8.66 (dd, 1H), 8.35 (s, 1H), 7.85 - 7.76 (m, 1H), 7.57 - 7.35 (m, 6H), 1.89 (d, 6H).

Step 4: 5-(4-((4-(1-(4-((5-bromo-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperidin-4-yl)piperazin-1-yl)methyl)piperidin-1-yl)-2-(2,6-dioxopiperidin-3-yl)isoindoline-1,3-dione **(compound 23)**; 2,2,2-trifluoroacetic acid

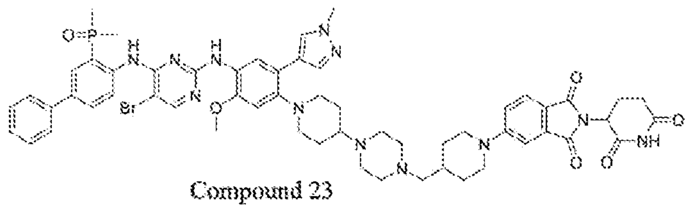

Compound 23

**4D** (87 mg, 0.12 mmol) and **23D** (58 mg, 0.13 mmol) were dissolved in DMF (10 mL); p-toluenesulfonic acid monohydrate (68 mg, 0.36 mmol) was added; and under nitrogen protection, the mixture was stirred at 100°C for 16 h, and cooled to room temperature. 20 mL of saturated aqueous sodium bicarbonate solution and 50 mL of dichloromethane were added, and the layers were separated. The organic layer was concentrated under reduced pressure, and the residue was purified by silica gel column chromatography (mobile phase: DCM/MeOH (V/V) = 100/1-20/1). The crude was further subjected to preparative HPLC (instrument: waters 2767 (preparative liquid phase chromatographic instrument); chromatographic column: XBridge@ Prep C18 (30 mm × 150 mm); composition of mobile phases: mobile phase A: acetonitrile, and mobile phase B: water (containing 0.1% TFA)) purification to obtain the trifluoroacetate of **compound 23** (30 mg).

LCMS m/z = 562.3 $[(M+2H)/2]^+$.

$^1H$ NMR (400 MHz, DMSO-$d_6$) δ 11.11 (s, 1H), 11.05 (s, 1H), 8.43 - 8.34 (m, 1H), 8.27 (s, 1H), 8.19 (s, 1H), 8.01 (s, 1H), 7.85 (s, 1H), 7.73 - 7.61 (m, 2H), 7.61 - 7.50 (m, 3H), 7.45 (t, 2H), 7.40 - 7.33 (m, 1H), 7.33 - 7.29 (m, 1H), 7.27 - 7.13 (m, 2H), 6.83 (s, 1H), 5.06 (dd, 1H), 4.10 - 3.98 (m, 2H), 3.79 (s, 3H), 3.76 (s, 3H), 3.14 - 3.04 (m, 2H), 3.04 - 2.81 (m, 4H), 2.65 - 2.52 (m, 6H), 2.44 - 2.30 (m, 4H), 2.25 - 1.95 (m, 5H), 1.92 - 1.73 (m, 11H), 1.61 - 1.45 (m, 2H), 1.21 - 1.08 (m, 2H).

**Example 23-1: Preparation of compound 23**

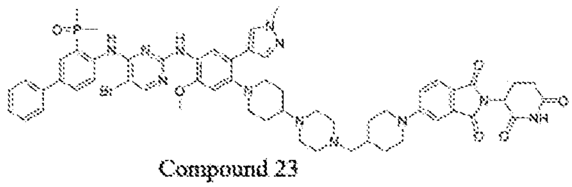

Compound 23

The trifluoroacetate of **compound 23** (60 mg) was dissolved in 20 mL of dichloromethane; 20 mL of water and 1 mL of saturated sodium bicarbonate solution were added. The organic layers were separated, and the aqueous layer was extracted with dichloromethane (20 mL × 2). The organic phases were combined, dried over anhydrous sodium sulphate and filtered. The filtrate was concentrated under reduced pressure to obtain **compound 23** (42 mg).

LCMS m/z = 562.3 $[(M+2H)/2]^+$.

$^1$H NMR (400 MHz, DMSO-$d_6$) δ 11.10 (s, 1H), 11.05 (s, 1H), 8.31 - 8.22 (m, 1H), 8.27 (s, 1H), 8.18 (s, 1H), 8.00 (s, 1H), 7.85 (s, 1H), 7.75 - 7.61 (m, 2H), 7.61 - 7.50 (m, 3H), 7.45 (t, 2H), 7.40 - 7.32 (m, 1H), 7.30 (s, 1H), 7.27 - 7.08 (m, 2H), 6.83 (s, 1H), 5.06 (dd, 1H), 4.10 - 3.93 (m, 2H), 3.79 (s, 3H), 3.76 (s, 3H), 3.14 - 3.03 (m, 2H), 3.03 - 2.81 (m, 4H), 2.65 - 2.52 (m, 6H), 2.44 - 2.30 (m, 4H), 2.23 - 1.95 (m, 5H), 1.89 - 1.72 (m, 11H), 1.59 - 1.45 (m, 2H), 1.19 - 1.07 (m, 2H).

**Example 24: Preparation of compound 24**

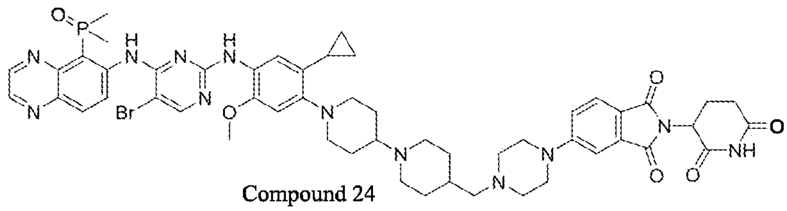

Compound 24

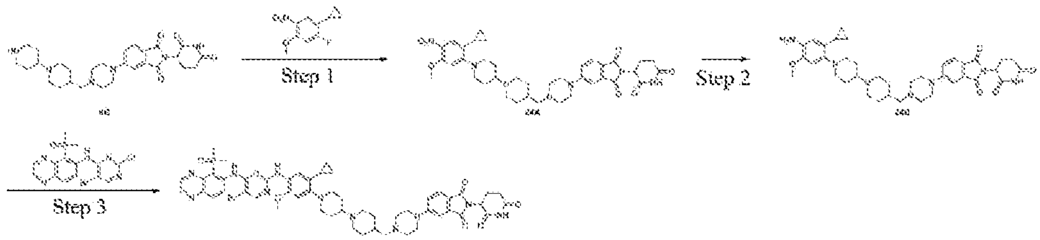


Compound 24

Step 1: 5-(4-((1'-(2-cyclopropyl-5-methoxy-4-nitrophenyl)-[1,4'-bipiperidin]-4-yl)methyl)piperazin-1-yl)-2-(2,6-dioxopiperidin-3-yl-yl)isoindoline-1,3-dione **(24A)**

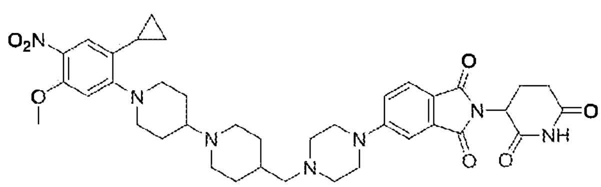

**24A**

**8G** (0.84 g, 1.61 mmol) and 1-cyclopropyl-2-fluoro-4-methoxy-5-nitrobenzene **(2E)** (226 mg, 1.07 mmol) were

dissolved in DMSO (10 mL); a sodium bicarbonate solid (405 mg, 4.82 mmol) was added; and the mixture was reacted overnight at 100°C. After the reaction was completed, the mixture was cooled to room temperature and extracted by adding 30 mL of ethyl acetate and 30 mL of water. The organic layer was dried over anhydrous sodium sulphate and concentrated under reduced pressure, and the residue was purified by column chromatography (dichloromethane: methanol (V/V) = 100/1-10/1) to obtain **24A** as a yellow solid (0.61 g, yield: 53%).

LCMS m/z = 714.4 $[M+H]^+$.

Step 2: 5-(4-((1'-(4-amino-2-cyclopropyl-5-methoxyphenyl)-[1,4'-bipiperidin] -4-yl)methyl)piperazin-1-yl)-2-(2,6-dioxopiperidin-3-yl)isoindoline-1,3-dione **(24B)**

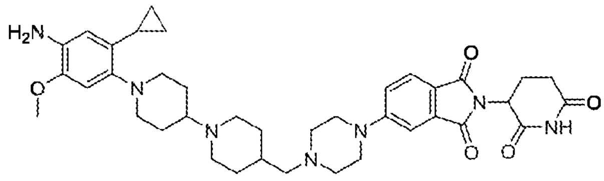

**24B**

**24A** (0.61 g, 0.85 mmol) was dissolved in ethanol (5 mL) and water (1 mL); an ammonium chloride solid (455 mg, 8.5 mmol) and iron powder (476 mg, 8.5 mmol) were added; and the mixture was subjected to $N_2$ replacement 3 times and stirred at 80°C for 1 h. After the reaction was completed, the mixture was cooled to room temperature and filtered. The filter cake was washed with 50 mL of dichloromethane and concentrated under reduced pressure. The resultant obtained from the concentration under reduced pressure was dissolved in 50 mL of dichloromethane and extracted. The organic layer was dried over anhydrous sodium sulphate and concentrated under reduced pressure, and the residue was purified by silica gel column chromatography (mobile phase: dichloromethane/methanol (V/V) = 100/1-10/1) to obtain **24B** as a yellow solid (0.32 g, yield: 55%).

LCMS m/z = 684.4 $[M+H]^+$.

Step 3: 5-(4-((1'-(4-((5-bromo-4-((5-(dimethylphosphoryl)quinoxalin-6-yl)amino) pyrimidin-2-yl)amino)-2-cyclopropyl-5-methoxyphenyl)-[1,4'-bipiperidin]-4-yl)methyl)piperazin-1-yl)-2-(2,6-dioxopiperidin-3-yl)isoindoline-1,3-dione **(compound 24)**

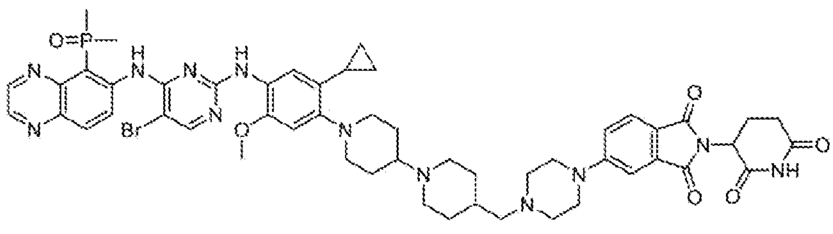

Compound 24

**24B** (0.32 g, 0.468 mmol) and **2J** (193 mg, 0.468 mmol) were dissolved in DMF (5 mL); p-toluenesulfonic acid monohydrate (266 mg, 1.40 mmol) was added; and under nitrogen protection, the mixture was reacted overnight at 100°C and cooled to room temperature. 20 mL of saturated aqueous sodium bicarbonate solution was added, and the mixture was subjected to suction filtration. The filter cake was dissolved in 50 mL of dichloromethane and extracted. The organic phase was dried over anhydrous sodium sulphate and then concentrated under reduced pressure, and the residue was purified by silica gel column chromatography (dichloromethane/methanol (V/V) = 100/1-10/1) to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.05% ammonia water)) to obtain **compound 24** (60 mg, yield: 12%).

LCMS m/z = 1059.4 $[M+H]^+$.

$^1H$ NMR (400 MHz, DMSO-$d_6$) δ 12.64 (s, 1H), 11.06 (s, 1H), 8.90 - 8.74 (m, 3H), 8.31 - 8.22 (m, 2H), 7.87 (d, 1H), 7.67 (d, 1H), 7.32 (s, 1H), 7.27 - 7.21 (m, 1H), 6.86 (s, 1H), 6.74 (s, 1H), 5.07 (dd, 1H), 3.76 (s, 3H), 3.44 - 3.41

(m, 3H), 3.34 - 3.31 (m, 3H), 2.94 - 2.83 (m, 3H), 2.73 - 2.64 (m, 2H), 2.63 - 2.52 (m, 2H), 2.49 - 2.43 (m, 4H), 2.24 - 2.07 (m, 5H), 2.06 - 1.97 (m, 7H), 1.89 (s, 1H), 1.87 - 1.79 (m, 2H), 1.76 - 1.60 (m, 4H), 1.56 - 1.46 (m, 1H), 1.16 - 1.04 (m, 2H), 0.79 - 0.66 (m, 2H), 0.44 - 0.32 (m, 2H).

**Example 25: Preparation of compound 25**

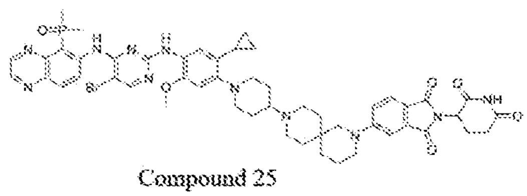

Compound 25

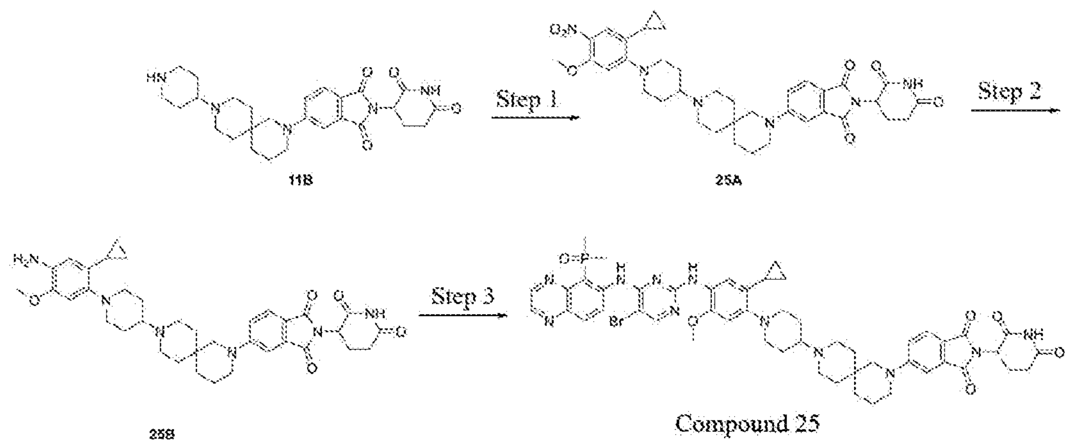


11B

25A

25B

Compound 25

Step 1: 5-(9-(1-(2-cyclopropyl-5-methoxy-4-nitrophenyl)piperidin-4-yl)-2,9-diazaspiro[5.5]undecan-2-yl)-2-(2,6-dioxopiperidin-3-yl)isoindoline-1,3-dione **(25A)**

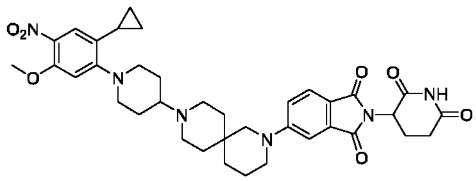

25A

The hydrochloride of **11B** (728 mg) was dissolved in DMSO (10 mL); 1-cyclopropyl-2-fluoro-4-methoxy-5-nitrobenzene **(2E)** (350 mg, 1.66 mmol) and sodium bicarbonate (353 mg, 4.2 mmol) were successively added; and the mixture was reacted at 100°C for 5 h, and cooled to room temperature. 10 mL of water was added, and the mixture was filtered. The filter cake was dried under reduced pressure and then purified by silica gel column chromatography (mobile phase: dichloromethane/methanol (V/V) = 100/1-20/1) to obtain **25A** (540 mg, yield: 60%).

LCMS m/z = 685.3 $[M+H]^+$.

Step 2: 5-(9-(1-(4-amino-2-cyclopropyl-5-methoxyphenyl)piperidin-4-yl)-2,9-diazaspiro[5.5]undecan-2-yl)-2-(2,6-dioxopiperidin-3-yl)isoindoline-1,3-dione **(25B)**

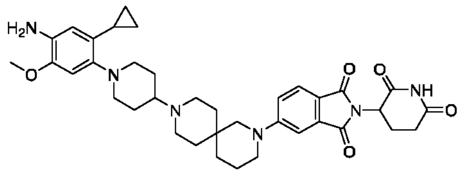

25B

**25A** (540 mg, 0.79 mmol) was dissolved in ethanol/water (16 mL, 3:1); iron powder (530 mg, 9.5 mmol) and ammonium chloride (510 mg, 9.5 mmol) were successively added; and under nitrogen protection, the mixture was reacted at 80°C for 1 h, cooled to room temperature and subjected to suction filtration under reduced pressure. The filtrate was concentrated under reduced pressure, and then the crude was purified by silica gel column chromatography (mobile phase: dichloromethane/methanol (V/V) = 100/1-20/1) to obtain **25B** (250 mg, yield: 48%).

LCMS m/z = 655.4 $[M+H]^+$.

Step 3: 5-(9-(1-(4-((5-bromo-4-((5-(dimethylphosphoryl)quinoxalin-6-yl)amino) pyrimidin-2-yl)amino)-2-cyclopropyl-5-methoxyphenyl)piperidin-4-yl)-2,9-diazaspiro[5.5]undecan-2-yl)-2-(2,6-dioxopiperidin-3-yl)isoindoline-1,3-dione **(compound 25)**

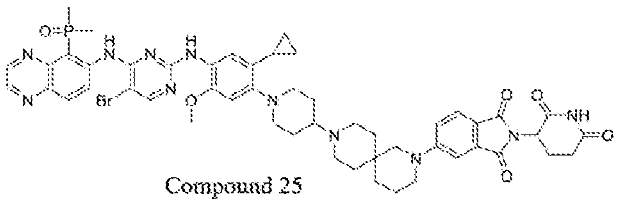

Compound 25

**25B** (125 mg, 0.19 mmol) and **2J** (87 mg, 0.21 mmol) were dissolved in DMF (5 mL); p-toluenesulfonic acid monohydrate (110 mg, 0.57 mmol) was added; and under nitrogen protection, the mixture was reacted at 100°C for 16 h, and cooled to room temperature. 20 mL of saturated aqueous sodium bicarbonate solution and 50 mL of dichloromethane were added, and the layers were separated. The organic layer was concentrated under reduced pressure, and the residue was purified by silica gel column chromatography (mobile phase: dichloromethane/methanol (V/V) = 100/1-20/1) to obtain **compound 25** (40 mg, yield: 25.4%).

LCMS m/z = 515.8 $[(M+2H)/2]^+$.

$^1H$ NMR (400 MHz, DMSO-$d_6$) $\delta$ 12.65 (s, 1H), 11.05 (s, 1H), 8.98 - 8.70 (m, 3H), 8.33 - 8.20 (m, 2H), 7.86 (d, 1H), 7.63 (d, 1H), 7.36 - 7.20 (m, 2H), 6.86 (s, 1H), 6.74 (s, 1H), 5.05 (dd, 1H), 3.76 (s, 3H), 3.50 - 3.43 (m, 2H), 3.39 - 3.32 (m, 3H), 2.95 - 2.82 (m, 1H), 2.76 - 2.53 (m, 7H), 2.14 - 1.82 (m, 11H), 1.76 - 1.37 (m, 12H), 0.74 - 0.64 (m, 2H), 0.44 - 0.27 (m, 2H).

**Example 26: Preparation of compound 26**

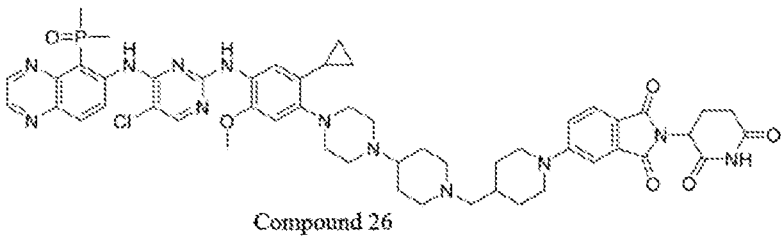

Compound 26

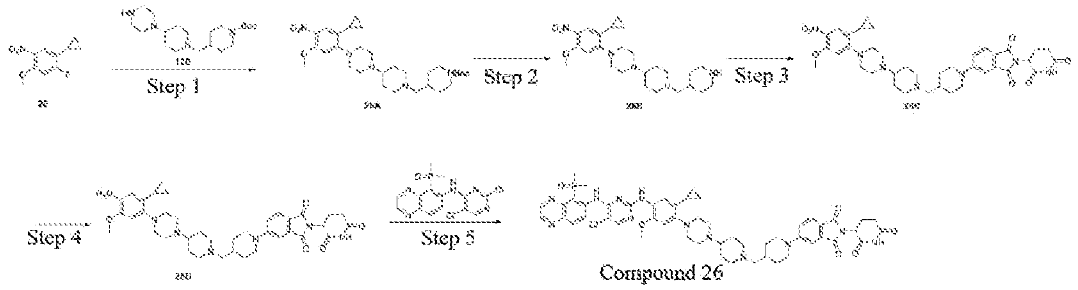


Step 1: tert-butyl 4-((4-(4-(2-cyclopropyl-5-methoxy-4-nitrophenyl)piperazin-1-yl)piperidin-1-yl)methyl)piperidine-1-carboxylate **(26A)**

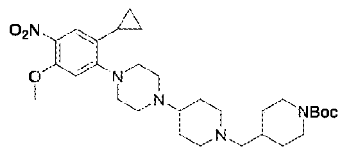

**26A**

**12E** (0.8 g, 2.18 mmol) and **2E** (0.50 g, 2.4 mmol) were dissolved in DMSO (20 mL); potassium carbonate (0.59 mg, 4.26 mmol) was added; and the mixture was reacted at 120°C for 6 h, cooled to room temperature and extracted by adding 30 mL of ethyl acetate and 30 mL of water. The organic layer was washed three times with saturated brine (20 mL × 3). dried over anhydrous sodium sulphate and concentrated under reduced pressure, and the residue was purified by column chromatography (dichloromethane: methanol (V/V) = 100/1-20/1) to obtain **26A** (0.6 g, yield: 49%).

LCMS m/z = 558.4[M+H]$^+$.

Step 2: 1-(2-cyclopropyl-5-methoxy-4-nitrophenyl)-4-(1-(piperidin-4-ylmethyl) piperidin-4-yl)piperazine **(26B)**

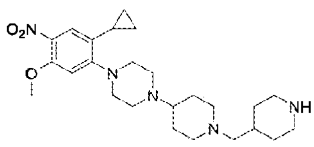

**26B**

**26A** (0.6 g, 1.07 mmol) was dissolved in dichloromethane (6 mL); trifluoroacetic acid (3.06 g, 26.84 mmol) was added at room temperature; and the mixture was stirred at room temperature for 2 h, and concentrated under reduced pressure. 20 mL of dichloromethane was added to the residue, and the mixture was adjusted to pH = 10 with 1 N aqueous sodium hydroxide solution. Liquid separation was performed. The organic layer was then dried over anhydrous sodium sulphate and concentrated under reduced pressure to obtain the crude of **26B** (0.5 g), which was directly used in the next step.

Step 3: 5-(4-((4-(4-(2-cyclopropyl-5-methoxy-4-nitrophenyl)piperazin-1-yl)piperidin-1-yl)methyl)piperidin-1-yl)-2-(2,6-dioxopiperidin-3-yl)isoindoline-1,3-dione **(26C)**

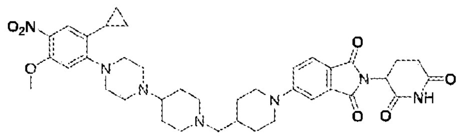

26C

**26B** (0.5 g) and 2-(2,6-dioxopiperidin-3-yl)-5-fluoroisoindoline-1,3-dione (0.30 g, 1.08 mmol) were dissolved in DMSO (10 mL); DIPEA (0.21 g, 1.62 mmol) was added; and the mixture was stirred at 90°C for 5 h and cooled to room temperature. 20 mL of water was added, and the mixture was filtered. The filter cake was dried under reduced pressure and then purified by silica gel column chromatography (dichloromethane: methanol (V/V) = 100/1-15/1) to obtain **26C** (0.40 g, yield: 52%)

LCMS m/z = 714.3 $[M+H]^+$.

Step 4: 5-(4-((4-(4-(4-amino-2-cyclopropyl-5-methoxyphenyl)piperazin-1-yl) piperidin-1-yl)methyl)piperidin-1-yl)-2-(2,6-dioxopiperidin-3-yl)isoindoline-1,3-dione **(26D)**

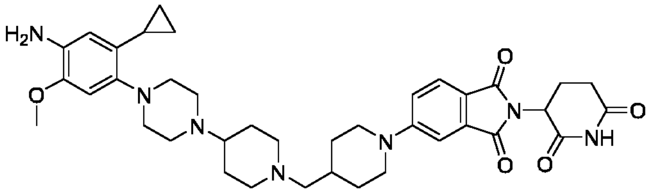

**26D**

**26C** (0.40 g, 0.58 mmol) was dissolved in ethanol (12 mL) and water (3 mL), and the mixture was warmed to 80°C. A mixture of ammonium chloride (0.22 g, 4.11 mmol) and iron powder (0.23 g, 4.12 mmol) was added, and the resulting mixture was stirred at 80°C for 0.5 h, cooled to room temperature and filtered. The filter cake was washed with 50 mL of dichloromethane. 10 mL of saturated brine was added to the filtrate. Liquid separation was performed. The organic layer was dried over anhydrous sodium sulphate and concentrated under reduced pressure to obtain **26D** (0.31 g, yield: 78%).

LCMS m/z = 684.3 $[M+H]^+$.

Step 5: 5-(4-((4-(4-(4-((5-chloro-4-((5-(dimethylphosphoryl)quinoxalin-6-yl)amino)pyrimidin-2-yl)amino)-2-cyclopropyl-5-methoxyphenyl)piperazin-1-yl)piperidin-1-yl)methyl)piperidin-1-yl)-2-(2,6-dioxopiperidin-3-yl)isoindoline-1,3-dione **(compound 26)**

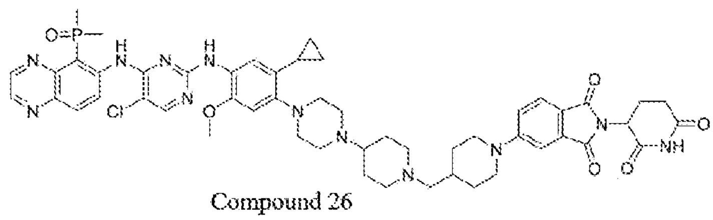

Compound 26

**26D** (0.31 g, 0.45 mmol) and **1l** (0.18 g, 0.5 mmol) were dissolved in DMF (5 mL); p-toluenesulfonic acid monohydrate (0.16 g, 0.84 mmol) was added; and under nitrogen protection, the mixture was stirred at 100°C for 16 h, and cooled to room temperature. 5 mL of water and 5 mL of saturated aqueous sodium bicarbonate solution were added, and the mixture was filtered. The filter cake was dried under reduced pressure and then purified by silica gel column chromatography (dichloromethane/methanol (V/V) = 100/1-12/1) to obtain a product, which was further purified by preparative HPLC (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane and extracted by adding

saturated sodium bicarbonate solution (50 ml). Liquid separation was performed. The organic layer was then dried over anhydrous sodium sulphate and concentrated under reduced pressure to obtain **compound 26** (30 mg, yield: 7%).

LCMS m/z = 508.3[(M+2H)/2]$^+$.

$^1$H NMR (400 MHz, DMSO-$d_6$) $\delta$ 12.88 (s, 1H), 11.06 (s, 1H), 9.06 - 8.91 (m, 1H), 8.90 - 8.80 (m, 2H), 8.33 (s, 1H), 8.17 (s, 1H), 7.89 (d, 1H), 7.65 (d, 1H), 7.31 (d, 1H), 7.23 (dd, 1H), 6.88 (s, 1H), 6.80 (s, 1H), 5.07 (dd, 1H), 4.10 - 3.98 (m, 2H), 3.77 (s, 3H), 3.09 - 2.83 (m, 9H), 2.76 - 2.53 (m, 6H), 2.30 - 2.09 (m, 4H), 2.08 - 1.96 (m, 7H), 1.94 - 1.71 (m, 7H), 1.55 - 1.39 (m, 2H), 1.22 - 1.08 (m, 2H), 0.82 - 0.71 (m, 2H), 0.49 - 0.40 (m, 2H).

**Example 27: Preparation of compound 27**

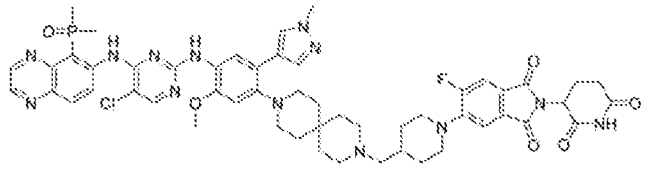

Compound 27

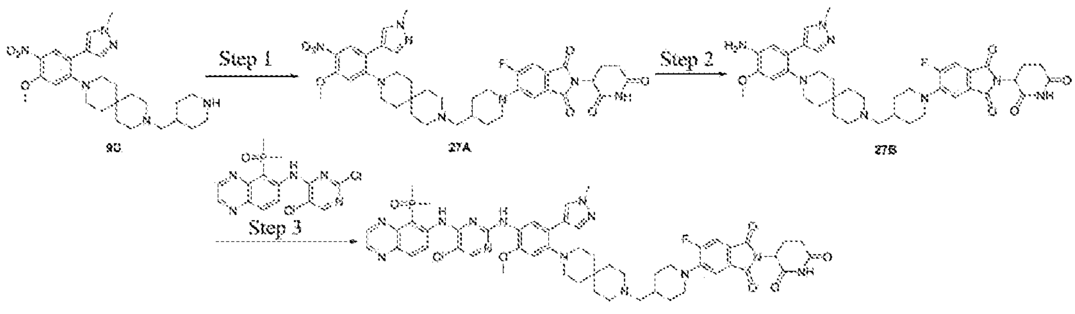


Compound 27

Step 1: 2-(2,6-dioxopiperidin-3-yl)-5-fluoro-6-(4-((9-(5-methoxy-2-(1-methyl-1H-pyrazol-4-yl)-4-nitrophenyl)-3,9-diazaspiro[5.5]undecan-3-yl)methyl)piperidin-1-yl)isoindoline-1,3-dione **(27A)**

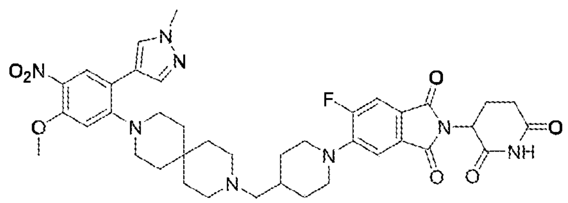

**27A**

The hydrochloride of **9D** (0.27 g) and 2-(2,6-dioxopiperidin-3-yl)-5,6-difluoroisodihydroindole-1,3-dione **(5A-1)** (0.16 g, 0.56 mmol) were dissolved in DMSO (15 mL); DIPEA (0.22 g, 1.68 mmol) was added dropwise; and the mixture was reacted at 90°C for 3 h. The reaction solution was cooled to room temperature. 20 mL of water was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase: dichloromethane/methanol (V/V) = 50/1-12/1) to obtain **27A** as a yellow solid (0.30 g, yield: 71%).

LCMS m/z = 757.4 [M+H]$^+$.

Step 2: 5-(4-((9-(4-amino-5-methoxy-2-(1-methyl-1H-pyrazol-4-yl)phenyl)-3,9-diazaspiro[5.5]undecan-3-yl)methyl)piperidin-1-yl)-2-(2,6-dioxopiperidin-3-yl)-6-fluoroisoindoline-1,3-dione **(27B)**

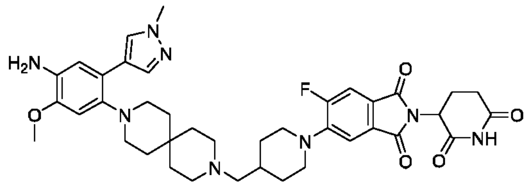

27B

**27A** (0.30 g, 0.40 mmol) was dissolved in ethanol (15 mL); reduced iron powder (0.11 g, 2.00 mmol) was added, and then an aqueous solution (5 mL) of ammonium chloride (0.11 g, 2.00 mmol) was added dropwise; and under nitrogen protection, the mixture was reacted at 80°C for 3 h. The reaction solution was cooled to room temperature. 5 mL of water was added, and then the mixture was extracted 3 times with dichloromethane. The organic phases were combined, dried over anhydrous sodium sulphate and then concentrated under reduced pressure to obtain **27B** as a yellow solid (0.26 g, yield: 89%).

LCMS m/z = 727.3 $[M+H]^+$.

Step 3: 5-(4-((9-(4-((5-chloro-4-((5-(dimethylphosphoryl)quinoxalin-6-yl)amino) pyrimidin-2-yl)amino)-5-methoxy-2-(1-methyl-1H-pyrazol-4-yl)phenyl)-3,9-diazaspiro[5.5]undecan-3-yl)methyl)piperidin-1-yl)-2-(2,6-dioxopiperidin-3-yl)-6-fluoroisoindoline-1,3-dione **(compound 27)**

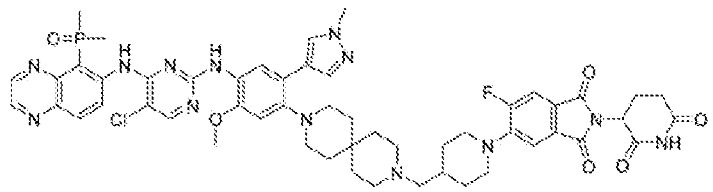

Compound 27

**27B** (0.13 g, 0.18 mmol) and **1l** (0.07 g, 0.18 mmol) were dissolved in DMF (10 mL); p-toluenesulfonic acid hydrate (0.10 g, 0.54 mmol) was added; and under nitrogen protection, the mixture was reacted at 100°C for 16 h. The reaction solution was cooled to room temperature. 10 mL of saturated aqueous sodium bicarbonate solution was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase: dichloromethane/methanol (V/V) = 50/1-12/1) to obtain **compound 27** (44 mg, yield: 23%).

LCMS m/z = 529.8 $[(M+2H)/2]^+$.

$^1$H NMR (400 MHz, DMSO-$d_6$) $\delta$ 12.87 (s, 1H), 11.08 (s, 1H), 9.00 - 8.92 (m, 1H), 8.87 - 8.78 (m, 2H), 8.38 (s, 1H), 8.19 (s, 1H), 8.01 (s, 1H), 7.82 (s, 1H), 7.69 (d, 1H), 7.57 (s, 2H), 7.44 (d, 1H), 6.89 (s, 1H), 5.10 (dd, 1H), 3.81 (s, 3H), 3.77 (s, 3H), 3.66 - 3.55 (m, 2H), 2.95 - 2.80 (m, 7H), 2.68 - 2.53 (m, 2H), 2.41 - 2.31 (m, 4H), 2.22 - 2.15 (m, 2H), 2.08 - 1.97 (m, 7H), 1.88-1.78 (m, 2H), 1.78 - 1.69 (m, 1H), 1.62 - 1.50 (m, 8H), 1.33 - 1.24 (m, 2H).

**Example 28: Preparation of compound 28**

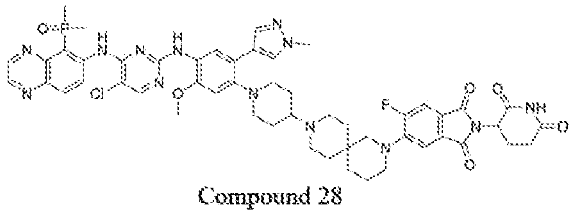

Compound 28

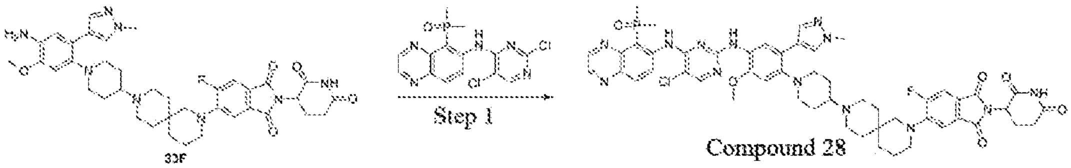


33F

Compound 28

Step 1: 5-(9-(1-(4-((5-chloro-4-((5-(dimethylphosphoryl)quinoxalin-6-yl) amino)pyrimidin-2-yl)amino)-5-methoxy-2-(1-methyl-1H-pyrazol-4-yl)phenyl)piperidin-4-yl)-2,9-diazaspiro[5.5]undecan-2-yl)-2-(2,6-dioxopiperidin-3-yl)-6-fluoroisoindoline-1,3-dione **(compound 28)**

**33F** (100 mg, 0.14 mmol) and **1l** (77 mg, 0.21 mmol) were dissolved in DMF (8 mL); p-toluenesulfonic acid monohydrate (80 mg, 0.42 mmol) was added; and under nitrogen protection, the mixture was reacted at 100°C for 16 h, and cooled to room temperature. 20 mL of saturated aqueous sodium bicarbonate solution and 50 mL of dichloromethane were added, and the layers were separated. The organic layer was concentrated under reduced pressure, and the crude was purified by silica gel column chromatography (dichloromethane/methanol (V/V) = 100/1-10/1) to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.05% ammonia water)) to obtain **compound 28** (20 mg, yield: 14%).

LCMS m/z = 1044.4 $[M+H]^+$.

$^1H$ NMR (400 MHz, DMSO-$d_6$) δ 12.89 (s, 1H), 11.08 (s, 1H), 9.04 - 8.91 (m, 1H), 8.83 (dd, 2H), 8.37 (s, 1H), 8.19 (s, 1H), 7.99 (s, 1H), 7.80 (s, 1H), 7.71 (d, 1H), 7.63 - 7.51 (m, 2H), 7.47 (d, 1H), 6.83 (s, 1H), 5.10 (dd, 1H), 3.80 (s, 3H), 3.77 (s, 3H), 3.23 - 3.10 (m, 4H), 3.06 (s, 2H), 2.95 - 2.82 (m, 1H), 2.70 - 2.52 (m, 6H), 2.41 - 2.28 (m, 1H), 2.10 - 1.94 (m, 7H), 1.90 - 1.41 (m, 12H), 1.34 - 1.20 (m, 2H).

**Example 29: Preparation of compound 29**

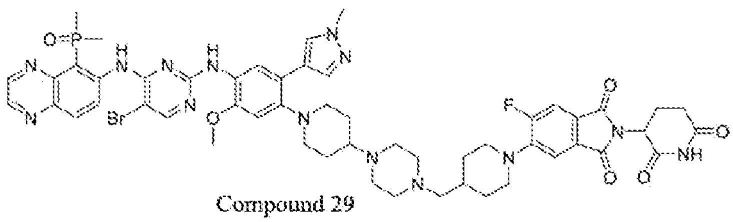

Compound 29

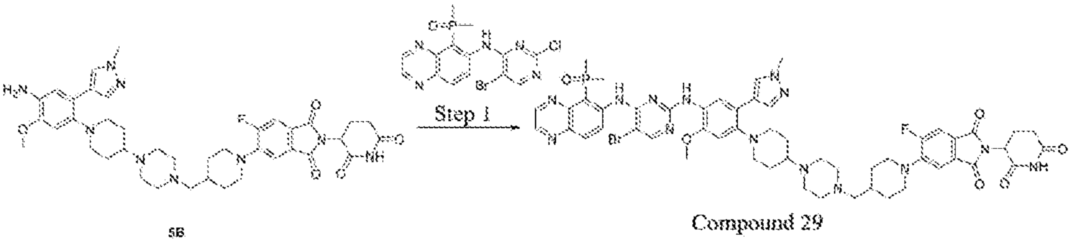


5B

Compound 29

Step 1: 5-(4-((4-(1-(4-((5-bromo-4-((5-(dimethylphosphoryl)quinoxalin-6-yl) amino)pyrimidin-2-yl)amino)-5-methoxy-2-(1-methyl-1H-pyrazol-4-yl)phenyl)piperidin-4-yl)piperazin-1-yl)methyl)piperidin-1-yl)-2-(2,6-dioxopiperidin-3-yl)-6-fluoroisoindoline-1,3-dione **(compound 29)**

**5B** (0.16 g, 0.21 mmol) and **2J** (0.09 g, 0.21 mmol) were dissolved in DMF (10 mL); p-toluenesulfonic acid hydrate (0.12 g, 0.63 mmol) was added; and under nitrogen protection, the mixture was reacted at 100°C for 16 h. The reaction solution was cooled to room temperature. 10 mL of saturated aqueous sodium bicarbonate solution was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase: dichloromethane/methanol (V/V) = 50/1-12/1) to obtain **compound 29** (51 mg, yield: 22%).

LCMS m/z = 559.4 $[(M+2H)/2]^+$.

$^1$H NMR (400 MHz, DMSO-$d_6$) δ 12.68 (s, 1H), 11.08 (s, 1H), 8.90 - 8.71 (m, 3H), 8.36 (s, 1H), 8.27 (s, 1H), 7.97 (s, 1H), 7.78 (s, 1H), 7.70 (d, 1H), 7.64 - 7.49 (m, 2H), 7.44 (d, 1H), 6.82 (s, 1H), 5.10 (dd, 1H), 3.81 (s, 3H), 3.77 (s, 3H), 3.66 - 3.57 (m, 2H), 3.20 - 3.10 (m, 2H), 2.94 - 2.84 (m, 3H), 2.70 - 2.51 (m, 8H), 2.47-2.10 (m, 7H), 2.07 - 1.95 (m, 7H), 1.90 - 1.68 (m, 5H), 1.66 - 1.43 (m, 2H), 1.31 - 1.25 (m, 2H).

**Example 30: Preparation of compound 30**

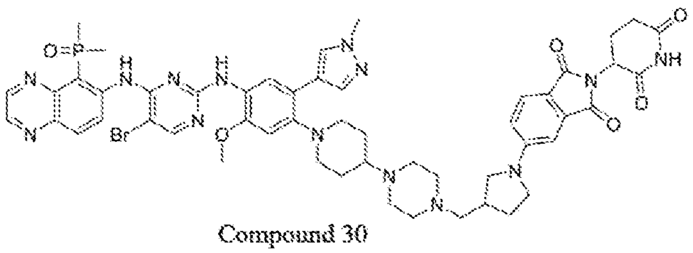

Compound 30

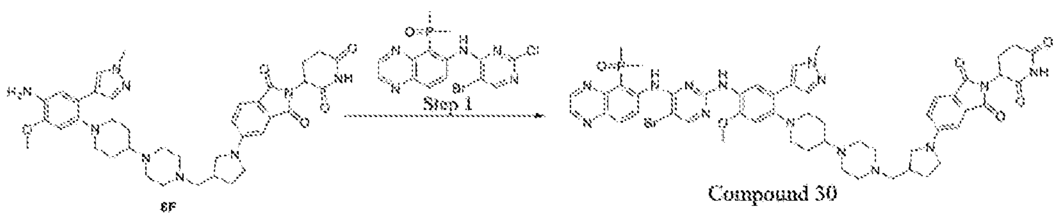


6F

Compound 30

Step 1: 5-(3-((4-(1-(4-((5-bromo-4-((5-(dimethylphosphoryl)quinoxalin-6-yl) amino)pyrimidin-2-yl)amino)-5-methoxy-2-(1-methyl-1H-pyrazol-4-yl)phenyl)piperidin-4-yl)piperazin-1-yl)methyl)pyrrolidin-1-yl)-2-(2,6-dioxopiperidin-3-yl)isoindoline-1,3-dione **(compound 30)**

**6F** (0.11 g, 0.15 mmol) and **2J** (0.06 g, 0.15 mmol) were dissolved in DMF (10 mL); p-toluenesulfonic acid hydrate (0.09 g, 0.45 mmol) was added; and under nitrogen protection, the mixture was reacted at 100°C for 16 h. The reaction solution was cooled to room temperature. 10 mL of saturated aqueous sodium bicarbonate solution was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase: dichloromethane/methanol (V/V) = 50/1-12/1) to obtain **compound 30** (31 mg, yield: 19%).

LCMS m/z = 543.3 $[(M+2H)/2]^+$.

$^1$H NMR (400 MHz, DMSO-$d_6$) δ 12.67 (s, 1H), 11.04 (s, 1H), 8.90 - 8.75 (m, 3H), 8.35 (s, 1H), 8.27 (s, 1H), 7.98 (s, 1H), 7.76 (s, 1H), 7.68 - 7.46 (m, 3H), 6.93 - 6.79 (m, 3H), 5.05 (dd, 1H), 3.80 (s, 3H), 3.77 (s, 3H), 3.58 - 3.48 (m, 2H), 3.44 - 3.36 (m, 1H), 3.19 - 3.06 (m, 3H), 2.95 - 2.81 (m, 1H), 2.75 - 2.52 (m, 9H), 2.47 - 2.08 (m, 8H), 2.07 - 1.96 (m, 7H), 1.91 - 1.69 (m, 3H), 1.66 - 1.49 (m, 2H).

Example 31: **Preparation of compound 31**

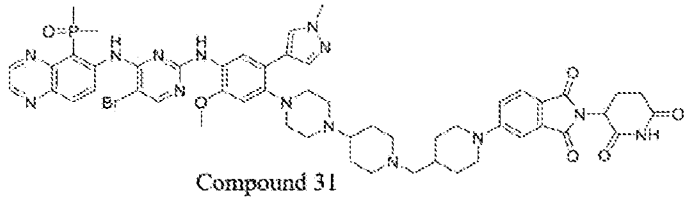

Compound 31

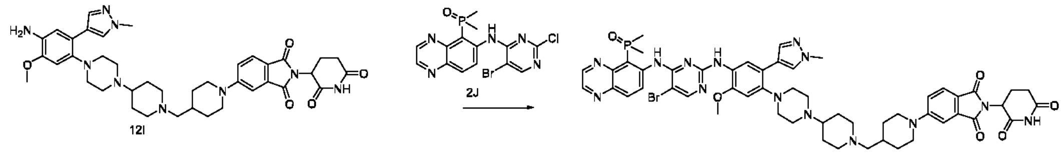


Compound 31

Step 1: 5-(4-((4-(4-(4-((5-bromo-4-((5-(dimethylphosphoryl)quinoxalin-6-yl)amino)pyrimidin-2-yl)amino)-5-methoxy-2-(1-methyl-1H-pyrazol-4-yl)phenyl)piperazin-1-yl)piperidin-1-yl)methyl)piperidin-1-yl)-2-(2,6-dioxopiperidin-3-yl)isoindoline-1,3-dione **(compound 31)**

**121** (0.25 g, 0.34 mmol) and **2J** (0.18 g, 0.44 mmol) were dissolved in DMF (5 mL); p-toluenesulfonic acid monohydrate (0.16 g, 0.84 mmol) was added; and under nitrogen protection, the mixture was stirred at 100°C for 16 h, and cooled to room temperature. 5 mL of water and 5 mL of saturated aqueous sodium bicarbonate solution were added, and the mixture was filtered. The filter cake was dried under reduced pressure and then purified by silica gel column chromatography (dichloromethane/methanol (V/V) = 100/1-12/1) to obtain a product, which was further purified by preparative HPLC (composition of mobile phases: mobile phase A: acetonitrile, and mobile phase B: water (containing 0.1% TFA)). The resultant was lyophilized to obtain a solid, which was dissolved in 20 mL of dichloromethane and extracted by adding saturated sodium bicarbonate solution (50 ml). Liquid separation was performed. The organic layer was then dried over anhydrous sodium sulphate and concentrated under reduced pressure to obtain **compound 31** (30 mg, yield: 8%).

LCMS m/z = 550.3[$(M+2H)/2]^+$.

$^1$H NMR (400 MHz, DMSO-$d_6$) $\delta$ 12.68 (s, 1H), 11.05 (s, 1H), 8.88 - 8.75 (m, 3H), 8.37 (s, 1H), 8.27 (s, 1H), 7.97 (s, 1H), 7.78 (s, 1H), 7.65 (d, 1H), 7.62 - 7.50 (m, 2H), 7.30 (d, 1H), 7.23 (dd, 1H), 6.85 (s, 1H), 5.06 (dd, 1H), 4.09 - 3.98 (m, 2H), 3.81 (s, 3H), 3.75 (s, 3H), 3.03 - 2.82 (m, 9H), 2.69 - 2.52 (m, 6H), 2.24 - 2.08 (m, 3H), 2.07 - 1.96 (m, 7H), 1.93 - 1.73 (m, 7H), 1.51 - 1.37 (m, 2H), 1.21 - 1.08 (m, 2H).

**Example 32: Preparation of compound 32**

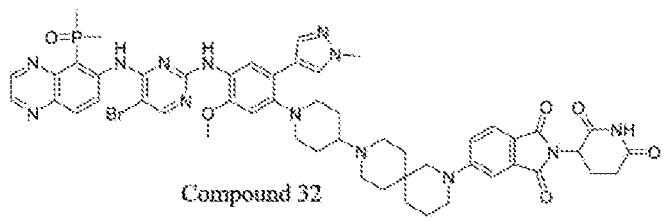

Compound 32

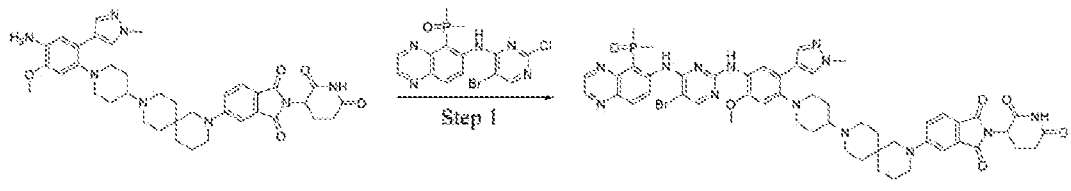


11D Compound 32

Step 1: 5-(9-(1-(4-((5-bromo-4-((5-(dimethylphosphoryl)quinoxalin-6-yl)amino)pyrimidin-2-yl)amino)-5-methoxy-2-(1-methyl-1H-pyrazol-4-yl)phenyl)piperidin-4-yl)-2,9-diazaspiro[5.5]undecan-2-yl)-2-(2,6-dioxopiperidin-3-yl)isoindoline-1,3-dione **(compound 32)**

**11D** (100 mg, 0.14 mmol) and **2J** (0.21 g, 0.51 mmol) were in DMF (10 mL); p-toluenesulfonic acid monohydrate (87 mg, 0.46 mmol) was added; and under nitrogen protection, the mixture was reacted at 100°C for 16 h, and cooled to room temperature. 20 mL of saturated aqueous sodium bicarbonate solution and 50 mL of dichloromethane were added, and the layers were separated. The organic layer was concentrated under reduced pressure, and the residue was purified by silica gel column chromatography (dichloromethane/methanol (V/V) = 100/1-10/1) to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.05% ammonia water)) to obtain **compound 32** (22 mg, yield: 15%).

LCMS m/z = 1070.3 $[M+H]^+$.

$^1H$ NMR (400 MHz, DMSO-$d_6$) δ 12.66 (s, 1H), 11.05 (s, 1H), 8.88 - 8.73 (m, 3H), 8.34 (s, 1H), 8.26 (s, 1H), 7.95 (s, 1H), 7.76 (s, 1H), 7.67 - 7.46 (m, 3H), 7.34 -7.16 (m, 2H), 6.81 (s, 1H), 5.05 (dd, 1H), 3.80 (s, 3H), 3.75 (s, 3H), 3.51 - 3.35 (m, 4H), 3.18 - 3.05 (m, 2H), 2.96 - 2.81 (m, 1H), 2.69 - 2.52 (m, 6H), 2.34 - 2.24 (m, 1H), 2.08 - 1.94 (m, 8H), 1.89 - 1.74 (m, 2H), 1.70 - 1.34 (m, 11H).

**Example 33: Preparation of compound 33**

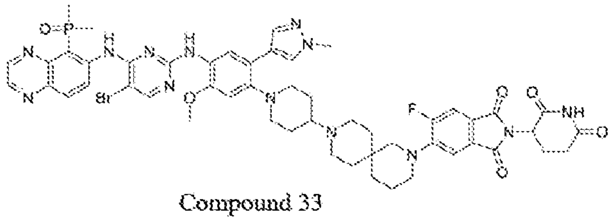

Compound 33

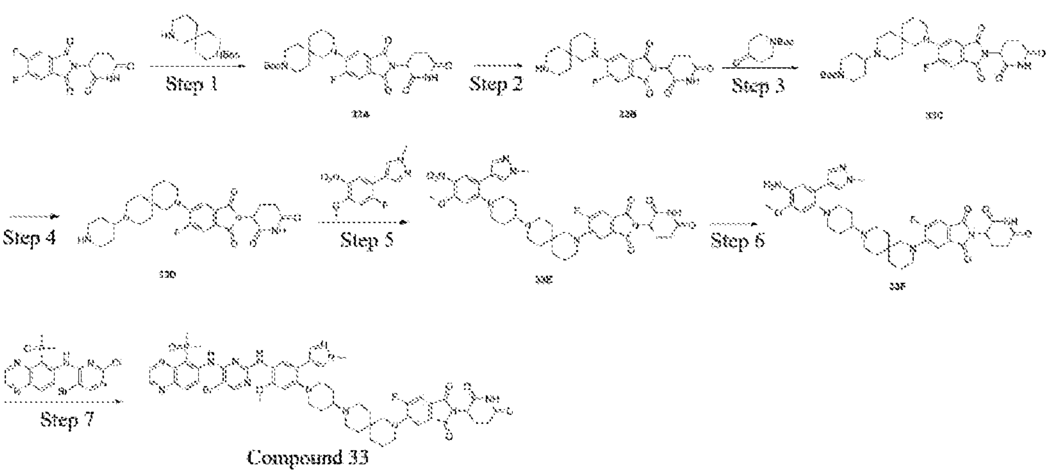

Step 1: tert-butyl 2-(2-(2,6-dioxopiperidin-3-yl)-6-fluoro-1,3-dioxoisoindolin-5-yl)-2,9-diazaspiro[5.5]undecane-9-carboxylate **(33A)**

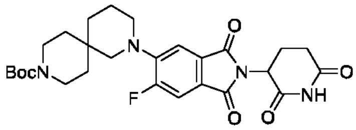

**33A**

2-(2,6-dioxopiperidin-3-yl)-5,6-difluoroisoindoline-1,3-dione (**5A-1**) (700 mg, 2.38 mmol) was dissolved in DMSO (10 mL); tert-butyl 2,9-diazaspiro[5.5]undecane-9-carboxylate (786 mg, 3.09 mmol) and sodium bicarbonate (800 mg, 9.52 mmol) were successively added; and the mixture was stirred at 100°C for 4 h, and cooled to room temperature. 10 mL of water was added, and the mixture was filtered. The filter cake was dried under reduced pressure and then purified by silica gel column chromatography (mobile phase: dichloromethane/methanol (V/V) = 100/1-20/1) to obtain **33A** as a yellow solid (0.6 g, yield: 48%).

LCMS m/z = 529.3 $[M+H]^+$.

Step 2: 2-(2,6-dioxopiperidin-3-yl)-5-fluoro-6-(2,9-diazaspiro[5.5]undecan-2-yl)isoindoline-1,3-dione (**33B**)

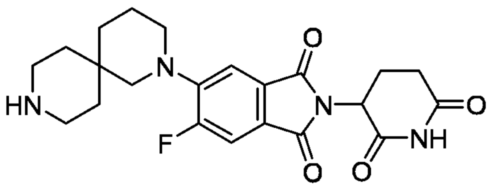

**33B**

**33A** (600 mg, 1.14 mmol) was dissolved in methanol (2 mL); a solution of hydrochloric acid in dioxane (2 M, 6 mL) was added; and the mixture was reacted at room temperature for 1 h and concentrated under reduced pressure. 20 mL of dichloromethane was added to the residue, and the mixture was adjusted to pH = 9-10 with 1 M aqueous NaOH solution. Liquid separation was performed. The organic layer was concentrated under reduced pressure to obtain **33B** (462 mg, yield: 95%).

Step 3: tert-butyl 4-(2-(2-(2,6-dioxopiperidin-3-yl)-6-fluoro-1,3-dioxoisoindolin-5-yl)-2,9-diazaspiro[5.5]undecan-9-yl)piperidine-1-carboxylate (**33C**)

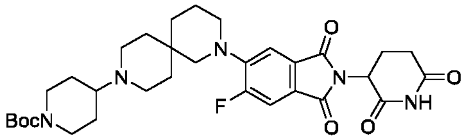

**33C**

**33B** (462 mg, 1.08 mmol) and N-tert-butoxycarbonyl-4-piperidone (279 mg, 1.40 mmol) were dissolved in DMAc (8 mL), and acetic acid (65 mg, 1.08 mmol) was added. The mixture was stirred at room temperature for 30 min, and then sodium triacetoxyborohydride (342 mg, 1.61 mmol) was added; and the resulting mixture was reacted overnight at room temperature, and extracted by adding 20 mL of saturated aqueous sodium bicarbonate solution and 20 mL of dichloromethane. The organic layer was concentrated under reduced pressure, and the residue was purified by silica gel column chromatography (mobile phase: dichloromethane/methanol (V/V) = 100/1-20/1) to obtain **33C** (400 mg, yield: 61%).

LCMS m/z = 612.3 $[M+H]^+$.

Step 4: 2-(2,6-dioxopiperidin-3-yl)-5-fluoro-6-(9-(piperidin-4-yl)-2,9-diazaspiro [5.5]undecan-2-yl)isoindoline-1,3-dione (**33D**); HCl

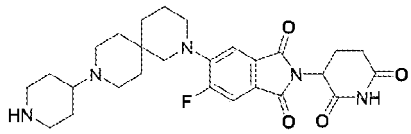

**33D**

**33C** (400 mg, 0.65 mmol) was dissolved in methanol (2 mL); a solution of hydrochloric acid in dioxane (2 *N,* 6 mL) was added; and the mixture was reacted at room temperature for 1 h and concentrated under reduced pressure, and the residue was directly used in the next step.

Step 5: 2-(2,6-dioxopiperidin-3-yl)-5-fluoro-6-(9-(1-(5-methoxy-2-(1-methyl-1H-pyrazol-4-yl)-4-nitrophenyl)piperidin-4-yl)-2,9-diazaspiro[5.5]undecan-2-yl)isoindoline-1,3-dione (**33E**)

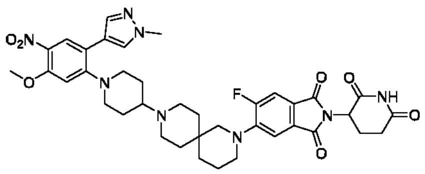

**33E**

The crude hydrochloride of **33D** from the previous step was dissolved in DMSO (5 mL); 4-(2-fluoro-4-methoxy-5-nitrophenyl)-1-methyl-1H-pyrazole (**1D**) (196 mg, 0.78 mmol) and sodium bicarbonate (202 mg, 2.4 mmol) were successively added; and the mixture was reacted at 100°C for 5 h, and cooled to room temperature. 10 mL of water was added, and the mixture was filtered. The filter cake was dried under reduced pressure and then purified by silica gel column chromatography (mobile phase: dichloromethane/methanol (V/V) = 100/1-20/1) to obtain **33E** (178 mg, yield: 37%).

LCMS m/z = 743.3 $[M+H]^+$.

Step 6: 5-(9-(1-(4-amino-5-methoxy-2-(1-methyl-1H-pyrazol-4-yl)phenyl) piperidin-4-yl)-2,9-diazaspiro[5.5]undecan-2-yl)-2-(2,6-dioxopiperidin-3-yl)-6-fluoroisoindoline-1,3-dione (**33F**)

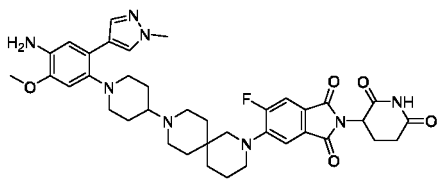

**33F**

**33E** (178 mg, 0.24 mmol) was dissolved in ethanol/water (8 mL, 3:1); iron powder (67 mg, 1.20 mmol) and ammonium chloride (64 mg, 1.20 mmol) were successively added; and under nitrogen protection, the mixture was reacted at 80°C for 1 h, cooled to room temperature and subjected to suction filtration under reduced pressure. The filtrate was concentrated under reduced pressure, and then the crude was purified by silica gel column chromatography (mobile phase: dichloromethane/methanol (V/V) = 100/1-20/1) to obtain **33F** (100 mg, yield: 58%).

Step 7: 5-(9-(1-(4-((5-bromo-4-((5-(dimethylphosphoryl)quinoxalin-6-yl)amino) pyrimidin-2-yl)amino)-5-methoxy-2-(1-methyl-1H-pyrazol-4-yl)phenyl)piperidin-4-yl)-2,9-diazaspiro[5.5]undecan-2-yl)-2-(2,6-dioxopiperidin-3-yl)-6-fluoroisoindoline-1,3-dione (**compound 33**)

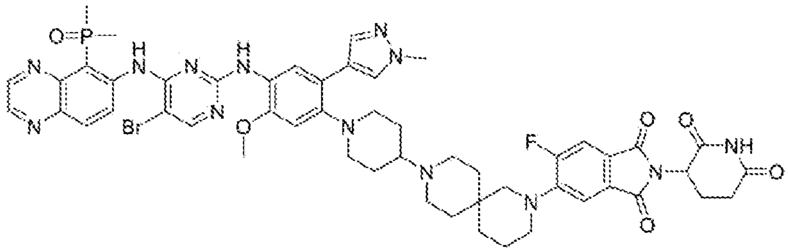

Compound 33

**33F** (100 mg, 0.14 mmol) and **2J** (87 mg, 0.21 mmol) were dissolved in DMF (8 mL); p-toluenesulfonic acid monohydrate (80 mg, 0.42 mmol) was added; and under nitrogen protection, the mixture was reacted at 100°C for 16 h, and cooled to room temperature. 20 mL of saturated aqueous sodium bicarbonate solution and 50 mL of dichloromethane were added, and the layers were separated. The organic layer was concentrated under reduced pressure, and the residue was purified by silica gel column chromatography (dichloromethane/methanol (V/V) = 100/1-10/1) to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.05% ammonia water)) to obtain **compound 33** (30 mg, yield: 20%).

LCMS m/z = 1088.3 $[M+H]^+$.

$^1H$ NMR (400 MHz, DMSO-$d_6$) δ 12.66 (s, 1H), 11.09 (s, 1H), 8.90 - 8.73 (m, 3H), 8.34 (s, 1H), 8.26 (s, 1H), 7.95 (s, 1H), 7.77 (s, 1H), 7.70 (d, 1H), 7.62 - 7.49 (m, 2H), 7.46 (d, 1H), 6.81 (s, 1H), 5.10 (dd, 1H), 3.80 (s, 3H), 3.75 (s, 3H), 3.24 - 2.99 (m, 7H), 2.95 - 2.82 (m, 1H), 2.68 - 2.51 (m, 7H), 2.36 - 2.25 (m, 1H), 2.09 - 1.94 (m, 7H), 1.89 - 1.39 (m, 12H).

**Example 34: Preparation of compound 34**

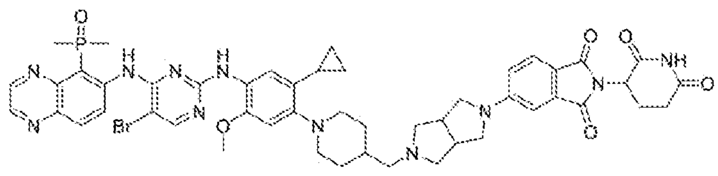

Compound 34

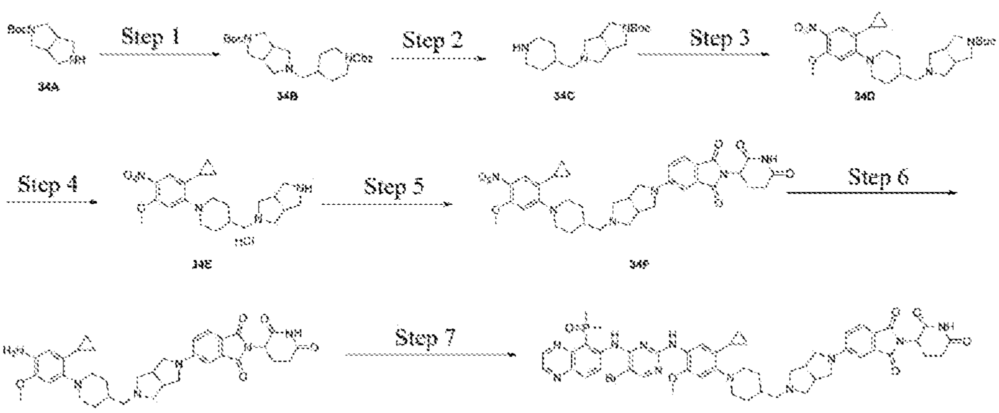


Compound 34

Step 1: tert-butyl 5-((1-((benzyloxy)carbonyl)piperidin-4-yl)methyl) hexahydropyrrolo[3,4-c]pyrrole-2(1H)-carboxylate (**34B**)

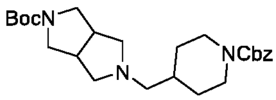

**34B**

2-tert-butoxycarbonyl-hexahydropyrrolo[3,4-c]pyrrole (**34A**) (1.06 g, 5 mmol) and benzyl 4-formylpiperidine-1-carboxylate (2.47 g, 10 mmol) were dissolved in 1,2-dichloroethane (18 mL), and acetic acid (600 mg, 10 mmol) was added. The mixture was stirred at room temperature for 30 min, and then sodium triacetoxyborohydride (3.18 g, 15 mmol) was added; and the resulting mixture was reacted overnight at room temperature, and extracted by adding 20 mL of aqueous sodium hydroxide solution (4 N) and 20 mL of dichloromethane. The organic layer was concentrated under reduced pressure, and the residue was purified by silica gel column chromatography (mobile phase: dichloromethane/methanol (V/V) = 100/1-20/1) to obtain the crude of **34B** (3.55 g), which was directly used in the next step.

LCMS m/z = 444.3 $[M+H]^+$.

Step 2: tert-butyl 5-(piperidin-4-ylmethyl)hexahydropyrrolo[3,4-c]pyrrole-2(1H)-carboxylate (**34C**)

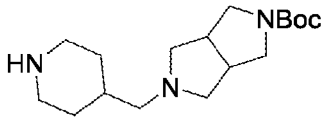

**34C**

**34B** (3.55 g, 5 mmol) was dissolved in ammonia/methanol (30 mL); palladium on carbon (wt% = 10%, 355 mg) was added; and the mixture was subjected to hydrogen replacement 3 times, stirred under hydrogen atmosphere (balloon pressure) at room temperature for 3 h and filtered. The filtrate was concentrated under reduced pressure, and the residue was purified by silica gel column chromatography (mobile phase: dichloromethane/methanol (V/V) = 100/1-20/1) to obtain **34C** (474 mg, yield: 31%).

LCMS m/z = 310.3 $[M+H]^+$.

Step 3: tert-butyl 5-((1-(2-cyclopropyl-5-methoxy-4-nitrophenyl)piperidin-4-yl)methyl)hexahydropyrrolo[3,4-c]pyrrole-2(1H)-carboxylate (**34D**)

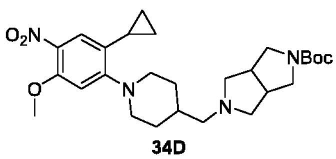

**34D**

**2E** (356 mg, 1.69 mmol) and **34C** (474 mg, 1.53 mmol) were dissolved in DMSO (10 mL); potassium carbonate (634 mg, 4.59 mmol) was added; and the mixture was reacted at 120°C for 6 h, cooled to room temperature and extracted by adding 60 mL of ethyl acetate and 60 mL of water. The organic layer was washed with saturated brine (20 mL × 3), dried over anhydrous sodium sulphate and concentrated under reduced pressure, and the residue was purified by column chromatography (dichloromethane: methanol (V/V) = 100/1-20/1) to obtain the crude of **34D** (766 mg), which was directly used in the next reaction.

LCMS m/z = 501.3 $[M+H]^+$.

Step 4: 2-((1-(2-cyclopropyl-5-methoxy-4-nitrophenyl)piperidin-4-yl)methyl) octahydropyrrolo[3,4-c]pyrrole hydrochloride (**34E**); HCl

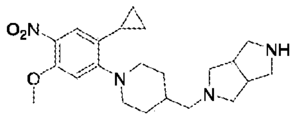

**34E**

A solution of hydrochloric acid in dioxane (4 N, 6 mL, 24 mmol) was added to the crude of **34D** (766 mg); and the mixture was stirred at room temperature for 5 min. (6 mL) was added. The reaction solution was treated until a clear solution was obtained, and the solution was stirred at room temperature for 5 min and subjected to LCMS for monitoring the reaction process. The reaction solution was concentrated to dryness under reduced pressure to obtain the crude hydrochloride of **34E**, which was directly used in the next step.
LCMS m/z = 401.3 $[M+H]^+$.

Step 5: 5-(5-((1-(2-cyclopropyl-5-methoxy-4-nitrophenyl)piperidin-4-yl)methyl) hexahydropyrrolo[3,4-c]pyrrol-2(1H)-yl)-2-(2,6-dioxopiperidin-3-yl)isoindoline-1,3-dione (**34F**)

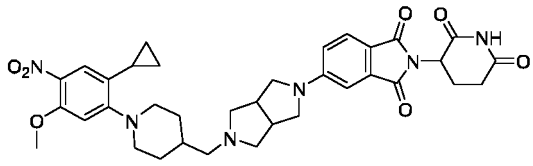

**34F**

The crude hydrochloride of **34E** from the previous step and 2-(2,6-dioxopiperidin-3-yl)-5-fluoroisoindole-1,3-dione (422 m g, 1.53 mmol) were dissolved in DMSO (10 mL); DIPEA (593 mg, 4.59 mmol) was added; and the mixture was stirred at 100°C for 5 h and cooled to room temperature. 20 mL of water was added, with a solid precipitated, and the mixture was filtered. The filter cake was dried under reduced pressure and then purified by silica gel column chromatography (dichloromethane: methanol (V/V) = 100/1-30/1) to obtain **34F** (611 mg, yield: 61%).
LCMS m/z = 657.3 $[M+H]^+$.

Step 6: 5-(5-((1-(4-amino-2-cyclopropyl-5-methoxyphenyl)piperidin-4-yl)methyl) hexahydropyrrolo[3,4-c]pyrrol-2(1H)-yl)-2-(2,6-dioxopiperidin-3-yl)isoindoline-1,3-dione (**34G**)

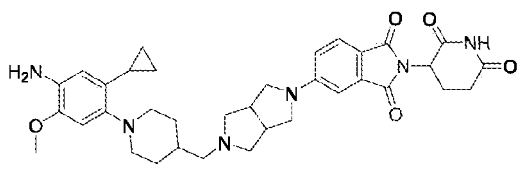

**34G**

**34F** (611 mg, 0.93 mmol) was dissolved in methanol (12 mL) and water (3 mL), and the mixture was warmed to 80°C. A mixture of ammonium chloride (611 mg, 11.5 mmol) and iron powder (611 mg, 11 mmol) was added. At this temperature, the resulting mixture was stirred for 0.5 h, cooled to room temperature and filtered. The filter cake was washed with 50 mL of dichloromethane. 10 mL of saturated brine was added to the filtrate. Liquid separation was

performed. The organic layer was dried over anhydrous sodium sulphate and concentrated under reduced pressure, and the residue was subjected to preparative plate purification (DCM/MeOH = 10/1) to obtain **34G** (140 mg, yield: 24%).

LCMS m/z = 627.3 $[M+H]^+$.

Step 7: 5-(5-((1-(4-((5-bromo-4-((5-(dimethylphosphoryl)quinoxalin-6-yl)amino) pyrimidin-2-yl)amino)-2-cyclopropyl-5-methoxyphenyl)piperidin-4-yl)methyl)hexahydropyrrolo[3,4-c]pyrrol-2(1H)-yl)-2-(2,6-dioxopiperidin-3-yl)isoindoline-1,3-dione (**compound 34**)

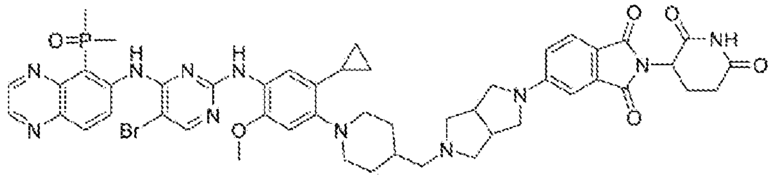

Compound 34

**34G** (0.14 g, 0.22 mmol) and **2J** (92 mg, 0.22 mmol) were dissolved in DMF (5 mL); p-toluenesulfonic acid monohydrate (127 mg, 0.67 mmol) was added; and under nitrogen protection, the mixture was stirred at 100°C for 16 h, and cooled to room temperature. 5 mL of water and 5 mL of saturated aqueous sodium bicarbonate solution were added, and the mixture was filtered. The filter cake was dried under reduced pressure and then purified by silica gel column chromatography (dichloromethane/methanol (V/V) = 100/1-12/1) to obtain a product, which was further purified by preparative HPLC (composition of mobile phases: mobile phase A: acetonitrile, and mobile phase B: water (containing 0.05% ammonia water)) to obtain **compound 34** (35 mg, yield: 16%).

LCMS m/z = 501.8 $[(M+2H)/2]^+$.

$^1H$ NMR (400 MHz, DMSO-$d_6$) δ 12.64 (s, 1H), 11.04 (s, 1H), 8.90 - 8.61 (m, 3H), 8.31 - 8.10 (m, 2H), 7.95 - 7.78 (m, 1H), 7.65 (d, 1H), 7.03 - 6.65 (m, 4H), 5.04 (dd, 1H), 3.79 - 3.63 (m, 5H), 3.29 - 3.13 (m, 4H), 3.06 - 2.94 (m, 2H), 2.93 - 2.80 (m, 1H), 2.74 - 2.59 (m, 4H), 2.59 - 2.48 (m, 4H), 2.38 - 2.27 (m, 2H), 2.12 - 1.92 (m, 8H), 1.86 - 1.76 (m, 2H), 1.63 - 1.53 (m, 1H), 1.37 - 1.24 (m, 2H), 0.73 - 0.58 (m, 2H), 0.43 - 0.20 (m, 2H).

**Example 35: Preparation of compound 35**

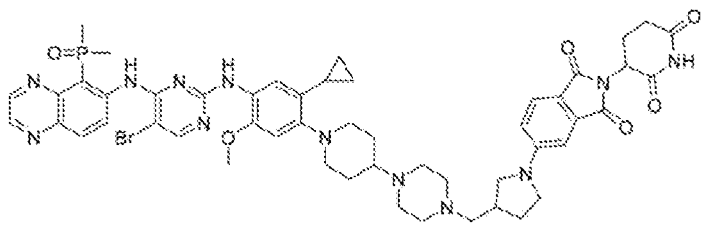

Compound 35

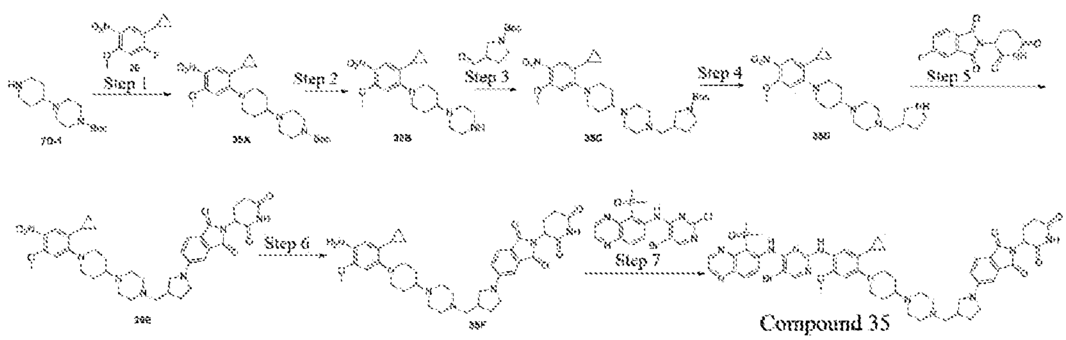


Compound 35

Step 1: tert-butyl 4-(1-(2-cyclopropyl-5-methoxy-4-nitrophenyl)piperidin-4-yl)piperazine-1-carboxylate (**35A**)

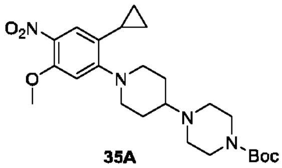

**35A**

Tert-butyl 4-(piperidin-4-yl)piperazine-1-carboxylate **(7D-1)** (0.51 g, 1.89 mmol), 1-cyclopropyl-2-fluoro-4-methoxy-5-nitrobenzene **(2E)** (0.40 g, 1.89 mmol) and potassium carbonate (0.78 g, 5.67 mmol) were mixed and dissolved in DMSO (15 mL), and the mixture was stirred at 120°C for 3 h, cooled to room temperature and extracted by adding 50 mL of water and 50 mL of ethyl acetate. The organic layer was concentrated under reduced pressure, and the residue was purified by column chromatography (mobile phase: dichloromethane/methanol (V/V) = 100/1-20/1) to obtain **35A** (340 mg, yield: 39%).

LCMS m/z = 461.3 $[M+H]^+$.

Step 2: 1-(1-(2-cyclopropyl-5-methoxy-4-nitrophenyl)piperidin-4-yl)piperazine (**compound 35B**)

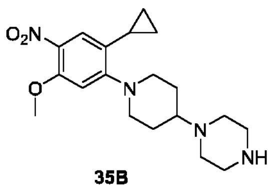

**35B**

**35A** (0.34 g, 0.74 mmol) was dissolved in DCM (15 mL); trifluoroacetic acid (5 mL) was added at room temperature; and the mixture was stirred for 1 h and concentrated under reduced pressure. 30 mL of dichloromethane was added to the residue, and the mixture was adjusted to pH = 9-10 with 1 N aqueous NaOH solution. Liquid separation was performed. The organic layer was concentrated under reduced pressure to obtain **35B** (265 mg), which was directly used in the next step.

LCMS m/z = 361.3 $[M+H]^+$.

Step 3: tert-butyl 3-((4-(1-(2-cyclopropyl-5-methoxy-4-nitrophenyl)piperidin-4-yl)piperazin-1-yl)methyl)pyrrolidine-1-carboxylate (**compound 35C**)

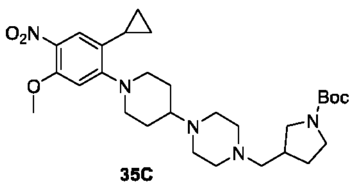

**35C**

**35B** (0.27 g, 0.74 mmol) and tert-butyl 3-formylpyrrolidine-1-carboxylate (0.18 g, 0.89 mmol) were dissolved in dichloromethane (15 mL); acetic acid (0.09 g, 1.48 mmol) and sodium triacetoxyborohydride (0.31 g, 1.48 mmol) were successively added; and the mixture was reacted at room temperature for 2 h, adjusted to a basic pH by adding 1 N aqueous sodium hydroxide solution and extracted 3 times with dichloromethane. The organic phases were combined, dried over anhydrous sodium sulphate and concentrated under reduced pressure, and the residue was purified by silica gel column chromatography (mobile phase: ethyl acetate/petroleum ether (V/V) = 1/1 for removing impurities, dichloromethane/methanol (V/V) = 20/1 for collecting a product) to obtain **35C** (400 mg, yield: 99%).

LCMS m/z = 544.3 $[M+H]^+$.

Step 4: 1-(1-(2-cyclopropyl-5-methoxy-4-nitrophenyl)piperidin-4-yl)-4-(pyrrolidin-3-ylmethyl)piperazine (**35D**)

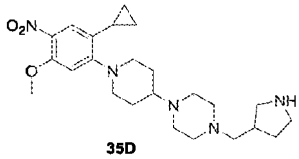

**35D**

**35C** (0.40 g, 0.74 mmol) was dissolved in DCM (15 mL); trifluoroacetic acid (5 mL) was added at room temperature; and the mixture was stirred for 1 h and concentrated under reduced pressure. 30 mL of dichloromethane was added to the residue, and the mixture was adjusted to pH = 9-10 with 1 N aqueous NaOH solution. Liquid separation was performed. The organic layer was concentrated under reduced pressure to obtain **35D** (305 mg), which was directly used in the next step.
LCMS m/z = 444.3 $[M+H]^+$.

Step 5: 5-(3-((4-(1-(2-cyclopropyl-5-methoxy-4-nitrophenyl)piperidin-4-yl) piperazin-1-yl)methyl)pyrrolidin-1-yl)-2-(2,6-dioxopiperidin-3-yl)isoindoline-1,3-dione **(35E)**

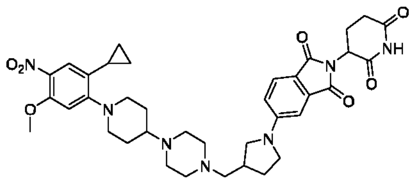

**35E**

**35D** (0.31 g, 0.69 mmol) and 2-(2,6-dioxopiperidin-3-yl)-5-fluoro-2,3-dihydro-1H-isoindole-1,3-dione (0.19 g, 0.69 mmol) were dissolved in DMSO (10 mL); DIPEA (0.27 g, 2.07 mmol) was added dropwise; and the mixture was reacted at 90°C for 3 h. The reaction solution was cooled to room temperature. 20 mL of water was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase: dichloromethane/methanol (V/V) = 50/1-12/1) to obtain **35E** (365 mg, yield: 76%).
LCMS m/z = 700.3 $[M+H]^+$.

Step 6: 5-(3-((4-(1-(4-amino-2-cyclopropyl-5-methoxyphenyl)piperidin-4-yl) piperazin-1-yl)methyl)pyrrolidin-1-yl)-2-(2,6-dioxopiperidin-3-yl)isoindoline-1,3-dione **(35F)**

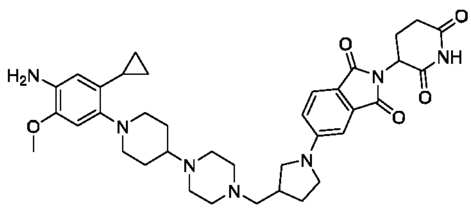

**35F**

**35E** (0.18 g, 0.26 mmol) was dissolved in ethanol (15 mL); reduced iron powder (0.07 g, 1.30 mmol) was added, and then an aqueous solution (5 mL) of ammonium chloride (0.07 g, 1.30 mmol) was added dropwise; and under nitrogen protection, the mixture was reacted at 80°C for 3 h. The reaction solution was cooled to room temperature. 5 mL of water was added, and then the mixture was extracted 3 times with dichloromethane. The organic phases were combined,

dried over anhydrous sodium sulphate and then concentrated under reduced pressure to obtain **35F** (170 mg), which was directly used in the next step.

LCMS m/z = 670.3 $[M+H]^+$.

Step 7: 5-(3-((4-(1-(4-((5-bromo-4-((5-(dimethylphosphoryl)quinoxalin-6-yl)amino)pyrimidin-2-yl)amino)-2-cyclopropyl-5-methoxyphenyl)piperidin-4-yl)piperazin-1-yl)methyl)pyrrolidin-1-yl)-2-(2,6-dioxopiperidin-3-yl)isoindoline-1,3-dione (**compound 35**)

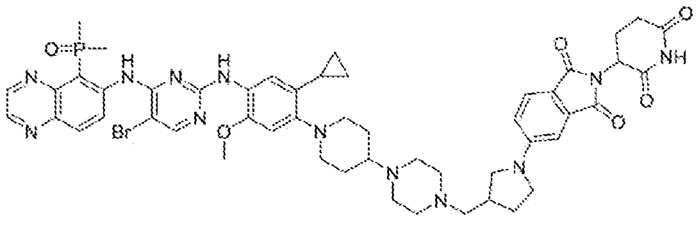

Compound 35

**35F** (0.17 g, 0.25 mmol) and **2J** (0.10 g, 0.25 mmol) were dissolved in DMF (12 mL); p-toluenesulfonic acid hydrate (0.14 g, 0.75 mmol) was added; and under nitrogen protection, the mixture was reacted at 100°C for 16 h. The reaction solution was cooled to room temperature. 10 mL of saturated aqueous sodium bicarbonate solution was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase: dichloromethane/methanol (V/V) = 50/1-12/1) to obtain a product, which was further purified by preparative HPLC (composition of mobile phases: mobile phase A: acetonitrile, and mobile phase B: water (containing 0.05% ammonia water)) to obtain **compound 35** (80 mg, yield: 31%).

LCMS m/z = 523.3 $[(M+2H)/2]^+$.

$^1H$ NMR (400 MHz, DMSO-$d_6$) δ 12.65 (s, 1H), 11.04 (s, 1H), 8.89 - 8.74 (m, 3H), 8.26 (s, 1H), 8.24 (s, 1H), 7.87 (d, 1H), 7.64 (d, 1H), 6.94 - 6.83 (m, 2H), 6.81 (dd, 1H), 6.75 (s, 1H), 5.05 (dd, 1H), 3.76 (s, 3H), 3.59 - 3.45 (m, 2H), 3.44-3.35 (m, 1H), 3.34 - 3.22 (m, 2H), 3.17 - 3.09 (m, 1H), 2.93 - 2.83 (m, 1H), 2.76 - 2.52 (m, 9H), 2.48 - 2.27 (m, 7H), 2.17-2.07 (m, 2H), 2.06 - 1.95 (m, 7H), 1.93 - 1.82 (m, 2H), 1.80-1.71 (m, 1H), 1.68 - 1.57 (m, 2H), 0.79 - 0.64 (m, 2H), 0.46 - 0.31 (m, 2H).

**Example 36: Preparation of compound 36**

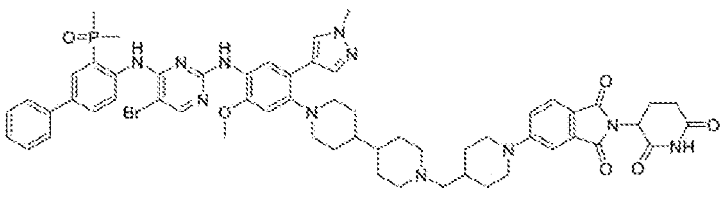

Compound 36

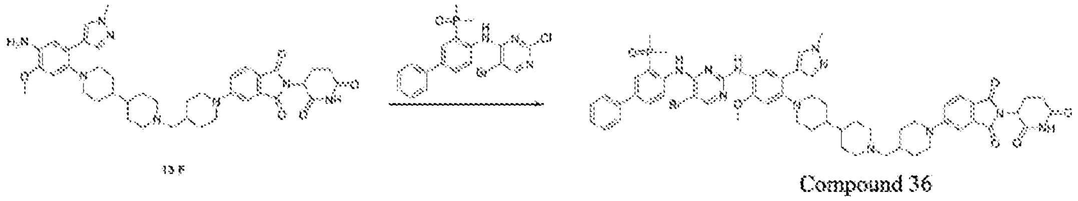

13 F

Compound 36

Step 1: 5-(4-((1'-(4-((5-bromo-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)-[4,4'-bipiperidin] -1 -yl)m ethyl)piperidin-1 -yl)-2-(2,6-dioxopiperidin-3 - yl)isoindoline-1,3-dione (**compound 36**)

**13F** (315 mg, 0.44 mmol) and **23D** (224 mg, 0.51 mmol) were dissolved in DMF (10 mL); p-toluenesulfonic acid monohydrate (251 mg, 1.32 mmol) was added; and under nitrogen protection, the mixture was reacted at 100°C for 16 h, and cooled to room temperature. 20 mL of saturated aqueous sodium bicarbonate solution and 50 mL of dichloromethane were added, and the layers were separated. The organic layer was concentrated under reduced pressure, and the residue was purified by silica gel column chromatography to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 ml). Liquid separation was performed. The organic layer was then dried over anhydrous sodium sulphate and concentrated under reduced pressure to obtain **compound 36** (110 mg, yield: 22%).

LCMS m/z = 561.8 $[(M+2H)/2]^+$.

$^1$H NMR (400 MHz, DMSO-$d_6$) δ 11.11 (s, 1H), 11.05 (s, 1H), 8.44 - 8.33 (m, 1H), 8.28 (s, 1H), 8.18 (s, 1H), 8.00 (s, 1H), 7.86 (s, 1H), 7.74 - 7.61 (m, 2H), 7.61 - 7.50 (m, 3H), 7.49 - 7.33 (m, 3H), 7.30 (d, 1H), 7.36 (s, 1H), 7.27 - 7.12 (m, 2H), 6.83 (s, 1H), 5.06 (dd, 1H), 4.09 - 3.97 (m, 2H), 3.79 (s, 3H), 3.75 (s, 3H), 3.15 - 3.03 (m, 2H), 3.03 - 2.81 (m, 5H), 2.64 - 2.52 (m, 3H), 2.18 - 2.07 (m, 2H), 2.05 - 1.96 (m, 1H), 1.89 - 1.75 (m, 10H), 1.75 - 1.65 (m, 2H), 1.34 - 1.04 (m, 10H).

**Example 37: Preparation of compound 37**

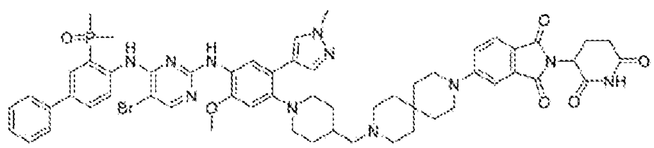

Compound 37

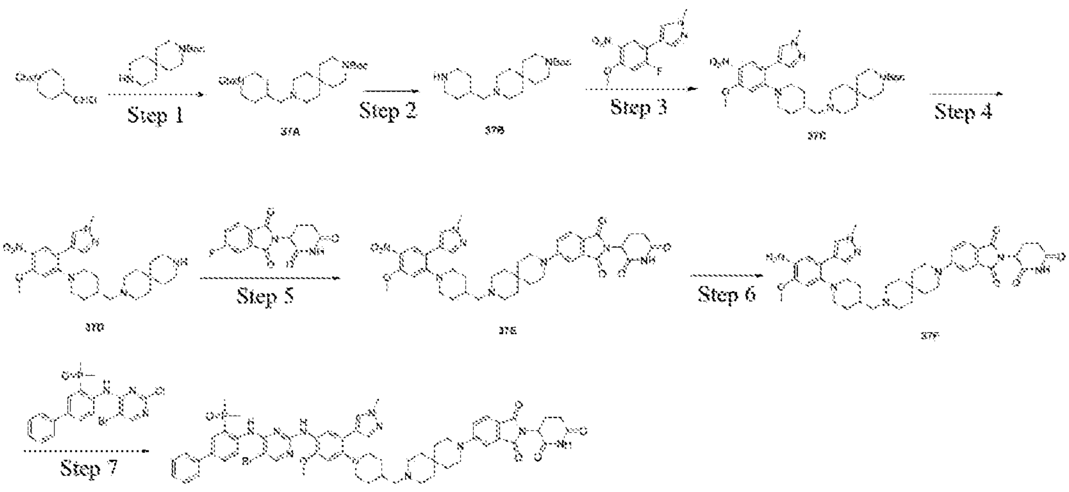


Compound 37

Step 1: tert-butyl 9-((1-((benzyloxy)carbonyl)piperidin-4-yl)methyl)-3,9-diazaspiro[5.5]undecane-3-carboxylate (**37A**)

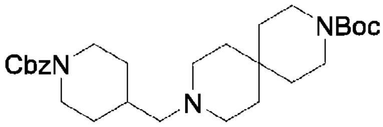

**37A**

The compound benzyl 4-formylpiperidine-1-carboxylate (6.0 g, 24.26 mmol) and tert-butyl 3,9-diazaspiro[5.5]undecane-3-carboxylate (8.0 g, 31.45 mmol) were dissolved in DMAC (100 mL); ten drops of acetic acid was added dropwise (by a 3 mL disposable plastic dropper); and then the mixture was stirred at room temperature for 1 h. Sodium triacetoxyborohydride (7.7 g, 36.33 mmol) was added, and the mixture was reacted overnight at room temperature, adjusted to a basic pH by adding saturated aqueous sodium bicarbonate solution and extracted with ethyl acetate (50 mL × 3). The organic phases were combined, washed sequentially with water (200 mL × 2) and saturated brine (200 mL), dried over anhydrous sodium sulphate and concentrated under reduced pressure, and the residue was purified by silica gel column chromatography (mobile phase: ethyl acetate/petroleum ether (V/V) = 1/1 to dichloromethane/methanol (V/V) = 20/1) to obtain **37A** (10 g, yield: 85%).

LCMS m/z = 486.4$[M+H]^+$.

Step 2: tert-butyl 9-(piperidin-4-ylmethyl)-3,9-diazaspiro[5.5]undecane-3-carboxylate **(37B)**

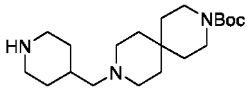

**37B**

**37A** (4.2 g, 8.65 mmol) and palladium on carbon (2.5 g, 10 wt%) were successively added and dissolved in 60 mL of methanol. The mixture was subjected to hydrogen replacement three times, reacted at room temperature for 2 h and subjected to suction filtration over celite. The filtrate was concentrated under reduced pressure, and the residue was purified by silica gel column chromatography (mobile phase: dichloromethane/methanol (V/V) = 25/1-10/1) to obtain **37B** (2.8 g, yield: 92%).

LCMS m/z = 352.3 $[M+H]^+$.

Step 3: tert-butyl 9-((1-(5-methoxy-2-(1-methyl-1H-pyrazol-4-yl)-4-nitrophenyl) piperidin-4-yl)methyl)-3,9-diazaspiro[5.5]undecane-3-carboxylate (**37C**)

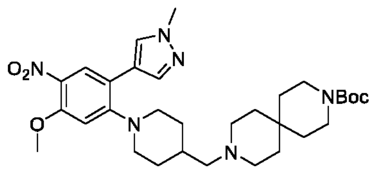

**37C**

**37B** (1.6 g, 4.55 mmol) and 4-(2-fluoro-4-methoxy-5-nitrophenyl)-1-methyl-1H-pyrazole (**1D**) (1.49 g, 5.93 mmol) were dissolved in DMSO (20 mL); sodium bicarbonate (1.53 g, 18.2 mmol) was added; and the mixture was reacted at 100°C for 6 h, cooled to room temperature and extracted by adding 50 mL of ethyl acetate and 30 mL of water. The organic layer was washed with saturated brine (20 mL × 3), dried over anhydrous sodium sulphate and concentrated under reduced pressure, and the residue was purified by column chromatography (dichloromethane: methanol (V/V) = 100/1-20/1) to obtain **37C** (1.4 g, yield: 53%).

LCMS m/z = 583.3$[M+H]^+$.

Step 4: 3-((1-(5-methoxy-2-(1-methyl-1H-pyrazol-4-yl)-4-nitrophenyl) piperidin-4-yl)methyl)-3,9-diazaspiro[5.5]undecane(**37D**); HCl

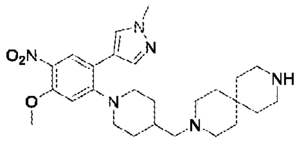

**37D**

**37C** (1.2 g, 2.06 mmol) was dissolved in methanol (4 mL); a solution of hydrochloric acid in dioxane (2 *N,* 15 mL) was added; and the mixture was reacted at room temperature for 1 h and concentrated under reduced pressure, and the residue was directly used in the next step.

Step 5: 2-(2,6-dioxopiperidin-3-yl)-5-(9-((1-(5-methoxy-2-(1-methyl-1H-pyrazol-4-yl)-4-nitrophenyl)piperidin-4-yl)methyl)-3,9-diazaspiro[5.5]undecan-3-yl)isoindoline-1,3-dione (**37E**)

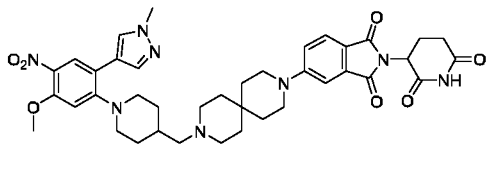

**37E**

The crude hydrochloride of **37D** from the previous step was dissolved in DMSO (20 mL); 2-(2,6-dioxopiperidin-3-yl)-5-fluoroisoindoline-1,3-dione (683 mg, 2.47 mmol) and sodium bicarbonate (692 mg, 8.24 mmol) were successively added; and the mixture was stirred at 100°C for 5 h, and cooled to room temperature. 10 mL of water was added, and the mixture was filtered. The filter cake was dried under reduced pressure and then purified by silica gel column chromatography (mobile phase: dichloromethane/methanol (V/V) = 100/1-20/1) to obtain **37E** (1.4 g, yield: 92%).
LCMS m/z = 739.3 $[M+H]^+$.

Step 6: 5-(9-((1-(4-amino-5-methoxy-2-(1-methyl-1H-pyrazol-4-yl)phenyl) piperidin-4-yl)methyl)-3,9-diazaspiro[5.5]undecan-3-yl)-2-(2,6-dioxopiperidin-3-yl)isoindoline-1,3-dione (**37F**)

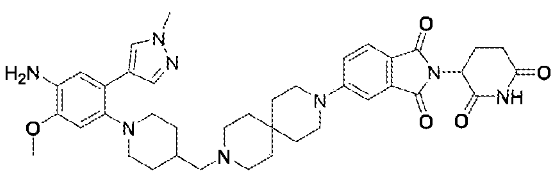

**37F**

**37E** (1.0 g, 1.35 mmol) was dissolved in ethanol/water (8 mL, 3:1); iron powder (375 mg, 6.71 mmol) and ammonium chloride (361 mg, 6.75 mmol) were successively added; and under nitrogen protection, the mixture was stirred at 80°C for 1 h, cooled to room temperature and subjected to suction filtration under reduced pressure. The filtrate was concentrated under reduced pressure, and then residue was purified by silica gel column chromatography (mobile phase: dichloromethane/methanol (V/V) = 100/1-20/1) to obtain **37F** (700 mg, yield: 73%).
LCMS m/z = 709.4 $[M+H]^+$.

Step 7: 5-(9-((1-(4-((5-bromo-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperidin-4-yl)methyl)-3,9-diazaspiro[5.5]undecan-3-yl)-2-(2,6-dioxopiperidin-3-yl)isoindoline-1,3-dione (**compound 37**)

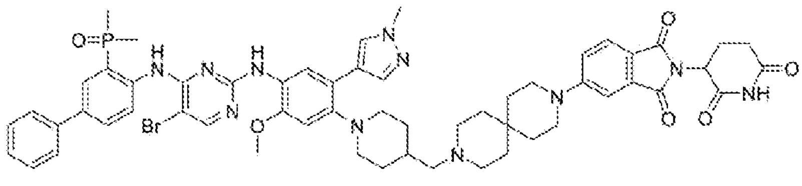

Compound 37

**37F** (200 mg, 0.28 mmol) and **23D** (148 mg, 0.34 mmol) were dissolved in DMF (10 mL); p-toluenesulfonic acid monohydrate (160 mg, 0.84 mmol) was added; and under nitrogen protection, the mixture was reacted at 100°C for 16 h, and cooled to room temperature. 10 mL of saturated aqueous sodium bicarbonate solution and 20 mL of dichloromethane were added, and the layers were separated. The organic layer was concentrated under reduced pressure, and the residue was purified by silica gel column chromatography to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 ml). Liquid separation was performed. The organic layer was then dried over anhydrous sodium sulphate and concentrated under reduced pressure to obtain **compound 37** (84 mg, yield: 27%).

LCMS m/z = 1108.4 $[M+H]^+$.

$^1H$ NMR (400 MHz, DMSO-$d_6$) δ 11.12 (s, 1H), 11.05 (s, 1H), 8.45 - 8.34 (m, 1H), 8.29 (s, 1H), 8.18 (s, 1H), 8.01 (s, 1H), 7.86 (s, 1H), 7.74 - 7.62 (m, 2H), 7.61 - 7.49 (m, 3H), 7.48 - 7.39 (m, 2H), 7.38 - 7.27 (m, 2H), 7.25 - 7.10 (m, 2H), 6.85 (s, 1H), 5.06 (dd, 1H), 3.79 (s, 3H), 3.76 (s, 3H), 3.53 - 3.39 (m, 4H), 3.12 - 2.99 (m, 2H), 2.95 - 2.80 (m, 1H), 2.64-2.52 (m, 4H), 2.43 - 2.28 (m, 4H), 2.27 - 2.17 (m, 2H), 2.07 - 1.95 (m, 1H), 1.83 (d, 6H), 1.76 - 1.66 (m, 2H), 1.62 - 1.43 (m, 9H), 1.35 - 1.23 (m, 2H).

**Example 38: Preparation of compound 38**

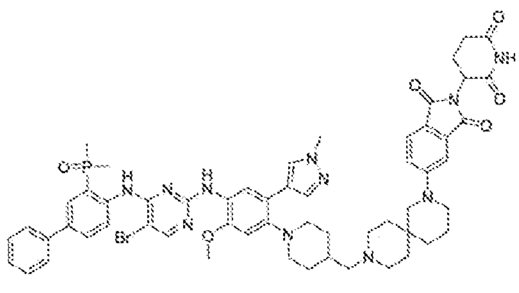

Compound 38

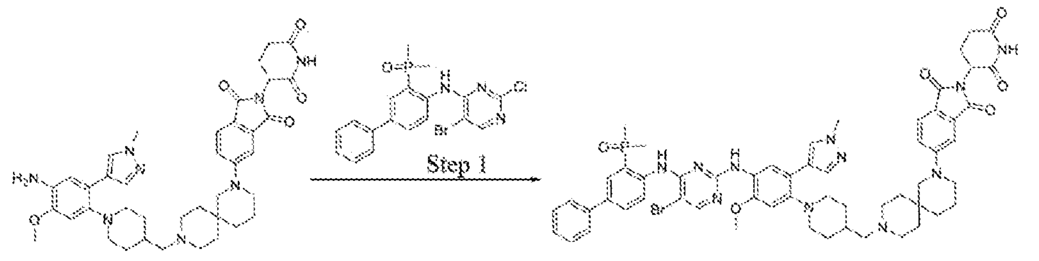


10G

Compound 38

Step 1: 5-(9-((1-(4-((5-bromo-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperidin-4-yl)methyl)-2,9-diazaspiro[5.5]undecan-2-yl)-2-(2,6-dioxopiperidin-3-yl)isoindoline-1,3-dione (**compound 38**)

**10G** (0.08 g, 0.11 mmol) and **23D** (0.05 g, 0.11 mmol) were dissolved in DMF (10 mL); p-toluenesulfonic acid hydrate (0.06 g, 0.33 mmol) was added; and under nitrogen protection, the mixture was reacted at 100°C for 16 h. The reaction solution was cooled to room temperature. 10 mL of saturated aqueous sodium bicarbonate solution was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase: dichloromethane/methanol (V/V) = 50/1-15/1) to obtain a product, which was further purified by preparative HPLC (composition of mobile phases: mobile phase A: acetonitrile, and mobile phase B: water (containing 0.05% trifluoroacetic acid)) to obtain a product, which was alkalized with aqueous sodium bicarbonate solution, extracted with dichloromethane, concentrated and dried to obtain **compound 38** (30 mg, yield: 25%).

LCMS m/z = 554.9 $[(M+2H)/2]^+$.

$^1H$ NMR (400 MHz, DMSO-$d_6$) δ 11.12 (s, 1H), 11.05 (s, 1H), 8.43 - 8.36 (m, 1H), 8.28 (s, 1H), 8.19 (s, 1H), 8.00 (s, 1H), 7.87 (s, 1H), 7.76 - 7.62 (m, 2H), 7.62 - 7.48 (m,3H), 7.47 - 7.39 (m, 2H), 7.37 - 7.28 (m, 2H), 7.28 - 7.21 (m, 1H), 7.20 - 7.12 (m, 1H), 6.82 (s, 1H), 5.05 (dd, 1H), 3.79 (s, 3H), 3.75 (s, 3H), 3.55 - 3.33 (m, 4H), 3.15-3.00 (m, 2H), 2.94 - 2.83 (m, 1H), 2.70 - 2.51 (m, 4H), 2.49 - 2.07 (m, 6H), 2.04 - 1.95 (m, 1H), 1.85 (s, 3H), 1.81 (s, 3H), 1.78 - 1.25 (m, 13H).

**Example 39: Preparation of compound 39**

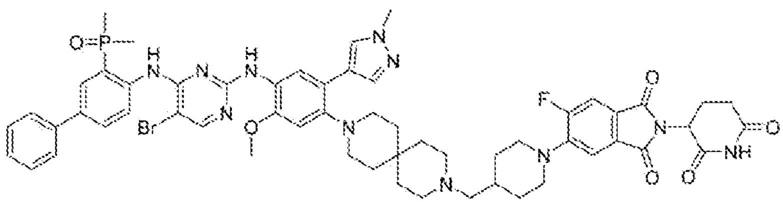

Compound 39

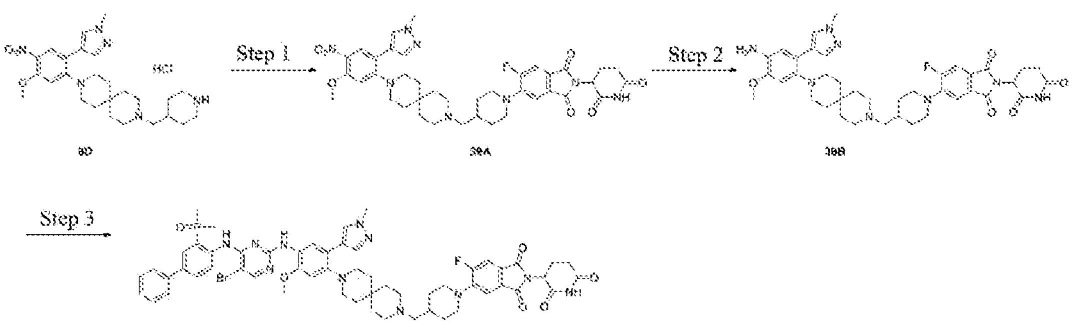


8D

39A

39B

Compound 39

Step 1: 2-(2,6-dioxopiperidin-3-yl)-5-fluoro-6-(4-((9-(5-methoxy-2-(1-methyl-1H-pyrazol-4-yl)-4-nitrophenyl)-3,9-diazaspiro[5.5]undecan-3-yl)methyl)piperidin-1-yl)isoindoline-1,3-dione (**39A**)

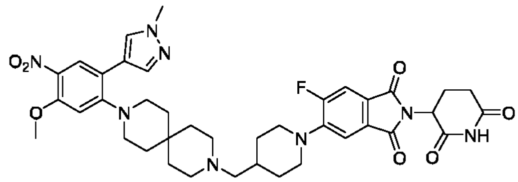

**39A**

The hydrochloride of **9D** (820 mg) and 2-(2,6-dioxopiperidin-3-yl)-5,6-difluoroisodihydroindole-1,3-dione (**5A-1**) (557 mg, 1.89 mmol) were dissolved in DMSO (10 mL); N,N-diisopropylethylamine (413 mg, 3.2 mmol) was added; and the mixture was reacted at 90°C for 6 h, cooled to room temperature and extracted by adding 60 mL of ethyl acetate and 60 mL of water. The organic layer was washed with saturated brine (20 mL × 3), dried over anhydrous sodium sulphate and concentrated under reduced pressure, and the residue was purified by column chromatography (dichloromethane: methanol (V/V) = 100/1-15/1) to obtain **39A** (0.91 g, yield: 76%).
LCMS m/z = 757.3 $[M+H]^+$.

Step 2: 5-(4-((9-(4-amino-5-methoxy-2-(1-methyl-1H-pyrazol-4-yl)phenyl)-3,9-diazaspiro [5.5]undecan-3-yl)methyl)piperidin-1-yl)-2-(2,6-dioxopiperidin-3 - yl)-6-fluoroisoindoline-1,3-dione (**39B**)

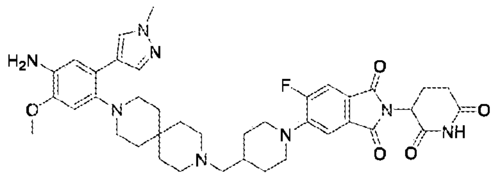

**39B**

**39A** (910 mg, 1.2 mmol) was dissolved in methanol/water (8 mL, 3 : 1); iron powder (910 mg, 17 mmol) and ammonium chloride (66.9 mg, 17.8 mmol) were successively added; and under nitrogen protection, the mixture was stirred at 80°C for 1 h, cooled to room temperature and subjected to suction filtration under reduced pressure. The filtrate was concentrated under reduced pressure, and then the crude was purified by silica gel column chromatography (mobile phase: dichloromethane/methanol (V/V) = 100/1-20/1) to obtain **39B** (345 mg, yield: 39%).
LCMS m/z = 727.4 $[M+H]^+$.

Step 3: 5-(4-((9-(4-((5-bromo-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)-3,9-diazaspiro[5.5]undecan-3-yl)methyl)piperidin-1-yl)-2-(2,6-dioxopiperidin-3-yl)-6-fluoroisoindoline-1,3-dione (**compound 39**)

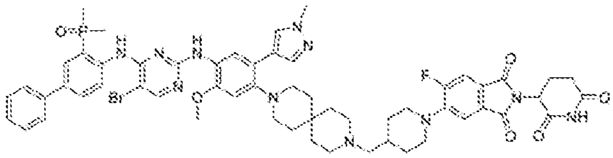

Compound 39

**39B** (154 mg, 0.21 mmol) and **23D** (99 mg, 0.23 mmol) were dissolved in DMF (10 mL); p-toluenesulfonic acid

monohydrate (119.8 mg, 0.63 mmol) was added; and under nitrogen protection, the mixture was stirred at 100°C for 16 h, and cooled to room temperature. 20 mL of saturated aqueous sodium bicarbonate solution and 50 mL of dichloromethane were added, and the layers were separated. The organic layer was concentrated under reduced pressure, and the residue was purified by silica gel column chromatography (mobile phase: dichloromethane/methanol (V/V) = 100/1-20/1) to obtain the crude. The resulting crude was separated and purified by preparative liquid phase chromatography to obtain **compound 39** (35 mg, yield: 15%).

LCMS m/z = 563.8 [(M+2H)/2]$^+$.

$^1$H NMR (400 MHz, DMSO-$d_6$) δ 11.23 - 10.92 (m, 2H), 8.45 - 8.34 (m, 1H), 8.29 (s, 1H), 8.18 (s, 1H), 8.01 (s, 1H), 7.87 (s, 1H), 7.74 - 7.62 (m, 2H), 7.60 - 7.49 (m, 3H), 7.48 - 7.39 (m, 3H), 7.39 - 7.31 (m, 1H), 7.26 - 7.11 (m, 1H), 6.89 (s, 1H), 5.09 (dd, 1H), 3.80 (s, 3H), 3.76 (s, 3H), 3.66 - 3.54 (m, 2H), 2.99 - 2.69 (m, 7H), 2.65 - 2.51 (m, 2H), 2.40 - 2.25 (m, 4H), 2.22 - 2.12 (m, 2H), 2.07 - 1.98 (m, 1H), 1.90 - 1.64 (m, 9H), 1.62 - 1.39 (m, 8H), 1.32 - 1.23 (m, 2H).

**Example 40: Preparation of compound 40**

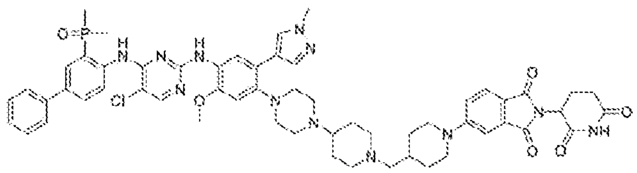

Compound 40

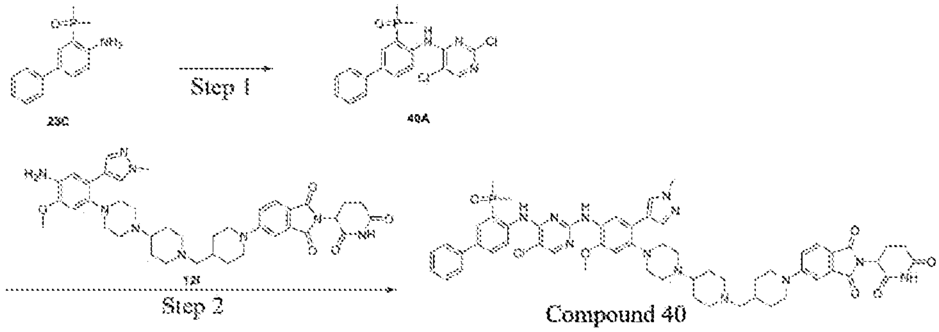


Step 1: (4-((2,5-dichloropyrimidin-4-yl)amino)-[1,1'-biphenyl]-3-yl)dimethylphosphine oxide **(40A)**

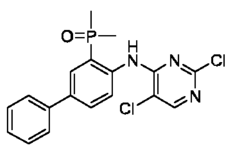

**40A**

23C (4.3 g, 17.5 mmol) and 2,4,5-trichloropyrimidine (6.4 g, 34.9 mmol) were dissolved in 50 mL of NMP; DIPEA (2.7 g, 20.9 mmol) was added; and the mixture was stirred at 130°C for 3 h and cooled to room temperature. 80 mL of water was added, and the mixture was filtered. The filter cake was dried under reduced pressure and then purified by silica gel column chromatography (petroleum ether: ethyl acetate (V/V) = 10/1-1/10) to obtain **40A** (5.7 g, yield: 83%).

LCMS m/z = 392.0[M+H]$^+$.

Step 2: 5-(4-((4-(4-(4-((5-chloro-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperazin-1-yl)piperidin-1-yl)methyl)piperidin-1-yl)-2-(2,6-dioxopiperidin-3-yl)isoindoline-1,3-dione (**compound 40**)

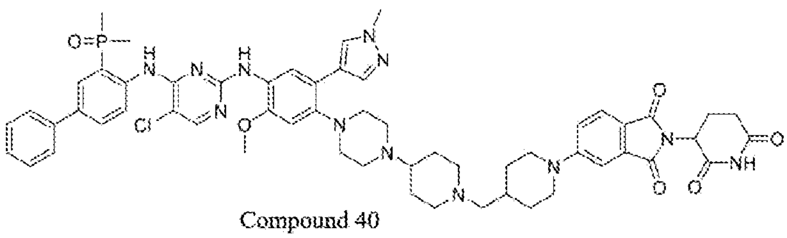

Compound 40

**121** (0.20 g, 0.28 mmol) and **40A** (0.18 g, 0.46 mmol) were dissolved in DMF (5 mL); p-toluenesulfonic acid monohydrate (0.16 g, 0.84 mmol) was added; and under nitrogen protection, the mixture was stirred at 100°C for 16 h, and cooled to room temperature. 5 mL of water and 5 mL of saturated aqueous sodium bicarbonate solution were added, and the mixture was filtered. The filter cake was dried under reduced pressure and then purified by silica gel column chromatography (dichloromethane/methanol (V/V) = 100/1-12/1) to obtain a product, which was further purified by preparative HPLC (composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 ml). Liquid separation was performed. The organic layer was then dried over anhydrous sodium sulphate and concentrated under reduced pressure to obtain **compound 40** (12 mg, yield: 4%).

LCMS m/z = 540.4 $[(M+2H)/2]^+$.

$^1H$ NMR (400 MHz, DMSO-$d_6$) δ 11.37 (s, 1H), 11.05 (s, 1H), 8.54 - 8.45 (m, 1H), 8.26 (s, 1H), 8.11 (s, 1H), 8.03 (s, 1H), 7.88 (s, 1H), 7.74 - 7.68 (m, 1H), 7.67 - 7.60 (m, 2H), 7.59 - 7.52 (m, 2H), 7.49 - 7.42 (m, 2H), 7.39 - 7.33 (m, 1H), 7.30 (d, 1H), 7.23 (dd, 1H), 7.20 - 7.13 (m, 1H), 6.86 (s, 1H), 5.06 (dd, 1H), 4.09 - 3.98 (m, 2H), 3.80 (s, 3H), 3.75 (s, 3H), 3.02 - 2.81 (m, 9H), 2.69 - 2.53 (m, 6H), 2.26 - 2.09 (m, 3H), 2.06 - 1.97 (m, 1H), 1.93 - 1.73 (m, 13H), 1.49 - 1.41 (m, 2H), 1.17 - 1.11 (m, 2H).

**Example 41: Preparation of compound 41**

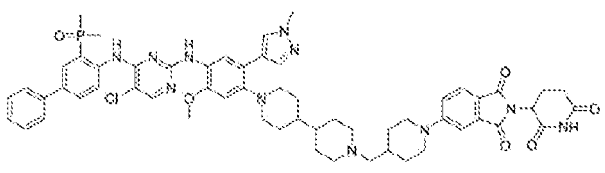

Compound 41

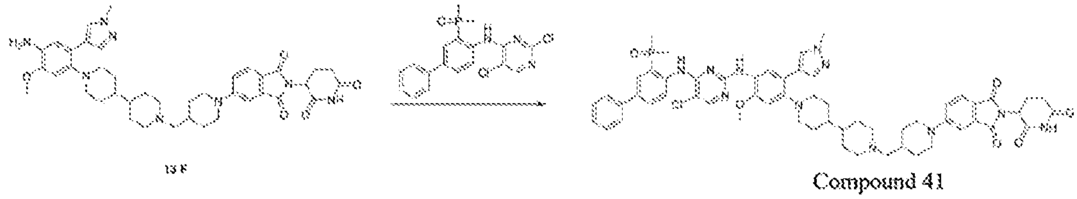

13F

Compound 41

5-(4-((1'-(4-((5-chloro-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)-[4,4'-bipiperidin] -1 -yl)m ethyl)piperidin-1 -yl)-2-(2,6-dioxopiperidin-3 - yl)isoindoline-1,3-dione (**compound 41**)

**13F** (235 mg, 0.33 mmol) and **40A** (157 mg, 0.4 mmol) were dissolved in DMF (10 mL); p-toluenesulfonic acid monohydrate (188 mg, 0.99 mmol) was added; and under nitrogen protection, the mixture was stirred at 100°C for 16 h, and cooled to room temperature. 20 mL of saturated aqueous sodium bicarbonate solution and 50 mL of dichloromethane were added, and the layers were separated. The organic layer was concentrated under reduced pressure, and the residue was purified by silica gel column chromatography (mobile phase: dichloromethane/methanol (V/V) = 100/1-10/1) to obtain **compound 41** (82 mg, yield: 23%).

LCMS m/z = 539.8 $[(M+2H)/2]^+$.

$^1H$ NMR (400 MHz, DMSO-$d_6$) δ 11.36 (s, 1H), 11.05 (s, 1H), 8.54 - 8.42 (m, 1H), 8.27 (s, 1H), 8.11 (s, 1H),

8.02 (s, 1H), 7.87 (s, 1H), 7.71 (dd, 1H), 7.65 (d, 1H), 7.61 - 7.52 (m, 3H), 7.48 - 7.33 (m, 3H), 7.30 (d, 1H), 7.26 - 7.12 (m, 2H), 6.84 (s, 1H), 5.06 (dd, 1H), 4.10 - 3.97 (m, 2H), 3.79 (s, 3H), 3.75 (s, 3H), 3.15 - 3.04 (m, 2H), 3.03 - 2.79 (m, 5H), 2.64 - 2.52 (m, 4H), 2.17 - 1.97 (m, 3H), 1.89 - 1.65 (m, 14H), 1.37 - 1.05 (m, 9H).

**Example 42: Preparation of compound 42**

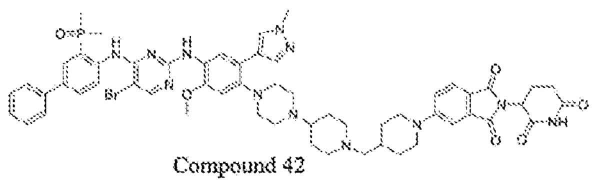

Compound 42

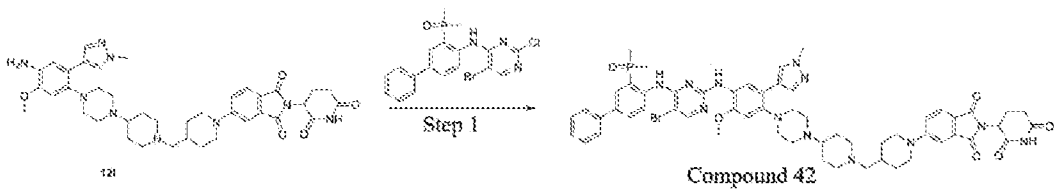


121

Compound 42

5-(4-((4-(4-(4-((5-bromo-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperazin-1-yl)piperidin-1-yl)methyl)piperidin-1-yl)-2-(2,6-dioxopiperidin-3-yl)isoindoline-1,3-dione (**compound 42**)

**121** (0.20 g, 0.28 mmol) and **23D** (0.17 g, 0.39 mmol) were dissolved in DMF (5 mL); p-toluenesulfonic acid monohydrate (0.16 g, 0.84 mmol) was added; and under nitrogen protection, the mixture was stirred at 100°C for 16 h, and cooled to room temperature. 5 mL of water and 5 mL of saturated aqueous sodium bicarbonate solution were added, and the mixture was filtered. The filter cake was dried under reduced pressure and then purified by silica gel column chromatography (dichloromethane/methanol (V/V) = 100/1-12/1) to obtain a product, which was further purified by preparative HPLC (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 ml). Liquid separation was performed. The organic layer was then dried over anhydrous sodium sulphate and concentrated under reduced pressure to obtain **compound 42** (12 mg, yield: 4%).

LCMS m/z = 562.4 $[(M+2H)/2]^+$.

**Example 43: Preparation of compound 43**

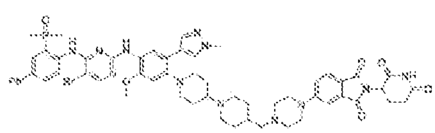

Compound 43

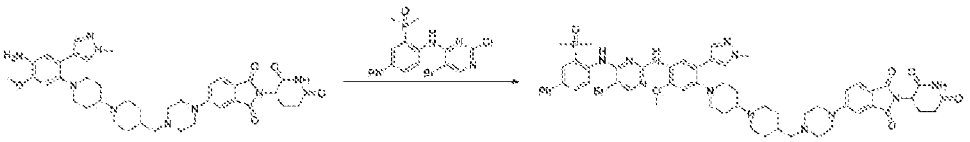

81

Compound 43

5-(4-((1'-(4-((5-bromo-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)-[1,4'-bipiperidin]-4-yl)methyl)piperazin-1-yl)-2-(2,6-dioxopiperidin-3-yl)isoindoline-1,3-dione (**compound 43**)

**8I** (180 mg, 0.249 mmol) and **23D** (109 mg, 0.25 mmol) were dissolved in DMF (5 mL); p-toluenesulfonic acid monohydrate (142 mg, 0.747 mmol) was added; and under nitrogen protection, the mixture was reacted overnight at 100°C, and cooled to room temperature. 20 mL of saturated aqueous sodium bicarbonate solution was added, and the mixture was subjected to suction filtration. The filter cake was dissolved in 50 mL of dichloromethane and extracted. The organic phase was dried over anhydrous sodium sulphate and then concentrated under reduced pressure, and the residue was purified by silica gel column chromatography (dichloromethane/methanol (V/V) = 100/1-10/1) to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)) to obtain the trifluoroacetate of **compound 43** (84 mg). 20 mL of dichloromethane and 50 mL of saturated sodium bicarbonate solution were added to the **trifluoroacetate of compound 43,** and the layers were separated. The organic phase was dried over anhydrous sodium sulphate and then concentrated under reduced pressure to obtain **compound 43** (60 mg, yield: 21%).

LCMS m/z = 1123.4 $[M+H]^+$.

$^1H$ NMR (400 MHz, DMSO-$d_6$) δ 11.11 (s, 1H), 11.05 (s, 1H), 8.42 - 8.35 (m, 1H), 8.27 (s, 1H), 8.18 (s, 1H), 8.00 (s, 1H), 7.86 (s, 1H), 7.72 - 7.65 (m, 2H), 7.59 - 7.52 (m, 3H), 7.47 - 7.42 (m, 2H), 7.39 - 7.35 (m, 1H), 7.34 - 7.31 (m, 1H), 7.25 (dd, 1H), 7.22 - 7.15 (m, 1H), 6.83 (s, 1H), 5.07 (dd, 1H), 3.77 (d, 6H), 3.48 - 3.39 (m, 4H), 3.13 - 3.06 (m, 2H), 2.95 - 2.87 (m, 2H), 2.64 - 2.53 (m, 4H), 2.49 - 2.45 (m, 3H), 2.20 - 2.12 (m, 4H), 2.04 - 1.97 (m, 2H), 1.83 (d, 6H), 1.78 - 1.71 (m, 4H), 1.62 - 1.48 (m, 5H), 1.17 - 1.14 (m, 2H).

**Example 44: Preparation of compound 44**

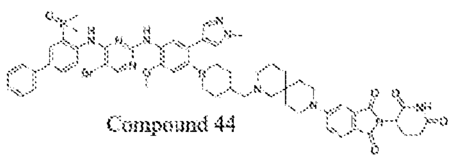

Compound 44

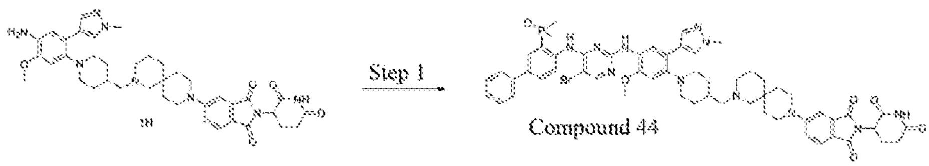


Compound 44

5-(2-((1-(4-((5-bromo-4-((3-(dimethylphosphoryl)-[1,1'-biphenyl]-4-yl)amino)pyrimidin-2-yl)amino)-5-methoxy-2-(1 -methyl-1H-pyrazol-4-yl)phenyl)piperidin-4-yl)methyl)-2,9-diazaspiro[5.5]undecan-9-yl)-2-(2,6-dioxopiperidin-3-yl)isoindoline-1,3-dione (**compound 44**)

**1H** (142 mg, 0.2 mmol) and **23D** (99 mg, 0.23 mmol) were dissolved in DMF (5 mL); p-toluenesulfonic acid monohydrate (0.16 g, 0.84 mmol) was added; and under nitrogen protection, the mixture was stirred at 100°C for 16 h, and cooled to room temperature. 5 mL of water and 5 mL of saturated aqueous sodium bicarbonate solution were added, and the mixture was filtered. The filter cake was dried under reduced pressure and then purified by silica gel column chromatography (dichloromethane/methanol (V/V) = 100/1-12/1) to obtain a product, which was further purified by preparative HPLC (composition of mobile phases: mobile phase A: acetonitrile, and mobile phase B: water (containing 0.1% TFA)). The resultant was lyophilized. The resulting solid was dissolved in 20 mL of dichloromethane, and saturated sodium bicarbonate solution (50 ml) was added. Liquid separation was performed. The organic layer was then dried over anhydrous sodium sulphate and concentrated under reduced pressure to obtain **compound 44** (34 mg, yield: 15.3%).

LCMS m/z = 554.8 $[(M+2H)/2]^+$.

$^1H$ NMR (400 MHz, DMSO-$d_6$) δ 11.11 (s, 1H), 11.04 (s, 1H), 8.47 - 8.33 (m, 1H), 8.29 (s, 1H), 8.18 (s, 1H), 8.00 (s, 1H), 7.87 (s, 1H), 7.75 - 7.50 (m, 5H), 7.48 - 7.38 (m, 2H), 7.36 - 7.26 (m, 2H), 7.24 - 7.06 (m, 2H), 6.83 (s, 1H), 5.05 (dd, 1H), 3.77 (s, 3H), 3.75 (s, 3H), 3.56 - 3.39 (m, 4H), 3.14 - 3.00 (m, 2H), 2.97 - 2.81 (m, 1H), 2.75 - 2.51 (m, 6H), 2.41 - 2.11 (m, 4H), 2.04 - 1.96 (m, 1H), 1.83 (d, 6H), 1.76 - 1.44 (m, 9H), 1.40 - 1.23 (m,4H).

**Example 45: Preparation of compound 45**

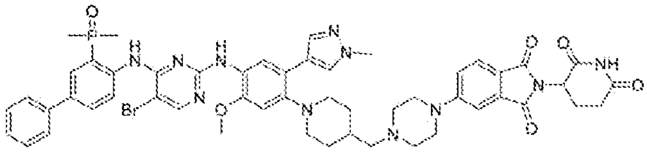

Compound 45

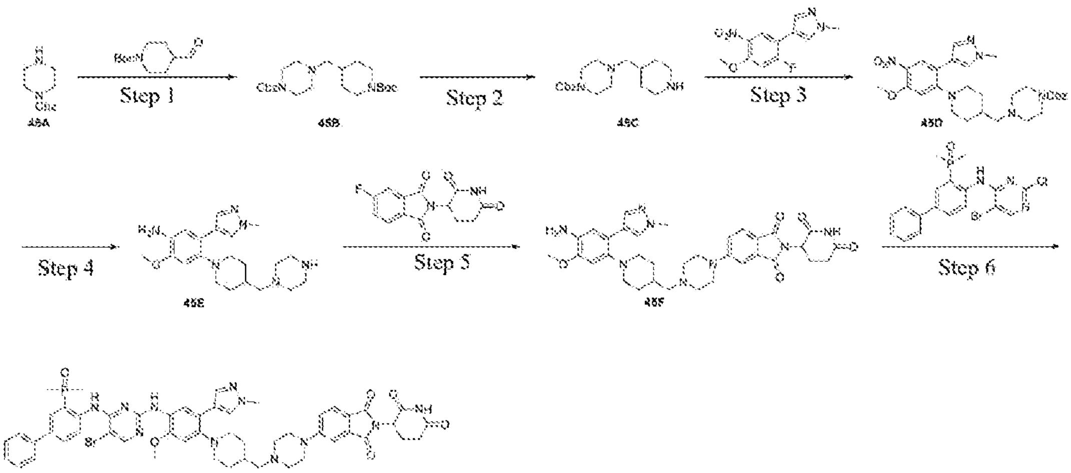


Compound 45

Step 1: benzyl 4-((1-(tert-butoxycarbonyl)piperidin-4-yl)methyl)piperazine-1-carboxylate (**45B**)

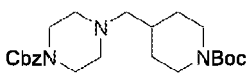

**45B**

**45A** (6.0 g, 27.24 mmol) and tert-butyl 4-formylpiperidine-1-carboxylate (11.62 g, 54.48 mmol) were dissolved in DMAc (50 mL); acetic acid (3.27 g, 54.45 mmol) was added; and the mixture was stirred at room temperature for 2 h. Sodium triacetoxyborohydride (17.32 g, 81.72 mmol) was added, and the mixture was reacted overnight at room temperature. After the reaction was completed, 100 mL of ethyl acetate and 200 mL of water were added. The organic phase was dried over anhydrous sodium sulphate and then concentrated under reduced pressure, and the residue was purified by silica gel column chromatography (mobile phase: dichloromethane/methanol (V/V) = 100/1-10/1) to obtain **45B** as a white solid (6.0 g, yield: 53%).

LCMS m/z = 418.3 $[M+H]^+$.

Step 2: benzyl 4-(piperidin-4-ylmethyl)piperazine-1-carboxylate (**45C**); HCl

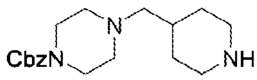

**45C**

**45B** (3.0 g, 7.18 mmol) was dissolved in 5 mL of methanol; a solution of hydrogen chloride in dioxane (1 M, 30 mL) was added to the reaction system; and the mixture was reacted at room temperature for 1 h. After the reaction was completed, the mixture was concentrated under reduced pressure to obtain the hydrochloride of **45C** (2.24 g), which was directly used in the next reaction.

LCMS m/z = 318.2 $[M+H]^+$.

Step 3: benzyl 4-((1-(5-methoxy-2-(1-methyl-1H-pyrazol-4-yl)-4-nitrophenyl) piperidin-4-yl) methyl)piperazine-1-carboxylate (**45D**)

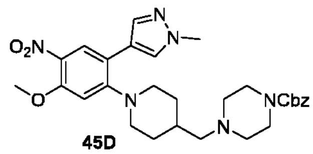

**45D**

The crude **hydrochloride of 45C** (2.24 g) was dissolved in DMSO (20 mL); solid potassium carbonate (8.13 g, 58.8 mmol) was added in batches; and the mixture was stirred for 5 min. The compound 4-(2-fluoro-4-methoxy-5-nitrophenyl)-1-methyl-1H-pyrazole (**1D**) (1.48 g, 5.89 mmol) was added, and the mixture was reacted overnight at 100°C. After the reaction was completed, the mixture was cooled to room temperature and extracted with 50 mL of ethyl acetate and 200 mL of water. The organic phase was dried over anhydrous sodium sulphate and then concentrated under reduced pressure, and the residue was purified by silica gel column chromatography (mobile phase: dichloromethane/methanol (V/V) = 100/1-10/1) to obtain **45D** (2.28 g, yield: 58%).

LCMS m/z = 549.3 $[M+H]^+$.

Step 4: 2-methoxy-5-(1-methyl-1H-pyrazol-4-yl)-4-(4-(piperazin-1-ylmethyl) piperidin-1-yl)aniline (**45E**)

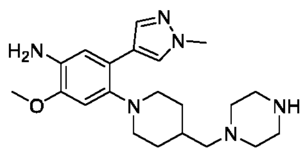

**45E**

Compound **45D** (2.28 g, 4.16 mmol) was dissolved in 20 mL of methanol; palladium on carbon (44 mg) was added; and the mixture was subjected to hydrogen replacement three times. Under hydrogen atmosphere, the mixture was reacted overnight at room temperature. After the reaction was completed, the mixture was subjected to suction filtration, and the filtrate was concentrated under reduced pressure to obtain the crude of **45E** (1.6 g).

LCMS m/z = 385.3 $[M+H]^+$.

Step 5: 5-(4-((1-(4-amino-5-methoxy-2-(1-methyl-1H-pyrazol-4-yl)phenyl) piperidin-4-yl)methyl)piperazin-1-yl)-2-(2,6-dioxopiperidin-3-yl)isoindoline-1,3-dione (**45F**)

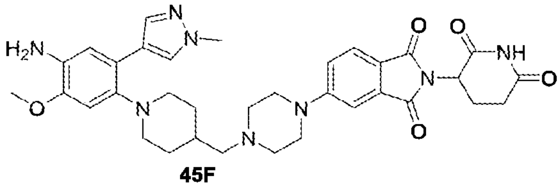

**45F**

The **crude of 45E** (800 mg) was dissolved in DMSO (10 mL); 2-(2,6-dioxopiperidin-3-yl)-5-fluoroisoindoline-1,3-dione (1.14 g, 4.13 mmol) and DIPEA (538 mg, 4.16 mmol) were successively added; and the mixture was reacted

at 100°C for 3 h, and cooled to room temperature. 10 mL of water was added, and the mixture was subjected to suction filtration. The filter cake was dissolved in 50 mL of dichloromethane and extracted. The organic phase was dried over anhydrous sodium sulphate and then concentrated under reduced pressure, and the residue was purified by silica gel column chromatography (mobile phase: dichloromethane/methanol (V/V) = 100/1-10/1) to obtain **45F** as a yellow solid (1.0 g, yield: 75%).

LCMS m/z = 641.3 $[M+H]^+$.

$^1H$ NMR (400 MHz, $CD_3OD$) δ 7.98 (s, 1H), 7.89 (s, 1H), 7.68 (d, 1H), 7.36 (d, 1H), 7.22 (dd, 1H), 6.87 (s, 1H), 6.74 (s, 1H), 5.06 (dd, 1H), 3.90 (s, 3H), 3.85 (s, 3H), 3.53 - 3.42 (m, 4H), 3.08 - 2.96 (m, 2H), 2.91 - 2.69 (m, 3H), 2.69 - 2.54 (m, 6H), 2.41 - 2.28 (m, 2H), 2.15 - 2.06 (m, 1H), 1.90 - 1.78 (m, 2H), 1.77 - 1.61 (m, 1H), 1.43 - 1.24 (m, 2H).

Step 6: 5-(4-((1-(4-((5-bromo-4-((3-(dimethylphosphoryl)-[1,1'-biphenyl]-4-yl) amino)pyrimidin-2-yl)amino)-5-methoxy-2-(1-methyl-1H-pyrazol-4-yl)phenyl)piperidin-4-yl)methyl)piperazin-1-yl)-2-(2,6-dioxopiperidin-3-yl)isoindoline-1,3-dione (**compound 45**)

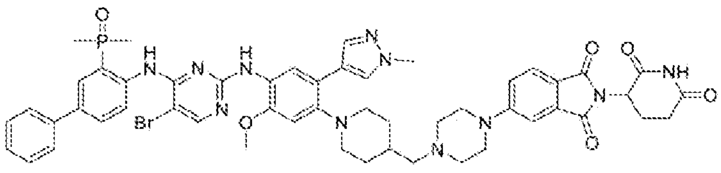

Compound 45

**45F** (200 mg, 0.31 mmol) and **23D** (203 mg, 0.465 mmol) were dissolved in DMF (5 mL); p-toluenesulfonic acid monohydrate (177 mg, 0.93 mmol) was added; and under nitrogen protection, the mixture was reacted overnight at 100°C, and cooled to room temperature. 20 mL of saturated aqueous sodium bicarbonate solution was added, and the mixture was subjected to suction filtration. The filter cake was dissolved in 50 mL of dichloromethane and extracted. The organic phase was dried over anhydrous sodium sulphate and then concentrated under reduced pressure, and the residue was purified by silica gel column chromatography (dichloromethane/methanol (V/V) = 100/1-10/1) to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)) to obtain the trifluoroacetate of **compound 45** (60 mg). 20 mL of dichloromethane and 50 mL of saturated sodium bicarbonate solution were added to the trifluoroacetate of **compound 45,** and the layers were separated. The organic phase was dried over anhydrous sodium sulphate and then concentrated under reduced pressure to obtain **compound 45** (45 mg, yield: 14%).

LCMS m/z = 1040.3 $[M+H]^+$.

$^1H$ NMR (400 MHz, $D_2O/CF_3COOD$(v/v = 1:1))δ 8.30 (s, 1H), 8.13 - 8.00 (m, 2H), 7.89 - 7.68 (m, 4H), 7.68 - 7.55 (m, 4H), 7.55 - 7.43 (m, 2H), 7.43 - 7.20 (m, 3H), 5.13 (dd, 1H), 4.25 - 4.09 (m, 2H), 4.06 (s, 3H), 3.94 - 3.64 (m, 6H), 3.61 - 3.46 (m, 5H), 3.39 - 3.12 (m, 4H), 2.96 - 2.85 (m, 2H), 2.83 - 2.67 (m, 1H), 2.57 - 2.38 (m, 1H), 2.30 - 2.11 (m, 3H), 2.09 - 1.85 (m, 8H).

**Example 46: Preparation of compound 46**

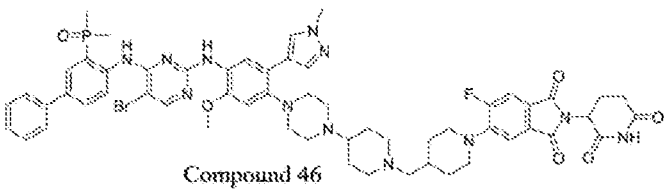

Compound 46

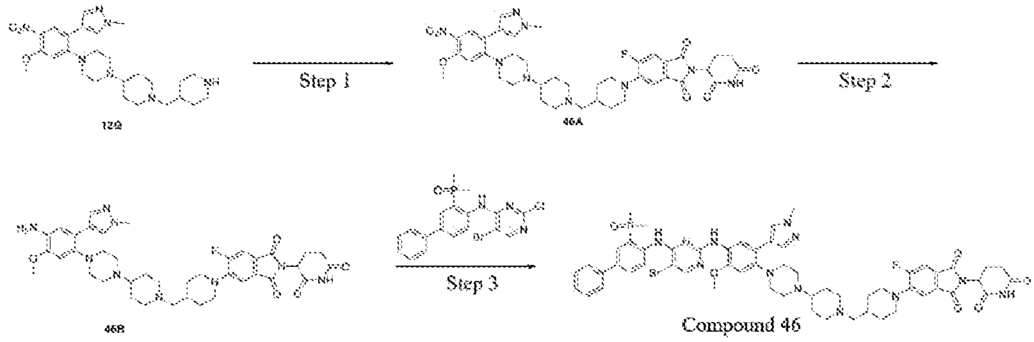


Step 1: 2-(2,6-dioxopiperidin-3-yl)-5-fluoro-6-(4-((4-(4-(5-methoxy-2-(1-methyl-1H-pyrazol-4-yl)-4-nitrophenyl)piperazin-1-yl)piperidin-1-yl)methyl)piperidin-1-yl)isoindoline-1,3-dione (**46A**)

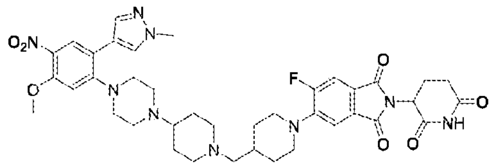

**46A**

**12G** (0.3 g, 0.60 mmol) and 2-(2,6-dioxopiperidin-3-yl)-5,6-difluoroisoindoline-1,3-dione (**5A-1**) (0.25 g, 0.85 mmol) were dissolved in DMSO (10 mL); DIPEA (0.21 g, 1.62 mmol) was added; and the mixture was stirred at 90°C for 5 h and cooled to room temperature. 20 mL of water was added, and the mixture was filtered. The filter cake was dried under reduced pressure and then purified by silica gel column chromatography (dichloromethane: methanol (V/V) = 100/1-15/1) to obtain **46A** (0.31 g, yield: 67%)
LCMS m/z = 772.4 $[M+H]^+$.

Step 2: 5-(4-((4-(4-(4-amino-5-methoxy-2-(1-methyl-1H-pyrazol-4-yl)phenyl) piperazin-1-yl)piperidin-1-yl)methyl)piperidin-1-yl)-2-(2,6-dioxopiperidin-3-yl)-6-fluoroisoindoline-1,3-dione (**46B**)

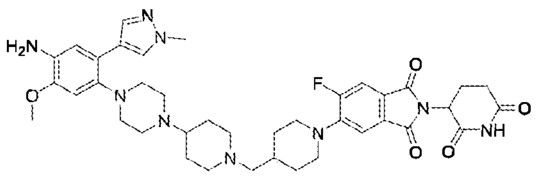

**46B**

**46A** (0.31 g, 0.40 mmol) was dissolved in ethanol (12 mL) and water (3 mL), and the mixture was warmed to 80°C. A mixture of ammonium chloride (0.22 g, 4.11 mmol) and iron powder (0.23 g, 4.12 mmol) was added. At this temperature, the resulting mixture was stirred for 0.5 h, cooled to room temperature and filtered. The filter cake was washed with 50 mL of dichloromethane. 10 mL of saturated brine was added to the filtrate. Liquid separation was performed. The organic layer was dried over anhydrous sodium sulphate and concentrated under reduced pressure to obtain the crude of **46B** (0.19 g), which was directly used in the next step.
LCMS m/z = 742.3 $[M+H]^+$.

Step 3: 5-(4-((4-(4-(4-((5-bromo-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperazin-1-yl)piperidin-1-yl)methyl)piperidin-1-yl)-2-(2,6-dioxopiperidin-3-yl)-6-fluoroisoindoline-1,3-dione (**compound 46**)

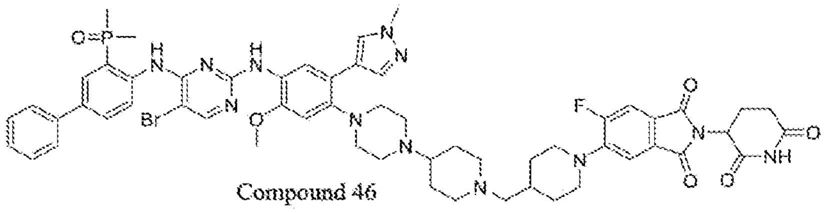

Compound 46

The **crude of 46B** (0.15 g) and **23D** (0.12 g, 0.27 mmol) were dissolved in DMF (5 mL); p-toluenesulfonic acid monohydrate (0.16 g, 0.84 mmol) was added; and under nitrogen protection, the mixture was stirred at 100°C for 16 h, and cooled to room temperature. 5 mL of water and 5 mL of saturated aqueous sodium bicarbonate solution were added, and the mixture was filtered. The filter cake was dried under reduced pressure and then purified by silica gel column chromatography (dichloromethane/methanol (V/V) = 100/1-12/1) to obtain a product, which was further purified by preparative HPLC (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane, and saturated sodium bicarbonate solution (50 ml) was added. Liquid separation was performed. The organic layer was then dried over anhydrous sodium sulphate and concentrated under reduced pressure to obtain **compound 46** (26 mg, yield: 7%).

LCMS m/z = 571.3 $[(M+2H)/2]^+$.

$^1H$ NMR (400 MHz, DMSO-$d_6$) δ 11.11 (s, 1H), 11.08 (s, 1H), 8.39 (s, 1H), 8.26 (s, 1H), 8.19 (s, 1H), 8.01 (s, 1H), 7.87 (s, 1H), 7.73 - 7.70 (m, 1H), 7.68 (s, 1H), 7.59 (s, 1H), 7.55 (d, 2H), 7.48 - 7.43 (m, 3H), 7.36 (t, 1H), 7.18 (m, 1H), 6.85 (s, 1H), 5.10 (dd, 1H), 3.78 (d, 3H), 3.75 (s, 3H), 3.61 (d, 2H), 2.92 - 2.83 (m, 8H), 2.66 - 2.56 (m, 5H), 2.54 - 2.52 (m, 1H), 2.25 - 2.14 (m, 3H), 2.09 - 2.00 (m, 1H), 1.94 - 1.72 (m, 13H), 1.48 - 1.39 (m, 2H), 1.33 - 1.20 (m, 3H).

**Example 47: Compound 47**

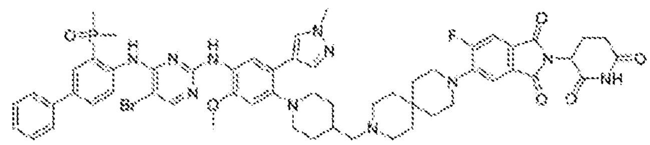

Compound 47

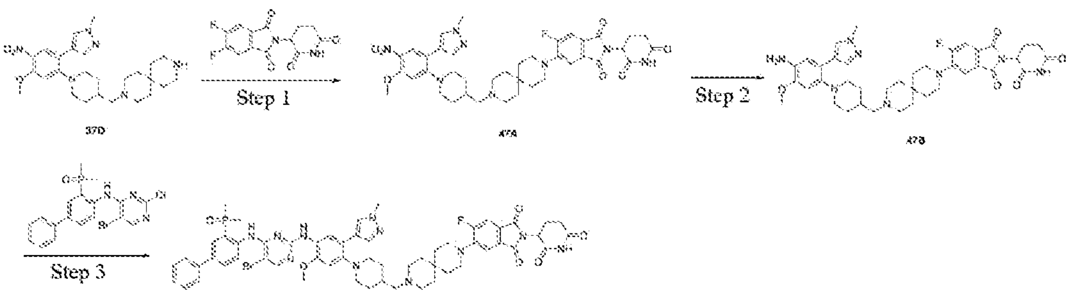


Compound 47

Step 1: 2-(2,6-dioxopiperidin-3-yl)-5-fluoro-6-(9-((1-(5-methoxy-2-(1-methyl-1H-pyrazol-4-yl)-4-nitrophenyl)piperidin-4-yl)methyl)-3,9-diazaspiro[5.5]undecan-3-yl)isoindoline-1,3-dione (**47A**)

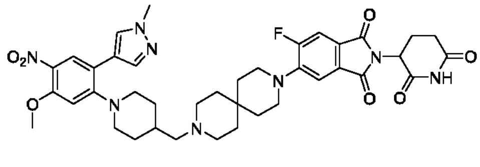

47A

The crude hydrochloride of **37D** (333 mg) was dissolved in DMSO (10 mL); 2-(2,6-dioxopiperidin-3-yl)-5,6-difluoroisoindoline-1,3-dione (**5A-1**) (265 mg, 0.90 mmol) and sodium bicarbonate (232 mg, 2.76 mmol) were successively added; and the mixture was stirred at 100°C for 5 h, and cooled to room temperature. 10 mL of water was added, and the mixture was filtered. The filter cake was dried under reduced pressure and then purified by silica gel column chromatography (mobile phase: dichloromethane/methanol (V/V) = 100/1-20/1) to obtain **47A** (420 mg).

Step 2: 5-(9-((1-(4-amino-5-methoxy-2-(1-methyl-1H-pyrazol-4-yl)phenyl) piperidin-4-yl)methyl)-3,9-diazaspiro[5.5]undecan-3-yl)-2-(2,6-dioxopiperidin-3-yl)-6-fluoroisoindoline-1,3-dione (**47B**)

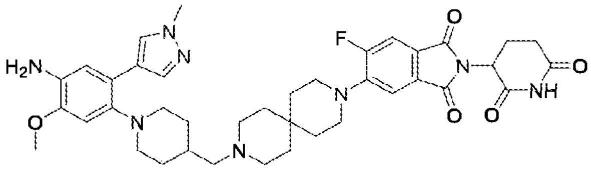

47B

**47A** (420 mg, 0.55 mmol) was dissolved in ethanol/water (8 mL, 3:1); iron powder (154 mg, 2.75 mmol) and ammonium chloride (147 mg, 2.75 mmol) were successively added; and under nitrogen protection, the mixture was stirred at 80°C for 1 h, cooled to room temperature and subjected to suction filtration under reduced pressure. The filtrate was concentrated under reduced pressure, and then the crude was purified by silica gel column chromatography (mobile phase: dichloromethane/methanol (V/V) = 100/1-20/1) to obtain **47B** (380 mg, yield: 95%).

LCMS m/z = 727.4 $[M+H]^+$.

Step 3: 5-(9-((1-(4-((5-bromo-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperidin-4-yl)methyl)-3,9-diazaspiro[5.5]undecan-3-yl)-2-(2,6-dioxopiperidin-3-yl)-6-fluoroisoindoline-1,3-dione (**compound 47**)

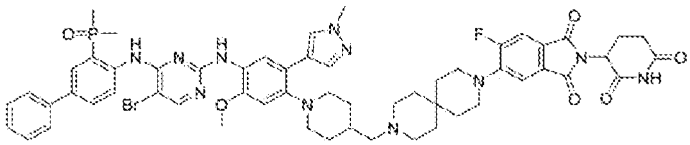

Compound 47

**47B** (200 mg, 0.28 mmol) and **23D** (148 mg, 0.34 mmol) were dissolved in DMF (10 mL); p-toluenesulfonic acid monohydrate (160 mg, 0.84 mmol) was added; and under nitrogen protection, the mixture was stirred at 100°C for 16 h, and cooled to room temperature. 10 mL of saturated aqueous sodium bicarbonate solution and 20 mL of dichloromethane were added, and the layers were separated. The organic layer was concentrated under reduced pressure, and the residue was purified by silica gel column chromatography to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 ml). Liquid separation was performed. The organic

layer was then dried over anhydrous sodium sulphate and concentrated under reduced pressure to obtain **compound 47** (48 mg, yield: 15%).

LCMS m/z = 563.8 [(M+2H)/2]$^+$.

$^1$H NMR (400 MHz, DMSO-$d_6$) δ 11.12 (s, 1H), 11.08 (s, 1H), 8.44 - 8.32 (m, 1H), 8.29 (s, 1H), 8.18 (s, 1H), 8.01 (s, 1H), 7.86 (s, 1H), 7.75 - 7.64 (m, 2H), 7.60 - 7.50 (m, 3H), 7.49 - 7.39 (m, 3H), 7.39 - 7.30 (m, 1H), 7.27 - 7.10 (m, 1H), 6.85 (s, 1H), 5.10 (dd, 1H), 3.79 (s, 3H), 3.76 (s, 3H), 3.28 - 3.19 (m, 4H), 3.14 - 2.99 (m, 2H), 2.95 - 2.82 (m, 1H), 2.68 - 2.53 (m, 4H), 2.42 - 2.30 (m, 4H), 2.28 - 2.16 (m, 2H), 2.08 - 1.97 (m, 1H), 1.83 (d, 6H), 1.77 - 1.65 (m, 2H), 1.65 - 1.44 (m, 9H), 1.34-1.23 (m, 2H).

**Example 48: Preparation of compound 48**

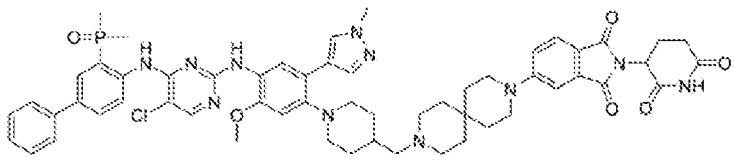

Compound 48

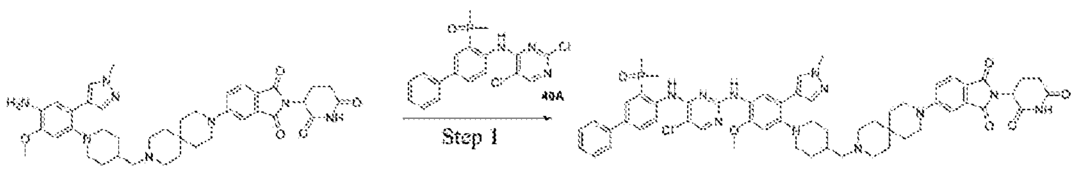


37F

Compound 48

Step 1: 5-(9-((1-(4-((5-chloro-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperidin-4-yl)methyl)-3,9-diazaspiro[5.5]undecan-3-yl)-2-(2,6-dioxopiperidin-3-yl)isoindoline-1,3-dione (**compound 48**)

**37F** (235 mg, 0.33 mmol) and **40A** (157 mg, 0.4 mmol) were dissolved in DMF (10 mL); p-toluenesulfonic acid monohydrate (188 mg, 0.99 mmol) was added; and under nitrogen protection, the mixture was stirred at 100°C for 16 h, and cooled to room temperature. 10 mL of saturated aqueous sodium bicarbonate solution and 20 mL of dichloromethane were added, and the layers were separated. The organic layer was concentrated under reduced pressure, and the residue was purified by silica gel column chromatography to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane, and saturated sodium bicarbonate solution (50 ml) was added. Liquid separation was performed. The organic layer was then dried over anhydrous sodium sulphate and concentrated under reduced pressure to obtain **compound 48** (82 mg, yield: 23%).

LCMS m/z = 1064.4 [M+H]$^+$.

$^1$H NMR (400 MHz, DMSO-$d_6$) δ 11.37 (s, 1H), 11.05 (s, 1H), 8.49 (s, 1H), 8.28 (s, 1H), 8.11 (s, 1H), 8.03 (s, 1H), 7.87 (s, 1H), 7.71 (dd, 1H), 7.65 (d, J = 8.5 Hz, 1H), 7.62 - 7.50 (m, 3H), 7.44 (t, 2H), 7.39 - 7.31 (m, 1H), 7.33 - 7.26 (m, 1H), 7.26 - 7.12 (m, 2H), 6.85 (s, 1H), 5.06 (dd, 1H), 3.79 (s, 3H), 3.76 (s, 3H), 3.55 - 3.40 (m, 4H), 3.07 (d, 2H), 2.98 - 2.81 (m, 1H), 2.70 - 2.53 (m, 4H), 2.36 (s, 4H), 2.29 - 2.17 (m, 2H), 2.07 - 1.95 (m, 1H), 1.84 (d, 6H), 1.72 (d, 2H), 1.64 - 1.45 (m, 9H), 1.38 - 1.20 (m, 3H).

**Example 49: Preparation of compound 49**

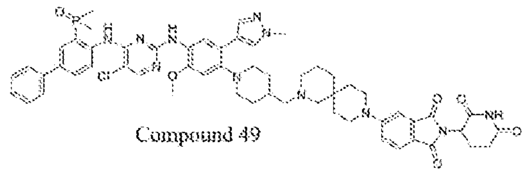

Compound 49

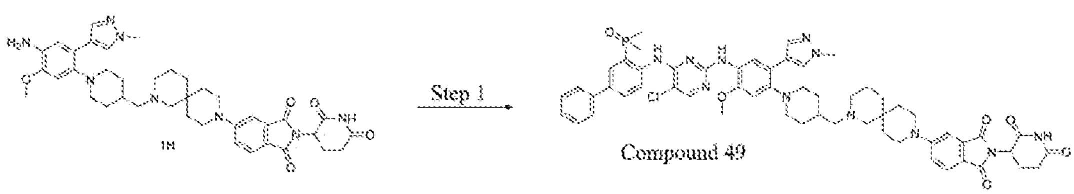


1H

Compound 49

Step 1: 5-(2-((1-(4-((5-chloro-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperidin-4-yl)methyl)-2,9-diazaspiro[5.5]undecan-9-yl)-2-(2,6-dioxopiperidin-3-yl)isoindoline-1,3-dione (**compound 49**)

**1H** (142 mg, 0.2 mmol) and (4-((2,5-dichloropyrimidin-4-yl)amino)-[1,1'-biphenyl]-3-yl)dimethylphosphine oxide (**40A**) (90 mg, 0.23 mmol) were dissolved in DMF (5 mL); p-toluenesulfonic acid monohydrate (0.16 g, 0.84 mmol) was added; and under nitrogen protection, the mixture was stirred at 100°C for 16 h, and cooled to room temperature. 5 mL of water and 5 mL of saturated aqueous sodium bicarbonate solution were added, and the mixture was filtered. The filter cake was dried under reduced pressure and then purified by silica gel column chromatography (dichloromethane/methanol (V/V) = 100/1-12/1) to obtain a product, which was further purified by preparative HPLC (composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 ml). Liquid separation was performed. The organic layer was then dried over anhydrous sodium sulphate and concentrated under reduced pressure to obtain **compound 49** (50 mg, yield: 23.5%).

LCMS m/z = 532.8 $[(M+2H)/2]^+$.

$^1$H NMR (400 MHz, DMSO-$d_6$) δ 11.37 (s, 1H), 11.05 (s, 1H), 8.56 - 8.40 (m, 1H), 8.28 (s, 1H), 8.11 (s, 1H), 8.02 (s, 1H), 7.88 (s, 1H), 7.70 (d, 1H), 7.65 - 7.49 (m, 4H), 7.42 (t, 2H), 7.35 - 7.24 (m, 2H), 7.22 - 7.07 (m, 2H), 6.84 (s, 1H), 5.05 (dd, 1H), 3.77 (s, 3H), 3.75 (s, 3H), 3.58 - 3.35 (m, 4H), 3.14 - 3.02 (m, 2H), 2.94 - 2.82 (m, 1H), 2.73 - 2.52 (m, 4H), 2.42 - 2.11 (m, 6H), 2.05 - 1.93 (m, 1H), 1.84 (d, 6H), 1.78 - 1.46 (m, 9H), 1.45 - 1.17 (m, 4H).

**Example 50: Preparation of compound 50**

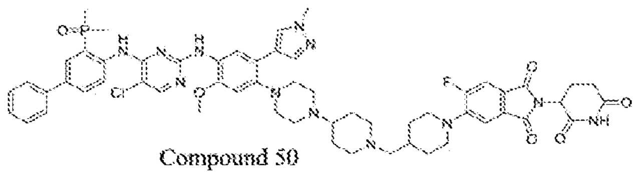

Compound 50

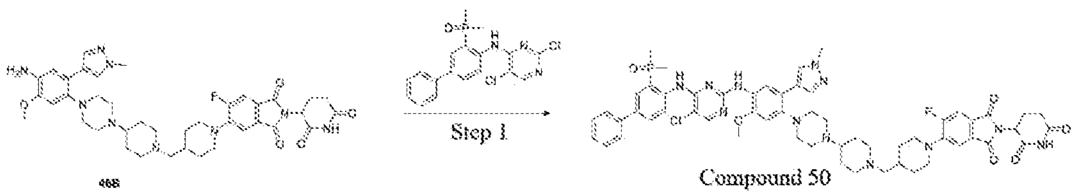


46B

Compound 50

Step 1: 5-(4-((4-(4-(4-((5-chloro-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperazin-1-yl)piperidin-1-yl)methyl)piperidin-1-yl)-2-(2,6-dioxopiperidin-3-yl)-6-fluoroisoindoline-1,3-dione (**compound 50**)

**46B** (0.15 g, 0.20 mmol) and **40A** (0.13 g, 0.33 mmol) were dissolved in DMF (5 mL); p-toluenesulfonic acid

monohydrate (0.16 g, 0.84 mmol) was added; and under nitrogen protection, the mixture was stirred at 100°C for 16 h, and cooled to room temperature. 5 mL of water and 5 mL of saturated aqueous sodium bicarbonate solution were added, and the mixture was filtered. The filter cake was dried under reduced pressure and then purified by silica gel column chromatography (dichloromethane/methanol (V/V) = 100/1-12/1) to obtain a product, which was further purified by preparative HPLC (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 ml). Liquid separation was performed. The organic layer was then dried over anhydrous sodium sulphate and concentrated under reduced pressure to obtain **compound 50** (30 mg, yield: 14%).

LCMS m/z = 549.4 [(M/2+H)]$^+$.

$^1$H NMR (400 MHz, DMSO-$d_6$) δ 11.37 (s, 1H), 11.08 (s, 1H), 8.54 - 8.44 (m, 1H), 8.26 (s, 1H), 8.11 (s, 1H), 8.03 (s, 1H), 7.88 (s, 1H), 7.75 - 7.66 (m, 2H), 7.61 (s, 1H), 7.58 - 7.51 (m, 2H), 7.50 - 7.40 (m, 3H), 7.40 - 7.33 (m, 1H), 7.21 - 7.09 (m, 1H), 6.86 (d, 1H), 5.10 (dd, 1H), 3.80 (s, 3H), 3.75 (s, 3H), 3.67 - 3.55 (m, 2H), 3.00 - 2.77 (m, 10H), 2.70 - 2.52 (m, 6H), 2.18 - 2.11 (m, 2H), 2.06 - 1.99 (m, 1H), 1.96 - 1.65 (m, 13H), 1.50 - 1.35 (m, 2H), 1.31 - 1.19 (m, 2H).

**Example 51: Preparation of compound 51**

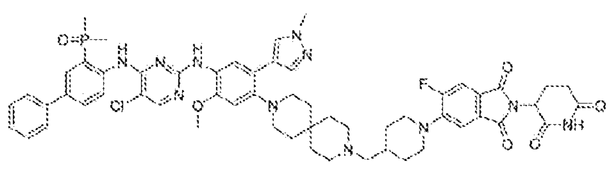

Compound 51

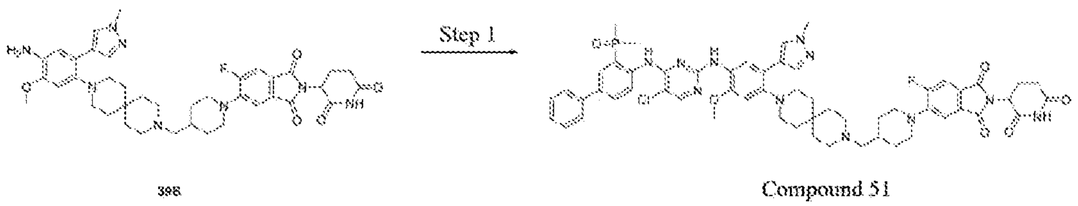


39B

Compound 51

Step 1: 5-(4-((9-(4-((5-chloro-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)-3,9-diazaspiro[5.5]undecan-3-yl)methyl)piperidin-1-yl)-2-(2,6-dioxopiperidin-3-yl)-6-fluoroisoindoline-1,3-dione (**compound 51**)

**39B** (100 mg, 0.138 mmol) and (4-((2,5-dichloropyrimidin-4-yl)amino)-[1,1'-biphenyl]-3-yl)dimethylphosphine oxide **(40A)** (56 mg, 0.143 mmol) were dissolved in DMF (10 mL); p-toluenesulfonic acid monohydrate (79 mg, 0.413 mmol) was added; and under nitrogen protection, the mixture was stirred at 100°C for 16 h, and cooled to room temperature. 20 mL of saturated aqueous sodium bicarbonate solution and 50 mL of dichloromethane were added, and the layers were separated. The organic layer was concentrated under reduced pressure, and the residue was purified by silica gel column chromatography (mobile phase: dichloromethane/methanol (V/V) = 100/1-20/1) to obtain the crude. The resulting crude was separated and purified by preparative liquid phase chromatography to obtain **compound 51** (18 mg, yield: 12%).

LCMS m/z = 541.8 [(M+2H)/2]$^+$.

$^1$H NMR (400 MHz, DMSO-$d_6$) δ 11.44 - 11.32 (m, 1H), 11.21 - 10.93 (m, 1H), 8.54 - 8.39 (m, 1H), 8.28 (s, 1H), 8.10 (s, 1H), 8.03 (s, 1H), 7.88 (s, 1H), 7.75 - 7.63 (m, 2H), 7.61 - 7.50 (m, 3H), 7.49 - 7.40 (m, 3H), 7.39 - 7.30 (m, 1H), 7.28 - 7.13 (m, 1H), 6.89 (s, 1H), 5.10 (dd, 1H), 3.80 (s, 3H), 3.76 (s, 3H), 3.65 - 3.56 (m, 2H), 2.95 - 2.74 (m, 7H), 2.64 - 2.52 (m, 2H), 2.37 - 2.26 (m, 4H), 2.22 - 2.12 (m, 2H), 2.09 - 1.98 (m, 1H), 1.87 - 1.77 (m, 8H), 1.76 - 1.68 (m, 1H), 1.59 - 1.45 (m, 8H), 1.31 - 1.22 (m, 2H).

**Example 52: Preparation of compound 52**

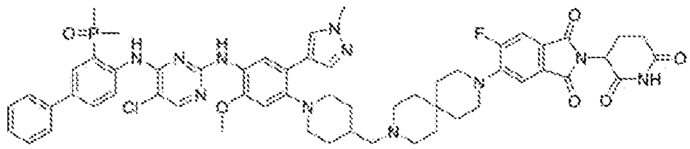

Compound 52

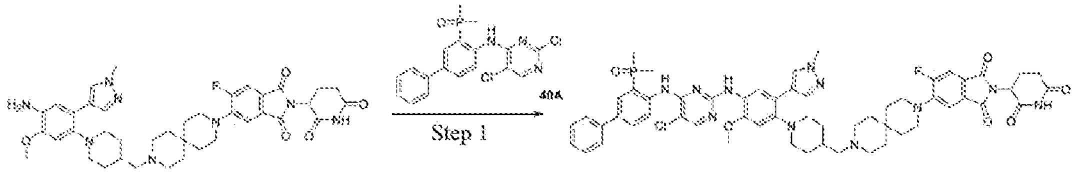


47B

Compound 52

Step 1: 5-(9-((1-(4-((5-chloro-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperidin-4-yl)methyl)-3,9-diazaspiro[5.5]undecan-3-yl)-2-(2,6-dioxopiperidin-3-yl)-6-fluoroisoindoline-1,3-dione (**compound 52**)

**47B** (200 mg, 0.28 mmol) and **40A** (133 mg, 0.34 mmol) were dissolved in DMF (10 mL); p-toluenesulfonic acid monohydrate (160 mg, 0.84 mmol) was added; and under nitrogen protection, the mixture was stirred at 100°C for 16 h, and cooled to room temperature. 10 mL of saturated aqueous sodium bicarbonate solution and 20 mL of dichloromethane were added, and the layers were separated. The organic layer was concentrated under reduced pressure, and the residue was purified by silica gel column chromatography to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane, and saturated sodium bicarbonate solution (50 ml) was added. Liquid separation was performed. The organic layer was then dried over anhydrous sodium sulphate and concentrated under reduced pressure to obtain **compound 52** (56 mg, yield: 18%).

LCMS m/z = 1082.4 $[M+H]^+$.

$^1H$ NMR (400 MHz, DMSO-$d_6$) δ 11.37 (s, 1H), 11.08 (s, 1H), 8.57 - 8.39 (m, 1H), 8.28 (s, 1H), 8.11 (s, 1H), 8.03 (s, 1H), 7.87 (s, 1H), 7.76 - 7.64 (m, 2H), 7.63 - 7.50 (m, 3H), 7.50 - 7.40 (m, 3H), 7.40 - 7.32 (m, 1H), 7.23 - 7.11 (m, 1H), 6.85 (s, 1H), 5.10 (dd, 1H), 3.79 (s, 3H), 3.76 (s, 3H), 3.28 - 3.16 (m, 4H), 3.13 - 3.03 (m, 2H), 2.95 - 2.82 (m, 1H), 2.68 - 2.52 (m, 4H), 2.44 - 2.29 (m, 4H), 2.29 - 2.17 (m, 2H), 2.08 - 1.98 (m, 1H), 1.84 (d, 6H), 1.79 - 1.68 (m, 2H), 1.63 - 1.47 (m, 9H), 1.35 - 1.25 (m, 3H).

**Example 53: Preparation of compound 53**

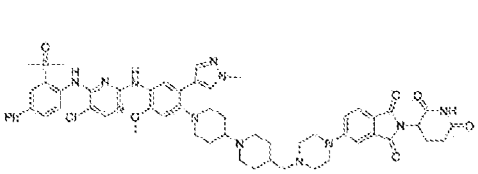

Compound 53

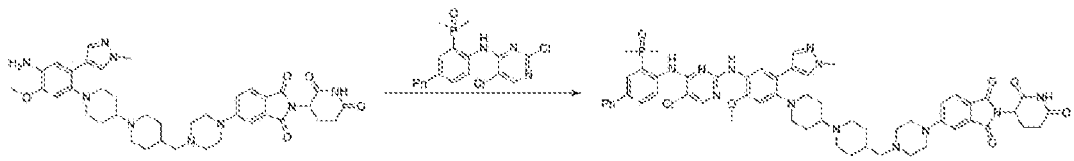

21

Compound 53

Step 1: 5-(4-((1'-(4-((5-chloro-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)-[1,4'-bipiperidin]-4-yl)methyl)piperazin-1-yl)-2-(2,6-dioxopiperidin-3-yl)isoindoline-1,3-dione (**compound 53**)

**81** (180 mg, 0.249 mmol) and **40A** (98 mg, 0.249 mmol) were dissolved in DMF (5 mL); p-toluenesulfonic acid monohydrate (142 mg, 0.747 mmol) was added; and under nitrogen protection, the mixture was reacted overnight at 100°C, and cooled to room temperature. 20 mL of saturated aqueous sodium bicarbonate solution was added, and the mixture was subjected to suction filtration. The filter cake was dissolved in 50 mL of dichloromethane and extracted. The organic phase was dried over anhydrous sodium sulphate and then concentrated under reduced pressure, and the residue was purified by silica gel column chromatography (dichloromethane/methanol (V/V) = 100/1-10/1) to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)) to obtain the trifluoroacetate of **compound 53** (160 mg). 20 mL of dichloromethane and 50 mL of saturated sodium bicarbonate solution were added to the trifluoroacetate of **compound 53,** and the layers were separated. The organic phase was dried over anhydrous sodium sulphate and then concentrated under reduced pressure to obtain **compound 53** (120 mg, yield: 45%).

LCMS m/z = 1079.5 $[M+H]^+$.

$^1H$ NMR (400 MHz, DMSO-$d_6$) δ 11.37 (s, 1H), 11.05 (s, 1H), 8.52 - 8.45 (m, 1H), 8.26 (s, 1H), 8.11 (s, 1H), 8.02 (s, 1H), 7.87 (s, 1H), 7.74 - 7.66 (m, 2H), 7.61 - 7.53 (m, 3H), 7.48 - 7.43 (m, 2H), 7.39 - 7.36 (m, 1H), 7.35 - 7.33 (m, 1H), 7.25 (dd, 1H), 7.20 - 7.14 (m, 1H), 6.83 (s, 1H), 5.07 (dd, 1H), 3.77 (d, 6H), 3.48 - 3.40 (m, 4H), 3.14 - 3.09 (m, 2H), 2.99 - 2.91 (m, 2H), 2.63 - 2.55 (m, 3H), 2.49 - 2.46 (m, 3H), 2.21 - 2.13 (m, 4H), 2.03 - 1.96 (m, 2H), 1.84 (d, 6H), 1.80 - 1.72 (m, 4H), 1.66 - 1.42 (m, 6H), 1.17 - 1.13 (m, 2H).

**Example 54: Preparation of compound 54**

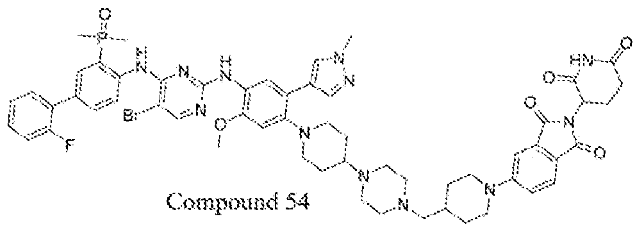

Compound 54

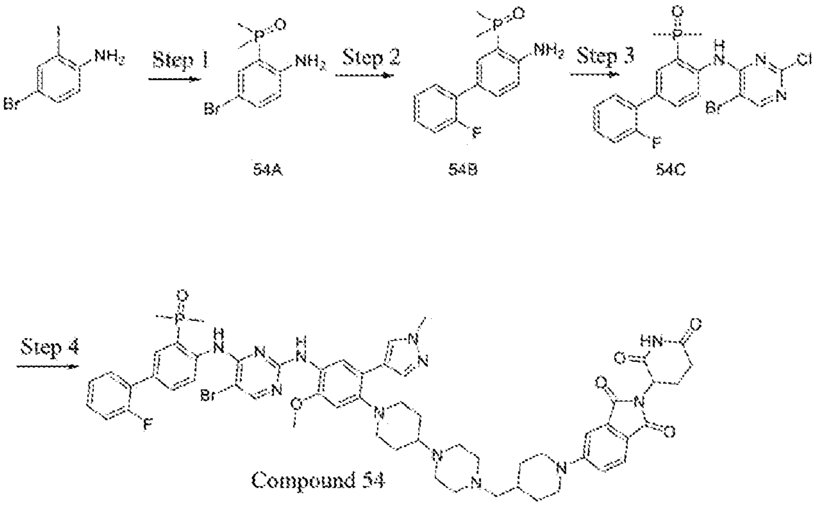


54A

54B

54C

Compound 54

Step 1: (2-amino-5-bromophenyl)dimethylphosphine oxide (**54A**)

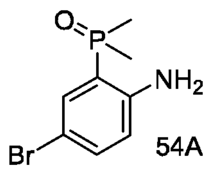

54A

4-bromo-2-iodoaniline (0.5 g, 1.68 mmol), dimethylphosphine oxide (0.13 g, 1.68 mmol), Xant-Phos (4,5-bisdiphenylphosphine-9,9-dimethylxanthene, 49 mg, 0.084 mmol) and anhydrous potassium phosphate (0.46 g, 2.18 mmol) were successively added to 10 mL of 1,4-dioxane, and palladium acetate (19 mg, 0.084 mmol) was added under stirring. The mixture was subjected to nitrogen replacement three times, stirred at 80°C for 5 h, cooled to room temperature and filtered. The filter cake was washed with 20 mL of ethyl acetate. The filtrates were combined, and concentrated under reduced pressure. The residue was slurried by adding 5 mL of methyl tert-butyl ether and filtered. The filter cake was dried to obtain **54A** (0.3 g, yield: 72%).

LCMS m/z = 248.1 $[M+H]^+$.

Step 2: (4-amino-2'-fluoro-[1,1'-biphenyl]-3-yl)dimethylphosphine oxide (**54B**)

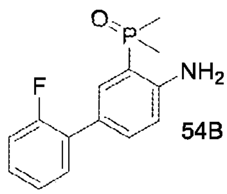

54B

**54A** (0.3 g, 1.21 mmol) and 2-fluorophenylboronic acid (0.34 g, 2.42 mmol) were dissolved in 10 mL of dioxane and 2 mL of water, and then $Pd(dppf)Cl_2 \cdot DCM$ (44 mg, 0.06 mmol) and potassium carbonate (670 mg, 4.84 mmol) were added. The mixture was subjected to nitrogen replacement three times, reacted at 100°C for 2 h and cooled to room temperature. The reaction solution was poured into water and extracted three times with ethyl acetate. The organic phases were combined, dried over anhydrous sodium sulphate and concentrated under reduced pressure, and the residue was purified by silica gel column chromatography to obtain **54B** (140 mg, yield: 44%).

LCMS m/z = 264.1$[M+H]^+$.

Step 3: (4-((5-bromo-2-chloropyrimidin-4-yl)amino)-2'-fluoro-[1,1'-biphenyl]-3-yl)dimethylphosphine oxide (**54C**)

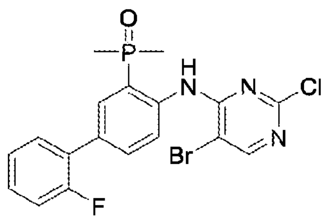

54C

54B (0.14 g, 0.53 mmol) and 5-bromo-2,4,-dichloropyrimidine (0.24 g, 1.06 mmol) were dissolved in 5 mL of NMP; DIPEA (82 mg, 0.64 mmol) was added; and the mixture was stirred at 120°C for 2 h and cooled to room temperature. 8 mL of water was added, and the mixture was filtered. The filter cake was dried under reduced pressure and then purified by silica gel column chromatography (petroleum ether: ethyl acetate (V/V) = 10/1-1/10) to obtain **54C** (0.16 g, yield: 66%).

$^1H$ NMR (400 MHz, $CDCl_3$) $\delta$ 11.47 (s, 1H), 8.72-8.66 (m, 1H), 8.36 (s, 1H), 7.75 (d, 1H), 7.52 - 7.46 (m, 1H), 7.46 -7.40 (m, 1H), 7.39 - 7.33 (m, 1H), 7.28 - 7.13 (m, 2H), 1.88 (d, 6H).

Step 4: 5-(4-((4-(1-(4-((5-bromo-4-((3-(dimethylphosphoryl)-2'-fluoro-[1,1'-biphenyl]-4-yl)amino)pyrimidin-2-yl)amino)-5-methoxy-2-(1-methyl-1H-pyrazol-4-yl)phenyl)piperidin-4-yl)piperazin-1-yl)methyl)piperidin-1-yl)-2-(2,6-dioxopiperidin-3-yl)isoindoline-1,3-dione (**compound 54**)

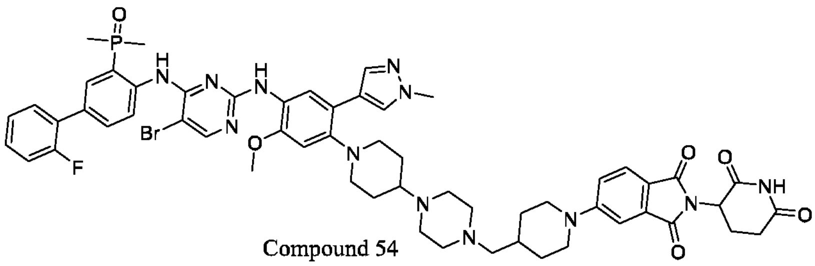

Compound 54

**4D** (250 mg, 0.35 mmol) and **54B** (190 mg, 0.42 mmol) were dissolved in a mixed solution of 1,4-dioxane (4 mL) and NMP (1 mL); p-toluenesulfonic acid monohydrate (200 mg, 1.05 mmol) was added; and under nitrogen protection, the mixture was reacted overnight at 100°C, and cooled to room temperature. 20 mL of saturated aqueous sodium bicarbonate solution was added, and the mixture was subjected to suction filtration. The filter cake was dissolved in 50 mL of dichloromethane and extracted. The organic phase was dried over anhydrous sodium sulphate and then concentrated under reduced pressure, and the residue was purified by silica gel column chromatography (dichloromethane/methanol (V/V) = 100/1-10/1) to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)) to obtain the trifluoroacetate of **compound 54.** 20 mL of dichloromethane and 10 mL of saturated sodium bicarbonate solution were added to the trifluoroacetate of **compound 54,** and the layers were separated. The organic phase was dried over anhydrous sodium sulphate and then concentrated under reduced pressure to obtain **compound 54** (120 mg, yield: 30%).

LCMS m/z = 571.3 $[(M+2H)/2]^+$.

$^1H$ NMR (400 MHz, DMSO-$d_6$) δ 11.13 (s, 1H), 11.05 (s, 1H), 8.47 - 8.37 (m, 1H), 8.32 (s, 1H), 8.19 (s, 1H), 7.98 (s, 1H), 7.82 (s, 1H), 7.71 - 7.57 (m, 2H), 7.53 (s, 1H), 7.48 - 7.37 (m, 2H), 7.35 - 7.02 (m, 5H), 6.80 (s, 1H), 5.06 (dd, 1H), 4.12 - 3.95 (m, 2H), 3.77 (s, 3H), 3.76 (s, 3H), 3.15 - 3.04 (m, 2H), 3.03 - 2.92 (m, 2H), 2.92 - 2.82 (m, 1H), 2.68 - 2.51 (m, 8H), 2.46-2.26 (m, 4H), 2.22 - 2.09 (m, 3H), 2.05 - 1.94 (m, 1H), 1.91 - 1.68 (m, 11H), 1.59 - 1.43 (m, 2H), 1.21 - 1.08 (m, 2H).

**Example 55: Preparation of compound 55**

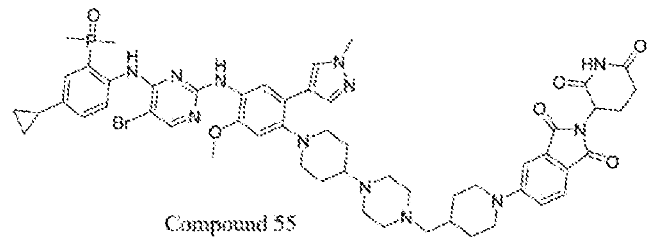

Compound 55

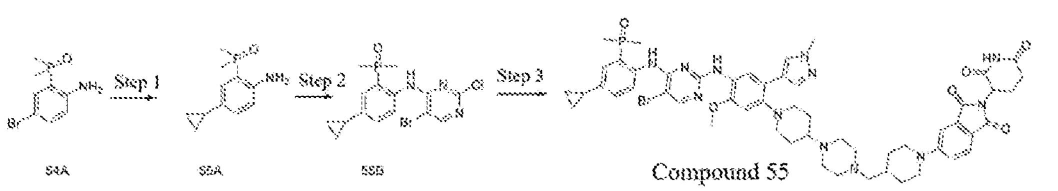


Step 1: (2-amino-5-cyclopropylphenyl)dimethylphosphine oxide (**55A**)

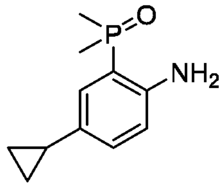

55A

**54A** (2.3 g, 9.27 mmol) and cyclopropyl boronic acid (2.39 g, 27.81 mmol) were dissolved in 20 mL of dioxane and 4 mL of water, and then tricyclohexylphosphine (0.52 g, 1.85 mmol), palladium acetate (0.21 g, 0.93 mmol) and potassium phosphate (7.87 g, 37.08 mmol) were added. The mixture was subjected to nitrogen replacement three times, reacted at 100°C for 24 h and cooled to room temperature. The reaction solution was poured into water and extracted three times with ethyl acetate. The organic phases were combined, dried over anhydrous sodium sulphate and concentrated under reduced pressure, and the residue was purified by silica gel column chromatography to obtain **55A** (1.26 g, yield: 65%).

$^1H$ NMR (400 MHz, $CDCl_3$) $\delta$ 6.94 (d, 1H), 6.88-6.80 (m, 1H), 6.63-6.57 (m, 1H), 1.82-1.71 (m, 7H), 0.91 - 0.83 (m, 2H), 0.59 - 0.51 (m, 2H).

Step 2: (2-((5-bromo-2-chloropyrimidin-4-yl)amino)-5-cyclopropylphenyl) dimethylphosphine oxide (**55B**)

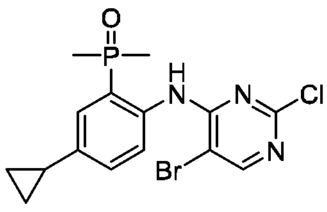

55B

Compound 55A (0.26 g, 1.24 mmol) and 5-bromo-2,4,-dichloropyrimidine (0.57 g, 2.48 mmol) were dissolved in 5 mL of NMP; DIPEA (190 mg, 1.49 mmol) was added; and the mixture was stirred at 120°C for 2 h and cooled to room temperature. 8 mL of water was added, and the mixture was filtered. The filter cake was dried under reduced pressure and then purified by silica gel column chromatography (petroleum ether: ethyl acetate (V/V) = 10/1-0/10). The residue was slurried with a mixed solvent of ethyl acetate/petroleum ether (10 mL, V/V = 1/2) and filtered. The filter cake was dried under reduced pressure to obtain **55B** (0.28 g, yield: 56%).

$^1H$ NMR (400 MHz, $CDCl_3$) $\delta$ 11.20 (s, 1H), 8.45-8.39 (m, 1H), 8.30 (s, 1H), 7.24-7.19 (m, 1H), 7.06-7.00 (m, 1H), 1.93 - 1.79(m, 7H), 1.05 - 0.96 (m, 2H), 0.78 - 0.65 (m, 2H).

Step 3: 5-(4-((4-(1-(4-((5-bromo-4-((4-cyclopropyl-2-(dimethylphosphoryl) phenyl)amino)pyrimidin-2-yl)amino)-5-methoxy-2-(1-methyl-1H-pyrazol-4-yl)phenyl)piperidin-4-yl)piperazin-1-yl)methyl)piperidin-1-yl)-2-(2,6-dioxopiperidin-3-yl)isoindoline-1,3-dione (**compound 55**)

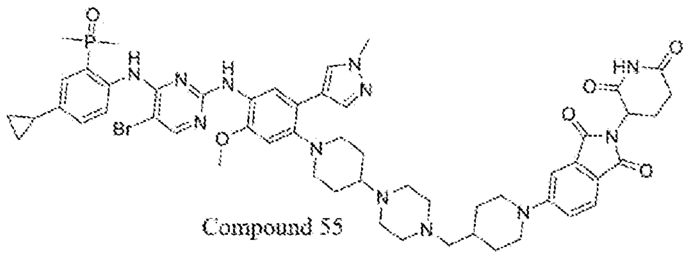

Compound 55

**4D** (200 mg, 0.28 mmol) and **55B** (130 mg, 0.33 mmol) were dissolved in DMF (4 mL); p-toluenesulfonic acid monohydrate (160 mg, 0.84 mmol) was added; and under nitrogen protection, the mixture was reacted overnight at

100°C, and cooled to room temperature. 20 mL of saturated aqueous sodium bicarbonate solution was added, and the mixture was subjected to suction filtration. The filter cake was dissolved in 50 mL of dichloromethane and extracted. The organic phase was dried over anhydrous sodium sulphate and then concentrated under reduced pressure, and the residue was purified by silica gel column chromatography (dichloromethane/methanol (V/V) = 100/1-10/1) to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)) to obtain the trifluoroacetate of **compound** 55. 20 mL of dichloromethane and 10 mL of saturated sodium bicarbonate solution were added to the trifluoroacetate of **compound 55,** and the layers were separated. The organic phase was dried over anhydrous sodium sulphate and then concentrated under reduced pressure to obtain **compound 55** (90 mg, yield: 29%).

LCMS m/z = 544.3 $[(M+2H)/2]^+$.

$^1H$ NMR (400 MHz, DMSO-$d_6$) δ 11.05 (s, 1H), 10.84 (s, 1H), 8.19 - 8.05 (m, 3H), 7.96 (s, 1H), 7.86 (s, 1H), 7.65 (d, 1H), 7.57 (s, 1H), 7.32 - 7.10 (m, 3H), 6.79 (s, 1H), 6.55 - 6.34 (m, 1H), 5.06 (dd, 1H), 4.11 - 3.95 (m, 2H), 3.84 (s, 3H), 3.78 (s, 3H), 3.18 - 3.06 (m, 2H), 3.02 - 2.81 (m, 3H), 2.70 - 2.51 (m, 8H), 2.45 - 2.30 (m, 4H), 2.29 - 2.09 (m, 3H), 2.05 - 1.96 (m, 1H), 1.89 - 1.68 (m, 12H), 1.64 - 1.50 (m, 2H), 1.21 - 1.05 (m, 2H), 0.96 - 0.86 (m, 2H), 0.57 - 0.43 (m, 2H).

**Example 56: Preparation of compound 56**

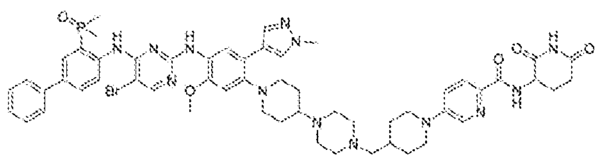

Compound 56

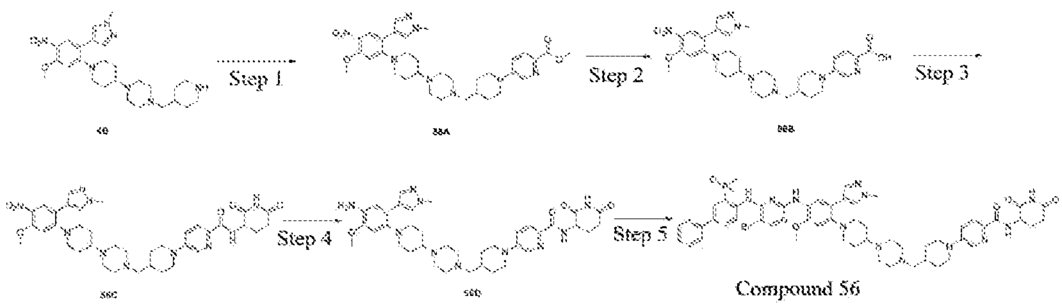


Step 1: Methyl 5-(4-((4-(1-(5-methoxy-2-(1-methyl-1H-pyrazol-4-yl)-4-nitrophenyl)piperidin-4-yl)piperazin-1-yl)methyl)piperidin-1-yl)picolinate (56A)

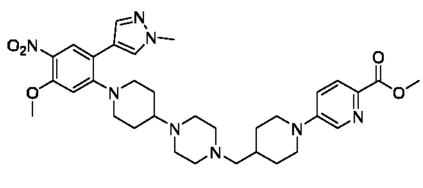

**56A**

**4B** (0.22 g, 0.44 mmol) and methyl 5-fluoropyridine-2-carboxylate (0.08 g, 0.52 mmol) were dissolved in DMSO (10 mL); potassium carbonate (0.15 mg, 1.09 mmol) was added; and the mixture was reacted at 90°C for 3 h. The reaction solution was cooled to room temperature. 40 mL of saturated aqueous sodium bicarbonate solution was added, and the mixture was then extracted by adding 50 ml of ethyl acetate. The organic phase was dried over anhydrous sodium sulphate and concentrated under reduced pressure, and the residue was purified by column chromatography

(mobile phase: dichloromethane/methanol (V/V) = 50/1-12/1) to obtain **56A** (0.18 g, yield: 65%).

LCMS m/z = 633.3 $[M+H]^+$.

Step 2: 5-(4-((4-(1-(5-methoxy-2-(1-methyl-1H-pyrazol-4-yl)-4-nitrophenyl) piperidin-4-yl)piperazin-1-yl)methyl)piperidin-1-yl)picolinic acid (**56B**)

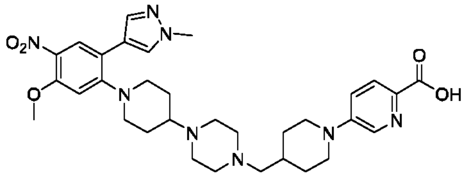

**56B**

**56A** (0.18 g, 0.28 mmol) was dissolved in a mixed solution of tetrahydrofuran (12 mL) and water (4 mL); lithium hydroxide monohydrate (0.05 g, 1.19 mmol) was added; and the mixture was reacted at room temperature for 4 h. After the reaction was completed, the reaction solution was adjusted to pH = 7 by adding dilute hydrochloric acid (2 mol/L) and concentrated under reduced pressure to obtain the crude of **56B** (0.18 g), which was directly used in the next step.

LCMS m/z = 619.3 $[M+H]^+$.

Step 3: N-(2,6-dioxopiperidin-3-yl)-5-(4-((4-(1-(5-methoxy-2-(1-methyl-1H-pyrazol-4-yl)-4-nitrophenyl)piperidin-4-yl)piperazin-1-yl)methyl)piperidin-1-yl)picolinamide (**56C**)

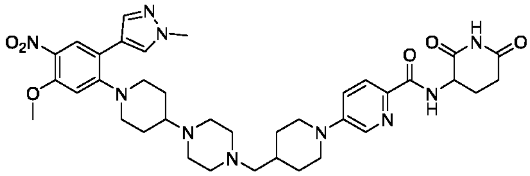

**56C**

**56B** (0.18 g) and 3-aminopiperidine-2,6-dione hydrochloride (0.06 g, 0.36 mmol) were dissolved in DMF (10 mL); DIPEA (0.10 g, 0.77 mmol) and HATU (0.14 g, 0.37 mmol) were added; and the mixture was reacted at room temperature for 3 h. After the reaction was completed, 40 mL of saturated aqueous sodium bicarbonate solution was added, and the mixture was then extracted by adding 50 ml of ethyl acetate. The organic phase was dried over anhydrous sodium sulphate and concentrated under reduced pressure, and the residue was purified by column chromatography (mobile phase: dichloromethane/methanol (V/V) = 50/1-10/1) to obtain **56C** (0.15 g, two-step yield: 74%).

LCMS m/z = 729.4 $[M+H]^+$.

Step 4: 5-(4-((4-(1-(4-amino-5-methoxy-2-(1-methyl-1H-pyrazol-4-yl)phenyl) piperidin-4-yl)piperazin-1-yl)methyl)piperidin-1-yl)-N-(2,6-dioxopiperidin-3-yl)picolinamide (**56D**)

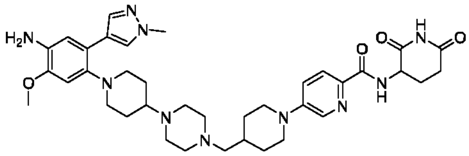

**56D**

**56C** (0.15 g, 0.21 mmol) was dissolved in ethanol (9 mL) and water (3 mL); iron powder (0.04 g, 0.72 mmol) was added, and then ammonium chloride (0.04 g, 0.75 mmol) was added dropwise; and under nitrogen protection, the mixture was reacted at 80°C for 3 h. The reaction solution was cooled to room temperature and concentrated under reduced pressure. 20 ml of saturated aqueous sodium bicarbonate solution was added, and the mixture was extracted twice with dichloromethane (20 ml). The organic phases were combined, dried over anhydrous sodium sulphate and then concentrated under reduced pressure to obtain the crude of **56D** as a white solid (0.12 g), which was directly used in the next step.

LCMS m/z = 699.4 $[M+H]^+$.

Step 5: 5-(4-((4-(1-(4-((5-bromo-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperidin-4-yl)piperazin-1-yl)methyl)piperidin-1-yl)-N-(2,6-dioxopiperidin-3-yl)picolinamide (**compound 56**)

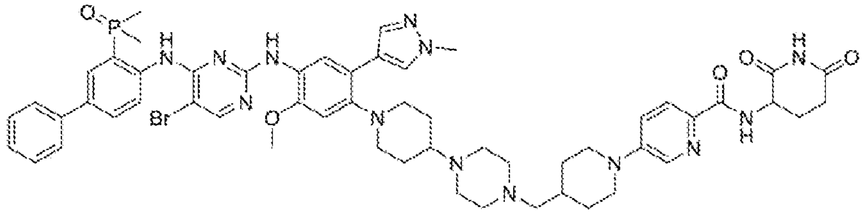

Compound 56

**56D** (0.12 g) and **23D** (0.08 g, 0.18 mmol) were dissolved in DMF (10 mL); p-toluenesulfonic acid monohydrate (0.09 g, 0.47 mmol) was added; and under nitrogen protection, the mixture was reacted at 100°C for 16 h. The reaction solution was cooled to room temperature. 5 mL of water and 5 mL of saturated aqueous sodium bicarbonate solution were added, and the mixture was filtered. The filter cake was dried under reduced pressure and then purified by silica gel column chromatography (dichloromethane/methanol (V/V) = 100/1-10/1) to obtain a product, which was further purified by preparative HPLC (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 ml). Liquid separation was performed. The organic layer was then dried over anhydrous sodium sulphate and concentrated under reduced pressure to obtain **compound 56** (8 mg, yield: 3%).

LCMS m/z = 549.8 $[(M+2H)/2]^+$.

$^1H$ NMR (400 MHz, DMSO-$d_6$) δ 11.11 (s, 1H), 10.82 (s, 1H), 8.68 (d, 1H), 8.39 (s, 1H), 8.31 - 8.25 (m, 2H), 8.18 (s, 1H), 8.00 (s, 1H), 7.87 - 7.80 (m, 2H), 7.70 (dd, 1H), 7.60 - 7.51 (m, 3H), 7.49 - 7.33 (m, 4H), 7.23 - 7.14 (m, 1H), 6.83 (s, 1H), 4.82 - 4.65 (m, 1H), 3.93 (d, 2H), 3.79 (s, 3H), 3.76 (s, 3H), 3.10 (d, 2H), 2.86 (t, 2H), 2.80 - 2.73 (m, 1H), 2.64 - 2.52 (m, 6H), 2.45 - 2.32 (m, 4H), 2.23 - 2.12 (m, 4H), 2.04 - 1.97 (m, 1H), 1.89 - 1.75 (m, 10H), 1.60 - 1.48 (m, 3H), 1.22 - 1.18 (m, 3H).

**Example 57: Preparation of compound 57**

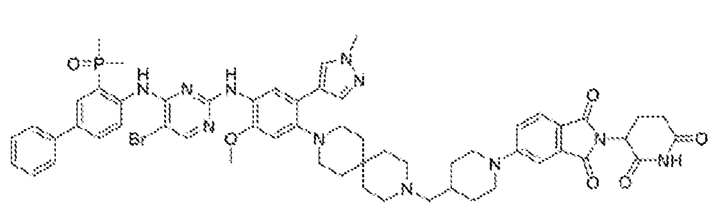

Compound 57

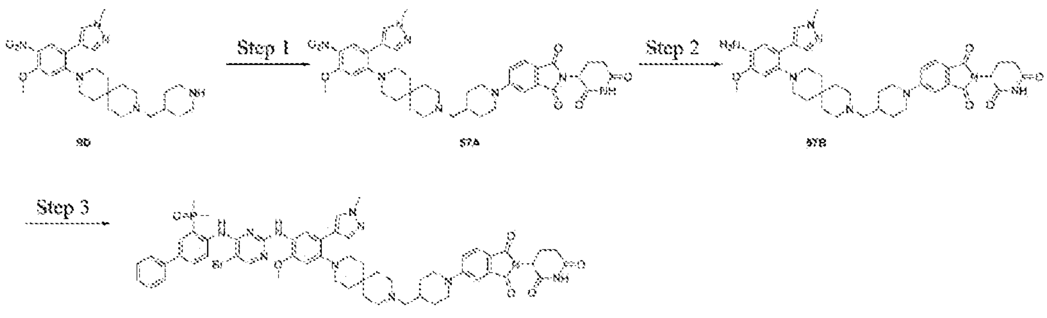


Compound 57

Step 1: 2-(2,6-dioxopiperidin-3-yl)-5-(4-((9-(5-methoxy-2-(1-methyl-1H-pyrazol-4-yl)-4-nitrophenyl)-3,9-diazaspiro[5.5]undecan-3-yl)methyl)piperidin-1-yl)isoindoline-1,3-dione (**57A**)

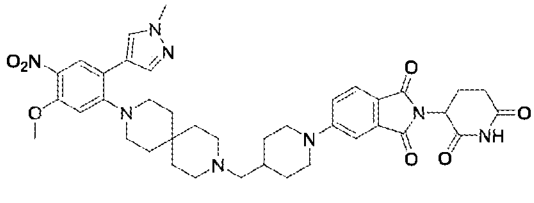

**57A**

The hydrochloride of compound **9D** (820 mg) and 2-(2,6-dioxopiperidin-3-yl)-5-fluoroisoindole-1,3-dione (523 mg, 1.9 mmol) were dissolved in DMSO (20 mL); N,N-diisopropylethylamine (612 mg, 4.74 mmol) was added; and the mixture was reacted at 90°C for 6 h, cooled to room temperature and extracted by adding 60 mL of ethyl acetate and 60 mL of water. The organic layer was washed three times with saturated brine (20 mL $\times$ 3), dried over anhydrous sodium sulphate and concentrated under reduced pressure, and the residue was purified by column chromatography (dichloromethane: methanol (V/V) = 100/1-15/1) to obtain **57A** (0.39 g, yield: 33%).

LCMS m/z = 739.4[M+H]$^+$.

Step 2: 5-(4-((9-(4-amino-5-methoxy-2-(1-methyl-1H-pyrazol-4-yl)phenyl)-3,9-diazaspiro[5.5]undecan-3-yl)methyl)piperidin-1-yl)-2-(2,6-dioxopiperidin-3-yl)isoindoline-1,3-dione (**57B**)

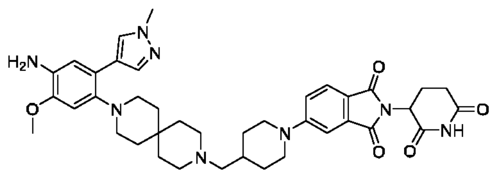

**57B**

**57A** (390 mg, 0.53 mmol) was dissolved in methanol/water (8 mL, 3:1); iron powder (390 mg, 6.98 mmol) and ammonium chloride (390 mg, 7.29 mmol) were successively added; and under nitrogen protection, the mixture was stirred at 80°C for 1 h, cooled to room temperature and subjected to suction filtration under reduced pressure. The filtrate was concentrated under reduced pressure, and then the crude was purified by silica gel column chromatography (mobile phase: dichloromethane/methanol (V/V) = 100/1-20/1) to obtain **57B** (270 mg, yield: 72%).

LCMS m/z = 709.3 [M+H]$^+$.

Step 3: 5-(4-((9-(4-((5-bromo-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)-3,9-diazaspiro[5.5]undecan-3-yl)methyl)piperidin-1-yl)-2-(2,6-dioxopiperidin-3-yl)isoindoline-1,3-dione (**compound 57**)

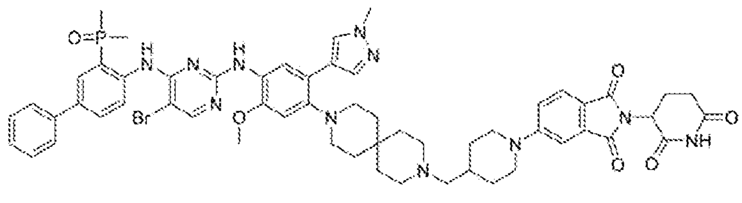

Compound 57

**57B** (134 mg, 0.19 mmol) and **23D** (90 mg, 0.21 mmol) were dissolved in DMF (10 mL); p-toluenesulfonic acid monohydrate (119.8 mg, 0.63 mmol) was added; and under nitrogen protection, the mixture was stirred at 100°C for 16 h, and cooled to room temperature. 20 mL of saturated aqueous sodium bicarbonate solution and 50 mL of dichloromethane were added, and the layers were separated. The organic layer was concentrated under reduced pressure, and the residue was purified by silica gel column chromatography (mobile phase: dichloromethane/methanol (V/V) = 100/1-20/1) to obtain the crude, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 ml). Liquid separation was performed. The organic layer was then dried over anhydrous sodium sulphate and concentrated under reduced pressure to obtain **compound 57** (22 mg, yield: 10.5%).

LCMS m/z = 554.7[$(M+2H)/2]^+$.

$^1$H NMR (400 MHz, DMSO-$d_6$) δ 11.17 - 10.96 (m, 2H), 8.47 - 8.34 (m, 1H), 8.29 (s, 1H), 8.18 (s, 1H), 8.01 (s, 1H), 7.87 (s, 1H), 7.73 - 7.62 (m, 2H), 7.60 - 7.49 (m, 3H), 7.44 (t, 2H), 7.36 (t, 1H), 7.33 - 7.10 (m, 3H), 6.89 (s, 1H), 5.06 (dd, 1H), 4.12 - 3.95 (m, 2H), 3.80 (s, 3H), 3.76 (s, 3H), 3.06 - 2.71 (m, 7H), 2.68 - 2.52 (m, 2H), 2.44 - 2.26 (m, 4H), 2.24 - 2.10 (m, 2H), 2.06 - 1.92 (m, 1H), 1.90 - 1.73 (m, 9H), 1.59 - 1.44 (m, 8H), 1.23 - 1.09 (m, 2H).

**Example 58: Preparation of compound 58**

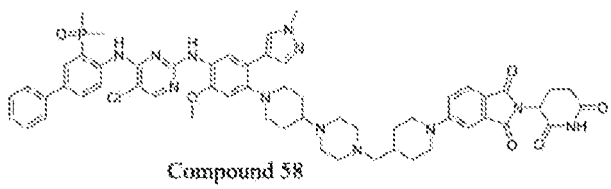

Compound 58

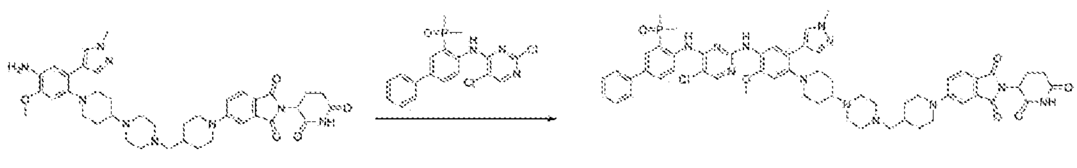

4D

Compound 58

Step 1: 5-(4-((4-(1-(4-((5-chloro-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperidin-4-yl)piperazin-1-yl)methyl)piperidin-1-yl)-2-(2,6-dioxopiperidin-3-yl)isoindoline-1,3-dione; (**compound 58**)

**4D** (87 mg, 0.12 mmol) and **40A** (56 mg, 0.14 mmol) were dissolved in DMF (5 mL); p-toluenesulfonic acid monohydrate (68 mg, 0.36 mmol) was added; and the mixture was stirred at 100°C for 16 h, and cooled to room temperature. 10 mL of saturated aqueous sodium bicarbonate solution was added, with a solid precipitated. Suction

filtration was performed for collecting the solid. The residue was purified sequentially by silica gel column chromatography (mobile phase: DCM/MeOH (V/V) = 100/1-20/1) and preparative HPLC (instrument: waters 2767 (preparative liquid phase chromatographic instrument); chromatographic column: XBridge@ Prep C18 (30 mm × 150 mm); composition of mobile phases: mobile phase A: acetonitrile, and mobile phase B: water (containing 0.1% TFA)) to obtain a preparative solution, which was concentrated, and then 20 mL of dichloromethane and 3 mL of saturated sodium bicarbonate solution were added to the concentrated solution. The organic phases were separated. The aqueous phase was extracted with dichloromethane (10 mL × 2). The organic phases were combined, dried over anhydrous sodium sulphate and concentrated under reduced pressure to obtain **compound 58** (15 mg, yield: 11.6%).

LCMS m/z = 540.3 [(M+2H)/2]$^+$.

$^1$H NMR (400 MHz, DMSO-$d_6$) δ **11.36 (s, 1H), 11.05 (s, 1H), 8.54** - 8.44 (m, 1H), 8.27 (s, 1H), 8.11 (s, 1H), 8.02 (s, 1H), 7.86 (s, 1H), 7.71 (dd, 1H), 7.65 (d, 1H), 7.59 (s, 1H), 7.58 - 7.51 (m, 2H), 7.45 (t, 2H), 7.40 - 7.34 (m, 1H), 7.30 (s, 1H), 7.26 - 7.20 (m, 1H), 7.20 - 7.12 (m, 1H), 6.84 (s, 1H), 5.06 (dd, 5.4 Hz, 1H), 4.10 - 3.98 (m, 2H), 3.79 (s, 3H), 3.76 (s, 3H), 3.11 (d, 3H), 3.03 - 2.82 (m, 5H), 2.68 - 2.53 (m, 6H), 2.25 - 2.08 (m, 4H), 2.07 - 1.95 (m, 2H), 1.88 - 1.74 (m, 10H), 1.63 - 1.45 (m, 3H), 1.21 - 1.07 (m, 3H).

**Example 59: Preparation of compound 59**

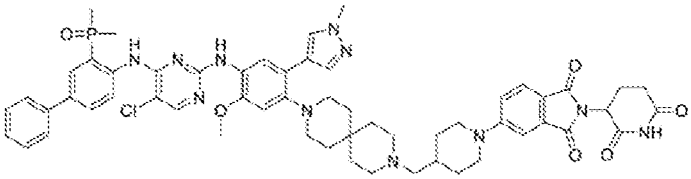

Compound 59

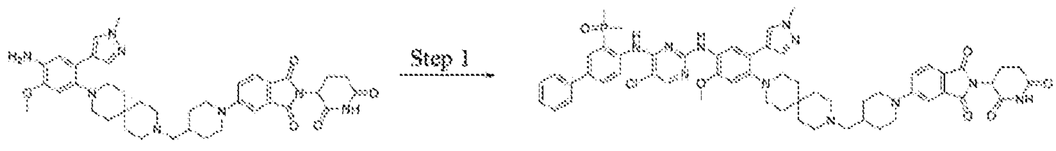


57B

Compound 59

5-(4-((9-(4-((5-chloro-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)-3,9-diazaspiro[5.5]undecan-3-yl)methyl)piperidin-1-yl)-2-(2,6-dioxopiperidin-3-yl)isoindoline-1,3-dione **(compound 59)**

**57B** (134 mg, 0.19 mmol) and (4-((2,5-dichloropyrimidin-4-yl)amino)-[1,1'-biphenyl]-3-yl)dimethylphosphine oxide **(40A)** (81 mg, 0.21 mmol) were dissolved in DMF (10 mL); p-toluenesulfonic acid monohydrate (119.8 mg, 0.63 mmol) was added; and under nitrogen protection, the mixture was stirred at 100°C for 16 h, and cooled to room temperature. 20 mL of saturated aqueous sodium bicarbonate solution and 50 mL of dichloromethane were added, and the layers were separated. The organic layer was concentrated under reduced pressure, and the residue was purified by silica gel column chromatography (mobile phase: dichloromethane/methanol (V/V) = 100/1-20/1) to obtain the crude, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 ml). Liquid separation was performed. The organic layer was then dried over anhydrous sodium sulphate and concentrated under reduced pressure to obtain **compound 59** (25 mg, yield: 12.4%).

LCMS m/z = 532.8[(M+2H)/2]$^+$.

$^1$H NMR (400 MHz, DMSO-$d_6$) δ **11.37** (s, **1H), 11.20** - 10.75 (m, 1H), 8.61 - 8.39 (m, 1H), 8.29 (s, 1H), 8.11 (s, 1H), 8.03 (s, 1H), 7.88 (s, 1H), 7.76 - 7.61 (m, 2H), 7.59 - 7.50 (m, 3H), 7.45 (t, 2H), 7.36 (t, 1H), 7.32 - 7.12 (m, 3H), 6.89 (s, 1H), 5.06 (dd, 1H), 4.14 - 3.93 (m, 2H), 3.80 (s, 3H), 3.76 (s, 3H), 3.05 - 2.73 (m, 7H), 2.69 - 2.51 (m, 2H), 2.43 - 2.22 (m, 4H), 2.22 - 2.06 (m, 2H), 2.05 - 1.94 (m, 1H), 1.89 - 1.72 (m, 9H), 1.65 - 1.45 (m, 8H), 1.20 - 1.06 (m, 2H).

**Example 60: Preparation of compound 60**

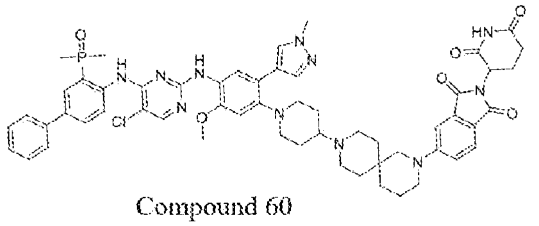

Compound 60

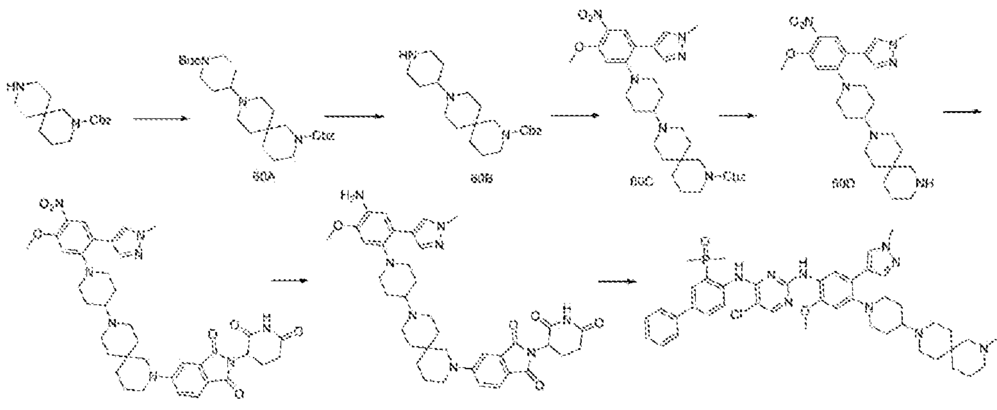

60A

60B

60C

60D

60E

60F

Compound 60

Step 1: benzyl 9-(1-(tert-butoxycarbonyl)piperidin-4-yl)-2,9-diazaspiro [5.5]undecane-2-carboxylate **(60A)**

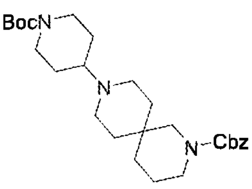

**60A**

Benzyl 2,9-diazaspiro[5.5]undecane-2-carboxylate (2.60 g, 9.02 mmol), N-BOC-piperidone (2.16 g, 10.82 mmol), anhydrous sodium sulphate (2.56 g, 18.04 mmol) and acetic acid (1.08 g, 18.04 mmol) were successively added to dichloromethane (20 ml), and sodium triacetoxyborohydride (3.82 g, 18.04 mmol) was added under stirring at room temperature. After the addition, the mixture was stirred overnight at room temperature, adjusted to pH>8 with 1 N aqueous sodium hydroxide solution and extracted by adding 30 mL of dichloromethane. The organic layer was concentrated under reduced pressure, and the residue was purified by column chromatography to obtain 60A (4.3 g, yield: 98%).

LCMS m/z = 472.3 $[M+1]^+$.

Step 2: benzyl 9-(piperidin-4-yl)-2,9-diazaspiro[5.5]undecane-2-carboxylate (60B)

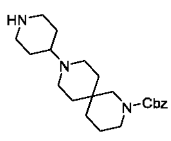

60B

**60B** (4.50 g, 9.54 mmol) was dissolved in DCM (30 ml); trifluoroacetic acid (15.30 g, 134.23 mmol) was added; and the mixture was stirred at room temperature for 2 h and concentrated under reduced pressure. The residue was adjusted to pH = 9-10 by adding 1 N aqueous NaOH solution. The organic layer was concentrated under reduced pressure to obtain a product, which was directly used in the next step.

Step 3: benzyl 9-(1-(5-methoxy-2-(1-methyl-1H-pyrazol-4-yl)-4-nitrophenyl) piperidin-4-yl)-2,9-diazaspiro[5.5]undecane-2-carboxylate **(60C)**

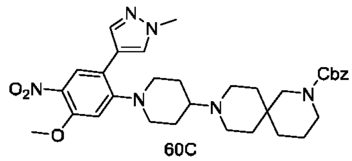

60C

4-(2-fluoro-4-methoxy-5-nitrophenyl)-1-methyl-1H-pyrazole **(1D)** (1.70 g, 6.77 mmol), compound **60B** (3.27 g, 8.80 mmol) and potassium carbonate (3.74 g, 27.08 mmol) were added to DMSO (20 ml), and the mixture was stirred at 120°C for 6 h, cooled to room temperature and extracted by adding 100 mL of ethyl acetate and 100 mL of water. The organic layer was washed with saturated brine (50 mL × 3), and concentrated under reduced pressure, and the residue was purified by column chromatography to obtain **60C** (2.3 g, yield: 56%).
LCMS m/z = 603.3 $[M+1]^+$.

Step 4: 9-(1-(5-methoxy-2-(1-methyl-1H-pyrazol-4-yl)-4-nitrophenyl) piperidin-4-yl)-2,9-diazaspiro[5.5]undecane **(60D)**

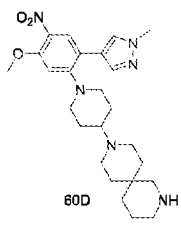

60D

**60C** (2.20 g, 3.65 mmol) was dissolved in acetic acid (6 ml); a solution of hydrogen bromide in acetic acid (10 mL, wt% = 33%) was added at room temperature; and the mixture was stirred for 0.5 h. 30 mL of MTBE was added, with a large amount of solids precipitated. The mixture was filtered. The filter cake was dissolved in 20 mL of water and extracted with 20 mL of ethyl acetate. The organic layer was discarded. The aqueous phase was extracted by adding 30 mL of dichloromethane and adjusted to pH>8 with 1 N aqueous sodium hydroxide solution. The organic layer was dried over anhydrous sodium sulphate and concentrated under reduced pressure to obtain **60D,** which was directly used in the next step.
LCMS m/z = 469.3 $[M+1]^+$.

Step 5: 2-(2,6-dioxopiperidin-3-yl)-5-(9-(1-(5-methoxy-2-(1-methyl-1H-pyrazol-4-yl)-4-nitrophenyl)piperidin-4-yl)-2,9-diazaspiro[5.5]undecan-2-yl)isoindoline-1,3-dione **(60E)**

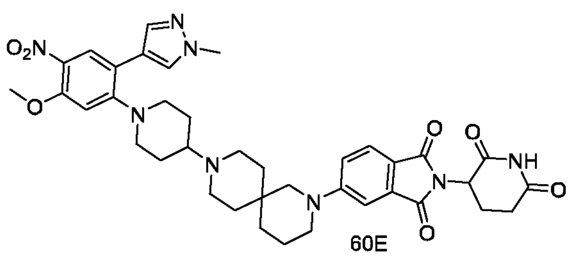

60E

**60D** (0.4 g, 0.85 mmol) and 2-(2,6-dioxopiperidin-3-yl)-5-fluoroisoindoline-1,3-dione (0.26 g, 0.94 mmol) were dissolved in DMSO (5 ml); DIPEA (0.22 g, 1.7 mmol) was added; and the mixture was stirred at 90°C for 5 h and cooled to room temperature. 10 mL of water was added, with a solid precipitated, and the mixture was filtered. The filter cake was dried under reduced pressure and then purified by silica gel column chromatography to obtain **60E** (0.45 g, yield: 73%).

Step 6: 5-(9-(1-(4-amino-5-methoxy-2-(1-methyl-1H-pyrazol-4-yl)phenyl)piperidin-4-yl)-2,9-diazaspiro[5.5]undecan-2-yl)-2-(2,6-dioxopiperidin-3-yl)isoindoline-1,3-dione **(60F)**

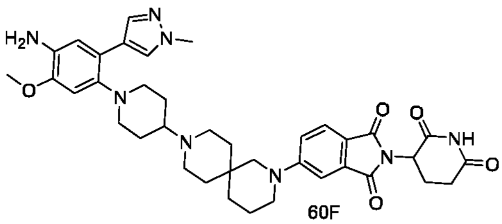

60F

**60E** (400 mg, 0.55 mmol) was dissolved in ethanol/water (8 mL, 3:1); iron powder (310 mg, 5.5 mmol) and ammonium chloride (290 mg, 5.5 mmol) were successively added; and under nitrogen protection, the mixture was stirred at 80°C for 1 h, cooled to room temperature and subjected to suction filtration under reduced pressure. The filtrate was extracted by adding 20 mL of dichloromethane and 20 mL of saturated aqueous sodium chloride solution. The organic layer was dried and then concentrated under reduced pressure to obtain **60F** (380 mg), which was directly used in the next step.

Step 7: 5-(9-(1-(4-((5-chloro-4-((3-(dimethylphosphoryl)-[1,1'-biphenyl]-4-yl) amino) pyrimidin-2-yl)amino)-5-methoxy-2-(1-methyl-1H-pyrazol-4-yl)phenyl)piperidin-4-yl)-2,9-diazaspiro [5.5]undecan-2-yl)-2-(2,6-dioxopiperidin-3-yl)isoindoline-1,3-dione (compound 60)

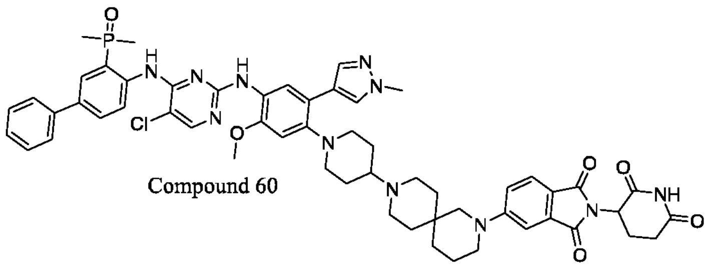

Compound 60

**60F** (140 mg, 0.20 mmol) and **40A** (78 mg, 0.20 mmol) were dissolved in a mixed solution of 1,4-dioxane (4 mL) and NMP (1 mL); p-toluenesulfonic acid monohydrate (110 mg, 0.60 mmol) was added; and under nitrogen protection, the mixture was reacted overnight at 100°C, and cooled to room temperature. 20 mL of saturated aqueous sodium bicarbonate solution was added, and the mixture was subjected to suction filtration. The filter cake was dissolved in 50 mL of dichloromethane and extracted. The organic phase was dried over anhydrous sodium sulphate and then concentrated under reduced pressure, and the residue was purified by silica gel column chromatography (dichloromethane/methanol (V/V) = 100/1-10/1) to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)) to obtain the trifluoroacetate of **compound 60.** 20 mL of dichloromethane and 10 mL of saturated sodium bicarbonate solution were added to the trifluoroacetate of **compound 60,** and the layers were separated. The organic phase was dried over anhydrous sodium sulphate and then concentrated under reduced pressure to obtain **compound 60** (60 mg, yield: 28%).

LCMS m/z = 525.8 [(M+2H)/2]$^+$.

$^1$H NMR (400 MHz, DMSO-$d_6$) δ **11.36 (s, 1H), 11.05 (s, 1H), 8.65** - 8.32 (m, 1H), 8.26 (s, 1H), 8.11 (s, 1H), 8.01 (s, 1H), 7.87 (s, 1H), 7.71 (dd, 1H), 7.67 - 7.49 (m, 4H), 7.49 - 7.40 (m, 2H), 7.39 - 7.29 (m, 2H), 7.28 - 7.09 (m,

2H), 6.83 (s, 1H), 5.05 (dd, 1H), 3.79 (s, 3H), 3.75 (s, 3H), 3.52 - 3.42 (m, 2H), 3.41 - 3.34 (m, 2H), 3.18 - 3.03 (m, 2H), 2.98 - 2.81 (m, 1H), 2.72 - 2.50 (m, 9H), 2.09 - 1.97 (m, 1H), 1.93 - 1.32 (m, 18H).

**Example 61: Preparation of compound 61**

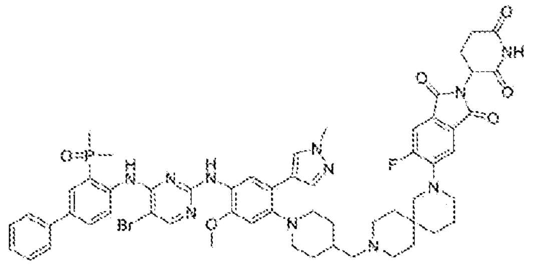

Compound 61

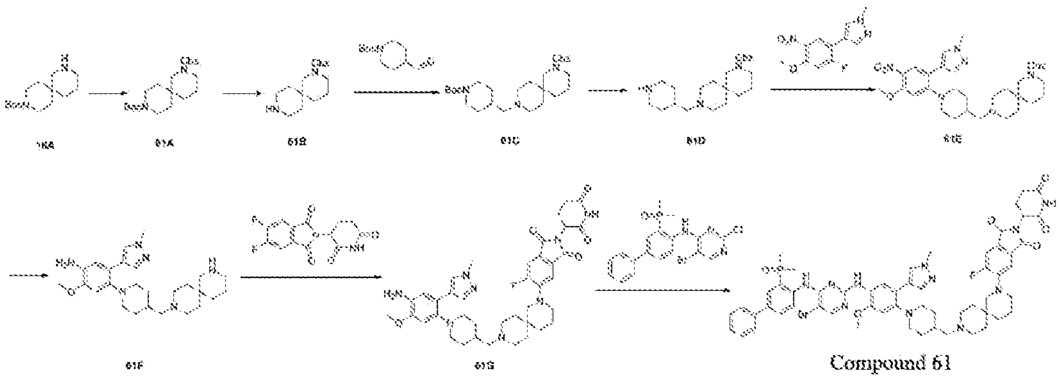

Step 1: 2-benzyl 9-(tert-butyl) 2,9-diazaspiro[5.5]undecane-2,9-dicarboxylate **(61A)**

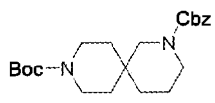

61A

**10A** (5.00 g, 19.66 mmol) was dissolved in dichloromethane (50 mL); triethylamine (4.18 g, 41.29 mmol) was added; and then benzyl chloroformate (3.42 g, 20.05 mmol) was added dropwise under an ice bath. After the dropwise addition, the mixture was reacted at room temperature for 1 h. The reaction was quenched by adding water and extracted with 400 mL of ethyl acetate. The organic phases were collected, dried over anhydrous sodium sulphate and concentrated under reduced pressure, and the residue was purified by column chromatography (mobile phase: petroleum ether/ethyl acetate (V/V) = 20/1-5/1) to obtain **61A** (6.07 g, yield: 79%).

LCMS m/z = 333.2 [M-55]$^+$.

Step 2: benzyl 2,9-diazaspiro[5.5]undecane-2-carboxylate **(61B)**

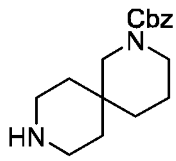

**61B**

**61A** (2.00 g, 5.15 mmol) was dissolved in dichloromethane (30 mL); trifluoroacetic acid (10 mL) was slowly added; and the mixture was reacted at room temperature for 1 h, concentrated under reduced pressure to remove dichloromethane and trifluoroacetic acid, redissolved by adding 50 mL of dichloromethane and adjusted to a basic pH with 1 N aqueous sodium hydroxide solution. Liquid separation was performed. The organic phases were collected, and the aqueous phase was extracted 3 times with dichloromethane. All organic phases were combined, dried over anhydrous sodium sulphate and concentrated under reduced pressure to obtain **61B** (1.35 g), which was directly used in the next step.
LCMS m/z = 289.2$[M+H]^+$.

Step 3: benzyl 9-((1-(tert-butoxycarbonyl)piperidin-4-yl)methyl)-2,9-diazaspiro[5.5] undecane-2-carboxylate **(61C)**

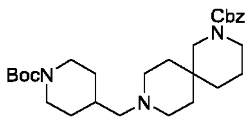

**61C**

**61B** (1.35 g) and tert-butyl 4-formylpiperidine-1-carboxylate (1.00 g, 4.68 mmol) were dissolved in dichloromethane (50 mL); acetic acid (0.56 g, 9.36 mmol) and sodium triacetoxyborohydride (1.98 g, 9.36 mmol) were successively added; and the mixture was reacted at room temperature for 2 h, adjusted to a basic pH by adding 1 N aqueous sodium hydroxide solution and extracted 3 times with dichloromethane. The organic phases were combined, dried over anhydrous sodium sulphate and concentrated under reduced pressure, and the residue was purified by silica gel column chromatography (mobile phase: ethyl acetate/petroleum ether (V/V) = 1/1 for removing impurities, dichloromethane/methanol (V/V) = 20/1 for collecting a product) to obtain **61C** (2.07 g, yield: 91%).
LCMS m/z = 486.4 $[M+H]^+$.

Step 4: benzyl 9-(piperidin-4-ylmethyl)-2,9-diazaspiro[5.5]undecane-2-carboxylate **(61D)**

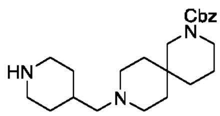

**61D**

Compound **61C** (2.07 g, 4.26 mmol) was dissolved in dichloromethane (30 mL); trifluoroacetic acid (10 mL) was slowly added; and the mixture was reacted at room temperature for 1 h, concentrated under reduced pressure to remove dichloromethane and trifluoroacetic acid, redissolved by adding 50 mL of dichloromethane and adjusted to a basic pH with 1 N aqueous sodium hydroxide solution. Liquid separation was performed. The organic phases were collected, and the aqueous phase was extracted 3 times with dichloromethane. All organic phases were combined, dried over anhydrous sodium sulphate and concentrated under reduced pressure to obtain **61D** (1.64 g), which was directly used in the next step.
LCMS m/z = 386.3$[M+H]^+$.

Step 5: benzyl 9-((1-(5-methoxy-2-(1-methyl-1H-pyrazol-4-yl)-4-nitrophenyl) piperidin-4-yl) methyl)-2,9-diazaspiro[5.5]undecane-2-carboxylate **(61E)**

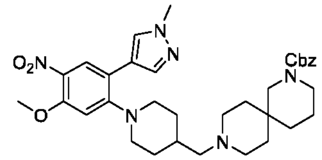

**61E**

**61D** (1.64 g, 4.25 mmol), 4-(2-fluoro-4-methoxy-5-nitrophenyl)-1-methyl-1H-pyrazole **(1D)** (1.07 g, 4.25 mmol) and potassium carbonate (2.94 g, 21.25 mmol) were mixed and dissolved in DMSO (50 mL); and the mixture was stirred at 120°C for 3 h, and cooled to room temperature. The reaction solution was diluted by adding 200 mL of ethyl acetate and washed 3 times with water. The organic phase was dried over anhydrous sodium sulphate and then concentrated under reduced pressure, and the residue was purified by column chromatography (mobile phase: dichloromethane/methanol (V/V) = 100/1-20/1) to obtain **61E** (640 mg, yield: 24%).

LCMS m/z = 617.3 $[M+H]^+$.

Step 6: 4-(4-((2,9-diazaspiro[5.5]undecan-9-yl)methyl)piperidin-1-yl)-2-methoxy-5-(1-methyl-1H-pyrazol-4-yl)aniline **(61F)**

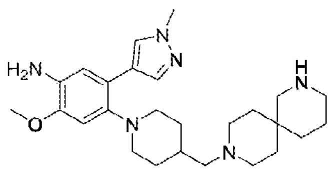

**61F**

**61E** (0.64 g, 1.04 mmol) was dissolved in a mixed solvent of isopropanol (20 mL) and ammonia methanol solution (5 mL); palladium on carbon (wt% = 10%, 0.33 g) was added; and the mixture was subjected to 1 atm hydrogen replacement 3 times, reacted at room temperature for 1.5 h and filtered over celite. The filter cake was washed with dichloromethane/methanol (V/V = 10/1), and the filtrate was concentrated under reduced pressure to obtain **61F** (450 mg), which was directly used in the next step.

LCMS m/z = 453.3 $[M+H]^+$.

Step 7: 5-(9-((1-(4-amino-5-methoxy-2-(1-methyl-1H-pyrazol-4-yl)phenyl) piperidin-4-yl)methyl)-2,9-diazaspiro[5.5]undecan-2-yl)-2-(2,6-dioxopiperidin-3-yl)-6-fluoroisoindoline-1,3-dione **(61G)**

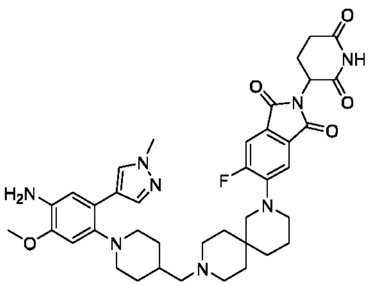

**61G**

**61F** (0.23 g, 0.50 mmol) and 2-(2,6-dioxopiperidin-3-yl)-5,6-difluoroisoindoline-1,3-dione **(5A-1)** (0.15 g, 0.50 mmol) were dissolved in DMSO (10 mL); DIPEA (0.19 g, 1.50 mmol) was added dropwise; and the mixture was reacted at 90°C for 3 h. The reaction solution was cooled to room temperature. 20 mL of water was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase: dichloromethane/methanol (V/V) = 50/1-10/1) to obtain **61G** (167 mg, yield: 46%).

LCMS m/z = 727.3 $[M+H]^+$.

Step 8: 5-(9-((1-(4-((5-bromo-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperidin-4-yl)methyl)-2,9-diazaspiro[5.5]undecan-2-yl)-2-(2,6-dioxopiperidin-3-yl)-6-fluoroisoindoline-1,3-dione **(compound 61)**

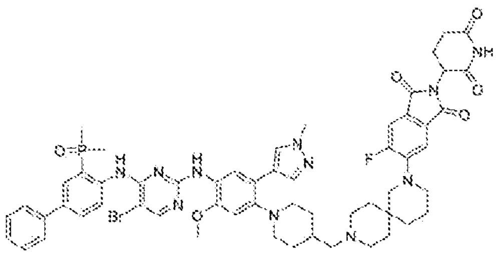

Compound 61

**61G** (0.08 g, 0.11 mmol) and (4-((5-bromo-2-chloropyrimidin-4-yl)amino)-[1,1'-biphenyl]-3-yl)dimethylphosphine oxide **(23D)** (0.05 g, 0.11 mmol) were dissolved in DMF (10 mL); p-toluenesulfonic acid hydrate (0.06 g, 0.33 mmol) was added; and under nitrogen protection, the mixture was reacted at 100°C for 16 h. The reaction solution was cooled to room temperature. 10 mL of saturated aqueous sodium bicarbonate solution was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase: dichloromethane/methanol (V/V) = 50/1-15/1) to obtain a product, which was further purified by preparative HPLC (composition of mobile phases: mobile phase A: acetonitrile, and mobile phase B: water (containing 0.05% trifluoroacetic acid)) to obtain a product, which was alkalized with aqueous sodium bicarbonate solution, extracted with dichloromethane, concentrated and dried to obtain **compound 61** (15 mg, yield: 12%).

LCMS m/z = 563.8 $[(M+2H)/2]^+$.

$^1$H NMR (400 MHz, DMSO-$d_6$) δ **11.12 (s, 1H), 11.08 (s, 1H), 8.43** - 8.35 (m, 1H), 8.29 (s, 1H), 8.18 (s, 1H), 8.01 (s, 1H), 7.85 (s, 1H), 7.75 - 7.62 (m, 2H), 7.60 - 7.51 (m, 3H), 7.50 - 7.39 (m, 3H), 7.33 (t, 1H), 7.25 - 7.07 (m, 1H), 6.84 (s, 1H), 5.10 (dd, 1H), 3.78 (s, 3H), 3.75 (s, 3H), 3.24 - 3.13 (m, 2H), 3.12 - 2.97 (m, 4H), 2.94 - 2.80 (m, 1H), 2.69 - 2.52 (m, 4H), 2.45 - 2.14 (m, 6H), 2.08 - 1.95 (m, 1H), 1.83 (d, 6H), 1.76 - 1.63 (m, 4H), 1.63 - 1.38 (m, 7H), 1.35 - 1.24 (m, 2H).

**Example 62: Preparation of compound 62**

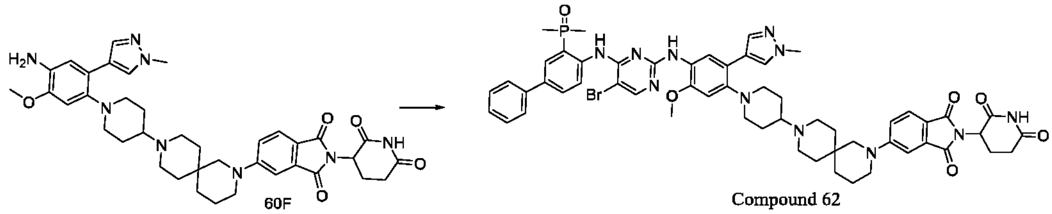

60F

Compound 62

Step 1: 5-(9-(1-(4-((5-bromo-4-((3-(dimethylphosphoryl)-[1,1'-biphenyl]-4-yl)amino) pyrimidin-2-yl)amino)-5-methoxy-2-(1-methyl-1H-pyrazol-4-yl)phenyl)piperidin-4-yl)-2,9-diazaspiro[5.5] undecan-2-yl)-2-(2,6-dioxopiperidin-3-yl)isoindoline-1,3-dione **(compound 62)**

**60F** (140 mg, 0.20 mmol) and **23D** (87 mg, 0.20 mmol) were dissolved in DMF (5 mL); p-toluenesulfonic acid monohydrate (110 mg, 0.60 mmol) was added; and under nitrogen protection, the mixture was reacted overnight at 100°C, and cooled to room temperature. 20 mL of saturated aqueous sodium bicarbonate solution was added, and the mixture was subjected to suction filtration. The filter cake was dissolved in 50 mL of dichloromethane and extracted. The organic phase was dried over anhydrous sodium sulphate and then concentrated under reduced pressure, and the residue was purified by silica gel column chromatography (dichloromethane/methanol (V/V) = 100/1-10/1) to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)) to obtain the trifluoroacetate of **compound 62.** 20 mL of dichloromethane and 10 mL of saturated sodium bicarbonate solution were added to the trifluoroacetate of **compound 62,** and the layers were separated. The organic phase was dried over anhydrous sodium sulphate and then concentrated under reduced pressure to obtain **compound 62** (30 mg, yield: 14%).

LCMS m/z = 547.8 $[(M+2H)/2]^+$.

$^1H$ NMR (400 MHz, DMSO-$d_6$) δ **11.10 (s, 1H), 11.05 (s, 1H), 8.47** - 8.32 (m, 1H), 8.26 (s, 1H), 8.19 (s, 1H), 7.99 (s, 1H), 7.86 (s, 1H), 7.69 (d, 1H), 7.63 (d, 1H), 7.60 - 7.48 (m, 3H), 7.45 (t, 2H), 7.40 - 7.07 (m, 4H), 6.82 (s, 1H), 5.05 (dd, 1H), 3.79 (s, 3H), 3.75 (s, 3H), 3.56 - 3.37 (m, 4H), 3.17 - 3.04 (m, 2H), 2.95 - 2.81 (m, 1H), 2.72 - 2.51 (m, 9H), 2.05 - 1.95 (m, 1H), 1.92 - 1.74 (m, 8H), 1.74 - 1.35 (m, 10H).

**Example 63: Preparation of compound 63**

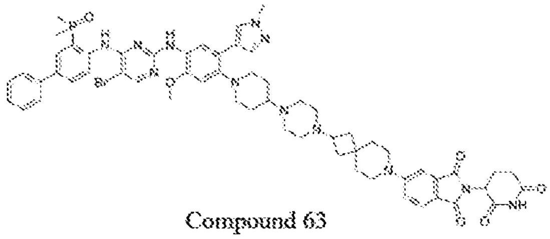

Compound 63

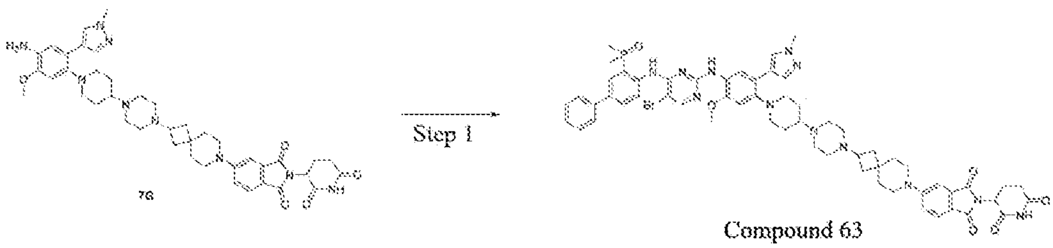


7G

Compound 63

Step 1: 5-(2-(4-(1-(4-((5-bromo-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperidin-4-yl)piperazin-1-yl)-7-azaspiro[3.5]nonan-7-yl)-2-(2,6-dioxopiperidin-3-yl)isoindoline-1,3-dione **(compound 63)**

**7G** (0.15 g, 0.20 mmol) and **23D** (0.10 g, 0.23 mmol) were dissolved in DMF (10 mL); p-toluenesulfonic acid monohydrate (0.09 g, 0.47 mmol) was added; and under nitrogen protection, the mixture was reacted at 100°C for 16 h. The reaction solution was cooled to room temperature. 5 mL of water and 5 mL of saturated aqueous sodium bicarbonate solution were added, and the mixture was filtered. The filter cake was dried under reduced pressure and then purified by silica gel column chromatography (dichloromethane/methanol (V/V) = 100/1-12/1) to obtain a product, which was further purified by preparative HPLC (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 ml). Liquid separation was performed. The organic layer was then dried over anhydrous sodium sulphate and concentrated under reduced pressure to obtain **compound**

**63** (30 mg, yield: 13%).

LCMS m/z = 575.4 $[(M+2H)/2]^+$.

$^1H$ NMR (400 MHz, DMSO-$d_6$) δ **11.11 (d, 1H), 11.05 (s, 1H), 8.39 (s, 1H),** 8.27 (s, 1H), 8.18 (s, 1H), 8.00 (s, 1H), 7.85 (s, 1H), 7.69 (dd, 1H), 7.64 (d, 1H), 7.59 - 7.52 (m, 3H), 7.45 (t, 2H), 7.39 - 7.34 (m, 1H), 7.31 (d, 1H), 7.23 (dd, 1H), 7.21 - 7.13 (m, 1H), 6.83 (s, 1H), 5.06 (dd, 1H), 3.79 (s, 3H), 3.76 (s, 3H), 3.50 - 3.44 (m, 2H), 3.41 - 3.36 (m, 2H), 3.29 - 3.27 (m, 1H), 3.09 (d, 2H), 2.92 - 2.84 (m, 1H), 2.72 - 2.65 (m, 1H), 2.63 - 2.51 (m, 6H), 2.36 - 2.16 (m, 5H), 2.04 - 1.95 (m, 3H), 1.87 - 1.75 (m, 8H), 1.67 - 1.47 (m, 9H).

### Example 64: Preparation of compound 64

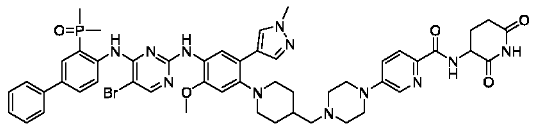

Compound 64

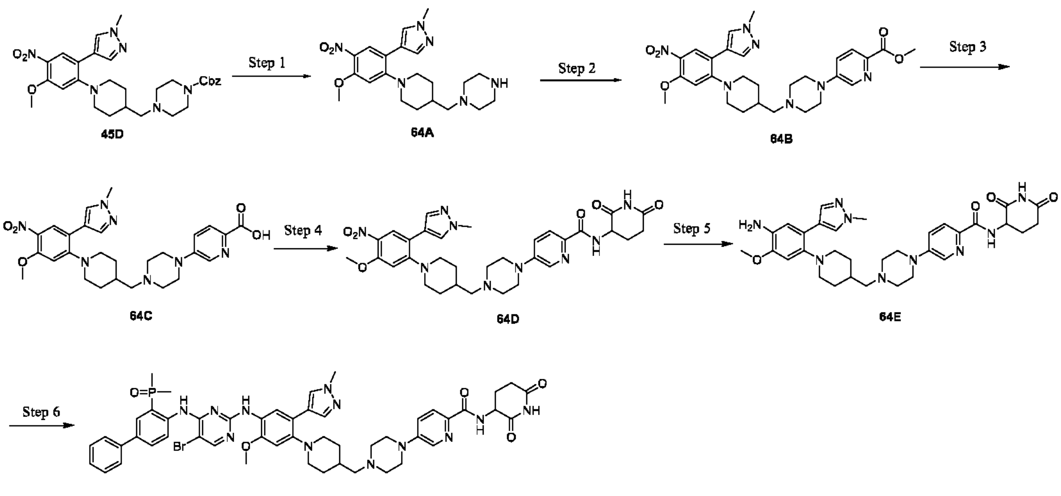


45D 64A 64B

64C 64D 64E

Compound 64

Step 1: 1-((1-(5-methoxy-2-(1-methyl-1H-pyrazol-4-yl)-4-nitrophenyl) piperidin-4-yl)methyl)piperazine **(64A)**

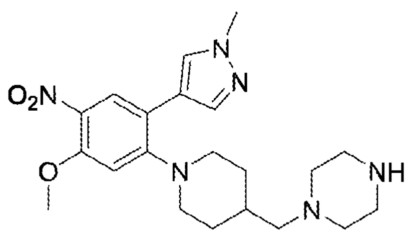

**45D** (1.4 g, 2.55 mmol) was dissolved in 5 mL of acetic acid; 10 mL of HBr-AcOH was added; and the mixture was stirred at room temperature for 30 min. The reaction system was poured into 40 mL of water, with a solid precipitated, and the mixture was filtered. The filter cake was dissolved in 150 ml of dichloromethane, washed with aqueous sodium bicarbonate solution (100 mL × 2), dried over anhydrous sodium sulphate and concentrated to obtain **64A** (1.0 g, yield: 95.2%).

LCMS m/z = 415.3 $[M +1]^+$.

Step 2: methyl 5-(4-((1-(5-methoxy-2-(1-methyl-1H-pyrazol-4-yl) -4-nitrophenyl) piperidin-4-yl) methyl) piperazin-1-yl)picolinate **(64B)**

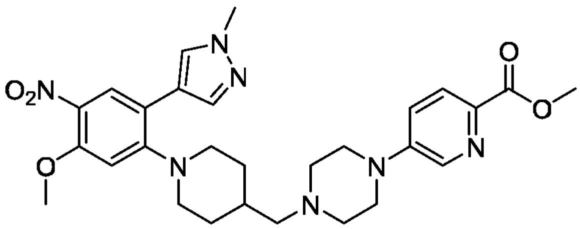

**64A** (1.0 g, 2.41 mmol) was added to 30 mL of dimethyl sulfoxide; methyl 5-fluoropyridinecarboxylate (0.56 g, 3.61 mmol) and triethylamine (1.22 g, 12.08 mmol) were added; and the mixture was reacted at 90°C for 3 h, and cooled to room temperature. 100 mL of water was added, with a solid precipitated, and the mixture was filtered. The filter cake was dissolved in dichloromethane, dried over anhydrous sodium sulphate and filtered. The filtrate was concentrated to an oil, and the residue was purified by column chromatography (dichloromethane/methanol = 10/1 (V/V) as a mobile phase) to obtain **64B** (1.0 g, yield: 81%).

LCMS m/z = 550.3 $[M +1]^+$.

Step 3: 5-(4-((1-(5-methoxy-2-(1-methyl-1H-pyrazol-4-yl)-4-nitrophenyl) piperidin-4-yl)methyl)piperazin-1-yl)picolinic acid **(64C)**

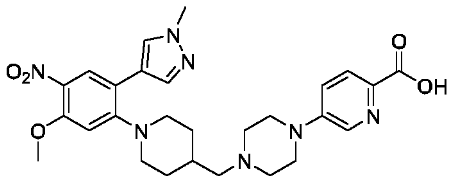

**64B** (1.0 g, 1.82 mmol) was added to 40 mL of tetrahydrofuran and 4 mL of water; lithium hydroxide monohydrate (153 mg, 3.64 mmol) was added; and the mixture was reacted at room temperature for 18 h. The system was adjusted to a neutral pH with 1 N HCl and concentrated under reduced pressure to obtain **64C** (1.2 g, crude).

LCMS m/z = 536.3 $[M +1]^+$.

Step 4: N-(2,6-dioxopiperidin-3-yl)-5-(4-((1-(5-methoxy-2-(1-methyl-1H-pyrazol-4-yl)-4-nitrophenyl)piperidin-4-yl)methyl)piperazin-1-yl)picolinamide **(64D)**

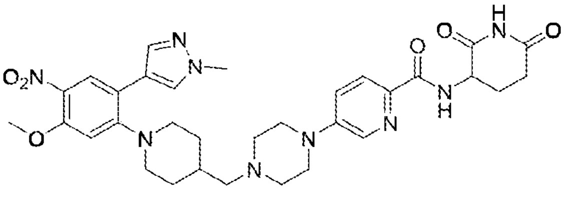

**64C** (1.2 g, crude) was added to 30 mL of DMF; 3-aminopiperidine-2,6-dione hydrochloride (0.36 g, 2.20 mmol), DIPEA (1.64 g, 12.7 mmol) and HATU (1.04 g, 2.74 mmol) were added; and the mixture was reacted at room temperature for 16 h. 120 mL of purified water was added, with a solid precipitated. The mixture was subjected to suction filtration and dried, and the residue was purified by column chromatography (dichloromethane/methanol = 10/1 (V/V) as a mobile phase) to obtain **64D** (1.0 g, yield: 87.5%)

LCMS m/z = 646.2 $[M +1]^+$.

Step 5: 5-(4-((1-(4-amino-5-methoxy-2-(1-methyl-1H-pyrazol-4-yl)phenyl) piperidin-4-yl)methyl)piperazin-1-yl)-N-(2,6-dioxopiperidin-3-yl)picolinamide **(64E)**

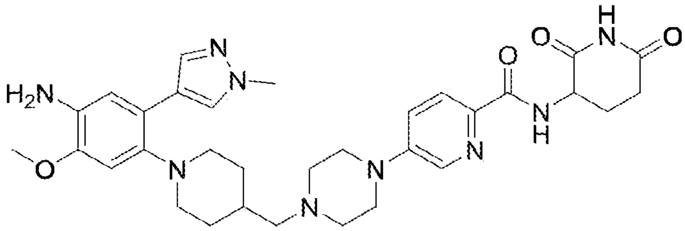

**64D** (1.0 g, 1.55 mmol) was dissolved in a mixed solution of THF (10 mL), ethanol (30 mL) and water (6 mL); reduced iron powder (875 mg, 15.63 mmol) and ammonium chloride (830 mg, 15.63 mmol) were added; and the mixture was reacted at 85°C for 2 h. The reaction solution was cooled to room temperature and concentrated in vacuo to remove a solvent. 50 mL of water and 50 mL of dichloromethane were added to the residue, and then the mixture was extracted 3 times with dichloromethane. The organic phases were combined, dried over anhydrous sodium sulphate and then concentrated under reduced pressure to obtain **64E** (0.7 g, yield: 73%)

LCMS m/z = 616.3 $[M+H]^+$.

Step 6: 5-(4-((1-(4-((5-bromo-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperidin-4-yl)methyl)piperazin-1-yl)-N-(2,6-dioxopiperidin-3-yl)picolinamide **(compound 64)**

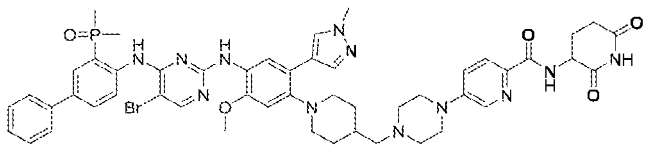

**64E** (350 mg, 0.57 mmol) and (4-((5-bromo-2-chloropyrimidin-4-yl)amino)-[1,1'-biphenyl]-3-yl)dimethylphosphine oxide **(23D)** (373 mg, 0.85 mmol) were dissolved in DMF (20 mL); p-toluenesulfonic acid monohydrate (325 mg, 1.71 mmol) was added; and the mixture was stirred at 100°C for 8 h, and cooled to room temperature. 200 mL of saturated aqueous sodium bicarbonate solution was added, with a white solid precipitated. Suction filtration was performed for collecting the solid, and the filter cake was washed with water (100 ml × 3) and redissolved in dichloromethane. The organic layers were separated, dried over anhydrous sodium sulphate and concentrated, and the crude was further subjected to preparative HPLC (instrument: waters 2767 (preparative liquid phase chromatographic instrument); chromatographic column: XBridge@ Prep C18 (30 mm × 150 mm); composition of mobile phases: mobile phase A: acetonitrile, and mobile phase B: water (containing 0.1% TFA)) purification to obtain a preparative solution, which was concentrated and adjusted to a basic pH by adding dichloromethane and saturated sodium bicarbonate solution. The organic phase was separated. The aqueous phase was extracted with dichloromethane (100 ml × 3). The organic phase was dried over anhydrous sodium sulphate and concentrated, and the crude was subjected to prep-TLC (DCM: MeOH (V/V) = 15 : 1) purification to obtain **compound 64** (200 mg, yield: 35%).

LCMS m/z = 1015.3 $[M+1]^+$.

$^1$H NMR (400 MHz, DMSO-$d_6$) δ **11.12 (s, 1H), 10.84 (s, 1H), 8.72 (d, 1H),** 8.46 - 8.24 (m, 3H), 8.19 (s, 1H), 8.01 (s, 1H), 7.88 (t, 2H), 7.70 (dd, 1H), 7.63 - 7.50 (m, 3H), 7.49 - 7.40 (m, 3H), 7.39 - 7.31 (m, 1H), 7.25 - 7.12 (m, 1H), 6.85 (s, 1H), 4.81 - 4.70 (m, 1H), 3.80 (s, 3H), 3.76 (s, 3H), 3.44 - 3.33 (m, 4H), 3.14 - 3.02 (m, 2H), 2.86 - 2.73 (m, 1H), 2.71 - 2.51 (m, 7H), 2.29 (d, 2H), 2.25 - 2.13 (m, 1H), 2.07 - 2.00 (m, 1H), 1.83 (d, 6H), 1.79-1.70 (m, 2H), 1.69 - 1.54 (m, 1H), 1.39 - 1.24 (m, 2H).

**Example 65: Preparation of compound 65**

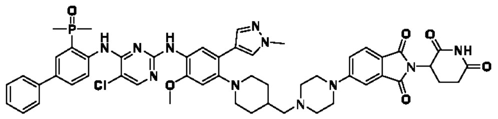

Compound 65

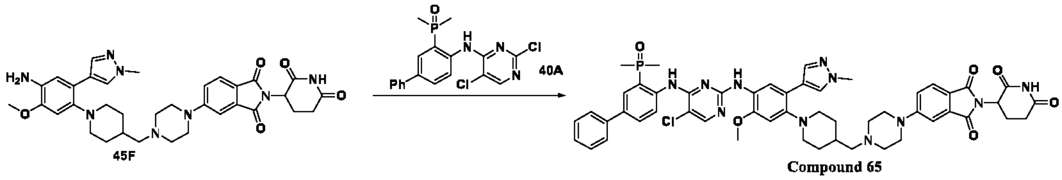


45F

Compound 65

Step 1: 5-(4-((1-(4-((5-chloro-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperidin-4-yl)methyl)piperazin-1-yl)-2-(2,6-dioxopiperidin-3-yl)isoindoline-1,3-dione **(compound 65)**

**45F** (250 mg, 0.39 mmol) and **40A** (153 mg, 0.39 mmol) were dissolved in DMF (5 mL); p-toluenesulfonic acid monohydrate (223 mg, 1.17 mmol) was added; and under nitrogen protection, the mixture was reacted overnight at 100°C, and cooled to room temperature. 20 mL of saturated aqueous sodium bicarbonate solution was added, and the mixture was subjected to suction filtration. The filter cake was dissolved in 50 mL of dichloromethane and extracted. The organic phase was dried over anhydrous sodium sulphate and then concentrated under reduced pressure, and the residue was purified by silica gel column chromatography (dichloromethane/methanol (V/V) = 100/1-10/1) to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)) to obtain the trifluoroacetate of **compound 65** (63 mg, yellow solid). The trifluoroacetate of **compound 65** was extracted by adding 20 mL of dichloromethane and 50 mL of saturated sodium bicarbonate solution. The organic phase was dried over anhydrous sodium sulphate and then concentrated under reduced pressure to obtain **compound 65** as a yellow solid (45 mg, yield: 12%).

LCMS m/z = 996.4 $[M+H]^+$.

$^1H$ NMR (400 MHz, $D_2O/CF_3COOD$(v/v = 1:1)) δ 8.27 - 8.14 (m, 2H), 8.10 (s, 1H), 7.88 - 7.69 (m, 4H), 7.65 - 7.55 (m, 4H), 7.55 - 7.42 (m, 2H), 7.38 - 7.22 (m, 3H), 5.12 (dd, 1H), 4.22 - 4.08 (m, 2H), 4.05 (s, 3H), 3.89 - 3.69 (m, 6H), 3.61 - 3.47 (m, 5H), 3.37 - 3.13 (m, 4H), 2.95 - 2.85 (m, 2H), 2.82 - 2.65 (m, 1H), 2.56 - 2.39 (m, 1H), 2.30 - 2.13 (m, 3H), 2.08 - 1.86 (m, 8H).

**Example 66: Preparation of compound 66**

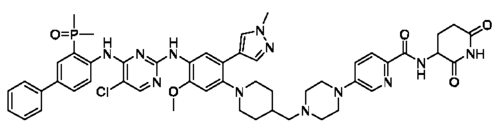

Compound 66

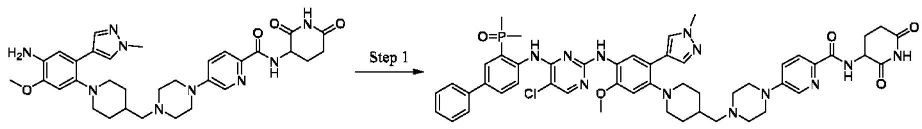


64E

Compound 66

Step 1: 5-(4-((1-(4-((5-chloro-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperidin-4-yl)methyl)piperazin-1-yl)-N-(2,6-dioxopiperidin-3-yl)picolinamide **(compound 66)**

**64E** (350 mg, 0.57 mmol) and (4-((2,5-dichloropyrimidin-4-yl)amino)-[1,1'-biphenyl]-3-yl)dimethylphosphine oxide **(40A)** (336 mg, 0.85 mmol) were dissolved in DMF (20 mL); p-toluenesulfonic acid monohydrate (325 mg, 1.71

mmol) was added; and the mixture was stirred at 100°C for 8 h, and cooled to room temperature. 200 mL of saturated aqueous sodium bicarbonate solution was added, with a white solid precipitated. Suction filtration was performed for collecting the solid, and the filter cake was washed with water (100 ml × 3) and redissolved in dichloromethane. The organic layers were separated, dried over anhydrous sodium sulphate and concentrated, and the crude was further subjected to preparative HPLC (instrument: waters 2767 (preparative liquid phase chromatographic instrument); chromatographic column: XBridge@ Prep C18 (30 mm × 150 mm); composition of mobile phases: mobile phase A: acetonitrile, and mobile phase B: water (containing 0.1% TFA)) purification to obtain a preparative solution, which was concentrated and adjusted to a basic pH by adding dichloromethane and saturated sodium bicarbonate solution. The organic phase was separated. The aqueous phase was extracted with dichloromethane (100 ml × 3). The organic phase was dried over anhydrous sodium sulphate and concentrated, and the crude was subjected to prep-TLC (DCM: MeOH (V/V) = 15:1) purification to obtain **compound 66** (230 mg, yield: 41%).

LCMS m/z = 971.3 $[M+1]^+$.

$^1H$ NMR (400 MHz, DMSO-$d_6$) δ **11.38 (s, 1H), 10.84 (s, 1H), 8.72 (d, 1H),** 8.58 - 8.42 (m, 1H), 8.33 (d, 1H), 8.29 (s, 1H), 8.12 (s, 1H), 8.03 (s, 1H), 7.88 (d, 2H), 7.71 (dd, 1H), 7.64 - 7.50 (m, 3H), 7.49 - 7.39 (m, 3H), 7.39 - 7.29 (m, 1H), 7.27 - 7.05 (m, 1H), 6.86 (s, 1H), 4.82 - 4.67 (m, 1H), 3.80 (s, 3H), 3.76 (s, 3H), 3.46 - 3.33 (m, 4H), 3.15 - 3.01 (m, 2H), 2.86 - 2.72 (m, 1H), 2.70 - 2.51 (m, 7H), 2.29 (d, 2H), 2.25 - 2.10 (m, 1H), 2.10 - 1.97 (m, 1H), 1.84 (d, 6H), 1.80 - 1.69 (m, 2H), 1.69 - 1.54 (m, 1H), 1.40 - 1.25 (m, 2H).

**Example 67: Preparation of compound 67**

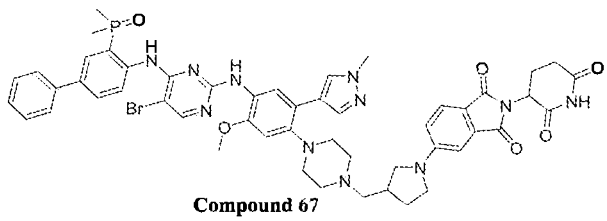

Compound 67

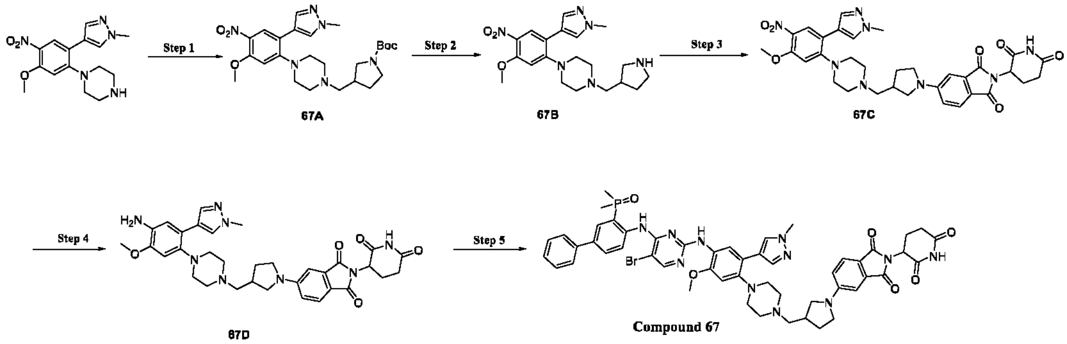


67A 67B 67C 67D Compound 67

Step 1: tert-butyl 3-((4-(5-methoxy-2-(1-methyl-1H-pyrazol-4-yl)-4-nitrophenyl)piperazin-1-yl)methyl)pyrrolidine-1-carboxylate **(67A)**

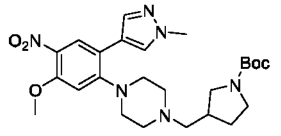

67A

The hydrochloride (1.0 g) of 1-(5-methoxy-2-(1-methyl-1H-pyrazol-4-yl)-4-nitrophenyl)piperazine (compound **80B)** and tert-butyl 3-formylpyrrolidine-1-carboxylate (0.8 g, 4.02 mmol) were dissolved in DMAc (50 mL); acetic acid (1.0 g, 16.65 mmol) was added; and the mixture was stirred at room temperature for 2 h. Sodium triacetoxyborohydride

(1.0 g, 4.72 mmol) was added, and the mixture was reacted overnight at room temperature. After the reaction was completed, the mixture was washed by adding 100 mL of ethyl acetate and 200 mL of saturated aqueous sodium bicarbonate solution. Liquid separation was performed. The organic phase was dried over anhydrous sodium sulphate and then concentrated under reduced pressure, and the residue was purified by silica gel column chromatography (mobile phase: dichloromethane/methanol (V/V) = 100/1-10/1) to obtain **67A** as a yellow solid (1.1 g, yield: 70%).

LCMS m/z = 501.2 $[M+H]^+$.

Step 2: 1-(5-methoxy-2-(1-methyl-1H-pyrazol-4-yl)-4-nitrophenyl)-4-(pyrrolidin-3-ylmethyl)piperazine **(67B);** HCl

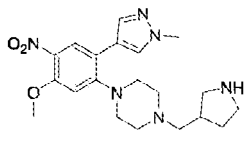

67B

Compound **67A** (1.1 g, 2.20 mmol) was dissolved in methanol (14 mL); a solution of hydrochloric acid in dioxane (4 *N,* 30 mL) was added; and the mixture was reacted at room temperature for 1 h and concentrated under reduced pressure, and the residue was directly used in the next step.

Step 3: 2-(2,6-dioxopiperidin-3-yl)-5-(3-((4-(5-methoxy-2-(1-methyl-1H-pyrazol -4-yl)-4-nitrophenyl)piperazin-1-yl)methyl)pyrrolidin-1-yl)isoindoline-1,3-dione **(67C)**

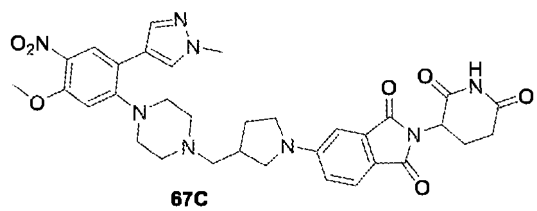

67C

The crude hydrochloride of **67B** from the previous step was dissolved in DMSO (20 mL); 2-(2,6-dioxopiperidin-3-yl)-5-fluoroisoindoline-1,3-dione (683 mg, 2.47 mmol) and sodium bicarbonate (692 mg, 8.24 mmol) were successively added; and the mixture was stirred at 100°C for 5 h, and cooled to room temperature. 30 mL of water was added, and the mixture was filtered. The filter cake was dried under reduced pressure and then purified by silica gel column chromatography (mobile phase: dichloromethane/methanol (V/V) = 100/1-20/1) to obtain **67C** (0.95 g, two-step yield: 66%).

LCMS m/z = 657.3 $[M+H]^+$.

Step 4: 5-(3-((4-(4-amino-5-methoxy-2-(1-methyl-1H-pyrazol-4-yl)phenyl) piperazin-1-yl)methyl)pyrrolidin-1-yl)-2-(2,6-dioxopiperidin-3-yl)isoindoline-1,3-dione **(67D)**

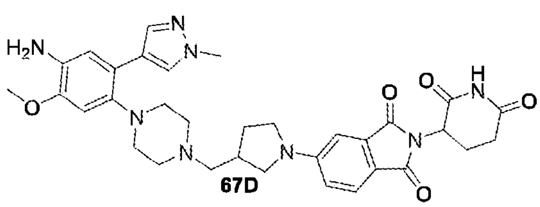

67D

**67C** (0.95 g, 1.45 mmol) was dissolved in ethanol/water (20 mL, 3:1); iron powder (375 mg, 6.71 mmol) and ammonium chloride (361 mg, 6.75 mmol) were successively added; and under nitrogen protection, the mixture was stirred at 80°C for 1 h, cooled to room temperature and subjected to suction filtration under reduced pressure. The filtrate

was concentrated under reduced pressure, and then the crude was purified by silica gel column chromatography (mobile phase: dichloromethane/methanol (V/V) = 100/1-20/1) to obtain **67D** (700 mg, yield: 77%).

LCMS m/z = 627.3 $[M+H]^+$.

Step 5: 5-(3-((4-(4-((5-bromo-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperazin-1-yl)methyl)pyrrolidin-1-yl)-2-(2,6-dioxopiperidin-3-yl)isoindoline-1,3-dione **(compound 67)**

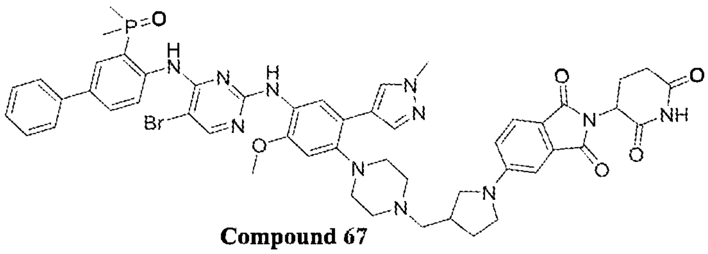

Compound 67

**67D** (200 mg, 0.32 mmol) and (4-((5-bromo-2-chloropyrimidin-4-yl)amino)-[1,1'-biphenyl]-3-yl)dimethylphosphine oxide **(23D)** (148 mg, 0.34 mmol) were dissolved in DMF (10 mL); p-toluenesulfonic acid monohydrate (160 mg, 0.84 mmol) was added; and under nitrogen protection, the mixture was stirred at 100°C for 16 h, and cooled to room temperature. 10 mL of saturated aqueous sodium bicarbonate solution and 20 mL of dichloromethane were added, and the layers were separated. The organic layer was concentrated under reduced pressure. The concentrated solution was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 ml). Liquid separation was performed. The organic layer was then dried over anhydrous sodium sulphate and concentrated under reduced pressure to obtain **compound 67** (40 mg, yield: 12%).

LCMS m/z = 513.7 $[(M+2H)/2]^+$.

$^1H$ NMR (400 MHz, DMSO-$d_6$) δ **11.12 (s, 1H), 11.04 (s, 1H), 8.45** - 8.34 (m, 1H), 8.28 (s, 1H), 8.19 (s, 1H), 8.03 (s, 1H), 7.88 (s, 1H), 7.70 (dd, 1H), 7.65 (d, 1H), 7.60 (s, 1H), 7.55 (d, 2H), 7.45 (t, 2H), 7.33 (t, 1H), 7.23 - 7.11 (m, 1H), 6.91 (d, 1H), 6.87 (s, 1H), 6.82 (dd, 1H), 5.05 (dd, 1H), 3.78 (d, 6H), 3.64 - 3.48 (m, 2H), 3.48 - 3.37 (m, 1H), 3.18 (dd, 1H), 3.00 - 2.77 (m, 5H), 2.71 - 2.52 (m, 7H), 2.47 - 2.40 (m, 2H), 2.21 - 2.09 (m, 1H), 2.07 - 1.95 (m, 1H), 1.90 - 1.73 (m, 7H).

**Example 68: Preparation of compound 68**

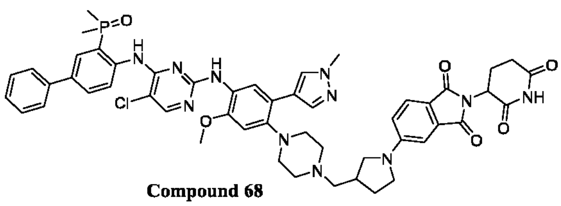

Compound 68

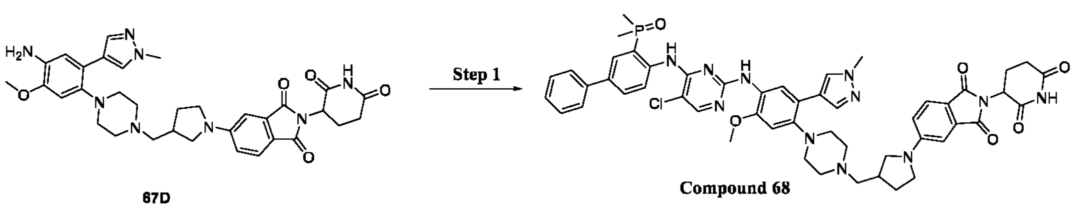


67D

Compound 68

Step 1: 5-(3-((4-(4-((5-chloro-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperazin-1-yl)methyl)pyrrolidin-1-yl)-2-(2,6-dioxopiperidin-3-yl)isoindoline-1,3-dione **(compound 68)**

**67D** (200 mg, 0.32 mmol) and (4-((2,5-dichloropyrimidin-4-yl)amino)-[1,1'-biphenyl]-3-yl)dimethylphosphine oxide **(40A)** (180 mg, 0.46 mmol) were dissolved in DMF (10 mL); p-toluenesulfonic acid monohydrate (160 mg, 0.84 mmol) was added; and under nitrogen protection, the mixture was stirred at 100°C for 16 h, and cooled to room temperature. 10 mL of saturated aqueous sodium bicarbonate solution and 20 mL of dichloromethane were added, and the layers were separated. The organic layer was concentrated under reduced pressure. The concentrated solution was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 ml). Liquid separation was performed. The organic layer was then dried over anhydrous sodium sulphate and concentrated under reduced pressure to obtain **compound 68** (30 mg, yield: 10%).

LCMS m/z = 491.7 $[(M+2H)/2]^+$.

$^1H$ NMR (400 MHz, DMSO-$d_6$) δ **11.37 (s, 1H), 11.05 (s, 1H), 8.54** - 8.45 (m, 1H), 8.27 (s, 1H), 8.12 (s, 1H), 8.05 (s, 1H), 7.89 (s, 1H), 7.71 (dd, 1H), 7.65 (d, 1H), 7.62 (s, 1H), 7.55 (d, 2H), 7.45 (t, 2H), 7.34 (t, 1H), 7.21 - 7.10 (m, 1H), 6.90 (d, 1H), 6.88 (s, 1H), 6.81 (dd, 1H), 5.05 (dd, 1H), 3.78 (d, 6H), 3.61 - 3.48 (m, 2H), 3.48 - 3.37 (m, 1H), 3.18 (dd, 1H), 2.97 - 2.82 (m, 5H), 2.69 - 2.52 (m, 7H), 2.47 - 2.40 (m, 2H), 2.21 - 2.10 (m, 1H), 2.05 - 1.96 (m, 1H), 1.90 - 1.73 (m, 7H).

**Example 69: Preparation of compound 69**

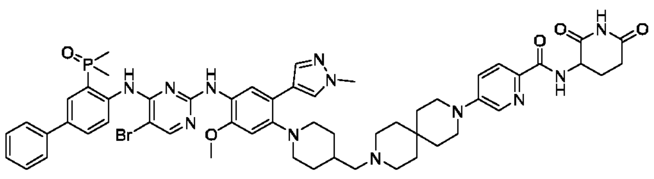

**Compound 69**

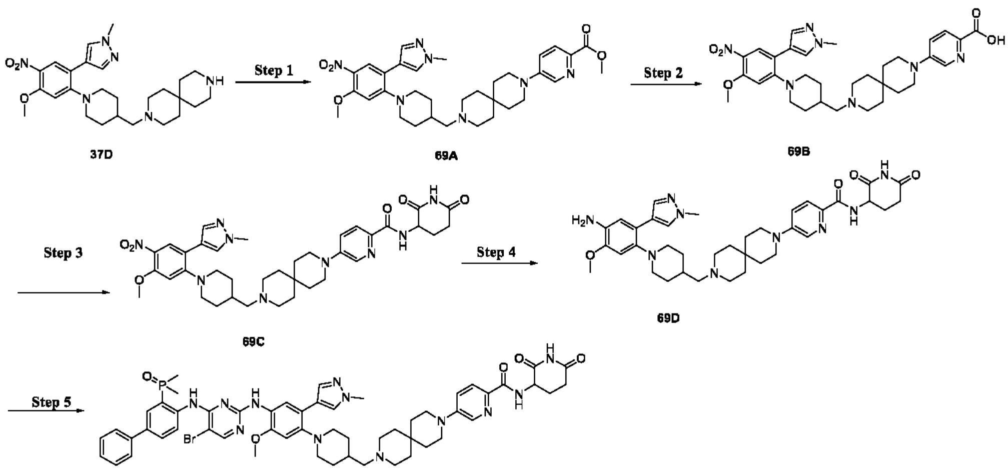


Compound 69

Step 1: methyl 5-(9-((1-(5-methoxy-2-(1-methyl-1H-pyrazol-4-yl)-4-nitrophenyl) piperidin-4-yl)methyl)-3,9-diazaspiro[5.5]undecan-3-yl)picolinate **(69A)**

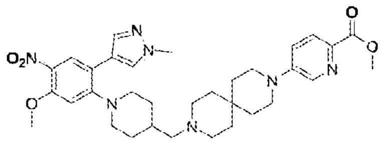

69A

**37D** (1.00 g, 2.07 mmol) was dissolved in DMSO (20 mL); methyl 5-fluoropyridine-2-carboxylate (388 mg, 2.50 mmol) and sodium carbonate (530 mg, 5.00 mmol) were successively added; and the mixture was stirred at 100°C for 5 h, and cooled to room temperature. 30 mL of water was added, and the mixture was filtered. The filter cake was dried under reduced pressure and then purified by silica gel column chromatography (mobile phase: dichloromethane/methanol (V/V) = 100/1-20/1) to obtain **69A** (0.95 g, yield: 74%).
LCMS m/z = 618.3 $[M+H]^+$.

Step 2: 5-(9-((1-(5-methoxy-2-(1-methyl-1H-pyrazol-4-yl)-4-nitrophenyl) piperidin-4-yl)methyl)-3,9-diazaspiro[5.5]undecan-3-yl)picolinic acid **(69B)**

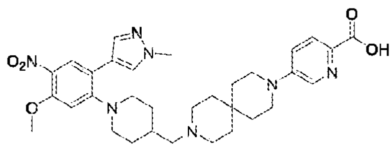

69B

**69A** (0.95 g, 1.54 mmol) was dissolved in tetrahydrofuran (20 mL); water (2 mL) was added, and then lithium hydroxide monohydrate (126 mg, 3.00 mmol) was added. The mixture was heated to 50°C and reacted for 4 h. After the reaction was completed, the mixture was adjusted to pH = 7 by dropwise adding dilute hydrochloric acid (2 mol/L) and concentrated under reduced pressure, and the residue was directly used in the next step.
LCMS m/z = 604.3 $[M+H]^+$.

Step 3: N-(2,6-dioxopiperidin-3-yl)-5-(9-((1-(5-methoxy-2-(1-methyl-1H-pyrazol-4-yl)-4-nitrophenyl)piperidin-4-yl)methyl)-3,9-diazaspiro[5.5]undecan-3-yl)picolinamide **(69C)**

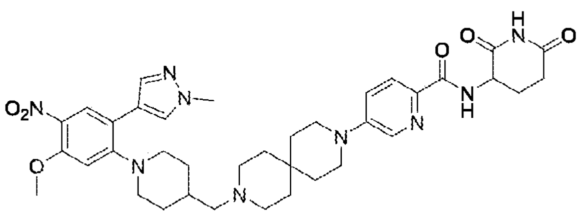

69C

The crude of **69B** from the previous step and 3-aminopiperidine-2,6-dione hydrochloride (0.30 g, 1.82 mmol) were dissolved in DMF (10 mL); DIPEA (0.50 g, 3.87 mmol) and HATU (0.70 g, 1.84 mmol) were added; and the mixture was reacted at room temperature for 3 h, and then extracted by adding 40 mL of saturated aqueous sodium bicarbonate solution and 50 ml of ethyl acetate. The organic phase was dried over anhydrous sodium sulphate and concentrated under reduced pressure, and the residue was purified by column chromatography (mobile phase: dichloromethane/methanol (V/V) = 50/1-10/1) to obtain **(69C)** (0.73 g, two-step yield: 66%).
LCMS m/z = 714.3 [M+H].

Step 4: 5-(9-((1-(4-amino-5-methoxy-2-(1-methyl-1H-pyrazol-4-yl)phenyl) piperidin -4-yl)methyl)-3,9-diazaspiro[5.5]undecan-3-yl)-N-(2,6-dioxopiperidin-3-yl)picolinamide **(69D)**

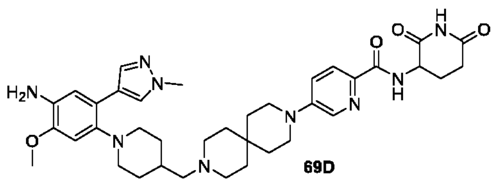

69D

**69C** (0.73 g, 1.02 mmol) was dissolved in ethanol/water (20 mL, 3:1); iron powder (375 mg, 6.71 mmol) and ammonium chloride (361 mg, 6.75 mmol) were successively added; and under nitrogen protection, the mixture was stirred at 80°C for 1 h, cooled to room temperature and subjected to suction filtration under reduced pressure. The filtrate was concentrated under reduced pressure, and then the crude was purified by silica gel column chromatography (mobile phase: dichloromethane/methanol (V/V) = 100/1-20/1) to obtain **69D** (600 mg, yield: 86%).

LCMS m/z = 342.8 $[(M+2H)/2]^+$.

Step 5: 5-(9-((1-(4-((5-bromo-4-((3-(dimethylphosphoryl)-[1,1'-biphenyl]-4-yl) amino)pyrimidin-2-yl)amino)-5-methoxy-2-(1-methyl-1H-pyrazol-4-yl)phenyl)piperidin-4-yl)methyl)-3,9-diazaspiro[5.5]undecan-3-yl)-N-(2,6-dioxopiperidin-3-yl)picolinamide **(compound 69)**

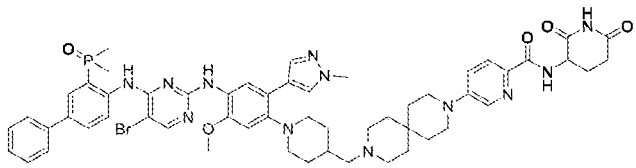

**Compound 69**

**69D** (200 mg, 0.29 mmol) and (4-((5-bromo-2-chloropyrimidin-4-yl)amino)-[1,1'-biphenyl]-3-yl)dimethylphosphine oxide **(23D)** (148 mg, 0.34 mmol) were dissolved in DMF (10 mL); p-toluenesulfonic acid monohydrate (160 mg, 0.84 mmol) was added; and under nitrogen protection, the mixture was stirred at 100°C for 16 h, and cooled to room temperature. 10 mL of saturated aqueous sodium bicarbonate solution and 20 mL of dichloromethane were added, and the layers were separated. The organic layer was concentrated under reduced pressure. The concentrated solution was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 ml). Liquid separation was performed. The organic layer was then dried over anhydrous sodium sulphate and concentrated under reduced pressure to obtain **compound 69** (45 mg, yield: 14%).

LCMS m/z = 542.3 $[(M+2H)/2]^+$.

$^1$H NMR (400 MHz, DMSO-$d_6$) δ **11.12 (s, 1H), 10.82 (s, 1H), 8.68 (d, 1H),** 8.42 - 8.35 (m, 1H), 8.30 (d, 2H), 8.18 (s, 1H), 8.01 (s, 1H), 7.87 - 7.82 (m, 2H), 7.69 (dd, 1H), 7.58 - 7.52 (m, 3H), 7.47 - 7.33 (m, 4H), 7.23 - 7.15 (m, 1H), 6.85 (s, 1H), 4.78 - 4.68 (m, 1H), 3.79 (s, 3H), 3.76 (s, 3H), 3.42 - 3.32 (m, 4H), 3.06 (d, 2H), 2.84 - 2.73 (m, 1H), 2.64 - 2.52 (m, 3H), 2.36 (s, 4H), 2.24 - 2.13 (m, 3H), 2.05 - 1.98 (m, 1H), 1.83 (s, 3H), 1.81 (s, 3H), 1.71 (d 2H), 1.59 - 1.49 (m, 8H), 1.32 - 1.22 (m, 3H).

**Example 70: Preparation of compound 70**

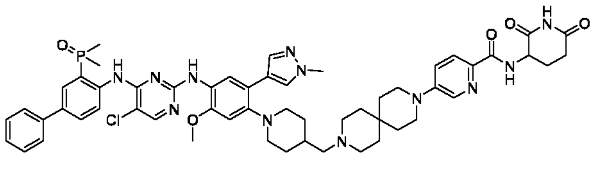

Compound 70

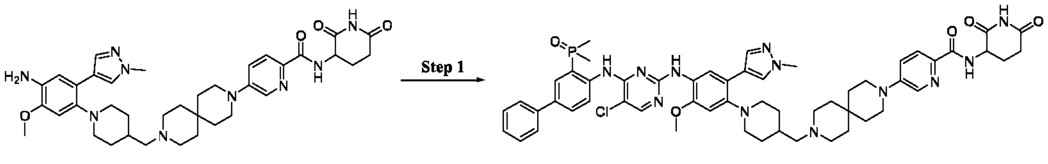

69D

Compound 70

Step 1: 5-(9-((1-(4-((5-chloro-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperidin-4-yl)methyl)-3,9-diazaspiro[5.5]undecan-3-yl)-N-(2,6-dioxopiperidin-3-yl)picolinamide **(compound 70)**

**69D** (200 mg, 0.29 mmol) and (4-((2,5-dichloropyrimidin-4-yl)amino)-[1,1'-biphenyl]-3-yl)dimethylphosphine oxide **(40A)** (180 mg, 0.46 mmol) were dissolved in DMF (10 mL); p-toluenesulfonic acid monohydrate (160 mg, 0.84 mmol) was added; and under nitrogen protection, the mixture was stirred at 100°C for 16 h, and cooled to room temperature. 10 mL of saturated aqueous sodium bicarbonate solution and 20 mL of dichloromethane were added, and the layers were separated. The organic layer was concentrated under reduced pressure. The concentrated solution was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 mL). Liquid separation was performed. The organic layer was then dried over anhydrous sodium sulphate and concentrated under reduced pressure to obtain **compound 70** (40 mg, yield: 13%).

LCMS m/z = 520.3 $[(M+2H)/2]^+$.

$^1H$ NMR (400 MHz, DMSO-$d_6$) $\delta$ **11.37 (s, 1H), 10.82 (s, 1H), 8.68 (d, 1H),** 8.51 - 8.45 (m, 1H), 8.32 - 8.27 (m, 2H), 8.11 (s, 1H), 8.03 (s, 1H), 7.88 - 7.82 (m, 2H), 7.71 (dd, 1H), 7.60 - 7.53 (m, 3H), 7.47 - 7.32 (m, 4H), 7.21 - 7.10 (m, 1H), 6.85 (s, 1H), 4.78 - 4.68 (m, 1H), 3.79 (s, 3H), 3.76 (s, 3H), 3.38 - 3.33 (m, 4H), 3.07 (d, 2H), 2.83 - 2.75 (m, 1H), 2.66 - 2.53 (m, 3H), 2.42 - 2.30 (m, 4H), 2.27 - 2.14 (m, 3H), 2.05 - 1.99 (m, 1H), 1.85 (s, 3H), 1.82 (s, 3H), 1.72 (d, 2H), 1.59 - 1.48 (m, 8H), 1.32 - 1.18 (m, 3H).

**Example 71: Preparation of compound 71**

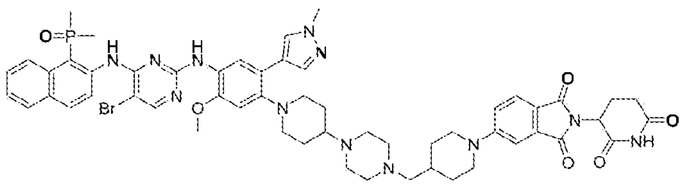

Compound 71

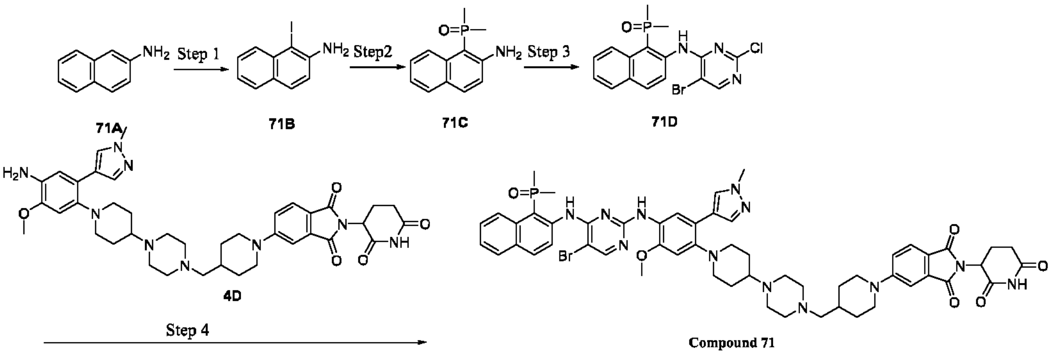


Step 1: 1-iodonaphthalen-2-amine **(71B)**

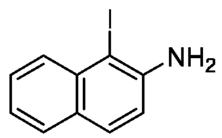

**71B**

**71A** (2.00 g, 13.97 mmol) was dissolved in DMSO (30 mL), and NIS (3.14 g, 13.97 mmol) was added at room temperature. After the addition, the mixture was reacted at 20°C for 2 h. The reaction solution was diluted by adding 50 mL of ethyl acetate, washed 3 times with water and washed once with saturated sodium chloride. The organic phases were collected, dried over anhydrous sodium sulphate and concentrated under reduced pressure. The residue was purified by column chromatography (mobile phase: petroleum ether/ethyl acetate (V/V) = 7/1) to obtain **71B** (2.69 g, yield: 72%).
LCMS m/z = 270.0$[M+H]^+$.

Step 2: (2-aminonaphthalen-1-yl)dimethylphosphine oxide **(71C)**

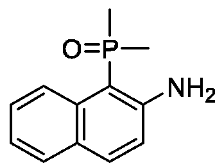

**71C**

**71B** (2.68 g, 9.96 mmol) and dimethylphosphine oxide (0.78 g, 9.96 mmol) were dissolved in 1,4-dioxane (80 mL); 4,5-bisdiphenylphosphine-9,9-dimethylxanthene (1.15 g, 1.99 mmol), palladium acetate (0.22 g, 1.00 mmol) and potassium phosphate (4.23 g, 19.92 mmol) were added; and the mixture was subjected to nitrogen replacement 3 times and reacted at 100°C for 5 h. The reaction solution was cooled to room temperature, diluted by adding 200 mL of ethyl acetate, washed 3 times with water and washed once with saturated sodium chloride. The organic phase was dried over anhydrous sodium sulphate and concentrated under reduced pressure, and the residue was purified by column chromatography (mobile phase: dichloromethane/methanol (V/V) = 50/1-15/1) to obtain **71C** (1.68 g, yield: 77%).
LCMS m/z = 220.1$[M+H]^+$.

Step 3: (2-((5-bromo-2-chloropyrimidin-4-yl)amino)naphthalen-1-yl) dimethylphosphine oxide **(71D)**

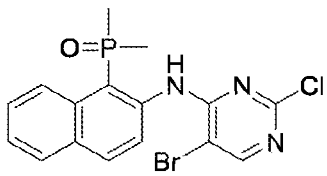

**71D**

**71C** (0.87 g, 3.97 mmol) and 5-bromo-2,4-dichloropyrimidine (1.81 g, 7.94 mmol) were dissolved in NMP (25 mL); DIPEA (1.03 g, 7.94 mmol) was added dropwise; and the mixture was reacted at 130°C for 3 h. The reaction solution was cooled to room temperature, diluted by adding 200 mL of ethyl acetate, washed 3 times with water and washed once with saturated sodium chloride. The organic phase was dried over anhydrous sodium sulphate and concentrated under reduced pressure, and the residue was purified by column chromatography (mobile phase: dichloromethane/methanol (V/V) = 50/1-15/1) to obtain **71D** (580 mg, yield: 36%).

LCMS m/z = 410.0 $[M+H]^+$.

Step 4: 5-(4-((4-(1-(4-((5-bromo-4-((1-(dimethylphosphoryl)naphthalen-2-yl) amino)pyrimidin-2-yl)amino)-5-methoxy-2-(1-methyl-1H-pyrazol-4-yl)phenyl)piperidin-4-yl)piperazin-1-yl)methyl)piperidin-1-yl)-2-(2,6-dioxopiperidin-3-yl)isoindoline-1,3-dione **(compound 71)**

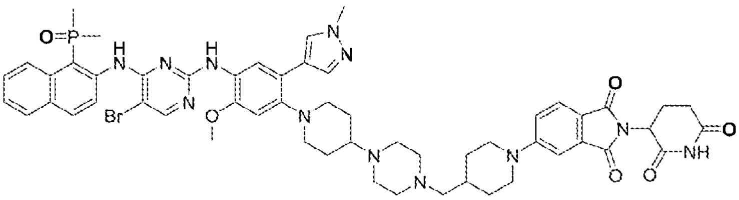

**Compound 71**

**71D** (0.17 g, 0.41 mmol) and **4D** (0.30 g, 0.41 mmol) were dissolved in DMF (15 mL); p-toluenesulfonic acid hydrate (0.23 g, 1.23 mmol) was added; and under nitrogen protection, the mixture was reacted at 100°C for 16 h. The reaction solution was cooled to room temperature. 10 mL of saturated aqueous sodium bicarbonate solution was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase: dichloromethane/methanol (V/V) = 50/1-15/1) to obtain a product, which was further purified by preparative HPLC (composition of mobile phases: mobile phase A: acetonitrile, and mobile phase B: water (containing 0.05% trifluoroacetic acid)) to obtain a product, which was alkalized with aqueous sodium bicarbonate solution, extracted with dichloromethane, concentrated and dried to obtain **compound 71** (30 mg, yield: 7%).

LCMS m/z = 549.4$[(M+2H)/2]^+$.

$^1$H NMR (400 MHz, DMSO-$d_6$) δ **12.46 (s, 1H), 11.05 (s, 1H), 8.33** - 8.24 (m, 1H), 8.20 (s, 1H), 8.10 (s, 1H), 7.93 (d, 1H), 7.89 - 7.77 (m, 2H), 7.72 - 7.49 (m, 4H), 7.49 - 7.33 (m, 2H), 7.34 - 7.17 (m, 2H), 6.78 (s, 1H), 5.06 (dd, 1H), 4.15 - 3.99 (m, 2H), 3.79 (s, 3H), 3.72 (s, 3H), 3.16 - 3.02 (m, 2H), 3.03 - 2.81 (m, 3H), 2.69 - 2.51 (m, 8H), 2.45 - 2.18 (m, 5H), 2.14 (d, 2H), 2.09 - 1.92 (m, 7H), 1.92 - 1.73 (m, 5H), 1.63 - 1.43 (m, 2H), 1.22 - 1.06 (m, 2H).

**Example 72: Preparation of compound 72**

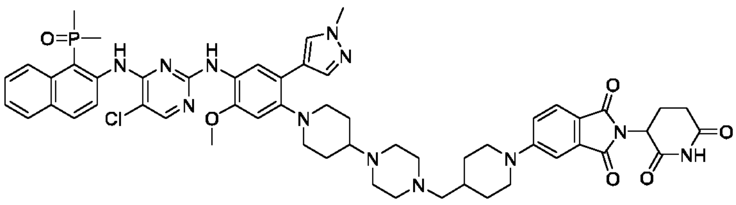

Compound 72

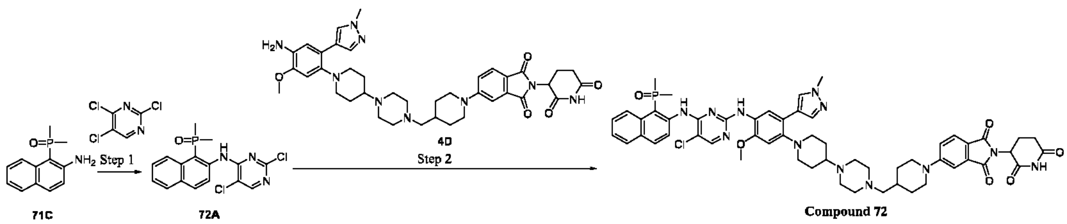

Step 1: (2-((2,5-dichloropyrimidin-4-yl)amino)naphthalen-1-yl)dimethylphosphine oxide **(72A)**

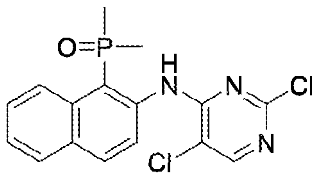

**72A**

71C (0.87 g, 3.97 mmol) and 2,4,5-trichloropyrimidine (1.46 g, 7.94 mmol) were dissolved in NMP (25 mL); DIPEA (1.03 g, 7.94 mmol) was added dropwise; and the mixture was reacted at 130°C for 3 h. The reaction solution was cooled to room temperature, diluted by adding 200 mL of ethyl acetate, washed 3 times with water and washed once with saturated sodium chloride. The organic phase was dried over anhydrous sodium sulphate and concentrated under reduced pressure, and the residue was purified by column chromatography (mobile phase: dichloromethane/methanol (V/V) = 50/1-15/1) to obtain **72A** (720 mg, yield: 50%).

LCMS m/z = 366.0 $[M+H]^+$.

Step 2: 5-(4-((4-(1-(4-((5-chloro-4-((1-(dimethylphosphoryl)naphthalen-2-yl) amino)pyrimidin-2-yl)amino)-5-methoxy-2-(1-methyl-1H-pyrazol-4-yl)phenyl)piperidin-4-yl)piperazin-1-yl)methyl)piperidin-1-yl)-2-(2,6-dioxopiperidin-3-yl)isoindoline-1,3-dione **(compound 72)**

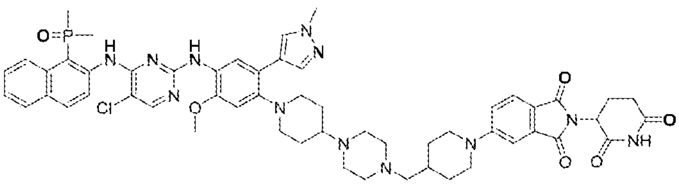

Compound 72

**72A** (0.15 g, 0.41 mmol) and **4D** (0.30 g, 0.41 mmol) were dissolved in DMF (15 mL); p-toluenesulfonic acid hydrate (0.23 g, 1.23 mmol) was added; and under nitrogen protection, the mixture was reacted at 100°C for 16 h. The reaction solution was cooled to room temperature. 10 mL of saturated aqueous sodium bicarbonate solution was added, with a yellow solid precipitated, and the mixture was subjected to suction filtration. The filter cake was washed 3 times with water, redissolved in dichloromethane, dried over anhydrous sodium sulphate and concentrated under reduced

pressure, and the residue was purified by column chromatography (mobile phase: dichloromethane/methanol (V/V) = 50/1-15/1) to obtain a product, which was further purified by preparative HPLC (composition of mobile phases: mobile phase A: acetonitrile, and mobile phase B: water (containing 0.05% trifluoroacetic acid)) to obtain a product, which was alkalized with aqueous sodium bicarbonate solution, extracted with dichloromethane, concentrated and dried to obtain **compound 72** (40 mg, yield: 9%).

LCMS m/z = 527.3 $[(M+2H)/2]^+$.

$^1H$ NMR (400 MHz, DMSO-$d_6$) $\delta$ **12.69 (s, 1H), 11.06 (s, 1H), 8.49** - 8.40 (m, 1H), 8.19 - 8.03 (m, 2H), 7.97 - 7.79 (m, 3H), 7.76 - 7.50 (m, 4H), 7.49 - 7.32 (m, 2H), 7.30 (d, 1H), 7.27 - 7.18 (m, 1H), 6.79 (s, 1H), 5.06 (dd, 1H), 4.11 - 3.97 (m, 2H), 3.80 (s, 3H), 3.72 (s, 3H), 3.14 - 2.81 (m, 5H), 2.70 - 2.51 (m, 8H), 2.48 - 2.18 (m, 5H), 2.14 (d, 2H), 2.10 - 1.93 (m, 7H), 1.90 - 1.69 (m, 5H), 1.66 - 1.45 (m, 2H), 1.21 - 1.08 (m, 2H).

**Example 73: Preparation of compound 73**

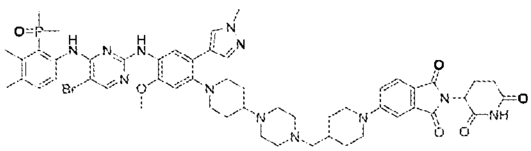

Compound 73

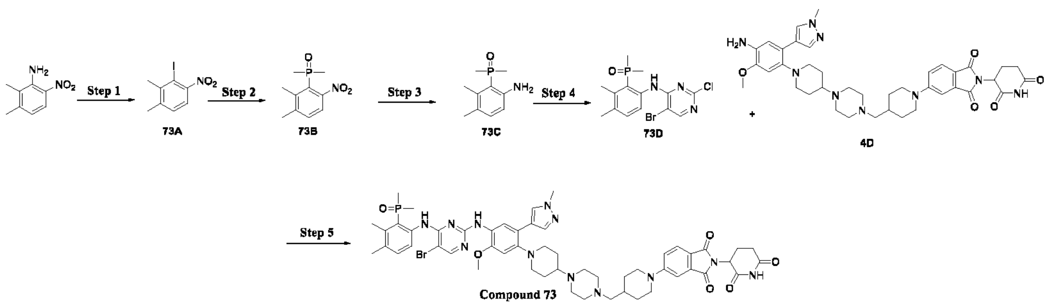


Step 1: 2-iodo-3,4-dimethyl-1-nitrobenzene **(73A)**

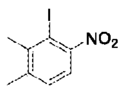

73A

To a reaction flask were added 2,3-dimethyl-6-nitroaniline (6.0 g, 36.14 mmol) and hydrochloric acid (30 mL, 360.14 mmol). At 0°C, a solution of potassium nitrate (2.99 g, 43.33 mmol) in water (15 mL) was slowly added dropwise. At this temperature, the mixture was stirred for 1 h, and then a solution of potassium iodide (9.0 g, 54.22 mmol) in water (30 mL) was added. After the addition, the mixture was naturally warmed to room temperature and stirred for another 1 h. After the reaction was completed, 60 mL of water was added, and the mixture was extracted with ethyl acetate (60 mL × 3). The organic layer was washed sequentially with saturated sodium thiosulphate solution (40 mL × 2) and saturated brine (40 mL × 2), dried over anhydrous sodium sulphate and concentrated under reduced pressure, and the residue was purified by column chromatography (petroleum ether: ethyl acetate (V/V) = 10/1) to obtain **73A** (5.3 g, yield: 53%).

Step 2: (2,3-dimethyl-6-nitrophenyl)dimethylphosphine oxide **(73B)**

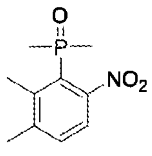

73B

Under nitrogen protection, **73A** (5.29 g, 19.09 mmol), dimethylphosphine oxide (1.94 g, 24.82 mmol), potassium phosphate (8.1 g, 38 mmol), palladium acetate (429 mg, 1.91 mmol) and 4,5-bisdiphenylphosphine-9,9-dimethylxanthene (2.21 g, 3.82 mmol) were added to 1,4-dioxane (150 mL), and the mixture was reacted at 100°C for 16 h. After the reaction was completed, the mixture was cooled to room temperature. Water (50 mL) was added, and the mixture was extracted with ethyl acetate (50 mL × 3). The organic phase was washed with water (50 mL × 2) and saturated brine (20 mL), dried over anhydrous sodium sulphate and concentrated, and the residue was purified by silica gel column chromatography (methanol/dichloromethane (V/V) = 1/50-1/20) to obtain **73B** (2.7 g, yield: 62.3%).
LCMS m/z = 228.1 $[M+H]^+$.

Step 3: (6-amino-2,3-dimethylphenyl)dimethylphosphine oxide **(73C)**

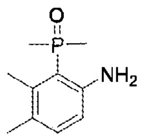

73C

**73B** (2.7 g, 11.88 mmol) was dissolved in ethanol/water (30 mL/10 mL); iron powder (5.31 g, 95 mmol) and ammonium chloride (5.08 mg, 95 mmol) were successively added; and under nitrogen protection, the mixture was stirred at 80°C for 3 h, cooled to room temperature and subjected to suction filtration under reduced pressure. The filtrate was concentrated under reduced pressure, and then the crude was purified by silica gel column chromatography (mobile phase: dichloromethane/methanol (V/V) = 100/1-20/1) to obtain **73C** (1.3 g, yield: 98.2%).
LCMS m/z = 198.1 $[M+H]^+$.

Step 4: (6-((5-bromo-2-chloropyrimidin-4-yl)amino)-2,3-dimethylphenyl) dimethylphosphine oxide **(73D)**

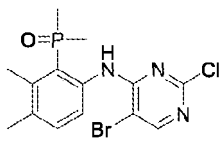

73D

(6-amino-2,3-dimethylphenyl)dimethylphosphine oxide **(73C)** (2.5 g, 12.7 mmol) and 5-bromo-2,4-dichloropyrimidine (4.3 g, 19.0 mmol) were dissolved in DMF (20 mL); potassium carbonate (3.5 mg, 25.4 mmol) was added; and under nitrogen protection, the mixture was stirred at 100°C for 16 h, cooled to room temperature and extracted by adding 80 mL of ethyl acetate and 80 mL of water. The organic layer was washed with saturated brine (30 mL × 3), dried over anhydrous sodium sulphate and then concentrated under reduced pressure, and the residue was purified by silica gel column chromatography (methanol/dichloromethane (V/V) = 1/50-1/20) to obtain **73D** (3.5 g, yield: 71.2%).
LCMS m/z = 388.0 $[M+H]^+$.

Step 5: 5-(4-((4-(1-(4-((5-bromo-4-((2-(dimethylphosphoryl)-3,4-dimethylphenyl) amino)pyrimidin-2-yl)amino)-5-methoxy-2-(1-methyl-1H-pyrazol-4-yl)phenyl)piperidin-4-yl)piperazin-1-yl)methyl)piperidin-1-yl)-2-(2,6-dioxopiperidin-3-yl)isoindoline-1,3-dione **(compound 73)**

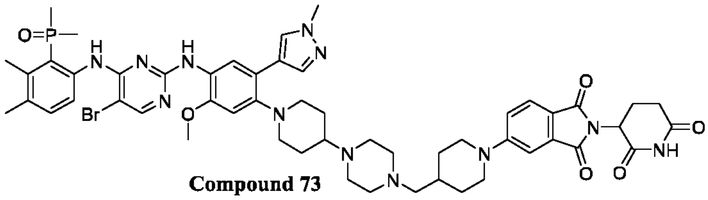

**Compound 73**

**4D** (250 mg, 0.35 mmol) and **73D** (160 mg, 0.42 mmol) were dissolved in 1,4-dioxane and NMP (4 mL/1 mL); p-toluenesulfonic acid monohydrate (200 mg, 1.05 mmol) was added; and under nitrogen protection, the mixture was stirred at 100°C for 16 h, and cooled to room temperature. 10 mL of saturated aqueous sodium bicarbonate solution and 30 mL of dichloromethane were added, and the layers were separated. The organic layer was concentrated under reduced pressure, and the residue was purified by silica gel column chromatography to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 ml). Liquid separation was performed. The organic layer was then dried over anhydrous sodium sulphate and concentrated under reduced pressure to obtain **compound 73** (70 mg, yield: 18.6%).

LCMS m/z = 1075.4 $[M+H]^+$.

$^1H$ NMR (400 MHz, DMSO-$d_6$) δ **11.91 (s, 1H), 11.05 (s, 1H), 8.11 (s, 1H),** 8.03 - 7.84 (m, 3H), 7.75 (s, 1H), 7.70 - 7.53 (m, 2H), 7.30 (s, 1H), 7.22 (dd, 1H), 6.85 - 6.56 (m, 2H), 5.06 (dd, 1H), 4.13 - 3.94 (m, 2H), 3.84 (s, 3H), 3.78 (s, 3H), 3.15 - 3.03 (m, 2H), 2.96 (t, 2H), 2.91 - 2.80 (m, 1H), 2.72 - 2.51 (m, 8H), 2.46 - 2.31 (m, 4H), 2.28 - 2.19 (m, 4H), 2.18 - 2.10 (m, 2H), 2.07 (s, 3H), 2.04 - 1.95 (m, 1H), 1.93 - 1.71 (m, 11H), 1.65 - 1.42 (m, 2H), 1.21 - 1.03 (m, 2H).

**Example 74: Preparation of compound 74**

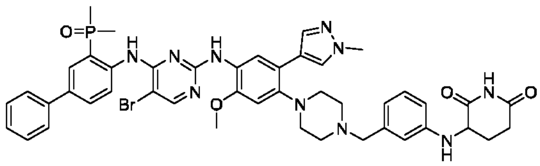

**Compound 74**

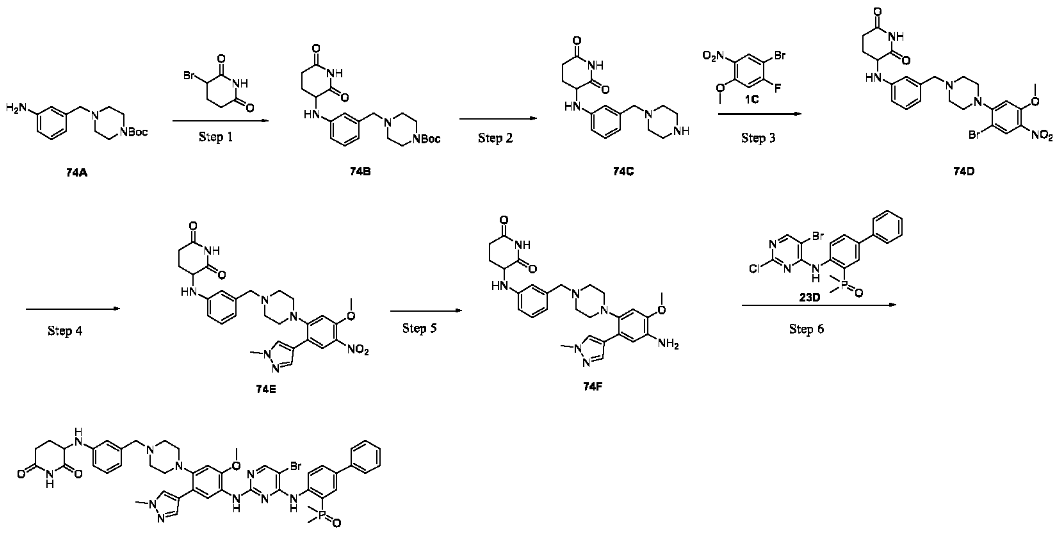


Compound 74

Step 1: tert-butyl 4-(3-((2,6-dioxopiperidin-3-yl)amino)benzyl)piperazine-1-carboxylate **(74B)**

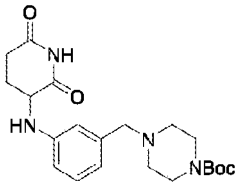

**74B**

**74A** (synthesized with reference to patent WO 2017018803, 3 g, 10.30 mmol) and 3-bromopiperidine-2,6-dione (3.96 g, 20.6 mmol) were dissolved in DMSO (30 mL); sodium bicarbonate (4.33 g, 51.5 mmol) was added; and the mixture was reacted at 100°C for 6 h, cooled to room temperature and extracted by adding 100 mL of ethyl acetate and 100 mL of water. The organic layer was washed with saturated brine (30 mL × 3), dried over anhydrous sodium sulphate and concentrated under reduced pressure, and the residue was separated and purified by silica gel column chromatography (ethyl acetate: petroleum ether (V/V) = 10/1-2/1) to obtain **74B** (2.57 g, yield: 62%).

LCMS m/z = 403.1$[M+H]^+$.

Step 2: 3-((3-(piperazin-1-ylmethyl)phenyl)amino)piperidine-2,6-dione **(74C);** HCl

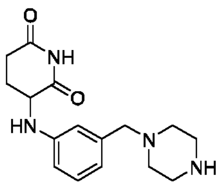

**74C**

To a 50 mL round bottom flask were successively added **74B** (1.06 g, 2.63 mmol) and a solution of hydrogen chloride in 1,4-dioxane (30 mL, 4 mol/L); and the mixture was reacted at room temperature for 2 h and concentrated to dryness under reduced pressure to obtain the hydrochloride of **74C,** which was directly used in the next reaction.

Step 3: 3-((3-((4-(2-bromo-5-methoxy-4-nitrophenyl)piperazin-1-yl)methyl) phenyl)amino)piperidine-2,6-dione **(74D)**

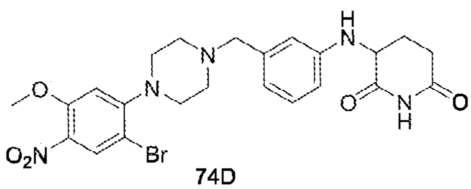

74D

1-bromo-2-fluoro-4-methoxy-5-nitrobenzene **(1C)** (0.6 g, 2.40 mmol), the hydrochloride of **74C** from the previous step and sodium bicarbonate (1.01 g, 12 mmol) were dissolved in 20 mL of DMSO, and the mixture was reacted at 100°C for 3 h. After the reaction was completed, the mixture was cooled to room temperature and extracted by adding 50 mL of water and 50 mL of ethyl acetate. The aqueous layer was extracted with ethyl acetate (50 mL × 3). The organic phases were combined, washed sequentially with water (30 mL × 2) and saturated brine (30 mL), dried over anhydrous sodium sulphate and concentrated under reduced pressure, and the residue was purified by silica gel column chromatography (mobile phase: ethyl acetate/petroleum ether (V/V) = 1/1 to dichloromethane/methanol (V/V) = 20/1) to obtain **74D** (1.0 g, yield: 71%).

Step 4: 3-((3-((4-(5-methoxy-2-(1-methyl-1H-pyrazol-4-yl)-4-nitrophenyl) piperazin-1-yl)methyl)phenyl)amino)piperidine-2,6-dione **(74E)**

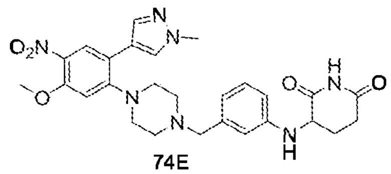

74E

Under nitrogen protection, **74D** (202 mg, 0.38 mmol) and 1-methyl-1H-pyrazole-4-boronic acid (95.7 mg, 0.76 mmol) were added to a 50 mL single-necked flask and dissolved in 20 mL of dioxane and 4 mL of water; Pd(dppf)$Cl_2$·DCM (31 mg, 0.04 mmol) and sodium bicarbonate (96 mg, 1.14 mmol) were added; and the mixture was subjected to nitrogen replacement three times, reacted at 100°C for 3 h and cooled to room temperature. The reaction solution was poured into water and extracted three times with ethyl acetate. The organic phases were combined, dried over anhydrous sodium sulphate and concentrated under reduced pressure, and the residue was purified by silica gel column chromatography to obtain **74E** (190 mg, yield: 94%).

LCMS m/z = 534.2 $[M+H]^+$.

Step 5: 3-((3-((4-(4-amino-5-methoxy-2-(1-methyl-1H-pyrazol-4-yl)phenyl) piperazin-1-yl)methyl)phenyl)amino)piperidine-2,6-dione **(74F)**

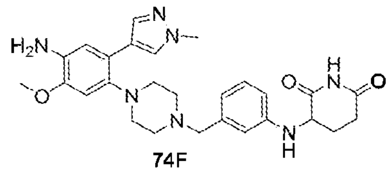

74F

**74E** (190 mg, 0.36 mmol) was dissolved in methanol (10 mL); palladium on carbon 10% (190 mg) was added at room temperature; and the mixture was reacted under hydrogen atmosphere for 2 h. After the reaction was completed, the mixture was subjected to suction filtration over celite. The filtrate was concentrated to obtain **74F,** which was directly used in the next reaction.

LCMS m/z = 504.3 $[M+H]^+$.

Step 6: 3-((3-((4-(4-((5-bromo-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperazin-1-yl)methyl)phenyl)amino)piperidine-2,6-dione **(compound 74)**

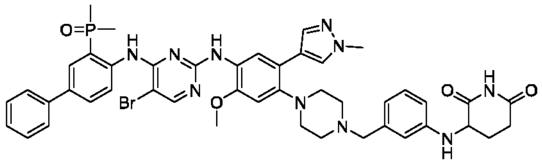

**Compound 74**

**74F** (101 mg, 0.2 mmol) from the previous step and (4-((5-bromo-2-chloropyrimidin-4-yl)amino)-[1,1'-biphenyl]-3-yl)dimethylphosphine oxide **(23D)** (105 mg, 0.24 mmol) were dissolved in DMF (10 mL); p-toluenesulfonic acid monohydrate (114 mg, 0.6 mmol) was added; and the mixture was stirred at 100°C for 16 h, and cooled to room temperature. 20 mL of saturated aqueous sodium bicarbonate solution and 50 mL of dichloromethane were added, and the layers were separated. The organic layer was concentrated under reduced pressure, and the residue was purified by silica gel column chromatography (mobile phase: DCM/MeOH (V/V) = 100/1-20/1). The crude was further subjected to preparative

HPLC (instrument: waters 2767 (preparative liquid phase chromatographic instrument); chromatographic column: XBridge@ Prep C18 (30 mm × 150 mm); composition of mobile phases: mobile phase A: acetonitrile, and mobile phase B: water (containing 0.1% TFA)) purification to obtain a preparative solution, which was concentrated. 50 mL of DCM was added, and then the mixture was adjusted to a basic pH by adding saturated sodium bicarbonate solution. The dichloromethane layers were separated, and the aqueous layer was extracted with dichloromethane (2 × 30 mL). The dichloromethane layers were combined, dried over anhydrous sodium sulphate, filtered and concentrated to obtain **compound 74** (15 mg, yield: 8.3%).

LCMS m/z = 452.3 $[(M+2H)/2]^+$.

$^1H$ NMR (400 MHz, DMSO-$d_6$) δ **11.11 (s, 1H), 10.76 (s, 1H), 8.46 -** 8.33 (m, 1H), 8.28 (s, 1H), 8.18 (s, 1H), 8.00 (s, 1H), 7.87 (s, 1H), 7.76 - 7.64 (m, 1H), 7.58 (s, 1H), 7.56 - 7.46 (m, 2H), 7.44 - 7.25 (m, 3H), 7.15 (s, 1H), 7.06 (t, 1H), 6.86 (s, 1H), 6.68(s, 1H), 6.64-6.53(m, 2H), 5.80 (d, 1H), 4.38-4.28 (m, 1H), 3.80 (s, 3H), 3.75 (s, 3H), 3.43 (s, 2H), 2.97 - 2.82 (m, 4H),2.81-2.69 (m, 1H), 2.67 - 2.52 (m, 5H), 2.18 - 2.07 (m, 1H), 1.94 - 1.75 (m, 7H).

**Example 75: Preparation of compound 75**

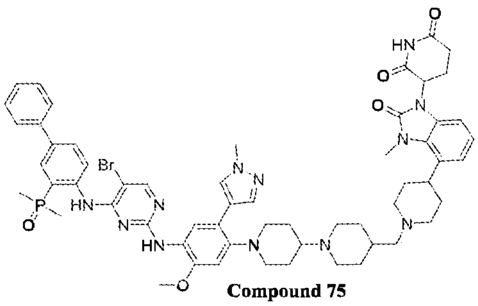

Compound 75

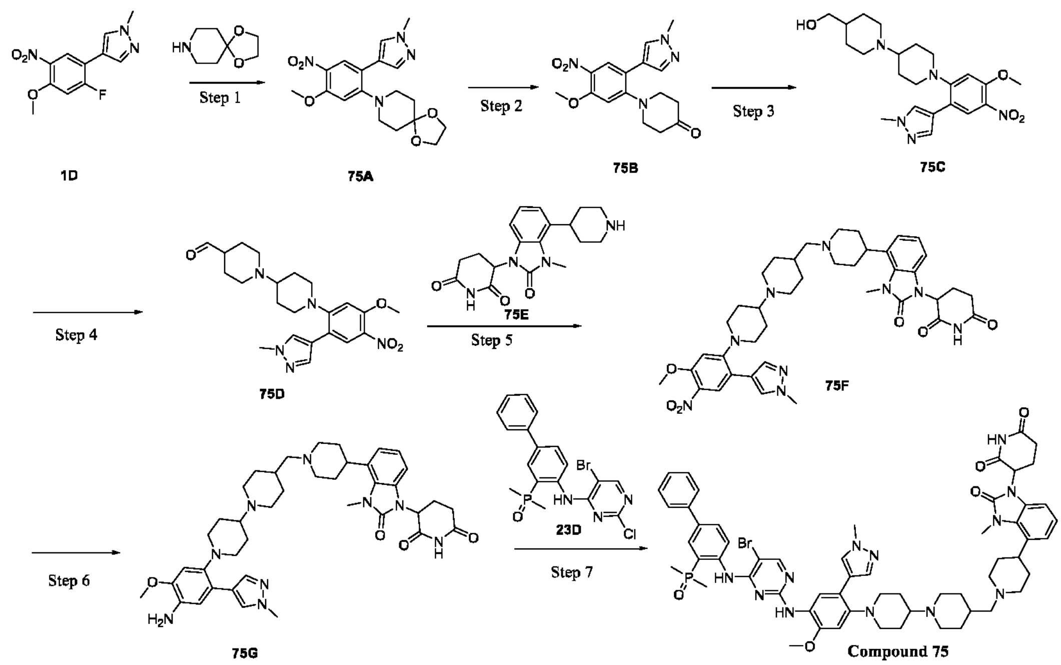


1D, 75A, 75B, 75C, 75D, 75E, 75F, 75G, 23D, Compound 75

Step 1: 8-(5-methoxy-2-(1-methyl-1H-pyrazol-4-yl)-4-nitrophenyl)-1,4-dioxa-8-azaspiro[4.5]decane **(75A)**

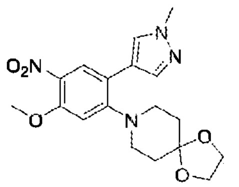

75A

1,4-dioxa-8-azaspiro[4.5]decane (1.37 g, 9.56 mmol), **1D** (1.2 g, 4.78 mmol) and potassium carbonate (3.30 g, 23.90 mmol) were mixed and dissolved in DMSO (30 mL), and the mixture was stirred at 120°C for 5 h, cooled to room temperature and extracted by adding 50 mL of water and 50 mL of ethyl acetate. The organic layer was concentrated under reduced pressure, and the residue was purified by column chromatography (mobile phase: ethyl acetate/petroleum ether (V/V) = 1/1) to obtain **75A** (1 g, yield: 56%).

LCMS m/z = 375.1 $[M+H]^+$.

Step 2: 1-(5-methoxy-2-(1-methyl-1H-pyrazol-4-yl)-4-nitrophenyl)piperidin-4-one **(75B)**

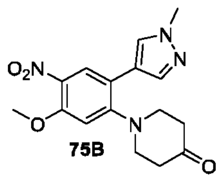

75B

To a 50 mL round bottom flask were successively added **75A** (1 g, 2.67 mmol) and an aqueous solution of acetone (30 mL) in hydrochloric acid (3 mL, 4 mol/L); and the mixture was reacted overnight at room temperature. 50 mL of DCM was added, and the mixture was adjusted to a basic pH by adding saturated sodium bicarbonate solution. The organic layer was separated, and the aqueous layer was extracted with DCM (2 × 30 mL). The organic layer was dried over anhydrous sodium sulphate, filtered and concentrated to obtain **75B,** which was directly used in the next reaction.

$^1H$ NMR (400 MHz, $CDCl_3$) δ **7.92 (s, 1H), 7.90 (s, 1H), 7.27 (s, 1H), 6.65 (s,** 1H), 3.98 (s, 3H), 3.97 (s, 3H), 3.31 (t, 4H), 2.53 (t, 4H).

Step 3: (1'-(5-methoxy-2-(1-methyl-1H-pyrazol-4-yl)-4-nitrophenyl)-[1,4'-bipiperidin]-4-yl)methanol **(75C)**

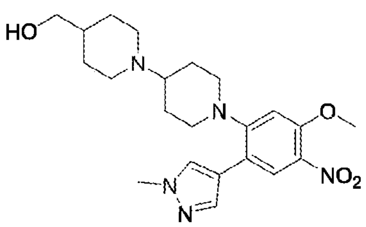

75C

**75B** (787 mg, 2.38 mmol) and piperidin-4-ylmethanol (329 mg, 2.86 mmol) were mixed in dichloroethane (50 mL); acetic acid (143 mg, 2.38 mmol) was added; and the mixture was reacted at 50°C for 2 h and cooled to room temperature. Sodium triacetoxyborohydride (1 g, 4.72 mmol) was added, and the mixture was stirred overnight at room temperature. 50 mL of dichloromethane and 50 mL of saturated aqueous sodium bicarbonate solution were added, and then the layers were separated. The organic layer was dried under reduced pressure, and the residue was purified by silica gel column chromatography (mobile phase: dichloromethane/methanol (V/V) = 100/1-10/1) to obtain **75C** (430 mg, yield: 42%).

LCMS m/z = 430.2 $[M+H]^+$.

Step 4: 1'-(5-methoxy-2-(1-methyl-1H-pyrazol-4-yl)-4-nitrophenyl)-[1,4'-bipiperidine]-4-carbaldehyde **(75D)**

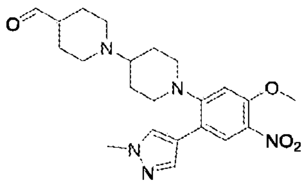

**75D**

**75C** (430 mg, 1.00 mmol) and Dess-Martin periodinane (1.27 g, 3 mmol) were dissolved in 20 mL of dichloromethane; and the mixture was reacted at room temperature for 2 h, concentrated and purified by silica gel column chromatography (mobile phase: dichloromethane/methanol (V/V) = 100/1-10/1) to obtain **75D** (150 mg, yield: 35%).

LCMS m/z = 428.3 $[M+H]^+$.

Step 5: 3-(4-(1-((1'-(5-methoxy-2-(1-methyl-1H-pyrazol-4-yl)-4-nitrophenyl)-[1,4'-bipiperidin]-4-yl)methyl)piperidin-4-yl)-3-methyl-2-oxo-2,3-dihydro-1H-benzo[d]imidazol-1-yl)piperidine-2,6-dione **(75F)**

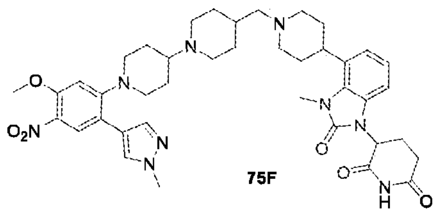

**75F**

**75E** (synthesized with reference to patent WO 2021127283, 156 mg, 0.46 mmol) and **75D** (150 mg, 0.35 mmol) were dissolved in DMAC (10 mL); a 4Å molecular sieve (200 mg) and acetic acid (21 mg, 0.35 mmol) were successively added; and the mixture was reacted at 50°C for 2 h and then cooled to room temperature. Sodium triacetoxyborohydride (148 mg, 0.70 mmol) was added, and the mixture was stirred overnight at room temperature. After the reaction was completed, the reaction was quenched by adding 50 ml of water, adjusted to a basic pH by adding saturated sodium bicarbonate solution, extracted with ethyl acetate (3 × 50 ml), dried over anhydrous sodium sulphate, filtered and concentrated, and the residue was purified by silica gel column chromatography (DCM: MeOH: $Et_3N$ = 10:1:0.01) to obtain **75F** (100 mg, yield: 38%).

Step 6: 3-(4-(1-((1'-(4-amino-5-methoxy-2-(1-methyl-1H-pyrazol-4-yl)phenyl) -[1,4'-bipiperidin]-4-yl)methyl)piperidin-4-yl)-3-methyl-2-oxo-2,3-dihydro-1H-benzo[d]imidazol-1-yl)piperidine-2,6-dione **(75G)**

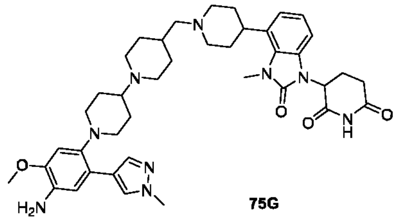

**75G**

**75F** (100 mg, 0.13 mmol) was dissolved in methanol (10 mL); palladium on carbon 10% (100 mg) was added at room temperature; and the mixture was reacted under hydrogen atmosphere for 2 h. After the reaction was completed, the mixture was subjected to suction filtration over celite. The filtrate was concentrated to obtain **75G,** which was directly used in the next reaction.

Step 7: 3-(4-(1-((1'-(4-((5-bromo-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)-[1,4'-bipiperidin]-4-yl)methyl)piperidin-4-yl)-3-methyl-2-oxo-2,3-dihydro-1H-benzo[d]imidazol-1-yl)piperidine-2,6-dione **(compound 75)**

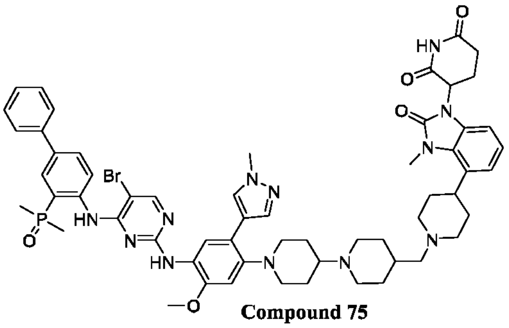

Compound 75

**75G** (85 mg, 0.12 mmol) and **23D** (63 mg, 0.14 mmol) were dissolved in DMF (10 mL); p-toluenesulfonic acid monohydrate (68 mg, 0.36 mmol) was added; and the mixture was stirred at 100°C for 16 h, and cooled to room temperature. 20 mL of saturated aqueous sodium bicarbonate solution and 50 mL of dichloromethane were added, and the layers were separated. The organic layer was concentrated under reduced pressure, and the residue was purified by silica gel column chromatography (mobile phase: DCM/MeOH (V/V) = 100/1-20/1). The crude was further subjected to preparative HPLC (instrument: waters 2767 (preparative liquid phase chromatographic instrument); chromatographic column: XBridge@ Prep C18 (30 mm × 150 mm); composition of mobile phases: mobile phase A: acetonitrile, and mobile phase B: water (containing 0.1% TFA)) purification to obtain a preparative solution, which was concentrated. 30 mL of DCM was added, and then the mixture was adjusted to a basic pH by adding saturated sodium bicarbonate solution. The dichloromethane layers were separated, and the aqueous layer was extracted with dichloromethane (2 × 30 mL). The dichloromethane layers were combined, dried over anhydrous sodium sulphate, filtered and concentrated to obtain **compound 75** (5 mg, yield: 3.7%).

$^1$H NMR (400 MHz, DMSO-$d_6$) δ **11.10** (s, 1H), 8.45 - 8.33 (s, 1H), 8.28 (s, 1H), 8.18 (s, 1H), 8.00 (s, 1H), 7.86 (s, 1H), 7.70 (d, 1H), 7.62 - 7.50 (m, 3H), 7.50 - 7.41 (m, 3H), 7.40 - 7.33 (m, 1H), 7.20 (s, 1H), 7.08 (s, 1H), 6.95 - 6.87 (m, 2H), **6.83 (s, 1H).** δ **4.85 (dd, 1H), 3.79 (s, 3H),** 3.76 (s, 3H), 3.46 (s, 3H), 3.10 (d, 2H), 2.99 - 2.86 (m, 4H), 2.68 - 2.34 (m, 5H), 2.30 - 2.05 (m, 6H), 2.05 - 1.91 (m, 2H), 1.85 (s, 3H), 1.81 (s, 3H), 1.79 - 1.65 (m, 8H), 1.65 - 1.42 (m, 4H), 1.18 - 1.03 (m, 2H).

**Example 76: Preparation of compound 76**

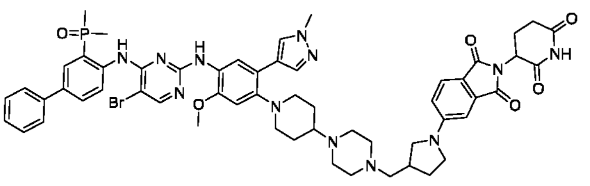

Compound 76

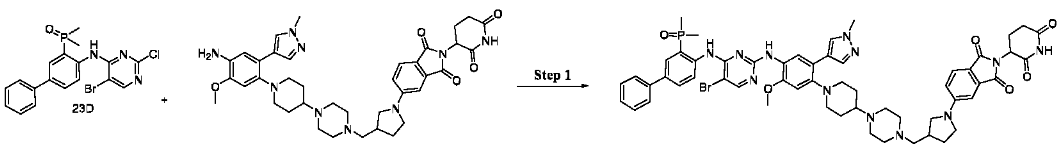

Step 1: 5-(3-((4-(1-(4-((5-bromo-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperidin-4-yl)piperazin-1-yl)methyl)pyrrolidin-1-yl)-2-(2,6-dioxopiperidin-3-yl)isoindoline-1,3-dione **(compound 76)**

**6F** (200 mg, 0.28 mmol) and **23D** (150 mg, 0.34 mmol) were dissolved in DMF (10 mL); p-toluenesulfonic acid monohydrate (160 mg, 0.84 mmol) was added; and under nitrogen protection, the mixture was stirred at 100°C for 16 h, and cooled to room temperature. 10 mL of saturated aqueous sodium bicarbonate solution and 20 mL of dichloromethane were added, and the layers were separated. The organic layer was concentrated under reduced pressure. The concentrated solution was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 ml). Liquid separation was performed. The organic layer was then dried over anhydrous sodium sulphate and concentrated under reduced pressure to obtain **compound 76** (40 mg, yield: 13%).

LCMS m/z = 555.3 $[(M+2H)/2]^+$.

$^1$H NMR (400 MHz, DMSO-$d_6$) δ **11.11 (s, 1H), 11.04 (s, 1H), 8.43** - 8.34 (m, 1H), 8.27 (s, 1H), 8.18 (s, 1H), 8.00 (s, 1H), 7.86 (s, 1H), 7.70 (dd, 1H), 7.64 (d, 1H), 7.59 - 7.51 (m, 3H), 7.45 (t, 2H), 7.36 (t, 1H), 7.23 - 7.14 (m, 1H), 6.90 (d, 1H), 6.82 (dd, 2H), 5.05 (dd, 1H), 3.79 (s, 3H), 3.76 (s, 3H), 3.59 - 3.37 (m, 4H), 3.19 - 3.03 (m, 3H), 2.94 - 2.83 (m, 1H), 2.65 - 2.51 (m, 10H), 2.47 - 2.32 (m, 5H), 2.25 - 2.09 (m, 2H), 2.05 - 1.99 (m, 1H), 1.85 - 1.73 (m, 8H), 1.60 - 1.49 (m, 2H).

**Example 77: Preparation of compound 77**

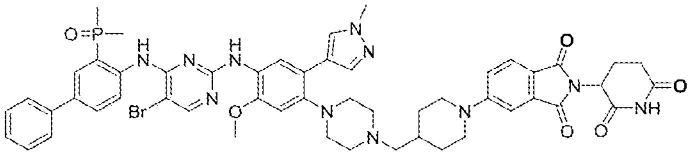

**Compound 77**

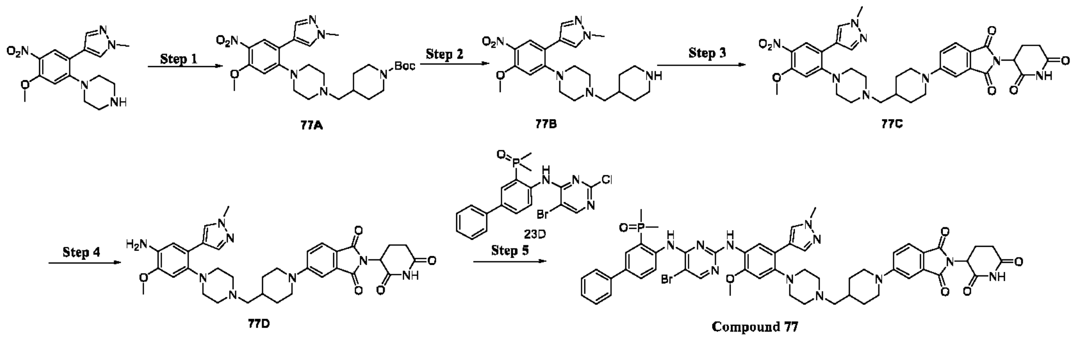


Step 1: tert-butyl 4-((4-(5-methoxy-2-(1-methyl-1H-pyrazol-4-yl)-4-nitrophenyl)piperazin-1-yl) methyl)piperidine-1-carboxylate **(77A)**

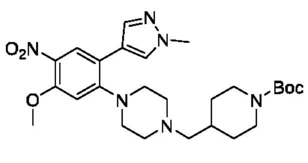

**77A**

The hydrochloride (1.0 g) of 1-(5-methoxy-2-(1-methyl-1H-pyrazol-4-yl)-4-nitrophenyl)piperazine **(80B)** and 1-tert-butoxycarbonylpiperidine-4-carbaldehyde (0.8 g, 3.75 mmol) were dissolved in DMAc (50 mL); acetic acid (1.0 g, 16.65 mmol) was added; and the mixture was stirred at room temperature for 2 h. Sodium triacetoxyborohydride (1.0 g, 4.72 mmol) was added, and the mixture was reacted overnight at room temperature. After the reaction was completed, 100 mL of ethyl acetate and 200 mL of saturated aqueous sodium bicarbonate solution were added. The organic phase was dried over anhydrous sodium sulphate and then concentrated under reduced pressure, and the residue was purified by silica gel column chromatography (mobile phase: dichloromethane/methanol (V/V) = 100/1-10/1) to obtain compound **(77A)** as a yellow solid (1.2 g, yield: 74%).

LCMS m/z = 515.3 $[M+H]^+$.

Step 2: 1-(5-methoxy-2-(1-methyl-1H-pyrazol-4-yl)-4-nitrophenyl)-4-(piperidin-4-ylmethyl)piperazine **(77B);** HCl

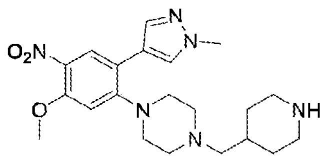

**77B**

**77A** (1.2 g, 2.33 mmol) was dissolved in methanol (14 mL); a solution of hydrochloric acid in dioxane (4 *N,* 30 mL) was added; and the mixture was reacted at room temperature for 1 h and concentrated under reduced pressure, and the residue was directly used in the next step.

Step 3: 2-(2,6-dioxopiperidin-3-yl)-5-(4-((4-(5-methoxy-2-(1-methyl-1H-pyrazol-4-yl)-4-nitrophenyl)piperazin-1-yl)methyl)piperidin-1-yl)isoindoline-1,3-dione **(77C)**

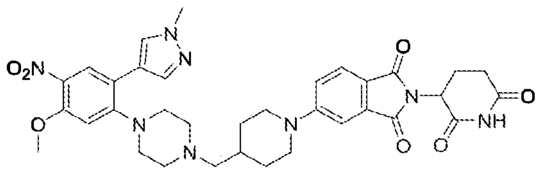

**77C**

The crude hydrochloride of **77B** from the previous step was dissolved in DMSO (20 mL); 2-(2,6-dioxopiperidin-3-yl)-5-fluoroisoindoline-1,3-dione (683 mg, 2.47 mmol) and sodium bicarbonate (692 mg, 8.24 mmol) were successively added; and the mixture was stirred at 100°C for 5 h, and cooled to room temperature. 30 mL of water was added, and the mixture was filtered. The filter cake was dried under reduced pressure and then purified by silica gel column chromatography (mobile phase: dichloromethane/methanol (V/V) = 100/1-20/1) to obtain **77C** (0.90 g, two-step yield: 58%).

LCMS m/z = 671.3 $[M+H]^+$.

Step 4: 5-(4-((4-(4-amino-5-methoxy-2-(1-methyl-1H-pyrazol-4-yl)phenyl) piperazin-1-yl)methyl)piperidin-1-yl)-2-(2,6-dioxopiperidin-3-yl)isoindoline-1,3-dione **(77D)**

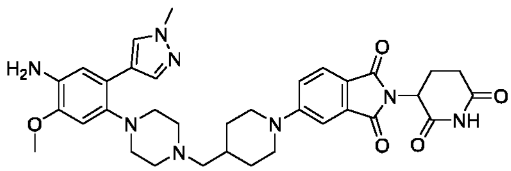

**77D**

**77C** (0.90 g, 1.34 mmol) was dissolved in ethanol/water (20 mL, 3:1); iron powder (375 mg, 6.71 mmol) and ammonium chloride (361 mg, 6.75 mmol) were successively added; and under nitrogen protection, the mixture was stirred at 80°C for 1 h, cooled to room temperature and subjected to suction filtration under reduced pressure. The filtrate was concentrated under reduced pressure, and then the crude was purified by silica gel column chromatography (mobile phase: dichloromethane/methanol (V/V) = 100/1-20/1) to obtain **77D** (650 mg, yield: 76%).

LCMS m/z = 641.3 $[M+H]^+$.

Step 5: 5-(4-((4-(4-((5-bromo-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperazin-1-yl)methyl)piperidin-1-yl)-2-(2,6-dioxopiperidin-3-yl)isoindoline-1,3-dione **(compound 77)**

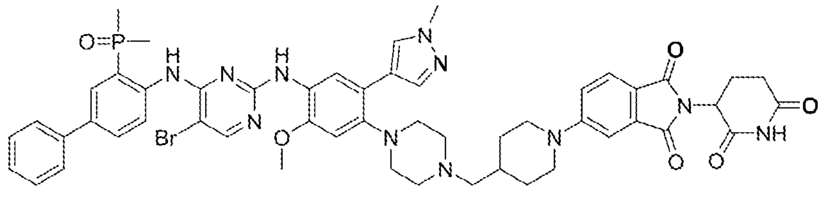

**Compound 77**

**77D** (200 mg, 0.31 mmol) and **23D** (150 mg, 0.34 mmol) were dissolved in DMF (10 mL); p-toluenesulfonic acid monohydrate (160 mg, 0.84 mmol) was added; and under nitrogen protection, the mixture was stirred at 100°C for 16 h, and cooled to room temperature. 10 mL of saturated aqueous sodium bicarbonate solution and 20 mL of dichloromethane were added, and the layers were separated. The organic layer was concentrated under reduced pressure. The concentrated solution was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 mL). Liquid separation was performed. The organic layer was then dried over anhydrous sodium sulphate and concentrated under reduced pressure to obtain **compound 77** (50 mg, yield: 15%).

LCMS m/z = 520.7 $[(M+2H)/2]^+$.

$^1H$ NMR (400 MHz, $D_2O/CF_3COOD$(v/v = **1:1))** δ **8.28 (s, 1H), 8.25** - 8.03 (m, 6H), 7.83 (s, 1H), 7.75 (d, 1H), 7.63 - 7.45 (m, 5H), 7.43 - 7.27 (m, 1H), 6.98 (s, 1H), 5.23 (dd, 1H), 4.00 - 3.90 (m, 5H), 3.90 - 3.71 (m, 5H), 3.71 - 3.59 (m, 2H), 3.44 - 3.07 (m, 8H), 2.97 - 2.89 (m, 2H), 2.87 - 2.71 (m, 1H), 2.66 - 2.48 (m, 1H), 2.48 - 2.32 (m, 2H), 2.32 - 2.21 (m, 1H), 2.21 - 2.07 (m, 2H), 2.02 (d, 6H).

**Example 78: Preparation of compound 78**

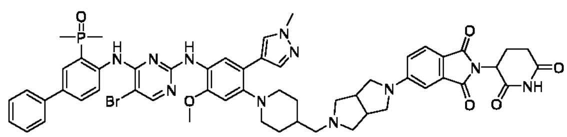

**Compound 78**

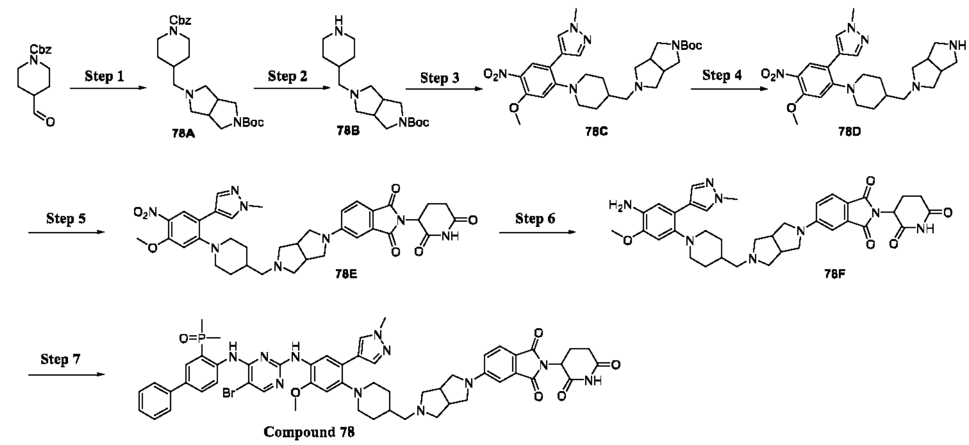


Step 1: tert-butyl 5-((1-((benzyloxy)carbonyl)piperidin-4-yl)methyl) hexahydropyrrolo[3,4-c]pyrrole-2(1H)-carboxylate **(78A)**

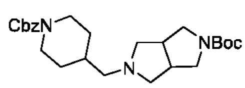

78A

Benzyl 4-formylpiperidine-1-carboxylate (2.5 g, 10.11 mmol) and tert-butyl hexahydropyrrolo[3,4-c]pyrrole-2(1H)-carboxylate (3.58 g, 16.88 mmol) were dissolved in dichloromethane (100 mL); acetic acid (610 mg, 10.11 mmol) was added; and the mixture was stirred at room temperature for 1 h. Sodium triacetoxyborohydride (4.29 g, 20.22 mmol) was added; and the mixture was reacted overnight at room temperature, adjusted to a basic pH by adding saturated aqueous sodium bicarbonate solution and extracted with dichloromethane (50 mL × 3). The organic phases were combined, washed with water (100 mL × 2), dried over anhydrous sodium sulphate and concentrated under reduced pressure, and the residue was purified by silica gel column chromatography (mobile phase: ethyl acetate/petroleum ether (V/V) = 1/1 to dichloromethane/methanol (V/V) = 20/1) to obtain **78A** (3.7 g, yield: 82.5%).

LCMS m/z = 444.3 $[M+H]^+$.

Step 2: tert-butyl 5-(piperidin-4-ylmethyl)hexahydropyrrolo[3,4-c]pyrrole-2(1H)-carboxylate **(78B)**

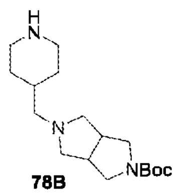

78B

**78A** (3.7 g, 8.34 mmol) and palladium on carbon (1.5 g, 10 wt%) were successively added and dissolved in 60 mL of methanol, and then the mixture was subjected to hydrogen replacement three times, reacted at room temperature for 3 h and subjected to suction filtration over celite. The filtrate was concentrated under reduced pressure to obtain **78B** (2.5 g, yield: 96.8%).

Step 3: tert-butyl 5-((1-(5-methoxy-2-(1-methyl-1H-pyrazol-4-yl)-4-nitrophenyl) piperidin-4-yl) methyl)hexahydropyrrolo[3,4-c]pyrrole-2(1H)-carboxylate **(78C)**

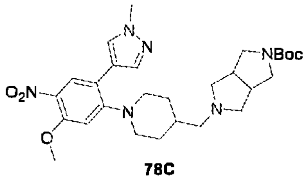

78C

**78B** (2.46 g, 7.96 mmol) and 4-(2-fluoro-4-methoxy-5-nitrophenyl)-1-methyl-1H-pyrazole **(1D)** (2.0 g, 7.96 mmol) were dissolved in DMSO (10 mL); anhydrous potassium carbonate (3.3 g, 23.88 mmol) was added; and the mixture was reacted at 120°C for 6 h, cooled to room temperature and extracted by adding 50 mL of ethyl acetate and 30 mL of water. The organic layer was washed three times with saturated brine (20 mL × 3), dried over anhydrous sodium sulphate and concentrated under reduced pressure, and the residue was purified by column chromatography (dichloromethane: methanol (V/V) = 100/1-20/1) to obtain **78C** (0.8 g, yield: 18%).

LCMS m/z = 541.3$[M+H]^+$.

Step 4: 2-((1-(5-methoxy-2-(1-methyl-1H-pyrazol-4-yl)-4-nitrophenyl)piperidin-4-yl)methyl)octahydropyrrolo[3,4-c]pyrrole **(78D);** HCl

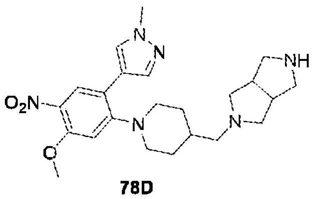

78D

**78C** (800 mg, 1.48 mmol) was dissolved in methanol (2 mL); a solution of hydrochloric acid in dioxane (4 *N,* 4 mL) was added; and the mixture was reacted at room temperature for 1 h and concentrated under reduced pressure, and the residue was directly used in the next step.

LCMS m/z = 441.3$[M+H]^+$.

Step 5: 2-(2,6-dioxopiperidin-3-yl)-5-(5-((1-(5-methoxy-2-(1-methyl-1H-pyrazol-4-yl)-4-nitrophenyl)piperidin-4-yl)methyl)hexahydropyrrolo[3,4-c]pyrrol-2(1H)-yl)isoindoline-1,3-dione **(78E)**

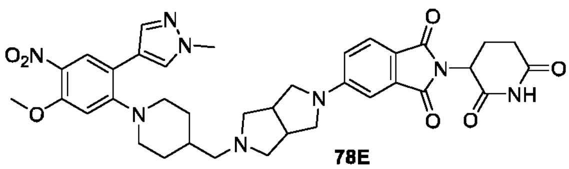

78E

The crude hydrochloride of **78D** from the previous step was dissolved in DMSO (6 mL); 2-(2,6-dioxopiperidin-3-yl)-5-fluoroisoindoline-1,3-dione (410 mg, 1.48 mmol) and DIPEA (1.15 g, 8.88 mmol) were successively added; and the mixture was stirred at 100°C for 5 h, and cooled to room temperature. 10 mL of water was added, and the mixture was filtered. The filter cake was dried under reduced pressure and then purified by silica gel column chromatography (mobile phase: dichloromethane/methanol (V/V) = 100/1-20/1) to obtain **78E** (318 mg, yield: 30.8%).

LCMS m/z = 697.3$[M+H]^+$.

Step 6: 5-(5-((1-(4-amino-5-methoxy-2-(1-methyl-1H-pyrazol-4-yl)phenyl) piperidin-4-yl)methyl)hexahydropyrrolo[3,4-c]pyrrol-2(1H)-yl)-2-(2,6-dioxopiperidin-3-yl)isoindoline-1,3-dione **(78F)**

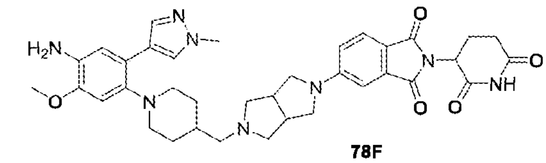

78F

Compound **78E** (318 mg, 0.46 mmol) was dissolved in ethanol/water (9 mL/3 mL); iron powder (210 mg, 3.68 mmol) and ammonium chloride (200 mg, 3.68 mmol) were successively added; and under nitrogen protection, the mixture was stirred at 80°C for 3 h, cooled to room temperature and subjected to suction filtration under reduced pressure. The filtrate was concentrated under reduced pressure, and then the crude was purified by silica gel column chromatography (mobile phase: dichloromethane/methanol (V/V) = 50/1-20/1) to obtain **78F** (240 mg, yield: 78%).

Step 7: 5-(5-((1-(4-((5-bromo-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperidin-4-yl)methyl)hexahydropyrrolo[3,4-c]pyrrol-2(1H)-yl)-2-(2,6-dioxopiperidin-3-yl)isoindoline-1,3-dione **(compound 78)**

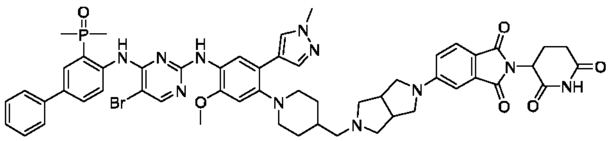

Compound 78

Compound **78F** (120 mg, 0.18 mmol) and **23D** (86 mg, 0.20 mmol) were dissolved in 1,4-dioxane (2 mL) and NMP (0.5 mL); p-toluenesulfonic acid monohydrate (93 mg, 0.54 mmol) was added; and under nitrogen protection, the mixture was stirred at 100°C for 16 h, and cooled to room temperature. 10 mL of saturated aqueous sodium bicarbonate solution and 20 mL of dichloromethane were added, and the layers were separated. The organic layer was concentrated under reduced pressure, and the residue was purified by silica gel column chromatography to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 ml). Liquid separation was performed. The organic layer was then dried over anhydrous sodium sulphate and concentrated under reduced pressure to obtain **compound 78** (20 mg, yield: 10.4%).

LCMS m/z = 533.8 $[(M+2H)/2]^+$.

$^1H$ NMR (400 MHz, DMSO-$d_6$) δ **11.24** - 10.92 (m, 2H), 8.50 - 8.32 (m, 1H), 8.28 (s, 1H), 8.18 (s, 1H), 7.98 (s, 1H), 7.83 (s, 1H), 7.77 - 7.60 (m, 2H), 7.59 - 7.45 (m, 3H), 7.39 (t, 2H), 7.25 (t, 1H), 7.22 - 7.06 (m, 1H), 6.97 (d, 1H), 6.88 (dd, 1H), 6.83 (s, 1H), 5.05 (dd, 1H), 3.86 - 3.65 (m, 8H), 3.30 - 3.19 (m, 2H), 3.12 - 2.93 (m, 4H), 2.93 - 2.82 (m, 1H), 2.71 - 2.51 (m, 8H), 2.34 (d, 2H), 2.05 - 1.96 (m, 1H), 1.82 (d, 6H), 1.76 - 1.63 (m, 2H), 1.60 - 1.44 (m, 1H), 1.36 - 1.24 (m, 2H).

**Example 79: Preparation of compound 79**

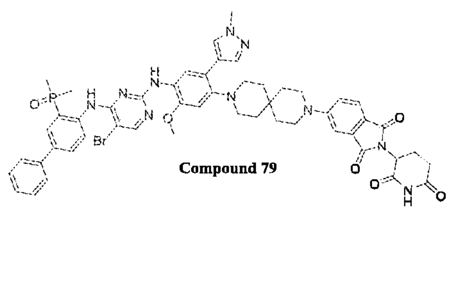

Compound 79

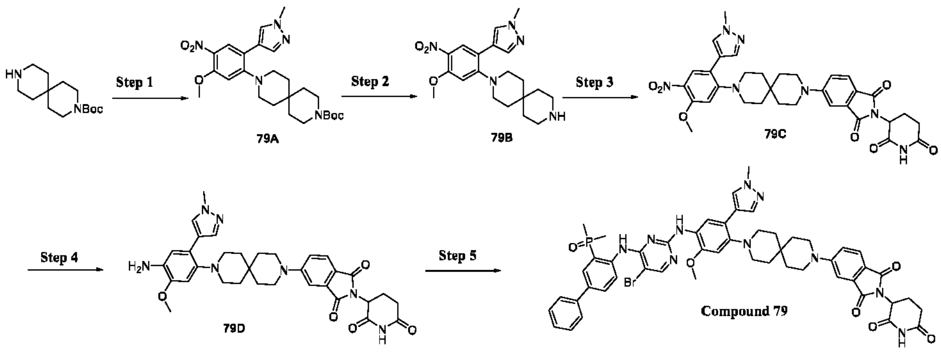

79A 79B 79C 79D Compound 79

Step 1: tert-butyl 9-(5-methoxy-2-(1-methyl-1H-pyrazol-4-yl)-4-nitrophenyl)-3,9-diazaspiro[5.5]undecane-3-carboxylate (**79A**)

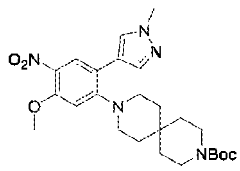

**79A**

Tert-butyl 3,9-diazaspiro[5.5]undecane-3-carboxylate (1.27 g, 4.98 mmol), 4-(2-fluoro-4-methoxy-5-nitrophenyl)-1-methyl-1H-pyrazole **(1D)** (1.5 g, 5.98 mmol) and potassium carbonate (2.75 g, 19.92 mmol) were mixed and dissolved in DMSO (10 mL), and the mixture was stirred at 120°C for 16 h, cooled to room temperature and extracted by adding 50 mL of water and 50 mL of ethyl acetate. The organic layer was concentrated under reduced pressure, and the residue was purified by column chromatography (mobile phase: dichloromethane/methanol (V/V) = 100/1-20/1) to obtain tert-butyl 9-(5-methoxy-2-(1-methyl-1H-pyrazol-4-yl)-4-nitrophenyl)-3,9-diazaspiro[5.5]undecane-3-carboxylate (**79A**) (2.0 g, yield: 83%).

LCMS m/z = 486.3 $[M+H]^+$.

Step 2: 3-(5-methoxy-2-(1-methyl-1H-pyrazol-4-yl)-4-nitrophenyl)-3,9-diazaspiro[5.5]undecane **(79B)**

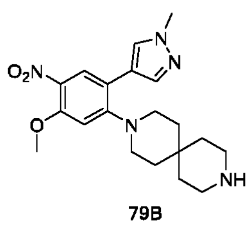

**79B**

Compound **79A** (2.0 g, 4.12 mmol) was dissolved in DCM (20 mL); trifluoroacetic acid (6 mL) was added at room temperature; and the mixture was stirred for 3 h and concentrated under reduced pressure. 20 mL of dichloromethane was added to the residue, and the mixture was adjusted to pH = 9-10 with 1 N aqueous NaOH solution. Liquid separation was performed. The organic layer was concentrated under reduced pressure to obtain the compound 3-(5-methoxy-2-(1-methyl-1H-pyrazol-4-yl)-4-nitrophenyl)-3,9-diazaspiro[5.5]undecane **(79B)** (1.5 g), which was directly used in the next step.

Step 3: 2-(2,6-dioxopiperidin-3-yl)-5-(9-(5-methoxy-2-(1-methyl-1H-pyrazol-4-yl)-4-nitrophenyl)-3,9-diazaspiro[5.5]undecan-3-yl)isoindoline-1,3-dione **(79C)**

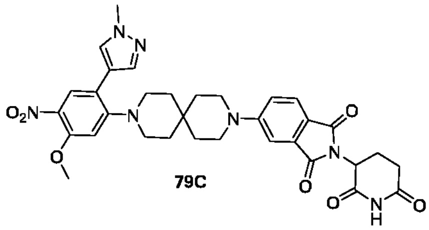

79C

Compound **79B** (1.5 g, 3.89 mmol) was dissolved in DMSO (5 mL); 2-(2,6-dioxopiperidin-3-yl)-5-fluoroisoindole-1,3-dione (1.29 g, 4.67 mmol) and DIPEA (1.01 g, 7.78 mmol) were successively added; and the mixture was stirred at 90°C for 3 h, and cooled to room temperature. 10 mL of water was added, and the mixture was filtered. The filter cake was dried under reduced pressure and then purified by silica gel column chromatography (mobile phase: dichloromethane/methanol (V/V) = 100/1-20/1) to obtain 2-(2,6-dioxopiperidin-3-yl)-5-(9-(5-methoxy-2-(1-methyl-1H-pyrazol-4-yl)-4-nitrophenyl)-3,9-diazaspiro[5.5]undecan-3-yl)isoindoline-1,3-dione **(79C)** (1.4 g, yield: 56.1%).

LCMS m/z = 642.3 $[M+H]^+$.

Step 4: 5-(9-(4-amino-5-methoxy-2-(1-methyl-1H-pyrazol-4-yl)phenyl)-3,9-diazaspiro[5.5]undecan-3-yl)-2-(2,6-dioxopiperidin-3-yl)isoindoline-1,3-dione **(79D)**

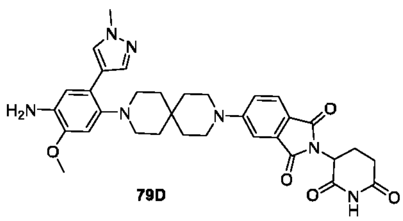

79D

Compound **79C** (1.4 g, 2.18 mmol), iron powder (0.97 mg, 17.44 mmol) and ammonium chloride (0.93 mg, 17.44 mmol) were dissolved in ethanol (30 mL) and water (10 mL), and the mixture was stirred at 80°C for 2 h, cooled to room temperature and subjected to suction filtration. The filtrate was concentrated to obtain the title compound 5-(9-(4-amino-5-methoxy-2-(1-methyl-1H-pyrazol-4-yl)phenyl)-3,9-diazaspiro[5.5]undecan-3-yl)-2-(2,6-dioxopiperidin-3-yl)isoindoline-1,3-dione **(79D)** (1.0 g), which was directly used in the next step.

LCMS m/z = 612.3$[M+H]^+$.

Step 5: 5-(9-(4-((5-bromo-4-((3-(dimethylphosphoryl)-[1,1'-biphenyl]-4-yl)amino) pyrimidin-2-yl)amino)-5-methoxy-2-(1-methyl-1H-pyrazol-4-yl)phenyl)-3,9-diazaspiro[5.5]undecan-3-yl)-2-(2,6-dioxopiperidin-3-yl)isoindoline-1,3-dione (**compound 79**)

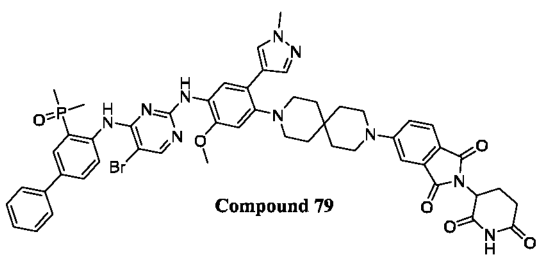

Compound 79

**79D** (180 mg, 0.29 mmol) and (4-((5-bromo-2-chloropyrimidin-4-yl)amino)-[1,1'-biphenyl]-3-yl)dimethylphosphine oxide (**23D**) (150 mg, 0.35 mmol) were dissolved in 1,4-dioxane (3 mL) and NMP (1 mL); p-toluenesulfonic acid monohydrate (165 mg, 0.87 mmol) was added; and under nitrogen protection, the mixture was stirred at 100°C for 16 h, and cooled to room temperature. 10 mL of saturated aqueous sodium bicarbonate solution and 20 mL of dichloromethane were added, and the layers were separated. The organic layer was concentrated under reduced pressure, and the

residue was purified by silica gel column chromatography to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 ml). Liquid separation was performed. The organic layer was then dried over anhydrous sodium sulphate and concentrated under reduced pressure to obtain **compound 79** (22 mg, yield: 7.5%).

LCMS m/z = 506.3 [(M+2H)/2]$^+$.

$^1$H NMR (400 MHz, DMSO-$d_6$) δ 11.13 (s, 1H), 11.06 (s, 1H), 8.45 - 8.36 (m, 1H), 8.32 (s, 1H), 8.18 (s, 1H), 8.04 (s, 1H), 7.88 (s, 1H), 7.75 - 7.64 (m, 2H), 7.59 - 7.50 (m, 3H), 7.50 - 7.41 (m, 2H), 7.41 - 7.29 (m, 2H), 7.30 - 7.16 (m, 2H), 6.92 (s, 1H), 5.07 (dd, 1H), 3.81 (s, 3H), 3.77 (s, 3H), 3.54 - 3.39 (m, 4H), 2.96 - 2.78 (m, 5H), 2.69 - 2.52 (m, 2H), 2.06 - 1.96 (m, 1H), 1.83 (d, 6H), 1.71 - 1.51 (m, 8H).

**Example 80: Preparation of compound 80**

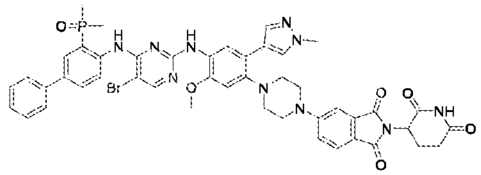

Compound 80

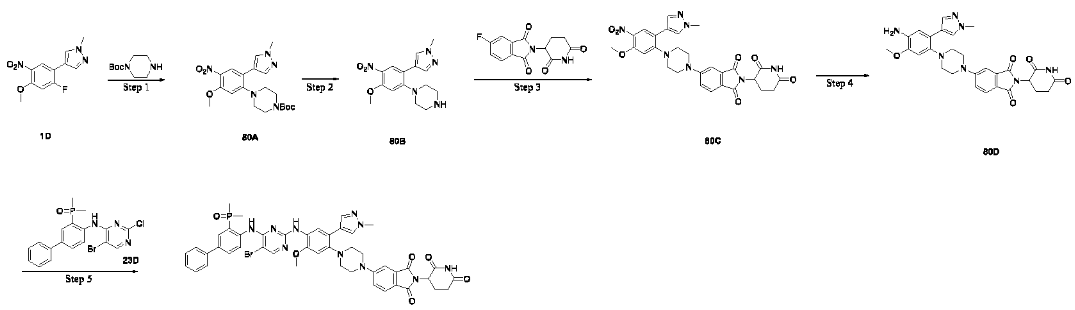


Compound 80

Step 1: tert-butyl 4-(5-methoxy-2-(1-methyl-1H-pyrazol-4-yl)-4-nitrophenyl) piperazine-1-carboxylate (**80A**)

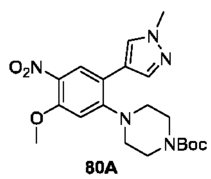

80A

**1D** (0.8 g, 3.18 mmol) was dissolved in DMSO (20 mL); potassium carbonate (1.3 g, 9.41 mmol) and tert-butyl piperazine-1-carboxylate (712 mg, 3.82 mmol) were successively added; and the mixture was reacted at 100°C for 6 h. After the reaction was completed, the mixture was extracted with 50 mL of ethyl acetate and 200 mL of water. The organic phase was dried over anhydrous sodium sulphate and then concentrated under reduced pressure, and the residue was purified by silica gel column chromatography (mobile phase: dichloromethane/methanol (V/V) = 100/1-10/1) to obtain **80A** (650 mg, yield: 49%).

LCMS m/z = 418.2 [M+H]$^+$.

Step 2: 1-(5-methoxy-2-(1-methyl-1H-pyrazol-4-yl)-4-nitrophenyl)piperazine (**80B**); HCl

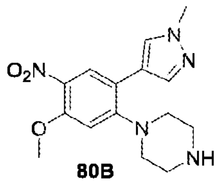

80B

**80A** (400 mg, 0.96 mmol) was dissolved in methanol (2 mL); a solution of hydrochloric acid in dioxane (4 *N,* 6 mL) was added; and the mixture was reacted at room temperature for 1 h and concentrated under reduced pressure, and the residue was directly used in the next step.

Step 3: 2-(2,6-dioxopiperidin-3-yl)-5-(4-(5-methoxy-2-(1-methyl-1H-pyrazol-4-yl)-4-nitrophenyl)piperazin-1-yl)isoindoline-1,3-dione (**80C**)

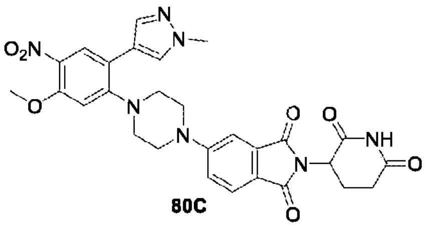

80C

The crude hydrochloride of **80B** from the previous step was dissolved in DMSO (8 mL); 2-(2,6-dioxopiperidin-3-yl)-5-fluoroisoindoline-1,3-dione (318 mg, 1.15 mmol) and sodium bicarbonate (323 mg, 3.84 mmol) were successively added; and the mixture was stirred at 100°C for 5 h, and cooled to room temperature. 10 mL of water was added, and the mixture was filtered. The filter cake was dried under reduced pressure and then purified by silica gel column chromatography (mobile phase: dichloromethane/methanol (V/V) = 100/1-20/1) to obtain **80C** (284 mg, two-step yield: 52%).

LCMS m/z = 574.2 $[M+H]^+$.

Step 4: 5-(4-(4-amino-5-methoxy-2-(1-methyl-1H-pyrazol-4-yl)phenyl)piperazin -1-yl)-2-(2,6-dioxopiperidin-3-yl)isoindoline-1,3 -dione (**80D**)

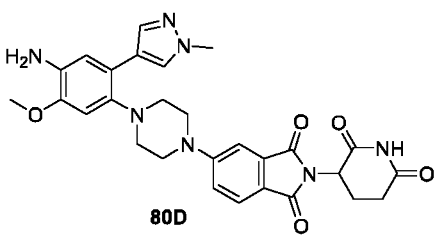

80D

**80C** (284 mg, 0.50 mmol) was dissolved in ethanol/water (8 mL, 3:1); iron powder (140 mg, 2.5 mmol) and ammonium chloride (134 mg, 2.5 mmol) were successively added; and under nitrogen protection, the mixture was stirred at 80°C for 1 h, cooled to room temperature and subjected to suction filtration under reduced pressure. The filtrate was concentrated under reduced pressure, and then the crude was purified by silica gel column chromatography (mobile phase: dichloromethane/methanol (V/V) = 100/1-20/1) to obtain **80D** (260 mg, yield: 96%).

LCMS m/z = 544.2 $[M+H]^+$.

Step 5: 5-(4-(4-((5-bromo-4-((3-(dimethylphosphoryl)-[1,1'-biphenyl]-4-yl) amino)pyrimidin-2-yl)amino)-5-methoxy-2-(1-methyl-1H-pyrazol-4-yl)phenyl)piperazin-1-yl)-2-(2,6-dioxopiperidin-3-yl)isoindoline-1,3-dione (**compound 80**)

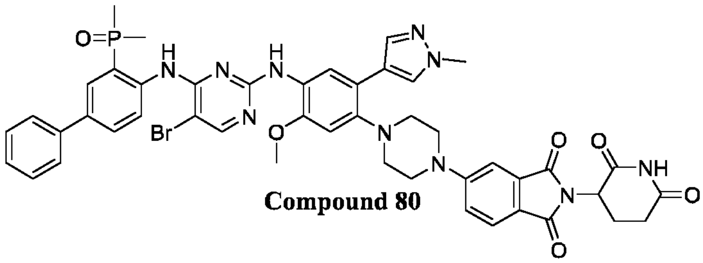

Compound 80

**80D** (130 mg, 0.24 mmol) and (4-((5-bromo-2-chloropyrimidin-4-yl)amino)-[1,1'-biphenyl]-3-yl)dimethylphosphine oxide (**23D**) (135 mg, 0.31 mmol) were dissolved in DMF (10 mL); p-toluenesulfonic acid monohydrate (137 mg, 0.72 mmol) was added; and under nitrogen protection, the mixture was stirred at 100°C for 16 h, and cooled to room temperature. 10 mL of saturated aqueous sodium bicarbonate solution and 20 mL of dichloromethane were added, and the layers were separated. The organic layer was concentrated under reduced pressure, and the residue was purified by silica gel column chromatography to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 mL). Liquid separation was performed. The organic layer was then dried over anhydrous sodium sulphate and concentrated under reduced pressure to obtain **compound 80** (77 mg, yield: 34%).

LCMS m/z = 943.2 $[M+H]^+$.

$^1H$ NMR (400 MHz, DMSO-$d_6$) δ 11.10 (s, 1H), 11.07 (s, 1H), 8.38 (s, 1H), 8.29 (s, 1H), 8.20 (s, 1H), 8.15 (s, 1H), 7.88 (s, 1H), 7.77 - 7.63 (m, 3H), 7.55 (d, 2H), 7.48 - 7.40 (m, 2H), 7.41 - 7.25 (m, 3H), 7.18 (s, 1H), 6.87 (s, 1H), 5.09 (dd, 1H), 3.81 (s, 3H), 3.77 (s, 3H), 3.67 - 3.54 (m, 4H), 3.05 - 2.92 (m, 4H), 2.92 - 2.83 (m, 1H), 2.68 - 2.54 (m, 2H), 2.06 - 1.94 (m, 1H), 1.83 (d, 6H).

**Example 81: Preparation of compound 81**

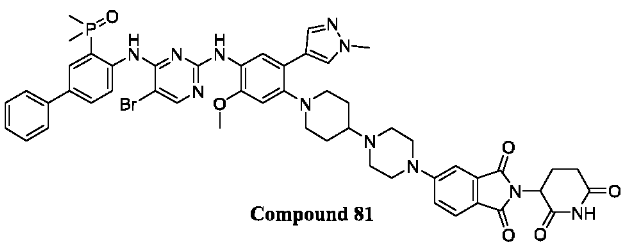

Compound 81

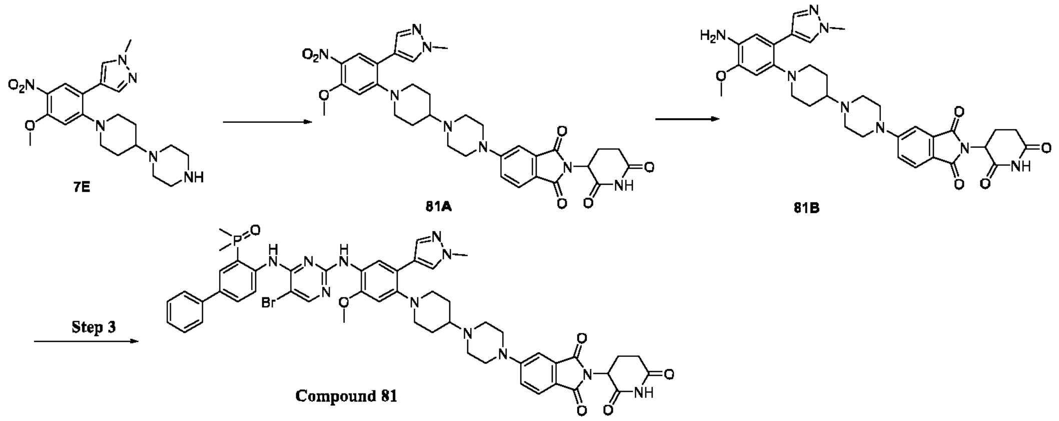

7E
81A
81B
Compound 81

Step 1: 2-(2,6-dioxopiperidin-3-yl)-5-(4-(1-(5-methoxy-2-(1-methyl-1H-pyrazol-4-yl)-4-nitrophenyl)piperidin-4-yl)piperazin-1-yl)isoindoline-1,3-dione (**81A**)

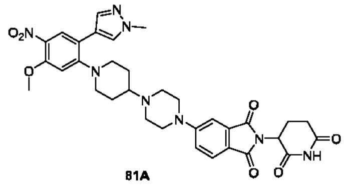
81A

**7E** (1.0 g, 2.50 mmol) was dissolved in DMSO (20 mL); 2-(2,6-dioxopiperidin-3-yl)-5-fluoroisoindoline-1,3-dione (828 mg, 3.00 mmol) and sodium bicarbonate (692 mg, 8.24 mmol) were successively added; and the mixture was stirred at 100°C for 5 h, and cooled to room temperature. 30 mL of water was added, and the mixture was filtered. The filter cake was dried under reduced pressure and then purified by silica gel column chromatography (mobile phase: dichloromethane/methanol (V/V) = 100/1-20/1) to obtain **81A** (0.90 g, yield: 55%).
LCMS m/z = 657.3 $[M+H]^+$.

Step 2: 5-(4-(1-(4-amino-5-methoxy-2-(1-methyl-1H-pyrazol-4-yl)phenyl)piperidin-4-yl)piperazin-1-yl)-2-(2,6-dioxopiperidin-3-yl)isoindoline-1,3-dione (**81B**)

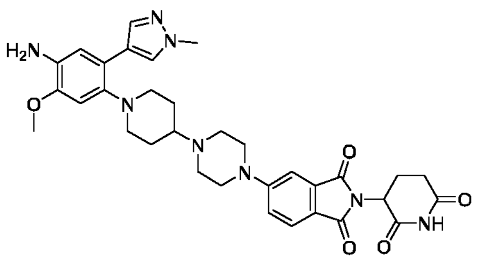
81B

**81A** (0.90 g, 1.37 mmol) was dissolved in ethanol/water (20 mL, 3:1); iron powder (375 mg, 6.71 mmol) and ammonium chloride (361 mg, 6.75 mmol) were successively added; and under nitrogen protection, the mixture was stirred at 80°C for 1 h, cooled to room temperature and subjected to suction filtration under reduced pressure. The filtrate was concentrated under reduced pressure, and then the crude was purified by silica gel column chromatography (mobile phase: dichloromethane/methanol (V/V) = 100/1-20/1) to obtain **81B** (700 mg, yield: 82%).
LCMS m/z = 627.3 $[M+H]^+$.

Step 3: 5-(4-(1-(4-((5-bromo-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperidin-4-yl)piperazin-1-yl)-2-(2,6-dioxopiperidin-3-yl)isoindoline-1,3-dione (**compound 81**)

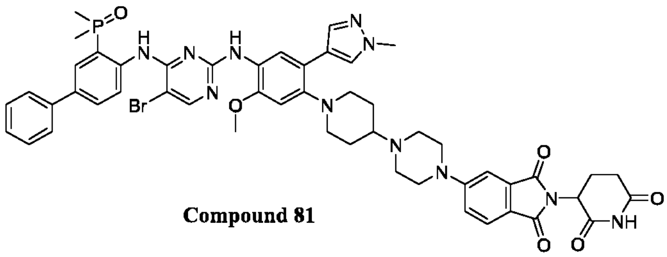

Compound 81

**81B** (200 mg, 0.32 mmol) and **23D** (150 mg, 0.34 mmol) were dissolved in DMF (10 mL); p-toluenesulfonic acid monohydrate (160 mg, 0.84 mmol) was added; and under nitrogen protection, the mixture was stirred at 100°C for 16 h, and cooled to room temperature. 10 mL of saturated aqueous sodium bicarbonate solution and 20 mL of dichloromethane were added, and the layers were separated. The organic layer was concentrated under reduced pressure. The concentrated solution was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 mL). Liquid separation was performed. The organic layer was then dried over anhydrous sodium sulphate and concentrated under reduced pressure to obtain **compound 81** (55 mg, yield: 17%).

LCMS m/z = 513.7 $[(M+2H)/2]^+$.

$^1H$ NMR (400 MHz, DMSO-$d_6$) δ 11.11 (s, 1H), 11.06 (s, 1H), 8.44 - 8.33 (m, 1H), 8.29 (s, 1H), 8.19 (s, 1H), 8.01 (s, 1H), 7.87 (s, 1H), 7.75 - 7.65 (m, 2H), 7.61 - 7.52 (m, 3H), 7.46 (t, 2H), 7.40 - 7.33 (m, 2H), 7.32 - 7.24 (m, 1H), 7.23 - 7.12 (m, 1H), 6.84 (s, 1H), 5.08 (dd, 1H), 3.79 (s, 3H), 3.77 (s, 3H), 3.52 - 3.39 (m, 4H), 3.18 - 3.05 (m, 2H), 2.95 - 2.80 (m, 1H), 2.75 - 2.52 (m, 8H), 2.36 - 2.23 (m, 1H), 2.08 - 1.99 (m, 1H), 1.88 - 1.75 (m, 8H), 1.65 - 1.51 (m, 2H).

**Example 82: Preparation of compound 82**

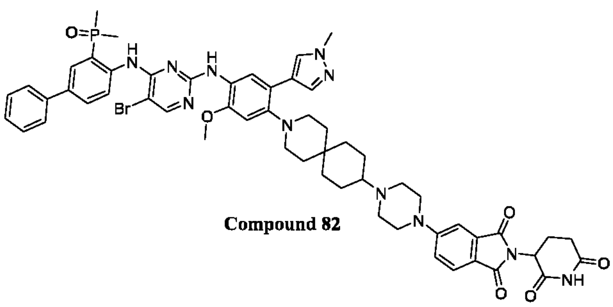

Compound 82

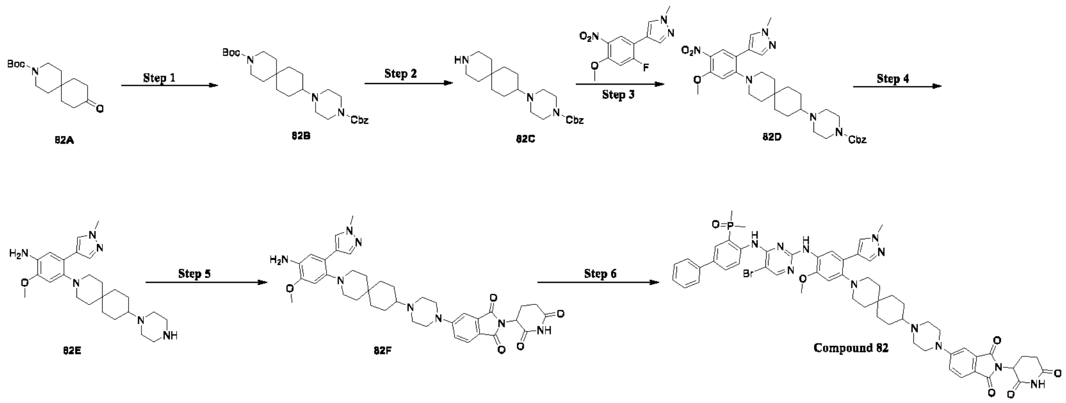


Step 1: tert-butyl 9-(4-((benzyloxy)carbonyl)piperazin-1-yl)-3-azaspiro[5.5] undecane-3-carboxylate (**82B**)

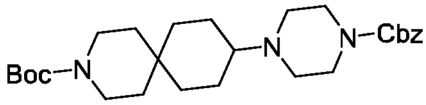

**82B**

Tert-butyl 9-oxo-3-azaspiro[5.5]undecane-3-carboxylate (**82A**) (1.50 g, 5.61 mmol) and benzyl piperazine-1-carboxylate (1.23 g, 5.58 mmol) were dissolved in DMAc (50 mL); acetic acid (1.0 g, 16.65 mmol) was added; and the mixture was stirred at room temperature for 2 h. Sodium triacetoxyborohydride (2.0 g, 9.44 mmol) was added, and the mixture was reacted overnight at room temperature. After the reaction was completed, 100 mL of ethyl acetate and 200 mL of saturated aqueous sodium bicarbonate solution were added. The organic phase was dried over anhydrous sodium sulphate and then concentrated under reduced pressure, and the residue was purified by silica gel column chromatography (mobile phase: petroleum ether/ethyl acetate (V/V) = 100/1-3/1) to obtain **82B** as a white solid (1.6 g, yield: 61%).

LCMS m/z = 472.3 $[M+H]^+$.

Step 2: benzyl 4-(3-azaspiro[5.5]undecan-9-yl)piperazine-1-carboxylate (**82C**); HCl

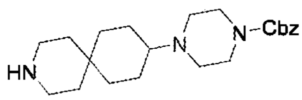

**82C**

**82B** (1.6 g, 3.39 mmol) was dissolved in methanol (10 mL); a solution of hydrochloric acid in dioxane (4 *N,* 40 mL) was added; and the mixture was reacted at room temperature for 1 h and concentrated under reduced pressure, and the residue was directly used in the next step.

Step 3: benzyl 4-(3-(5-methoxy-2-(1-methyl-1H-pyrazol-4-yl)-4-nitrophenyl)-3-azaspiro [5.5]undecan-9-yl)piperazine-1-carboxylate (**82D**)

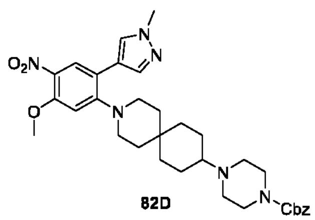

**82D**

The crude hydrochloride of **82C** from the previous step was dissolved in DMSO (20 mL); 4-(2-fluoro-4-methoxy-5-nitrophenyl)-1-methyl-1H-pyrazole (**1D**) (851 mg, 3.39 mmol) and potassium carbonate (690 mg, 5.00 mmol) were successively added; and the mixture was stirred at 100°C for 5 h, and cooled to room temperature. 30 mL of water was added, and the mixture was filtered. The filter cake was dried under reduced pressure and then purified by silica gel column chromatography (mobile phase: dichloromethane/methanol (V/V) = 100/1-20/1) to obtain **82D** (1.0 g two-step yield: 49%).

LCMS m/z = 603.3 [M+H]+.

Step 4: 2-methoxy-5-(1-methyl-1H-pyrazol-4-yl)-4-(9-(piperazin-1-yl)-3-azaspiro [5.5]undecan-3-yl)aniline (**82E**)

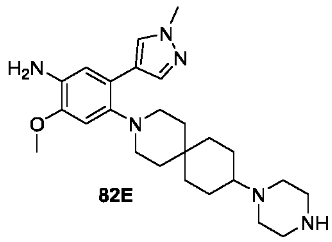

**82E**

**82D** (1.0 g, 1.66 mmol) was dissolved in methanol (40 mL). Under nitrogen protection, palladium on carbon (wt% = 10%, 500 mg) was added, and the mixture was subjected to 1 atm hydrogen replacement 3 times and reacted at room temperature for 1.5 h. After the reaction was completed, the mixture was filtered over celite. The filter cake was washed with dichloromethane/methanol (V/V = 10/1), and the filtrate was concentrated under reduced pressure to obtain **82E** (650 mg, yield: 89%).

LCMS m/z = 439.3 [M+H]+.

Step 5: 5-(4-(3-(4-amino-5-methoxy-2-(1-methyl-1H-pyrazol-4-yl)phenyl)-3-azaspiro[5.5]undecan-9-yl)piperazin-1-yl)-2-(2,6-dioxopiperidin-3-yl)isoindoline-1,3-dione (**82F**)

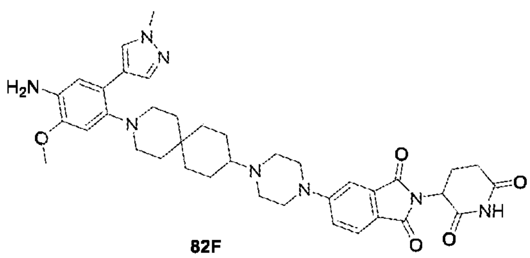

**82F**

**82E** (650 mg, 1.48 mmol) was dissolved in DMSO (20 mL); 2-(2,6-dioxopiperidin-3-yl)-5-fluoroisoindoline-1,3-dione (552 mg, 2.00 mmol) and sodium bicarbonate (692 mg, 8.24 mmol) were successively added; and the mixture was stirred at 100°C for 5 h, and cooled to room temperature. 30 mL of water was added, and the mixture was filtered. The filter cake was dried under reduced pressure and then purified by silica gel column chromatography (mobile phase: dichloromethane/methanol (V/V) = 100/1-20/1) to obtain **82F** (400 mg, yield: 39%).

LCMS m/z = 695.3 [M+H]+.

Step 6: 5-(4-(3-(4-((5-bromo-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)-3-azaspiro[5.5]undecan-9-yl)piperazin-1-yl)-2-(2,6-dioxopiperidin-3-yl)isoindoline-1,3-dione (**compound 82**)

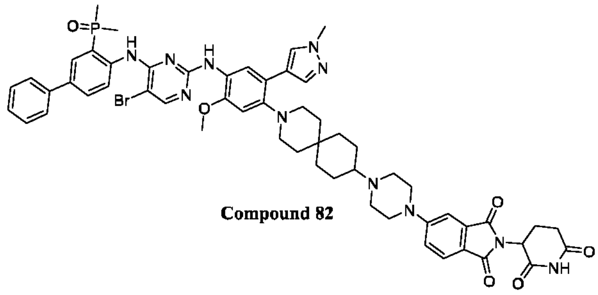

Compound 82

**82F** (200 mg, 0.29 mmol) and **23D** (148 mg, 0.34 mmol) were dissolved in DMF (10 mL); p-toluenesulfonic acid monohydrate (160 mg, 0.84 mmol) was added; and under nitrogen protection, the mixture was stirred at 100°C for 16 h, and cooled to room temperature. 10 mL of saturated aqueous sodium bicarbonate solution and 20 mL of dichloromethane were added, and the layers were separated. The organic layer was concentrated under reduced pressure. The concentrated solution was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 mL). Liquid separation was performed. The organic layer was then dried over anhydrous sodium sulphate and concentrated under reduced pressure to obtain **compound 82** (30 mg, yield: 9%).

LCMS m/z = 547.8 $[(M+2H)/2]^+$.

$^1H$ NMR (400 MHz, DMSO-$d_6$) δ 11.13 (s, 1H), 11.06 (s, 1H), 8.44 - 8.35 (m, 1H), 8.30 (s, 1H), 8.18 (s, 1H), 8.02 (s, 1H), 7.87 (s, 1H), 7.75 - 7.63 (m, 2H), 7.62 - 7.50 (m, 3H), 7.44 (t, 2H), 7.39 - 7.30 (m, 2H), 7.25 (dd, 1H), 7.22 - 7.12 (m, 1H), 6.89 (s, 1H), 5.07 (dd, 1H), 3.80 (s, 3H), 3.76 (s, 3H), 3.50 - 3.35 (m, 4H), 2.96 - 2.72 (m, 5H), 2.69 - 2.51 (m, 6H), 2.34 - 2.22 (m, 1H), 2.09 - 1.99 (m, 1H), 1.93 - 1.71 (m, 8H), 1.68 - 1.51 (m, 4H), 1.49 - 1.31 (m, 4H), 1.19 - 1.03 (m, 2H).

### Example 83: Preparation of compound 83

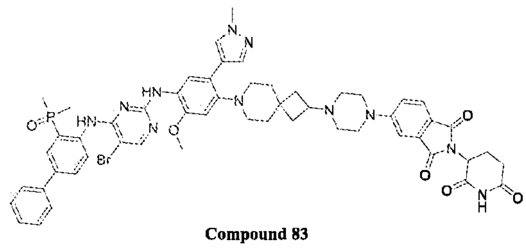

Compound 83

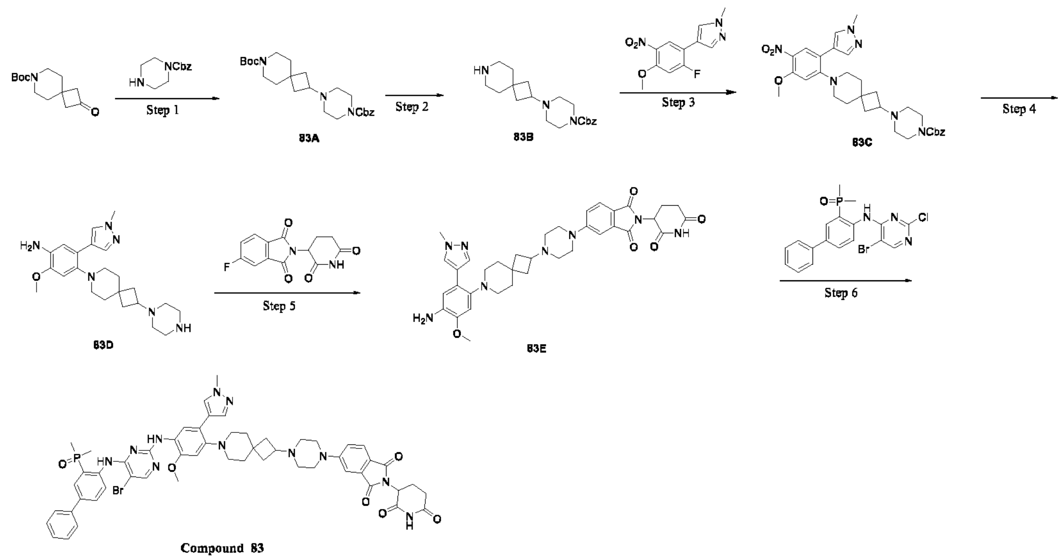


83A 83B 83C 83D 83E

Compound 83

Step 1: tert-butyl 2-(4-((benzyloxy)carbonyl)piperazin-1-yl)-7-azaspiro[3.5] nonane-7-carboxylate (**83A**)

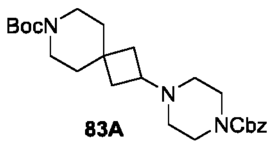

83A

Tert-butyl 2-oxo-7-azaspirocyclo[3.5]nonane-7-carboxylate (8.1 g, 33.84 mmol) and benzyl piperazine-1-carboxylate (5 g, 22.70 mmol) were dissolved in 1,2-dichloroethane (100 mL); ten drops of acetic acid was added; and then the mixture was stirred at room temperature for 1 h. Sodium triacetoxyborohydride (9.6 g, 45.30 mmol) was added, and the mixture was reacted overnight at room temperature, adjusted to a basic pH by adding saturated aqueous sodium bicarbonate solution and extracted with dichloromethane (50 mL × 3). The organic phases were combined, dried over anhydrous sodium sulphate and concentrated under reduced pressure, and the residue was purified by silica gel column chromatography (mobile phase: ethyl acetate/petroleum ether (V/V) = 100/1-1/1) to obtain **83A** (9.3 g, yield: 92%).
LCMS m/z = 444.3 $[M+H]^+$.

Step 2: benzyl 4-(7-azaspiro[3.5]nonan-2-yl)piperazine-1-carboxylate (**83B**); HCl

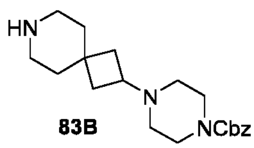

83B

Compound **83A** (3 g, 6.76 mmol) was dissolved in methanol (5 mL); a solution of hydrochloric acid in dioxane (4 *N,* 30 mL) was added; and the mixture was reacted at room temperature for 1 h and concentrated under reduced pressure, and the residue was directly used in the next step.

Step 3: benzyl 4-(7-(5-methoxy-2-(1-methyl-1H-pyrazol-4-yl)-4-nitrophenyl)-7-azaspiro[3.5] nonan-2-yl)piperazine-1-carboxylate (**83C**)

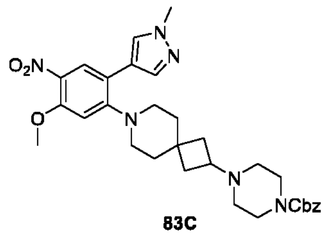

83C

The hydrochloride of **83B** from the previous step and **1D** (1.49 g, 5.93 mmol) were dissolved in DMSO (20 mL); sodium bicarbonate (1.53 g, 18.2 mmol) was added; and the mixture was reacted at 100°C for 6 h, cooled to room temperature and extracted by adding 50 mL of ethyl acetate and 30 mL of water. The organic layer was washed three times with saturated brine (20 mL × 3), dried over anhydrous sodium sulphate and concentrated under reduced pressure, and the residue was purified by column chromatography (dichloromethane: methanol (V/V) = 100/1-20/1) to obtain **83C** (2 g, yield: 59%).

LCMS m/z = 575.3[M+H]$^+$.

Step 4: 2-methoxy-5-(1-methyl-1H-pyrazol-4-yl)-4-(2-(piperazin-1-yl)-7-azaspiro[3.5]nonan-7-yl)aniline (**83D**)

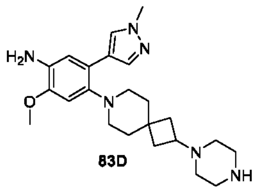

83D

**83C** (1 g, 1.74 mmol) and palladium on carbon (1 g, 10 wt%) were successively added and dissolved in 60 mL of methanol, and then the mixture was subjected to hydrogen replacement three times, reacted at room temperature for 2 h and subjected to suction filtration over celite. The filtrate was concentrated under reduced pressure to obtain **83D** (600 mg, yield: 84%)

LCMS m/z = 411.3[M+H]$^+$.

Step 5: 5-(4-(7-(4-amino-5-methoxy-2-(1-methyl-1H-pyrazol-4-yl)phenyl)-7-azaspiro[3.5]nonan-2-yl)piperazin-1-yl)-2-(2,6-dioxopiperidin-3-yl)isoindoline-1,3-dione (**83E**)

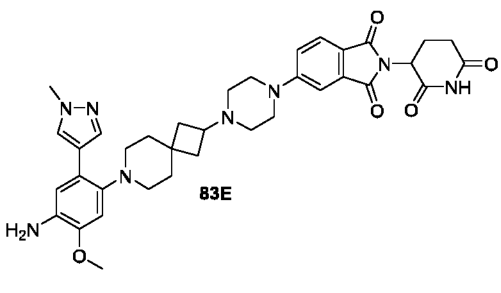

83E

**83D** (600 mg, 1.46 mmol) from the previous step was dissolved in DMSO (20 mL); 2-(2,6-dioxopiperidin-3-yl)-5-fluoroisoindoline-1,3-dione (604 mg, 2.19 mmol) and DIPEA (942 mg, 7.29 mmol) were successively added; and the mixture was stirred at 100°C for 3 h, and cooled to room temperature. 10 mL of water was added, and the mixture was filtered. The filter cake was dried under reduced pressure and then purified by silica gel column chromatography (mobile phase: dichloromethane/methanol (V/V) = 100/1-20/1) to obtain **83E** (540 mg, yield: 55.5%).

LCMS m/z = 667.3 [M+H]$^+$.

Step 6: 5-(4-(7-(4-((5-bromo-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)-7-azaspiro[3.5]nonan-2-yl)piperazin-1-yl)-2-(2,6-dioxopiperidin-3-yl)isoindoline-1,3-dione (**compound 83**)

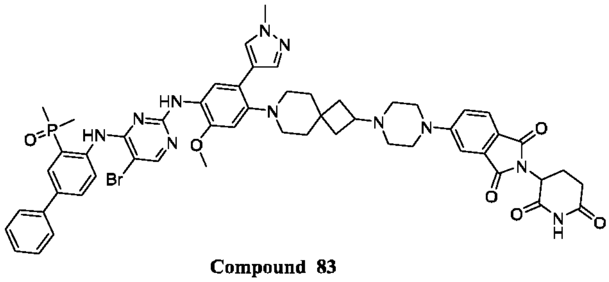

Compound 83

**83E** (270 mg, 0.40 mmol) and **23D** (265 mg, 0.61 mmol) were dissolved in DMF (10 mL); p-toluenesulfonic acid monohydrate (230 mg, 1.21 mmol) was added; and the mixture was stirred at 100°C for 16 h, and cooled to room temperature. 10 mL of saturated aqueous sodium bicarbonate solution and 20 mL of dichloromethane were added, and the layers were separated. The organic layer was concentrated under reduced pressure, and the residue was purified by silica gel column chromatography (dichloromethane: methanol (V/V) = 100/1-10/1) to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)) to obtain a preparative solution, which was concentrated. 50 mL of dichloromethane was added, and the mixture was adjusted to a basic pH by adding saturated sodium bicarbonate solution. Liquid separation was performed. The organic layer was then dried over anhydrous sodium sulphate and concentrated under reduced pressure to obtain **compound 83** (20 mg, yield: 5%).

LCMS m/z = 533.7 $[(M+2H)/2]^+$.

$^1H$ NMR (400 MHz, DMSO-$d_6$) δ 11.13 (s, 1H), 11.06 (s, 1H), 8.48 - 8.35 (m, 1H), 8.31 (s, 1H), 8.18 (s, 1H), 8.03 (s, 1H), 7.87 (s, 1H), 7.76 - 7.63 (m, 2H), 7.61 - 7.50 (m, 3H), 7.45 (t, 2H), 7.40 - 7.31 (m, 2H), 7.27 (dd, 1H), 7.22 - 7.09 (m, 1H), 6.83 (s, 1H), 5.07 (dd, 1H), 3.79 (s, 3H), 3.76 (s, 3H), 3.59 - 3.40 (m, 4H), 2.94 - 2.83 (m, 1H), 2.82 - 2.67 (m, 5H), 2.66 - 2.53 (m, 2H), 2.47 - 2.25 (m, 4H), 2.10 - 1.95 (m, 3H), 1.83 (d, 6H), 1.75 - 1.51 (m, 6H).

## Example 84: Preparation of compound 84

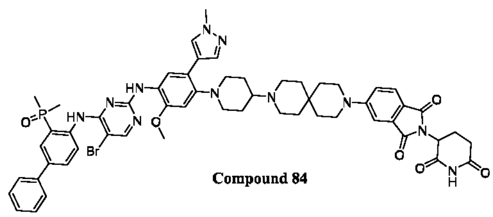

Compound 84

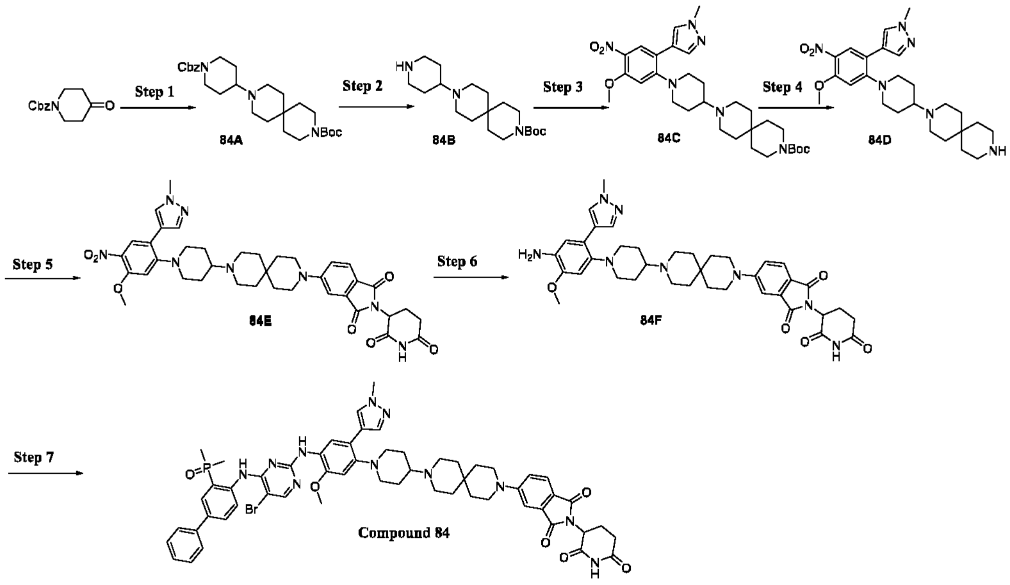


Step 1: tert-butyl 9-(1-((benzyloxy)carbonyl)piperidin-4-yl)-3,9-diazaspiro [5.5]undecane-3-carboxylate (**84A**)

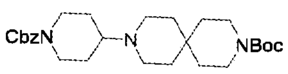

Benzyl 4-oxopiperidine-1-carboxylate (2.75 g, 11.79 mmol) and tert-butyl 3,9-diazaspiro[5.5]undecane-3-carboxylate (3.0 g, 11.79 mmol) were dissolved in dichloromethane (100 mL); acetic acid (710 mg, 11.79 mmol) was added; and the mixture was stirred at room temperature for 1 h. Sodium triacetoxyborohydride (5.0 g, 23.58 mmol) was added, and the mixture was reacted overnight at room temperature, adjusted to a basic pH by adding saturated aqueous sodium bicarbonate solution and extracted with dichloromethane (50 mL × 3). The organic phases were combined, washed with water (100 mL × 2), dried over anhydrous sodium sulphate and concentrated under reduced pressure, and the residue was purified by silica gel column chromatography (mobile phase: ethyl acetate/petroleum ether (V/V) = 1/1 to dichloromethane/methanol (V/V) = 20/1) to obtain **84A** (2.5 g, yield: 45.0%).

LCMS m/z = 472.3 $[M+H]^+$.

Step 2: tert-butyl 9-(piperidin-4-yl)-3,9-diazaspiro[5.5]undecane-3-carboxylate (**84B**)

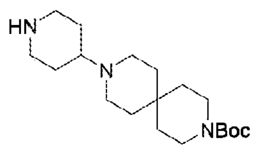

**84A** (2.5 g, 5.30 mmol) and palladium on carbon (1.5 g, 10 wt%) were dissolved in 60 mL of methanol, and then the mixture was subjected to hydrogen replacement three times, reacted at room temperature for 3 h and subjected to suction filtration over celite. The filtrate was concentrated under reduced pressure, and the residue was purified by silica gel column chromatography (mobile phase: dichloromethane/methanol (V/V) = 25/1-10/1) to obtain **84B** (1.5 g, yield: 83.9%).

Step 3: tert-butyl 9-(1-(5-methoxy-2-(1-methyl-1H-pyrazol-4-yl)-4-nitrophenyl) piperidin-4-yl)- 3,9-diazaspiro[5.5]undecane-3-carboxylate (**84C**)

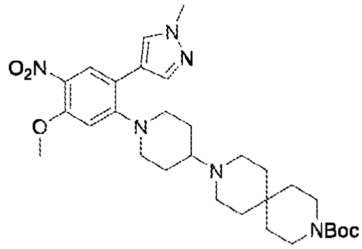

**84B** (1.5 g, 4.44 mmol) and 4-(2-fluoro-4-methoxy-5-nitrophenyl)-1-methyl-1H-pyrazole (**1D**) (1.1 g, 4.44 mmol) were dissolved in DMSO (10 mL); anhydrous potassium carbonate (1.84 g, 13.32 mmol) was added; and the mixture was reacted at 120°C for 6 h, cooled to room temperature and extracted by adding 50 mL of ethyl acetate and 30 mL of water. The organic layer was washed with saturated brine (20 mL × 3), dried over anhydrous sodium sulphate and concentrated under reduced pressure, and the residue was purified by column chromatography (dichloromethane: methanol (V/V) = 100/1-20/1) to obtain **84C** (1.0 g, yield: 39.6%).

LCMS m/z = 569.3 $[M+H]^+$.

Step 4: 3-(1-(5-methoxy-2-(1-methyl-1H-pyrazol-4-yl)-4-nitrophenyl) piperidin-4-yl)-3,9-diazaspiro[5.5]undecane (**84D**); HCl

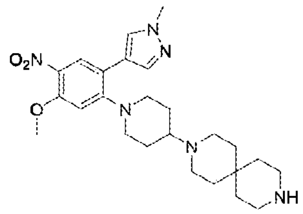

**84C** (1.0 g, 1.76 mmol) was dissolved in methanol (3 mL); a solution of hydrochloric acid in dioxane (4 *N*, 15 mL) was added; and the mixture was reacted at room temperature for 1 h and concentrated under reduced pressure, and the residue was directly used in the next step.

LCMS m/z = 469.2 $[M+H]^+$.

Step 5: 2-(2,6-dioxopiperidin-3-yl)-5-(9-(1-(5-methoxy-2-(1-methyl-1H-pyrazol-4-yl)-4-nitrophenyl)piperidin-4-yl)-3,9-diazaspiro[5.5]undecan-3-yl)isoindoline-1,3-dione (**84E**)

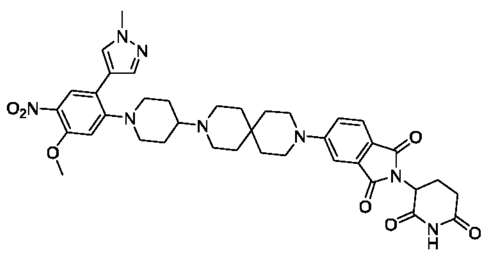

The crude hydrochloride of **84D** from the previous step was dissolved in DMSO (10 mL); 2-(2,6-dioxopiperidin-3-yl)-5-fluoroisoindoline-1,3-dione (490 mg, 1.76 mmol) and DIPEA (1.14 g, 8.80 mmol) were successively added; and the mixture was stirred at 100°C for 5 h, and cooled to room temperature. 10 mL of water was added, and the mixture was filtered. The filter cake was dried under reduced pressure and then purified by silica gel column chromatography (mobile phase: dichloromethane/methanol (V/V) = 100/1-20/1) to obtain **84E** (600 mg, yield: 47.0%).

LCMS m/z = 725.4 $[M+H]^+$.

Step 6: 5-(9-(1-(4-amino-5-methoxy-2-(1-methyl-1H-pyrazol-4-yl)phenyl) piperidin-4-yl)-3,9-diazaspiro[5.5]undecan-3-yl)-2-(2,6-dioxopiperidin-3-yl)isoindoline-1,3-dione (**84F**)

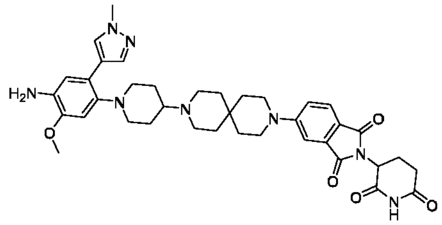

**84E** (600 mg, 0.83 mmol) was dissolved in ethanol/water (9 mL/3 mL); iron powder (370 mg, 6.64 mmol) and ammonium chloride (360 mg, 6.64 mmol) were successively added; and under nitrogen protection, the mixture was stirred at 80°C for 3 h, cooled to room temperature and subjected to suction filtration under reduced pressure. The filtrate was concentrated under reduced pressure, and then the crude was purified by silica gel column chromatography (mobile phase: dichloromethane/methanol (V/V) = 100/1-20/1) to obtain **84F** (520 mg, yield: 90%).

Step 7: 5-(9-(1-(4-((5-bromo-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperidin-4-yl)-3,9-diazaspiro[5.5]undecan-3-yl)-2-(2,6-dioxopiperidin-3-yl)isoindoline-1,3-dione (**compound 84**)

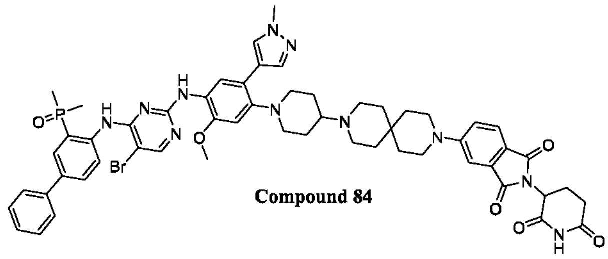

**Compound 84**

**84F** (220 mg, 0.32 mmol) and **23D** (150 mg, 0.35 mmol) were dissolved in DMF (3 mL); p-toluenesulfonic acid monohydrate (182 mg, 0.96 mmol) was added; and under nitrogen protection, the mixture was stirred at 100°C for 16 h, and cooled to room temperature. 10 mL of saturated aqueous sodium bicarbonate solution and 20 mL of dichloromethane were added, and the layers were separated. The organic layer was concentrated under reduced pressure, and the residue was purified by silica gel column chromatography to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 ml). Liquid separation was performed. The organic layer was then dried over anhydrous sodium sulphate and concentrated under reduced pressure to obtain **compound 84** (100 mg, yield: 28.5%).

LCMS m/z = 547.8 $[(M+2H)/2]^+$.

$^1$H NMR (400 MHz, DMSO-$d_6$) δ 11.11 (s, 1H), **11.05** (s, 1H), 8.48 - 8.34 (m, 1H), 8.27 (s, 1H), 8.18 (s, 1H), 8.00 (s, 1H), 7.86 (s, 1H), 7.76 - 7.62 (m, 2H), 7.61 - 7.49 (m, 3H), 7.45 (t, 2H), 7.36 (t, 1H), 7.30 (s, 1H), 7.25 - 7.08 (m, 2H), 6.83 (s, 1H), 5.06 (dd, 1H), 3.79 (s, 3H), 3.76 (s, 3H), 3.55 - 3.39 (m, 4H), 3.18 - 3.01 (m, 2H), 2.97 - 2.77 (m, 1H), 2.70 - 2.50 (m, 8H), 2.31 - 2.16 (m, 1H), 2.06 - 1.95 (m, 1H), 1.91 - 1.71 (m, 8H), 1.65 - 1.41 (m, 10H).

**Example 85: Preparation of compound 85**

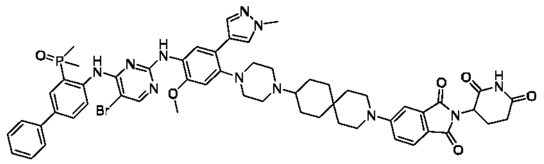

Compound 85

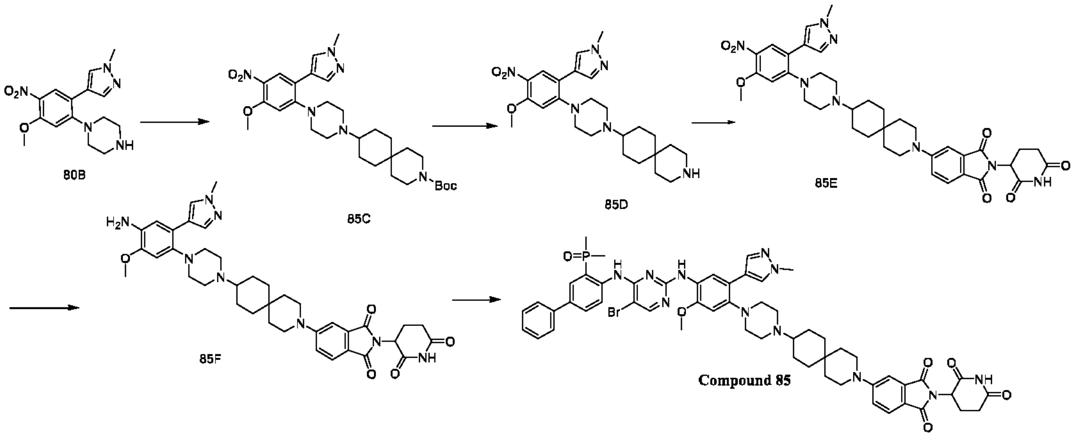


Step 1: tert-butyl 9-(4-(5-methoxy-2-(1-methyl-1H-pyrazol-4-yl)-4-nitrophenyl) piperazin-1-yl)-3-azaspiro[5.5] undecane-3-carboxylate (**85C**)

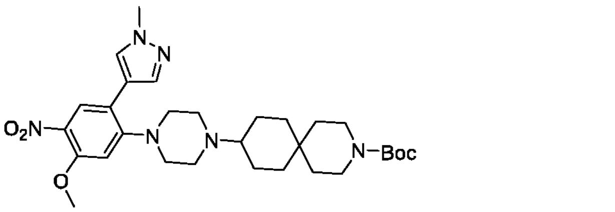

1-(5-methoxy-2-(1-methyl-1H-pyrazol-4-yl)-4-nitrophenyl)piperazine (**80B**) hydrochloride (1.05 g, crude) was added to 30 mL of THF; sodium bicarbonate (806 mg, 9.60 mmol) was added; and the mixture was stirred at room temperature for 20 min. 3-Boc-9-oxo-3-azaspiro[5.5]undecane (1.28 g, 4.80 mmol) and 0.3 mL of acetic acid were added, and then the mixture was stirred at room temperature for 2 h. Sodium triacetoxyborohydride (1.02 g, 4.80 mmol) was added, and the mixture was reacted at room temperature for 16 h, adjusted to a basic pH by adding saturated aqueous sodium bicarbonate solution and extracted 3 times with ethyl acetate. The organic phases were combined, washed sequentially with water and saturated brine, dried over anhydrous sodium sulphate and concentrated under reduced pressure, and the residue was purified by silica gel column chromatography (mobile phase: DCM/MeOH (V/V) = 50/1-20/1) to obtain **85C** (1.1 g, yield: 82%).

LCMS m/z = 569.3 $[M+1]^+$.

Step 2: 9-(4-(5-methoxy-2-(1-methyl-1H-pyrazol-4-yl)-4-nitrophenyl)piperazin-1-yl)-3-azaspiro[5.5]undecane (**85D**); 2,2,2-trifluoroacetate

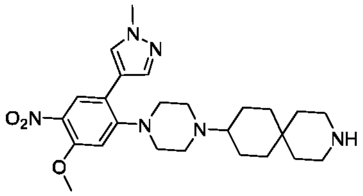

**85C** (1.0 g, 1.76 mmol) was dissolved in 25 mL of DCM; 10 mL of trifluoroacetic acid was added; and the mixture was stirred at room temperature for 2 h. The reaction solution was concentrated under reduced pressure to obtain the trifluoroacetate of **85D** (1.2 g, crude).

LC-MS m/z = 469.3$[M+1]^+$.

Step 3: 2-(2,6-dioxopiperidin-3-yl)-5-(9-(4-(5-methoxy-2-(1-methyl-1H-pyrazol-4-yl)-4-nitrophenyl)piperazin-1-yl)-3-azaspiro[5.5]undecan-3-yl)isoindoline-1,3-dione (**85E**)

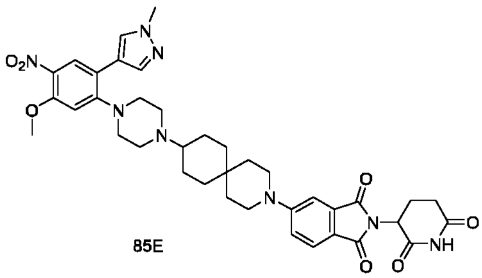

85E

The trifluoroacetate of **85D** (1.2 g, crude) was dissolved in 20 mL of DMSO; solid sodium bicarbonate (740 mg, 8.81 mmol) was added; and the mixture was stirred at room temperature for 10 min. 3 mL of DIPEA and 2-(2,6-dioxo-piperidin-3-yl)-5-fluoroisoindoline-1,3-dione (585 mg, 2.12 mmol) were added, and the reaction was stirred at 80°C for 5 h. The reaction solution was cooled to room temperature. 200 mL of water was added, and the mixture was filtered to collect the solid, which was washed with water, dissolved in DCM, dried over anhydrous sodium sulphate and concentrated under reduced pressure. The residue was purified by silica gel column chromatography (mobile phase: DCM/MeOH (V/V) = 15/1) to obtain **85E** (1.1 g, yield: 88%).

LCMS m/z = 725.3 $[M +1]^+$.

Step 4: 5-(9-(4-(4-amino-5-methoxy-2-(1-methyl-1H-pyrazol-4-yl)phenyl) piperazin-1-yl)-3-azaspiro[5.5]undecan-3-yl)-2-(2,6-dioxopiperidin-3-yl)isoindoline-1,3-dione (**85F**)

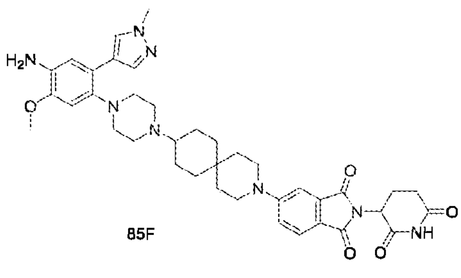

85F

**85E** (1.1 g, 1.52 mmol) was dissolved in a mixed solution of THF (10 mL), ethanol (30 mL) and water (6 mL); reduced iron powder (827 mg, 14.77 mmol) and ammonium chloride (790 mg, 14.77 mmol) were added; and the mixture was reacted at 85°C for 2 h. The reaction solution was cooled to room temperature and concentrated in vacuo to remove a solvent. 50 mL of water and 50 mL of dichloromethane were added to the residue, and then the mixture was extracted 3 times with dichloromethane. The organic phases were combined, dried over anhydrous sodium sulphate and then concentrated under reduced pressure to obtain **85F** (0.71 g, yield: 66%).

LCMS m/z = 695.3 $[M+H]^+$.

Step 5: 5-(9-(4-(4-((5-bromo-4-((3-(dimethylphosphoryl)-[1,1'-biphenyl]-4-yl) amino)pyrimidin-2-yl)amino)-5-methoxy-2-(1-methyl-1H-pyrazol-4-yl)phenyl)piperazin-1-yl)-3-azaspiro[5.5]undecan-3-yl)-2-(2,6-dioxopiperidin-3-yl)isoindoline-1,3-dione (**compound 85**)

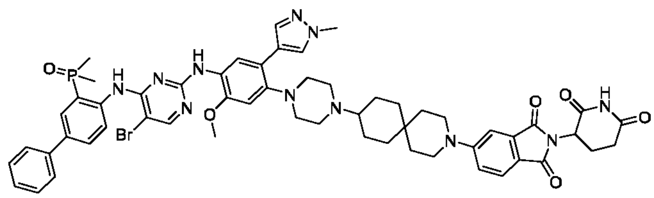

**85F** (320 mg, 0.46 mmol) and **23D** (241 mg, 0.55 mmol) were dissolved in DMF (20 mL); p-toluenesulfonic acid monohydrate (263 mg, 1.38 mmol) was added; and the mixture was stirred at 100°C for 8 h, and cooled to room temperature. 100 mL of saturated aqueous sodium bicarbonate solution was added, with a yellow solid precipitated. Suction filtration was performed for collecting the solid, and the filter cake was washed with water (100 ml × 3) and redissolved in dichloromethane. The organic layers were separated, dried over anhydrous sodium sulphate and concentrated, and the crude was further subjected to preparative HPLC (instrument: waters 2767 (preparative liquid phase chromatographic instrument); chromatographic column: XBridge@ Prep C18 (30 mm × 150 mm); composition of mobile phases: mobile phase A: acetonitrile, and mobile phase B: water (containing 0.1% TFA)) purification to obtain a preparative solution, which was concentrated and adjusted to a basic pH by adding dichloromethane and saturated sodium bicarbonate solution. The organic phase was separated. The aqueous phase was extracted with dichloromethane (100 ml × 3). The organic phase was dried over anhydrous sodium sulphate and concentrated, and the crude was subjected to prep-TLC (DCM: MeOH (V/V) = 15:1) purification to obtain **compound 85** (180 mg, yield: 33%).

LCMS m/z = 1094.3 $[M+1]^+$.

$^1H$ NMR (400 MHz, DMSO-$d_6$) δ 11.11 (s, 1H), 11.05 (s, 1H), 8.51 - 8.34 (m, 1H), 8.27 (s, 1H), 8.19 (s, 1H), 8.00 (s, 1H), 7.88 (s, 1H), 7.75 - 7.62 (m, 2H), 7.62 - 7.50 (m, 3H), 7.45 (t, 2H), 7.36 (t, 1H), 7.33 - 7.10 (m, 3H), 6.85 (s, 1H), 5.06 (dd, 1H), 3.80 (s, 3H), 3.75 (s, 3H), 3.57 - 3.40 (m, 4H), 2.94 - 2.77 (m, 5H), 2.70 - 2.52 (m, 6H), 2.30 - 2.20 (m, 1H), 2.08 - 1.98 (m, 1H), 1.89 - 1.73 (m, 8H), 1.72-1.64 (m, 2H), 1.63 - 1.52 (m, 2H), 1.50 - 1.32 (m, 4H), 1.21 - 1.06 (m, 2H).

**Example 86: Preparation of compound 86**

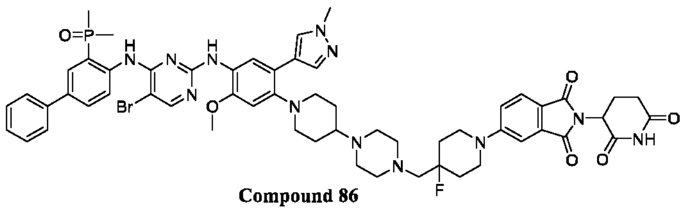

Compound 86

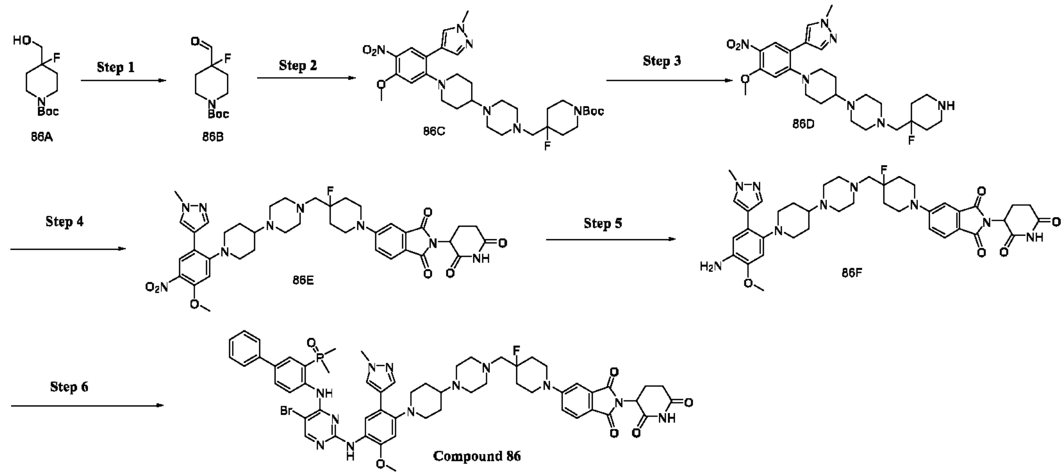


Step 1: tert-butyl 4-fluoro-4-formylpiperidine-1-carboxylate **(86B)**

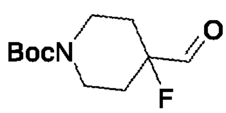

86B

At 0°C, Dess-Martin periodinane (8.48 g, 20 mmol) was added in batches to a solution of **86A** (2.33 g, 10 mmol) in dichloromethane, and the mixture was stirred at room temperature for 2 h and extracted by adding 20 mL of saturated aqueous sodium bicarbonate solution and 20 mL of dichloromethane. The organic layer was concentrated under reduced pressure, and the residue was purified by silica gel column chromatography (mobile phase: PE/EA (V/V) = 10/1-1/1) to obtain **86B** (2.1 g, yield: 90.9%).

$^1$H NMR (400 MHz, $CDCl_3$) δ 9.66 (d, 1H), 4.11 - 3.85 (m, 2H), 3.17-2.84 (m, 2H), 1.86 - 1.59 (m, 4H), 1.47-1.35 (m, 9H).

Step 2: tert-butyl 4-fluoro-4-((4-(1-(5-methoxy-2-(1-methyl-1H-pyrazol-4-yl)-4-nitrophenyl)piperidin-4-yl)piperazin-1-yl)methyl)piperidine-1-carboxylate **(86C)**

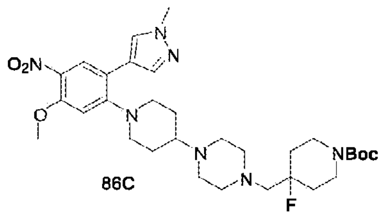

86C

**7E** (400 mg, 1.0 mmol) and **86B** (462 mg, 2.0 mmol) were dissolved in 1,2-dichloroethane (10 mL); a 4Å molecular sieve (200 mg) and acetic acid (0.12 g, 2 mmol) were successively added. The mixture was stirred at room temperature for 2 h; sodium triacetoxyborohydride (0.38 g, 1.79 mol) was added; and the resulting mixture was reacted overnight at room temperature. After the reaction was completed, the reaction system was directly concentrated under reduced pressure, and then the residue was purified by silica gel column chromatography (mobile phase: dichloromethane/methanol (V/V) = 100/1-10/1) to obtain **86C** (492 mg, yield: 80%).

LCMS m/z = 616.3[M+H]$^+$.

Step 3: 1-((4-fluoropiperidin-4-yl)methyl)-4-(1-(5-methoxy-2-(1-methyl-1H-pyrazol-4-yl)-4-nitrophenyl)piperidin-4-yl)piperazine **(86D)**

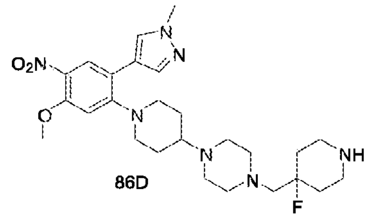

86D

**86C** (492 mg, 0.8 mmol) was dissolved in DCM (15 mL); trifluoroacetic acid (5 mL) was added at room temperature; and the mixture was stirred for 1 h and concentrated under reduced pressure. 30 mL of dichloromethane was added to the residue, and the mixture was adjusted to pH = 9-10 with 1 N aqueous NaOH solution. Liquid separation was performed. The organic layer was concentrated under reduced pressure to obtain **86D** (412 mg), which was directly used in the next step.
LCMS m/z = 516.3 $[M+H]^+$.

Step 4: 2-(2,6-dioxopiperidin-3 -yl)- 5-(4-fluoro-4-((4-(1-(5-methoxy-2-(1-methyl-1H-pyrazol-4-yl)-4-nitrophenyl)piperidin-4-yl)piperazin-1-yl)methyl)piperidin-1-yl)isoindoline-1,3-dione **(86E)**

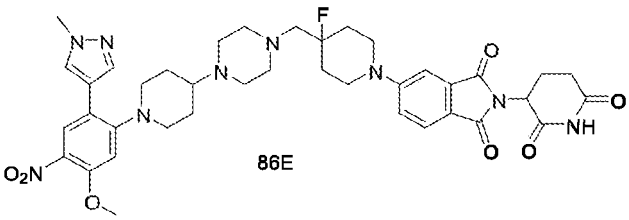

86E

**86D** (412 mg, 0.8 mmol) and 2-(2,6-dioxopiperidin-3-yl)-5-fluoroisoindole-1,3-dione (276 mg, 1 mmol) were dissolved in DMSO (10 mL); DIPEA (0.27 g, 2.07 mmol) was added dropwise; and the mixture was reacted at 90°C for 3 h. The reaction solution was cooled to room temperature. 20 mL of water was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase: dichloromethane/methanol (V/V) = 50/1-12/1) to obtain **86E** (370 mg, yield: 60%).
LCMS m/z = 772.3 $[M+H]^+$.

Step 5: 5-(4-((4-(1-(4-amino-5-methoxy-2-(1-methyl-1H-pyrazol-4-yl)phenyl) piperidin-4-yl)piperazin-1-yl)methyl)-4-fluoropiperidin-1-yl)-2-(2,6-dioxopiperidin-3-yl)isoindoline-1,3-dione **(86F)**

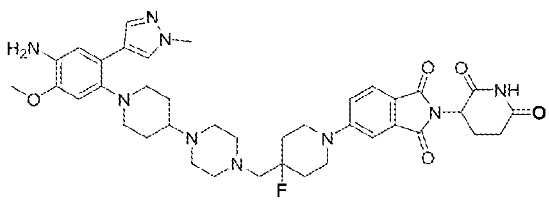

86F

**86E** (0.37 g, 0.48 mmol) was dissolved in ethanol (15 mL); reduced iron powder (0.07 g, 1.30 mmol) was added,

and then an aqueous solution (5 mL) of ammonium chloride (0.07 g, 1.30 mmol) was added dropwise; and under nitrogen protection, the mixture was reacted at 80°C for 3 h. The reaction solution was cooled to room temperature. 5 mL of water was added, and then the mixture was extracted 3 times with dichloromethane. The organic phases were combined, dried over anhydrous sodium sulphate and then concentrated under reduced pressure, and the residue was purified by column chromatography (mobile phase: dichloromethane/methanol (V/V) = 50/1-12/1) to obtain **86F** (170 mg, yield: 47.9%)

LCMS m/z = 371.7 $[(M+2H)/2]^+$.

Step 6: 5-(4-((4-(1-(4-((5-bromo-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperidin-4-yl)piperazin-1-yl)methyl)-4-fluoropiperidin-1-yl)-2-(2,6-dioxopiperidin-3-yl)isoindoline-1,3-dione (**compound 86**); 2,2,2-trifluoroacetic acid

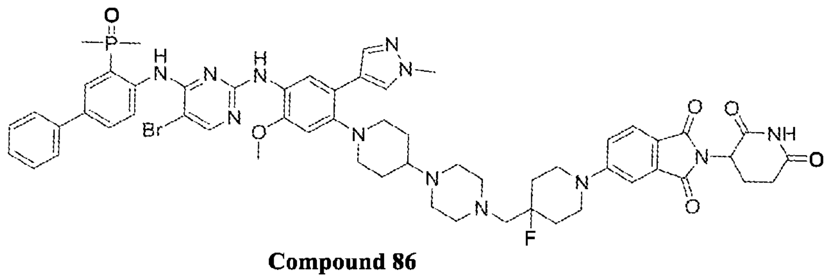

**Compound 86**

**86F** (0.13 g, 0.18 mmol) and **23D** (0.08 g, 0.18 mmol) were dissolved in DMF (10 mL); p-toluenesulfonic acid monohydrate (0.09 g, 0.47 mmol) was added; and under nitrogen protection, the mixture was reacted at 100°C for 16 h. The reaction solution was cooled to room temperature. 5 mL of water and 5 mL of saturated aqueous sodium bicarbonate solution were added, and the mixture was filtered. The filter cake was dried under reduced pressure and then purified by silica gel column chromatography (dichloromethane/methanol (V/V) = 100/1-10/1) to obtain a product, which was further purified by preparative HPLC (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized to obtain the **trifluoroacetate of compound 86** (10 mg).

LCMS m/z = 571.3 $[(M+2H)/2]^+$.

$^1H$ NMR (400 MHz, $CD_3OD$) δ 8.29 - 8.16 (m, 2H), 7.83 - 7.72 (m, 3H), 7.71 - 7.59 (m, 2H), 7.55 - 7.42 (m, 4H), 7.42 - 7.29 (m, 3H), 7.26 (dd, 1H), 6.83 (s, 1H), 5.06 (dd, 1H), 3.95 - 3.79 (m, 5H), 3.68 (s, 3H), 3.62 - 3.32 (m, 5H), 3.28 - 3.03 (m, 6H), 2.96 - 2.45 (m, 9H), 2.17 - 2.03 (m, 5H), 2.01 - 1.70 (m, 10H).

**Example 87: Preparation of compound 87**

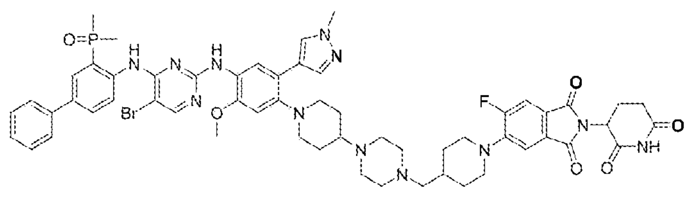

**Compound 87**

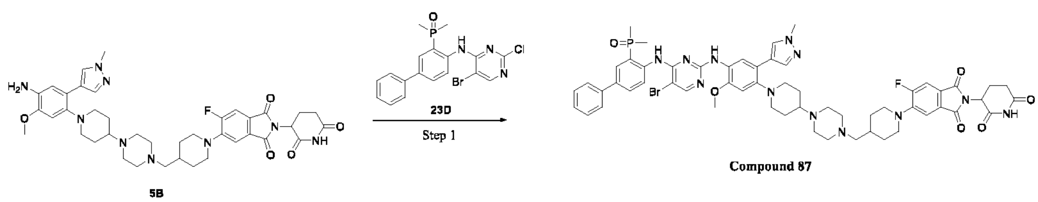


5B

Compound 87

Step 1: 5-(4-((4-(1-(4-((5-bromo-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperidin-4-yl)piperazin-1-yl)methyl)piperidin-1-yl)-2-(2,6-dioxopiperidin-3-yl)-6-fluoroisoindoline-1,3-dione (**compound 87**)

**5B** (150 mg, 0.20 mmol) and **23D** (96 mg, 0.22 mmol) were dissolved in DMF (10 mL); p-toluenesulfonic acid monohydrate (114 mg, 0.6 mmol) was added; and the mixture was reacted at 100°C for 16 h. The reaction solution was cooled to room temperature. 10 mL of saturated aqueous sodium bicarbonate solution was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase: dichloromethane/methanol (V/V) = 50/1-10/1) to obtain the crude, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)) to obtain a preparative solution, which was concentrated. 30 mL of dichloromethane was added, and the mixture was adjusted to a basic pH by adding saturated sodium bicarbonate solution. Liquid separation was performed. The organic layer was then dried over anhydrous sodium sulphate and concentrated under reduced pressure to obtain **compound 87** (25 mg, yield: 11%).

LCMS m/z = 571.2 $[(M+2H)/2]^+$.

$^1H$ NMR (400 MHz, DMSO-$d_6$) δ 11.11 (s, 1H), 11.08 (s, 1H), 8.47 - 8.32 (m, 1H), 8.27 (s, 1H), 8.18 (s, 1H), 8.01 (s, 1H), 7.85 (s, 1H), 7.78 - 7.63 (m, 2H), 7.62 - 7.40 (m, 6H), 7.36 (t, 1H), 7.29 - 7.05 (m, 1H), 6.83 (s, 1H), 5.10 (dd, 1H), 3.79 (s, 3H), 3.76 (s, 3H), 3.68 - 3.51 (m, 2H), 3.15 - 3.01 (m, 2H), 2.98 - 2.80 (m, 3H), 2.71 - 2.51 (m, 8H), 2.46 - 2.29 (m, 4H), 2.27 - 2.11 (m, 3H), 2.10 - 1.96 (m, 1H), 1.91 - 1.68 (m, 11H), 1.63 - 1.45 (m, 2H), 1.35 - 1.22 (m, 2H).

**Example 88: Preparation of compound 88**

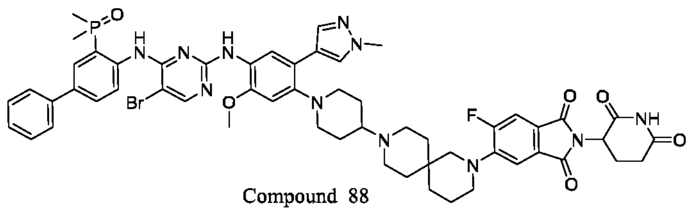

Compound 88

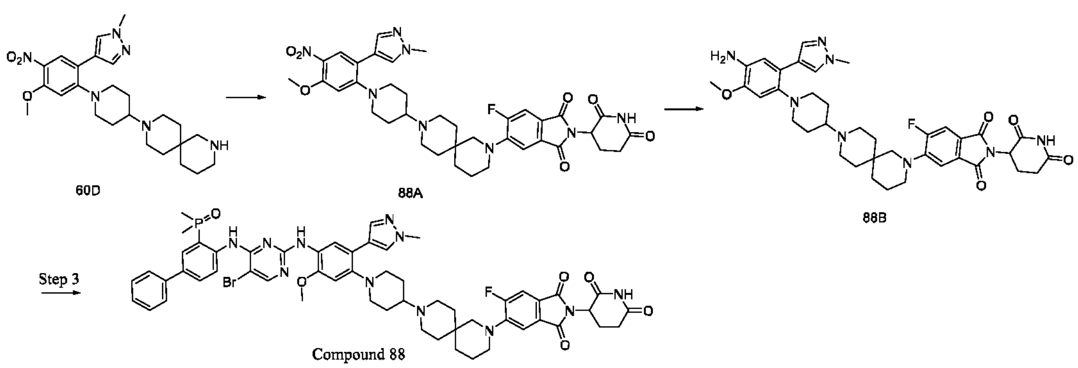


60D

88A

88B

Compound 88

Step 1: 2-(2,6-dioxopiperidin-3-yl)-5-fluoro-6-(9-(1-(5-methoxy-2-(1-methyl-1H-pyrazol-4-yl)-4-nitrophenyl)piperidin-4-yl)-2,9-diazaspiro[5.5]undecan-2-yl)isoindoline-1,3-dione (**88A**)

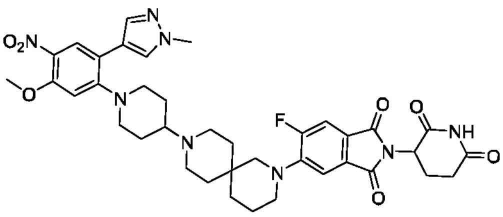

88A

**60D** (0.4 g, 0.85 mmol) and 2-(2,6-dioxopiperidin-3-yl)-5,6-difluoroisoindoline-1,3-dione (**5A-1**) (0.26 g, 0.94 mmol) were dissolved in DMSO (5 ml); DIPEA (0.22 g, 1.7 mmol) was added; and the mixture was stirred at 90°C for 5 h and cooled to room temperature. 10 mL of water was added, with a solid precipitated, and the mixture was filtered. The filter cake was dried under reduced pressure and then purified by silica gel column chromatography to obtain **88A** (0.5 g, yield: 79%).

Step 2: 5-(9-(1-(4-amino-5-methoxy-2-(1-methyl-1H-pyrazol-4-yl)phenyl) piperidin-4-yl)-2,9-diazaspiro[5.5]undecan-2-yl)-2-(2,6-dioxopiperidin-3-yl)-6-fluoroisoindoline-1,3-dione (**88B**)

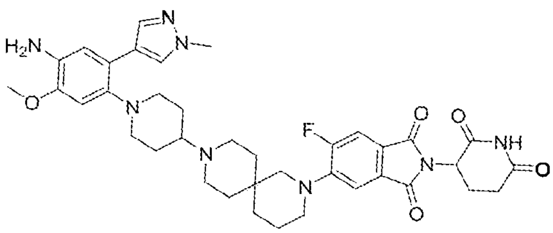

88B

**88A** (500 mg, 0.67 mmol) was dissolved in ethanol/water (8 mL, 3:1); iron powder (370 mg, 6.7 mmol) and ammonium chloride (360 mg, 6.7 mmol) were successively added; and under nitrogen protection, the mixture was stirred at 80°C for 1 h, cooled to room temperature and subjected to suction filtration under reduced pressure. The filtrate was extracted by adding 20 mL of dichloromethane and 20 mL of saturated aqueous sodium chloride solution. The organic layer was dried and then concentrated under reduced pressure to obtain **88B** (260 mg), which was directly used in the next step.

Step 3: 5-(9-(1-(4-((5-bromo-4-((3-(dimethylphosphoryl)-[1,1'-biphenyl]-4-yl) amino)pyrimidin-2-yl)amino)-5-methoxy-2-(1-methyl-1H-pyrazol-4-yl)phenyl)piperidin-4-yl)-2,9-diazaspiro[5.5]undecan-2-yl)-2-(2,6-dioxopiperidin-3-yl)-6-fluoroisoindoline-1,3-dione (**compound 88**)

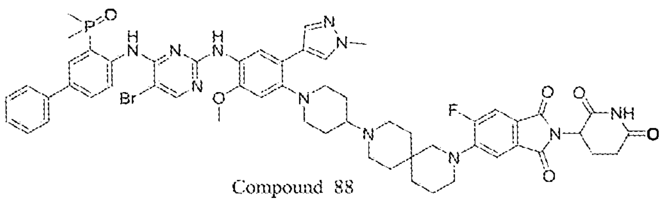

Compound 88

**88B** (140 mg, 0.20 mmol) and **23D** (87 mg, 0.20 mmol) were dissolved in DMF (5 mL) solution; p-toluenesulfonic acid monohydrate (110 mg, 0.60 mmol) was added; and under nitrogen protection, the mixture was reacted overnight at 100°C, and cooled to room temperature. 20 mL of saturated aqueous sodium bicarbonate solution was added, and the mixture was subjected to suction filtration. The filter cake was dissolved in 50 mL of dichloromethane and extracted. The organic phase was dried over anhydrous sodium sulphate and then concentrated under reduced pressure, and the residue was purified by silica gel column chromatography (dichloromethane/methanol (V/V) = 100/1-10/1) to obtain a product, which was further purified by preparative liquid phase chromatography (instrument: waters 2767 preparative

chromatographic column; SunFire@ Prep C18 (19 mm × 150 mm); composition of mobile phases: mobile phase A: acetonitrile, and mobile phase B: water (containing 0.1% TFA)) to obtain the trifluoroacetate of **compound 88.** 20 mL of dichloromethane and 10 mL of saturated sodium bicarbonate solution were added to the trifluoroacetate of **compound 88,** and the layers were separated. The organic phase was dried over anhydrous sodium sulphate and then concentrated under reduced pressure to obtain **compound 88** (40 mg, yield: 18%).

LCMS m/z = 556.8 $[(M+2H)/2]^+$.

$^1$H NMR (400 MHz, DMSO-$d_6$) δ 11.11 (s, 1H), 11.09 (s, 1H), 8.50 - 8.32 (m, 1H), 8.26 (s, 1H), 8.19 (s, 1H), 8.00 (s, 1H), 7.86 (s, 1H), 7.75 - 7.64 (m, 2H), 7.64 - 7.51 (m, 3H), 7.51 - 7.41 (m, 3H), 7.34 (t, 1H), 7.23 - 7.07 (m, 1H), 6.82 (s, 1H), 5.10 (dd, 1H), 3.79 (s, 3H), 3.76 (s, 3H), 3.22 - 3.02 (m, 6H), 2.95 - 2.82 (m, 1H), 2.68 - 2.50 (m, 9H), 2.07 - 1.95 (m, 1H), 1.91 - 1.39 (m, 18H).

**Example 89: Preparation of compound 89**

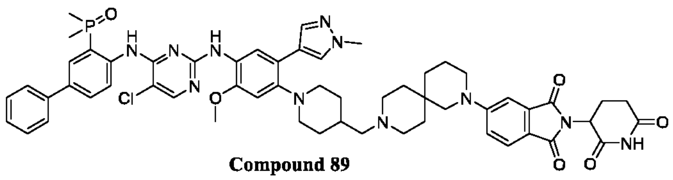

Compound 89

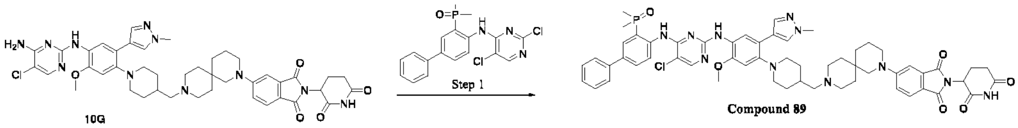


10G

Compound 89

Step 1: 5-(9-((1-(4-((5-chloro-4-((3-(dimethylphosphoryl)-[1,1'-biphenyl]-4-yl) amino)pyrimidin-2-yl)amino)-5-methoxy-2-(1-methyl-1H-pyrazol-4-yl)phenyl)piperidin-4-yl)methyl)-2,9-diazaspiro[5.5]undecan-2-yl)-2-(2,6-dioxopiperidin-3-yl)isoindoline-1,3-dione (**compound 89**)

**10G** (0.20 g, 0.28 mmol) and **40A** (0.11 g, 0.28 mmol) were dissolved in a mixed solvent of 1,4-dioxane (10 mL) and NMP (2 mL); p-toluenesulfonic acid hydrate (0.16 g, 0.84 mmol) was added; and under nitrogen protection, the mixture was reacted at 100°C for 16 h. The reaction solution was cooled to room temperature. 10 mL of saturated aqueous sodium bicarbonate solution was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase: dichloromethane/methanol (V/V) = 50/1-15/1) to obtain a product, which was further purified by preparative HPLC (composition of mobile phases: mobile phase A: acetonitrile, and mobile phase B: water (containing 0.05% trifluoroacetic acid)) to obtain a product, which was alkalized with aqueous sodium bicarbonate solution, extracted with dichloromethane, concentrated and dried to obtain **compound 89** (30 mg, yield: 10%).

LCMS m/z = 532.8 $[(M+2H)/2]^+$.

$^1$H NMR (400 MHz, DMSO-$d_6$) δ 11.37 (s, 1H), 11.04 (s, 1H), 8.55 - 8.40 (m, 1H), 8.28 (s, 1H), 8.11 (s, 1H), 8.03 (s, 1H), 7.86 (s, 1H), 7.70 (dd, 1H), 7.62 (d, 1H), 7.59 - 7.47 (m, 3H), 7.42 (t, 2H), 7.36 - 7.26 (m, 2H), 7.24 (dd, 1H), 7.20 - 7.05 (m, 1H), 6.85 (s, 1H), 5.05 (dd, 1H), 3.78 (s, 3H), 3.75 (s, 3H), 3.55 - 3.34 (m, 4H), 3.11 - 3.00 (m, 2H), 2.95 - 2.79 (m, 1H), 2.71 - 2.51 (m, 4H), 2.44 - 2.16 (m, 6H), 2.04 - 1.95 (m, 1H), 1.83 (d, 6H), 1.74-1.35 (m, 11H), 1.32 - 1.25 (m, 2H).

**Example 90: Preparation of compound 90**

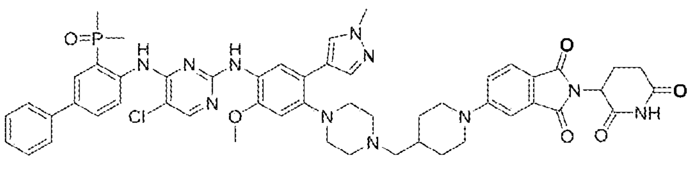

**Compound 90**

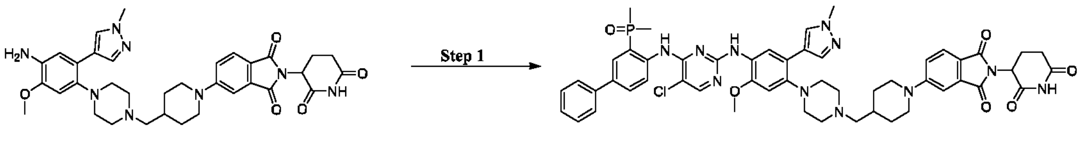

Step 1: 5-(4-((4-(4-((5-chloro-4-((3-(dimethylphosphoryl)-[1,1'-biphenyl]-4-yl) amino)pyrimidin-2-yl)amino)-5-methoxy-2-(1-methyl-1H-pyrazol-4-yl)phenyl)piperazin-1-yl)methyl)piperidin-1-yl)-2-(2,6-dioxopiperidin-3-yl)isoindoline-1,3-dione (**compound 90**)

**77D** (200 mg, 0.31 mmol) and **40A** (180 mg, 0.46 mmol) were dissolved in DMF (10 mL); p-toluenesulfonic acid monohydrate (160 mg, 0.84 mmol) was added; and under nitrogen protection, the mixture was stirred at 100°C for 16 h, and cooled to room temperature. 10 mL of saturated aqueous sodium bicarbonate solution and 20 mL of dichloromethane were added, and the layers were separated. The organic layer was concentrated under reduced pressure. The concentrated solution was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 ml). Liquid separation was performed. The organic layer was then dried over anhydrous sodium sulphate and concentrated under reduced pressure to obtain **compound 90** (50 mg, yield: 16%).

LCMS m/z = 498.8 $[(M+2H)/2]^+$.

$^1H$ NMR (400 MHz, $D_2O/CF_3COOD$(v/v = 1:1)) δ 8.32 - 8.03 (m, 7H), 7.86 (s, 1H), 7.72 (d, 1H), 7.60 - 7.45 (m, 5H), 7.40 - 7.23 (m, 1H), 6.98 (s, 1H), 5.21 (dd, 1H), 3.98 - 3.89 (m, 5H), 3.89 - 3.72 (m, 5H), 3.72 - 3.64 (m, 2H), 3.42 - 3.10 (m, 8H), 2.97 - 2.89 (m, 2H), 2.85 - 2.70 (m, 1H), 2.65 - 2.47 (m, 1H), 2.45 - 2.32 (m, 2H), 2.32 - 2.21 (m, 1H), 2.21 - 2.07 (m, 2H), 2.02 (d, 6H).

**Example 91: Preparation of compound 91**

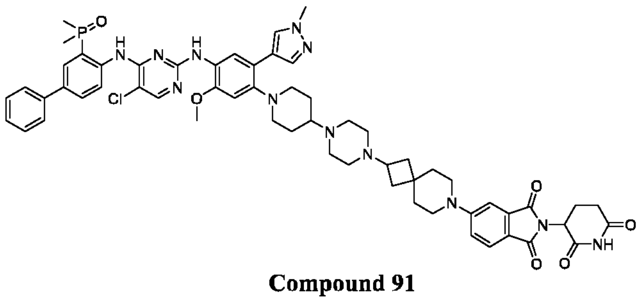

**Compound 91**

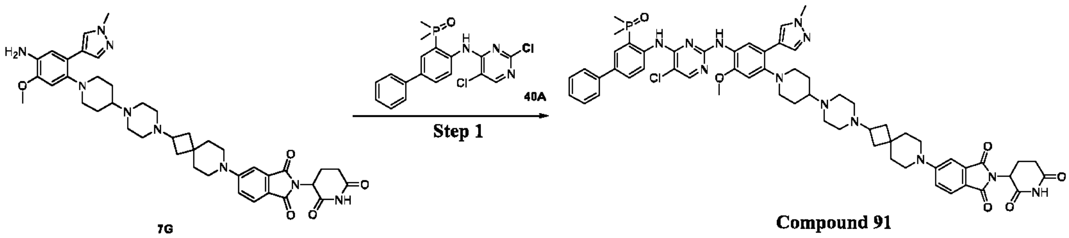


7G

Compound 91

Step 1: 5-(2-(4-(1-(4-(5-chloro-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperidin-4-yl)piperazin-1-yl)-7-azaspiro[3.5]nonan-7-yl)-2-(2,6-dioxopiperidin-3-yl)isoindoline-1,3-dione (**compound 91**)

**7G** (285 mg, 0.38 mmol) and **40A** (179 mg, 0.46 mmol) were dissolved in DMF (5 mL); p-toluenesulfonic acid monohydrate (217 mg, 1.14 mmol) was added; and under nitrogen protection, the mixture was reacted overnight at 100°C, and cooled to room temperature. 20 mL of saturated aqueous sodium bicarbonate solution was added, and the mixture was subjected to suction filtration. The filter cake was dissolved in dichloromethane and extracted. The organic phase was dried over anhydrous sodium sulphate and then concentrated under reduced pressure, and the residue was purified by silica gel column chromatography (dichloromethane/methanol (V/V) = 100/1-10/1) to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 ml). Liquid separation was performed. The organic layer was then dried over anhydrous sodium sulphate and concentrated under reduced pressure to obtain **compound 91** (65 mg, yield: 15%).

LCMS m/z = 1105.4 $[M+H]^+$.

$^1H$ NMR (400 MHz, DMSO-$d_6$) δ 11.37 (s, 1H), 11.06 (s, 1H), 8.63 - 8.39 (m, 1H), 8.27 (s, 1H), 8.11 (s, 1H), 8.02 (s, 1H), 7.86 (s, 1H), 7.71 (dd, 1H), 7.64 (d, 1H), 7.60 (s, 1H), 7.58 - 7.50 (m, 2H), 7.45 (t, 2H), 7.41 - 7.34 (m, 1H), 7.33 - 7.26 (m, 1H), 7.27 - 7.08 (m, 2H), 6.84 (s, 1H), 5.06 (dd, 1H), 3.79 (s, 3H), 3.76 (s, 3H), 3.52 - 3.35 (m, 4H), 3.18 - 3.03 (m, 2H), 2.97 - 2.79 (m, 1H), 2.76 - 2.50 (m, 8H), 2.44 - 2.11 (m, 6H), 2.10 - 1.91 (m, 3H), 1.91 - 1.71 (m, 8H), 1.69 - 1.48 (m, 8H).

**Example 92: Preparation of compound 92**

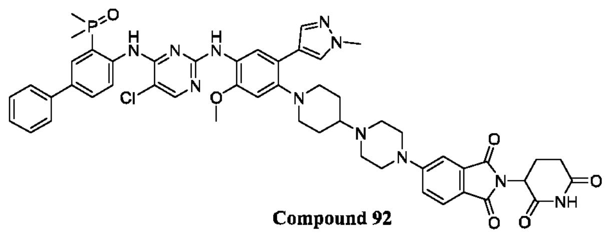

Compound 92

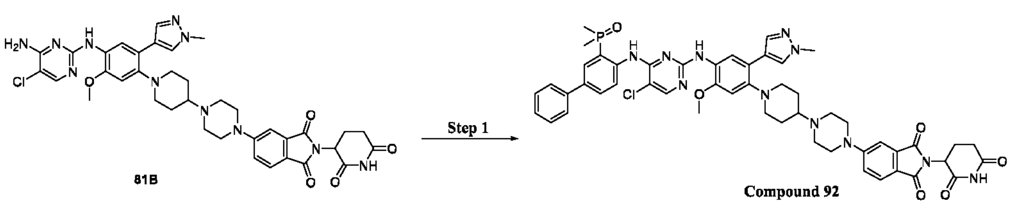


81B

Compound 92

Step 1: 5-(4-(1-(4-((5-chloro-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperidin-4-yl)piperazin-1-yl)-2-(2,6-dioxopiperidin-3-yl)isoindoline-1,3-dione (**compound 92**)

**81B** (200 mg, 0.32 mmol) and **40A** (180 mg, 0.46 mmol) were dissolved in DMF (10 mL); p-toluenesulfonic

acid monohydrate (160 mg, 0.84 mmol) was added; and under nitrogen protection, the mixture was stirred at 100°C for 16 h, and cooled to room temperature. 10 mL of saturated aqueous sodium bicarbonate solution and 20 mL of dichloromethane were added, and the layers were separated. The organic layer was concentrated under reduced pressure. The concentrated solution was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 mL). Liquid separation was performed. The organic layer was then dried over anhydrous sodium sulphate and concentrated under reduced pressure to obtain **compound 92** (40 mg, yield: 13%).

LCMS m/z = 491.7 $[(M+2H)/2]^+$.

$^1H$ NMR (400 MHz, DMSO-$d_6$) δ 11.37 (s, 1H), 11.06 (s, 1H), 8.55 - 8.41 (m, 1H), 8.28 (s, 1H), 8.11 (s, 1H), 8.03 (s, 1H), 7.88 (s, 1H), 7.76 - 7.65 (m, 2H), 7.65 - 7.51 (m, 3H), 7.46 (t, 2H), 7.41 - 7.33 (m, 2H), 7.31 - 7.11 (m, 2H), 6.85 (s, 1H), 5.08 (dd, 1H), 3.80 (s, 3H), 3.77 (s, 3H), 3.53 - 3.39 (m, 4H), 3.19 - 3.05 (m, 2H), 2.97 - 2.83 (m, 1H), 2.74 - 2.51 (m, 8H), 2.36 - 2.22 (m, 1H), 2.08 - 1.92 (m, 1H), 1.89 - 1.74 (m, 8H), 1.67 - 1.53 (m, 2H).

**Example 93: Preparation of compound 93**

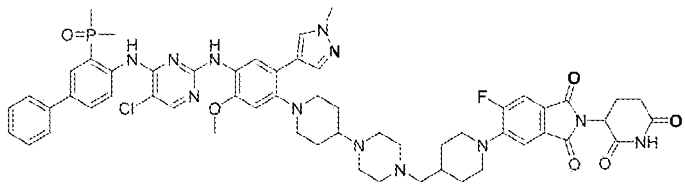

**Compound 93**

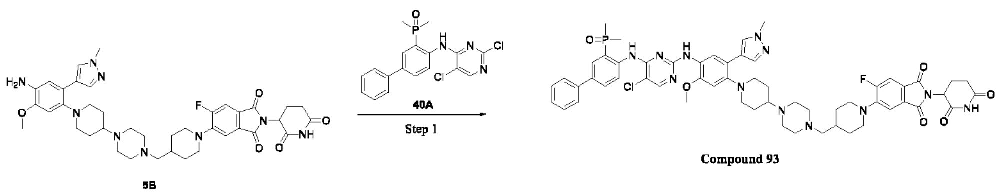


Step 1: 5-(4-((4-(1-(4-((5-chloro-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperidin-4-yl)piperazin-1-yl)methyl)piperidin-1-yl)-2-(2,6-dioxopiperidin-3-yl)-6-fluoroisoindoline-1,3-dione (**compound 93**)

**5B** (150 mg, 0.20 mmol) and **40A** (94 mg, 0.24 mmol) were dissolved in DMF (10 mL); p-toluenesulfonic acid monohydrate (114 mg, 0.6 mmol) was added; and the mixture was reacted at 100°C for 16 h. The reaction solution was cooled to room temperature. 10 mL of saturated aqueous sodium bicarbonate solution was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase: dichloromethane/methanol (V/V) = 50/1-10/1) to obtain the crude, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)) to obtain a preparative solution, which was concentrated. 30 mL of dichloromethane was added, and the mixture was adjusted to a basic pH by adding saturated sodium bicarbonate solution. Liquid separation was performed. The organic layer was then dried over anhydrous sodium sulphate and concentrated under reduced pressure to obtain **compound 93** (20 mg, yield: 9%).

LCMS m/z = 549.3 $[(M+2H)/2]^+$.

$^1H$ NMR (400 MHz, DMSO-$d_6$) δ 11.36 (s, 1H), 11.08 (s, 1H), 8.60 - 8.38 (m, 1H), 8.26 (s, 1H), 8.11 (s, 1H), 8.03 (s, 1H), 7.86 (s, 1H), 7.77 - 7.65 (m, 2H), 7.64 - 7.51 (m, 3H), 7.51 - 7.41 (m, 3H), 7.40 - 7.32 (m, 1H), 7.23 - 7.06 (m, 1H), 6.84 (s, 1H), 5.10 (dd, 1H), 3.79 (s, 3H), 3.76 (s, 3H), 3.67 - 3.51 (m, 2H), 3.15 - 3.03 (m, 2H), 2.96 - 2.78 (m, 3H), 2.70 - 2.51 (m, 8H), 2.47 - 2.29 (m, 4H), 2.26 - 2.12 (m, 3H), 2.08-1.98 (m, 1H), 1.91 - 1.66 (m, 11H), 1.62 - 1.45 (m, 2H), 1.34 - 1.18 (m, 2H).

## Example 94: Preparation of compound 94

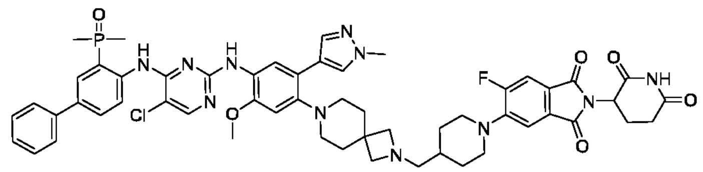

**Compound 94**

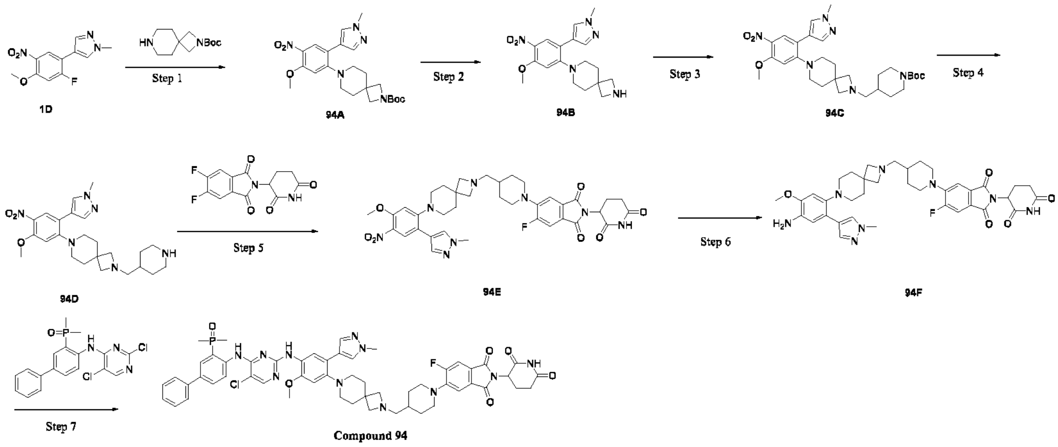


Step 1: tert-butyl 7-(5-methoxy-2-(1-methyl-1H-pyrazol-4-yl)-4-nitrophenyl)-2,7-diazaspiro[3.5]nonane-2-carboxylate (**94A**)

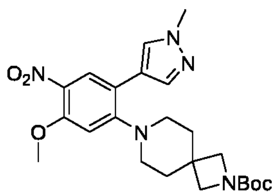

**94A**

**1D** (2 g, 7.96 mmol) and tert-butyl 2,7-diazaspiro[3.5]nonane-2-carboxylate (2.67 g, 11.80 mmol) were dissolved in DMSO (30 mL); potassium carbonate (3.26 mg, 23.59 mmol) was added; and the mixture was reacted at 120°C for 6 h, cooled to room temperature and extracted by adding 50 mL of ethyl acetate and 50 mL of water. The organic layer was washed with saturated brine (30 mL × 3), dried over anhydrous sodium sulphate and concentrated under reduced pressure, and the residue was purified by column chromatography (dichloromethane: methanol (V/V) = 100/1-20/1) to obtain **94A** (2.47 g, yield: 68%).

LCMS m/z = 458.3[M+H]$^+$.

Step 2: 7-(5-methoxy-2-(1-methyl-1H-pyrazol-4-yl)-4-nitrophenyl)-2,7-diazaspiro[3.5]nonane (**94B**)

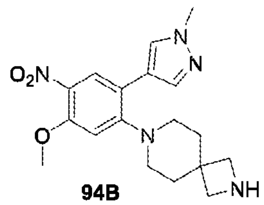

94B

**94A** (2.47 g, 5.40 mmol) was dissolved in methanol (5 mL); a solution of hydrogen chloride in dioxane (4 *N,* 20 mL) was added; and the mixture was reacted at room temperature for 1 h and concentrated under reduced pressure. 20 mL of dioxane and 2 mL of ammonia water were added to the residue, and the mixture was concentrated under reduced pressure to obtain the crude of **94B,** which was directly used in the next step.

Step 3: tert-butyl 4-((7-(5-methoxy-2-(1-methyl-1H-pyrazol-4-yl)-4-nitrophenyl)-2,7- diazaspiro[3.5]nonan-2-yl)methyl)piperidine-1-carboxylate (**94C**)

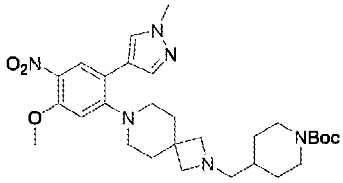

94C

The crude of **94B** from the previous step and tert-butyl 4-formylpiperidine-1-carboxylate (2.30 g, 10.78 mmol) were dissolved in DMAC (30 mL), and acetic acid (323 mg, 5.37 mmol) was added. The mixture was stirred at room temperature for 30 min, and then sodium triacetoxyborohydride (3.43 g, 16.18 mmol) was added; and the resulting mixture was reacted overnight at room temperature. The reaction was quenched by adding 50 mL of saturated aqueous sodium bicarbonate solution and extracted wish ethyl acetate (50 mL × 3). The organic layer was washed with saturated brine (3 × 30 mL), dried over anhydrous sodium sulphate, filtered and concentrated under reduced pressure, and the residue was purified by silica gel column chromatography (mobile phase: DCM/MeOH (V/V) = 100/1-20/1) to obtain **94C** (2.3 g, yield: 77%).

LCMS m/z = 555.4 $[M+H]^+$.

Step 4: 7-(5-methoxy-2-(1-methyl-1H-pyrazol-4-yl)-4-nitrophenyl)-2-(piperidin-4-ylmethyl)-2,7-diazaspiro[3.5]nonane (**94D**); HCl

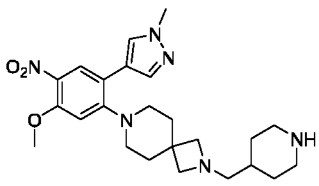

94D

**94C** (2.3 g, 4.15 mmol) was dissolved in methanol (5 mL); a solution of hydrogen chloride in dioxane ( 4 *N,* 25 mL) was added; and the mixture was reacted at room temperature for 1 h and concentrated under reduced pressure, and the residue was directly used in the next step.

Step 5: 2-(2,6-dioxopiperidin-3-yl)-5-fluoro-6-(4-((7-(5-methoxy-2-(1-methyl-1H-pyrazol-4-yl)-4-nitrophenyl)-2,7-diazaspiro[3.5]nonan-2-yl)methyl)piperidin-1-yl)isoindoline-1,3-dione (**94E**)

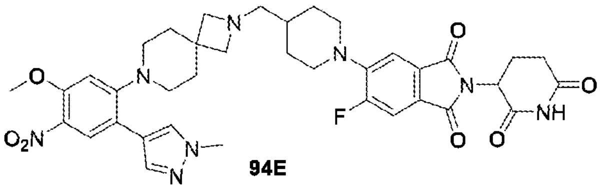

94E

The crude hydrochloride of **94D** from the previous step was dissolved in DMSO (30 mL); 2-(2,6-dioxopiperidin-3-yl)-5,6-difluoroisoindoline-1,3-dione (**5A-1**) (1.46 g, 4.96 mmol) and DIPEA (2.67 g, 20.7 mmol) were successively added; and the mixture was stirred at 100°C for 5 h, and cooled to room temperature. 30 mL of water was added, and the mixture was filtered. The filter cake was dried under reduced pressure and then purified by silica gel column chromatography (mobile phase: dichloromethane/methanol (V/V) = 100/1-20/1) to obtain **94E** (1.8 g, yield: 60%).

LCMS m/z = 729.3 $[M+H]^+$.

Step 6: 5-(4-((7-(4-amino-5-methoxy-2-(1-methyl-1H-pyrazol-4-yl)phenyl)-2,7-diazaspiro[3.5]nonan-2-yl)methyl)piperidin-1-yl)-2-(2,6-dioxopiperidin-3-yl)-6-fluoroisoindoline-1,3-dione (**94F**)

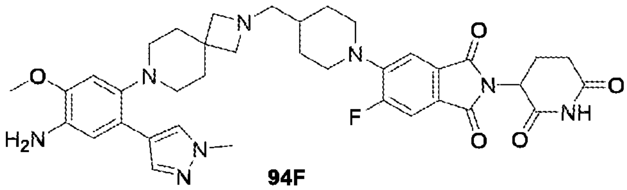

94F

**94E** (700 mg, 0.96 mmol) was dissolved in ethanol/water (20 mL, 3:1); iron powder (280 mg, 5 mmol) and ammonium chloride (335 mg, 6.26 mmol) were successively added; and under nitrogen protection, the mixture was stirred at 85°C for 2 h, cooled to room temperature and subjected to suction filtration under reduced pressure. The filtrate was concentrated, diluted by adding 20 mL of water, extracted with dichloromethane (3 × 30 mL), dried over anhydrous sodium sulphate, filtered and concentrated to obtain **94F** (600 mg, yield: 89%).

LCMS m/z = 699.3 $[M+H]^+$.

Step 7: 5-(4-((7-(4-((5-chloro-4-((3-(dimethylphosphoryl)-[1,1'-biphenyl]-4-yl) amino)pyrimidin-2-yl)amino)-5-methoxy-2-(1-methyl-1H-pyrazol-4-yl)phenyl)-2,7-diazaspiro[3.5]nonan-2-yl)methyl)piperidin-1-yl)-2-(2,6-dioxopiperidin-3-yl)-6-fluoroisoindoline-1,3-dione (**compound 94**)

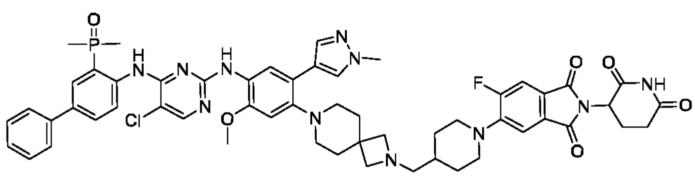

**Compound 94**

**94F** (150 mg, 0.21 mmol) and **40A** (124 mg, 0.32 mmol) were dissolved in DMF (10 mL); p-toluenesulfonic acid monohydrate (120 mg, 0.63 mmol) was added; and the mixture was stirred at 100°C for 16 h, and cooled to room temperature. 20 mL of saturated aqueous sodium bicarbonate solution and 50 mL of dichloromethane were added, and the layers were separated. The organic layer was concentrated under reduced pressure, and the residue was purified

by silica gel column chromatography (mobile phase: dichloromethane/methanol (V/V) = 100/1-10/1). The crude was further subjected to preparative HPLC (instrument: waters 2767 (preparative liquid phase chromatographic instrument); chromatographic column: XBridge@ Prep C18 (30 mm × 150 mm); composition of mobile phases: mobile phase A: acetonitrile, and mobile phase B: water (containing 0.1% TFA)) purification, lyophilization and silica gel preparative plate purification (dichloromethane/methanol (V/V) = 15/1) to obtain **compound 94** (15 mg, yield: 6.8%).

LCMS m/z = 527.8 $[(M+2H)/2]^+$.

$^1H$ NMR (400 MHz, DMSO-$d_6$) δ 11.38 (s, 1H), 11.08 (s, 1H), 8.61 - 8.42 (m, 1H), 8.29 (s, 1H), 8.11 (s, 1H), 8.06 (s, 1H), 7.87 (s, 1H), 7.75 - 7.64 (m, 2H), 7.62 - 7.52 (m, 3H), 7.51 - 7.40 (m, 3H), 7.39 - 7.32 (m, 1H), 7.23 - 7.02 (m, 1H), 6.84 (s, 1H), 5.09 (dd, 1H), 3.79 (s, 3H), 3.76 (s, 3H), 3.66 - 3.52 (m, 2H), 3.20 - 2.95 (m, 4H), 2.94 - 2.82 (m, 3H), 2.81 - 2.68 (m, 4H), 2.65 - 2.52 (m, 2H), 2.48 - 2.34 (m, 2H), 2.08 - 1.93 (m, 1H), 1.92 - 1.69 (m, 12H), 1.61 - 1.43 (m, 1H), 1.41 - 1.25 (m, 2H).

**Example 95: Preparation of compound 95**

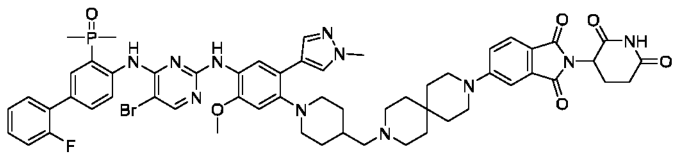

Compound 95

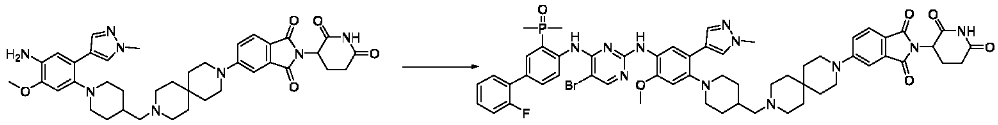

37F

Compound 95

Step 1: 5-(9-((1-(4-((5-bromo-4-((3-(dimethylphosphoryl)-2'-fluoro-[1,1,-biphenyl]-4-yl)amino)pyrimidin-2-yl)amino)-5-methoxy-2-(1-methyl-1H-pyrazol-4-yl)phenyl)piperidin-4-yl)methyl)-3,9-diazaspiro[5.5]undecan-3-yl)-2-(2,6-dioxopiperidin-3-yl)isoindoline-1,3-dione (**compound 95**)

**37F** (1.7 g, 2.40 mmol) and **54C** (1.09 g, 2.40 mmol) were dissolved in DMF (15 mL) solution; p-toluenesulfonic acid monohydrate (0.91 g, 4.8 mmol) was added; and the mixture was reacted overnight at 100°C, and cooled to room temperature. 30 mL of saturated aqueous sodium bicarbonate solution was added, and the mixture was subjected to suction filtration. The filter cake was dissolved in 50 mL of dichloromethane and extracted. The organic phase was dried over anhydrous sodium sulphate and then concentrated under reduced pressure, and the residue was purified by silica gel column chromatography (dichloromethane/methanol (V/V) = 100/1-10/1) to obtain a product, which was further subjected to medium-pressure preparative reversed-phase column purification (acetonitrile/water (containing 0.1% TFA) = 3% - 40%) to obtain the preparative solution of the trifluoroacetate of **compound 95.** 10 mL of saturated aqueous sodium bicarbonate solution and 50 mL of dichloromethane were added, and the mixture was stirred for 5 min. Liquid separation and extraction were performed. The organic layer was dried over anhydrous sodium sulphate and then concentrated under reduced pressure to obtain **compound 95** (600 mg, yield: 22%).

LCMS m/z = 563.8 $[(M+2H)/2]^+$.

$^1H$ NMR (400 MHz, DMSO-$d_6$) δ 11.14 (s, 1H), 11.05 (s, 1H), 8.48 - 8.36 (m, 1H), 8.33 (s, 1H), 8.19 (s, 1H), 7.99 (s, 1H), 7.83 (s, 1H), 7.69 - 7.56 (m, 2H), 7.52 (s, 1H), 7.46 - 7.34 (m, 2H), 7.34 - 7.03 (m, 5H), 6.81 (s, 1H), 5.06 (dd, 1H), 3.77 (s, 3H), 3.75 (s, 3H), 3.54 - 3.40 (m, 4H), 3.11 - 2.97 (m, 2H), 2.96 - 2.79 (m, 1H), 2.69 - 2.51 (m, 4H), 2.43 - 2.27 (m, 4H), 2.21 (d, 2H), 2.08 - 1.96 (m, 1H), 1.80 (d, 6H), 1.73 - 1.63 (m, 2H), 1.61 - 1.41 (m, 9H), 1.31 - 1.17 (m, 2H).

**Example 96: Preparation of compound 96**

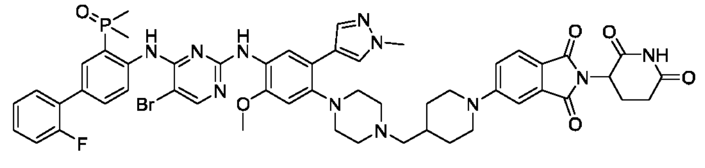

**Compound 96**

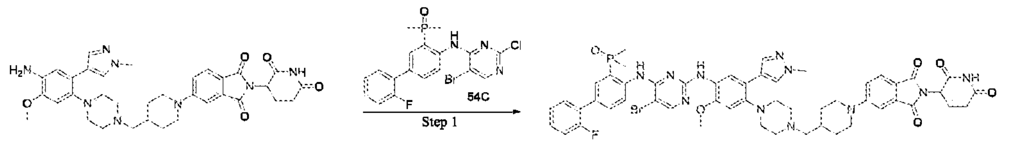


77D

Compound 96

Step 1: 5-(4-((4-(4-((5-bromo-4-((3-(dimethylphosphoryl)-2'-fluoro-[1,1'-biphenyl]-4-yl)amino)pyrimidin-2-yl)amino)-5-methoxy-2-(1-methyl-1H-pyrazol-4-yl)phenyl)piperazin-1-yl)methyl)piperidin-1-yl)-2-(2, 6-dioxopiperidin-3-yl)isoindoline-1,3-dione (**compound 96**)

**77D** (3.5 g, 5.46 mmol) and **54C** (2.98 g, 6.55 mmol) were dissolved in DMF (50 mL); p-toluenesulfonic acid monohydrate (3.1 g, 16.30 mmol) was added; and under nitrogen protection, the mixture was reacted overnight at 100°C, and cooled to room temperature. 100 mL of saturated aqueous sodium bicarbonate solution was added, and the mixture was subjected to suction filtration. The filter cake was dissolved in dichloromethane and extracted. The organic phase was dried over anhydrous sodium sulphate and then concentrated under reduced pressure, and the residue was purified by silica gel column chromatography (dichloromethane/methanol (V/V) = 100/1-10/1) to obtain a product, which was further subjected to reversed-phase preparative column C18 purification ( composition of mobile phases: mobile phase A: water (containing 0.1% TFA), mobile phase B: acetonitrile). The resulting solid was dissolved in 100 mL of dichloromethane and extracted by adding saturated sodium bicarbonate solution (50 mL). Liquid separation was performed. The organic layer was then dried over anhydrous sodium sulphate and concentrated under reduced pressure to obtain **compound 96** (1.5 g, yield: 26%).

LCMS m/z = 529.7 [(M+2H)/2]$^+$.

$^1$H NMR (400 MHz, $D_2O/CF_3COOD$(v/v = 1:1)) δ 8.33 - 8.06 (m, 7H), 7.76 - 7.62 (m, 2H), 7.52 - 7.37 (m, 2H), 7.37 - 7.21 (m, 3H), 7.00 (s, 1H), 5.22 (dd, 1H), 4.01 - 3.90 (m, 8H), 3.90 - 3.78 (m, 2H), 3.77 - 3.63 (m, 2H), 3.44 - 3.15 (m, 8H), 2.96 - 2.89 (m, 2H), 2.85 - 2.70 (m, 1H), 2.64 - 2.50 (m, 1H), 2.46 - 2.32 (m, 2H), 2.32 - 2.22 (m, 1H), 2.21 - 2.05 (m, 2H), 1.99 (d, 6H)

**Example 97: Preparation of compound 97**

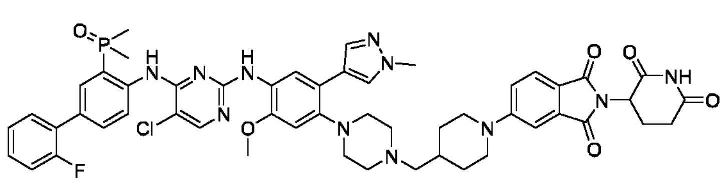

**Compound 97**

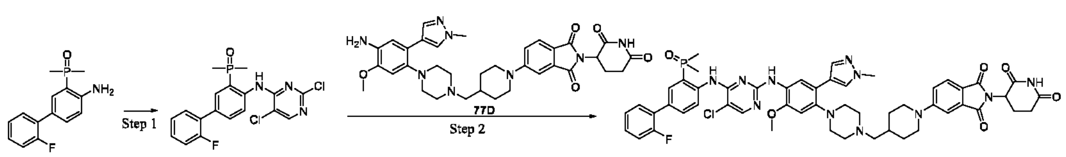


97A

Compound 97

Step 1: (4-((2,5-dichloropyrimidin-4-yl)amino)-2'-fluoro-[1,1'-biphenyl]-3-yl) dimethylphosphine oxide (**97A**)

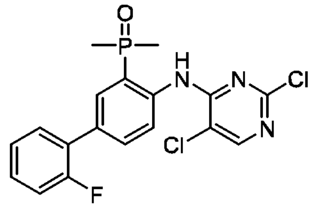

**97A**

54B (4.5 g, 17.09 mmol) and 2,4,5-trichloropyrimidine (6.27 g, 34.18 mmol) were dissolved in 20 mL of NMP; DIPEA (2.65 g, 20.51 mmol) was added; and the mixture was stirred at 120°C for 2 h and cooled to room temperature. 50 mL of water was added, and the mixture was filtered. The filter cake was dried under reduced pressure. The residue was slurried by adding 10 mL of ethanol and 30 mL of methyl tert-butyl ether for 1 h and filtered. The filter cake was washed with 10 mL of methyl tert-butyl ether and dried under reduced pressure to obtain **97A** (4.5 g, yield: 64%).

$^1$H NMR (400 MHz, $CDCl_3$) δ 11.69 (s, 1H), 8.82-8.76 (m, 1H), 8.24 (s, 1H), 7.76 (d, 1H), 7.52-7.40 (m, 2H), 7.40-7.32 (m, 1H), 7.28 - 7.14 (m, 2H), 1.88 (d, 6H).

Step 2: 5-(4-((4-(4-((5-chloro-4-((3-(dimethylphosphoryl)-2'-fluoro-[1,1'-biphenyl]-4-yl)amino)pyrimidin-2-yl)amino)-5-methoxy-2-(1-methyl-1H-pyrazol-4-yl)phenyl)piperazin-1-yl)methyl)piperidin-1-yl)-2-(2,6-dioxopiperidin-3-yl)isoindoline-1,3-dione (**compound 97**)

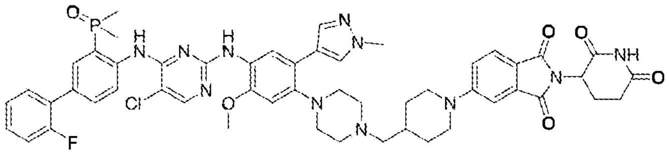

**Compound 97**

**77D** (3.5 g, 5.46 mmol) and **97A** (2.69 g, 6.56 mmol) were dissolved in DMF (50 mL); p-toluenesulfonic acid monohydrate (3.1 g, 16.30 mmol) was added; and under nitrogen protection, the mixture was reacted overnight at 100°C, and cooled to room temperature. 100 mL of saturated aqueous sodium bicarbonate solution was added, and the mixture was subjected to suction filtration. The filter cake was dissolved in dichloromethane and extracted. The organic phase was dried over anhydrous sodium sulphate and then concentrated under reduced pressure, and the residue was purified by silica gel column chromatography (dichloromethane/methanol (V/V) = 100/1-10/1) to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)) to obtain a preparative solution, which was concentrated. 100 mL of dichloromethane was added, and the mixture was adjusted to a basic pH by adding saturated sodium bicarbonate solution. Liquid separation was performed. The organic layer was then dried over anhydrous sodium sulphate and concentrated under reduced pressure to obtain **compound 97** (1.2 g, yield: 22%).

LCMS m/z = 507.8 $[(M+2H)/2]^+$.

$^1$H NMR (400 MHz, $D_2O/CF_3COOD$(v/v = 1:1)) δ 8.37 - 8.23 (m, 3H), 8.23 - 8.07 (m, 4H), 7.78 - 7.62 (m, 2H), 7.52 - 7.37 (m, 2H), 7.37 - 7.20 (m, 3H), 7.01 (s, 1H), 5.22 (dd, 1H), 4.00 - 3.90 (m, 8H), 3.90 - 3.79 (m, 2H), 3.77 - 3.67 (m, 2H), 3.45 - 3.20 (m, 8H), 2.96 - 2.90 (m, 2H), 2.85 - 2.71 (m, 1H), 2.65 - 2.51 (m, 1H), 2.48 - 2.33 (m, 2H), 2.32 - 2.22 (m, 1H), 2.21 - 2.06 (m, 2H), 2.00 (d, 6H).

**Example 98: Preparation of compound 98**

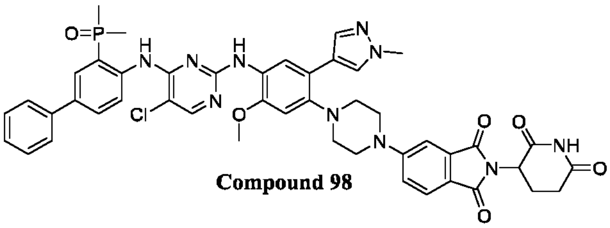

Compound 98

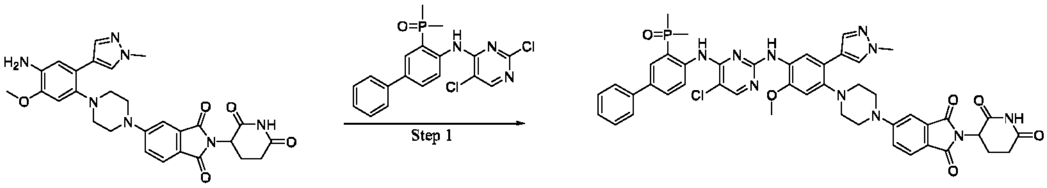



80D

Compound 98

Step 1: 5-(4-(4-((5-chloro-4-((3-(dimethylphosphoryl)-[I, I'-biphenyl]-4-yl)amino)pyrimidin-2-yl)amino)-5-methoxy-2-(1-methyl-1H-pyrazol-4-yl)phenyl)piperazin-1-yl)-2-(2, 6-dioxopiperidin-3-yl)isoindoline-1,3-dione (**compound 98**)

**80D** (130 mg, 0.24 mmol) and **40A** (122 mg, 0.31 mmol) were dissolved in DMF (10 mL); p-toluenesulfonic acid monohydrate (137 mg, 0.72 mmol) was added; and under nitrogen protection, the mixture was stirred at 100°C for 16 h, and cooled to room temperature. 10 mL of saturated aqueous sodium bicarbonate solution and 20 mL of dichloromethane were added, and the layers were separated. The organic layer was concentrated under reduced pressure, and the residue was purified by silica gel column chromatography to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 mL). Liquid separation was performed. The organic layer was then dried over anhydrous sodium sulphate and concentrated under reduced pressure to obtain **compound 98** (55 mg, yield: 25%).

LCMS m/z = 899.3 $[M+H]^+$.

$^1H$ NMR (400 MHz, DMSO-$d_6$) δ 11.35 (s, 1H), 11.07 (s, 1H), 8.64 - 8.36 (m, 1H), 8.28 (s, 1H), 8.17 (s, 1H), 8.13 (s, 1H), 7.89 (s, 1H), 7.76 - 7.64 (m, 3H), 7.61 - 7.51 (m, 2H), 7.50 - 7.24 (m, 5H), 7.23 - 7.04 (m, 1H), 6.88 (s, 1H), 5.09 (dd, 1H), 3.81 (s, 3H), 3.77 (s, 3H), 3.69 - 3.47 (m, 4H), 3.06 - 2.83 (m, 5H), 2.70 - 2.52 (m, 2H), 2.10 - 1.97 (m, 1H), 1.84 (d, 6H).

**Example 99: Preparation of compound 99**

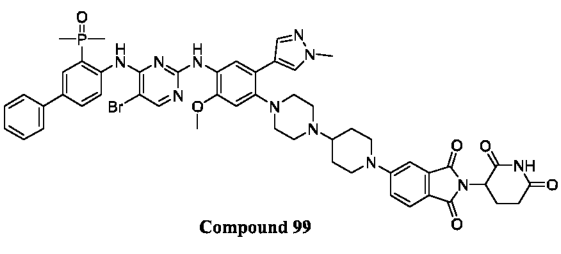

Compound 99

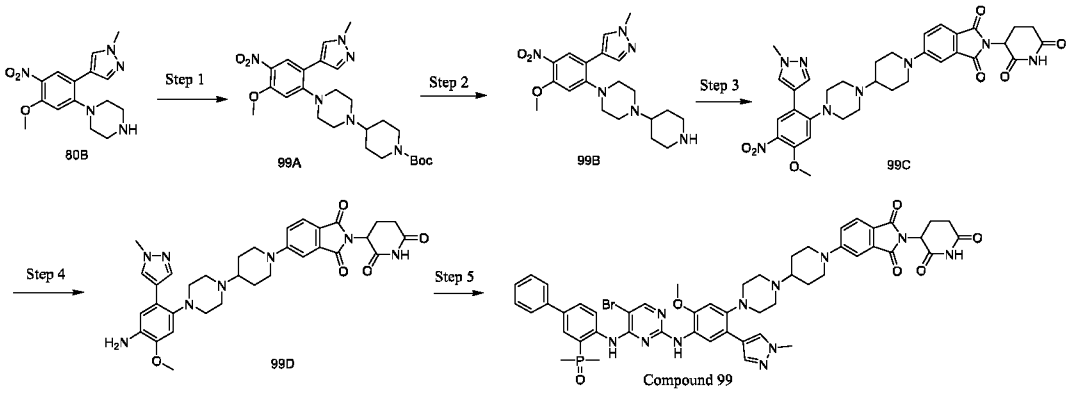

Step 1: tert-butyl 4-(4-(5-methoxy-2-(1-methyl-1H-pyrazol-4-yl)-4-nitrophenyl)piperazin-1-yl) piperidine-1-carboxylate (**99A**)

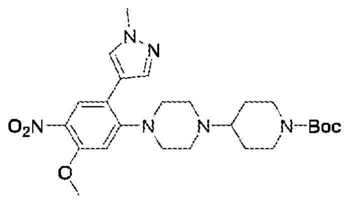

The hydrochloride of **80B** (1.05 g, crude) was added to 30 mL of THF; sodium bicarbonate (806 mg, 9.60 mmol) was added; and the mixture was stirred at room temperature for 20 min. (955 mg, 4.80 mmol) and 0.3 mL of acetic acid were added, and then the mixture was stirred at room temperature for 2 h. Sodium triacetoxyborohydride (1.02 g, 4.80 mmol) was added, and the mixture was reacted at room temperature for 16 h, adjusted to a basic pH by adding saturated aqueous sodium bicarbonate solution and extracted 3 times with ethyl acetate. The organic phases were combined, washed sequentially with water and saturated brine, dried over anhydrous sodium sulphate and concentrated under reduced pressure, and the residue was purified by silica gel column chromatography (mobile phase: DCM/MeOH (V/V) = 50/1-20/1) to obtain **99A** (1.0 g, yield: 84.6%).

LCMS m/z = 501.3 [M +1]$^+$.

Step 2: 1-(5-methoxy-2-(1-methyl-1H-pyrazol-4-yl)-4-nitrophenyl)-4-(piperidin-4-yl)piperazine (**99B**); 2,2,2-trifluoroacetate

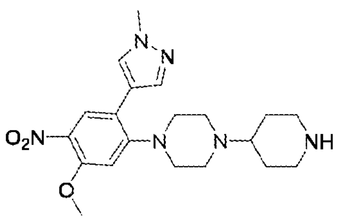

**99A** (1.0 g, 2.0 mmol) was dissolved in 25 mL of DCM; 10 mL of trifluoroacetic acid was added; and the mixture was stirred at room temperature for 2 h. The reaction solution was concentrated under reduced pressure to obtain the trifluoroacetate of **99B** (1.2 g, crude).

LC-MS m/z = 401.3[M+1]$^+$.

Step 3: 2-(2,6-dioxopiperidin-3-yl)-5-(4-(4-(5-methoxy-2-(1-methyl-1H-pyrazol-4-yl)-4-nitrophenyl)piperazin-1-yl)piperidin-1-yl)isoindoline-1,3-dione (**99C**)

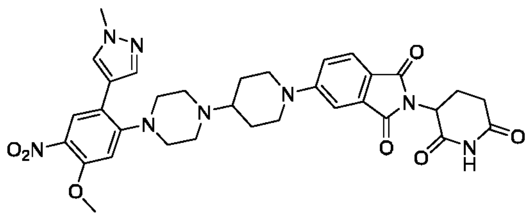

The trifluoroacetate of **99B** (1.2 g, crude) was dissolved in 20 mL of DMSO; solid sodium bicarbonate (840 mg, 10.0 mmol) was added; and the mixture was stirred at room temperature for 10 min. 3 mL of DIPEA and 2-(2,6-dioxopiperidin-3-yl)-5-fluoroisoindoline-1,3-dione (662 mg, 2.40 mmol) were added, and the reaction was stirred at 80°C for 5 h. The reaction solution was cooled to room temperature. 200 mL of water was added; and the mixture was filtered to collect the solid, which was washed with water, dissolved in DCM, dried over anhydrous sodium sulphate and concentrated under reduced pressure. The residue was subjected to silica gel column chromatography (mobile phase: DCM/MeOH (V/V) = 15/1) purification to obtain **99C** (0.72 g, yield: 55.3%).

LCMS m/z = 657.3 $[M+1]^+$.

Step 4: 5-(4-(4-(4-amino-5-methoxy-2-(1-methyl-1H-pyrazol-4-yl)phenyl) piperazin-1-yl)piperidin-1-yl)-2-(2,6-dioxopiperidin-3-yl)isoindoline-1,3-dione (**99D**)

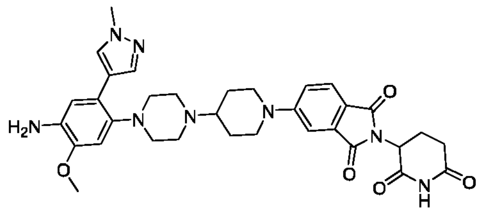

**99C** (0.72 g, 1.10 mmol) was dissolved in a mixed solution of THF (10 mL), ethanol (30 mL) and water (6 mL); reduced iron powder (614 mg, 10.96 mmol) and ammonium chloride (587 mg, 10.97 mmol) were added; and the mixture was reacted at 85°C for 2 h. The reaction solution was cooled to room temperature and concentrated in vacuo to remove a solvent. 50 mL of water and 50 mL of dichloromethane were added to the residue, and then the mixture was extracted 3 times with dichloromethane. The organic phases were combined, dried over anhydrous sodium sulphate and then concentrated under reduced pressure to obtain **99D** (0.52 g, yield: 75.4%)

LCMS m/z = 627.3 $[M+H]^+$.

Step 5: 5-(4-(4-(4-((5-bromo-4-((3-(dimethylphosphoryl)-[1,1'-biphenyl]-4-yl) amino)pyrimidin-2-yl)amino)-5-methoxy-2-(1-methyl-1H-pyrazol-4-yl)phenyl)piperazin-1-yl)piperidin-1-yl)-2-(2,6-dioxopiperidin-3-yl)isoindoline-1,3-dione (**compound 99**)

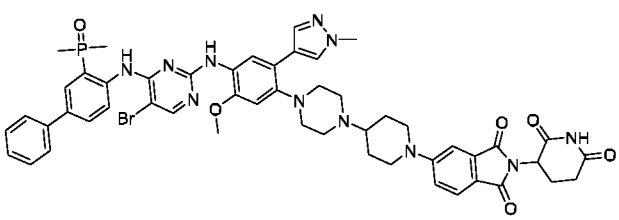

**99D** (250 mg, 0.40 mmol) and **23D** (260 mg, 0.60 mmol) were dissolved in DMF (20 mL); p-toluenesulfonic

acid monohydrate (230 mg, 1.21 mmol) was added; and the mixture was stirred at 100°C for 8 h, and cooled to room temperature. 100 mL of saturated aqueous sodium bicarbonate solution was added, with a yellow solid precipitated. Suction filtration was performed for collecting the solid, and the filter cake was washed with water (100 mL × 3) and redissolved in dichloromethane. The organic layers were separated, dried over anhydrous sodium sulphate and concentrated, and the crude was further subjected to preparative HPLC (instrument: waters 2767 (preparative liquid phase chromatographic instrument); chromatographic column: XBridge@ Prep C18 (30 mm × 150 mm); composition of mobile phases: mobile phase A: acetonitrile, and mobile phase B: water (containing 0.1% TFA)) purification to obtain a preparative solution, which was concentrated and adjusted to a basic pH by adding dichloromethane and saturated sodium bicarbonate solution. The organic phase was separated. The aqueous phase was extracted with dichloromethane (100 ml × 3). The organic phase was dried over anhydrous sodium sulphate and concentrated, and the crude was subjected to prep-TLC (DCM: MeOH (V/V) = 15:1) purification to obtain **compound 99** (120 mg, yield: 28%).

LCMS m/z = 1026.3 $[M+1]^+$.

$^1H$ NMR (400 MHz, DMSO-$d_6$) δ 11.11 (s, 1H), 11.06 (s, 1H), 8.45 - 8.33 (m, 1H), 8.28 (s, 1H), 8.18 (s, 1H), 8.01 (s, 1H), 7.88 (s, 1H), 7.76 - 7.64 (m, 2H), 7.61 - 7.50 (m, 3H), 7.43 (t, 2H), 7.40 - 7.30 (m, 2H), 7.27 (dd, 1H), 7.22 - 7.08 (m, 1H), 6.85 (s, 1H), 5.07 (dd, 1H), 4.15 - 3.96 (m, 2H), 3.79 (s, 3H), 3.75 (s, 3H), 3.11 - 2.98 (m, 2H), 2.97 - 2.77 (m, 5H), 2.71 - 2.51 (m, 7H), 2.10 - 1.87 (m, 3H), 1.83 (d, 6H), 1.60 - 1.41 (m, 2H).

**Example 100: Preparation of compound 100**

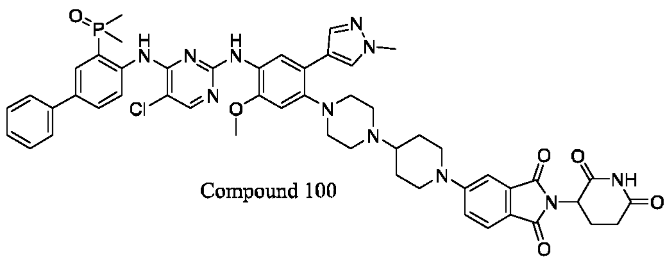

Compound 100

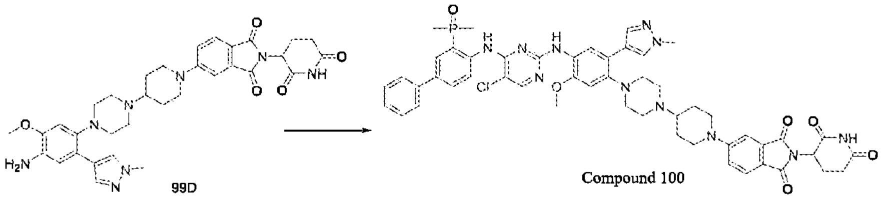

99D

Compound 100

Step 1: 5-(4-(4-(4-((5-chloro-4-((3-(dimethylphosphoryl)-[1,1'-biphenyl]-4-yl) amino)pyrimidin-2-yl)amino)-5-methoxy-2-(1-methyl-1H-pyrazol-4-yl)phenyl)piperazin-1-yl)piperidin-1-yl)-2-(2,6-dioxopiperidin-3-yl)isoindoline-1,3-dione (**compound 100**)

**99D** (250 mg, 0.40 mmol) and **40A** (188 mg, 0.48 mmol) were dissolved in DMF (20 mL); p-toluenesulfonic acid monohydrate (263 mg, 1.38 mmol) was added; and the mixture was stirred at 100°C for 8 h, and cooled to room temperature. 100 mL of saturated aqueous sodium bicarbonate solution was added, with a white solid precipitated. Suction filtration was performed for collecting the solid, and the filter cake was washed with water (100 ml × 3) and redissolved in dichloromethane. The organic layers were separated, dried over anhydrous sodium sulphate and concentrated, and the crude was further subjected to preparative HPLC (instrument: waters 2767 (preparative liquid phase chromatographic instrument); chromatographic column: XBridge@ Prep C18 (30 mm × 150 mm); composition of mobile phases: mobile phase A: acetonitrile, and mobile phase B: water (containing 0.1% TFA)) purification to obtain a preparative solution, which was concentrated and adjusted to a basic pH by adding dichloromethane and saturated sodium bicarbonate solution. The organic phase was separated. The aqueous phase was extracted with dichloromethane (100 ml × 3). The organic phase was dried over anhydrous sodium sulphate and concentrated, and the crude was subjected to prep-TLC (DCM: MeOH (V/V) = 15:1) purification to obtain **compound 100** (100 mg, yield: 25%).

LCMS m/z = 982.3 $[M+1]^+$.

$^1H$ NMR (400 MHz, DMSO-$d_6$) δ 11.36 (s, 1H), 11.06 (s, 1H), 8.65 - 8.35 (m, 1H), 8.28 (s, 1H), 8.11 (s, 1H), 8.03 (s, 1H), 7.89 (s, 1H), 7.78 - 7.65 (m, 2H), 7.64 - 7.48 (m, 3H), 7.44 (t, 2H), 7.40 - 7.29 (m, 2H), 7.27 (dd, 1H), 7.22

- 7.09 (m, 1H), 6.85 (s, 1H), 5.07 (dd, 1H), 4.19 - 3.96 (m, 2H), 3.79 (s, 3H), 3.75 (s, 3H), 3.11 - 2.96 (m, 2H), 2.95 - 2.73 (m, 5H), 2.73 - 2.51 (m, 7H), 2.10 - 1.88 (m, 3H), 1.84 (d, 6H), 1.61 - 1.41 (m, 2H).

**Example 101: Preparation of compound 101**

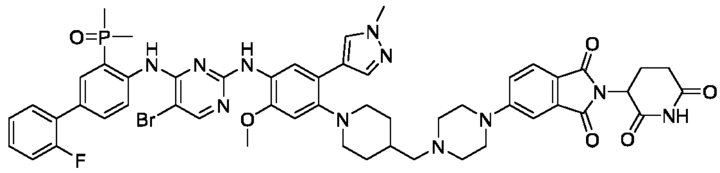

Compound 101

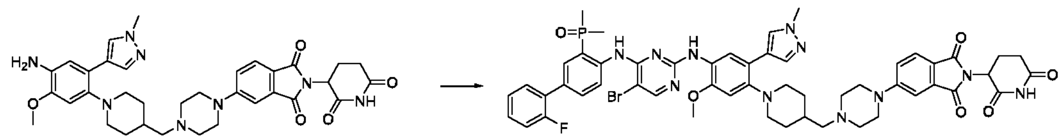

45F

Compound 101

5-(4-((1-(4-((5-bromo-4-((3-(dimethylphosphoryl)-2'-fluoro-[1,1'-biphenyl]-4-yl)amino)pyrimidin-2-yl)amino)-5-methoxy-2-(1-methyl-1H-pyrazol-4-yl)phenyl)piperidin-4-yl)methyl)piperazin-1-yl)-2-(2,6-dioxopiperidin-3-yl)isoindoline-1,3-dione (**compound 101**)

**45F** (2.5 g, 3.90 mmol) and **54C** (1.77 g, 3.90 mmol) were dissolved in DMF (20 mL) solution; p-toluenesulfonic acid monohydrate (1.48 g, 7.8 mmol) was added, and the mixture was reacted overnight at 100°C, and cooled to room temperature. 30 mL of saturated aqueous sodium bicarbonate solution was added, and the mixture was subjected to suction filtration. The filter cake was dissolved in 50 mL of dichloromethane and extracted. The organic phase was dried over anhydrous sodium sulphate and then concentrated under reduced pressure, and the residue was purified by silica gel column chromatography (dichloromethane/methanol (V/V) = 100/1-10/1) to obtain a product, which was further subjected to medium-pressure preparative reversed-phase column purification (acetonitrile/water (containing 0.1% TFA) = 3% - 40%) to obtain the preparative solution of the trifluoroacetate of **compound 101.** 10 mL of saturated aqueous sodium bicarbonate solution and 50 mL of dichloromethane were added, and the mixture was stirred for 5 min. Liquid separation and extraction were performed. The organic layer was dried over anhydrous sodium sulphate and then concentrated under reduced pressure to obtain **compound 101** (1.5 g, yield: 36%).

LCMS m/z = 529.7 [(M+2H)/2]$^+$.

$^1$H NMR (400 MHz, $D_2O/CF_3COOD$(v/v = 1:1)) δ 8.28 (s, 1H), 8.19 - 8.07 (m, 1H), 7.93 (s, 1H), 7.87 - 7.78 (m, 3H), 7.76 - 7.65 (m, 1H), 7.53 - 7.41 (m, 3H), 7.40 - 7.21 (m, 5H), 5.12 (dd, 1H), 4.21 - 4.07 (m, 2H), 4.03 (s, 3H), 3.89 - 3.66 (m, 9H), 3.59 - 3.47 (m, 2H), 3.37 - 3.15 (m, 4H), 2.94 - 2.83 (m, 2H), 2.82 - 2.67 (m, 1H), 2.56 - 2.40 (m, 1H), 2.31 - 2.13 (m, 3H), 2.07 - 1.87 (m, 8H).

**Example 102: Preparation of compound 102**

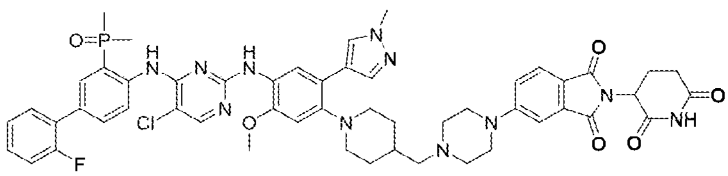

Compound 102

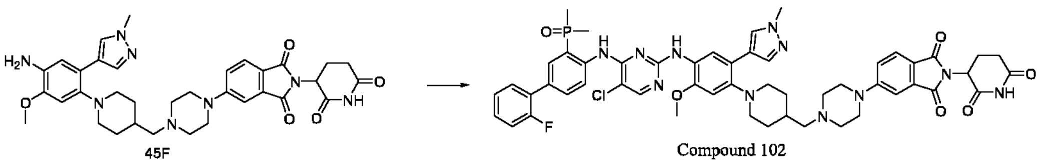

Step 1: 5-(4-((1-(4-((5-chloro-4-((3-(dimethylphosphoryl)-2'-fluoro-[1,1'-biphenyl]-4-yl)amino)pyrimidin-2-yl)amino)-5-methoxy-2-(1-methyl-1H-pyrazol-4-yl)phenyl)piperidin-4-yl)methyl)piperazin-1-yl)-2-(2,6-dioxopiperidin-3-yl)isoindoline-1,3-dione (**compound 102**)

**45F** (2.5 g, 3.90 mmol) and **97A** (1.6 g, 3.90 mmol) were dissolved in DMF (20 mL) solution; p-toluenesulfonic acid monohydrate (1.48 g, 7.8 mmol) was added, and the mixture was reacted overnight at 100°C, and cooled to room temperature. 30 mL of saturated aqueous sodium bicarbonate solution was added, and the mixture was subjected to suction filtration. The filter cake was dissolved in 50 mL of dichloromethane and extracted. The organic phase was dried over anhydrous sodium sulphate and then concentrated under reduced pressure, and the residue was purified by silica gel column chromatography (dichloromethane/methanol (V/V) = 100/1-10/1) to obtain a product, which was further subjected to medium-pressure preparative reversed-phase column purification (acetonitrile/water (containing 0.1% TFA) = 3% - 40%) to obtain the preparative solution of the trifluoroacetate of **compound 102.** 10 mL of saturated aqueous sodium bicarbonate solution and 50 mL of dichloromethane were added, and the mixture was stirred for 5 min. Liquid separation and extraction were performed. The organic layer was dried over anhydrous sodium sulphate and then concentrated under reduced pressure to obtain **compound 102** (1.5 g, yield: 38%).

LCMS m/z = 507.7 $[(M+2H)/2]^+$.

$^1$H NMR (400 MHz, $D_2O/CF_3COOD$(v/v = 1:1))δ 8.28 - 8.21 (m, 1H), 8.19 (s, 1H), 7.98 (s, 1H), 7.91 - 7.77 (m, 3H), 7.74 - 7.64 (m, 1H), 7.54 - 7.40 (m, 3H), 7.40 - 7.21 (m, 5H), 5.12 (dd, 1H), 4.24 - 4.07 (m, 2H), 4.04 (s, 3H), 3.89 - 3.69 (m, 9H), 3.61 - 3.48 (m, 2H), 3.37 - 3.12 (m, 4H), 2.95 - 2.84 (m, 2H), 2.83 - 2.67 (m, 1H), 2.58 - 2.41 (m, 1H), 2.28 - 2.11 (m, 3H), 2.06 - 1.89 (m, 8H).

**Example 103: Preparation of compound 103**

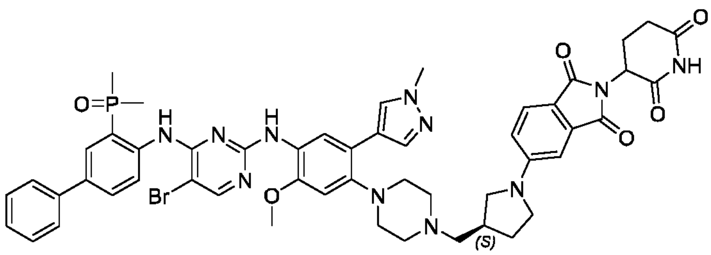

**Compound 103**

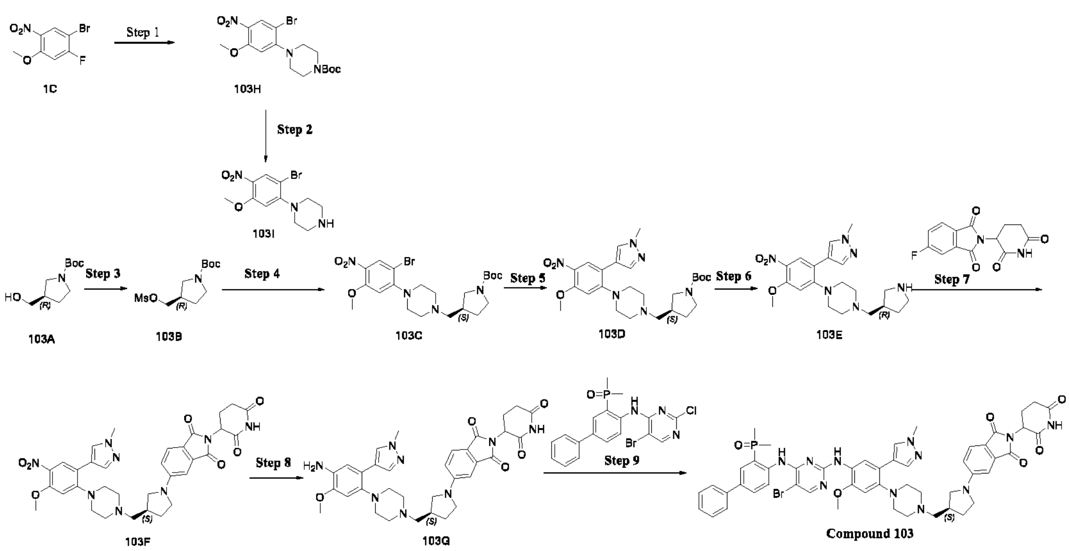

1C 103H 103I 103A 103B 103C 103D 103E 103F 103G Compound 103

Step 1: tert-butyl 4-(2-bromo-5-methoxy-4-nitrophenyl)piperazine-1-carboxylate (**103H**)

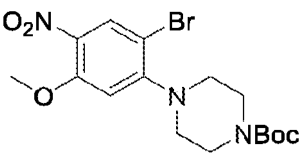

**103H**

**1C** (30 g, 120.48 mmol) and 1-tert-butoxycarbonylpiperazine (24.65 g, 132.52 mmol) were dissolved in DMSO (300 mL); potassium carbonate (50 g, 361.44 mmol) was added; and the mixture was reacted at 120°C for 6 h. The reaction solution was poured into water under stirring, with a solid precipitated, and the mixture was subjected to suction filtration and washed 3 times with water. The filter cake was dried in vacuo to obtain **103H** (50 g, yield: 99%).

LCMS m/z = 360.0[M-55]$^+$.

Step 2: 1-(2-bromo-5-methoxy-4-nitrophenyl)piperazine (**103I**)

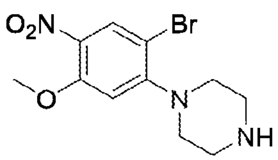

**103I**

**103H** (33 g, 80 mmol) was dissolved in dichloromethane (100 mL); trifluoroacetic acid (50 mL) was slowly added; and the mixture was reacted at room temperature for 3 h, concentrated under reduced pressure to remove dichloromethane and trifluoroacetic acid, redissolved by adding 100 mL of dichloromethane and adjusted to a basic pH with 2 N aqueous sodium hydroxide solution. Liquid separation was performed. The organic phases were collected, and the aqueous phase was extracted 3 times with dichloromethane. All organic phases were combined, dried over anhydrous sodium sulphate and concentrated under reduced pressure to obtain **103I** (24.5 g, yield: 98%), which was directly used in the next step.

LCMS m/z = 316.0 [M+H]$^+$.

Step 3: tert-butyl (R)-3-(((methylsulfonyl)oxy)methyl)pyrrolidine-1-carboxylate (103B)

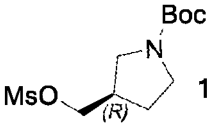

103B

**103A** (tert-butyl(R)-3-(hydroxymethyl)pyrrolidine-1-carboxylate) (5.00 g, 24.84 mmol) was dissolved in DCM (50 mL); triethylamine (7.54 g, 74.52 mmol) was added; and under an ice bath, methanesulfonyl chloride (5.69 g, 49.68 mmol) was added dropwise. After the addition, the mixture was reacted at 20°C for 2 h, and the reaction was quenched by adding water. The reaction solution was diluted with 200 mL of dichloromethane, washed 3 times with 0.1 N dilute hydrochloric acid, and washed once with aqueous sodium bicarbonate solution and saturated sodium chloride. The organic phases were collected, dried over anhydrous sodium sulphate and concentrated under reduced pressure to obtain **103B** (6.90 g, which was directly used in the next step).

LCMS m/z = 224.1[M-55]$^+$.

Step 4: tert-butyl (S)-3-((4-(2-bromo-5-methoxy-4-nitrophenyl)piperazin-1-yl)methyl) pyrrolidine-1-carboxylate (**103C**)

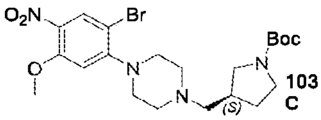

103 C

**103I** (5.20 g, 16.45 mmol) and **103B** (5.51 g, 19.74 mmol) were dissolved in DMF (50 mL); sodium iodide (0.99 g, 6.58 mmol) and potassium carbonate (6.82 g, 49.35 mmol) were added; and the mixture was reacted at 100°C for 16 h. The reaction solution was cooled to room temperature, diluted by adding 200 mL of ethyl acetate, washed 3 times with water and washed once with saturated sodium chloride. The organic phase was dried over anhydrous sodium sulphate and concentrated under reduced pressure, and the residue was purified by column chromatography (mobile phase: petroleum ether/ethyl acetate (V/V) = 2/1-pure ethyl acetate) to obtain **103C** (3.30 g, yield: 40%).

LCMS m/z = 499.1[M+H]$^+$.

Step 5: tert-butyl (S)-3-((4-(5-methoxy-2-(1-methyl-1H-pyrazol-4-yl)-4-nitrophenyl)piperazin-1-yl) methyl)pyrrolidine-1-carboxylate (**103D**)

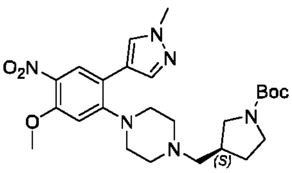

103D

**103C** (3.30 g, 6.61 mmol) and 1-methyl-1H-pyrazole-4-boronic acid (1.33 g, 10.58 mmol) were dissolved in 1,4-dioxane (50 mL); Pd(dppf)$Cl_2$·DCM (0.54 g, 0.66 mmol) and an aqueous solution (10 mL) of potassium carbonate (1.83 g, 13.22 mmol) were added; and the mixture was subjected to nitrogen replacement 3 times and reacted at 90°C for 5 h. The reaction solution was cooled to room temperature, diluted by adding 300 mL of ethyl acetate, washed 3 times with water and washed once with saturated aqueous sodium chloride solution. The organic phase was dried over anhydrous sodium sulphate and concentrated under reduced pressure, and the residue was purified by column chromatography (mobile phase: petroleum ether/ethyl acetate (V/V) = 3/1-pure ethyl acetate) to obtain **103D** (2.89 g, yield: 87%).

LCMS m/z = 501.3 [M+H]$^+$.

Step 6: (R)-1-(5-methoxy-2-(1-methyl-1H-pyrazol-4-yl)-4-nitrophenyl)-4-(pyrrolidin-3-ylmethyl) piperazine (**103E**)

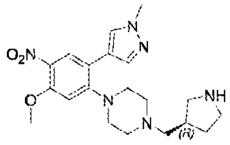

103E

**103D** (2.89 g, 5.77 mmol) was dissolved in dichloromethane (30 mL); trifluoroacetic acid (10 mL) was slowly added; and the mixture was reacted at room temperature for 1 h, concentrated under reduced pressure to remove dichloromethane and trifluoroacetic acid, redissolved by adding 100 mL of dichloromethane and adjusted to a basic pH with 1 N aqueous sodium hydroxide solution. Liquid separation was performed. The organic phases were collected, and the aqueous phase was extracted 3 times with dichloromethane. All organic phases were combined, dried over anhydrous sodium sulphate and concentrated under reduced pressure to obtain **103E** (2.26 g, yield: 98%), which was directly used in the next step.

LCMS m/z = 401.3$[M+H]^+$.

Step 7: 2-(2,6-dioxopiperidin-3-yl)-5-((S)-3-((4-(5-methoxy-2-(1-methyl-1H-pyrazol-4-yl)-4-nitrophenyl)piperazin-1-yl)methyl)pyrrolidin-1-yl)isoindoline-1,3-dione (103F)

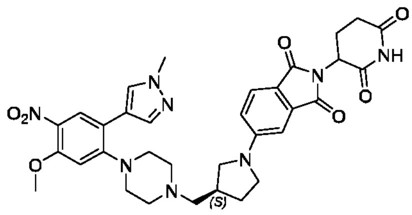

103F

**103E** (2.26 g, 5.64 mmol) and 2-(2,6-dioxopiperidin-3-yl)-5-fluoroisoindole-1,3-dione (1.87 g, 6.77 mmol) were dissolved in DMSO (30 mL); DIPEA (2.19 g, 16.92 mmol) was added dropwise; and the mixture was reacted at 90°C for 16 h. The reaction solution was cooled to room temperature. 20 mL of water was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase: dichloromethane/methanol (V/V) = 50/1-12/1) to obtain **103F** (3.58 g, yield: 97%).

LCMS m/z = 657.2$[M+H]^+$.

Step 8: 5-((S)-3-((4-(4-amino-5-methoxy-2-(1-methyl-1H-pyrazol-4-yl)phenyl) piperazin-1-yl)methyl)pyrrolidin-1-yl)-2-(2,6-dioxopiperidin-3-yl)isoindoline-1,3-dione (**103G**)

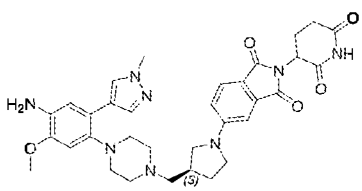

103G

**103F** (3.58 g, 5.45 mmol) was dissolved in ethanol (60 mL); reduced iron powder (1.83 g, 32.70 mmol) was

added, and then an aqueous solution (20 mL) of ammonium chloride (1.75 g, 32.70 mmol) was added dropwise; and under nitrogen protection, the mixture was reacted at 90°C for 3 h. The reaction solution was cooled to room temperature. 20 mL of water was added, and then the mixture was extracted 3 times with dichloromethane. The organic phases were combined, dried over anhydrous sodium sulphate and then concentrated under reduced pressure to obtain **103G** (3.40 g, yield: 99%), which was directly used in the next step.

LCMS m/z = 627.3 $[M+H]^+$.

Step 9: 5-((S)-3-((4-(4-((5-bromo-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperazin-1-yl)methyl)pyrrolidin-1-yl)-2-(2,6-dioxopiperidin-3-yl)isoindoline-1,3-dione (**compound 103**)

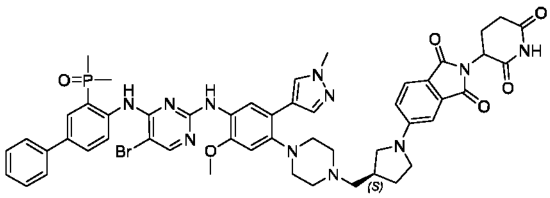

Compound 103

**103G** (1.70 g, 2.71 mmol) and **23D** (1.18 g, 2.71 mmol) were dissolved in DMF (40 mL); p-toluenesulfonic acid hydrate (1.55 g, 8.13 mmol) was added; and under nitrogen protection, the mixture was reacted at 100°C for 16 h. The reaction solution was cooled to room temperature. 50 mL of saturated aqueous sodium bicarbonate solution was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase: dichloromethane/methanol (V/V) = 50/1-15/1) to obtain a product, which was further purified by preparative HPLC (composition of mobile phases: mobile phase A: acetonitrile, and mobile phase B: water (containing 0.1% TFA)) to obtain a product, which was alkalized with aqueous sodium bicarbonate solution, extracted with dichloromethane, concentrated and dried to obtain **compound 103** (580 mg, yield: 21%).

LCMS m/z = 513.7$[(M+2H)/2]^+$.

$^1$H NMR (400 MHz, DMSO-$d_6$) δ 11.12 (s, 1H), 11.04 (s, 1H), 8.46 - 8.33 (m, 1H), 8.28 (s, 1H), 8.19 (s, 1H), 8.03 (s, 1H), 7.88 (s, 1H), 7.74 - 7.50 (m, 5H), 7.45 (t, 2H), 7.33 (t, 1H), 7.26 - 7.07 (m, 1H), 6.97 - 6.85 (m, 2H), 6.82 (dd, 1H), 5.05 (dd, 1H), 3.81 (s, 3H), 3.76 (s, 3H), 3.62 - 3.48 (m, 2H), 3.48 - 3.36 (m, 1H), 3.23 - 3.11 (m, 1H), 3.00 - 2.79 (m, 5H), 2.74 - 2.51 (m, 7H), 2.47 - 2.31 (m, 2H), 2.24 - 2.09 (m, 1H), 2.08 - 1.94 (m, 1H), 1.91 - 1.68 (m, 7H).

**Example 104: Preparation of compound 104**

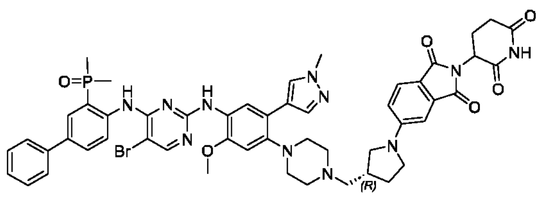

Compound 104

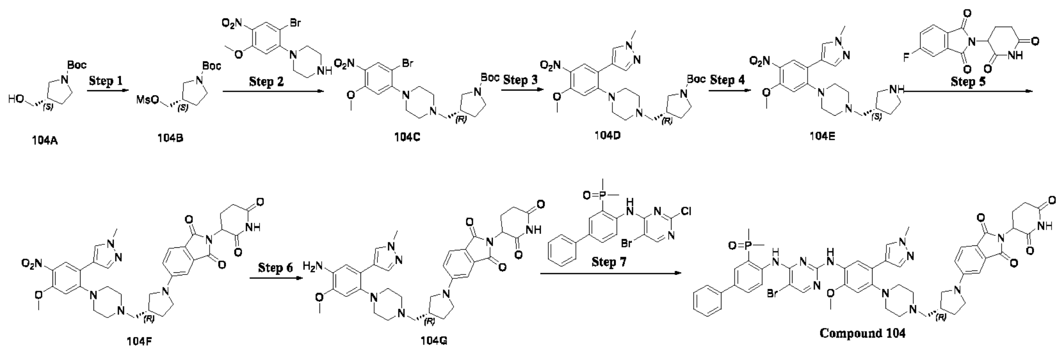


Step 1: tert-butyl (S)-3-(((methylsulfonyl)oxy)methyl)pyrrolidine-1-carboxylate (**104B**)

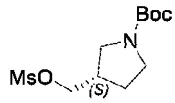

**104B**

**104A** (5.00 g, 24.84 mmol) was dissolved in DCM (50 mL); triethylamine (7.54 g, 74.52 mmol) was added; and under an ice bath, methanesulfonyl chloride (5.69 g, 49.68 mmol) was added dropwise. The mixture was reacted at 20°C for 2 h, and the reaction was quenched by adding water. The reaction solution was diluted with 200 mL of dichloromethane, washed 3 times with 0.1 N dilute hydrochloric acid, and washed once with aqueous sodium bicarbonate solution and saturated aqueous sodium chloride solution. The organic phases were collected, dried over anhydrous sodium sulphate and concentrated under reduced pressure to obtain **104B** (7 g, which was directly used in the next step).

Step 2: tert-butyl (R)-3-((4-(2-bromo-5-methoxy-4-nitrophenyl)piperazin-1-yl)methyl) pyrrolidine-1-carboxylate (**compound 104C**)

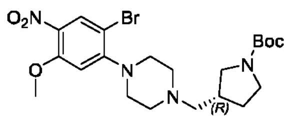

**104C**

**103I** (6.7 g, 21.19 mmol) and **104B** (7 g, 25.40 mmol) were dissolved in DMF (50 mL); sodium iodide (0.32 g, 2.12 mmol) and potassium carbonate (8.79 g, 63.57 mmol) were added; and the mixture was reacted at 100°C for 16 h. The reaction solution was cooled to room temperature, diluted by adding 200 mL of ethyl acetate, washed 3 times with water, and washed once with saturated aqueous sodium chloride solution. The organic phase was dried over anhydrous sodium sulphate and concentrated under reduced pressure, and the residue was purified by column chromatography (mobile phase: petroleum ether/ethyl acetate (V/V) = 2/1-pure ethyl acetate) to obtain **104C** (5 g, yield: 47%).

LCMS m/z = 499.1[M+H]$^+$.

Step 3: tert-butyl (R)-3-((4-(5-methoxy-2-(1-methyl-1H-pyrazol-4-yl)-4-nitrophenyl) piperazin-1-yl)methyl)pyrrolidine-1-carboxylate (104D)

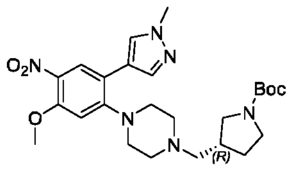

104D

**104C** (5 g, 10.01 mmol) and 1-methyl-1H-pyrazole-4-boronic acid (2.52 g, 20.02 mmol) were dissolved in 1,4-dioxane (50 mL); Pd(dppf)$Cl_2$·DCM (0.82 g, 1.00 mmol) and an aqueous solution (15 mL) of potassium carbonate (4.15 g, 30.03 mmol) were added; and the mixture was subjected to nitrogen replacement 3 times and reacted at 100°C for 4 h. The reaction solution was cooled to room temperature, diluted by adding 300 mL of ethyl acetate, washed 3 times with water and washed once with saturated sodium chloride. The organic phase was dried over anhydrous sodium sulphate and concentrated under reduced pressure, and the residue was purified by column chromatography (mobile phase: petroleum ether/ethyl acetate (V/V) = 3/1-pure ethyl acetate) to obtain **104D** (5 g, yield: 99%).

LCMS m/z = 501.3 $[M+H]^+$.

Step 4: (S)-1-(5-methoxy-2-(1-methyl-1H-pyrazol-4-yl)-4-nitrophenyl)-4-(pyrrolidin-3-ylmethyl)piperazine (**104E**)

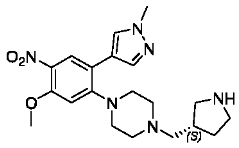

104E

**104D** (5 g, 9.99 mmol) was dissolved in dichloromethane (30 mL); trifluoroacetic acid (10 mL) was slowly added; and the mixture was reacted at room temperature for 1 h, concentrated under reduced pressure to remove dichloromethane and trifluoroacetic acid, redissolved by adding 100 mL of dichloromethane and adjusted to a basic pH with 1 N aqueous sodium hydroxide solution. Liquid separation was performed. The organic phases were collected, and the aqueous phase was extracted 3 times with dichloromethane. All organic phases were combined, dried over anhydrous sodium sulphate and concentrated under reduced pressure to obtain **104E** (3.8 g, yield: 95%), which was directly used in the next step.

Step 5: 2-(2,6-dioxopiperidin-3-yl)-5-((R)-3-((4-(5-methoxy-2-(1-methyl-1H-pyrazol-4-yl)-4-nitrophenyl)piperazin-1-yl)methyl)pyrrolidin-1-yl)isoindoline-1,3-dione (**104F**)

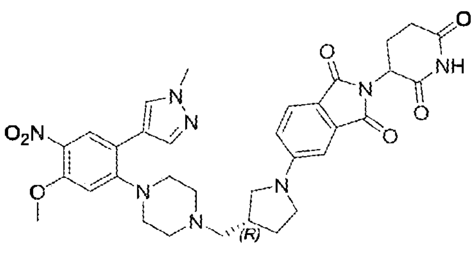

104F

**104E** (3.8 g, 9.49 mmol) and 2-(2,6-dioxopiperidin-3-yl)-5-fluoroisoindole-1,3-dione (2.88 g, 10.44 mmol) were dissolved in DMSO (30 mL); DIPEA (2.45 g, 18.98 mmol) was added dropwise; and the mixture was reacted at 90°C for 1.5 h. The reaction solution was cooled to room temperature. 30 mL of water was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase: dichloromethane/methanol (V/V) = 50/1-12/1) to obtain **104F** (3.8 g,

yield: 60%).

LCMS m/z = 657.3 [M+ H]+.

Step 6: 5-((R)-3-((4-(4-amino-5-methoxy-2-(1-methyl-1H-pyrazol-4-yl) phenyl)piperazin-1-yl)methyl)pyrrolidin-1-yl)-2-(2,6-dioxopiperidin-3-yl)isoindoline-1,3-dione (**104G**)

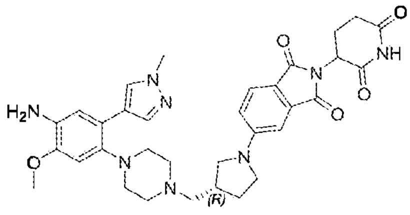

104G

**104F** (3.8 g, 5.79 mmol) was dissolved in ethanol (60 mL); reduced iron powder (3.23 g, 57.9 mmol) was added, and then an aqueous solution (20 mL) of ammonium chloride (3.1 g, 57.9 mmol) was added dropwise; and under nitrogen protection, the mixture was reacted at 80°C for 0.5 h. The reaction solution was cooled to room temperature and filtered. The filter cake was washed with 100 mL of dichloromethane. 50 mL of saturated brine was added, and then the mixture was extracted 3 times with dichloromethane. The organic phases were combined, dried over anhydrous sodium sulphate and then concentrated under reduced pressure to obtain **104G** (3.8 g, yield: 100%), which was directly used in the next step.

Step 7: 5-((R)-3-((4-(4-((5-bromo-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperazin-1-yl)methyl)pyrrolidin-1-yl)-2-(2,6-dioxopiperidin-3-yl)isoindoline-1,3-dione (**compound 104**)

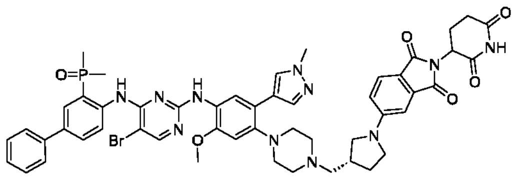

Compound 104

**23D** (1.32 g, 3.03 mmol) and **104G** (1.9 g, 3.03 mmol) were dissolved in DMF (15 mL); p-toluenesulfonic acid hydrate (1.15 g, 6.06 mmol) was added; and under nitrogen protection, the mixture was reacted at 100°C for 16 h. The reaction solution was cooled to room temperature. 10 mL of saturated aqueous sodium bicarbonate solution was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase: dichloromethane/methanol (V/V) = 50/1-15/1) to obtain a product, which was further subjected to medium-pressure preparative reversed-phase column purification (acetonitrile/water (containing 0.1% TFA) = 3%-40%) to obtain the preparative solution of the trifluoroacetate of **compound 104**. 10 mL of saturated aqueous sodium bicarbonate solution and 50 mL of dichloromethane were added; and the mixture was stirred for 5 min. Liquid separation and extraction were performed. The organic layer was dried over anhydrous sodium sulphate and then concentrated under reduced pressure to obtain **compound 104** (1.0 g, yield: 32%).

LCMS m/z = 513.8 [(M+2H)/2]+.

$^1$H NMR (400 MHz, DMSO-$d_6$) δ 11.11 (s, 1H), 11.04 (s, 1H), 8.50 - 8.34 (m, 1H), 8.28 (s, 1H), 8.19 (s, 1H), 8.03 (s, 1H), 7.88 (s, 1H), 7.74 - 7.50 (m, 5H), 7.45 (t, 2H), 7.33 (t, 1H), 7.26 - 7.03 (m, 1H), 6.98 - 6.85 (m, 2H), 6.82 (dd, 1H), 5.05 (dd, 1H), 3.81 (s, 3H), 3.76 (s, 3H), 3.63 - 3.47 (m, 2H), 3.47 - 3.36 (m, 1H), 3.22 - 3.07 (m, 1H), 2.97 - 2.76 (m, 5H), 2.71 - 2.51 (m, 7H), 2.44 (d, 2H), 2.23 - 2.06 (m, 1H), 2.06 - 1.93 (m, 1H), 1.91 - 1.67 (m, 7H).

**Example 105: Preparation of compound 105**

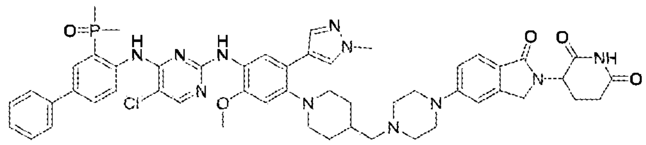

**Compound 105**

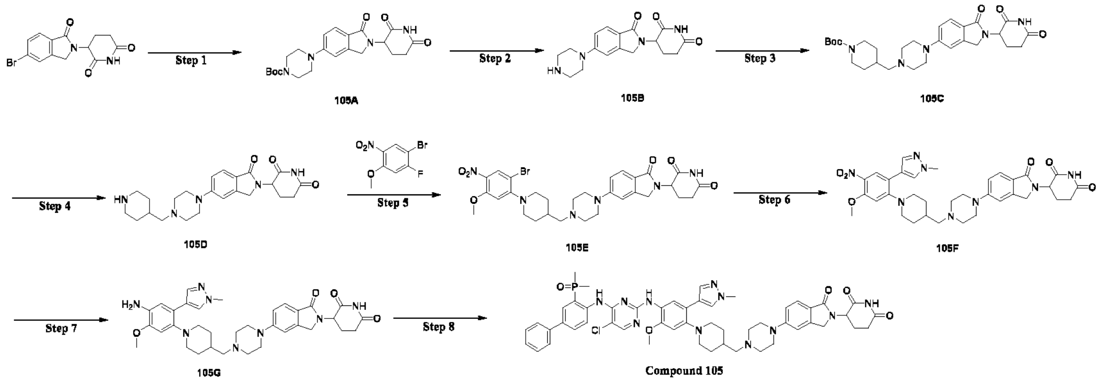


Step 1: tert-butyl 4-(2-(2,6-dioxopiperidin-3-yl)-1-oxoisoindolin-5-yl) piperazine-1-carboxylate (105A)

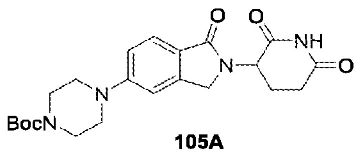

**105A**

3-(5-bromo-1-oxoisoindolin-2-yl)piperidine-2,6-dione (4.0 g, 12.4 mmol) and tert-butyl piperazine-1-carboxylate (4.0 g, 21.5 mmol) were dissolved in ultra-dry DMF (60 mL); caesium carbonate (8.1 g, 24.9 mmol) and RuPhosPdG3 (1.0 g, 1.2 mmol) were successively added; and under nitrogen protection, the mixture was stirred at 100°C for 16 h. After the reaction was completed, 200 mL of ethyl acetate and 100 mL of aqueous solution were added, and then the layers were separated. The organic layer was dried under reduced pressure, and the residue was purified by silica gel column chromatography (mobile phase: dichloromethane/methanol (V/V) = 100/1-10/1) to obtain **105A** (1.3 g, yield: 24%).
LCMS m/z = 429.2 $[M+H]^+$.

Step 2: 3-(1-oxo-5-(piperazin-1-yl)isoindolin-2-yl)piperidine-2,6-dione (**105B**); HCl

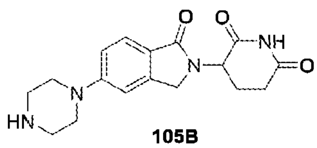

**105B**

**105A** (1.3 g, 3.0 mmol) was dissolved in methanol (3 mL); a solution of hydrochloric acid in dioxane (4 *N*, 15 mL) was added; and the mixture was reacted at room temperature for 1 h and concentrated under reduced pressure, and the residue was directly used in the next step.
LCMS m/z = 329.2 $[M+H]^+$.

Step 3: tert-butyl 4-((4-(2-(2,6-dioxopiperidin-3-yl)-1-oxoisoindolin-5-yl) piperazin-1-yl)methyl)piperidine-1-carboxylate (**105C**)

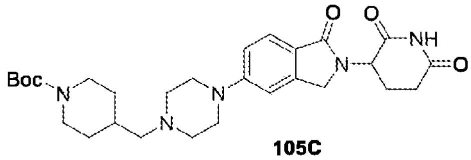

**105C**

The crude hydrochloride of **105B** (1.1 g) and tert-butyl 4-formylpiperidine-1-carboxylate (1.1 g, 5.16 mmol) were dissolved in DMAC (30 mL); 0.1 ml of acetic acid was added; and the mixture was stirred at room temperature for 1 h. Sodium triacetoxyborohydride (1.1 g, 5.2 mmol) was added, and the mixture was reacted overnight at room temperature, adjusted to a basic pH by adding saturated aqueous sodium bicarbonate solution and extracted with dichloromethane (50 mL × 3). The organic phases were combined, washed with water (100 mL × 2), dried over anhydrous sodium sulphate and concentrated under reduced pressure, and the residue was purified by silica gel column chromatography (mobile phase: ethyl acetate/petroleum ether (V/V) = 1/1 to dichloromethane/methanol (V/V) = 20/1) to obtain **105C** (1.0 g, two-step yield: 63%).

LCMS m/z = 526.3 $[M+H]^+$.

Step 4: 3-(1-oxo-5-(4-(piperidin-4-ylmethyl)piperazin-1-yl)isoindolin-2-yl) piperidine-2,6-dione (**105D**); HCl

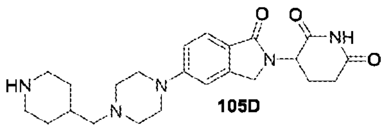

**105D**

**105C** (1.0 g, 1.9 mmol) was dissolved in methanol (3 mL); a solution of hydrochloric acid in dioxane (4 A, 15 mL) was added; and the mixture was reacted at room temperature for 1 h and concentrated under reduced pressure, and the residue was directly used in the next step.

LCMS m/z = 426.3 $[M+H]^+$.

Step 5: 3-(5-(4-((1-(2-bromo-5-methoxy-4-nitrophenyl)piperidin-4-yl)methyl) piperazin-1-yl)-1-oxoisoindolin-2-yl)piperidine-2,6-dione **(105E)**

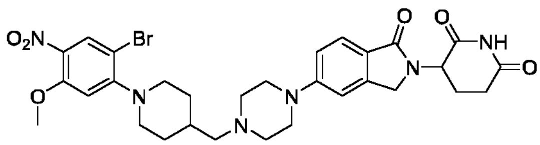

**105E**

The crude hydrochloride of **105D** (900 mg) was dissolved in DMSO (25 mL); 1-bromo-2-fluoro-4-methoxy-5-nitrobenzene (**1C**) (500 mg, 2.0 mmol) and sodium bicarbonate (504 mg, 6.0 mmol) were successively added; and the mixture was stirred at 90°C for 3 h, and cooled to room temperature. 80 mL of water was added, and the mixture was filtered. The filter cake was dried under reduced pressure and then purified by silica gel column chromatography (mobile phase: dichloromethane/methanol (V/V) = 100/1-20/1) to obtain **105E** (700 mg, two-step yield: 56%).

LCMS m/z = 655.3 $[M+H]^+$.

Step 6: 3-(5-(4-((1-(5-methoxy-2-(1-methyl-1H-pyrazol-4-yl)-4-nitrophenyl) piperidin-4-yl)methyl)piperazin-1-yl)-1-oxoisoindolin-2-yl)piperidine-2,6-dione **(105F)**

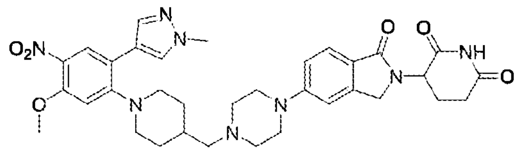

105F

Under nitrogen protection, **105E** (300 mg, 0.46 mmol) and N-methylpyrazole-4-boronic acid (115 mg, 0.91 mmol) were added to a 50 mL single-necked flask and dissolved in 10 mL of dioxane and 2 mL of water, and then Pd(dppf)$Cl_2$·DCM (81 mg, 0.10 mmol) and potassium carbonate (168 mg, 1.2 mmol) were added. The mixture was subjected to nitrogen replacement three times, reacted at 80°C for 2 h and cooled to room temperature. The reaction solution was poured into 100 ml of water and extracted with 100 ml of ethyl acetate. The organic phase was dried over anhydrous sodium sulphate and concentrated under reduced pressure, and the residue was purified by silica gel column chromatography to obtain **105F** (220 mg, yield: 73%).
LCMS m/z = 657.3 $[M+H]^+$.

Step 7: 3-(5-(4-((1-(4-amino-5-methoxy-2-(1-methyl-1H-pyrazol-4-yl)phenyl) piperidin-4-yl)methyl)piperazin-1-yl)-1-oxoisoindolin-2-yl)piperidine-2,6-dione **(105G)**

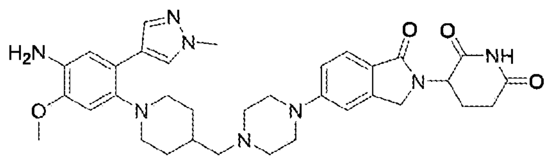

105G

**105F** (220 mg, 0.33 mmol), iron powder (200 mg, 3.57 mmol) and ammonium chloride (200 mg, 3.77 mmol) were dissolved in ethanol (30 mL) and water (10 mL), and the mixture was stirred at 80°C for 2 h, cooled to room temperature and subjected to suction filtration. The filtrate was concentrated. 30 mL of water was added, and then the mixture was extracted with 30 ml of dichloromethane. The organic phase was dried over anhydrous sodium sulphate and then concentrated under reduced pressure to obtain **105G** (170 mg, yield: 81%), which was directly used in the next step.
LCMS m/z = 627.3 $[(M+H]^+$.

Step 8: 3-(5-(4-((1-(4-((5-chloro-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperidin-4-yl)methyl)piperazin-1-yl)-1-oxoisoindolin-2-yl)piperidine-2,6-dione **(compound 105)**

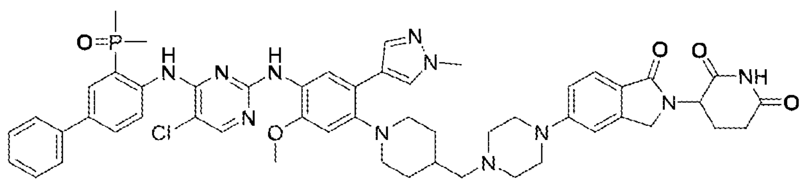

**Compound 105**

**105G** (170 mg, 0.27 mmol) and **40A** (180 mg, 0.46 mmol) were dissolved in DMF (10 mL); p-toluenesulfonic acid monohydrate (160 mg, 0.84 mmol) was added; and under nitrogen protection, the mixture was stirred at 100°C for 16 h, and cooled to room temperature. 20 mL of saturated aqueous sodium bicarbonate solution and 30 mL of dichloromethane were added, and the layers were separated. The organic layer was concentrated under reduced pressure. The concentrated solution was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 mL). Liquid separation was performed. The organic layer was then dried over anhydrous sodium sulphate and concentrated under reduced pressure to obtain **compound 105** (25 mg, yield: 9.3%).

LCMS m/z = 491.7 $[(M+2H)/2]^+$.

$^1$H NMR (400 MHz, DMSO-$d_6$) δ 11.37 (s, 1H), 10.93 (s, 1H), 8.63 - 8.41 (m, 1H), 8.30 (s, 1H), 8.11 (s, 1H), 8.04 (s, 1H), 7.88 (s, 1H), 7.71 (d, 1H), 7.64 - 7.48 (m, 4H), 7.45 (t, 2H), 7.36 (t, 1H), 7.26 - 7.11 (m, 1H), 7.11 - 7.00 (m, 2H), 6.86 (s, 1H), 5.05 (dd, 1H), 4.41 - 4.17 (m, 2H), 3.80 (s, 3H), 3.76 (s, 3H), 3.39 - 3.22 (m, 4H), 3.17 - 3.00 (m, 2H), 2.98 - 2.81 (m, 1H), 2.69 - 2.52 (m, 6H), 2.43 - 2.16 (m, 4H), 2.02 - 1.92 (m, 1H), 1.89 - 1.72 (m, 8H), 1.69 - 1.53 (m, 1H), 1.45 - 1.26 (m, 2H).

**Example 106: Preparation of compound 106**

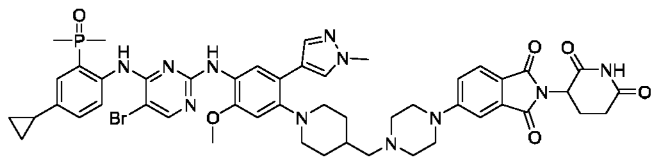

**Compound 106**

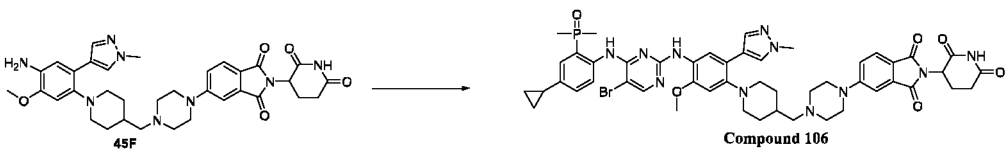

45F

Compound 106

Step 1: 5-(4-((1-(4-((5-bromo-4-((4-cyclopropyl-2-(dimethylphosphoryl) phenyl) amino)pyrimidin-2-yl)amino)-5-methoxy-2-(1-methyl-1H-pyrazol-4-yl)phenyl)piperidin-4-yl)methyl)piperazin-1-yl)-2-(2,6-dioxopiperidin-3-yl)isoindoline-1,3-dione (**compound 106**)

**45F** (256 mg, 0.4 mmol) and (2-((5-bromo-2-chloropyrimidin-4-yl)amino)-5-cyclopropylphenyl) dimethylphosphine oxide (**55B**) (200 mg, 0.5 mmol) were dissolved in DMF (5 mL); p-toluenesulfonic acid monohydrate (228 mg, 1.2 mmol) was added; and under nitrogen protection, the mixture was reacted overnight at 100°C, and cooled to room temperature. 20 mL of saturated aqueous sodium bicarbonate solution was added, and the mixture was subjected to suction filtration. The filter cake was dissolved in 50 mL of dichloromethane and extracted. The organic phase was dried over anhydrous sodium sulphate and then concentrated under reduced pressure, and the residue was purified by silica gel column chromatography (dichloromethane/methanol (V/V) = 100/1-10/1) to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)) to obtain the trifluoroacetate of **compound 106**. 20 mL of dichloromethane and 50 mL of saturated sodium bicarbonate solution were added to the trifluoroacetate of **compound 106,** and the layers were separated. The organic phase was dried over anhydrous sodium sulphate and then concentrated under reduced pressure to obtain **compound 106** (125 mg, yield: 31%).

LCMS m/z = 502.7 $[(M+2H)/2]^+$.

$^1$H NMR (400 MHz, $D_2O/CF_3COOD$(v/v = 1:1)) δ 8.23 (s, 1H), 7.96 - 7.75 (m, 5H), 7.46 (s, 1H), 7.35 (s, 1H), 7.31 - 7.15 (m, 2H), 6.91 (d, 1H), 5.12 (dd, 1H), 4.22 - 4.07 (m, 5H), 4.03 (s, 3H), 3.93 - 3.70 (m, 6H), 3.62-3.48 (m, 2H), 3.39 - 3.18 (m, 4H), 2.95 - 2.85 (m, 2H), 2.82 - 2.67 (m, 1H), 2.63 - 2.44 (m, 1H), 2.37 - 2.16 (m, 3H), 2.12 - 1.88 (m, 9H), 1.19 - 1.08 (m, 2H), 0.73 - 0.65 (m, 2H).

**Example 107: Preparation of compound 107**

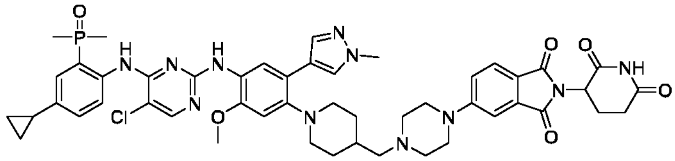

**Compound 107**

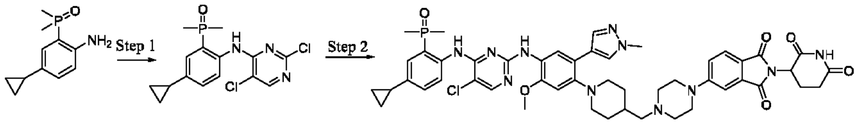


55A 107A Compound 107

Step 1: (5-cyclopropyl-2-((2,5-dichloropyrimidin-4-yl)amino)phenyl) dimethylphosphine oxide (107A)

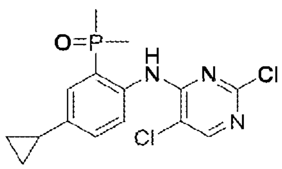

**107A**

55A (10.7 g, 51.14 mmol) and 2,4,5-trichloropyrimidine (13.96 g, 76.71 mmol) were dissolved in NMP (40 mL); DIPEA (9.91 g, 76.71 mmol) was added; and under nitrogen protection, the mixture was stirred at 120°C for 2 h, cooled to room temperature and extracted by adding 200 mL of ethyl acetate and 50 mL of water. The organic layer was washed 3 times with 50 mL of saturated brine, dried over anhydrous sodium sulphate and then concentrated under reduced pressure, and the residue was purified by silica gel column chromatography (dichloromethane/methanol (V/V) = 1/100-1/20) to obtain **107A** (15.6 g, yield: 86%).

LCMS m/z = 356.1 $[M+H]^+$.

Step 2: 5-(4-((1-(4-((5-chloro-4-((4-cyclopropyl-2-(dimethylphosphoryl)phenyl) amino)pyrimidin-2-yl)amino)-5-methoxy-2-(1-methyl-1H-pyrazol-4-yl)phenyl)piperidin-4-yl)methyl)piperazin-1-yl)-2-(2,6-dioxopiperidin-3-yl)isoindoline-1,3-dione (**compound 107**)

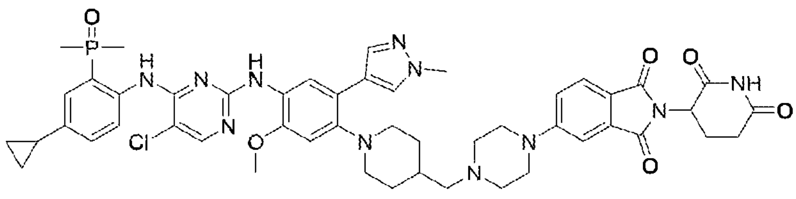

**Compound 107**

**45F** (256 mg, 0.4 mmol) and **107A** (178 mg, 0.5 mmol) were dissolved in DMF (5 mL); p-toluenesulfonic acid monohydrate (228 mg, 1.2 mmol) was added; and under nitrogen protection, the mixture was reacted overnight at 100°C, and cooled to room temperature. 20 mL of saturated aqueous sodium bicarbonate solution was added, and the mixture was filtered. The filter cake was dissolved in 50 mL of dichloromethane and extracted. The organic phase was dried over anhydrous sodium sulphate and then concentrated under reduced pressure, and the residue was purified by silica

gel column chromatography (dichloromethane/methanol (V/V) = 100/1-10/1) to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)) to obtain the trifluoroacetate of **compound 107**. 20 mL of dichloromethane and 50 mL of saturated sodium bicarbonate solution were added to the trifluoroacetate of **compound 107**, and the layers were separated. The organic phase was dried over anhydrous sodium sulphate and then concentrated under reduced pressure to obtain **compound 107** (120 mg, yield: 31.3%).

LCMS m/z = 480.8 [(M+2H)/2]$^+$.

$^1$H NMR (400 MHz, $D_2O/CF_3COOD$(v/v = 1:1)) δ 8.13 (s, 1H), 8.02 - 7.91 (m, 3H), 7.86 (s, 1H), 7.82 (d, 1H), 7.46 (s, 1H), 7.36 (s, 1H), 7.33 - 7.11 (m, 2H), 6.90 (d, 1H), 5.12 (dd, 1H), 4.23 - 4.07 (m, 5H), 4.04 (s, 3H), 3.93 - 3.74 (m, 6H), 3.63 - 3.48 (m, 2H), 3.39 - 3.20 (m, 4H), 2.95 - 2.85 (m, 2H), 2.82 - 2.65 (m, 1H), 2.63 - 2.45 (m, 1H), 2.37 - 2.17 (m, 3H), 2.14 - 1.88 (m, 9H), 1.18 - 1.07 (m, 2H), 0.71 - 0.63 (m, 2H).

**Example 108: Preparation of compound 108**

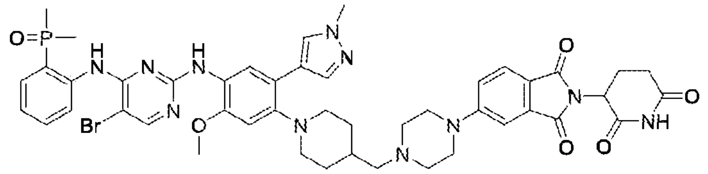

**Compound 108**

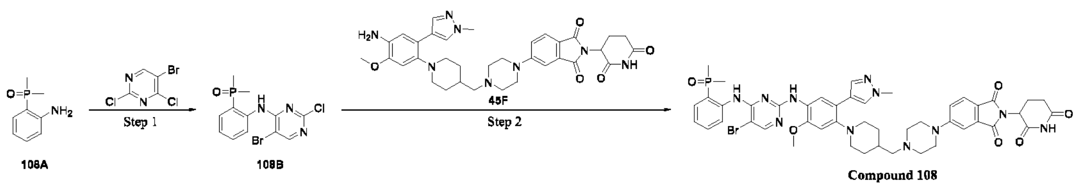

108A 108B Compound 108

Step 1: (2-((5-bromo-2-chloropyrimidin-4-yl)amino)phenyl)dimethylphosphine oxide (108B)

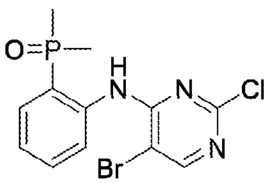

**108B**

**108A** (5 g, 29.56 mmol) and 5-bromo-2,4-dichloropyrimidine (13.47 g, 59.12 mmol) were dissolved in NMP (50 mL); DIPEA (5.73 g, 44.34 mmol) was added; and under nitrogen protection, the mixture was stirred at 120°C for 2 h, cooled to room temperature and extracted by adding 300 mL of ethyl acetate and 300 mL of water. The organic layer was washed with 150 mL of saturated brine, dried over anhydrous sodium sulphate and then concentrated under reduced pressure, and the residue was purified by silica gel column chromatography (mobile phase: DCM/MeOH (V/V) = 100/1-10/1) to obtain **108B** (8.6 g, yield: 80.6%)

LCMS m/z = 360.0 [M+H]$^+$.

$^1$H NMR (400 MHz, DMSO-$d_6$) δ 11.53 (s, 1H), 8.61 - 8.47 (m, 1H), 8.37 - 8.21 (m, 1H), 7.76 - 7.45 (m, 2H), 7.31 - 7.13 (m, 1H), 1.81 (d, 6H).

Step 2: 5-(4-((1-(4-((5-bromo-4-((2-(dimethylphosphoryl)phenyl)amino) pyrimidin-2-yl)amino)-5-methoxy-2-(1-methyl-1H-pyrazol-4-yl)phenyl)piperidin-4-yl)methyl)piperazin-1-yl)-2-(2,6-dioxopiperidin-3-yl)isoindoline-1,3-dione; **(compound 108)**

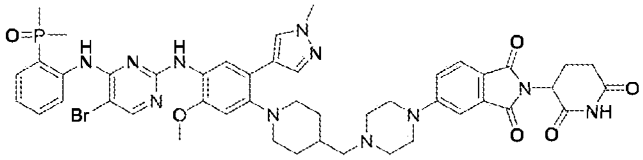

**Compound 108**

**45F** (2 g, 3.12 mmol) and **108B** (1.24 g, 3.43 mmol) were dissolved in DMF (30 mL); p-toluenesulfonic acid monohydrate (1.78 g, 9.36 mmol) was added; and under nitrogen protection, the mixture was reacted overnight at 100°C, and cooled to room temperature. 100 mL of water and 100 mL of saturated aqueous sodium bicarbonate solution were added, and the mixture was subjected to suction filtration. The filter cake was dissolved in dichloromethane and extracted. The organic phase was dried over anhydrous sodium sulphate and then concentrated under reduced pressure, and the residue was purified by silica gel column chromatography (dichloromethane/methanol (V/V) = 100/1-10/1) to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)) to obtain the **trifluoroacetate of compound 108.** 200 mL of dichloromethane and 100 mL of saturated sodium bicarbonate solution were added to the **trifluoroacetate of compound 108**, and the layers were separated. The organic phase was dried over anhydrous sodium sulphate and then concentrated under reduced pressure to obtain **compound 108** (1.1 g, yield: 36.5%).

LCMS m/z = 964.3 $[M+H]^+$.

$^1H$ NMR (400 MHz, $D_2O/CF_3COOD$(v/v = 1:1)) δ 8.29 (s, 1H), 7.94 (s, 1H), 7.91 - 7.77 (m, 4H), 7.59 (dd, 1H), 7.46 (d, 1H), 7.39 - 7.23 (m, 3H), 7.19 (t, 1H), 5.12 (dd, 1H), 4.23 - 4.08 (m, 5H), 4.05 (s, 3H), 3.97 - 3.73 (m, 6H), 3.60-3.48 (m, 2H), 3.39 - 3.15 (m, 4H), 2.95 - 2.85 (m, 2H), 2.83 - 2.65 (m, 1H), 2.65 - 2.46 (m, 1H), 2.37 - 2.16 (m, 3H), 2.13 - 1.88 (m, 8H).

**Example 109: Preparation of compound 109**

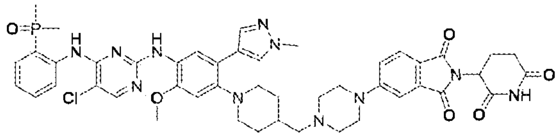

**Compound 109**

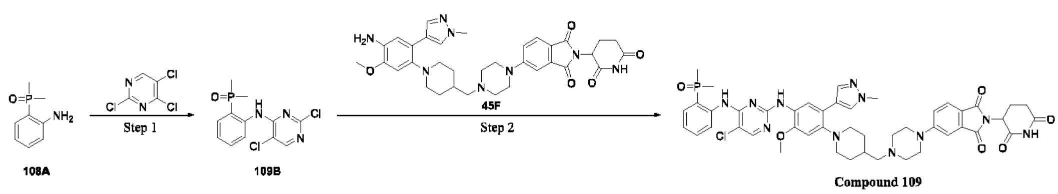


108A 109B Compound 109

Step 1: (2-((2,5-dichloropyrimidin-4-yl)amino)phenyl)dimethylphosphine oxide (109B)

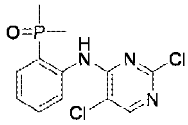

**109B**

108A (5 g, 29.56 mmol) and 2,4,5-trichloropyrimidine (10.84 g, 59.12 mmol) were dissolved in NMP (50 mL); DIPEA (5.73 g, 44.34 mmol) was added; and under nitrogen protection, the mixture was stirred at 120°C for 2 h, cooled to room temperature and extracted by adding 300 mL of ethyl acetate and 300 mL of water. The organic layer was washed with 150 mL of saturated brine, dried over anhydrous sodium sulphate and then concentrated under reduced pressure, and the residue was purified by silica gel column chromatography (mobile phase: DCM/MeOH (V/V) = 100/1-10/1) to obtain **109B** (8.3 g, yield: 88%)

LCMS m/z = 316.0 $[M+H]^+$.

$^1H$ NMR (400 MHz, DMSO-$d_6$) δ 11.82 (s, 1H), 8.49 - 8.36 (m, 2H), 7.70 - 7.56 (m, 2H), 7.33 - 7.18 (m, 1H), 1.82 (d, 6H).

Step 2: 5-(4-((1-(4-((5-chloro-4-((2-(dimethylphosphoryl)phenyl)amino) pyrimidin-2-yl)amino)-5-methoxy-2-(1-methyl-1H-pyrazol-4-yl)phenyl)piperidin-4-yl)methyl)piperazin-1-yl)-2-(2,6-dioxopiperidin-3-yl)isoindoline-1,3-dione **(compound 109)**

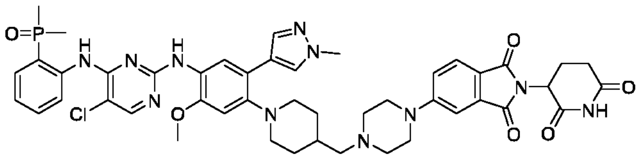

**Compound 109**

**45F** (3 g, 4.68 mmol) and **109B** (1.63 g, 5.15 mmol) were dissolved in DMF (30 mL); p-toluenesulfonic acid monohydrate (2.67 g, 14.04 mmol) was added; and under nitrogen protection, the mixture was reacted overnight at 100°C, and cooled to room temperature. 100 mL of water and 100 mL of saturated aqueous sodium bicarbonate solution were added, and the mixture was filtered. The filter cake was dissolved in dichloromethane and extracted. The organic phase was dried over anhydrous sodium sulphate and then concentrated under reduced pressure, and the residue was purified by silica gel column chromatography (dichloromethane/methanol (V/V) = 100/1-10/1) to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)) to obtain the **trifluoroacetate of compound 109** 200 mL of dichloromethane and 100 mL of saturated sodium bicarbonate solution were added to the **trifluoroacetate of compound 109,** and the layers were separated. The organic phase was dried over anhydrous sodium sulphate and then concentrated under reduced pressure to obtain **compound 109** (1.9 g, yield: 44.1%).

LCMS m/z = 920.3 $[M+H]^+$.

$^1H$ NMR (400 MHz, $D_2O/CF_3COOD$(v/v = 1:1)) δ 8.20 (s, 1H), 8.01 - 7.91 (m, 2H), 7.90 - 7.77 (m, 3H), 7.58 (dd, 1H), 7.46 (s, 1H), 7.39 - 7.24 (m, 3H), 7.17 (t, 1H), 5.12 (dd, 1H), 4.23 - 4.09 (m, 5H), 4.06 (s, 3H), 3.96 - 3.73 (m, 6H), 3.64 - 3.48 (m, 2H), 3.39 - 3.16 (m, 4H), 2.96 - 2.86 (m, 2H), 2.82 - 2.67 (m, 1H), 2.64 - 2.47 (m, 1H), 2.36 - 2.15 (m, 3H), 2.13 - 1.88 (m, 8H).

**Example 110: Preparation of compound 110**

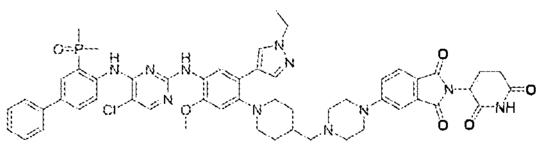

**Compound 110**

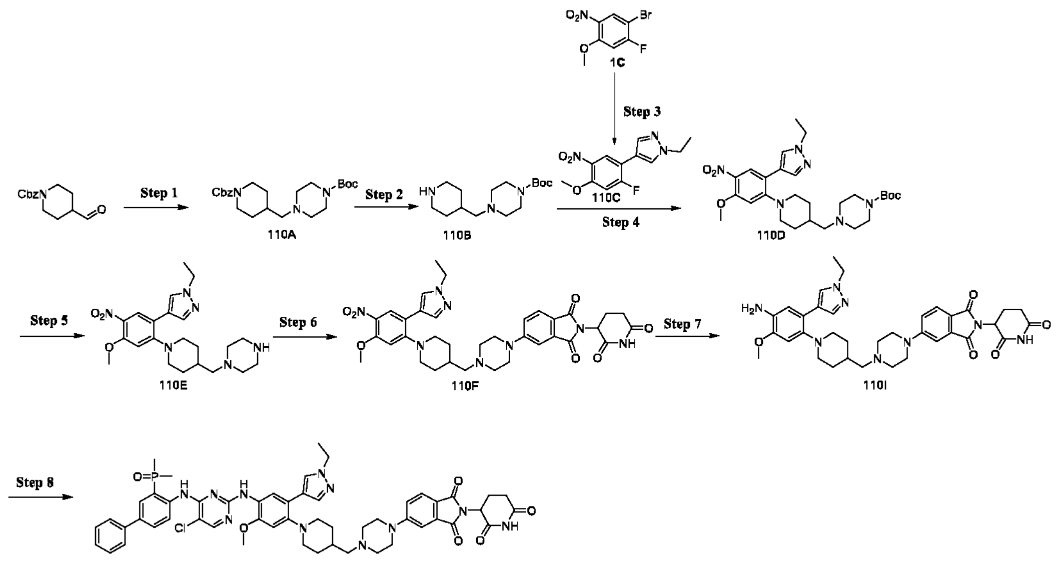


Compound 110

Step 1: tert-butyl 4-((1-((benzyloxy)carbonyl)piperidin-4-yl)methyl)piperazine-1-carboxylate (**110A**)

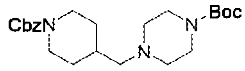

**110A**

Benzyl 4-formylpiperidine-1-carboxylate (2.47 g, 10 mmol) and tert-butylpiperazine-1-carboxylate (1.86 g, 10 mmol) were mixed in dichloromethane (100 mL); acetic acid (1.2 g, 20 mmol) and sodium triacetoxyborohydride (4.24 g, 20 mmol) were successively added; and the mixture was stirred overnight at room temperature. 100 mL of dichloromethane and 50 mL of 1 N aqueous sodium hydroxide solution were added, and then the layers were separated. The organic layer was dried under reduced pressure, and the residue was purified by silica gel column chromatography (mobile phase: ethyl acetate/petroleum ether (V/V) = 10/1-1/1) to obtain **110A** (3.54 g, yield: 85%).

LCMS m/z = 418.2 $[M+H]^+$.

Step 2: tert-butyl 4-(piperidin-4-ylmethyl)piperazine-1-carboxylate **(110B)**

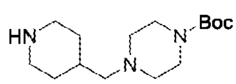

**110B**

**110A** (3.54 g, 8.49 mmol) was dissolved in methanol (50 mL); palladium on carbon (wt% = 10%, 500 mg) was added; the mixture was subjected to hydrogen replacement 3 times, stirred overnight under hydrogen atmosphere (balloon pressure) at room temperature and filtered; and the filtrate was concentrated under reduced pressure to obtain the crude of **110B**, which was directly used in the next step.

Step 3: 1-ethyl-4-(2-fluoro-4-methoxy-5-nitrophenyl)-1H-pyrazole **(110C)**

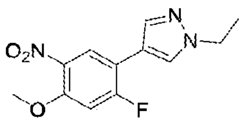

110C

Under nitrogen protection, 1-bromo-2-fluoro-4-methoxy-5-nitrobenzene **(1C)** (2.49 g, 10 mmol) and 1-ethyl-1H-pyrazole-4-pinacolatodiboron (Cas: 847818-70-6, 3.33 g, 15 mmol) were added to a 500 mL single-necked flask and dissolved in 100 mL of 1,4-dioxane and 10 mL of water, and then Pd(dppf)$Cl_2$·DCM (400 mg, 0.5 mmol) and potassium carbonate (4.14 g, 30 mmol) were added. The mixture was subjected to nitrogen replacement three times, reacted at 100°C for 16 h and cooled to room temperature. The reaction solution was poured into water and extracted three times with ethyl acetate. The organic phases were combined, dried over anhydrous sodium sulphate and concentrated under reduced pressure, and the residue was purified by silica gel column chromatography to obtain **110C** (2.1 g, yield: 80%).

LCMS m/z = 266.1 $[M+H]^+$.

Step 4: tert-butyl 4-((1-(2-(1-ethyl-1H-pyrazol-4-yl)-5-methoxy-4-nitrophenyl)piperidin-4-yl)methyl)piperazine-1-carboxylate **(110D)**

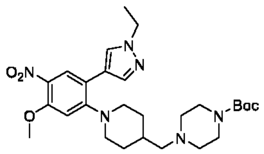

110D

**110B** (8.49 mmol), **110C** (2.1 g, 7.92 mmol) and potassium carbonate (3.51 g, 25.5 mmol) were mixed and dissolved in DMSO (30 mL), and the mixture was stirred at 120°C for 16 h, cooled to room temperature and extracted by adding 100 mL of water and 100 mL of ethyl acetate. The organic layer was concentrated under reduced pressure, and the residue was purified by column chromatography (mobile phase: dichloromethane/methanol (V/V) = 100/1-20/1) to obtain **110D** (2.5 g, yield: 60%).

LCMS m/z = 529.1 $[M+H]^+$.

Step 5: 1-((1-(2-(1-ethyl-1H-pyrazol-4-yl)-5-methoxy-4-nitrophenyl) piperidin-4-yl)methyl)piperazine (**110E**); 2,2,2-trifluoroacetate

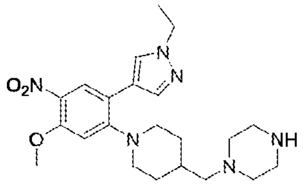

110E

**110D** (1.1 g, 2.0 mmol) was dissolved in 5 mL of dichloromethane; 3 mL of trifluoroacetic acid was added; and the mixture was stirred at room temperature for 1 h. The reaction solution was concentrated under reduced pressure to obtain the crude trifluoroacetate of **110E**, which was directly used in the next step.

Step 6: 2-(2,6-dioxopiperidin-3-yl)-5-(4-((1-(2-(1-ethyl-1H-pyrazol-4-yl)-5-methoxy-4-nitrophenyl)piperidin-4-yl)methyl)piperazin-1-yl)isoindoline-1,3-dione (**110F**)

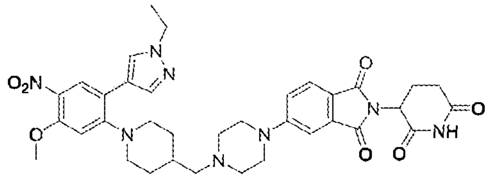

110F

The crude trifluoroacetate of **110E** (2.08 mmol) was dissolved in 10 mL of DMSO; solid sodium bicarbonate (840 mg, 10.0 mmol) was added; and the mixture was stirred at room temperature for 10 min. DIPEA (3.45 mL, 20.8 mmol) and 2-(2,6-dioxopiperidin-3-yl)-5-fluoroisoindoline-1,3-dione (662 mg, 2.40 mmol) were added, and the reaction was stirred at 80°C for 5 h. The reaction solution was cooled to room temperature. 100 mL of water was added; and the mixture was filtered to collect the solid, which was washed with water, dissolved in DCM, dried over anhydrous sodium sulphate and concentrated under reduced pressure to obtain the crude. The crude was purified by silica gel column chromatography (mobile phase: DCM/MeOH (V/V) = 15/1) to obtain **110F** (0.73 g, yield: 51%).

LCMS m/z = 685.3 $[M+H]^+$.

Step 7: 5-(4-((1-(4-amino-2-(1-ethyl-1H-pyrazol-4-yl)-5-methoxyphenyl) piperidin-4-yl)methyl)piperazin-1-yl)-2-(2,6-dioxopiperidin-3-yl)isoindoline-1,3-dione (**110I**)

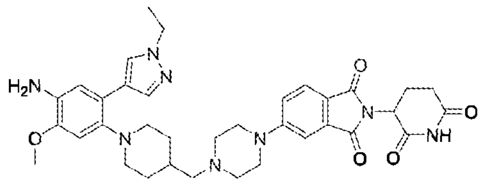

110I

**110F** (0.73 g, 1.07 mmol) was dissolved in a mixed solution of ethanol (20 mL) and water (5 mL); reduced iron powder (480 mg, 8.56 mmol) and ammonium chloride (460 mg, 8.56 mmol) were added; and the mixture was reacted at 85°C for 4 h. The reaction solution was cooled to room temperature and concentrated in vacuo to remove a solvent. 10 mL of water and 50 mL of dichloromethane were added to the residue, and then the mixture was extracted 3 times with dichloromethane. The organic phases were combined, dried over anhydrous sodium sulphate and then concentrated under reduced pressure to obtain **110I** (620 mg, yield: 88%)

LCMS m/z = 655.4 $[M+H]^+$.

Step 8: 5-(4-((1-(4-((5-chloro-4-((3-(dimethylphosphoryl)-[1,1'-biphenyl]-4-yl)amino)pyrimidin-2-yl)amino)-2-(1-ethyl-1H-pyrazol-4-yl)-5-methoxyphenyl)piperidin-4-yl)methyl)piperazin-1-yl)-2-(2,6-dioxopiperidin-3-yl)isoindoline-1,3-dione (**compound 110**)

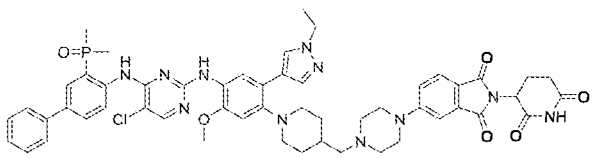

**Compound 110**

**110I** (310 mg, 0.47 mmol) and **40A** (200 mg, 0.52 mmol) were dissolved in DMF (5 mL); p-toluenesulfonic acid monohydrate (268 mg, 1.41 mmol) was added; and the mixture was stirred at 100°C for 16 h, and cooled to room temperature. 30 mL of saturated aqueous sodium bicarbonate solution was added, with a yellow solid precipitated. Suction filtration was performed for collecting the solid, and the filter cake was washed with water (20 mL × 3) and redissolved in dichloromethane. The organic layers were separated, dried over anhydrous sodium sulphate and concentrated, and the crude was further subjected to preparative HPLC (instrument: waters 2767 (preparative liquid phase

chromatographic instrument); chromatographic column: XBridge@ Prep C18 (30 mm × 150 mm); composition of mobile phases: mobile phase A: acetonitrile, and mobile phase B: water (containing 0.1% TFA)) purification to obtain a preparative solution, which was concentrated and adjusted to a basic pH by adding dichloromethane and saturated sodium bicarbonate solution. The organic phase was separated. The aqueous phase was extracted with dichloromethane (30 ml × 3), dried over anhydrous sodium sulphate and concentrated. Water was added, and the mixture was lyophilized to obtain **compound 110** (120 mg, yield: 25%).

LCMS m/z = 505.8[(M+2H)/2]$^+$.

$^1$H NMR (400 MHz, DMSO-$d_6$) δ 11.37 (s, 1H), 11.07 (s, 1H), 8.60 - 8.39 (m, 1H), 8.31 (s, 1H), 8.11 (s, 1H), 8.10 (s, 1H), 7.90 (s, 1H), 7.76 - 7.63 (m, 2H), 7.62 - 7.51 (m, 3H), 7.44 (t, 2H), 7.40 - 7.31 (m, 2H), 7.27 (d, 1H), 7.23 - 7.05 (m, 1H), 6.86 (s, 1H), 5.08 (dd, 1H), 4.06 (q, 2H), 3.80 (s, 3H), 3.56 - 3.40 (m, 4H), 3.17 - 3.01 (m, 2H), 2.96 - 2.80 (m, 1H), 2.70 - 2.50 (m, 8H), 2.29 (d, 2H), 2.11 - 1.97 (m, 1H), 1.91 - 1.72 (m, 8H), 1.72 - 1.47 (m, 1H), 1.41 - 1.25 (m, 5H).

**Example 111: Preparation of compound 111**

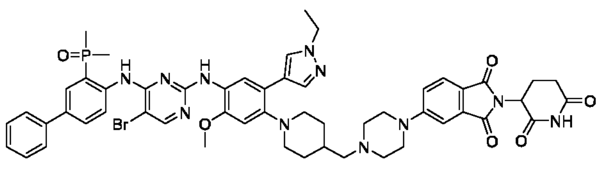

Compound 111

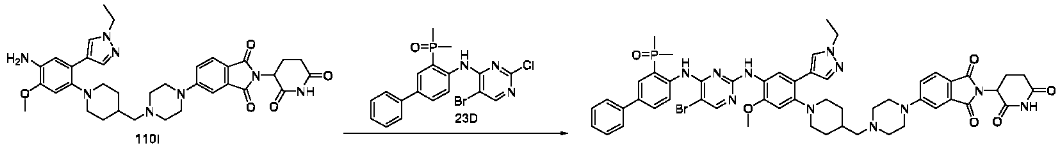


Compound 111

Step 1: 5-(4-((1-(4-((5-bromo-4-((3-(dimethylphosphoryl)-[1,1'-biphenyl]-4-yl) amino)pyrimidin-2-yl)amino)-2-(1-ethyl-1H-pyrazol-4-yl)-5-methoxyphenyl)piperidin-4-yl)methyl)piperazin-1-yl)-2-(2,6-dioxopiperidin-3-yl)isoindoline-1,3-dione (**compound 111**)

**110l** (310 mg, 0.47 mmol) and **23D** (230 mg, 0.52 mmol) were dissolved in DMF (5 mL); p-toluenesulfonic acid monohydrate (268 mg, 1.41 mmol) was added; and the mixture was stirred at 100°C for 16 h, and cooled to room temperature. 30 mL of saturated aqueous sodium bicarbonate solution was added, with a yellow solid precipitated. Suction filtration was performed for collecting the solid, and the filter cake was washed with water (20 ml × 3) and redissolved in dichloromethane. The organic layers were separated, dried over anhydrous sodium sulphate and concentrated, and the crude was further subjected to preparative HPLC (instrument: waters 2767 (preparative liquid phase chromatographic instrument); chromatographic column: XBridge@ Prep C18 (30 mm × 150 mm); composition of mobile phases: mobile phase A: acetonitrile, and mobile phase B: water (containing 0.1% TFA)) purification to obtain a preparative solution, which was concentrated and adjusted to a basic pH by adding dichloromethane and saturated sodium bicarbonate solution. The organic phase was separated. The aqueous phase was extracted with dichloromethane (30 ml × 3), dried over anhydrous sodium sulphate and concentrated. Water was added, and the mixture was lyophilized to obtain **compound 111** (120 mg, yield: 24%).

LCMS m/z = 527.8 [(M+2H)/2]$^+$.

$^1$H NMR (400 MHz, DMSO-$d_6$) δ 11.12 (s, 1H), 11.07 (s, 1H), 8.50 - 8.35 (m, 1H), 8.32 (s, 1H), 8.19 (s, 1H), 8.09 (s, 1H), 7.89 (s, 1H), 7.79 - 7.63 (m, 2H), 7.63 - 7.50 (m, 3H), 7.44 (t, 2H), 7.41 - 7.30 (m, 2H), 7.27 (d, 1H), 7.21 - 7.02 (m, 1H), 6.85 (s, 1H), 5.08 (dd, 1H), 4.06 (q, 2H), 3.79 (s, 3H), 3.57 - 3.38 (m, 4H), 3.16 - 3.00 (m, 2H), 2.97 - 2.79 (m, 1H), 2.71 - 2.51 (m, 8H), 2.28 (d, 2H), 2.10 - 1.96 (m, 1H), 1.90 - 1.69 (m, 8H), 1.69 - 1.52 (m, 1H), 1.39 - 1.25 (m, 5H).

**Example 112: Preparation of compound 112**

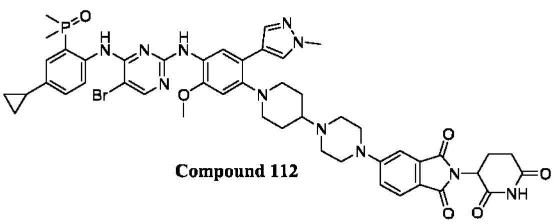

Compound 112

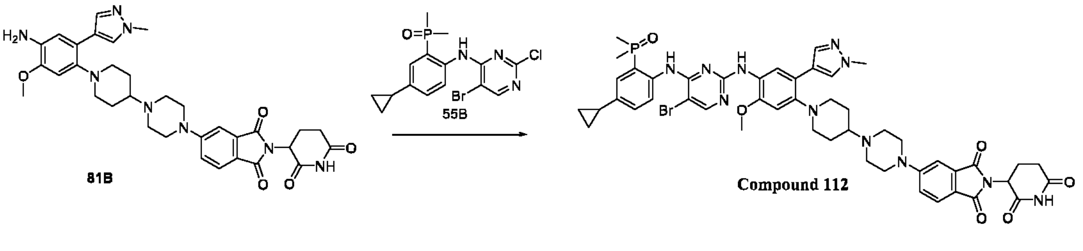

81B

55B

Compound 112

Step 1: 5-(4-(1-(4-((5-bromo-4-((4-cyclopropyl-2-(dimethylphosphoryl)phenyl) amino)pyrimidin-2-yl)amino)-5-methoxy-2-(1-methyl-1H-pyrazol-4-yl)phenyl)piperidin-4-yl)piperazin-1-yl)-2-(2,6-dioxopiperidin-3-yl)isoindoline-1,3-dione **(compound 112)**

**81B** (2.3 g, 3.30 mmol) and **55B** (1.45 g, 3.63 mmol) were dissolved in DMF (20 mL); p-toluenesulfonic acid monohydrate (1.88 g, 9.90 mmol) was added; and under nitrogen protection, the mixture was stirred at 100°C for 16 h, and cooled to room temperature. 100 mL of saturated aqueous sodium bicarbonate solution and 200 mL of dichloromethane were added, and the layers were separated. The organic layer was concentrated under reduced pressure. The concentrated solution was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 100 mL of dichloromethane and extracted by adding saturated sodium bicarbonate solution (100 mL). Liquid separation was performed. The organic layer was then dried over anhydrous sodium sulphate and concentrated under reduced pressure to obtain **compound 112** (1.0 g, yield: 31%).

LCMS m/z = 495.8 $[(M+2H)/2]^+$.

$^1$H NMR (400 MHz, DMSO-$d_6$) δ 11.07 (s, 1H), 10.84 (s, 1H), 8.25 - 8.07 (m, 3H), 7.98 (s, 1H), 7.88 (s, 1H), 7.69 (d, 1H), 7.57 (s, 1H), 7.41 - 7.13 (m, 3H), 6.80 (s, 1H), 6.57 - 6.34 (m, 1H), 5.07 (dd, 1H), 3.84 (s, 3H), 3.78 (s, 3H), 3.60 - 3.40 (m, 4H), 3.20 - 3.06 (m, 2H), 2.97 - 2.82 (m, 1H), 2.75 - 2.55 (m, 8H), 2.40 - 2.29 (m, 1H), 2.09 - 1.97 (m, 1H), 1.95 - 1.85 (m, 2H), 1.85 - 1.70 (m, 7H), 1.69 - 1.54 (m, 2H), 0.95 - 0.81 (m, 2H), 0.57 - 0.39 (m, 2H).

**Example 113: Preparation of compound 113**

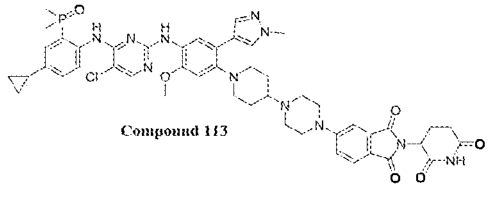

Compound 113

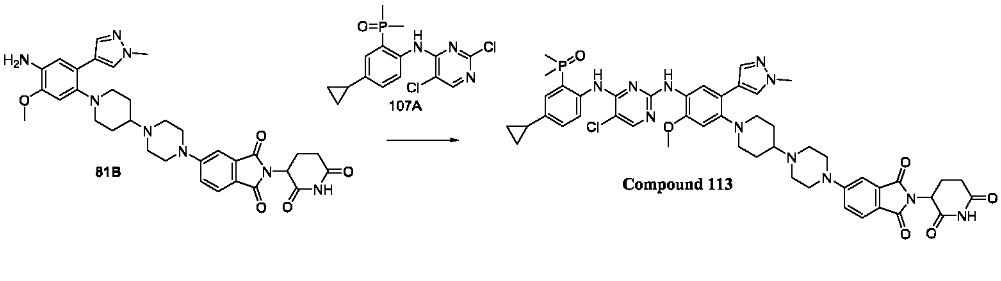

81B

107A

Compound 113

Step 1: 5-(4-(1-(4-((5-chloro-4-((4-cyclopropyl-2-(dimethylphosphoryl)phenyl) amino)pyrimidin-2-yl)amino)-5-methoxy-2-(1-methyl-1H-pyrazol-4-yl)phenyl)piperidin-4-yl)piperazin-1-yl)-2-(2, 6-dioxopiperidin-3-yl)isoindoline-1,3-dione **(compound 113)**

**81B** (2.3 g, 3.30 mmol) and **107A** (1.29 g, 3.63 mmol) were dissolved in DMF (20 mL); p-toluenesulfonic acid monohydrate (1.88 g, 9.90 mmol) was added; and under nitrogen protection, the mixture was stirred at 100°C for 16 h, and cooled to room temperature. 50 mL of saturated aqueous sodium bicarbonate solution and 200 mL of dichloromethane were added, and the layers were separated. The organic layer was concentrated under reduced pressure. The concentrated solution was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 200 mL of dichloromethane and extracted by adding saturated sodium bicarbonate solution (100 mL). Liquid separation was performed. The organic layer was then dried over anhydrous sodium sulphate and concentrated under reduced pressure to obtain **compound 113** (1.1 g, yield: 32%).

LCMS m/z = 473.8 $[(M+2H)/2]^+$.

$^1H$ NMR (400 MHz, DMSO-$d_6$) δ 11.11 (s, 1H), 11.07 (s, 1H), 8.31 - 8.19 (m, 1H), 8.15 (s, 1H), 8.07 (s, 1H), 8.00 (s, 1H), 7.89 (s, 1H), 7.69 (d, 1H), 7.59 (s, 1H), 7.41 - 7.15 (m, 3H), 6.81 (s, 1H), 6.54 - 6.31 (m, 1H), 5.07 (dd, 1H), 3.85 (s, 3H), 3.78 (s, 3H), 3.54 - 3.41 (m, 4H), 3.20 - 3.08 (m, 2H), 2.97 - 2.82 (m, 1H), 2.76 - 2.54 (m, 8H), 2.41 - 2.28 (m, 1H), 2.09 - 1.97 (m, 1H), 1.95 - 1.86 (m, 2H), 1.85 - 1.70 (m, 7H), 1.70-1.55 (m, 2H), 1.01 - 0.79 (m, 2H), 0.62 - 0.45 (m, 2H).

**Example 114: Preparation of compound 114**

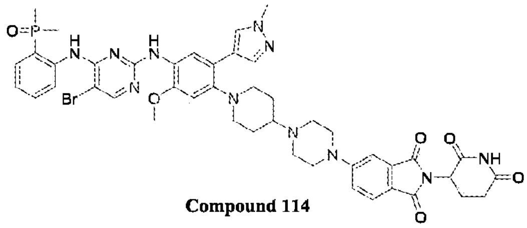

Compound 114

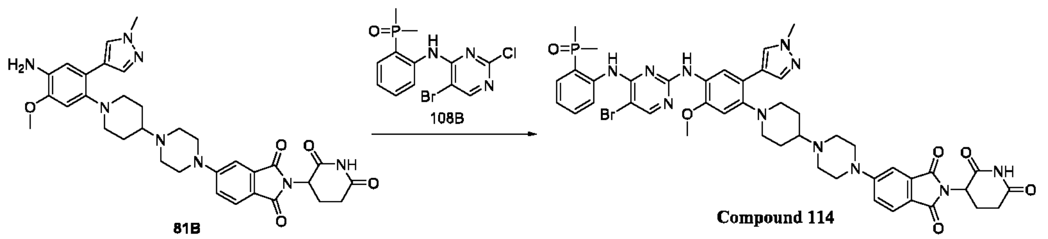


81B

Compound 114

Step 1: 5-(4-(1-(4-((5-bromo-4-((2-(dimethylphosphoryl)phenyl)amino) pyrimidin-2-yl)amino)-5-methoxy-2-(1-methyl-1H-pyrazol-4-yl)phenyl)piperidin-4-yl)piperazin-1-yl)-2-(2,6-dioxopiperidin-3-yl)isoindoline-1,3-dione (**compound 114**)

**81B** (2.24 g, 3.57 mmol) and **108B** (1.29 g, 3.57 mmol) were dissolved in DMF (30 mL); p-toluenesulfonic acid hydrate (2.04 g, 10.71 mmol) was added; and under nitrogen protection, the mixture was reacted at 100°C for 16 h. The reaction solution was cooled to room temperature. 50 mL of saturated aqueous sodium bicarbonate solution was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase: dichloromethane/methanol (V/V) = 50/1-15/1) to obtain a product, which was further purified by preparative HPLC (composition of mobile phases: mobile phase A: acetonitrile, and mobile phase B: water (containing 0.1% TFA)) to obtain a product, which was alkalized with aqueous sodium bicarbonate solution, extracted with dichloromethane, concentrated and dried to obtain **compound 114** (1.1 g, yield: 32%).

LCMS m/z = 475.7$[(M+2H)/2]^+$.

$^1H$ NMR (400 MHz, DMSO-$d_6$) δ 11.07 (s, 1H), 10.94 (s, 1H), 8.40 - 8.23 (m, 1H), 8.24 - 8.08 (m, 2H), 7.95 (s,

1H), 7.84 (s, 1H), 7.69 (d, 1H), 7.59 (s, 1H), 7.55 - 7.44 (m, 1H), 7.39 - 7.20 (m, 2H), 7.06 - 6.86 (m, 2H), 6.80 (s, 1H), 5.07 (dd, 1H), 3.83 (s, 3H), 3.79 (s, 3H), 3.53 - 3.42 (m, 4H), 3.20 - 3.05 (m, 2H), 2.96 - 2.81 (m, 1H), 2.76 - 2.53 (m, 8H), 2.40 - 2.27 (m, 1H), 2.07 - 1.97 (m, 1H), 1.93 - 1.83 (m, 2H), 1.76 (d, 6H), 1.68 - 1.50 (m, 2H).

**Example 115: Preparation of compound 115**

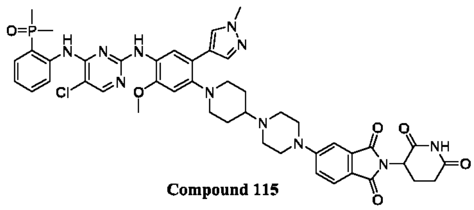

Compound 115

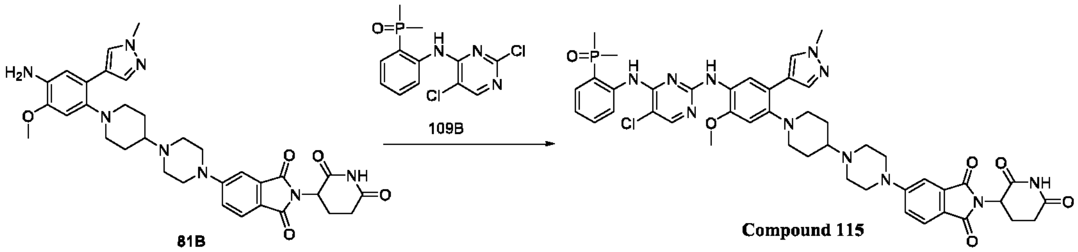

81B

109B

Compound 115

Step 1: 5-(4-(1-(4-((5-chloro-4-((2-(dimethylphosphoryl)phenyl)amino) pyrimidin-2-yl)amino)-5-methoxy-2-(1-methyl-1H-pyrazol-4-yl)phenyl)piperidin-4-yl)piperazin-1-yl)-2-(2,6-dioxopiperidin-3-yl)isoindoline-1,3-dione (**compound 115**)

**81B** (2.24 g, 3.57 mmol) and **109B** (1.13 g, 3.57 mmol) were dissolved in DMF (30 mL); p-toluenesulfonic acid hydrate (2.04 g, 10.71 mmol) was added; and under nitrogen protection, the mixture was reacted at 100°C for 16 h. The reaction solution was cooled to room temperature. 50 mL of saturated aqueous sodium bicarbonate solution was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase: dichloromethane/methanol (V/V) = 50/1-15/1) to obtain a product, which was further purified by preparative HPLC (composition of mobile phases: mobile phase A: acetonitrile, and mobile phase B: water (containing 0.1% TFA)) to obtain a product, which was alkalized with aqueous sodium bicarbonate solution, extracted with dichloromethane, concentrated and dried to obtain **compound 115** (780 mg, yield: 24%).

LCMS m/z = 453.8[$(M+2H)/2]^+$.

$^1$H NMR (400 MHz, DMSO-$d_6$) δ 11.21 (s, 1H), 11.07 (s, 1H), 8.55 - 8.39 (m, 1H), 8.20 (s, 1H), 8.10 (s, 1H), 7.97 (s, 1H), 7.85 (s, 1H), 7.69 (d, 1H), 7.63 - 7.44 (m, 2H), 7.40 - 7.20 (m, 2H), 7.08 - 6.86 (m, 2H), 6.80 (s, 1H), 5.07 (dd, 1H), 3.83 (s, 3H), 3.79 (s, 3H), 3.55 - 3.36 (m, 4H), 3.21 - 3.07 (m, 2H), 2.97 - 2.82 (m, 1H), 2.75 - 2.53 (m, 8H), 2.39 - 2.24 (m, 1H), 2.07 - 1.97 (m, 1H), 1.94 - 1.84 (m, 2H), 1.76 (d, 6H), 1.67 - 1.50 (m, 2H).

**Example 116: Preparation of compound 116**

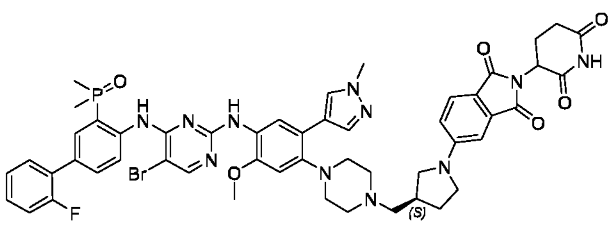

Compound 116

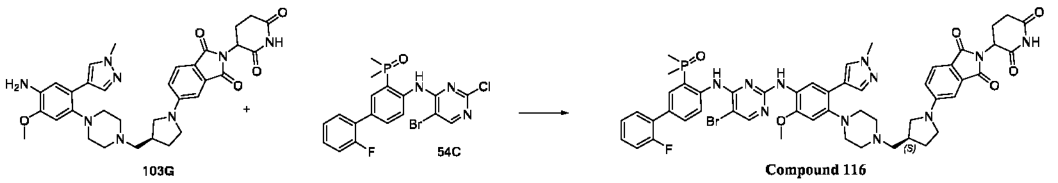

103G

54C

Compound 116

Step 1: 5-((S)-3-((4-(4-((5-bromo-4-((3-(dimethylphosphoryl)-2'-fluoro-[1,1'-biphenyl]-4-yl)amino)pyrimidin-2-yl)amino)-5-methoxy-2-(1-methyl-1H-pyrazol-4-yl)phenyl)piperazin-1-yl)methyl)pyrrolidin-1-yl)-2-(2,6-dioxopiperidin-3-yl)isoindoline-1,3-dione (**compound 116**)

**103G** (1.70 g, 2.71 mmol) and **54C** (1.23 g, 2.71 mmol) were dissolved in DMF (40 mL); p-toluenesulfonic acid hydrate (1.55 g, 8.13 mmol) was added; and under nitrogen protection, the mixture was reacted at 100°C for 16 h. The reaction solution was cooled to room temperature. 50 mL of saturated aqueous sodium bicarbonate solution was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase: dichloromethane/methanol (V/V) = 50/1-15/1) to obtain a product, which was further purified by preparative HPLC (composition of mobile phases: mobile phase A: acetonitrile, and mobile phase B: water (containing 0.1% TFA)) to obtain a product, which was alkalized with aqueous sodium bicarbonate solution, extracted with dichloromethane, concentrated and dried to obtain **compound 116** (750 mg, yield: 26%).

LCMS m/z = 522.7[$(M+2H)/2]^+$.

$^1H$ NMR (400 MHz, DMSO-$d_6$) δ 11.14 (s, 1H), 11.05 (s, 1H), 8.51 - 8.37 (m, 1H), 8.34 (s, 1H), 8.19 (s, 1H), 8.02 (s, 1H), 7.86 (s, 1H), 7.71 - 7.48 (m, 3H), 7.48 - 7.33 (m, 2H), 7.33 - 7.22 (m, 2H), 7.20 - 7.00 (m, 1H), 6.96 - 6.72 (m, 3H), 5.05 (dd, 1H), 3.79 (s, 3H), 3.75 (s, 3H), 3.63 - 3.47 (m, 2H), 3.47 - 3.37 (m, 1H), 3.22 - 3.10 (m, 1H), 2.96 - 2.77 (m, 5H), 2.72 - 2.51 (m, 7H), 2.46 - 2.36 (m, 2H), 2.21 - 2.09 (m, 1H), 2.07 - 1.93 (m, 1H), 1.90 - 1.69 (m, 7H).

**Example 117: Preparation of compound 117**

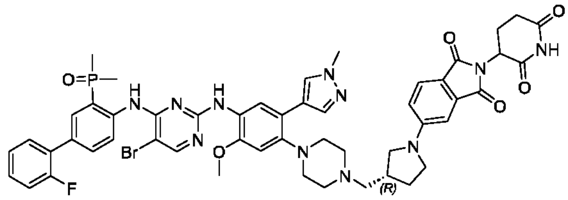

Compound 117

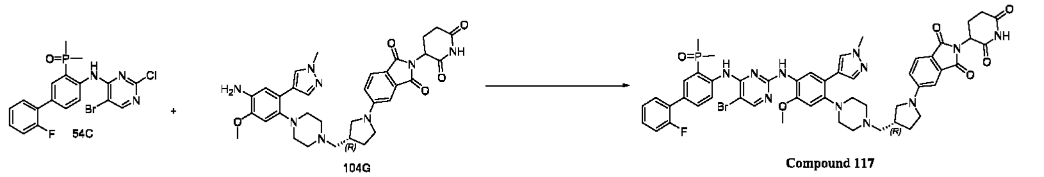

54C

104G

Compound 117

Step 1: 5-((R)-3-((4-(4-((5-bromo-4-((3-(dimethylphosphoryl)-2'-fluoro-[1,1'-biphenyl]-4-yl)amino)pyrimidin-2-yl)amino)-5-methoxy-2-(1-methyl-1H-pyrazol-4-yl)phenyl)piperazin-1-yl)methyl)pyrrolidin-1-yl)-2-(2,6-dioxopiperidin-3-yl)isoindoline-1,3-dione (**compound 117**)

**54C** (1.38 g, 3.03 mmol) and **104G** (1.9 g, 3.03 mmol) were dissolved in DMF (15 mL); p-toluenesulfonic acid hydrate (1.15 g, 6.06 mmol) was added; and under nitrogen protection, the mixture was reacted at 100°C for 16 h. The reaction solution was cooled to room temperature. 10 mL of saturated aqueous sodium bicarbonate solution was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase: dichloromethane/methanol (V/V) = 50/1-15/1) to obtain a product, which was further subjected to medium-pressure preparative reversed-phase column

purification (acetonitrile/water (containing 0.1% TFA) = 3% - 40%) to obtain the preparative solution of the trifluoroacetate of **compound 117**. 10 mL of saturated aqueous sodium bicarbonate solution and 50 mL of dichloromethane were added; and the mixture was stirred for 5 min. Liquid separation and extraction were performed. The organic layer was dried over anhydrous sodium sulphate and then concentrated under reduced pressure to obtain **compound 117** (1.1 g, yield: 35%).

LCMS m/z = 522.7 [(M+2H)/2]$^+$.

$^1$H NMR (400 MHz, DMSO-$d_6$) δ 11.14 (s, 1H), 11.04 (s, 1H), 8.57 - 8.37 (m, 1H), 8.32 (s, 1H), 8.19 (s, 1H), 8.01 (s, 1H), 7.85 (s, 1H), 7.73 - 7.48 (m, 3H), 7.47 - 7.34 (m, 2H), 7.34 - 7.22 (m, 2H), 7.19 - 7.00 (m, 1H), 6.96 - 6.69 (m, 3H), 5.05 (dd, 1H), 3.79 (s, 3H), 3.75 (s, 3H), 3.63 - 3.48 (m, 2H), 3.47 - 3.38 (m, 1H), 3.21 - 3.10 (m, 1H), 3.00 - 2.76 (m, 5H), 2.73 - 2.51 (m, 7H), 2.45 - 2.35 (m, 2H), 2.21 - 2.09 (m, 1H), 2.08 - 1.96 (m, 1H), 1.93 - 1.67 (m, 7H).

**Example 118: Preparation of compound 118**

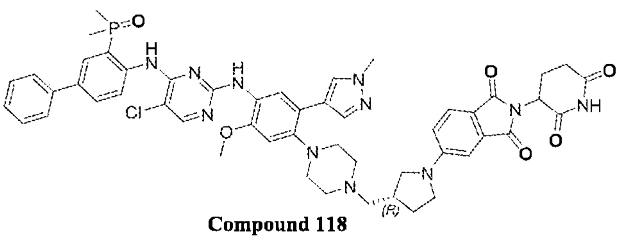

Compound 118

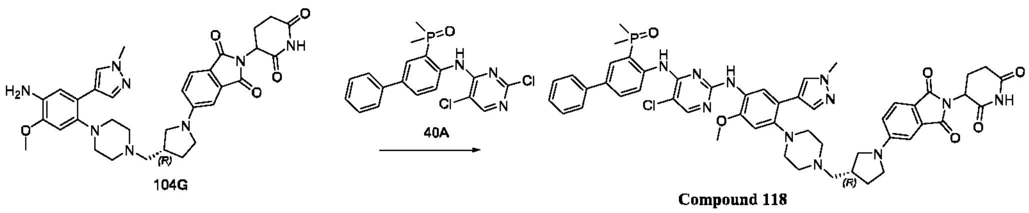


Step 1: 5-((R)-3-((4-(4-((5-chloro-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperazin-1-yl)methyl)pyrrolidin-1-yl)-2-(2,6-dioxopiperidin-3-yl)isoindoline-1,3-dione (**compound 118**)

**104G** (250 mg, 0.40 mmol) and **40A** (200 mg, 0.53 mmol) were dissolved in DMF (10 mL); p-toluenesulfonic acid monohydrate (160 mg, 0.84 mmol) was added; and under nitrogen protection, the mixture was stirred at 100°C for 16 h, and cooled to room temperature. 10 mL of saturated aqueous sodium bicarbonate solution and 20 mL of dichloromethane were added, and the layers were separated. The organic layer was concentrated under reduced pressure. The concentrated solution was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 mL). Liquid separation was performed. The organic layer was then dried over anhydrous sodium sulphate and concentrated under reduced pressure to obtain **compound 118** (60 mg, yield: 15%).

LCMS m/z = 982.3 [M+H]$^+$.

$^1$H NMR (400 MHz, DMSO-$d_6$) δ 11.37 (s, 1H), 11.04 (s, 1H), 8.59 - 8.42 (m, 1H), 8.28 (s, 1H), 8.12 (s, 1H), 8.05 (s, 1H), 7.89 (s, 1H), 7.71 (dd, 1H), 7.67 - 7.59 (m, 2H), 7.59 - 7.50 (m, 2H), 7.45 (t, 2H), 7.33 (t, 1H), 7.22 - 7.06 (m, 1H), 6.98 - 6.86 (m, 2H), 6.82 (dd, 1H), 5.05 (dd, 1H), 3.81 (s, 3H), 3.76 (s, 3H), 3.62 - 3.48 (m, 2H), 3.48 - 3.37 (m, 1H), 3.22 - 3.13 (m, 1H), 3.00 - 2.78 (m, 5H), 2.73 - 2.52 (m, 7H), 2.45 (d, 2H), 2.23 - 2.09 (m, 1H), 2.06 - 1.96 (m, 1H), 1.90 - 1.71 (m, 7H).

**Example 119: Preparation of compound 119**

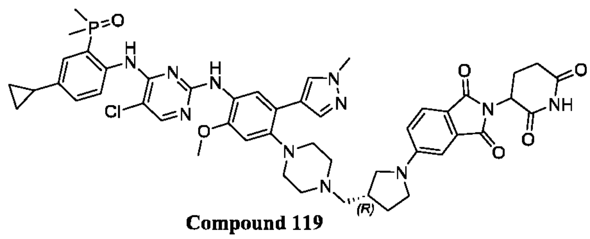

**Compound 119**

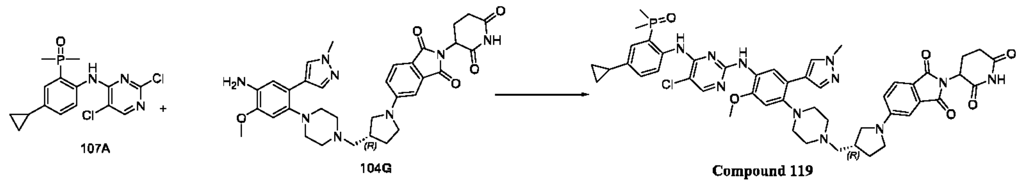

107A + 104G → Compound 119

Step 1: 5-((R)-3-((4-(4-((5-chloro-4-((4-cyclopropyl-2-(dimethylphosphoryl) phenyl)amino)pyrimidin-2-yl)amino)-5-methoxy-2-(1-methyl-1H-pyrazol-4-yl)phenyl)piperazin-1-yl)methyl)pyrrolidin-1-yl)-2-(2,6-dioxopiperidin-3-yl)isoindoline-1,3-dione (**compound 119**)

**104G** (250 mg, 0.40 mmol) and **107A** (200 mg, 0.56 mmol) were dissolved in DMF (10 mL); p-toluenesulfonic acid monohydrate (160 mg, 0.84 mmol) was added; and under nitrogen protection, the mixture was stirred at 100°C for 16 h, and cooled to room temperature. 10 mL of saturated aqueous sodium bicarbonate solution and 20 mL of dichloromethane were added, and the layers were separated. The organic layer was concentrated under reduced pressure. The concentrated solution was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 mL). Liquid separation was performed. The organic layer was then dried over anhydrous sodium sulphate and concentrated under reduced pressure to obtain **compound 119** (100 mg, yield: 27%).

LCMS m/z = 473.8 $[(M+2H)/2]^+$.

$^1H$ NMR (400 MHz, DMSO-$d_6$) δ 11.24 - 10.78 (m, 2H), 8.37 - 8.20 (m, 1H), 8.16 (s, 1H), 8.07 (s, 1H), 8.01 (s, 1H), 7.90 (s, 1H), 7.65 (d, 1H), 7.59 (s, 1H), 7.23 (dd, 1H), 6.94 - 6.76 (m, 3H), 6.58 - 6.32 (m, 1H), 5.05 (dd, 1H), 3.84 (s, 3H), 3.80 (s, 3H), 3.63 - 3.47 (m, 2H), 3.47 - 3.36 (m, 1H), 3.23 - 3.13 (m, 1H), 3.00 - 2.77 (m, 5H), 2.75 - 2.51 (m, 7H), 2.46 (d, 2H), 2.23 - 2.10 (m, 1H), 2.06 - 1.94 (m, 1H), 1.86 - 1.71 (m, 8H), 0.95 - 0.85 (m, 2H), 0.60 - 0.46 (m, 2H).

**Example 120: Preparation of compound 120**

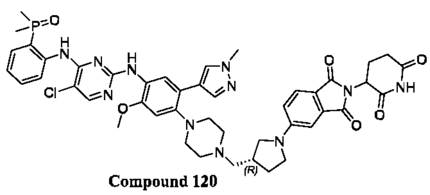

**Compound 120**

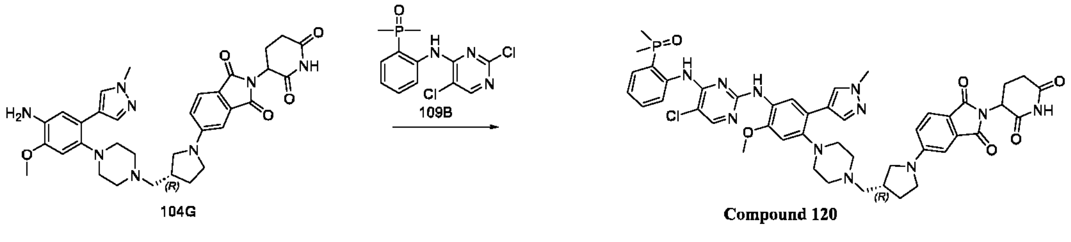

104G; 109B; Compound 120

Step 1: 5-((R)-3-((4-(4-((5-chloro-4-((2-(dimethylphosphoryl)phenyl)amino) pyrimidin-2-yl)amino)-5-methoxy-2-(1-methyl-1H-pyrazol-4-yl)phenyl)piperazin-1-yl)methyl)pyrrolidin-1-yl)-2-(2,6-dioxopiperidin-3-yl)isoindoline-1,3-dione (**compound 120**)

**104G** (250 mg, 0.40 mmol) and **109B** (200 mg, 0.63 mmol) were dissolved in DMF (10 mL); p-toluenesulfonic acid monohydrate (160 mg, 0.84 mmol) was added; and under nitrogen protection, the mixture was stirred at 100°C for 16 h, and cooled to room temperature. 10 mL of saturated aqueous sodium bicarbonate solution and 20 mL of dichloromethane were added, and the layers were separated. The organic layer was concentrated under reduced pressure. The concentrated solution was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 20 mL of dichloromethane and extracted by adding saturated sodium bicarbonate solution (50 mL). Liquid separation was performed. The organic layer was then dried over anhydrous sodium sulphate and concentrated under reduced pressure to obtain **compound 120** (120 mg, yield: 33%).

LCMS m/z = 906.3 $[M+H]^+$.

$^1H$ NMR (400 MHz, DMSO-$d_6$) δ 11.21 (s, 1H), 11.04 (s, 1H), 8.57 - 8.31 (m, 1H), 8.19 (s, 1H), 8.10 (s, 1H), 7.98 (s, 1H), 7.86 (s, 1H), 7.70 - 7.57 (m, 2H), 7.56 - 7.44 (m, 1H), 7.05 - 6.93 (m, 2H), 6.92 - 6.86 (m, 1H), 6.86 - 6.73 (m, 2H), 5.05 (dd, 1H), 3.82 (s, 3H), 3.81 (s, 3H), 3.63 - 3.46 (m, 2H), 3.46 - 3.35 (m, 1H), 3.22 - 3.11 (m, 1H), 3.01 - 2.79 (m, 5H), 2.73 - 2.52 (m, 7H), 2.45 (d, 2H), 2.21 - 2.08 (m, 1H), 2.06 - 1.93 (m, 1H), 1.86 - 1.66 (m, 8H).

**Example 121: Preparation of compound 121**

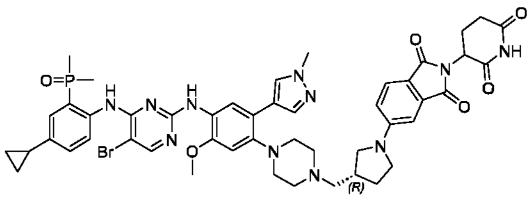

Compound 121

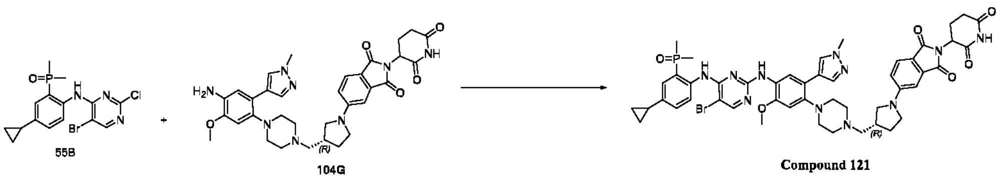

55B

104G

Compound 121

Step 1: 5-((R)-3-((4-(4-((5-bromo-4-((4-cyclopropyl-2-(dimethylphosphoryl) phenyl)amino)pyrimidin-2-yl)amino)-5-methoxy-2-(1-methyl-1H-pyrazol-4-yl)phenyl)piperazin-1-yl)methyl)pyrrolidin-1-yl)-2-(2,6-dioxopiperidin-3-yl)isoindoline-1,3-dione (**compound 121**)

**55B** (0.26 g, 0.64 mmol) and **104G** (0.4 g, 0.64 mmol) were dissolved in DMF (5 mL); p-toluenesulfonic acid hydrate (0.24 g, 1.28 mmol) was added; and under nitrogen protection, the mixture was reacted at 100°C for 16 h. The reaction solution was cooled to room temperature. 10 mL of saturated aqueous sodium bicarbonate solution was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase: dichloromethane/methanol (V/V) = 50/1-15/1) to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.05% ammonia water)) to obtain **compound 121** (0.2 g, yield: 32%).

LCMS m/z = 495.8 $[(M+2H)/2]^+$.

$^1H$ NMR (400 MHz, DMSO-$d_6$) δ 11.33 - 10.89 (m, 1H), 10.84 (s, 1H), 8.26 - 8.10 (m, 3H), 7.99 (s, 1H), 7.90 (s, 1H), 7.65 (d, 1H), 7.58 (s, 1H), 7.23 (dd, 1H), 6.98 - 6.76 (m, 3H), 6.48 (s, 1H), 5.05 (dd, 1H), 3.84 (s, 3H), 3.80 (s, 3H), 3.64 - 3.36 (m, 3H), 3.25 - 3.14 (m, 1H), 2.99 - 2.80 (m, 5H), 2.75 - 2.51 (m, 7H), 2.48 - 2.37 (m, 2H), 2.22 - 2.08

(m, 1H), 2.07 - 1.93 (m, 1H), 1.88 - 1.67 (m, 8H), 0.98 - 0.78 (m, 2H), 0.62 - 0.37 (m, 2H).

**Example 122: Preparation of compound 122**

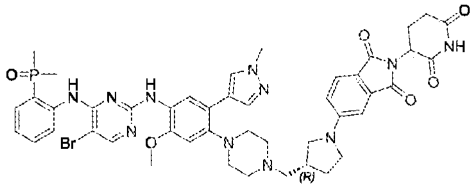

Compound 122

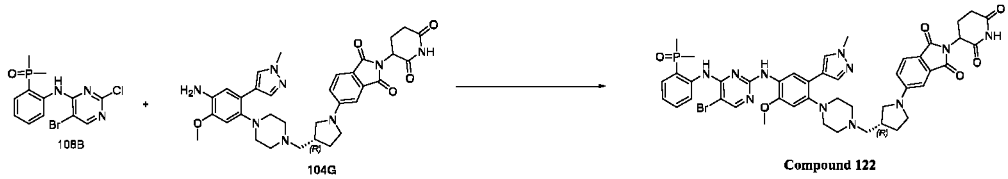


Step 1: 5-((R)-3-((4-(4-((5-bromo-4-((2-(dimethylphosphoryl)phenyl)amino) pyrimidin-2-yl)amino)-5-methoxy-2-(1-methyl-1H-pyrazol-4-yl)phenyl)piperazin-1-yl)methyl)pyrrolidin-1-yl)-2-(2,6-dioxopiperidin-3-yl)isoindoline-1,3-dione (**compound 122**)

**108B** (0.23 g, 0.64 mmol) and **104G** (0.4 g, 0.64 mmol) were dissolved in DMF (5 mL); p-toluenesulfonic acid hydrate (0.24 g, 1.28 mmol) was added; and under nitrogen protection, the mixture was reacted at 100°C for 16 h. The reaction solution was cooled to room temperature. 10 mL of saturated aqueous sodium bicarbonate solution was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase: dichloromethane/methanol (V/V) = 50/1-15/1) to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)) to obtain the trifluoroacetate of **compound 122**. 20 mL of dichloromethane and 20 mL of saturated sodium bicarbonate solution were added to the trifluoroacetate of **compound 122**, and the layers were separated. The organic phase was dried over anhydrous sodium sulphate and then concentrated under reduced pressure to obtain **compound 122** (0.18 g, yield: 30%).

LCMS m/z = 475.7 $[(M+2H)/2]^+$.

$^1$H NMR (400 MHz, DMSO-$d_6$) δ 11.04 (s, 1H), 10.94 (s, 1H), 8.42 - 8.28 (m, 1H), 8.25 - 8.10 (m, 2H), 7.96 (s, 1H), 7.85 (s, 1H), 7.65 (d, 1H), 7.61 (s, 1H), 7.56 - 7.38 (m, 1H), 7.15 - 6.87 (m, 3H), 6.87 - 6.67 (m, 2H), 5.05 (dd, 1H), 3.82 (s, 3H), 3.81 (s, 3H), 3.67 - 3.36 (m, 3H), 3.22 - 3.12 (m, 1H), 3.02 - 2.82 (m, 5H), 2.76 - 2.51 (m, 7H), 2.47 - 2.36 (m, 2H), 2.22 - 2.11 (m, 1H), 2.04 - 1.94 (m, 1H), 1.88 - 1.63 (m, 7H).

**Example 123: Preparation of compound 123**

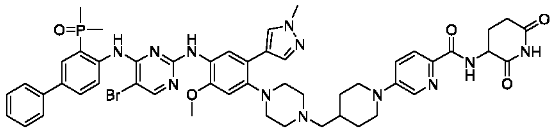

Compound 123

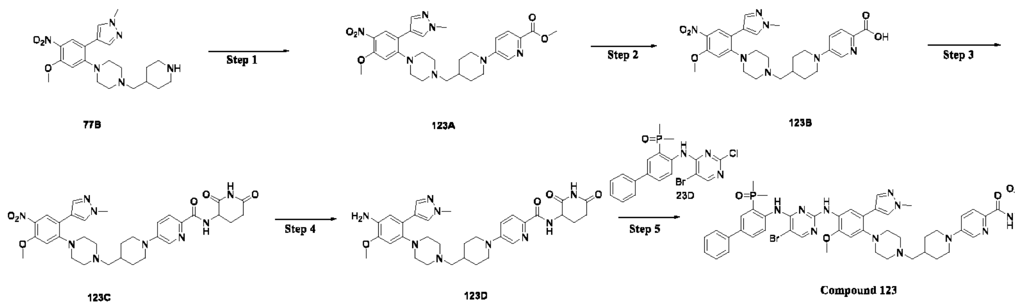


Step 1: methyl 5-(4-((4-(5-methoxy-2-(1-methyl-1H-pyrazol-4-yl)-4-nitrophenyl) piperazin-1-yl)methyl)piperidin-1-yl)picolinate (123A)

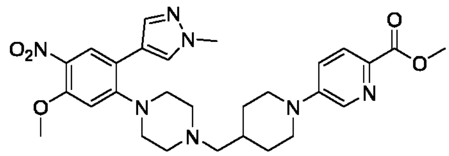

**123A**

The crude hydrochloride of **77B** (prepared from 4.43 mmol of **77A**) and methyl 5-fluoropyridine-2-carboxylate (1.5 g, 9.66 mmol) were dissolved in DMSO (30 mL); potassium carbonate (3.34 g, 24.15 mmol) was added; and the mixture was reacted at 100°C for 16 h. The reaction solution was cooled to room temperature. 100 mL of water was added, and the mixture was extracted by adding 100 mL of ethyl acetate. The organic layers were separated. The aqueous layer was extracted with ethyl acetate (3 × 30 mL). The organic phases were combined, washed sequentially with 50 mL of water and 50 mL of saturated brine, dried over anhydrous sodium sulphate and concentrated under reduced pressure, and the residue was purified by column chromatography (mobile phase: dichloromethane/methanol (V/V) = 100/1-19/1) to obtain **123A** (1.66 g, yield: 68%).

LCMS m/z = 550.3 $[M+H]^+$.

Step 2: 5-(4-((4-(5-methoxy-2-(1-methyl-1H-pyrazol-4-yl)-4-nitrophenyl) piperazin-1-yl)methyl)piperidin-1-yl)picolinic acid (**123B**)

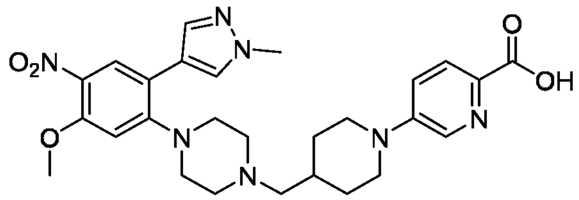

**123B**

**123A** (1.66 g, 3.02 mmol) was dissolved in a mixed solution of tetrahydrofuran (45 mL) and water (15 mL); lithium hydroxide monohydrate (0.44 g, 10.48 mmol) was added; and the mixture was reacted at room temperature for 16 h. After the reaction was completed, the reaction solution was adjusted to pH = 7 by adding dilute hydrochloric acid (2 mol/L) and concentrated under reduced pressure to obtain the crude of **123B**, which was directly used in the next step.

LCMS m/z = 536.3 $[M+H]^+$.

Step 3: *N*-(2,6-dioxopiperidin-3-yl)-5-(4-((4-(5-methoxy-2-(1-methyl-1H-pyrazol-4-yl)-4-nitrophenyl)piperazin-1-yl)methyl)piperidin-1-yl)picolinamide (**123C**)

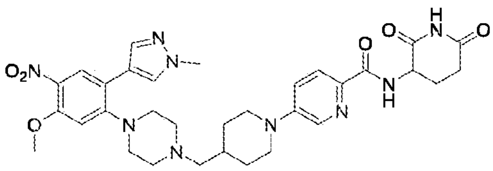

123C

The crude of **123B** from the previous step and 3-aminopiperidine-2,6-dione hydrochloride (0.75 g, 4.53 mmol) were dissolved in DMF (30 mL); DIPEA (1.95 g, 15.1 mmol) and HATU (1.72 g, 4.53 mmol) were added; and the mixture was reacted at room temperature for 3 h. After the reaction was completed, 100 mL of water was added, and the mixture was extracted by adding 100 ml of ethyl acetate. The organic layers were separated. The aqueous layer was extracted with ethyl acetate (3 × 30 mL). The organic phases were combined, washed sequentially with 50 mL of water and 50 mL of saturated brine, dried over anhydrous sodium sulphate and concentrated under reduced pressure, and the residue was purified by column chromatography (mobile phase: dichloromethane/methanol (V/V) = 100/1-19/1) to obtain **123C** (1.48 g, two-step yield: 76.4%).

LCMS m/z = 646.3 $[M+H]^+$.

Step 4: 5-(4-((4-(4-amino-5-methoxy-2-(1-methyl-1H-pyrazol-4-yl)phenyl) piperazin-1-yl)methyl)piperidin-1-yl)-N (2,6-dioxopiperidin-3-yl)picolinamide **(123D)**

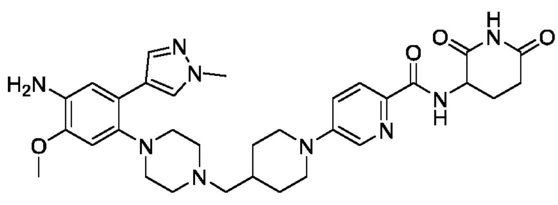

123D

**123C** (1.9 g, 2.94 mmol) was dissolved in ethanol (100 mL) and water (30 mL); iron powder (1.64 g, 29.4 mmol) was added, and ammonium chloride (1.57 g, 29.4 mmol) was then added; and under nitrogen protection, the mixture was reacted at 85°C for 2 h. The reaction solution was cooled to room temperature and subjected to suction filtration over celite. The filtrate was extracted with dichloromethane (3 × 50 ml). The organic phases were combined, dried over anhydrous sodium sulphate, filtered and concentrated under reduced pressure to obtain the crude of **123D** as a white solid (1.27 g), which was directly used in the next step.

LCMS m/z = 616.3 $[M+H]^+$.

Step 5: 5-(4-((4-(4-((5-bromo-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperazin-1-yl)methyl)piperidin-1-yl)-A-(2,6-dioxopiperidin-3 - yl)picolinamide (**compound 123**)

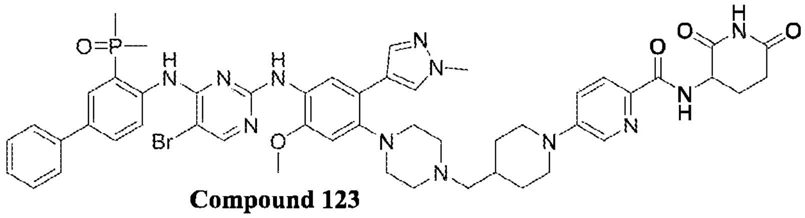

**Compound 123**

**123D** (265 mg) and **23D** (244 mg, 0.56 mmol) were dissolved in DMF (10 mL); p-toluenesulfonic acid monohydrate (245 mg, 1.29 mmol) was added; and under nitrogen protection, the mixture was reacted at 100°C for 16 h. The reaction solution was cooled to room temperature. 20 mL of saturated aqueous sodium bicarbonate solution was added,

and the mixture was extracted with dichloromethane, concentrated in vacuo, and purified by preparative HPLC (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). 50 mL of dichloromethane was added to the preparative solution. The mixture was extracted by adding saturated sodium bicarbonate solution (50 ml). Liquid separation was performed. The organic layer was then dried over anhydrous sodium sulphate and concentrated under reduced pressure to obtain **compound 123** (50 mg, yield: 8%).

LCMS m/z = 1015.3 $[M+H]^+$.

$^1H$ NMR (400 MHz, DMSO-$d_6$) δ 11.12 (s, 1H), 10.82 (s, 1H), 8.68 (d, 1H), 8.39 (s, 1H), 8.34 - 8.26 (m, 2H), 8.19 (s, 1H), 8.02 (s, 1H), 7.91 - 7.83 (m, 2H), 7.70 (dd, 1H), 7.62 - 7.51 (m, 3H), 7.49 - 7.33 (m, 4H), 7.17 (s, 1H), 6.86 (s, 1H), 4.81 - 4.64 (m, 1H), 3.95 (d, 2H), 3.80 (s, 3H), 3.76 (s, 3H), 2.97 - 2.70 (m, 7H), 2.59-2.49 (m, 5H), 2.32 - 2.12 (m, 3H), 2.01-1.97 (m, 1H), 1.88-1.76 (m, 9H), 1.31-1.16 (m, 2H).

**Example 124: Preparation of compound 124**

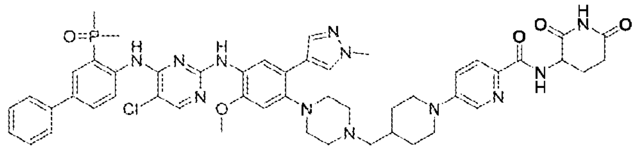

Compound 124

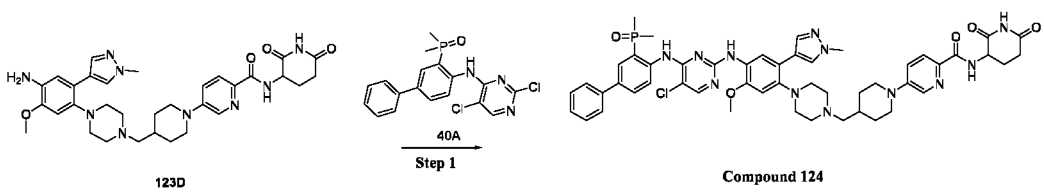


123D

Compound 124

Step 1: 5-(4-((4-(4-((5-chloro-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperazin-1-yl)methyl)piperidin-1-yl)-*N*-(2,6-dioxopiperidin-3-yl)picolinamide (**compound 124**)

**123D** (265 mg) and **40A** (219 mg, 0.56 mmol) were dissolved in DMF (10 mL); p-toluenesulfonic acid monohydrate (245 mg, 1.29 mmol) was added; and under nitrogen protection, the mixture was reacted at 100°C for 16 h. The reaction solution was cooled to room temperature. 20 mL of saturated aqueous sodium bicarbonate solution was added, and the mixture was extracted with dichloromethane, concentrated in vacuo, and purified by preparative HPLC (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). 50 mL of dichloromethane was added to the preparative solution. The mixture was extracted by adding saturated sodium bicarbonate solution (50 ml). Liquid separation was performed. The organic layer was then dried over anhydrous sodium sulphate and concentrated under reduced pressure to obtain **compound 124** (80 mg, yield: 13%).

LCMS m/z = 486.3 $[(M+2H)/2]^+$.

$^1H$ NMR (400 MHz, DMSO-$d_6$) δ 11.37 (s, 1H), 10.83 (s, 1H), 8.68 (d, 1H), 8.49 (s, 1H), 8.35 - 8.25 (m, 2H), 8.12 (s, 1H), 8.04 (s, 1H), 7.93 - 7.82 (m, 2H), 7.71 (dd,, 1H), 7.61 (s, 1H), 7.56 (d, 2H), 7.50 - 7.34 (m, 4H), 7.16 (s, 1H), 6.87 (s, 1H), 4.84 - 4.68 (m, 1H), 3.95 (d, 2H), 3.81 (s, 3H), 3.76 (s, 3H), 3.00 - 2.71 (m, 7H), 2.59 - 2.50 (m, 5H), 2.31 - 2.10 (m, 3H), 2.05-2.00 (m, 1H), 1.92-1.72 (m, 9H), 1.31-1.14 (m, 2H).

**Example 125: Preparation of compound 125**

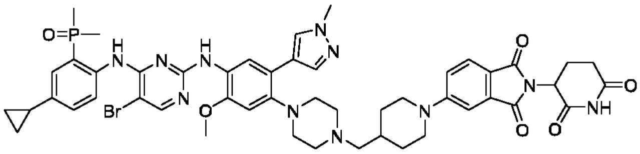

Compound 125

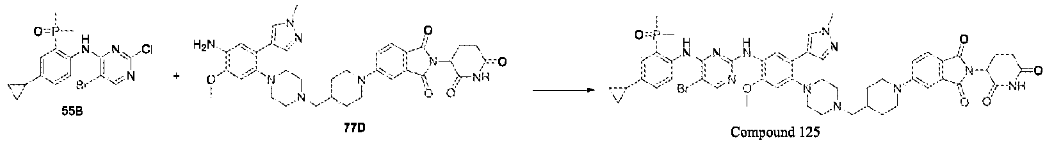

55B + 77D → Compound 125

Step 1: 5-(4-((4-(4-((5-bromo-4-((4-cyclopropyl-2-(dimethylphosphoryl) phenyl)amino)pyrimidin-2-yl)amino)-5-methoxy-2-(1-methyl-1H-pyrazol-4-yl)phenyl)piperazin-1-yl)methyl)piperidin-1-yl)-2-(2,6-dioxopiperidin-3-yl)isoindoline-1,3-dione (**compound 125**)

**77D** (210 mg, 0.33 mmol) and **55B** (158 mg, 0.40 mmol) were dissolved in DMF (5 mL); p-toluenesulfonic acid monohydrate (188 mg, 0.99 mmol) was added; and under nitrogen protection, the mixture was stirred at 100°C for 16 h, and cooled to room temperature. 10 mL of saturated aqueous sodium bicarbonate solution and 20 mL of dichloromethane were added, and the layers were separated. The organic layer was concentrated under reduced pressure. The concentrated solution was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 40 mL of dichloromethane. The mixture was extracted by adding saturated sodium bicarbonate solution (20 mL). Liquid separation was performed. The organic layer was then dried over anhydrous sodium sulphate and concentrated under reduced pressure to obtain **compound 125** (100 mg, yield: 30%).

LCMS m/z = 1004.3 $[M+H]^+$.

$^1H$ NMR (400 MHz, DMSO-$d_6$) δ 11.05 (s, 1H), 10.84 (s, 1H), 8.25 - 8.09 (m, 3H), 7.98 (s, 1H), 7.89 (s, 1H), 7.65 (d, 1H), 7.59 (s, 1H), 7.36 - 7.28 (m, 1H), 7.27 - 7.14 (m, 2H), 6.83 (s, 1H), 6.59 - 6.28 (m, 1H), 5.06 (dd, 1H), 4.11 - 3.97 (m, 2H), 3.83 (s, 3H), 3.79 (s, 3H), 3.05 - 2.78 (m, 7H), 2.64 - 2.51 (m, 6H), 2.25 (d, 2H), 2.06 - 1.95 (m, 1H), 1.95 - 1.79 (m, 4H), 1.74 (d, 6H), 1.30 - 1.08 (m, 2H), 0.95 - 0.83 (m, 2H), 0.56 - 0.45 (m, 2H).

**Example 126: Preparation of compound 126**

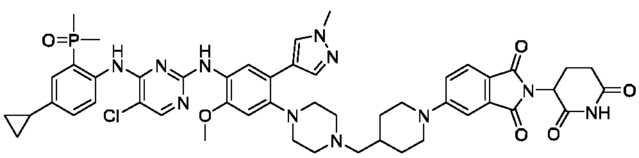

Compound 126

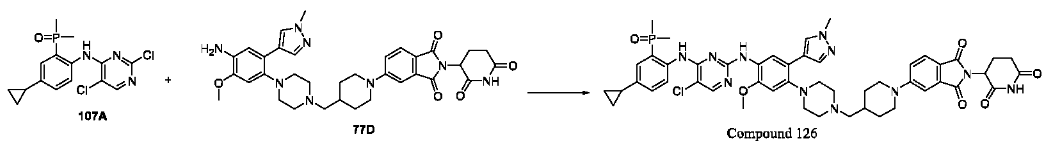

107A + 77D → Compound 126

Step 1: 5-(4-((4-(4-((5-chloro-4-((4-cyclopropyl-2-(dimethylphosphoryl) phenyl)amino)pyrimidin-2-yl)amino)-5-methoxy-2-(1-methyl-1H-pyrazol-4-yl)phenyl)piperazin-1-yl)methyl)piperidin-1-yl)-2-(2,6-dioxopiperidin-3-yl)isoindoline-1,3-dione (**compound 126**)

**77D** (250 mg, 0.39 mmol) and **107A** (170 mg, 0.47 mmol) were dissolved in DMF (5 mL); p-toluenesulfonic acid monohydrate (223 mg, 1.17 mmol) was added; and under nitrogen protection, the mixture was stirred at 100°C for

16 h, and cooled to room temperature. 10 mL of saturated aqueous sodium bicarbonate solution and 20 mL of dichloromethane were added, and the layers were separated. The organic layer was concentrated under reduced pressure. The concentrated solution was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The resultant was lyophilized. The resulting solid was dissolved in 40 mL of dichloromethane. The mixture was extracted by adding saturated sodium bicarbonate solution (20 mL). Liquid separation was performed. The organic layer was then dried over anhydrous sodium sulphate and concentrated under reduced pressure to obtain **compound 126** (80 mg, yield: 21%).

LCMS m/z = 480.8 [(M+2H)/2]$^+$.

$^1$H NMR (400 MHz, DMSO-$d_6$) δ 11.30 - 10.80 (m, 2H), 8.42 - 7.94 (m, 4H), 7.89 (s, 1H), 7.72 - 7.53 (m, 2H), 7.42 - 7.14 (m, 3H), 6.83 (s, 1H), 6.56 - 6.38 (m, 1H), 5.05 (dd, 1H), 4.13 - 3.96 (m, 2H), 3.83 (s, 3H), 3.80 (s, 3H), 3.05 - 2.78 (m, 7H), 2.64 - 2.51 (m, 6H), 2.25 (d, 2H), 2.06 - 1.95 (m, 1H), 1.93 - 1.78 (m, 4H), 1.75 (d, 6H), 1.29 - 1.08 (m, 2H), 1.00 - 0.79 (m, 2H), 0.59 - 0.38 (m, 2H).

**Example 127: Preparation of compound 127**

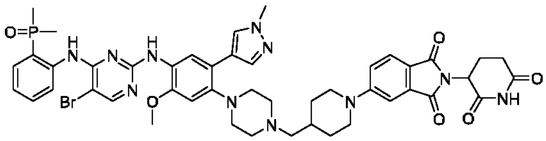

Compound 127

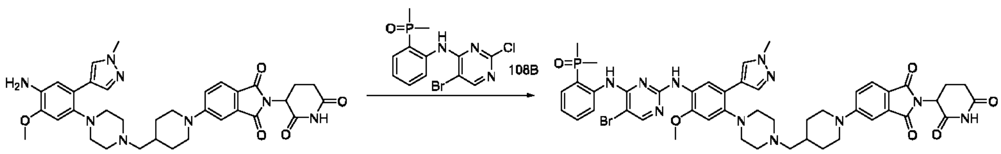


77D

Compound 127

Step 1: 5-(4-((4-(4-((5-bromo-4-((2-(dimethylphosphoryl)phenyl)amino) pyrimidin-2-yl)amino)-5-methoxy-2-(1-methyl-1H-pyrazol-4-yl)phenyl)piperazin-1-yl)methyl)piperidin-1-yl)-2-(2,6-dioxopiperidin-3-yl)isoindoline-1,3-dione (**compound 127**)

**77D** (600 mg, 0.94 mmol) and **108B** (372.83 mg, 1.03 mmol) were dissolved in DMF (5 mL); p-toluenesulfonic acid monohydrate (536.42 mg, 2.82 mmol) was added; and under nitrogen protection, the mixture was reacted overnight at 100°C, and cooled to room temperature. 20 mL of water and 20 mL of saturated aqueous sodium bicarbonate solution were added, and the mixture was subjected to suction filtration. The filter cake was dissolved in dichloromethane and extracted. The organic phase was dried over anhydrous sodium sulphate and then concentrated under reduced pressure, and the residue was purified by silica gel column chromatography (dichloromethane/methanol (V/V) = 100/1-10/1) to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)) to obtain the trifluoroacetate of **compound 127**. 60 mL of dichloromethane and 30 mL of saturated sodium bicarbonate solution were added to the trifluoroacetate of **compound 127**, and the layers were separated. The organic phase was dried over anhydrous sodium sulphate and then concentrated under reduced pressure to obtain **compound 127** (260 mg, yield: 28.26%).

LCMS m/z = 964.3 [M+H]$^+$.

$^1$H NMR (400 MHz, DMSO-$d_6$) δ 11.05 (s, 1H), 10.94 (s, 1H), 8.40 - 8.27 (m, 1H), 8.22 - 8.14 (m, 2H), 7.95 (s, 1H), 7.84 (s, 1H), 7.65 (d, 1H), 7.60 (s, 1H), 7.55 - 7.41 (m, 1H), 7.36 - 7.27 (m, 1H), 7.23 (dd, 1H), 7.04 - 6.90 (m, 2H), 6.82 (s, 1H), 5.06 (dd, 1H), 4.10 - 3.97 (m, 2H), 3.82 (s, 3H), 3.80 (s, 3H), 3.07 - 2.79 (m, 7H), 2.68 - 2.50 (m, 6H), 2.28 - 2.17 (m, 2H), 2.09 - 1.97 (m, 1H), 1.92 - 1.79 (m, 3H), 1.76 (d, 6H), 1.27 - 1.08 (m, 2H).

**Example 128: Preparation of compound 128**

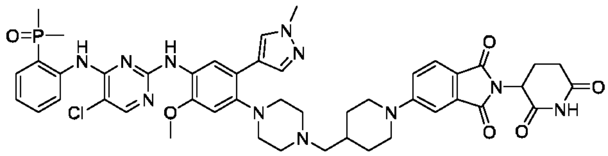

**Compound 128**

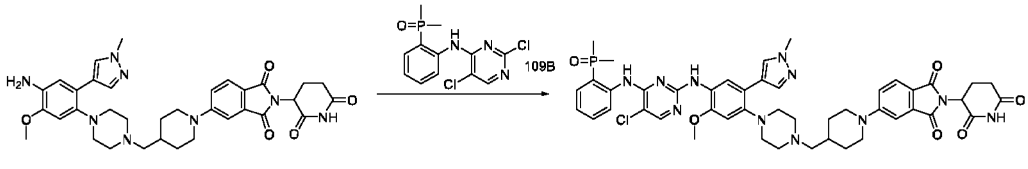


77D

Compound 128

Step 1: 5-(4-((4-(4-((5-chloro-4-((2-(dimethylphosphoryl)phenyl)amino) pyrimidin-2-yl)amino)-5-methoxy-2-(1-methyl-1H-pyrazol-4-yl)phenyl)piperazin-1-yl)methyl)piperidin-1-yl)-2-(2,6-dioxopiperidin-3-yl)isoindoline-1,3-dione (**compound 128**)

**77D** (600 mg, 0.94 mmol) and **109B** (329.84 mg, 1.04 mmol) were dissolved in DMF (5 mL); p-toluenesulfonic acid monohydrate (536.42 mg, 2.82 mmol) was added; and under nitrogen protection, the mixture was reacted overnight at 100°C, and cooled to room temperature. 20 mL of water and 20 mL of saturated aqueous sodium bicarbonate solution were added, and the mixture was subjected to suction filtration. The filter cake was dissolved in dichloromethane and extracted. The organic phase was dried over anhydrous sodium sulphate and then concentrated under reduced pressure, and the residue was purified by silica gel column chromatography (dichloromethane/methanol (V/V) = 100/1-10/1) to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)) to obtain the trifluoroacetate of **compound 128**. 60 mL of dichloromethane and 30 mL of saturated sodium bicarbonate solution were added to the trifluoroacetate of **compound 128**, and the layers were separated. The organic phase was dried over anhydrous sodium sulphate and then concentrated under reduced pressure to obtain **compound 128** (200 mg, yield: 23.12%).

LCMS m/z = 920.3 $[M+H]^+$.

$^1H$ NMR (400 MHz, DMSO-$d_6$) δ 11.21 (s, 1H), 11.05 (s, 1H), 8.58 - 8.34 (m, 1H), 8.19 (s, 1H), 8.10 (s, 1H), 7.97 (s, 1H), 7.85 (s, 1H), 7.65 (d, 1H), 7.62 (s, 1H), 7.57 - 7.40 (m, 1H), 7.31 (s, 1H), 7.28 - 7.14 (m, 1H), 7.07 - 6.90 (m, 2H), 6.83 (s, 1H), 5.06 (dd, 1H), 4.23 - 3.93 (m, 2H), 3.82 (s, 3H), 3.81 (s, 3H), 3.05 - 2.78 (m, 7H), 2.67 - 2.50 (m, 6H), 2.24 (d, 2H), 2.08 - 1.94 (m, 1H), 1.93 - 1.80 (m, 3H), 1.76 (d, 6H), 1.29 - 1.08 (m, 2H).

**Example 129: Preparation of compound 129**

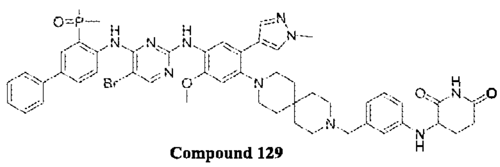

**Compound 129**

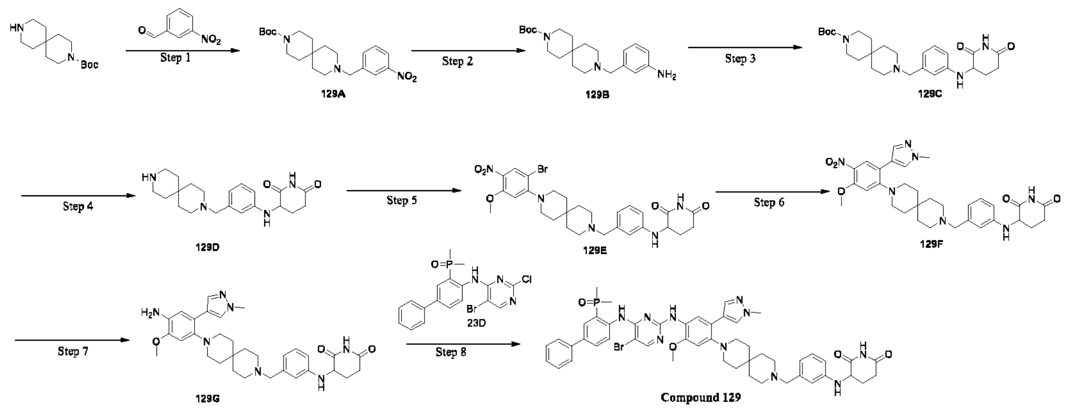


Step 1: tert-butyl 9-(3-nitrobenzyl)-3,9-diazaspiro[5.5]undecane-3-carboxylate (129A)

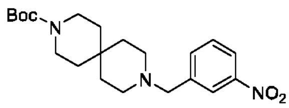

**129A**

Tert-butyl 3,9-diazaspiro[5.5]undecane-3-carboxylate (3.0 g, 11.8 mmol) and 3-nitrobenzaldehyde (1.3 g, 8.6 mmol) were dissolved in DMAC (30 mL); 1 ml of acetic acid was added; and the mixture was stirred at room temperature for 1 h. Sodium triacetoxyborohydride (2.1 g, 10 mmol) was added, and the mixture was reacted overnight at room temperature, adjusted to a basic pH by adding saturated aqueous sodium bicarbonate solution and extracted with dichloromethane (50 mL × 3). The organic phases were combined, washed with water (100 mL × 2), dried over anhydrous sodium sulphate and concentrated under reduced pressure, and the residue was purified by silica gel column chromatography (mobile phase: ethyl acetate/petroleum ether (V/V) = 1/1 to dichloromethane/methanol (V/V) = 20/1) to obtain **129A** (3.5 g, yield: 76%).

LCMS m/z = 390.3$[M+H]^+$.

Step 2: tert-butyl 9-(3-aminobenzyl)-3,9-diazaspiro[5.5]undecane-3-carboxylate **(129B)**

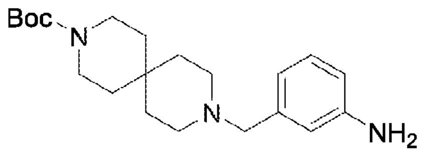

**129B**

**129A** (3.5 g, 9.0 mmol), iron powder (5 g, 89.3 mmol) and ammonium chloride (5 g, 93.5 mmol) were dissolved in ethanol (60 mL) and water (20 mL); and the mixture was stirred at 80°C for 2 h, cooled to room temperature and subjected to suction filtration. The filtrate was concentrated. 60 mL of water was added, and then the mixture was extracted with 60 ml of dichloromethane. The organic phase was dried over anhydrous sodium sulphate and then concentrated under reduced pressure to obtain **129B** (3.0 g, yield: 93%), which was directly used in the next step.

LCMS m/z = 360.3 $[(M+H]^+$.

Step 3: tert-butyl 9-(3-((2,6-dioxopiperidin-3-yl)amino)benzyl)-3,9-diazaspiro [5.5]undecane-3-carboxylate (**129C**)

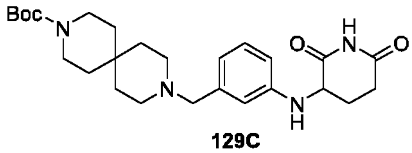

129C

129B (3.0 g, 8.36 mmol) and 3-bromopiperidine-2,6-dione (3.96 g, 20.6 mmol) were dissolved in DMSO (40 mL); sodium bicarbonate (4.0 g, 47.6 mmol) was added; and the mixture was reacted at 100°C for 6 h, and cooled to room temperature and extracted by adding 100 mL of ethyl acetate and 100 mL of water. The organic layer was washed with saturated brine (30 mL × 3), dried over anhydrous sodium sulphate and concentrated under reduced pressure, and the residue was separated and purified by silica gel column chromatography (ethyl acetate: petroleum ether (V/V) = 10/1-2/1) to obtain **129C** (2.4 g, yield: 61%).
LCMS m/z = 471.3[M+H]$^+$.

Step 4: 3-((3-((3,9-diazaspiro[5.5]undecan-3-yl)methyl)phenyl)amino)piperidine-2,6-dione (**129D**); HCl

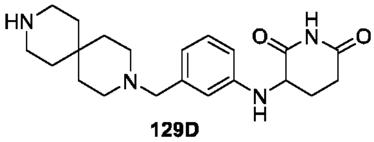

129D

To a 50 mL round bottom flask were successively added 129C (1.0 g, 2.1 mmol) and a solution of hydrogen chloride in 1,4-dioxane (30 mL, 4 mol/L); and the mixture was reacted at room temperature for 2 h and concentrated to dryness under reduced pressure to obtain the hydrochloride of 129D, which was directly used in the next reaction.

Step 5: 3-((3-((9-(2-bromo-5-methoxy-4-nitrophenyl)-3,9-diazaspiro[5.5] undecan-3-yl)methyl)phenyl)amino)piperidine-2,6-dione (129E)

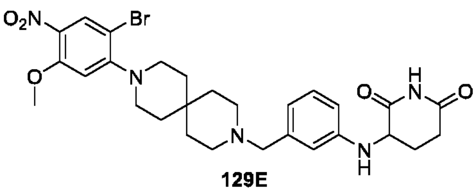

129E

The compound 1-bromo-2-fluoro-4-methoxy-5-nitrobenzene **(1C)** (0.6 g, 2.40 mmol), the hydrochloride of **129D** from the previous step and sodium bicarbonate (1.0 g, 11.9 mmol) were dissolved in 20 mL of DMSO; and the mixture was reacted at 100°C for 3 h. After the reaction was completed, the mixture was cooled to room temperature. 50 mL of water and 50 mL of ethyl acetate were added. The ethyl acetate layer was separated. The aqueous layer was extracted with ethyl acetate (50 mL × 3). The organic phases were combined, washed with saturated brine (30 mL), dried over anhydrous sodium sulphate and concentrated under reduced pressure, and the residue was purified by silica gel column chromatography (mobile phase: ethyl acetate/petroleum ether (V/V) = 1/1 to dichloromethane/methanol (V/V) = 20/1) to obtain **129E** (0.8 g, two-step yield: 63%).
LCMS m/z = 600.2 [M+H]$^+$.

Step 6: 3-((3-((9-(5-methoxy-2-(1 -methyl- 1H-pyrazol-4-yl)-4-nitrophenyl)-3,9-diazaspiro[5.5]undecan-3-yl)methyl)phenyl)amino)piperidine-2,6-dione (**129F**)

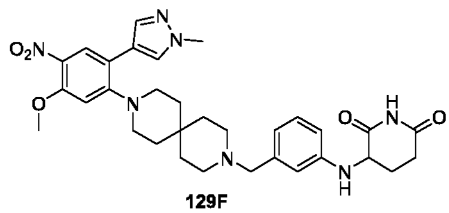

129F

Under nitrogen protection, **129E** (400 mg, 0.67 mmol) and 1-methyl-1H-pyrazole-4-boronic acid (95.7 mg, 0.76 mmol) were added to a 50 mL single-necked flask and dissolved in 20 mL of dioxane and 4 mL of water, and then Pd(dppf)$Cl_2$·DCM (31 mg, 0.04 mmol) and sodium bicarbonate (96 mg, 1.14 mmol) were added. The mixture was subjected to nitrogen replacement three times, reacted at 100°C for 3 h and cooled to room temperature. The reaction solution was poured into water and extracted three times with ethyl acetate. The organic phases were combined, dried over anhydrous sodium sulphate and concentrated under reduced pressure, and the residue was purified by silica gel column chromatography (mobile phase: ethyl acetate/petroleum ether (V/V) = 1/1 to dichloromethane/methanol (V/V) = 20/1) to obtain **129F** (180 mg, yield: 45%).

LCMS m/z = 602.3 $[M+H]^+$.

Step 7: 3-((3-((9-(4-amino-5-methoxy-2-(1-methyl-1H-pyrazol-4-yl)phenyl)-3,9-diazaspiro[5.5]undecan-3-yl)methyl)phenyl)amino)piperidine-2,6-dione (**129G**)

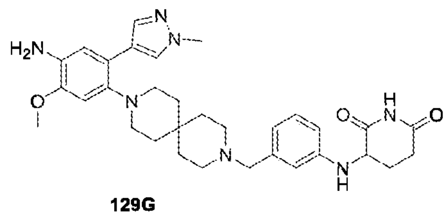

129G

129F (180 mg, 0.30 mmol) was dissolved in methanol (10 mL); palladium on carbon 10% (180 mg) was added at room temperature; and the mixture was reacted under hydrogen atmosphere for 2 h. After the reaction was completed, the mixture was subjected to suction filtration over celite. The filtrate was concentrated to obtain **129G** (140 mg, yield: 82%).

LCMS m/z = 572.3 $[M+H]^+$.

Step 8: 3-((3-((9-(4-((5-bromo-4-((3-(dimethylphosphoryl)-[1,1'-biphenyl] -4-yl)amino)pyrimidin-2-yl)amino)-5-methoxy-2-(1-methyl-1H-pyrazol-4-yl)phenyl)-3,9-diazaspiro[5.5]undecan-3-yl)methyl)phenyl)amino)piperidine-2,6-dione (**compound 129**)

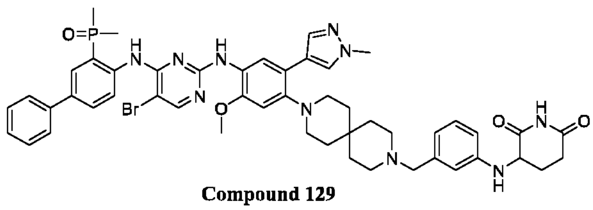

Compound 129

**129G** (140 mg, 0.25 mmol) from the previous step and 23D (140 mg, 0.32 mmol) were dissolved in DMF (10 mL); p-toluenesulfonic acid monohydrate (114 mg, 0.6 mmol) was added; and the mixture was stirred at 100°C for 16 h, and cooled to room temperature. 20 mL of saturated aqueous sodium bicarbonate solution and 50 mL of dichloromethane were added, and the layers were separated. The organic layer was concentrated under reduced pressure, and the residue was purified by silica gel column chromatography (mobile phase: DCM/MeOH (V/V) = 100/1-20/1). The crude was further subjected to preparative HPLC (instrument: waters 2767 (preparative liquid phase chromatographic instrument); chromatographic column: XBridge@ Prep C18 (30 mm × 150 mm); composition of mobile phases: mobile phase

A: acetonitrile, and mobile phase B: water (containing 0.1% TFA)) purification to obtain a preparative solution, which was concentrated. 50 mL of DCM was added, and then the mixture was adjusted to a basic pH by adding saturated sodium bicarbonate solution. The dichloromethane layers were separated, and the aqueous layer was extracted with dichloromethane (2 × 30 mL). The dichloromethane layers were combined, dried over anhydrous sodium sulphate, filtered and concentrated to obtain **compound 129** (30 mg, yield: 13%).

LCMS m/z = 486.3 [(M+2H)/2]$^+$.

$^1$H NMR (400 MHz, DMSO-$d_6$) δ 11.12 (s, 1H), 10.75 (s, 1H), 8.48 - 8.35 (m, 1H), 8.30 (s, 1H), 8.18 (s, 1H), 8.01 (s, 1H), 7.86 (s, 1H), 7.68 (dd, 1H), 7.59 - 7.47 (m, 3H), 7.42 (t, 2H), 7.33 (t, 1H), 7.25 - 7.10 (m, 1H), 7.03 (t, 1H), 6.88 (s, 1H), 6.65 (s, 1H), 6.60 - 6.43 (m, 2H), 5.78 (d, 1H), 4.37 - 4.25 (m, 1H), 3.79 (s, 3H), 3.75 (s, 3H), 3.39 - 3.32 (m, 2H), 2.86 - 2.70 (m, 5H), 2.69 - 2.54 (m, 1H), 2.41 - 2.22 (m, 4H), 2.17 - 2.04 (m, 1H), 1.98 - 1.78 (m, 7H), 1.62 - 1.37 (m, 8H).

**Example 130: Preparation of compound 130**

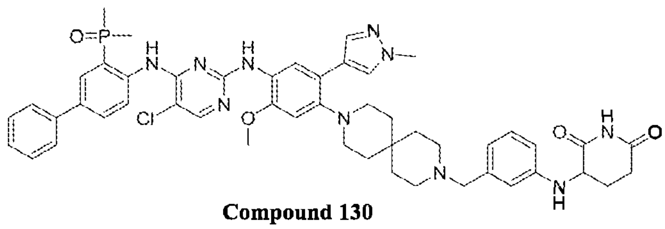

**Compound 130**

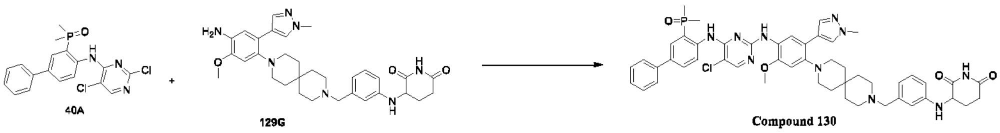

40A

129G

Compound 130

Step 1: 3-((3-((9-(4-((5-chloro-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)-3,9-diazaspiro[5.5]undecan-3-yl)methyl)phenyl)amino)piperidine-2,6-dione (**compound 130**)

**129G** (140 mg, 0.25 mmol) and **40A** (140 mg, 0.74 mmol) were dissolved in DMF (10 mL); p-toluenesulfonic acid monohydrate (140 mg, 0.81 mmol) was added; and the mixture was stirred at 100°C for 16 h, and cooled to room temperature. 20 mL of saturated aqueous sodium bicarbonate solution and 50 mL of dichloromethane were added, and the layers were separated. The organic layer was concentrated under reduced pressure, and the residue was purified by silica gel column chromatography (mobile phase: DCM/MeOH (V/V) = 100/1-20/1). The crude was further subjected to preparative HPLC (instrument: waters 2767 (preparative liquid phase chromatographic instrument); chromatographic column: XBridge@ Prep C18 (30 mm × 150 mm); composition of mobile phases: mobile phase A: acetonitrile, and mobile phase B: water (containing 0.1% TFA)) purification to obtain a preparative solution, which was concentrated. 50 mL of DCM was added, and then the mixture was adjusted to a basic pH by adding saturated sodium bicarbonate solution. The dichloromethane layers were separated, and the aqueous layer was extracted with dichloromethane (2 × 30 mL). The dichloromethane layers were combined, dried over anhydrous sodium sulphate, filtered and concentrated to obtain **compound 130** (20 mg, yield: 8.8%).

LCMS m/z = 464.3 [(M+2H)/2]$^+$.

$^1$H NMR (400 MHz, DMSO-$d_6$) δ 11.37 (s, 1H), 10.75 (s, 1H), 8.52 - 8.43 (m, 1H), 8.30 (s, 1H), 8.10 (s, 1H), 8.03 (s, 1H), 7.87 (s, 1H), 7.69 (dd, 1H), 7.60 -7.48 (m, 3H), 7.42 (t, 2H), 7.33 (t, 1H), 7.23 - 7.12 (m, 1H), 7.03 (t, 1H), 6.89 (s, 1H), 6.65 (s, 1H), 6.61 - 6.45 (m, 2H), 5.78 (d, 1H), 4.36 - 4.25 (s, 1H), 3.79 (s, 3H), 3.75 (s, 3H), 3.40 - 3.30 (m, 2H), 2.88 - 2.69 (m, 6H), 2.65 - 2.54 (m, 1H), 2.42 - 2.25 (m, 4H), 2.17 - 2.07 (m, 1H), 1.96 - 1.77 (m, 7H), 1.62 - 1.42 (s, 7H).

Example 131: Preparation of compound 131

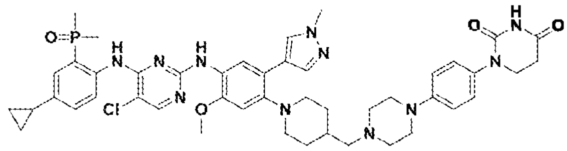

Compound 131

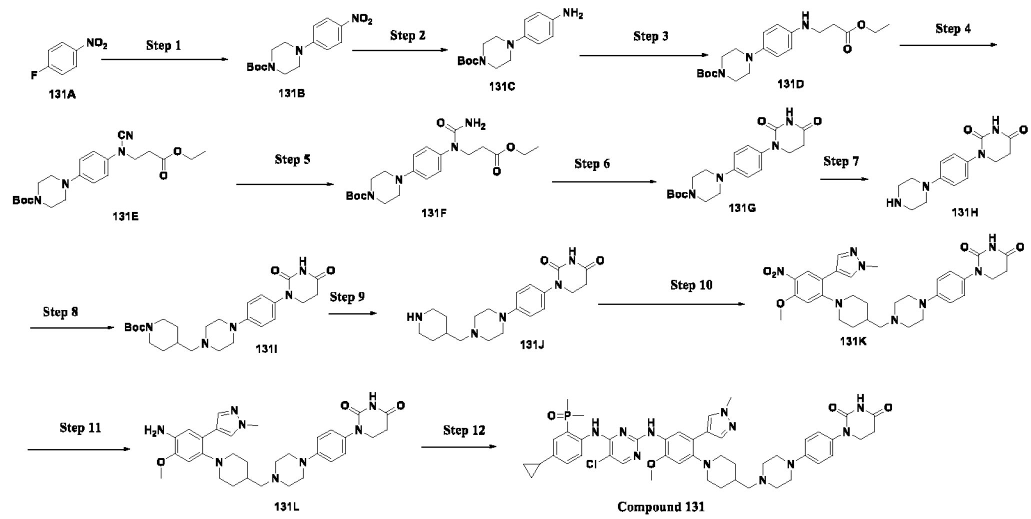


Step 1: tert-butyl 4-(4-nitrophenyl)piperazine-1-carboxylate (131B)

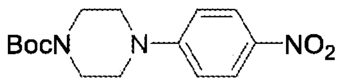

**131B**

131A (14.1 g, 10 mmol) and 1-tert-butoxycarbonylpiperazine (18.6 g, 10 mmol) were dissolved in DMF (100 mL); potassium carbonate (34.5 g, 25 mmol) was added; and the mixture was reacted at 50°C for 3 h. The reaction solution was cooled to room temperature. 100 mL of water was added, with a yellow solid precipitated, and the mixture was subjected to suction filtration. The filter cake was slurried with petroleum ether (300 mL), subjected to suction filtration and concentrated under reduced pressure to obtain **131B** (29.2 g, yield: 95%).

LCMS m/z = 252.2[M-55]$^+$.

Step 2: tert-butyl 4-(4-aminophenyl)piperazine-1-carboxylate **(131C)**

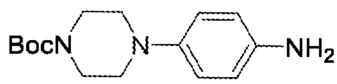

**131C**

**131B** (11.9 g, 38.8 mmol) and palladium on carbon (1.2 g, 10 wt%) were dissolved in 60 mL of methanol and 60 mL of tetrahydrofuran, and then the mixture was subjected to hydrogen replacement three times, reacted at room temperature for 8 h and subjected to suction filtration over celite. The filtrate was concentrated under reduced pressure to obtain **131C** (10.7 g, yield: 100%).

LCMS m/z = 278.2[M+H]$^+$.

Step 3: tert-butyl 4-(4-((3-ethoxy-3-oxopropyl)amino)phenyl)piperazine-1-carboxylate **(131D)**

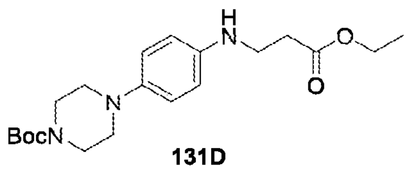

**131D**

Lactic acid (7.2 g, 80 mmol) was added dropwise to a solution of 1,8-diazabicyclo[5.4.0]undec-7-ene (12.2 g, 80 mmol) in diethyl ether (100 ml), and the mixture was stirred overnight at room temperature, concentrated under reduced pressure and then dissolved in 1,4-dioxane (200 mL). **131C** (10.7 g, 38.6 mmol) and ethyl acrylate (8 g, 80 mmol) were successively added, and the mixture was reacted at 90°C for 24 h. After the reaction was completed, the mixture was cooled to room temperature and extracted with 300 mL of ethyl acetate and 200 mL of water. The organic phase was dried over anhydrous sodium sulphate and then concentrated under reduced pressure, and the residue was purified by silica gel column chromatography (mobile phase: petroleum ether/ethyl acetate (V/V) = 20/1-4/1) to obtain **131D** (7.3 g, yield: 50%).

LCMS m/z = 378.3 $[M+H]^+$.

$^1H$ NMR (400 MHz, $CDCl_3$) $\delta$ **6.91** - 6.71 (m, 2H), 6.69 - 6.39 (m, 2H), 4.08 (q, 2H), 3.61 - 3.45 (m, 4H), 3.43 - 3.24 (m, 2H), 3.08 - 2.80 (m, 4H), 2.57 - 2.48 (m, 2H), 1.41 (s, 9H), 1.19 (t, 3H).

Step 4: tert-butyl4-(4-(N-(3-ethoxy-3-oxopropyl)cyanamido)phenyl)piperazine-1-carboxylate **(131E)**

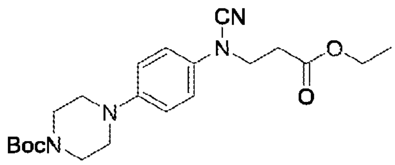

**131E**

**131D** (7.3 g, 19.4 mmol) and cyanogen bromide (4.24 g, 40 mmol) were dissolved in tetrahydrofuran (100 mL); sodium bicarbonate (10.08 g, 120 mmol) was added; and under nitrogen protection, the mixture was reacted at room temperature for 3 h. After the reaction was completed, the mixture was extracted with 200 mL of ethyl acetate and 100 mL of water. The organic phase was dried over anhydrous sodium sulphate and then concentrated under reduced pressure, and the residue was purified by silica gel column chromatography (mobile phase: dichloromethane/methanol (V/V) = 100/1-10/1) to obtain **131E** (6.5 g, yield: 83.5%).

LCMS m/z = 403.3$[M+H]^+$.

Step 5: tert-butyl 4-(4-(1-(3-ethoxy-3-oxopropyl)ureido)phenyl)piperazine-1-carboxylate **(131F)**

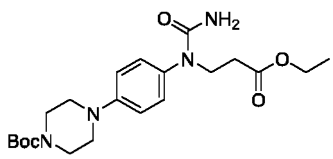

**131F**

**131E** (6.5 g, 16.2 mmol) was dissolved in toluene (100 mL); acetaldoxime (2.95 g, 50 mmol) and indium (III) chloride tetrahydrate (1.46 g, 5 mmol) were successively added; and the mixture was reacted at 130°C for 3 h, and cooled to room temperature. 100 mL of ethyl acetate and 100 mL of saturated aqueous sodium bicarbonate solution were added, and then the layers were separated. The organic layer was dried under reduced pressure, and the residue

was purified by silica gel column chromatography (mobile phase: dichloromethane/methanol (V/V) = 50/1-10/1) to obtain **131F** (5.6 g, 82.5%).

LCMS m/z = 421.2[M+H]$^+$.

Step 6: tert-butyl 4-(4-(2,4-dioxotetrahydropyrimidin-1(2H)-yl)phenyl) piperazine-1-carboxylate **(131G)**

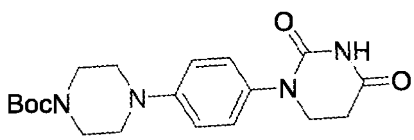

**131G**

**131F** (5.6 g, 13.3 mmol) was dissolved in acetonitrile (50 mL); benzyltrimethylammonium hydroxide (40% in methanol) (8.3 g, 20 mmol) was added; and under nitrogen protection, the mixture was reacted at 90°C for 15 min. After the reaction was completed, the mixture was cooled to room temperature and extracted with 150 mL of ethyl acetate and 60 mL of water. The organic phase was dried over anhydrous sodium sulphate and then concentrated under reduced pressure, and the residue was purified by silica gel column chromatography (mobile phase: dichloromethane/methanol (V/V) = 100/1-10/1) to obtain **131G** (3.7 g, yield: 74%).

LCMS m/z = 375.2[M+H]$^+$.

$^1$H NMR (400 MHz, $CDCl_3$) δ **7.64 (s, 1H), 7.11 (d, 2H), 6.86 (d, 2H), 3.74 (t,** 2H), 3.63 - 3.45 (m, 4H), 3.16 - 3.02 (m, 4H), 2.74 (t, 2H), 1.41 (s, 9H).

Step 7: 1-(4-(piperazin-1-yl)phenyl)dihydropyrimidine-2,4(1H,3H)-dione **(131H)**

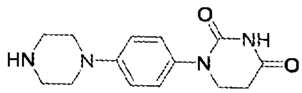

**131H**

**131G** (3.7 g, 9.9 mmol) was dissolved in DCM (40 mL); trifluoroacetic acid (10 mL) was added at room temperature; and the mixture was stirred for 3 h and concentrated under reduced pressure. 20 mL of dichloromethane was added to the residue, and the mixture was adjusted to pH = 7-8 with 1 N aqueous NaOH solution and concentrated under reduced pressure to obtain **131H** (2.7 g), which was directly used in the next step.

Step 8: tert-butyl 4-((4-(4-(2,4-dioxotetrahydropyrimidin-1(2H)-yl)phenyl) piperazin-1-yl)methyl)piperidine-1-carboxylate **(131I)**

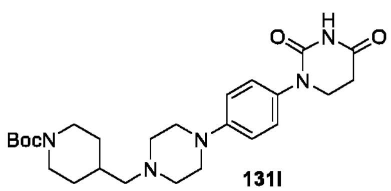

**131I**

**131H** (1.37 g, 5 mmol) and 1-tert-butoxycarbonylpiperidine-4-carbaldehyde were mixed in dichloromethane (20 mL) and 2 ml of acetic acid, and the mixture was stirred at room temperature for 10 min. Sodium triacetoxyborohydride (2.12 g, 10 mmol) was added, and the mixture was stirred at room temperature for 0.5 h. 100 mL of dichloromethane was added. The mixture was adjusted to pH = 7-8 with 1 N aqueous NaOH solution, and then the layers were separated. The organic layer was dried under reduced pressure, and the residue was purified by silica gel column chromatography (mobile phase: dichloromethane/methanol (V/V) = 20/1-10/1) to obtain **131I** (1.3 g, yield: 55%).

LCMS m/z = 472.1 $[M+H]^+$.

Step 9: 1-(4-(4-(piperidin-4-ylmethyl)piperazin-1-yl)phenyl)dihydropyrimidine-2,4(1H,3H)-dione **(131J);** Trifluoroacetate

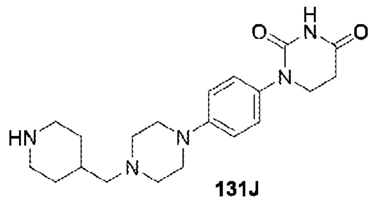

**131J**

**131I** (1.3 g, 2.76 mmol) was dissolved in dichloromethane (10 mL); trifluoroacetic acid (3 mL) was added; and the mixture was stirred at room temperature for 3 h and concentrated under reduced pressure, and the residue was directly used in the next step.

Step 10: 1-(4-(4-((1-(5-methoxy-2-(1-methyl-1H-pyrazol-4-yl)-4-nitrophenyl) piperidin-4-yl)methyl)piperazin-1-yl)phenyl)dihydropyrimidine-2,4(1H,3H)-dione **(131K)**

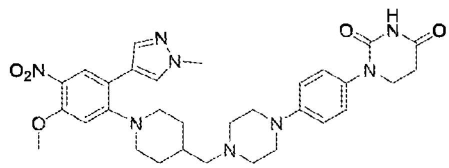

**131K**

The crude trifluoroacetate of **131J** from the previous step was dissolved in DMSO (25 mL); 4-(2-fluoro-4-methoxy-5-nitrophenyl)-1-methyl-1H-pyrazole **(1D)** (828 mg, 3.3 mmol) and sodium bicarbonate (2.77 g, 33 mmol) were successively added; and the mixture was stirred at 100°C for 12 h, and cooled to room temperature. 10 mL of water was added, and the mixture was filtered. The filter cake was dried under reduced pressure and then purified by silica gel column chromatography (mobile phase: dichloromethane/methanol (V/V) = 100/1-10/1) to obtain **131K** (1.1 g, yield: 66%).
LCMS m/z = 603.3 $[M+H]^+$.

Step 11: 1-(4-(4-((1-(4-amino-5-methoxy-2-(1-methyl-1H-pyrazol-4-yl) phenyl)piperidin-4-yl)methyl)piperazin-1-yl)phenyl)dihydropyrimidine-2,4(1H,3H)-dione **(131L)**

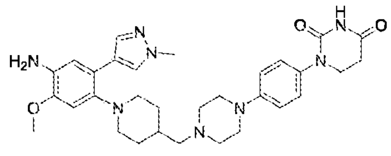

**131L**

**131K** (1.1 g, 1.83 mmol) was dissolved in ethanol/water (20 mL, 3:1); iron powder (530 mg, 9.5 mmol) and ammonium chloride (500 mg, 9.3 mmol) were successively added; and under nitrogen protection, the mixture was stirred at 80°C for 1 h, cooled to room temperature and subjected to suction filtration under reduced pressure. The filtrate was concentrated under reduced pressure, and then the crude was purified by silica gel column chromatography (mobile phase: dichloromethane/methanol (V/V) = 100/1-10/1) to obtain **131L** as a yellow solid (860 mg, yield: 82.3%).
LCMS m/z = 573.4 $[M+H]^+$.

Step 12: 1-(4-(4-((1-(4-((5-chloro-4-((4-cyclopropyl-2-(dimethylphosphoryl) phenyl)amino)pyrimidin-2-yl)amino)-5-methoxy-2-(1-methyl-1H-pyrazol-4-yl)phenyl)piperidin-4-yl)methyl)piperazin-1-yl)phenyl)dihydropyrimidine-2,4(1H,3H)-dione **(compound 131)**

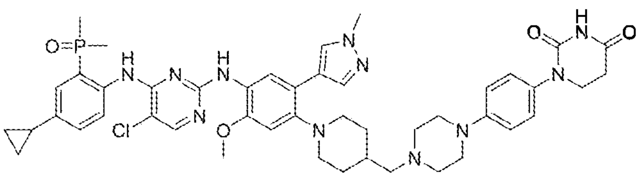

**Compound 131**

**131L** (200 mg, 0.35 mmol) and **107A** (178 mg, 0.5 mmol) were dissolved in DMF (10 mL); p-toluenesulfonic acid monohydrate (228 mg, 1.2 mmol) was added; and under nitrogen protection, the mixture was reacted overnight at 100°C, and cooled to room temperature. 20 mL of saturated aqueous sodium bicarbonate solution was added, and the mixture was subjected to suction filtration. The filter cake was dissolved in 50 mL of dichloromethane and extracted. The organic phase was dried over anhydrous sodium sulphate and then concentrated under reduced pressure, and the residue was purified by silica gel column chromatography (dichloromethane/methanol (V/V) = 100/1-10/1) to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)) to obtain the trifluoroacetate of **compound 131.** 20 mL of dichloromethane and 50 mL of saturated sodium bicarbonate solution were added to the trifluoroacetate of **compound 131,** and the layers were separated. The organic phase was dried over anhydrous sodium sulphate and then concentrated under reduced pressure to obtain **compound 131** (80 mg, yield: 25.6%).

LCMS m/z = 446.7 $[(M+2H)/2]^+$.

$^1H$ NMR (400 MHz, $D_2O/CF_3COOD$(v/v = 1:1)) δ 8.15 (s, 1H), 8.00 - 7.89 (m, 3H), 7.86 (s, 1H), 7.66 - 7.45 (m, 4H), 7.34 (s, 1H), 7.20 (d, 1H), 6.88 (d, 1H), 4.19 - 3.98 (m, 10H), 3.98 - 3.69 (m, 10H), 3.44 - 3.23 (m, 2H), 2.97 - 2.84 (m, 2H), 2.62 - 2.45 (m, 1H), 2.37 - 2.19 (m, 2H), 2.12 - 1.98 (m, 2H), 1.97 - 1.81 (m, 7H), 1.15 - 1.00 (m, 2H), 0.70 - 0.53 (m, 2H).

**Example 132: Preparation of compound 132**

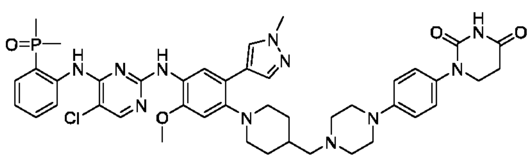

**Compound 132**

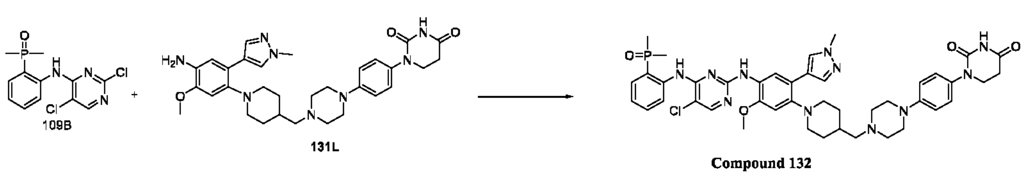

109B

131L

Compound 132

Step 1: 1-(4-(4-((1-(4-((5-chloro-4-((2-(dimethylphosphoryl)phenyl)amino) pyrimidin-2-yl)amino)-5-methoxy-2-(1-methyl-1H-pyrazol-4-yl)phenyl)piperidin-4-yl)methyl)piperazin-1-yl)phenyl)dihydropyrimidine-2,4(1H,3H)-dione **(compound 132)**

**131L** (200 mg, 0.35 mmol) and **109B** (158 mg, 0.5 mmol) were dissolved in DMF (10 mL); p-toluenesulfonic acid monohydrate (228 mg, 1.2 mmol) was added; and under nitrogen protection, the mixture was reacted overnight at

100°C, and cooled to room temperature. 20 mL of saturated aqueous sodium bicarbonate solution was added, and the mixture was subjected to suction filtration. The filter cake was dissolved in 50 mL of dichloromethane and extracted. The organic phase was dried over anhydrous sodium sulphate and then concentrated under reduced pressure, and the residue was purified by silica gel column chromatography (dichloromethane/methanol (V/V) = 100/1-10/1) to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)) to obtain the trifluoroacetate of **compound 132.** 20 mL of dichloromethane and 50 mL of saturated sodium bicarbonate solution were added to the trifluoroacetate of **compound 132,** and the layers were separated. The organic phase was dried over anhydrous sodium sulphate and then concentrated under reduced pressure to obtain **compound 132** (60 mg, yield: 20.2%).

LCMS m/z = 426.8 $[(M+2H)/2]^+$.

$^1H$ NMR (400 MHz, $D_2O/CF_3COOD$(v/v = **1:1))** δ **8.20 (s, 1H), 8.00 -** 7.91 (m, 2H), 7.88 - 7.78 (m, 2H), 7.66 - 7.47 (m, 5H), 7.42 - 7.25 (m, 2H), 7.18 (t, 1H), 4.18 (s, 3H), 4.13 - 4.03 (m, 7H), 3.98 - 3.77 (m, 10H), 3.43 - 3.31 (m, 2H), 2.93 (t, 2H), 2.66 - 2.46 (m, 1H), 2.38 - 2.21 (m, 2H), 2.15 - 1.99 (m, 2H), 1.94 (d, 6H).

**Example 133: Preparation of compound 133**

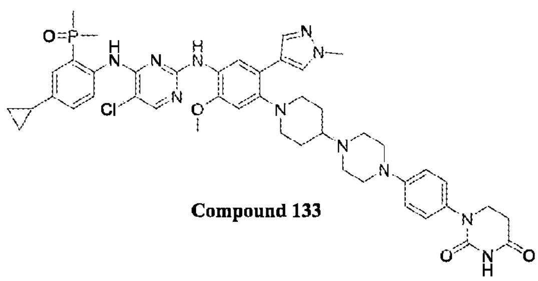

Compound 133

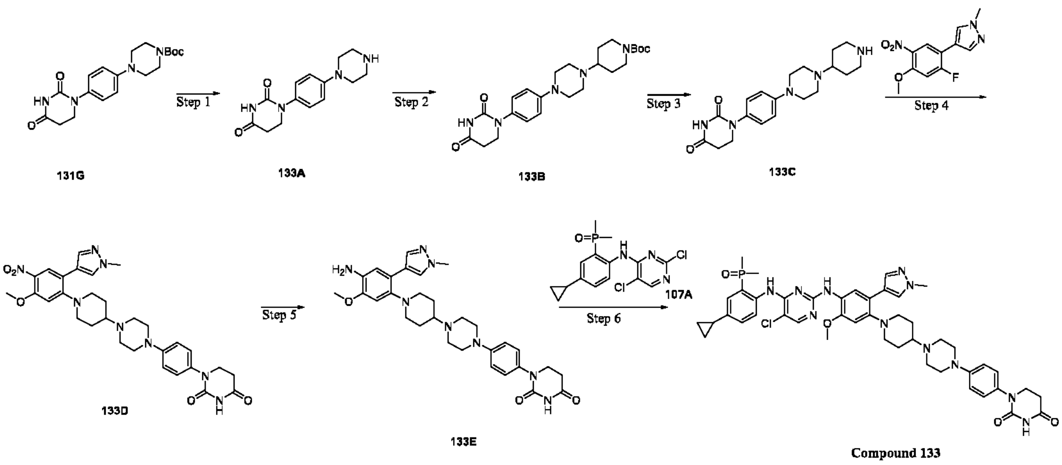


131G 133A 133B 133C 133D 133E 107A Compound 133

Step 1: 1-(4-(piperazin-1-yl)phenyl)dihydropyrimidine-2,4(1H,3H)-dione **(133A)**

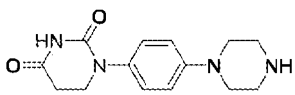

133A

**131G** (2.4 g, 6.41 mmol) was dissolved in DCM (20 mL); trifluoroacetic acid (20 mL) was added at room temperature; and the mixture was stirred for 3 h and concentrated under reduced pressure. 20 mL of dichloromethane was added to the residue, and the mixture was adjusted to pH = 9-10 with 1 N aqueous NaOH solution. Liquid separation was performed. The organic layer was concentrated under reduced pressure to obtain the crude of **133A** (1.5 g).

Step 2: tert-butyl 4-(4-(4-(2,4-dioxotetrahydropyrimidin-1(2H)-yl)phenyl) piperazin-1-yl)piperidine-1-carboxylate **(133B)**

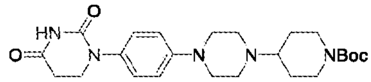

133B

**133A** (1.5 g) and tert-butyl 4-oxopiperidine-1-carboxylate (1.3 g, 6.56 mmol) were dissolved in dichloromethane (20 mL); acetic acid (394 mg, 6.56 mmol) and sodium triacetoxyborohydride (1.7 g, 8.21 mmol) were successively added; and the mixture was reacted overnight at room temperature, adjusted to a basic pH by adding 1 N aqueous sodium hydroxide solution and extracted 3 times with dichloromethane. The organic phases were combined, dried over anhydrous sodium sulphate and concentrated under reduced pressure, and the residue was purified by silica gel column chromatography (mobile phase: ethyl acetate/petroleum ether (V/V) = 1/1 to dichloromethane/methanol (V/V) = 20/1) to obtain **133B** (1.2 g, yield: 48%).

LCMS m/z = 458.3[M+H]$^+$.

Step 3: 1-(4-(4-(piperidin-4-yl)piperazin-1-yl)phenyl)dihydropyrimidine-2,4 (1H,3H)-dione **(133C);** trifluoroacetic acid

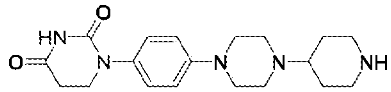

133C

**133B** (1.2 g, 2.62 mmol) was dissolved in DCM (15 mL); trifluoroacetic acid (15 mL) was added at room temperature; and the mixture was stirred at room temperature for 1 h and concentrated under reduced pressure, and the residue was directly used in the next step.

Step 4: 1-(4-(4-(1-(5-methoxy-2-(1-methyl-1H-pyrazol-4-yl)-4-nitrophenyl) piperidin-4-yl)piperazin-1-yl)phenyl)dihydropyrimidine-2,4(1H,3H)-dione **(133D)**

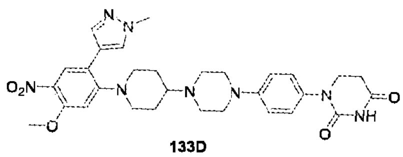

133D

The crude trifluoroacetate of **133C** from the previous step was dissolved in DMSO (10 mL); 4-(2-fluoro-4-methoxy-5-nitrophenyl)-1-methyl-1H-pyrazole **(1D)** (942 mg, 3.75 mmol) and sodium bicarbonate (1.7 g, 20 mmol) were successively added; and the mixture was stirred at 100°C for 7 h, and cooled to room temperature. 10 mL of water was added, and the mixture was filtered. The filter cake was dried under reduced pressure and then purified by silica gel column chromatography (mobile phase: dichloromethane/methanol (V/V) = 100/1-20/1) to obtain **133D** as a yellow solid (0.3 g, yield: 20%).

LCMS m/z = 589.3 [M+H]$^+$.

Step 5: 1-(4-(4-(1-(4-amino-5-methoxy-2-(1-methyl-1H-pyrazol-4-yl)phenyl) piperidin-4-yl)piperazin-1-yl)phenyl)dihydropyrimidine-2,4(1H,3H)-dione **(133E)**

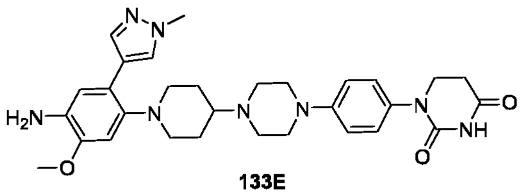

133E

**133D** (0.3 g, 0.51 mmol) was dissolved in ethanol/water (8 mL, 3:1); iron powder (142 mg, 2.55 mmol) and ammonium chloride (136 mg, 2.55 mmol) were successively added; and under nitrogen protection, the mixture was stirred at 80°C for 1 h, cooled to room temperature and subjected to suction filtration under reduced pressure. The filtrate was concentrated under reduced pressure, and then the crude was purified by silica gel column chromatography (mobile phase: dichloromethane/methanol (V/V) = 100/1-20/1) to obtain **133E** (220 mg, yield: 77%).

Step 6: 1-(4-(4-(1-(4-((5-chloro-4-((4-cyclopropyl-2-(dimethylphosphoryl) phenyl)amino)pyrimidin-2-yl)amino)-5-methoxy-2-(1-methyl-1H-pyrazol-4-yl)phenyl)piperidin-4-yl)piperazin-1-yl)phenyl)dihydropyrimidine-2,4(1H,3H)-dione **(compound 133)**

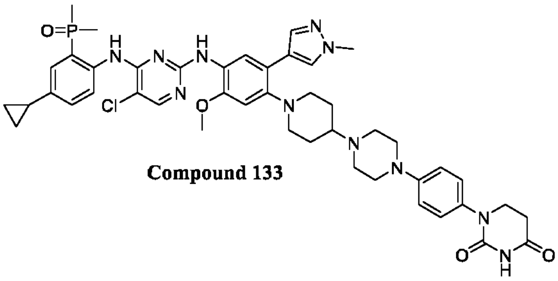

Compound 133

**133E** (90 mg, 0.16 mmol) and **107A** (76 mg, 0.21 mmol) were dissolved in DMF (4 mL); p-toluenesulfonic acid monohydrate (61 mg, 0.32 mmol) was added; and under nitrogen protection, the mixture was reacted overnight at 100°C, cooled to room temperature and extracted by adding 20 mL of saturated aqueous sodium bicarbonate solution and 20 mL of dichloromethane. The organic layer was concentrated under reduced pressure, and the residue was purified by silica gel column chromatography (mobile phase: dichloromethane/methanol (V/V) = 100/1-20/1). The residue was further subjected to preparative HPLC (instrument: waters 2767 (preparative liquid phase chromatographic instrument); chromatographic column: XBridge@ Prep C18 (30 mm × 150 mm); composition of mobile phases: mobile phase A: acetonitrile, and mobile phase B: water (containing 0.1% TFA)) purification to obtain the trifluoroacetate of compound **133.** The resulting solid was added to saturated aqueous sodium bicarbonate solution (20 mL). The mixture was extracted with dichloromethane (40 mL × 3). The organic phases were combined, dried over anhydrous sodium sulphate and concentrated under reduced pressure to obtain compound **133** (15 mg, white solid, yield: 11%).

LCMS m/z = 439.8 $[(M+2H)/2]^+$.

$^1$H NMR(400 MHz, $D_2O/CF_3COOD$(v/v = **1:1))**δ **8.24 (s, 1H), 8.05 (s, 1H),** 7.99 (s, 1H), 7.95 (dd, 1H), 7.64 - 7.54 (m, 3H), 7.52 - 7.45 (m, 2H), 7.17 (d, 1H), 6.98 (s, 1H), 6.79 - 6.71 (m, 1H), 4.14 (s, 3H), 4.10 - 4.00 (m, 4H), 3.97 - 3.81 (m, 9H), 3.62 - 3.52 (m, 1H), 3.49 - 3.37 (m, 2H), 3.14 - 3.00 (m, 2H), 2.93 - 2.83 (m, 2H), 2.45 - 2.32 (m, 2H), 2.19-2.05 (m, 2H), 1.93 - 1.78 (m, 7H), 1.08 - 0.97 (m, 2H), 0.55 - 0.46 (m, 2H).

**Example 134: Preparation of compound 134**

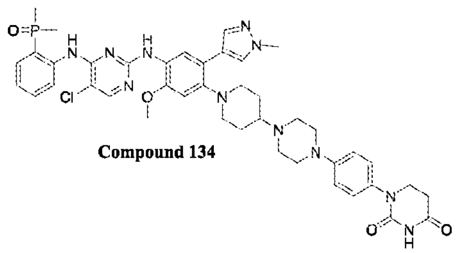

Compound 134

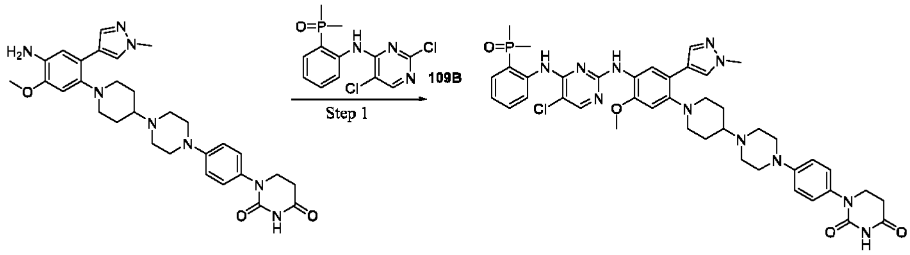


133E

Compound 134

Step 1: 1-(4-(4-(1-(4-((5-chloro-4-((2-(dimethylphosphoryl)phenyl)amino) pyrimidin-2-yl)amino)-5-methoxy-2-(1-methyl-1H-pyrazol-4-yl)phenyl)piperidin-4-yl)piperazin-1-yl)phenyl) dihydropyrimidine-2,4(1H,3H)-dione **(compound 134)**

**133E** (90 mg, 0.16 mmol) and **109B** (76 mg, 0.24 mmol) were dissolved in DMF (4 mL); p-toluenesulfonic acid monohydrate (61 mg, 0.32 mmol) was added; and under nitrogen protection, the mixture was reacted at 100°C overnight, cooled to room temperature and extracted by adding 20 mL of saturated aqueous sodium bicarbonate solution and 20 mL of dichloromethane. The organic layer was concentrated under reduced pressure, and the residue was purified by silica gel column chromatography (mobile phase: dichloromethane/methanol (V/V) = 100/1-20/1). The residue was further subjected to preparative HPLC (instrument: waters 2767 (preparative liquid phase chromatographic instrument); chromatographic column: XBridge@ Prep C18 (30 mm × 150 mm); composition of mobile phases: mobile phase A: acetonitrile, and mobile phase B: water (containing 0.1% TFA)) purification to obtain the trifluoroacetate of compound **134.** The resulting solid was added to saturated aqueous sodium bicarbonate solution (20 mL). The mixture was extracted with dichloromethane (40 mL × 3). The organic phases were combined, dried over anhydrous sodium sulphate and concentrated under reduced pressure to obtain compound **134** (31 mg, white solid, yield: 23%).

LCMS m/z = 419.8 [(M+H)/2]$^+$.

$^1$H NMR(400 MHz, = **1:1))** δ **8.23 -** 8.19 (m, 1H), 8.13 - 8.05 (m, 3H), 7.73 - 7.67 (m, 1H), 7.67 - 7.58 (m, 2H), 7.59 - 7.47 (m, 3H), 7.33 - 7.25 (m, 1H), 7.21 - 7.12 (m, 1H), 7.08 (s, 1H), 4.19 (s, 3H), 4.16 - 4.04 (m,4H), 4.03 - 3.88 (m, 7H), 3.74 - 3.63 (m, 1H), 3.59 - 3.49 (m, 1H), 3.30 - 3.17 (m, 1H), 3.04 - 2.87 (m, 4H), 2.52 - 2.38 (m, 1H), 2.30 - 2.13 (m, 1H), 1.95 (s, 3H), 1.91 (s, 3H), 1.89 - 1.76 (m, 2H), 0.68 - 0.58 (m, 2H).

**Example 135: Preparation of compound 135**

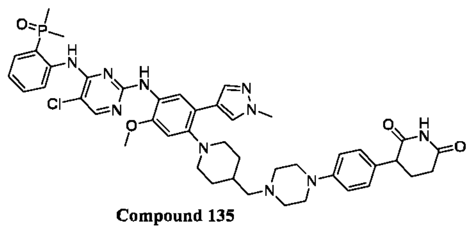

Compound 135

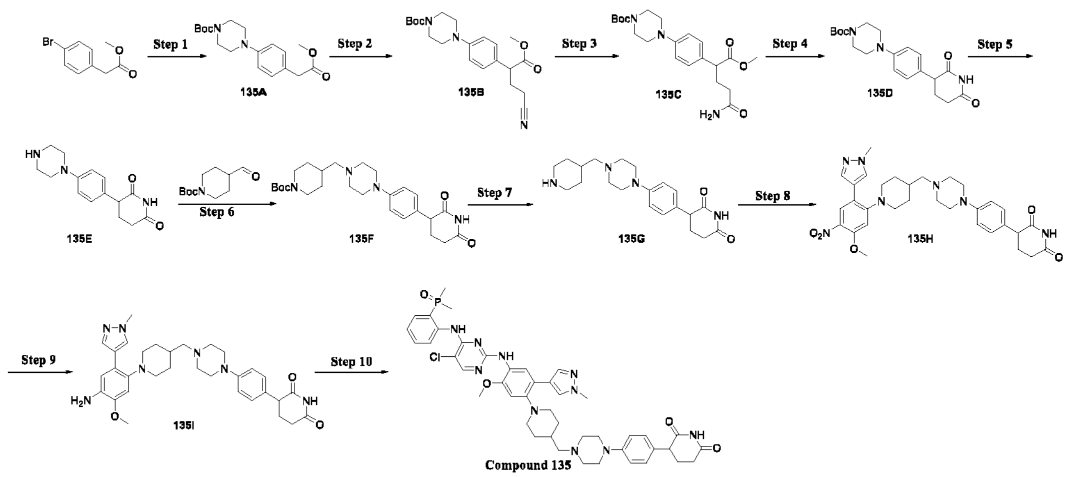


Step 1: tert-butyl 4-(4-(2-methoxy-2-oxoethyl)phenyl)piperazine-1-carboxylate **(135A)**

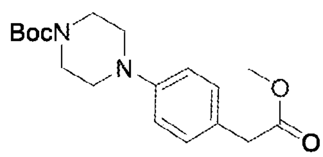

**135A**

Under nitrogen protection, methyl 2-(4-bromophenyl)acetate (10.0 g, 43.65 mmol) and tert-butylpiperazine-1-carboxylate (12.2 g, 65.47 mmol) were added to a 500 mL single-necked flask and dissolved in 300 mL of 1,4-dioxane, and then S-(-)-1,1'-binaphthalene-2,2'-bisdiphenylphosphine (2.72 g, 4.37 mmol), palladium acetate (0.98 g, 4.37 mmol) and caesium carbonate (35.56 g, 109.13 mmol) were added. The mixture was subjected to nitrogen replacement three times, reacted at 100°C for 16 h, and cooled to room temperature. The reaction solution was filtered, and the filtrate was concentrated. The residue was dissolved in ethyl acetate, and water was added. The mixture was extracted three times with ethyl acetate. The organic phases were combined, dried over anhydrous sodium sulphate and concentrated under reduced pressure, and the residue was purified by silica gel column chromatography to obtain **135A** (9.5 g, yield: 65%).
LCMS m/z = 335.1 $[M+H]^+$.

Step 2: tert-butyl 4-(4-(4-cyano-1-methoxy-1-oxobutan-2-yl)phenyl) piperazine-1-carboxylate **(135B)**

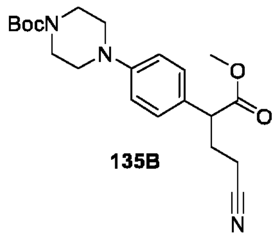

**135B**

**Compound 135A** (8.0 g, 23.95 mmol) was dissolved in toluene (80 mL); allyl cyanide (1.93 g, 28.7 mmol) and a solution of benzyltrimethylammonium hydroxide in methanol (wt% = 40%, 501 mg, 1.2 mmol) were added; and the mixture was stirred overnight at room temperature. The mixture was extracted by adding 50 mL of water and 100 mL of ethyl acetate. Liquid separation was performed. The organic phases were collected and concentrated under reduced pressure. The residue was purified by column chromatography (mobile phase: petroleum ether/ethyl acetate (V/V) = 6/1-3/1) to obtain **135B** (2.2 g, yield: 24%).
LCMS m/z = 388.2 $[M+H]^+$.

Step 3: tert-butyl 4-(4-(5-amino-1-methoxy-1,5-dioxopentan-2-yl)phenyl) piperazine-1-carboxylate **(135C)**

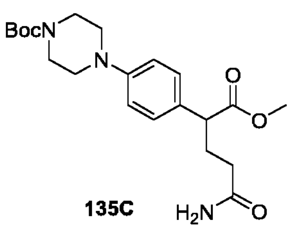

**135C**

**135B** (2.2 g, 5.68 mmol) was dissolved in toluene (20 mL); acetaldoxime (1.0 g, 17.0 mmol) and indium chloride tetrahydrate (832 mg, 2.84 mmol) were added; and the mixture was stirred at 130°C for 3 h, cooled to room temperature, and then extracted by adding 50 mL of ethyl acetate and 20 mL of water. Liquid separation was performed. The organic phases were collected, washed by adding saturated sodium chloride solution, dried over anhydrous sodium sulphate and filtered. The filtrate was concentrated under reduced pressure to obtain **135C** (1.0 g, yield: 43%).
LCMS m/z = 406.3 $[M+H]^+$.

Step 4: tert-butyl 4-(4-(2,6-dioxopiperidin-3-yl)phenyl)piperazine-1-carboxylate **(135D)**

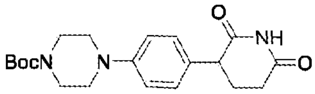

**135D**

**135C** (1.0 g, 2.47 mmol) was dissolved in acetonitrile (20 mL), and the mixture was warmed to 60°C. A solution of benzyltrimethylammonium hydroxide in methanol (wt% = 40%, 2.06 g, 4.94 mmol) was added dropwise to the reaction solution, and the mixture was stirred for 20 min, cooled to room temperature and extracted by adding 20 mL of water and 50 mL of dichloromethane. The organic layer was collected and washed by adding 20 mL of water. The organic phase was dried over anhydrous sodium sulphate and filtered. The filtrate was concentrated under reduced pressure, and the residue was purified by column chromatography (mobile phase: dichloromethane/methanol (V/V) = 100/1-25/1) to obtain **135D** (420 mg, yield: 46%).
LCMS m/z = 374.3 $[M+H]^+$.

Step 5: 3-(4-(piperazin-1-yl)phenyl)piperidine-2,6-dione **(135E);** HCl

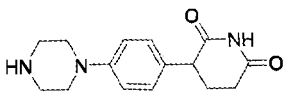

**135E**

**135D** (400 mg, 1.07 mmol) was dissolved in 1 mL of methanol; 4 *N* hydrochloric acid/dioxane solution (3 mL) was added; and the mixture was stirred at room temperature for 1 h. The reaction solution was concentrated under reduced pressure to obtain the crude hydrochloride of **135E,** which was directly used in the next step.

Step 6: tert-butyl 4-((4-(4-(2,6-dioxopiperidin-3-yl)phenyl)piperazin-1-yl)methyl)piperidine-1-carboxylate **(135F)**

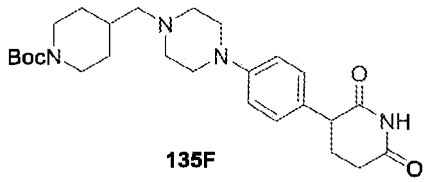

135F

The crude hydrochloride of **135E** was dissolved in 10 mL of methanol; a solution of sodium bicarbonate (180 mg, 2.14 mmol) in water (0.2 mL) was added; and the mixture was stirred at room temperature for 10 min, concentrated under reduced pressure to remove water, and dissolved in a mixed solution of dichloromethane (20 mL) and acetic acid (2 mL). 1-tert-butoxycarbonylpiperidine-4-carbaldehyde (227 mg, 1.07 mmol) was added, and the mixture was stirred at room temperature for 1 h. Sodium triacetylborohydride (677 mg, 3.21 mmol) was added, and the mixture was stirred at room temperature for another 2 h. 20 mL of dichloromethane and 5 mL of 1 N aqueous sodium hydroxide solution were added, and then the layers were separated. The organic layer was collected and dried under reduced pressure, and the residue was purified by silica gel column chromatography (mobile phase: dichloromethane/methanol (V/V) = 100/1-20/1) to obtain **135F** (300 mg, yield: 60%).

LCMS m/z = 471.2 $[M+H]^+$.

Step 7: 3-(4-(4-(piperidin-4-ylmethyl)piperazin-1-yl)phenyl)piperidine-2,6-dione (135G); HCl

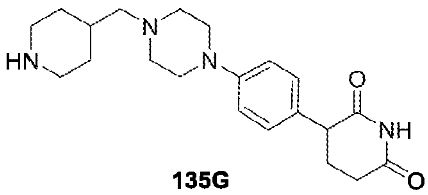

135G

**135F** (300 mg, 0.64 mmol) was dissolved in 0.5 mL of methanol; 4 N hydrochloric acid/dioxane solution (3 mL) was added; and the mixture was stirred at room temperature for 1 h. The reaction solution was concentrated under reduced pressure to obtain the crude hydrochloride of **135G,** which was directly used in the next step.

Step 8: 3-(4-(4-((1-(5-methoxy-2-(1-methyl-1H-pyrazol-4-yl)-4-nitrophenyl) piperidin-4-yl)methyl)piperazin-1-yl)phenyl)piperidine-2,6-dione **(135H)**

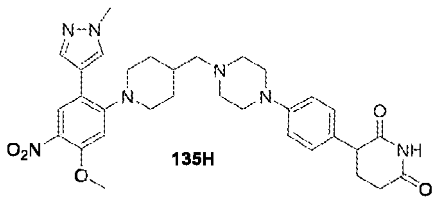

135H

The crude hydrochloride of **135G** was dissolved in 10 mL of dimethyl sulfoxide; a sodium bicarbonate (538 mg, 6.4 mmol) solid was added; and the mixture was stirred at room temperature for 10 min. 4-(2-fluoro-4-methoxy-5-nitrophenyl)-1-methyl-1H-pyrazole **(1D)**(161 mg, 0.64 mmol) was then added, and the mixture was reacted overnight at 100°C. The reaction solution was cooled to room temperature, diluted by adding 30 mL of ethyl acetate and then washed with water (20 mL × 3). The organic phases were combined, dried over anhydrous sodium sulphate and filtered. The filtrate was concentrated under reduced pressure to obtain the crude, which was purified by silica gel column chromatography (mobile phase: DCM/MeOH (V/V) = 15/1) to obtain **135B** (180 mg, two-step yield: 47%).

LCMS m/z = 602.3 $[M+H]^+$.

Step 9: 3-(4-(4-((1-(4-amino-5-methoxy-2-(1-methyl-1H-pyrazol-4-yl)phenyl)piperidin-4-yl)methyl)piperazin-1-yl)phenyl)piperidine-2,6-dione **(135I)**

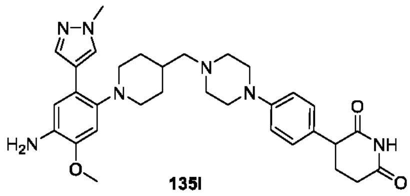

135I

**135B** (180 mg, 0.3 mmol) was dissolved in tetrahydrofuran (12 mL); a solution of ammonium chloride (80 mg, 1.5 mmol) in water (2 mL) was added; zinc powder (98 mg, 1.5 mmol) was added; and the mixture was reacted at room temperature for 20 min. The reaction solution was filtered over celite. 10 mL of saturated sodium bicarbonate solution was added to the filtrate, and the mixture was extracted 3 times with dichloromethane. The organic phases were combined, dried over anhydrous sodium sulphate and then concentrated under reduced pressure to obtain **135I** (130 mg, yield: 74%).
LCMS m/z = 286.7 $[(M+2H)/2]^+$.

Step 10: 3-(4-(4-((1-(4-((5-chloro-4-((2-(dimethylphosphoryl)phenyl)amino) pyrimidin-2-yl)amino)-5-methoxy-2-(1-methyl-1H-pyrazol-4-yl)phenyl)piperidin-4-yl)methyl)piperazin-1-yl)phenyl)piperidine-2,6-dione **(compound 135)**

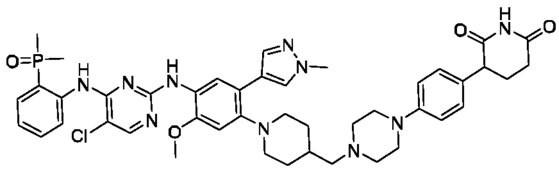

**Compound 135**

**135I** (130 mg, 0.23 mmol) and **109B** (79 mg, 0.25 mmol) were dissolved in DMF (3 mL); p-toluenesulfonic acid monohydrate (131 mg, 0.69 mmol) was added; and the mixture was stirred at 100°C for 16 h, cooled to room temperature and adjusted to a basic pH by adding dichloromethane and saturated sodium bicarbonate solution. The organic phase was separated. The aqueous phase was extracted with dichloromethane (10 ml × 3), dried over anhydrous sodium sulphate and concentrated, and the residue was purified by silica gel column chromatography (mobile phase: dichloromethane/methanol (V/V) = 100/1-10/1) to obtain **compound 135** (19 mg, yield: 10%).
LCMS m/z = 426.3 $[(M+2H)/2]^+$.
$^1$H NMR (400 MHz, DMSO-$d_6$) δ **11.19 (s, 1H), 10.75 (s, 1H), 8.50 -** 8.36 (m, 1H), 8.19 (s, 1H), 8.10 (s, 1H), 7.96 (s, 1H), 7.84 (s, 1H), 7.60 (s, 1H), 7.57 - 7.44 (m, 1H), 7.29 - 7.12 (m, 1H), 7.11 - 7.03 (m, 2H), 7.02 - 6.96 (m, 1H), 6.93 - 6.86 (m, 2H), 6.81 (s, 1H), 3.83 (s, 3H), 3.79 (s, 3H), 3.73 (dd, 1H), 3.18 - 3.03 (m, 6H), 2.73 - 2.57 (m, 3H), 2.54 - 2.40 (m, 5H), 2.36 - 2.22 (m, 2H), 2.20 - 2.06 (m, 1H), 2.06 - 1.99 (m, 1H), 1.83 - 1.71 (m, 8H), 1.69 - 1.60 (m, 1H), 1.39 - 1.27 (m, 2H).

**Example 136: Preparation of compound 136**

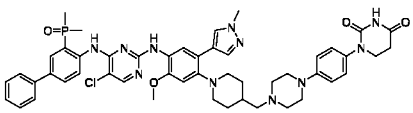

**Compound 136**

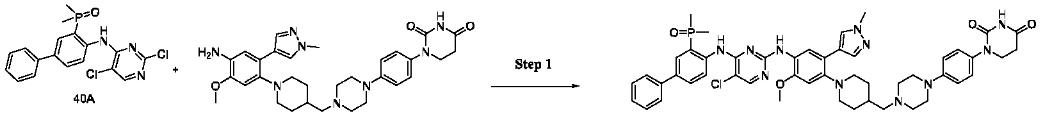


40A

131L

Compound 136

Step 1: 1-(4-(4-((1-(4-((5-chloro-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperidin-4-yl)methyl)piperazin-1-yl)phenyl)dihydropyrimidine-2,4(1H,3H)-dione **(compound 136)**

**131L** (200 mg, 0.35 mmol) and **40A** (156 mg, 0.4 mmol) were dissolved in DMF (10 mL); p-toluenesulfonic acid monohydrate (228 mg, 1.2 mmol) was added; and under nitrogen protection, the mixture was reacted overnight at 100°C, and cooled to room temperature. 20 mL of saturated aqueous sodium bicarbonate solution was added, and the mixture was subjected to suction filtration. The filter cake was dissolved in 50 mL of dichloromethane and extracted. The organic phase was dried over anhydrous sodium sulphate and then concentrated under reduced pressure, and the residue was purified by silica gel column chromatography (dichloromethane/methanol (V/V) = 100/1-10/1) to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)) to obtain the trifluoroacetate of **compound** 136. 20 mL of dichloromethane and 50 mL of saturated sodium bicarbonate solution were added to the trifluoroacetate of **compound 136,** and the layers were separated. The organic phase was dried over anhydrous sodium sulphate and then concentrated under reduced pressure to obtain **compound 136** (100 mg, yield: 30.9%).

LCMS m/z = 928.3 $[M+H]^+$.

$^1H$ NMR (400 MHz, = 1:1)) δ 8.49 - 8.42 (m, 1H), 8.42 - 8.35 (m, 1H), 8.33 - 8.25 (m, 1H), 8.08 - 7.99 (m, 2H), 7.99 - 7.89 (m, 1H), 7.88 - 7.77 (m, 6H), 7.76 - 7.69 (m, 3H), 7.60 - 7.48 (m, 2H), 4.40 - 3.66 (m, 20H), 3.62 - 3.46 (m, 2H), 3.19 - 3.05 (m, 2H), 2.77 - 2.60 (m, 1H), 2.47 - 2.33 (m, 2H), 2.28 - 2.12 (m, 8H).

**Example 137: Preparation of compound 137**

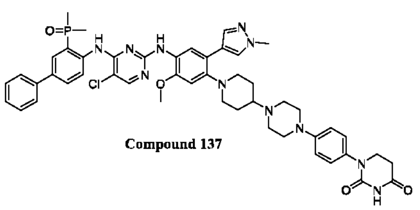

Compound 137

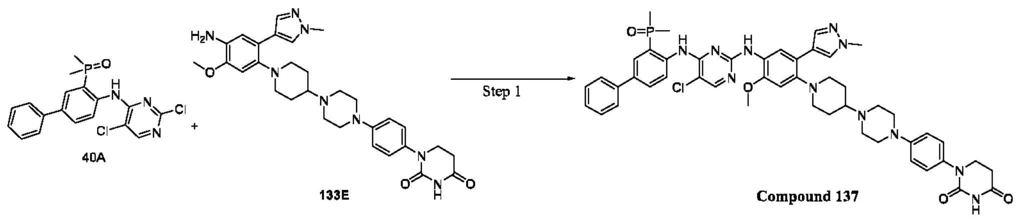


40A

133E

Compound 137

Step 1: 1-(4-(4-(1-(4-((5-chloro-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperidin-4-yl)piperazin-1-yl)phenyl)dihydropyrimidine-2,4(1H,3H)-dione **(compound 137)**

**133E** (70 mg, 0.13 mmol) and **40A** (78 mg, 0.20 mmol) were dissolved in DMF (4 mL); p-toluenesulfonic acid monohydrate (49 mg, 0.26 mmol) was added; and under nitrogen protection, the mixture was reacted overnight at 100°C, cooled to room temperature and extracted by adding 20 mL of saturated aqueous sodium bicarbonate solution and 20 mL of dichloromethane. The organic layer was concentrated under reduced pressure, and the residue was purified by silica gel column chromatography (mobile phase: dichloromethane/methanol (V/V) = 100/1-20/1). The residue was further subjected to preparative HPLC (instrument: waters 2767 (preparative liquid phase chromatographic instrument); chromatographic column: XBridge@ Prep C18 (30 mm × 150 mm); composition of mobile phases: mobile phase A: acetonitrile, and mobile phase B: water (containing 0.1% TFA)) purification to obtain the trifluoroacetate of compound **137.** The resulting solid was added to saturated aqueous sodium bicarbonate solution (20 mL). The mixture was extracted with dichloromethane (40 mL × 3). The organic phases were combined, dried over anhydrous sodium sulphate and concentrated under reduced pressure to obtain **compound 137** (17 mg, yield: 14%).

LCMS m/z = 457.8 [(M+2H)/2]$^+$.

$^1$H NMR(400 MHz, = **1:1))**δ **8.21 (s, 1H), 8.10 (d, 3H),** 7.69 (s, 1H), 7.62 (d, 2H), 7.59 - 7.47 (m, 3H), 7.29 (t, 1H), 7.17 (t, 1H), 7.08 (s, 1H), 4.20 (s, 3H), 4.16 - 4.03 (m, 5H), 4.00 - 3.86 (m, 9H), 3.54 (d, 2H), 3.24 (t, 2H), 2.93 (t, 2H), 2.46 (d, 2H), 2.29 - 2.16 (m, 2H), 1.94 (d, 6H).

**Example 138 and example 139: Preparation of compound 138 and compound 139**

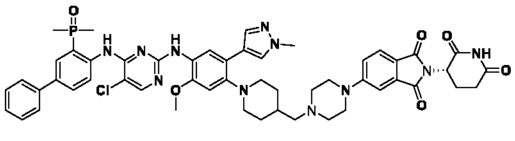
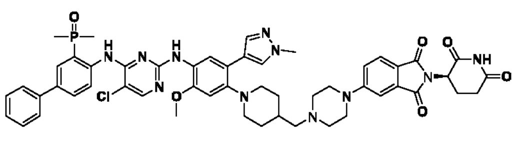

**Compound 138 and compound 139**

500 mg of **compound 65** was subjected to preparative SFC separation and purification. Preparative SFC separation conditions: instrument: Waters 150 MGM; chromatographic column: DAICEL CHIRALCEL AD (250 mm × **30 mm, 10 μm);** mobile phase: A for $CO_2$ and B for IPA+ACN (0.1% $\mathbf{NH_3{\bullet}H_2O}$**);** gradient: 70% phase B isocratic elution; flow rate: 100 mL/min; back pressure: 100 bar; column temperature: 35°C; wavelength: 220 nm.

SFC analysis conditions: instrument: SHIMADZU LC-30AD sfc; chromatographic column: Chiralpak AD-3 50 × **4.6 mm I.D., 3 μm; mobile phase:** A for $CO_2$ and B for IPA+ACN (0.05% DEA); gradient: B 60%; flow rate: 3 mL/min; back pressure: 100 bar; column temperature: 35°C; wavelength: 220 nm.

After preparative separation, the components with the same retention time were combined and concentrated under reduced pressure to obtain **compound 138** (220 mg) and **compound 139** (200 mg).

**Compound 138:**

retention time under SFC analysis conditions: 1.6 min,
LCMS m/z = 498.8[(M+2H)/2]$^+$.
$^1$H NMR (400 MHz, DMSO-$d_6$) δ **11.37 (s, 1H), 11.06 (s, 1H), 8.58 -** 8.40 (m, 1H), 8.29 (s, 1H), 8.11 (s, 1H), 8.03 (s, 1H), 7.88 (s, 1H), 7.78 - 7.64 (m, 2H), 7.63 - 7.52 (m, 3H), 7.45 (t, 2H), 7.36 (t, 2H), 7.27 (dd, 1H), 7.22 - 7.10 (m, 1H), 6.86 (s, 1H), 5.07 (dd, 1H), 3.80 (s, 3H), 3.76 (s, 3H), 3.57 - 3.39 (m, 4H), 3.15 - 3.00 (m, 2H), 2.97 - 2.77 (m, 1H), 2.72 - 2.51 (m, 8H), 2.35 - 2.21 (m, 2H), 2.07 - 1.94 (m, 1H), 1.91 - 1.71 (m, 8H), 1.70 - 1.56 (m, 1H), 1.40 - 1.26 (m, 2H).

**Compound 139:**

retention time under SFC analysis conditions: 2.5 min,
LCMS m/z = 498.8 [(M+2H)/2]$^+$.
$^1$H NMR (400 MHz, DMSO-$d_6$) δ **11.37 (s, 1H), 11.06 (s, 1H), 8.58** - 8.41 (m, 1H), 8.29 (s, 1H), 8.11 (s, 1H), 8.03 (s, 1H), 7.88 (s, 1H), 7.75 - 7.65 (m, 2H), 7.62 - 7.51 (m, 3H), 7.45 (t, 2H), 7.36 (t, 2H), 7.28 (dd, 1H), 7.22 - 7.09 (m, 1H), 6.86 (s, 1H), 5.07 (dd, 1H), 3.80 (s, 3H), 3.76 (s, 3H), 3.55 - 3.39 (m, 4H), 3.14 - 3.01 (m, 2H), 2.99 - 2.79 (m, 1H), 2.69 - 2.51 (m, 8H), 2.35 - 2.23 (m, 2H), 2.06 - 1.96 (m, 1H), 1.90 - 1.72 (m, 8H), 1.70 - 1.57 (m, 1H), 1.40 - 1.26 (m, 2H).

**Example 140: Preparation of compound 140**

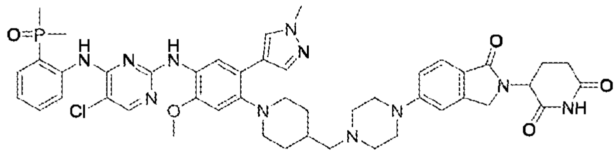

**Compound 140**

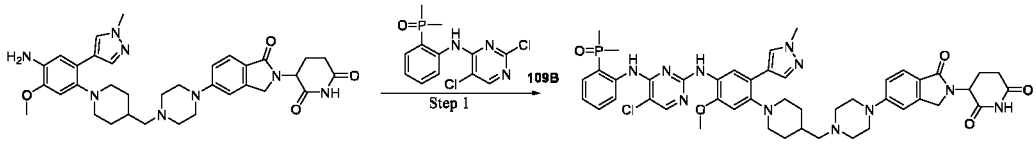


105G

Compound 140

Step 1: 3-(5-(4-((1-(4-((5-chloro-4-((2-(dimethylphosphoryl)phenyl)amino) pyrimidin-2-yl)amino)-5-methoxy-2-(1-methyl-1H-pyrazol-4-yl)phenyl)piperidin-4-yl)methyl)piperazin-1-yl)-1-oxoisoindolin-2-yl)piperidine-2,6-dione **(compound 140)**

**105G** (300 mg, 0.48 mmol) and **109B** (228 mg, 0.72 mmol) were dissolved in DMF (5 mL); p-toluenesulfonic acid monohydrate (182 mg, 0.96 mmol) was added; and under nitrogen protection, the mixture was reacted overnight at 100°C, cooled to room temperature and extracted by adding 20 mL of saturated aqueous sodium bicarbonate solution and 20 mL of dichloromethane. The organic layer was concentrated under reduced pressure, and the residue was purified by silica gel column chromatography (mobile phase: dichloromethane/methanol (V/V) = 100/1-20/1). The residue was further subjected to preparative HPLC (instrument: waters 2767 (preparative liquid phase chromatographic instrument); chromatographic column: XBridge@ Prep C18 (30 mm × 150 mm); composition of mobile phases: mobile phase A: acetonitrile, and mobile phase B: water (containing 0.1% TFA)) purification to obtain the trifluoroacetate of compound **140.** The resulting solid was added to saturated aqueous sodium bicarbonate solution (20 mL). The mixture was extracted with dichloromethane (40 mL × 3). The organic phases were combined, dried over anhydrous sodium sulphate and concentrated under reduced pressure to obtain compound **140** (150 mg, yield: 35%).

LCMS m/z = 906.3 $[M+H]^+$.

$^1H$ NMR (400 MHz, = **1:1))** $\delta$ **11.21 (s, 1H), 10.93 (s,** 1H), 8.44 (s, 1H), 8.19 (s, 1H), 8.10 (s, 1H), 7.96 (s, 1H), 7.85 (s, 1H), 7.61 (s, 1H), 7.56 - 7.45 (m, 2H), 7.10 - 7.04 (m, 2H), 7.04 - 6.90 (m, 2H), 6.81 (s, 1H), 5.05 (dd, 1H), 4.45 - 4.14 (m, 2H), 3.81 (d, 6H), 3.09 (d, 2H), 2.90 (ddd, 1H), 2.68 - 2.54 (m, 3H), 2.29 (d, 2H), 2.03 - 1.91 (m, 1H), 1.88 - 1.71 (m, 8H).

**Example 141: Preparation of compound 141**

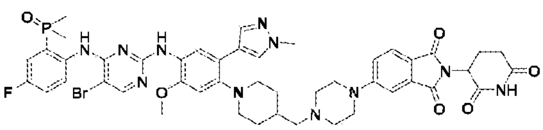

**Compound 141**

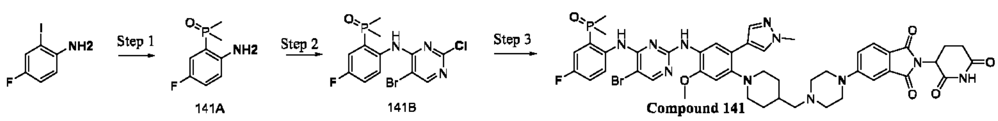


141A 141B Compound 141

Step 1: (2-amino-5-fluorophenyl)dimethylphosphine oxide **(141A)**

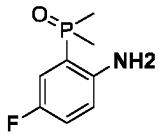

4-fluoro-2-iodoaniline (5.00 g, 21.10 mmol), dimethylphosphine oxide (2.14 g, 27.43 mmol), potassium phosphate (6.72 g, 31.65 mmol), palladium acetate (0.24 g, 1.06 mmol), Xant-Phos (0.61 g, 1.06 mmol) and anhydrous magnesium sulphate (2.54 g, 21.1 mmol) were successively added to 1,4-dioxane (50 ml), and the mixture was subjected to nitrogen replacement three times, stirred at 100°C for 8 h, cooled to room temperature and filtered. The filtrate was concentrated under reduced pressure, and the residue was purified by column chromatography (ethyl acetate/petroleum ether (V/V) = 1/1 to dichloromethane/methanol (V/V) = 30/1) to obtain **141A** (3 g, yield: 76%).
LCMS m/z = 188.1 $[M+H]^+$.

Step 2: (2-((5-bromo-2-chloropyrimidin-4-yl)amino)-5-fluorophenyl) dimethylphosphine oxide **(141B)**

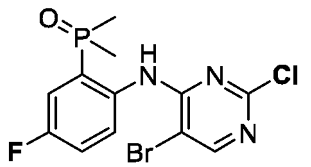

**141A** (1.50 g, 7.97 mmol) and 5-bromo-2,4-dichloropyrimidine (3.63 g, 15.94 mmol) were dissolved in NMP (10 ml); DIPEA (1.24 g, 9.56 mmol) was added; and the mixture was stirred at 120°C for 1 h, cooled to room temperature and extracted by adding 30 mL of water and 30 mL of ethyl acetate. The organic layer was washed twice with saturated brine, and concentrated under reduced pressure. The residue was slurried with 10 mL of MTBE, filtered and dried under reduced pressure to obtain **141B** (1.5 g, yield: 50%).
LCMS m/z = 378.0 $[M+H]^+$.

Step 3: 5-(4-((1-(4-((5-bromo-4-((2-(dimethylphosphoryl)-4-fluorophenyl) amino)pyrimidin-2-yl)amino)-5-methoxy-2-(1-methyl-1H-pyrazol-4-yl)phenyl)piperidin-4-yl)methyl)piperazin-1-yl)-2-(2,6-dioxopiperidin-3-yl)isoindoline-1,3-dione **(compound 141)**

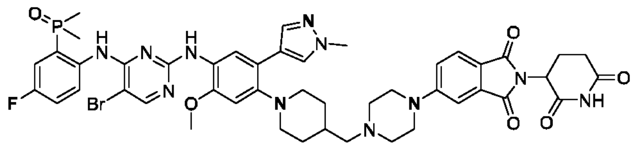

**45F** (1.20 g, 1.87 mmol) and **141B** (0.71 g, 1.87 mmol) were dissolved in DMF (10 mL); p-toluenesulfonic acid monohydrate (0.71 g, 3.74 mmol) was added; and the mixture was stirred overnight at 100°C, and cooled to room temperature. 10 mL of saturated aqueous sodium bicarbonate solution was added, with a solid precipitated, and the mixture was filtered. The filter cake was dried and then purified by silica gel column chromatography (dichloromethane/methanol (V/V) = 100/3-100/5). 8 mL of acetonitrile was added to the resulting residue, and the mixture was stirred at 70°C for 1 h and filtered. The filter cake was dried under reduced pressure to obtain **compound 141** (0.44 g, yield: 24%).
LCMS m/z = 491.7 $[(M+2H)/2]^+$.
$^1$H NMR (400 MHz, DMSO-$d_6$) $\delta$ **11.06 (s, 1H), 10.78 (s, 1H), 8.30 (s, 1H),** 8.17 (s, 1H), 8.16 (s, 1H), 7.98 (s, 1H), 7.82 (s, 1H), 7.68 (d, 1H), 7.61 (s, 1H), 7.44 - 7.36 (m, 1H), 7.34 (d, 1H), 7.26 (d, 1H), 6.81 (s, 1H), 6.68 (s, 1H), 5.11-5.03 (m, 1H), 3.84 (s, 3H), 3.79 (s, 3H), 3.49-3.40 (m, 4H), 3.13-3.02 (m, 2H), 2.95 - 2.81 (m, 1H), 2.70 - 2.45 (m, 8H), 2.29 (d, 2H), 2.06-1.97 (m, 1H), 1.78 (d, 6H), 1.84-1.60 (m, 3H), 1.39 - 1.27 (m, 2H).

**Example 142: Preparation of compound 142**

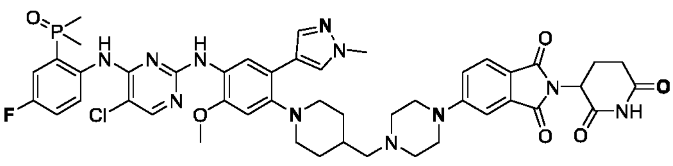

**Compound 142**

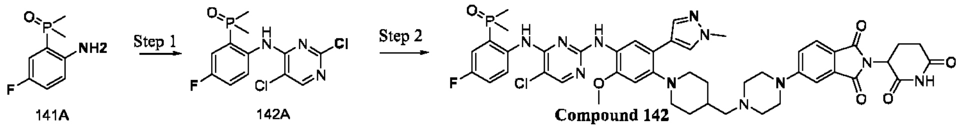

Step 1: (2-((2,5-dichloropyrimidin-4-yl)amino)-5-fluorophenyl) dimethylphosphine oxide (142A)

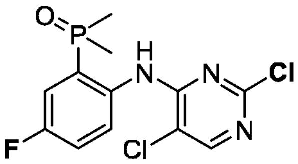

**141A** (1.50 g, 7.97 mmol) and 2,4,5-trichloropyrimidine (2.92 g, 15.94 mmol) were dissolved in NMP (10 ml); DIPEA (1.24 g, 9.56 mmol) was added; and the mixture was stirred at 120°C for 1 h, cooled to room temperature and extracted by adding 30 mL of water and 30 mL of ethyl acetate. The organic layer was washed twice with saturated brine, and concentrated under reduced pressure. The residue was slurried with 10 mL of MTBE, filtered and dried under reduced pressure to obtain **142A** (1.4 g, yield: 52%).

LCMS m/z = 334.0 $[M+H]^+$.

Step 2: 5-(4-((1-(4-((5-chloro-4-((2-(dimethylphosphoryl)-4-fluorophenyl) amino)pyrimidin-2-yl)amino)-5-methoxy-2-(1-methyl-1H-pyrazol-4-yl)phenyl)piperidin-4-yl)methyl)piperazin-1-yl)-2-(2,6-dioxopiperidin-3-yl)isoindoline-1,3-dione **(compound 142)**

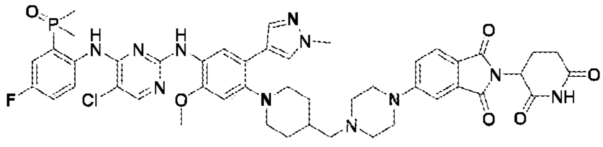

**Compound 142**

**45F** (1.20 g, 1.87 mmol) and **142A** (0.62 g, 1.87 mmol) were dissolved in DMF (10 ml); p-toluenesulfonic acid monohydrate (0.71 g, 3.74 mmol) was added; and the mixture was stirred overnight at 100°C, and cooled to room temperature. 10 mL of saturated aqueous sodium bicarbonate solution was added, with a solid precipitated, and the mixture was filtered. The filter cake was dried and then purified by silica gel column chromatography (dichloromethane/methanol (V/V) = 100/3-100/5). 8 mL of acetonitrile was added to the resulting residue, and the mixture was stirred at 70°C for 1 h and filtered. The filter cake was dried under reduced pressure to obtain **compound 142** (0.8 g, yield: 45%).

LCMS m/z = 469.8 $[(M+2H)/2]^+$.

$^1H$ NMR (400 MHz, DMSO-$d_6$) δ **11.08-**11.03 (m, 1H), 8.42 (s, 1H), 8.17 (s, 1H), 8.10 (s, 1H), 8.00 (s, 1H), 7.83 (s, 1H), 7.68 (d, 1H), 7.62 (s, 1H), 7.46-7.38 (m, 1H), 7.34 (d, 1H), 7.26 (d, 1H), 6.81 (s, 1H), 6.67 (s, 1H), 5.10-5.03

(m, 1H), 3.84 (s, 3H), 3.80 (s, 3H) 3.49-3.40 (m, 4H), 3.15-3.01 (m, 2H), 2.96 - 2.79 (m, 1H), 2.70 - 2.45 (m, 8H), 2.29 (d, 2H), 2.05-1.97 (m, 1H), 1.79 (d, 6H), 1.84-1.60 (m, 3H), 1.41 - 1.28 (m, 2H).

**Example 143: Preparation of compound 143**

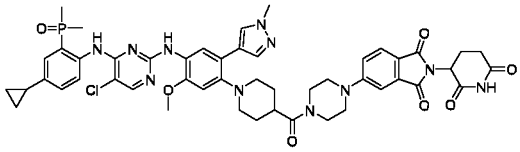

Compound 143

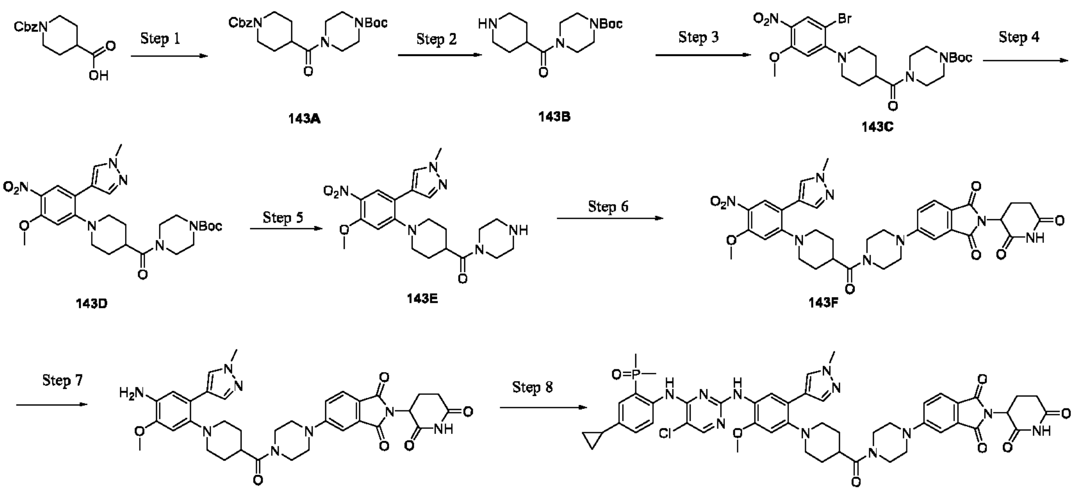


143A 143B 143C 143D 143E 143F 143G Compound 143

Step 1: tert-butyl4-(1-((benzyloxy)carbonyl)piperidine-4-carbonyl)piperazine-1-carboxylate **(143A)**

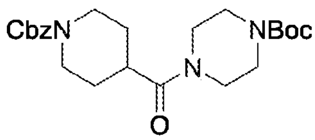

N-Cbz-piperidine-4-carboxylic acid (7.80 g, 29.63 mmol), N-Boc-piperazine (5.52 g, 29.63 mmol), HOBT (4.40 g, 32.59 mmol) and dichloromethane (80 mL) were added to a reaction flask. Under ice-water bath cooling, EDCI (8.52 g, 44.44 mmol) was added. After the addition, the mixture was reacted at room temperature for 3 h. The reaction solution was washed once with saturated sodium bicarbonate solution (100 mL), dried over anhydrous sodium sulphate, filtered and concentrated to dryness under reduced pressure. The residue was purified by column chromatography (eluent: DCM/$CH_3OH$ = 50/1 to 20/1) to obtain **143A** (10 g, yield: 78.2%).

Step 2: tert-butyl 4-(piperidine-4-carbonyl)piperazine-1-carboxylate **(143B)**

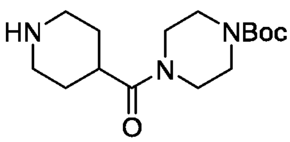

**143A** (10 g, 23.17 mmol), methanol (100 mL) and palladium on carbon (2 g) were added to a reaction flask, and the mixture was subjected to hydrogen replacement three times, reacted at room temperature for about 18 h and subjected to suction filtration over an appropriate amount of celite. The filter cake was washed with a small amount of methanol. The filtrate was concentrated to dryness under reduced pressure, stirred for 30 min under stirring by adding MTBE (50 mL) and subjected to suction filtration under reduced pressure. The filter cake was concentrated to dryness under reduced pressure to obtain **143B** (6.30 g, yield: 91.4%).

Step 3: tert-butyl 4-(1-(2-bromo-5-methoxy-4-nitrophenyl)piperidine-4-carbonyl)piperazine-1-carboxylate **(143C)**

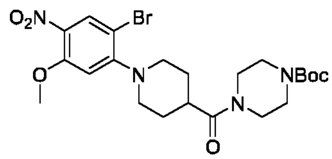

**143B** (6.30 g, 21.18 mmol), 1-bromo-2-fluoro-4-methoxy-5-nitrobenzene (1C, 5.30 g, 21.18 mmol), potassium carbonate (4.39 g, 31.77 mmol) and DMSO (70 mL) were added to a reaction flask, and the mixture was reacted at 100°C for 2-3 h. The reaction solution was cooled to room temperature. Methanol (35 mL) was added, and water (200 mL) was added dropwise. After the addition, the mixture was stirred for 1 h and subjected to suction filtration. The filter cake was washed with a small amount of water and subjected to suction filtration until dryness. The filter cake was transferred to a reaction flask. MTBE (100 mL) was added; under stirring, petroleum ether (50 mL) was added dropwise; and the mixture was slurried under stirring for 1 h and subjected to suction filtration. The filter cake was washed with a small amount of petroleum ether and concentrated to dryness under reduced pressure to obtain **143C** (10 g, yield: 89.5%).

Step 4: tert-butyl 4-(1-(5-methoxy-2-(1-methyl-1H-pyrazol-4-yl)-4-nitrophenyl)piperidine-4-carbonyl)piperazine-1-carboxylate **(143D)**

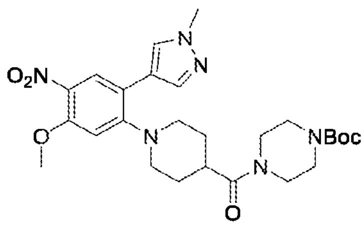

**143C** (10 g, 18.96 mmol), 1-methyl-1H-pyrazole-4-boronic acid (3.58 g, 28.44 mmol), Pd(dppf)$Cl_2$·DCM (2.31 g, 2.84 mmol), potassium carbonate (8.14 g, 56.88 mmol), 1,4-dioxane (100 mL) and water (30 mL) were added to a reaction flask, and the mixture was subjected to nitrogen replacement three times, reacted at 100°C for about 3 h, and cooled in a water bath. Ethyl acetate (100 mL) and water (50 mL) were added. The mixture was stirred, and the layers were separated. The organic layer was washed with saturated sodium chloride solution (50 mL × 2), dried over anhydrous sodium sulphate, filtered and concentrated to dryness under reduced pressure, and the residue was purified by column chromatography (eluent: DCM/$CH_3OH$ = DCM to 50/1 to 30/1 to obtain **143D** (8.2 g, yield: 81.81%).

Step 5: (1-(5-methoxy-2-(1-methyl-1H-pyrazol-4-yl)-4-nitrophenyl)piperidin-4-yl)(piperazin-1-yl)methanone **(143E)**

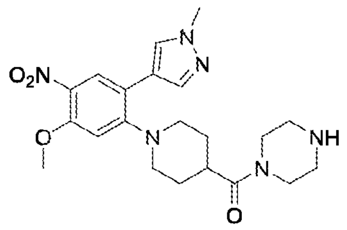

**143D** (8.2 g, 15.51 mmol) and DCM (40 mL) were added to a reaction flask; trifluoroacetic acid (16 mL) was added under stirring; and the mixture was reacted overnight at room temperature for about 16 h. The reaction solution was concentrated to dryness under reduced pressure. Dichloromethane (50 mL) was added to the residue, and the mixture was adjusted to pH 12-13 by dropwise adding 1 N sodium hydroxide solution. The layers were separated. The organic layer was dried over anhydrous sodium sulphate, filtered and concentrated to dryness under reduced pressure to obtain **143E** (6.2 g, yield: 93.3%).

Step 6: 2-(2,6-dioxopiperidin-3-yl)-5-(4-(1-(5-methoxy-2-(1-methyl-1H-pyrazol-4-yl)-4-nitrophenyl)piperidine-4-carbonyl)piperazin-1-yl)isoindoline-1,3-dione **(143F)**

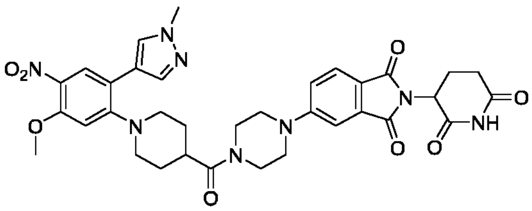

**143E** (4.0 g, 9.34 mmol), 2-(2,6-dioxo-piperidin-3-yl)-5-fluoro-isoindole-1,3-dione (2.58 g, 9.34 mmol), DIPEA (1.81 g, 14.01 mmol) and DMSO (40 mL) were added to a reaction flask, and the mixture was reacted at 90°C for 3 h and cooled in an ice water. Ethyl acetate (150 mL) and water (100 mL) were added, and the mixture was stirred for 5 min and allowed to stand for layer separation. The organic layer was washed with saturated sodium chloride solution (100 mL × 2), dried over anhydrous sodium sulphate, filtered and concentrated to dryness under reduced pressure, and the residue was purified by column chromatography (eluent: DCM/$CH_3OH$ = DCM to 50/1 to 20/1 to obtain **143F** (3 g, yield: 46.9%).

Step 7: 5-(4-(1-(4-amino-5-methoxy-2-(1-methyl-1H-pyrazol-4-yl)phenyl) piperidine-4-carbonyl)piperazin-1-yl)-2-(2,6-dioxopiperidin-3-yl)isoindoline-1,3-dione **(143G)**

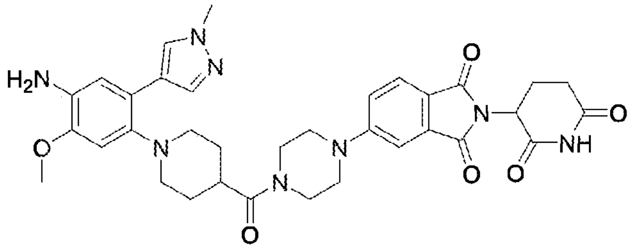

**143F** (2 g, 2.92 mmol), zinc powder (0.95 g, 14.60 mmol), ammonium chloride (0.78 g, 14.60 mmol), tetrahydrofuran (20 mL) and water (8 mL) were added to a reaction flask, and the mixture was reacted at room temperature for 1 h. Dichloromethane (50 mL) and saturated sodium bicarbonate solution (20 mL) were added, and the mixture was stirred for 2 min, subjected to suction filtration over an appropriate amount of celite and washed with dichloromethane (30 mL × 2). The layers in the filtrate were separated. The organic layer was washed with water (20 mL × 1), dried over anhydrous sodium sulphate, filtered and concentrated to dryness under reduced pressure to obtain **143G** (yellow solid, 1.6 g, yield: 83.7%).

Step 8: 5-(4-(1-(4-((5-chloro-4-((4-cyclopropyl-2-(dimethylphosphoryl)phenyl) amino)pyrimidin-2-yl)amino)-5-methoxy-2-(1-methyl-1H-pyrazol-4-yl)phenyl)piperidine-4-carbonyl)piperazin-1-yl)-2-(2,6-dioxopiperidin-3-yl)isoindoline-1,3-dione **(compound 143)**

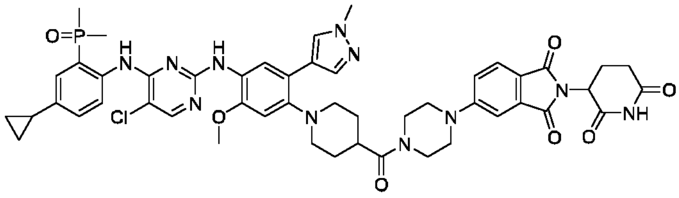

Compound 143

**143G** (0.4 g, 0.61 mmol), **107A** (0.22 g, 0.61 mmol), p-toluenesulfonic acid monohydrate (0.23 g, 1.22 mmol) and DMF (5 mL) were added to a reaction flask, and the mixture was reacted at 100°C for about 18 h. Under ice water cooling, dichloromethane (30 mL) and saturated sodium bicarbonate solution (20 mL) were added, and the mixture was stirred and allowed to stand for layer separation. The organic layer was washed with saturated sodium chloride solution (20 mL × 3), dried over anhydrous sodium sulphate, filtered and concentrated to dryness under reduced pressure, and the residue was purified by column chromatography (eluent: DCM/CH3OH = DCM to 50/1 to 30/1 to 20/1 to obtain **compound 143** (0.23 g, yield: 38.7%).

LCMS m/z = 487.7 $[(M+2H)/2]^+$.

$^1$H NMR (400 MHz, DMSO-$d_6$) δ **11.11 (s, 1H), 11.07 (s, 1H), 8.29** - 8.21 (m, 1H), 8.14 (s, 1H), 8.07 (s, 1H), 7.96 (s, 1H), 7.91 (s, 1H), 7.71 (d, 1H), 7.62 (s, 1H), 7.36 (d, 1H), 7.29-7.20 (m, 2H), 6.82 (s, 1H), 6.53-6.33 (m, 1H), 5.12-5.04 (m, 1H), 3.83 (s, 3H), 3.79 (s, 3H), 3.76-3.63 (m, 4H), 3.58-3.45 (m, 4H), 3.17-3.07 (m, 2H), 2.95 - 2.67 (m, 4H), 2.64 - 2.45 (m, 2H), 2.07 - 2.00 (m, 1H), 1.85 - 1.72 (m, 11H), 0.95 - 0.88 (m, 2H), 0.54-0.48 (m, 2H).

**Example 144: Preparation of compound 144**

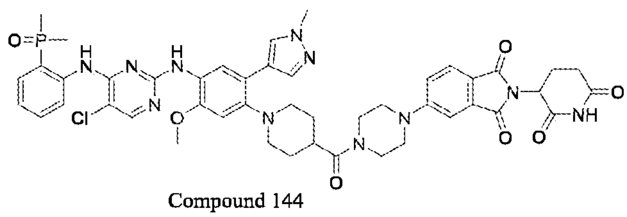

Compound 144

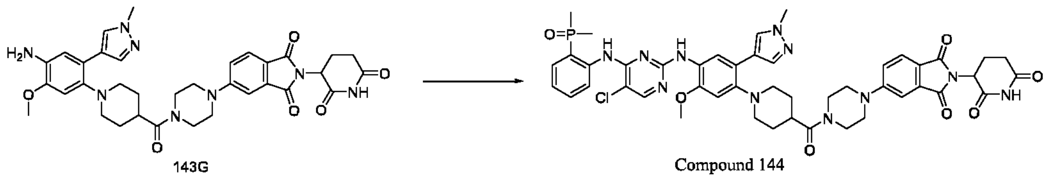

143G

Compound 144

Step 1: 5-(4-(1-(4-((5-chloro-4-((2-(dimethylphosphoryl)phenyl)amino) pyrimidin-2-yl)amino)-5-methoxy-2-(1-methyl-1H-pyrazol-4-yl)phenyl)piperidine-4-carbonyl)piperazin-1-yl)-2-(2,6-dioxopiperidin-3-yl)isoindoline-1,3-dione **(compound 144)**

**143G** (0.4 g, 0.61 mmol), (2-((2,5-dichloropyrimidin-4-yl)amino)phenyl)dimethylphosphine oxide **(109B,** 0.22 g, 0.61 mmol), p-toluenesulfonic acid monohydrate (0.23 g, 1.22 mmol) and DMF (5 mL) were added to a reaction flask, and the mixture was reacted at 100°C for about 18 h. Under ice water cooling, dichloromethane (30 mL) and saturated sodium bicarbonate solution (20 mL) were added, and the mixture was stirred and allowed to stand for layer separation. The organic layer was washed with saturated sodium chloride solution (20 mL × 3), dried over anhydrous sodium sulphate, filtered and concentrated to dryness under reduced pressure, and the residue was purified by column chromatography (eluent: DCM/$CH_3OH$ = DCM to 50/1 to 30/1 to 20/1 to obtain **compound 144** (0.26 g, yield: 45.55%).

LCMS m/z = 467.8 [(M+2H)/2]$^+$.

$^1$H NMR (400 MHz, DMSO-$d_6$) δ **11.22 (s, 1H), 11.07 (s, 1H), 8.50-**8.39 (m, 1H), 8.19 (s, 1H), 8.11 (s, 1H), 7.93 (s, 1H), 7.87 (s, 1H), 7.71 (d, 1H), 7.63 (s, 1H), 7.55 - 7.46 (m, 1H), 7.36 (d, 1H), 7.28-7.22 (m, 1H), 7.05 - 6.91 (m, 2H), 6.81 (s, 1H), 5.12-5.04 (m, 1H), 3.82 (s, 3H), 3.80 (s, 3H), 377-3.61 (m, 4H), 3.57-3.43 (m, 4H), 3.17-3.07 (m, 2H), 2.96 - 2.66 (m, 4H), 2.64 - 2.45 (m, 2H), 2.08 - 1.98 (m, 1H), 1.86-1.68 (m, 10H).

**Example 145: Preparation of compound 145**

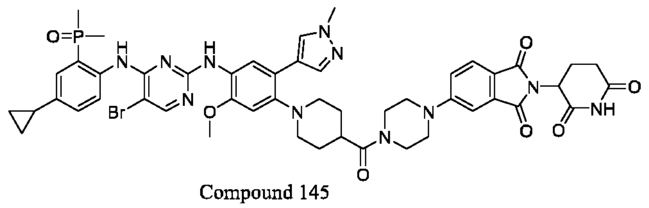

Compound 145

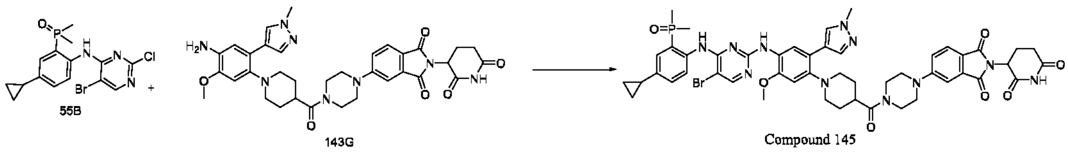

55B

143G

Compound 145

Step 1: 5-(4-(1-(4-((5-bromo-4-((4-cyclopropyl-2-(dimethylphosphoryl) phenyl)amino)pyrimidin-2-yl)amino)-5-methoxy-2-(1-methyl-1H-pyrazol-4-yl)phenyl)piperidine-4-carbonyl)piperazin-1-yl)-2-(2,6-dioxopiperidin-3-yl)isoindoline-1,3-dione **(compound 145)**

**143G** (0.4 g, 0.61 mmol), **55B** (0.24 g, 0.61 mmol), p-toluenesulfonic acid monohydrate (0.23 g, 1.22 mmol) and DMF (5 mL) were added to a reaction flask, and the mixture was reacted at 100°C for about 18 h. Under ice water cooling, dichloromethane (30 mL) and saturated sodium bicarbonate solution (20 mL) were added, and the mixture was stirred and allowed to stand for layer separation. The organic layer was washed with saturated sodium chloride solution (20 mL × 3), dried over anhydrous sodium sulphate, filtered and concentrated to dryness under reduced pressure, and the residue was purified by column chromatography (eluent: DCM/$CH_3OH$ = DCM to 50/1 to 30/1 to 20/1. The resulting crude was further subjected to preparative HPLC (instrument: waters 2767 (preparative liquid phase chromatographic instrument); chromatographic column: XBridge@ Prep C18 (30 mm × 150 mm); composition of mobile phases: mobile phase A: acetonitrile, and mobile phase B: water (containing 0.1% TFA)) purification to obtain a preparative product, which was alkalized with saturated aqueous sodium bicarbonate solution to obtain **compound 145** (0.06 g, yield: 9.65%).

LCMS m/z = 509.7 [(M+2H)/2]$^+$.

$^1$H NMR (400 MHz, DMSO-$d_6$) δ **11.06 (s, 1H), 10.84 (s, 1H), 8.20**-8.10 (m, 3H), 7.94 (s, 1H), 7.91 (s, 1H), 7.71 (d, 1H), 7.60 (s, 1H), 7.37(d, 1H), 7.29 - 7.19 (m, 2H), 6.81 (s, 1H), 6.54-6.35 (m, 1H), 5.12-5.03 (m, 1H), 3.83 (s, 3H), 3.79 (s, 3H), 3.75-3.63 (m, 4H), 3.58-3.45 (m, 4H), 3.16-3.07 (m, 2H), 2.96 - 2.67 (m, 4H), 2.65 - 2.45 (m, 2H), 2.06 - 1.99 (m, 1H), 1.85-1.71 (m, 11H), 0.94-0.87 (m, 2H), 0.53-0.47 (m, 2H).

**Example 146: Preparation of compound 146**

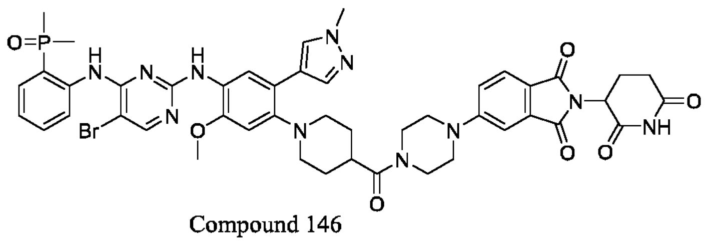

Compound 146

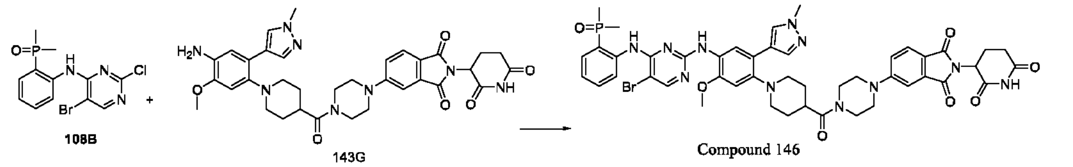

108B

143G

Compound 146

Step 1: 5-(4-(1-(4-((5-bromo-4-((2-(dimethylphosphoryl)phenyl)amino) pyrimidin-2-yl)amino)-5-methoxy-2-(1-methyl-1H-pyrazol-4-yl)phenyl)piperidine-4-carbonyl)piperazin-1-yl)-2-(2,6-dioxopiperidin-3-yl)isoindoline-1,3-dione **(compound 146)**

**143G** (0.4 g, 0.61 mmol), **108B** (0.22 g, 0.61 mmol), p-toluenesulfonic acid monohydrate (0.23 g, 1.22 mmol) and DMF (5 mL) were added to a reaction flask, and the mixture was reacted at 100°C for about 18 h. Under ice water cooling, dichloromethane (30 mL) and saturated sodium bicarbonate solution (20 mL) were added, and the mixture was stirred and allowed to stand for layer separation. The organic layer was washed with saturated sodium chloride solution (20 mL × 3), dried over anhydrous sodium sulphate, filtered and concentrated to dryness under reduced pressure, and the residue was purified by column chromatography (eluent: DCM/$CH_3OH$ = DCM to 50/1 to 30/1 to 20/1. The resulting crude was further subjected to preparative HPLC (instrument: waters 2767 (preparative liquid phase chromatographic instrument); chromatographic column: XBridge@ Prep C18 (30 mm × 150 mm); composition of mobile phases: mobile phase A: acetonitrile, and mobile phase B: water (containing 0.1% TFA)) purification to obtain a product, which was alkalized with saturated aqueous sodium bicarbonate solution to obtain **compound 146** (0.09 g, yield: 15%).

LCMS m/z = 489.6 $[(M+2H)/2]^+$.

$^1H$ NMR (400 MHz, DMSO-$d_6$) δ **11.06 (s, 1H), 10.95 (s, 1H), 8.40**-8.28 (m, 1H), 8.18 (s, 2H), 7.91 (s, 1H), 7.86 (s, 1H), 7.71 (d, 1H), 7.61 (s, 1H), 7.53-7.44 (m, 1H), 7.36 (d, 1H), 7.29-7.23 (m, 1H), 7.05-6.90 (m, 2H), 6.80 (s, 1H), 5.11-5.03 (m, 1H), 3.82 (s, 3H), 3.80 (s, 3H), 3.75-3.63 (m, 4H), 3.57-3.45 (m, 4H), 3.16-3.07 (m, 2H), 2.94-2.66 (m, 4H), 2.65 - 2.45 (m, 2H), 2.05 - 1.97 (m, 1H), 1.83-1.70 (m, 10H).

**Example 147: Preparation of compound 147**

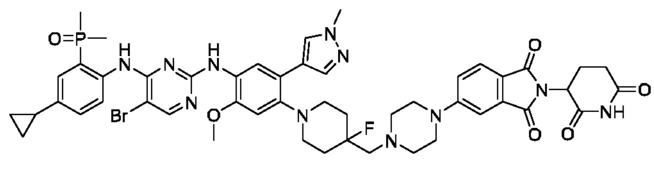

Compound 147

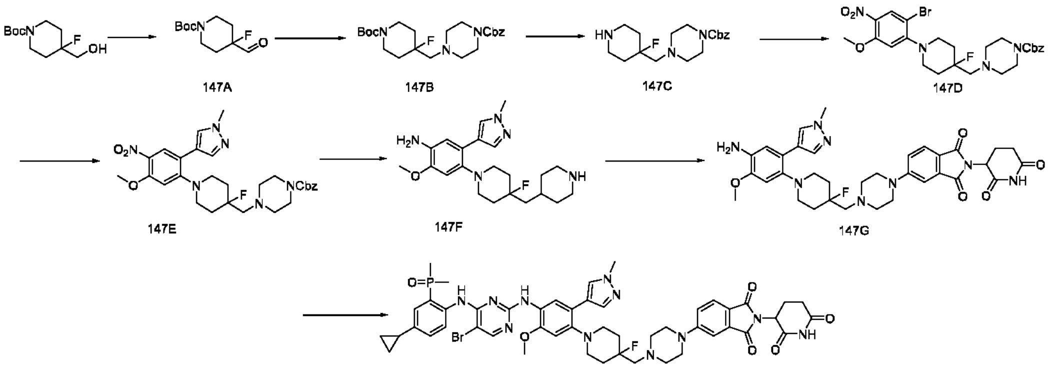

147A

147B

147C

147D

147E

147F

147G

Compound 147

Step 1: tert-butyl 4-fluoro-4-formylpiperidine-1-carboxylate **(147A)**

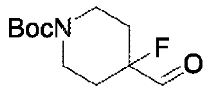

1-tert-butoxycarbonyl-4-fluoro-4-(hydroxymethyl)piperidine (14 g, 60.01 mmol) and dichloromethane (210 mL) were added to a reaction flask, and the mixture was stirred until a clear solution was obtained. Under ice water cooling, Dess-Martin periodinane (80.91 g, 120.02 mmol) was added, and the mixture was reacted at room temperature for about 3 h. Saturated sodium bicarbonate solution (300 mL) was added, and the mixture was stirred for 5 min and subjected to suction filtration over an appropriate amount of celite. The filter cake was washed with dichloromethane (100 mL × 2), dried over anhydrous sodium sulphate, filtered and concentrated under reduced pressure. The residue was purified by column chromatography (eluent: PE/EA = 1/1) to obtain **147A** (5 g, yield: 36.03%).

Step 2: benzyl 4-((1-(tert-butoxycarbonyl)-4-fluoropiperidin-4-yl)methyl)piperazine-1-carboxylate **(147B)**

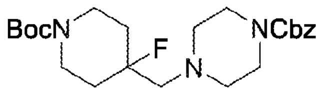

**147A** (5 g, 21.62 mmol), benzyl-1-piperazine carbonate (4.76 g, 21.62 mmol) and dichloromethane (100 mL) were added to a reaction flask; glacial acetic acid (2.59 g, 43.24 mmol) and sodium triacetoxyborohydride (11.45 g, 54.05 mmol) were added under stirring; and the mixture was reacted overnight at room temperature for about 18 h. Water (100 mL) was added, and the mixture was stirred for 5 min and allowed to stand for layer separation. The organic layer was washed with saturated sodium bicarbonate solution (100 mL × 1), dried over anhydrous sodium sulphate, filtered and concentrated to dryness under reduced pressure, and the residue was purified by column chromatography (eluent: PE/EA = 2/1) to obtain **147B** (6.2 g, yield: 65.84%).

Step 3: benzyl 4-((4-fluoropiperidin-4-yl)methyl)piperazine-1-carboxylate **(147C)**

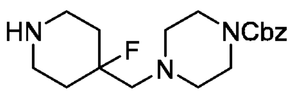

147C

**147B** (6.2 g, 14.24 mmol) and dichloromethane (30 mL) were added to a reaction flask, and the mixture was stirred and dissolved. Trifluoroacetic acid (12 mL) was added dropwise. After the addition, the mixture was reacted at room temperature for 3 h and concentrated to dryness under reduced pressure. DCM (50 mL) was added to the residue, and the mixture was adjusted to about pH 12 with 1 N sodium hydroxide solution and allowed to stand for layer separation. The aqueous layer was extracted once with DCM (30 mL). The organic layers were combined, washed with saturated sodium chloride solution (60 mL × 1), dried over anhydrous sodium sulphate, filtered and concentrated to dryness under reduced pressure to obtain **147C** (4.2 g, yield: 87.93%).

Step 4: benzyl 4-((1-(2-bromo-5-methoxy-4-nitrophenyl)-4-fluoropiperidin-4-yl)methyl)piperazine-1-carboxylate **(147D)**

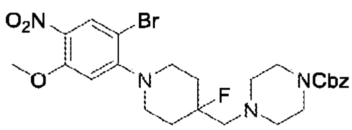

147D

**147C** (4.2 g, 12.52 mmol), 1-bromo-2-fluoro-4-methoxy-5-nitrobenzene **(1C,** 3.13 g, 12.52 mmol), potassium

carbonate (3.46 g, 25.04 mmol) and DMSO (40 mL) were added to a reaction flask, and the mixture was reacted at 100°C for about 3 h and cooled to room temperature. Ethyl acetate (100 mL) and water (100 mL) were added, and the mixture was stirred and allowed to stand for layer separation. The organic layer was washed with saturated sodium chloride solution (50 mL × 2), dried over anhydrous sodium sulphate, filtered and concentrated under reduced pressure. The residue was purified by silica gel column chromatography (eluent: PE/EA = PE to 5/1 to 3/1 to 2/1) to obtain **147D** (5 g, yield: 70.63%).

Step 5: Benzyl 4-((4-fluoro-1-(5-methoxy-2-(1-methyl-1H-pyrazol-4-yl)-4-nitrophenyl)piperidin-4-yl)methyl)piperazine-1-carboxylate **(147E)**

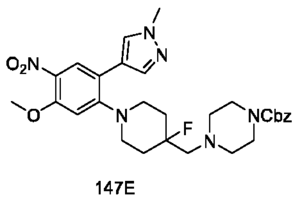

147E

**147D** (5 g, 8.84 mmol), 1-methyl-1H-pyrazole-4-boronic acid (1.67 g, 13.26 mmol), Pd(dppf)$Cl_2$·DCM (1.07 g, 1.33 mmol), potassium carbonate (2.44 g, 17.68 mmol), 1,4-dioxane (50 mL) and water (20 mL) were added to a reaction flask, and the mixture was subjected to nitrogen replacement three times, reacted at 100°C for about 3 h and cooled in a water bath. Ethyl acetate (100 mL) and water (50 mL) were added. The mixture was stirred, and the layers were separated. The organic layer was washed with saturated sodium chloride solution (50 mL × 2), dried over anhydrous sodium sulphate, filtered and concentrated to dryness under reduced pressure, and the residue was purified by column chromatography (eluent: DCM/$CH_3OH$ = DCM to 50/1 to 30/1 to obtain **147E** (4.1 g, yield: 81.85%).

Step 6: 4-(4-fluoro-4-(piperidin-4-ylmethyl)piperidin-1-yl)-2-methoxy-5-(1-methyl-1H-pyrazol-4-yl)aniline **(147F)**

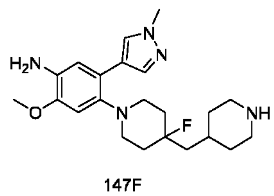

147F

**147E** (0.5 g, 0.88 mmol), Pd/C (0.1 g), methanol (5 mL) and glacial acetic acid (1 mL) were added to a reaction flask, and the mixture was subjected to hydrogen replacement three times, subjected to hydrogenation at room temperature for about 20 h and filtered over an appropriate amount of celite. The filter cake was washed with a small amount of methanol, and the filtrate was concentrated to dryness under reduced pressure to obtain **147F,** which was directly used in the next step.

Step 7: 5-(4-((1-(4-amino-5-methoxy-2-(1-methyl-1H-pyrazol-4-yl)phenyl)-4-fluoropiperidin-4-yl)methyl)piperazin-1-yl)-2-(2,6-dioxopiperidin-3-yl)isoindoline-1,3-dione **(147G)**

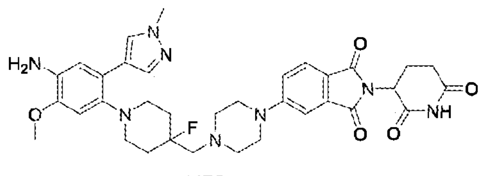

147G

**147F** (0.35 g, 0.88 mmol), 2-(2,6-dioxo-piperidin-3-yl)-5-fluoro-isoindole-1,3-dione (0.24 g, 0.88 mmol), DIPEA (0.17 g, 1.32 mmol) and DMSO (5 mL) were added to a reaction flask, and the mixture was reacted at 100°C for 3 h and cooled in ice water. Ethyl acetate (30 mL) and water (20 mL) were added, and the mixture was stirred for 5 min and allowed to stand for layer separation. The organic layer was washed with saturated sodium chloride solution (10 mL × 2), dried over anhydrous sodium sulphate, filtered and concentrated to dryness under reduced pressure, and the residue was purified by column chromatography (eluent: DCM/$CH_3OH$ = DCM to 50/1 to 30/1 to obtain **147G** (0.10 g, yield: 17.45%).

Step 8: 5-(4-((1-(4-((5-bromo-4-((4-cyclopropyl-2-(dimethylphosphoryl) phenyl)amino)pyrimidin-2-yl)amino)-5-methoxy-2-(1-methyl-1H-pyrazol-4-yl)phenyl)-4-fluoropiperidin-4-yl)methyl)piperazin-1-yl)-2-(2,6-dioxopiperidin-3-yl)isoindoline-1,3-dione **(compound 147)**

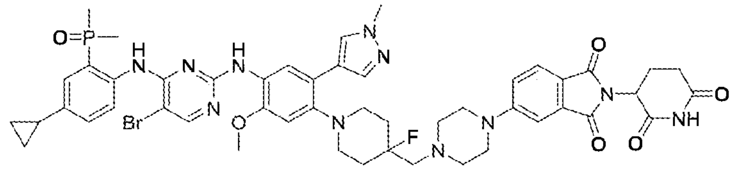

Compound 147

**147G** (0.10 g, 0.15 mmol), (2-((5-bromo-2-chloropyrimidin-4-yl)amino)-5-cyclopropylphenyl) dimethylphosphine oxide **(55B,** 0.06 g, 0.15 mmol), p-toluenesulfonic acid monohydrate (0.057 g, 0.30 mmol) and DMF (2 mL) were added to a reaction flask, and the mixture was reacted at 100°C for about 18 h. Under ice water cooling, dichloromethane (30 mL) and saturated sodium bicarbonate solution (20 mL) were added, and the mixture was stirred and allowed to stand for layer separation. The organic layer was washed with saturated sodium chloride solution (20 mL × 2), dried over anhydrous sodium sulphate, filtered and concentrated to dryness under reduced pressure, and the residue was purified by column chromatography (eluent: DCM/$CH_3OH$ = DCM to 50/1 to 30/1 to obtain **compound 147** (0.09 g, yield: 58.66%).

LCMS m/z = 511.7 $[(M+2H)/2]^+$.

$^1H$ NMR (400 MHz, DMSO-$d_6$) δ **11.06 (s, 1H), 10.84 (s, 1H), 8.22 -** 8.10 (m, 3H), 7.99 (s, 1H), 7.90 (s, 1H), 7.68 (d, 1H), 7.59 (s, 1H), 7.35 (s, 1H), 7.30 - 7.19 (m, 2H), 6.83 (s, 1H), 6.48 (s, 1H), 5.07 (dd, 1H), 3.84 (s, 3H), 3.80 (s, 3H), 3.47 (s, 4H), 2.98-2.82 (m, 5H), 2.76 - 2.52 (m, 8H), 2.09 - 1.93 (m, 4H), 1.91 - 1.67 (m, 8H), 0.94 - 0.83 (m, 2H), 0.56-0.48 (m, 2H).

**Example 148: Preparation of compound 148**

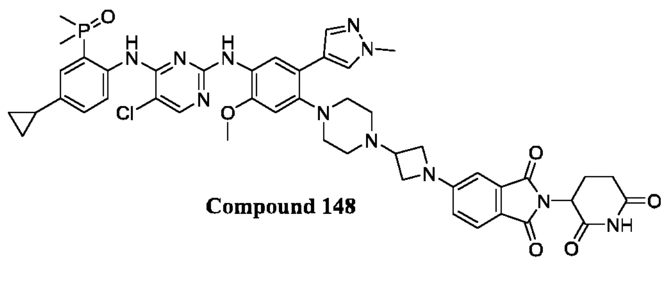

Compound 148

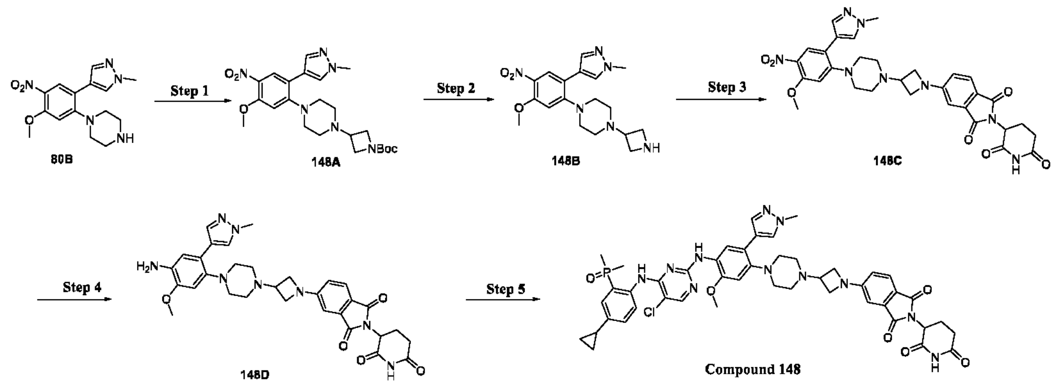


Step 1: tert-butyl 3-(4-(5-methoxy-2-(1-methyl-1H-pyrazol-4-yl)-4-nitrophenyl)piperazin-1-yl)azetidine-1-carboxylate **(148A)**

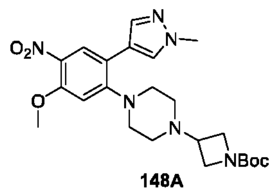

**148A**

**80B** (3.17 g, 9.99 mmol) and tert-butyl 3-oxoazetidine-1-carboxylate (1.71 g, 9.99 mmol) were dissolved in DCM (100 mL); acetic acid (1.71 mL, 29.97 mmol) was added; and the mixture was stirred at 40°C for 1 h, and then cooled to room temperature. Sodium triacetoxyborohydride (4.23 g, 19.98 mmol) was added, and the mixture was reacted at room temperature for 3 h. After the reaction was completed, the mixture was washed by adding 50 mL of saturated aqueous sodium bicarbonate solution. Liquid separation was performed. The organic phase was dried over anhydrous sodium sulphate and then concentrated under reduced pressure, and the residue was purified by silica gel column chromatography (mobile phase: dichloromethane/methanol (V/V) = 100/1-20/1) to obtain **148A** (2.42 g, yield: 51%).

LCMS m/z = 473.2 $[M+H]^+$.

Step 2: 1-(azetidin-3-yl)-4-(5-methoxy-2-(1-methyl-1H-pyrazol-4-yl)-4-nitrophenyl)piperazine **(148B)**

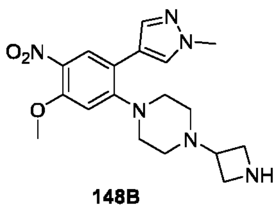

**148B**

**148A** (2.40 g, 5.08 mmol) was added to a flask. Under stirring, a solution of hydrochloric acid in dioxane (4 *N,* 13 mL) was slowly added, and the mixture was reacted at room temperature for 1 h and concentrated under reduced pressure. 20 mL of dichloromethane and a sodium bicarbonate solid (2.13 g, 25.4 mmol) were added to the residue, and the mixture was stirred at room temperature for 10 min and filtered to remove excess sodium bicarbonate solids. The filtrate was concentrated to obtain **148B** (1.5 g, yield: 79%).

Step 3: 2-(2,6-dioxopiperidin-3-yl)-5-(3-(4-(5-methoxy-2-(1-methyl-1H-pyrazol-4-yl)-4-nitrophenyl)piperazin-1-yl)azetidin-1-yl)isoindoline-1,3-dione **(148C)**

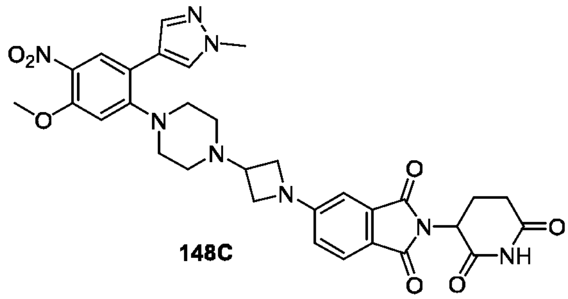

148C

**148B** (1.5 g, 4.03 mmol) was dissolved in DMSO (15 mL); 2-(2,6-dioxopiperidin-3-yl)-5-fluoroisoindoline-1,3-dione (1.11 g, 4.03 mmol) and DIPEA (2.0 mL, 12.09 mmol) were successively added; and the mixture was stirred at 90°C overnight, and cooled to room temperature. Saturated sodium bicarbonate solution was added, with a large amount of solids precipitated, and the mixture was filtered. The filter cake was dried under reduced pressure and then purified by silica gel column chromatography (mobile phase: dichloromethane/methanol (V/V) = 100/1-10/1) to obtain **148C** (1.35 g, yield: 53%).

LCMS m/z = 629.2 $[M+H]^+$.

Step 4: 5-(3-(4-(4-amino-5-methoxy-2-(1-methyl-1H-pyrazol-4-yl)phenyl) piperazin-1-yl)azetidin-1-yl)-2-(2,6-dioxopiperidin-3-yl)isoindoline-1,3-dione **(148D)**

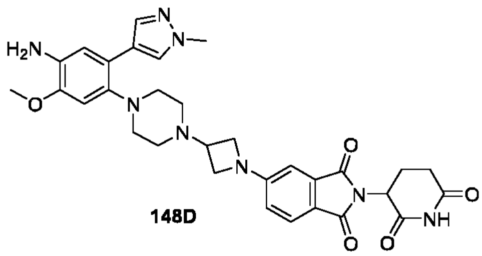

148D

**148C** (1.3 g, 2.07 mmol) was dissolved in tetrahydrofuran (20 mL), and a solution of ammonium chloride (1.11 g, 20.7 mmol) in water (5 mL) and zinc powder (1.35 g, 20.7 mmol) were successively added. After the addition, the mixture was stirred at 40°C for 0.5 h. After the reaction was completed, the mixture was cooled to room temperature and subjected to suction filtration under reduced pressure. The filtrate was extracted by adding dichloromethane (20 mL) and saturated aqueous sodium bicarbonate solution (10 mL). Liquid separation was performed. The organic phase was then dried over anhydrous sodium sulphate and filtered, and the filtrate was concentrated to obtain **148D** (900 mg, yield: 73%).

LCMS m/z = 599.2 $[M+H]^+$.

Step 5: 5-(3-(4-(4-((5-chloro-4-((4-cyclopropyl-2-(dimethylphosphoryl)phenyl) amino)pyrimidin-2-yl)amino)-5-methoxy-2-(1-methyl-1H-pyrazol-4-yl)phenyl)piperazin-1-yl)azetidin-1-yl)-2-(2,6-dioxopiperidin-3-yl)isoindoline-1,3-dione **(compound 148)**

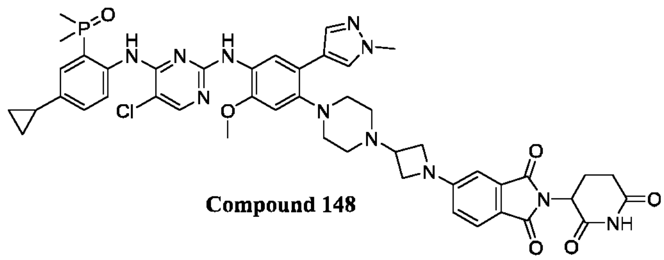

Compound 148

**148D** (200 mg, 0.33 mmol) and **107A** (120 mg, 0.33 mmol) were dissolved in DMF (8 mL); p-toluenesulfonic acid monohydrate (190 mg, 0.99 mmol) was added; and the mixture was stirred at 100°C for 16 h, and cooled to room temperature. Saturated aqueous sodium bicarbonate solution was added to the reaction solution, with a yellow solid precipitated, and the mixture was filtered to obtain a solid, which was purified by silica gel column chromatography (mobile phase: dichloromethane/methanol (V/V) = 100/1-10/1) to obtain a purified crude product. The crude product obtained from the column chromatography was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The collected preparative solution was adjusted to a basic pH by adding dichloromethane and saturated sodium bicarbonate solution. The organic phase was separated, dried over anhydrous sodium sulphate and then concentrated to obtain **compound 148** (60 mg, yield: 20%).

LCMS m/z = 459.8 $[(M+2H)/2]^+$.

$^1H$ NMR (400 MHz, DMSO-$d_6$) δ **11.10 (s, 1H), 11.05 (s, 1H), 8.29** - 8.23 (m, 1H), 8.13 (s, 1H), 8.07 (s, 1H), 8.00 (s, 1H), 7.89 (s, 1H), 7.66 (d, 1H), 7.62 (s, 1H), 7.22 (dd, 1H), 6.83 - 6.82 (m, 2H), 6.69 (dd, 1H), 6.51 - 6.40 (m, 1H), 5.08 - 5.03 (m, 1H), 4.16 (t, 2H), 3.91-3.88 (m, 2H), 3.83 (s, 3H), 3.79 (s, 3H), 3.52 - 3.44 (m, 1H), 2.99 - 2.84 (m, 5H), 2.64 - 2.51 (m, 6H), 2.06 - 1.96 (m, 1H), 1.87 - 1.78 (m, 1H), 1.75 (d, 6H), 0.92 - 0.83 (m, 2H), 0.58 - 0.41 (m, 2H).

**Example 149: Preparation of compound 149**

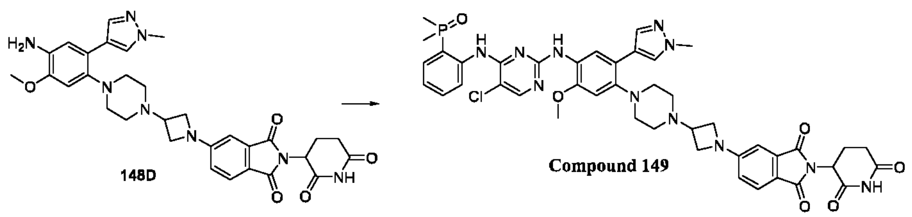

Step 1: 5-(3-(4-(4-((5-chloro-4-((2-(dimethylphosphoryl)phenyl)amino) pyrimidin-2-yl)amino)-5-methoxy-2-(1-methyl-1H-pyrazol-4-yl)phenyl)piperazin-1-yl)azetidin-1-yl)-2-(2,6-dioxopiperidin-3-yl)isoindoline-1,3-dione **(compound 149)**

**148D** (200 mg, 0.33 mmol) and (2-((2,5-dichloropyrimidin-4-yl)amino)phenyl)dimethylphosphine oxide **(109B)** (100 mg, 0.33 mmol) were dissolved in DMF (8 mL); p-toluenesulfonic acid monohydrate (190 mg, 0.99 mmol) was added; and the mixture was stirred at 100°C for 16 h, and cooled to room temperature. Saturated aqueous sodium bicarbonate solution was added to the reaction solution, with a yellow solid precipitated, and the mixture was filtered to obtain a solid, which was purified by silica gel column chromatography (mobile phase: dichloromethane/methanol (V/V) = 100/1-10/1) to obtain a purified crude product. The crude product obtained from the column chromatography was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The collected preparative solution was adjusted to a basic pH by adding dichloromethane and saturated sodium bicarbonate solution. The organic phase was separated, dried over anhydrous sodium sulphate and then concentrated to obtain **compound 149** (50 mg, yield: 17%).

LCMS m/z = 439.6 $[(M+2H)/2]^+$.

$^1H$ NMR (400 MHz, DMSO-$d_6$) δ **11.21 (s, 1H), 11.10** - 10.96 (m, 1H), 8.68 - 8.64 (m, 1H), 8.52 - 8.32 (m, 1H), 8.21 (s, 1H), 8.10 (s, 1H), 7.98 (s, 1H), 7.86 (s, 1H), 7.66 (d, 1H), 7.60 (s, 1H), 7.54 - 7.42 (m, 1H), 7.08 - 6.94 (m, 2H), 6.85 - 6.76 (m, 2H), 6.68 (dd, 1H), 5.06 (dd, 1H), 4.15 (t, 2H), 3.96 - 3.86 (m, 2H), 3.81 (d, 6H), 3.50 - 3.40 (m, 1H), 2.97 - 2.80 (m, 5H), 2.59 - 2.52 (m, 4H), 2.06 - 1.97 (m, 1H), 1.76 (d, 6H).

**Example 150: Preparation of compound 150**

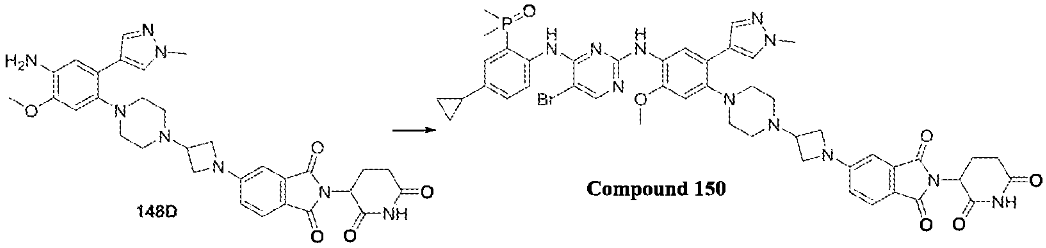

148D

Compound 150

Step 1: 5-(3-(4-(4-((5-bromo-4-((4-cyclopropyl-2-(dimethylphosphoryl) phenyl)amino)pyrimidin-2-yl)amino)-5-methoxy-2-(1-methyl-1H-pyrazol-4-yl)phenyl)piperazin-1-yl)azetidin-1-yl)-2-(2,6-dioxopiperidin-3-yl)isoindoline-1,3-dione **(compound 150)**

**148D** (200 mg, 0.33 mmol) and (2-((5-bromo-2-chloropyrimidin-4-yl)amino)-5-cyclopropylphenyl) dimethylphosphine oxide **(55B)** (130 mg, 0.5 mmol) were dissolved in DMF (8 mL); p-toluenesulfonic acid monohydrate (190 mg, 0.99 mmol) was added; and the mixture was stirred at 100°C for 16 h, and cooled to room temperature. Saturated aqueous sodium bicarbonate solution was added to the reaction solution, with a yellow solid precipitated, and the mixture was filtered to obtain a solid, which was purified by silica gel column chromatography (mobile phase: dichloromethane/methanol (V/V) = 100/1-10/1) to obtain a purified crude product. The crude product obtained from the column chromatography was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The collected preparative solution was adjusted to a basic pH by adding dichloromethane and saturated sodium bicarbonate solution. The organic phase was separated, dried over anhydrous sodium sulphate and then concentrated to obtain **compound 150** (50 mg, yield: 16%).

LCMS m/z = 481.7 $[(M+2H)/2]^+$.

$^1H$ NMR (400 MHz, DMSO-$d_6$) $\delta$ **11.05 (s, 1H), 10.84 (s, 1H), 8.23** - 8.10 (m, 3H), 7.98 (s, 1H), 7.88 (s, 1H), 7.66 (d, 1H), 7.61 (s, 1H), 7.21 (dd, 1H), 6.85 - 6.78 (m, 2H), 6.68 (dd, 1H), 6.57 - 6.37 (m, 1H), 5.06 (dd, 1H), 4.16 (t, 2H), 3.93 - 3.87 (m, 2H), 3.84 (s, 3H), 3.79 (s, 3H), 3.55 - 3.40 (m, 1H), 2.98 - 2.80 (m, 5H), 2.63 - 2.51 (m, 6H), 2.08 - 1.96 (m, 1H), 1.85 - 1.79 (m, 1H), 1.74 (d, 6H), 0.95 - 0.83 (m, 2H), 0.56 - 0.44 (m, 2H).

**Example 151: Preparation of compound 151**

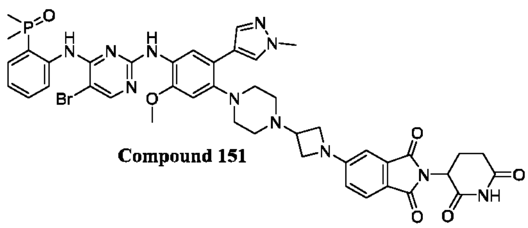

Compound 151

**Step 1:** 5-(3-(4-(4-((5-bromo-4-((2-(dimethylphosphoryl)phenyl)amino) pyrimidin-2-yl)amino)-5-methoxy-2-(1-methyl-1H-pyrazol-4-yl)phenyl)piperazin-1-yl)azetidin-1-yl)-2-(2,6-dioxopiperidin-3-yl)isoindoline-1,3-dione **(compound 151)**

**148D** (200 mg, 0.33 mmol) and (2-((5-bromo-2-chloropyrimidin-4-yl)amino)phenyl)dimethylphosphine oxide **(108B)** (120 mg, 0.33 mmol) were dissolved in DMF (8 mL); p-toluenesulfonic acid monohydrate (190 mg, 0.99 mmol) was added; and the mixture was stirred at 100°C for 16 h, and cooled to room temperature. Saturated aqueous sodium bicarbonate solution was added to the reaction solution, with a yellow solid precipitated, and the mixture was filtered to obtain a solid, which was purified by silica gel column chromatography (mobile phase: dichloromethane/methanol (V/V) = 100/1-10/1) to obtain a purified crude product. The crude product obtained from the column chromatography was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The collected preparative solution was adjusted to a basic pH by adding dichloromethane and saturated sodium bicarbonate solution. The organic phase was separated, dried over anhydrous sodium sulphate and then concentrated to obtain **compound 151** (50 mg, yield: 16%).

LCMS m/z = 461.6 $[(M+2H)/2]^+$.

$^1$H NMR (400 MHz, DMSO-$d_6$) δ **11.05 (s, 1H), 10.94 (s, 1H), 8.38** - 8.27 (m, 1H), 8.20 (s, 1H), 8.18 (s, 1H), 7.96 (s, 1H), 7.85 (s, 1H), 7.66 (d, 1H), 7.59 (s, 1H), 7.53 - 7.41 (m, 1H), 7.05 - 6.93 (m, 2H), 6.82 (s, 2H), 6.68 (dd, 1H), 5.06 (dd, 1H), 4.15 (t, 2H), 3.95 - 3.86 (m, 2H), 3.81 (d, 6H), 3.50 - 3.41 (m, 1H), 2.96 - 2.81 (m, 5H), 2.62 - 2.53 (m, 4H), 2.05 - 1.95 (m, 1H), 1.75 (d, 6H).

**Example 152: Preparation of compound 152**

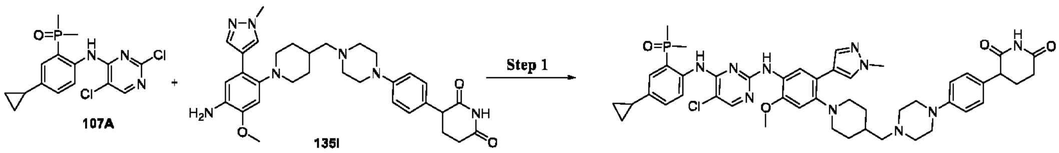


Compound 152

Step 1: 3-(4-(4-((1-(4-((5-chloro-4-((4-cyclopropyl-2-(dimethylphosphoryl)phenyl)amino)pyrimidin-2-yl)amino)-5-methoxy-2-(1-methyl-1H-pyrazol-4-yl)phenyl)piperidin-4-yl)methyl)piperazin-1-yl)phenyl)piperidine-2,6-dione **(compound 152)**

**135l** (165 mg, 0.29 mmol) and **1**07A (110 mg, 0.32 mmol) were dissolved in DMF (4 mL); p-toluenesulfonic acid monohydrate (170 mg, 0.87 mmol) was added; and the mixture was stirred at 100°C for 16 h, and cooled to room temperature. Saturated aqueous sodium bicarbonate solution was added to the reaction solution, with a yellow solid precipitated, and the mixture was filtered to obtain a solid, which was purified by silica gel column chromatography (mobile phase: dichloromethane/methanol (V/V) = 100/1-10/1) to obtain a purified crude product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The collected preparative solution was adjusted to a basic pH by adding dichloromethane and saturated sodium bicarbonate solution. The organic phase was separated, dried over anhydrous sodium sulphate and then concentrated to obtain **compound 152** (50 mg, yield: 19%).

LCMS m/z = 446.3 [(M+2H)/2]$^+$.

$^1$H NMR (400 MHz, DMSO-$d_6$) δ **11.19** - 11.04 (m, 1H), 10.75 (s, 1H), 8.33 - 8.21 (m, 1H), 8.14 (s, 1H), 8.06 (s, 1H), 7.99 (s, 1H), 7.88 (s, 1H), 7.59 (s, 1H), 7.23 (dd, 1H), 7.05 (d, 2H), 6.89 (d, 2H), 6.82 (s, 1H), 6.45 (s, 1H), 3.84 (s, 3H), 3.78 (s, 3H), 3.72 (dd, 1H), 3.18 - 3.03 (m, 6H), 2.68 - 2.57 (m, 3H), 2.55 - 2.51 (m, 5H), 2.34 - 2.26 (m, 2H), 2.20 - 2.07 (m, 1H), 2.04 - 1.94 (m, 1H), 1.85 - 1.78 (m, 3H), 1.75 (d, 6H), 1.70 - 1.60 (m, 1H), 1.42 - 1.26 (m, 2H), 0.93 - 0.85 (m, 2H), 0.53 - 0.46 (m, 2H).

**Example 153: Preparation of compound 153**

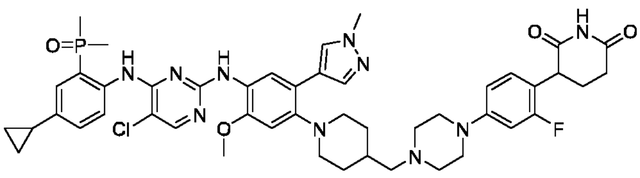

Compound 153

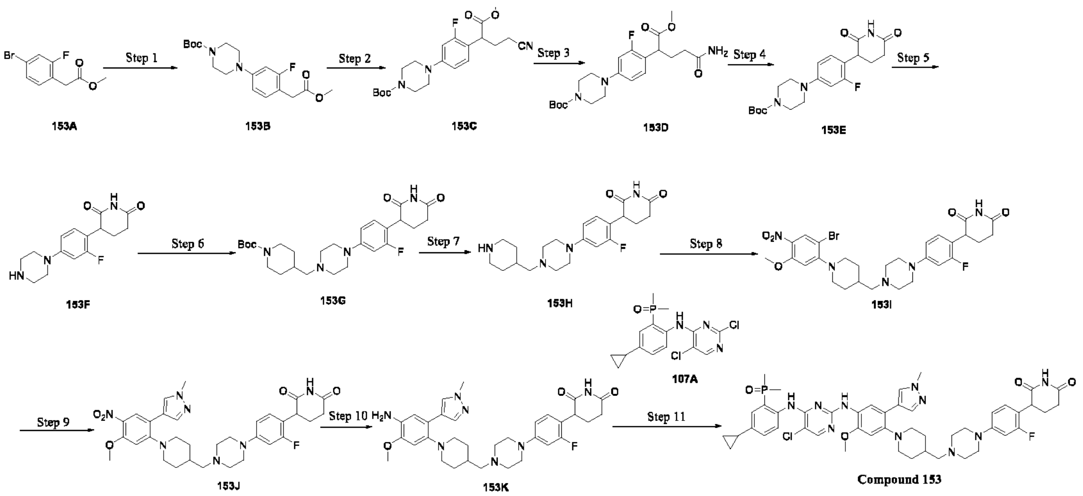


Step 1: tert-butyl 4-(3-fluoro-4-(2-methoxy-2-oxoethyl)phenyl)piperazine-1-carboxylate **(153B)**

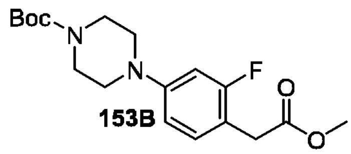

153B

**153A** (10.00 g, 40.48 mmol) and N-Boc-piperazine (11.31 g, 60.72 mmol) were dissolved in 1,4-dioxane (150 mL); palladium acetate (0.91 g, 4.05 mmol), (S)-BINAP (2.52 g, 4.05 mmol) and caesium carbonate (32.97 g, 101.20 mmol) were added; and under nitrogen protection, the mixture was reacted at 100°C for 16 h, cooled to room temperature and filtered over celite. The filter cake was washed 3 times with dichloromethane. The organic phases were collected and concentrated under reduced pressure, and the residue was subjected to flash column chromatography (mobile phase: petroleum ether/ethyl acetate (V/V) = 3/1) to obtain **153B** (12.30 g, yield: 86%)

LCMS m/z = 297.2[M-55]$^+$.

Step 2: tert-butyl 4-(4-(4-cyano-1-methoxy-1-oxobutan-2-yl)-3-fluorophenyl) piperazine-1-carboxylate **(153C)**

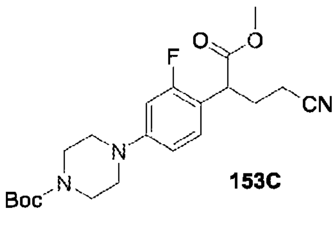

153C

**153B** (12.60 g, 35.75 mmol) and acrylonitrile (2.85 g, 53.63 mmol) were dissolved in toluene (100 mL); benzyltrimethylammonium hydroxide (0.30 g, 1.79 mmol) was added; and the mixture was reacted at room temperature for 16 h. The reaction solution was diluted with 500 mL of ethyl acetate and washed 3 times with water. The organic phase was dried over anhydrous sodium sulphate, and purified by flash column chromatography (mobile phase: petroleum ether/ethyl acetate (V/V) = 3/1) to obtain **153C** (5.00 g, yield: 34%).

Step 3: tert-butyl 4-(4-(5-amino-1-methoxy-1,5-dioxopentan-2-yl)-3-fluorophenyl)piperazine-1-carboxylate **(153D)**

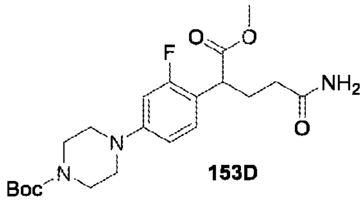

153D

**153C** (8.70 g, 21.46 mmol) was dissolved in toluene (80 mL); acetaldoxime(3.80 g, 64.38 mmol) and indium (III) chloride tetrahydrate (3.15 g, 10.73 mmol) were added; and the mixture was reacted at 130°C for 2 h. The reaction solution was cooled to room temperature and concentrated under reduced pressure, and the residue was purified by column chromatography (mobile phase: dichloromethane/methanol (V/V) = 50/1-20/1) to obtain **153D** (4.7 g, yield: 52%).

LCMS m/z = 368.2[M-55]$^+$.

Step 4: tert-butyl 4-(4-(2,6-dioxopiperidin-3-yl)-3-fluorophenyl)piperazine-1-carboxylate **(153E)**

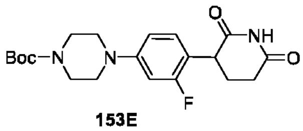

153E

**153D** (4.70 g, 11.10 mmol) was dissolved in acetonitrile (50 mL); benzyltrimethylammonium hydroxide (9.28 g, 22.20 mmol) was added; and the mixture was reacted at 60°C for 1 h. The reaction solution was cooled to room temperature, diluted by adding 500 mL of ethyl acetate, washed 3 times with water, washed once with saturated sodium chloride, dried over anhydrous sodium sulphate and concentrated under reduced pressure, and the residue was purified by column chromatography (mobile phase: dichloromethane/methanol (V/V) = 50/1-15/1) to obtain **153E** (3.4 g, yield: 78%).

LCMS m/z = 336.1[M-55]$^+$.

Step 5: 3-(2-fluoro-4-(piperazin-1-yl)phenyl)piperidine-2,6-dione **(153F)**

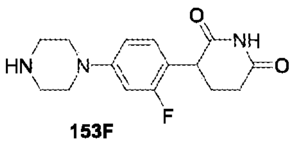

153F

**153E** (3.50 g, 8.94 mmol) was dissolved in methanol (30 mL); a solution of 1,4-dioxane in hydrochloric acid (10 mL, 4 mol/L) was added dropwise at room temperature; and the mixture was reacted at room temperature for 3 h and concentrated under reduced pressure to remove a solvent. The residue was then redissolved in 300 mL of dichloromethane, and adjusted to a basic pH with aqueous sodium bicarbonate solution. Liquid separation was performed. The organic phases were collected, and the aqueous phase was extracted twice with dichloromethane. The organic phases were combined, dried over anhydrous sodium sulphate and concentrated under reduced pressure to obtain **153F** (2.6 g), which was directly used in the next reaction.

LCMS m/z = 292.2[M+H]$^+$.

Step 6: tert-butyl 4-((4-(4-(2,6-dioxopiperidin-3-yl)-3-fluorophenyl)piperazin-1-yl)methyl)piperidine-1-carboxylate **(153G)**

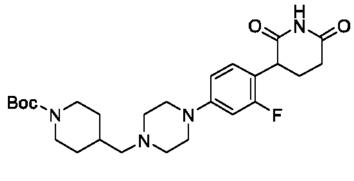

153G

**153F** (2.60 g, 8.92 mmol) and 1-tert-butoxycarbonylpiperidine-4-carbaldehyde (1.90 g, 8.92 mmol) were dissolved in dichloromethane (40 mL); acetic acid (2.14 g, 35.68 mmol) and sodium triacetoxyborohydride (3.78 g, 17.84 mmol) were successively added; and the mixture was reacted at room temperature for 1 h, adjusted to a basic pH by adding aqueous sodium bicarbonate solution and extracted 3 times with dichloromethane. The organic phases were combined, dried over anhydrous sodium sulphate and concentrated under reduced pressure, and the residue was purified by silica gel column chromatography (mobile phase: dichloromethane/methanol (V/V) = 20/1) to obtain **153G** (3.48 g, yield: 80%).

Step 7: 3-(2-fluoro-4-(4-(piperidin-4-ylmethyl)piperazin-1-yl)phenyl)piperidine-2,6-dione **(153H)**

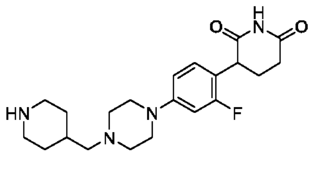

153H

**153G** (3.48 g, 7.12 mmol) was dissolved in dichloromethane (20 mL); a solution of 1,4-dioxane in hydrochloric acid (6 mL, 4 mol/L) was added dropwise at room temperature; and the mixture was reacted at room temperature for 3 h, concentrated under reduced pressure to remove a solvent and dissolved in 200 mL of methanol. A potassium carbonate solid was added, and the mixture was stirred for 30 min, adjusted to a basic pH and subjected to suction filtration. The filter cake was washed with dichloromethane. The organic phases were combined and concentrated under reduced pressure to obtain **153H** (2.7 g, yield: 98%).

LCMS m/z = 389.2$[M+H]^+$.

Step 8: 3-(4-(4-((1-(2-bromo-5-methoxy-4-nitrophenyl)piperidin-4-yl)methyl) piperazin-1-yl)-2-fluorophenyl)piperidine-2,6-dione **(compound 153I)**

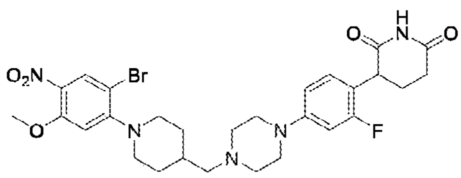

153I

**153H** (2.00 g, 5.15 mmol) and 1-bromo-2-fluoro-4-methoxy-5-nitrobenzene (1C) (1.42 g, 5.67 mmol) were dissolved in DMSO (50 mL); potassium carbonate (1.42 g, 10.30 mmol) was added; and the mixture was reacted at 100°C for 2 h. The reaction solution was cooled to room temperature, diluted by adding 200 mL of ethyl acetate, washed 3 times with water, washed once with saturated sodium chloride, dried over anhydrous sodium sulphate and concentrated under reduced pressure, and the residue was purified by column chromatography (mobile phase: dichloromethane/methanol (V/V) = 50/1-20/1) to obtain **153I** (2.4 g, yield: 75%).

LCMS m/z = 618.1$[M+H]^+$.

Step 9: 3-(2-fluoro-4-(4-((1-(5-methoxy-2-(1-methyl-1H-pyrazol-4-yl)-4-nitrophenyl)piperidin-4-yl)methyl)piperazin-1-yl)phenyl)piperidine-2,6-dione **(153J)**

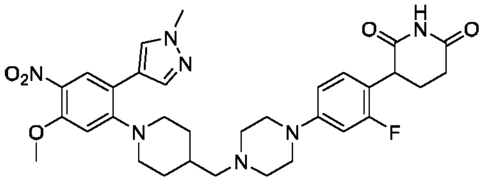

**153J**

**153I** (2.40 g, 3.88 mmol) and (1-methyl-1H-pyrazol-4-yl)boronic acid (0.78 g, 6.21 mmol) were dissolved in 1,4-dioxane (80 mL); Pd(dppf)$Cl_2$·DCM (0.32 g, 0.39 mmol) and an aqueous solution (10 mL) of potassium carbonate (1.07 g, 7.76 mmol) were added; and the mixture was subjected to nitrogen replacement 3 times and reacted at 80°C for 2 h. The reaction solution was cooled to room temperature, diluted by adding 200 mL of ethyl acetate, washed 3 times with water and washed once with saturated sodium chloride. The organic phase was dried over anhydrous sodium sulphate and concentrated under reduced pressure, and the residue was purified by column chromatography (mobile phase: petroleum ether/ethyl acetate (V/V) = 3/1-pure ethyl acetate) to obtain **153J** (1.58 g, yield: 66%).
LCMS m/z = 620.3 $[M+H]^+$.

Step 10: 3-(4-(4-((1-(4-amino-5-methoxy-2-(1-methyl-1H-pyrazol-4-yl) phenyl)piperidin-4-yl)methyl)piperazin-1-yl)-2-fluorophenyl)piperidine-2,6-dione **(153K)**

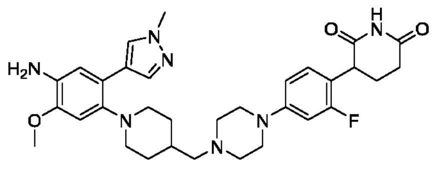

**153K**

**153J** (1.63 g, 2.63 mmol) was dissolved in tetrahydrofuran (30 mL); zinc powder (0.86 g, 13.15 mmol) was added, and then an aqueous solution (5 mL) of ammonium chloride (0.70 g, 13.15 mmol) was added dropwise; and the mixture was reacted at room temperature for 1 h. 5 mL of ammonia water and 20 mL of water were added to the reaction solution, and the mixture was extracted 3 times with dichloromethane. The organic phases were combined, dried over anhydrous sodium sulphate and then concentrated under reduced pressure to obtain **153K** (1.5 g), which was directly used in the next step.
LCMS m/z = 590.3 $[M+H]^+$.

Step 11: 3-(4-(4-((1-(4-((5-chloro-4-((4-cyclopropyl-2-(dimethylphosphoryl) phenyl)amino)pyrimidin-2-yl)amino)-5-methoxy-2-(1-methyl-1H-pyrazol-4-yl)phenyl)piperidin-4-yl)methyl)piperazin-1-yl)-2-fluorophenyl)piperidine-2,6-dione **(compound 153)**

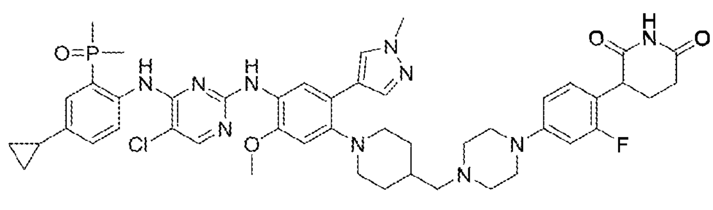

**Compound 153**

**153K** (0.40 g, 0.68 mmol) and **107A** (0.24 g, 0.68 mmol) were dissolved in DMF (20 mL); p-toluenesulfonic acid hydrate (0.39 g, 2.04 mmol) was added; and under nitrogen protection, the mixture was reacted at 100°C for 16 h. The reaction solution was cooled to room temperature. 50 mL of saturated aqueous sodium bicarbonate solution was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase: dichloromethane/methanol (V/V) = 50/1-15/1) to obtain a product, which was further purified by preparative HPLC (composition of mobile phases: mobile phase A: acetonitrile, and mobile phase B: water (containing 0.1% TFA)) to obtain a product, which was alkalized with aqueous sodium bicarbonate solution, extracted with dichloromethane, concentrated and dried to obtain **compound 153** (0.16 g, yield: 26%).

LCMS m/z = 455.2[(M+2H)/2]$^+$.

$^1$H NMR (400 MHz, DMSO-$d_6$) δ **11.10 (s, 1H), 10.78 (s, 1H), 8.30** - 8.22 (m, 1H), 8.14 (s, 1H), 8.06 (s, 1H), 7.99 (s, 1H), 7.88 (s, 1H), 7.59 (s, 1H), 7.23 (dd, 1H), 7.09 (t, 1H), 6.82 (s, 1H), 6.76 - 6.72 (m, 1H), 6.71 (s, 1H), 6.45 (s, 1H), 3.89 (dd, 1H), 3.84 (s, 3H), 3.78 (s, 3H), 3.19 - 3.14 (m, 4H), 3.09 (d, 2H), 2.77 - 2.67 (m, 1H), 2.63 (t, 2H), 2.54 - 2.48 (m, 5H), 2.29 (d, 2H), 2.20 - 2.08 (m, 1H), 2.01 - 1.92 (m, 1H), 1.85-1.64 (m, 3H), 1.76 (s, 3H), 1.73 (s, 3H), 1.71 - 1.63 (m, 1H), 1.39 - 1.29 (m, 2H), 0.93 - 0.86 (m, 2H), 0.53 - 0.43 (m, 2H).

**Example 154: Preparation of compound 154**

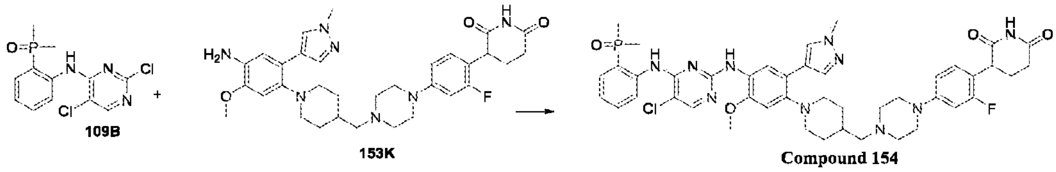


Step 1: 3-(4-(4-((1-(4-((5-chloro-4-((2-(dimethylphosphoryl)phenyl)amino) pyrimidin-2-yl)amino)-5-methoxy-2-(1-methyl-1H-pyrazol-4-yl)phenyl)piperidin-4-yl)methyl)piperazin-1-yl)-2-fluorophenyl)piperidine-2,6-dione **(compound 154)**

**153K** (0.40 g, 0.68 mmol) and **109B** (0.21 g, 0.68 mmol) were dissolved in DMF (20 mL); p-toluenesulfonic acid hydrate (0.39 g, 2.04 mmol) was added; and under nitrogen protection, the mixture was reacted at 100°C for 16 h. The reaction solution was cooled to room temperature. 50 mL of saturated aqueous sodium bicarbonate solution was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase: dichloromethane/methanol (V/V) = 50/1-15/1) to obtain a product, which was further purified by preparative HPLC (composition of mobile phases: mobile phase A: acetonitrile, and mobile phase B: water (containing 0.1% TFA)) to obtain a product, which was alkalized with aqueous sodium bicarbonate solution, extracted with dichloromethane, concentrated and dried to obtain **compound 154** (0.17 g, yield: 30%).

LCMS m/z = 435.3[(M+2H)/2]$^+$.

$^1$H NMR (400 MHz, DMSO-$d_6$) δ **11.21 (s, 1H), 10.78 (s, 1H),** 8.44 **(s, 1H),** 8.19 (s, 1H), 8.10 (s, 1H), 7.96 (s, 1H), 7.84 (s, 1H), 7.60 (s, 1H), 7.53 - 7.46 (m, 1H), 7.09 (t, 1H), 7.02 - 6.92 (m, 2H), 6.81 (s, 1H), 6.75 - 6.72 (m, 1H), 6.71 (s, 1H), 3.89 (dd, 1H), 3.83 (s, 3H), 3.79 (s, 3H), 3.19 - 3.13 (m, 4H), 3.09 (d, 2H), 2.77 - 2.66 (m, 1H), 2.61 (t, 2H), 2.55 - 2.45 (m, 5H), 2.28 (d, 2H), 2.20 - 2.08 (m, 1H), 2.00 - 1.92 (m, 1H), 1.83-1.72 (m, 2H), 1.78(s, 3H), 1.74 (s, 3H), 1.70 - 1.60 (m, 1H), 1.37 - 1.28 (m, 2H).

**Example 155: Preparation of compound 155**

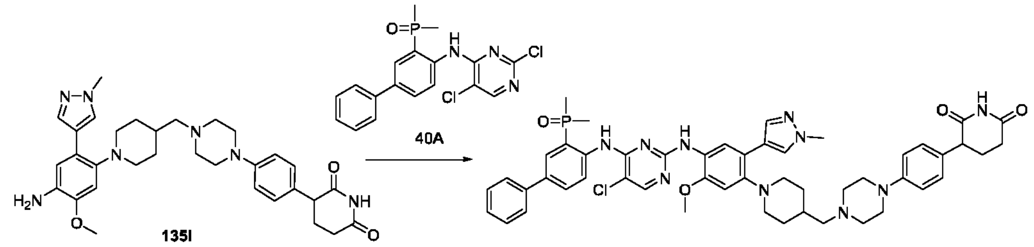

135I

40A

Compound 155

Step 1: 3-(4-(4-((1-(4-((5-chloro-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperidin-4-yl)methyl)piperazin-1-yl)phenyl)piperidine-2,6-dione **(compound 155)**

**135I** (165 mg, 0.29 mmol) and **40A** (130 mg, 0.32 mmol) were dissolved in DMF (4 mL); p-toluenesulfonic acid monohydrate (170 mg, 0.87 mmol) was added; and the mixture was stirred at 100°C for 16 h, and cooled to room temperature. Saturated aqueous sodium bicarbonate solution was added to the reaction solution, with a yellow solid precipitated, and the mixture was filtered to obtain a solid, which was purified by silica gel column chromatography (mobile phase: dichloromethane/methanol (V/V) = 100/1-10/1) to obtain the crude, which was further purified by preparative SFC (instrument: waters 2767 preparative chromatographic column; SunFire@ Prep C18 (19 mm × 150 mm); composition of mobile phases: mobile phase A: acetonitrile, and mobile phase B: water (containing 0.1% TFA)). The collected preparative solution was adjusted to a basic pH by adding dichloromethane and saturated sodium bicarbonate solution. The organic phase was separated, dried over anhydrous sodium sulphate and then concentrated to obtain **compound 155** (50 mg, yield: 19%).

LCMS m/z = 464.3 [(M+2H)/2] $^+$.

$^1$H NMR (400 MHz, DMSO-$d_6$) δ **11.37 (s, 1H), 10.75 (s, 1H), 8.56** - 8.41 (m, 1H), 8.29 (s, 1H), 8.11 (s, 1H), 8.03 (s, 1H), 7.87 (s, 1H), 7.71 (dd, 1H), 7.62 - 7.52 (m, 3H), 7.45 (t, 2H), 7.39 - 7.31 (m, 1H), 7.25 - 7.13 (m, 1H), 7.06 (d, 2H), 6.90 (d, 2H), 6.86 (s, 1H), 3.78 (d, 6H), 3.75 - 3.68 (m, 1H), 3.19 - 3.03 (m, 6H), 2.71 - 2.57 (m, 3H), 2.56 - 2.51 (m, 5H), 2.28 (d, 2H), 2.20 - 2.06 (m, 1H), 2.05 - 1.95 (m, 1H), 1.84 (d, 6H), 1.78 - 1.71 (m, 2H), 1.69 - 1.58 (m, 1H), 1.39 - 1.26 (m, 2H).

**Example 156: Preparation of compound 156**

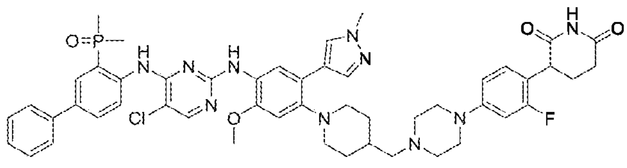

**Compound 156**

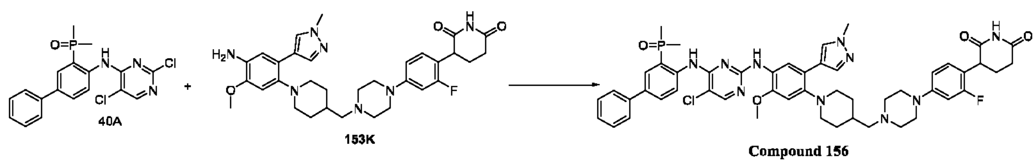

40A

153K

Compound 156

Step 1: 3-(4-(4-((1-(4-((5-chloro-4-((3-(dimethylphosphoryl)-[1,1'-biphenyl]-4-yl)amino)pyrimidin-2-yl)amino)-5-methoxy-2-(1-methyl-1H-pyrazol-4-yl)phenyl)piperidin-4-yl)methyl)piperazin-1-yl)-2-fluorophenyl)piperidine-2,6-dione **(compound 156)**

**153K** (0.40 g, 0.68 mmol) and **40A** (0.27 g, 0.68 mmol) were dissolved in DMF (20 mL); p-toluenesulfonic acid hydrate (0.39 g, 2.04 mmol) was added; and under nitrogen protection, the mixture was reacted at 100°C for 16 h. The reaction solution was cooled to room temperature. 50 mL of saturated aqueous sodium bicarbonate solution was added, with a yellow solid precipitated, and the mixture was subjected to suction filtration. The filter cake was washed 3 times

with water, redissolved in dichloromethane, dried over anhydrous sodium sulphate and concentrated under reduced pressure, and the residue was purified by column chromatography (mobile phase: dichloromethane/methanol (V/V) = 50/1-15/1) to obtain a product, which was further purified by preparative HPLC (composition of mobile phases: mobile phase A: acetonitrile, and mobile phase B: water (containing 0.1% TFA)) to obtain a product, which was alkalized with aqueous sodium bicarbonate solution, extracted with dichloromethane, concentrated and dried to obtain **compound 156** (0.11 g, yield: 17%).

LCMS m/z = 473.3 $[(M+2H)/2]^+$.

$^1H$ NMR (400 MHz, DMSO-$d_6$) δ 11.37 (s, 1H), 10.79 (s, 1H), 8.52 - 8.44 (m, 1H), 8.29 (s, 1H), 8.11 (s, 1H), 8.03 (s, 1H), 7.87 (s, 1H), 7.71 (dd, 1H), 7.58(d, 2H), 7.55 (s, 1H), 7.45 (t, 2H), 7.36 (t, 1H), 7.25 - 7.14 (m, 1H), 7.09 (t, 1H), 6.86 (s, 1H), 6.77 - 6.73 (m, 1H), 6.72 (s, 1H), 3.89 (dd, 1H), 3.79 (s, 3H), 3.76 (s, 3H), 3.20 - 3.15(m, 4H), 3.09 (d, 2H), 2.77 - 2.66 (m, 1H), 2.62 (t, 2H), 2.54 - 2.48 (m, 5H), 2.28 (d, 2H), 2.20 - 2.08 (m, 1H), 2.01 - 1.92 (m, 1H), 1.85 (s, 3H), 1.82 (s, 3H), 1.79-1.72 (m, 2H), 1.67 - 1.58 (m, 1H), 1.36 - 1.26 (m, 2H).

**Example 157: Preparation of compound 157**

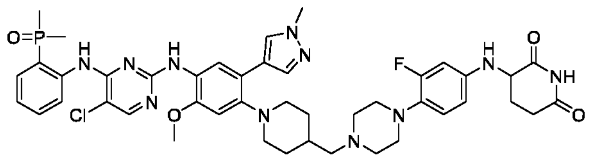

**Compound 157**

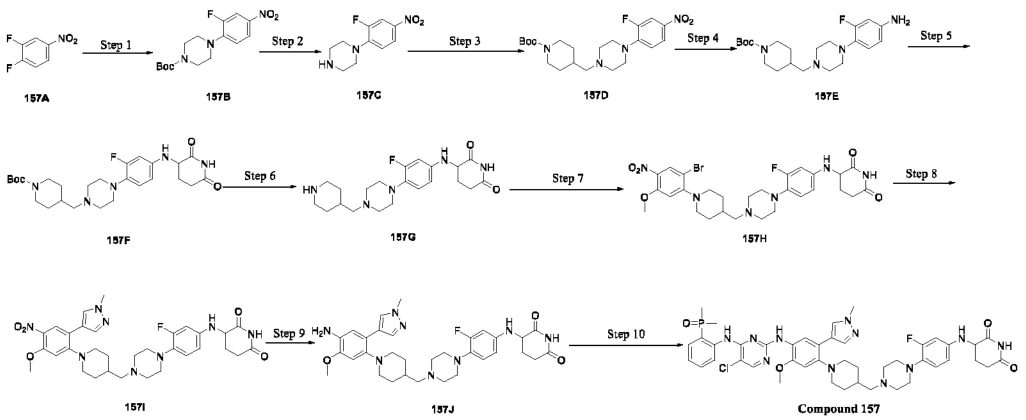


Step 1: tert-butyl 4-(2-fluoro-4-nitrophenyl)piperazine-1-carboxylate **(157B)**

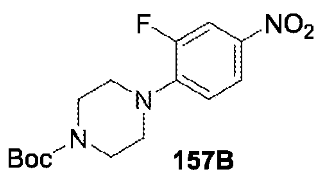

**157B**

**157A** (10.00 g, 62.86 mmol) and N-Boc-piperazine (11.71, 62.86 mmol) were dissolved in DMF (50 mL); potassium carbonate (17.38 g, 125.72 mmol) was added; and the mixture was reacted at 50°C for 12 h. The reaction solution was cooled to room temperature and poured into 200 mL of water, with a yellow solid precipitated, and the mixture was subjected to suction filtration. The filter cake was washed 3 times with water, redissolved in 300 mL of dichloromethane, dried over anhydrous sodium sulphate and concentrated under reduced pressure to obtain **157B** (20.0 g), which was directly used in the next reaction.

LCMS m/z = 270.1$[M-55]^+$.

Step 2: 1-(2-fluoro-4-nitrophenyl)piperazine **(157C)**

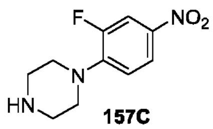

**157C**

**157B** (20.00 g, 61.47 mmol) was dissolved in dichloromethane (50 mL); a solution of 1,4-dioxane in hydrochloric acid (20 mL, 4 mol/L) was added dropwise at room temperature; and the mixture was reacted at room temperature for 1 h, concentrated under reduced pressure to remove a solvent, dissolved by adding a small amount of water, adjusted to a basic pH with aqueous sodium hydroxide solution and extracted 3 times with dichloromethane. The organic phases were combined, dried over anhydrous sodium sulphate and concentrated under reduced pressure to obtain **157C** (13.8 g, yield: 99%), which was directly used in the next reaction.

LCMS m/z = 226.1$[M+H]^+$.

Step 3: tert-butyl 4-((4-(2-fluoro-4-nitrophenyl)piperazin-1-yl)methyl) piperidine-1-carboxylate **(157D)**

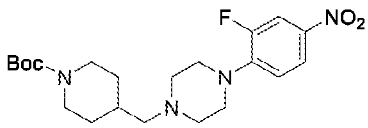

**157D**

**157C** (12.8 g, 56.83 mmol) and 1-tert-butoxycarbonylpiperidine-4-carbaldehyde (12.12, 56.83 mmol) were dissolved in dichloromethane (130 mL); acetic acid (13.65 g, 227.32 mmol) and sodium triacetoxyborohydride (24.09 g, 113.66 mmol) were successively added; and the mixture was reacted at room temperature for 4 h. The reaction solution was adjusted to a basic pH with aqueous sodium hydroxide solution. Liquid separation was performed. The organic phases were collected. The aqueous phase was extracted 3 times with dichloromethane, combined with the previous organic phases, dried over anhydrous sodium sulphate and concentrated under reduced pressure, and the residue was purified by silica gel column chromatography (mobile phase: petroleum ether/ethyl acetate (V/V) = 1/1 for removing small polar impurities, dichloromethane/methanol (V/V) = 20/1) to obtain **157D** (24.0 g, yield: 99%).

LCMS m/z = 423.2 $[M+H]^+$.

Step 4: tert-butyl 4-((4-(4-amino-2-fluorophenyl)piperazin-1-yl)methyl) piperidine-1-carboxylate **(157E)**

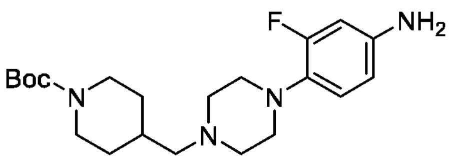

**157E**

**157D** (12.00 g, 28.40 mmol) was dissolved in ethyl acetate (250 mL); palladium on carbon (wt% = 10%, 5.00 g) was added; and the mixture was subjected to 1 atm hydrogen replacement 3 times, reacted at 30°C for 4 h and directly filtered over celite to remove palladium on carbon. The filter cake was washed with dichloromethane. The organic phases were combined and concentrated under reduced pressure to obtain **157E** (10.9 g, yield: 98%), which was directly used in the next step.

LCMS m/z = 393.3$[M+H]^+$.

Step 5: tert-butyl 4-((4-(4-((2,6-dioxopiperidin-3-yl)amino)-2-fluorophenyl) piperazin-1-yl)methyl)piperidine-1-carboxylate **(157F)**

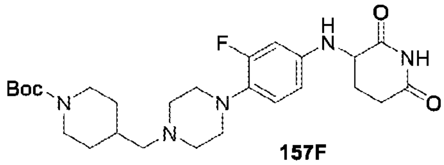

157F

In a 150 mL sealed tube, **157E** (3.50 g, 8.92 mmol) and the compound 3-bromopiperidin-2,6-dione (5.14 g, 26.76 mmol) were dissolved in DMF (60 mL); sodium bicarbonate (4.50 g, 53.52 mmol) was added; and the mixture was reacted at 90°C for 16 h under a sealed condition. The reaction solution was cooled to room temperature, diluted by adding 300 mL of ethyl acetate, washed 3 times with water and washed once with saturated brine. The organic phase was dried over anhydrous sodium sulphate and concentrated under reduced pressure, and the residue was purified by column chromatography (mobile phase: petroleum ether/ethyl acetate (V/V) = 1/1) to obtain **157F** (2.7 g, yield: 60%).
LCMS m/z = 504.3 $[M+H]^+$.

Step 6: 3-((3-fluoro-4-(4-(piperidin-4-ylmethyl)piperazin-1-yl)phenyl)amino) piperidine-2,6-dione **(compound 157G)**

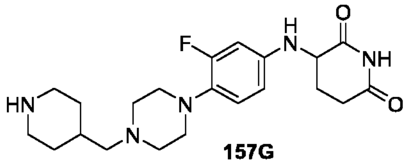

157G

**157F** (2.70 g, 5.36 mmol) was dissolved in dichloromethane (50 mL); a solution of 1,4-dioxane in hydrochloric acid (15 mL, 4 mol/L) was added dropwise at room temperature; and the mixture was reacted at room temperature for 0.5 h and concentrated under reduced pressure to remove a solvent. The residue was redissolved in 200 mL of methanol; a potassium carbonate solid was added; and the mixture was stirred for 30 min, adjusted to a basic pH and subjected to suction filtration. The filter cake was washed with dichloromethane. The organic phases were combined and concentrated under reduced pressure to obtain **157G** (2.16 g, yield: 99%).
LCMS m/z = 404.2$[M+H]^+$.

Step 7: 3-((4-(4-((1-(2-bromo-5-methoxy-4-nitrophenyl)piperidin-4-yl)methyl) piperazin-1-yl)-3-fluorophenyl)amino)piperidine-2,6-dione **(157H)**

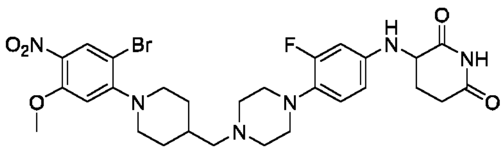

157H

**157G** (2.06, 5.11 mmol) and 1-bromo-2-fluoro-4-methoxy-5-nitrobenzene **(1C)** (1.28 g, 5.11 mmol) were dissolved in DMSO (50 mL); sodium bicarbonate (2.15 g, 25.55 mmol) was added; and the mixture was reacted at 100°C for 3 h. The reaction solution was cooled to room temperature, diluted by adding 200 mL of ethyl acetate, washed 3 times with water, washed once with saturated sodium chloride, dried over anhydrous sodium sulphate and concentrated under reduced pressure, and the residue was purified by column chromatography (mobile phase: dichloromethane/methanol (V/V) = 50/1-20/1) to obtain **157H** (1.71 g, yield: 53%).

LCMS m/z = 633.2[M+H]+.

Step 8: 3-((3-fluoro-4-(4-((1-(5-methoxy-2-(1-methyl-1H-pyrazol-4-yl)-4-nitrophenyl)piperidin-4-yl)methyl)piperazin-1-yl)phenyl)amino)piperidine-2, 6-dione **(compound 157I)**

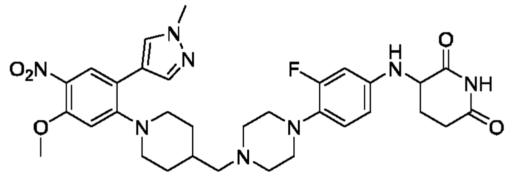

**157I**

**157H** (1.81 g, 2.86 mmol) and (1-methyl-1H-pyrazol-4-yl)boronic acid (0.58 g, 4.58 mmol) were dissolved in 1,4-dioxane (40 mL); Pd(dppf)$Cl_2$·DCM (0.23 g, 0.29 mmol) and an aqueous solution (5 mL) of potassium carbonate (0.79 g, 5.72 mmol) were added; and the mixture was subjected to nitrogen replacement 3 times and reacted at 80°C for 3 h. The reaction solution was cooled to room temperature, diluted by adding 200 mL of ethyl acetate, washed 3 times with water and washed once with saturated sodium chloride. The organic phase was dried over anhydrous sodium sulphate and concentrated under reduced pressure, and the residue was purified by column chromatography (mobile phase: petroleum ether/ethyl acetate (V/V) = 3/1-pure ethyl acetate) to obtain **157I** (0.43 g, yield: 24%).
LCMS m/z = 635.3 [M+H]+.

Step 9: 3-((4-(4-((1-(4-amino-5-methoxy-2-(1-methyl-1H-pyrazol-4-yl)phenyl)piperidin-4-yl)methyl)piperazin-1-yl)-3 -fluorophenyl)amino)piperidine-2,6-dione **(157J)**

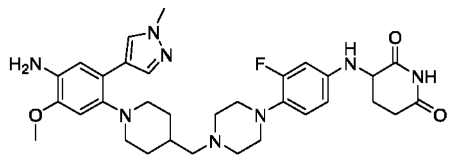

**157J**

**157I** (0.43 g, 0.67 mmol) was dissolved in tetrahydrofuran (18 mL); zinc powder (0.22 g, 3.35 mmol) was added, and then an aqueous solution (3 mL) of ammonium chloride (0.18 g, 3.35 mmol) was added dropwise; and the mixture was reacted at room temperature for 0.5 h. 2 mL of ammonia water and 10 mL of water were added to the reaction solution, and the mixture was extracted 3 times with dichloromethane. The organic phases were combined, dried over anhydrous sodium sulphate and then concentrated under reduced pressure to obtain **157J** (0.41 g).
LCMS m/z = 605.3 [M+H]+.

Step 10: 3-((4-(4-((1-(4-((5-chloro-4-((2-(dimethylphosphoryl)phenyl)amino) pyrimidin-2-yl)amino)-5-methoxy-2-(1-methyl-1H-pyrazol-4-yl)phenyl)piperidin-4-yl)methyl)piperazin-1-yl)-3-fluorophenyl)amino)piperidine-2,6-dione **(compound 157)**

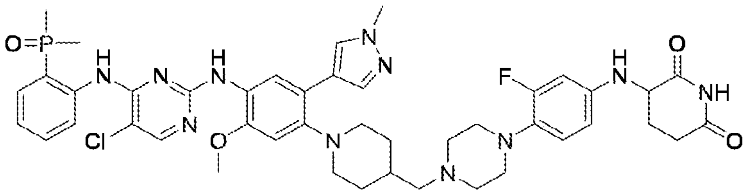

**Compound 157**

**157J** (0.13 g, 0.22 mmol) and **109B** (0.07 g, 0.22 mmol) were dissolved in DMF (8 mL); p-toluenesulfonic acid hydrate (0.13 g, 0.66 mmol) was added; and under nitrogen protection, the mixture was reacted at 100°C for 16 h. The reaction solution was cooled to room temperature. 50 mL of saturated aqueous sodium bicarbonate solution was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase: dichloromethane/methanol (V/V) = 50/1-15/1) to obtain a product, which was further purified by preparative HPLC (composition of mobile phases: mobile phase A: acetonitrile, and mobile phase B: water (containing 0.1% TFA)) to obtain a product, which was alkalized with aqueous sodium bicarbonate solution, extracted with dichloromethane, concentrated and dried to obtain **compound 157** (10 mg, yield: 5%).

LCMS m/z = 442.7[(M+2H)/2]$^+$.

$^1H$ NMR (400 MHz, DMSO-$d_6$) δ 11.20 (s, 1H), 10.75 (s, 1H), 8.48 - 8.39 (m, 1H), 8.19 (s, 1H), 8.10 (s, 1H), 7.97 (s, 1H), 7.84 (s, 1H), 7.60 (s, 1H), 7.50 (dd, 1H), 7.04 - 6.93 (m, 2H), 6.84 (d, 1H), 6.81 (s, 1H), 6.51 (dd, 1H), 6.43 (dd, 1H), 5.78 (d, 1H), 4.29 - 4.21 (m, 1H), 3.83 (s, 3H), 3.79 (s, 3H), 3.08 (d, 2H), 2.90 - 2.82 (m, 4H), 2.78 - 2.68 (m, 1H), 2.66 - 2.54 (m, 4H), 2.54 - 2.45 (m, 2H), 2.27 (d, 2H), 2.13 - 2.05 (m, 1H), 1.90 - 1.82 (m, 1H), 1.82 - 1.70 (m, 9H), 1.70 - 1.60 (m, 1H), 1.31 (d, 2H).

**Example 158: Preparation of compound 158**

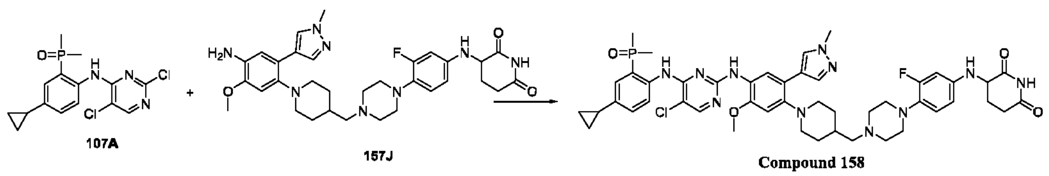

107A

157J

Compound 158

Step 1: 3-((4-(4-((1-(4-((5-chloro-4-((4-cyclopropyl-2-(dimethylphosphoryl) phenyl)amino)pyrimidin-2-yl)amino)-5-methoxy-2-(1-methyl-1H-pyrazol-4-yl)phenyl)piperidin-4-yl)methyl)piperazin-1-yl)-3 -fluorophenyl)amino)piperidine-2,6-dione **(compound 158)**

**157J** (0.13 g, 0.22 mmol) and **107A** (0.08 g, 0.22 mmol) were dissolved in DMF (8 mL); p-toluenesulfonic acid hydrate (0.13 g, 0.66 mmol) was added; and under nitrogen protection, the mixture was reacted at 100°C for 16 h. The reaction solution was cooled to room temperature. 50 mL of saturated aqueous sodium bicarbonate solution was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase: dichloromethane/methanol (V/V) = 50/1-15/1) to obtain a product, which was further purified by preparative HPLC (composition of mobile phases: mobile phase A: acetonitrile, and mobile phase B: water (containing 0.1% TFA)) to obtain a product, which was alkalized with aqueous sodium bicarbonate solution, extracted with dichloromethane, concentrated and dried to obtain **compound 158** (10 mg, yield: 5%).

LCMS m/z = 462.8[(M+2H)/2]$^+$.

$^1H$ NMR (400 MHz, DMSO-$d_6$) δ 11.10 (s, 1H), 10.75 (s, 1H), 8.30 - 8.20 (m, 1H), 8.14 (s, 1H), 8.06 (s, 1H), 7.99 (s, 1H), 7.88 (s, 1H), 7.59 (s, 1H), 7.23 (dd, 1H), 6.87 - 6.79 (m, 2H), 6.51 (dd, 1H), 6.48 - 6.38 (m, 2H), 5.79 (d, 1H), 4.29 - 4.19 (m, 1H), 3.84 (s, 3H), 3.78 (s, 3H), 3.08 (d, 2H), 2.88 (s, 4H), 2.77 - 2.68 (m, 1H), 2.67 - 2.50 (m, 6H), 2.36 - 2.23 (m, 2H), 2.13 - 2.05 (m, 1H), 2.04 - 1.85 (m, 1H), 1.85 - 1.77 (m, 4H), 1.77 (s, 3H), 1.73 (s, 3H), 1.71 - 1.61 (m, 1H), 1.40 - 1.28 (m, 2H), 0.93 - 0.87 (m, 2H), 0.54 - 0.48 (m, 2H).

**Example 159: Preparation of compound 159**

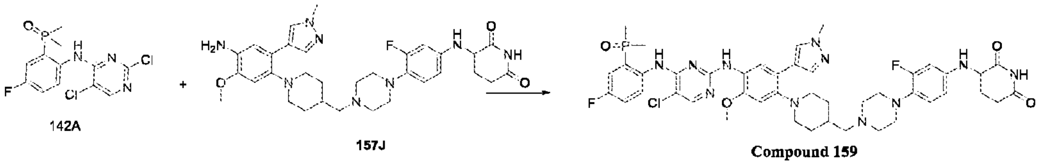

142A 157J Compound 159

Step 1: 3-((4-(4-((1-(4-((5-chloro-4-((2-(dimethylphosphoryl)-4-fluorophenyl) amino)pyrimidin-2-yl)amino)-5-methoxy-2-(1-methyl-1H-pyrazol-4-yl)phenyl)piperidin-4-yl)methyl)piperazin-1-yl)-3 -fluorophenyl)amino)piperidine-2,6-dione **(compound 159)**

**157J** (0.13 g, 0.22 mmol) and **142A** (0.07 g, 0.22 mmol) were dissolved in DMF (8 mL); p-toluenesulfonic acid hydrate (0.13 g, 0.66 mmol) was added; and under nitrogen protection, the mixture was reacted at 100°C for 16 h. The reaction solution was cooled to room temperature. 50 mL of saturated aqueous sodium bicarbonate solution was added, with a yellow solid precipitated, and the mixture was subjected to suction filtration. The filter cake was washed 3 times with water, redissolved in dichloromethane, dried over anhydrous sodium sulphate and concentrated under reduced pressure, and the residue was purified by column chromatography (mobile phase: dichloromethane/methanol (V/V) = 50/1-15/1) to obtain a product, which was further purified by preparative HPLC (composition of mobile phases: mobile phase A: acetonitrile, and mobile phase B: water (containing 0.1% TFA)) to obtain a product, which was alkalized with aqueous sodium bicarbonate solution, extracted with dichloromethane, concentrated and dried to obtain **compound 159** (12 mg, yield: 6%).

LCMS m/z = 451.8[$(M+2H)/2]^+$.

$^1H$ NMR (400 MHz, DMSO-$d_6$) δ 11.06 (s, 1H), 10.76 (s, 1H), 8.41 (s, 1H), 8.18 (s, 1H), 8.10 (s, 1H), 8.00 (s, 1H), 7.82 (s, 1H), 7.61 (s, 1H), 7.46 - 7.38 (m, 1H), 6.87 - 6.78 (m, 2H), 6.75 - 6.59 (m, 1H), 6.51 (dd, 1H), 6.42 (d, 1H), 5.79 (d, 1H), 4.31 - 4.20 (m, 1H), 3.84 (s, 3H), 3.79 (s, 3H), 3.12 - 3.04 (m, 2H), 2.91 - 2.82 (m, 4H), 2.78 - 2.66 (m, 1H), 2.65 - 2.52 (m, 4H), 2.49 - 2.43 (m, 2H), 2.28 (d, 2H), 2.14 - 2.04 (m, 1H), 2.04 - 1.73 (m, 10H), 1.70 - 1.59 (m, 1H), 1.35 - 1.28 (m, 2H).

**Example 160: Preparation of compound 160**

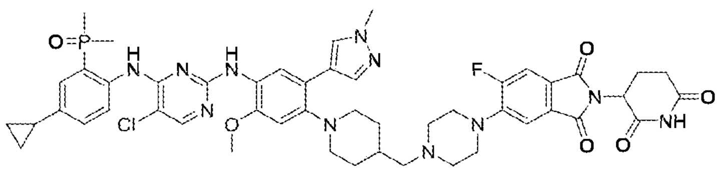

Compound 160

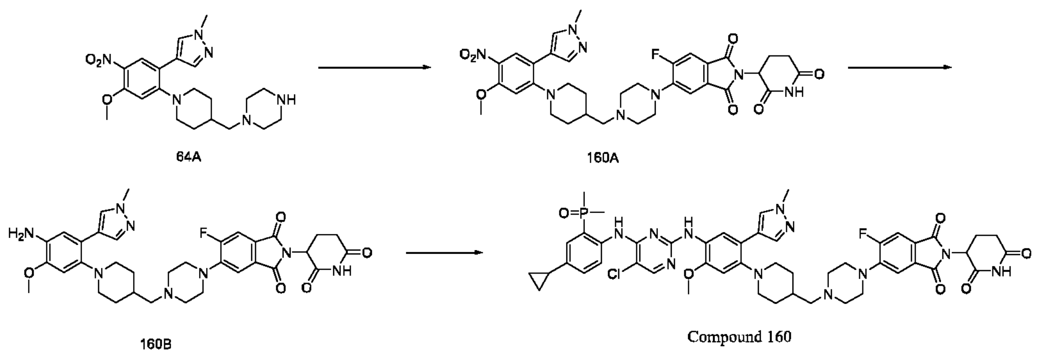

64A 160A 160B Compound 160

Step 1: 2-(2,6-dioxopiperidin-3-yl)-5-fluoro-6-(4-((1-(5-methoxy-2-(1-methyl-1H-pyrazol-4-yl)-4-nitrophenyl)piperidin-4-yl)methyl)piperazin-1-yl)isoindoline-1,3-dione **(160A)**

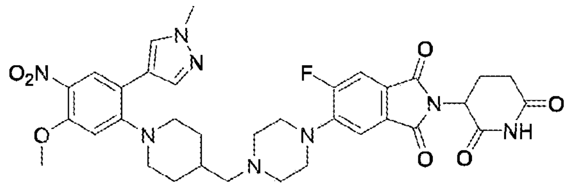

160A

**64A** (1.5 g, 3.62 mmol), 2-(2,6-dioxopiperidin-3-yl)-5,6-difluoroisoindoline-1,3-dione (1.07 g, 3.62 mmol), DIPEA (0.70 g, 5.43 mmol) and DMSO (15 mL) were added to a reaction flask, and the mixture was reacted at 90°C for 3 h and cooled in ice water. Ethyl acetate (100 mL) and water (100 mL) were added, and the mixture was stirred for 5 min and allowed to stand for layer separation. The organic layer was washed with saturated sodium chloride solution (100 mL × 2), dried over anhydrous sodium sulphate, filtered and concentrated to dryness under reduced pressure, and the residue was purified by column chromatography (eluent: DCM/$CH_3OH$ = DCM to 50/1 to 20/1) to obtain **160A** (1.7 g, yield: 68.19%).

Step 2: 5-(4-((1-(4-amino-5-methoxy-2-(1-methyl-1H-pyrazol-4-yl)phenyl) piperidin-4-yl)methyl)piperazin-1-yl)-2-(2,6-dioxopiperidin-3-yl)-6-fluoroisoindoline-1,3-dione **(160B)**

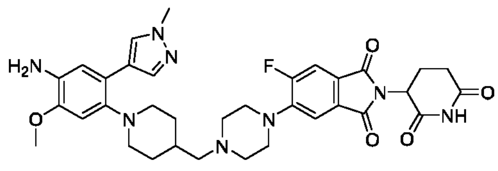

160B

**160A** (0.5 g, 0.73 mmol), zinc powder (0.24 g, 3.63 mmol), ammonium chloride (0.19 g, 3.63 mmol), tetrahydrofuran (6 mL) and water (2 mL) were added to a reaction flask, and the mixture was reacted at room temperature for 1 h. Dichloromethane (50 mL) and ammonia water (10 mL) were added, and the mixture was stirred for 2 min, subjected to suction filtration over an appropriate amount of celite and washed with an appropriate amount of dichloromethane. The layers in the filtrate were separated. The organic layer was washed with water (20 mL × 1), dried over anhydrous sodium sulphate and filtered, and the filtrate was concentrated under reduced pressure to obtain **160B** (0.40 g, yield: 83.18%).

Step 3: 5-(4-((1-(4-((5-chloro-4-((4-cyclopropyl-2-(dimethylphosphoryl) phenyl)amino)pyrimidin-2-yl)amino)-5-methoxy-2-(1-methyl-1H-pyrazol-4-yl)phenyl)piperidin-4-yl)methyl)piperazin-1-yl)-2-(2,6-dioxopiperidin-3-yl)-6-fluoroisoindoline-1,3-dione **(compound 160)**

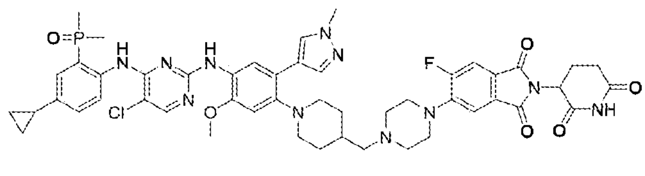

Compound 160

**160B** (0.4 g, 0.61 mmol), **107A** (0.22 g, 0.61 mmol), p-toluenesulfonic acid monohydrate (0.23 g, 1.22 mmol)

and DMF (5 mL) were added to a reaction flask, and the mixture was reacted at 100°C for about 18 h. Under ice water cooling, ethyl acetate (20 mL) and saturated sodium bicarbonate solution (20 mL) were added, and the mixture was stirred and allowed to stand for layer separation. The organic layer was washed with saturated sodium chloride solution (20 mL × 2), dried over anhydrous sodium sulphate, filtered and concentrated to dryness under reduced pressure, and the residue was purified by column chromatography (eluent: DCM/$CH_3OH$ = 50/1 to 30/1 to 20/1. The resulting crude was subjected to preparative HPLC (instrument: waters 2767 (preparative liquid phase chromatographic instrument); chromatographic column: XBridge@ Prep C18 (30 mm × 150 mm); composition of mobile phases: mobile phase A: acetonitrile, and mobile phase B: water (containing 0.1% TFA)) purification to obtain a preparative product, which was alkalized with saturated aqueous sodium bicarbonate solution to obtain **compound 160** (0.11 g, yield: 18.43%).

LCMS m/z = 489.8 $[(M+2H)/2]^+$.

$^1H$ NMR (400 MHz, DMSO-$d_6$) δ 11.15-11.03 (m, 2H), 8.31 - 8.23 (m, 1H), 8.13 (s, 1H), 8.07 (s, 1H), 7.99 (s, 1H), 7.89 (s, 1H), 7.72 (d, 1H), 7.60 (s, 1H), 7.46 (d, 1H), 7.24 (dd, 1H), 6.82 (s, 1H), 6.45 (s, 1H), 5.10 (dd, 1H), 3.84 (s, 3H), 3.78 (s, 3H), 3.27 (s, 4H), 3.15-3.04 (m, 2H), 2.95-2.83 (m, 1H), 2.68 - 2.51 (m, 8H), 2.31 (d, 2H), 2.05 - 1.97 (m, 2H), 1.89 - 1.56 (m, 11H),1.42-1.30(m, 1H), 0.95 - 0.87 (m, 2H), 0.54-0.47 (m, 2H).

**Example 161: Preparation of compound 161**

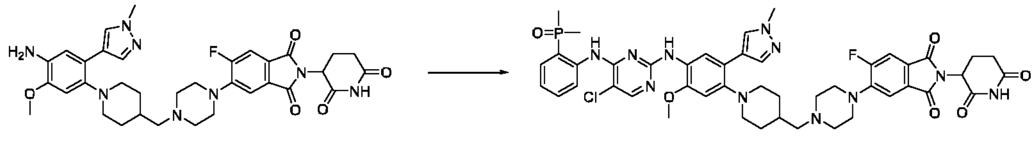

160B

Compound 161

Step 1: 5-(4-((1-(4-((5-chloro-4-((2-(dimethylphosphoryl)phenyl)amino) pyrimidin-2-yl)amino)-5-methoxy-2-(1-methyl-1H-pyrazol-4-yl)phenyl)piperidin-4-yl)methyl)piperazin-1-yl)-2-(2,6-dioxopiperidin-3-yl)-6-fluoroisoindoline-1,3-dione **(compound 161)**

**160B** (0.41 g, 0.64 mmol), **109B** (0.2 g, 0.64 mmol), p-toluenesulfonic acid monohydrate (0.24 g, 1.28 mmol) and DMF (5 mL) were added to a reaction flask, and the mixture was reacted at 100°C for about 18 h. Under ice water cooling, ethyl acetate (20 mL) and saturated sodium bicarbonate solution (20 mL) were added, and the mixture was stirred and allowed to stand for layer separation. The organic layer was washed with saturated sodium chloride solution (20 mL × 2), dried over anhydrous sodium sulphate, filtered and concentrated to dryness under reduced pressure, and the residue was purified by column chromatography (eluent: DCM/$CH_3OH$ = 50/1 to 30/1 to 20/1. The resulting crude was further subjected to preparative HPLC (instrument: waters 2767 (preparative liquid phase chromatographic instrument); chromatographic column: XBridge@ Prep C18 (30 mm × 150 mm); composition of mobile phases: mobile phase A: acetonitrile, and mobile phase B: water (containing 0.1% TFA)) purification to obtain a preparative product, which was alkalized with saturated aqueous sodium bicarbonate solution to obtain **compound 161** (0.22 g, yield: 36.63%).

LCMS m/z = 469.7 $[(M+2H)/2]^+$.

$^1H$ NMR (400 MHz, DMSO-$d_6$) δ **11.21** (s, 1H), **11.09** (s, 1H), 8.52 - 8.36 (m, 1H), 8.28 - 8.14 (m, 1H), 8.14 - 8.07 (m, 1H), 8.02 - 7.90 (m, 1H), 7.90 - 7.78 (m, 1H), 7.76 - 7.40 (m, 4H), 7.08 - 6.90 (m, 2H), 6.86 - 6.73 (m, 1H), 5.16 - 5.05 (m, 1H), 3.93 - 3.73 (m, 6H), 3.33 - 3.17 (m, 4H), 3.15 - 3.00 (m, 2H), 2.98 - 2.79 (m, 1H), 2.70 - 2.45 (m, 8H), 2.40 - 2.20 (m, 2H), 2.08 - 1.99 (m, 1H), 1.90 - 1.50 (m, 9H), 1.42 - 1.22 (m, 2H).

**Example 162: Preparation of compound 162**

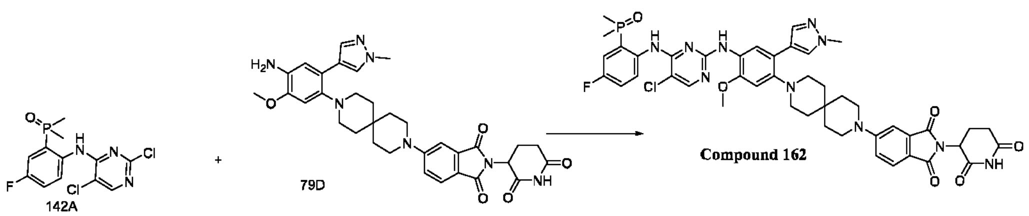

142A

79D

Compound 162

Step 1: 5-(9-(4-((5-chloro-4-((2-(dimethylphosphoryl)-4-fluorophenyl)amino) pyrimidin-2-yl)amino)-5-methoxy-2-(1-methyl-1H-pyrazol-4-yl)phenyl)-3,9-diazaspiro[5.5]undecan-3-yl)-2-(2,6-dioxopiperidin-3-yl)isoindoline-1,3-dione **(compound 162)**

**79D** (397.60 mg, 0.65 mmol) and **142A** (238.89 mg, 0.72 mmol) were dissolved in DMF (8 mL); p-toluenesulfonic acid monohydrate (370.93 mg, 1.95 mmol) was added; and under nitrogen protection, the mixture was reacted overnight at 100°C, and cooled to room temperature. 20 mL of water and 20 mL of saturated aqueous sodium bicarbonate solution were added, and the mixture was subjected to suction filtration. The filter cake was dissolved in dichloromethane and extracted. The organic phase was dried over anhydrous sodium sulphate and then concentrated under reduced pressure, and the residue was purified by silica gel column chromatography (dichloromethane/methanol (V/V) = 100/1-15/1) to obtain **compound 162** (140 mg, yield: 23.69%).

LCMS m/z = 909.3 $[M+H]^+$.

$^1$H NMR (400 MHz, DMSO-$d_6$) δ 11.05 (s, 2H), 8.42 (br.s, 1H), 8.17 (s, 1H), 8.10 (s, 1H), 8.03 (s, 1H), 7.85 (s, 1H), 7.70 - 7.57 (m, 2H), 7.48 - 7.17 (m, 3H), 6.87 (s, 1H), 6.67 (br.s, 1H), 5.11 - 5.00 (m, 1H), 3.93 - 3.75 (m, 6H), 3.60 - 3.40 (m, 4H), 3.00 - 2.75 (m, 5H), 2.70 - 2.50 (m, 2H), 2.10 - 1.92 (m, 1H), 1.88 - 1.72 (m, 6H), 1.72 - 1.52 (m, 8H).

**Example 163: Preparation of compound 163**

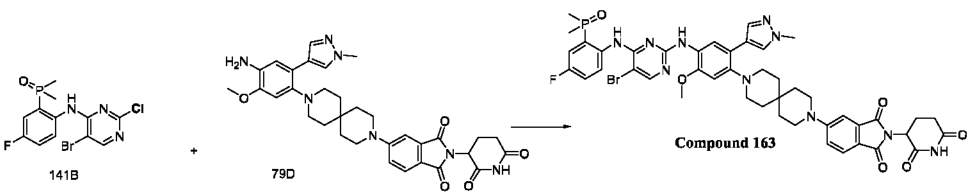

141B + 79D → Compound 163

Step 1: 5-(9-(4-((5-bromo-4-((2-(dimethylphosphoryl)-4-fluorophenyl)amino) pyrimidin-2-yl)amino)-5-methoxy-2-(1-methyl-1H-pyrazol-4-yl)phenyl)-3,9-diazaspiro[5.5]undecan-3-yl)-2-(2,6-dioxopiperidin-3-yl)isoindoline-1,3-dione **(compound 163)**

**79D** (400 mg, 0.65 mmol) and **141B** (270.67 mg, 0.72 mmol) were dissolved in DMF (8 mL); p-toluenesulfonic acid monohydrate (370.93 mg, 1.95 mmol) was added; and under nitrogen protection, the mixture was reacted overnight at 100°C, and cooled to room temperature. 20 mL of water and 20 mL of saturated aqueous sodium bicarbonate solution were added, and the mixture was subjected to suction filtration. The filter cake was dissolved in DCM and extracted. The organic phase was dried over anhydrous sodium sulphate and then concentrated under reduced pressure, and the residue was purified by silica gel column chromatography (dichloromethane/methanol (V/V) = 100/1-15/1) to obtain **compound 163** (150 mg, yield: 24.19%).

LCMS m/z = 953.3 $[M+H]^+$.

$^1$H NMR (400 MHz, DMSO-$d_6$) δ 11.06 (s, 1H), 10.78 (s, 1H), 8.30 (br.s, 1H), 8.20 - 8.13 (m, 2H), 8.01 (s, 1H), 7.84 (s, 1H), 7.69 - 7.57 (m, 2H), 7.45 - 7.19 (m, 3H), 6.87 (s, 1H), 6.69 (br.s, 1H), 5.11 - 5.01 (m, 1H), 3.92 - 3.75 (m, 6H), 3.58 - 3.42 (m, 4H), 2.97 - 2.77 (m, 5H), 2.65 - 2.50 (m, 2H), 2.10 - 1.95 (m, 1H), 1.85 - 1.72 (m, 6H), 1.72 - 1.54 (m, 8H).

**Example 164: Preparation of compound 164**

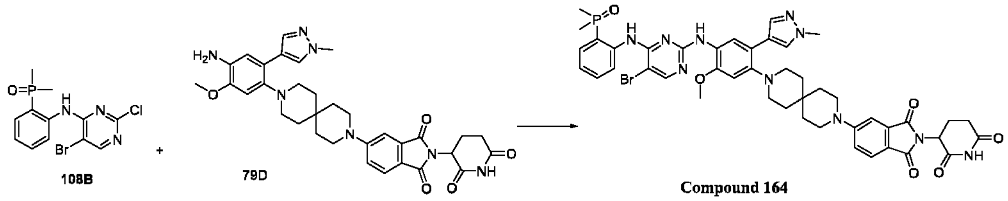

108B + 79D → Compound 164

Step 1: 5-(9-(4-((5-bromo-4-((2-(dimethylphosphoryl)phenyl)amino) pyrimidin-2-yl)amino)-5-methoxy-2-(1-methyl-1H-pyrazol-4-yl)phenyl)-3,9-diazaspiro[5.5]undecan-3-yl)-2-(2,6-dioxopiperidin-3-yl)isoindoline-1,3-dione **(compound 164)**

**79D** (400 mg, 0.65 mmol) and **108B** (257.81 mg, 0.72 mmol) were dissolved in DMF (8 mL); p-toluenesulfonic acid monohydrate (370.93 mg, 1.95 mmol) was added; and under nitrogen protection, the mixture was reacted overnight at 100°C, and cooled to room temperature. 20 mL of water and 20 mL of saturated aqueous sodium bicarbonate solution were added, and the mixture was subjected to suction filtration. The filter cake was dissolved in dichloromethane and extracted. The organic phase was dried over anhydrous sodium sulphate and then concentrated under reduced pressure, and the residue was purified by silica gel column chromatography (dichloromethane/methanol (V/V) = 100/1-10/1) to obtain a product, which was further purified by preparative liquid phase chromatography (instrument: waters 2767 preparative chromatographic column; SunFire@ Prep C18 (19 mm × 150 mm); composition of mobile phases: mobile phase A: acetonitrile, and mobile phase B: water (containing 0.1% TFA)) to obtain the trifluoroacetate of **compound 164.** 60 mL of dichloromethane and 30 mL of saturated sodium bicarbonate solution were added to the trifluoroacetate of **compound 164,** and the layers were separated. The organic phase was dried over anhydrous sodium sulphate and then concentrated under reduced pressure to obtain **compound 164** (120 mg, yield: 19.73%).

LCMS m/z = 935.3 $[M+H]^+$.

$^1H$ NMR (400 MHz, DMSO-$d_6$) δ 11.05 (s, 1H), 10.94 (s, 1H), 8.33 (br.s, 1H), 8.22 - 8.13 (m, 2H), 7.96 (s, 1H), 7.86 (s, 1H), 7.70 - 7.43 (m, 3H), 7.36 - 7.21 (m, 2H), 7.07 - 6.92 (m, 2H), 6.86 (s, 1H), 5.12 - 4.99 (m, 1H), 3.88 - 3.77 (m, 6H), 3.58 - 3.45 (m, 4H), 2.96 - 2.77 (m, 5H), 2.70 - 2.50 (m, 2H), 2.10 - 1.95 (m, 1H), 1.84 - 1.51 (m, 14H).

**Example 165: Preparation of compound 165**

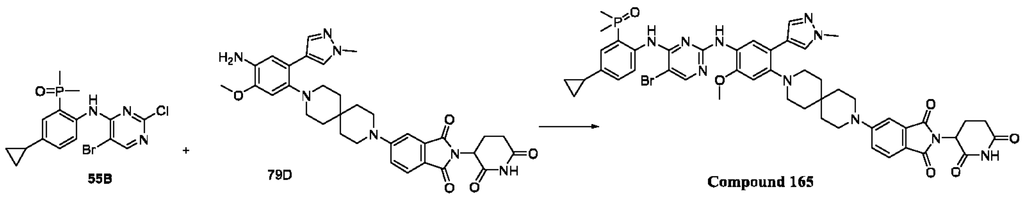

Step 1: 5-(9-(4-((5-bromo-4-((4-cyclopropyl-2-(dimethylphosphoryl)phenyl) amino)pyrimidin-2-yl)amino)-5-methoxy-2-(1 -methyl-1H-pyrazol-4-yl)phenyl)-3,9-diazaspiro[5.5]undecan-3-yl)-2-(2,6-dioxopiperidin-3-yl)isoindoline-1,3-dione **(compound 165)**

**79D** (397.60 mg, 0.65 mmol) and **55B** (286.46 mg, 0.72 mmol) were dissolved in DMF (8 mL); p-toluenesulfonic acid monohydrate (370.93 mg, 1.95 mmol) was added; and under nitrogen protection, the mixture was reacted overnight at 100°C, and cooled to room temperature. 20 mL of water and 20 mL of saturated aqueous sodium bicarbonate solution were added, and the mixture was subjected to suction filtration. The filter cake was dissolved in dichloromethane and extracted. The organic phase was dried over anhydrous sodium sulphate and then concentrated under reduced pressure, and the residue was purified by silica gel column chromatography (dichloromethane/methanol (V/V) = 100/1-15/1) to obtain **compound 165** (190 mg, yield: 29.95%).

LCMS m/z = 975.3 $[M+H]^+$.

$^1H$ NMR (400 MHz, DMSO-$d_6$) δ **11.05** (s, **1H), 10.85** (s, **1H),** 8.24 - 8.08 (m, 3H), 8.00 (s, 1H), 7.90 (s, 1H), 7.72 - 7.50 (m, 2H), 7.38 - 7.16 (m, 3H), 6.88 (s, 1H), 6.48 (br.s, 1H), 5.12 - 5.01 (m, 1H), 3.96 - 3.72 (m, 6H), 3.60 - 3.44 (m, 4H), 3.00 - 2.77 (m, 5H), 2.70 - 2.50 (m, 2H), 2.10 - 1.50 (m, 16H), 1.00 - 0.80 (m, 2H), 0.60 - 0.40 (m, 2H).

**Example 166: Preparation of compound 166**

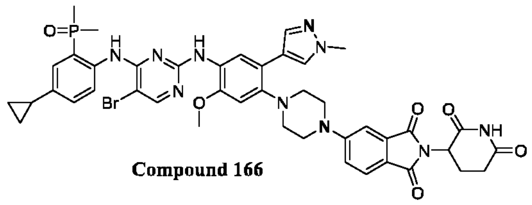

Compound 166

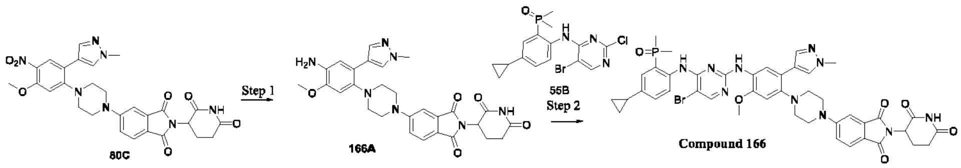


Step 1: 5-(4-(4-amino-5-methoxy-2-(1-methyl-1H-pyrazol-4-yl)phenyl) piperazin-1-yl)-2-(2,6-dioxopiperidin-3-yl)isoindoline-1,3-dione (166A)

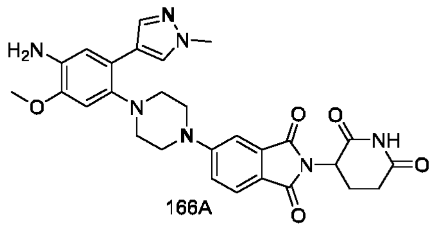

166A

**80C** (10 g, 18.40 mmol) was placed in a 500 mL single-necked flask; ammonium chloride (14.76 g, 276 mmol), THF (100 ml), ethanol (100 ml), water (50 ml) and iron powder (15.41 g, 276 mmol) were successively added; and after the addition, the mixture was stirred at 80°C for 2 h, cooled to room temperature and filtered. The filter cake was washed with a mixed solvent of dichloromethane/methanol (v/v = 10/1). The organic phases were combined and washed with saturated brine. The organic layer was dried over anhydrous sodium sulphate and then concentrated under reduced pressure to obtain **166A** (2 g, yield: 20%).

Step 2: 5-(4-(4-((5-bromo-4-((4-cyclopropyl-2-(dimethylphosphoryl)phenyl) amino)pyrimidin-2-yl)amino)-5-methoxy-2-(1-methyl-1H-pyrazol-4-yl)phenyl)piperazin-1-yl)-2-(2,6-dioxopiperidin-3-yl)isoindoline-1,3-dione **(compound 166)**

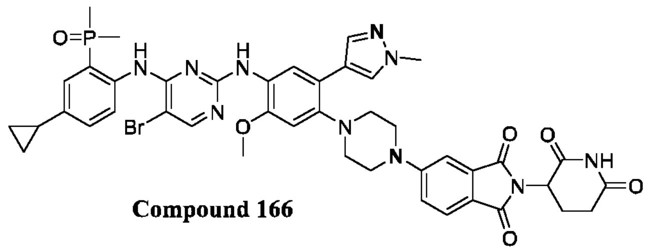

Compound 166

**166A** (0.4 g, 0.74 mmol) and **55B** (0.3 g, 0.74 mmol) were dissolved in DMF (5 ml); p-toluenesulfonic acid monohydrate (0.28 g, 1.48 mmol) was added; and the mixture was stirred overnight at 100°C, and cooled to room temperature. 10 mL of saturated aqueous sodium bicarbonate solution was added, with a solid precipitated, and the mixture was filtered. The filter cake was dried and then purified by silica gel column chromatography (dichloromethane/methanol (V/V) = 100/3-100/5). 8 mL of acetonitrile was added to the resulting residue, and the mixture was stirred at 70°C for 1 h and filtered. The filter cake was dried under reduced pressure to obtain **compound 166** (0.2 g, yield: 30%).

LCMS m/z = 454.3 $[(M+2H)/2]^+$.

$^1H$ NMR (400 MHz, DMSO-$d_6$) δ 11.07 (s, 1H), 10.83 (s, 1H), 8.27 - 8.07 (m, 4H), 7.89 (s, 1H), 7.71 (d, 1H),

7.65 (s, 1H), 7.45 - 7.39 (m, 1H), 7.36 - 7.29 (m, 1H), 7.25 - 7.14 (m, 1H), 6.84 (s, 1H), 6.57 - 6.44 (m, 1H), 5.19 - 4.99 (m, 1H), 3.83 (d, 6H), 3.66 (s, 4H), 3.01 (s, 4H), 2.95 - 2.82 (m, 1H), 2.67 - 2.55 (m, 2H), 2.06 - 1.99 (m, 1H), 1.82 - 1.68 (m, 7H), 0.94 - 0.82 (m, 2H), 0.60 - 0.46 (m, 2H).

**Example 167: Preparation of compound 167**

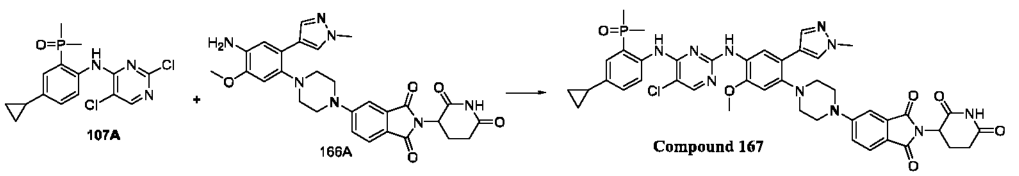

Step 1: 5-(4-(4-((5-chloro-4-((4-cyclopropyl-2-(dimethylphosphoryl)phenyl) amino)pyrimidin-2-yl)amino)-5-methoxy-2-(1-methyl-1H-pyrazol-4-yl)phenyl)piperazin-1-yl)-2-(2,6-dioxopiperidin-3-yl)isoindoline-1,3-dione **(compound 167)**

**166A** (0.4 g, 0.74 mmol) and **107A** (0.26 g, 0.74 mmol) were dissolved in DMF (5 ml); p-toluenesulfonic acid monohydrate (0.28 g, 1.48 mmol) was added; and the mixture was stirred overnight at 100°C, and cooled to room temperature. 10 mL of saturated aqueous sodium bicarbonate solution was added, with a solid precipitated, and the mixture was filtered. The filter cake was dried and then purified by silica gel column chromatography (dichloromethane/methanol (V/V) = 100/3-100/5). 8 mL of acetonitrile was added to the resulting residue, and the mixture was stirred at 70°C for 1 h and filtered. The filter cake was dried under reduced pressure to obtain **compound 167** (0.25 g, yield: 39%).

LCMS m/z = 432.2 $[(M+2H)/2]^+$.

$^1H$ NMR (400 MHz, DMSO-$d_6$) δ 11.10 (s, 1H), 11.07 (s, 1H), 8.36 - 8.22 (m, 1H), 8.21 - 8.11 (m, 2H), 8.08 (s, 1H), 7.90 (s, 1H), 7.71 (d, 1H), 7.66 (s, 1H), 7.46 - 7.39 (m, 1H), 7.38 - 7.30 (m, 1H), 7.26 - 7.15 (m, 1H), 6.85 (s, 1H), 6.57 - 6.39 (m, 1H), 5.13 - 5.00 (m, 1H), 3.83 (d, 6H), 3.66 (s, 4H), 3.01 (s, 4H), 2.96 - 2.82 (m, 1H), 2.69 - 2.52 (m, 2H), 2.09 - 1.97 (m, 1H), 1.83 - 1.67 (m, 7H), 0.94 - 0.83 (m, 2H), 0.59 - 0.48 (m, 2H).

**Example 168: Preparation of compound 168**

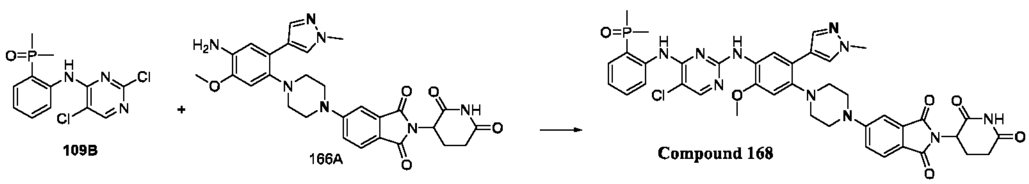

Step 1: 5-(4-(4-((5-chloro-4-((2-(dimethylphosphoryl)phenyl)amino)pyrimidin -2-yl)amino)-5-methoxy-2-(1-methyl-1H-pyrazol-4-yl)phenyl)piperazin-1-yl)-2-(2,6-dioxopiperidin-3-yl)isoindoline-1,3-dione **(compound 168)**

**166A** (0.45 g, 0.83 mmol) and **109B** (0.26 g, 0.83 mmol) were dissolved in DMF (5 mL); p-toluenesulfonic acid monohydrate (0.32 g, 1.66 mmol) was added; and the mixture was stirred overnight at 100°C, and cooled to room temperature. 10 mL of saturated aqueous sodium bicarbonate solution was added, with a solid precipitated, and the mixture was filtered. The filter cake was dried and then purified by silica gel column chromatography (dichloromethane/methanol (V/V) = 100/3-100/5). 8 mL of acetonitrile was added to the resulting residue, and the mixture was stirred at 70°C for 1 h and filtered. The filter cake was dried under reduced pressure to obtain **compound 168** (0.3 g, yield: 44%).

LCMS m/z = 412.2 $[(M+2H)/2]^+$.

$^1H$ NMR (400 MHz, DMSO-$d_6$) δ 11.21 (s, 1H), 11.07 (s, 1H), 8.48 - 8.39 (m, 1H), 8.22 (s, 1H), 8.13 - 8.06 (m, 2H), 7.86 (s, 1H), 7.71 (d, 1H), 7.66 (s, 1H), 7.56 - 7.46 m, 1H), 7.44 - 7.39 (m, 1H), 7.37 - 7.28 (m, 1H), 7.07 - 6.92 (m, 2H), 6.85 (s, 1H), 5.14 - 5.01 (m, 1H), 3.83 (d, 6H), 3.64 (s, 4H), 3.01 (s, 4H), 2.95 - 2.84 (m, 1H), 2.68 - 2.53 (m, 2H), 2.05 - 1.98 (m, 1H), 1.76 (d, 6H).

**Example 169: Preparation of compound 169**

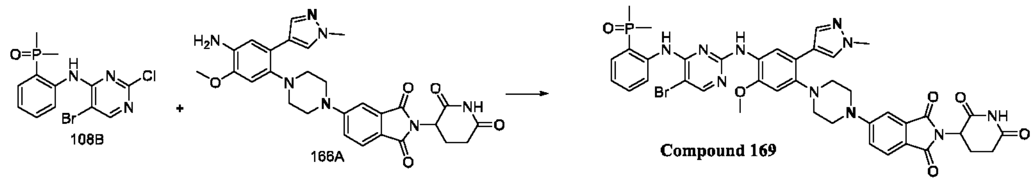


Step 1: 5-(4-(4-((5-bromo-4-((2-(dimethylphosphoryl)phenyl)amino) pyrimidin-2-yl)amino)-5-methoxy-2-(1-methyl-1H-pyrazol-4-yl)phenyl)piperazin-1-yl)-2-(2,6-dioxopiperidin-3-yl)isoindoline-1,3-dione **(compound 169)**

**166A** (0.4 g, 0.74 mmol) and **108B** (0.27 g, 0.74 mmol) were dissolved in DMF (5 ml); p-toluenesulfonic acid monohydrate (0.28 g, 1.48 mmol) was added; and the mixture was stirred overnight at 100°C, and cooled to room temperature. 10 mL of saturated aqueous sodium bicarbonate solution was added, with a solid precipitated, and the mixture was filtered. The filter cake was dried and then purified by silica gel column chromatography (dichloromethane/methanol (V/V) = 100/3-100/5). 8 mL of acetonitrile was added to the resulting residue, and the mixture was stirred at 70°C for 1 h and filtered. The filter cake was dried under reduced pressure to obtain **compound 169** (0.25 g, yield: 39%).

LCMS m/z = 434.2 $[(M+2H)/2]^+$.

$^1H$ NMR (400 MHz, DMSO-$d_6$) $\delta$ 11.07 (s, 1H), 10.94 (s, 1H), 8.39 - 8.28 (m, 1H), 8.25 - 8.16 (m, 2H), 8.07 (s, 1H), 7.85 (s, 1H), 7.71 (d, 1H), 7.65 (s, 1H), 7.54 - 7.45 (m, 1H), 7.44 - 7.39 (m, 1H), 7.36 - 7.29 (m, 1H), 7.09 - 6.92 (m, 2H), 6.84 (s, 1H), 5.15 - 5.04 (m, 1H), 3.82 (d, 6H), 3.64 (s, 4H), 3.00 (s, 4H), 2.95 - 2.81 (m, 1H), 2.68 - 2.53 (m, 2H), 2.09 - 1.93 (m, 1H), 1.76 (d, 6H).

**Example 170 and example 171: Preparation of compound 170 and compound 171**

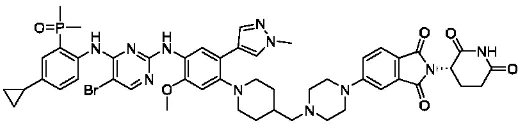
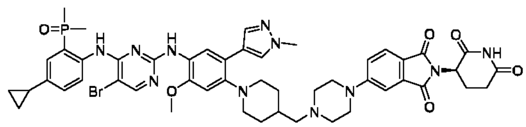

Compound 170 and compound 171

300 mg of **compound 106** was subjected to preparative SFC separation and purification. Preparative SFC separation conditions: instrument: Waters 150 MGM; chromatographic column: Chiralpak Column; mobile phase: A for $CO_2$ and B for IPA (isopropanol)+ACN (acetonitrile); gradient: 65% phase B isocratic elution; flow rate: 120 mL/min; back pressure: 100 bar; column temperature: 35°C; wavelength: 220 nm.

SFC analysis conditions: instrument: SHIMADZU LC-30AD sfc; chromatographic column: Chiralpak AD-3 50 $\times$ 4.6 mm I.D., 3 $\mu$m; mobile phase: A for $CO_2$ and B for IPA (isopropanol) + ACN (acetonitrile) (0.05% DEA (diethylamine)); gradient: B 60%; flow rate: 3 mL/min; back pressure: 100 bar; column temperature: 35°C; wavelength: 220 nm.

After preparative separation, the components with the same retention time were combined, lyophilized, redissolved in 20 mL of DCM and extracted by adding water (10 mL) and saturated sodium bicarbonate solution (1 mL). The organic layers were separated, dried over anhydrous sodium sulphate, filtered and concentrated to obtain **compound 170** (30 mg) and **compound 171** (70 mg).

**Compound 170:**

retention time under SFC analysis conditions: 1.6 min,
LCMS m/z = 502.8$[(M+2H)/2]^+$.
$^1H$ NMR(400 MHz, $D_2O/CF_3COOD$(v/v = 1:1)) $\delta$ 8.23 (s, 1H), 7.96 - 7.75 (m, 5H), 7.46 (s, 1H), 7.35 (s, 1H), 7.31 - 7.15 (m, 2H), 6.92 (d, 1H), 5.12 (dd, 1H), 4.22 - 4.07 (m, 5H), 4.03 (s, 3H), 3.93 - 3.70 (m, 6H), 3.62 - 3.48 (m, 2H), 3.39 - 3.18 (m, 4H), 2.95 - 2.85 (m, 2H), 2.82 - 2.67 (m, 1H), 2.63 - 2.44 (m, 1H), 2.37 - 2.16 (m, 3H), 2.12 - 1.88 (m, 9H), 1.19 - 1.08 (m, 2H), 0.73 - 0.65 (m, 2H).

**Compound 171:**

retention time under SFC analysis conditions: 2.7 min,
LCMS m/z = 502.8 [(M+2H)/2]$^+$.
$^1$H NMR(400 MHz, $D_2O/CF_3COOD$(v/v = 1:1))δ 8.23 (s, 1H), 7.96 - 7.75 (m, 5H), 7.46 (s, 1H), 7.35 (s, 1H), 7.31 - 7.15 (m, 2H), 6.92 (d, 1H), 5.12 (dd, 1H), 4.22 - 4.07 (m, 5H), 4.03 (s, 3H), 3.93 - 3.70 (m, 6H), 3.62 - 3.48 (m, 2H), 3.39 - 3.18 (m, 4H), 2.95 - 2.85 (m, 2H), 2.82 - 2.67 (m, 1H), 2.63 - 2.44 (m, 1H), 2.37 - 2.16 (m, 3H), 2.12 - 1.88 (m, 9H), 1.19 - 1.08 (m, 2H), 0.73 - 0.65 (m, 2H).

**Example 172 and example 173: Preparation of compound 172 and compound 173**

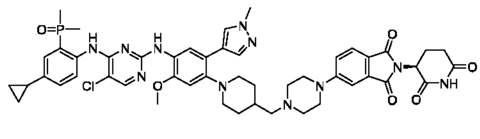

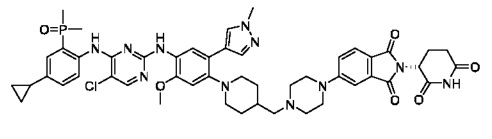

Compound 172 and compound 173

345 mg of **compound 107** was subjected to preparative SFC separation and purification. Preparative SFC separation conditions: instrument: Waters 150 MGM; chromatographic column: Chiralpak Column; mobile phase: A for $CO_2$ and B for IPA+ACN; gradient: 70% phase B isocratic elution; flow rate: 100 mL/min; back pressure: 100 bar; column temperature: 35°C; wavelength: 220 nm.
SFC analysis conditions: instrument: SHIMADZU LC-30AD sfc; chromatographic column: Chiralpak AD-3 50 × 4.6 mm I.D., 3 μm; mobile phase: A for $CO_2$ and B for IPA+ACN (0.05% DEA); gradient: B 60%; flow rate: 3 mL/min; back pressure: 100 bar; column temperature: 35°C; wavelength: 220 nm.
After preparative separation, the components with the same retention time were combined and concentrated under reduced pressure to obtain **compound 172** (160 mg) and **compound 173** (165 mg).
**Compound 172:** retention time under SFC analysis conditions: 1.5 min, LCMS m/z = 960.2[M+H]$^+$.
$^1$H NMR (400 MHz, $D_2O/CF_3COOD$(v/v = 1:1)) δ 8.14 (s, 1H), 8.02 - 7.91 (m, 3H), 7.86 (s, 1H), 7.82 (d, 1H), 7.46 (s, 1H), 7.36 (s, 1H), 7.31 - 7.13 (m, 2H), 6.90 (d, 1H), 5.12 (dd, 1H), 4.25 - 4.08 (m, 5H), 4.04 (s, 3H), 3.95 - 3.73 (m, 6H), 3.63 - 3.45 (m, 2H), 3.39 - 3.18 (m, 4H), 2.94 - 2.85 (m, 2H), 2.83 - 2.67 (m, 1H), 2.63 - 2.44 (m, 1H), 2.37 - 2.19 (m, 3H), 2.13 - 1.88 (m, 9H), 1.20 - 1.06 (m, 2H), 0.72 - 0.64 (m, 2H).
**Compound 173:** retention time under SFC analysis conditions: 2.5 min, LCMS m/z = 960.2[M+H]$^+$.
$^1$H NMR (400 MHz, $D_2O/CF_3COOD$(v/v = 1:1)) δ 8.14 (s, 1H), 8.03 - 7.91 (m, 3H), 7.86 (s, 1H), 7.82 (d, 1H), 7.46 (s, 1H), 7.37 (s, 1H), 7.30 - 7.16 (m, 2H), 6.90 (d, 1H), 5.12 (dd, 1H), 4.25 - 4.08 (m, 5H), 4.04 (s, 3H), 3.94 - 3.75 (m, 6H), 3.62 - 3.45 (m, 2H), 3.38 - 3.15 (m, 4H), 2.94 - 2.85 (m, 2H), 2.83 - 2.66 (m, 1H), 2.63 - 2.45 (m, 1H), 2.35 - 2.17 (m, 3H), 2.12 - 1.89 (m, 9H), 1.18 - 1.06 (m, 2H), 0.74 - 0.64 (m, 2H).

| | |
|---|---|
| Control compound A, prepared with reference to WO 2021036922 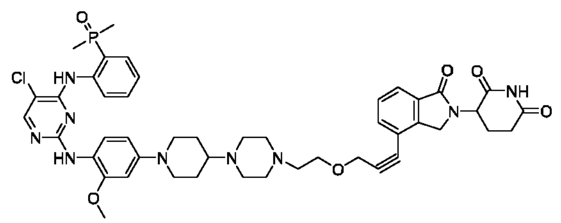 | Control compound B, prepared with reference to WO 2019114770 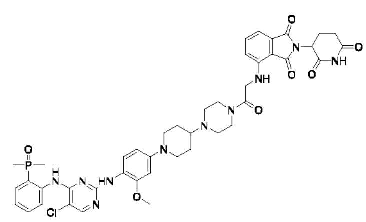 |

**Biological test examples**

**Test example 1: Inhibitory activity on proliferation of NCI-H1975 (EGFR-L858R-T790M) and A431 (EGFR-WT) cells**

NCI-H1975 (EGFR-L858R-T790M) and A431 (EGFR-WT) cells were purchased from ATCC and cultured in

an incubator at 37°C with 5% $CO_2$, and the culture mediums were RPMI1640+10% FBS and DMEM+10% FBS, respectively. On the first day, NCI-H1975 (EGFR-L858R-T790M) and A431 (EGFR-WT) cells in the logarithmic phase were collected, and viable cells were counted using an automatic cell analyser (countstar). The cell suspensions were adjusted with the culture mediums and plated on a 96-well cell culture plate at 1000 cells/well for NCI-H1975 (EGFR-L858R-T790M) cells and at 3000 cells/well for A431 cells. The next day, the culture mediums were aspirated, and 90 μL of fresh culture mediums and 10 μL of the compounds at different concentrations were added to each well, wherein the final concentration of DMSO in each well was 0.1%. The plate was cultured in the incubator at 37°C with 5% $CO_2$ for 72 hours. After 72 hours of drug treatment, 50 μL of CTG solution (promega, G7572), which was already pre-melted and equilibrated to room temperature, was added to each well, and the mixtures were uniformly mixed for 2 min using a microplate shaker. The plate was placed at room temperature for 10 min, and then fluorescence signal values were measured using a microplate reader (PHERAstar FSX).

Surviving cells% was calculated based on $V_{sample}/V_{vehicle\ control} \times 100\%$, wherein $V_{sample}$ was the readout of the drug-treated group, and $V_{vehicle\ control}$ was the average value of the vehicle control group. Using the origin 9.2 software and nonlinear regression model, S-shaped dose-survival curves were plotted, and $IC_{50}$ values were calculated.

Table 1 Inhibitory activity results of test compounds on proliferation of NCI-H1975 (EGFR-L858R-T790M) cells

| Compound No. | IC50 (μM) | Compound No. | IC50 (μM) |
|---|---|---|---|
| Compound 1 | A (0.043) | Compound 85 | A (0.077) |
| Compound 2 | B (0.151) | Trifluoroacetate of compound 86 | A (0.077) |
| Compound 3 | A (0.075) | Compound 87 | A (0.045) |
| Compound 4 | A (0.021) | Compound 88 | A (0.067) |
| Compound 5 | A (0.052) | Compound 89 | A (0.084) |
| Compound 6 | A (0.015) | Compound 90 | A (0.046) |
| Trifluoroacetate of compound 7 | A (0.02) | Compound 91 | A (0.078) |
| Compound 8 | A (0.034) | Compound 92 | A (0.071) |
| Compound 9 | A (0.014) | Compound 93 | A (0.049) |
| Compound 10 | A (0.013) | Compound 94 | A (0.077) |
| Compound 11 | A (0.007) | Compound 95 | A (0.049) |
| Trifluoroacetate of compound 12 | A (0.059) | Compound 96 | A (0.036) |
| Trifluoroacetate of compound 13 | A (0.037) | Compound 97 | A (0.062) |
| Trifluoroacetate of compound 14 | A (0.049) | Compound 98 | B (0.101) |
| Compound 15 | A (0.066) | Compound 99 | A (0.033) |
| Compound 16 | A (0.073) | Compound 100 | A (0.048) |
| Compound 17 | B | Compound 101 | B (0.107) |
| Compound 18 | A (0.012) | Compound 102 | B (0.15) |
| Trifluoroacetate of compound 19 | A (0.042) | Compound 103 | A (0.061) |
| Trifluoroacetate of compound 20 | A (0.014) | Compound 104 | A (0.054) |
| Compound 21 | A (0.039) | Compound 105 | A (0.058) |
| Trifluoroacetate of compound 23 | A (0.041) | Compound 106 | A (0.054) |
| Compound 24 | B (0.163) | Compound 107 | A (0.056) |
| Compound 26 | B | Compound 108 | A (0.042) |
| Compound 27 | A (0.02) | Compound 109 | A (0.052) |
| Compound 28 | A (0.033) | Compound 110 | B (0.232) |
| Compound 29 | A (0.033) | Compound 111 | B (0.209) |
| Compound 30 | A (0.012) | Compound 112 | A (0.083) |

(continued)

| Compound No. | IC50 (μM) | Compound No. | IC50 (μM) |
|---|---|---|---|
| Compound 31 | A (0.015) | Compound 113 | A (0.068) |
| Compound 32 | A (0.01) | Compound 114 | A (0.06) |
| Compound 33 | A (0.028) | Compound 115 | A (0.073) |
| Compound 35 | B (0.114) | Compound 116 | A (0.094) |
| Compound 36 | B (0.165) | Compound 117 | A (0.051) |
| Compound 37 | A (0.038) | Compound 118 | A (0.04) |
| Compound 38 | A (0.06) | Compound 119 | A (0.029) |
| Compound 39 | A (0.051) | Compound 120 | A (0.025) |
| Compound 40 | A (0.055) | Compound 121 | A (0.016) |
| Compound 41 | B (0.353) | Compound 122 | A (0.016) |
| Compound 42 | A (0.059) | Compound 123 | A (0.051) |
| Compound 43 | A (0.08) | Compound 124 | A (0.071) |
| Compound 44 | B (0.301) | Compound 125 | A (0.023) |
| Compound 45 | B (0.105) | Compound 126 | A (0.039) |
| Compound 46 | A (0.071) | Compound 127 | A (0.019) |
| Compound 47 | B (0.11) | Compound 128 | A (0.025) |
| Compound 48 | A (0.032) | Compound 129 | B (0.125) |
| Compound 49 | B (0.266) | Compound 130 | B (0.118) |
| Compound 50 | B (0.133) | Compound 131 | B (0.214) |
| Compound 51 | A (0.072) | Compound 132 | B (0.296) |
| Compound 52 | A (0.084) | Compound 135 | A (0.082) |
| Compound 53 | B (0.182) | Compound 136 | B (0.393) |
| Compound 54 | A (0.069) | Compound 138 | A (0.075) |
| Compound 55 | A (0.03) | Compound 140 | A (0.032) |
| Compound 56 | A (0.061) | Compound 141 | A (0.027) |
| Compound 57 | A (0.021) | Compound 142 | A (0.033) |
| Compound 58 | A (0.072) | Compound 143 | B (0.11) |
| Compound 59 | A (0.092) | Compound 145 | A (0.069) |
| Compound 60 | A (0.034) | Compound 146 | A (0.095) |
| Compound 61 | B (0.176) | Compound 147 | B (0.103) |
| Compound 62 | A (0.028) | Compound 148 | A (0.079) |
| Compound 63 | A (0.09) | Compound 149 | B (0.116) |
| Compound 64 | A (0.082) | Compound 150 | A (0.04) |
| Compound 65 | B (0.129) | Compound 151 | A (0.035) |
| Compound 66 | A (0.099) | Compound 152 | A (0.068) |
| Compound 67 | A (0.037) | Compound 153 | A (0.073) |
| Compound 68 | A (0.066) | Compound 154 | A (0.046) |
| Compound 69 | A (0.034) | Compound 155 | A (0.043) |

(continued)

| Compound No. | IC50 (μM) | Compound No. | IC50 (μM) |
|---|---|---|---|
| Compound 70 | A (0.039) | Compound 156 | A (0.064) |
| Compound 71 | A (0.029) | Compound 157 | A (0.029) |
| Compound 72 | A (0.031) | Compound 158 | A (0.026) |
| Compound 73 | A (0.026) | Compound 159 | A (0.021) |
| Compound 74 | B (0.313) | Compound 160 | B (0.152) |
| Compound 76 | A (0.031) | Compound 162 | B (0.134) |
| Compound 77 | A (0.032) | Compound 163 | A (0.076) |
| Compound 78 | A (0.051) | Compound 164 | B (0.109) |
| Compound 79 | B (0.314) | Compound 166 | B (0.115) |
| Compound 80 | B (0.129) | Compound 169 | B (0.111) |
| Compound 81 | A (0.071) | Compound 170 | A (0.047) |
| Compound 82 | A (0.09) | Compound 171 | A (0.077) |
| Compound 83 | B (0.126) | Compound 172 | A (0.049) |
| Compound 84 | A (0.043) | Compound 173 | A (0.081) |
| Control compound A | B (0.549) | Control compound B | B (0.658) |
| Notes: A < 0.1 μM, 0.1 μM ≤ B < 0.5 μM, and 0.5 μM < C < 1 μM in table 1. | | | |

Inhibitory activity of test compounds on proliferation of A431 (WT) cells:
Inhibitory activity of compounds 1, 2, 4 and 6, trifluoroacetate of compound 7, trifluoroacetate of compound 14, compound 18, trifluoroacetate of compound 23, compounds 24, 30, 34, 35, 36, 37, 38, 40, 41, 42, 45, 46, 47, 48, 50, 54, 55, 57, 58, 59, 60, 61, 62, 63, 65, 66, 67, 68, 71, 72, 73, 76, 77, 78, 79, 80, 81, 82, 83, 84 and 85, trifluoroacetate of compound 86, and compounds 87, 88, 89, 90, 91, 92, 93, 95, 96, 97, 98, 99, 100, 101, 102, 103, 104, 105, 106, 107, 108, 109, 110, 111, 112, 113, 116, 117, 118, 119, 121, 125, 126, 133, 137, 138, 139, 143, 145, 146, 147, 148, 150, 160, 162, 163, 164, 165, 166, 167, 170, 171, 172 and 173 on proliferation of A431 (EGFR-WT) cells showed IC50≥10 μM.

Conclusion: the compounds of the present invention have good inhibitory activity on proliferation of NCI-H1975 (EGFR-L858R-T790M) cells, and poor inhibitory activity on proliferation of and good selectivity for A431 (EGFR-WT) cells.

**Test example 2: Inhibitory activity on proliferation of Ba/F3-TEL-EGFR-T790M-L858R-C797S cells**

Ba/F3-TEL-EGFR-T790M-L858R-C797S cells were cultured in an incubator at 37°C with 5% $CO_2$. The cells in the logarithmic phase were collected, counted and plated in a 96-well plate at a density of 2000 cells/well. The compounds at different concentrations were added, and a vehicle control group (cells plus DMSO) and a blank control group (cell culture mediums plus DMSO) were provided, wherein the concentration of DMSO was both 0.1%. The cell culture plate was cultured in an incubator at 37°C with 5% $CO_2$ for 72 hours. After the incubation was completed, the cell culture plate was removed and allowed to equilibrate to room temperature for 10 min. According to operation instructions for a CellTiter-Glo kit (Promega, G7573), 50 μL of CTG solution, which was already pre-melted and equilibrated to room temperature, was added to each well, and the mixtures were uniformly mixed for 2 min using a microplate shaker. The plate was placed at room temperature for 10 min, and then fluorescence signal values were measured using a microplate reader (SpectraMax Paradigm). The cell proliferation inhibition rate data was processed based on formula (1). The inhibition rates corresponding to different concentrations of compounds were calculated, and using the GraphPad Prism software, the inhibition rate curves were plotted, and $IC_{50}$ values were calculated. wherein $RLU_{Drug}$ was the readout of the drug-treated group, $RLU_{Max}$ was the readout of the control group, and $RLU_{Min}$ was the readout of the blank group.

$$\text{Inhibition Rate (Inh\%)} = (1 - (RLU_{Drug} - RLU_{Min})/(RLU_{Max} - RLU_{Min})) \times 100\% \quad \text{(formula 1)}$$

Conclusion: the compounds of the present invention have good inhibitory activity on proliferation of Ba/F3-TEL-EGFR-T790M-L858R-C797S cells, for example, the IC50 of compound 106 is 15.6 nM, and the IC50 of compound 107 is 15.6 nM.

**Test example 3: Inhibitory activity on proliferation of NCI-H1975 EGFR-L858R-T790M-C797S cells**

NCI-H1975 EGFR-L858R-T790M-C797S cells were cultured in an incubator at 37°C with 5% $CO_2$, and the culture medium was RPMI1640+10% FBS+100 μg/mL hygromycin. The cells in the logarithmic phase were collected, and the cell suspension was adjusted to an appropriate concentration with a hygromycin-free culture medium, and then plated on a 96-well plate at a density of 1500 cells/well and a volume of 90 μL. 10 μL of the compounds at different concentrations were added, and a vehicle control group (cells plus DMSO) was provided, wherein the concentration of DMSO was 0.1%. The cell culture plate was cultured in an incubator at 37°C with 5% $CO_2$ for 72 hours. After the incubation was completed, according to operation instructions for a CellTiter-Glo kit (Promega, G7572), 50 μL of CTG solution, which was already pre-melted and equilibrated to room temperature, was added to each well, and the mixtures were uniformly mixed for 2 min using a microplate shaker. The plate was placed at room temperature for 10 min, and then fluorescence signal values were measured using a microplate reader (Envision 2104). Surviving cells% data was processed based on formula (2), and using the GraphPad Prism 5.0 software and nonlinear regression model, S-shaped dose-survival curves were plotted, and $IC_{50}$ values were calculated. wherein $V_{sample}$ was the readout of the drug-treated group, and $V_{vehicle\ control}$ was the readout of the control group.

$$\text{Surviving cells\%} = V_{sample}/V_{vehicle\ control} \times 100\% \quad \text{(formula 2)}$$

Table 2 Inhibitory activity of compounds on proliferation of NCI-H1975 EGFR-L858R-T790M-C797S cells

| Compound No. | $IC_{50}$ (μM) | Compound No. | $IC_{50}$ (μM) |
|---|---|---|---|
| Compound 2 | B (0.299) | Compound 84 | A (0.047) |
| Compound 6 | A (0.027) | Compound 90 | A (0.068) |
| Trifluoroacetate of compound 7 | A (0.030) | Compound 92 | A (0.065) |
| Compound 9 | A (0.039) | Compound 96 | A (0.060) |
| Compound 18 | A (0.024) | Compound 97 | A (0.079) |
| Trifluoroacetate of compound 23 | A (0.083) | Compound 99 | A (0.057) |
| Compound 37 | A (0.032) | Compound 100 | A (0.084) |
| Compound 45 | B (0.364) | Compound 101 | A (0.152) |
| Compound 47 | A (0.072) | Compound 102 | A (0.152) |
| Compound 54 | A (0.056) | Compound 103 | A (0.101) |
| Compound 57 | A (0.065) | Compound 104 | A (0.082) |
| Compound 65 | A (0.136) | Compound 106 | A (0.112) |
| Compound 67 | A (0.115) | Compound 107 | A (0.045) |
| Compound 68 | B (0.280) | Compound 108 | A (0.135) |
| Compound 77 | A (0.046) | Compound 112 | A (0.127) |
| Compound 81 | A (0.049) | Compound 117 | A (0.079) |
| Control compound A | 1.383 | Control compound B | C (0.704) |
| Notes: A < 0.2 μM, 0.2 μM ≤ B < 0.5 μM, and 0.5 μM < C < 1 μM in table 2. | | | |

Conclusion: the compounds of the present invention have good inhibitory activity on proliferation of NCI-H1975 EGFR-L858R-T790M-C797S cells, which is obviously better than that of the control compounds A and B.

**Test example 4: Pharmacokinetic test in mice**

**Test objective:** In this experiment, a single dose of test compounds was administered to ICR mice intravenously and intragastrically, the concentrations of the test compounds in plasma of mice were measured, and the pharmacokinetic characteristics and bioavailability of the test compounds in mice were evaluated.
**Experimental animals:** male ICR mice, 20-25 g, purchased from BEIJING HFK BIOSCIENCE CO., LTD. with the laboratory animal production license number of SCXK (JING) 2019-0008; or purchased from CHENGDU DDOSSY EXPERIMENTAL ANIMALS CO., LTD. (SCXK (CHUAN) 2020-030); or purchased from HUNAN SJA LABORATORY ANIMAL CO., LTD. (SCXK (XIANG) 2019-0004).
**Experimental method:** on the day of the experiment, ICR mice were randomly grouped according to their body weights. The mice were fasted with water available for 12 to 14 hours one day before the administration, and were fed 4 hours after the administration.

| Group | Administration information | | | | | | |
|---|---|---|---|---|---|---|---|
| | Test compound | Administration dosage* (mg/kg) | Administrati on concentratio n (mg/mL) | Administrati on volume (mL/kg) | Colle cted sampl e | Mode of adminis tration | Vehicle |
| G1 | Compound of the present invention | 2.5 | 0.5 | 5 | Plas ma | Intraven ously | 10% DMA+10% Solutol+80% (saline) or 5% DMA+5% Solutol+90% (saline) |
| G2 | Compound of the present invention | 1 | 0.2 | 5 | Plas ma | Intraven ously | 10% DMA+10% Solutol+80% (saline) |
| G3 | Compound of the present invention | 10 | 1 | 10 | Plas ma | Oral (intraga strically) | 5% DMSO+5% Solutol+30% PEG400+60% (20% SBE-CD) |
| *The dosage is calculated on the basis of free base, and the dosage for intravenous administration is either 2.5 mg/kg or 1 mg/kg. | | | | | | | |

**Sampling:** before and after the administration, blood was taken from the orbits of the mice under isoflurane anaesthesia, and placed in an $EDTAK_2$ centrifuge tube. Centrifugation was carried out at 5000 rpm at 4°C for 10 min, and the plasma was collected.
Time points for plasma collection in G1&G2 groups: 0, 5 min, 15 min, 30 min, 1 h, 2 h, 4 h, 6 h, 8 h, and 24 h;

time points for plasma collection in G3 group: 0, 5 min, 15 min, 30 min, 1 h, 2 h, 4 h, 6 h, 8 h, and 24 h;
before analysis and detection, all samples were stored at -80°C. The samples were analysed quantitatively by LC-MS/MS.

Table 3-1 Oral absorption results of test compounds in mice (administration i.g. (10 mg/kg))

| Compound No. | $AUC_{0-t}$ (h.ng. $mL^{-1}$) | Compound No. | $AUC_{0-t}$ (h.ng. $mL^{-1}$) |
|---|---|---|---|
| Compound 37 | 21686 | Compound 71 | 24666 |

(continued)

| Compound No. | $AUC_{0-t}$ (h.ng. $mL^{-1}$) | Compound No. | $AUC_{0-t}$ (h.ng. $mL^{-1}$) |
|---|---|---|---|
| Compound 38 | 3732 | Compound 72 | 16430 |
| Compound 39 | 5995 | Compound 73 | 13682 |
| Compound 40 | Oral absorption | Compound 76 | 19649 |
| Compound 42 | Oral absorption | Compound 81 | 51014 |
| Compound 45 | 59156 | Compound 84 | 4570 |
| Compound 46 | Oral absorption | Compound 87 | 2342 |
| Compound 47 | 30626 | Compound 88 | 5125 |
| Compound 48 | 7791 | Compound 89 | 8428 |
| Compound 50 | Oral absorption | Compound 91 | 7583 |
| Compound 54 | 29840 | Compound 93 | 4625 |
| Compound 55 | 19937 | Compound 95 | 20810 |
| Compound 56 | 28570 | Compound 96 | 46965 |
| Compound 58 | 16267 | Compound 97 | 42269 |
| Compound 59 | 4459 | Compound 99 | 24957 |
| Compound 60 | 3205 | Compound 103 | 34704 |
| Compound 62 | 2314 | Compound 104 | 47276 |
| Compound 63 | 14682 | Compound 117 | 101122 |
| Compound 65 | 62338 | | |

Table 3-2 PK data of test compounds in mice (administration i.g. (10 mg/kg))

| Compound No. | AUC (ng.h.$mL^{-1}$) | $T_{1/2}$ (h) |
|---|---|---|
| Compound 68 | 53240 | 3.47 |
| Compound 67 | 70884 | 3.86 |
| Compound 90 | 35887 | 4.16 |
| Compound 77 | 47347 | 5.79 |
| Compound 92 | 63006 | 6.88 |
| Compound 80 | 81905 | 10.4 |
| Compound 98 | 69837 | 10.9 |
| Compound 100 | 33340 | 2.49 |
| Compound 101 | 117559 | 12.7 |
| Compound 102 | 87882 | 9.49 |
| Compound 107 | 129648 | 13.9 |
| Compound 106 | 144575 | 18.0 |
| Compound 108 | 71014 | 4.83 |
| Compound 109 | 81455 | 4.53 |
| Compound 141 | 86626 | 5.91 |
| Compound 142 | 104039 | 5.12 |

(continued)

| Compound No. | AUC ($ng.h.mL^{-1}$) | $T_{1/2}$ (h) |
|---|---|---|
| Compound 163 | 93054 | 6.68 |
| Control compound B | 2086 | 1.23 |
| Control compound A | 563 | 1.54 |
| *Note: i.g. (intragastrically) administration of compounds. | | |

Conclusion: the compounds synthesized by using the technology of the present invention have a certain oral absorption performance in mice, which is better than that of the control compounds A and B.

**Test example 5: Degradation activity on EGFR protein in H1975-EGFR-T790M-L858R-C797S cells**

Method I:

Protein sample preparation: NCI-H1975 EGFR-L858R-T790M-C797S cells were cultured in an incubator at 37°C with 5% CO2, and the culture medium was RPMI1640+10% FBS+100 μg/mL hygromycin. The cells in the logarithmic phase were collected, and the cell suspension was adjusted to an appropriate concentration with a hygromycin-free culture medium, plated on a 6-well plate at a density of 200000 cells/well and a volume of 2 mL, and cultured overnight in an incubator at 37°C with 5% $CO_2$. The next day, the compounds at different concentrations were added. DMSO control wells were provided, wherein the concentration of DMSO in all wells was 0.1%. The 6-well plate was cultured in an incubator at 37°C with 5% $CO_2$ for 72 h, and then 30 μL of RIPA lysates (Beyotime, Cat. P0013B) containing protease inhibitors (Beyotime, Cat. P0013B) were added. The mixtures were lysed on ice for 30 min. Centrifugation was carried out at 13000 rpm/min at 4°C for 20 min to collect protein supernatant samples, and protein quantification was performed by the BCA method. Samples for western blot detection were prepared.

Western blot detection: 20 μg of protein samples were added to each well, subjected to polyacrylamide gel electrophoresis and transferred to a membrane, and then diluted anti-EGFR (CST, Cat. 4267S) and NADPH (Kangchen, Cat. KC-5G4) antibodies were added and incubated overnight at 4°C. After washing the membrane, diluted goat anti-rabbit (Licor, Cat. 926-32211) and goat anti-mouse (Licor, Cat. 926-68070) antibodies were added and incubated in the dark for 45 min. Scanning and detection at wavelengths of 700 nm and 800 nm were performed with a far-infrared imaging system (Odyssey).

According to formula (3), the expression level of EGFR protein in the cells relative to the DMSO control group was calculated after incubation with different concentrations of compounds, wherein $EGFR_{compound}$ was the fluorescence value of EGFR protein after incubation with compounds, and $EGFR_{vehicle}$ was the fluorescence value of EGFR protein in the DMSO control group. Using the Origin 9.2 software, the drug concentration $DC_{50}$ values when the expression level of EGFR protein was 50% relative to the DMSO control group were calculated.

$$EGFR\% = EGFR_{compound}/EGFR_{vehicle} \times 100\% \text{ formula (3)}$$

Method II:

Protein sample preparation: NCI-H1975 EGFR-L858R-T790M-C797S cells were cultured in an incubator at 37°C with 5% CO2, and the culture medium was RPMI1640+10% FBS+100 μg/mL hygromycin. The cells in the logarithmic phase were collected, and the cell suspension was adjusted to an appropriate concentration with a hygromycin-free culture medium, plated on a 6-well plate at a density of 350000 cells/well and a volume of 2 mL, and cultured overnight in an incubator at 37°C with 5% CO2. The next day, the compounds at different concentrations were added. DMSO control wells were provided, wherein the concentration of DMSO in all wells was 0.1%. The 6-well plate was cultured in an incubator at 37°C with 5% $CO_2$ for 48 h, and then 30 μL of RIPA lysates (Beyotime, Cat. P0013B) containing protease inhibitors (Beyotime, Cat. P0013B) were added. The mixtures were lysed on ice for 30 min. Centrifugation was carried out at 13000 rpm/min at 4°C for 20 minutes to collect protein supernatant samples, and protein quantification was performed by the BCA method. Western Blot detection samples were prepared.

Western blot detection: 20 μg of protein samples were added to each well, subjected to polyacrylamide gel electrophoresis and transferred to a membrane, and then diluted anti-EGFR (CST, Cat. 4267S) and NADPH (Kangchen, Cat. KC-5G4) antibodies were added and incubated overnight at 4°C. After washing the membrane, diluted goat anti-rabbit (Licor, Cat. 926-32211) and goat anti-mouse (Licor, Cat. 926-68070) antibodies were added and incubated in the dark for 45 min. Scanning and detection at wavelengths of 700 nm and 800 nm were performed with a far-infrared imaging system (Odyssey).

According to formula (3), the expression level of EGFR protein in the cells relative to the DMSO control group was calculated after incubation with different concentrations of compounds, wherein $EGFR_{compound}$ was the fluorescence value of EGFR protein after incubation with compounds, and $EGFR_{vehicle}$ was the fluorescence value of EGFR protein in the DMSO control group. Using the Origin 9.2 software, the drug concentration $DC_{50}$ values when the expression level of EGFR protein was 50% relative to the DMSO control group were calculated.

$$EGFR\% = EGFR_{compound}/EGFR_{vehicle} \times 100\% \text{ formula (3)}$$

Table 4 Degradation activity results of compounds on EGFR protein in NCI-H1975 EGFR-L858R-T790M-C797S cells

| Compound No. | Test method | $DC_{50}$ (μM) | Compound No. | Test method | $DC_{50}$ (μM) |
|---|---|---|---|---|---|
| Compound 2 | Method I | A | Compound 96 | Method I | A |
| Trifluoroacetate of compound 23 | Method I | A | Compound 97 | Method I | A |
| Compound 37 | Method I | A | Compound 99 | Method I | A |
| Compound 54 | Method I | A | Compound 100 | Method I | A |
| Compound 57 | Method I | A | Compound 102 | Method II | A |
| Compound 59 | Method I | A | Compound 103 | Method II | A |
| Compound 60 | Method I | A | Compound 104 | Method II | A |
| Compound 62 | Method I | A | Compound 106 | Method II | A |
| Compound 67 | Method I | A | Compound 107 | Method II | A |
| Compound 68 | Method I | A | Compound 108 | Method II | A |
| Compound 77 | Method I | A | Compound 109 | Method II | A |
| Compound 81 | Method I | A | Compound 112 | Method II | A |
| Compound 84 | Method I | A | Compound 113 | Method II | A |
| Compound 90 | Method I | A | Compound 116 | Method II | A |
| Compound 92 | Method I | A | Compound 117 | Method II | A |
| Notes: A < 0.1 μM, 0.1 μM ≤ B < 0.5 μM, and 0.5 μM < C < 1 μM in table 4. | | | | | |

Conclusion: the compounds of the present invention have good degradation effects on EGFR protein in H1975-EGFR-T790M-L858R-C797S cells.

## Claims

**1.** A compound or a stereoisomer, a deuterated compound, a solvate, a prodrug, a metabolite, a pharmaceutically acceptable salt or a co-crystal thereof, wherein, the compound is selected from a compound shown in general formula (I),

B-L-K (I);

L is selected from a bond or $-C_{1-50}$ hydrocarbyl-, wherein the hydrocarbyl has 0 to 20 methylene units optionally

further replaced by -Ak- or -Cy-;

each -Ak- is independently selected from $-(CH_2)_q-$, $-(CH_2)_q-O-$, $-O-(CH_2)_q-$, $-(CH_2)_q-NR^L-$, $-NR^L-(CH_2)_q-$, $-(CH_2)_q-NR^LC(=O)-$, $-NR^L(CH_2)_qC(=O)-$, $-(CH_2)_q-C(=O)NR^L-$, -C(=O)-, $-C(=O)-(CH_2)_q-NR^L-$, $-(C\equiv C)_q-$, -CH=CH-, $-Si(R^L)_2-$, $-Si(OH)(R^L)-$, $-Si(OH)_2-$, $-P(=O)(OR^L)-$, $-P(=O)(R^L)-$, -S-, -S(=O)-, $-S(=O)_2-$ or a bond, wherein the $-CH_2-$ is optionally further substituted with 0 to 2 substituents selected from H, halogen, OH, CN, $NH_2$, $C_{1-6}$ alkyl, $C_{1-6}$ alkoxy, halogen-substituted $C_{1-6}$ alkyl, hydroxyl-substituted $C_{1-6}$ alkyl or cyano-substituted $C_{1-6}$ alkyl;

each q is independently selected from 0, 1, 2, 3, 4, 5 or 6;

each $R^L$ is independently selected from H, $C_{1-6}$ alkyl, 3- to 7-membered heterocyclyl, 3- to 7-membered cycloalkyl, phenyl or 5- to 6-membered heteroaryl;

each -Cy- is independently selected from a bond, a 4- to 8-membered mono-heterocyclic ring, a 4- to 10-membered fused-heterocyclic ring, a 5- to 12-membered spiro-heterocyclic ring, a 7- to 10-membered bridged-heterocyclic ring, 3- to 7-membered monocycloalkyl, 4- to 10-membered fused cycloalkyl, 5- to 12-membered spiro cycloalkyl, 7- to 10-membered bridged cycloalkyl, 5- to 10-membered heteroaryl or 6- to 10-membered aryl, wherein the aryl, heteroaryl, cycloalkyl, mono-heterocyclic ring, fused-heterocyclic ring, spiro-heterocyclic ring or bridged-heterocyclic ring is optionally further substituted with 0 to 4 substituents selected from H, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the heteroaryl, mono-heterocyclic ring, fused-heterocyclic ring, spiro-heterocyclic ring or bridged-heterocyclic ring contains 1 to 4 heteroatoms selected from O, S or N, and is optionally further substituted with 0, 1 or 2 =O when the heteroatom is selected from S;

B is selected from

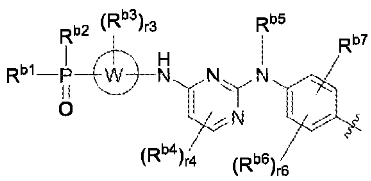

or

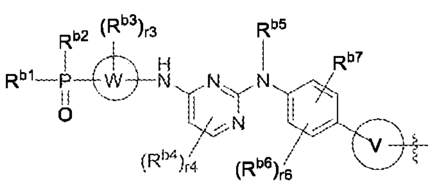

;

r3 is selected from 0, 1, 2, 3 or 4;

r4 is selected from 0, 1 or 2;

r6 is selected from 0, 1, 2 or 3;

ring W is selected from $C_{6-10}$ carbocycle, 5- to 10-membered heterocycle, a $C_{6-10}$ aromatic ring or a 5- to 10-membered heteroaromatic ring, wherein the heterocycle or heteroaromatic ring contains 1 to 5 heteroatoms selected from O, S or N;

ring V is selected from 4- to 10-membered heterocycle or $C_{3-10}$ carbocycle, wherein the heterocycle or carbocycle is optionally further substituted with 0 to 4 substituents selected from H, F, Cl, Br, I, OH, =O, CN, $C_{1-4}$ alkyl, halogen-substituted $C_{1-4}$ alkyl, cyano-substituted $C_{1-4}$ alkyl or $C_{3-6}$ cycloalkyl;

$R^{b1}$ and $R^{b2}$ are each independently selected from H, OH or $C_{1-4}$ alkyl;

each $R^{b3}$ is independently selected from H, F, Cl, Br, I, OH, CN, $C_{1-4}$ alkyl, $C_{2-4}$ alkynyl, $C_{1-4}$ alkoxy, $C_{3-6}$ cycloalkyl, 3- to 6-membered heterocyclyl, phenyl or 5- to 6-membered heteroaryl, wherein the alkyl, alkynyl, alkoxy, cycloalkyl, phenyl, heterocyclyl or heteroaryl is optionally further substituted with 0 to 4 substituents selected from H, F, Cl, Br, I, OH, =O, $NH_2$, CN, COOH, $C_{1-4}$ alkyl, $C_{1-4}$ alkoxy, halogen-substituted $C_{1-4}$ alkyl, cyano-substituted $C_{1-4}$ alkyl or $C_{3-6}$ cycloalkyl, and the heterocyclyl or heteroaryl contains 1 to 3 heteroatoms selected from O, S or N;

each $R^{b4}$ is independently selected from H, F, Cl, Br, I, OH, CN, $C_{1-4}$ alkyl or $C_{1-4}$ alkoxy, wherein the alkyl or alkoxy is optionally further substituted with 0 to 4 substituents selected from H, F, Cl, Br, I, OH, CN, $C_{1-4}$ alkyl, $C_{1-4}$ alkoxy or $C_{3-6}$ cycloalkyl;

$R^{b5}$ is selected from H or $C_{1-4}$ alkyl, wherein the alkyl is optionally further substituted with 0 to 4 substituents

selected from H, F, Cl, Br, I, $C_{1-4}$ alkyl, $C_{1-4}$ alkoxy or $C_{3-6}$ cycloalkyl;
each $R^{b6}$ is independently selected from H, F, Cl, Br, I, $C_{1-4}$ alkyl, $C_{2-4}$ alkynyl or $C_{1-4}$ alkoxy, wherein the alkyl, alkynyl or alkoxy is optionally further substituted with 0 to 4 substituents selected from H, F, Cl, Br, I, OH, CN, $C_{1-4}$ alkyl, $C_{1-4}$ alkoxy or $C_{3-6}$ cycloalkyl;
$R^{b7}$ is selected from 4- to 7-membered heterocycle or $C_{3-7}$ carbocycle, wherein the carbocycle or heterocycle is optionally further substituted with 0 to 4 substituents selected from H, F, Cl, Br, I, OH, =O, CN, $C_{1-4}$ alkyl, deuterated $C_{1-4}$ alkyl, $C_{1-4}$ alkoxy, halogen-substituted $C_{1-4}$ alkyl, cyano-substituted $C_{1-4}$ alkyl or $C_{3-6}$ cycloalkyl, and the heterocyclyl contains 1 to 3 heteroatoms selected from O, S or N;
K is selected from

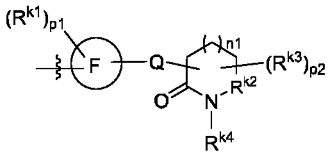 , 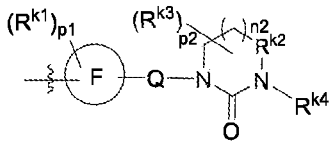 ,

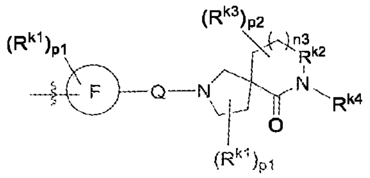 , 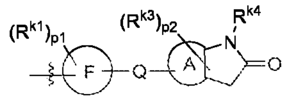 , 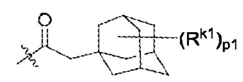 ,

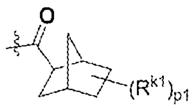 , 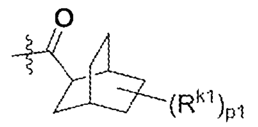 , 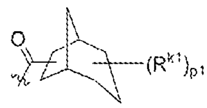 , 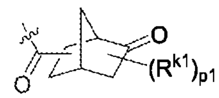 ,

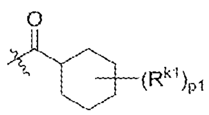 , 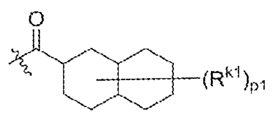 , 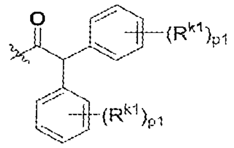 , 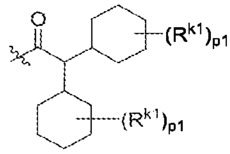 ,

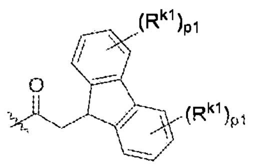 , 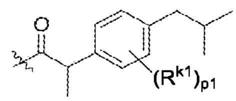 , 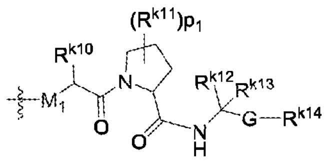 ,

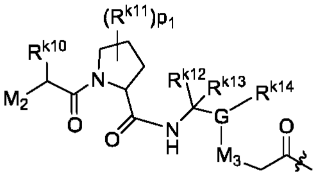 or 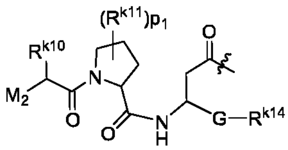 ;

each Q is independently selected from a bond, -O-, -S-, $-CH_2-$, $-NR^4-$, -CO-, $-NR^qCO-$, $-CONR^q-$ or 3- to 12-membered heterocycle, wherein the heterocycle is optionally further substituted with 0 to 4 substituents selected from H, F, Cl, Br, I, OH, =O, $NH_2$, CN, COOH, $CONH_2$, $C_{1-4}$ alkyl or $C_{1-4}$ alkoxy, and the heterocycle contains 1 to 4 heteroatoms selected from O, S or N;
$R^q$ is selected from H or $C_{1-6}$ alkyl;

A is selected from $C_{3-10}$ carbocycle, a $C_{6-10}$ aromatic ring, 3- to 10-membered heterocycle or a 5- to 10-membered heteroaromatic ring, wherein the heterocycle or heteroaromatic ring contains 1 to 4 heteroatoms selected from O, S or N;

each F is independently selected from $C_{3-20}$ carbocycle, a $C_{6-20}$ aromatic ring, 3- to 20-membered heterocycle or a 5- to 20-membered heteroaromatic ring, wherein the heterocycle or heteroaromatic ring contains 1 to 4 heteroatoms selected from O, S or N;

each $R^{k2}$ is independently selected from a bond, -CO-, $-SO_2-$, -SO- or - $C(R^{k3})_2$-;

each $R^{k1}$ is independently selected from H, F, Cl, Br, I, OH, =O, $NH_2$, CN, COOH, $CONH_2$, $C_{1-6}$ alkyl, $C_{1-6}$ alkoxy or $C_{3-6}$ cycloalkyl, wherein the alkyl, alkoxy or cycloalkyl is optionally further substituted with 0 to 4 substituents selected from H, F, Cl, Br, I, OH, =O, $NH_2$, CN, COOH, $CONH_2$, $C_{1-4}$ alkyl, $C_{1-4}$ alkoxy or $C_{3-6}$ cycloalkyl;

each $R^{k3}$ is independently selected from H, F, Cl, Br, I, OH, =O, $NH_2$, CN, COOH, $CONH_2$, $C_{1-6}$ alkyl, $C_{1-6}$ alkoxy, $C_{3-8}$ cycloalkyl or 3- to 8-membered heterocyclyl, wherein the alkyl, alkoxy, cycloalkyl or heterocyclyl is optionally further substituted with 0 to 4 substituents selected from H, F, Cl, Br, I, OH, =O, $NH_2$, CN, COOH, $CONH_2$, $C_{1-4}$ alkyl or $C_{1-4}$ alkoxy, and the heterocyclyl contains 1 to 4 heteroatoms selected from O, S or N;

or two $R^{k3}$ together with the carbon atoms or ring backbones to which they are directly attached form 3- to 8-membered carbocycle or 3- to 8-membered heterocycle, and two $R^{k1}$ together with the carbon atoms or ring backbones to which they are directly attached form 3- to 8-membered carbocycle or 3- to 8-membered heterocycle, wherein the carbocycle or heterocycle is optionally further substituted with 0 to 4 substituents selected from H, F, Cl, Br, I, OH, =O, $NH_2$, CN, COOH, $CONH_2$, $C_{1-4}$ alkyl or $C_{1-4}$ alkoxy, and the heterocycle contains 1 to 4 heteroatoms selected from O, S or N;

each $R^{k4}$ is independently selected from H, OH, $NH_2$, CN, $CONH_2$, $C_{1-6}$ alkyl, $C_{3-8}$ cycloalkyl or 3- to 8-membered heterocyclyl, wherein the alkyl, cycloalkyl or heterocyclyl is optionally further substituted with 0 to 4 substituents selected from H, F, Cl, Br, I, OH, =O, $NH_2$, CN, COOH, $CONH_2$, $C_{1-4}$ alkyl or $C_{1-4}$ alkoxy, and the heterocyclyl contains 1 to 4 heteroatoms selected from O, S or N;

$M_1$ is selected from a bond, $-CH_2-C(=O)NH-$ or $-C(=O)CH_2NH-$;

$M_2$ is selected from -NHC(=O)-$C_{1-6}$ alkyl, -NHC(=O)-$C_{3-6}$ cycloalkyl or 4- to 10-membered heterocyclyl, wherein the alkyl, cycloalkyl or heterocyclyl is optionally further substituted with 0 to 4 substituents selected from H, F, Cl, Br, I, =O, OH, $NH_2$, $C_{1-4}$ alkyl or $C_{1-4}$ alkoxy, and the heterocyclyl contains 1 to 4 heteroatoms selected from O, S or N;

$M_3$ is selected from -NH- or -O-;

$R^{k10}$ is selected from $C_{1-6}$ alkyl, wherein the alkyl is optionally further substituted with 0 to 4 substituents selected from H, F, Cl, Br, I, =O, OH, $C_{1-6}$ alkyl or $C_{3-6}$ cycloalkyl;

each $R^{k11}$ is independently selected from H, F, Cl, Br, I, =O, OH, SH, $C_{1-6}$ alkyl, $C_{1-6}$ alkoxy, $C_{1-6}$ alkylthio or -O-C(=O)-$C_{1-6}$ alkyl, wherein the alkyl, alkoxy or alkylthio is optionally further substituted with 0 to 4 substituents selected from H, F, Cl, Br, I, OH, $C_{1-4}$ alkyl or $C_{1-4}$ alkoxy;

$R^{k12}$ and $R^{k13}$ are each independently selected from H, $C_{1-6}$ alkyl or $C_{3-6}$ cycloalkyl, wherein the alkyl or cycloalkyl is optionally further substituted with 0 to 4 substituents selected from H, F, Cl, Br, I, =O, OH, $NH_2$, $C_{1-4}$ alkyl or $C_{1-4}$ alkoxy;

$R^{k14}$ is selected from 5- to 6-membered heteroaryl, wherein the heteroaryl is optionally further substituted with 0 to 4 substituents selected from H, F, Cl, Br, I, OH, =O, $CF_3$, CN, $C_{1-4}$ alkyl, halogen-substituted $C_{1-4}$ alkyl, hydroxyl-substituted $C_{1-4}$ alkyl, $C_{1-4}$ alkoxy or $C_{3-6}$ cycloalkyl, and the heteroaryl contains 1 to 4 heteroatoms selected from N, O or S;

G is selected from a 6- to 10-membered aromatic ring or a 5- to 10-membered heteroaromatic ring, wherein the aromatic ring or heteroaromatic ring is optionally further substituted with 0 to 4 substituents selected from H, F, Cl, Br, I, OH, =O, $CF_3$, CN, $C_{1-4}$ alkyl, halogen-substituted $C_{1-4}$ alkyl, hydroxyl-substituted $C_{1-4}$ alkyl, $C_{1-4}$ alkoxy or $C_{3-6}$ cycloalkyl, and the heteroaromatic ring contains 1 to 4 heteroatoms selected from N, O or S;

n1, n2 and n3 are each independently selected from 0, 1, 2 or 3;

each p1 or p2 is independently selected from 0, 1, 2, 3, 4 or 5.

**2.** The compound or the stereoisomer, deuterated compound, solvate, prodrug, metabolite, pharmaceutically acceptable salt or co-crystal thereof according to claim 1, wherein,

L is selected from -Cy1-Ak1-Cy2-Ak2-Cy3-Ak3-Cy4-Ak4-Cy5-Ak5-, -Cy1-Cy2-Cy3-Cy4-Ak1-Ak2-Ak3-Ak4-Ak5-, -Cy1-Ak1-Cy2-Ak2-Cy3 -Ak3 -Cy4-Ak4-Ak5-, -Ak1-Cy1-Ak2-Cy2-Ak3-Cy3-Ak4-Cy4-Ak5-, -Cy1-Ak1-Cy2-Ak2-Cy3-Cy4-Ak3-Ak4-Ak5-, -Cy1-Ak1-Cy2-Ak2-Ak3-Cy3-Cy4-Ak4-Ak5-, -Cy1-Ak1-Ak2-Ak3-Ak4-Ak5-Cy2-Cy3-Cy4-, -Cy1-Cy2-Ak1-Ak2-Ak3-Ak4-Ak5-Cy3-Cy4-, -Cy 1 -Cy2-Cy3 - Ak 1 - Ak2-Ak3 - Ak4-Ak5 -Cy4-, -Cy1-Cy2-Cy3-Cy4-Ak1-Ak2-Ak3-Ak4-Ak5-, -Cy1-Ak1-Cy2-Cy3-Cy4-Ak2-Ak3-Ak4-Ak5-, -Cy1-Cy2-Ak1-Cy3-Cy4-Ak2-Ak3-Ak4-Ak5-, -Cy1-Cy2-Cy3-Ak1-Cy4-Ak2-Ak3-Ak4-Ak5-, - Cy1-Ak1-Ak2-Cy2-Cy3 -Cy4-Ak3 -Ak4-Ak5-, -Cy1-Cy2-Ak1-Ak2-Cy3-Cy4-Ak3 - Ak4-Ak5-, -Cy1-Cy2-Cy3-Ak1-Ak2-Cy4-Ak3-Ak4-Ak5-, -Cy1-Ak1-Ak2-Ak3-Cy2-Cy3-Cy4-Ak4-Ak5-, -Cy1-Cy2-Ak1-Ak2-Ak3-Cy3-Cy4-Ak4-Ak5-, -Cy1-Cy2-Cy3-Ak1-Ak2-Ak3-Cy4-Ak4-Ak5-, -Cy1-Ak1-Ak2-Ak3-Ak4-Cy2-Cy3-Cy4-Ak5-, -Cy1-Cy2-Ak1-Ak2-Ak3-Ak4-Cy3-Cy4-Ak5-, -Cy1-Cy2-Cy3-Ak1-Ak2-Ak3-Ak4-Cy4-Ak5-, - Ak 1 - Ak2-Ak3 - Ak4-Ak5 -Cy 1 -Cy2-Cy3 -Cy4-, - Ak 1 -Cy 1 - Cy2-Cy3-Cy4-Ak2-Ak3-Ak4-Ak5-, -Ak1-Ak2-Cy1-Cy2-Cy3-Cy4-Ak3-Ak4-Ak5-, - Ak 1 - Ak2-Ak3 -Cy 1 -Cy2-Cy3 -Cy4-Ak4-Ak5 -, - Ak 1 - Ak2-Ak3 - Ak4-Cy 1 -Cy2-Cy3-Cy4-Ak5-, -Ak1-Cy1-Ak2-Ak3-Ak4-Ak5-Cy2-Cy3-Cy4-, -Ak1-Cy1-Cy2-Ak2-Ak3-Ak4-Ak5-Cy3-Cy4-, -Ak1-Cy 1-Cy2-Cy3 -Ak2-Ak3-Ak4-Ak5-Cy4-, - Ak1-Ak2-Cy1-Ak3-Ak4-Ak5-Cy2-Cy3-Cy4-, -Ak1-Ak2-Cy1-Cy2-Ak3-Ak4-Ak5-Cy3-Cy4-, - Ak 1 - Ak2-Cy 1 -Cy2-Cy3 - Ak3 - Ak4-Ak5 -Cy4-, - Ak 1 - Ak2-Ak3 -Cy 1 - Ak4-Ak5-Cy2-Cy3-Cy4-, -Ak1-Ak2-Ak3-Cy 1-Cy2-Ak4-Ak5-Cy3 -Cy4-, -Ak1-Ak2-Ak3-Cy 1-Cy2-Cy3 -Ak4-Ak5-Cy4-, -Ak1-Ak2-Ak3-Ak4-Cy1-Ak5-Cy2-Cy3-Cy4-, -Ak1-Ak2-Ak3-Ak4-Cy1-Cy2-Ak5-Cy3-Cy4-, or -Ak1-Ak2-Ak3-Ak4-Cy1-Cy2-Cy3-Ak5-Cy4-;

Ak1, Ak2, Ak3, Ak4 and Ak5 are each independently selected from $-(CH_2)_q-$, $-(CH_2)_q-O-$, $-O-(CH_2)_q-$, $-(CH_2)_q-NR^L-$, $-NR^L-(CH_2)_q-$, $-(CH_2)q-NR^LC(=O)-$, - $(CH_2)_q-C(=O)NR^L-$, $-C(=O)-$, $-C(=O)-(CH_2)_q-NR^L-$, $-(C\equiv C)_q-$ or a bond, wherein the $-CH_2-$ is optionally further substituted with 0 to 2 substituents selected from H, halogen, OH, CN, $NH_2$, $C_{1-4}$ alkyl, $C_{1-4}$ alkoxy, halogen-substituted $C_{1-4}$ alkyl, hydroxyl-substituted $C_{1-4}$ alkyl or cyano-substituted $C_{1-4}$ alkyl;

each Cy1, Cy2, Cy3, Cy4 or Cy5 is independently selected from a bond, a 4-to 7-membered mono-heterocyclic ring, a 4- to 10-membered fused-heterocyclic ring, a 5- to 12-membered spiro-heterocyclic ring, a 7- to 10-membered bridged-heterocyclic ring, 3- to 7-membered monocycloalkyl, 4- to 10-membered fused cycloalkyl, 5- to 12-membered spiro cycloalkyl, 7- to 10-membered bridged cycloalkyl, 5- to 10-membered heteroaryl or 6- to 10-membered aryl, wherein the aryl, heteroaryl, cycloalkyl, mono-heterocyclic ring, fused-heterocyclic ring, spiro-heterocyclic ring or bridged-heterocyclic ring is optionally further substituted with 0 to 4 substituents selected from H, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the heteroaryl, mono-heterocyclic ring, fused-heterocyclic ring, spiro-heterocyclic ring or bridged-heterocyclic ring contains 1 to 4 heteroatoms selected from O, S or N, and is optionally further substituted with 0, 1 or 2 =O when the heteroatom is selected from S;

each q is independently selected from 0, 1, 2, 3 or 4;

each $R^L$ is independently selected from H or $C_{1-6}$ alkyl.

**3.** The compound or the stereoisomer, deuterated compound, solvate, prodrug, metabolite, pharmaceutically acceptable salt or co-crystal thereof according to claim 2, wherein,

each Cy1, Cy2, Cy3, Cy4 or Cy5 is independently selected from a bond, a 4-to 7-membered nitrogen-containing mono-heterocyclic ring, a 4- to 10-membered nitrogen-containing fused-heterocyclic ring, a 5- to 12-membered nitrogen-containing spiro-heterocyclic ring, a 7- to 10-membered nitrogen-containing bridged-heterocyclic ring, 3- to 7-membered monocycloalkyl, 4- to 10-membered fused cycloalkyl, 5- to 12-membered spiro cycloalkyl, 7- to 10-membered bridged cycloalkyl, 5- to 10-membered heteroaryl or 6- to 10-membered aryl, wherein the mono-heterocyclic ring, fused-heterocyclic ring, bridged-heterocyclic ring, spiro-heterocyclic ring, cycloalkyl, aryl or heteroaryl is optionally further substituted with 0 to 4 substituents selected from H, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the mono-heterocyclic ring, fused-heterocyclic ring, bridged-heterocyclic ring, spiro-heterocyclic ring or heteroaryl contains 1 to 4 heteroatoms selected from O, S or N, and is optionally further substituted with 0, 1 or 2 =O when the heteroatom is selected from S;

each $R^L$ is independently selected from H or $C_{1-4}$ alkyl;

ring W is selected from a benzene ring, a naphthalene ring, a 5- to 6-membered monocyclic heteroaromatic ring, a 9- to 10-membered fused heteroaromatic ring or 9- to 10-membered heterocycle, wherein the heterocycle or heteroaromatic ring contains 1 to 5 heteroatoms selected from O, S or N;

ring V is selected from 4- to 6-membered heterocycle or $C_{3-6}$ carbocycle, wherein the heterocycle or carbocycle is optionally further substituted with 0 to 4 substituents selected from H, F, Cl, Br, I, OH, =O, CN, $C_{1-4}$ alkyl, halogen-substituted $C_{1-4}$ alkyl, cyano-substituted $C_{1-4}$ alkyl or $C_{3-6}$ cycloalkyl;

K is selected from

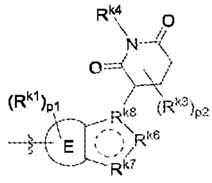,
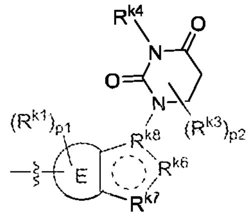,
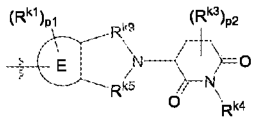,

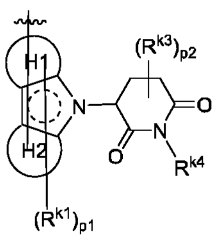,
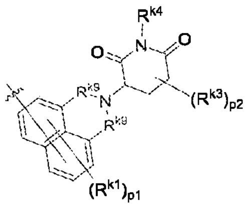,
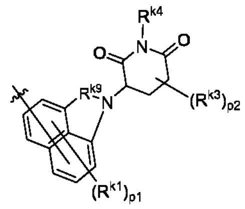,
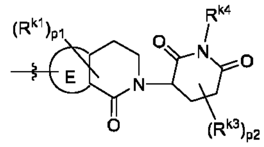,

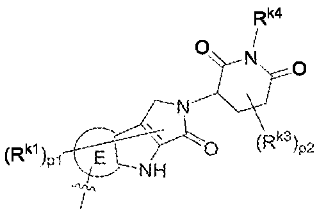,
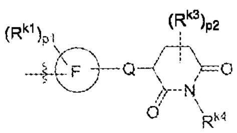,
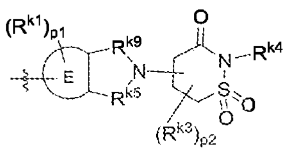,

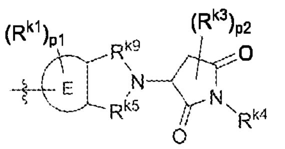,
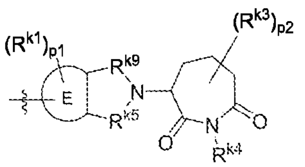,
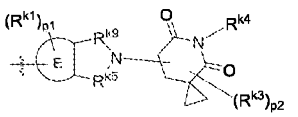,

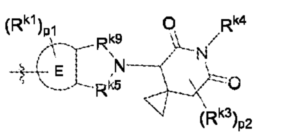,
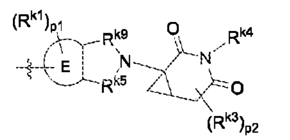,
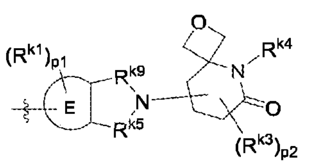,

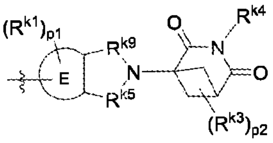,
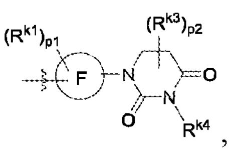,
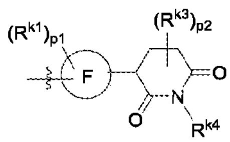,
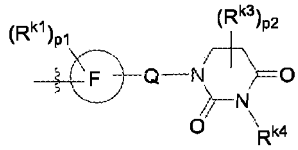,

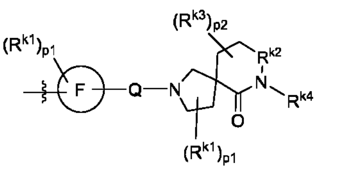,
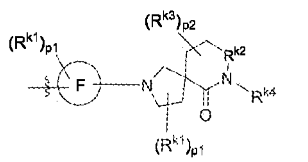,
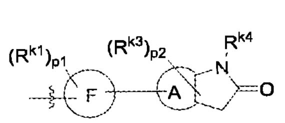,

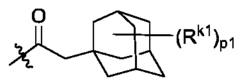, 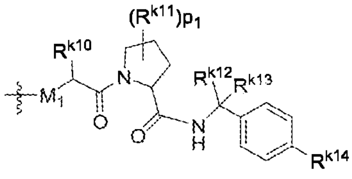, 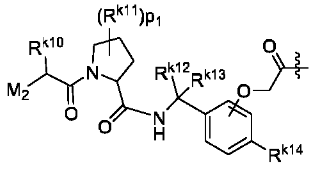

or

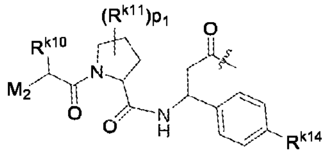;

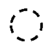

represents a ring selected from an aromatic ring or a non-aromatic ring;

each Q is independently selected from -O-, -S-, $-CH_2-$, $-NR^q-$, -CO-, $-NR^qCO-$, $-CONR^q-$ or 4- to 7-membered heterocycle, wherein the heterocycle is optionally further substituted with 0 to 4 substituents selected from H, F, Cl, Br, I, OH, =O, $NH_2$, CN, COOH, $CONH_2$, $C_{1-4}$ alkyl or $C_{1-4}$ alkoxy, and the heterocycle contains 1 to 4 heteroatoms selected from O, S or N;

$R^q$ is selected from H or $C_{1-4}$ alkyl;

$R^{k1}$ and $R^{k3}$ are each independently selected from H, F, Cl, Br, I, OH, =O, $NH_2$, $CF_3$, CN, COOH, $CONH_2$, $C_{1-4}$ alkyl or $C_{1-4}$ alkoxy, wherein the alkyl or alkoxy is optionally further substituted with 0 to 4 substituents selected from H, F, Cl, Br, I, OH or $NH_2$;

or two $R^{k3}$ together with the carbon atoms or ring backbones to which they are directly attached form 3- to 6-membered carbocycle or 3- to 7-membered heterocycle, and two $R^{k1}$ together with the carbon atoms or ring backbones to which they are directly attached form 3- to 6-membered carbocycle or 3- to 7-membered heterocycle, wherein the carbocycle or heterocycle is optionally further substituted with 0 to 4 substituents selected from H, F, Cl, Br, I, OH, =O, $NH_2$, CN, COOH, $CONH_2$, $C_{1-4}$ alkyl or $C_{1-4}$ alkoxy, and the heterocycle contains 1 to 4 heteroatoms selected from O, S or N;

each $R^{k4}$ is independently selected from H, OH, $NH_2$, $CF_3$, CN or $C_{1-4}$ alkyl;

each $R^{k5}$ is independently selected from

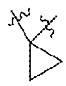,

$C(CH_3)_2$, CO, $CH_2$, $SO_2$,

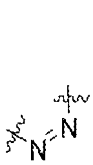, 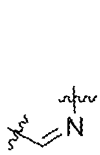 or 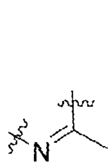;

each $R^{k6}$ is independently selected from CO, CH, SO, $SO_2$, $CH_2$ or N;

each $R^{k7}$ is independently selected from

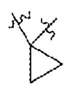,

$C(CH_3)_2$, CO, CH, N, $CH_2$, O, S, $N(CH_3)$, $N(CH_2CH_3)$, N(cyclopropyl) or NH;

each $R^{k8}$ is independently selected from C, N or CH;
each $R^{k9}$ is independently selected from a bond,

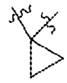,

$C(CH_3)_2$, CO, $CH_2$, $CH_2CH_2$ or $SO_2$;
each A, H1 or H2 is independently selected from $C_{3-8}$ carbocycle, a benzene ring, 4- to 7-membered heterocycle or a 5- to 6-membered heteroaromatic ring, wherein the heterocycle or heteroaromatic ring contains 1 to 4 heteroatoms selected from O, S or N;
each E is independently selected from $C_{3-8}$ carbocycle, a benzene ring, 4- to 7-membered heterocycle, 8- to 12-membered heterocycle, a 7- to 12-membered heteroaromatic ring or a 5- to 6-membered heteroaromatic ring, wherein the heterocycle or heteroaromatic ring contains 1 to 4 heteroatoms selected from O, S or N;
each F is independently selected from 3- to 7-membered monocycloalkyl, 4-to 10-membered fused cycloalkyl, 5- to 12-membered spiro cycloalkyl, 5- to 10-membered bridged cycloalkyl, a 4- to 7-membered mono-heterocyclic ring, a 4- to 10-membered fused-heterocyclic ring, a 5- to 12-membered spiro-heterocyclic ring, a 5- to 10-membered bridged-heterocyclic ring, $C_{6-14}$ aryl or 5- to 10-membered heteroaryl, wherein the mono-heterocyclic ring, fused-heterocyclic ring, spiro-heterocyclic ring, bridged-heterocyclic ring or heteroaryl contains 1 to 4 heteroatoms selected from O, S or N .

4. The compound or the stereoisomer, deuterated compound, solvate, prodrug, metabolite, pharmaceutically acceptable salt or co-crystal thereof according to claim 3, wherein,

$R^L$ is selected from H, methyl or ethyl;
each q is independently selected from 0, 1 or 2;
each Cy1, Cy2, Cy3, Cy4 or Cy5 is independently selected from a bond or one of the following substituted or unsubstituted groups: cyclopropyl, cyclobutyl, cyclopentyl, cyclohexyl, azetidinyl, azacyclopentyl, azacyclohexenyl, piperidine, morpholine, piperazine, phenyl, cyclopropyl-fused-cyclopropyl, cyclopropyl-fused-cyclobutyl, cyclopropyl-fused-cyclopentyl, cyclopropyl-fused-cyclohexyl, cyclobutyl-fused-cyclobutyl, cyclobutyl-fused-cyclopentyl, cyclobutyl-fused-cyclohexyl, cyclopentyl-fused-cyclopentyl, cyclopentyl-fused-cyclohexyl, cyclohexyl-fused-cyclohexyl, cyclopropyl-spiro-cyclopropyl, cyclopropyl-spiro-cyclobutyl, cyclopropyl-spiro-cyclopentyl, cyclopropyl-spiro-cyclohexyl, cyclobutyl-spiro-cyclobutyl, cyclobutyl-spiro-cyclopentyl, cyclobutyl-spiro-cyclohexyl, cyclopentyl-spiro-cyclopentyl, cyclopentyl-spiro-cyclohexyl, cyclohexyl-spiro-cyclohexyl, cyclopropyl-fused-azetidinyl, cyclopropyl-fused-azacyclopentyl, cyclopropyl-fused-azacyclohexyl, cyclobutyl-fused-azetidinyl, cyclobutyl-fused-azacyclopentyl, cyclobutyl-fused-azacyclohexyl, cyclopentyl-fused-azetidinyl, cyclopentyl-fused-azacyclopentyl, cyclopentyl-fused-azacyclohexyl, cyclohexyl-fused-azetidinyl, cyclohexyl-fused-azacyclopentyl, cyclohexyl-fused-azacyclohexyl, azetidinyl-fused-azetidinyl, azetidinyl-fused-azacyclopentyl, azetidinyl-fused-azacyclohexyl, azacyclopentyl-fused-azetidinyl, azacyclopentyl-fused-azacyclopentyl, azacyclopentyl-fused-azacyclohexyl, azacyclohexyl-fused-azetidinyl, azacyclohexyl-fused-azacyclopentyl, azacyclohexyl-fused-azacyclohexyl, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, cyclohexyl-spiro-azacyclohexyl, azetidinyl-spiro-azetidinyl, azetidinyl-spiro-azacyclopentyl, azetidinyl-spiro-azacyclohexyl, azacyclopentyl-spiro-azetidinyl, azacyclopentyl-spiro-azacyclopentyl, azacyclopentyl-spiro-azacyclohexyl, azacyclohexyl-spiro-azetidinyl, azacyclohexyl-spiro-azacyclopentyl, azacyclohexyl-spiro-azacyclohexyl,

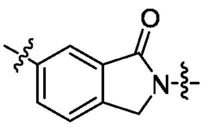, 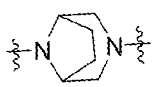, 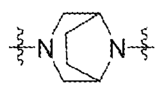, 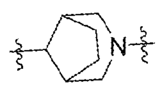,

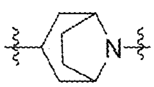, 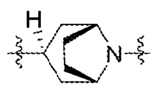, 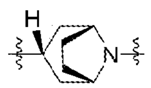, 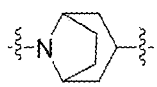, 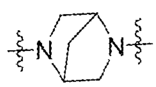, 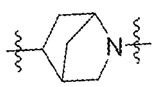,

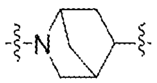, 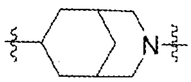, 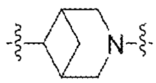, 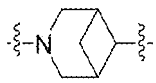, 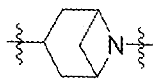, 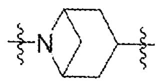,

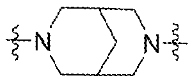, 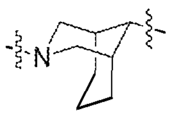, 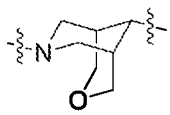, 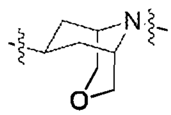, 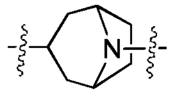,

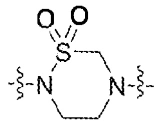 or 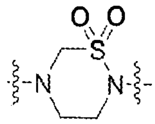,

which, when substituted, is optionally further substituted with 0 to 4 substituents selected from H, F, Cl, Br, I, OH, $NH_2$, COOH, CN, =O, $C_{1-4}$ alkyl, halogen-substituted $C_{1-4}$ alkyl, hydroxyl-substituted $C_{1-4}$ alkyl or $C_{1-4}$ alkoxy;

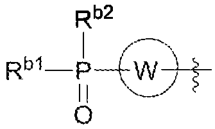

is selected from

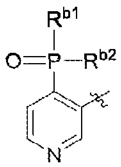, 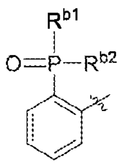, 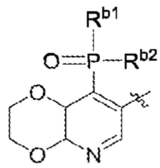,

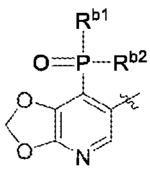, 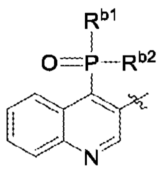, 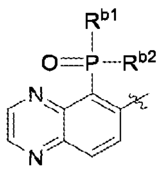, 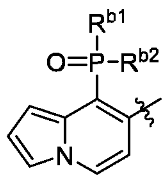, 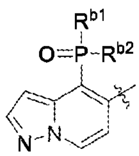, 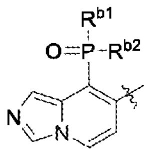,

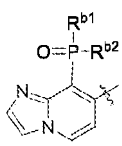, 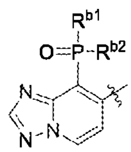, 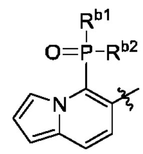, 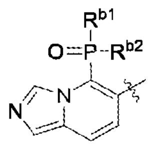, 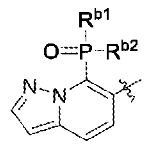, 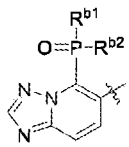, 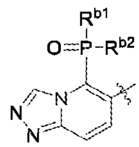

or

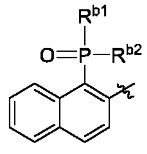;

ring V is selected from azetidinyl, azacyclopentyl, piperidyl or piperazinyl, wherein the azetidinyl, azacyclopentyl, piperidyl or piperazinyl is optionally further substituted with 0 to 4 substituents selected from H, F, Cl, Br, I, OH, =O, CN or $C_{1-4}$ alkyl;

$R^{b1}$ and $R^{b2}$ are each independently selected from methyl or ethyl;

each $R^{b3}$ is independently selected from H, F, Cl, Br, I, OH, CN, methyl, ethyl, isopropyl, propyl, ethynyl, methoxy, ethoxy, propoxy, isopropyloxy, cyclopropyl, cyclobutyl, cyclopentyl, cyclohexyl, phenyl or 5- to 6-membered heteroaryl, wherein the methyl, ethyl, isopropyl, propyl, ethynyl, methoxy, ethoxy, propoxy, isopropyloxy, cyclopropyl, cyclobutyl, cyclopentyl, cyclohexyl, phenyl or 5- to 6-membered heteroaryl is optionally further substituted with substituted with 0 to 4 substituents selected from H, F, Cl, Br, I, OH, =O, $NH_2$, CN, $C_{1-4}$ alkyl, $C_{1-4}$ alkoxy, halogen-substituted $C_{1-4}$ alkyl, cyano-substituted $C_{1-4}$ alkyl or $C_{3-6}$ cycloalkyl, and the heteroaryl contains 1 to 3 heteroatoms selected from O, S or N;

each $R^{b4}$ is independently selected from H, F, Cl, Br, I, OH, CN, methyl, ethyl, isopropyl, propyl, methoxy, ethoxy, propoxy or isopropoxy, wherein the methyl, ethyl, isopropyl, propyl, methoxy, ethoxy, propoxy or isopropoxy is optionally further substituted with 0 to 4 substituents selected from H, F, Cl, Br, I, OH, CN, $C_{1-4}$ alkyl, $C_{1-4}$ alkoxy or $C_{3-6}$ cycloalkyl;

$R^{b5}$ is selected from H;

each $R^{b6}$ is independently selected from H, F, Cl, Br, I, methyl, ethyl, isopropyl, propyl, ethynyl, methoxy, ethoxy, propoxy or isopropoxy, wherein the methyl, ethyl, isopropyl, propyl, ethynyl, methoxy, ethoxy, propoxy or isopropoxy is optionally further substituted with 0 to 4 substituents selected from H, F, Cl, Br, I, OH, CN, $C_{1-4}$ alkyl, $C_{1-4}$ alkoxy or $C_{3-6}$ cycloalkyl;

$R^{b7}$ is selected from cyclopropyl, cyclobutyl, cyclopentyl, cyclohexyl, pyrrole, pyrazole, pyridine or phenyl, wherein the $R^{b7}$ is optionally further substituted with 0 to 4 substituents selected from H, F, Cl, Br, I, OH, =O, CN, deuterated $C_{1-4}$ alkyl, $C_{1-4}$ alkyl, $C_{1-4}$ alkoxy, halogen-substituted $C_{1-4}$ alkyl, cyano-substituted $C_{1-4}$ alkyl or $C_{3-6}$ cycloalkyl;

K is selected from

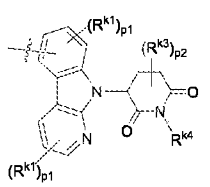 , 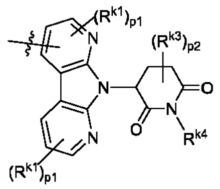 , 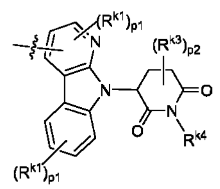 ,

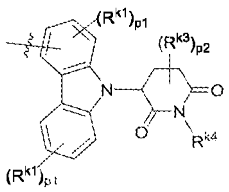 ,, 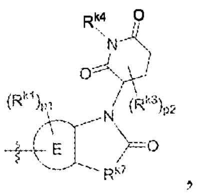 , 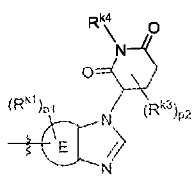 , 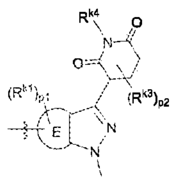 , 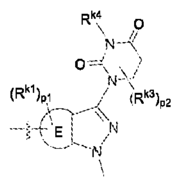 ,

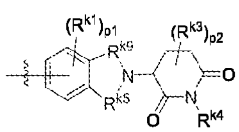 , 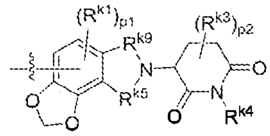 , 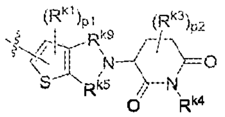 , 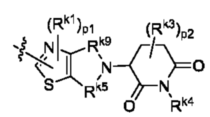 ,

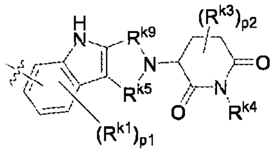 , 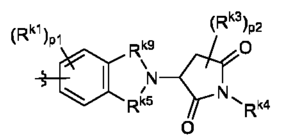 , 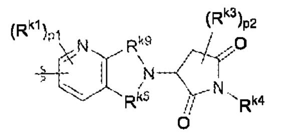 ,

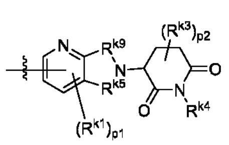 , 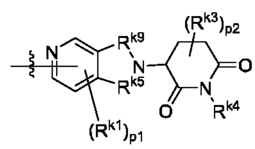 , 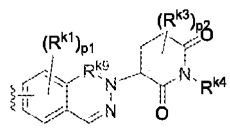 , 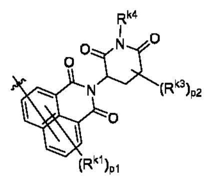 ,

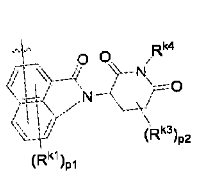,
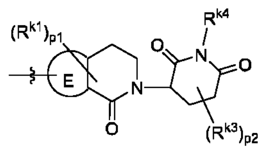,
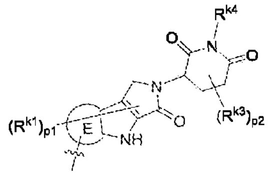,
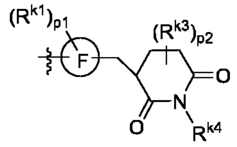,

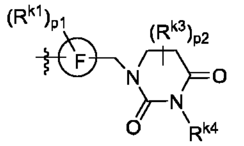,
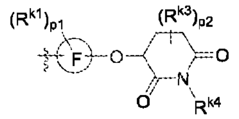,
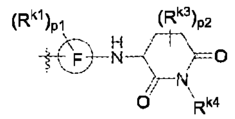,
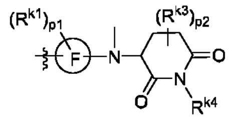,

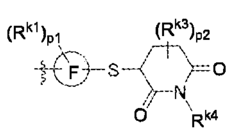,
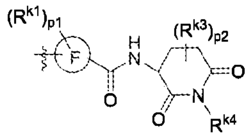,
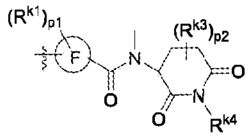,
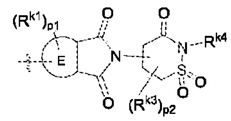,

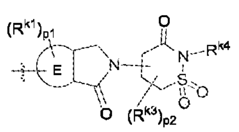,
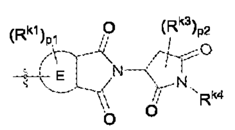,
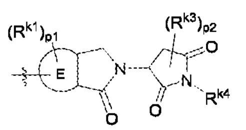,
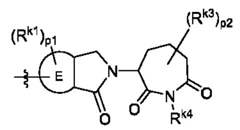,

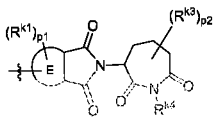,
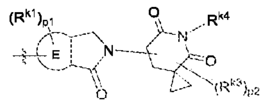,
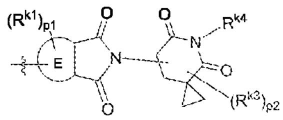,
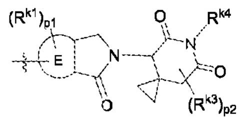,

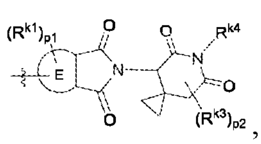,
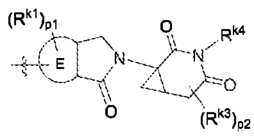,
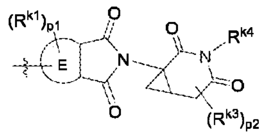,
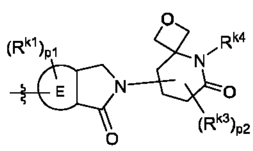,

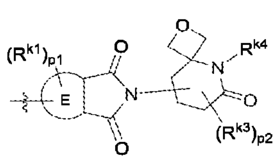,
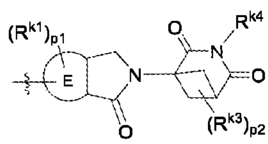,
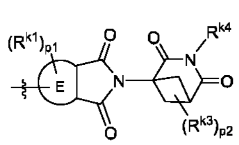,
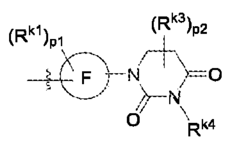,

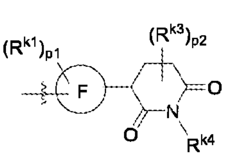,
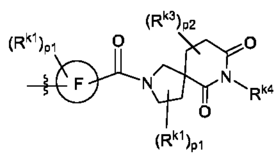,
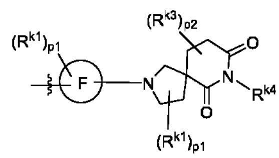,
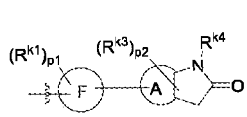,

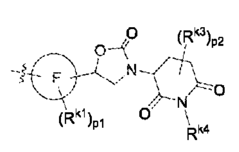,
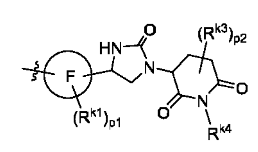,
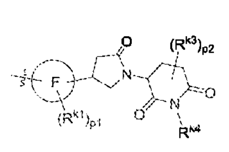,
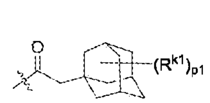,

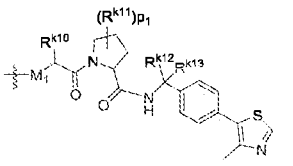, 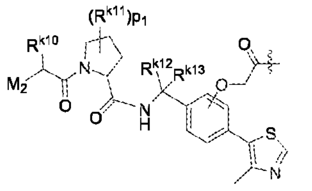, 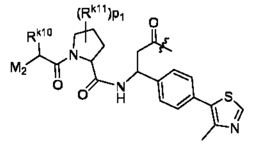,

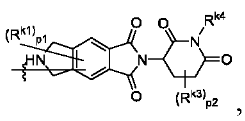, 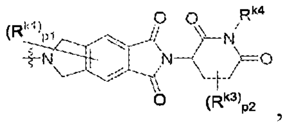, 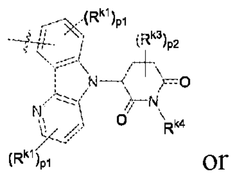 or 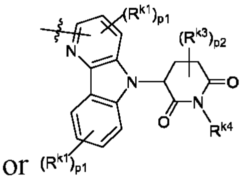;

each E is independently selected from phenyl, pyridyl, pyridazinyl, pyrazinyl, pyrimidinyl, pyrrolyl, pyrazolyl, imidazolyl, thiazolyl, furyl, thienyl or oxazolyl;

each A is independently selected from phenyl, pyridyl, pyridazinyl, pyrazinyl, pyrimidinyl, pyrrolyl, pyrazolyl, imidazolyl, thiazolyl, furyl, thienyl or oxazolyl;

each F is independently selected from cyclopropyl, cyclobutyl, cyclopentyl, cyclohexyl, bicyclo[1.1.1]pentyl, 6,7-dihydro-5H-cyclopenta[c]pyridyl, 2,3-dihydro-1H-indenyl, phenyl, naphthyl, anthryl, phenanthryl, azetidinyl, azacyclopentyl, piperidyl, morpholinyl, pyridyl, pyrimidinyl, pyridazinyl, pyrazinyl, triazinyl, pyrrolyl, pyrazolyl, imidazolyl, triazolyl, oxazolyl, furyl, thienyl, thiazolyl, 2-pyridone, benzoxazolyl, pyridoimidazolyl, benzoimidazolyl, benzopyrazolyl, benzothiazolyl, benzothienyl, benzofuryl, benzopyrrolyl, benzopyridyl, benzopyrazinyl, benzopyrimidinyl, benzopyridazinyl, benzotriazinyl, pyrrolopyrrolyl, pyrrolopyridyl, pyrrolopyrimidinyl, pyrrolopyridazinyl, pyrrolopyrazinyl, imidazopyrimidinyl, imidazopyridyl, imidazopyrazinyl, imidazopyridazinyl, pyrazolopyridyl, pyrazolopyrimidinyl, pyrazolopyridazinyl, pyrazolopyrazinyl, pyrimidopyridyl, pyrimidopyrazinyl, pyrimidopyridazinyl, pyrimidopyrimidinyl, pyridopyridyl, pyridopyrazinyl, pyridopyridazinyl, pyridazinopyridazinyl, pyridazinopyrazinyl or pyrazinopyrazinyl;

each $R^{k7}$ is independently selected from

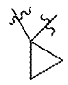,

, $C(CH_3)_2$, $CH_2$, O, $N(CH_3)$, $N(CH_2CH_3)$, N(cyclopropyl) or NH;

each p1 or p2 is independently selected from 0, 1 or 2;.

**5.** The compound or the stereoisomer, deuterated compound, solvate, prodrug, metabolite, pharmaceutically acceptable salt or co-crystal thereof according to claim 4, wherein,

each Cy1, Cy2, Cy3, Cy4 or Cy5 is independently selected from a bond or one of the following substituted or unsubstituted groups:

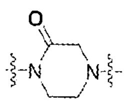, 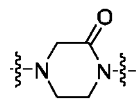,

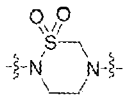, 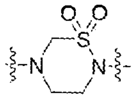, 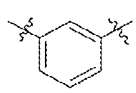, 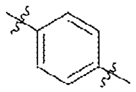, 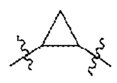, 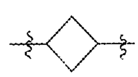, 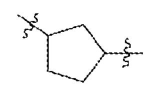,

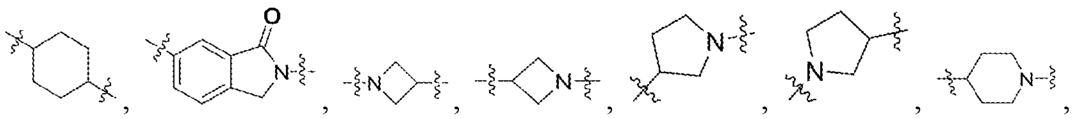
, , , , , , , ,

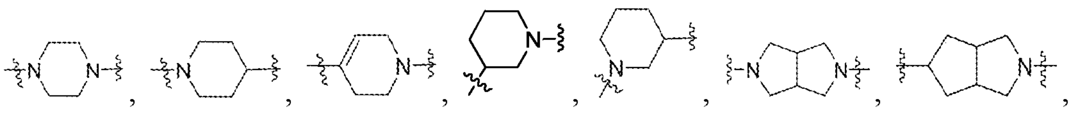
, , , , , , ,

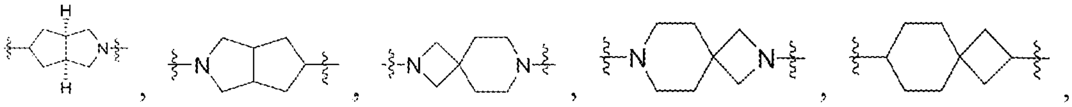
, , , , , ,

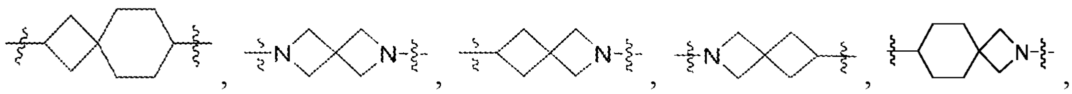
, , , , , ,

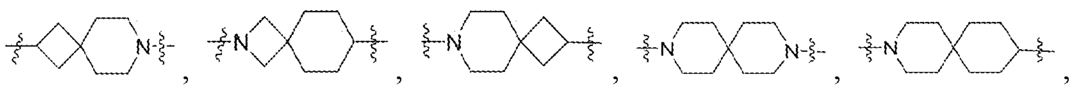
, , , , ,

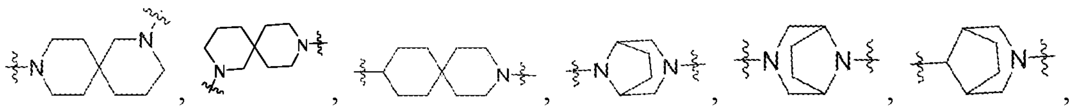
, , , , , ,

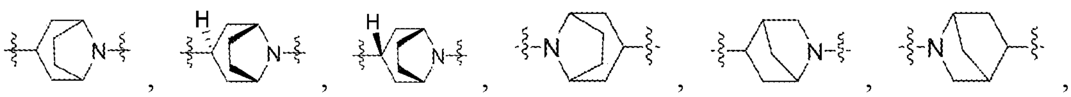
, , , , , ,

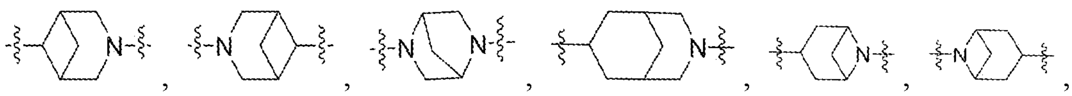
, , , , , ,

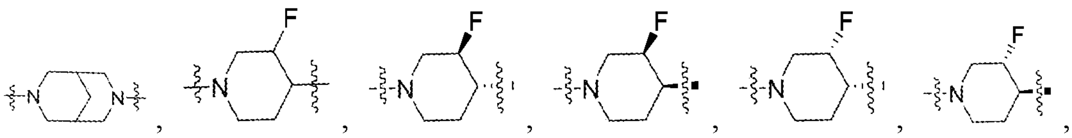
, , , , , , ,

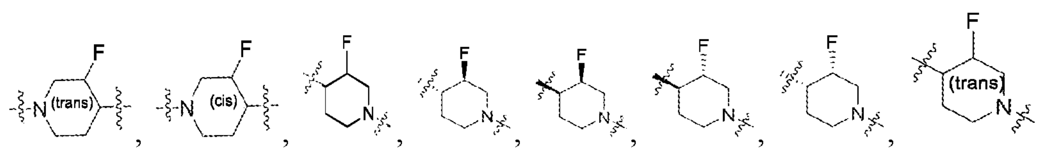
, , , , , , ,

or

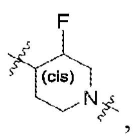
,

which, when substituted, is optionally further substituted with 0 to 4 substituents selected from H, F, $CF_3$, methyl, =O, hydroxymethyl, COOH, CN or $NH_2$;

B is selected from

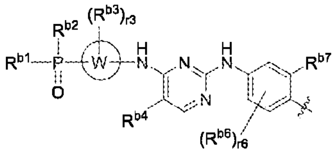
,
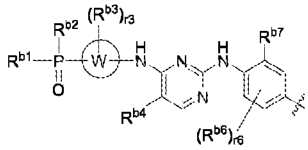
,

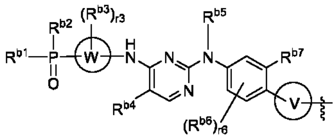
or
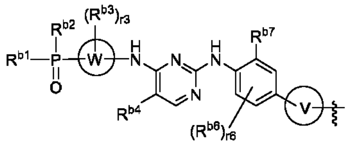
;

ring V is selected from piperidine or piperazine, wherein the piperidine or piperazine is optionally further substituted with 0 to 2 F;
$R^{b1}$ and $R^{b2}$ are selected from methyl;
K is selected from one of the structural fragments shown in Table K-1;.

**6.** The compound or the stereoisomer, deuterated compound, solvate, prodrug, metabolite, pharmaceutically acceptable salt or co-crystal thereof according to claim 5, wherein,

L is selected from -Cy1-, -Cy1-Ak1-, -Cy1-Ak1-Ak2-, -Cy1-Ak1-Ak2-Ak3-, -Cy1-Ak1-Ak2-Ak3-Ak4-, -Cy1-Cy2-, -Cy1-Ak1-Cy2-, -Cy1-Cy2-Ak2-, -Cyl-Ak1-Cy2-Ak2-, -Cy1-Ak1-Cy2-Ak2-Ak3-, -Cy1-Ak1-Cy2-Ak2-Ak3-Ak4-, -Cyl-Cy2-Ak2-Ak3-, -Cy1-Cy2-Ak2-Ak3-Ak4-, -Cy1-Ak1-Cy2-Ak2-Ak3-Ak4-, -Cyl-Ak1-Ak2-Cy3-, -Cy1-Ak1-Ak2-Cy3-Ak3-, -Cy1-Cy2-Cy3-, -Cy1-Ak1-Cy2-Cy3-, -Cy1-Cy2-Ak2-Cy3-, -Cy1-Cy2-Cy3-Ak3-, -Cy1-Ak1-Cy2-Cy3-Ak3-, -Cy1-Cy2-Ak2-Cy3-Ak3-, -Cy1-Ak1-Cy2-Ak2-Cy3-, -Cy1-Ak1-Cy2-Ak2-Cy3-Ak3-, -Cyl-Cy2-Cy3-Ak3-Ak4-, -Cy1-Cy2-Cy3-Ak3-Cy4-, -Cy1-Cy2-Cy3-Cy4-, -Cy1-Ak1-Cy2-Cy3-Cy4-, -Cy1-Cy2-Ak2-Cy3-Cy4-, -Cy1-Cy2-Cy3-Ak3-Cy4-, -Cy1-Cy2-Cy3-Cy4-Ak4-, -Cy1-Ak1-Cy2-Ak2-Cy3-Ak3-Cy4-, -Cy1-Ak1-Cy2-Ak2-Cy3-Cy4-, -Ak1-Ak2-Cy3-Cy4-, -Ak1-Cy2-Ak2-Cy3-, -Ak1-Cy2-Cy3-Ak3-Cy4-, - Ak1-Cy2-Cy3-Cy4-Ak4-Cy5-, -Ak1-Cy2-Ak2-, -Ak1-Ak2-Ak3-Ak4-, -Ak1-Ak2-Ak3-, -Ak1-Ak2-, -Ak1-Ak2-Ak3-Ak4-Ak5-, -Cy1-Cy2-Cy3-Ak3-Ak4-Ak5-, - Cy1-Cy2-Ak2-Cy3-Ak3-Ak4-Ak5-, -Cy1-Ak1-Cy2-Ak2-Ak3-Ak4-Ak5-, -Cyl-Cy2-Cy3-Cy4-Ak4-Ak5-, -Cy1-Ak1-Ak2-Ak3-Ak4-Ak5-, -Ak1-Cy2-Ak2-Ak3-Ak4-Ak5-, -Ak1-Cy2-, -Ak1-Cy2-Ak2-Ak3-Ak4- or -Ak1-Cy2-Ak2-Ak3-;
Ak1, Ak2, Ak3, Ak4 and Ak5 are each independently selected from -O-, - $OCH_2$-, -$CH_2O$-, -$OCH_2CH_2$-, -$CH_2CH_2O$-, -C≡C-, -$C(CH_3)_2$-, -$CH_2$-, -$CH_2CH_2$-, - $CH_2CH_2CH_2$-, -$N(CH_3)$-, -NH-, -$CH_2N(CH_3)$-, -$CH_2NH$-, -$NHCH_2$-, - $CH_2CH_2N(CH_3)$-, -$CH_2CH_2NH$-, -$NHCH_2CH_2$-, -C(=O)-, -C(=O)$CH_2$NH-, - $CH_2$C(=O)NH-, -C(=O)$N(CH_3)$-, -$N(CH_3)$C(=O)-, -C(=O)NH- or -NHC(=O)-.

**7.** The compound or the stereoisomer, deuterated compound, solvate, prodrug, metabolite, pharmaceutically acceptable salt or co-crystal thereof according to claim 6, wherein,
L is selected from a bond or a group in Table A, wherein the left side of the group is linked to B.

**8.** The compound or the stereoisomer, deuterated compound, solvate, prodrug, metabolite, pharmaceutically acceptable salt or co-crystal thereof according to claim 7, wherein,
B is selected from one of the following structural fragments:

| | | |
|---|---|---|
| 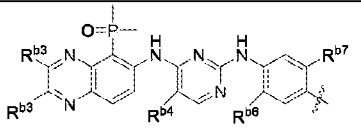 | 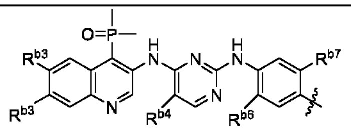 | 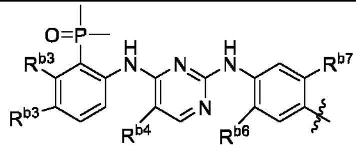 |
| 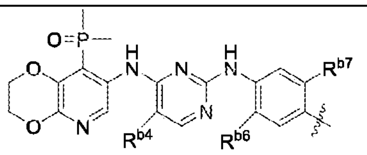 | 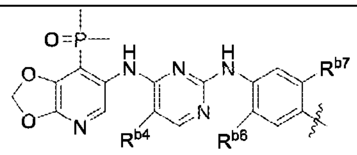 | 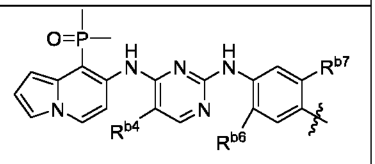 |
| 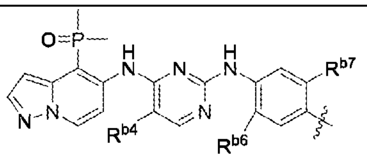 | 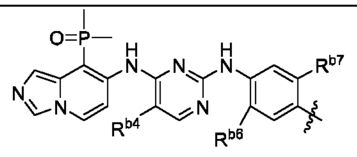 | 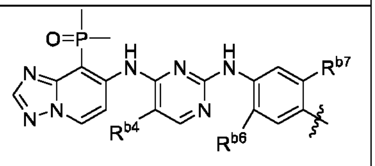 |
| 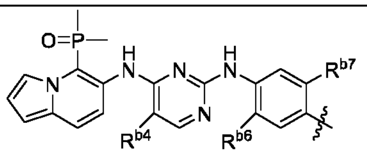 | 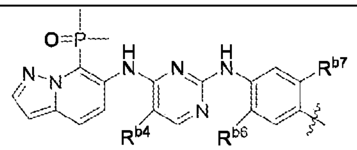 | 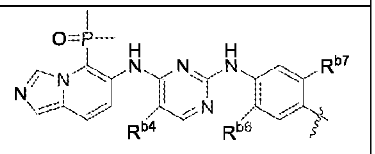 |
| 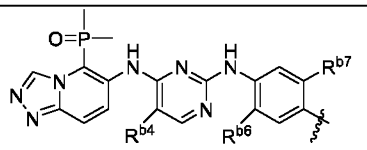 | 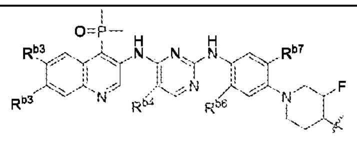 | 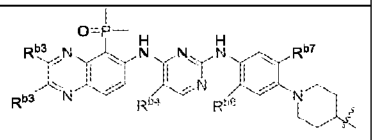 |
| 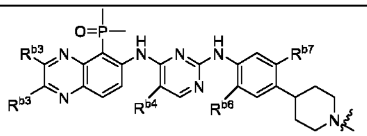 | 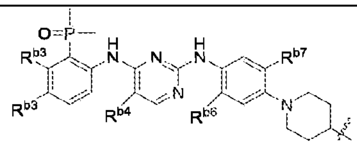 | 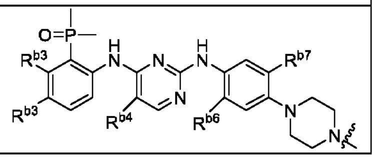 |

| | | |
|---|---|---|
| 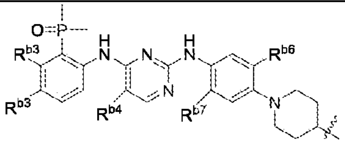 | 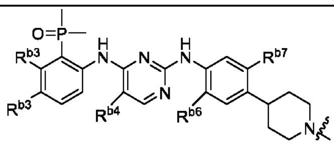 | 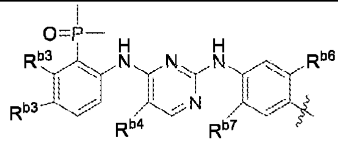 |
| 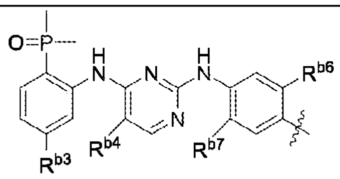 | | |
| 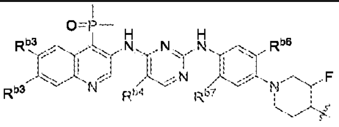 | 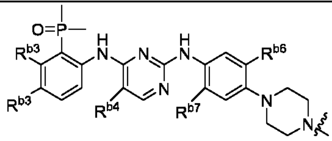 | 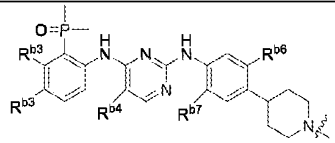 |
| 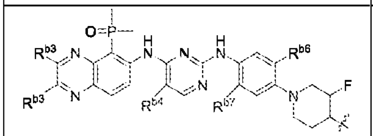 | 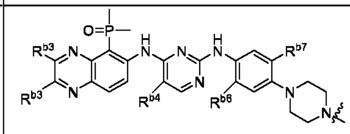 | 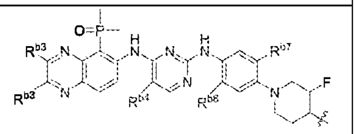 |
| 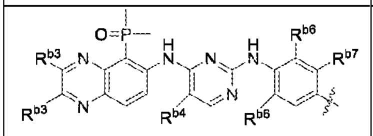 | 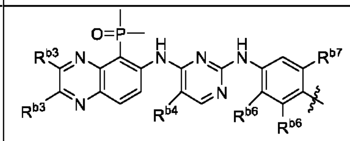 | 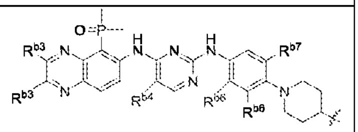 |
| 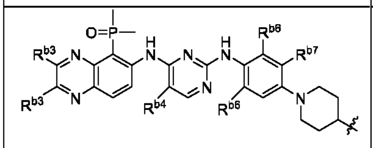 | 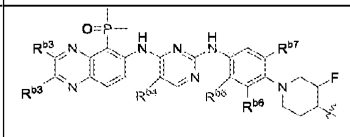 | 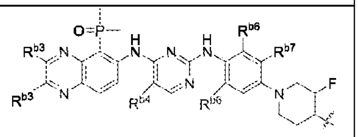 |
| 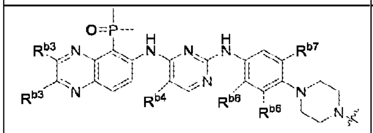 | 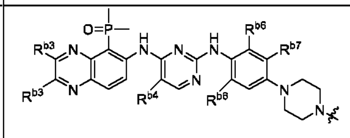 | 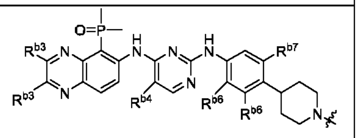 |
| 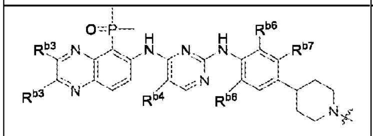 | 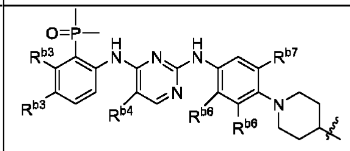 | 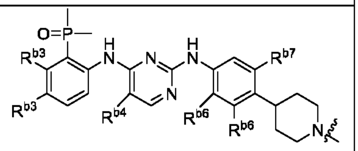 |
| 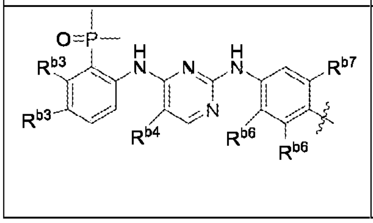 | 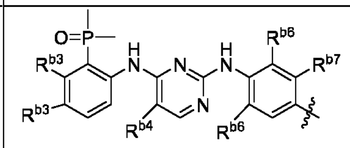 | 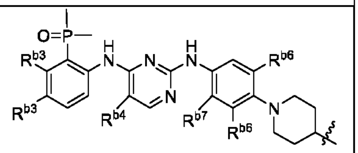 |

| | | |
|---|---|---|
| 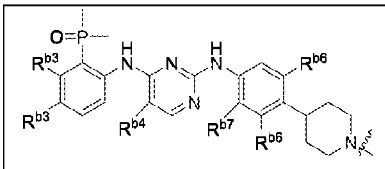 | 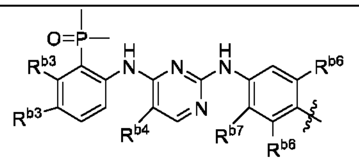 | 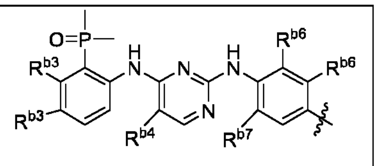 |
| 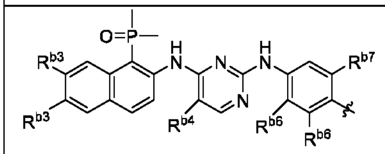 | 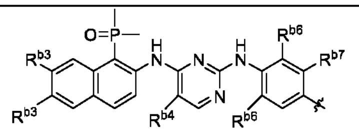 | 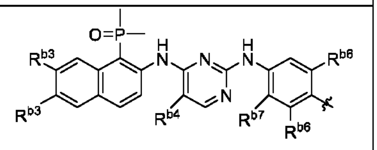 |
| 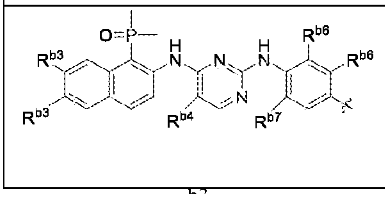 | 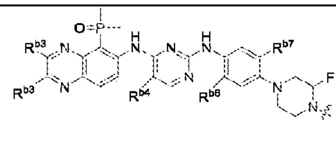; | |

each $R^{b3}$ is independently selected from H, F, Cl, Br, I, OH, CN, methyl, ethyl, isopropyl, propyl, ethynyl, methoxy, cyclopropyl, cyclobutyl, cyclopentyl, cyclohexyl, phenyl or 5- to 6-membered heteroaryl, wherein the phenyl or 5- to 6-membered heteroaryl is optionally further substituted with 0 to 4 substituents selected from H, F, Cl, Br, methyl, ethyl, methoxy or ethoxy, and the heteroaryl contains 1 to 3 heteroatoms selected from O, S or N;
$R^{b4}$ is selected from H, F, Cl or Br;
each $R^{b6}$ is independently selected from H, F, Cl, Br, methyl, ethyl, methoxy or ethoxy;
$R^{b7}$ is selected from cyclopropyl, cyclobutyl, cyclopentyl, cyclohexyl, pyrrole, pyrazole, pyridine or phenyl, wherein the $R^{b7}$ is optionally further substituted with 0 to 4 substituents selected from H, F, Cl, Br, I, OH, =O, CN, $CH_2D$, $CHD_2$, $CD_3$, methyl or ethyl;
K is selected from one of the structural fragments shown in Table K-2.

**9.** The compound or the stereoisomer, deuterated compound, solvate, prodrug, metabolite, pharmaceutically acceptable salt or co-crystal thereof according to claim 8, wherein,

B is selected from one of the structural fragments shown in Table B-1;
K is selected from one of the structural fragments shown in Table K-3.

**10.** The compound or the stereoisomer, deuterated compound, solvate, prodrug, metabolite, pharmaceutically acceptable salt or co-crystal thereof according to claim 1, wherein the compound is selected from one of the structures shown in Table E-1.

**11.** A pharmaceutical composition, comprising a therapeutically effective amount of the compound or the stereoisomer, deuterated compound, solvate, prodrug, metabolite, pharmaceutically acceptable salt or co-crystal thereof according to any one of claims 1 to 10, and a pharmaceutically acceptable carrier, optionally comprising one or more other chemotherapeutic agents.

**12.** The pharmaceutical composition according to claim 11, wherein the therapeutically effective amount means that the pharmaceutical composition is administered at a dosage suitable for a disease to be treated (or prevented), for example, about 0.5 μg to about 50 mg of at least one compound/kg body weight of a subject, preferably about 10 μg to about 100 mg/kg body weight/day.

**13.** Use of the compound or the stereoisomer, deuterated compound, solvate, prodrug, metabolite, pharmaceutically acceptable salt or co-crystal thereof according to any one of claims 1 to 10, or the composition according to claim 11 or 12 in the preparation of a medicament for treating a disease related to EGFR activity or expression level.

**14.** Use of the compound or the stereoisomer, deuterated compound, solvate, prodrug, metabolite, pharmaceutically acceptable salt or co-crystal thereof according to any one of claims 1 to 10, or the composition according to claim

11 or 12 in the preparation of a medicament for treating a disease related to the inhibition or degradation of EGFR.

**15.** The use according to claim 13 or 14, **characterized in that** the disease is selected from cancer, preferably non-small cell lung cancer.

**INTERNATIONAL SEARCH REPORT**

International application No.
**PCT/CN2022/090243**

**A. CLASSIFICATION OF SUBJECT MATTER**

C07D 401/14(2006.01)i; C07F 9/53(2006.01)i; A61K 31/506(2006.01)i; A61K 31/675(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D; C07F; A61K; A61P

Documentation searched other than minimum documentation to the extent that such documents are included in the fields searched

Electronic data base consulted during the international search (name of data base and, where practicable, search terms used)

CNKI, WPI, CNABS, CNPAT, STN: 四川海思科制药有限公司, 膦酰, 嘧啶, 苯, 癌症, 抑制剂, pyrimidin, phosphoryl, phenyl, cancer, inhibitor, structural formula search

**C. DOCUMENTS CONSIDERED TO BE RELEVANT**

| Category* | Citation of document, with indication, where appropriate, of the relevant passages | Relevant to claim No. |
|---|---|---|
| X | CN 109912655 A (SHANGHAI TECHNOLOGY UNIVERSITY) 21 June 2019 (2019-06-21) embodiments 1-86 and 158-160, and claims 1-44 | 1-15 |
| X | WO 2021036922 A1 (BEIJING TIDE PHARMACEUTICAL CO., LTD.) 04 March 2021 (2021-03-04) claims 1-33 | 1-15 |
| X | CN 112079866 A (SHANGHAI TECH UNIVERSITY) 15 December 2020 (2020-12-15) claims 1-47, and embodiments 15-23, 27-34 and 36-42 | 1-15 |
| X | CN 110357889 A (SHANGHAI TECH UNIVERSITY) 22 October 2019 (2019-10-22) embodiments 52-88, claims 1-82, and description, paragraph 0172 | 1-15 |
| X | CN 111285849 A (SHANGHAI QINGDONG BIOTECHNOLOGY CO., LTD.) 16 June 2020 (2020-06-16) embodiment 17, and claims 1-22 | 1-15 |
| PX | WO 2022012622 A1 (BEIGENE, LTD. et al.) 20 January 2022 (2022-01-20) claims 1-54, and embodiments 1-756 | 1-15 |
| PX | WO 2022012623 A1 (BEIGENE, LTD. et al.) 20 January 2022 (2022-01-20) claims 1-52, and embodiments 1-756 | 1-15 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **15 July 2022** | **27 July 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

INTERNATIONAL SEARCH REPORT

International application No.
PCT/CN2022/090243

C. DOCUMENTS CONSIDERED TO BE RELEVANT

| Category* | Citation of document, with indication, where appropriate, of the relevant passages | Relevant to claim No. |
|---|---|---|
| PX | WO 2021173677 A1 (DANA-FARBER CANCER INSTITUTE, INC. et al.) 02 September 2021 (2021-09-02)<br>claims 1-47, and embodiment 14 | 1-15 |
| PX | REN， Chaowei 等 (REN, Chaowei et al.). "Discovery of a Brigatinib Degrader SIAIS164018 with Destroying Metastasis-Related Oncoproteins and a Reshuffling Kinome Profile"<br>*J. Med. Chem.,* Vol. 64, 17 June 2021 (2021-06-17),<br>pp. 9152-9165 | 1-15 |

Form PCT/ISA/210 (second sheet) (January 2015)

**INTERNATIONAL SEARCH REPORT**
**Information on patent family members**

International application No.
**PCT/CN2022/090243**

| Patent document cited in search report | Publication date (day/month/year) | Patent family member(s) | Publication date (day/month/year) |
|---|---|---|---|
| CN 109912655 A | 21 June 2019 | AU 2018382122 A1 | 16 July 2020 |
| | | US 2020306273 A1 | 01 October 2020 |
| | | JP 2021506820 A | 22 February 2021 |
| | | EP 3725771 A1 | 21 October 2020 |
| | | WO 2019114770 A1 | 20 June 2019 |
| | | KR 20200108838 A | 21 September 2020 |
| | | CA 3085645 A1 | 20 June 2019 |
| WO 2021036922 A1 | 04 March 2021 | TW 202115026 A | 16 April 2021 |
| | | EP 4019021 A1 | 29 June 2022 |
| | | KR 20220051244 A | 26 April 2022 |
| CN 112079866 A | 15 December 2020 | KR 20220024507 A | 03 March 2022 |
| | | WO 2020249048 A1 | 17 December 2020 |
| | | CA 3141413 A1 | 17 December 2020 |
| CN 110357889 A | 22 October 2019 | WO 2019196812 A1 | 17 October 2019 |
| | | AU 2019251151 A1 | 26 November 2020 |
| | | JP 2021521163 A | 26 August 2021 |
| | | EP 3778590 A1 | 17 February 2021 |
| | | KR 20210006364 A | 18 January 2021 |
| | | CA 3096790 A1 | 17 October 2019 |
| | | US 2022117982 A1 | 21 April 2022 |
| CN 111285849 A | 16 June 2020 | None | |
| WO 2022012622 A1 | 20 January 2022 | None | |
| WO 2022012623 A1 | 20 January 2022 | None | |
| WO 2021173677 A1 | 02 September 2021 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020147838 A
- WO 2017018803 A
- WO 2021127283 A
- WO 2021036922 A
- WO 2019114770 A


### Non-patent literature cited in the description


- *Nat. Rev. Cancer,* 2007, vol. 7, 169-181
- *Expert Opin. Ther. Targets,* 2012, vol. 16, 15-31
- *Cells,* 2019, vol. 8, 350-361
- *ACS Chem. Biol.,* 2017, vol. 12, 892-898
- *Drug Discovery Today Technol.,* 2019, vol. 31, 15-27
- *CHEMICAL ABSTRACTS,* 25952-53-8
- *CHEMICAL ABSTRACTS,* 2592-95-2
- *CHEMICAL ABSTRACTS,* 1445085-77-7
- *CHEMICAL ABSTRACTS,* 95464-05-4
- *CHEMICAL ABSTRACTS,* 847818-55-7
- *CHEMICAL ABSTRACTS,* 847818-70-6